# EUROPEAN PATENT SPECIFICATION

(11) **EP 4 132 908 B1**
(45) Date of publication and mention of the grant of the patent: **16.09.2026**
(21) Application number: 21725849.0
(22) Date of filing: 02.04.2021
(51) Int. Cl.: C07D 209/08, C07D 209/18, C07D 209/30, C07D 401/06, C07D 403/04, C07D 403/06, C07D 405/14, C07D 409/04, C07D 409/14, C07D 413/04, C07D 413/06, C07D 417/04, C07D 417/06, C07D 471/04, C07D 493/08, A61K 31/404, A61K 31/35, A61K 31/381, A61P 1/00, A61P 1/14, A61P 1/16

(54) **INDOLE DERIVATIVES AS ALPHA-1-ANTITRYPSIN MODULATORS FOR TREATING ALPHA-1-ANTITRYPSIN DEFICIENCY (AATD)**
INDOLDERIVATE ALS ALPHA-1-ANTITRYPSINMODULATOREN ZUR BEHANDLUNG VON ALPHA-1-ANTITRYPSIN MANGEL (AATD)
DÉRIVÉS DE INDOLE EN TANT QUE MODULATEURS D'ALPHA-1-ANTITRYPSIN POUR LE TRAITEMENT DE CARENCE D'ALPHA-1-ANTITRYPSIN (AATD)

(30) Priority: 03.04.2020 US 202063004717 P
(43) Date of publication of application: 15.02.2023
(73) Proprietor: Vertex Pharmaceuticals Incorporated, Boston, MA 02210 (US)
(72) Inventor: GIROUX, Simon, Boston, Massachusetts 02210 (US); CLARK, Michael Philip, Boston, Massachusetts 02210 (US); BRODNEY, Michael Aaron, Boston, Massachusetts 02210 (US); NUHANT, Philippe Marcel, Boston, Massachusetts 02210 (US); ALLEN, Emily Elizabeth, Boston, Massachusetts 02210 (US); FIMOGNARI, JR., Robert Francis, Boston, Massachusetts 02210 (US); ZAKY, Mariam, Boston, Massachusetts 02210 (US); BOYD, Michael John, Boston, Massachusetts 02210 (US); DEININGER, David D., Boston, Massachusetts 02210 (US); ZHANG, Hu, Boston, Massachusetts 02210 (US); DENG, Hongbo, Boston, Massachusetts 02210 (US); COLLIER, Philip Noel, Boston, Massachusetts 02210 (US); MAXWELL, Brad, Boston, Massachusetts 02210 (US); WAAL, Nathan D., Boston, Massachusetts 02210 (US); RONKIN, Steven M., Boston, Massachusetts 02210 (US); WANG, Jian, Boston, Massachusetts 02210 (US); TANG, Qing, Boston, Massachusetts 02210 (US); FLEMING, Gabrielle Simone, Boston, Massachusetts 02210 (US); JONES, Peter, Boston, Massachusetts 02210 (US); BOUCHER, Diane Marie, Boston, Massachusetts 02210 (US); FANNING, Lev T.D., Boston, Massachusetts 02210 (US); HALL, Amy B., Boston, Massachusetts 02210 (US); HURLEY, Dennis James, Boston, Massachusetts 02210 (US); JOHNSON, JR., Mac Arthur, Boston, Massachusetts 02210 (US); MAXWELL, John Patrick, Boston, Massachusetts 02210 (US); SWETT, Rebecca Jane, Boston, Massachusetts 02210 (US); TAPLEY, Timothy Lewis, Boston, Massachusetts 02210 (US); THOMSON, Stephen A., Boston, Massachusetts 02210 (US); DAMAGNEZ, Veronique, Boston, Massachusetts 02210 (US); COTTRELL, Kevin Michael, Boston, Massachusetts 02210 (US); BANDARAGE, Upul Keerthi, Boston, Massachusetts 02210 (US)
(74) Representative: Carpmaels & Ransford LLP
(86) International application number: PCT/US2021/025614
(87) International publication number: WO 2021/203023

(56) References cited:
- WO-A1-2009/127686
- WO-A1-2019/243841
- WO-A1-96/37467
- US-A1- 2003 165 712
- US-A1- 2013 167 932
- US-A1- 2013 319 530
- JAFARPOUR FARNAZ ET AL: "A Fast Track to Indoles and Annulated Indoles through ortho - vs ipso- Amination of Aryl Halides", ORGANIC LETTERS ., vol. 21, no. 24, 20 December 2019 (2019-12-20), US, pages 10143 - 10148, XP055811010, ISSN: 1523-7060, DOI: 10.1021/acs.orglett.9b04202
- HE LIN ET AL: "Transition-metal-free synthesis of multisubstitutedN-arylindoles via reaction of arynes and [alpha]-amino ket", TETRAHEDRON, vol. 70, no. 14, 3 December 2019 (2019-12-03), pages 2400 - 2405, XP028638160, ISSN: 0040-4020, DOI: 10.1016/J.TET.2014.02.028

## Description

This application claims the benefit of priority of U.S. Provisional Application No. 63/004,717, filed April 3, 2020.

The disclosure provides compounds that are capable of modulating alpha-1 antitrypsin (AAT) activity and the comopounds for use in methods of treating alpha-1 antitrypsin deficiency (AATD) by administering one or more such compounds.

References in the present description to methods of medical treatment or medical uses have to be understood as references to the compounds of the present invention for use in methods of medical treatment. Methods of medical treatment or medical uses are not encompassed by the claimed invention.

AATD is a genetic disorder characterized by low circulating levels of AAT. While treatments for AATD exist, there is currently no cure. AAT is produced primarily in liver cells and secreted into the blood, but it is also made by other cell types including lung epithelial cells and certain white blood cells. AAT inhibits several serine proteases secreted by inflammatory cells (most notably neutrophil elastase [NE], proteinase 3, and cathepsin G) and thus protects organs such as the lung from protease-induced damage, especially during periods of inflammation.

The mutation most commonly associated with AATD involves a substitution of lysine for glutamic acid (E342K) in the SERPINA1 gene that encodes the AAT protein. This mutation, known as the Z mutation or the Z allele, leads to misfolding of the translated protein, which is therefore not secreted into the bloodstream and can polymerize within the producing cell. Consequently, circulating AAT levels in individuals homozygous for the Z allele (*PiZZ*) are markedly reduced; only approximately 15% of mutant Z-AAT protein folds correctly and is secreted by the cell. An additional consequence of the Z mutation is that the secreted Z-AAT has reduced activity compared to wild-type protein, with 40% to 80% of normal antiprotease activity (American thoracic society/European respiratory society, Am J Respir Crit Care Med. 2003;168(7):818-900; and Ogushi et al. J Clin Invest. 1987;80(5):1366-74).

The accumulation of polymerized Z-AAT protein within hepatocytes results in a gain-of-function cytotoxicity that can result in cirrhosis or liver cancer later in life and neonatal liver disease in 12% of patients. This accumulation may spontaneously remit but can be fatal in a small number of children. The deficiency of circulating AAT results in unregulated protease activity that degrades lung tissue over time, resulting in emphysema, a form of chronic obstructive pulmonary disease (COPD). This effect is severe in *PiZZ* individuals and typically manifests in middle age, resulting in a decline in quality of life and shortened lifespan (mean 68 years of age) (Tanash et al. Int J Chron Obstruct Pulm Dis. 2016;11:1663-9). The effect is more pronounced in *PiZZ* individuals who smoke, resulting in an even further shortened lifespan (58 years). (Piitulainen and Tanash, COPD 2015;12(1):36-41). *PiZZ* individuals account for the majority of those with clinically relevant AATD lung disease. Accordingly, there is a need for additional and effective treatments for AATD.

A milder form of AATD is associated with the SZ genotype in which the Z-allele is combined with an S-allele. The S allele is associated with somewhat reduced levels of circulating AAT but causes no cytotoxicity in liver cells. The result is clinically significant lung disease but not liver disease. (Fregonese and Stolk, Orphanet J Rare Dis. 2008; 33:16). As with the ZZ genotype, the deficiency of circulating AAT in subjects with the SZ genotype results in unregulated protease activity that degrades lung tissue over time and can result in emphysema, particularly in smokers.

The current standard of care for AAT deficient individuals who have or show signs of developing significant lung or liver disease is augmentation therapy or protein replacement therapy. Augmentation therapy involves administration of a human AAT protein concentrate purified from pooled donor plasma to augment the missing AAT. Although infusions of the plasma protein have been shown to improve survival or slow the rate of emphysema progression, augmentation therapy is often not sufficient under challenging conditions such as during an active lung infection. Similarly, although protein replacement therapy shows promise in delaying progression of disease, augmentation does not restore the normal physiological regulation of AAT in patients and efficacy has been difficult to demonstrate. In addition, augmentation therapy requires weekly visits for treatment and augmentation therapy cannot address liver disease, which is driven by the toxic gain-of-function of the Z allele. Thus, there is a continuing need for new and more effective treatments for AATD.

JAFARPOUR FARNAZ ET AL, "A Fast Track to Indoles and Annulated Indoles through ortho - vs ipso- Amination of Aryl Halides", ORGANIC LETTERS ., US, (20191220), vol. 21, no. 24, doi:10.1021/acs.orglett.9b04202, ISSN 1523-7060, pages 10143 - 10148, XP055811010 relates to *"[a] complementary site selective ortho- vs ipso-amination of aryl halides using non-electrophilic amine sources for construction of indole scaffolds".*

HE LIN ET AL, "Transition-metal-free synthesis of multisubstitutedN-arylindoles via reaction of arynes and [alpha]-amino ket", TETRAHEDRON, (20191203), vol. 70, no. 14, doi:10.1016/J.TET.2014.02.028, ISSN 0040-4020, pages 2400 - 2405, XP028638160 relates to *"[a] simple transition-metal-free protocol for the synthesis of indoles".*

US 2013/319530 A1 relates to *"a compound useful as a photoelectric conversion dye having excellent photoelectric conversion performance".*

US 2013/167932 A1 relates to *"an indole compound represented by [*...*] general formula (1)".*

WO 2009/127686 A1 relates to *"a compound of formula (I) or a pharmaceutically acceptable ester, amide, carbamate, solvate or salt thereof, including a salt of such an ester, amide or carbamate, and a solvate of such an ester, amide, carbamate or salt".*

US 2003/165712 A1 relates to *"[a]n organic EL device which contains an anode, a cathode, and at least one organic thin-file layer including a light emitting layer which contains a compound represented by [... ] general formula (1), (2) or (3)".*

WO 96/37467 A1 relates to a *"compound of Formula (I) useful in the treatment of cyclooxygenase-2 mediated diseases".*

WO 2019/243841 A1 relates to *"compounds, compositions, combinations and medicaments containing said compounds and processes for their preparation".*

The scope of the present invention is defined by the claims. The following aspects refer to the disclosure, and not necessarily to the claimed invention.

The following disclosure is considered as useful for understanding the invention, but the invention is defined by the claims. Compounds 143, 429-437, B1-B25, W1-32, P1-20, P59-P64, P69, and P94-P102 are not recited in claim 13.

One aspect of the disclosure provides compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives that can be employed in the treatment of AATD. For example, compounds of Formula (I), tautomers thereof, deuterated derivatives of those compounds or tautomers, or pharmaceutically acceptable salts of any of the foregoing, can be depicted as: 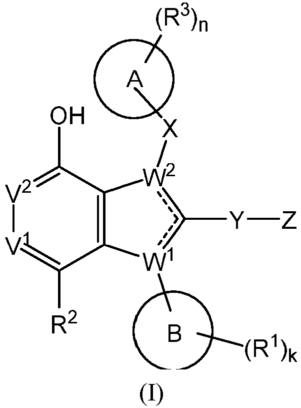 wherein:
-̅ -̅ -̅ -̅ -̅ -̅, for each of the two occurrences, is a single bond or a double bond, provided that one is a single bond and the other is a double bond;
**V¹** and **V²** are each independently N or -C**R²**;
**W¹** and **W²** are each independently N or C, provided that one of W¹ and W² is N and the other is C;
**X** is absent or a bond, -(C**R^{a}R^{b})ₚ**-, or -SO₂-;
**Y** is absent or a bond, -(C**R^{c}R^{d})_{q}**-, -C(=O)-, or -SO₂-;
**R^{a}** and **R^{b},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**R^{c}** and **R^{d},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**Ring A** is C₃-C₁₂ carbocyclyl, 3 to 12-membered heterocyclyl, C₆ or C₁₀ aryl, or 5 to 10-membered heteroaryl; provided that when **W¹** is N and **W²** is C, **Ring A** is not 1,5,6,7-tetrahydro-4*H*-indol-4-onyl or a tautomer thereof;
**Ring B** is C₄-C₁₂ cycloalkyl, C₆ or C₁₀ aryl, 5 to 10-membered heteroaryl, or benzyl;
**Z** is 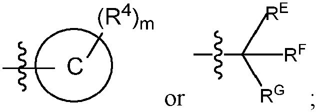 wherein:
   **Ring C** is C₃-C₁₂ cycloalkyl, 3 to 12-membered heterocyclyl, C₆ or C₁₀ aryl, or 5 to 10-membered heteroaryl;
   provided that when **Ring C** is phenyl, the phenyl is substituted with **R⁴;** provided that when **Ring C** is phenyl, **Y** cannot be -SO₂-; and
   provided that when **Ring B** is benzyl, **Ring C** cannot be pyridinyl or indolyl;
   **R^{E}, R^{F},** and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
      the C₁-C₆ alkyl or the C₂-C₆ alkenyl of any one of **R^{E}, R^{F},** and **R^{G}** is optionally substituted with 1 to 3 groups selected from cyano, -C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)ᵣ**R^{s}**, and -S(=O)ᵣN**R^{p}R^{q}**; wherein:
      **R^{p}, R^{q},** and **R^{r},** for each occurrence, are each independently hydrogen, C₁-C₄ alkyl, C₃-C₆ cycloalkyl, or 3 to 6-membered heterocyclyl; wherein:
         the C₁-C₄ alkyl of any one of **R^{p}**, **R^{q},** and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
         the C₃-C₆ cycloalkyl or the 3 to 6-membered heterocyclyl of any one of **R^{p}**, **R^{q},** and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
      **R^{s},** for each occurrence, is independently hydrogen, C₁-C₄ alkyl, C₃-C₆ cycloalkyl, phenyl, or 5 or 6-membered heteroaryl; wherein:
         the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
         the C₃-C₆ cycloalkyl, the phenyl, or the 5 or 6-membered heteroaryl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl);
   **R²,** for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, phenyl, or 5 or 6-membered heteroaryl; wherein:
      the C₁-C₆ alkyl, the C₂-C₆ alkenyl, or the C₃-C₆ cycloalkyl of **R²** is optionally substituted with 1 to 3 groups selected from cyano, -C(=O)**R^{k}**, -C(=O)O**R^{k}**, -C(=O)N**R^{h}Rⁱ**, -N**R^{h}Rⁱ**, -N**R^{h}**C(=O)**R^{k},** -N**R^{h}**C(=O)O**R^{k}**, -N**R^{h}**C(=O)N**RⁱR^{j}**, -N**R^{h}**S(=O)ₛ**R^{k}**, -O**R^{k}**, -OC(=O)**R^{k}**, -OC(=O)O**R^{k}**, -OC(=O)N**R^{h}Rⁱ**, -S(=O)ₛ**R^{k}**, and S(=O)ₛN**R^{h}Rⁱ**; wherein:
      **R^{h}, Rⁱ,** and **R^{j},** for each occurrence, are each independently hydrogen, C₁-C₄ alkyl, or C₃-C₆ cycloalkyl; wherein:
         the C₁-C₄ alkyl of any one of **R^{h}**, **Rⁱ,** and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl of any one of **R^{h}, Rⁱ,** and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
      **R^{k},** for each occurrence, is independently hydrogen, C₁-C₄ alkyl, C₃-C₆ cycloalkyl, phenyl, or 5 or 6-membered heteroaryl; wherein:
         -O**R^{k}** cannot be -OH; the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁- C₂ alkyl)₂;
   **R³** and **R⁴,** for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)**ₜR^{y}**, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R^{v}**S(=O)**ₜR^{y}**, -O**R^{y}**, -OC(=O)**R^{y}**, -OC(=O)O**R^{y}**, -OC(=O)N**R^{v}R^{w}**, -S(=O)ₜ**R^{y}**,
   -S(=O)**ₜ**N**R^{v}R^{w}**, -S(=O)**ₜ**N**R^{v}**C(=O)**R^{y}**, -P(=O)**R^{z}R^{z}**, phenyl, or a 5 or 6-membered heteroaryl; wherein:
      the C₁-C₆ alkyl, the C₂-C₆ alkenyl, or the C₃-C₆ cycloalkyl of any one of **R³** and **R⁴** is optionally substituted with 1 to 3 groups selected from cyano, -C(=O)**R^{y}**, -C(=O)O**R^{y}**, -C(=O)NR^{v}R^{w}, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R^{v}**S(=O)ᵣ**R^{y}**, -O**R^{y}**, -OC(=O)**R^{y}**, -OC(=O)O**R^{y}**, -OC(=O)N**R^{v}R^{w}**, -S(=O)**ₜR^{y}**, and -S(=O)**ₜ**N**R^{v}R^{w}**; wherein:
      **R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
         the C₁-C₄ alkyl of any one of **R^{v}**, **R^{w}**, and **R^{x}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl, the 5 or 6-membered heterocyclyl, or the 5 or 6-membered heteroaryl of any one of **R^{v}, R^{w},** and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
      **R^{y},** for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
         the C₁-C₄ alkyl of **R^{y}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, -NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{z},** for each occurrence, is independently C₁-C₂ alkyl, -OH, or -O(C₁-C₂ alkyl);
**k** is an integer selected from 1, 2, and 3;
**m** and **n** are each independently an integer selected from 0, 1, 2, and 3;
**p, r, s,** and **t** are each independently an integer selected from 1 and 2; and
**q** is an integer selected from 1, 2, and 3.

In some embodiments, in the compound, tautomer, deuterated derivative, or pharmaceutically acceptable salt of the disclosure, **p, q, r, s,** and **t** are each independently an integer selected from 1 and 2, and all other variables are as defined for Formula (I).

The compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)) are modulators of AAT activity. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of 2.0 µM or less when tested in an AAT Function Assay. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of less than 0.5 µM when tested in an AAT Function Assay.

In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an IC₅₀ of 5.0 µM or less when tested in a Z-AAT Elastase Activity Assay. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an IC₅₀ of less than 2.0 µM when tested in a Z-AAT Elastase Activity Assay.

In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of 2.0 µM or less when tested in an AAT Function Assay and have an IC₅₀ of 5.0 µM or less when tested in a Z-AAT Elastase Activity Assay. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of less than 0.5 µM when tested in an AAT Function Assay and have an IC₅₀ of 5.0 µM or less when tested in a Z-AAT Elastase Activity Assay. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of 2.0 µM or less when tested in an AAT Function Assay and have an IC₅₀ of less than 2.0 µM when tested in a Z-AAT Elastase Activity Assay. In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives have an EC₅₀ of less than 0.5 µM when tested in an AAT Function Assay and have an IC₅₀ of less than 2.0 µM when tested in a Z-AAT Elastase Activity Assay.

In some embodiments, the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those tautomers and compounds, and pharmaceutically acceptable salts of those compounds, tautomers, or deuterated derivatives are provided for use in the treatment of AATD. In some embodiments, the compounds are selected from Compounds 1-457, Compounds 458-532, Compounds P1-P225, Compounds B1-B25, and Compounds W1-W4, W6-W23, W25, W26, W28, W30, and W31 (e.g., Compounds 1-457, e.g., Compounds 1-142, 144-177, 179-399, 401-422, 425-433, and 435-457), tautomers of Compounds 1-457, Compounds 458-532, Compounds P1-P225, Compounds B1-B25, and Compounds W1-W4, W6-W23, W25, W26, W28, W30, and W31 (e.g., tautomers of Compounds 1-457), deuterated derivatives of those compounds or tautomers, and pharmaceutically acceptable salts of any of the foregoing and are for use in the treatment of AATD. In one aspect, the compounds of Formulae (I) are selected from Compounds 1-142, 144-177, 179-399, 401-422, 425-433, and 435-457, tautomers of those compounds, deuterated derivatives of those compounds or tautomers, and pharmaceutically acceptable salts of any of the foregoing and are for use in the treatment of AATD.

In some embodiments, the disclosure provides pharmaceutical compositions comprising at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing. In some embodiments, the pharmaceutical compositions may comprise a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing. These compositions may further include at least one additional active pharmaceutical ingredient and/or at least one carrier. These compositions may further include at least one additional active pharmaceutical ingredient. These compositions may further include at least one carrier. These compositions may further include at least one additional active pharmaceutical ingredient and at least one carrier. These compositions may further include at least one additional active pharmaceutical ingredient or at least one carrier.

In some embodiments, the disclosure provides pharmaceutical compositions comprising at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), (Xa)-(Xf), (XIa)-(Xe), and (XIIa)-(XIIe) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing. In some embodiments, the pharmaceutical compositions may comprise a compound selected from Compounds 1-457, Compounds 458-532, Compounds P1-P225, Compounds B1-B25, and Compounds W1-W32 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing. These compositions may further include at least one additional active pharmaceutical ingredient and/or at least one carrier.

Another aspect of the disclosure provides methods of treating AATD comprising administering to a subject in need thereof, at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing or a pharmaceutical composition comprising the at least one such compound, tautomer, deuterated derivative, or pharmaceutically acceptable salt. In some embodiments, the methods comprise administering a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing. In some embodiments, the subject in need of treatment carries the ZZ mutation. In some embodiments, the subject in need of treatment carries the SZ mutation.

In some embodiments, the methods of treatment include administration of at least one additional active agent to the subject in need thereof, either in the same pharmaceutical composition as the at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing, or as separate compositions. In some embodiments, the methods comprise administering a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing with at least one additional active agent either in the same pharmaceutical composition or in a separate composition. In some embodiments, the subject in need of treatment carries the ZZ mutation. In some embodiments, the subject in need of treatment carries the SZ mutation.

In some embodiments, the methods of treatment include administration of at least one additional active agent to the subject in need thereof, either in the same pharmaceutical composition as the at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing, or as separate compositions, wherein the additional active agent is alpha-1 antitrypsin protein (AAT) from the blood plasma of healthy human donors. In some embodiments, the methods comprise administering a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing with at least one additional active agent either in the same pharmaceutical composition or in a separate composition, wherein the additional active agent is alpha-1 antitrypsin protein (AAT) from the blood plasma of healthy human donors.

In some embodiments, the methods of treatment include administration of at least one additional active agent to the subject in need thereof, either in the same pharmaceutical composition as the at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds and tautomers, and pharmaceutically acceptable salts of any of the foregoing, or as separate compositions, wherein the additional active agent is recombinant AAT. In some embodiments, the methods comprise administering a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing with at least one additional active agent either in the same pharmaceutical composition or in a separate composition, wherein the additional active agent is recombinant AAT.

Also provided are methods of modulating AAT, comprising administering to a subject in need thereof, at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), and tautomers of those compounds, deuterated derivatives of those compounds and tautomers, and pharmaceutically acceptable salts of any of the foregoing or a pharmaceutical composition comprising the at least one compound, tautomer, deuterated derivative, or pharmaceutically acceptable salt. In some embodiments, the methods of modulating AAT comprise administering at least one compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing or a pharmaceutical composition comprising the at least one such compound, tautomer, deuterated derivative or pharmaceutically acceptable salt.

Another aspect of the disclosure provides Compounds B1-B25 and Compounds W1-W32, as well as tautomers of those compounds, deuterated derivatives of those tautomers and compounds, and pharmaceutically acceptable salts of those compounds, tautomers, or deuterated derivatives that can be employed in the treatment of AATD.

In some embodiments, the disclosure provides pharmaceutical compositions comprising at least one compound selected from compounds of Formulae (XIa)-(XIe) and (XIIa)-(XIIe) (e.g., Compounds B1-B25 and Compounds W1-W32), tautomers of those compounds, deuterated derivatives of those compounds and tautomers, and pharmaceutically acceptable salts of any of the foregoing. These compositions may further include at least one additional active pharmaceutical ingredient and/or at least one carrier.

Another aspect of the disclosure provides methods of treating AATD comprising administering to a subject in need thereof, at least one compound selected from compounds of Formulae (XIa)-(XIe) and (XIIa)-(XIIe) (e.g., Compounds B1-B25 and Compounds W1-W32), tautomers of those compounds, deuterated derivatives of those compounds and tautomers, and pharmaceutically acceptable salts of any of the foregoing or a pharmaceutical composition comprising the at least one such compound, tautomer, deuterated derivative, or pharmaceutically acceptable salt.

In some embodiments, the methods of treatment include administration of at least one additional active agent to the subject in need thereof, either in the same pharmaceutical composition as the at least one compound selected from compounds of Formulae (XIa)-(XIe) and (XIIa)-(XIIe) (e.g., Compounds B 1-B25 and Compounds W1-W32), tautomers of those compounds, deuterated derivatives of those compounds and tautomers, and pharmaceutically acceptable salts of any of the foregoing, or as separate compositions. In some embodiments, the subject in need of treatment carries the ZZ mutation. In some embodiments, the subject in need of treatment carries the SZ mutation.

In some embodiments, the methods of treatment include administration of at least one additional active agent to the subject in need thereof, either in the same pharmaceutical composition as the at least one compound selected from compounds of Formulae (XIa)-(XIe) and (XIIa)-(XIIe) (e.g., Compounds B 1-B25 and Compounds W1-W32), tautomers of those compounds, deuterated derivatives of those compounds and tautomers, and pharmaceutically acceptable salts of any of the foregoing, or as separate compositions, wherein the additional active agent is alpha-1 antitrypsin protein (AAT) from the blood plasma of healthy human donors.

In some embodiments, the methods of treatment include administration of at least one additional active agent to the subject in need thereof, either in the same pharmaceutical composition as the at least one compound selected from compounds of Formulae (XIa)-(XIe) and (XIIa)-(XIIe) (e.g., Compounds B1-B25 and Compounds W1-W32), tautomers of those compounds, deuterated derivatives of those compounds and tautomers, and pharmaceutically acceptable salts of any of the foregoing, or as separate compositions, wherein the additional active agent is recombinant AAT.

Also provided are methods of modulating AAT, comprising administering to a subject in need thereof, at least one compound selected from compounds of Formulae (XIa)-(XIe) and (XIIa)-(XIIe) (e.g., Compounds B1-B25 and Compounds W1-W32), and tautomers of those compounds, deuterated derivatives of those compounds and tautomers, and pharmaceutically acceptable salts of any of the foregoing or a pharmaceutical composition comprising the at least one compound, tautomer, deuterated derivative, or pharmaceutically acceptable salt.

Also provided is a compound of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), and tautomers of those compounds, deuterated derivatives of those compounds and tautomers, and pharmaceutically acceptable salts of any of the foregoing, for use in therapy. In some embodiments, there is provided a compound selected from Compounds 1-457, Compounds 458-531, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing, for use in therapy.

Also provided is a pharmaceutical composition comprising a compound of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), and tautomers of those compounds, deuterated derivatives of those compounds and tautomers, and pharmaceutically acceptable salts of any of the foregoing, for use in therapy. In some embodiments, there is provided a pharmaceutical composition comprising a compound selected from Compounds 1-457, Compounds 458-531, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing, for use in therapy.

Also provided is a compound of Formulae (XIa)-(XIe) and (XIIa)-(XIIe) (e.g., Compounds B1-B25 and Compounds W1-W32), and tautomers of those compounds, deuterated derivatives of those compounds and tautomers, and pharmaceutically acceptable salts of any of the foregoing, for use in therapy. In some embodiments, there is provided a compound selected from Compounds B1-B25 and Compounds W1-W32, tautomers of those compounds, deuterated derivatives of those compounds and tautomers, and pharmaceutically acceptable salts of any of the foregoing, for use in therapy.

Also provided is a pharmaceutical composition comprising a compound of Formulae (XIa)-(XIe) and (XIIa)-(XIIe) (e.g., Compounds B1-B25 and Compounds W1-W32), and tautomers of those compounds, deuterated derivatives of those compounds and tautomers, and pharmaceutically acceptable salts of any of the foregoing, for use in therapy. In some embodiments, there is provided a pharmaceutical composition comprising a compound selected from Compounds B1-B25 and Compounds W1-W32, tautomers of those compounds, deuterated derivatives of those compounds and tautomers, and pharmaceutically acceptable salts of any of the foregoing, for use in therapy.

### I. Definitions

The term "AAT" as used herein means alpha-1 antitrypsin or a mutation thereof, including, but not limited to, the AAT gene mutations such as Z mutations. As used herein, "Z-AAT" means AAT mutants which have the Z mutation.

As used herein, "mutations" can refer to mutations in the *SERPINA1* gene (the gene encoding AAT) or the effect of alterations in the gene sequence on the AAT protein. A *"SERPINA1* gene mutation" refers to a mutation in the *SERPINA1* gene, and an "AAT protein mutation" refers to a mutation that results in an alteration in the amino acid sequence of the AAT protein. A genetic defect or mutation, or a change in the nucleotides in a gene in general, results in a mutation in the AAT protein translated from that gene.

As used herein, a patient who is "homozygous" for a particular gene mutation has the same mutation on each allele.

As used herein, a patient who has the *PiZZ* genotype is a patient who is homozygous for the Z mutation in the AAT protein.

The term "AATD" as used herein means alpha-1 antitrypsin deficiency, which is a genetic disorder characterized by low circulating levels of AAT.

The term "compound," when referring to a compound of this disclosure, refers to a collection of molecules having an identical chemical structure unless otherwise indicated as a collection of stereoisomers (for example, a collection of racemates, a collection of cis/trans stereoisomers, or a collection of (*E*) and (*Z*) stereoisomers), except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound of this disclosure will depend upon a number of factors including the isotopic purity of reagents used to make the compound and the efficiency of incorporation of isotopes in the various synthesis steps used to prepare the compound. However, as set forth above the relative amount of such isotopologues in toto will be less than 49.9% of the compound. In other embodiments, the relative amount of such isotopologues in toto will be less than 47.5%, less than 40%, less than 32.5%, less than 25%, less than 17.5%, less than 10%, less than 5%, less than 3%, less than 1%, or less than 0.5% of the compound.

It will be appreciated that the phrase "optionally substituted" is used interchangeably with the phrase "substituted or unsubstituted." In general, the term "substituted," whether preceded by the term "optionally" or not, refers to the replacement of hydrogen radicals in a given structure with the radical of a specified substituent. Unless otherwise indicated, an "optionally substituted" group may have a substituent at each substitutable position of the group, and when more than one position in any given structure may be substituted with more than one substituent chosen from a specified group, the substituent may be either the same or different at every position. Combinations of substituents envisioned by this disclosure are those that result in the formation of stable or chemically feasible compounds.

The term "isotopologue" refers to a species in which the chemical structure differs from a specific compound of this disclosure only in the isotopic composition thereof. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C or ¹⁴C are within the scope of this disclosure.

Unless otherwise indicated, structures depicted herein are also meant to include e.g., racemic mixtures, cis/trans isomers, geometric (or conformational) isomers, such as (*Z*) and (*E*) double bond isomers, and (*Z*) and (*E*) conformational isomers. Therefore, geometric and conformational mixtures of the present compounds are within the scope of the disclosure. Unless otherwise stated, all tautomeric forms of the compounds of the disclosure are within the scope of the disclosure.

The term "tautomer," as used herein, refers to one of two or more isomers of a compound that exist together in equilibrium, and are readily interchanged by migration of an atom or group within the molecule.

"Stereoisomer" refers to both enantiomers and diastereomers.

As used herein, "deuterated derivative" refers to a compound having the same chemical structure as a reference compound, but with one or more hydrogen atoms replaced by a deuterium atom ("D"). It will be recognized that some variation of natural isotopic abundance occurs in a synthesized compound depending on the origin of chemical materials used in the synthesis. The concentration of naturally abundant stable hydrogen isotopes, notwithstanding this variation is small and immaterial as compared to the degree of stable isotopic substitution of deuterated derivatives described herein. Thus, unless otherwise stated, when a reference is made to a "deuterated derivative" of a compound of the disclosure, at least one hydrogen is replaced with deuterium at well above its natural isotopic abundance (which is typically about 0.015%). In some embodiments, the deuterated derivatives of the disclosure have an isotopic enrichment factor for each deuterium atom, of at least 3500 (52.5% deuterium incorporation at each designated deuterium) at least 4500, (67.5% deuterium incorporation), at least 5000 (75% deuterium incorporation) at least 5500 (82.5% deuterium incorporation), at least 6000 (90% deuterium incorporation), at lease 6333.3 (95% deuterium incorporation, at least 6466.7 (97% deuterium incorporation, or at least 6600 (99% deuterium incorporation).

The term "isotopic enrichment factor" as used herein means the ratio between the isotopic abundance and the natural abundance of a specified isotope.

The term "alkyl" as used herein, means a straight-chain (i.e., linear or unbranched) or branched hydrocarbon chain that is completely saturated. Unless otherwise specified, alkyl groups contain 1-12 alkyl carbon atoms. In some embodiments, alkyl groups contain 1-10 aliphatic carbon atoms. In other embodiments, alkyl groups contain 1-8 aliphatic carbon atoms. In still other embodiments, alkyl groups contain 1-6 alkyl carbon atoms, in other embodiments alkyl groups contain 1-4 alkyl carbon atoms, and in yet other embodiments alkyl groups contain 1-3 alkyl carbon atoms and 1-2 alkyl carbon atoms.

The term "alkenyl" as used herein, means a straight-chain (i.e., linear or unbranched) or branched hydrocarbon chain that contains one or more carbon-to-carbon double bonds.

The terms "cycloalkyl" and "cyclic alkyl" refer to a fused, spirocyclic, or bridged monocyclic C₃₋₉ hydrocarbon or a fused, spirocyclic, or bridged bicyclic or tricyclic, C₈₋₁₄ hydrocarbon that is completely saturated, wherein any individual ring in said bicyclic ring system has 3-9 members. Typically, the terms "cycloalkyl" and "cyclic alkyl" refer to a monocyclic C₃₋₉ hydrocarbon or a fused, spirocyclic, or bridged bicyclic or tricyclic, C₈₋₁₄ hydrocarbon that is completely saturated, wherein any individual ring in said bicyclic ring system has 3-9 members. The terms "carbocyclyl" and "carbocycle" refer to a fused, spirocyclic, or bridged monocyclic C₃₋₉ hydrocarbon or a fused, spirocyclic, or bridged bicyclic or tricyclic, C₈₋₁₄ hydrocarbon that is completely saturated or that contains one or more units of unsaturation, but which is not fully aromatic, wherein any individual ring in said bicyclic ring system has 3-9 members. Typically, the terms "carbocyclyl" and "carbocycle" refer to a monocyclic C₃₋₉ hydrocarbon or a fused, spirocyclic, or bridged bicyclic or tricyclic, C₈₋₁₄ hydrocarbon that is completely saturated or that contains one or more units of unsaturation, but which is not fully aromatic, wherein any individual ring in said bicyclic ring system has 3-9 members. Typically, a carbocyclyl may contain one or more units of unsaturation but is not aromatic. In some embodiments, the cycloalkyl or carbocycle group contains 3 to 12 carbon atoms. In some embodiments, the cycloalkyl or carbocycle group contains 3 to 8 carbon atoms. In some embodiments, the cycloalkyl or carbocycle group contains 3 to 6 carbon atoms.

The term "heterocycle," "heterocyclyl," or "heterocyclic" as used herein refers to fused, spirocyclic, or bridged non-aromatic, monocyclic, bicyclic, or tricyclic ring systems in which one or more ring members is a heteroatom. In some embodiments, "heterocycle," "heterocyclyl," or "heterocyclic" group has 3 to 14 ring members in which one or more ring members is a heteroatom independently selected from oxygen, sulfur, nitrogen, phosphorus, or silicon and each ring in the system contains 3 to 9 ring members. In some embodiments, the heterocyclyl contains 3 to 12 ring member atoms. In some embodiments, the heterocyclyl contains 3 to 8 ring member atoms. In some embodiments, the heterocyclyl contains 3 to 6 ring member atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term "alkoxy" as used herein, refers to an alkyl group, as previously defined, wherein one carbon of the alkyl group is replaced by an oxygen ("alkoxy") atom, respectively, provided that the oxygen atom is linked between two carbon atoms. A "cyclic alkoxy" refers to a monocyclic, fused, spirocyclic, bicyclic, bridged bicyclic, tricyclic, or bridged tricyclic hydrocarbon that contains at least one alkoxy group, but is not aromatic. Non-limiting examples of cyclic alkoxy groups include tetrahydropyranyl, tetrahydrofuranyl, oxetanyl, 8-oxabicyclo[3.2.1]octanyl, and oxepanyl.

The terms "haloalkyl" and "haloalkoxy" means an alkyl or alkoxy, as the case may be, which is substituted with one or more halogen atoms. The term "halogen" or means F, Cl, Br, or I. In some embodiments, the halogen is selected from F, Cl, and Br. Examples of haloalkyls include -CHF₂, -CH₂F, -CF₃, -CF₂-, or perhaloalkyl, such as, -CF₂CF₃.

As used herein, "=O" refers to an oxo group.

As used herein, a "cyano" or "nitrile" groups refers to -C≡N.

As used herein, a "hydroxy" group refers to -OH.

As used herein, "aromatic groups" or "aromatic rings" refer to chemical groups that contain conjugated, planar ring systems with delocalized pi electron orbitals comprised of [4n+2] p orbital electrons, wherein n is an integer ranging from 0 to 6. Nonlimiting examples of aromatic groups include aryl and heteroaryl groups.

The term "aryl" refers to monocyclic, bicyclic, and tricyclic ring systems having a total of 5 to 14 ring members, wherein at least one ring in the system is aromatic and wherein each ring in the system contains 3 to 7 ring members. In some embodiments, an aryl contains 6 or 10 carbon atoms. A nonlimiting example of an aryl group is a phenyl ring.

The term "heteroaryl" refers to monocyclic, bicyclic, and tricyclic ring systems having a total of 5 to 10 ring members, wherein at least one ring in the system is aromatic, at least one ring in the system contains one or more heteroatoms, and wherein each ring in the system contains 3 to 7 ring members. In some embodiments, a heteroaryl contains 6 or 10 ring atoms.

Examples of useful protecting groups for nitrogen-containing groups, such as amine groups, include, for example, t-butyl carbamate (Boc), benzyl (Bn), tetrahydropyranyl (THP), 9-fluorenylmethyl carbamate (Fmoc) benzyl carbamate (Cbz), acetamide, trifluoroacetamide, triphenylmethylamine, benzylideneamine, and p-toluenesulfonamide. Methods of adding (a process generally referred to as "protecting") and removing (process generally referred to as "deprotecting") such amine protecting groups are well-known in the art and available, for example, in P. J. Kocienski, Protecting Groups, Thieme, 1994, and in Greene and Wuts, Protective Groups in Organic Synthesis, 3rd Edition (John Wiley & Sons, New York, 1999).

Examples of suitable solvents that may be used in this disclosure include, but not limited to, water, methanol (MeOH), ethanol (EtOH), dichloromethane or "methylene chloride" (CH₂Cl₂), toluene, acetonitrile (MeCN), dimethylformamide (DMF), dimethyl sulfoxide (DMSO), methyl acetate (MeOAc), ethyl acetate (EtOAc), heptanes, isopropyl acetate (IPAc), *tert*-butyl acetate (*t*-BuOAc), isopropyl alcohol (IPA), tetrahydrofuran (THF), 2-methyl tetrahydrofuran (2-Me THF), methyl ethyl ketone (MEK), *tert*-butanol, diethyl ether (Et₂O), methyl-*tert*-butyl ether (MTBE), 1,4-dioxane, and *N*-methyl pyrrolidone (NMP).

Examples of suitable bases that may be used in this disclosure include, but not limited to, 1,8-diazabicyclo[5.4.0]undec-7-ene (DBU), potassium tert-butoxide (KOtBu), potassium carbonate (K₂CO₃), *N*-methylmorpholine (NMM), triethylamine (Et₃N; TEA), diisopropyl-ethyl amine (*i*-Pr₂EtN; DIPEA), pyridine, potassium hydroxide (KOH), sodium hydroxide (NaOH), lithium hydroxide (LiOH) and sodium methoxide (NaOMe; NaOCH₃).

The disclosure includes pharmaceutically acceptable salts of the compounds of the disclosure. A salt of a compound of is formed between an acid and a basic group of the compound, such as an amino functional group, or a base and an acidic group of the compound, such as a carboxyl functional group.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any non-toxic salt that, upon administration to a recipient, is capable of providing, either directly or indirectly, a compound of this disclosure. Suitable pharmaceutically acceptable salts are, for example, those disclosed in S. M. Berge, et al. J. Pharmaceutical Sciences, 1977, 66, 1-19.

Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methane sulfonate, propanesulfonate, naphthalene-1-sulfonate, naphthalene-2- sulfonate, mandelate and other salts. In some embodiments, pharmaceutically acceptable acid addition salts include those formed with mineral acids such as hydrochloric acid and hydrobromic acid, and those formed with organic acids such as maleic acid.

Pharmaceutically acceptable salts derived from appropriate bases include alkali metal, alkaline earth metal, ammonium, and N⁺(C₁₋₄alkyl)₄ salts. This disclosure also envisions the quaternization of any basic nitrogen-containing groups of the compounds disclosed herein. Suitable non-limiting examples of alkali and alkaline earth metal salts include sodium, lithium, potassium, calcium, and magnesium. Further non-limiting examples of pharmaceutically acceptable salts include ammonium, quaternary ammonium, and amine cations formed using counterions such as halide, hydroxide, carboxylate, sulfate, phosphate, nitrate, lower alkyl sulfonate and aryl sulfonate. Other suitable, non-limiting examples of pharmaceutically acceptable salts include besylate and glucosamine salts.

The terms "patient" and "subject" are used interchangeably and refer to an animal, including a human.

The terms "effective dose," "effective amount," "therapeutically effective dose," and "therapeutically effective amount" are used interchangeably herein and refer to that amount of a compound that produces the desired effect for which it is administered (e.g., improvement in AATD or a symptom of AATD, lessening the severity of AATD or a symptom of AATD, and/or reducing the rate of onset or incidence of AATD or a symptom of AATD). The exact amount of an effective dose will depend on the purpose of the treatment, and will be ascertainable by one skilled in the art using known techniques (see, e.g., Lloyd (1999) The Art, Science and Technology of Pharmaceutical Compounding).

As used herein, the term "treatment and its cognates (e.g., "treat," "treating") refer to improving AATD or its symptoms in a subject, delaying the onset of AATD or its symptoms in a subject, or lessening the severity of AATD or its symptoms in a subject. "Treatment" and its cognates as used herein, include, but are not limited to the following: improved liver and/or spleen function, lessened jaundice, improved lung function, lessened lung diseases and/or pulmonary exacerbations (e.g., emphysema), lessened skin disease (e.g., necrotizing panniculitis), increased growth in children, improved appetite, and reduced fatigue. Improvements in or lessening the severity of any of these symptoms can be readily assessed according to methods and techniques known in the art or subsequently developed.

The terms "about" and "approximately", when used in connection with doses, amounts, or weight percent of ingredients of a composition or a dosage form, include the value of a specified dose, amount, or weight percent or a range of the dose, amount, or weight percent that is recognized by one of ordinary skill in the art to provide a pharmacological effect equivalent to that obtained from the specified dose, amount, or weight percent. Typically, the term "about" refers to a variation of up to 10%, up to 5%, or up to 2% of a stated value.

Any one or more of the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds or tautomers, and pharmaceutically acceptable salts of any of the foregoing may be administered once daily, twice daily, or three times daily for the treatment of AATD. In some embodiments, the any one or more compounds are selected from Compounds 1-457, Compounds 458-532, Compounds P1-P225, Compounds B1-B25, and Compounds W1-W4, W6-W23, W25, W26, W28, W30, and W31 (e.g., Compounds 1-457, e.g., Compounds 1-142, 144-177, 179-399, 401-422, 425-433, and 435-457), tautomers of those compounds, deuterated derivatives of those compounds or tautomers, and pharmaceutically acceptable salts of any of the foregoing. In some embodiments, at least one compound chosen from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds or tautomers, and pharmaceutically acceptable salts of any of the foregoing is administered once daily. In some embodiments, a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds or tautomers, and pharmaceutically acceptable salts of any of the foregoing is administered once daily. In some embodiments, at least one compound selected from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds or tautomers, and pharmaceutically acceptable salts of any of the foregoing are administered twice daily. In some embodiments, a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds or tautomers, and pharmaceutically acceptable salts of any of the foregoing is administered twice daily. In some embodiments, at least one compound chosen from compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds or tautomers, and pharmaceutically acceptable salts of any of the foregoing are administered three times daily. In some embodiments, a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds or tautomers, and pharmaceutically acceptable salts of any of the foregoing is administered three times daily.

Any one or more of the compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), tautomers of those compounds, deuterated derivatives of those compounds or tautomers, and pharmaceutically acceptable salts of any of the foregoing may be administered in combination with AAT augmentation therapy or AAT replacement therapy for the treatment of AATD. In some embodiments, the any one or more compounds are selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457), tautomers of those compounds, deuterated derivatives of those compounds or tautomers, and pharmaceutically acceptable salts of any of the foregoing.

Any one or more of Compounds B1-B25 and W1-W32, tautomers of those compounds, deuterated derivatives of those compounds or tautomers, and pharmaceutically acceptable salts of any of the foregoing may be administered once daily, twice daily, or three times daily for the treatment of AATD. In some embodiments, at least one compound chosen from compounds of Formulae (XIa)-(XIe) and (XIIa)-(XIIe) (e.g., Compounds B1-B25 and Compounds W1-W32), tautomers of those compounds, deuterated derivatives of those compounds or tautomers, and pharmaceutically acceptable salts of any of the foregoing is administered once daily. In some embodiments, at least one compound selected from compounds of Formulae (XIa)-(XIe) and (XIIa)-(XIIe) (e.g., Compounds B1-B25 and Compounds W1-W32), tautomers of those compounds, deuterated derivatives of those compounds or tautomers, and pharmaceutically acceptable salts of any of the foregoing are administered twice daily. In some embodiments, at least one compound chosen from compounds of Formulae (XIa)-(XIe) and (XIIa)-(XIIe) (e.g., Compounds B1-B25 and Compounds W1-W32), tautomers of those compounds, deuterated derivatives of those compounds or tautomers, and pharmaceutically acceptable salts of any of the foregoing are administered three times daily.

Any one or more of Compounds B1-B25 and W1-W32, tautomers of those compounds, deuterated derivatives of those compounds or tautomers, and pharmaceutically acceptable salts of any of the foregoing may be administered in combination with AAT augmentation therapy or AAT replacement therapy for the treatment of AATD.

As used herein, "AAT augmentation therapy" refers to the use of alpha-1 antitrypsin protein (AAT) from the blood plasma of healthy human donors to augment (increase) the alpha-1 antitrypsin levels circulating in the blood. "AAT replacement therapy" refers to administration of recombinant AAT.

In some embodiments, 10 mg to 1,500 mg, 100 mg to 1800 mg, 100 mg to 500 mg, 200 mg to 600 mg, 200 mg to 800 mg, 400 mg to 2,000 mg, 400 mg to 2,500 mg or 400 mg to 600 mg of a compound of Formulae (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds or tautomers, and pharmaceutically acceptable salts of any of the foregoing is administered once daily, twice daily, or three times daily. In some embodiments, 10 mg to 1,500 mg, 100 mg to 1800 mg, 100 mg to 500 mg, 200 mg to 600 mg, 200 mg to 800 mg, 400 mg to 2000 mg, or 400 mg to 600 mg of a compound selected from Compounds 1-457, Compounds 458-532, Compounds P1-P225, Compounds B1-B25, and Compounds W1-W4, W6-W23, W25, W26, W28, W30, and W31 (e.g., Compounds 1-457, or e.g., Compounds 1-142, 144-177, 179-399, 401-422, 425-433, and 435-457) is administered once daily, twice daily, or three times daily.

In some embodiments, 10 mg to 1,500 mg, 100 mg to 1800 mg, 100 mg to 500 mg, 200 mg to 600 mg, 200 mg to 800 mg, 400 mg to 2,000 mg, 400 mg to 2,500 mg or 400 mg to 600 mg of a compound selected from compounds of Formulae (XIa)-(XIe) and (XIIa)-(XIIe) (e.g., Compounds B1-B25 and Compounds W1-W32), tautomers of those compounds, deuterated derivatives of those compounds or tautomers, and pharmaceutically acceptable salts of any of the foregoing is administered once daily, twice daily, or three times daily. In some embodiments, 10 mg to 1,500 mg, 100 mg to 1800 mg, 100 mg to 500 mg, 200 mg to 600 mg, 200 mg to 800 mg, 400 mg to 2000 mg, or 400 mg to 600 mg of a compound selected from compounds of Formulae (XIa)-(XIe) and (XIIa)-(XIIe) (e.g., Compounds B1-B25 and Compounds W1-W32) is administered once daily, twice daily, or three times daily.

One of ordinary skill in the art would recognize that, when an amount of a compound is disclosed, the relevant amount of a pharmaceutically acceptable salt form of the compound is an amount equivalent to the concentration of the free base of the compound. It is noted that the disclosed amounts of the compounds, tautomers, deuterated derivatives, and pharmaceutically acceptable salts are based upon the free base form of the reference compound. For example, "10 mg of at least one compound chosen from compounds of Formula (I) and pharmaceutically acceptable salts thereof" includes 10 mg of a compound of Formula (I) and a concentration of a pharmaceutically acceptable salt of compounds of Formula (I) equivalent to 10 mg of compounds of Formula (I).

As used herein, the term "ambient conditions" means room temperature, open air condition and uncontrolled humidity condition.

It should be understood that references herein to methods of treatment (e.g., methods of treating AATD) using one or more compounds (e.g., compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those compounds and tautomers, and pharmaceutically acceptable salts of those compounds) should be interpreted as references to one or more compounds (e.g., compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those compounds and tautomers, and pharmaceutically acceptable salts of those compounds) for use in methods of treating, e.g., AATD.

It should be understood that references herein to methods of treatment (e.g., methods of treating AATD) using one or more compounds (e.g., compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those compounds and tautomers, and pharmaceutically acceptable salts of those compounds) should also be interpreted as disclosing the use of one or more compounds (e.g., compounds of Formulae (I), (IIa)-(IIg), (IIIa)-(IIIc), (IVa)-(IVb), (Va)-(Vc), (VIa)-(VIg), (VIIa)-(VIIg), (IXa)-(IXe), and (Xa)-(Xf) (e.g., compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe)), as well as tautomers of those compounds, deuterated derivatives of those compounds and tautomers, and pharmaceutically acceptable salts of those compounds) in the manufacture of a medicament for treating, e.g., AATD.

### Example Embodiments 1:

The invention is defined by the claims. Non-limiting embodiments of the present disclosure include:
1. A compound represented by the following structural formula: 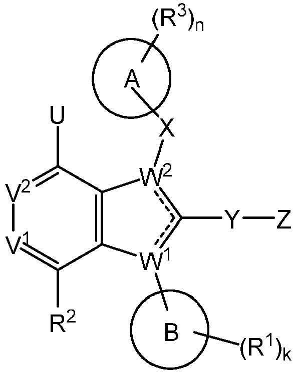 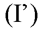 a tautomer thereof, a deuterated derivative of that compound or tautomer, or a pharmaceutically acceptable salt of the foregoing, wherein:
   ------, for each of the two occurrences, is a single bond or a double bond, provided that one is a single bond and the other is a double bond;
   **V¹** and **V²** are each independently N or -C**R²**;
   **W¹** and **W²** are each independently N or C, provided that one of W¹ and W² is N and the other is C;
   **U** is -OH, -CH₃, -NH₂, or halogen;
   **X** is absent or a bond, -(C**R^{a}R^{b}**)**ₚ**-, or -SO₂-; **Y** is absent or a bond, -(C**R^{c}R^{d})_{q}**-, -C(=O)-, or -SO₂-; **R^{a}** and **R^{b},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
   **R^{c}** and **R^{d},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
   **Ring A** is C₃-C₁₂ carbocyclyl, 3 to 12-membered heterocyclyl, C₆ or C₁₀ aryl, or 5 to 10-membered heteroaryl; provided that when **W¹** is N and **W²** is C, **Ring A** is not 1,5,6,7-tetrahydro-4*H*-indol-4-onyl or a tautomer thereof;
   **Ring B** is C₄-C₁₂ cycloalkyl, C₆ or C₁₀ aryl, 5 to 10-membered heteroaryl, or benzyl;
   **Z** is 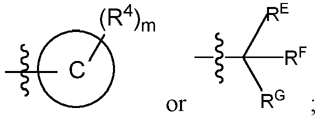 wherein:
      **Ring C** is C₃-C₁₂ cycloalkyl, 3 to 12-membered heterocyclyl, C₆ or C₁₀ aryl, or 5 to 10-membered heteroaryl;
      provided that when **Ring C** is phenyl, the phenyl is substituted with **R⁴;** provided that when **Ring C** is phenyl, **Y** cannot be -SO₂-; and
      provided that when **Ring B** is benzyl, **Ring C** cannot be pyridinyl or indolyl;
      **R^{E}, R^{F},** and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
         the C₁-C₆ alkyl or the C₂-C₆ alkenyl of any one of **R^{E}, R^{F},** and **R^{G}** is optionally substituted with 1 to 3 groups selected from cyano, -C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)**ᵣR^{s}**, and -S(=O)**ᵣ**N**R^{p}R^{q}**; wherein:
         **R^{p}, R^{q},** and **R^{r},** for each occurrence, are each independently hydrogen, C₁-C₄ alkyl, C₃-C₆ cycloalkyl, or 3 to 6-membered heterocyclyl; wherein:
            the C₁-C₄ alkyl of any one of **R^{p}**, **R^{q},** and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
            the C₃-C₆ cycloalkyl or the 3 to 6-membered heterocyclyl of any one of **R^{p}**, **R^{q},** and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
         **R^{s},** for each occurrence, is independently hydrogen, C₁-C₄ alkyl, C₃-C₆ cycloalkyl, phenyl, or 5 or 6-membered heteroaryl; wherein:
            the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
            the C₃-C₆ cycloalkyl, the phenyl, or the 5 or 6-membered heteroaryl of **R^{s}** is optionally substituted with 1 to 3 groups selected from halogen, cyano, -OH, -OCH₃, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂;
      **R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl);
      **R²,** for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, -N**R^{h}Rⁱ**, phenyl, or 5 or 6-membered heteroaryl; wherein:
         the C₁-C₆ alkyl, the C₂-C₆ alkenyl or the C₃-C₆ cycloalkyl of **R²** is optionally substituted with 1 to 3 groups selected from cyano, -C(=O)**R^{k}**, -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k}**, -OC(=O)**R^{k}**, -OC(=O)O**R^{k}**, -OC(=O)N**R^{h}Rⁱ**, -S(=O)**ₛR^{k}**, and S(=O)ₛN**R^{h}Rⁱ**; wherein:
         **R^{h}, Rⁱ,** and **R^{j},** for each occurrence, are each independently hydrogen, C₁-C₄ alkyl, or C₃-C₆ cycloalkyl; wherein:
            the C₁-C₄ alkyl of any one of **R^{h}, Rⁱ,** and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl of any one of **R^{h}, Rⁱ,** and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{k},** for each occurrence, is independently hydrogen, C₁-C₄ alkyl, C₃-C₆ cycloalkyl, phenyl, or 5 or 6-membered heteroaryl; wherein:
            -O**R^{k}** cannot be -OH;
            the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁- C₂ alkyl)₂;
      **R³** and **R⁴,** for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)**ₜR^{y}**, -N**R^{v}R^{w}**, - N**R^{v}**C(=O)**R^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, - OC(=O)O**R^{y}**, -OC(=O)N**R^{v}R^{w}**, -S(=O)ₜ**R^{y}**, -S(=O)**ₜ**N**R^{v}R^{w}**, -S(=O)**ₜ**N**R^{v}**C(=O)**R^{y}**, - P(=O)**R^{z}R^{z}**, phenyl, or a 5 or 6-membered heteroaryl; wherein:
         the C₁-C₆ alkyl, the C₂-C₆ alkenyl, or the C₃-C₆ cycloalkyl of any one of **R³** and **R⁴** is optionally substituted with 1 to 3 groups selected from cyano, - C(=O)**R^{y}**, -C(=O)O**R^{y}**, -C(=O)NR^{v}R^{w}, -N**R^{v}R^{w}**, -N**R^{v}**C(=O)**R^{y}**, -N**R^{v}**C(=O)O**R^{y}**, -N**R^{v}**C(=O)N**R^{w}R^{x},** -NR^{v}S(=O)ᵣ**R^{y}**, -O**R^{y}**, -OC(=O)**R^{y}**, - OC(=O)O**R^{y}**, -OC(=O)N**R^{v}R^{w}**, -S(=O)**ₜR^{y}**, and
      -S(=O)**ₜ**N**R^{v}R^{w}**; wherein:
         **R^{v}, R^{w},** and **R^{x},** for each occurrence, are each independently hydrogen, C₁-C₄ alkyl, C₃-C₆ cycloalkyl, 5 or 6-membered heterocyclyl, or 5 or 6-membered heteroaryl; wherein:
            the C₁-C₄ alkyl of any one of **R^{v}**, **R^{w},** and **R^{x}** is optionally substituted with 1 to 3 groups selected from halogen, cyano -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl, the 5 or 6-membered heterocyclyl, or the 5 or 6-membered heteroaryl of any one of **R^{v}, R^{w}**, and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{y},** for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
            the C₁-C₄ alkyl of **R^{y}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, -NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{z},** for each occurrence, is independently C₁-C₂ alkyl, -OH, or -O(C₁-C₂ alkyl);
   **k** is an integer selected from 1, 2, and 3;
   **m** and **n** a are each independently an integer selected from 0, 1, 2, and 3; and
   **p, q, r, s,** and **t** are each independently an integer selected from 1 and 2.
2. The compound, tautomer, deuterated derivative, or pharmaceutically acceptable salt according to embodiment 1 represented by one of the following structural formulae: 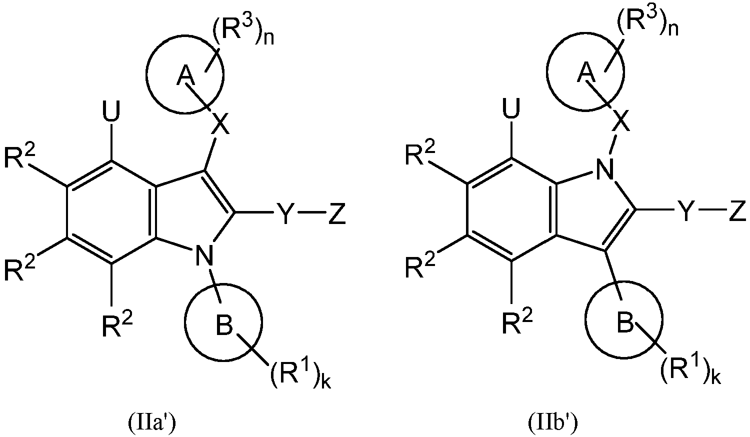 wherein:
   U is -OH, -CH₃, -NH₂, F, or Cl; and wherein all other variables not specifically defined herein are as defined in embodiment 1.
3. The compound, tautomer, deuterated derivative, or pharmaceutically acceptable salt according to embodiment 1 represented by one of the following structural formulae: 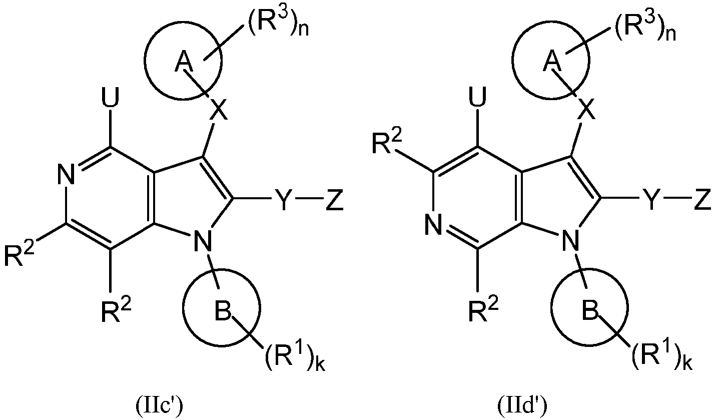 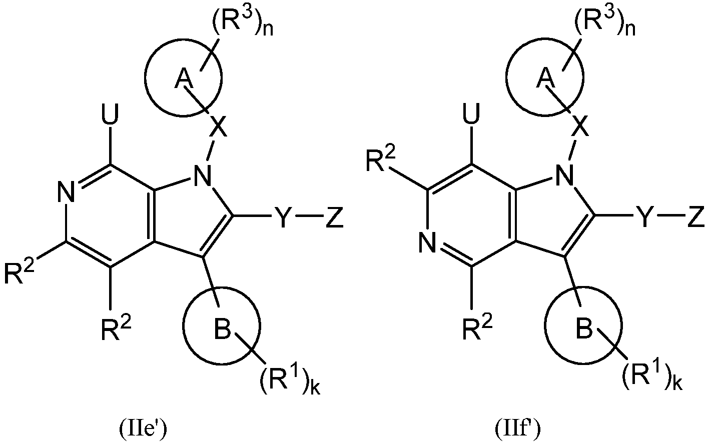 wherein:
   U is -OH, -CH₃, -NH₂, F, or Cl;
   and wherein all other variables not specifically defined herein are as defined in embodiment 1.
4. The compound, tautomer, deuterated derivative, or pharmaceutically acceptable salt according to embodiment 1 or embodiment 2 represented by one of the following structural formulae: 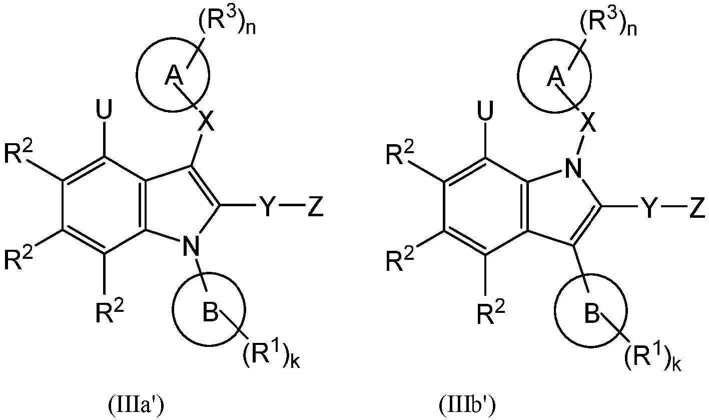 wherein:
   **U** is -OH or -NH₂;
   **Ring A** is optionally substituted with **R³** and **Ring A** is C₃-C₇ carbocyclyl, 6 to 9-membered heterocyclyl, phenyl, or 5 to 9-membered heteroaryl;
   **Ring B** is substituted with **R¹** and **Ring B** is C₄-C₆ cycloalkyl, phenyl, 5 to 6-membered heteroaryl, or benzyl; and
   when **Z** is **Ring C** optionally substituted with **R⁴, Ring C is** C₄-C₈ cycloalkyl, 4 to 8-membered heterocyclyl, phenyl, or 5 or 6-membered heteroaryl;
   and wherein all other variables not specifically defined herein are as defined in embodiment 1 or embodiment 2.
5. The compound, tautomer, deuterated derivative, or pharmaceutically acceptable salt according to any one of embodiments 1, 2, and 4 represented by one of the following structural formulae: 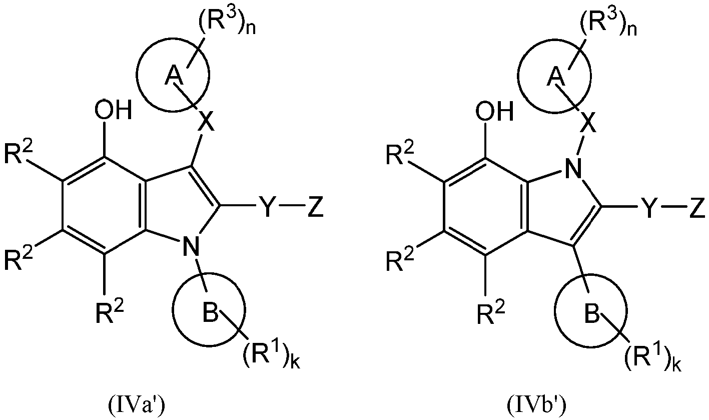 wherein:
   **Ring B** is substituted with **R¹** and **Ring B** is cyclohexyl, phenyl, pyridinyl, or benzyl; and wherein all other variables not specifically defined herein are as defined in any one of embodiments 1, 2, and 4.
6. The compound, tautomer, deuterated derivative, or pharmaceutically acceptable salt according to any one of embodiments 1, 2, 4, and 5 represented by the following structural formula: 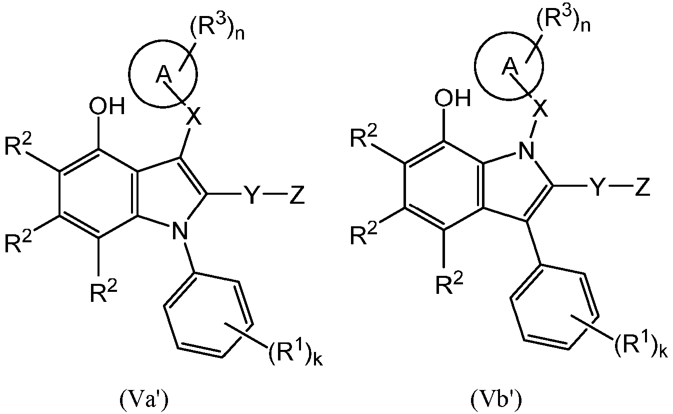 wherein:
   **R¹** is halogen, cyano, C₁-C₂ alkyl, C₁-C₂ haloalkyl, or C₁-C₂ alkoxy; and
   **k** is an integer selected from 1 and 2;
   and wherein all other variables not specifically defined herein are as defined in any one of embodiments 1, 2, 4, and 5.
7. The compound, tautomer, deuterated derivative, or pharmaceutically acceptable salt according to any one of embodiments 1 to 6, wherein **R¹** is cyano, F, Cl, -CH₃, -CHF₂, -CF₃, -OCH₃, or -OCH(CH₃)₂; and wherein all other variables not specifically defined herein are as defined in any one of the preceding embodiments.
8. The compound, tautomer, deuterated derivative, or pharmaceutically acceptable salt according to any one of embodiments 1 to 7, wherein at least one **R¹** is F; and wherein all other variables not specifically defined herein are as defined in any one of the preceding embodiments.
9. The compound, tautomer, deuterated derivative, or pharmaceutically acceptable salt according to any one of embodiments 1 to 8, wherein:
   **X** is absent or a bond, -(C**R^{a}R^{b}**)-, or -SO₂-;
   **R^{a}** and **R^{b},** for each occurrence, are each independently hydrogen, C₁-C₃ alkyl, C₁-C₃ haloalkyl, or C₁-C₃ alkoxy; and wherein all other variables not specifically defined herein are as defined in any one of the preceding embodiments.
10. The compound, tautomer, deuterated derivative, or pharmaceutically acceptable salt according to any one of embodiments 1 to 9, wherein **X** is absent or a bond, -CH₂-, or -SO₂-; and wherein all other variables not specifically defined herein are as defined in any one of the preceding embodiments.
11. The compound, tautomer, deuterated derivative, or pharmaceutically acceptable salt according to any one of embodiments 1 to 10, wherein:
   **Y** is absent or a bond, -(C**R^{c}R^{d})_{q}**-, -C(=O)-, or -SO₂-;
   **R^{c}** and **R^{d},** for each occurrence, are each independently hydrogen, C₁-C₃ alkyl, C₁-C₃ haloalkyl, or C₁-C₃ alkoxy; and wherein all other variables not specifically defined herein are as defined in any one of the preceding embodiments.
12. The compound, tautomer, deuterated derivative, or pharmaceutically acceptable salt according to any one of embodiments 1 to 11, wherein **Y** is absent or a bond, -CH₂-, -CHCH₃-, - C(CH₃)₂-, -C(=O)-, or -SO₂-; and wherein all other variables not specifically defined herein are as defined in any one of the preceding embodiments.
13. The compound, tautomer, deuterated derivative, or pharmaceutically acceptable salt according to any one of embodiments 1 to 12, wherein **Ring A** is optionally substituted with **R³** and **Ring A** is C₃-C₇ carbocyclyl, 6 to 9-membered heterocyclyl containing 1 to 3 oxygen atoms, phenyl, or 5 to 9-membered heteroaryl containing 1 to 3 heteroatoms selected from O and N; and wherein all other variables not specifically defined herein are as defined in any one of the preceding embodiments.
14. The compound, tautomer, deuterated derivative, or pharmaceutically acceptable salt according to any one of embodiments 1 to 13, wherein **Ring A** is optionally substituted with **R³** and **Ring A** is C₃-C₇ carbocyclyl, 6 to 9-membered heterocyclyl containing one or two oxygen atoms, phenyl, or 5 to 9-membered heteroaryl containing one or two nitrogen atoms or one or two oxygen atoms; and wherein all other variables not specifically defined herein are as defined in any one of the preceding embodiments.
15. The compound, tautomer, deuterated derivative, or pharmaceutically acceptable salt according to any one of embodiments 1 to 14, wherein **Ring A** is optionally substituted with **R³** and **Ring A** is selected from 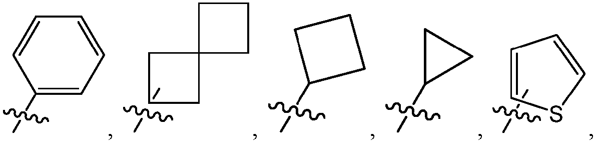 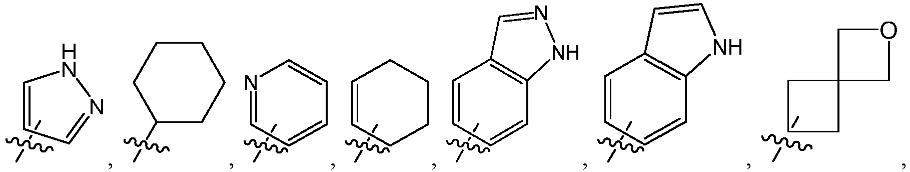 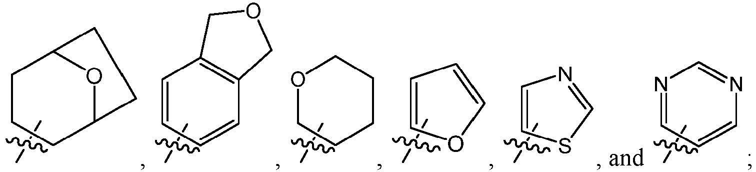 and wherein all other variables not specifically defined herein are as defined in any one of the preceding embodiments.
16. The compound, tautomer, deuterated derivative, or pharmaceutically acceptable salt according to any one of embodiments 1 to 15, wherein **Ring A** is optionally substituted with **R³** and **Ring A** is selected from 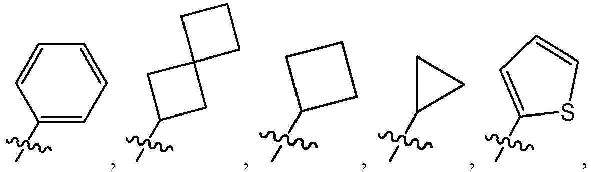 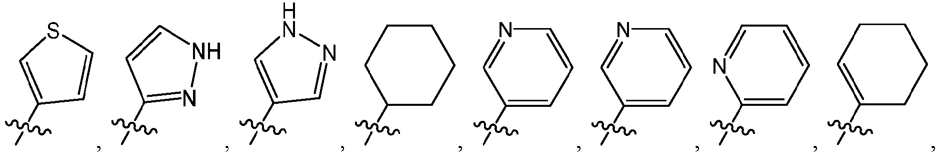 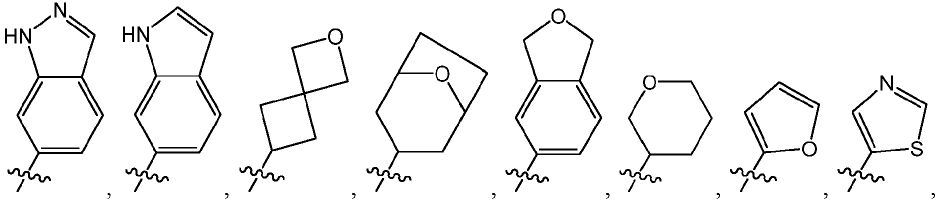 and 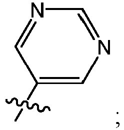 and wherein all other variables not specifically defined herein are as defined in any one of the preceding embodiments.
17. The compound, tautomer, deuterated derivative, or pharmaceutically acceptable salt according to any one of embodiments 1 to 16, wherein **Z** is **Ring C, Ring** C is optionally substituted with **R⁴,** and **Ring** C is C₄-C₈ cycloalkyl; 4 to 8-membered heterocyclyl containing one or two heteroatoms selected from O, N, and S; phenyl; or 5-membered heteroaryl containing one or two heteroatoms selected from O and N;
   and wherein all other variables not specifically defined herein are as defined in any one of the preceding embodiments.
18. The compound, tautomer, deuterated derivative, or pharmaceutically acceptable salt according to any one of embodiments 1 to 17, wherein **Z** is **Ring C, Ring C** is optionally substituted with **R⁴,** and **Ring C** is selected from 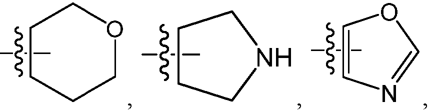 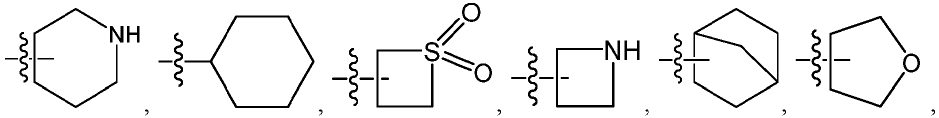 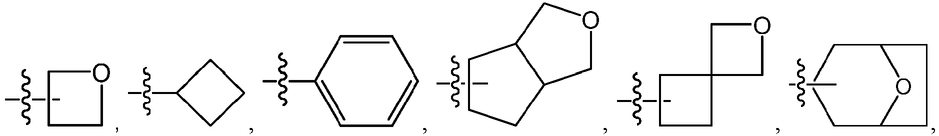 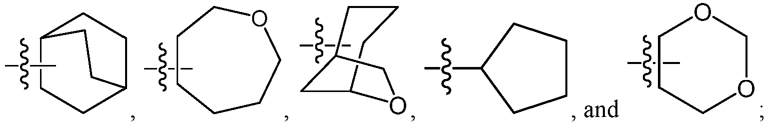 and wherein all other variables not specifically defined herein are as defined in any one of the preceding embodiments.
19. The compound, tautomer, deuterated derivative, or pharmaceutically acceptable salt according to any one of embodiments 1 to 18, wherein **Z** is **Ring C, Ring C** is optionally substituted with (**R⁴**)ₒ, and **Ring C** is selected from 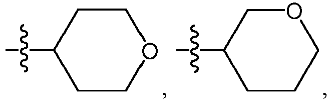 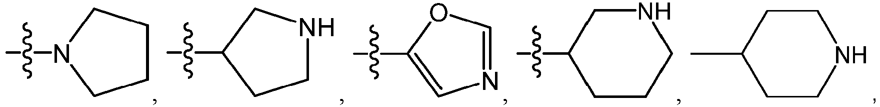 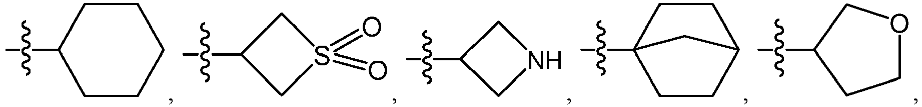 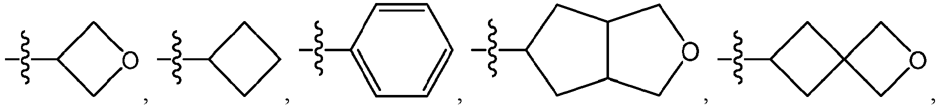 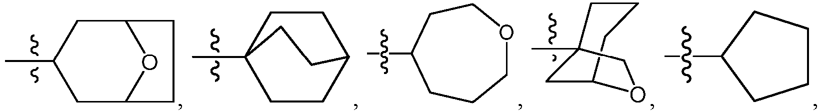 and 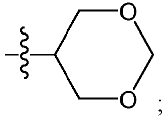 and wherein all other variables not specifically defined herein are as defined in any one of the preceding embodiments.
20. The compound, tautomer, deuterated derivative, or pharmaceutically acceptable salt according to any one of embodiments 1 to 19, wherein **R^{E}, R^{F},** and **R^{G}** are each independently hydrogen, halogen, cyano (-C≡N), C₁-C₄ alkyl, C₁-C₄ alkoxy, C₁-C₄ haloalkyl, -C(=O)O**R^{s}**, - C(=O)N**R^{p}R^{q}**, -C**R^{p}**(=N)O**R^{s}**, or -O**R^{s}**; wherein:
   the C₁-C₄ alkyl of any one of **R^{E}**, **R^{F}**, and **R^{G}** is optionally substituted with 1 to 3 groups selected from cyano, -C(=O)**R^{s}**, -C(=O)O**R^{s}**, -C(=O)N**R^{p}R^{q}**, -O**R^{s}**, -OC(=O)**R^{s}**, -OC(=O)O**R^{s}**, -OC(=O)N**R^{p}R^{q}**, and -S(=O)₂**R^{s}**; wherein:
   **R^{p}** and **R^{q}**, for each occurrence, are each independently hydrogen, C₁-C₂ alkyl, C₃-C₅ cycloalkyl, or 5 or 6-membered heterocyclyl; wherein:
      the C₁-C₂ alkyl of any one of **R^{p}** and **R^{q}** is optionally substituted with 1 to 3 groups selected from halogen, cyano, and -OH;
      the C₃-C₅ cycloalkyl or the 5 or 6-membered heteroaryl of **R^{p}** and **R^{q}** is optionally substituted with 1 to 3 groups selected from halogen, cyano, and -OH;
   **R^{s}**, for each occurrence, is independently hydrogen, C₁-C₂ alkyl, or 5 or 6-membered heteroaryl; wherein the C₁-C₂ alkyl of **R^{s}** is optionally substituted with 1 to 3 groups selected from halogen, cyano, -OH, and
      -NH₂; wherein:
      the 5 or 6-membered heteroaryl of **R^{s}** is optionally substituted with 1 to 3 groups selected from halogen, cyano, -OH, and -NH₂;
   and wherein all other variables not specifically defined herein are as defined in any one of the preceding embodiments.
21. The compound, tautomer, deuterated derivative, or pharmaceutically acceptable salt according to any one of embodiments 1 to 20, wherein **R^{E}**, **R^{F}**, and **R^{G}** are each independently hydrogen, F, Cl, C₁-C₂ alkyl, C₁-C₂ haloalkyl, -C(=O)N**R^{p}R^{q}**, -C**R^{p}**(=N)O**R^{s}**, or -O**R^{s}**; wherein:
   the C₁-C₂ alkyl of any one of **R^{E}**, **R^{F}**, and **R^{G}** is optionally substituted with 1 to 3 groups selected from cyano, -C(=O)N**R^{p}R^{q}**, -O**R^{s}**, -OC(=O)N**R^{p}R^{q}**, and -S(=O)₂**R^{s}**; wherein:
   **R^{p}** and **R^{q}**, for each occurrence, are each independently hydrogen, C₁-C₂ alkyl, cyclopentyl, or tetrahydrofuranyl; wherein:
      the C₁-C₂ alkyl of any one of **R^{p}** and **R^{q}** is optionally substituted with 1 to 3 halogen groups selected from F and Cl;
   **R^{s}**, for each occurrence, is independently hydrogen, C₁-C₂ alkyl, pyridinyl, or pyrimidinyl; wherein:
      the C₁-C₂ alkyl of **R^{s}** is optionally substituted with 1 to 3
      halogen groups selected from F and Cl;
   and wherein all other variables not specifically defined herein are as defined in any one of the preceding embodiments.
22. The compound, tautomer, deuterated derivative, or pharmaceutically acceptable salt according to any one of embodiments 1 to 21, wherein **R^{E}**, **R^{F}**, and **R^{G}** are each independently hydrogen, F, -OH, -CH(OH)CH₃, -C(=O)NHCH₃, -C(=N)OCH₃, -CH₃,
   -CF₃, -CH₂F, -CH₂CN, -(CH₂)₂CN, -CH₂OH, -C₂H₅, -(CH₂)₂OH, -CH₂OCH₃, -CH₂OC₂H₅, -(CH₂)₂OCH₃, -CH₂OCHF₂, -(CH₂)₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅,-CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), or -CH₂(O)(pyrimidin-2-yl); and wherein all other variables not specifically defined herein are as defined in any one of the preceding embodiments.
23. The compound, tautomer, deuterated derivative, or pharmaceutically acceptable salt according to any one of embodiments 1 to 22, wherein **R^{E}**, **R^{F}**, and **R^{G}** are each independently hydrogen, F, -CH(OH)CH₃, -CH₃, -CH₂CN, -CH₂OH, or -CH₂OCH₃; and wherein all other variables not specifically defined herein are as defined in any one of the preceding embodiments.
24. The compound, tautomer, deuterated derivative, or pharmaceutically acceptable salt according to any one of embodiments 1 to 23 represented by one of the following structural formulae: 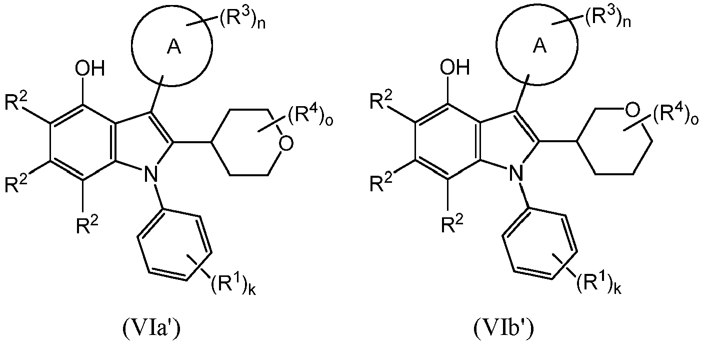 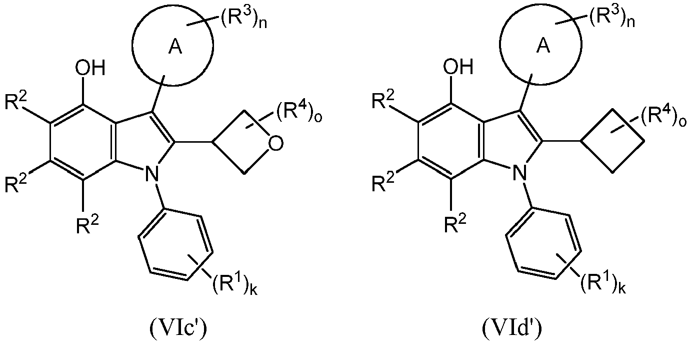 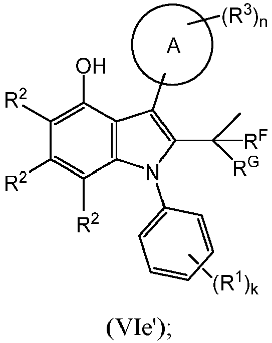 wherein all other variables not specifically defined herein are as defined in any one of the preceding embodiments.
25. The compound, tautomer, deuterated derivative, or pharmaceutically acceptable salt according to any one of embodiments 1 to 24 represented by one of the following structural formulae: 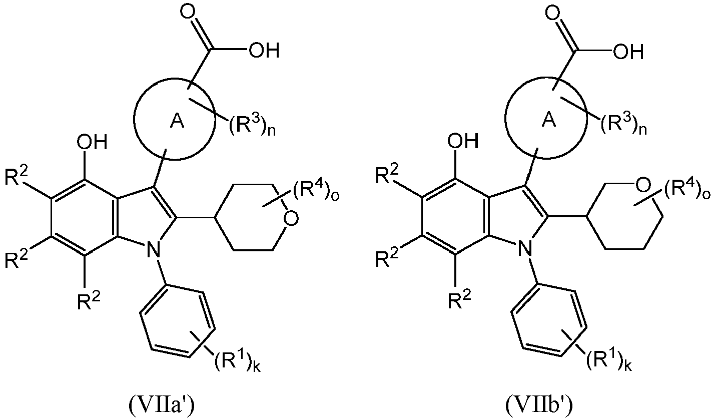 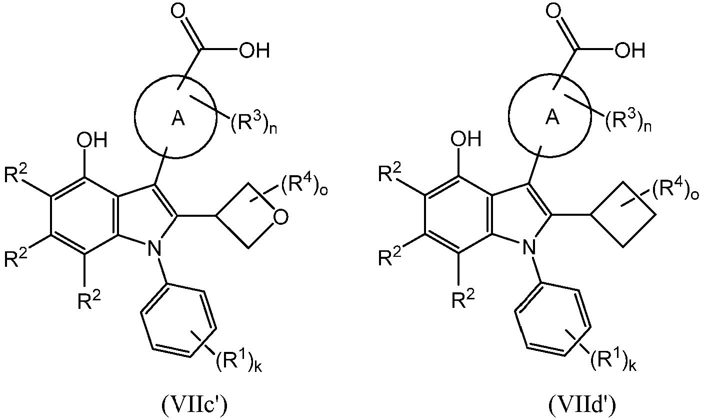 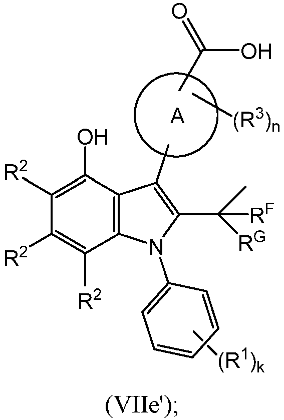 wherein n is an integer selected from 0, 1, and 2; and wherein all other variables not specifically defined herein are as defined in any one of the preceding embodiments.
26. The compound, tautomer, deuterated derivative, or pharmaceutically acceptable salt according to any one of embodiments 1 to 25 represented by one of the following structural formulae: 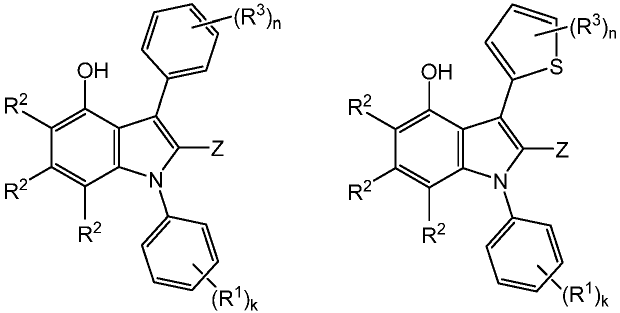 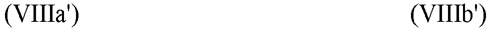 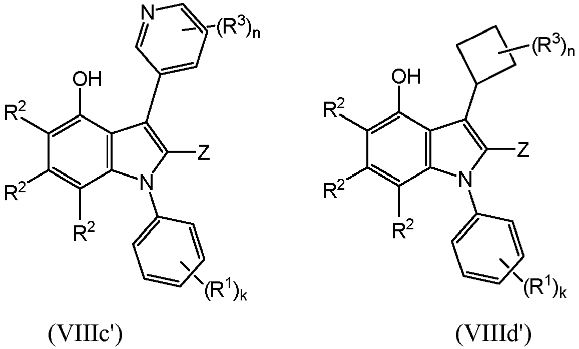 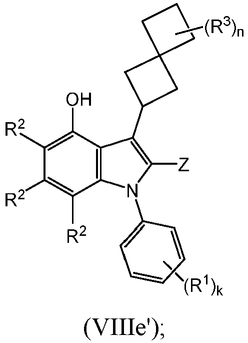 wherein all other variables not specifically defined herein are as defined in any one of the preceding embodiments.
27. The compound, tautomer, deuterated derivative, or pharmaceutically acceptable salt according to any one of embodiments 1 to 26 represented by one of the following structural formulae: 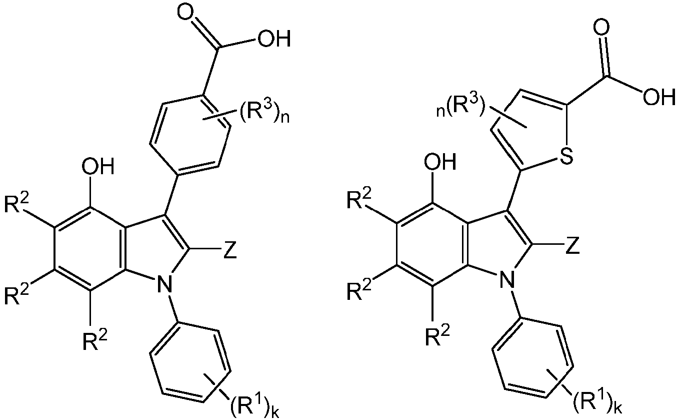 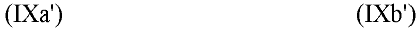 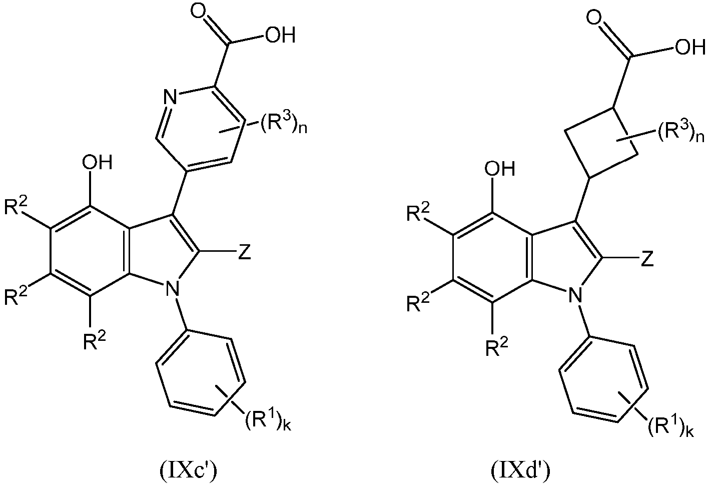 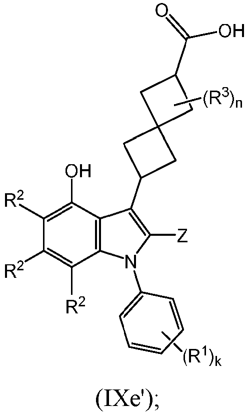 wherein **n** is an integer selected from 0, 1, and 2; and wherein all other variables not specifically defined herein are as defined in any one of the preceding embodiments.
28. The compound, tautomer, deuterated derivative, or pharmaceutically acceptable salt according to any one of embodiments 1 to 27, wherein **R²**, for each occurrence, is independently hydrogen, halogen, cyano, C₁-C₆ alkyl (optionally substituted with 1 to 3 groups selected from cyano, -OH, -OCH₃, and -NH₂), C₁-C₆ alkoxy, C₁-C₆ haloalkyl, -N**R^{h}Rⁱ**, or C₃-C₆ cycloalkyl; wherein **R^{h}** and **Rⁱ**, for each occurrence, are each independently hydrogen or C₁-C₄ alkyl; and wherein all other variables not specifically defined herein are as defined in any one of the preceding embodiments.
29. The compound, tautomer, deuterated derivative, or pharmaceutically acceptable salt according to any one of embodiments 1 to 28, wherein **R²**, for each occurrence, is independently hydrogen, halogen, cyano, C₁-C₄ alkyl (optionally substituted with 1 to 3 groups selected from cyano, -OH, -OCH₃, and -NH₂), C₁-C₄ alkoxy, C₁-C₄ haloalkyl, -N**R^{h}Rⁱ**, or C₃-C₅ cycloalkyl; wherein **R^{h}** and **Rⁱ**, for each occurrence, are each independently hydrogen or C₁-C₄ alkyl; and wherein all other variables not specifically defined herein are as defined in any one of the preceding embodiments.
30. The compound, tautomer, deuterated derivative, or pharmaceutically acceptable salt according to any one of embodiments 1 to 29, wherein **R²**, for each occurrence, is independently hydrogen, halogen, cyano, C₁-C₂ alkyl (optionally substituted with 1 to 3 groups selected from cyano, -OH, -OCH₃, and -NH₂), C₁-C₂ haloalkyl, -N**R^{h}Rⁱ**, or C₃-C₄ cycloalkyl; wherein **R^{h}** and **Rⁱ**, for each occurrence, are each independently hydrogen or -CH₃;
   and wherein all other variables not specifically defined herein are as defined in any one of the preceding embodiments.
31. The compound, tautomer, deuterated derivative, or pharmaceutically acceptable salt according to any one of embodiments 1 to 30, wherein **R²**, for each occurrence, is independently hydrogen, F, Cl, cyano, -CH₃, -CHF₂, -CF₃, -NH₂, or cyclopropyl; and wherein all other variables not specifically defined herein are as defined in any one of the preceding embodiments.
32. The compound, tautomer, deuterated derivative, or pharmaceutically acceptable salt according to any one of embodiments 1 to 31, wherein **R³,** for each occurrence, is independently halogen, cyano, =O, C₁-C₆ alkyl, C₁-C₆ alkoxy, C₁-C₆ haloalkyl, -C(=O)**R^{y}**, -C(=O)O**R^{y}**, -C(=O)N**R^{v}R^{w}**, -C(=O)N**R^{v}**O**R^{y}**,-C(=O)NR^{v}S(=O)₂**R^{y}**, -N**R**^{v}**R^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₄ alkyl of **R³** is optionally substituted with 1 to 3 groups selected from cyano, -O**R^{y}**, -C(=O)O**R^{y}**, and -N**R^{v}R^{w}**; wherein:
   **R^{v}**, **R^{w}**, and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with 1 to 3 groups selected from halogen, cyano, -OH, and -NH₂; and
   **R^{y}**, for each occurrence, is independently hydrogen, C₁-C₄ alkyl, or 5 or 6-membered heterocyclyl; wherein:
      the C₁-C₄ alkyl of **R^{y}** is optionally substituted with 1 to 3 groups selected from halogen, cyano, -OH, -NH₂, and -C(=O)OH; and
      the 5 or 6-membered heterocyclyl of **R^{y}** is optionally substituted with 1 to 3 groups selected from halogen, cyano, -OH,
      -NH₂, and -C(=O)OH;
   and wherein all other variables not specifically defined herein are as defined in any one of the preceding embodiments.
33. The compound, tautomer, deuterated derivative, or pharmaceutically acceptable salt according to any one of embodiments 1 to 32, wherein **R³**, for each occurrence, is independently halogen, cyano, =O, C₁-C₄ alkyl, C₁-C₄ alkoxy, C₁-C₄ haloalkyl, -C(=O)**R^{y}**, -C(=O)O**R^{y}**, -C(=O)N**^{v}R^{w}**,-C(=O)N**R^{v}**O**R^{y}**, -C(=O)N**R^{v}**S(=O)₂**R^{y}**, **-NR^{v}R^{w}, -** O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₄ alkyl **of R³** is optionally substituted with 1 to 3 groups selected from cyano, **-OR^{y}** and -C(=O)O**R^{y}**; wherein:
      **R^{v}**, **R^{w}**, and **R^{x}**, for each occurrence, are each independently hydrogen or C₁-C₄ alkyl; wherein the C₁-C₄ alkyl of any one of **R^{v}**, **R^{w}**, and **R^{x}** is optionally substituted with -OH; and
      **R^{y}**, for each occurrence, is independently hydrogen, C₁-C₂ alkyl, or 6-membered heterocyclyl; wherein:
         the C₁-C₂ alkyl of **R^{y}** is optionally substituted with 1 to 3 groups selected from -OH and -C(=O)OH; and
         the 6-membered heterocyclyl of **R^{y}** is optionally substituted with 1 to 3 groups selected from -OH and -C(=O)OH;
   **R^{z}**, for each occurrence, is independently -CH₃, -OH, or -OCH₃; and wherein all other variables not specifically defined herein are as defined in any one of the preceding embodiments.
34. The compound, tautomer, deuterated derivative, or pharmaceutically acceptable salt according to any one of embodiments 1 to 33, wherein **R³,** for each occurrence, is independently halogen, cyano, =O, C₁-C₂ alkyl, C₁-C₂ alkoxy, C₁-C₄ haloalkyl, -C(=O)**R^{y}**, -C(=O)O**R^{y}**, -C(=O)**NR^{v}R^{w}**,-C(=O)N**R^{v}**O**R^{y}**, -C(=O)N**R^{v}**S(=O)₂**R^{y}**, -N**R^{v}R^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₂ alkyl of **R³** is optionally substituted with 1 to 3 groups selected from -OH and -C(=O)OR^{y}; wherein:
      **R^{v}**, **R^{w}**, and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with -OH; and
      **R^{y}**, for each occurrence, is independently hydrogen, C₁-C₂ alkyl, or tetrahydro-2H-pyranyl; wherein:
         the C₁-C₂ alkyl of **R^{y}** is optionally substituted with -C(=O)OH; and
         the tetrahydro-2H-pyranyl of **R^{y}** is optionally substituted with 1 to 3 groups selected from -OH and -C(=O)OH;
   **R^{z}**, for each occurrence, is independently -CH₃ or -OH;
   and wherein all other variables not specifically defined herein are as defined in any one of the preceding embodiments.
35. The compound, tautomer, deuterated derivative, or pharmaceutically acceptable salt according to any one of embodiments 1 to 34, wherein **R³**, for each occurrence, is independently F, Cl, cyano, -OH, =O, -CH₃, -OCH₃, -CF₃, -CH₃CN, -C(CH₃)₂CH₂OH, -CH₂COOH, - CH₂OCH₃, -C(=O)CHCH₃OH, -COOH, -C(=O)O(2-tetrahydro-2H-pyranyl), -C(=O)NH₂, - C(=O)NH(CH₂)₂OH, -C(=O)NHOH, -C(=O)NHS(=O)₂CH₃, -NH₂, -NHCH₃, -OCH₂COOH, NHS(=O)₂CH₃,-S(=O)₂CH₃, -S(=O)₂NH₂, -S(=O)₂NHC(=O)CH₃, or -P(=O)(CH₃)₂; wherein the 2-tetrahydro-2H-pyranyl in -C(=O)O(2-tetrahydro-2H-pyranyl) is substituted with 1 to 3 groups selected from -OH and -C(=O)OH; and wherein all other variables not specifically defined herein are as defined in any one of the preceding embodiments.
36. The compound, tautomer, deuterated derivative, or pharmaceutically acceptable salt according to any one of embodiments 1 to 35, wherein **R⁴**, for each occurrence, is independently halogen, cyano, C₁-C₆ alkyl, C₁-C₆ alkoxy, C₁-C₆ haloalkyl, -C(=O)**R^{y}**, -C(=O)O**R^{y}**, -O**R^{y}**, or -S(=O)₂**R^{y}**; wherein:
   the C₁-C₆ alkyl **of R⁴** is optionally substituted with 1 to 3 groups selected from cyano, -O**R^{y}**, -C(=O)O**R^{y}**, and -N**R^{v}R^{w}**; wherein:
      **R^{v}** and **R^{w}**, for each occurrence, are each independently hydrogen or C₁-C₄ alkyl; and
      **R^{y}**, for each occurrence, is independently hydrogen and C₁-C₄ alkyl;
   wherein:
      the C₁-C₄ alkyl of **R^{y}** is optionally substituted with 1 to 3 groups selected from halogen, cyano, -OH, -OCH₃, and -NH₂;
   and wherein all other variables not specifically defined herein are as defined in any one of the preceding embodiments.
37. The compound, tautomer, deuterated derivative, or pharmaceutically acceptable salt according to any one of embodiments 1 to 36, wherein **R⁴**, for each occurrence, is independently halogen, cyano, C₁-C₄ alkyl, C₁-C₄ alkoxy, C₁-C₄ haloalkyl, -C(=O)**R^{y}**, -C(=O)O**R^{y}**, -O**R^{y}**, or -S(=O)₂**R^{y}**; wherein:
   the C₁-C₄ alkyl of **R⁴** is optionally substituted with 1 to 3 groups selected from cyano, -O**R^{y}**, -C(=O)O**R^{y}**, and -N**R^{v}R^{w}**; wherein:
   **R^{v}** and **R^{w}**, for each occurrence, are each independently hydrogen or C₁-C₄ alkyl;
   **R^{y}**, for each occurrence, is independently hydrogen or C₁-C₂ alkyl;
   wherein:
   the C₁-C₂ alkyl of any one of **R^{y}** is optionally substituted with 1 to 3 groups selected from halogen, cyano, -OH, -OCH₃, and -NH₂; and wherein o is an integer selected from 0, 1, and 2; and
   wherein all other variables not specifically defined herein are as defined in any one of the preceding embodiments.
38. The compound, tautomer, deuterated derivative, or pharmaceutically acceptable salt according to any one of embodiments 1 to 37, wherein **R⁴**, for each occurrence, is independently cyano, C₁-C₂ alkyl, C₁-C₂ alkoxy, C₁-C₂ haloalkyl, -C(=O)R^{y}, -C(=O)O**R^{y}**, - O**R^{y}**, or -S(=O)₂**R^{y}**; wherein:
   the C₁-C₂ alkyl of **R⁴** is optionally substituted with cyano, -OH, or -OCH₃;
   **R^{y}**, for each occurrence, is independently hydrogen or C₁-C₂ alkyl;
   wherein:
   the C₁-C₂ alkyl of **R^{y}** is optionally substituted with -OCH₃;
   wherein o is an integer selected from 0 and 1;
   and wherein all other variables not specifically defined herein are as defined in any one of the preceding embodiments.
39. The compound, tautomer, deuterated derivative, or pharmaceutically acceptable salt according to any one of embodiments 1 to 38, wherein **R⁴**, for each occurrence, is independently cyano, -OH, -OCH₃, -CH₃, -C₂H₅, -CF₃, -CH₂CN, -CH₂OH, -CH₂OCH₃, -COOH, -C(=O)CH₃, - C(=O)OCH₃, -C(=O)CH₂OCH₃, -S(=O)₂CH₃, S(=O)₂C₂H₅, or S(=O)₂CF₃; and wherein all other variables not specifically defined herein are as defined in any one of the preceding embodiments.
40. A compound selected from Compounds 1-457, tautomers thereof, deuterated derivatives of those compounds and tautomers and pharmaceutically acceptable salts of the compounds, tautomers, and deuterated derivatives.
41. A pharmaceutical composition comprising at least one compound according to any one of embodiments 1 to 40, a tautomer thereof, a deuterated derivative of that compound or tautomer, or a pharmaceutically acceptable salt of the foregoing.
42. A method of treating alpha-1 antitrypsin (AAT) deficiency comprising administering to a patient in need thereof a therapeutically effective amount of at least one compound, tautomer, deuterated derivative, or pharmaceutically acceptable salt according to any one of embodiments 1 to 40, or a therapeutically effective amount of a pharmaceutical composition according to embodiment 41.
43. A method of modulating alpha-1 antitrypsin (AAT) activity comprising the step of contacting said AAT with a therapeutically effective amount of at least one compound, tautomer, deuterated derivative, or pharmaceutically acceptable salt according to any one of embodiments 1 to 40, or a therapeutically effective amount of a pharmaceutical composition according to embodiment 41.
44. The method of embodiment 42 or embodiment 43, wherein said therapeutically effective amount of the at least one compound, tautomer, deuterated derivative, or pharmaceutically acceptable salt is administered in combination with AAT augmentation therapy and/or AAT replacement therapy.

For the avoidance of doubt, features described in connection with Formula (I') may also be combined with features described in connection with Formulae (I), (IIa)-(IIf), (IIIa)-(IIIb), (IVa)-(IVb), (Va)-(Vb), (VIa)-(VIe), (VIIa)-(VIIe), and (IXa)-(IXe).

### Example Embodiments 2:

The invention is defined by the claims. Non-limiting embodiments/clauses of the present disclosure include:
1. A compound represented by the following structural formula: 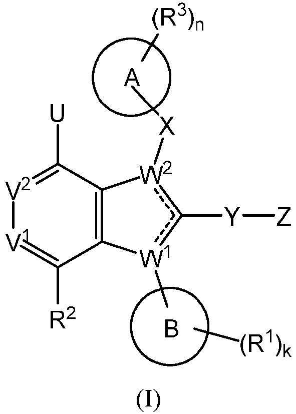 a tautomer thereof, a deuterated derivative of that compound or tautomer, or a pharmaceutically acceptable salt of the foregoing, wherein:
   ------, for each of the two occurrences, is a single bond or a double bond, provided that one is a single bond and the other is a double bond;
   **V¹** and **V²** are each independently N or -C**R²**;
   **W¹** and **W²** are each independently N or C, provided that one of W¹ and W² is N and the other is C;
   **U** is hydrogen, -OH, -CH₃, -NH₂, or halogen;
   **X** is absent or a bond, -(C**R^{a}R^{b}**)**ₚ-,** or -SO₂-;
   **Y** is absent or a bond, -(C**R^{c}R^{d}**)**_{q}-,** -C(=O)-, or -SO₂-;
   **R^{a}** and **R^{b},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
   **R^{c}** and **R^{d},** for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
   **Ring A** is C₃-C₁₂ carbocyclyl, 3 to 12-membered heterocyclyl, C₆ or C₁₀ aryl, or 5 to 10-membered heteroaryl; provided that when **W¹** is N and **W²** is C, **Ring A** is not 1,5,6,7-tetrahydro-4*H*-indol-4-onyl or a tautomer thereof;
   **Ring B** is C₄-C₁₂ cycloalkyl, C₆ or C₁₀ aryl, 5 to 10-membered heteroaryl, or benzyl;
   Z is 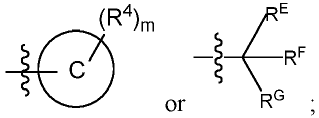 wherein:
      **Ring C** is C₃-C₁₂ cycloalkyl, 3 to 12-membered heterocyclyl, C₆ or C₁₀ aryl, or 5 to 10-membered heteroaryl;
      provided that when **Ring C** is phenyl, the phenyl is substituted with **R⁴**; provided that when **Ring C** is phenyl, **Y** cannot be -SO₂-; and
      provided that when **Ring B** is benzyl, **Ring C** cannot be pyridinyl or indolyl;
      **R^{E}**, **R^{F}**, and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
         the C₁-C₆ alkyl or the C₂-C₆ alkenyl of any one of **R^{E}**, **R^{F}**, and **R^{G}** is optionally substituted with 1 to 3 groups selected from cyano, -C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)ᵣ**R^{s}**, and -S(=O)ᵣN**R^{p}R^{q}**; wherein:
         **R^{p}**, **R^{q}**, and **R^{r}**, for each occurrence, are each independently hydrogen, C₁-C₄ alkyl, C₃-C₆ cycloalkyl, or 3 to 6-membered heterocyclyl; wherein:
            the C₁-C₄ alkyl of any one of **R^{p}**, **R^{q}**, and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
            the C₃-C₆ cycloalkyl or the 3 to 6-membered heterocyclyl of any one of **R^{p}**, **R^{q}**, and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
         **R^{s}**, for each occurrence, is independently hydrogen, C₁-C₄ alkyl, C₃-C₆ cycloalkyl, phenyl, or 5 or 6-membered heteroaryl; wherein:
            the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
            the C₃-C₆ cycloalkyl, the phenyl, or the 5 or 6-membered heteroaryl of **R^{s}** is optionally substituted with 1 to 3 groups selected from halogen, cyano, -OH, -OCH₃, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂;
      **R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl);
      **R²**, for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, -N**R^{h}Rⁱ**, phenyl, or 5 or 6-membered heteroaryl; wherein:
         the C₁-C₆ alkyl, the C₂-C₆ alkenyl or the C₃-C₆ cycloalkyl of **R²** is optionally substituted with 1 to 3 groups selected from cyano, -C(=O)**R^{k}**, -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**Rₖ**, -OC(=O)**R^{k}**, -OC(=O)O**R^{k}**, -OC(=O)**NR^{h}Rⁱ**, -S(=O)**ₛR^{k}**, and S(=O)ₛN**R^{h}Rⁱ**; wherein:
         **R^{h}**, **Rⁱ**, and **R^{j}**, for each occurrence, are each independently hydrogen, C₁-C₄ alkyl, or C₃-C₆ cycloalkyl; wherein:
            the C₁-C₄ alkyl of any one of **R^{h}**, **Rⁱ**, and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl of any one of **R^{h}**, **Rⁱ**, and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{k}**, for each occurrence, is independently hydrogen, C₁-C₄ alkyl, C₃-C₆ cycloalkyl, phenyl, or 5 or 6-membered heteroaryl; wherein:
            -O**R^{k}** cannot be -OH;
            the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁- C₂ alkyl)₂;
      **R³** and **R⁴**, for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)ₜ**R^{y}**, -N**R^{v}R^{w}**, - N**R^{v}**C(=O)**R^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, - OC(=O)O**R^{y}**, -OC(=O)N**R^{v}R^{w}**, -S(=O)ₜ**R^{y}**, -S(=O)ₜN**R^{v}R^{w}**, -S(=O)ₜN**R^{v}**C(=O)**R^{y}**, - P(=O)**R^{z}R^{z}**, phenyl, or a 5 or 6-membered heteroaryl; wherein:
         the C₁-C₆ alkyl, the C₂-C₆ alkenyl, or the C₃-C₆ cycloalkyl of any one of **R³** and **R⁴** is optionally substituted with 1 to 3 groups selected from cyano, - C(=O)**R^{y}**, -C(=O)O**R^{y}**, -C(=O)NR^{v}R^{w}, -N**R^{v}R^{w}**, -N**R^{v}**C(=O)**R^{y}**, -N**R^{v}**C(=O)O**R^{y}**, -N**R^{v}**C(=O)N**R^{w}R^{x}**, - -NR^{v}S(=O)ᵣ**R^{y}**, -O**R^{y}**, -OC(=O)**R^{y}**, - OC(=O)O**R^{y}**, -OC(=O)N**R^{v}R^{w}**, -S(=O)**ₜR^{y}**, and
         -S(=O)ₜN**R^{v}R^{w}**; wherein:
         **R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
            the C₁-C₄ alkyl of any one of **R^{v}, R^{w},** and **R^{x}** is optionally substituted with 1 to 3 groups selected from halogen, cyano -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
            the C₃-C₆ cycloalkyl, the 5 or 6-membered heterocyclyl, or the 5 or 6-membered heteroaryl of any one of **R^{v}**, **R^{w}**, and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy,
               -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{y}**, for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
            the C₁-C₄ alkyl of **R^{y}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl),
               -C(=O)NH₂, -C(=O)NH(C₁-C₂ alkyl), and -C(=O)N(C₁-C₂ alkyl)₂; and
            the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, -NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
         **R^{z}**, for each occurrence, is independently C₁-C₂ alkyl, -OH, or -O(C₁-C₂ alkyl);
   **k** is an integer selected from 1, 2, and 3;
   **m** and **n** a are each independently an integer selected from 0, 1, 2, and 3;
   **p**, **r**, **s**, and **t** are each independently an integer selected from 1 and 2; and
   **q** is an integer selected from 1, 2, and 3.
2. The compound, tautomer, deuterated derivative, or pharmaceutically acceptable salt according to clause 1 represented by one of the following structural formulae: 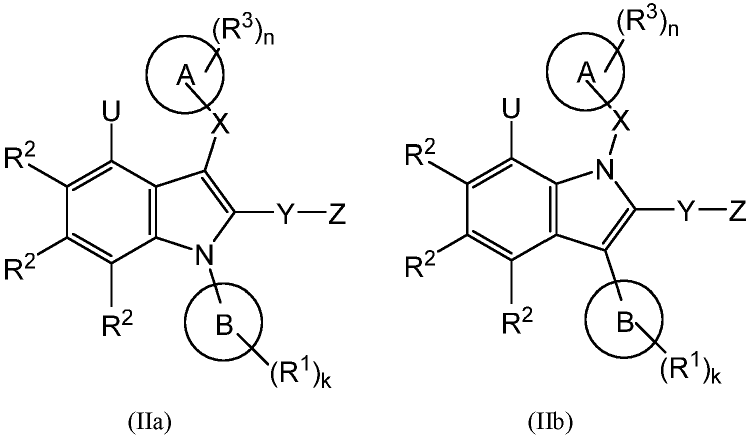 wherein:
   **U** is -OH, -CH₃, -NH₂, F, or Cl; and wherein all other variables not specifically defined herein are as defined in embodiment 1.
3. The compound, tautomer, deuterated derivative, or pharmaceutically acceptable salt according to clause 1 represented by one of the following structural formulae: 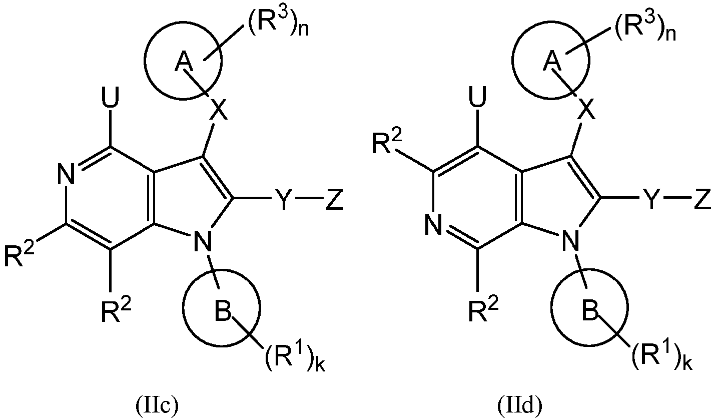 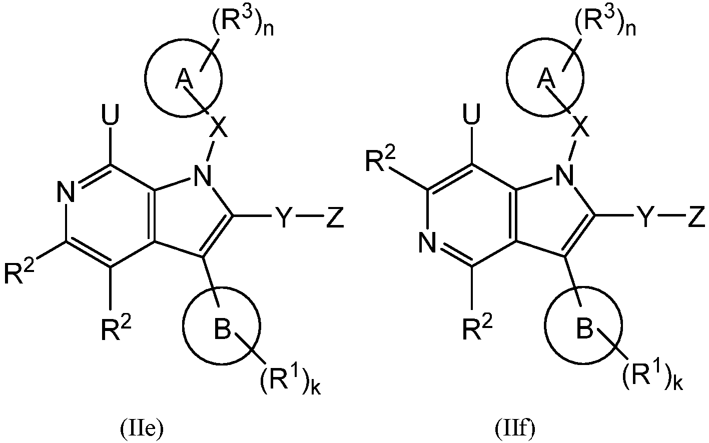 wherein:
   **U** is -OH, -CH₃, -NH₂, F, or Cl;
   and wherein all other variables not specifically defined herein are as defined in embodiment 1.
4. The compound, tautomer, deuterated derivative, or pharmaceutically acceptable salt according to clause 1 or clause 2 represented by one of the following structural formulae: 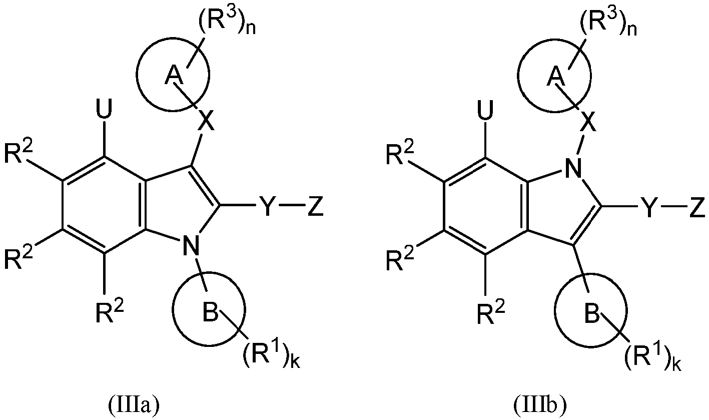 wherein:
   **U** is -OH or -NH₂;
   **Ring A** is optionally substituted with **R³** and **Ring A** is C₃-C₇ carbocyclyl, 6 to 9-membered heterocyclyl, phenyl, or 5 to 9-membered heteroaryl;
   **Ring B** is substituted with **R¹** and **Ring B** is C₄-C₆ cycloalkyl, phenyl, 5 to 6-membered heteroaryl, or benzyl; and
   when **Z** is **Ring C** optionally substituted with **R⁴**, **Ring C** is C₄-C₈ cycloalkyl, 4 to 8-membered heterocyclyl, phenyl, or 5 or 6-membered heteroaryl;
   and wherein all other variables not specifically defined herein are as defined in clause 1 or clause 2.
5. The compound, tautomer, deuterated derivative, or pharmaceutically acceptable salt according to any one of clauses 1, 2, and 4 represented by one of the following structural formulae: 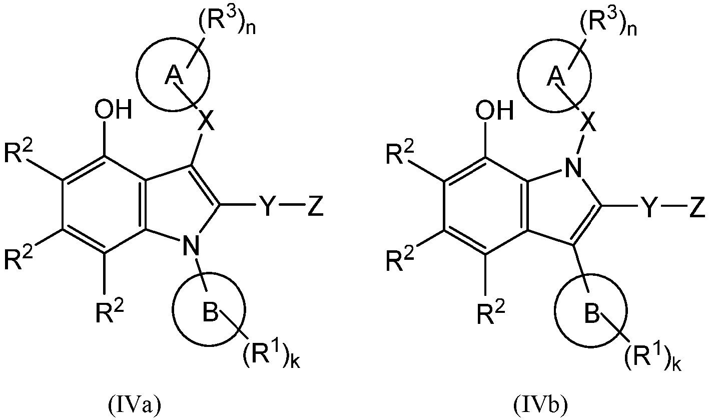 wherein:
   **Ring B** is substituted with **R¹** and **Ring B** is cyclohexyl, phenyl, pyridinyl, or benzyl; and wherein all other variables not specifically defined herein are as defined in any one of clauses 1, 2, and 4.
6. The compound, tautomer, deuterated derivative, or pharmaceutically acceptable salt according to any one of clauses 1, 2, 4, and 5 represented by the following structural formula: 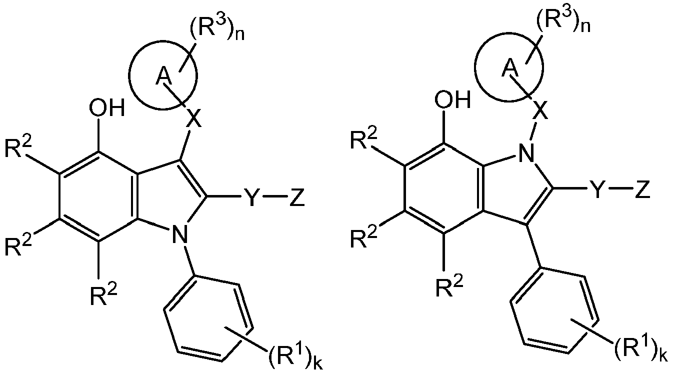 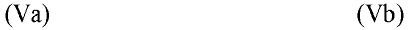 wherein:
   **R¹** is halogen, cyano, C₁-C₂ alkyl, C₁-C₂ haloalkyl, or C₁-C₂ alkoxy; and
   **k** is an integer selected from 1 and 2;
   and wherein all other variables not specifically defined herein are as defined in any one of clauses 1, 2, 4, and 5.
7. The compound, tautomer, deuterated derivative, or pharmaceutically acceptable salt according to any one of clauses 1 to 6, wherein **R¹** is cyano, F, Cl, -CH₃, -CHF₂, -CF₃, -OCH₃, or -OCH(CH₃)₂; and wherein all other variables not specifically defined herein are as defined in any one of the preceding clauses.
8. The compound, tautomer, deuterated derivative, or pharmaceutically acceptable salt according to any one of clauses 1 to 7, wherein at least one **R¹** is F; and wherein all other variables not specifically defined herein are as defined in any one of the preceding clauses.
9. The compound, tautomer, deuterated derivative, or pharmaceutically acceptable salt according to any one of clauses 1 to 8, wherein:
   X is absent or a bond, -(C**R^{a}R^{b}**)-, or -SO₂-;
   **R^{a}** and **R^{b}**, for each occurrence, are each independently hydrogen, C₁-C₃ alkyl, C₁-C₃ haloalkyl, or C₁-C₃ alkoxy; and wherein all other variables not specifically defined herein are as defined in any one of the preceding clauses.
10. The compound, tautomer, deuterated derivative, or pharmaceutically acceptable salt according to any one of clauses 1 to 9, wherein **X** is absent or a bond, -CH₂-, or -SO₂-; and wherein all other variables not specifically defined herein are as defined in any one of the preceding clauses.
11. The compound, tautomer, deuterated derivative, or pharmaceutically acceptable salt according to any one of clauses 1 to 10, wherein:
   **Y** is absent or a bond, -(C**R^{c}R^{d}**)_{q}-, -C(=O)-, or -SO₂-;
   **R^{c}** and **R^{d}**, for each occurrence, are each independently hydrogen, C₁-C₃ alkyl, C₁-C₃ haloalkyl, or C₁-C₃ alkoxy; and wherein all other variables not specifically defined herein are as defined in any one of the preceding clauses.
12. The compound, tautomer, deuterated derivative, or pharmaceutically acceptable salt according to any one of clauses 1 to 11, wherein **q** is an integer selected from 1 and 2; and wherein all other variables not specifically defined herein are as defined in any one of the preceding clauses.
13. The compound, tautomer, deuterated derivative, or pharmaceutically acceptable salt according to any one of clauses 1 to 12, wherein **Y** is absent or a bond, -CH₂-, -CHCH₃-, - C(CH₃)₂-, -C(=O)-, or -SO₂-; and wherein all other variables not specifically defined herein are as defined in any one of the preceding clauses.
14. The compound, tautomer, deuterated derivative, or pharmaceutically acceptable salt according to any one of clauses 1 to 13, wherein **Ring A** is optionally substituted with **R³** and **Ring A** is C₃-C₇ carbocyclyl, 6 to 9-membered heterocyclyl containing 1 to 3 oxygen atoms, phenyl, or 5 to 9-membered heteroaryl containing 1 to 3 heteroatoms selected from O and N; and wherein all other variables not specifically defined herein are as defined in any one of the preceding clauses.
15. The compound, tautomer, deuterated derivative, or pharmaceutically acceptable salt according to any one of clauses 1 to 14, wherein **Ring A** is optionally substituted with **R³** and **Ring A** is C₃-C₇ carbocyclyl, 6 to 9-membered heterocyclyl containing one or two oxygen atoms, phenyl, or 5 to 9-membered heteroaryl containing one or two nitrogen atoms or one or two oxygen atoms; and wherein all other variables not specifically defined herein are as defined in any one of the preceding clauses.
16. The compound, tautomer, deuterated derivative, or pharmaceutically acceptable salt according to any one of clauses 1 to 15, wherein **Ring A** is optionally substituted with **R³** and **Ring A** is selected from 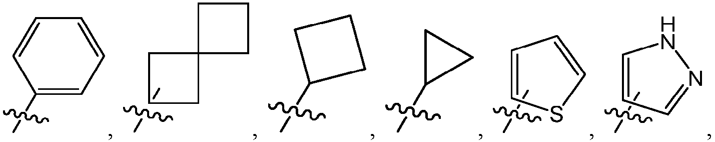 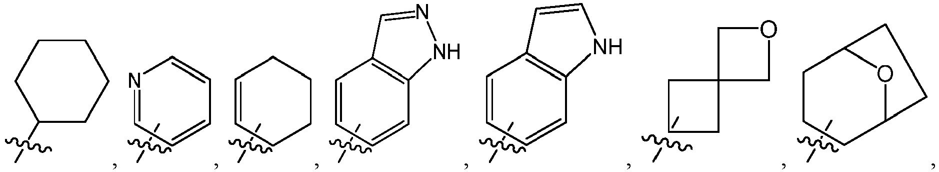 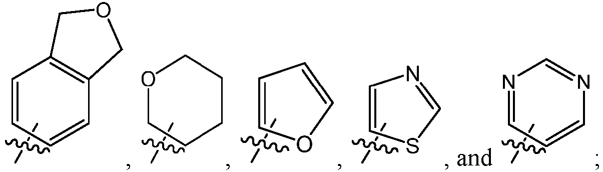 and wherein all other variables not specifically defined herein are as defined in any one of the preceding clauses.
17. The compound, tautomer, deuterated derivative, or pharmaceutically acceptable salt according to any one of clauses 1 to 16, wherein **Ring A** is optionally substituted with **R³** and **Ring** A is selected from 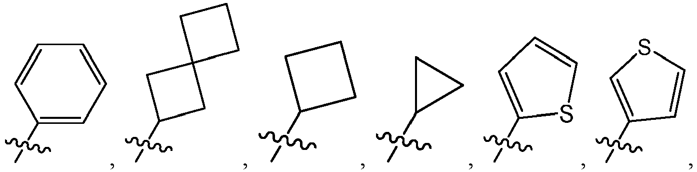 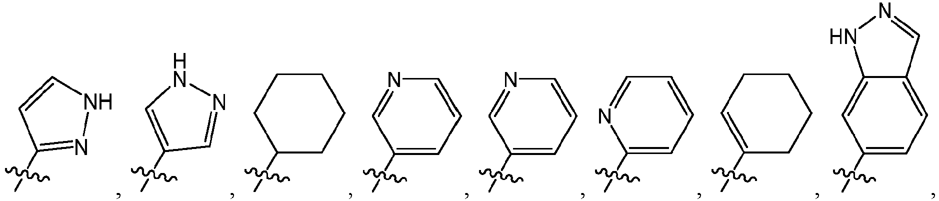 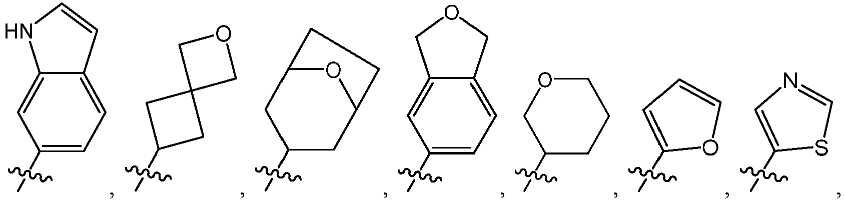 and 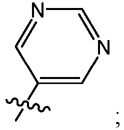 and wherein all other variables not specifically defined herein are as defined in any one of the preceding clauses.
18. The compound, tautomer, deuterated derivative, or pharmaceutically acceptable salt according to any one of clauses 1 to 17, wherein **Z** is **Ring C, Ring C** is optionally substituted with **R⁴**, and **Ring C** is C₄-C₈ cycloalkyl; 4 to 8-membered heterocyclyl containing one or two heteroatoms selected from O, N, and S; phenyl; or 5-membered heteroaryl containing one or two heteroatoms selected from O and N; and wherein all other variables not specifically defined herein are as defined in any one of the preceding clauses.
19. The compound, tautomer, deuterated derivative, or pharmaceutically acceptable salt according to any one of clauses 1 to 18, wherein **Z** is **Ring C, Ring C** is optionally substituted with **R⁴**, and **Ring C** is selected from 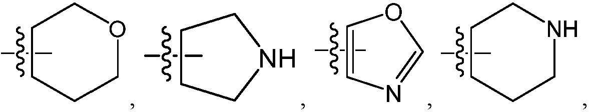 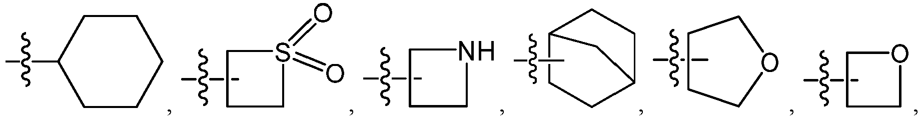 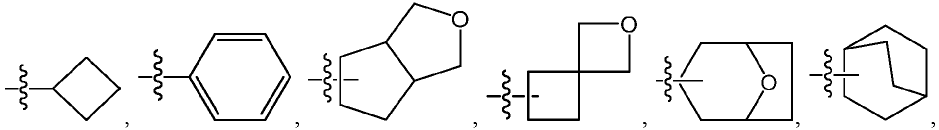 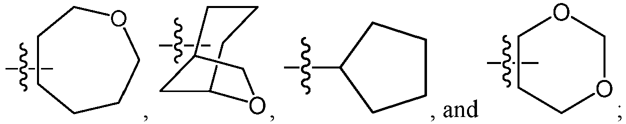 and wherein all other variables not specifically defined herein are as defined in any one of the preceding clauses.
20. The compound, tautomer, deuterated derivative, or pharmaceutically acceptable salt according to any one of embodiments 1 to 19, wherein **Z** is **Ring C**, **Ring C** is optionally substituted with (**R⁴**)ₒ, and **Ring C** is selected from 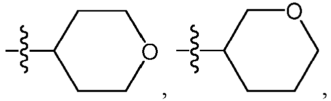 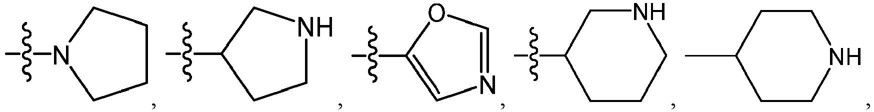 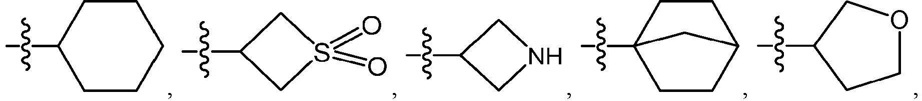 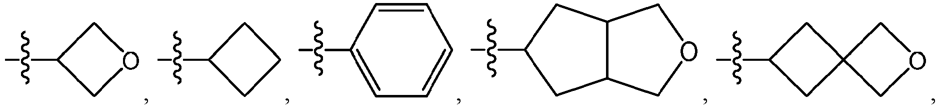 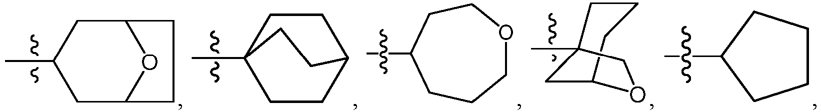 and 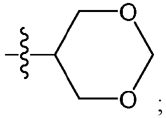 and wherein all other variables not specifically defined herein are as defined in any one of the preceding clauses.
21. The compound, tautomer, deuterated derivative, or pharmaceutically acceptable salt according to any one of clauses 1 to 20, wherein **R^{E}**, **R^{F}**, and **R^{G}** are each independently hydrogen, halogen, cyano (-C≡N), C₁-C₄ alkyl, C₁-C₄ alkoxy, C₁-C₄ haloalkyl, -C(=O)O**R^{s}**, - C(=O)N**R^{p}R^{q}**, -C**R^{p}**(=N)O**R^{s}**, or -O**R^{s}**; wherein:
   the C₁-C₄ alkyl of any one of **R^{E}**, **R^{F}**, and **R^{G}** is optionally substituted with 1 to 3 groups selected from cyano, -C(=O)**R^{s}**, -C(=O)O**R^{s}**, -C(=O)N**R^{p}R^{q}**, -O**R^{s}**, -OC(=O)**R^{s}**, -OC(=O)O**R^{s}**, -OC(=O)N**R^{p}R^{q}**, and -S(=O)₂**R^{s}**; wherein:
   **R^{p}** and **R^{q}**, for each occurrence, are each independently hydrogen, C₁-C₂ alkyl, C₃-C₅ cycloalkyl, or 5 or 6-membered heterocyclyl; wherein:
      the C₁-C₂ alkyl of any one of **R^{p}** and **R^{q}** is optionally substituted with 1 to 3 groups selected from halogen, cyano, and -OH;
      the C₃-C₅ cycloalkyl or the 5 or 6-membered heteroaryl of **R^{p}** and **R^{q}** is optionally substituted with 1 to 3 groups selected from halogen, cyano, and -OH;
   **R^{s}**, for each occurrence, is independently hydrogen, C₁-C₂ alkyl, or 5 or 6-membered heteroaryl; wherein the C₁-C₂ alkyl of **R^{s}** is optionally substituted with 1 to 3 groups selected from halogen, cyano, -OH, and
      -NH₂; wherein:
      the 5 or 6-membered heteroaryl of **R^{s}** is optionally substituted with 1 to 3 groups selected from halogen, cyano, -OH, and -NH₂;
      and wherein all other variables not specifically defined herein are as defined in any one of the preceding clauses.
22. The compound, tautomer, deuterated derivative, or pharmaceutically acceptable salt according to any one of clauses 1 to 21, wherein **R^{E}**, **R^{F}**, and **R^{G}** are each independently hydrogen, F, Cl, C₁-C₂ alkyl, C₁-C₂ haloalkyl, -C(=O)N**R^{p}R^{q}**, -C**R^{p}**(=N)O**R^{s}**, or -O**R^{s}**; wherein:
   the C₁-C₂ alkyl of any one of **R^{E}**, **R^{F}**, and **R^{G}** is optionally substituted with 1 to 3 groups selected from cyano, -C(=O)N**R^{p}R^{q}**, -O**R^{s}**, -OC(=O)N**R^{p}R^{q}**, and
   -S(=O)₂**R^{s}**; wherein:
   **R^{p}** and **R^{q}**, for each occurrence, are each independently hydrogen, C₁-C₂ alkyl, cyclopentyl, or tetrahydrofuranyl; wherein:
      the C₁-C₂ alkyl of any one of **R^{p}** and **R^{q}** is optionally substituted with 1 to 3 halogen groups selected from F and Cl;
   **R^{s}**, for each occurrence, is independently hydrogen, C₁-C₂ alkyl, pyridinyl, or pyrimidinyl; wherein:
      the C₁-C₂ alkyl of **R^{s}** is optionally substituted with 1 to 3 halogen groups selected from F and Cl;
   and wherein all other variables not specifically defined herein are as defined in any one of the preceding clauses.
23. The compound, tautomer, deuterated derivative, or pharmaceutically acceptable salt according to any one of clauses 1 to 22, wherein **R^{E}**, **R^{F}**, and **R^{G}** are each independently hydrogen, F, -OH, -CH(OH)CH₃, -C(=O)NHCH₃, -C(=N)OCH₃, -CH₃, -CF₃, -CH₂F, -CH₂CN, -(CH₂)₂CN, -CH₂OH, -C₂H₅, -(CH₂)₂OH, -CH₂OCH₃, -CH₂OC₂H₅, -(CH₂)₂OCH₃, -CH₂OCHF₂, -(CH₂)₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅,-CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), or -CH₂(O)(pyrimidin-2-yl); and wherein all other variables not specifically defined herein are as defined in any one of the preceding clauses.
24. The compound, tautomer, deuterated derivative, or pharmaceutically acceptable salt according to any one of clauses 1 to 23, wherein **R^{E}**, **R^{F}**, and **R^{G}** are each independently hydrogen, F, -CH(OH)CH₃, -CH₃, -CH₂CN, -CH₂OH, or -CH₂OCH₃; and wherein all other variables not specifically defined herein are as defined in any one of the preceding clauses.
25. The compound, tautomer, deuterated derivative, or pharmaceutically acceptable salt according to any one of clauses 1 to 24 represented by one of the following structural formulae: 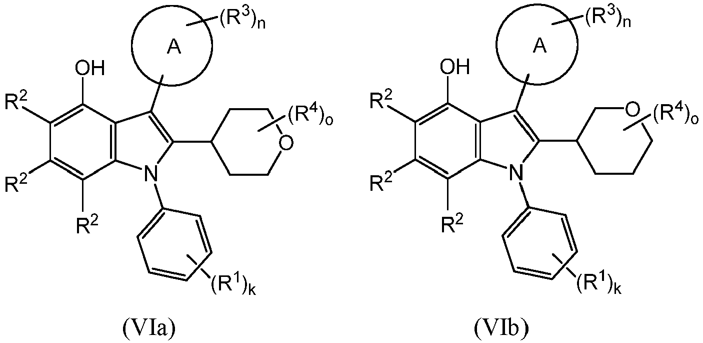 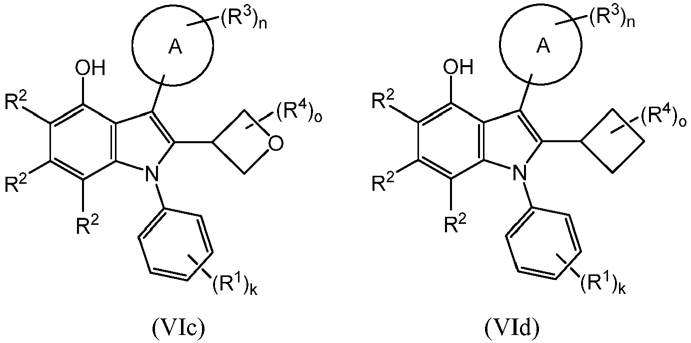 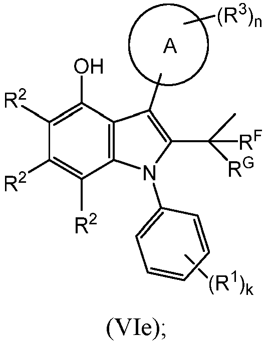 wherein all other variables not specifically defined herein are as defined in any one of the preceding clauses.
26. The compound, tautomer, deuterated derivative, or pharmaceutically acceptable salt according to any one of clauses 1 to 25 represented by one of the following structural formulae: 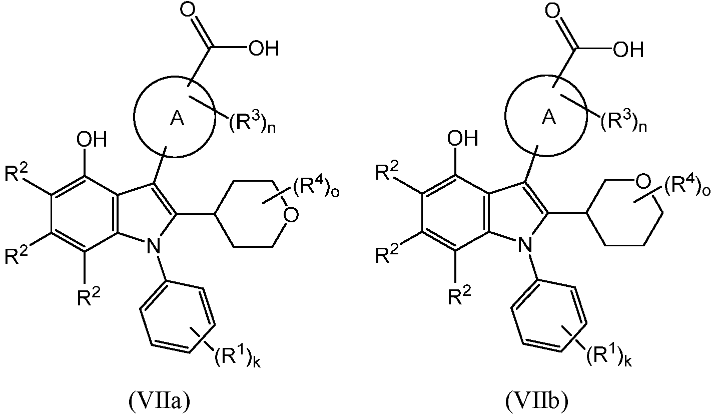 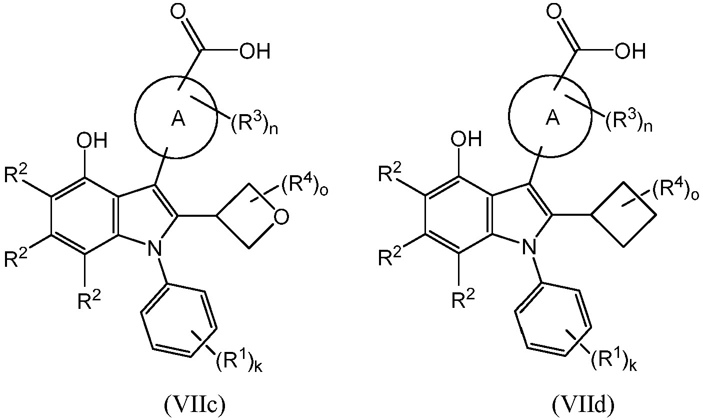 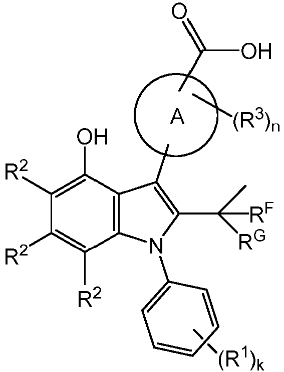 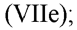 wherein **n** is an integer selected from 0, 1, and 2; and wherein all other variables not specifically defined herein are as defined in any one of the preceding clauses.
27. The compound, tautomer, deuterated derivative, or pharmaceutically acceptable salt according to any one of clauses 1 to 26 represented by one of the following structural formulae: 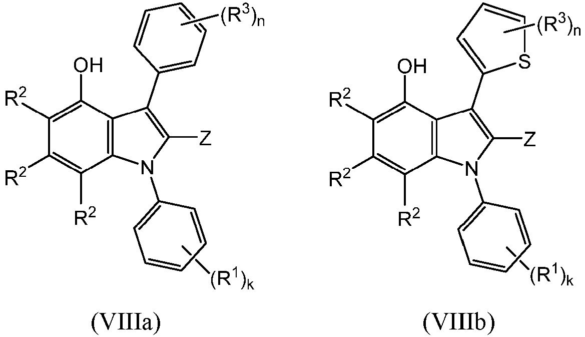 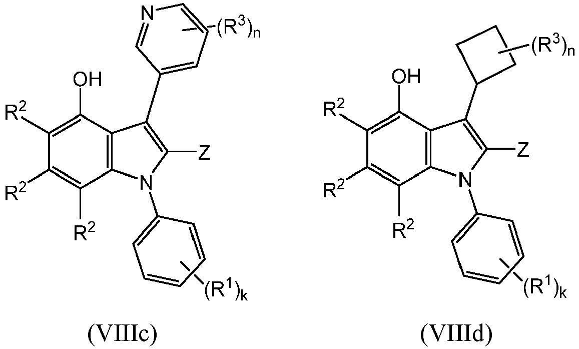 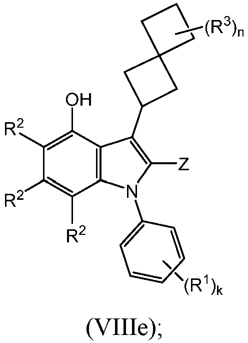 wherein all other variables not specifically defined herein are as defined in any one of the preceding clauses.
28. The compound, tautomer, deuterated derivative, or pharmaceutically acceptable salt according to any one of clauses 1 to 27 represented by one of the following structural formulae: 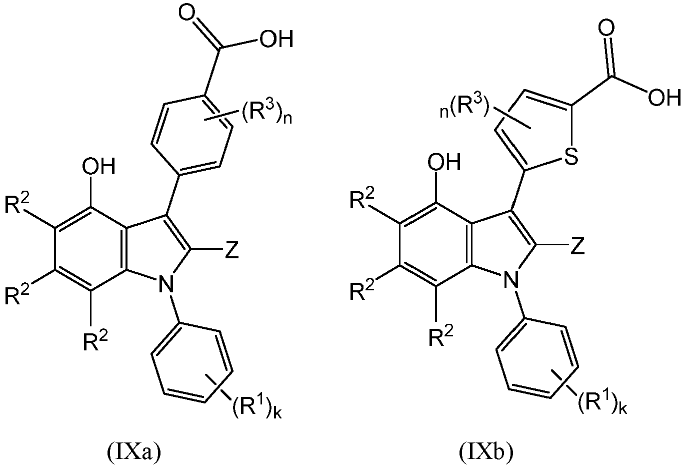 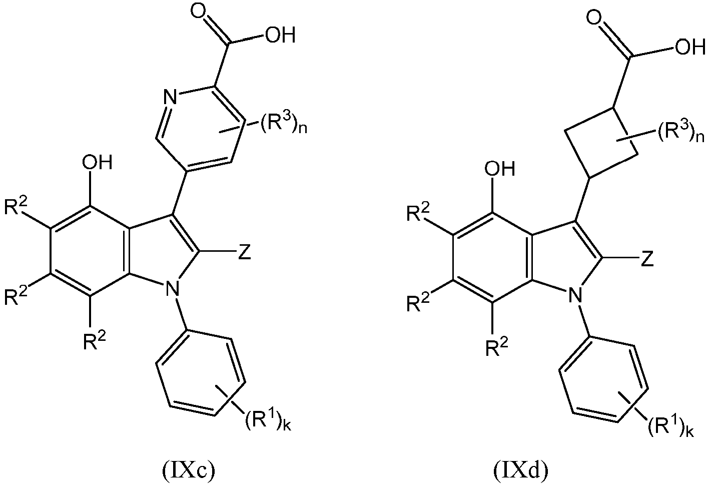 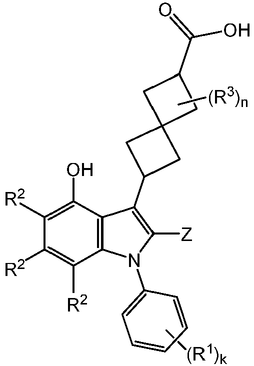 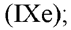 wherein **n** is an integer selected from 0, 1, and 2; and wherein all other variables not specifically defined herein are as defined in any one of the preceding clauses.
29. The compound, tautomer, deuterated derivative, or pharmaceutically acceptable salt according to any one of clauses 1 to 28, wherein **R²**, for each occurrence, is independently hydrogen, halogen, cyano, C₁-C₆ alkyl (optionally substituted with 1 to 3 groups selected from cyano, -OH, -OCH₃, and -NH₂), C₁-C₆ alkoxy, C₁-C₆ haloalkyl, -N**R^{h}Rⁱ**, or C₃-C₆ cycloalkyl; wherein **R^{h}** and **Rⁱ**, for each occurrence, are each independently hydrogen or C₁-C₄ alkyl; and wherein all other variables not specifically defined herein are as defined in any one of the preceding clauses.
30. The compound, tautomer, deuterated derivative, or pharmaceutically acceptable salt according to any one of clauses 1 to 29, wherein **R²**, for each occurrence, is independently hydrogen, halogen, cyano, C₁-C₄ alkyl (optionally substituted with 1 to 3 groups selected from cyano, -OH, -OCH₃, and -NH₂), C₁-C₄ alkoxy, C₁-C₄ haloalkyl, -N**R^{h}Rⁱ**, or C₃-C₅ cycloalkyl; wherein **R^{h}** and **Rⁱ**, for each occurrence, are each independently hydrogen or C₁-C₄ alkyl; and wherein all other variables not specifically defined herein are as defined in any one of the preceding clauses.
31. The compound, tautomer, deuterated derivative, or pharmaceutically acceptable salt according to any one of clauses 1 to 30, wherein **R²**, for each occurrence, is independently hydrogen, halogen, cyano, C₁-C₂ alkyl (optionally substituted with 1 to 3 groups selected from cyano, -OH, -OCH₃, and -NH₂), C₁-C₂ haloalkyl, -N**R^{h}Rⁱ**, or C₃-C₄ cycloalkyl; wherein **R^{h}** and **Rⁱ**, for each occurrence, are each independently hydrogen or -CH₃;
   and wherein all other variables not specifically defined herein are as defined in any one of the preceding clauses.
32. The compound, tautomer, deuterated derivative, or pharmaceutically acceptable salt according to any one of clauses 1 to 31, wherein **R²**, for each occurrence, is independently hydrogen, F, Cl, cyano, -CH₃, -CHF₂, -CF₃, -NH₂, or cyclopropyl; and wherein all other variables not specifically defined herein are as defined in any one of the preceding clauses.
33. The compound, tautomer, deuterated derivative, or pharmaceutically acceptable salt according to any one of clauses 1 to 32, wherein **R³**, for each occurrence, is independently halogen, cyano, =O, C₁-C₆ alkyl, C₁-C₆ alkoxy, C₁-C₆ haloalkyl,
   -C(=O)**R^{y}**, -C(=O)O**R^{y}**, -C(=O)N**R^{v}R^{w}**, -C(=O)N**R^{v}**O**R^{y}**,-C(=O)NR^{v}S(=O)₂**R^{y}**, -N**R^{v}R^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₄ alkyl of **R³** is optionally substituted with 1 to 3 groups selected from cyano, -O**R^{y}**, -C(=O)O**R^{y}**, and -N**R^{v}R^{w}**; wherein:
   **R^{v}**, **R^{w}**, and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with 1 to 3 groups selected from halogen, cyano, -OH, and -NH₂; and
   **R^{y}**, for each occurrence, is independently hydrogen, C₁-C₄ alkyl, or 5 or 6-membered heterocyclyl; wherein:
      the C₁-C₄ alkyl of **R^{y}** is optionally substituted with 1 to 3 groups selected from halogen, cyano, -OH, -NH₂, and -C(=O)OH; and
      the 5 or 6-membered heterocyclyl of **R^{y}** is optionally substituted with 1 to 3 groups selected from halogen, cyano, -OH,
      -NH₂, and -C(=O)OH;
   and wherein all other variables not specifically defined herein are as defined in any one of the preceding clauses.
34. The compound, tautomer, deuterated derivative, or pharmaceutically acceptable salt according to any one of clauses 1 to 33, wherein **R³,** for each occurrence, is independently halogen, cyano, =O, C₁-C₄ alkyl, C₁-C₄ alkoxy, C₁-C₄ haloalkyl,
   -C(=O)**R^{y}**, -C(=O)O**R^{y}**, -C(=O)N**R^{v}R^{w}**,-C(=O)N**R^{v}**O**R^{y}**, -C(=O)N**R^{v}**S(=O)2**R^{y}**, -N**R^{v}R^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₄ alkyl of **R³** is optionally substituted with 1 to 3 groups selected from cyano, -O**R^{y}** and -C(=O)O**R^{y}**; wherein:
      **R^{v}**, **R^{w}**, and **R^{x}**, for each occurrence, are each independently hydrogen or C₁-C₄ alkyl; wherein the C₁-C₄ alkyl of any one of **R^{v}**, **R^{w}**, and **R^{x}** is optionally substituted with -OH; and
      **R^{v}**, for each occurrence, is independently hydrogen, C₁-C₂ alkyl, or 6-membered heterocyclyl; wherein:
         the C₁-C₂ alkyl of **R^{y}** is optionally substituted with 1 to 3 groups selected from -OH and -C(=O)OH; and
         the 6-membered heterocyclyl of **R^{y}** is optionally substituted with 1 to 3 groups selected from -OH and -C(=O)OH;
   **R^{z}**, for each occurrence, is independently -CH₃, -OH, or -OCH₃; and wherein all other variables not specifically defined herein are as defined in any one of the preceding clauses.
35. The compound, tautomer, deuterated derivative, or pharmaceutically acceptable salt according to any one of clauses 1 to 34, wherein **R³**, for each occurrence, is independently halogen, cyano, =O, C₁-C₂ alkyl, C₁-C₂ alkoxy, C₁-C₄ haloalkyl,
   -C(=O)**R^{y},** -C(=O)O**R^{y}**, -C(=O)N**R^{v}R^{w}**,-C(=O)N**R^{v}**O**R^{y}**, -C(=O)N**R^{v}**S(=O)₂**R^{y}**, -N**R^{v}**R**^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
   the C₁-C₂ alkyl of **R³** is optionally substituted with 1 to 3 groups selected from -OH and -C(=O)O**R^{y}**; wherein:
      **R^{v}**, **R^{w}**, and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with -OH; and
      **R^{y}**, for each occurrence, is independently hydrogen, C₁-C₂ alkyl, or tetrahydro-2H-pyranyl; wherein:
         the C₁-C₂ alkyl of **R^{y}** is optionally substituted with -C(=O)OH; and
         the tetrahydro-2H-pyranyl of **R^{y}** is optionally substituted with 1 to 3 groups selected from -OH and -C(=O)OH;
   **R^{z}**, for each occurrence, is independently -CH₃ or -OH;
   and wherein all other variables not specifically defined herein are as defined in any one of the preceding clauses.
36. The compound, tautomer, deuterated derivative, or pharmaceutically acceptable salt according to any one of clauses 1 to 35, wherein **R³**, for each occurrence, is independently F, Cl, cyano, -OH, =O, -CH₃, -OCH₃, -CF₃, -CH₃CN, -C(CH₃)₂CH₂OH, -CH₂COOH, -CH₂OCH₃, - C(=O)CHCH₃OH, -COOH, -C(=O)O(2-tetrahydro-2H-pyranyl), -C(=O)NH₂, - C(=O)NH(CH₂)₂OH, -C(=O)NHOH, -C(=O)NHS(=O)₂CH₃, -NH₂, -NHCH₃, -OCH₂COOH, NHS(=O)₂CH₃,-S(=O)₂CH₃, -S(=O)₂NH₂, -S(=O)₂NHC(=O)CH₃, or -P(=O)(CH₃)₂; wherein the 2-tetrahydro-2H-pyranyl in -C(=0)0(2-tetrahydro-2H-pyranyl) is substituted with 1 to 3 groups selected from -OH and -C(=O)OH; and wherein all other variables not specifically defined herein are as defined in any one of the preceding clauses.
37. The compound, tautomer, deuterated derivative, or pharmaceutically acceptable salt according to any one of clauses 1 to 36, wherein **R⁴**, for each occurrence, is independently halogen, cyano, C₁-C₆ alkyl, C₁-C₆ alkoxy, C₁-C₆ haloalkyl,
   -C(=O)**R^{y},** -C(=O)O**R^{y}**, -O**R^{y}**, or -S(=O)₂**R^{y}**; wherein:
   the C₁-C₆ alkyl of **R⁴** is optionally substituted with 1 to 3 groups selected from cyano, -O**R^{y}**, -C(=O)O**R^{y}**, and -N**R^{v}R^{w}**; wherein:
      **R^{v}** and **R^{w},** for each occurrence, are each independently hydrogen or C₁-C₄ alkyl; and
      **R^{y}**, for each occurrence, is independently hydrogen and C₁-C₄ alkyl;
   wherein:
      the C₁-C₄ alkyl of **R^{y}** is optionally substituted with 1 to 3 groups selected from halogen, cyano, -OH, -OCH₃, and -NH₂;
      and wherein all other variables not specifically defined herein are as defined in any one of the preceding clauses.
38. The compound, tautomer, deuterated derivative, or pharmaceutically acceptable salt according to any one of clauses 1 to 37, wherein **R⁴**, for each occurrence, is independently halogen, cyano, C₁-C₄ alkyl, C₁-C₄ alkoxy, C₁-C₄ haloalkyl, -C(=O)**R^{y},**
   -C(=O)O**R^{y}**, -O**R^{y}**, or -S(=O)₂**R^{y}**; wherein:
   the C₁-C₄ alkyl of **R⁴** is optionally substituted with 1 to 3 groups selected from cyano, -O**R^{y}**, -C(=O)O**R^{y}**, and -N**R^{v}R^{w};** wherein:
      **R^{v}** and **R^{w},** for each occurrence, are each independently hydrogen or C₁-C₄ alkyl;
      **R^{y},** for each occurrence, is independently hydrogen or C₁-C₂ alkyl;
   wherein:
      the C₁-C₂ alkyl of any one of **R^{y}** is optionally substituted with 1 to 3 groups selected from halogen, cyano, -OH, -OCH₃, and -NH₂; and

   wherein o is an integer selected from 0, 1, and 2; and
   wherein all other variables not specifically defined herein are as defined in any one of the preceding clauses.
39. The compound, tautomer, deuterated derivative, or pharmaceutically acceptable salt according to any one of clauses 1 to 38, wherein **R⁴**, for each occurrence, is independently cyano, C₁-C₂ alkyl, C₁-C₂ alkoxy, C₁-C₂ haloalkyl, -C(=O)R^{y}, -C(=O)O**R^{y}**, -O**R^{y}**, or - S(=O)₂**R^{y}**; wherein:
   the C₁-C₂ alkyl of **R⁴** is optionally substituted with cyano, -OH, or -OCH₃;
   **R^{y},** for each occurrence, is independently hydrogen or C₁-C₂ alkyl;
   wherein:
   the C₁-C₂ alkyl of **R^{y}** is optionally substituted with -OCH₃;
   wherein o is an integer selected from 0 and 1; and wherein all other variables not specifically defined herein are as defined in any one of the preceding clauses.
40. The compound, tautomer, deuterated derivative, or pharmaceutically acceptable salt according to any one of clauses 1 to 39, wherein **R⁴**, for each occurrence, is independently cyano, -OH, -OCH₃, -CH₃, -C₂H₅, -CF₃, -CH₂CN, -CH₂OH, -CH₂OCH₃, -COOH, -C(=O)CH₃, - C(=O)OCH₃, -C(=O)CH₂OCH₃, -S(=O)₂CH₃, S(=O)₂C₂H₅, or S(=O)₂CF₃; and wherein all other variables not specifically defined herein are as defined in any one of the preceding clauses.
41. The compound, tautomer, deuterated derivative, or pharmaceutically acceptable salt according to any one of clauses 1 to 40, wherein **U** is -OH, -CH₃, -NH₂, or halogen; and wherein all other variables not specifically defined herein are as defined in any one of the preceding clauses.
42. The compound, tautomer, deuterated derivative, or pharmaceutically acceptable salt according to any one of clauses 1 to 40, wherein **U** is -OH; and wherein all other variables not specifically defined herein are as defined in any one of the preceding clauses.
43. The compound, tautomer, deuterated derivative, or pharmaceutically acceptable salt according to any one of clauses 1 to 40, wherein **U** is halogen; and wherein all other variables not specifically defined herein are as defined in any one of the preceding clauses.
44. The compound, tautomer, deuterated derivative, or pharmaceutically acceptable salt according to any one of clauses 1 to 40, wherein **U** is fluoro; and wherein all other variables not specifically defined herein are as defined in any one of the preceding clauses.
45. The compound, tautomer, deuterated derivative, or pharmaceutically acceptable salt according to any one of clauses 1 to 40, wherein **U** is hydrogen; and wherein all other variables not specifically defined herein are as defined in any one of the preceding clauses.
46. A compound selected from Compounds 1-457, Compounds 458-532, Compounds B1-B25, Compounds W1-W32, and Compounds P1-P225, tautomers thereof, deuterated derivatives of those compounds and tautomers and pharmaceutically acceptable salts of the compounds, tautomers, and deuterated derivatives.
47. A compound selected from Compounds 1-457, tautomers thereof, deuterated derivatives of those compounds and tautomers and pharmaceutically acceptable salts of the compounds, tautomers, and deuterated derivatives.
48. A compound selected from Compounds 458-532, tautomers thereof, deuterated derivatives of those compounds and tautomers and pharmaceutically acceptable salts of the compounds, tautomers, and deuterated derivatives.
49. A compound selected from Compounds B1-B25 and Compounds W1-W32, tautomers thereof, deuterated derivatives of those compounds and tautomers and pharmaceutically acceptable salts of the compounds, tautomers, and deuterated derivatives.
50. A compound selected from Compounds P1-P225, tautomers thereof, deuterated derivatives of those compounds and tautomers and pharmaceutically acceptable salts of the compounds, tautomers, and deuterated derivatives.
51. A pharmaceutical composition comprising at least one compound according to any one of clauses 1 to 50, a tautomer thereof, a deuterated derivative of that compound or tautomer, or a pharmaceutically acceptable salt of the foregoing.
52. A method of treating alpha-1 antitrypsin (AAT) deficiency comprising administering to a patient in need thereof a therapeutically effective amount of at least one compound, tautomer, deuterated derivative, or pharmaceutically acceptable salt according to any one of clauses 1 to 50, or a therapeutically effective amount of a pharmaceutical composition according to clause 51.
53. A method of modulating alpha-1 antitrypsin (AAT) activity comprising the step of contacting said AAT with a therapeutically effective amount of at least one compound, tautomer, deuterated derivative, or pharmaceutically acceptable salt according to any one of clauses 1 to 50, or a therapeutically effective amount of a pharmaceutical composition according to clause 51.
54. The method of clause 52 or clause 53, wherein said therapeutically effective amount of the at least one compound, tautomer, deuterated derivative, or pharmaceutically acceptable salt is administered in combination with AAT augmentation therapy and/or AAT replacement therapy.

### II. Compounds and Compositions

In some embodiments, a compound of the disclosure is a compound of Formula (I): 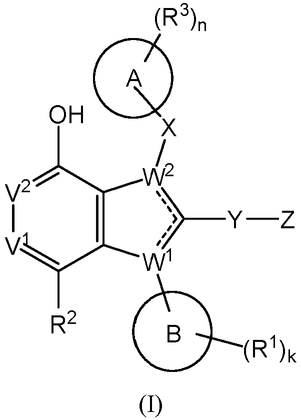 a tautomer thereof, a deuterated derivative of that compound or tautomer, or a pharmaceutically acceptable salt of any of the foregoing, wherein:
-̅ -̅ -̅ -̅ -̅ -̅, for each of the two occurrences, is a single bond or a double bond, provided that one is a single bond and the other is a double bond;
**V¹** and **V²** are each independently N or -C**R²**;
**W¹** and **W²** are each independently N or C, provided that one of W¹ and W² is N and the other is C;
**X** is absent or a bond, -(C**R^{a}R^{b}**)**ₚ**-, or -SO₂-;
**Y** is absent or a bond, -(C**R^{c}R^{d})_{q}-**, -C(=O)-, or -SO₂-;
**R^{a}** and **R^{b}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**R^{c}** and **R^{d}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**Ring** A is C₃-C₁₂ carbocyclyl, 3 to 12-membered heterocyclyl, C₆ or C₁₀ aryl, or 5 to 10-membered heteroaryl; provided that when **W¹** is N and **W²** is C, **Ring A** is not 1,5,6,7-tetrahydro-4*H*-indol-4-onyl or a tautomer thereof;
**Ring B is** C₄-C₁₂ cycloalkyl, C₆ or C₁₀ aryl, 5 to 10-membered heteroaryl, or benzyl;
**Z** is 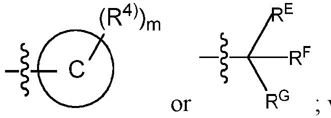 wherein:
   **Ring C** is C₃-C₁₂ cycloalkyl, 3 to 12-membered heterocyclyl, C₆ or C₁₀ aryl, or 5 to 10-membered heteroaryl;
   provided that when **Ring C** is phenyl, the phenyl is substituted with **R⁴**; provided that when **Ring C** is phenyl, **Y** cannot be -SO₂-; and
   provided that when **Ring B** is benzyl, **Ring C** cannot be pyridinyl or indolyl;
   **R^{E}**, **R^{F}**, and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q},** -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s},** -OC(=O)**R^{s}**, or -OC(=O)N**R^{p}R^{q}**; wherein:
      the C₁-C₆ alkyl or the C₂-C₆ alkenyl of any one of **R^{E}**, **R^{F}**, and **R^{G}** is optionally substituted with 1 to 3 groups selected from cyano, -C(=O)**R^{s}**, -C(=O)O**R^{s}**, -C(=O)N**R^{p}R^{q},** -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)**ᵣR^{s}**, and -S(=O)**ᵣ**N**R^{p}R^{q}**; wherein:
      **R^{p}**, **R^{q}**, and **R^{r}**, for each occurrence, are each independently hydrogen, C₁-C₄ alkyl, C₃-C₆ cycloalkyl, or 3 to 6-membered heterocyclyl; wherein:
         the C₁-C₄ alkyl of any one of **R^{p}**, **R^{q}**, and **R^{r}** is optionally substituted with 1 to 3 groups selected from halogen, cyano, -OH, C₁-C₃ alkoxy, - C(=O)NH(C₁-C₂ alkyl), and -C(=O)N(C₁-C₂ alkyl)₂; and
         the C₃-C₆ cycloalkyl or the 3 to 6-membered heterocyclyl of any one of **R^{p}**, **R^{q}**, and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
      **R^{s}**, for each occurrence, is independently hydrogen, C₁-C₄ alkyl, C₃-C₆ cycloalkyl, phenyl, or 5 or 6-membered heteroaryl; wherein:
         the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
         the C₃-C₆ cycloalkyl, the phenyl, or the 5 or 6-membered of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl) such as -O-(cyclopropyl) or -O-(cyclobutyl);
   **R²**, for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, phenyl, or 5 or 6-membered heteroaryl; wherein:
      the C₁-C₆ alkyl, the C₂-C₆ alkenyl or the C₃-C₆ cycloalkyl of **R²** is optionally substituted with 1 to 3 groups selected from cyano, -C(=O)**R^{k}**, -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k}**, -OC(=O)**R^{k}**, -OC(=O)O**R^{k}**, -OC(=O)N**R^{h}Rⁱ**, -S(=O)ₛ**R^{k}**, and S(=O)ₛN**R^{h}Rⁱ**; wherein:
      **R^{h}**, **Rⁱ**, and **R^{j}**, for each occurrence, are each independently hydrogen, C₁-C₄ alkyl, or C₃-C₆ cycloalkyl; wherein:
         the C₁-C₄ alkyl of any one of **R^{h}**, **Rⁱ**, and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl of any one of **R^{h}**, **Rⁱ**, and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
      **R^{k}**, for each occurrence, is independently hydrogen, C₁-C₄ alkyl, C₃-C₆ cycloalkyl, phenyl, or 5 or 6-membered heteroaryl; wherein:
         -O**R^{k}** cannot be -OH;
         the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
         the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁- C₂ alkyl)₂;
   **R³** and **R⁴**, for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)**ₜR^{y}**, -N**R^{v}**R**^{w}**, -N**R^{v}**C(=O)**R^{y}**, -N**R^{v}**C(=O)O**R^{y}**, -N**R^{v}**C(=O)NR**^{w}R^{x}**, -N**R^{v}**S(=O)ₜ**R^{y}**, -O**R^{y}**, -OC(=O)**R^{y}**, -OC(=O)O**R^{y}**, -OC(=O)N**R^{v}R^{w}**, -S(=O)ₜ**R^{y}**, -S(=O)**ₜ**N**R^{v}R^{w}**, -S(=O)**ₜ**N**R^{v}**C(=O)**R^{y}**, -P(=O)**R^{z}R^{z}**, phenyl, or a 5 or 6-membered heteroaryl;
   wherein:
   the C₁-C₆ alkyl, the C₂-C₆ alkenyl, or the C₃-C₆ cycloalkyl of any one of R³ and R⁴ is optionally substituted with 1 to 3 groups selected from cyano, - C(=O)**R^{y}**, -C(=O)O**R^{y}**, -C(=O)N**R^{v}R^{w}**, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R^{v}**S(=O)ᵣ**R^{y}**, -O**R^{y}**, -OC(=O)**R^{y}**, - OC(=O)O**R^{y}**, -OC(=O)N**R^{v}R^{w}**, -S(=O)**ₜR^{y}**, and -S(=O)**ₜ**N**R^{v}R^{w}**; wherein:
   **R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
      the C₁-C₄ alkyl of any one of **R^{v}**, **R^{w}**, and **R^{x}** is optionally substituted with 1 to 3 groups selected from halogen, cyano -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl, the 5 or 6-membered heterocyclyl, or the 5 or 6-membered heteroaryl of any one of **R^{v}**, **R^{w},** and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{y}**, for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
      the C₁-C₄ alkyl of **R^{y}** is optionally substituted with 1 to 3 groups selected from halogen, cyano, -OH, -OCH₃, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
      the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, -OCH₃, -NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{z}**, for each occurrence, is independently C₁-C₂ alkyl, -OH, or -O(C₁-C₂ alkyl);

   **k** is an integer selected from 1, 2, and 3;
   **m** and **n** are each independently an integer selected from 0, 1, 2, and 3;
   **p, r, s,** and **t** are each independently an integer selected from 1 and 2; and
   **q** is an integer selected from 1, 2, and 3.

In some embodiments, in the compound, tautomer, deuterated derivative, or pharmaceutically acceptable salt of the disclosure, **p, q, r, s,** and **t** are each independently an integer selected from 1 and 2, and all other variables are as defined for Formula (I).

In some embodiments, in the compound, tautomer, deuterated derivative, or pharmaceutically acceptable salt of the disclosure, **V²** is -C**R²**, wherein **R²** is halogen, wherein all other variables not specifically defined herein are as defined in any one of the preceding embodiments.

In some embodiments, in the compound, tautomer, deuterated derivative, or pharmaceutically acceptable salt of the disclosure, **V²** is -C**R²**, wherein **R²** is fluoro, wherein all other variables not specifically defined herein are as defined in any one of the preceding embodiments.

In some embodiments, in the compound, tautomer, deuterated derivative, or pharmaceutically acceptable salt of the disclosure, **V¹** is -C**R²**, wherein **R²** is halogen, wherein all other variables not specifically defined herein are as defined in any one of the preceding embodiments.

In some embodiments, in the compound, tautomer, deuterated derivative, or pharmaceutically acceptable salt of the disclosure, **V¹** is -C**R²**, wherein **R²** is fluoro, wherein all other variables not specifically defined herein are as defined in any one of the preceding embodiments.

In some embodiments, the compound, tautomer, deuterated derivative, or pharmaceutically acceptable salt of the disclosure is represented by Formula (IIa) or Formula (IIb): 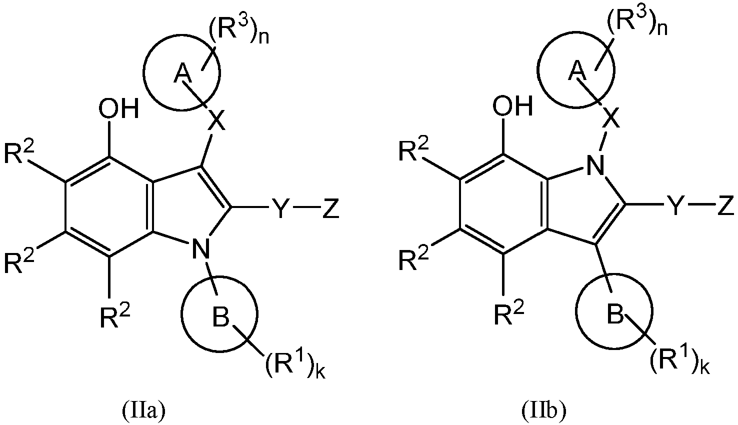 wherein all other variables not specifically defined herein are as defined in Formula (I).

In some embodiments, in the compound, tautomer, deuterated derivative, or pharmaceutically acceptable salt of the disclosure, **p, q, r, s,** and **t** are each independently an integer selected from 1 and 2, wherein all other variables not specifically defined herein are as defined in the preceding embodiment.

In some embodiments, the compound, tautomer, deuterated derivative, or pharmaceutically acceptable salt of the disclosure is represented by Formula (IIc), Formula (IId), Formula (IIe), or Formula (IIf): 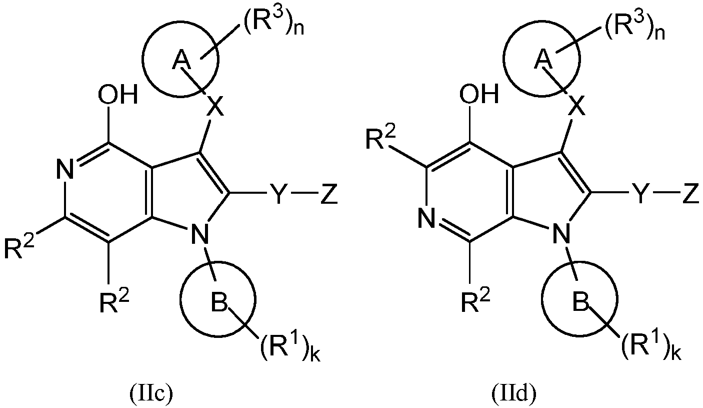 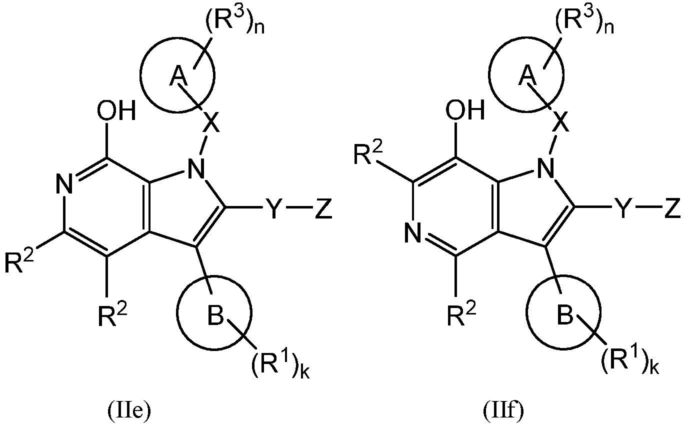 wherein all other variables not specifically defined herein are as defined in any one of the preceding embodiments.

In some embodiments, in the compound, tautomer, deuterated derivative, or pharmaceutically acceptable salt of the disclosure, **p, q, r, s,** and **t** are each independently an integer selected from 1 and 2, wherein all other variables not specifically defined herein are as defined in the preceding embodiments.

In some embodiments, the compound, tautomer, deuterated derivative, or pharmaceutically acceptable salt of the disclosure is represented by Formula (IIIa) or Formula (IIIb): 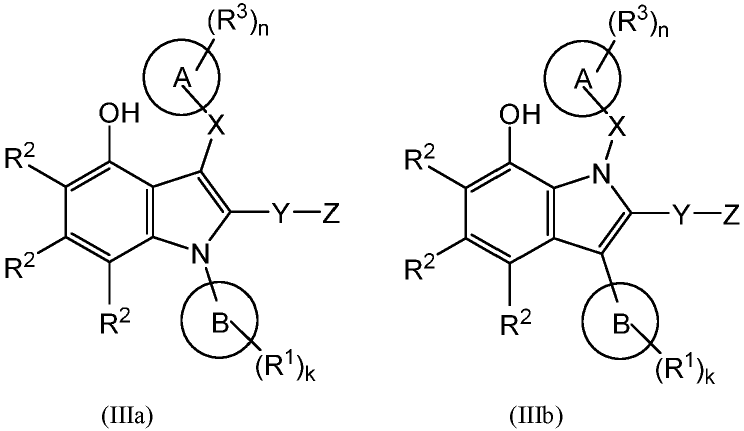 wherein:
**Ring A** is optionally substituted with **R³** and **Ring A** is C₃-C₇ carbocyclyl, 6 to 9-membered heterocyclyl, phenyl, or 5 to 9-membered heteroaryl;
**Ring B** is substituted with **R¹** and **Ring B** is C₄-C₆ cycloalkyl, phenyl, 5 to 6-membered heteroaryl, or benzyl; and
when **Z** is **Ring C** optionally substituted with **R⁴**, **Ring C** is C₄-C₈ cycloalkyl, 4 to 8-membered heterocyclyl, phenyl, or 5 or 6-membered heteroaryl; and
wherein all other variables not specifically defined herein are as defined in any one of the preceding embodiments.

For example, in some embodiments, in the compound, tautomer, deuterated derivative, or pharmaceutically acceptable salt of the disclosure, **p, q, r, s,** and **t** are each independently an integer selected from 1 and 2, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is C₃ carbocyclyl, C₄ carbocyclyl, or C₇ carbocyclyl, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is phenyl, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is pyridine, pyrimidine, pyrazole, thiophene, or oxadiazole, wherein all other variables not specifically defined herein are as defined in any one of the preceding embodiments.

In some embodiments, in the compound, tautomer, deuterated derivative, or pharmaceutically acceptable salt of the disclosure, **Ring B** is substituted with **R¹** and **Ring B** is phenyl, wherein all other variables not specifically defined herein are as defined in any one of the preceding embodiments.

In some embodiments, the compound, tautomer, deuterated derivative, or pharmaceutically acceptable salt of the disclosure is represented by Formula (IVa) or Formula (IVb): 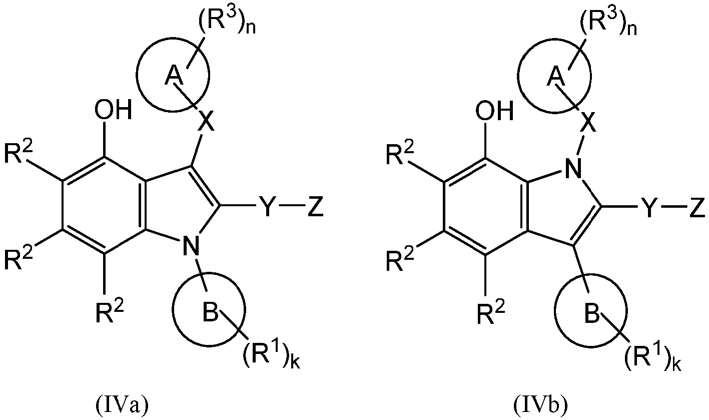 wherein:
**Ring B** is substituted with **R¹** and **Ring B** is cyclohexyl, phenyl, pyridinyl, or benzyl; or
**Ring B** is substituted with **R¹** and **Ring B** is cyclohexyl, phenyl, or benzyl; and wherein all other variables not specifically defined herein are as defined in any one of the preceding embodiments.

For example, in some embodiments, in the compound, tautomer, deuterated derivative, or pharmaceutically acceptable salt of the disclosure, **p, q, r, s,** and **t** are each independently an integer selected from 1 and 2, wherein all other variables not specifically defined herein are as defined in any one of the preceding embodiments.

In some embodiments, the compound, tautomer, deuterated derivative, or pharmaceutically acceptable salt of the disclosure is represented by Formula (Va) or Formula (Vb): 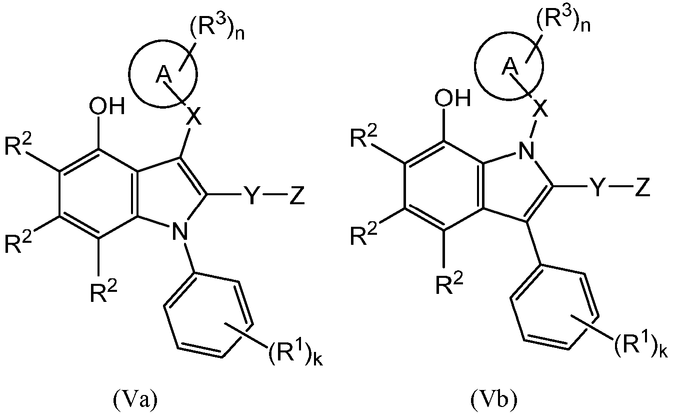 wherein:
**R¹** is halogen, cyano, C₁-C₂ alkyl, C₁-C₂ haloalkyl, or C₁-C₂ alkoxy; and
**k** is an integer selected from 1 and 2;
and wherein all other variables not specifically defined herein are as defined in any one of the preceding embodiments.

In some embodiments, in the compound, tautomer, deuterated derivative, or pharmaceutically acceptable salt of the disclosure, **p, q, r, s,** and **t** are each independently an integer selected from 1 and 2, wherein all other variables not specifically defined herein are as defined in any one of the preceding embodiments.

In some embodiments, in the compound, tautomer, deuterated derivative, or pharmaceutically acceptable salt of the disclosure, **R¹** is cyano, F, Cl, -CH₃, -CHF₂, -CF₃, - OCH₃, or -OCH(CH₃)₂; and all other variables not specifically defined herein are as defined in any one of the preceding embodiments.

In some embodiments, in the compound, tautomer, deuterated derivative, or pharmaceutically acceptable salt of the disclosure, at least one **R¹** is F; and all other variables not specifically defined herein are as defined in any one of the preceding embodiments.

In some embodiments, in the compound, deuterated derivative, pharmaceutically acceptable salt, or tautomer of the disclosure, **X** is absent or a bond, -(C**R^{a}R^{b}**)-, or -SO₂-; **R^{a}** and **R^{b}**, for each occurrence, are each independently hydrogen, C₁-C₃ alkyl, C₁-C₃ haloalkyl, or C₁-C₃ alkoxy; and all other variables not specifically defined herein are as defined in any one of the preceding embodiments.

In some embodiments, in the compound, tautomer, deuterated derivative, or pharmaceutically acceptable salt of the disclosure, **X** is absent or a bond, -CH₂-, or -SO₂-; and all other variables not specifically defined herein are as defined in any one of the preceding embodiments.

In some embodiments, in the compound, tautomer, deuterated derivative, or pharmaceutically acceptable salt of the disclosure, **Y** is absent or a bond, -(C**R^{c}R^{d}**)_{q}-, -C(=O)-, or -SO₂-; **R^{c}** and **R^{d}**, for each occurrence, are each independently hydrogen, C₁-C₃ alkyl, C₁-C₃ haloalkyl, or C₁-C₃ alkoxy; and all other variables not specifically defined herein are as defined in any one of the preceding embodiments.

In some embodiments, in the compound, tautomer, deuterated derivative, or pharmaceutically acceptable salt of the disclosure, **Y** is absent or a bond, -CH₂-, -CHCH₃-, -C(CH₃)₂-, -C(=O)-, or -SO₂-; and all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is C₃-C₇ carbocyclyl, 6 to 9-membered heterocyclyl containing 1 to 3 oxygen atoms, phenyl, or 5 to 9-membered heteroaryl containing 1 to 3 heteroatoms selected from O and N; and all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is C₃-C₇ carbocyclyl, 6 to 9-membered heterocyclyl containing one or two oxygen atoms, phenyl, or 5 to 9-membered heteroaryl containing one or two nitrogen atoms or one or two oxygen atoms; and all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is selected from 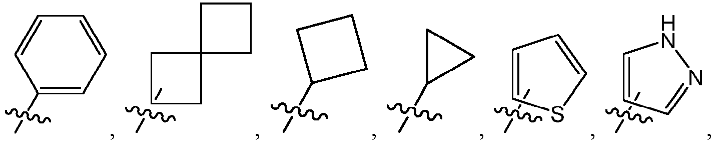 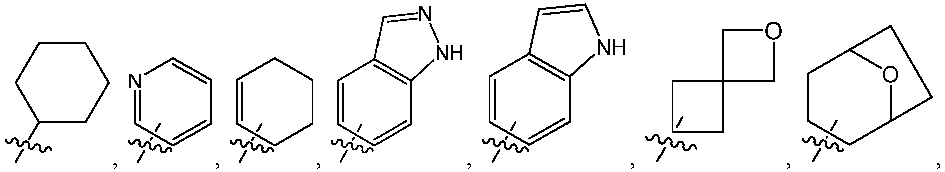 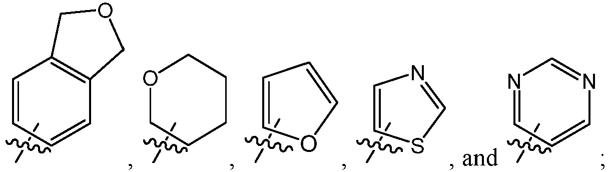 and all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is selected from 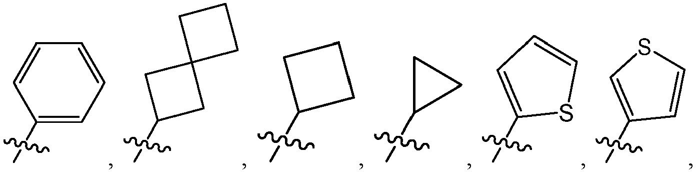 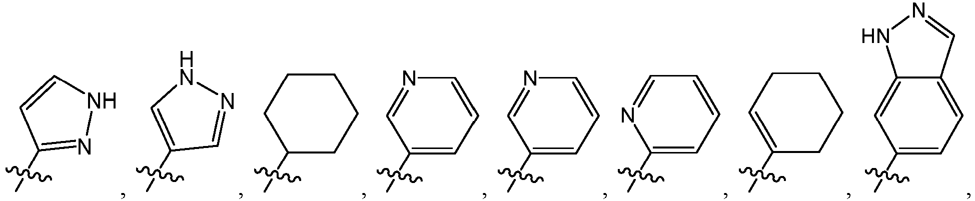 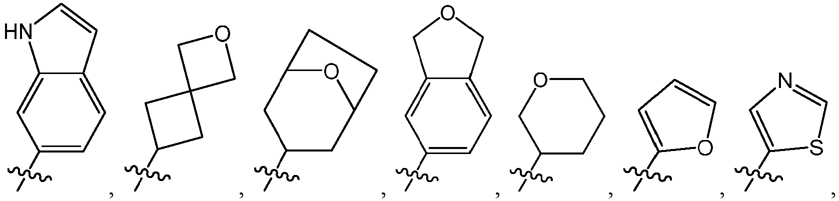 and 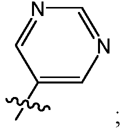 and all other variables not specifically defined herein are as defined in any one of the preceding embodiments.

In some embodiments, in the compound, tautomer, deuterated derivative, or pharmaceutically acceptable salt of the disclosure, wherein **Z** is **Ring C, Ring C** is optionally substituted with **R⁴**, and **Ring C** is selected from 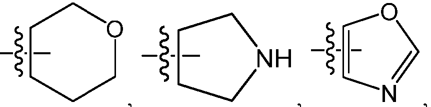 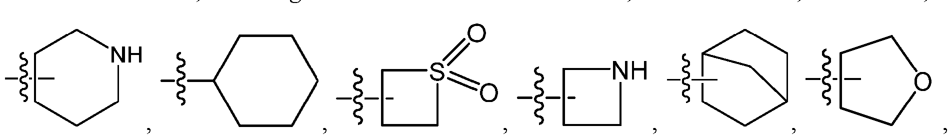 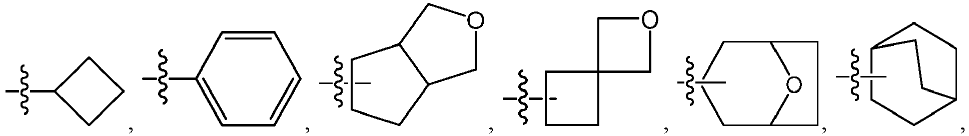 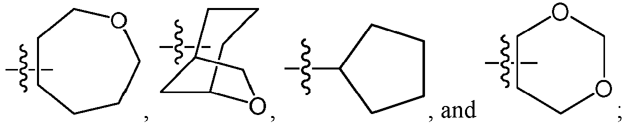 and all other variables not specifically defined herein are as defined in any one of the preceding embodiments.

In some embodiments, in the compound, tautomer, deuterated derivative, or pharmaceutically acceptable salt of the disclosure, **Z** is **Ring C, Ring C** is optionally substituted with **R⁴**, and **Ring C** is selected from 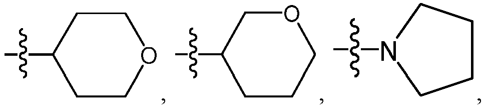 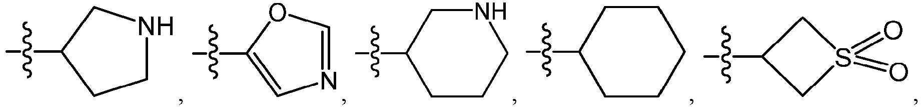 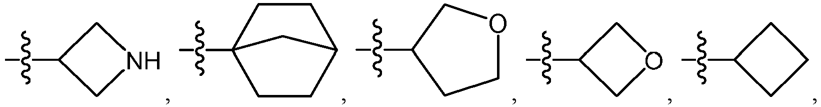 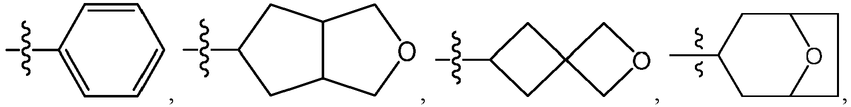 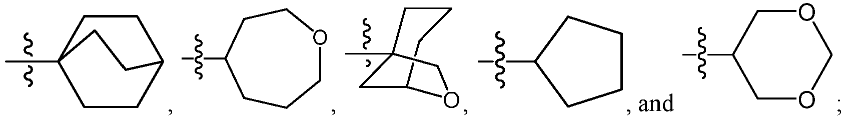 and all other variables not specifically defined herein are as defined in any one of the preceding embodiments.

In some embodiments, in the compound, tautomer, deuterated derivative, or pharmaceutically acceptable salt of the disclosure, **R^{E}, R^{F},** and **R^{G}** are each independently hydrogen, halogen, cyano , C₁-C₄ alkyl, C₁-C₄ alkoxy, C₁-C₄ haloalkyl, -C(=O)O**R^{s}**, - C(=O)N**R^{p}R^{q}**, -C**R^{p}**(=N)O**R^{s}**, or -O**R^{s}**; wherein:
the C₁-C₄ alkyl of any one of **R^{E}**, **R^{F}**, and **R^{G}** is optionally substituted with 1 to 3 groups selected from cyano, -C(=O)**R^{s}**, -C(=O)O**R^{s}**, -C(=O)N**R^{p}R^{q},** -O**R^{s}**, -OC(=O)**R^{s}**, -OC(=O)O**R^{s}**, -OC(=O)N**R^{p}R^{q}**, and -S(=O)₂**R^{s}**; wherein:
**R^{p}** and **R^{q}**, for each occurrence, are each independently hydrogen, C₁-C₂ alkyl, C₃-C₅ cycloalkyl, or 5 or 6-membered heterocyclyl; wherein the C₁-C₂ alkyl of any one of **R^{p}** and **R^{q}** is optionally substituted with 1 to 3 groups selected from halogen, cyano, and -OH; wherein:
   the C₃-C₅ cycloalkyl or the 5 or 6-membered heteroaryl of any one of **R^{p}** and **R^{q}** is optionally substituted with 1 to 3 groups selected from halogen, cyano, and -OH;
**R^{s}**, for each occurrence, is independently hydrogen, C₁-C₂ alkyl, or 5 or 6-membered heteroaryl; wherein:
   the C₁-C₂ alkyl of **R^{s}** is optionally substituted with 1 to 3 groups selected from halogen, cyano, -OH, and -NH₂;
   the 5 or 6-membered heteroaryl of **R^{s}** is optionally substituted with 1 to 3 groups selected from halogen, cyano, -OH, and -NH₂;
and all other variables not specifically defined herein are as defined in any one of the preceding embodiments.

In some embodiments, in the compound, tautomer, deuterated derivative, or pharmaceutically acceptable salt of the disclosure, **R^{E}**, **R^{F}**, and **R^{G}** are each independently hydrogen, F, Cl, C₁-C₂ alkyl, C₁-C₂ haloalkyl, -C(=O)N**R^{p}R^{q},** -C**R^{p}**(=N)O**R^{s}**, or -O**R^{s}**; wherein:
the C₁-C₂ alkyl of any one of **R^{E}**, **R^{F}**, and **R^{G}** is optionally substituted with 1 to 3 groups selected from cyano, -C(=O)N**R^{p}R^{q},** -O**R^{s}**, -OC(=O)N**R^{p}R^{q}**, and -S(=O)₂**R^{s}**; wherein:
**R^{p}** and **R^{q}**, for each occurrence, are each independently hydrogen, C₁-C₂ alkyl, cyclopentyl, or tetrahydrofuranyl; wherein:
   the C₁-C₂ alkyl of any one of **R^{p}** and **R^{q}** is optionally substituted with 1 to 3 halogen groups selected from F and Cl;
**R^{s}**, for each occurrence, is independently hydrogen, C₁-C₂ alkyl, pyridinyl, or pyrimidinyl; wherein:
   the C₁-C₂ alkyl of **R^{s}** is optionally substituted with 1 to 3 halogen groups selected from F and Cl;
and all other variables not specifically defined herein are as defined in any one of the preceding embodiments.

In some embodiments, in the compound, tautomer, deuterated derivative, or pharmaceutically acceptable salt of the disclosure, **R^{E}**, **R^{F}**, and **R^{G}** are each independently hydrogen, F, -OH, -CH(OH)CH₃, -C₂H₅, -C(=O)NHCH₃, -C(=N)OCH₃, -CH₃, -CH₂F, - CH₂CN, -(CH₂)₂CN, -CH₂OH, -(CH₂)₂OH, -CH₂OCH₃, -CH₂OC₂H₅, -(CH₂)₂OCH₃, - CH₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅, -CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), -CH₂(O)(pyrimidin-2-yl; and all other variables not specifically defined herein are as defined in any one of the preceding embodiments.

In some embodiments, in the compound, tautomer, deuterated derivative, or pharmaceutically acceptable salt of the disclosure, **R^{E}**, **R^{F}**, and **R^{G}** are each independently hydrogen, F, -CH(OH)CH₃, -CH₃, -CH₂CN, -CH₂OH, and -CH₂OCH₃; and all other variables not specifically defined herein are as defined in any one of the preceding embodiments.

In some embodiments, the compound, tautomer, deuterated derivative, or pharmaceutically acceptable salt of the disclosure is represented by Formula (VIa), Formula (VIb), Formula (VIc), Formula (VId), or Formula (VIe): 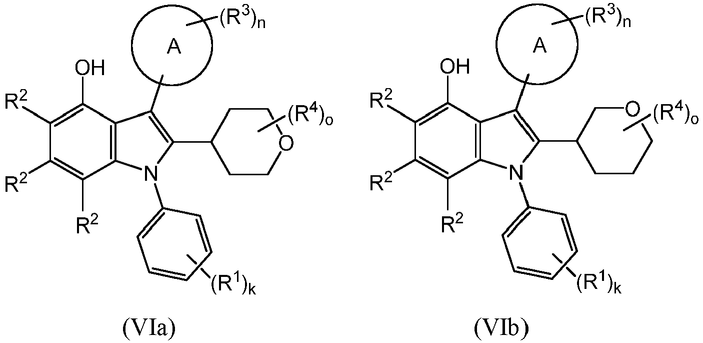 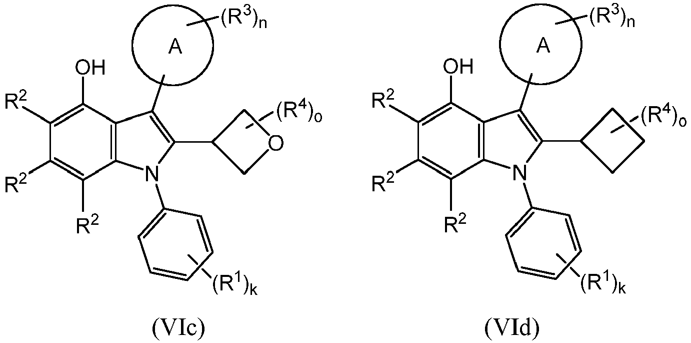 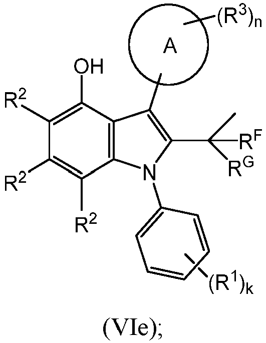 wherein all other variables not specifically defined herein are as defined in any one of the preceding embodiments. In some embodiments, o is an integer selected from 0, 1, and 2.

In some embodiments, in the compound, tautomer, deuterated derivative, or pharmaceutically acceptable salt of the disclosure, **R^{F}** and **R^{G}** are each independently hydrogen, F, -OH, -CH(OH)CH₃, -C₂H₅, -C(=O)NHCH₃, -C(=N)OCH₃, -CH₃, -CH₂F, - CH₂CN, -(CH₂)₂CN, -CH₂OH, -(CH₂)₂OH, -CH₂OCH₃, -CH₂OC₂H₅, -(CH₂)₂OCH₃, - CH₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅, -CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), -CH₂(O)(pyrimidin-2-yl; and all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, F, -CH(OH)CH₃, -CH₃, -CH₂CN, -CH₂OH, and -CH₂OCH₃; and all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, -OH, -CH₃, -CH₂CN, -CH₂OH, and -CH₂OCH₃; and all other variables not specifically defined herein are as defined in any one of the preceding embodiments.

In some embodiments, the compound, tautomer, deuterated derivative, or pharmaceutically acceptable salt of the disclosure is represented by Formula (VIIa), Formula (VIIb), Formula (VIIc), Formula (VIId), or Formula (VIIe): 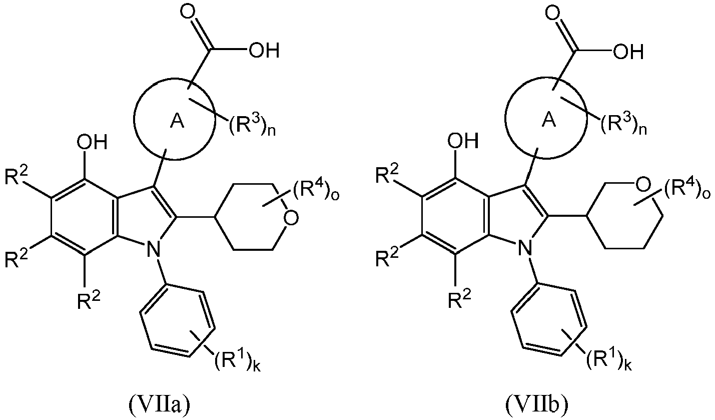 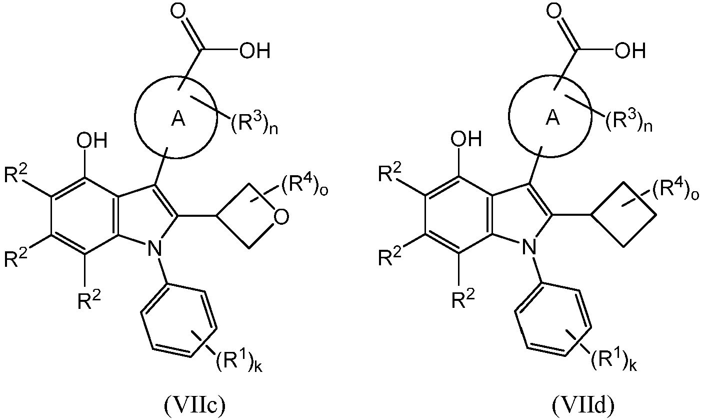 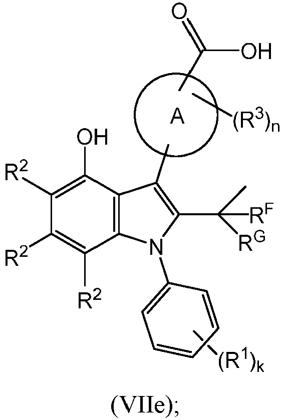 wherein n is an integer selected from 0, 1, and 2; and wherein all other variables not specifically defined herein are as defined in any one of the preceding embodiments. In some embodiments, **o** is an integer selected from 0, 1, and 2.

In some embodiments, in the compound, tautomer, deuterated derivative, or pharmaceutically acceptable salt of the disclosure, **Ring A** is optionally substituted with **R³** and **Ring A** is C₃-C₇ carbocyclyl, 6 to 9-membered heterocyclyl, phenyl, or 5 to 9-membered heteroaryl, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is C₃ carbocyclyl, C₄ carbocyclyl, or C₇ carbocyclyl, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is phenyl, wherein all other variables not specifically defined herein are as defined in any one of the preceding embodiments.

In some embodiments, in the compound, tautomer, deuterated derivative, or pharmaceutically acceptable salt of the disclosure, **Ring A** is optionally substituted with **R³** and **Ring A** is pyridine, pyrimidine, pyrazole, thiophene, or oxadiazole, wherein all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, F, -OH, -CH(OH)CH₃, -C₂H₅, -C(=O)NHCH₃, -C(=N)OCH₃, -CH₃, -CH₂F, - CH₂CN, -(CH₂)₂CN, -CH₂OH, -(CH₂)₂OH, -CH₂OCH₃, -CH₂OC₂H₅, -(CH₂)₂OCH₃, - CH₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅, -CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), -CH₂(O)(pyrimidin-2-yl; and all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, F, -CH(OH)CH₃, -CH₃, -CH₂CN, -CH₂OH, and -CH₂OCH₃; and all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, -OH, -CH₃, -CH₂CN, -CH₂OH, and -CH₂OCH₃; and all other variables not specifically defined herein are as defined in any one of the preceding embodiments.

In some embodiments, the compound, tautomer, deuterated derivative, or pharmaceutically acceptable salt of the disclosure is represented by Formula (VIIIa), Formula (VIIIb), Formula (VIIIc), Formula (VIIId), or Formula (VIIIe): 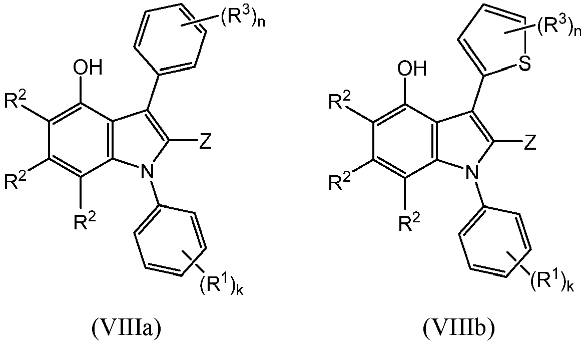 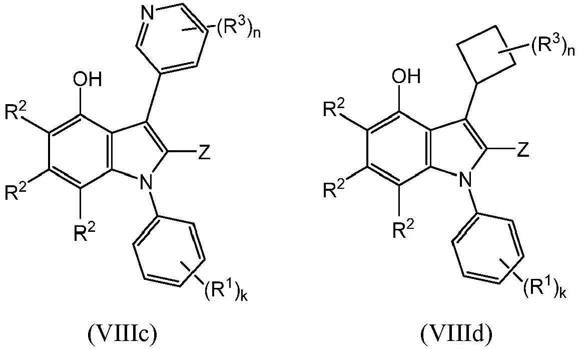 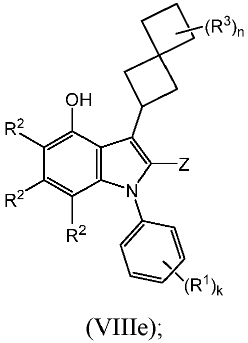 wherein all other variables not specifically defined herein are as defined in any one of the preceding embodiments.

In some embodiments, the compound, tautomer, deuterated derivative, or pharmaceutically acceptable salt of the disclosure is represented by Formula (IXa), Formula (IXb), Formula (IXc), Formula (IXd), or Formula (IXe): 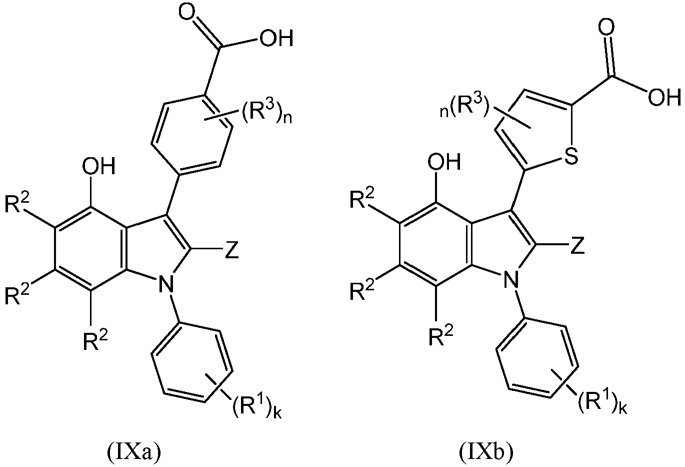 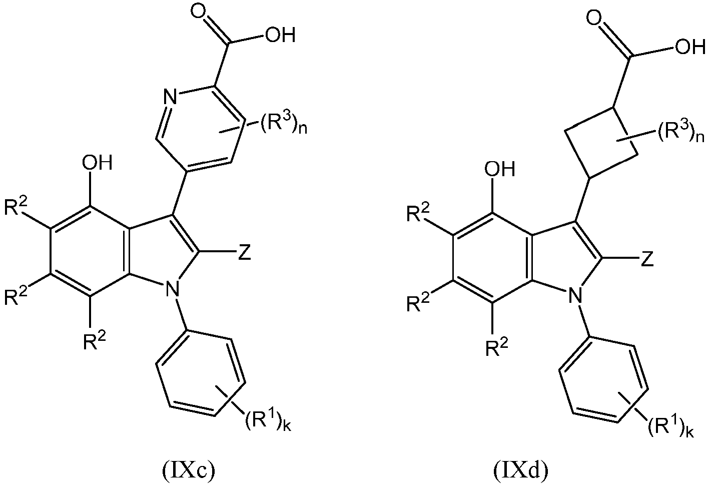 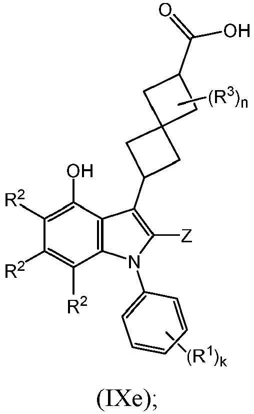 wherein n is an integer selected from 0, 1, and 2; and wherein all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, halogen, cyano, C₁-C₆ alkyl (optionally substituted with 1 to 3 groups selected from cyano, -OH, -OCH₃, and -NH₂), C₁-C₆ alkoxy, C₁-C₆ haloalkyl, -N**R^{h}Rⁱ**, or C₃-C₆ cycloalkyl; wherein **R^{h}** and **Rⁱ**, for each occurrence, are each independently hydrogen or C₁-C₄ alkyl; and all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, halogen, cyano, C₁-C₄ alkyl (optionally substituted with 1 to 3 groups selected from cyano, -OH, -OCH₃, and -NH₂), C₁-C₄ haloalkyl, -N**R^{h}Rⁱ**, or C₃-C₅ cycloalkyl; wherein **R^{h}** and **Rⁱ**, for each occurrence, are each independently hydrogen or C₁-C₂ alkyl; and all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, halogen, cyano, C₁-C₂ alkyl (optionally substituted with 1 to 3 groups selected from cyano, -OH, -OCH₃, and -NH₂), -N**R^{h}Rⁱ**, or C₃-C₄ cycloalkyl; wherein **R^{h}** and **Rⁱ**, for each occurrence, are each independently hydrogen or -CH₃; and all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, F, Cl, cyano, -CH₃, -CF₃, -NH₂, or cyclopropyl; and all other variables not specifically defined herein are as defined in any one of the preceding embodiments.

In some embodiments, in the compound, tautomer, deuterated derivative, or pharmaceutically acceptable salt of the disclosure, **R³**, for each occurrence, is independently halogen, cyano, =O, C₁-C₆ alkyl, C₁-C₆ alkoxy, C₁-C₆ haloalkyl,
-C(=O)**R^{y}**, -C(=O)O**R^{y}**, -C(=O)N**R^{v}R^{w}**, -C(=O)N**R^{v}**O**R^{y}**,-C(=O)NR^{v}S(=O)₂**R^{y}**, -N**R^{v}**R**^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
the C₁-C₄ alkyl of **R³** is optionally substituted with 1 to 3 groups selected from cyano, -O**R^{y}**, -C(=O)O**R^{y}**, and -N**R^{v}**R**^{w};** wherein:
**R^{v}**, **R^{w},** and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with 1 to 3 groups selected from halogen, cyano, -OH, and -NH₂; and
**R^{y}**, for each occurrence, is independently hydrogen, C₁-C₄ alkyl, or 5 or 6-membered heterocyclyl; wherein:
   the C₁-C₄ alkyl of **R^{y}** is optionally substituted with 1 to 3 groups selected from halogen, cyano, -OH, -NH₂, and -C(=O)OH; and
   the 5 or 6-membered heterocyclyl of **R^{y}** is optionally substituted with 1 to 3 groups selected from halogen, cyano, -OH,
      -NH₂, and -C(=O)OH;
and all other variables not specifically defined herein are as defined in any one of the preceding embodiments.

In some embodiments, in the compound, tautomer, deuterated derivative, or pharmaceutically acceptable salt of the disclosure, **R³**, for each occurrence, is independently halogen, cyano, =O, C₁-C₄ alkyl, C₁-C₄ alkoxy, C₁-C₄ haloalkyl,
-C(=O)**R^{y},** -C(=O)O**R^{y}**, -C(=O)N**R^{v}R^{w}**,-C(=O)N**R^{v}**O**R^{y}**, -C(=O)N**R^{v}**S(=O)₂**R^{y}**, -N**R^{v}**R**^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
the C₁-C₄ alkyl of **R³** is optionally substituted with 1 to 3 groups selected from cyano, -O**R^{y}** and -C(=O)O**R^{y}**; wherein:
   **R^{v}**, **R^{w}**, and **R^{x}**, for each occurrence, are each independently hydrogen or C₁-C₄ alkyl; wherein the C₁-C₄ alkyl of any one of **R^{v}**, **R^{w},** and **R^{x}** is optionally substituted with -OH; and
   **R^{y}**, for each occurrence, is independently hydrogen, C₁-C₂ alkyl, or 6-membered heterocyclyl; wherein:
      the C₁-C₂ alkyl of **R^{y}** is optionally substituted with 1 to 3 groups selected from -OH and -C(=O)OH; and
      the 6-membered heterocyclyl of **R^{y}** is optionally substituted with 1 to 3 groups selected from -OH and -C(=O)OH;
**R^{z}**, for each occurrence, is independently -CH₃, -OH, or -OCH₃; and all other variables not specifically defined herein are as defined in any one of the preceding embodiments.

In some embodiments, in the compound, tautomer, deuterated derivative, or pharmaceutically acceptable salt of the disclosure, **R³**, for each occurrence, is independently halogen, cyano, =O, C₁-C₂ alkyl, C₁-C₂ alkoxy, C₁-C₄ haloalkyl,
-C(=O)**R^{y},** -C(=O)O**R^{y}**, -C(=O)N**R^{v}R^{w}**,-C(=O)N**R^{v}**O**R^{y}**, -C(=O)N**R^{v}**S(=O)₂**R^{y}**, -N**R^{v}**R**^{w}**, - O**R^{y}**, -S(=O)₂**R^{y}**, -S(=O)₂N**R^{v}R^{w}**, -S(=O)₂N**R^{v}**C(=O)**R^{y}**, or -P(=O)**R^{z}R^{z}**; wherein:
the C₁-C₂ alkyl of **R³** is optionally substituted with 1 to 3 groups selected from -OH and -C(=O)O**R^{y}**; wherein:
   **R^{v}**, **R^{w}**, and **R^{x}**, for each occurrence, are each independently hydrogen or C₁-C₂ alkyl; wherein the C₁-C₂ alkyl of any one of **R^{v}**, **R^{w}**, and **R^{x}** is optionally substituted with -OH; and
   **R^{y}**, for each occurrence, is independently hydrogen, C₁-C₂ alkyl, or tetrahydro-2H-pyranyl; wherein:
      the C₁-C₂ alkyl of **R^{y}** is optionally substituted with -C(=O)OH; and the tetrahydro-2H-pyranyl of **R^{y}** is optionally substituted with 1 to 3 groups selected from -OH and -C(=O)OH;
**R^{z}**, for each occurrence, is independently -CH₃ or -OH;
and all other variables not specifically defined herein are as defined in any one of the preceding embodiments.

In some embodiments, in the compound, tautomer, deuterated derivative, or pharmaceutically acceptable salt of the disclosure, **R³**, for each occurrence, is independently F, Cl, cyano, -OH, =O, -CH₃, -OCH₃, -CF₃, -CH₃CN, -C(CH₃)₂CH₂OH, -CH₂COOH, -CH₂OCH₃, -C(=O)CHCH₃OH, -COOH, -C(=O)O(2-tetrahydro-2H-pyranyl), - C(=O)NH₂, -C(=O)NH(CH₂)₂OH, -C(=O)NHOH, -C(=O)NHS(=O)₂CH₃, -NH₂, -NHCH₃, NHS(=O)₂CH₃, -OCH₂COOH, -S(=O)₂CH₃, -S(=O)₂NH₂, - S(=O)₂NHC(=O)CH₃, or-P(=O)(CH₃)₂; wherein the 2-tetrahydro-2H-pyranyl in -C(=0)0(2-tetrahydro-2H-pyranyl) is substituted with 1 to 3 groups selected from -OH and - C(=O)OH; and all other variables not specifically defined herein are as defined in any one of the preceding embodiments.

In some embodiments, in the compound, tautomer, deuterated derivative, or pharmaceutically acceptable salt of the disclosure, **R⁴**, for each occurrence, is independently halogen, cyano, C₁-C₆ alkyl, C₁-C₆ alkoxy, C₁-C₆ haloalkyl, -C(=O)**R^{y},**
-C(=O)O**R^{y}**, -O**R^{y}**, or -S(=O)₂**R^{y}**; wherein:
the C₁-C₆ alkyl of **R⁴** is optionally substituted with 1 to 3 groups selected from cyano, -O**R^{y}**, -C(=O)O**R^{y}**, and -N**R^{v}**R**^{w};** wherein:
**R^{y}** and **R^{w},** for each occurrence, are each independently hydrogen or C₁-C₄ alkyl; and
**R^{y}**, for each occurrence, is independently hydrogen and C₁-C₄ alkyl;
wherein:
the C₁-C₄ alkyl of **R^{y}** is optionally substituted with 1 to 3 groups selected from halogen, cyano, -OH, -OCH₃, and -NH₂;
and all other variables not specifically defined herein are as defined in any one of the preceding embodiments.

In some embodiments, in the compound, tautomer, deuterated derivative, or pharmaceutically acceptable salt of the disclosure **R⁴**, for each occurrence, is independently halogen, cyano, C₁-C₄ alkyl, C₁-C₄ alkoxy, C₁-C₄ haloalkyl, -C(=O)**R^{y},**
-C(=O)O**R^{y}**, -O**R^{y}**, or -S(=O)₂**R^{y}**; wherein:
the C₁-C₄ alkyl of **R⁴** is optionally substituted with 1 to 3 groups selected from cyano, -O**R^{y}**, -C(=O)O**R^{y}**, and -N**R^{v}R^{w}**; wherein:
   **R^{v}** and **R^{w},** for each occurrence, are each independently hydrogen or C₁-C₄ alkyl;
   **R^{y}**, for each occurrence, is independently hydrogen or C₁-C₂ alkyl;
wherein:
   the C₁-C₂ alkyl of any one of **R^{y}** is optionally substituted with 1 to 3 groups selected from halogen, cyano, -OH, -OCH₃, and -NH₂; and
wherein **m** is an integer selected from 0, 1, and 2;
and all other variables not specifically defined herein are as defined in any one of the preceding embodiments.

In some embodiments, in the compound, tautomer, deuterated derivative, or pharmaceutically acceptable salt of the disclosure, **R⁴**, for each occurrence, is independently cyano, C₁-C₂ alkyl, C₁-C₂ alkoxy, C₁-C₂ haloalkyl, -C(=O)R^{y}, -C(=O)O**R^{y}**, -O**R^{y}**, or - S(=O)₂**R^{y}**; wherein:
the C₁-C₂ alkyl of **R⁴** is optionally substituted with cyano, -OH, or -OCH₃;
**R^{y}**, for each occurrence, is independently hydrogen or C₁-C₂ alkyl;

wherein the C₁-C₂ alkyl of **R^{y}** is optionally substituted with -OCH₃;
wherein **m** is an integer selected from 0 and 1;
and all other variables not specifically defined herein are as defined in any one of the preceding embodiments.

In some embodiments, in the compound, tautomer, deuterated derivative, or pharmaceutically acceptable salt of the disclosure, **R⁴**, for each occurrence, is independently cyano, -OH, -OCH₃, -CH₃, -C₂H₅, -CH₂CN, -CH₂OH, -CH₂OCH₃,
-COOH, -C(=O)CH₃, -C(=O)OCH₃, -C(=O)CH₂OCH₃, -S(=O)₂CH₃, S(=O)₂C₂H₅, or S(=O)₂CF₃; and all other variables not specifically defined herein are as defined in any one of the preceding embodiments.

In some embodiments, a compound of the disclosure is a compound of Formula (XIa), Formula (XIb), Formula (XIc), Formula (XId), Formula (XIe), or Formula (XIf): 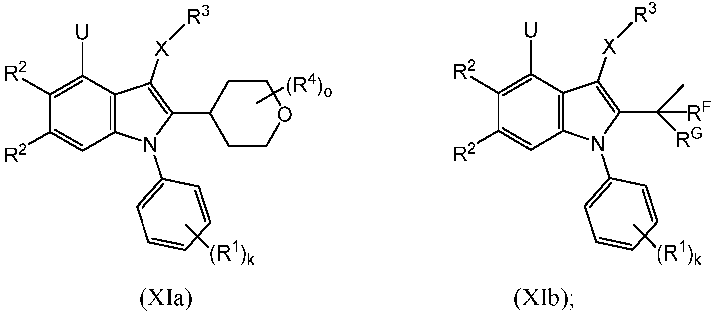 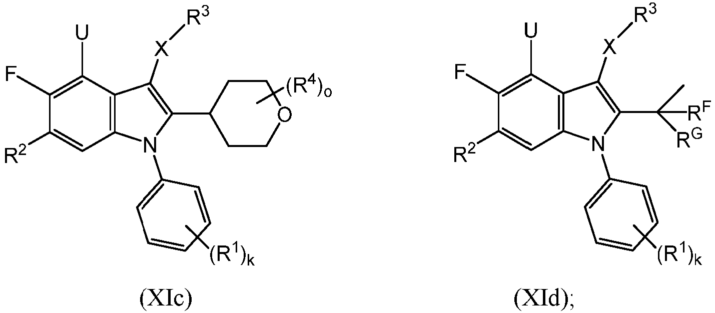 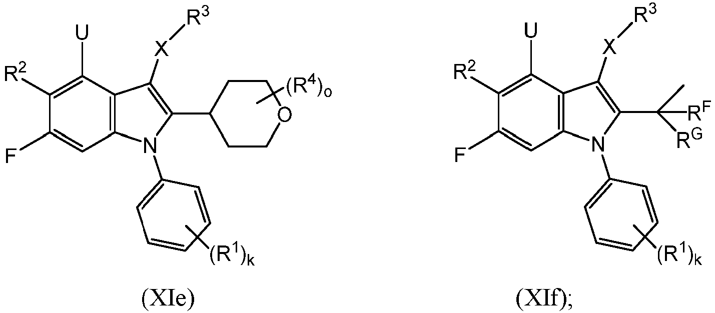 a tautomer thereof, a deuterated derivative of that compound or tautomer, or a pharmaceutically acceptable salt of any of the foregoing, wherein:
**U** is -OH;
**X** is absent or a bond, -(C**R^{a}R^{b}**)**ₚ**-, or -SO₂-;
**R^{a}** and **R^{b}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**R^{E}**, **R^{F}**, and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
   the C₁-C₆ alkyl or the C₂-C₆ alkenyl of any one of **R^{E}**, **R^{F}**, and **R^{G}** is optionally substituted with 1 to 3 groups selected from cyano,
   -C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)**ᵣR^{s}**, and -S(=O)**ᵣ**N**R^{p}R^{q}**; wherein:
   **R^{p}**, **R^{q}**, and **R^{r}**, for each occurrence, are each independently hydrogen, C₁-C₄ alkyl, C₃-C₆ cycloalkyl, or 3 to 6-membered heterocyclyl; wherein:
      the C₁-C₄ alkyl of any one of **R^{p}**, **R^{q}**, and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
      the C₃-C₆ cycloalkyl or the 3 to 6-membered heterocyclyl of any one of **R^{p}**, **R^{q}**, and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
   **R^{s}**, for each occurrence, is independently hydrogen, C₁-C₄ alkyl, C₃-C₆ cycloalkyl, phenyl, or 5 or 6-membered heteroaryl; wherein:
      the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
      the C₃-C₆ cycloalkyl, the phenyl, or the 5 or 6-membered heteroaryl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
**R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl);
**R²**, for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, phenyl, or 5 or 6-membered heteroaryl; wherein:
   the C₁-C₆ alkyl, the C₂-C₆ alkenyl, or the C₃-C₆ cycloalkyl of **R²** is optionally substituted with 1 to 3 groups selected from cyano, -C(=O)**R^{k}**,
   -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k},** -OC(=O)**R^{k}**, -OC(=O)O**R^{k}**, -OC(=O)N**R^{h}Rⁱ**, -S(=O)ₛ**R^{k}**, and S(=O)ₛN**R^{h}Rⁱ**; wherein:
   **R^{h}**, **Rⁱ**, and **R^{j}**, for each occurrence, are each independently hydrogen, C₁-C₄ alkyl, or C₃-C₆ cycloalkyl; wherein:
      the C₁-C₄ alkyl of any one of **R^{h}, Rⁱ,** and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl of any one of **R^{h}, Rⁱ**, and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy,
         -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{k}**, for each occurrence, is independently hydrogen, C₁-C₄ alkyl, C₃-C₆ cycloalkyl, phenyl, or 5 or 6-membered heteroaryl; wherein:
      -O**R**^{k} cannot be -OH; the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁- C₂ alkyl)₂;
**R³** and **R⁴,** for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y},** -C(=O)O**R^{y}**, -C(=O)N**R^{v}R^{w},** -C(=O)N**R^{v}**O**R^{y}**,
   -C(=O)N**R^{v}**S(=O)**ₜR^{y}**, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, -OC(=O)O**R^{y}**, -OC(=O)N**R^{v}R^{w}**, -S(=O)ₜ**R^{y}**, -S(=O)**ₜ**N**R^{v}R^{w}**, -S(=O)**ₜ**N**R^{v}**C(=O)**R^{y}**, -P(=O)**R^{z}R^{z},** phenyl, or a 5 or 6-membered heteroaryl; wherein:
   the C₁-C₆ alkyl, the C₂-C₆ alkenyl, or the C₃-C₆ cycloalkyl of any one of **R³** and **R⁴** is optionally substituted with 1 to 3 groups selected from cyano,
   -C(=O)**R^{y}**, -C(=O)O**R^{y}**, -C(=O)NR^{v}R^{w}, -N**R^{v}R^{w},** -N**R^{v}**C(=O)**R^{y}**, -N**R^{v}**C(=O)O**R^{y}**, -N**R^{v}**C(=O)N**R^{w}R^{x}**, -N**R^{v}**S(=O)ᵣ**R^{y}**, -O**R^{y}**, -OC(=O)**R^{y}**, -OC(=O)O**R^{y}**, -OC(=O)N**R^{v}R^{w}**, -S(=O)ₜ**R^{y}**, and -S(=O)**ₜ**N**R^{v}R^{w}**; wherein:
   **R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
      the C₁-C₄ alkyl of any one of **R^{v}**, **R^{w}**, and **R^{x}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, - C(=O)O(C₁-C₂ alkyl), -C(=O)NH₂, -C(=O)NH(C₁-C₂ alkyl), and - C(=O)N(C₁-C₂ alkyl)₂; and
      the C₃-C₆ cycloalkyl, the 5 or 6-membered heterocyclyl, or the 5 or 6-membered heteroaryl of any one of **R^{v}**, **R^{w}**, and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy,
         -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{y}**, for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
      the C₁-C₄ alkyl of **R^{y}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, - NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), - C(=O)NH₂, -C(=O)NH(C₁-C₂ alkyl), and
   -C(=O)N(C₁-C₂ alkyl)₂;
   **R^{z}**, for each occurrence, is independently C₁-C₂ alkyl, -OH, or -O(C₁-C₂ alkyl);
**k** is an integer selected from 1, 2, and 3; and
**o, p, r, s**, and **t** are each independently an integer selected from 1 and 2.

In some embodiments, in the compound, tautomer, deuterated derivative, or pharmaceutically acceptable salt of the disclosure, **R^{F}** and **R^{G}** are each independently hydrogen, F, -OH, -CH(OH)CH₃, -C₂H₅, -C(=O)NHCH₃, -C(=N)OCH₃, -CH₃, -CH₂F, - CH₂CN, -(CH₂)₂CN, -CH₂OH, -(CH₂)₂OH, -CH₂OCH₃, -CH₂OC₂H₅, -(CH₂)₂OCH₃, - CH₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅, -CH₂(O)C(=O)N(CH₃)₂, -CH₂(O)C(=O)N(C₂H₅)₂, -CH₂(O)C(=O)NH(cyclopentyl), -CH₂(O)C(=O)NH(tetrahydrofuranyl), -CH₂(O)(pyridin-2-yl), -CH₂(O)(pyrimidin-2-yl; and all other variables not specifically defined herein are as defined in Formula (XIa), Formula (XIb), Formula (XIc), Formula (XId), Formula (XIe), or Formula (XIf).

In some embodiments, in the compound, tautomer, deuterated derivative, or pharmaceutically acceptable salt of the disclosure, **R^{F}** and **R^{G}** are each independently hydrogen, F, -CH(OH)CH₃, -CH₃, -CH₂CN, -CH₂OH, and -CH₂OCH₃; and all other variables not specifically defined herein are as defined in Formula (XIa), Formula (XIb), Formula (XIc), Formula (XId), Formula (XIe), or Formula (XIf).

In some embodiments, in the compound, tautomer, deuterated derivative, or pharmaceutically acceptable salt of the disclosure, **R¹** is cyano, F, Cl, -CH₃, -CHF₂, -CF₃, - OCH₃, or -OCH(CH₃)₂; and all other variables not specifically defined herein are as defined in any one of the preceding embodiments.

In some embodiments, in the compound, tautomer, deuterated derivative, or pharmaceutically acceptable salt of the disclosure, at least one **R¹** is F; and all other variables not specifically defined herein are as defined in any one of the preceding embodiments.

In some embodiments, in the compound, deuterated derivative, pharmaceutically acceptable salt, or tautomer of the disclosure, **X** is absent or a bond, -(C**R^{a}R^{b}**)-, or -SO₂-; **R^{a}** and **R^{b}**, for each occurrence, are each independently hydrogen, C₁-C₃ alkyl, C₁-C₃ haloalkyl, or C₁-C₃ alkoxy; and all other variables not specifically defined herein are as defined in any one of the preceding embodiments.

In some embodiments, in the compound, tautomer, deuterated derivative, or pharmaceutically acceptable salt of the disclosure, **X** is absent or a bond, -CH₂-, or -SO₂-; and all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, F, Cl, cyano, -CH₃, -CF₃, or -NH₂; and all other variables not specifically defined herein are as defined in any one of the preceding embodiments.

In some embodiments, in the compound, tautomer, deuterated derivative, or pharmaceutically acceptable salt of the disclosure, **R³** is C₁-C₆ alkyl, C₂-C₆ alkenyl, C₁-C₆ alkoxy, C₁-C₆ haloalkyl, C₁-C₆ haloalkoxy, -C(=O)**R^{y}**, -C(=O)O**R^{y}**, -O**R^{y}**, -OC(=O)**R^{y}**, or - OC(=O)O**R^{y}**;
the C₁-C₆ alkyl or the C₂-C₆ alkenyl of **R³** is optionally substituted with 1 to 3 groups selected from cyano, -C(=O)**R^{y}**, -C(=O)O**R^{y}**, -O**R^{y}**, -OC(=O)**R^{y}**, and
-OC(=O)O**R^{y}**; wherein **R^{y}**, for each occurrence, is independently hydrogen or C₁-C₄ alkyl; and all other variables not specifically defined herein are as defined in any one of the preceding embodiments.

In some embodiments, a compound of the disclosure is a compound of Formula (XIIa), Formula (XIIb), Formula (XIIc), Formula (XIId), Formula (XIIe), or Formula (XIIf): 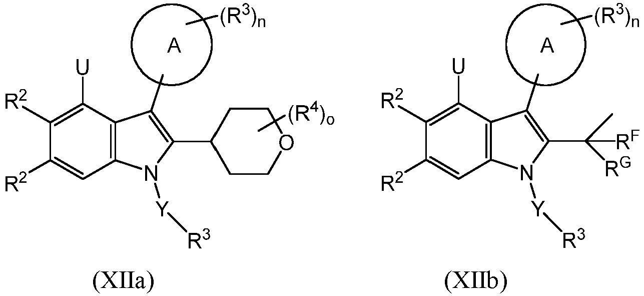 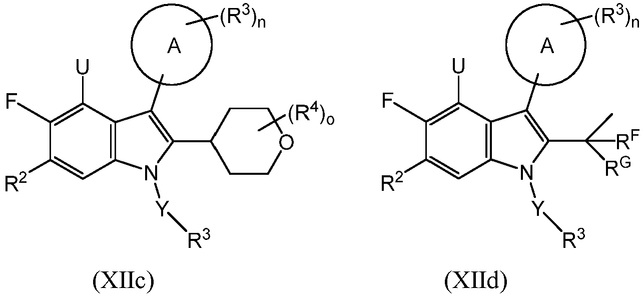 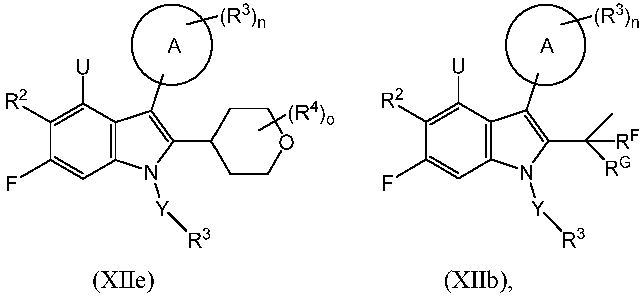 a tautomer thereof, a deuterated derivative of that compound or tautomer, or a pharmaceutically acceptable salt of any of the foregoing, wherein:
**U** is -OH;
**Y** is absent or a bond, -(C**R^{c}R^{d}**)**_{q}**-, -C(=O)-, or -SO₂-;
**R^{c}** and **R^{d}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**Ring A** is C₃-C₁₂ carbocyclyl, 3 to 12-membered heterocyclyl, C₆ or C₁₀ aryl, or 5 to 10-membered heteroaryl;
**R^{E}**, **R^{F}**, and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
   the C₁-C₆ alkyl or the C₂-C₆ alkenyl of any one of **R^{E}**, **R^{F}**, and **R^{G}** is optionally substituted with 1 to 3 groups selected from cyano,
   -C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{q}R^{q}**, -S(=O)ᵣ**R^{s}**, and -S(=O)**ᵣ**N**R^{p}R^{q}**; wherein:
   **R^{p}, R^{q}**, and **R^{r},** for each occurrence, are each independently hydrogen, C₁-C₄ alkyl, C₃-C₆ cycloalkyl, or 3 to 6-membered heterocyclyl; wherein:
      the C₁-C₄ alkyl of any one of **R^{p}**, **R^{q}**, and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
      the C₃-C₆ cycloalkyl or the 3 to 6-membered heterocyclyl of any one of **R^{p}**, **R^{q}**, and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
   **R^{s}**, for each occurrence, is independently hydrogen, C₁-C₄ alkyl, C₃-C₆ cycloalkyl, phenyl, or 5 or 6-membered heteroaryl; wherein:
      the C₁-C₄ alkyl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
      the C₃-C₆ cycloalkyl, the phenyl, or the 5 or 6-membered heteroaryl of **R^{s}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
**R²**, for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, phenyl, or 5 or 6-membered heteroaryl; wherein:
   the C₁-C₆ alkyl, the C₂-C₆ alkenyl, or the C₃-C₆ cycloalkyl of **R²** is optionally substituted with 1 to 3 groups selected from cyano, -C(=O)**R^{k}**,
   -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k}**, -OC(=O)**R^{k}**, -OC(=O)O**R^{k}**, -OC(=O)N**R^{h}Rⁱ**, -S(=O)**ₛR^{k}**, and S(=O)ₛN**R^{h}Rⁱ**; wherein:
   **R^{h}**, **Rⁱ**, and **R^{j}**, for each occurrence, are each independently hydrogen, C₁-C₄ alkyl, or C₃-C₆ cycloalkyl; wherein:
      the C₁-C₄ alkyl of any one of **R^{h}, Rⁱ**, and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl of any one of **R^{h}, Rⁱ**, and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy,
         -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{k},** for each occurrence, is independently hydrogen, C₁-C₄ alkyl, C₃-C₆ cycloalkyl, phenyl, or 5 or 6-membered heteroaryl; wherein:
      -O**R^{k}** cannot be -OH; the C₁-C₄ alkyl of **R^{k}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁- C₂ alkyl)₂;
**R³** and **R⁴**, for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w},** -C(=O)N**R^{v}**O**R^{y}**,
   -C(=O)N**R^{v}**S(=O)**ₜR^{y}**, -N**R^{v}R^{w},** -N**R^{v}**C(=O)R**^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, -OC(=O)O**R^{y}**, -OC(=O)N**R^{v}R^{w}**, -S(=O)ₜ**R^{y}**, -S(=O)**ₜ**N**R^{v}R^{w}**, -S(=O)**ₜ**N**R^{v}**C(=O)**R^{y}**, -P(=O)**R^{z}R^{z}**, phenyl, or a 5 or 6-membered heteroaryl;
wherein:
the C₁-C₆ alkyl, the C₂-C₆ alkenyl, or the C₃-C₆ cycloalkyl of any one of **R³** and **R⁴** is optionally substituted with 1 to 3 groups selected from cyano, -C(=O)**R^{y}**, -C(=O)O**R^{y}**, -C(=O)NR^{v}R^{w}, -N**R^{v}R^{w},** -N**R^{v}**C(=O)**R^{y}**, -N**R^{v}**C(=O)O**R^{y}**, -N**R^{v}**C(=O)N**R^{w}R^{x}**, -N**R^{v}**S(=O)ᵣ**R^{y}**, -O**R^{y}**, -OC(=O)**R^{y}**, -OC(=O)O**R^{y}**, -OC(=O)N**R^{v}R^{w}**, -S(=O)ₜ**R^{y}**, and -S(=O)**ₜ**N**R^{v}R^{w}**; wherein:
   **R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
      the C₁-C₄ alkyl of any one of **R^{v}**, **R^{w}**, and **R^{x}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, - C(=O)O(C₁-C₂ alkyl), -C(=O)NH₂, -C(=O)NH(C₁-C₂ alkyl), and - C(=O)N(C₁-C₂ alkyl)₂; and
      the C₃-C₆ cycloalkyl, the 5 or 6-membered heterocyclyl, or the 5 or 6-membered heteroaryl of any one of **R^{v}**, **R^{w}**, and **R^{x}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy,
         -C(=O)OH, -C(=O)O(C₁-C₂ alkyl), -C(=O)NH₂, -C(=O)NH(C₁-C₂ alkyl), and -C(=O)N(C₁-C₂ alkyl)₂;
   **R^{y}**, for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
      the C₁-C₄ alkyl of **R^{y}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, - C(=O)OH, -C(=O)O(C₁-C₂ alkyl), -C(=O)NH₂, -C(=O)NH(C₁-C₂ alkyl), and -C(=O)N(C₁-C₂ alkyl)₂; and
      the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, - NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), - C(=O)NH₂, -C(=O)NH(C₁-C₂ alkyl), and

         -C(=O)N(C₁-C₂ alkyl)₂;
      **R^{z}**, for each occurrence, is independently C₁-C₂ alkyl, -OH, or
-O(C₁-C₂ alkyl);

n is integer selected from 0, 1, 2, and 3; and
**o**, **q**, **r**, s, and t are each independently an integer selected from 1 and 2.

U is -OH.

In some embodiments, in the compound, tautomer, deuterated derivative, or pharmaceutically acceptable salt of the disclosure, **Ring A** is optionally substituted with **R³** and **Ring A** is C₃-C₇ carbocyclyl, 6 to 9-membered heterocyclyl, phenyl, or 5 to 9-membered heteroaryl, wherein all other variables not specifically defined herein are as defined in Formula (XIIa), Formula (XIIb), Formula (XIIc), Formula (XIId), or Formula (XIIe).

In some embodiments, in the compound, tautomer, deuterated derivative, or pharmaceutically acceptable salt of the disclosure, **Ring A** is optionally substituted with **R³** and **Ring A** is C₃ carbocyclyl, C₄ carbocyclyl, or C₇ carbocyclyl, wherein all other variables not specifically defined herein are as defined in Formula (XIIa), Formula (XIIb), Formula (XIIc), Formula (XIId), or Formula (XIIe).

In some embodiments, in the compound, tautomer, deuterated derivative, or pharmaceutically acceptable salt of the disclosure, **Ring A** is optionally substituted with **R³** and **Ring A** is phenyl, wherein all other variables not specifically defined herein are as defined in Formula (XIIa), Formula (XIIb), Formula (XIIc), Formula (XIId), or Formula (XIIe).

In some embodiments, in the compound, tautomer, deuterated derivative, or pharmaceutically acceptable salt of the disclosure, **Ring A** is optionally substituted with **R³** and **Ring A** is pyridine, pyrimidine, pyrazole, thiophene, or oxadiazole, wherein all other variables not specifically defined herein are as defined in Formula (XIIa), Formula (XIIb), Formula (XIIc), Formula (XIId), or Formula (XIIe).

In some embodiments, in the compound, tautomer, deuterated derivative, or pharmaceutically acceptable salt of the disclosure, **R^{F}** and **R^{G}** are each independently hydrogen, F, -OH, -CH(OH)CH₃, -C₂H₅, -C(=O)NHCH₃, -C(=N)OCH₃, -CH₃, -CH₂F, - CH₂CN, -(CH₂)₂CN, -CH₂OH, -(CH₂)₂OH, -CH₂OCH₃, -CH₂OC₂H₅, -(CH₂)₂OCH₃, - CH₂OCHF₂, -CH₂C(=O)NH₂, -CH₂C(=O)N(CH₃)₂, -CH₂S(=O)₂CH₃, -(CH₂)₂S(=O)₂CH₃, -CH₂(O)C(=O)NHCH₃, -CH₂(O)C(=O)N(CH₃)C₂H₅, -CH₂(O)C(=O)N(CH₃)₂, -CH₂(OC(=O(C₂H₅)₂, -CH₂(O)C(=O)NH(cyclopentyl), -CH₂(O)C(=O)NH(tetrahydrofuranyl), -CH₂(O)(pyridin-2-yl), -CH₂(O)(pyrimidin-2-yl); and all other variables not specifically defined herein are as defined in any one of the preceding embodiments.

In some embodiments, in the compound, tautomer, deuterated derivative, or pharmaceutically acceptable salt of the disclosure, **R^{F}** and **R^{G}** are each independently hydrogen, F, -CH(OH)CH₃, -CH₃, -CH₂CN, -CH₂OH, and -CH₂OCH₃; and all other variables not specifically defined herein are as defined in any one of the preceding embodiments.

In some embodiments, in the compound, tautomer, deuterated derivative, or pharmaceutically acceptable salt of the disclosure, **R¹** is cyano, F, Cl, -CH₃, -CHF₂, -CF₃, - OCH₃, or -OCH(CH₃)₂; and all other variables not specifically defined herein are as defined in any one of the preceding embodiments.

In some embodiments, in the compound, tautomer, deuterated derivative, or pharmaceutically acceptable salt of the disclosure, at least one **R¹** is F; and all other variables not specifically defined herein are as defined in any one of the preceding embodiments.

In some embodiments, in the compound, deuterated derivative, pharmaceutically acceptable salt, or tautomer of the disclosure, **X** is absent or a bond, -(C**R^{a}R^{b}**)-, or -SO₂-; **R^{a}** and **R^{b}**, for each occurrence, are each independently hydrogen, C₁-C₃ alkyl, C₁-C₃ haloalkyl, or C₁-C₃ alkoxy; and all other variables not specifically defined herein are as defined in any one of the preceding embodiments.

In some embodiments, in the compound, tautomer, deuterated derivative, or pharmaceutically acceptable salt of the disclosure, **X** is absent or a bond, -CH₂-, or -SO₂-; and all other variables not specifically defined herein are as defined in any one of the preceding embodiments.

In some embodiments, in the compound, tautomer, deuterated derivative, or pharmaceutically acceptable salt of the disclosure, **R²**, for each occurrence, is independently hydrogen, F, Cl, cyano, -CH₃, -CF₃, or -NH₂; and all other variables not specifically defined herein are as defined in any one of the preceding embodiments.

In some embodiments, in the compound, tautomer, deuterated derivative, or pharmaceutically acceptable salt of the disclosure, **R³** is C₁-C₆ alkyl, C₂-C₆ alkenyl, C₁-C₆ alkoxy, C₁-C₆ haloalkyl, C₁-C₆ haloalkoxy, -C(=O)**R^{y}**, -C(=O)O**R^{y}**, -O**R^{y}**, -OC(=O)**R^{y}**, or - OC(=O)O**R^{y}**;
the C₁-C₆ alkyl or the C₂-C₆ alkenyl of **R³** is optionally substituted with 1 to 3 groups selected from cyano, -C(=O)**R^{y}**, -C(=O)O**R^{y}**, -O**R^{y}**, -OC(=O)**R^{y}**, and
-OC(=O)O**R^{y}**; wherein **R^{y}**, for each occurrence, is independently hydrogen or C₁-C₄ alkyl; and all other variables not specifically defined herein are as defined in any one of the preceding embodiments.

In some embodiments, the compound of any one of Formulae (I), (IIa)-(IIf), (IIIa)-(IIIb), (IVa)-(IVb), (Va)-(Vb), (VIa)-(VIe), (VIIa)-(VIIe), and (IXa)-(IXe) is selected from Compounds 1-457 (Table A below) and tautomers of those compounds, deuterated derivatives of those tautomers and compounds, and pharmaceutically acceptable salt of any of the foregoing.

In some embodiments, the compound of any one of Formulae (I), (IIa)-(IIf), (IIIa)-(IIIb), (IVa)-(IVb), (Va)-(Vb), (VIa)-(VIe), (VIIa)-(VIIe), and (IXa)-(IXe) is selected from Compounds 458-532 (Table B below) and tautomers of those compounds, deuterated derivatives of those tautomers and compounds, and pharmaceutically acceptable salt of any of the foregoing.

In some embodiments, the compound of any one of Formulae (I), (IIa)-(IIf), (IIIa)-(IIIb), (IVa)-(IVb), (Va)-(Vb), (VIa)-(VIe), (VIIa)-(VIIe), and (IXa)-(IXe) is selected from Compounds P1-P225 (Table E below) and tautomers of those compounds, deuterated derivatives of those tautomers and compounds, and pharmaceutically acceptable salt of any of the foregoing.

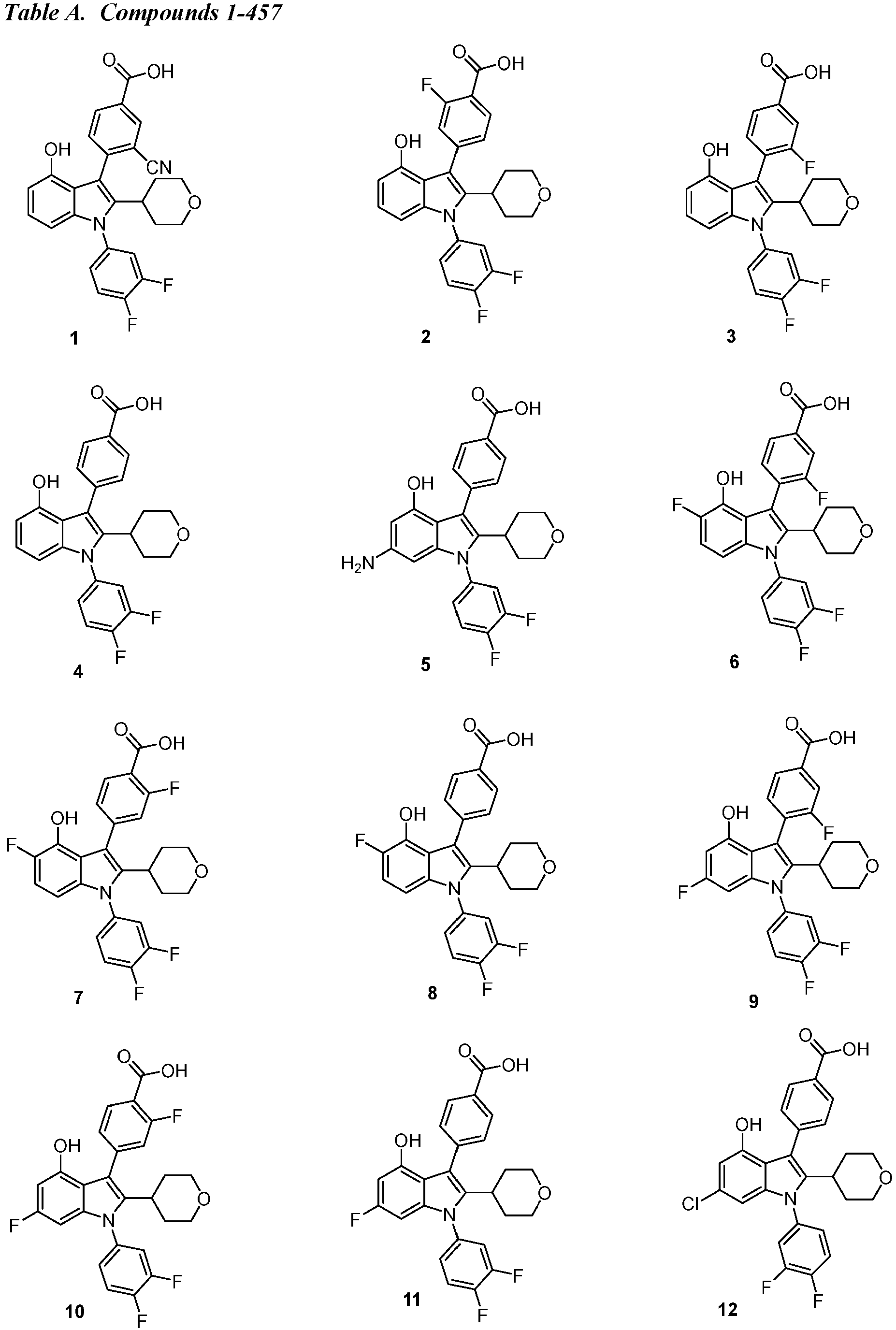

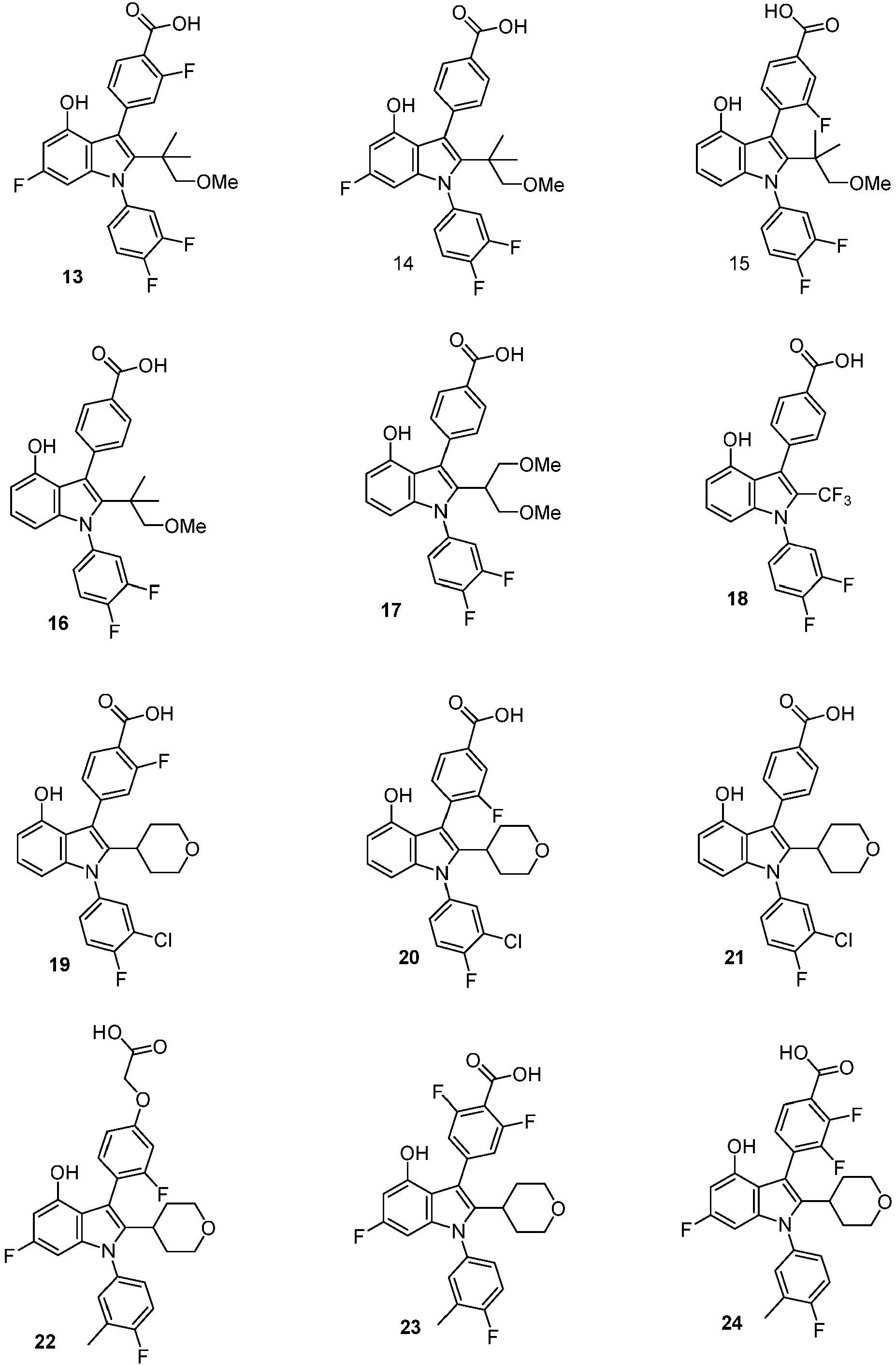

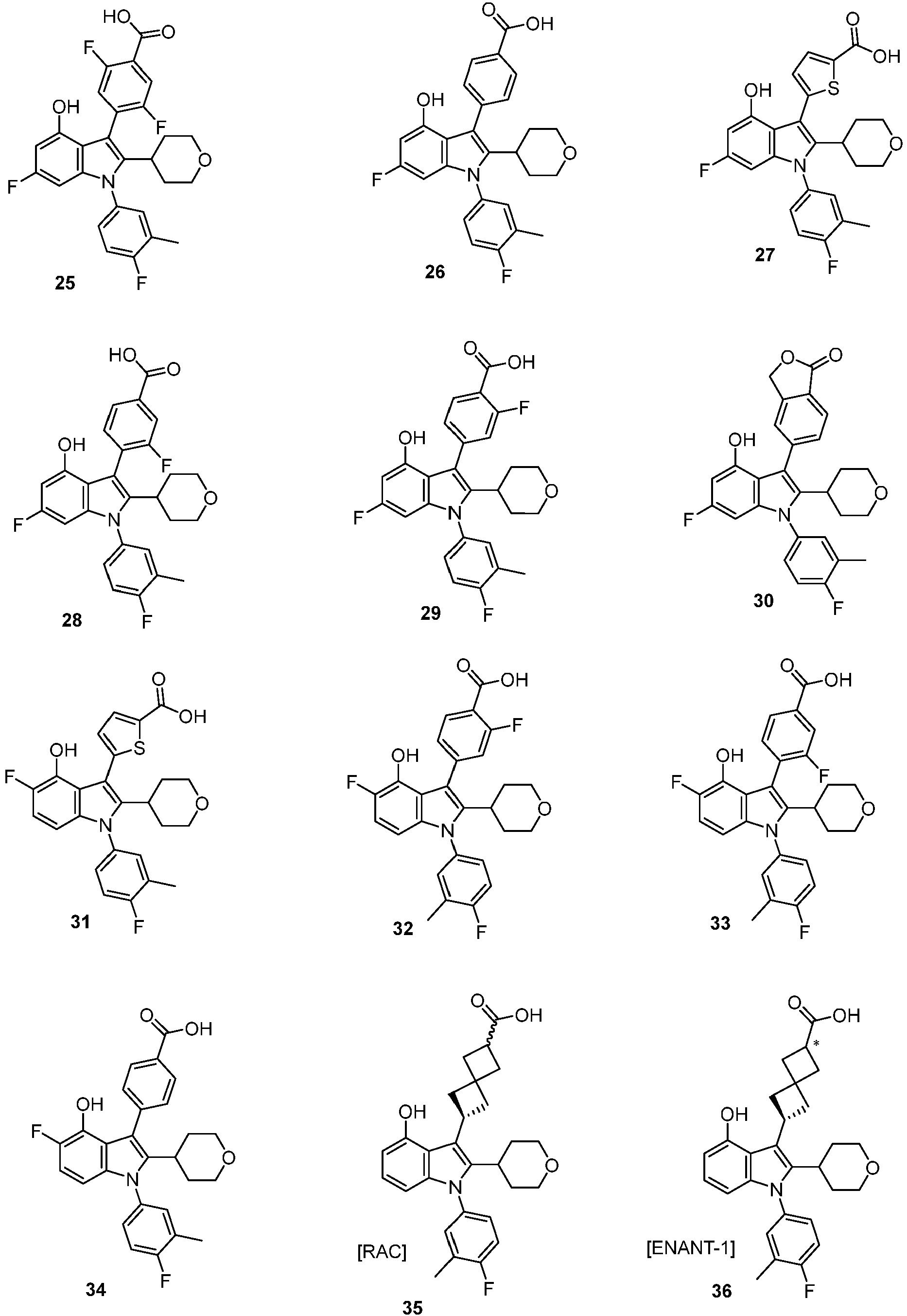

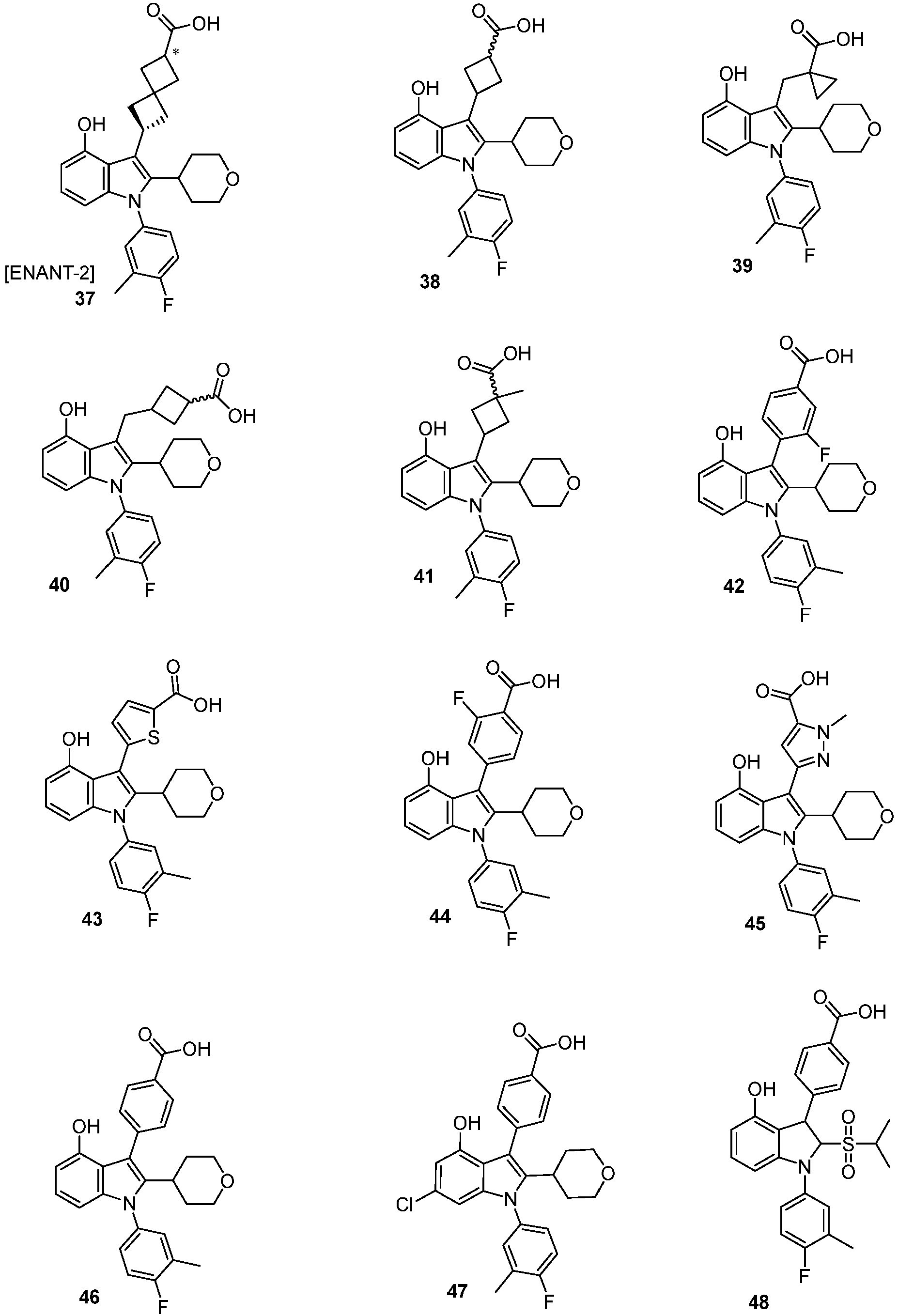

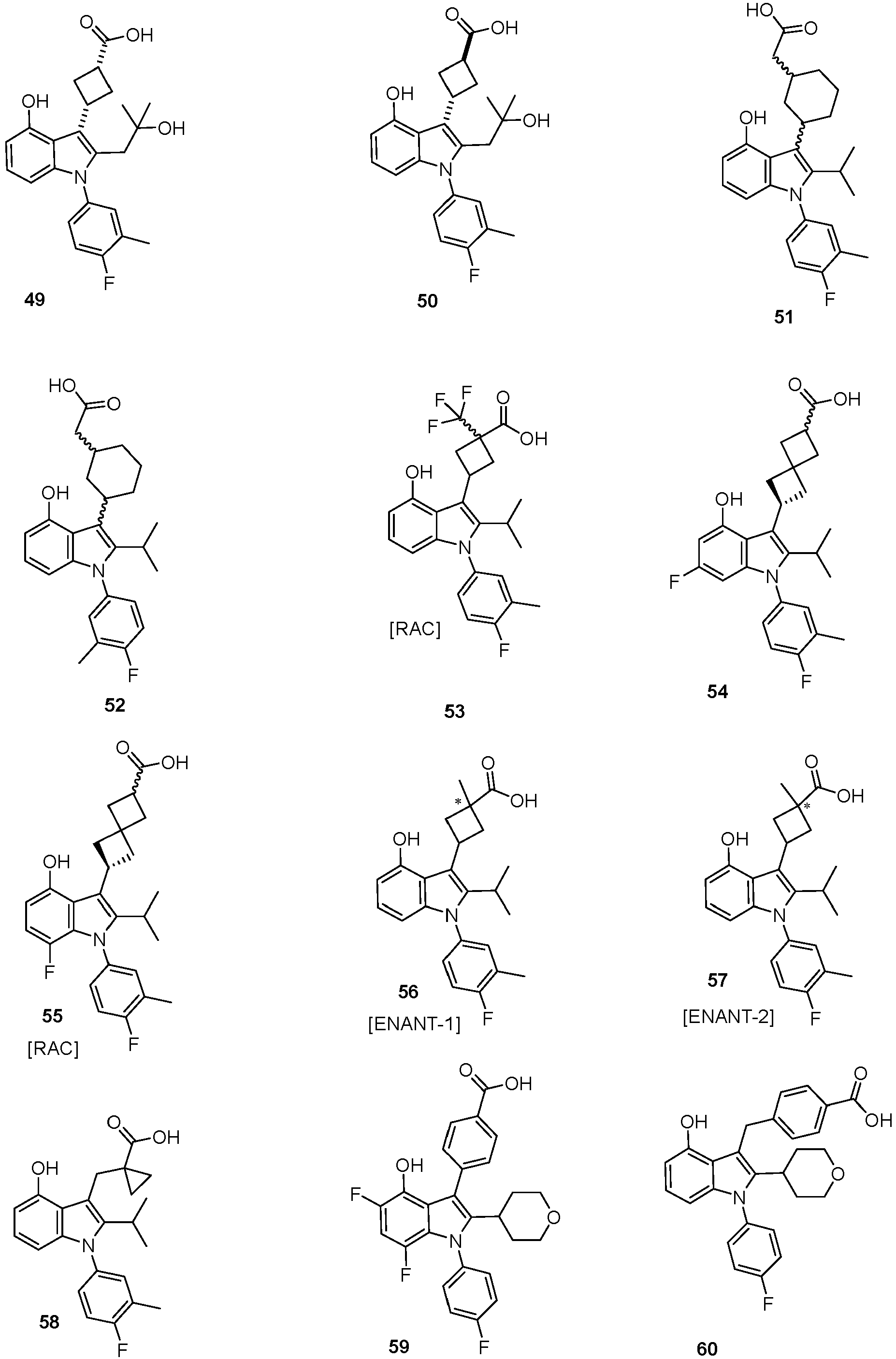

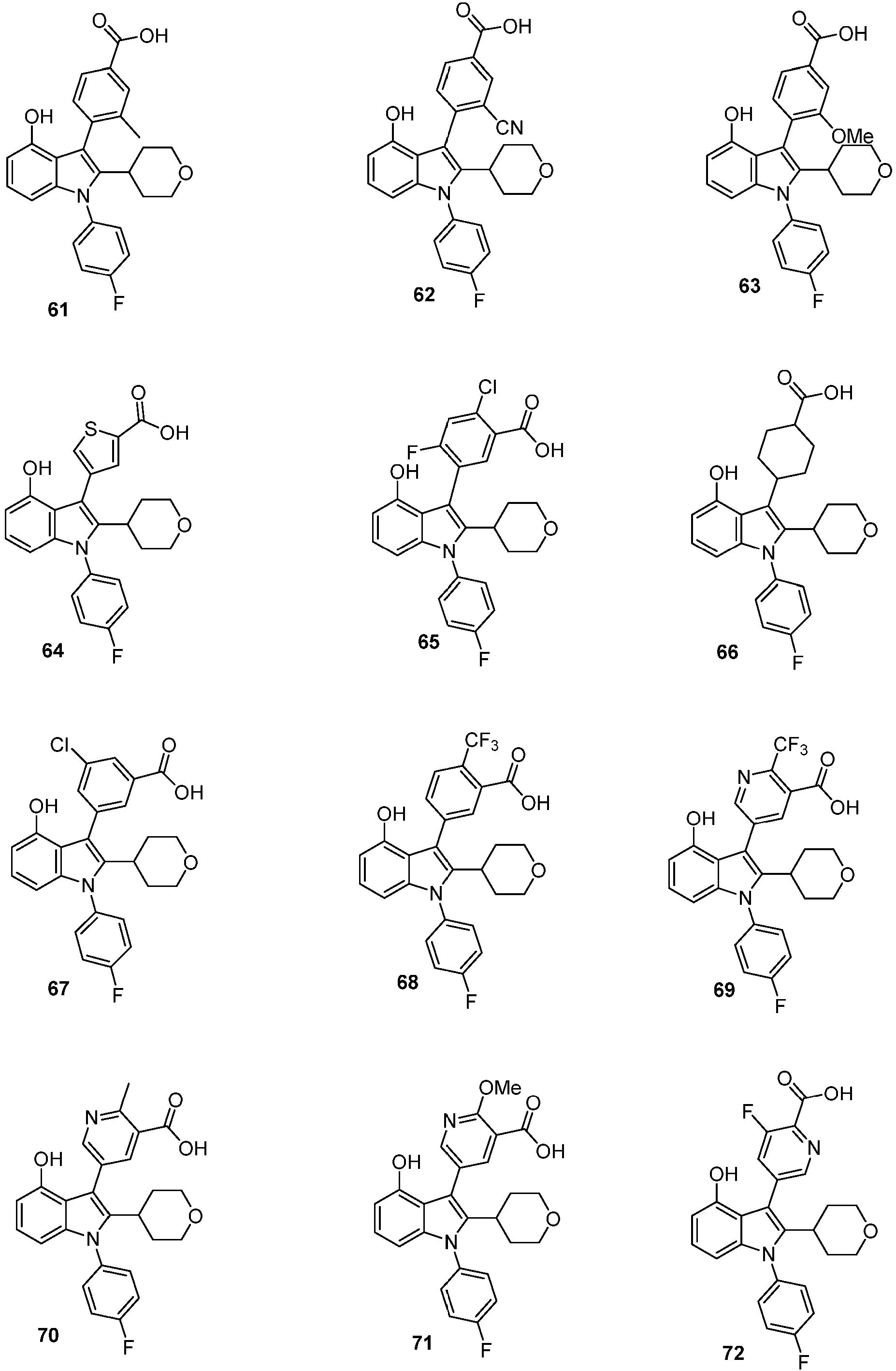

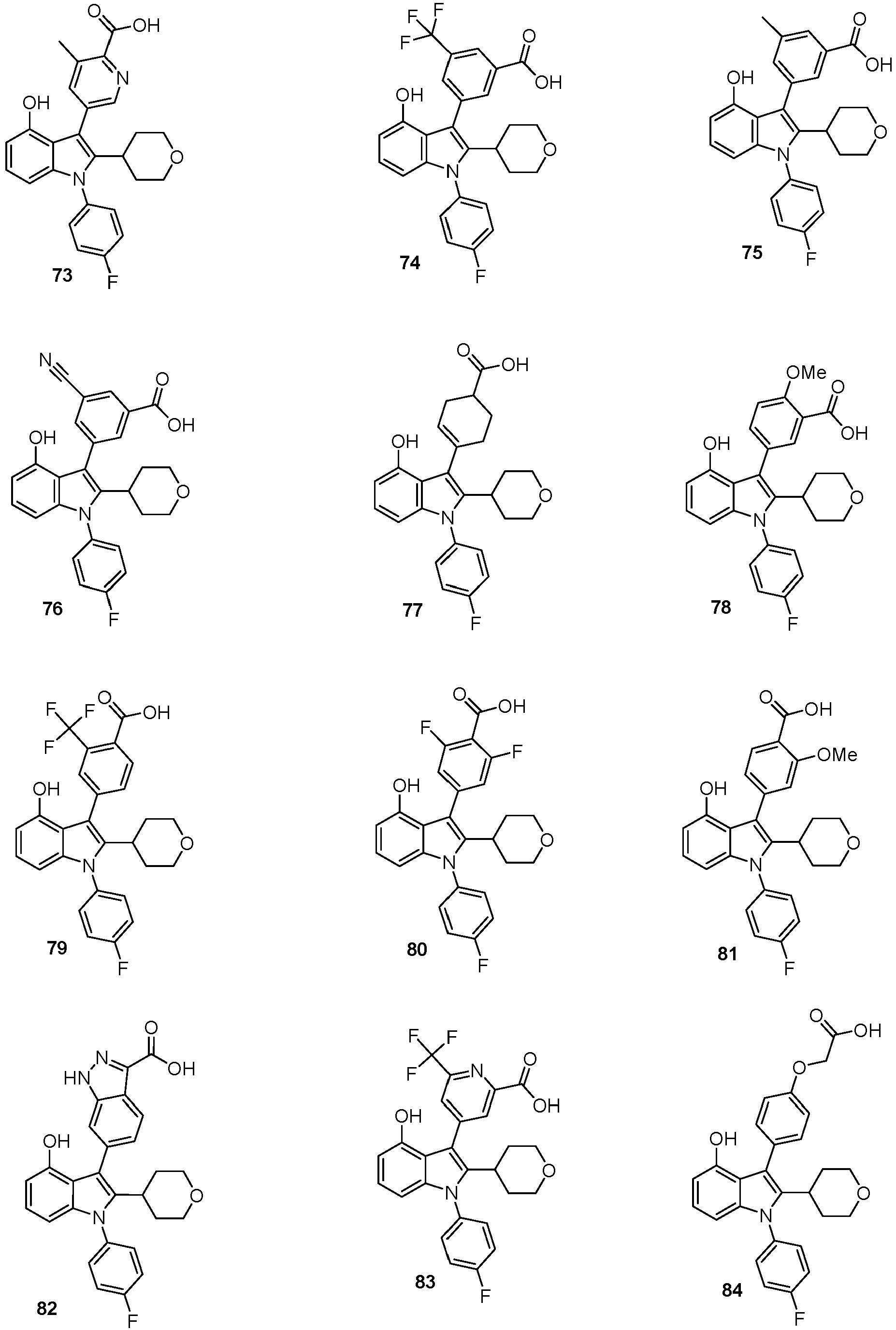

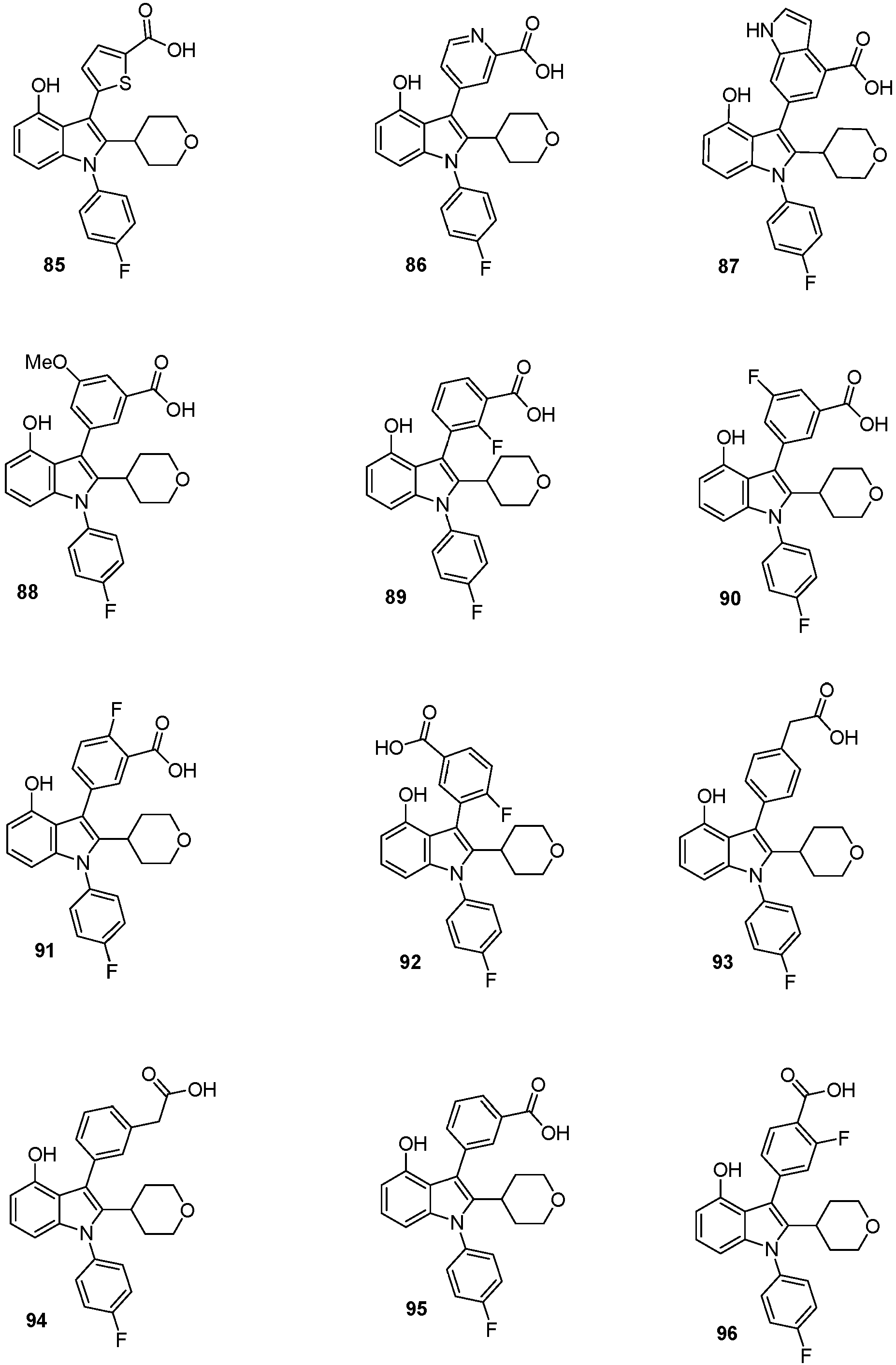

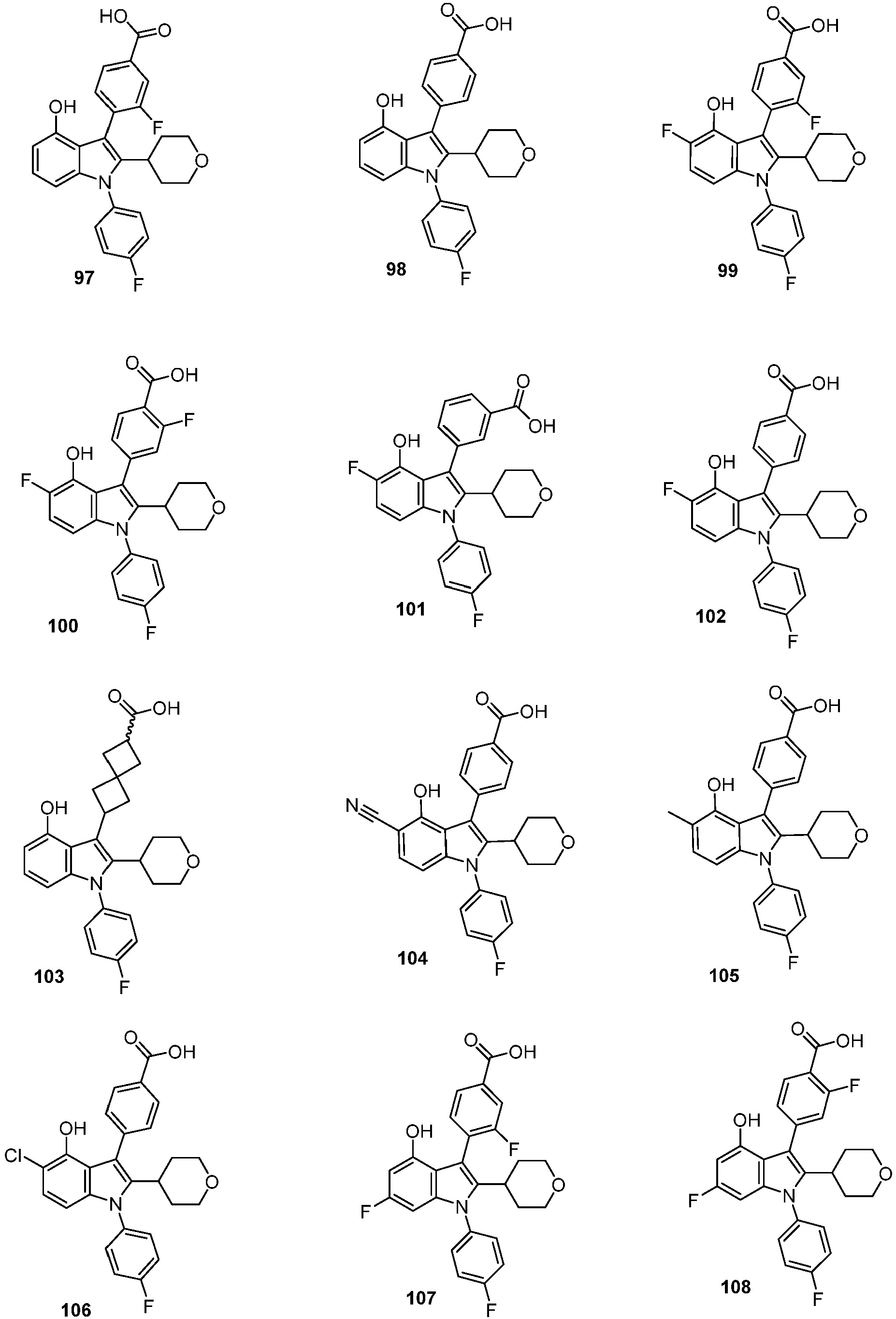

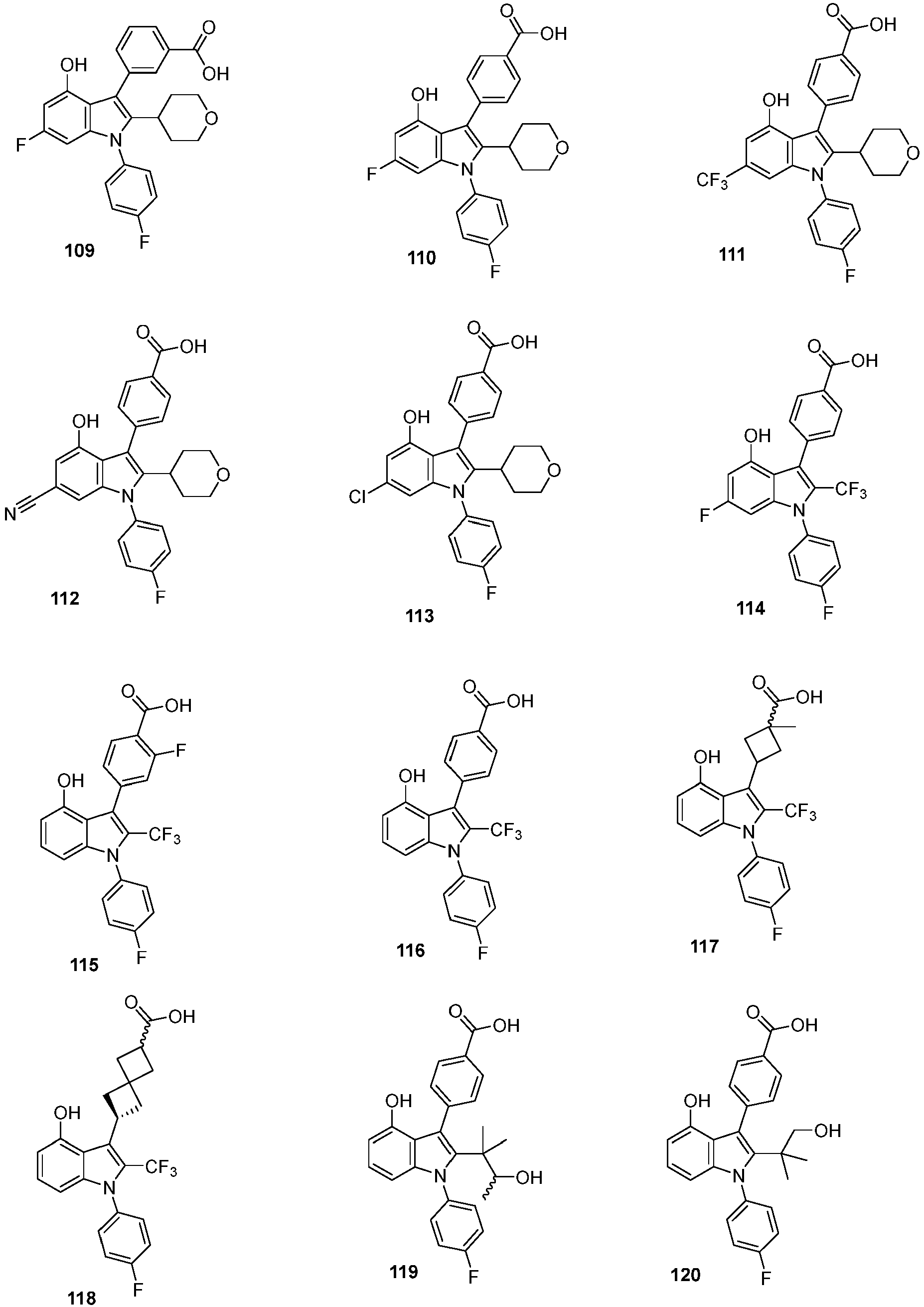

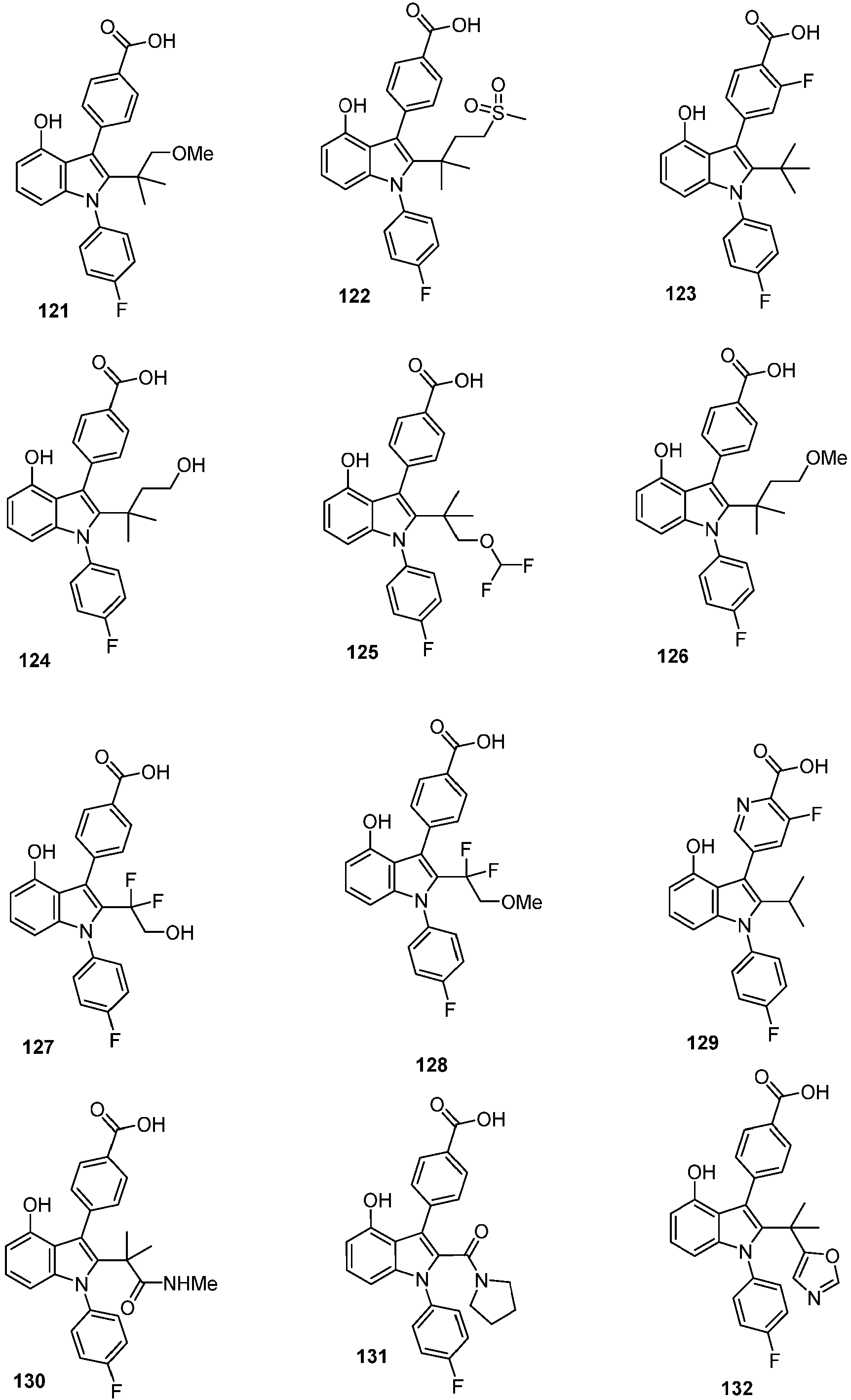

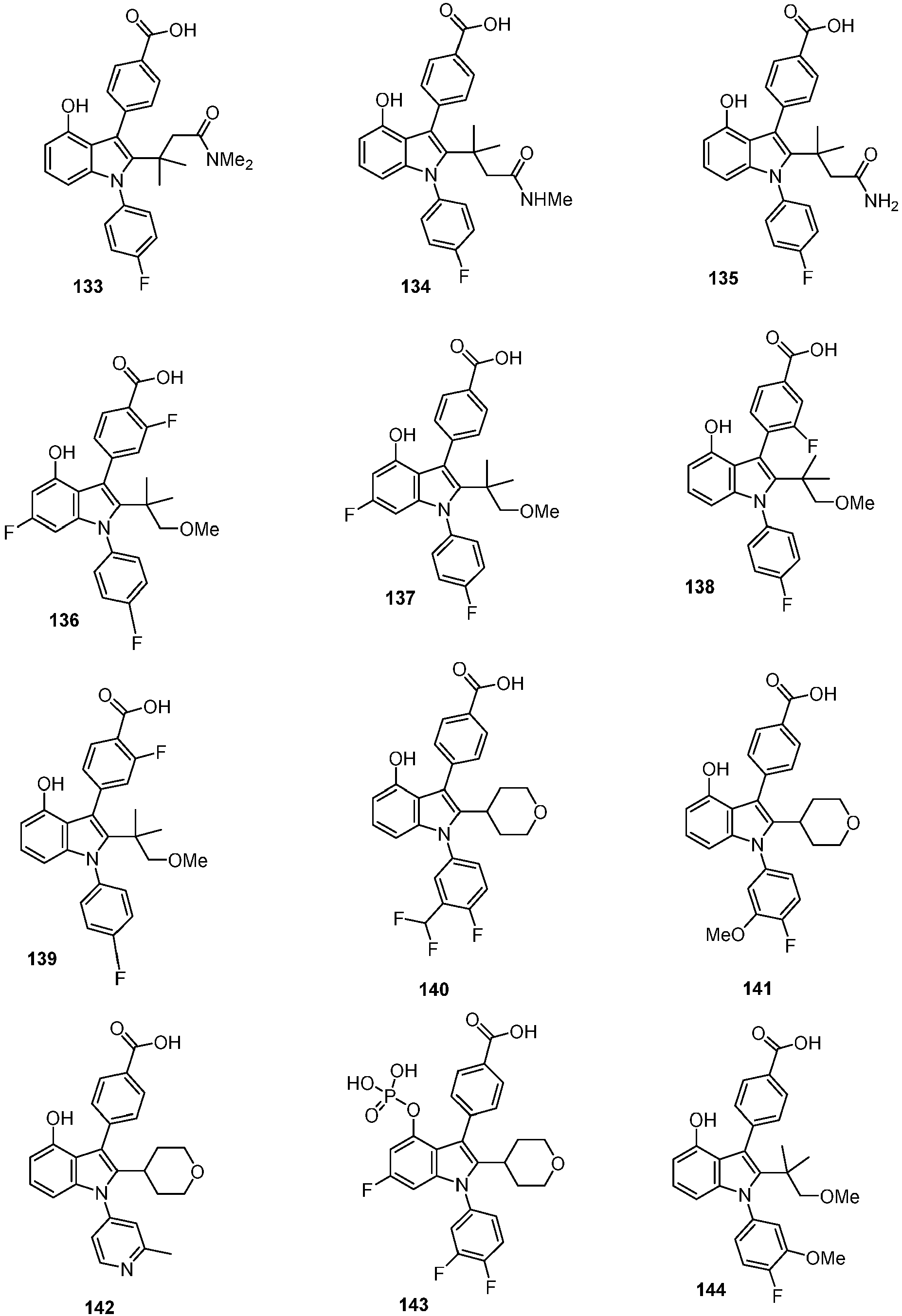

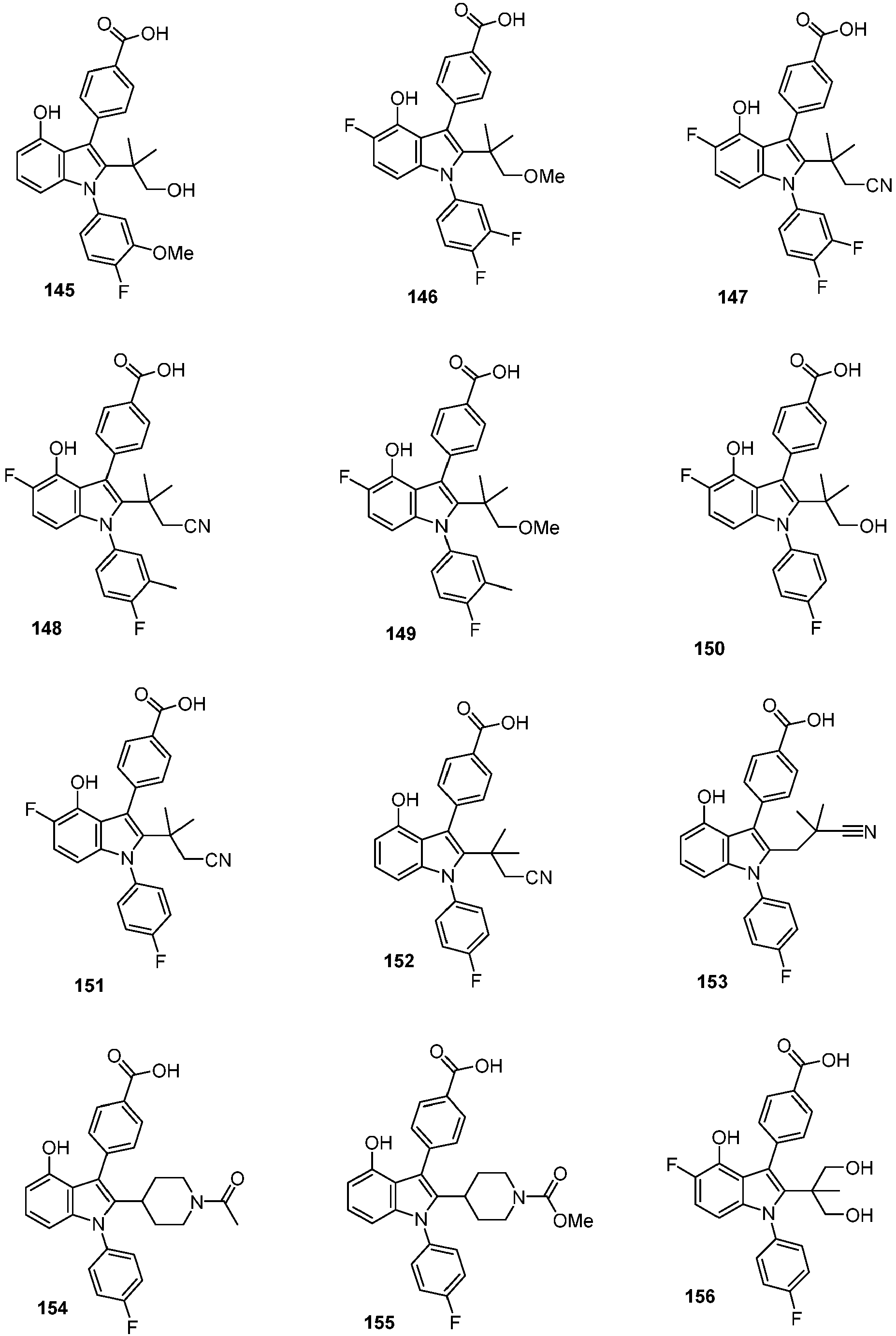

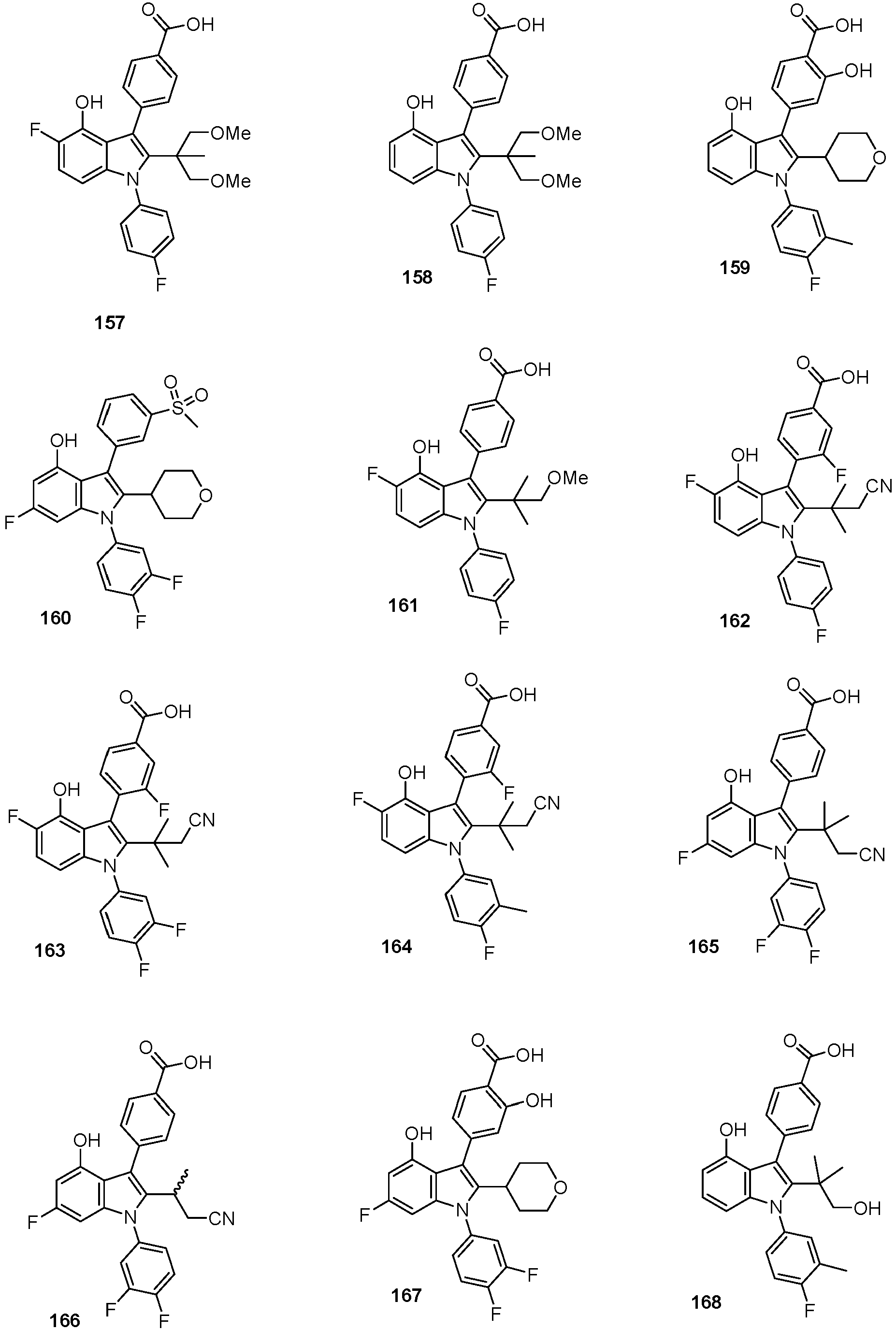

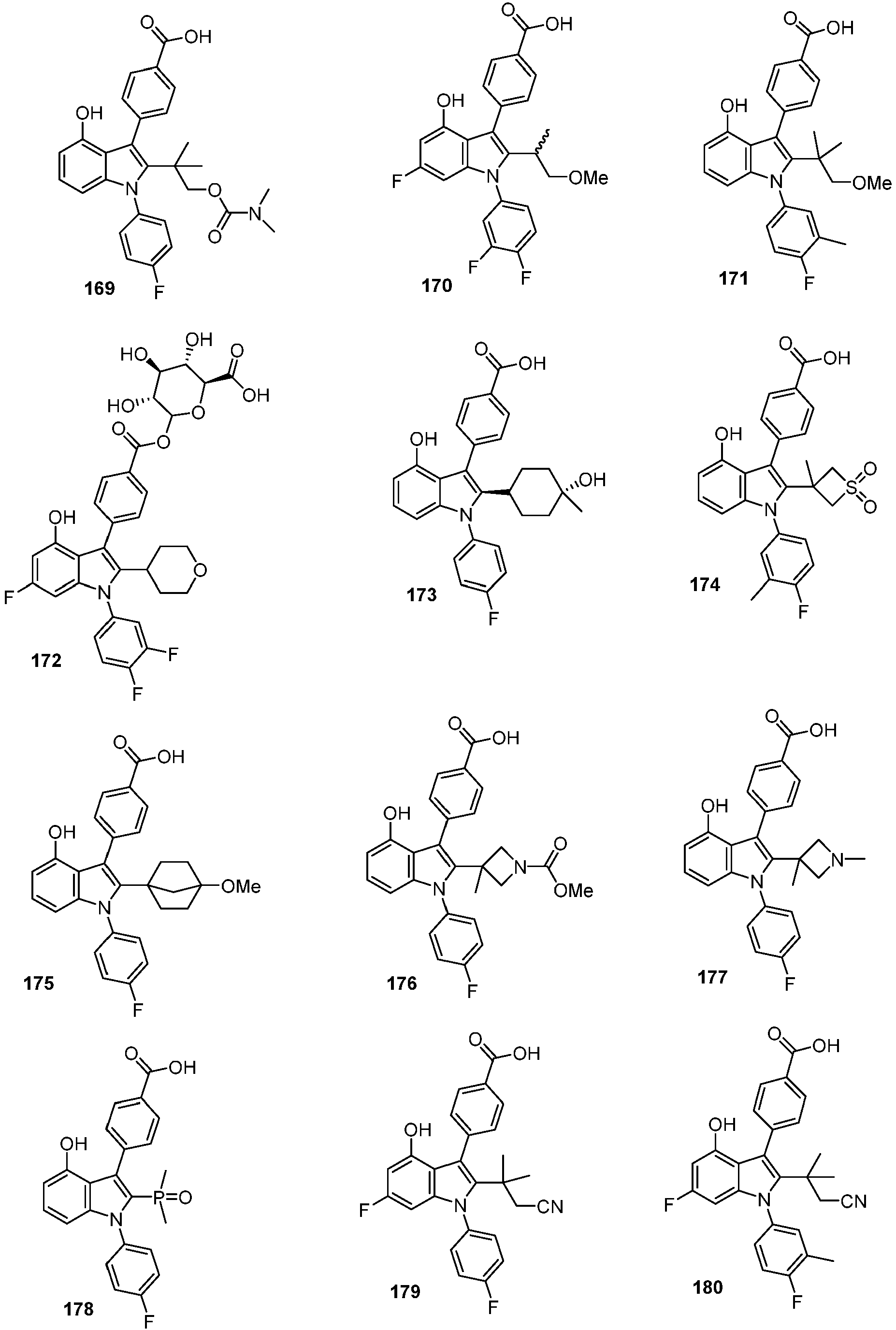

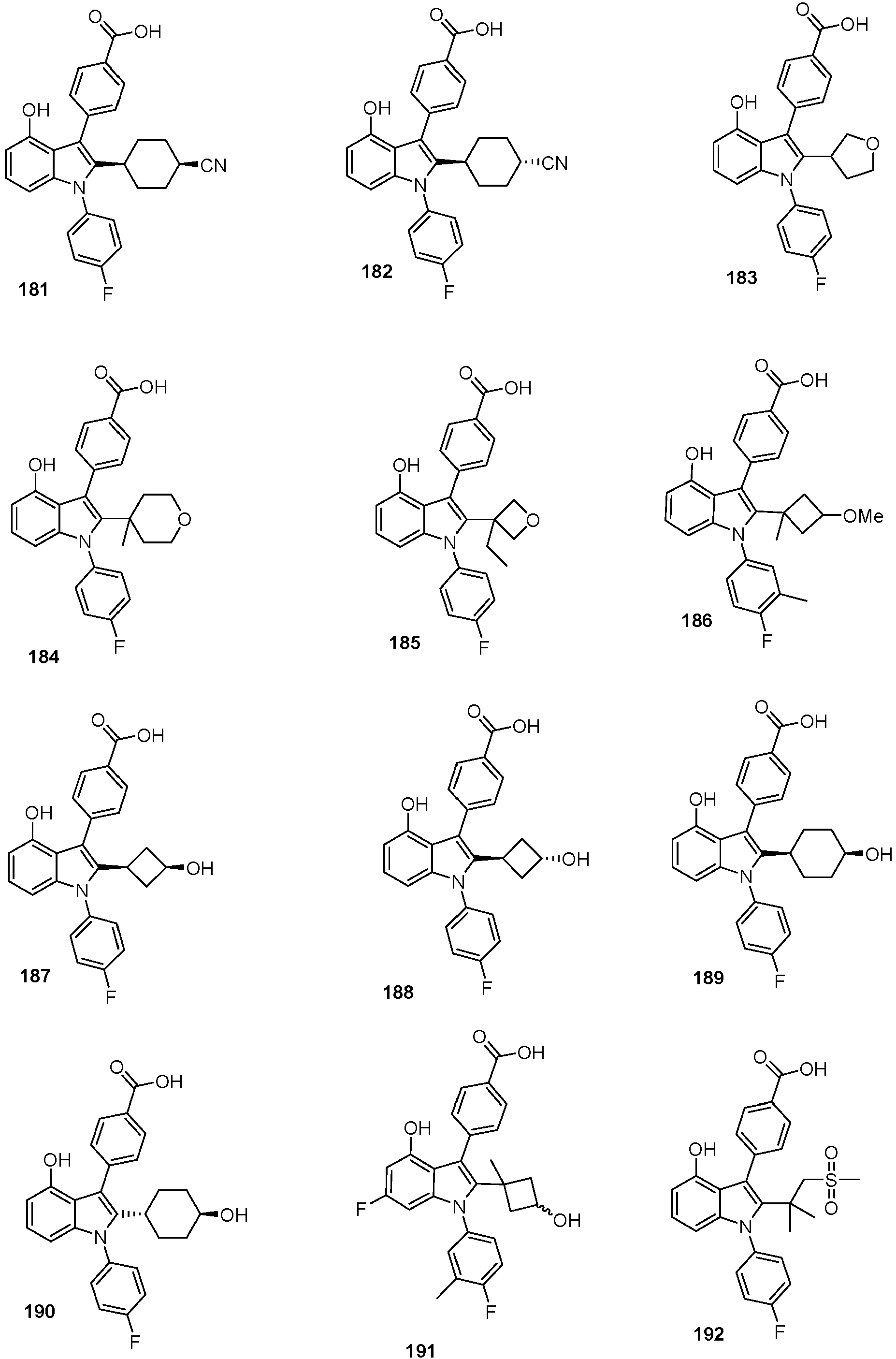

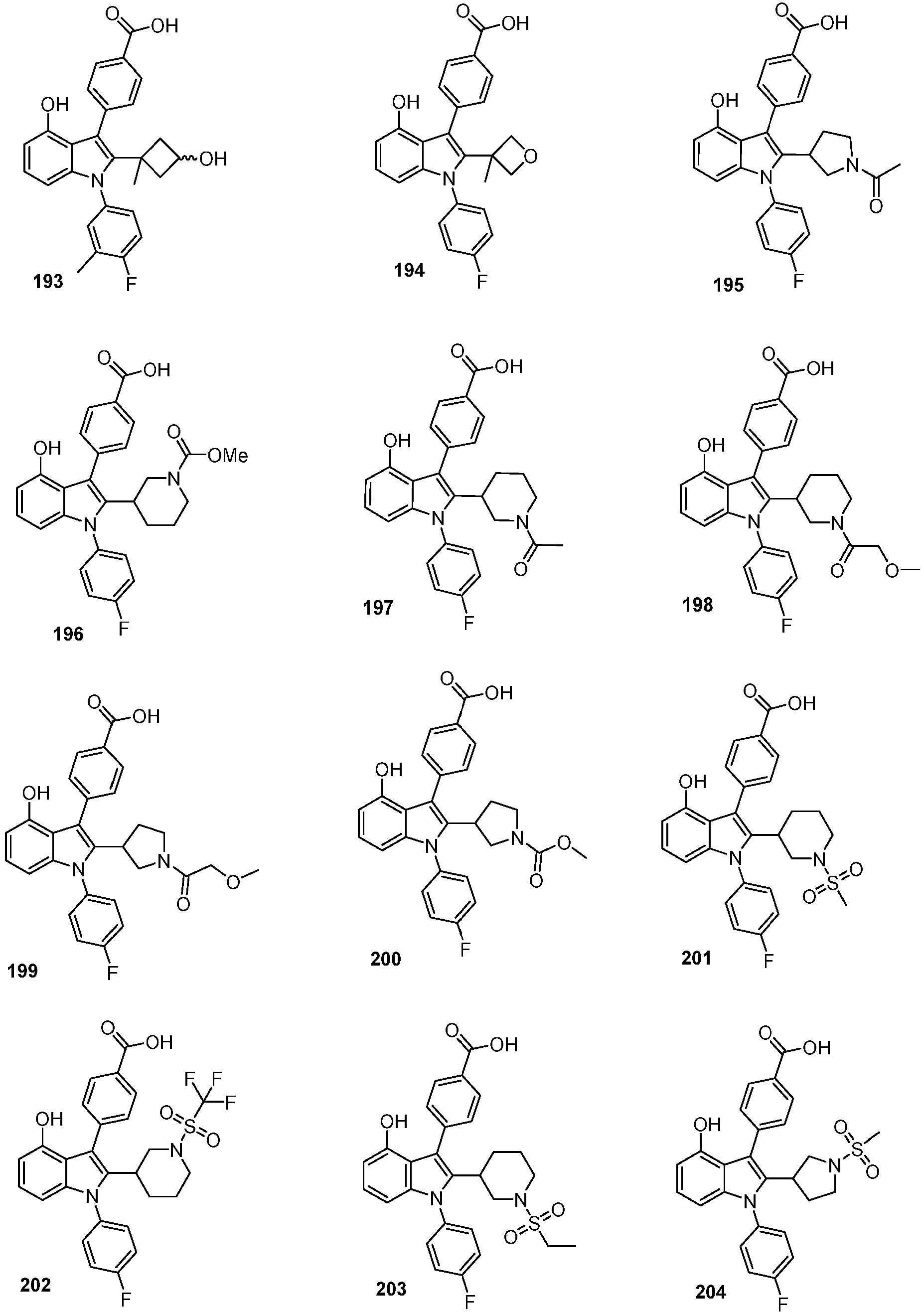

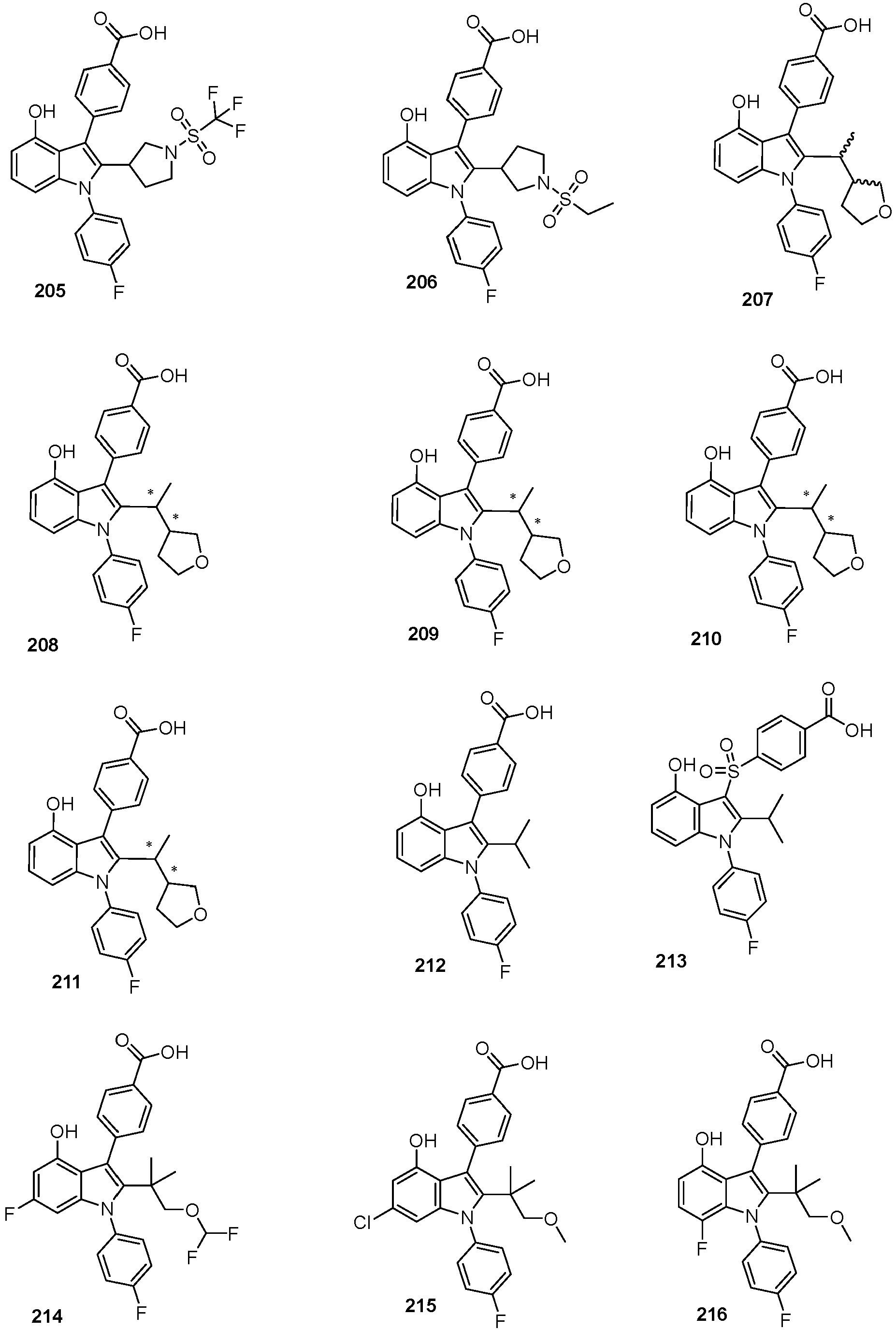

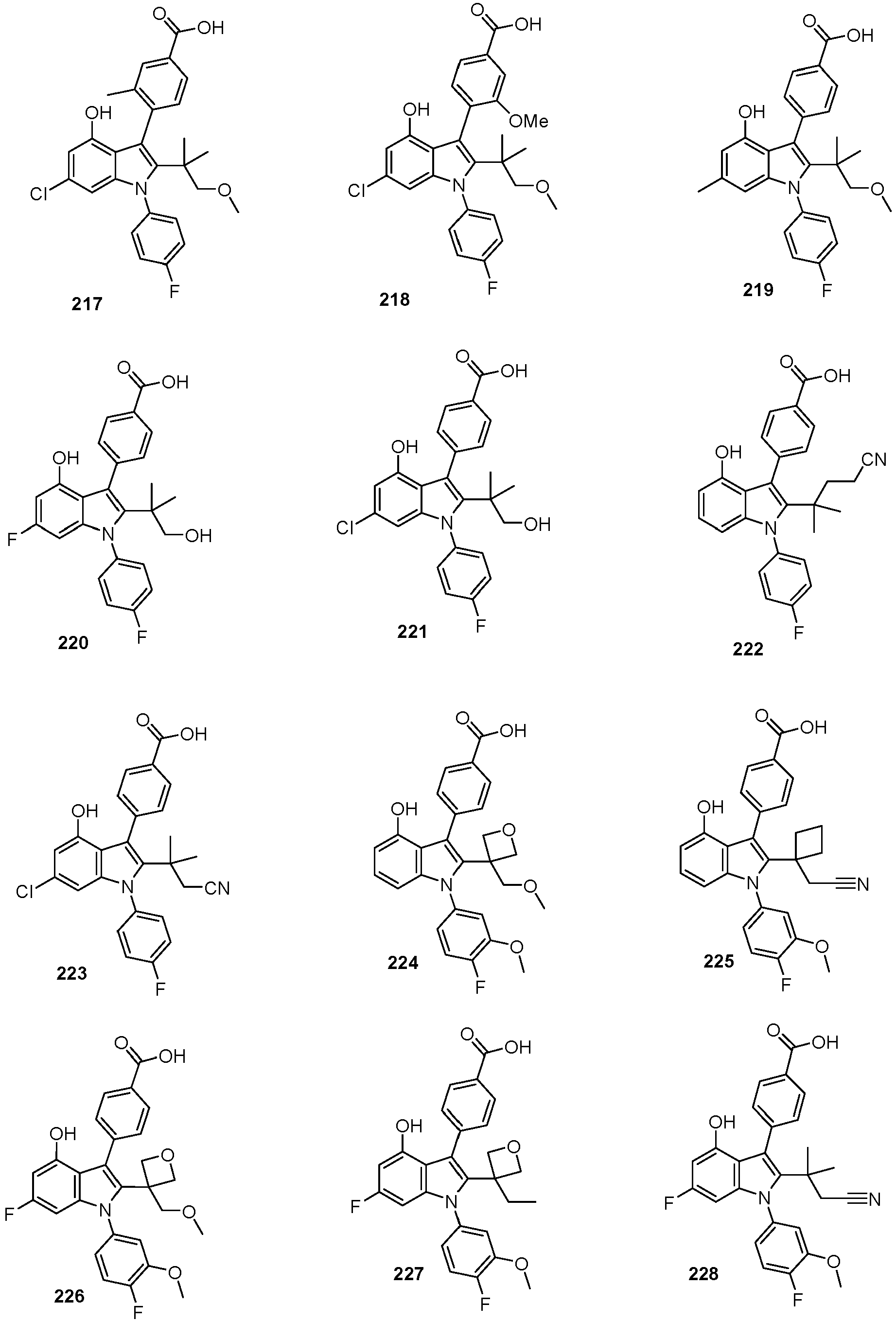

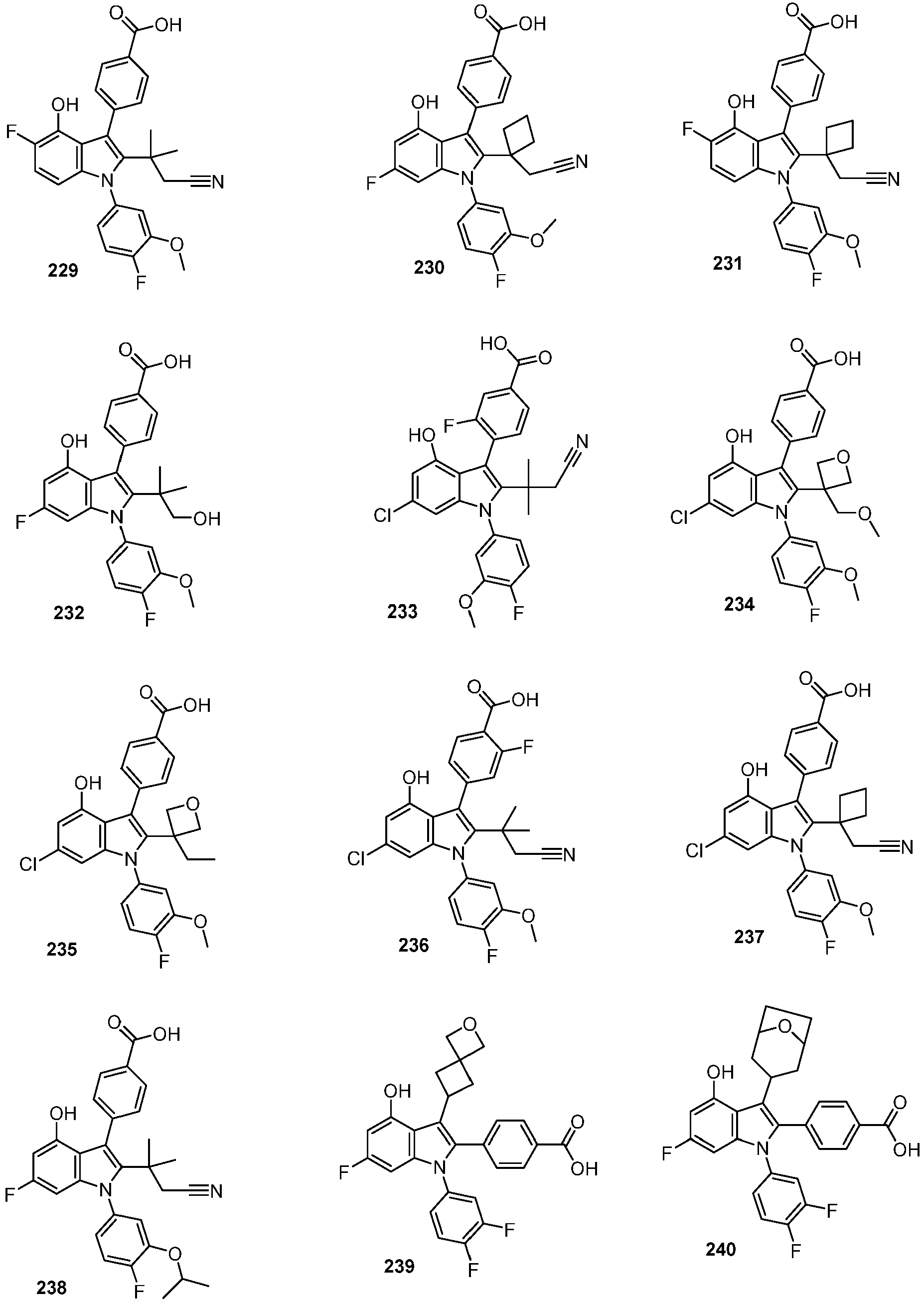

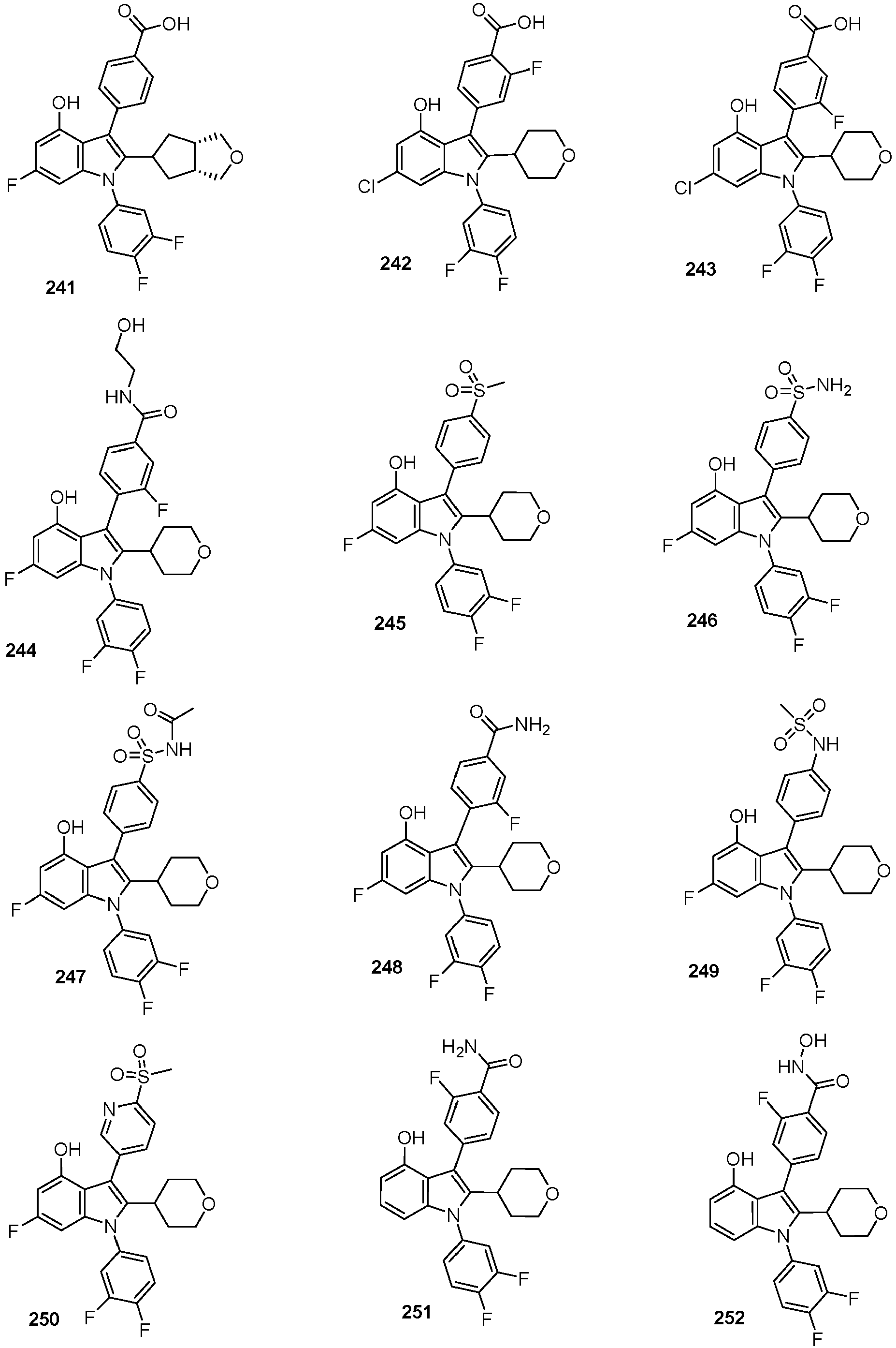

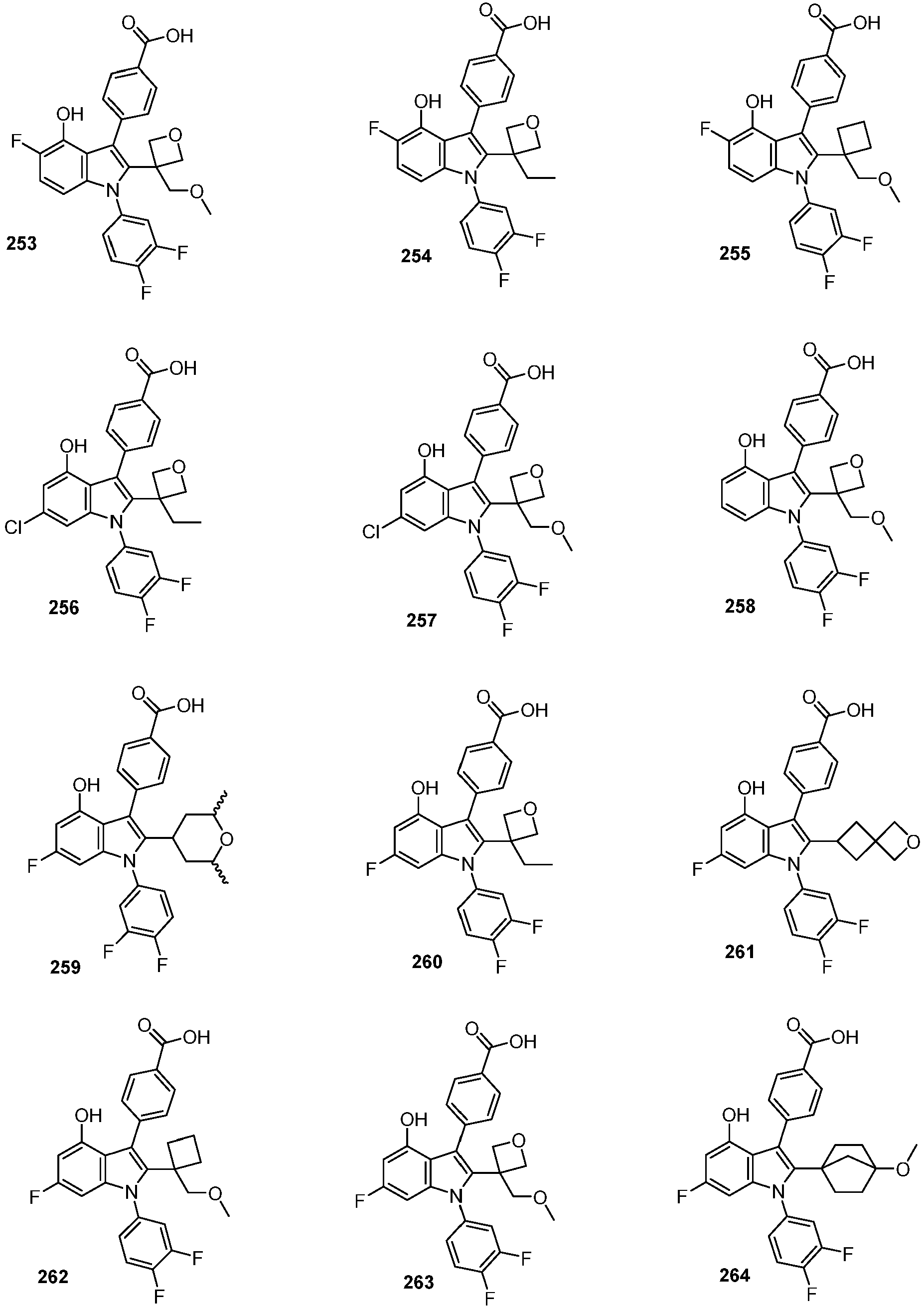

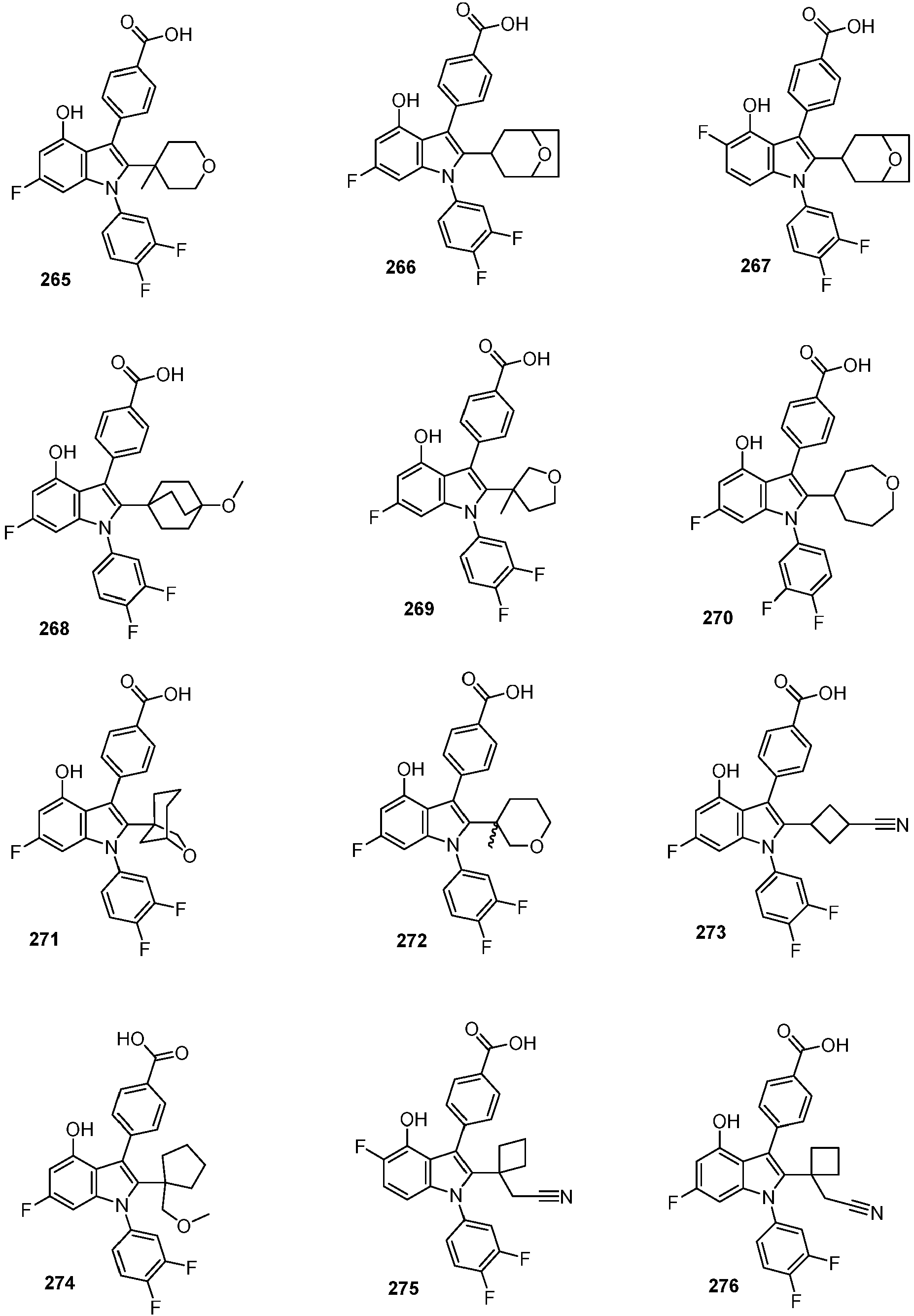

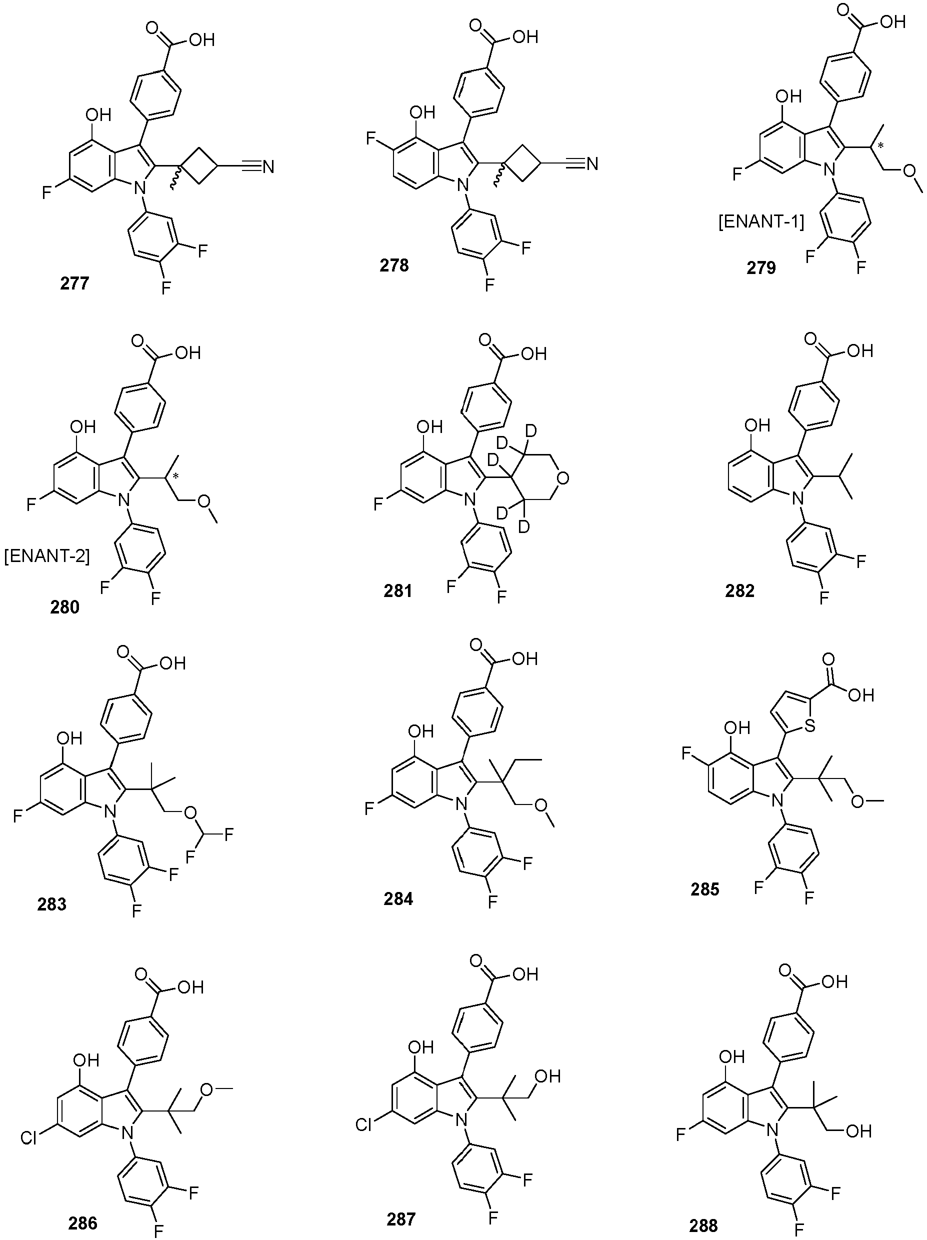

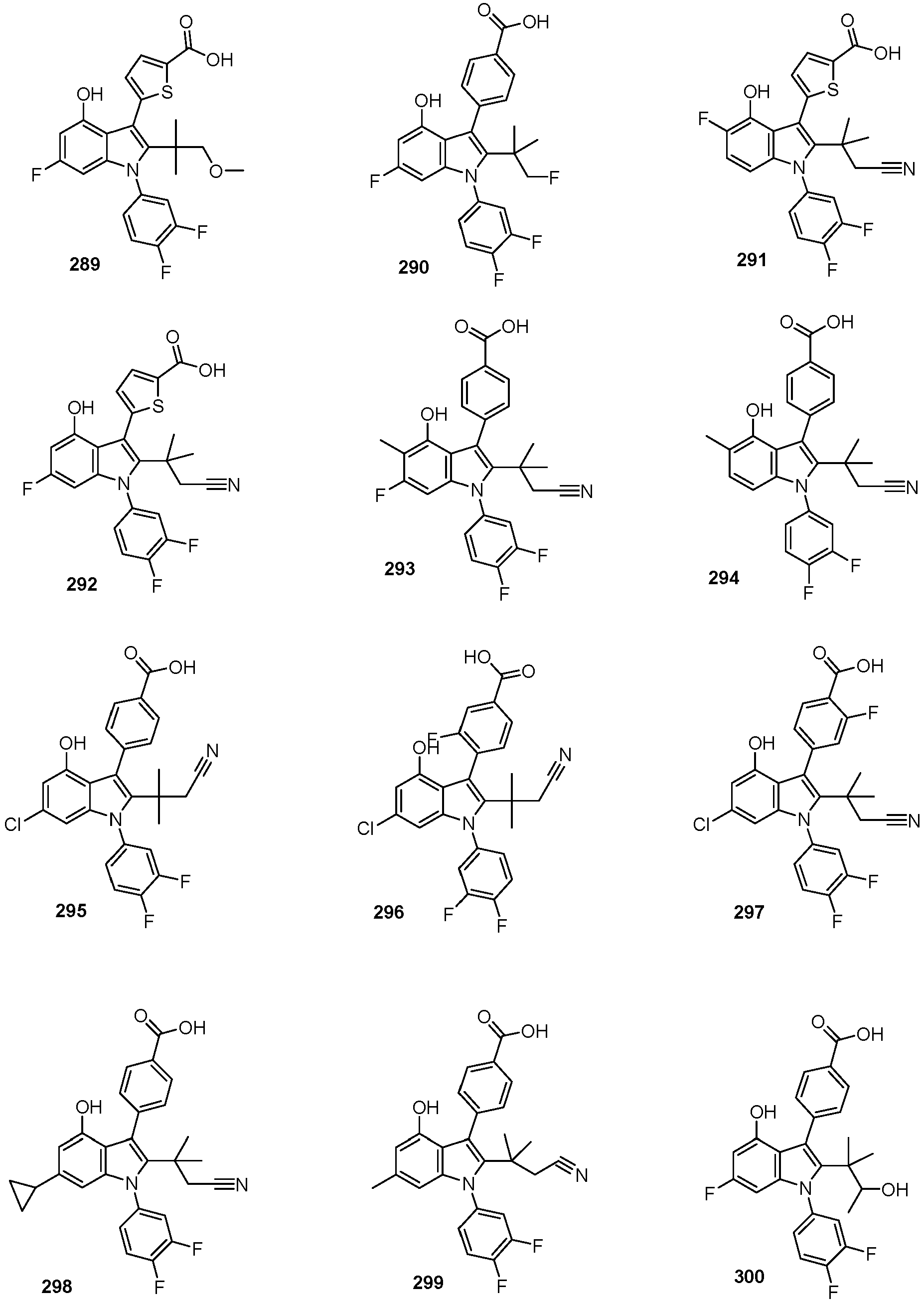

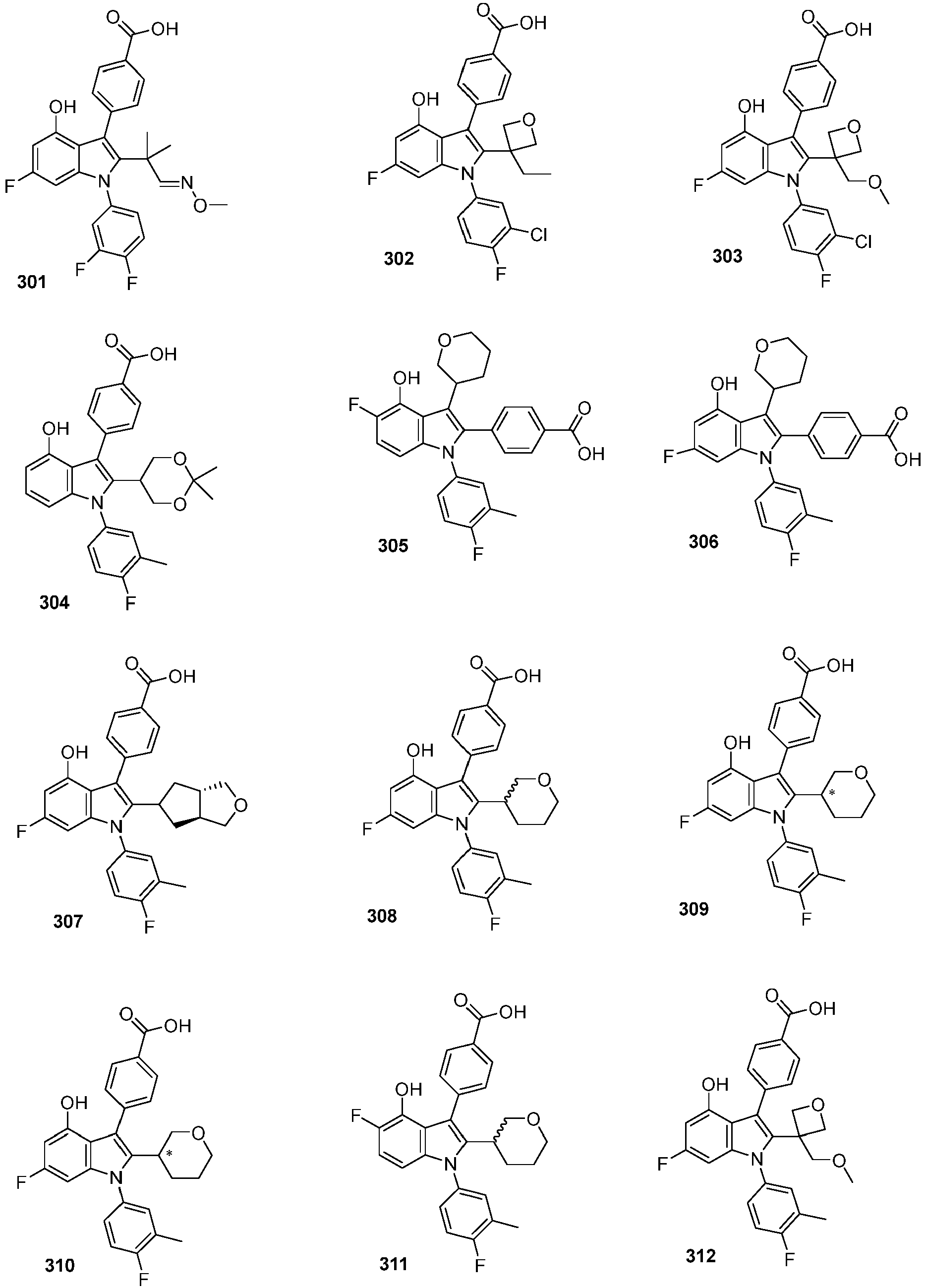

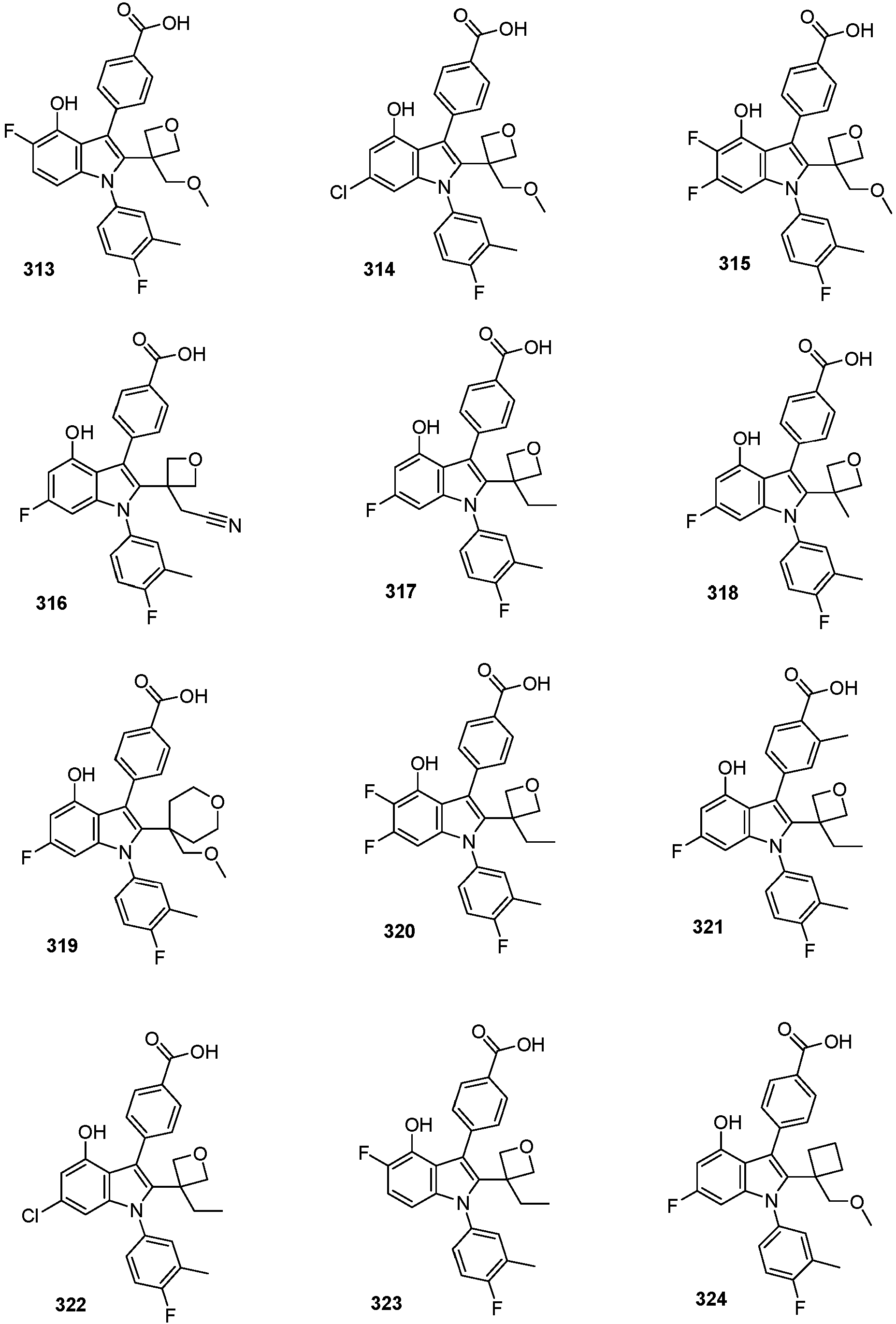

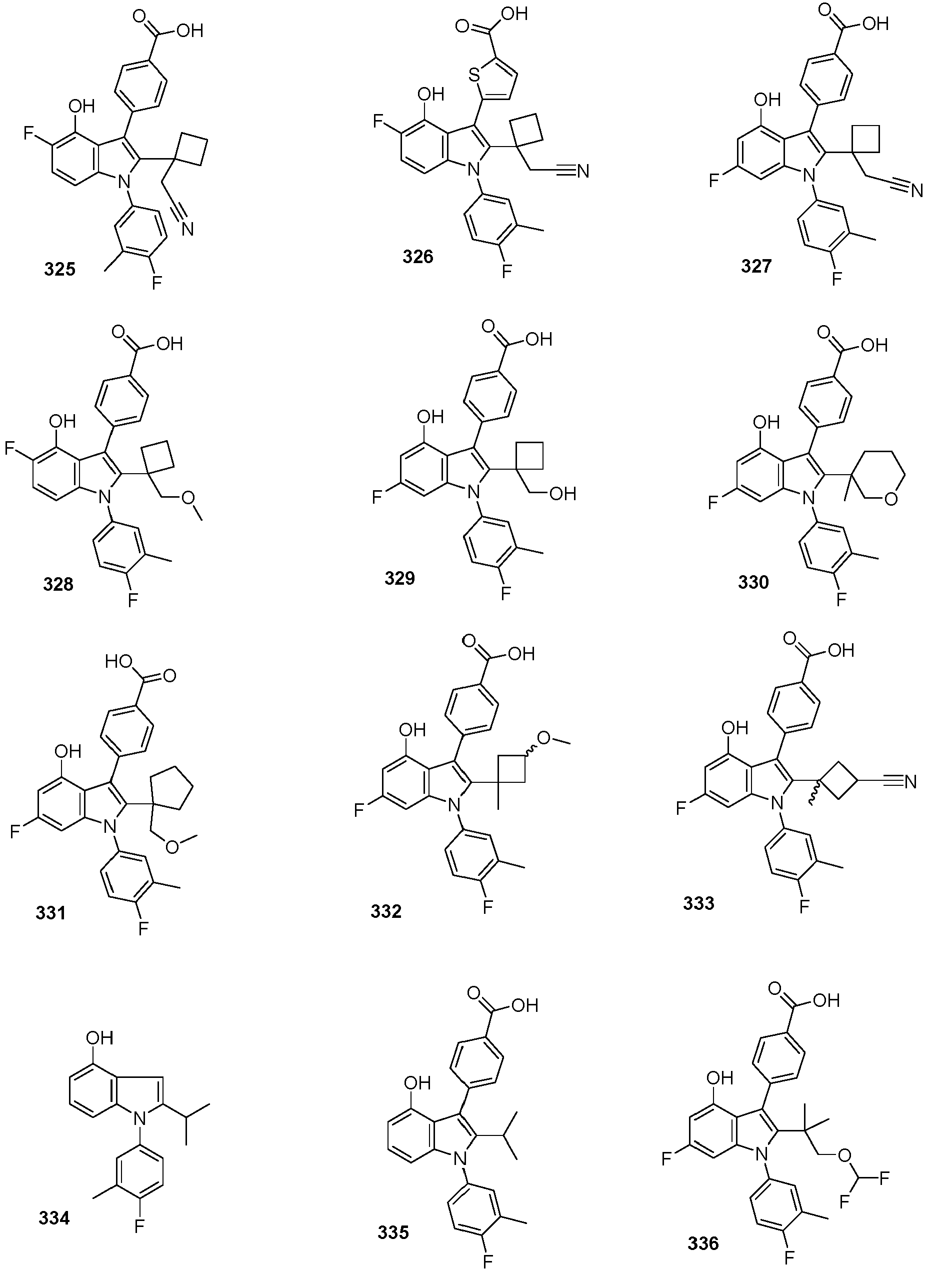

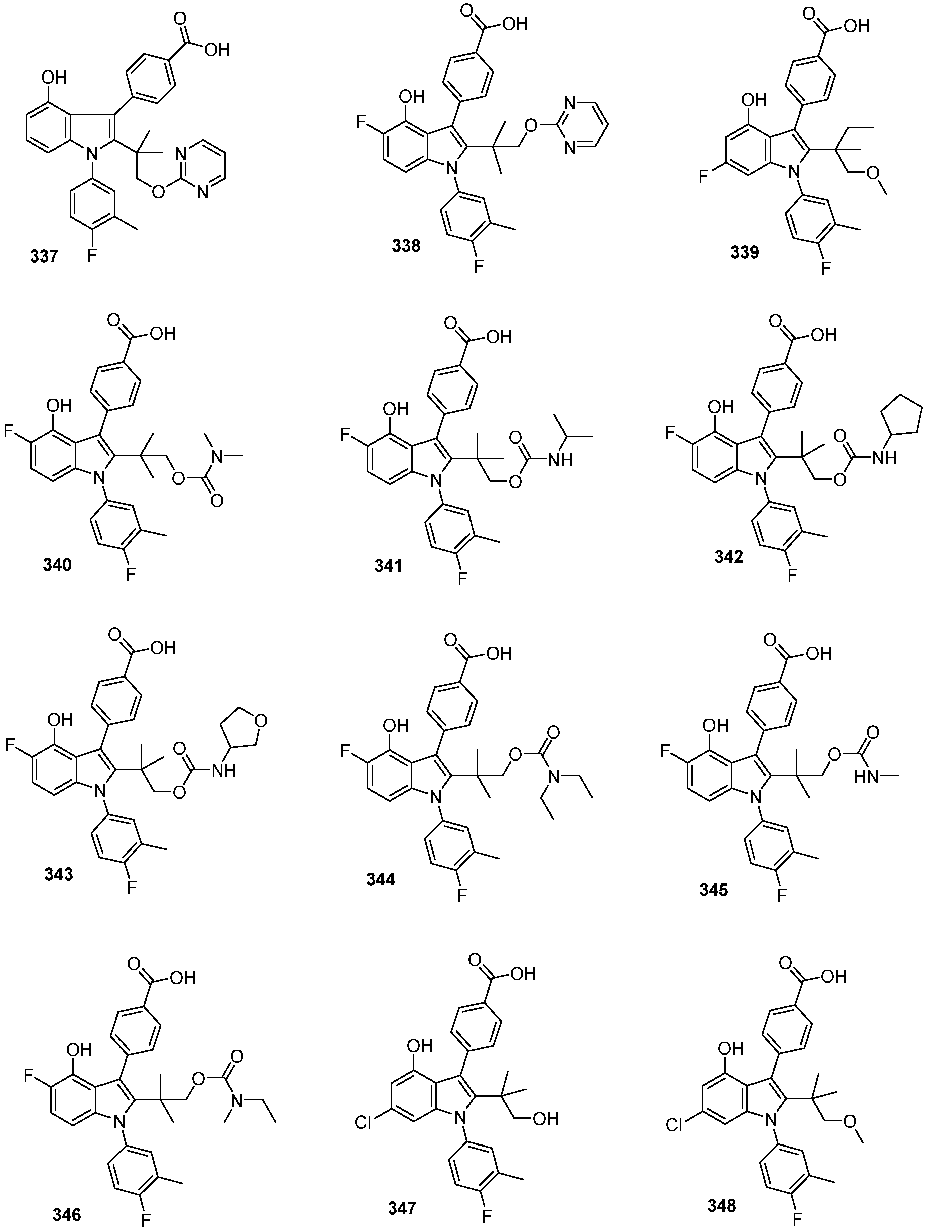

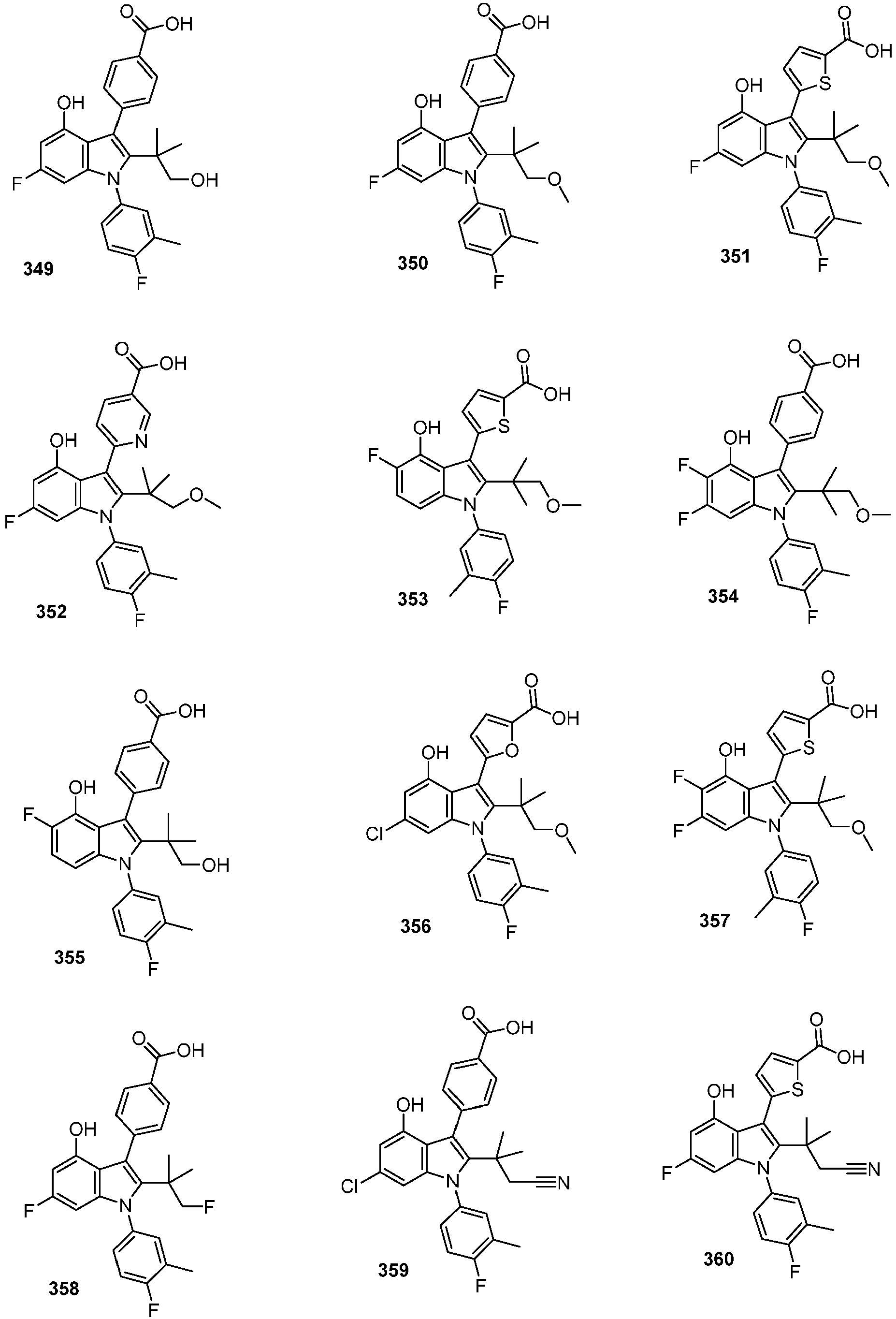

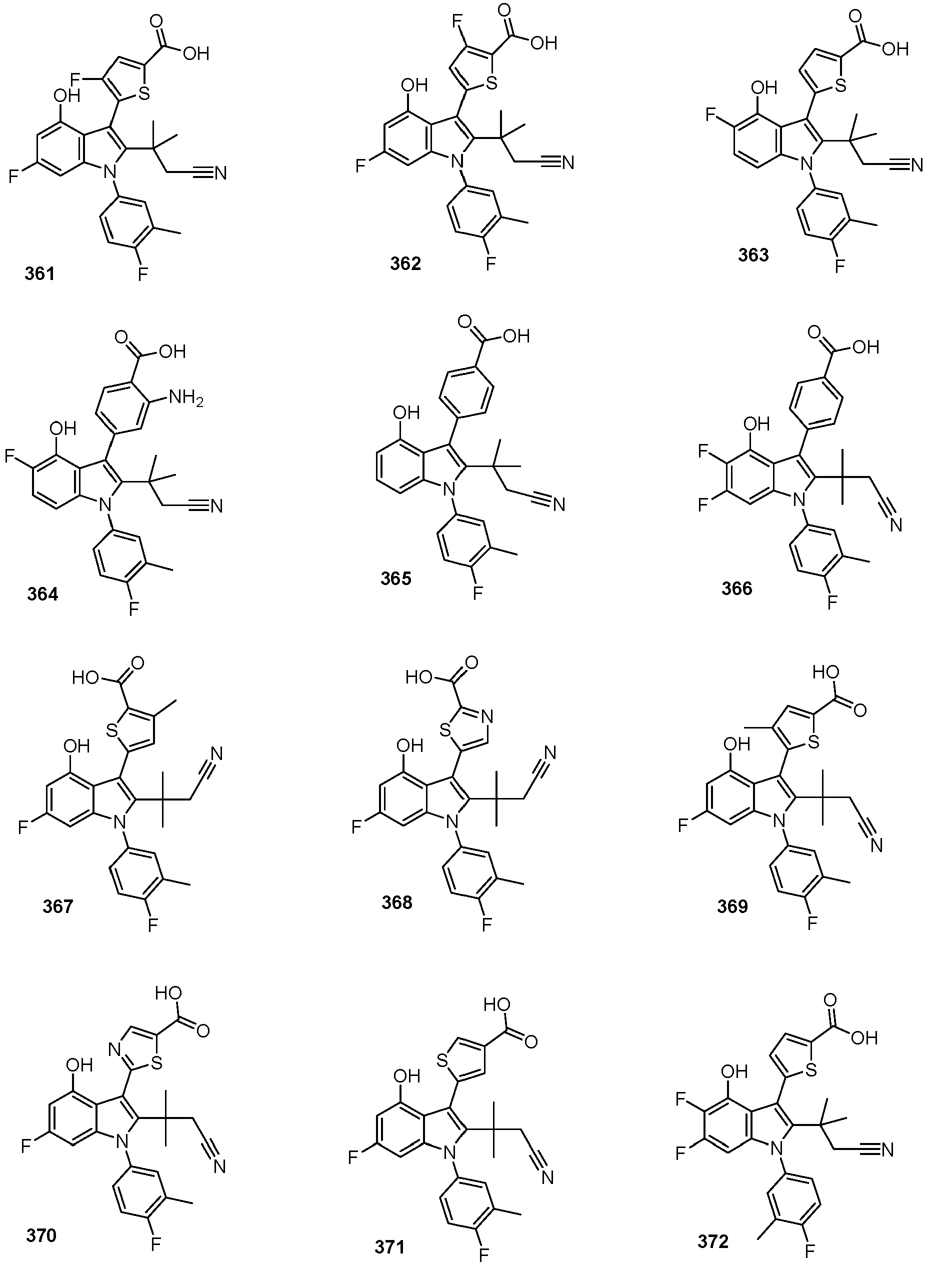

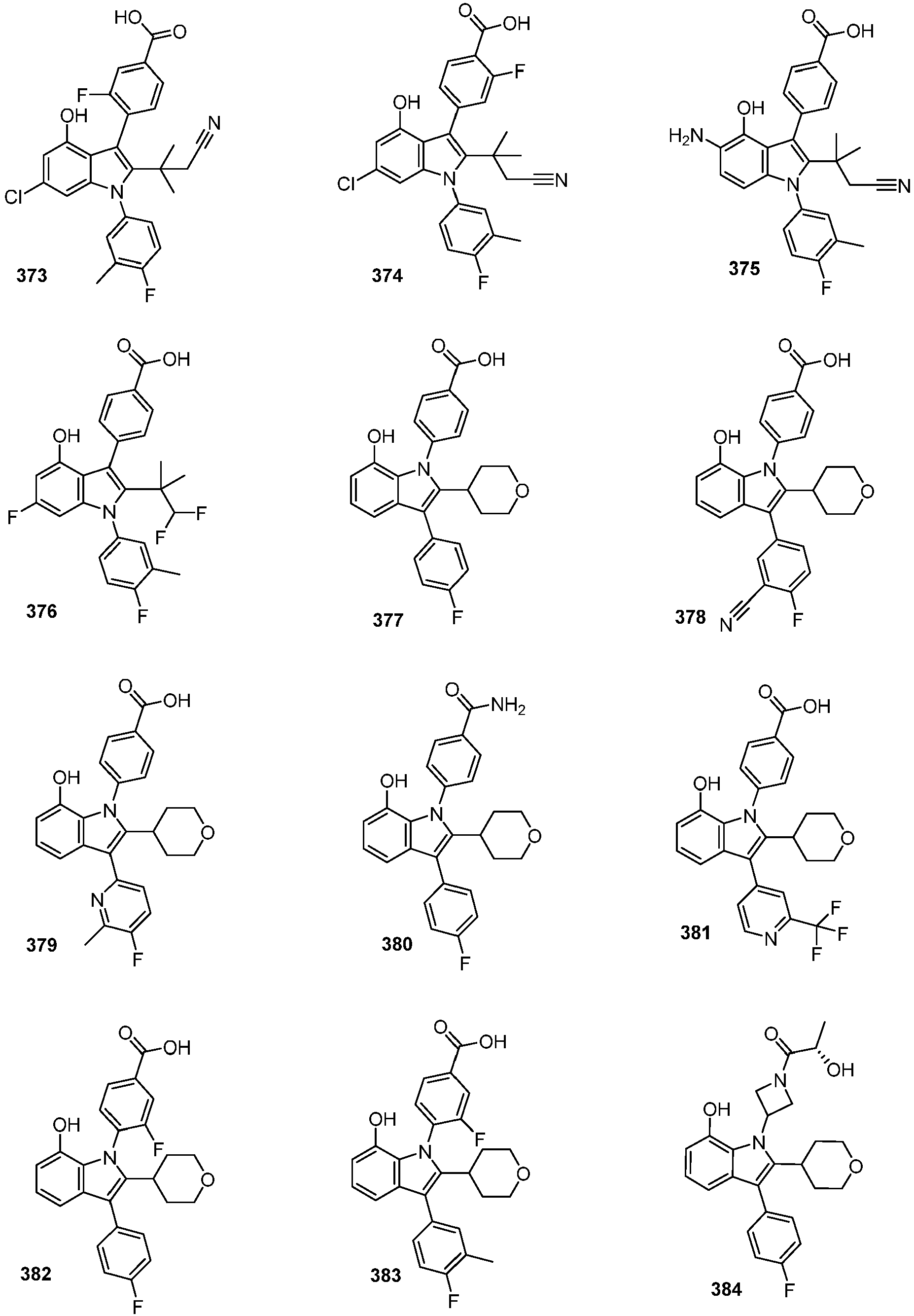

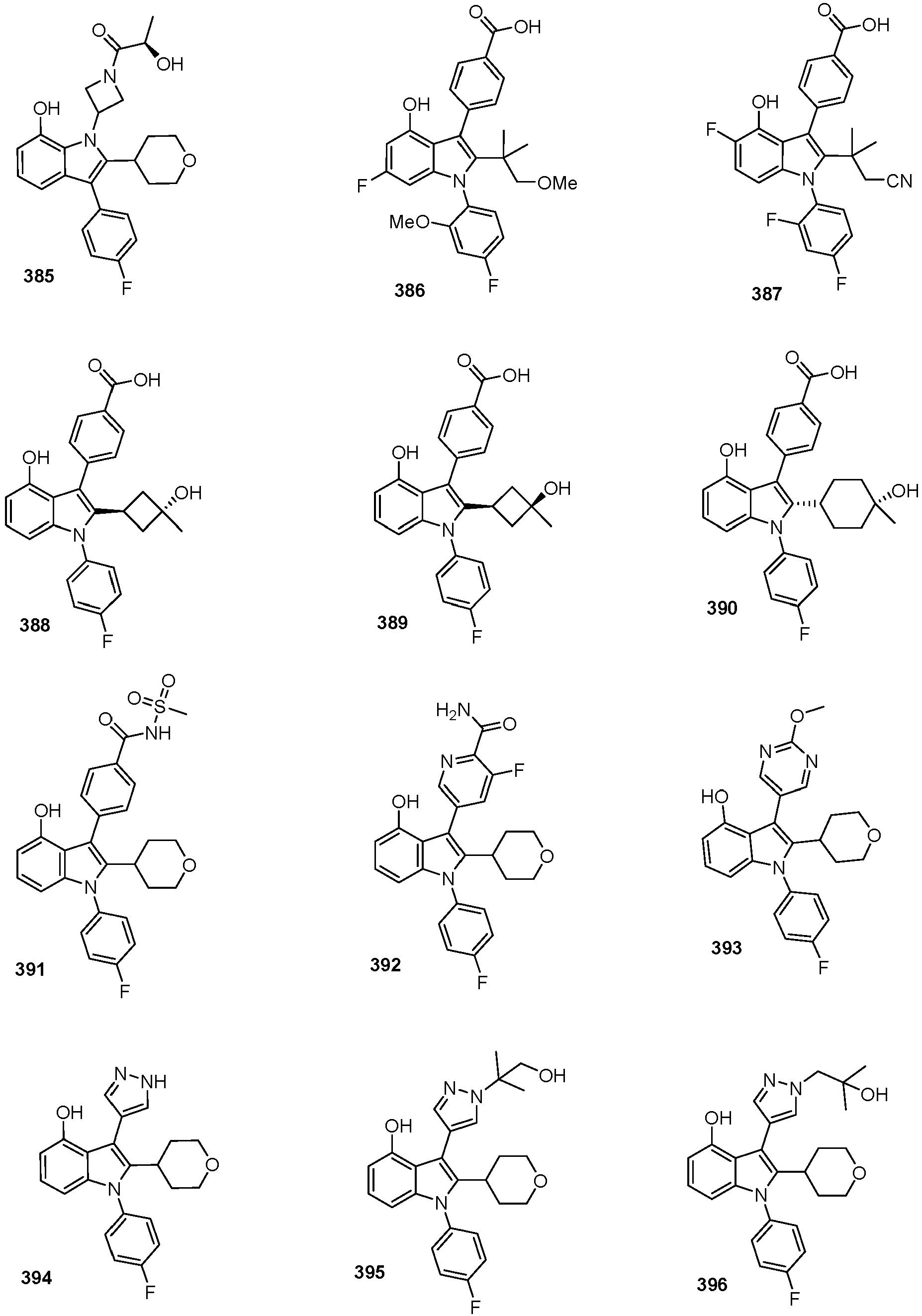

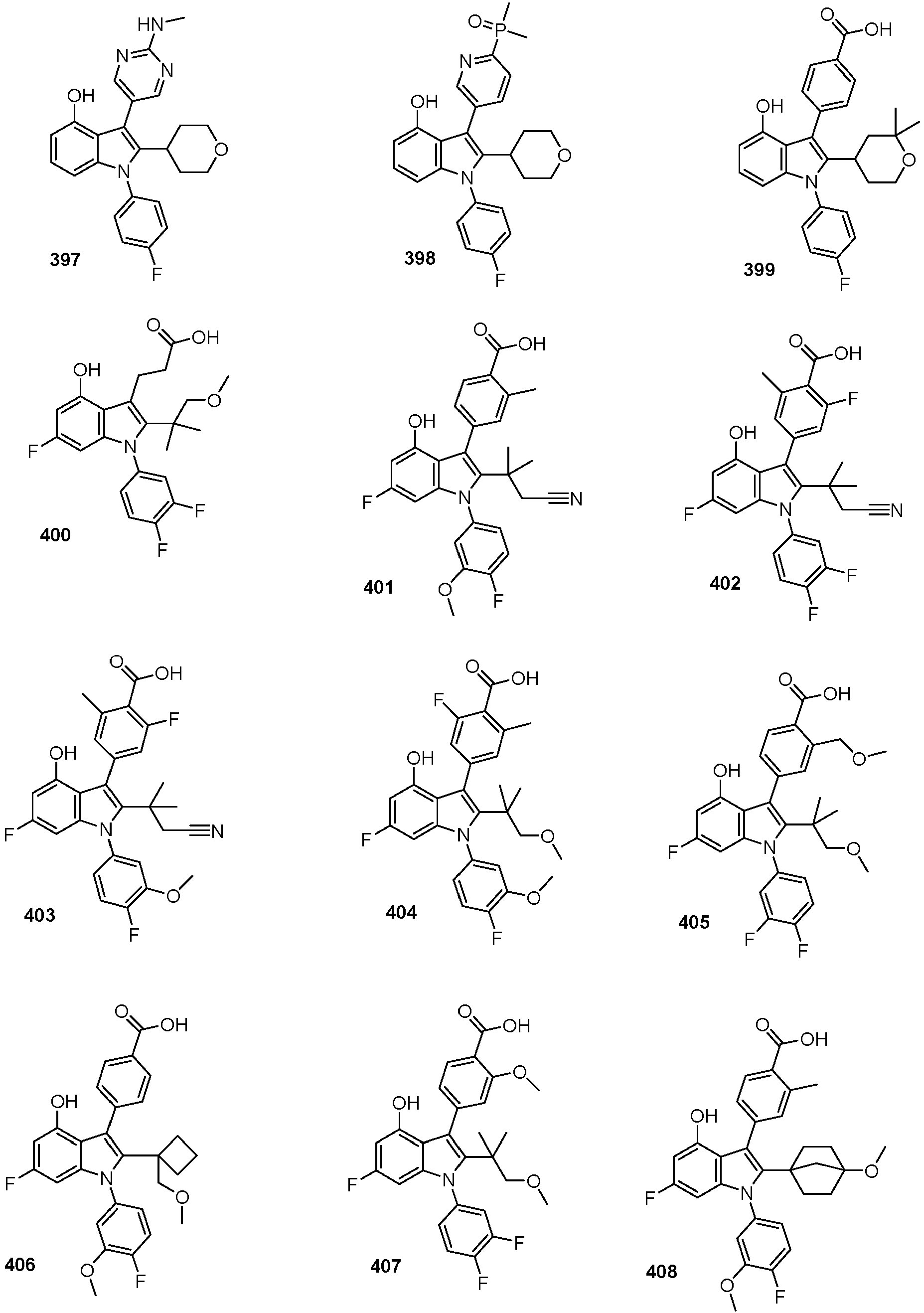

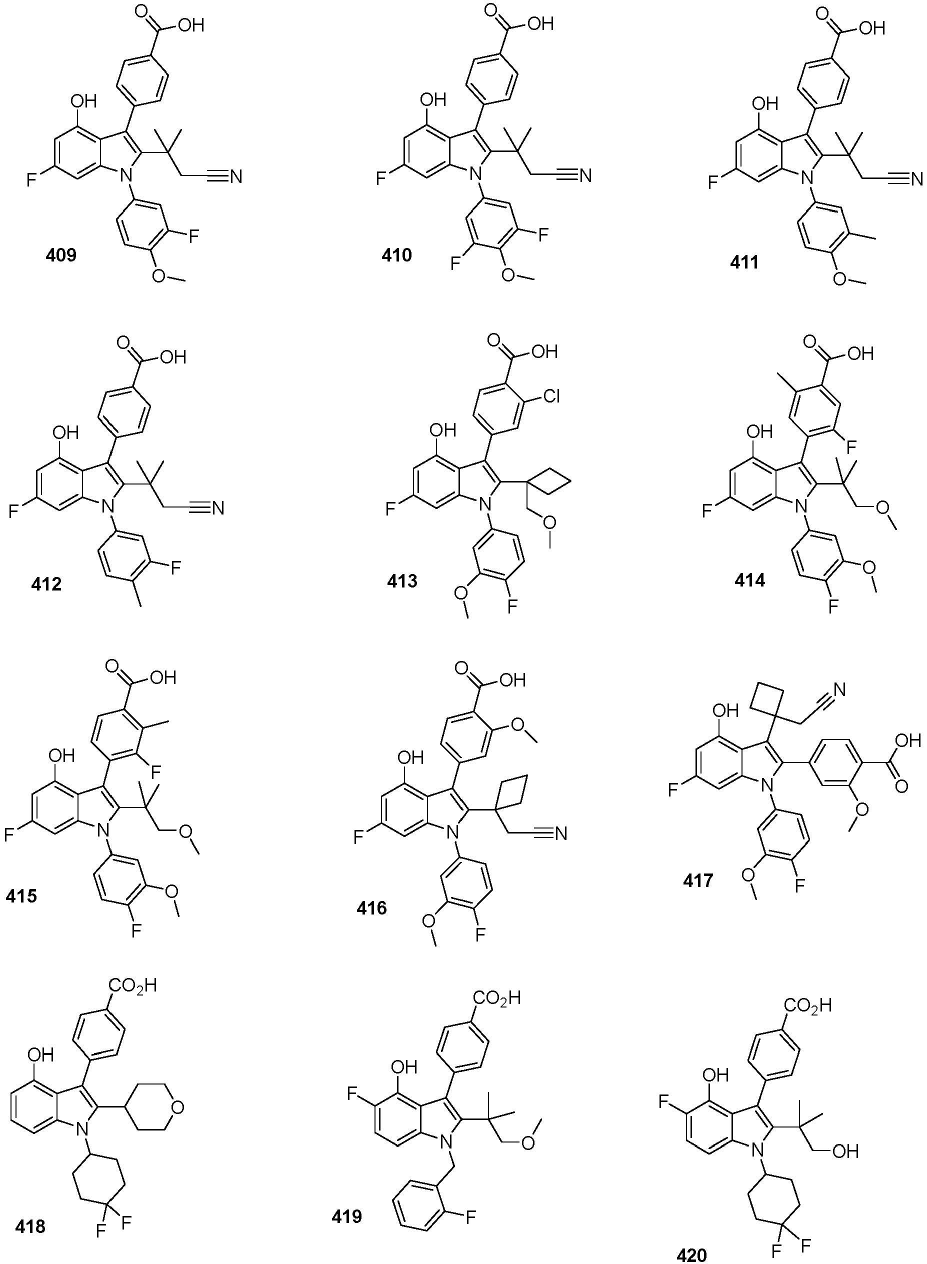

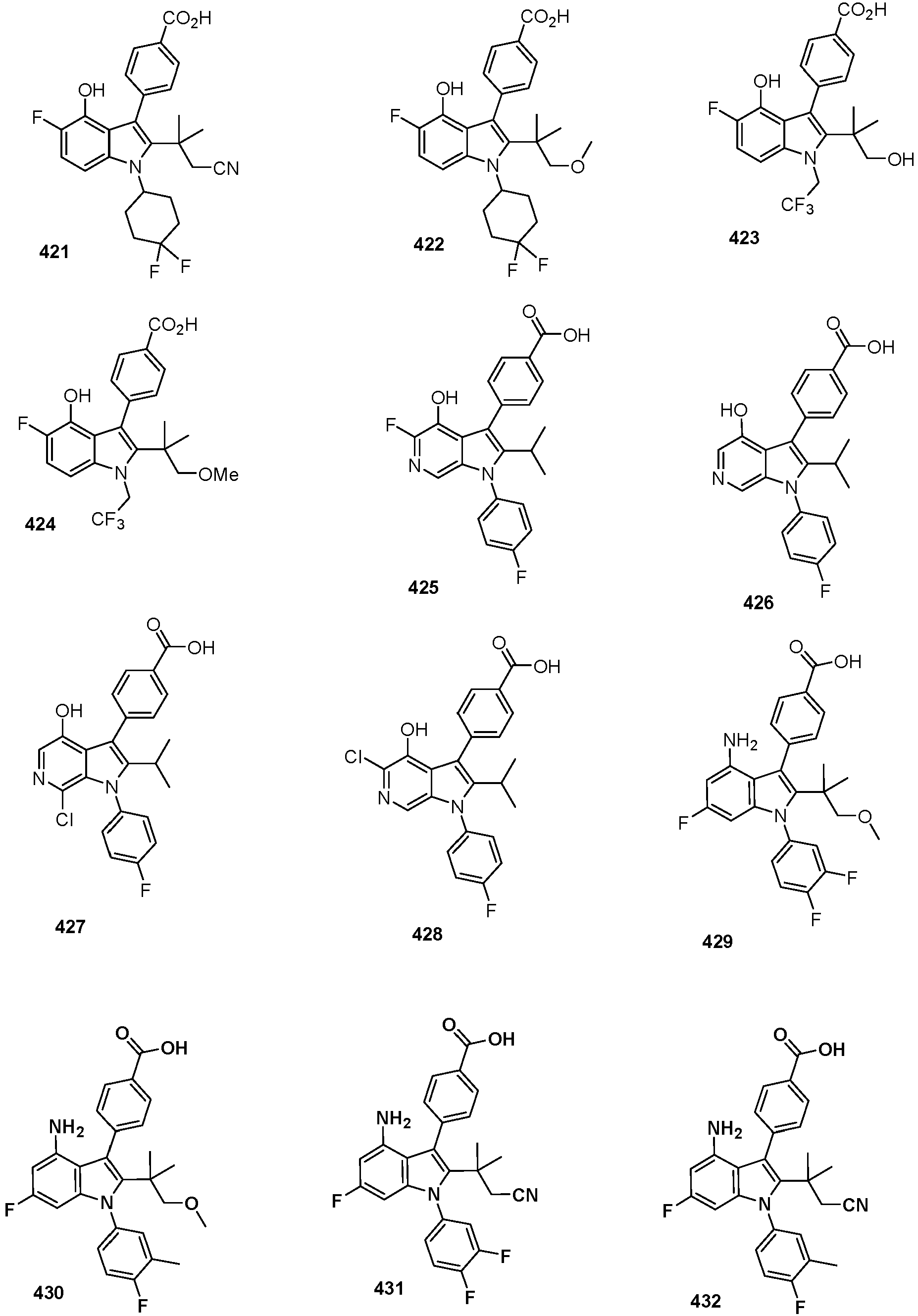

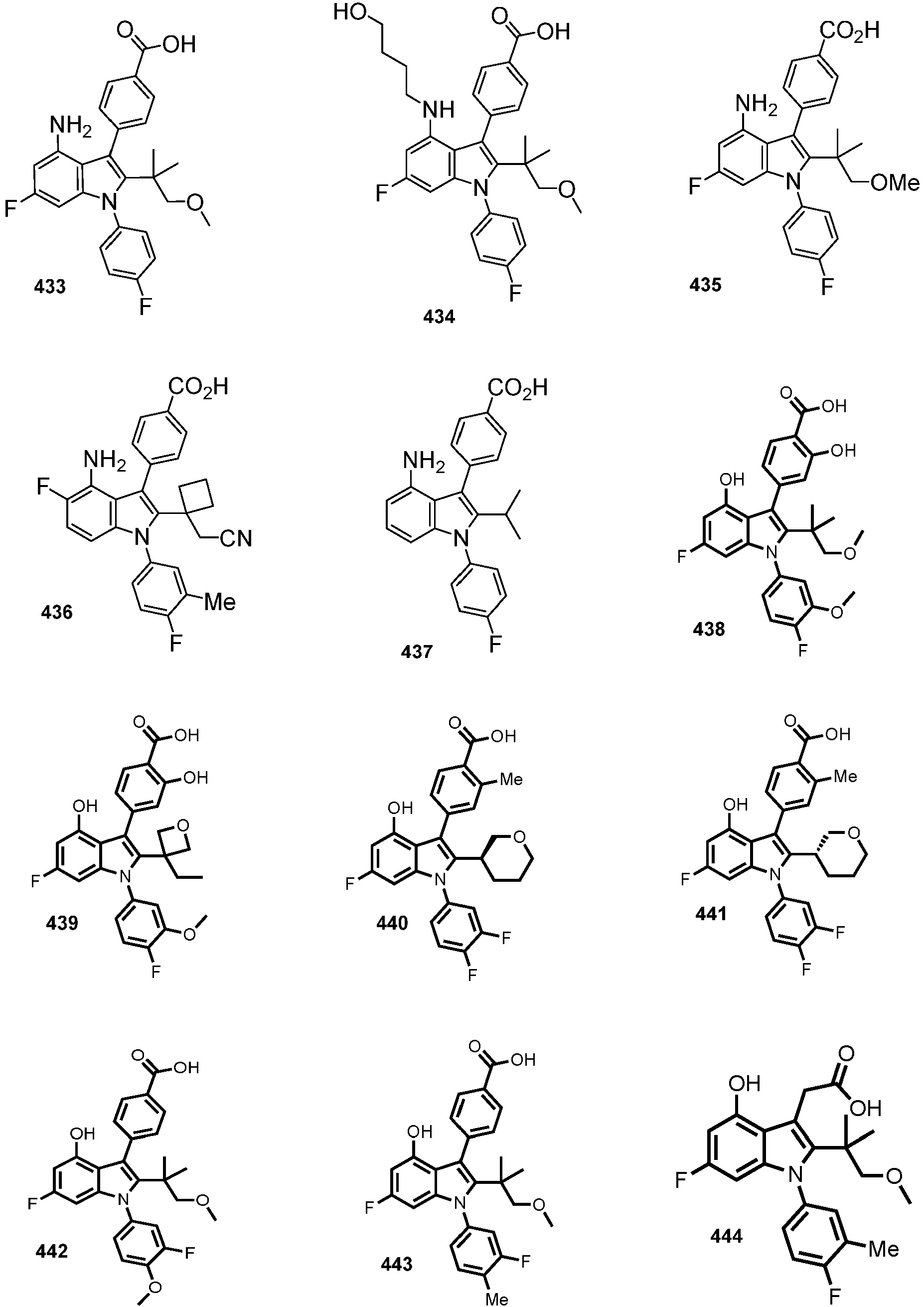

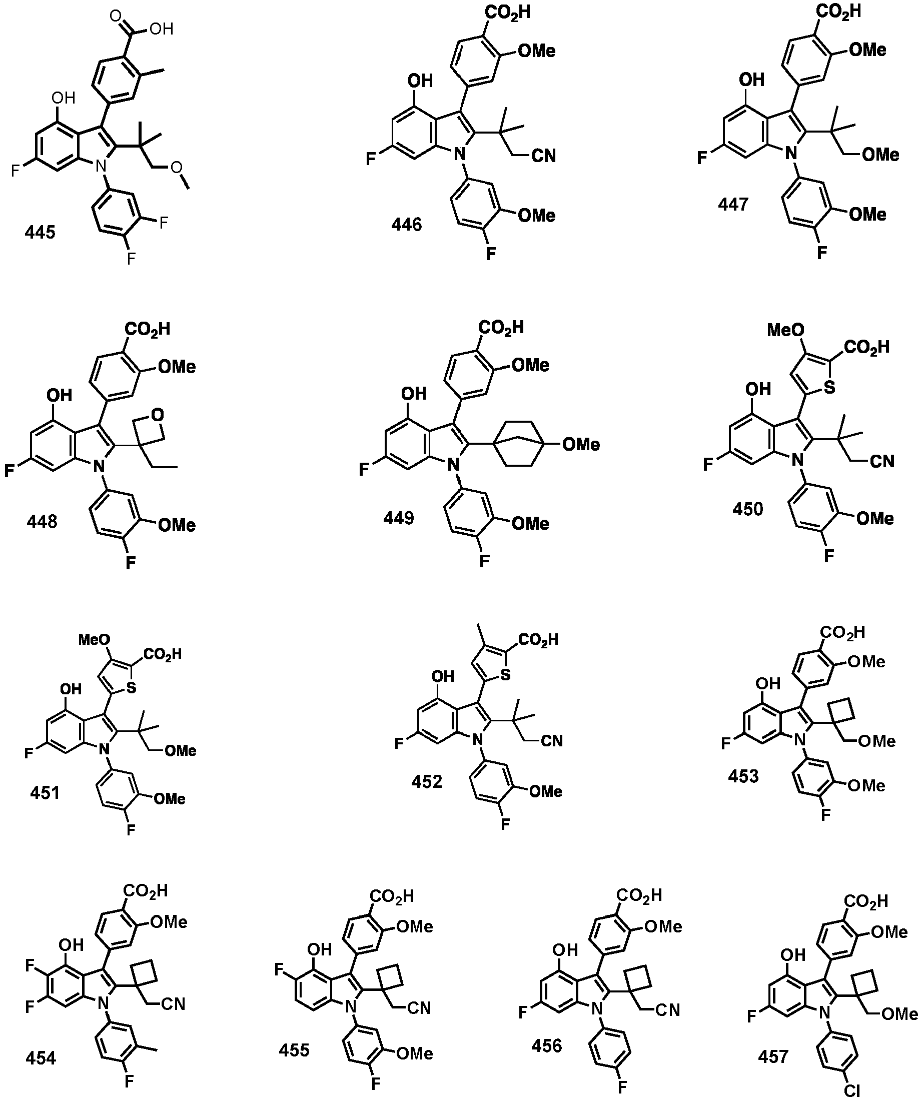

**Table B. Compounds 458-532**

| | | |
|---|---|---|
| Compound 458 | Compound 459 | Compound 460 |
| 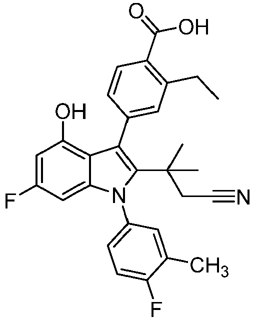 | 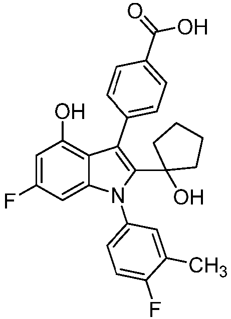 | 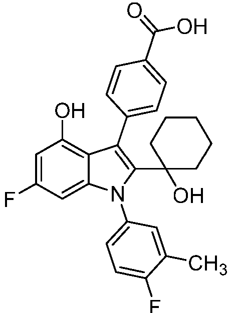 |
| Compound 461 | Compound 462 | Compound 463 |
| 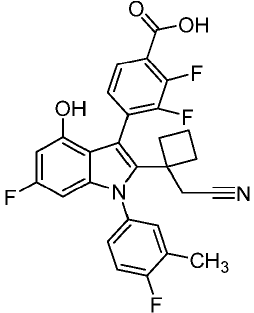 | 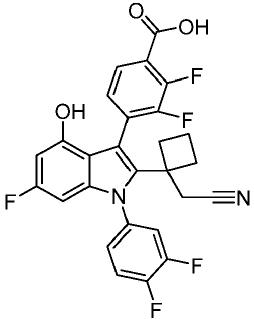 | 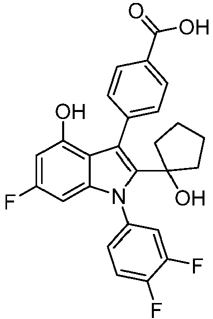 |
| Compound 464 | Compound 465 | Compound 466 |
| 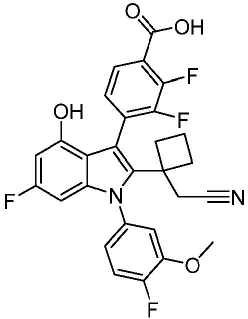 | 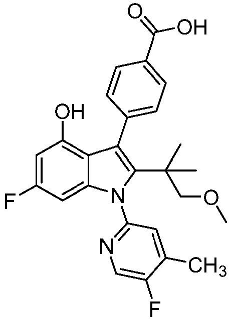 | 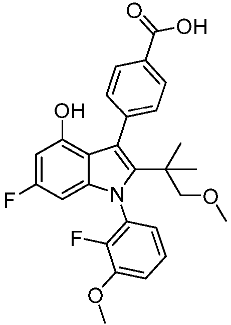 |
| Compound 467 | Compound 468 | Compound 469 |
| 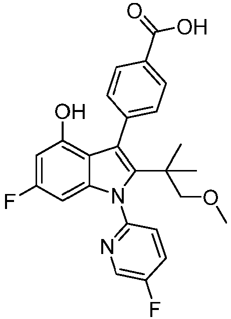 | 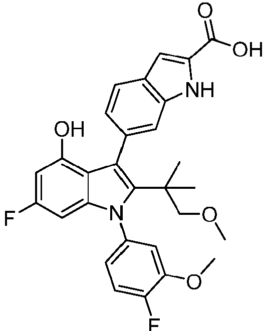 | 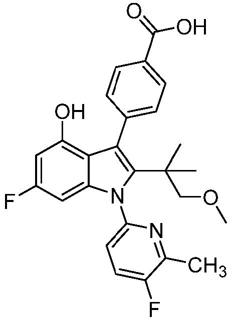 |
| Compound 470 | Compound 471 | Compound 472 |
| 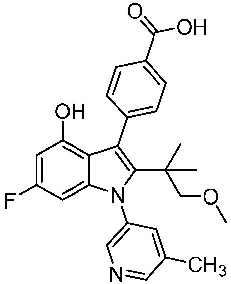 | 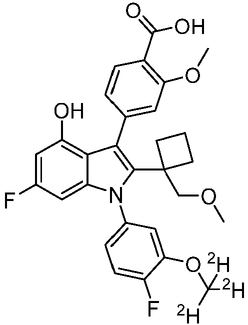 | 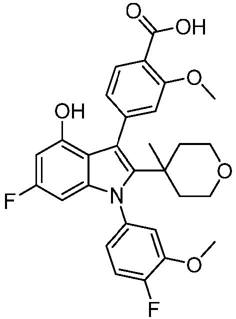 |
| Cornnound 473 | Compound 474 | Compound 475 |
| 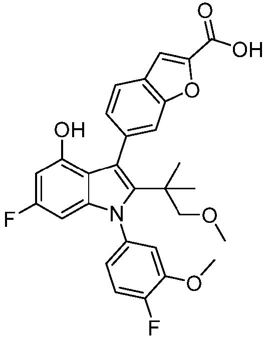 | 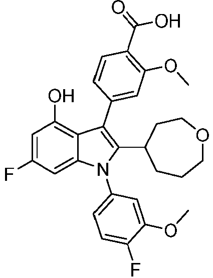 | 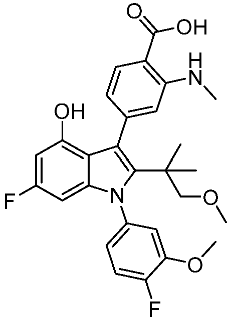 |
| Compound 476 | Compound 477 | Compound 478 |
| 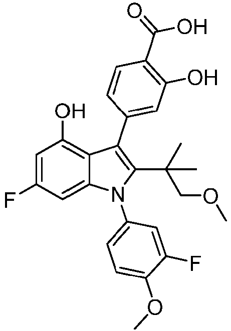 | 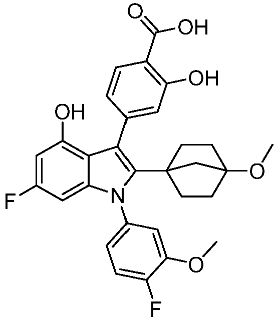 | 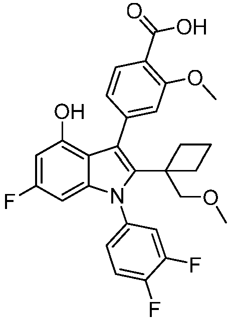 |
| Compound 479 | Compound 480 | Compound 481 |
| 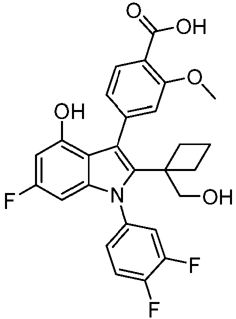 | 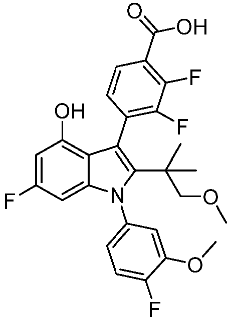 | 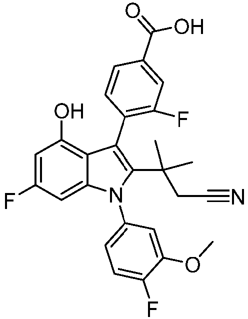 |
| Compound 482 | Compound 483 | Compound 484 |
| 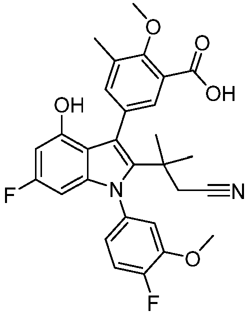 | 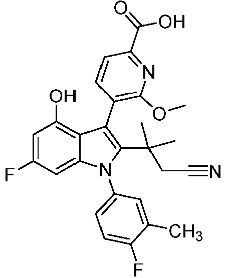 | 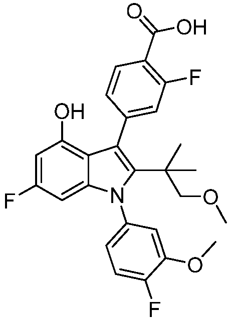 |
| Compound 485 | Compound 486 | Compound 487 |
| 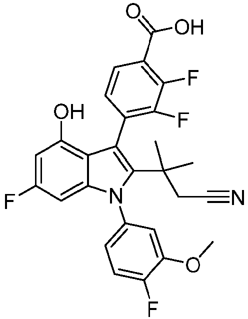 | 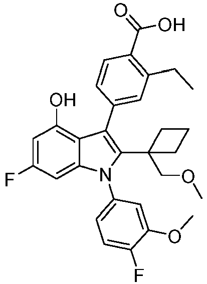 | 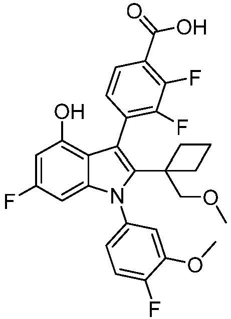 |
| Compound 488 | Compound 489 | Compound 490 |
| 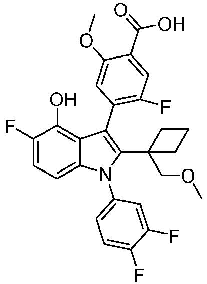 | 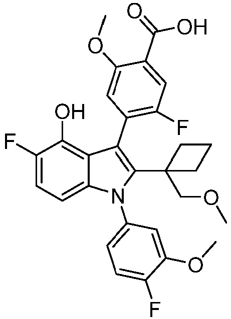 | 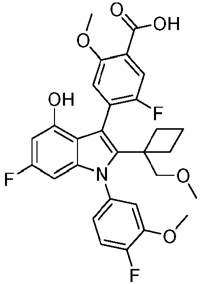 |
| Compound 491 | Compound 492 | Compound 493 |
| 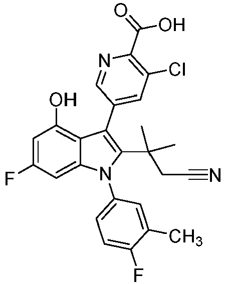 | 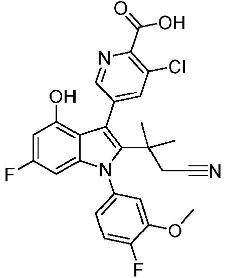 | 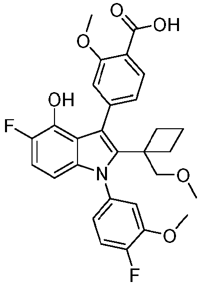 |
| Compound 494 | Compound 495 | Compound 496 |
| 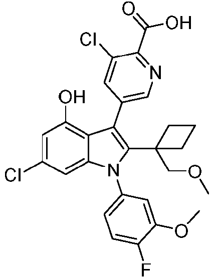 | 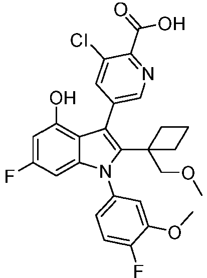 | 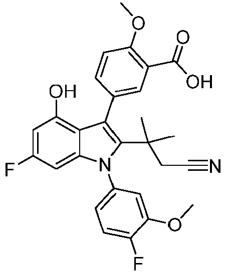 |
| Compound 497 | Compound 498 | Compound 499 |
| 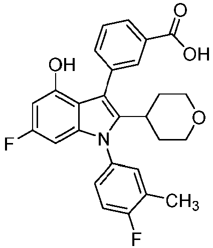 | 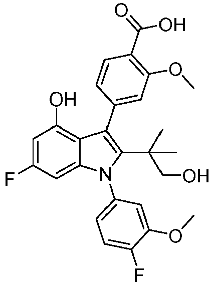 | 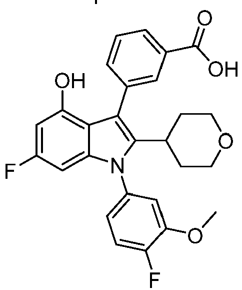 |
| Compound 500 | Compound 501 | Compound 502 |
| 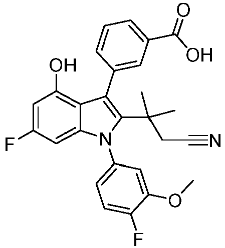 | 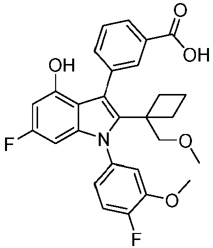 | 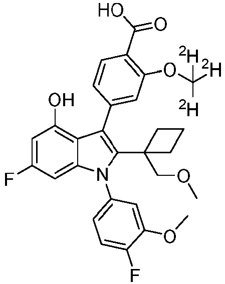 |
| Compound 503 | Compound 504 | Compound 505 |
| 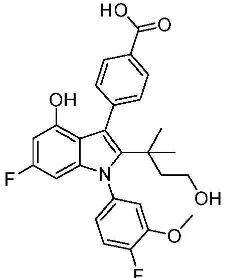 | 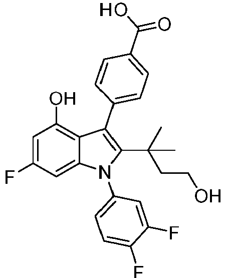 | 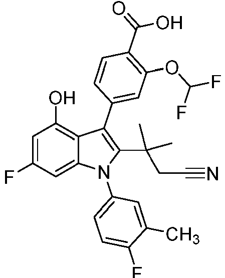 |
| Compound 506 | Compound 507 | Compound 508 |
| 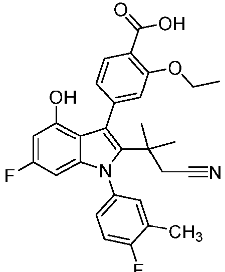 | 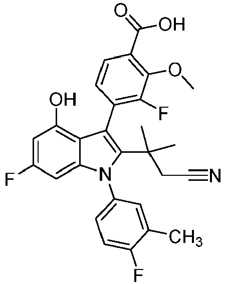 | 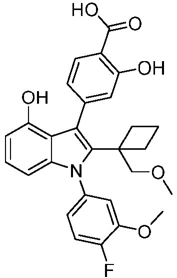 |
| Compound 509 | Compound 510 | Compound 511 |
| 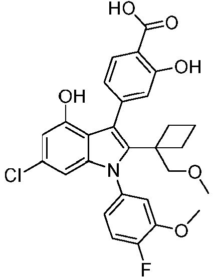 | 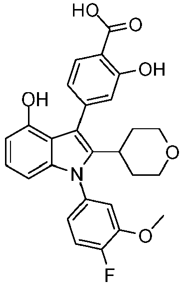 | 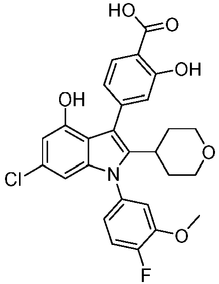 |
| Compound 512 | Compound 513 | Compound 514 |
| 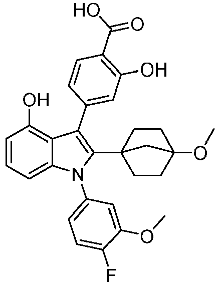 | 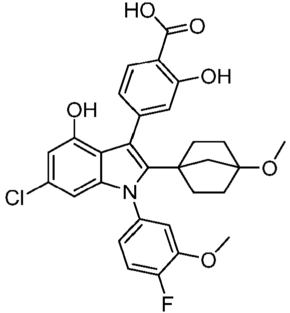 | 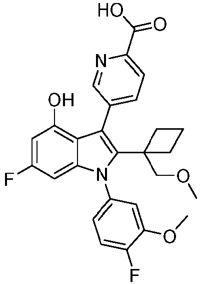 |
| Compound 515 | Compound 516 | Compound 517 |
| 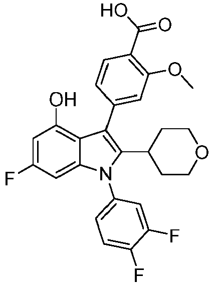 | 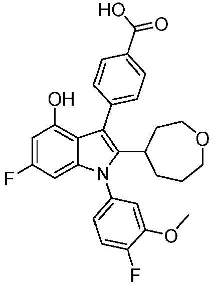 | 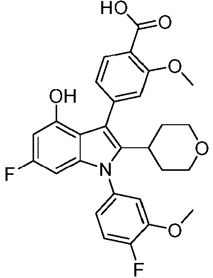 |
| Compound 518 | Compound 519 | Compound 520 |
| 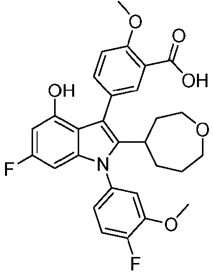 | 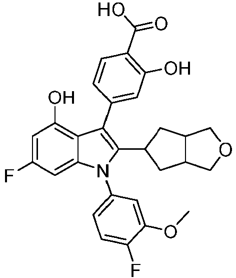 | 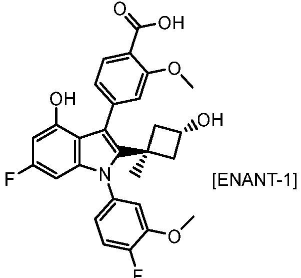 |
| Compound 521 | Compound 522 | Compound 523 |
| 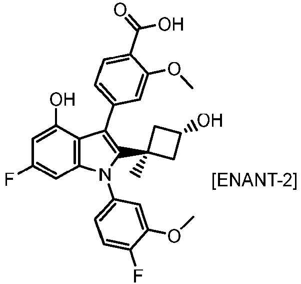 | 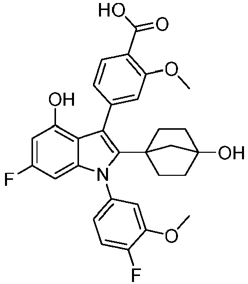 | 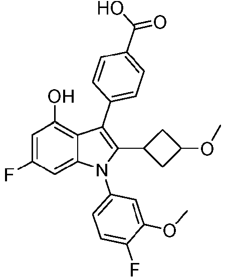 |
| Compound 524 | Compound 525 | Compound 526 |
| 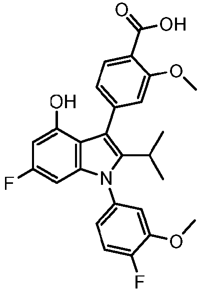 | 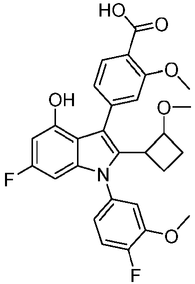 | 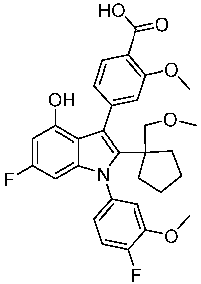 |
| Compound 527 | Compound 528 | Compound 529 |
| 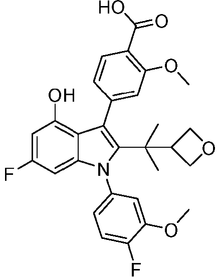 | 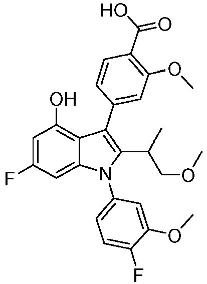 | 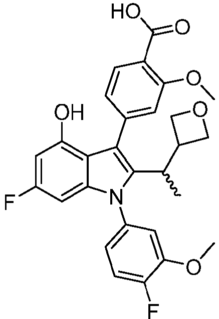 |
| Compound 530 | Compound 531 | Compound 532 |
| 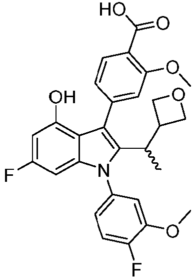 | 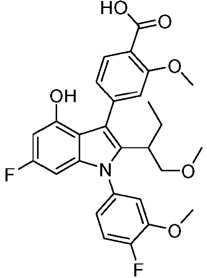 | 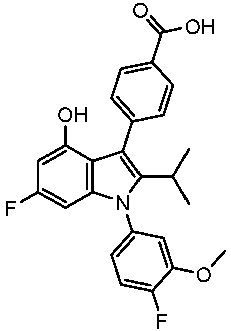 |

**Table C. Compounds B1-B25**

| | | |
|---|---|---|
| Compound B1 | Compound B2 | Compound B3 |
| 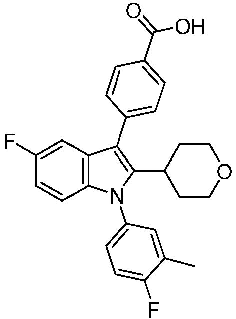 | 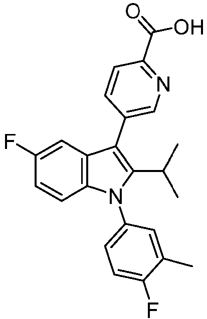 | 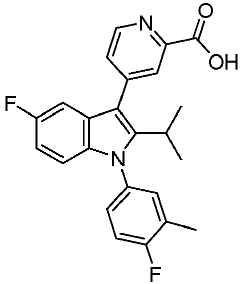 |
| Compound B4 | Compound B5 | Compound B6 |
| 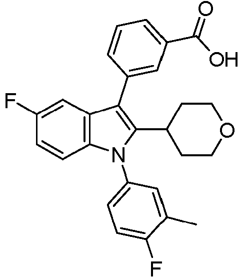 | 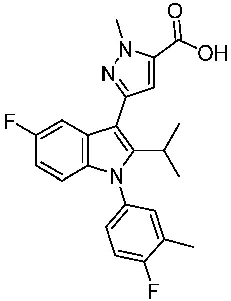 | 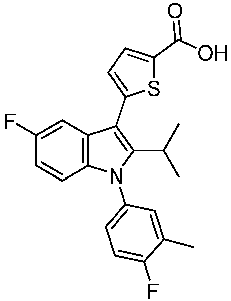 |
| Compound B7 | Compound B8 | Compound B9 |
| 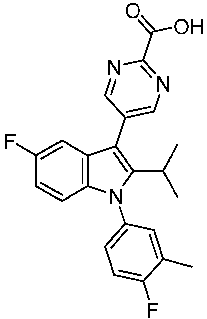 | 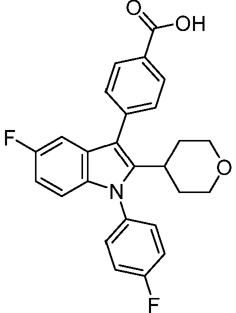 | 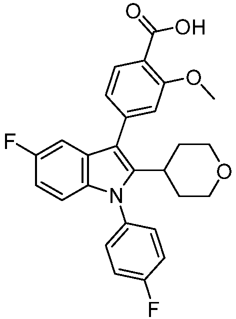 |
| Compound B10 | Compound B11 | Compound B12 |
| 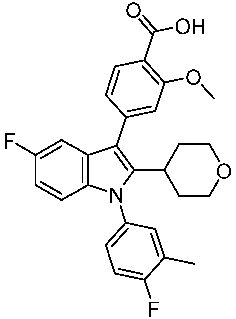 | 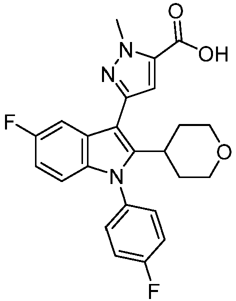 | 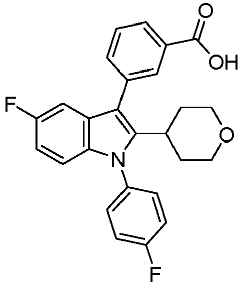 |
| Compound B13 | Compound B14 | Compound B15 |
| 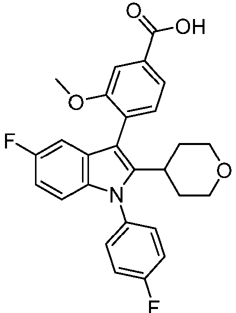 | 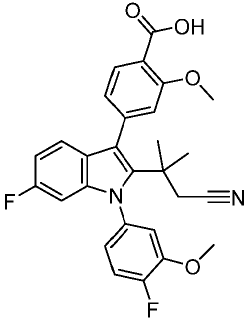 | 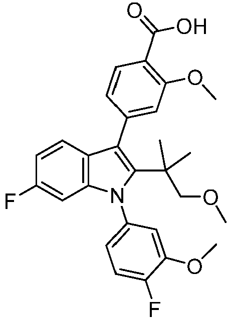 |
| Compound B16 | Compound B17 | Compound B18 |
| 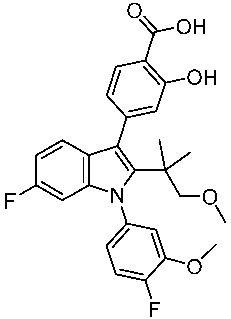 | 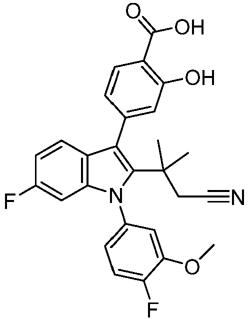 | 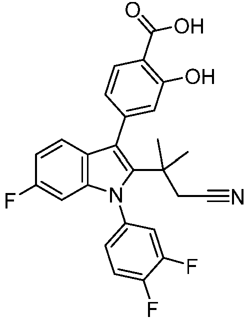 |
| Compound B19 | Compound B20 | Compound B21 |
| 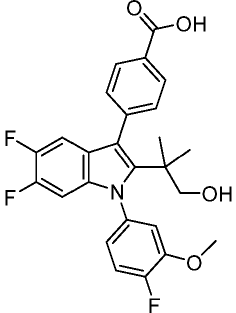 | 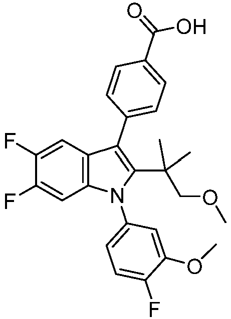 | 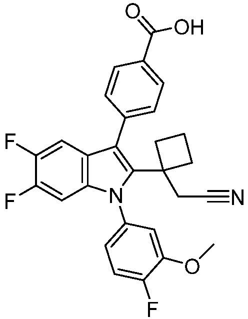 |
| Compound B22 | Compound B23 | Compound B24 |
| 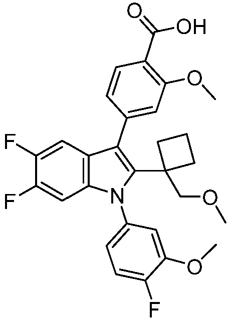 | 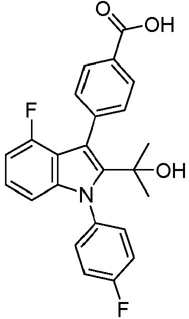 | 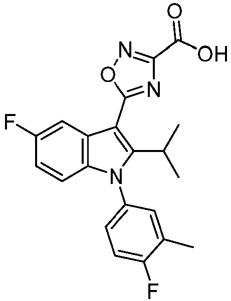 |
| Compound B25 | | |
| 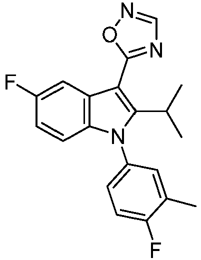 | | |

**Table D. Compounds W1-W32**

| | | |
|---|---|---|
| Compound W 1 | Compound W2 | Compound W3 |
| 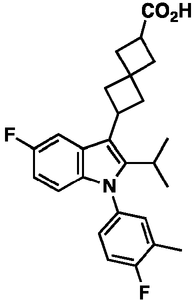 | 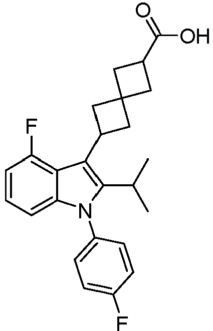 | 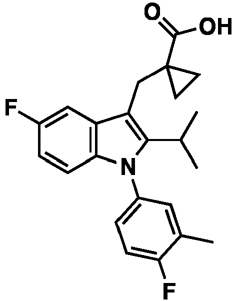 |
| Compound W4 | Compound W5 | Compound W6 |
| 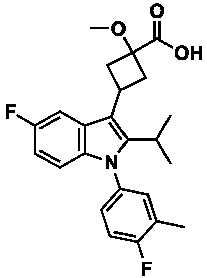 | 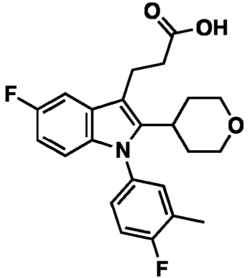 | 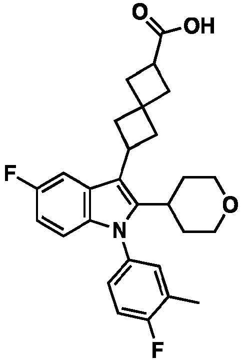 |
| Compound W7 | Compound W8 | Compound W9 |
| 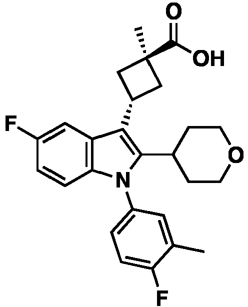 | 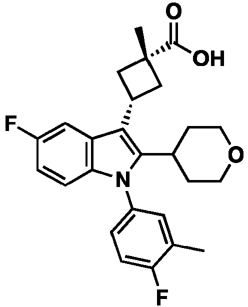 | 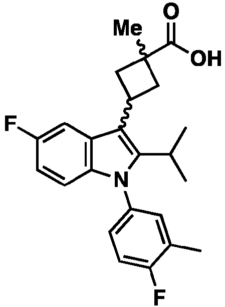 |
| Compound W 10 | Compound W11 | Compound W12 |
| 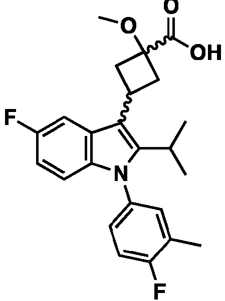 | 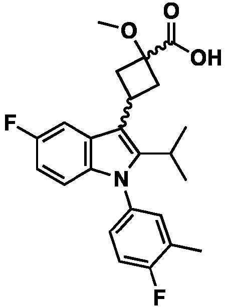 | 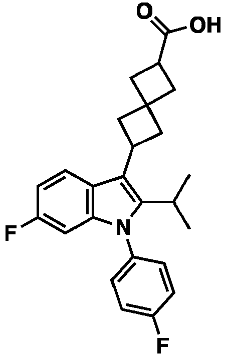 |
| Compound W13 | Compound W14 | Compound W15 |
| 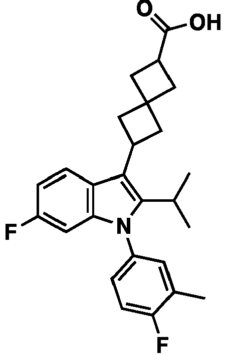 | 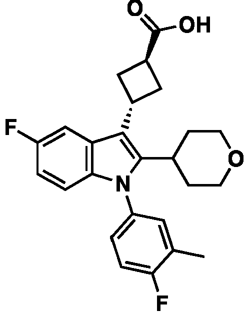 | 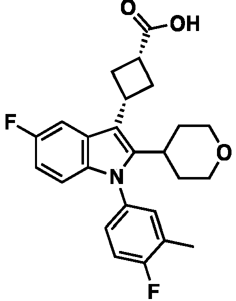 |
| Compound W16 | Compound W17 | Compound W18 |
| 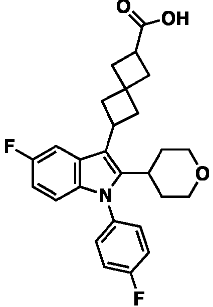 | 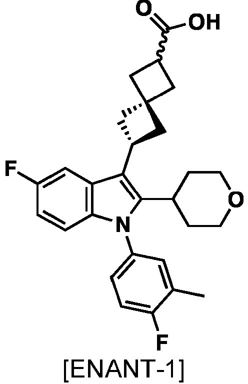 | 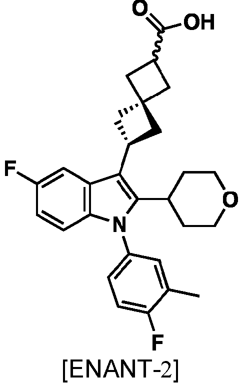 |
| Compound W19 | Compound W20 | Compound W21 |
| 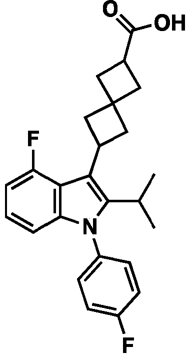 | 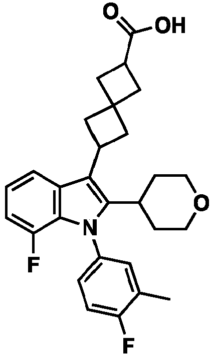 | 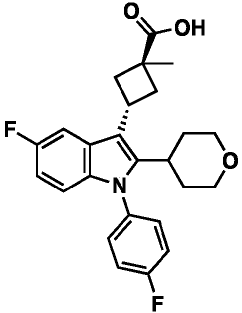 |
| Compound W22 | Compound W23 | Compound W24 |
| 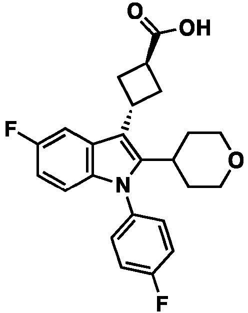 | 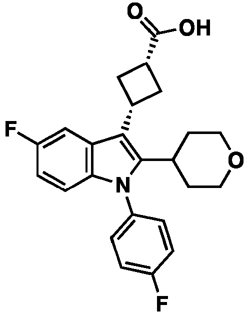 | 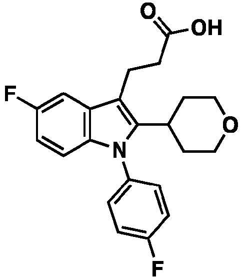 |
| Compound W25 | Compound W26 | Compound W27 |
| 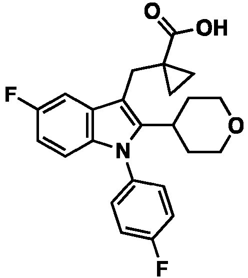 | 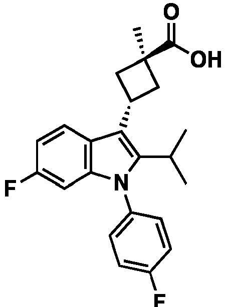 | 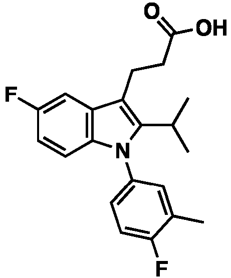 |
| Compound W28 | Compound W29 | Compound W30 |
| 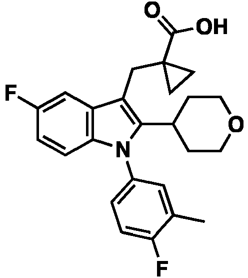 | 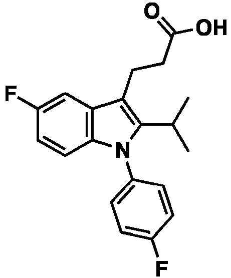 | 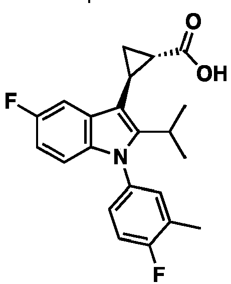 |
| Compound W31 | Compound W32 | |
| 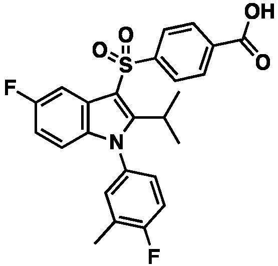 | 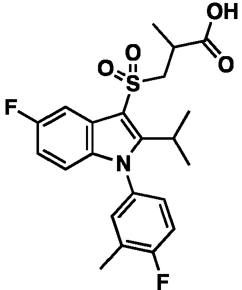 | |

**Table E. Prophetic Compounds P1-P225**

| | | |
|---|---|---|
| Compound P1 | Compound P2 | Compound P3 |
| 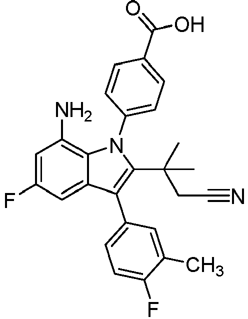 | 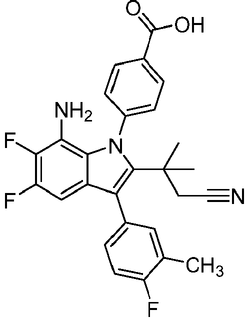 | 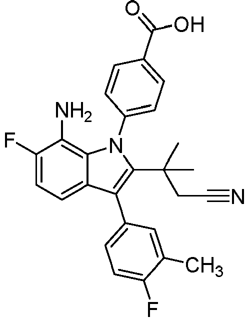 |
| Compound P4 | Compound P5 | Compound P6 |
| 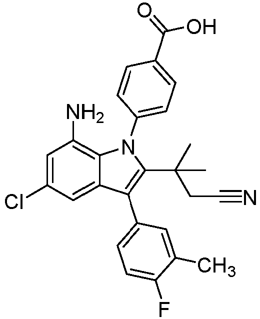 | 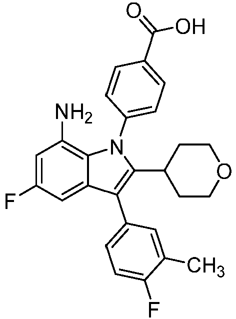 | 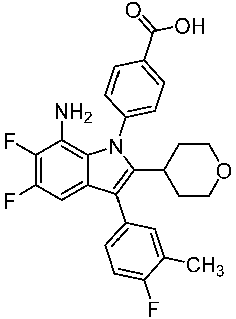 |
| Compound P7 | Compound P8 | Compound P9 |
| 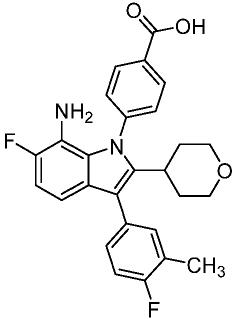 | 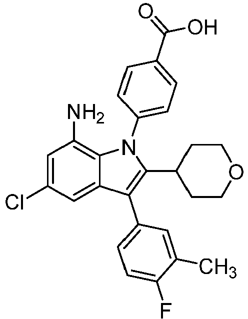 | 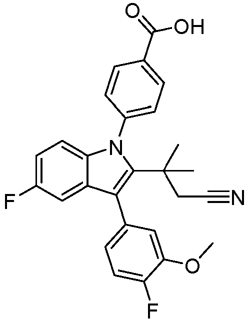 |
| Compound P10 | Compound P11 | Compound P12 |
| 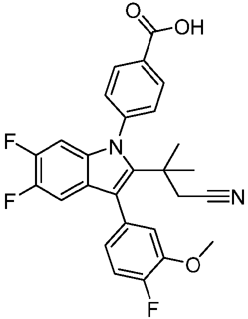 | 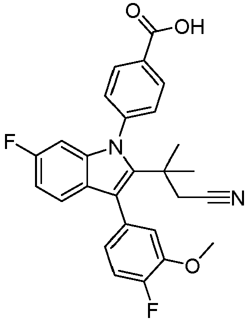 | 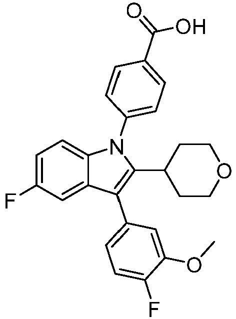 |
| Compound P13 | Compound P14 | Compound P15 |
| 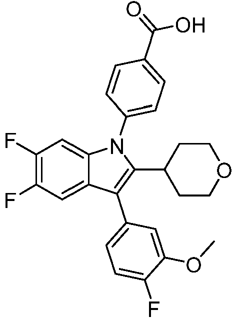 | 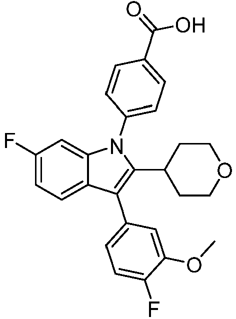 | 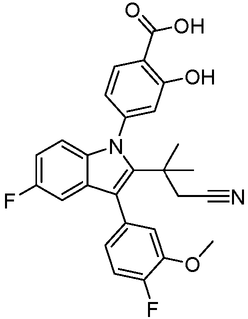 |
| Compound P16 | Compound P17 | Compound P18 |
| 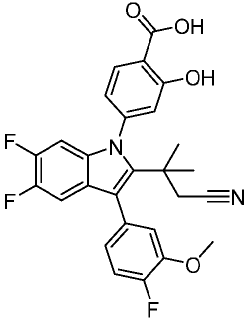 | 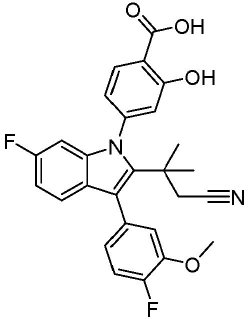 | 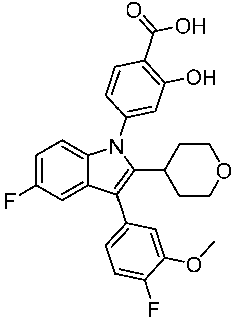 |
| Compound P19 | Compound P20 | Compound P21 |
| 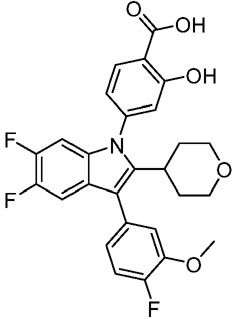 | 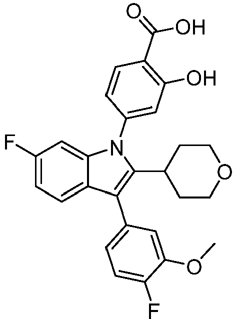 | 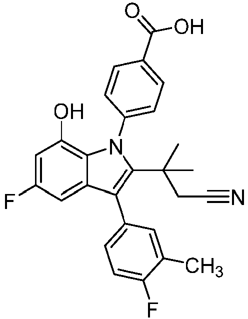 |
| Compound P22 | Compound P23 | Compound P24 |
| 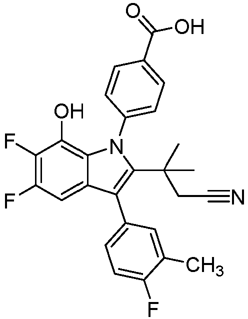 | 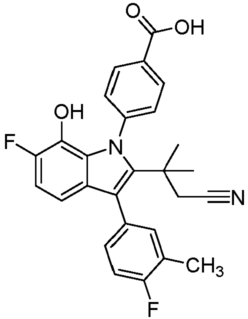 | 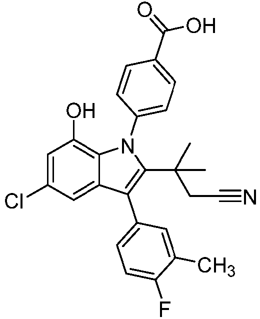 |
| Compound P25 | Compound P26 | Compound P27 |
| 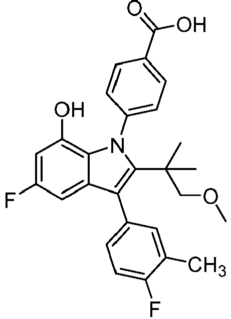 | 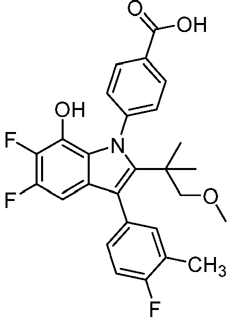 | 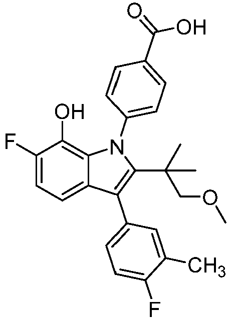 |
| Compound P28 | Compound P29 | Compound P30 |
| 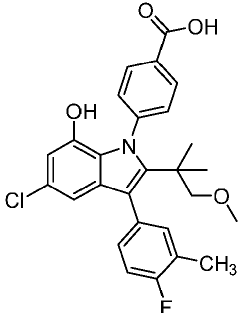 | 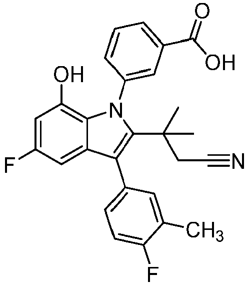 | 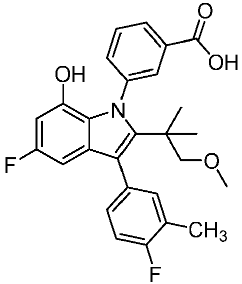 |
| Compound P31 | Compound P32 | Compound P33 |
| 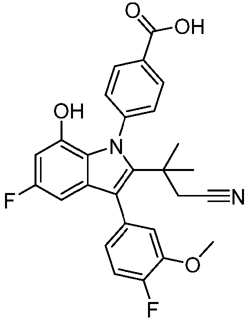 | 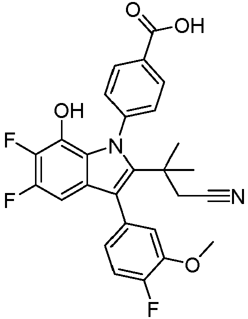 | 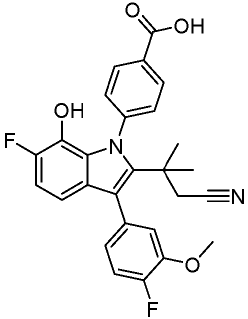 |
| Compound P34 | Compound P35 | Compound P36 |
| 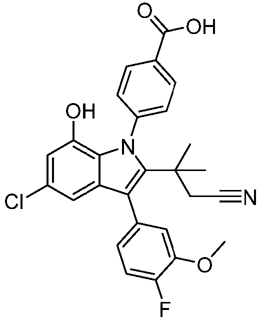 | 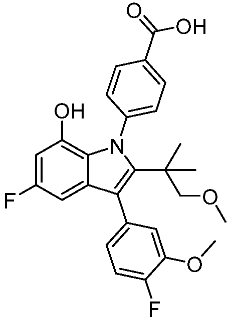 | 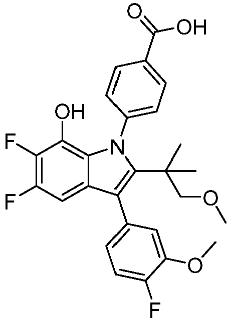 |
| Compound P37 | Compound P38 | Compound P39 |
| 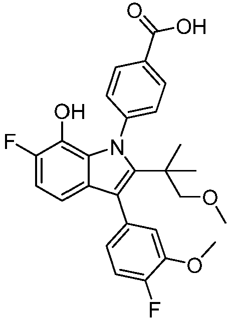 | 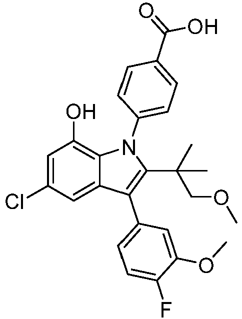 | 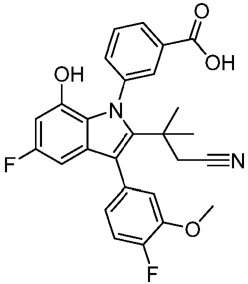 |
| Compound P40 | Compound P41 | Compound P42 |
| 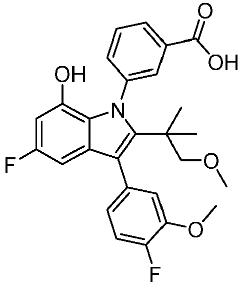 | 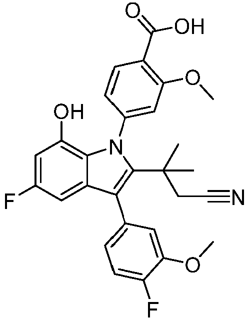 | 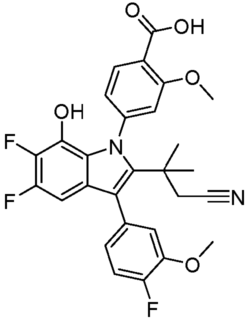 |
| Compound P43 | Compound P44 | Compound P45 |
| 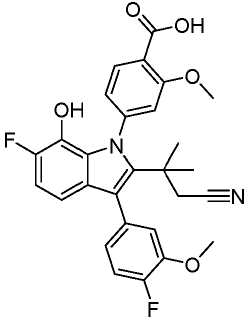 | 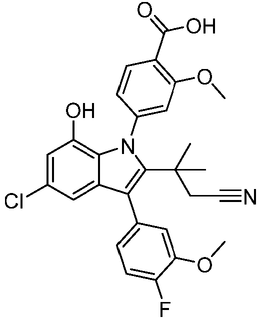 | 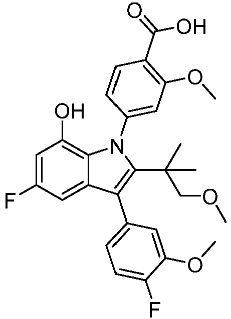 |
| Compound P46 | Compound P47 | Compound P48 |
| 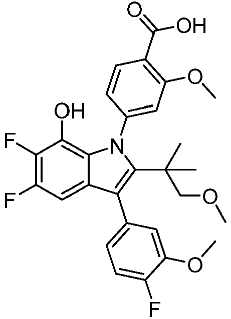 | 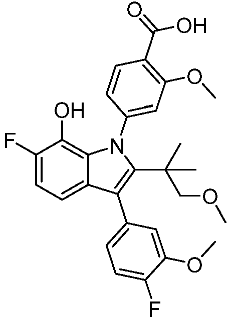 | 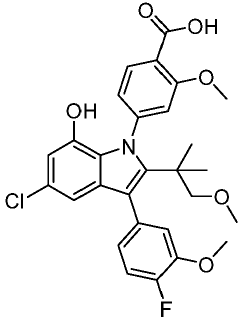 |
| Compound P49 | Compound P50 | Compound P51 |
| 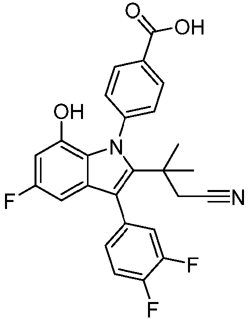 | 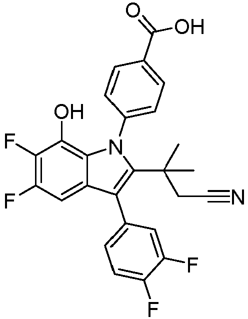 | 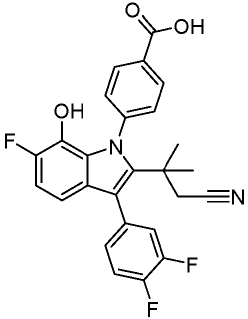 |
| Compound P52 | Compound P53 | Compound P54 |
| 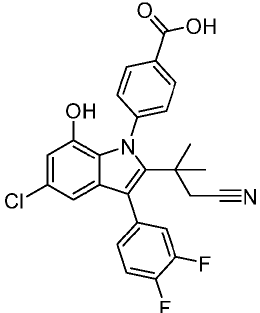 | 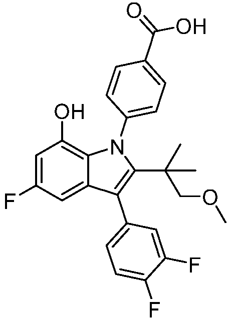 | 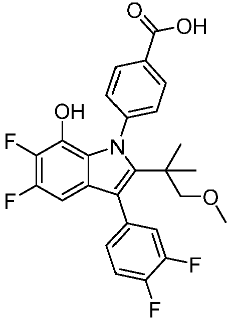 |
| Compound P55 | Compound P56 | Compound P57 |
| 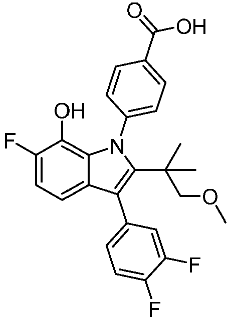 | 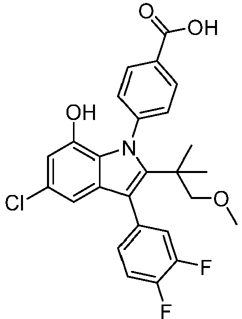 | 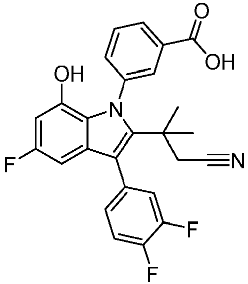 |
| Compound P58 | Compound P59 | Compound P60 |
| 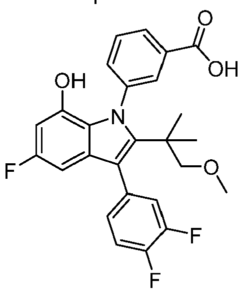 | 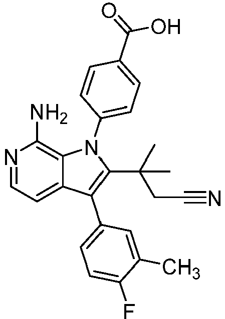 | 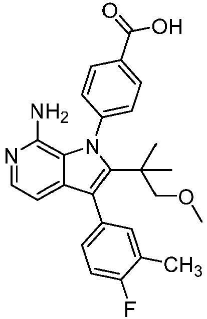 |
| Compound P61 | Compound P62 | Compound P63 |
| 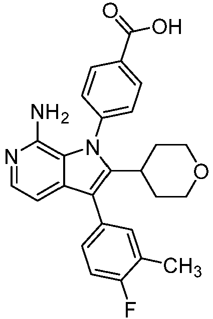 | 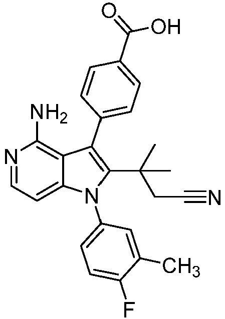 | 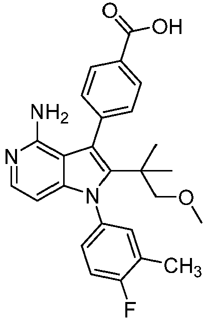 |
| Compound P64 | Compound P65 | Compound P66 |
| 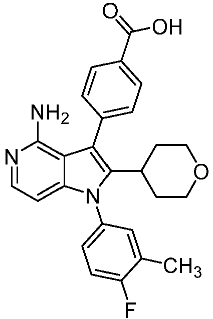 | 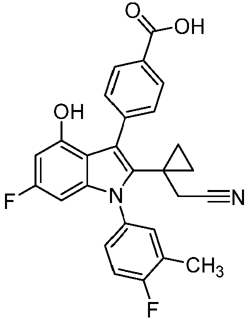 | 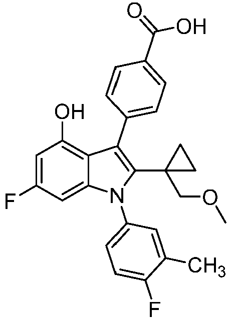 |
| Compound P67 | Compound P68 | Compound P69 |
| 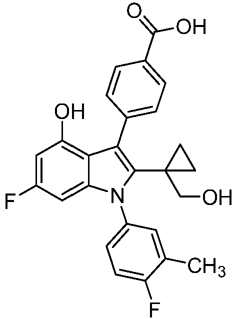 | 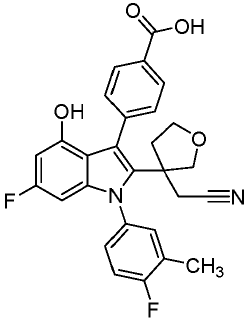 | 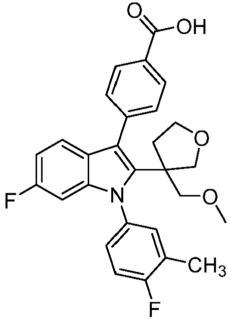 |
| Compound P70 | Compound P71 | Compound P72 |
| 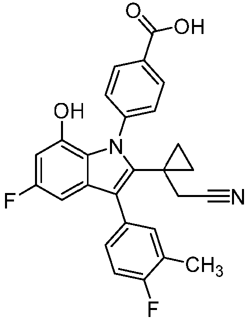 | 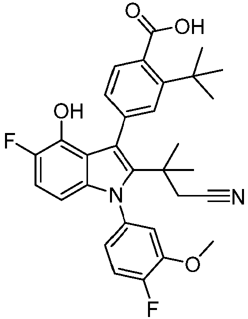 | 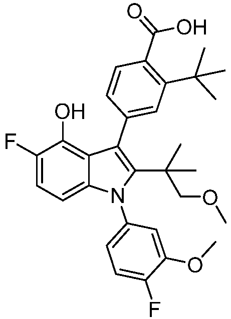 |
| Compound P73 | Compound P74 | Compound P75 |
| 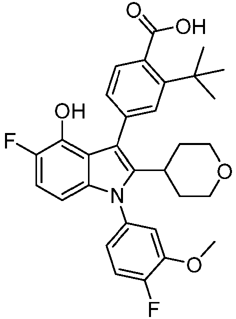 | 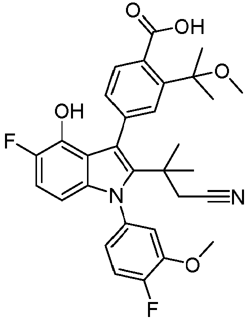 | 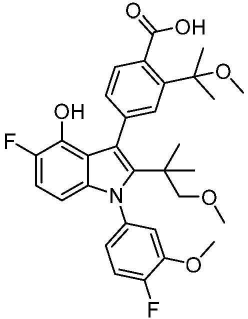 |
| Compound P76 | Compound P77 | Compound P78 |
| 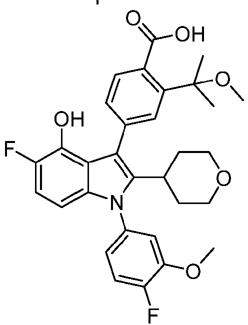 | 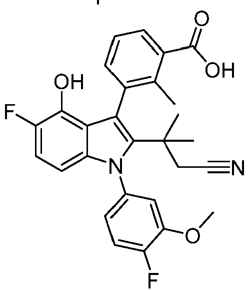 | 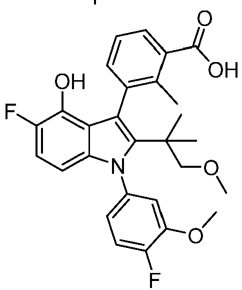 |
| Compound P79 | Compound P80 | Compound P81 |
| 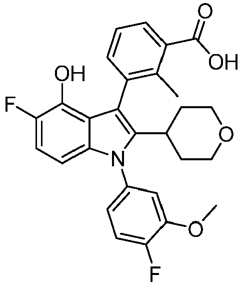 | 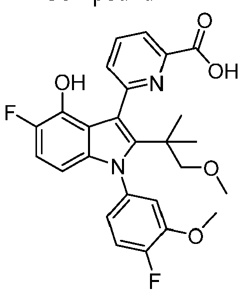 | 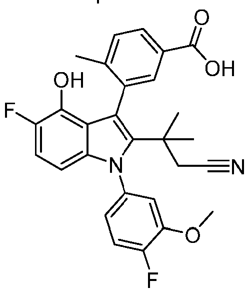 |
| Compound P82 | Compound P83 | Compound P84 |
| 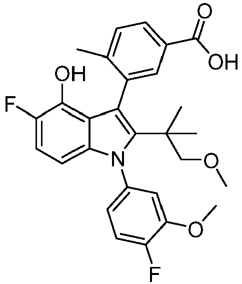 | 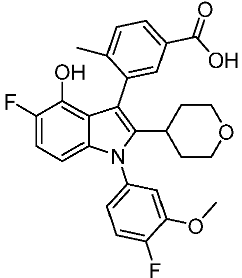 | 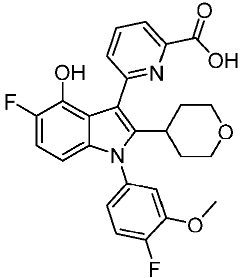 |
| Compound P85 | Compound P86 | Compound P87 |
| 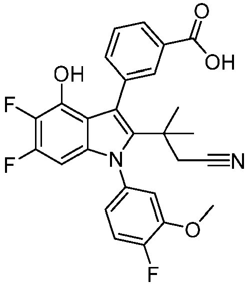 | 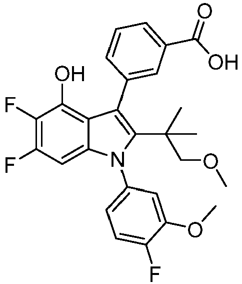 | 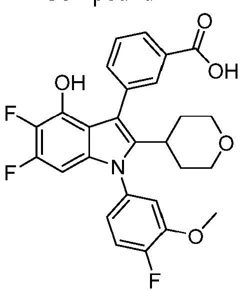 |
| Compound P88 | Compound P89 | Compound P90 |
| 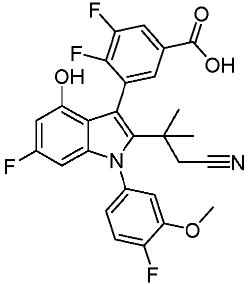 | 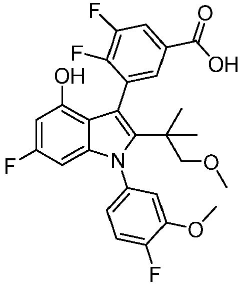 | 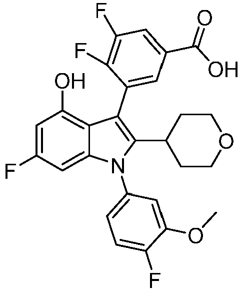 |
| Compound P91 | Compound P92 | Compound P93 |
| 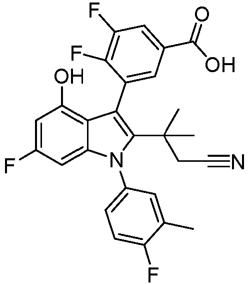 | 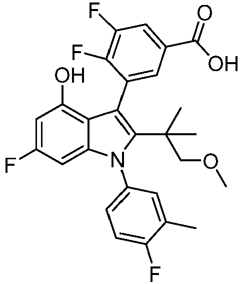 | 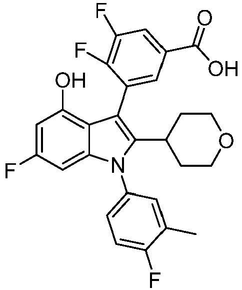 |
| Compound P94 | Compound P95 | Compound P96 |
| 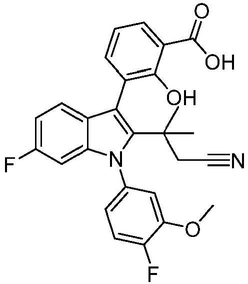 | 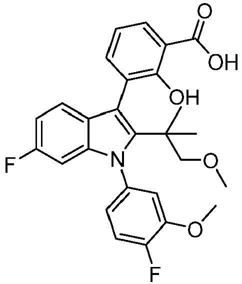 | 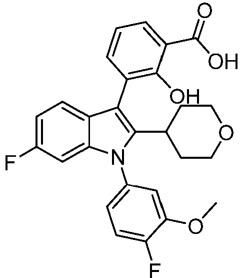 |
| Compound P97 | Compound P98 | Compound P99 |
| 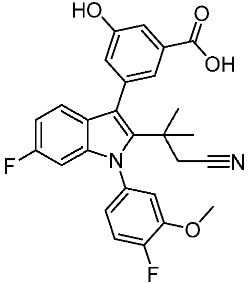 | 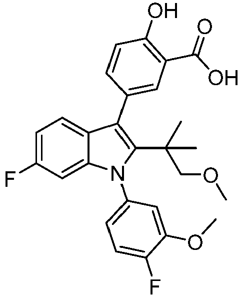 | 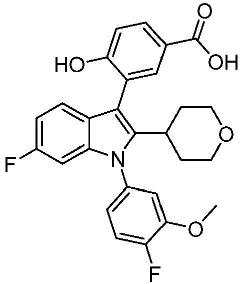 |
| Compound P100 | Compound P101 | Compound P102 |
| 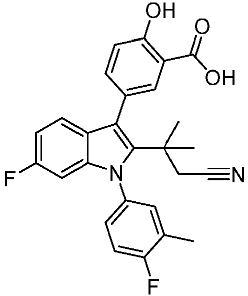 | 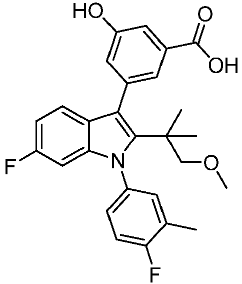 | 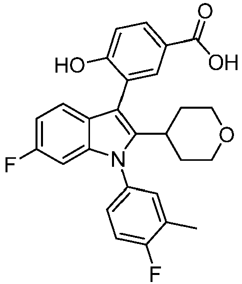 |
| Compound P103 | Compound P104 | Compound P105 |
| 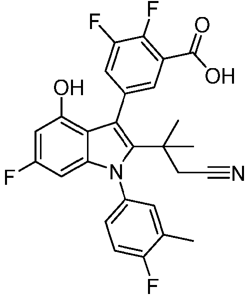 | 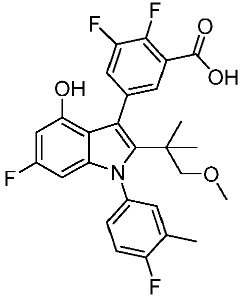 | 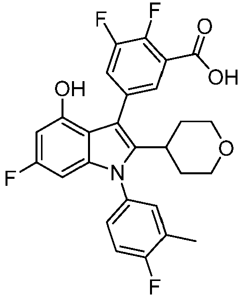 |
| Compound P106 | Compound P107 | Compound P108 |
| 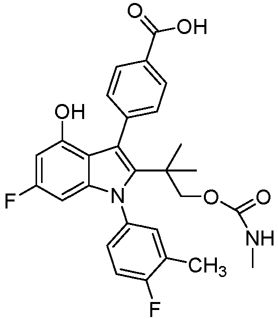 | 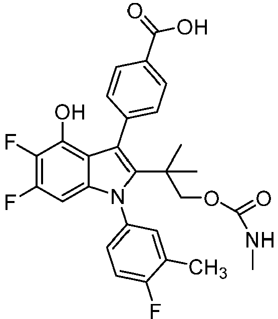 | 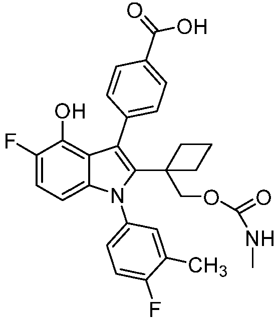 |
| Compound P109 | Compound P110 | Compound P 111 |
| 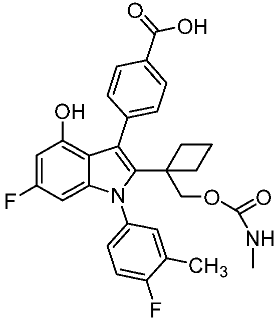 | 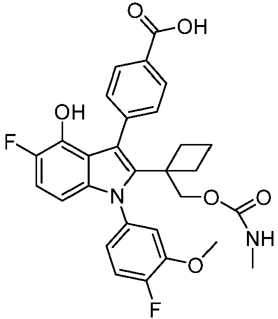 | 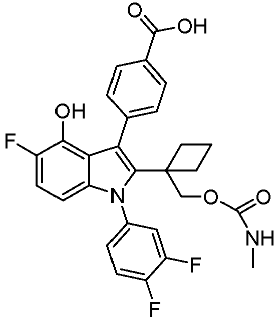 |
| Compound P112 | Compound P113 | Compound P114 |
| 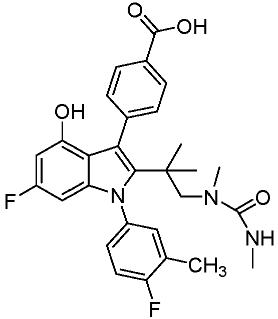 | 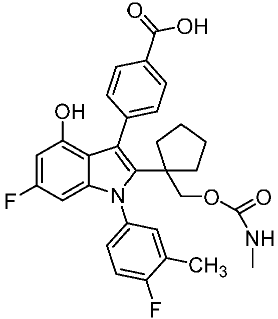 | 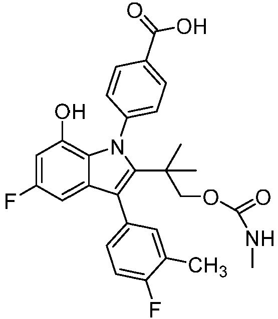 |
| Compound P115 | Compound P116 | Compound P117 |
| 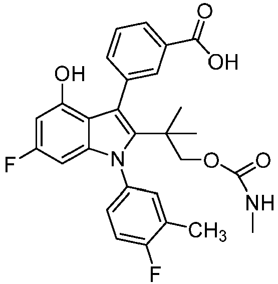 | 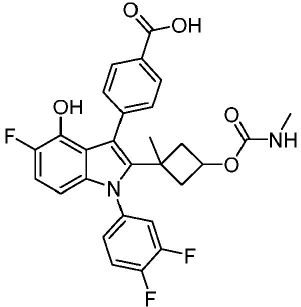 | 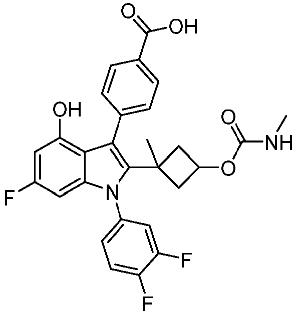 |
| Compound P118 | Compound P119 | Compound P120 |
| 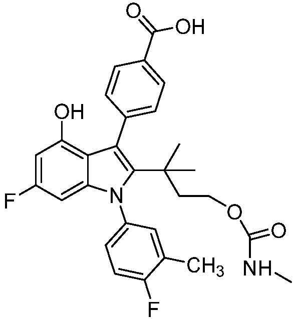 | 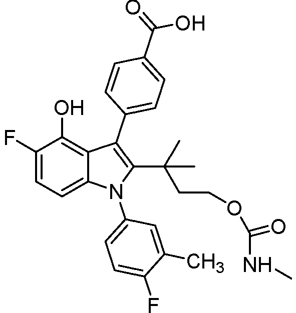 | 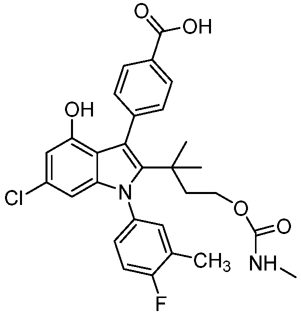 |
| Compound P121 | Compound P122 | Compound P123 |
| 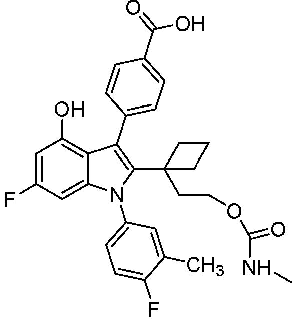 | 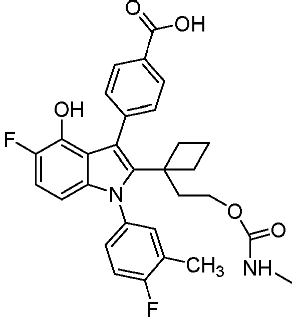 | 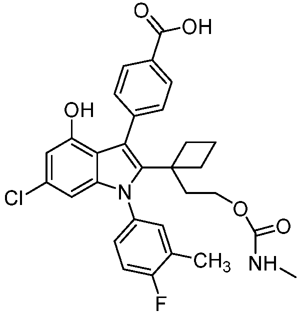 |
| Compound P124 | Compound P125 | Compound P126 |
| 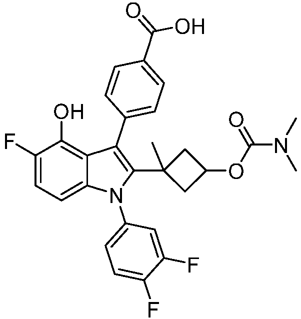 | 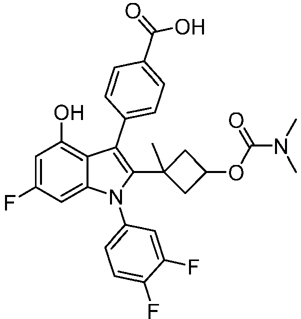 | 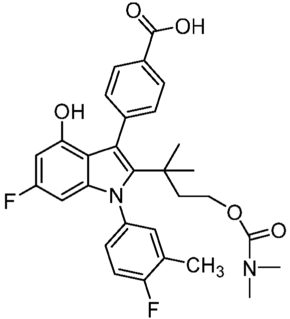 |
| Compound P127 | Compound P128 | Compound P129 |
| 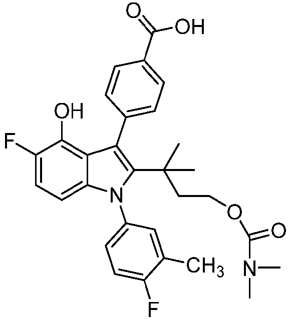 | 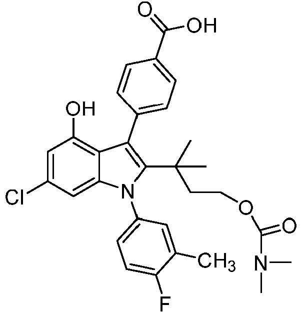 | 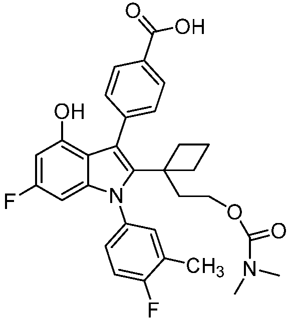 |
| Compound P130 | Compound P131 | Compound P132 |
| 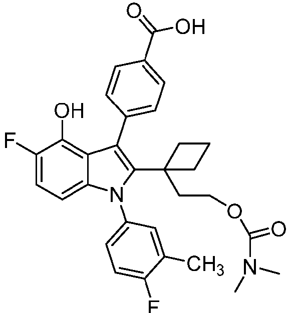 | 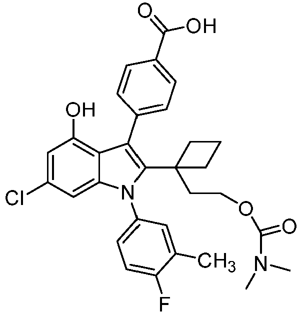 | 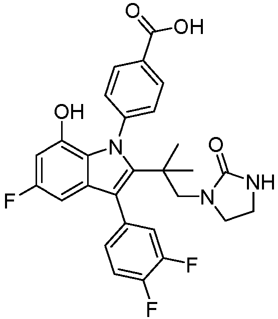 |
| Compound P133 | Compound P134 | Compound P135 |
| 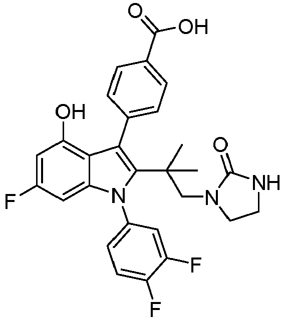 | 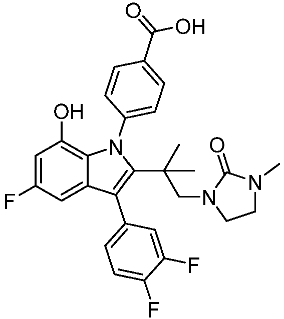 | 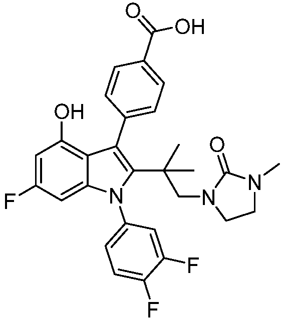 |
| Compound P136 | Compound P137 | Compound P138 |
| 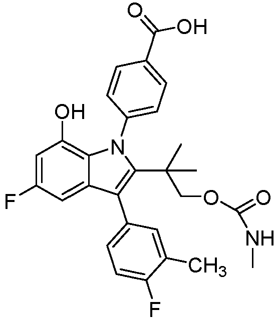 | 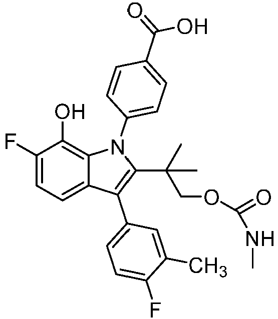 | 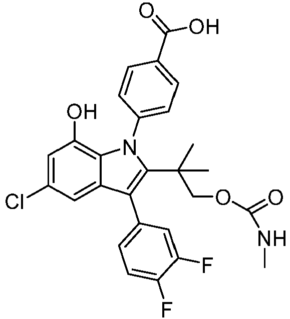 |
| Compound P139 | Compound P140 | Compound P141 |
| 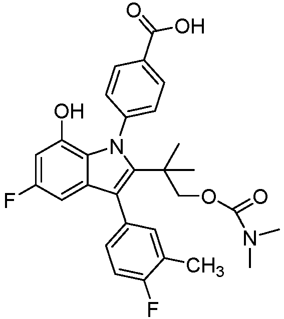 | 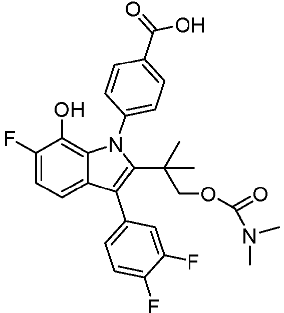 | 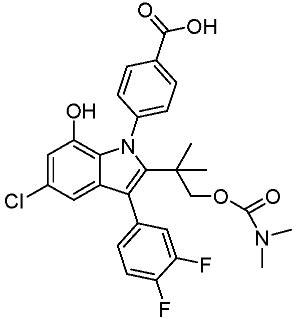 |
| Compound P142 | Compound P143 | Compound P144 |
| 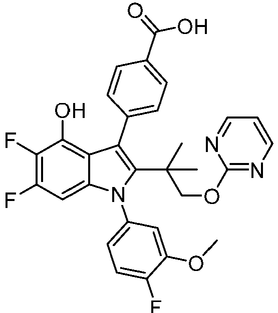 | 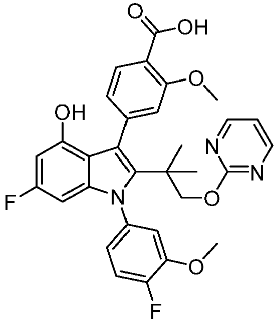 | 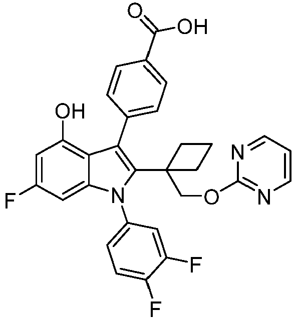 |
| Compound P145 | Compound P146 | Compound P147 |
| 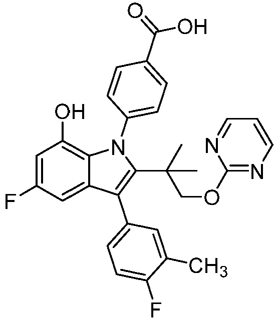 | 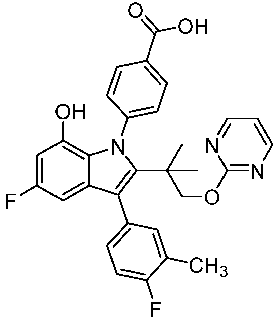 | 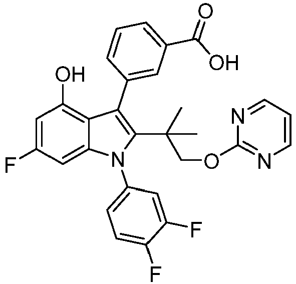 |
| Compound P148 | Compound P149 | Compound P150 |
| 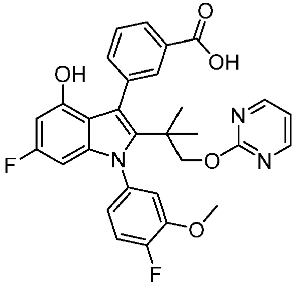 | 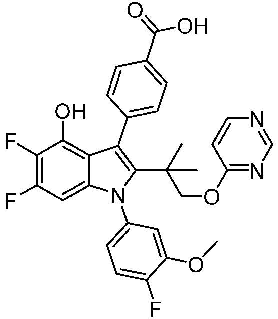 | 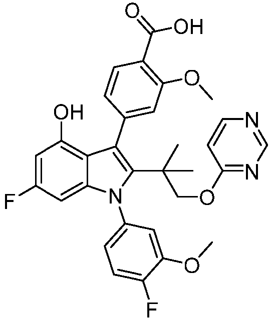 |
| Compound P151 | Compound P152 | Compound P153 |
| 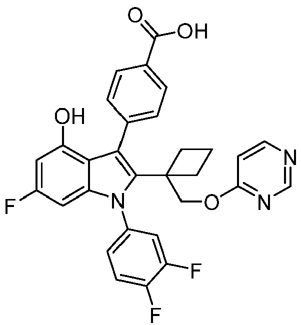 | 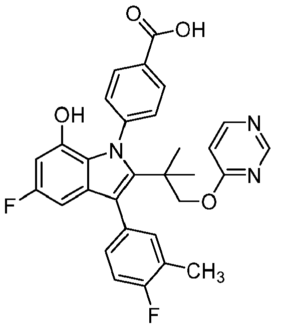 | 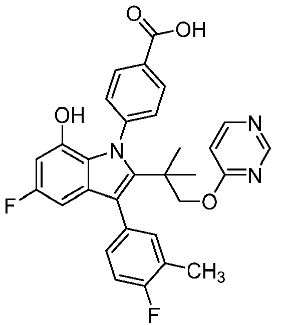 |
| Compound P154 | Compound P155 | Compound P156 |
| 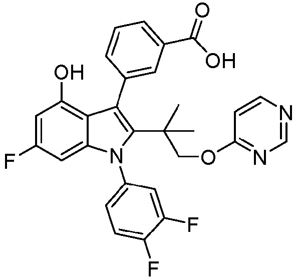 | 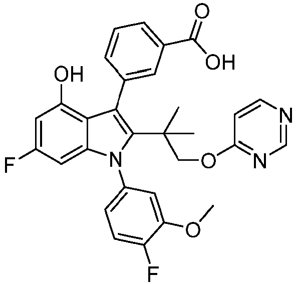 | 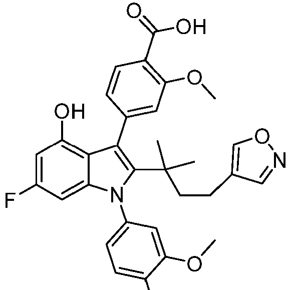 |
| Compound P157 | Compound P158 | Compound P159 |
| 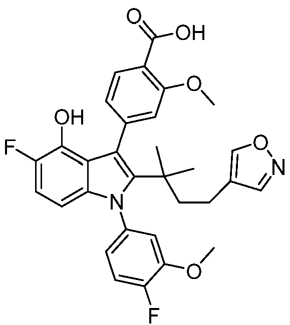 | 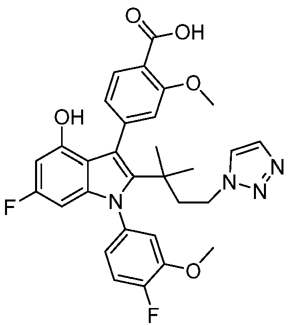 | 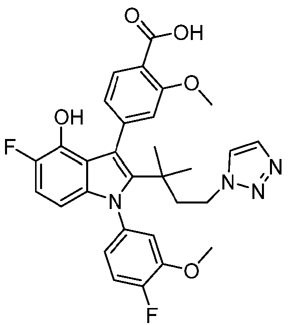 |
| Compound P160 | Compound P161 | Compound P162 |
| 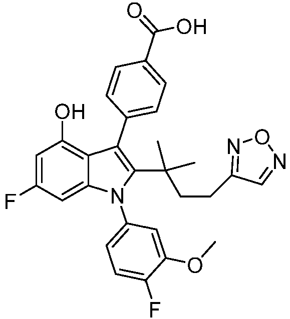 | 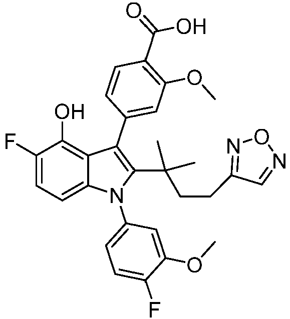 | 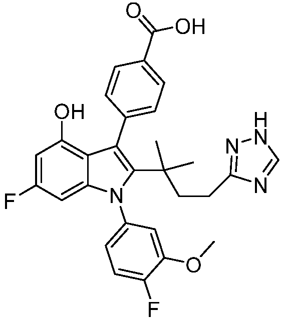 |
| Compound P163 | Compound P164 | Compound P165 |
| 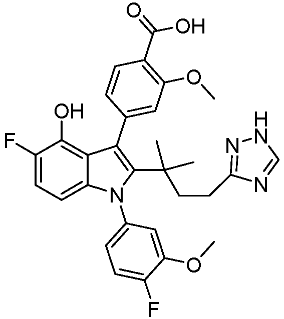 | 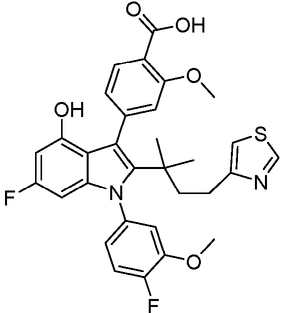 | 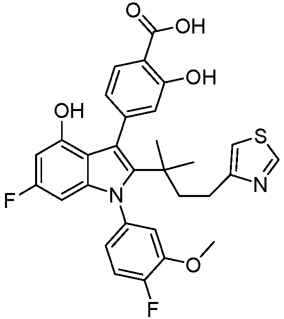 |
| Compound P166 | Compound P167 | Compound P168 |
| 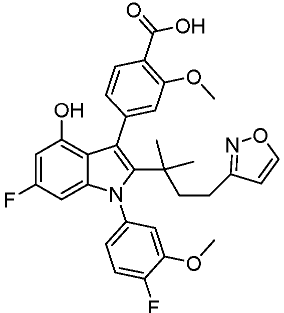 | 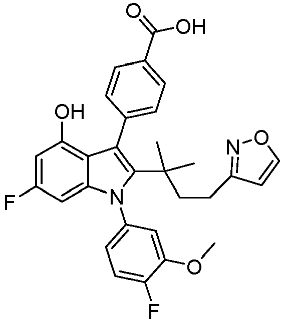 | 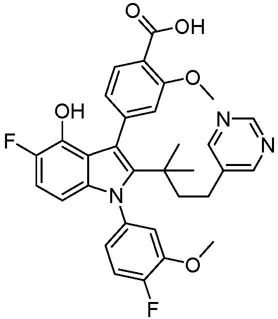 |
| Compound P169 | Compound P170 | Compound P171 |
| 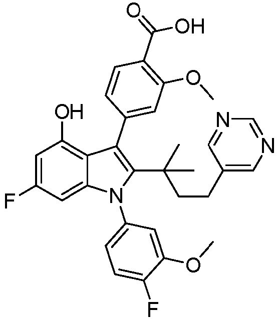 | 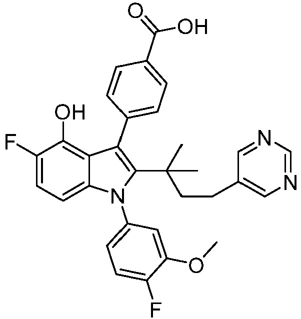 | 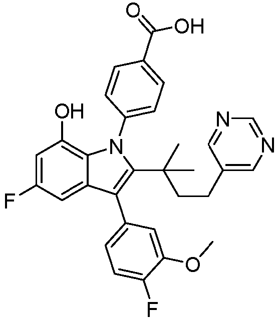 |
| Compound P172 | Compound P173 | Compound P174 |
| 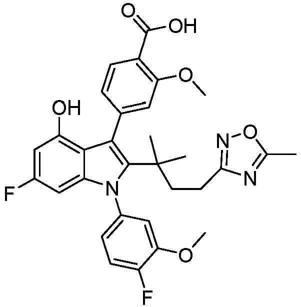 | 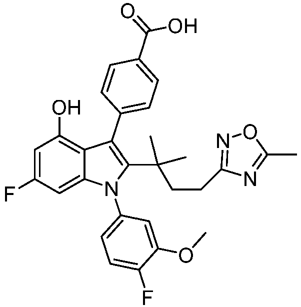 | 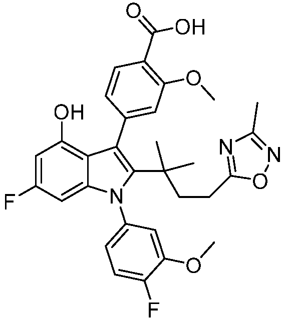 |
| Compound P175 | Compound P176 | Compound P177 |
| 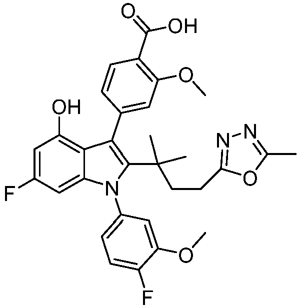 | 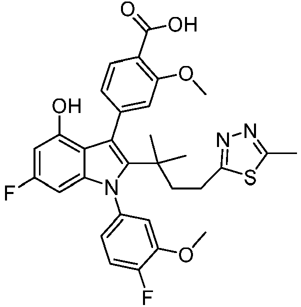 | 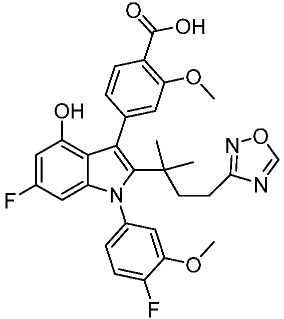 |
| Compound P178 | Compound P179 | Compound P180 |
| 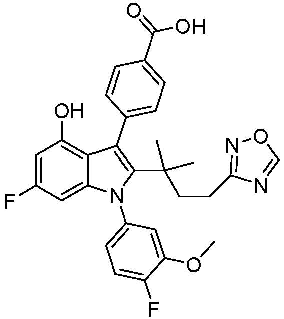 | 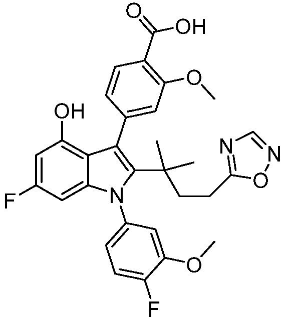 | 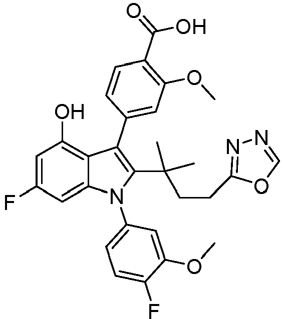 |
| Compound P181 | Compound P182 | Compound P183 |
| 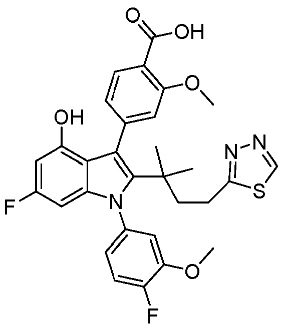 | 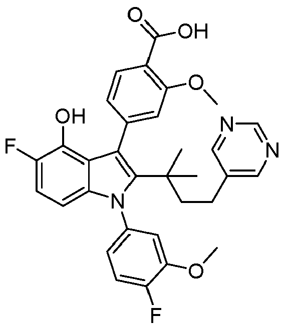 | 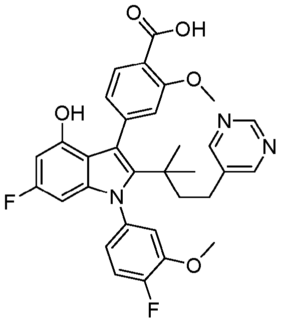 |
| Compound P184 | Compound P185 | Compound P186 |
| 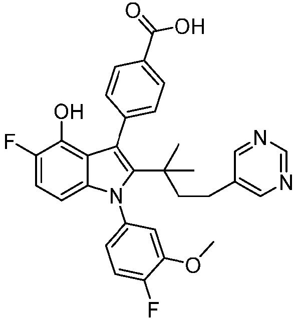 | 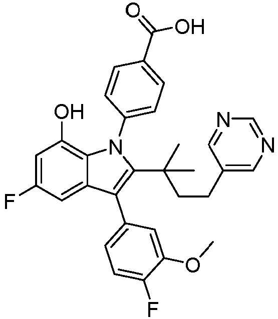 | 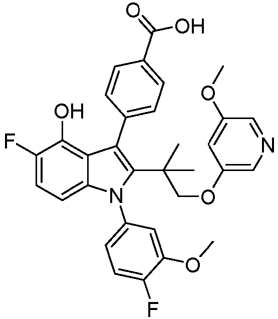 |
| Compound P187 | Compound P188 | Compound P189 |
| 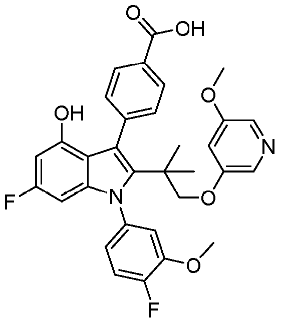 | 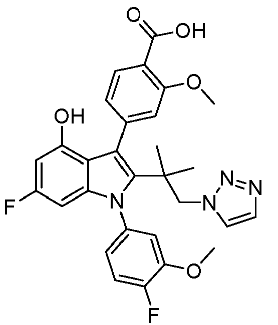 | 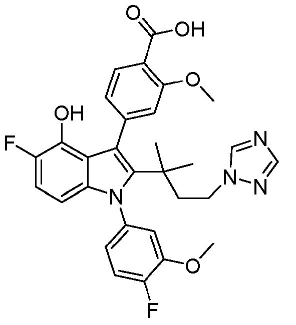 |
| Compound P190 | Compound P191 | Compound P192 |
| 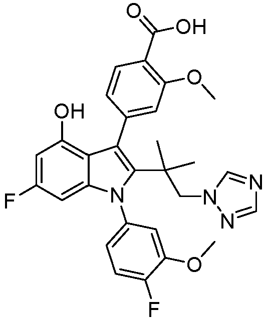 | 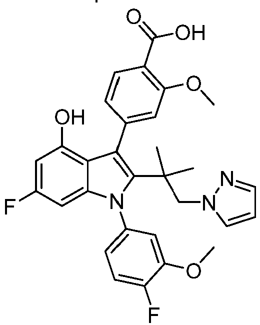 | 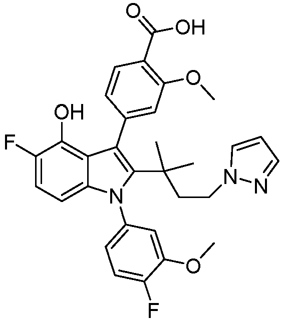 |
| Compound P193 | Compound P194 | Compound P195 |
| 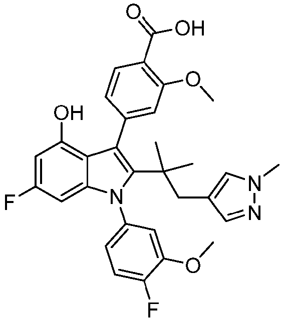 | 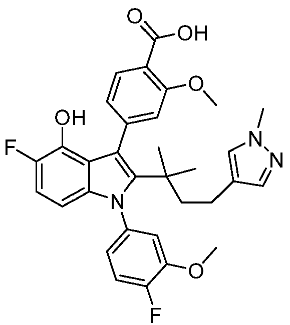 | 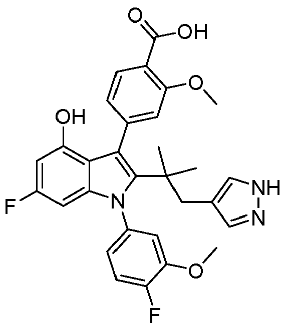 |
| Compound P196 | Compound P197 | Compound P198 |
| 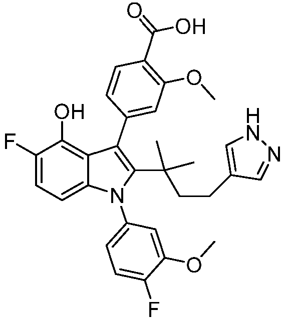 | 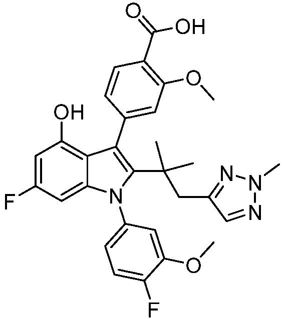 | 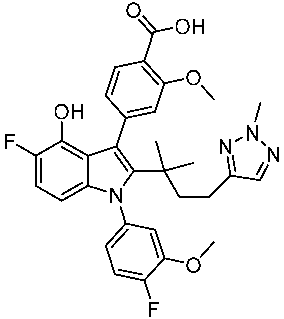 |
| Compound P199 | Compound P200 | Compound P201 |
| 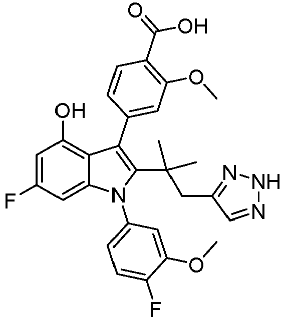 | 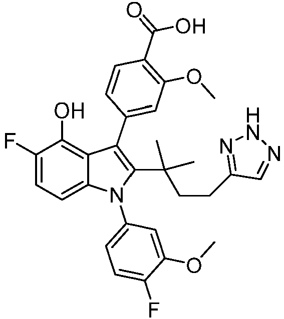 | 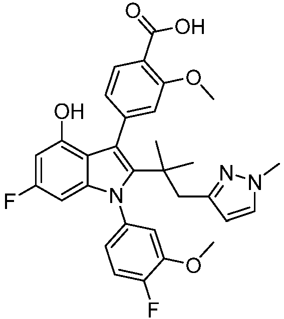 |
| Compound P202 | Compound P203 | Compound P204 |
| 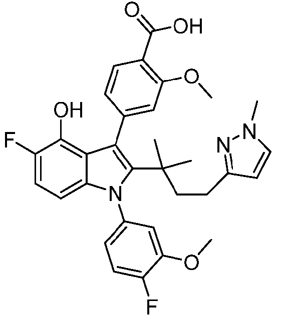 | 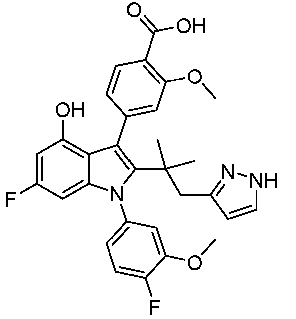 | 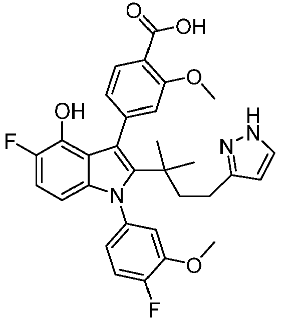 |
| Compound P205 | Compound P206 | Compound P206 |
| 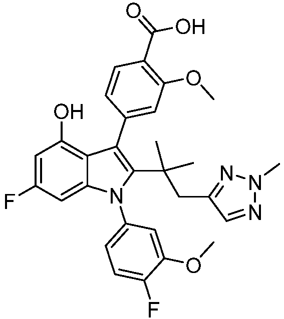 | 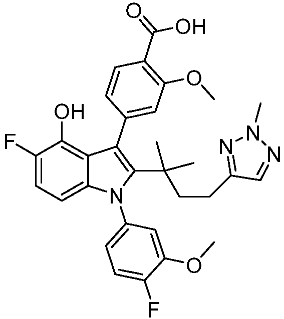 | 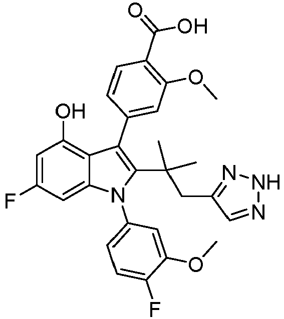 |
| Compound P208 | Compound P209 | Compound P210 |
| 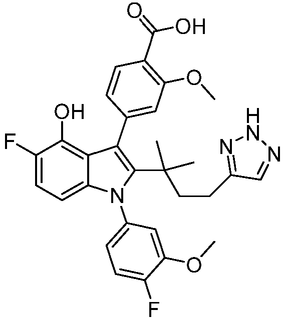 | 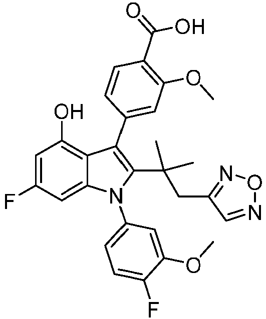 | 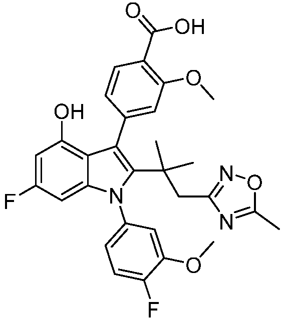 |
| Compound P211 | Compound P212 | Compound P213 |
| 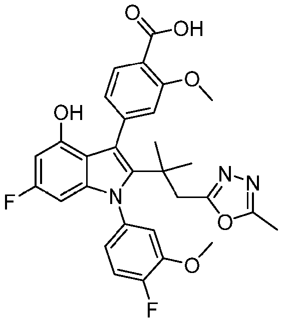 | 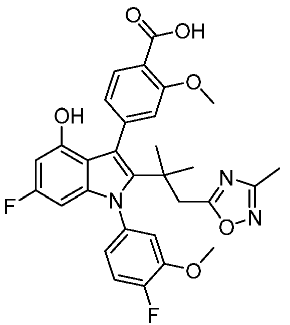 | 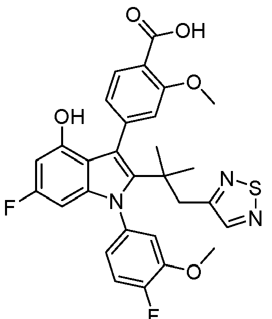 |
| Compound P214 | Compound P215 | Compound P216 |
| 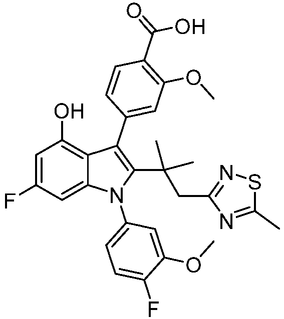 | 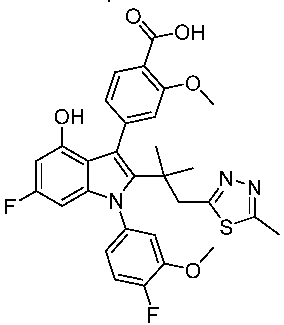 | 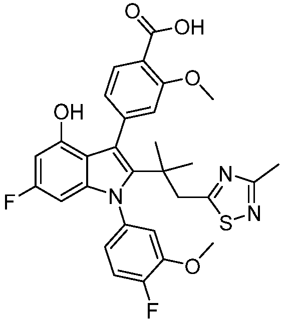 |
| Compound P217 | Compound P218 | Compound P219 |
| 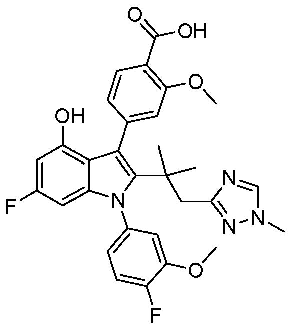 | 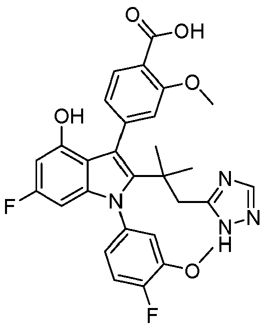 | 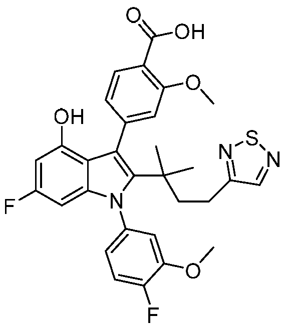 |
| Compound P220 | Compound P221 | Compound P222 |
| 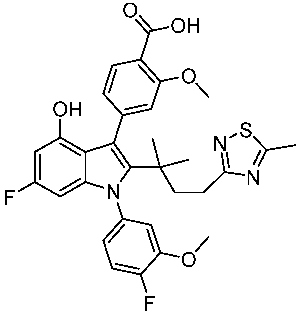 | 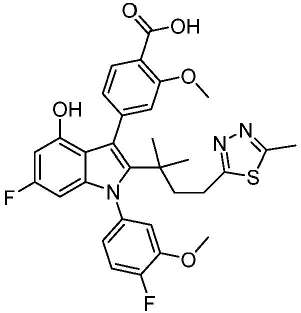 | 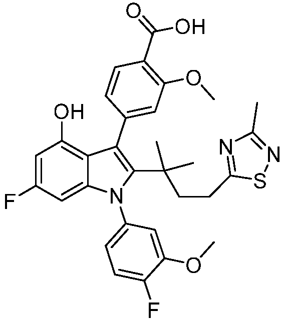 |
| Compound P223 | Compound P224 | Compound P225 |
| 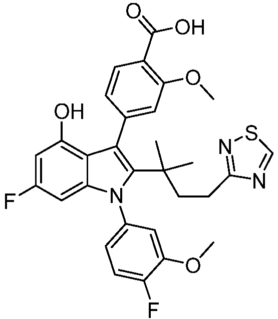 | 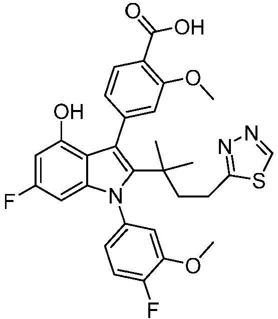 | 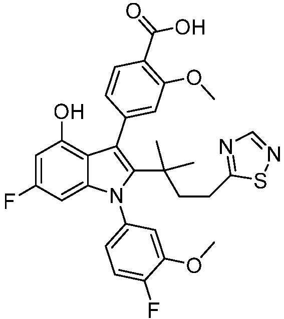 |

Some embodiments of the disclosure include derivatives of Compounds 1-457, Compounds 458-532, Compounds B1-B25, Compounds W1-W32, Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) or tautomers thereof. In some embodiments, the derivatives are silicon derivatives in which at least one carbon atom in a compound selected from Compounds 1-457, Compounds 458-532, Compounds B 1-B25, Compounds W1-W32, and Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) has been replaced by silicon. In some embodiments, the derivatives are boron derivatives, in which at least one carbon atom in a compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) or tautomers thereof has been replaced by boron. In other embodiments, the derivatives are phosphate derivatives, in which at least one carbon atom in a compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) or tautomers thereof has been replaced by phosphorus. Because the general properties of silicon, boron, and phosphorus are similar to those of carbon, replacement of carbon by silicon, boron, or phosphorus can result in compounds with similar biological activity to a carbon containing original compound.

In some embodiments, the derivative is a silicon derivative in which one carbon atom in a compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) and tautomers thereof has been replaced by silicon. In other embodiments, two carbon atoms have been replaced by silicon. The carbon replaced by silicon may be a non-aromatic carbon. In some embodiments, a quaternary carbon atom of a tert-butyl moiety may be replaced by silicon. In some embodiments, the silicon derivatives of the disclosure may include one or more hydrogen atoms replaced by deuterium. For example, one or more hydrogens of a tert-butyl moiety in which the carbon has been replaced by silicon, may be replaced by deuterium. In other embodiments, a silicon derivative of a compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457) or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) and tautomers thereof may have silicon incorporated into a heterocycle ring.

In some embodiments, examples of silicon derivatives of Compounds 1-457 or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) include the following compounds: 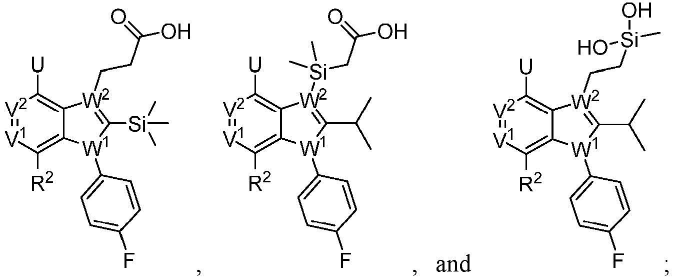 wherein the variables not specifically defined are as defined in any one of Formulae (I), (IIa)-(IIf), (IIIa)-(IIIb), (IVa)-(IVb), (Va)-(Vb), (VIa)-(VIe), (VIIa)-(VIIe), and (IXa)-(IXe).

In some embodiments, examples of boron derivatives of Compounds 1-457 or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) include the following compounds:

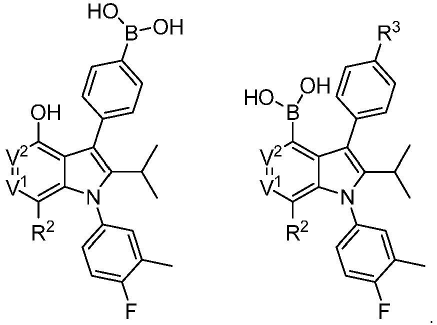

In some embodiments, examples of phosphate derivatives of Compounds 1-457 or compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe) include the following compounds: 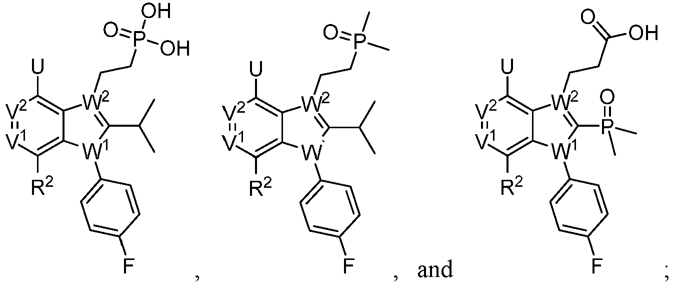 wherein the variables not specifically defined are as defined in any one of Formulae (I), (IIa)-(IIf), (IIIa)-(IIIb), (IVa)-(IVb), (Va)-(Vb), (VIa)-(VIe), (VIIa)-(VIIe), and (IXa)-(IXe).

In some embodiments, examples of phosphate derivatives of Formula (I) include the following compounds: 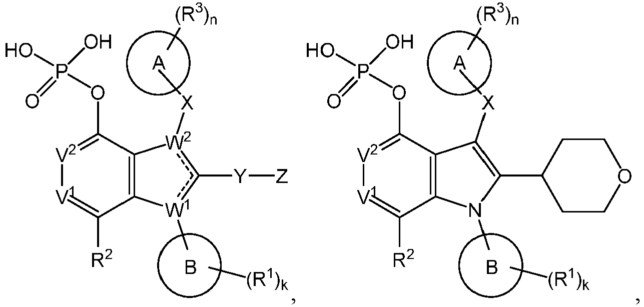 and 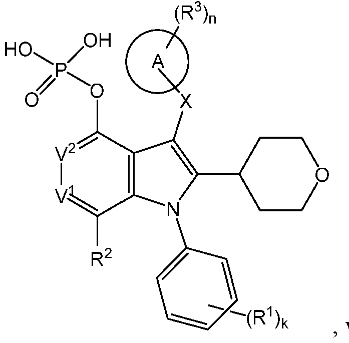 wherein the variables not specifically defined are as defined in
Formula (I).

In some embodiments, examples of phosphate derivatives of Formula (I) include the following compounds: 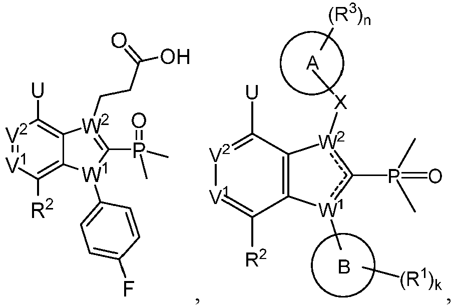 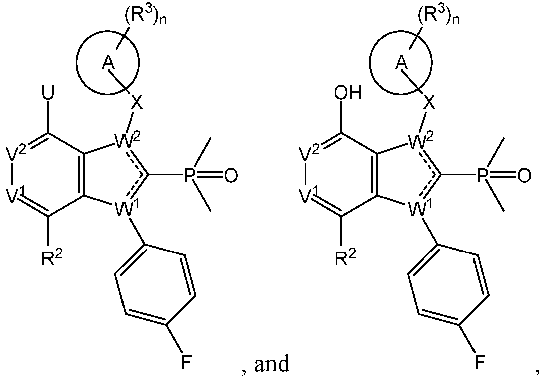 wherein the variables not specifically defined are as defined in Formula (I).

Another aspect of the disclosure provides pharmaceutical compositions comprising a compound selected from compounds according to any of Formulae (I), (IIa)-(IIf), (IIIa)-(IIIb), (IVa)-(IVb), (Va)-(Vb), (VIa)-(VIe), (VIIa)-(VIIe), and (IXa)-(IXe), Compounds 1-457, Compounds 458-532, Compounds B1-B25, Compounds W1-W32, Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing. In some embodiments, the pharmaceutical composition comprising at least one compound chosen from Formulae (I), (IIa)-(IIf), (IIIa)-(IIIb), (IVa)-(IVb), (Va)-(Vb), (VIa)-(VIe), (VIIa)-(VIIe), and (IXa)-(IXe) and Compounds 1-457, Compounds 458-532, Compounds B1-B25, Compounds W1-W32, Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing is administered to a patient in need thereof.

A pharmaceutical composition may further comprise at least one pharmaceutically acceptable carrier. In some embodiments, the at least one pharmaceutically acceptable carrier is chosen from pharmaceutically acceptable vehicles and pharmaceutically acceptable adjuvants. In some embodiments, the at least one pharmaceutically acceptable is chosen from pharmaceutically acceptable fillers, disintegrants, surfactants, binders, and lubricants.

It will also be appreciated that a pharmaceutical composition of this disclosure can be employed in combination therapies; that is, the pharmaceutical compositions described herein can further include at least one other active agent. Alternatively, a pharmaceutical composition comprising at least one compound of Formulae (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing can be administered as a separate composition concurrently with, prior to, or subsequent to, a composition comprising at least one additional active agent. In some embodiments, a pharmaceutical composition comprising at least one compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing can be administered as a separate composition concurrently with, prior to, or subsequent to, a composition comprising at least one additional active agent.

In some embodiments, a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, is combined with at least one additional active agent for simultaneous, separate, or sequential use in the treatment of AATD. In some embodiments, when the use is simultaneous, the compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, and the at least one additional active agent are in separate pharmaceutical compositons. In some embodiments, when the use is simultaneous, the compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, and the at least one additional active agent are together in the same pharmaceutical composition. In some embodiments, the compound is a compound selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, is provided for use in a method of treating AATD, wherein the method comprises co-administering the compound and an additional active agent. In some embodiments, the compound and the additional active agent are co-administered in the same pharmaceutical composition. In some embodiments, the compound and the additional active agent are co-administered in separate pharmaceutical compositions. In some embodiments, the compound and the additional active agent are co-administered simultaneously. In some embodiments, the compound and the additional active agent are co-administered sequentially. In some embodiments, the compound is selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, a combination of a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, and an additional active agent, is provided for use in a method of treating AATD. In some embodiments, the compound and the additional active agent are co-administered in the same pharmaceutical composition. In some embodiments, the compound and the additional active agent are co-administered in separate pharmaceutical compositions. In some embodiments, the compound and the additional active agent are co-administered simultaneously. In some embodiments, the compound and the additional active agent are co-administered sequentially. In some embodiments, the compound is selected from Compounds 1-457, Compounds 458-531, Compounds B 1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, an additional active agent is provided for use in a method of treating AATD, wherein the method comprises co-administrating the additional active agent and a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing. In some embodiments, the compound and the additional active agent are co-administered in the same pharmaceutical composition. In some embodiments, the compound and the additional active agent are co-administered in separate pharmaceutical compositions. In some embodiments, the compound and the additional active agent are co-administered simultaneously. In some embodiments, the compound and the additional active agent are co-administered sequentially. In some embodiments, the compound is selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, is provided for use in a method of treating AATD, wherein the compound is prepared for administration in combination with an additional active agent. In some embodiments, the compound and the additional active agent are prepared for administration in the same pharmaceutical composition. In some embodiments, the compound and the additional active agent are prepared for administration in separate pharmaceutical compositions. In some embodiments, the compound and the additional active agent are prepared for simultaneous administration. In some embodiments, the compound and the additional active agent are prepared for sequential administration. In some embodiments, the compound is selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, a combination of a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing, and an additional active agent, is provided for use in a method of treating AATD. In some embodiments, the compound and the additional active agent are prepared for administration in the same pharmaceutical composition. In some embodiments, the compound and the additional active agent are prepared for administration in separate pharmaceutical compositions. In some embodiments, the compound and the additional active agent are prepared for simultaneous administration. In some embodiments, the compound and the additional active agent are prepared for sequential administration. In some embodiments, the compound is selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, an additional active agent is provided for use in a method of treating AATD, wherein the additional active agent is prepared for administration in combination with a compound of Formula (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing. In some embodiments, the compound and the additional active agent are prepared for administration in the same pharmaceutical composition. In some embodiments, the compound and the additional active agent are prepared for administration in separate pharmaceutical compositions. In some embodiments, the compound and the additional active agent are prepared for simultaneous administration. In some embodiments, the compound and the additional active agent are prepared for sequential administration. In some embodiments, the compound is selected from Compounds 1-457, Compounds 458-531, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, the additional active agent is selected the group consisting of alpha-1 antitrypsin protein (AAT) from the blood plasma of healthy human donors and recombinant AAT. In some embodiments, the additional active agent is alpha-1 antitrypsin protein (AAT) from the blood plasma of healthy human donors. In some embodiments, the additional active agent is alpha-1 antitrypsin protein (AAT) from the blood plasma of healthy human donors.

As described above, pharmaceutical compositions disclosed herein may optionally further comprise at least one pharmaceutically acceptable carrier. The at least one pharmaceutically acceptable carrier may be chosen from adjuvants and vehicles. The at least one pharmaceutically acceptable carrier, as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York discloses various carriers used in formulating pharmaceutical compositions and known techniques for the preparation thereof. Except insofar as any conventional carrier is incompatible with the compounds of this disclosure, such as by producing any undesirable biological effect or otherwise interacting in a deleterious manner with any other component(s) of the pharmaceutical composition, its use is contemplated to be within the scope of this disclosure.

Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate), powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants.

In another aspect of the disclosure, the compounds and the pharmaceutical compositions, described herein, are used to treat AATD. In some embodiments, the subject in need of treatment with the compounds and compositions of the disclosure carries the ZZ mutation. In some embodiments, the subject in need of treatment with the compounds and compositions of the disclosure carries the SZ mutation.

In some embodiments, the methods of the disclosure comprise administering to a patient in need thereof a compound chosen from any of the compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing. In some embodiments, the compound is selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing. In some embodiments, said patient in need thereof has a Z mutation in the alpha-1 antitrypsin gene. In some embodiments said patient in need thereof is homozygous for the Z-mutation in the alpha-1 antitrypsin gene.

Another aspect of the disclosure provides methods of modulating alpha-1 antitrypsin activity comprising the step of contacting said alpha-1-antitrypsin with at least one compound of Formulae (I), (IIa)-(IIf), (IIIa)-(IIIb), (IVa)-(IVb), (Va)-(Vb), (VIa)-(VIe), (VIIa)-(VIIe), and (IXa)-(IXe), tautomers of those compounds, deuterated derivatives of those compounds and tautomers, and pharmaceutically acceptable salts of any of the foregoing. In some embodiments, the methods of modulating alpha-1 antitrypsin activity comprising the step of contacting said alpha-1-antitrypsin with at least one compound selected from Compounds 1-457, Compounds 458-532, Compounds B1-B25, Compounds W1-W32, and Compounds P1-P225 (e.g., Compounds 1-457, or e.g., Compounds 1-142, 144-177, 179-399, 401-422, 425-433, and 435-457), tautomers of those compounds, deuterated derivatives of those compounds and tautomers, and pharmaceutically acceptable salts of any of the foregoing.

In some embodiments, the methods of modulating alpha-1 antitrypsin activity take place *in vivo.* In some embodiments, the methods of modulating alpha-1 antitrypsin activity take place *ex vivo* and said alpha-1-antitrypsin is from a biological sample obtained from a human subject. In some embodiments, the methods of modulating AAT take place *in vitro* and said alpha-1-antitrypsin is from a biological sample obtained from a human subject. In some embodiments, the biological sample is a blood sample. In some embodiments, the biological sample is a sample taken from a liver biopsy.

### III Preparation of Compounds

All the generic, subgeneric, and specific compound formulae disclosed herein are considered part of the disclosure.

### A. Compounds of Formula I

The compounds of the disclosure may be made according to standard chemical practices or as described herein. Throughout the following synthetic schemes and in the descriptions for preparing compounds of Formulae (I), (IIa)-(IIf), (IIIa)-(IIIb), (IVa)-(IVb), (Va)-(Vb), (VIa)-(VIe), (VIIa)-(VIIe), and (IXa)-(IXe), Compounds 1-457, Compounds 458-532, Compounds B1-B25, Compounds W1-W32, Compounds P1-P225, tautomers of those compounds, deuterated derivatives of those compounds and tautomers, and pharmaceutically acceptable salts of any of the foregoing, the following abbreviations are used:

### Abbreviations

BrettPhos Pd G4 = dicyclohexyl-[3,6-dimethoxy-2-[2,4,6-tri(propan-2-yl)phenyl]phenyl]phosphane;methanesulfonic acid;N-methyl-2-phenylaniline;palladium
DIPEA = N,N-Diisopropylethylamine or N-ethyl-N-isopropyl-propan-2-amine
DMA = dimethyl acetamide
DMAP = dimethylamino pyridine
DME = dimethoxyethane
DMF = dimethylformamide
DMSO = dimethyl sulfoxide
EtOH = ethanol
EtOAc = ethyl acetate
HATU = [dimethylamino(triazolo[4,5-b]pyridin-3-yloxy)methylene]-dimethyl-ammonium (Phosphorus Hexafluoride Ion)
MeOH = methanol
MP-TMT scavenger resin = a macroporous polystyrene-bound trimercaptotriazine, a resin bound equivalent of 2,4,6-trimercaptotriazine (TMT).
MTBE = Methyl tert-butyl ether
NMM = N-methyl morpholine
NMP = N-methyl pyrrolidine
Pd(dppf)₂Cl₂ = [1,1'-Bis(diphenylphosphino)ferrocene]dichloropalladium(II)
PdCl₂ = palladium(II) dichloride
PdCl₂(PPh₃)₂ = Bis(triphenylphosphine)palladium(II) dichloride
SFC = super critical fluid chromatography
SPhos Pd G3 = (2-Dicyclohexylphosphino-2',6'-dimethoxybiphenyl) [2-(2'-amino-1,1'-biphenyl)]palladium(II) methanesulfonate
TBAF = Tetrabutylammonium fluoride
*t*BuXPhos Pd G1 = Chloro[2-(di-*tert*-butylphosphino)-2',4',6'-triisopropyl-1,1'-biphenyl][2-(2-aminoethyl)phenyl)]palladium(II) or t-BuXPhos palladium(II) phenethylamine chloride
tBuXPhos Pd G3 = [(2-Di-tert-butylphosphino-2',4',6'-triisopropyl-1,1'-biphenyl)-2-(2'-amino-1,1'-biphenyl)] palladium(II) methanesulfonate
tBuXPhos Pd G4 = ditert-butyl-[2-(2,4,6-triisopropylphenyl)phenyl]phosphane;dichloromethane;methanesulfonate;N-methyl-2-phenyl-aniline palladium (II)
TEA = triethylamine
TFA = trifluoroacetic acid
THF = tetrahydrofuran
XPhos Pd G1 = (2-Dicyclohexylphosphino-2',4',6'-triisopropyl-1,1'-biphenyl)[2-(2-aminoethyl)phenyl)]palladium(II) chloride or (XPhos) palladium(II) phenethylamine chloride

In some embodiments, processes for preparing compounds of Formula (I), tautomers thereof, deuterated derivatives of those compounds and tautomers, or pharmaceutically acceptable salts of any of the foregoing, comprise reacting a compound of Formula (I), tautomer, deuterated derivative, or pharmaceutically acceptable salt with a deprotection reagent as depicted in Schemes 1 through 11 below (wherein all variables are as defined for Formula (I) above):

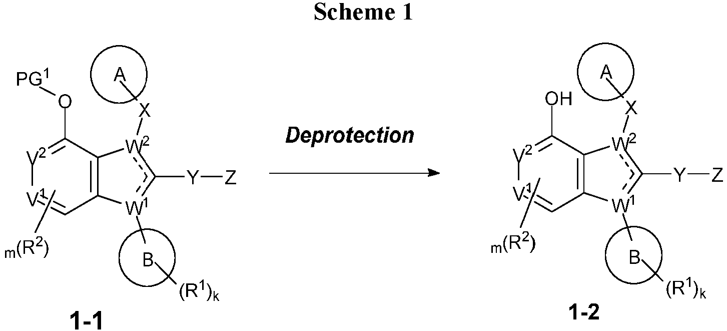

Scheme 1 shows methods for the preparation of a compound of formula **1-2.** PG¹ is an alcohol protecting group such as Benzyl (Bn), Methoxymethyl (MOM), or Methyl (Me). In some examples, where PG¹ is a benzyl group, a compound of formula **1-2** may be prepared by hydrogenolysis of a compound of formula **1-1** using a palladium on carbon catalyst, under an atmosphere of hydrogen. The reaction may be performed at elevated pressure. A solvent such as MeOH, EtOH or EtOAc may be used. Where PG¹ is a group such as MOM, a compound of formula (I) may be prepared by treatment with an acid such as HCl. In examples where PG¹ is a methyl group, the group may be removed by treatment with AlCl₃ in the presence of octanethiol. In some examples, a reagent such as BBr₃ may be used. Any other standard method suitable for the removal of an alcohol protecting group may be used to prepare a compound of formula **1-2** from compounds of formula **1-1.**

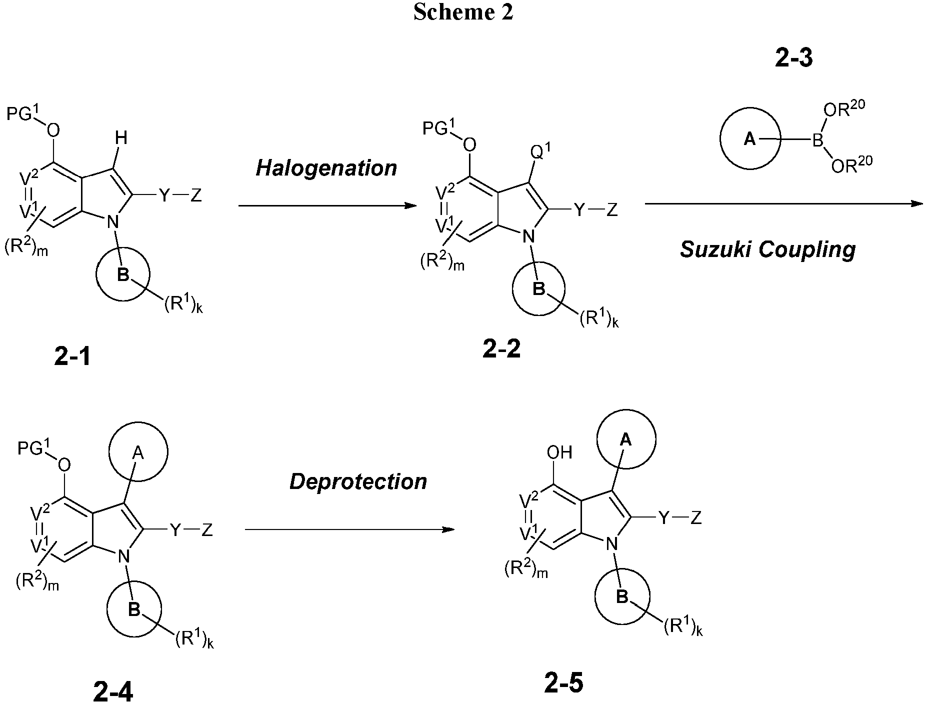

Scheme 2 shows methods for the preparation of compounds of formula **2-5.** Q¹ is a halogen such as Br , I or Cl. Compounds of formula **2-3** are boronic acids or esters with R²⁰ any suitable alkyl group (such a Me, Et), or hydrogen. All other variables are as defined above. Compounds of formula **2-1** may be transformed into compounds of formula **2-2** using any suitable method for the halogenation of an aromatic ring. For example, N-iodosuccinimide (NIS) or N-bromosuccinimide (NBS) in a solvent such as dichloromethane may be used. A compound of formula **2-4** may be prepared from **2-2** and **2-3** using standard Suzuki coupling conditions. In some examples, Suzuki coupling conditions may involve a catalyst such as Pd(dppf)Cl₂ and a base such as Na₂CO₃. In some examples, a catalyst such as Pd₂(dba)₃ in the presence of a ligand such as XPhos may be used. A solvent such as DMF or DME may be used. The reaction is performed in the presence of additional heat (e.g. 90 °C). A compound of formula **2-5** may be prepared from **2-4** using any suitable method for the removal of an alcohol protecting group.

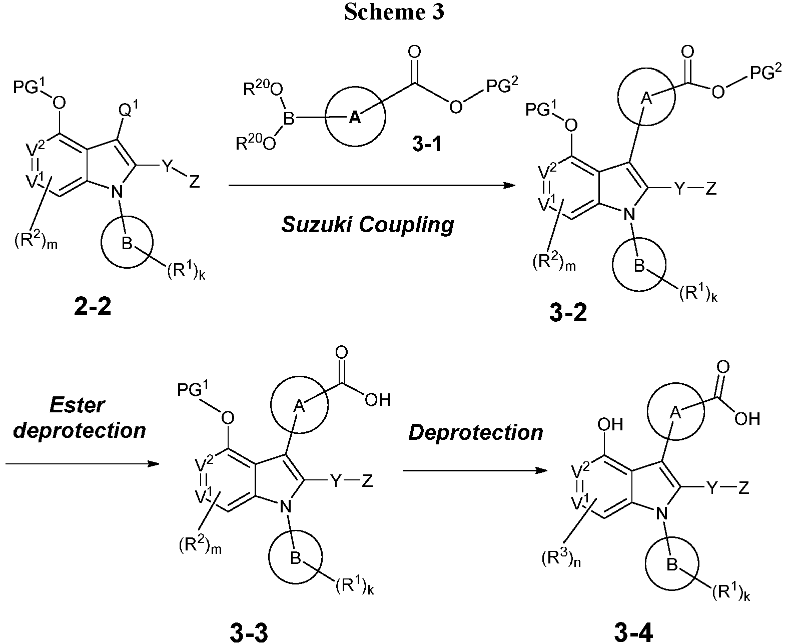

Processes for the preparation of compounds of Formula **3-4** are shown in Scheme **3.** PG² is any suitable carboxylic acid protecting group. For example, PG² may be Me, Et, Benzyl or tert-Butyl. All other variables are defined as above. Compounds of formula **3-2** may be prepared from compounds of formula **3-1** using any suitable method for Suzuki coupling. For example, Pd(dppf)Cl₂ in the presence of Na₂CO₃ may be used. Compounds of formula 3-3 may be prepared from compounds of formula **3-2** using any suitable method for the removal of a carboxylic acid protecting group. For example, where PG² is a methyl ester, hydrolysis with a base such as LiOH or NaOH, in a solvent such as THF and water may be used. Where PG² is a group such as tert-Butyl, treatment with an acid such as TFA or HCl affords compounds of formula **3-3.** In some examples, where PG¹ and PG² are both benzyl groups, a compound of formula **3-4** may be prepared directly from a compound of formula **3-2** by hydrogenation.

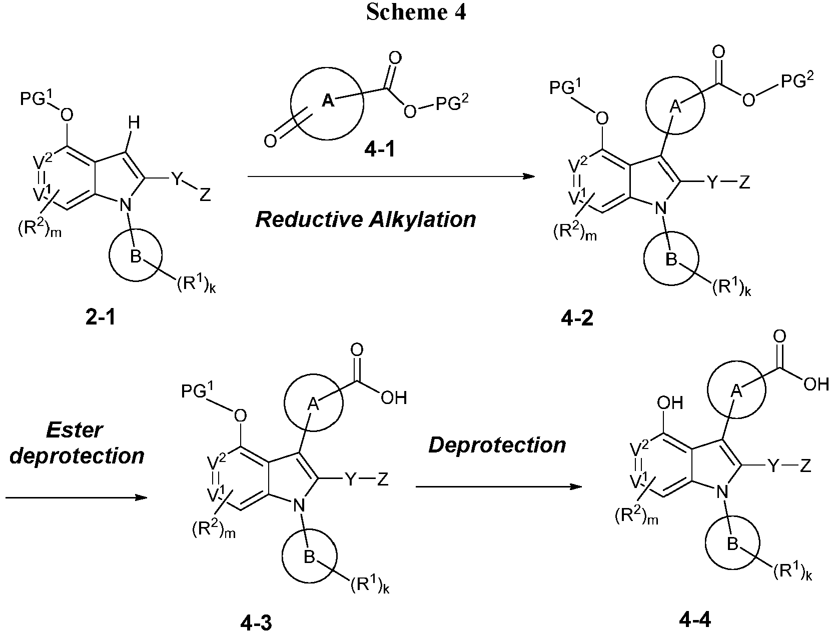

Scheme 4 shows processes for the preparation of compounds of formula **4-4.** All variables are defined as above. Compounds of formula **4-2** may be prepared by reductive alkylation between an indole of formula **2-1** and a ketone of formula **4-1.** In some examples, reductive alkylation may be performed in the presence of a reagent such as triethyl silane and an acid (such as trifluoroacetic acid or methanesulfonic acid). The reaction may be performed in a solvent such as dichloromethane.

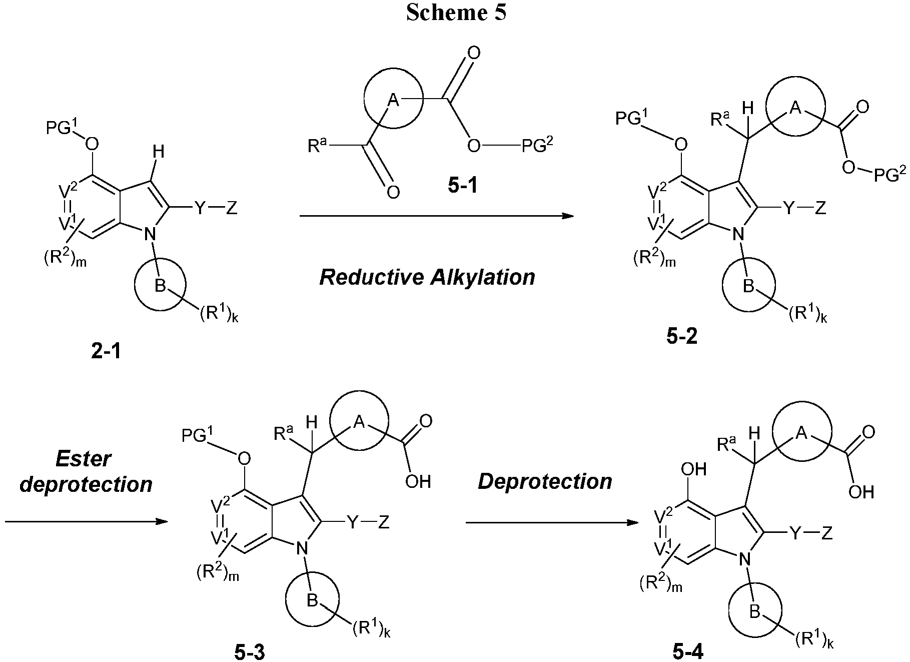

Scheme **5** depicts methods for the preparation of compounds of formula **5-4.** All variables are defined as above. Compound of formula **5-2** may be prepared from ketones or aldehydes of formula **5-1** and indoles of formula **2-1** using any suitable conditions for performing a reductive alkylation reaction. In some examples, the reaction may be performed in the presence of triethyl silane and trifluoroacetic acid. A solvent such as dichloromethane may be used. The reaction may be performed in the presence of added heat (e.g. at 40 °C).

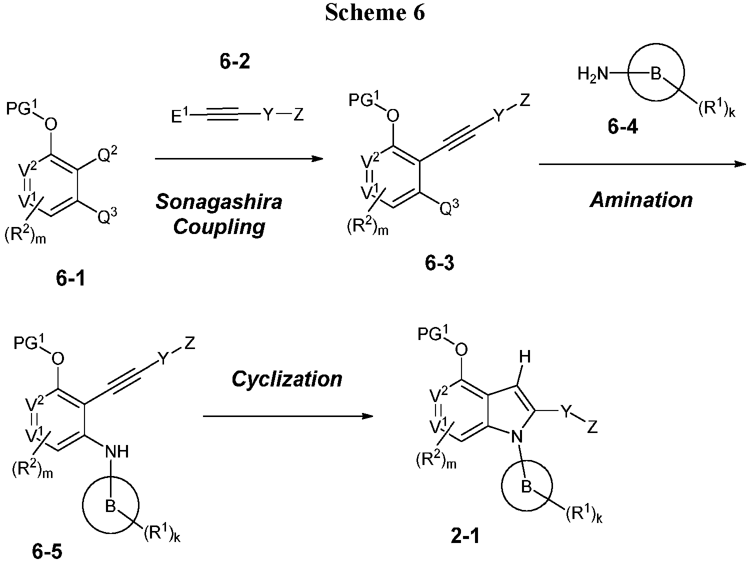

Scheme 6 shows processes for the preparation of indoles of formula **2-1.** Q² and Q³ are halogens such as Br, Cl or I. E¹ is hydrogen or SiMe₃. For example, in some processes Q² is iodine and Q³ is bromine. In some examples, compounds of formula **6-3** may be prepared from compound of formula **6-1** and alkynes of formula **6-2** using any suitable conditions for performing a Sonagashira coupling. In some examples, a catalyst such a Pd(PPh₃)₂Cl₂ in the presence of CuI may be used. A base such as triethylamine or diisopropylethylamine may be used. The reaction may be performed in a solvent such as DMF in the presence of added heat. In some examples, where E¹ is SiMe₃, the reaction may be performed in the presence of TBAF. Compounds of formula **6-5** may be prepared from compounds of formula **6-3** by transition metal catalyzed amination with an amine of formula **6-4.** Amination may be performed in the presence of a palladium catalyst such as tBuXPhos Pd G3, tBu XPhos Pd G, or any other suitable catalyst for performing Buchwald aminations. A base such as NaOtBu may be used. The reaction may be performed in a solvent such as xylene. The reaction may be performed at room temperature, or in the presence of added heat. In some examples, cyclization to compounds of formula **2-1** occurs spontaneously in the amination reaction. In some examples, compounds of formula **2-1** from **6-5** are prepared by treatment with PdCl₂ in a solvent such as MeCN. The reaction may be performed with added heat (e.g. at 50 °C).

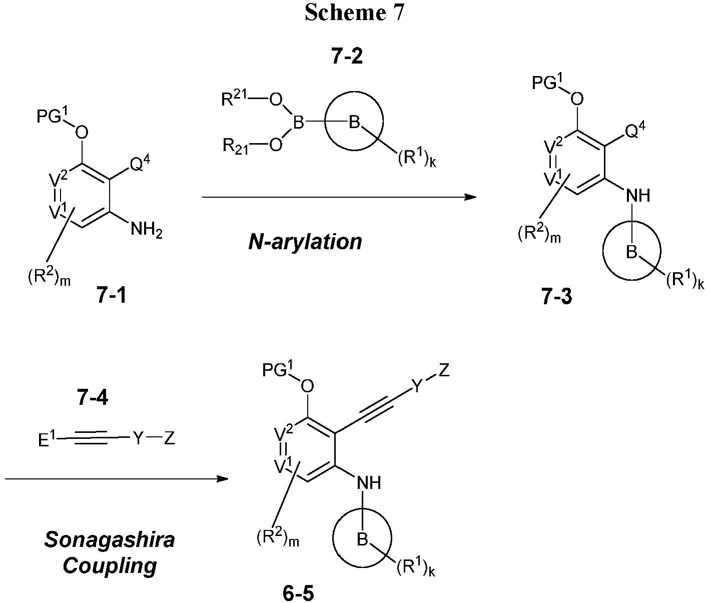

Scheme 7 shows an alternative process for the preparation of a compound of formula **6-5.** Q⁴ is a halogen such as Br or I. R²¹ is a hydrogen or a suitable alkyl group such as ethyl or methyl. An aniline of formula **7-1** may be arylated with a boronic acid or ester **7-2** using any suitable conditions for N-arylation to give a compound of formula **7-3.** In some examples, a Cu(OAc)₂ catalyst may be used. The reaction may be performed in the presence of a base such as K₂CO₃. A solvent such as DMSO may be used. A compound of formula **6-5** may be prepared by Sonagashira coupling of compounds of formula **7-3** with alkynes of formula **7-4** to afford compounds of Formula **6-5.**

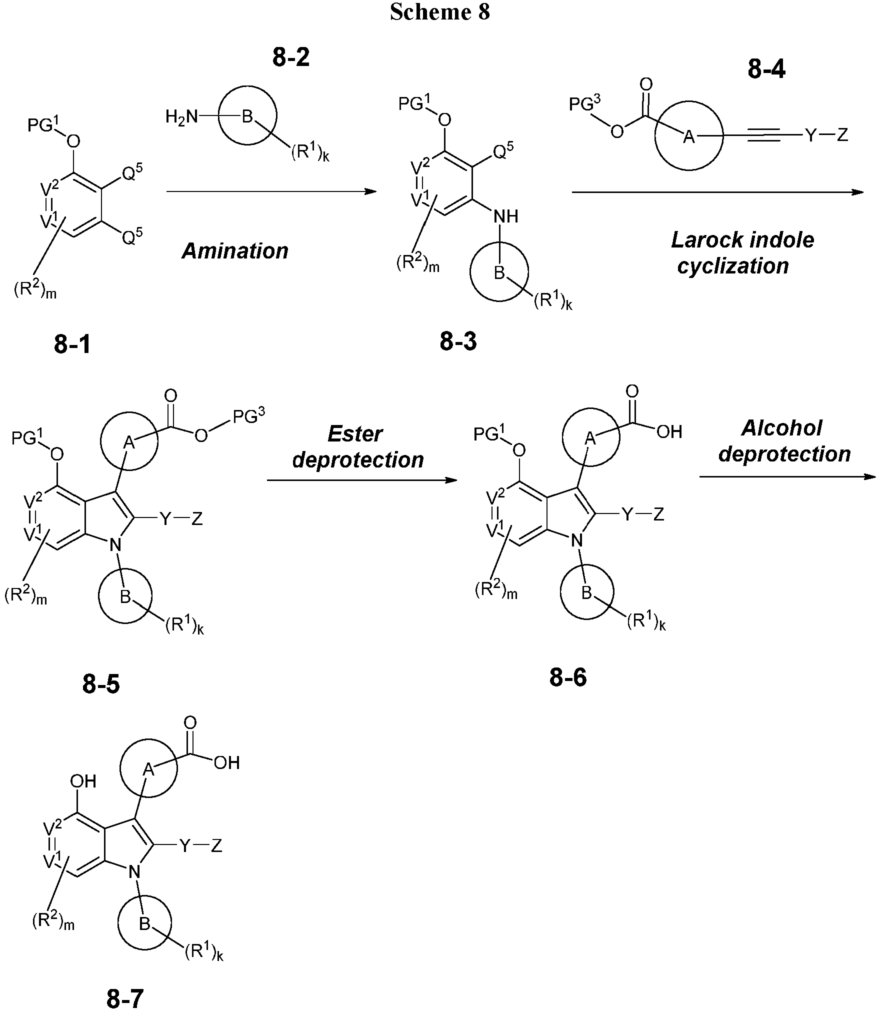

Scheme 8 depicts processes for the preparation of compounds of formula **8-7** from a dihaloaryl of general formula **8-1.** Q⁵ is a halogen such as Cl, Br, or I. In some embodiments, group A is an aromatic or heteroaromatic ring. Amination of compound of formula **8-1** with an amine of formula **8-2** affords compounds of formula **8-3.** Any suitable method for amination of an aryl halide with an amine may be used. For example, the reaction may be performed in the presence of a catalyst such as Pd(OAc)₂ in the presence of a ligand such as dppf. In some examples, the reaction may be performed in the presence of tBuXPhos Pd G1.The reaction may be performed in the presence of a base such as NaOtBu. Indoles of formula **8-5** may be prepared by reaction of compounds of formula **8-3** with disubstituted alkynes of formula **8-4** in the presence of a suitable palladium catalyst. For example, catalysts such as Pd(tBu₃P)₂ or JackiePhos Pd G3 may be used. In some alternative embodiments, Pd(OAc)₂ may be used. The reaction is performed in the presence of a suitable ligand. For example, dicyclohexyl methylamine (cHx)₂NMe may be used. The reaction may be performed in a solvent such as 1,4-dioxane, and in the presence of added heat (e.g.. 60 °C).

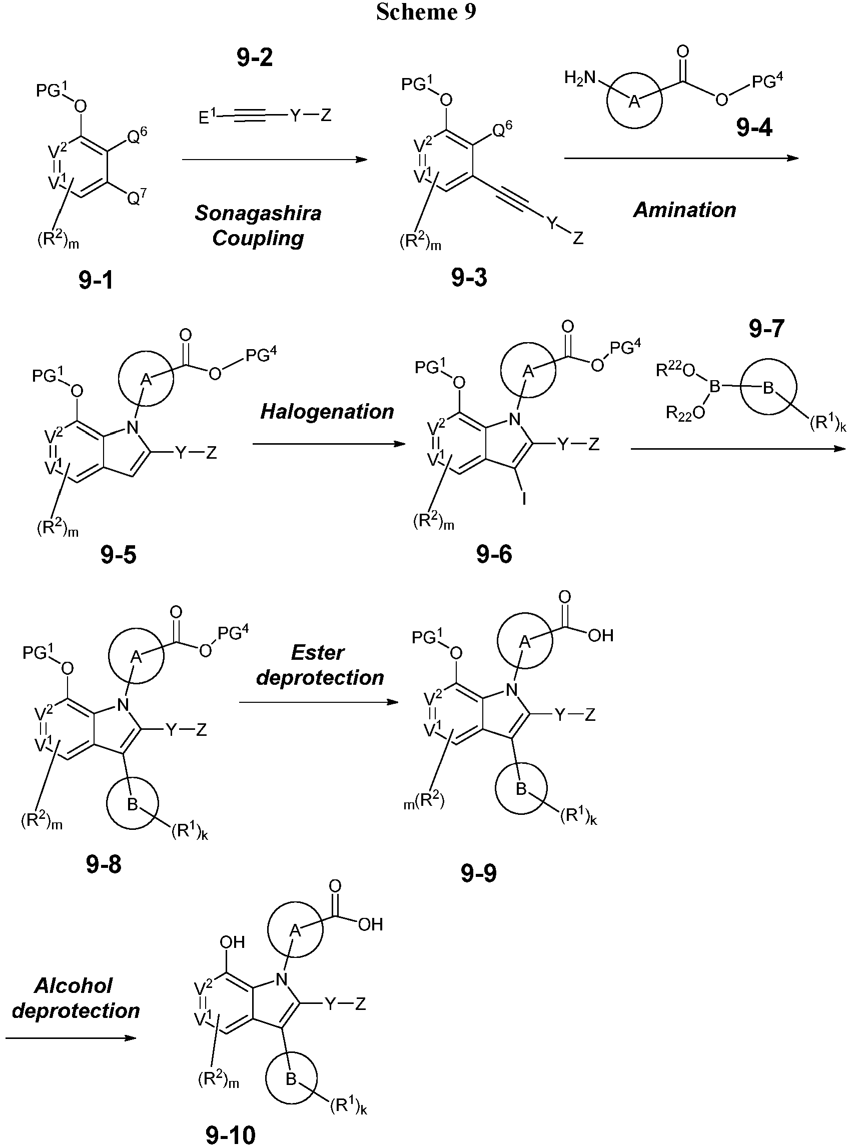

Any suitable conditions for Sonagashira coupling of a compound of formula 9-1 with an alkyne of formula 9-2, as shown in Scheme 9, may be used in the preparation of compounds of formula 9-3. PG⁴ is any suitable ester protecting group (e.g. benzyl, methyl, tert-butyl), All other variables are defined as above. Compounds of formula **9-5** may be prepared from compounds of formula **9-3** and amines of formula **9-4,** using any suitable method for amination of aryl halides. In some embodiments, the reaction is performed in the presence of a tBuXPhos Pd G3 catalyst and NaOtBu. A solvent such as m-xylene may be used. Any suitable halogenating reagent may be used to prepare compounds of formula **9-6** from indoles of formula **9-5.** For example, N-iodosuccinimide or N-bromosuccinimide may be used. In some embodiments, compounds of formula **9-8** may be prepared by Suzuki coupling of compounds of formula **9-7** with compounds of formula **9-6** using a suitable palladium catalyst and a base. For example, Pd(dppf)Cl₂ and K₂CO₃ may be used. Compounds of formula **9-10** may be prepared from compounds of formula **9-8** using standard methods for ester and alcohol protecting group removal, as appropriate for the protecting groups used in that embodiment.

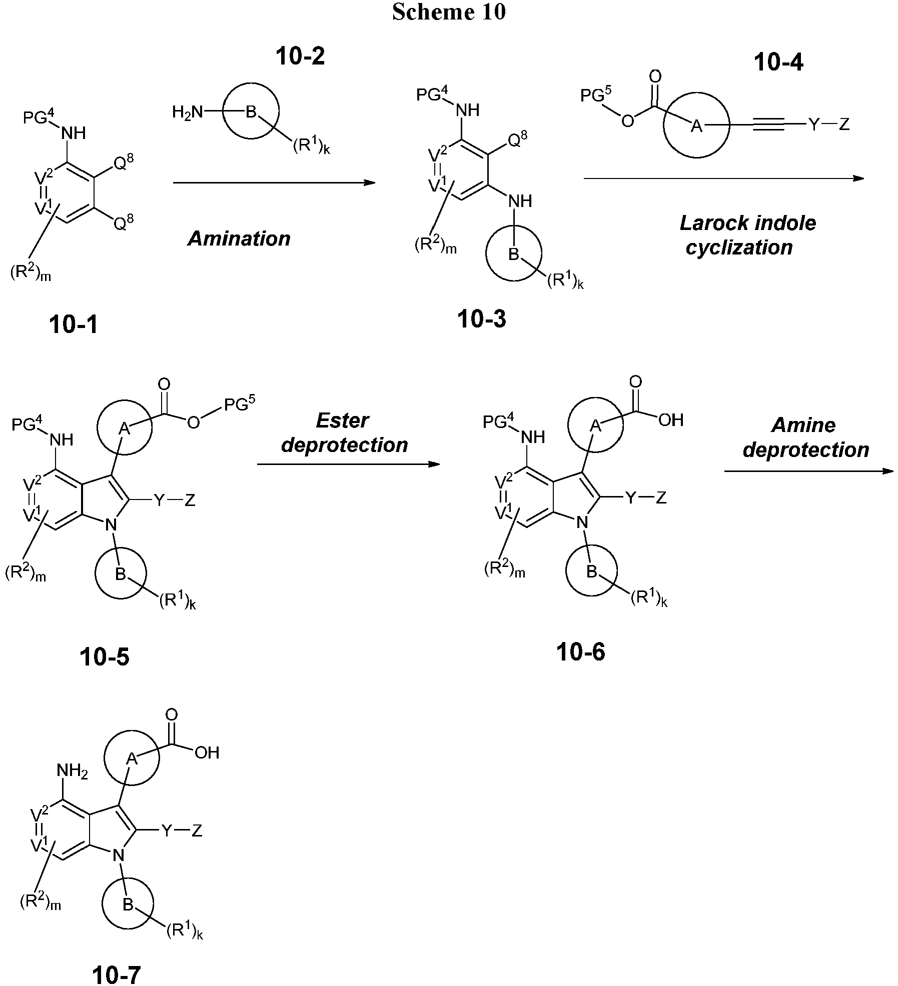

Scheme **10** refers to processes for the preparation of compounds of formula **10-7.** Q⁸ is a halogen such as Br, I, or Cl. PG⁴ is a standard amine protecting group (e.g. Bn, Boc, CBz). PG⁵ is an ester protecting group such as Me, Et, or tert-Butyl. A compound of formula **10-3** may be prepared from a compound of formula **10-1** and an amine of formula **10-2** by Buchwald amination. In some embodiments, a catalyst such as Pd(OAc)₂ with a ligand such as dppf may be used. The reaction may be performed in the presence of a base such as sodium tert-butoxide. A compound of formula **10-5** may be prepared from **10-3** and an alkyne of formula **10-4** using any suitable conditions for Larock indole cyclization. For example, catalysts such as Pd(tBu₃P)₂ or JackiePhos Pd G3 may be used. In some alternative embodiments, Pd(OAc)₂ may be used. The reaction is performed in the presence of a suitable ligand. For example, dicyclohexyl methylamine (cHx)₂NMe may be used. The reaction may be performed in a solvent such as 1,4-dioxane, and in the presence of added heat (e.g. 60 °C).A compound of formula 10-6 may be prepared from **10-5** using any suitable method for the removal of an ester protecting group. For example, where PG⁵ is methyl, a base such as LiOH or NaOH may be used. Any suitable method for the removal of a nitrogen protecting group may be used for the preparation of compound **10-7** from **10-6.** For example, where PG⁴ is a benzyl group hydrogenation using a palladium on carbon catalyst under an atmosphere of hydrogen may be used. In some embodiments, the reaction may be performed in a solvent such as THF.

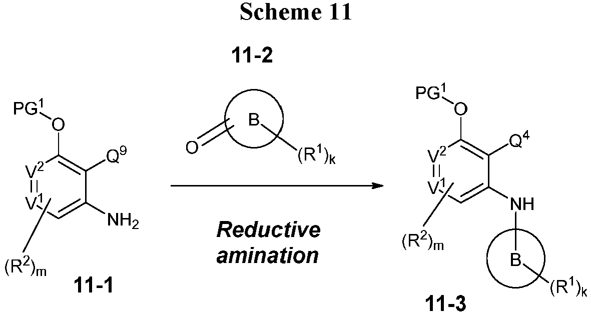

Scheme **11** refers to an alternative method of preparation of compounds of formula **11-3.** Ring B is an alkyl or alkoxy group. Reductive amination reaction between amines of formula **11-1** with a ketone or aldehyde of formula **11-2** affords compounds of formula **11-3.** In some examples, the reductive amination may be performed using a reagent such as sodium triacetoxyborohydride. A solvent such as AcOH may be used. The reaction may be performed at room temperature.

### EXAMPLES

In order that the disclosure described herein may be more fully understood, the following examples are set forth. It should be understood that these examples are for illustrative purposes only and are not to be construed as limiting this disclosure in any manner. The invention is defined by the claims.

### Example 1. Synthesis of Compounds

All the specific and generic compounds, the methods for making those compounds, and the intermediates disclosed for making those compounds, are considered to be part of the disclosure disclosed herein.

### A. Synthesis of Starting Materials

Preparations of **S1-S22** describe synthetic routes to intermediates used in the synthesis of Compound 1-457.

### Preparation of S1

### 4-benzyloxy-1-(3,4-difluorophenyl)-3-iodo-2-tetrahydropyran-4-yl-indole (S1)

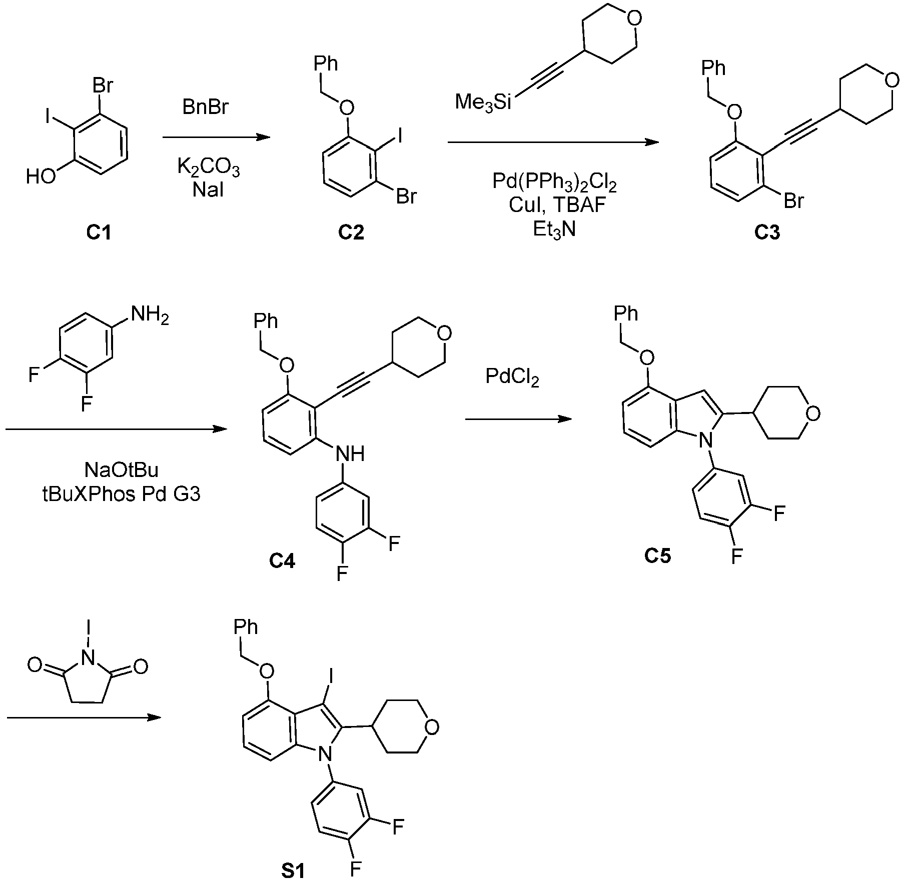

### Step 1. Synthesis of 1-benzyloxy-3-bromo-2-iodo-benzene (C2)

A solution of 3-bromo-2-iodo-phenol C1 (129 g, 431.6 mmol) in acetone (1.5 L) was stirred for 5 minutes. K₂CO₃ (75 g, 542.7 mmol), NaI (21 g, 140.1 mmol) and bromomethylbenzene (55 mL, 462.4 mmol) were added. The reaction mixture was stirred at 55 °C for 7 hours. The mixture was then cooled to room temperature, filtered, and washed with acetone (2 x 100 mL). The combined filtrates were concentrated *in vacuo.* The residue was dissolved in dichloromethane (1.5 L), washed with water (2 x 100 mL) and brine (100 mL). The organic phase was dried over MgSO₄, filtered and concentrated *in vacuo.* Purification by silica gel chromatography (0- 50% ethyl acetate in heptane) afforded the product as a white solid (162 g, 96%). ¹H NMR (300 MHz, Chloroform-*d*) δ 7.54 - 7.46 (m, 2H), 7.40 (ddd, J = 7.9, 7.0, 1.1 Hz, 2H), 7.37 - 7.31 (m, 1H), 7.28 (dd, J = 8.0, 1.3 Hz, 1H), 7.15 (t, J = 8.1 Hz, 1H), 6.76 (dd, J = 8.2, 1.3 Hz, 1H), 5.16 (s, 2H).

### Step 2. Synthesis of 4-[2-(2-benzyloxy-6-bromo-phenyl)ethynyl]tetrahydropyran (C3)

1-benzyloxy-3-bromo-2-iodo-benzene **C2** (200 g, 514.1 mmol), trimethyl(2-tetrahydropyran-4-ylethynyl)silane (140 g, 767.8 mmol), 1,4-dioxane (1200 mL), NEt₃ (430 mL, 3.09 mol), TBAF (720 mL of 1 M, 720.0 mmol solution in THF) and water (20 mL, 1.1 mol). The mixture was purged with N₂ for 10 minutes, and CuI (11 g, 57.8 mmol) was added. After purging with N₂, for a further 10 minutes, PdCl₂(PPh₃)₂ (22 g, 31.3 mmol) was added. The mixture was heated at 65 °C, After 6.5 hours, the heat was removed and the mixture stirred at room temperature overnight. Water (1 L) and saturated NH₄Cl (500 mL) were added, the mixture was stirred for 10 minutes, then split into two equal portions. Each portion was extracted with EtOAc (1.5 L), and the organic extract was washed successively with 1 M aq HCl (1 L), brine (1 L), then dried (MgSO₄) filtered and concentrated. The two portions were combined, dissolved in dichloromethane (300 mL). Silica gel chromatography (1.6 kg silica gel. Gradient: 0-40% EtOAc in heptane) afforded the product as an amber oil (151 g, 79%). ¹H NMR (300 MHz, Chloroform-*d*) δ 7.33 - 7.25 (m, 2H), 7.25 - 7.10 (m, 3H), 7.03 (dd, J = 8.1, 1.0 Hz, 1H), 6.89 (t, J = 8.2 Hz, 1H), 6.68 (dd, J = 8.3, 1.0 Hz, 1H), 4.97 (s, 2H), 3.79 (ddd, J = 11.6, 6.9, 3.4 Hz, 2H), 3.40 (ddd, J = 11.2, 7.2, 3.3 Hz, 2H), 2.84 (tt, J = 7.7, 4.2 Hz, 1H), 1.85 - 1.70 (m, 2H), 1.61 (dtd, J = 13.2, 7.3, 3.2 Hz, 2H).

### Step 3. Synthesis of 3-benzyloxy-N-(3, 4-difluorophenyl)-2-(2-tetrahydropyran-4-ylethynyl)aniline (C4)

A solution of 4-[2-(2-benzyloxy-6-bromo-phenyl)ethynyl]tetrahydropyran **C3** (262 g, 705.7 mmol) in m-xylene (3.9 L) was purged with nitrogen for 15 minutes. NaOtBu (200 g, 2.08 mol) was added, then N₂ bubbling was continued for 15 minutes. 3,4-difluoroaniline (84 mL) was added, and N₂ purging was continued for 15 minutes. tBuXPhos Pd G3 (15 g, 18.9 mmol) was added, then N₂ purging was continued for another 10 minutes. The mixture was allowed to stir, and after 90 minutes the internal temperature has risen from 21 °C to 29 °C. After a further 2.5 hours, internal temperature dropped to 24 °C. The mixture was then heated to 50 °C for 45 minutes. Additional tBuXPhos Pd G3 (2.0 g, 2.52 mmol) was added and the mixture stirred a further 2 hours at 50 °C. The reaction mixture was poured onto ice-water (6 L), then 2-MeTHF (3 L) was added. Following agitation, layers were separated. The organic layer was washed successively with water (4 L), 1 M aq HCl (3 L), saturated aqueous NaHCO₃ (3 L), and brine (3 L). The organic layer was then dried (MgSO₄) filtered and concentrated *in vacuo.* Purification by silica gel chromatography (3 kg silica gel. Gradient: 0-40% EtOAc in heptane) afforded the product 3-benzyloxy-N-(3,4-difluorophenyl)-2-(2-tetrahydropyran-4-ylethynyl)aniline **C4** in a 2:1 mixture with the cyclized product **C5** (277 g, 94%) as a brown oil. The mixture was carried to the subsequent step without further purification.

### Step 4. Synthesis of 4-benzyloxy-1-(3,4-difluorophenyl)-2-tetrahydropyran-4-yl-indole (C5)

A solution of 3-benzyloxy-N-(3,4-difluorophenyl)-2-(2-tetrahydropyran-4-ylethynyl)aniline (277 g, 660.4 mmol) (mixture of ~2:1 aniline **C4:** indole **C5**) in MeCN (1.9 L) was purged with nitrogen for 15 minutes. PdCl₂ (3.5 g, 19.7 mmol) was added, reaction was placed under a positive pressure of N₂, then heated to 50 °C for 1 hour. The heat was removed, and the reaction mixture was stirred overnight. After 16 hours, the resulting suspension was filtered, and the solid collected, washing with heptane (400 mL), and drying under suction to afford ~150 g of an off-white solid. The filtrate was concentrated, to give ~100 g of product as a dark oil which was purified by silica gel chromatography (1.6 kg silica gel column, Gradient: 0-40% EtOAc in heptane) (Note: compound does precipitate out on column resulting in product loss) to afford additional product (~90 g) as an off-white solid. This portion of the product from column chromatography was combined with product isolated from reaction mixture. The mixture was treated with EtOAc (300 mL), the slurry was heated to reflux, and then heptane (1 L) was added. The mixture was then allowed to stand at room temperature for 2 hours, then filtered, and the collected solid was washed with heptane (300 mL). The product was dried under vacuum to afford the product (224.6 g) as slightly off-white crystals. The filtrate yielded an additional 8.0 g off-white crystals, which were combined with the first crop to afford the product 4-benzyloxy-1-(3,4-difluorophenyl)-2-tetrahydropyran-4-yl-indole (232.6 g, 84%). ¹H NMR (300 MHz, Chloroform-*d*) δ 7.59 - 7.51 (m, 2H), 7.48 - 7.31 (m, 4H), 7.24 (ddd, J = 10.4, 7.0, 2.5 Hz, 1H), 7.14 (dddd, J = 8.7, 4.1, 2.5, 1.6 Hz, 1H), 7.09 - 7.00 (m, 1H), 6.66 (d, J = 0.7 Hz, 1H), 6.64 (s, 2H), 5.26 (s, 2H), 4.00 (ddd, J = 11.7, 4.2, 1.8 Hz, 2H), 3.37 (td, J = 11.7, 2.4 Hz, 2H), 2.80 (tt, J = 11.4, 4.0 Hz, 1H), 1.95 - 1.66 (m, 4H). 19F NMR (282 MHz, Chloroform-*d)* δ -134.09 (d, J = 21.5 Hz), -136.75 (d, J = 21.7 Hz). LCMS *m*/*z* 419.9 [M+1]⁺.

### Step 5. Synthesis of 4-benzyloxy-1-(3, 4-difluorophenyl)-3-iodo-2-tetrahydropyran-4-yl-indole (S1)

To a solution of 4-benzyloxy-1-(3,4-difluorophenyl)-2-tetrahydropyran-4-yl-indole **C5** (159 g, 379.1 mmol) in CH₂Cl₂ (2.5 L) cooled to 0 °C (ice/water bath), was added 1-iodopyrrolidine-2,5-dione (96 g, 413.9 mmol) in three portions over 10 minutes. The resulting reaction mixture was stirred at 0 °C for 2 hours. The reaction mixture was treated with water (600 mL) and 1 M aqueous Na₂S₂O₃ (600 mL). The organic layer was separated, washed successively with saturated aqueous NaHCO₃ (~600 mL) and brine (~600 mL each). The organic layer was dried (MgSO₄), filtered and concentrated. The residue was treated with EtOAc (~200 mL), heated to reflux for 15 minutes, to afford a suspension. Suspension was treated with heptane (~1 L), the resulting suspension was slowly allowed to room temperature over 14 hours (overnight), then filtered. The collected solid was washed with heptane (100 mL), then dried under vacuum oven at 45 °C for 2 hours to afford the product as a tan solid (180 g, 86%). ¹H NMR (300 MHz, DMSO-*d*₆) δ 7.81 - 7.54 (m, 4H), 7.49 - 7.37 (m, 2H), 7.37 - 7.26 (m, 2H), 6.99 (t, J = 8.1 Hz, 1H), 6.73 (d, J = 7.9 Hz, 1H), 6.43 (d, J = 8.2 Hz, 1H), 5.26 (s, 2H), 3.86 (dd, J = 11.5, 4.1 Hz, 2H), 3.21 (ddd, J = 12.0, 9.8, 5.1 Hz, 2H), 3.06 - 2.84 (m, 1H), 2.19 (dt, J = 12.7, 4.7 Hz, 2H), 1.56 (s, 2H). ¹⁹F NMR (282 MHz, DMSO-*d*₆) δ -135.42, -135.50, -136.83, - 136.91. LCMS *m*/*z* 545.21 [M+1]⁺.

### Preparation of S2

### 4-benzyloxy-1-(3,4-difluorophenyl)-5-fluoro-3-iodo-2-tetrahydropyran-4-yl-indole (S2)

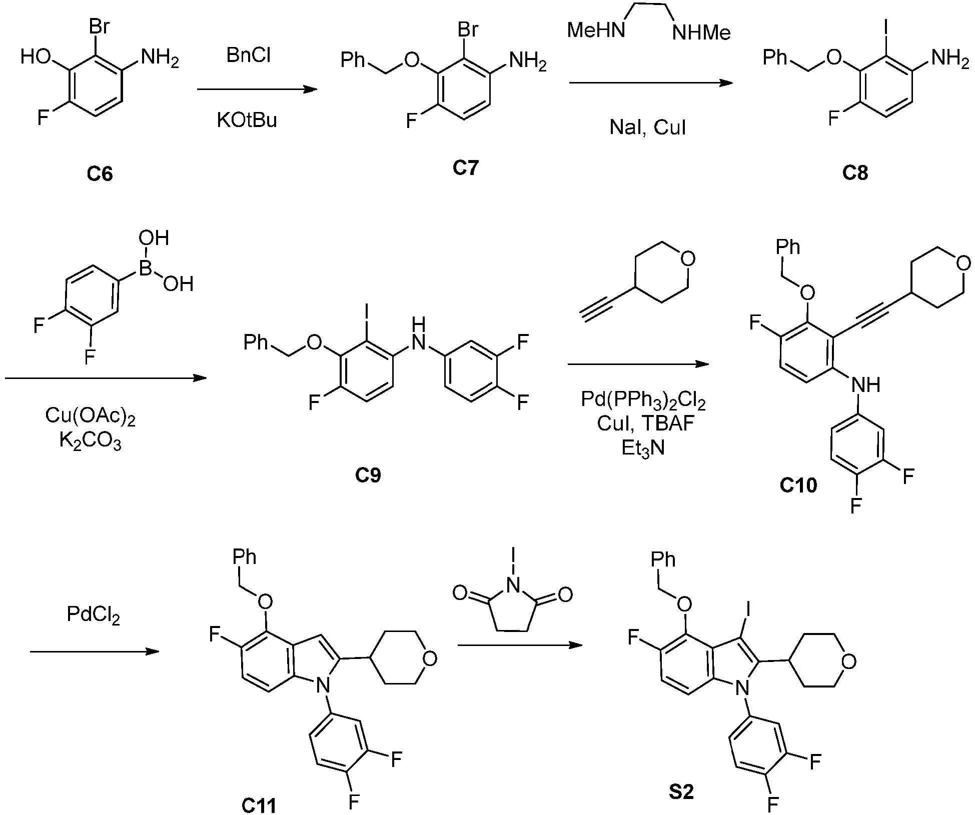

### Step 1. Synthesis of 3-benzyloxy-2-bromo-4-fluoro-aniline C7

KOtBu (8.68 g, 77.4 mmol) was added to a solution of 3-amino-2-bromo-6-fluorophenol **C6** (15.6 g, 75.8 mmol), chloromethylbenzene (9.6 g, 75.8 mmol) in DMF (120 mL). The reaction was stirred at room temperature overnight. The reaction was concentrated, diluted with EtOAc and washed with water. The organic layer was dried and concentrated. The crude was purified on silica gel (Gradient: 10-40% EtOAc in hexane) to afford the product. 3-benzyloxy-2-bromo-4-fluoro-aniline (17.8 g, 75%) LCMS *m*/*z* 295.94 [M+H]⁺.

### Step 2. Synthesis of 3-benzyloxy-4-fluoro-2-iodo-aniline (C8)

3-benzyloxy-2-bromo-4-fluoro-aniline (7.36 g, 24.9 mmol), NaI (15 g, 100.1 mmol), N,N'-dimethylethane-1,2-diamine (780 mg, 8.9 mmol) and CuI (980 mg, 5.146 mmol) were mixed into 1,4-dioxane (60 mL) and the reaction was stirred in a sealed vessel and heated at 140 degrees for overnight. The reaction was cooled to room temperature and diluted with EtOAc (100 ml) and filtered through a plug of Celite^{®}. The filtrate was washed with water, brine and dried over Na₂SO₄. The solvent was removed and the crude was purified on silica gel (220 g column, 10-90% EtOAc in hexane) to afford desired product. 3-benzyloxy-4-fluoro-2-iodoaniline (7.5 g, 84%) LCMS *m*/*z* 344.17 [M+H]⁺.

### Step 3. Synthesis of 3-benzyloxy-N-(3,4-difluorophenyl)-4-fluoro-2-iodo-aniline (C9)

3-benzyloxy-4-fluoro-2-iodo-aniline (2.68 g, 7.810 mmol), (3,4-difluorophenyl)boronic acid (1.82 g, 11.53 mmol), K₂CO₃ (3.2 g, 23.15 mmol) and diacetoxycopper (1.68 g, 9.25 mmol) were mixed into DMSO (20 mL) and the reaction was stirred at room temperature for overnight. The reaction was diluted with EtOAc and filtered through a plug of Celite^{®}. The filtrate was washed with water. The organic layer was dried and concentrated. The crude was purified on silica gel (120 g column, 10-40% EtOAc in hexane) to afford desired product. 3-benzyloxy-N-(3,4-difluorophenyl)-4-fluoro-2-iodo-aniline (1.8 g, 41%). LCMS *m*/*z* calc. 455.87 [M+H]⁺.

### Step 4. Synthesis of 3-benzyloxy-N-(3, 4-difluorophenyl)-4-fluoro-2-(2-tetrahydropyran-4-ylethynyl)aniline (C10)

3-benzyloxy-N-(3,4-difluorophenyl)-4-fluoro-2-iodo-aniline (1.8 g, 3.56 mmol), 4-ethynyltetrahydropyran (600 mg, 5.45 mmol), PdCl₂(PPh₃)₂ (400 g, 569.9 mmol) and CuI (110 mg, 0.58 mmol) were mixed into 1,4-dioxane (10 mL), Et₃N (10 mL) and the reaction was degassed with nitrogen for 30 seconds. The reaction was stirred at room temperature overnight. The reaction was concentrated and diluted with EtOAc and washed with water. The organic layer was dried and concentrated. The crude was purified on silica gel (4 g column, 10-40% Hex: EtOAc) to afford desired product. 3-benzyloxy-N-(3,4-difluorophenyl)-4-fluoro-2-(2-tetrahydropyran-4-ylethynyl)aniline (1.2 g, 75%) LCMS *m*/*z* 438.1 [M+1]⁺.

### Step 5. Synthesis of 4-benzyloxy-1-(3, 4-difluorophenyl)-5-fluoro-2-tetrahydropyran-4yl-indole (C11)

3-benzyloxy-N-(3,4-difluorophenyl)-4-fluoro-2-(2-tetrahydropyran-4-ylethynyl)aniline (1.6 g, 3.658 mmol) was dissolved into MeCN (20 mL) and PdCl₂ (120 mg, 0.68 mmol) was added. The reaction was heated at 45 degrees for overnight. The reaction was cooled to room temperature and filtered through a plug of Celite^{®} . The crude was diluted with EtOAc and washed with water. The organic layer was dried and concentrated. Purification by silica gel chromatography (40 g column, 10-90% EtOAc in hexane) afforded the product. 4-benzyloxy-1-(3,4-difluorophenyl)-5-fluoro-2-tetrahydropyran-4-yl-indole (1.4 g, 77%). ¹H NMR (400 MHz, DMSO-*d*₆) δ 7.76 (ddd, J = 11.2, 7.2, 2.6 Hz, 1H), 7.68 (dt, J = 10.6, 8.9 Hz, 1H), 7.58 - 7.49 (m, 2H), 7.47 - 7.39 (m, 2H), 7.39 - 7.29 (m, 2H), 6.95 (dd, J = 11.8, 8.8 Hz, 1H), 6.65 (ddd, J = 8.8, 3.5, 0.8 Hz, 1H), 6.60 (d, J = 0.8 Hz, 1H), 5.29 (s, 2H). LCMS *m*/*z* 438.06 [M+1]⁺

### Step 6. Synthesis of 4-benzyloxy-1-(3, 4-difluorophenyl)-5-fluoro-3-iodo-2-tetrahydropyran-4-yl-indole (S2)

A solution of 4-benzyloxy-1-(3,4-difluorophenyl)-5-fluoro-2-tetrahydropyran-4-yl-indole (730 mg, 1.33 mmol) in dichloromethane (10 mL) and 1-iodopyrrolidine-2,5-dione (345 mg, 1.533 mmol) was stirred at room temperature for overnight. The reaction was concentrated and diluted with EtOAc and washed with water. The organic layer was dried and concentrated. The crude was purified on silica gel (4 g column, 10-40% Hex: EtOAc) to afford desired product. 4-benzyloxy-1-(3,4-difluorophenyl)-5-fluoro-3-iodo-2-tetrahydropyran-4-yl-indole (720 mg, 81%). ¹H NMR (400 MHz, DMSO-*d*₆) δ 7.80 (ddd, J = 11.1, 7.3, 2.6 Hz, 1H), 7.71 (dt, J = 10.6, 8.9 Hz, 1H), 7.64 - 7.57 (m, 2H), 7.50 - 7.32 (m, 4H), 7.06 (dd, J = 11.5, 8.9 Hz, 1H), 6.61 (dd, J = 8.9, 3.7 Hz, 1H), 5.13 (s, 2H), 3.87 (dd, J = 11.5, 4.2 Hz, 2H), 3.22 (tdd, J = 12.0, 7.2, 2.0 Hz, 2H), 2.93 (tt, J = 12.4, 3.6 Hz, 1H), 2.21 (tq, J = 16.6, 5.7, 4.4 Hz, 2H), 1.58 (t, J = 10.1 Hz, 2H). LCMS *m*/*z* 563.08 [M+H]⁺.

### Preparation of S3

### 4-benzyloxy-1-(3,4-difluorophenyl)-6-fluoro-3-iodo-2-tetrahydropyran-4-yl-indole (S3)

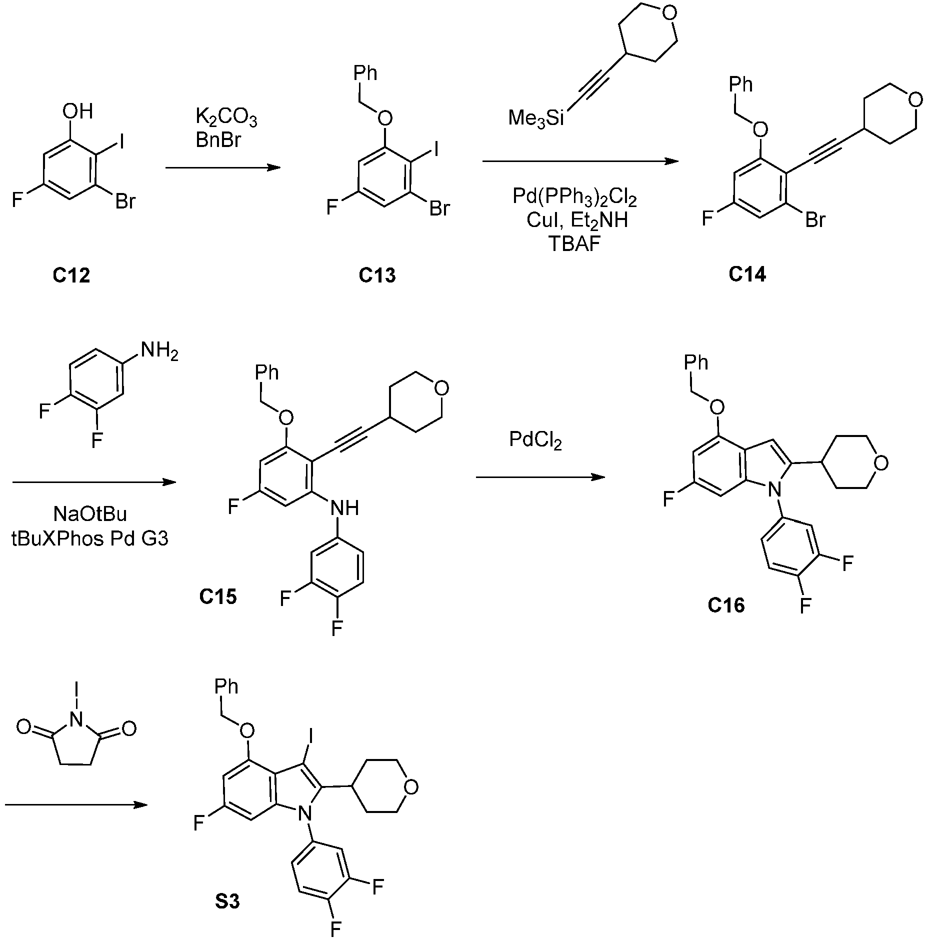

### Step 1. Synthesis of 1-benzyloxy-3-bromo-5-fluoro-2-iodo-benzene (C13)

A mixture of 3-bromo-5-fluoro-2-iodo-phenol **C12** (50 g, 157.8 mmol), bromomethylbenzene (27.8 g, 162.5 mmol), NaI (4 g, 26.7 mmol) and K₂CO₃ (45 g, 325.6 mmol) in acetone (500 mL) was stirred at room temperature overnight. The reaction mixture was then filtered over Celite^{®} and the filtrate was concentrated to dryness, Purification by silica gel chromatography (Gradient: 0-40% CH₂Cl₂ in heptane) afforded the product as a white solid. 1-benzyloxy-3-bromo-5-fluoro-2-iodo-benzene (55 g, 81%). LCMS *m*/*z* 406.56 [M+1]⁺.

### Step 2. Synthesis of 4-[2-(2-benzyloxy-6-bromo-4-fluoro-phenyl)ethynyl]tetrahydropyran (C14)

A solution of 1-benzyloxy-3-bromo-5-fluoro-2-iodo-benzene **C13** (19 g, 46.7 mmol), PdCl₂(PPh₃)₂ (1.98 g, 2.8 mmol) and CuI (890 mg, 4.673 mmol) in anhydrous DMF (150 mL) was degassed for 10 minutes. Trimethyl(2-tetrahydropyran-4-ylethynyl)silane (9.78 g, 53.64 mmol) and diethylamine (7.22 mL, 69.8 mmol) were then added, followed by TBAF (54 mL of 1 M, 54.00 mmol in THF). The reaction mixture was stirred overnight at 65 °C. Upon cooling to room temperature, the mixture was diluted with water and extracted with EtOAc. The organic layer was concentrated to dryness. Purification by silica gel chromatography (Gradient: 0-30% EtOAc in heptane) afforded the product as a light yellow solid. 4-[2-(2-benzyloxy-6-bromo-4-fluoro-phenyl)ethynyl]tetrahydropyran (11.3 g, 62%). ¹H NMR (400 MHz, Chloroform-d) δ 7.51 - 7.42 (m, 2H), 7.41 - 7.35 (m, 3H), 6.96 (dd, J = 8.1, 2.4 Hz, 1H), 6.61 (dd, J = 10.3, 2.4 Hz, 1H), 5.10 (s, 2H), 3.94 (ddd, J = 11.5, 6.9, 3.3 Hz, 2H), 3.61 - 3.53 (m, 2H), 2.99 (tt, J = 7.7, 4.2 Hz, 1H), 1.97 - 1.88 (m, 2H), 1.83 - 1.72 (m, 2H).

### Step 3. Synthesis of 3-benzyloxy-N-(3, 4-difluorophenyl)-5-fluoro-2-(2-tetrahydropyran-4-ylethynyl)aniline (C15)

A solution of 4-[2-(2-benzyloxy-6-bromo-4-fluoro-phenyl)ethynyl]tetrahydropyran C14 (2 g, 5.14 mmol) and 3,4-difluoroaniline (930 mg, 7.2 mmol) in xylene (30 mL) (light brown solution) was purged with nitrogen for 10 minutes, then NaOtBu (1.5 g, 15.61 mmol) and tBuXPhos Pd G3 (200 mg, 0.25 mmol) was added. The reaction was stirred at room temperature for 2 hours. The reaction was cooled to room temperature, then ice water (20 mL) and EtOAc (50 mL) were added. The organic layer was separated, washed with brine, dried and concentrated. Purification by silica gel chromatography (Gradient: 0 - 30% EtOAc in hexane) afforded the product as a light yellow solid. 3-benzyloxy-N-(3,4-difluorophenyl)-5-fluoro-2-(2-tetrahydropyran-4-ylethynyl)aniline (2.2 g, 97%). ¹H NMR (400 MHz, Chloroform-d) δ 7.54 - 7.45 (m, 2H), 7.42 (ddd, J = 7.9, 7.0, 1.1 Hz, 2H), 7.39 - 7.31 (m, 1H), 7.16 (dt, J = 10.0, 8.8 Hz, 1H), 7.06 (ddd, J = 11.6, 6.9, 2.7 Hz, 1H), 6.92 (dddd, J = 8.5, 4.0, 2.6, 1.6 Hz, 1H), 6.50 (s, 1H), 6.43 (dd, J = 11.0, 2.3 Hz, 1H), 6.21 (dd, J = 10.5, 2.3 Hz, 1H), 5.13 (s, 2H), 3.94 (ddd, J = 11.6, 6.2, 3.5 Hz, 2H), 3.56 (ddd, J = 11.4, 8.0, 3.1 Hz, 2H), 3.01 (tt, J = 8.2, 4.1 Hz, 1H), 2.03 - 1.89 (m, 2H), 1.78 (dtd, J = 13.3, 8.1, 3.5 Hz, 2H). LC-MS *m*/*z* 438.3 [M+1]⁺.

### Step 4. Synthesis of 4-benzyloxy-1-(3, 4-difluorophenyl)-6-fluoro-2-tetrahydropyran-4-yl-indole (C16)

To a solution of 3-benzyloxy-N-(3,4-difluorophenyl)-5-fluoro-2-(2-tetrahydropyran-4-ylethynyl)aniline **C15** (14.5 g, 33.0 mmol) in MeCN (150 mL) (light brown solution) was added PdCl₂ (600 mg, 3.4 mmol). The reaction was heated at 60 °C for 12 hours. The solution were concentrated to dryness and purified by silica gel chromatography (Gradient: 0-25% EtOAc in heptane) to give a white solid. 4-benzyloxy-1-(3,4-difluorophenyl)-6-fluoro-2-tetrahydropyran-4-yl-indole (13.6 g, 94%) LCMS *m*/*z* 438.3 [M+1]⁺.

### Step 5. Synthesis of 4-benzyloxy-1-(3, 4-difluorophenyl)-6-fluoro-3-iodo-2-tetrahydropyran-4-yl-indole (S3)

To a solution of 4-benzyloxy-1-(3,4-difluorophenyl)-6-fluoro-2-tetrahydropyran-4-yl-indole **C16** (46.7 g, 106.8 mmol) in dichloromethane (875 mL) at 3.5 °C (ice-water bath) was added N-iodosuccinimide (26.4 g, 117.3 mmol). The ice-water bath was slowly allowed to warm to room temperature and the reaction allowed to stir for 18 hours. The reaction mixture was washed successively with 1 M aq sodium thiosulfate, saturated aqueous NaHCO₃, and brine (800 mL each), then dried (MgSO₄), filtered and concentrated. The residue was treated with EtOAc (100 mL), and the resulting suspension was spun on a rotary evaporator at 75 °C for 1 hour. The suspension was treated with heptane (100 mL), then allowed to stand at room temperature for 2 hours. The resulting crystals were isolated via filtration, washing with heptane (100 mL), and then dried under suction to afford the product as an off-white solid. 4-benzyloxy-1-(3,4-difluorophenyl)-6-fluoro-3-iodo-2-tetrahydropyran-4-yl-indole (54.6 g, 91%). ¹H NMR (300 MHz, DMSO-*d*₆) δ 7.82 - 7.56 (m, 4H), 7.49 - 7.38 (m, 2H), 7.38 - 7.26 (m, 2H), 6.70 (dd, J = 12.0, 2.1 Hz, 1H), 6.24 (dd, J = 9.4, 2.1 Hz, 1H), 5.28 (s, 2H), 3.85 (dd, J = 11.4, 4.1 Hz, 2H), 3.20 (tdd, J = 11.7, 5.4, 2.0 Hz, 2H), 2.91 (ddd, J = 12.5, 8.8, 3.7 Hz, 1H), 2.16 (dq, J = 17.6, 7.2, 6.2 Hz, 2H), 1.65 - 1.44 (m, 2H). ¹⁹F NMR (282 MHz, DMSO-*d*₆) δ -116.95, -135.20 (d, J = 22.9 Hz), -136.62 (d, J = 22.9 Hz). LCMS *m*/*z* 563.12 [M+1]⁺.

### Preparation of S4

### 4-benzyloxy-1-(3,4-difluorophenyl)-6-fluoro-3-iodo-2-(2-methoxy-1,1-dimethyl-ethyl)indole (S4)

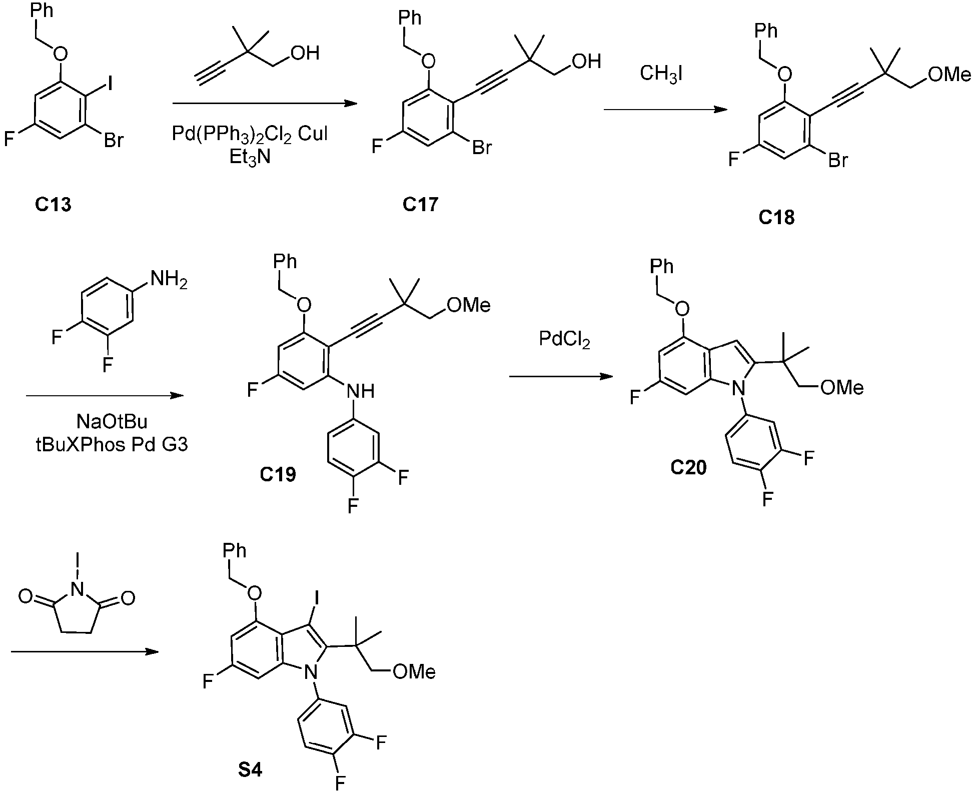

### Step 1. Synthesis of 4-(2-benzyloxy-6-bromo-4-fluoro-phenyl)-2,2-dimethyl-but-3-yn-1-ol (C17)

A solution of 1-benzyloxy-3-bromo-5-fluoro-2-iodo-benzene **C13** (5 g, 12.3 mmol), 2,2-dimethylbut-3-yn-1-ol (1.8 g, 18.3 mmol) in 1,4-dioxane (40 mL) and Et₃N (40 mL) was purged with nitrogen for 10 minutes, then added CuI (157 mg, 0.82 mmol) and PdCl₂(PPh₃)₂ (500 mg, 0.71 mmol) were added. The resulting reaction mixture was warmed to 50 °C, and stirred overnight. The reaction mixture was cooled to room temperature, poured into water (50 mL), and partitioned between sat. aqueous NH₄Cl solution (~50 mL) and ethyl acetate (~150 mL). Upon stirring for 10 minutes, the organic layer was separated, was washed with 1 *N* HCl solution (2 x 50 mL), water (30 mL), brine (30 mL), dried over MgSO₄, filtered and concentrated under reduced pressure. The residue was purified by silica gel chromatography (Gradient: 0-70% ethyl acetate in heptane) to afford the product as a clear yellow viscous oil. 4-(2-benzyloxy-6-bromo-4-fluoro-phenyl)-2,2-dimethyl-but-3-yn-1-ol (4.23 g, 90%). ¹H NMR (400 MHz, Chloroform-*d*) δ 7.49 (dtd, J = 6.9, 1.4, 0.7 Hz, 2H), 7.46 - 7.32 (m, 3H), 6.98 (dd, J = 8.0, 2.4 Hz, 1H), 6.65 (dd, J = 10.2, 2.4 Hz, 1H), 5.12 (s, 2H), 3.49 (d, J = 7.1 Hz, 2H), 1.34 (s, 6H). LCMS *m*/*z* 377.01 [M+1]⁺.

### Step 2. Synthesis of 1-benzyloxy-3-bromo-5-fluoro-2-(4-methoxy-3, 3-dimethyl-but-1-ynyl)benzene (C18)

A mixture of 4-(2-benzyloxy-6-bromo-4-fluoro-phenyl)-2,2-dimethyl-but-3-yn-1-ol **C17** (3.65 g, 9.5 mmol) and iodomethane (1.5 mL, 24.1 mmol) in THF (50 mL) under nitrogen, was cooled to 0 °C. NaH (600 mg, 15.0 mmol) was added and the resulting reaction mixture was stirred and allowed to warm to room temperature. The mixture was poured into water (50 mL), partitioned between sat. aqueous NH₄Cl solution (~50 mL) and ethyl acetate (~150 mL), then stirred for 10 minutes. The organic layer was separated, washed successively with 1 *N* HCl solution (2 x 50 mL), water (30 mL), and brine (30 mL). The organic layer was then dried over MgSO₄, filtered and concentrated under reduced pressure to afford the product as a dark oil, which turned into brown solid under vacuum. 1-benzyloxy-3-bromo-5-fluoro-2-(4-methoxy-3,3-dimethyl-but-1-ynyl)benzene (3.8 g, 100%). ¹H NMR (400 MHz, CD3CN) δ 7.51 (dtd, J = 6.9, 1.4, 0.7 Hz, 2H), 7.46 - 7.32 (m, 3H), 7.06 (dd, J = 8.4, 2.4 Hz, 1H), 6.87 (dd, J = 10.8, 2.4 Hz, 1H), 5.13 (s, 2H), 3.32 (s, 3H), 3.31 (s, 2H), 1.27 (s, 6H). LCMS *m*/*z* 390.96 [M+1]⁺.

### Step 3. Synthesis of 3-benzyloxy-N-(3,4-difluorophenyl)-5-fluoro-2-(4-methoxy-3,3-dimethyl-but-1-ynyl)aniline (C19)

To a solution of 1-benzyloxy-3-bromo-5-fluoro-2-(4-methoxy-3,3-dimethyl-but-1-ynyl)benzene **C18** (667 mg, 1.71 mmol) and 3,4-difluoroaniline (330 mg, 2.56 mmol) in degassed Xylene (12 mL) added NaOtBu (500 mg, 5.20 mmol) followed by tBuXPhos Pd G3 (70 mg, 0.09 mmol). The reaction mixture was stirred at room temperature for 12 hours. diluted with ice water (10 mL) and extracted EtOAc (3 x 10 mL). The combined organics were concentrated to dryness and purified via silica gel chromatography eluting (Gradient: 0-25% EtOAc in heptane) to afford the product as a brown oil. 3-benzyloxy-N-(3,4-difluorophenyl)-5-fluoro-2-(4-methoxy-3,3-dimethyl-but-1-ynyl)aniline (667 mg, 89%). ¹H NMR (400 MHz, Chloroform-d) δ 7.53 (d, J = 7.3 Hz, 2H), 7.42 (dd, J = 8.1, 6.8 Hz, 3H), 7.39 - 7.33 (m, 1H), 7.16 (dt, J = 10.4, 8.9 Hz, 1H), 7.07 (ddd, J = 11.8, 6.9, 2.6 Hz, 1H), 7.01 - 6.89 (m, 2H), 6.46 (dt, J = 11.1, 1.7 Hz, 1H), 6.18 (dt, J = 10.7, 1.5 Hz, 1H), 5.14 (s, 2H), 3.41 (d, J = 1.0 Hz, 3H), 3.37 (d, J = 1.1 Hz, 2H), 1.43 - 1.33 (m, 6H). LCMS *m*/*z* 440.42 [M+1]⁺.

### Step 5. Synthesis of 4-benzyloxy-1-(3, 4-difluorophenyl)-6-fluoro-2-(2-methoxy-1,1-dimethylethyl)indole (C20)

To a solution of 3-benzyloxy-N-(3,4-difluorophenyl)-5-fluoro-2-(4-methoxy-3,3-dimethyl-but-1-ynyl)aniline **C19** (515 mg, 1.17 mmol) in MeCN (5 mL) was added PdCl₂ (21 mg, 0.12 mmol). The reaction mixture was stirred at room temperature for 8 hours. The mixture was then diluted with ice water (100 mL) and extracted with EtOAc (3 x 50 mL). The combined organic layers were concentrated to dryness, and purified by silica gel chromatography (Gradient: 0-25% EtOAc in heptane) to give a white solid. 4-benzyloxy-1-(3,4-difluorophenyl)-6-fluoro-2-(2-methoxy-1,1-dimethyl-ethyl)indole (483 mg, 94%).¹H NMR (400 MHz, Chloroform-d) δ 7.45 - 7.38 (m, 2H), 7.38 - 7.30 (m, 2H), 7.30 - 7.20 (m, 2H), 7.18 - 7.10 (m, 1H), 7.06 (dddd, J = 8.5, 4.0, 2.5, 1.6 Hz, 1H), 6.52 (d, J = 0.8 Hz, 1H), 6.29 (dd, J = 11.6, 2.0 Hz, 1H), 5.89 (ddd, J = 9.4, 1.9, 0.7 Hz, 1H), 5.08 (s, 2H), 3.15 (s, 3H), 3.06 (s, 2H), 1.20 (s, 3H), 1.16 (s, 3H). LCMS *m*/*z* 440.37 [M+1]⁺.

### Step 6. Synthesis of 4-benzyloxy-1-(3,4-difluorophenyl)-6-fluoro-3-iodo-2-(2-methoxy-1,1-dimethyl-ethyl)indole (S4)

4-benzyloxy-1-(3,4-difluorophenyl)-6-fluoro-2-(2-methoxy-1,1-dimethyl-ethyl)indole C20 (357 mg, 0.81 mmol) in dichloromethane (5 mL) was added N-iodosuccinimide (190 mg, 0.84 mmol) at room temperature and stirred for 1 hour. The mixture was concentrated to dryness and purified by silica gel chromatography (Gradient: 0 - 25% EtOAc in Hexanes) to afford the product as a white solid. 4-benzyloxy-1-(3,4-difluorophenyl)-6-fluoro-3-iodo-2-(2-methoxy-1,1-dimethyl-ethyl)indole (432 mg, 93%). LCMS *m*/*z* 565.3 [M+1]⁺.

### Preparation of S5

### 4-benzyloxy-1-(3, 4-difluorophenyl)-3-iodo-2-(2-methoxy-1,1-dimethyl-ethyl)indole (S5)

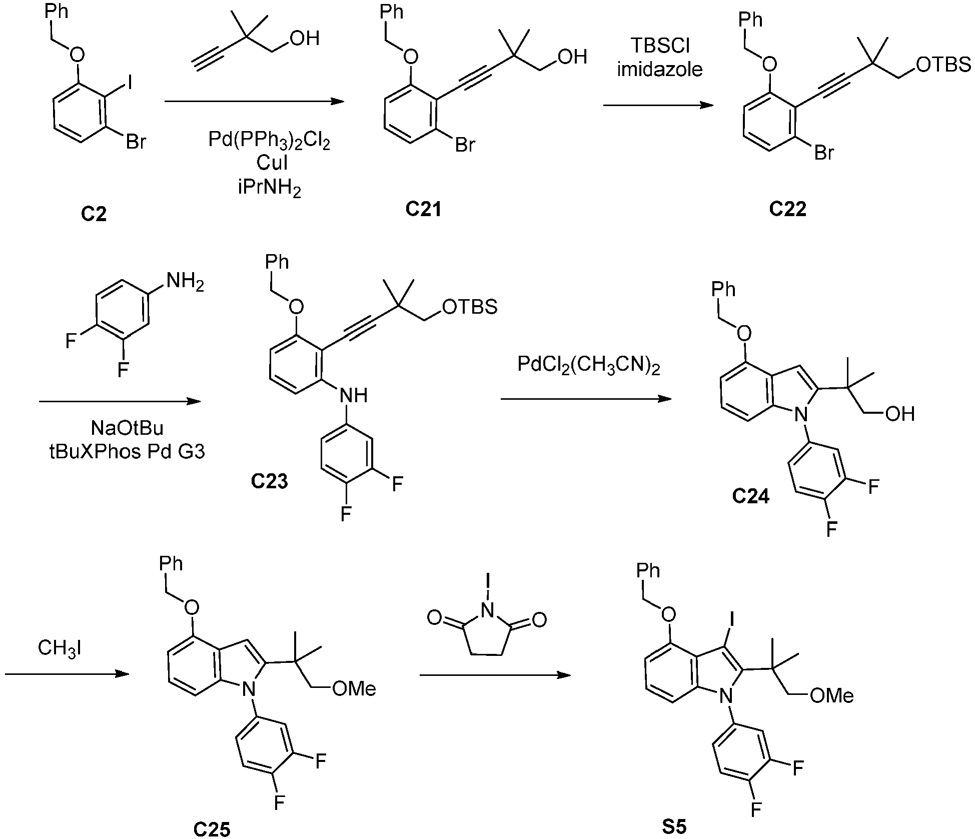

### Step 1. Synthesis of 4-(2-benzyloxy-6-bromo-phenyl)-2,2-dimethyl-but-3-yn-1-ol (C21)

A 3 L 3-neck RB flask with overhead stirrer, temperature probe, reflux condenser and nitrogen inlet was charged with 1-benzyloxy-3-bromo-2-iodo-benzene C2 (160 g, 411.3 mmol) and 2,2-dimethylbut-3-yn-1-ol (51 g, 519.6 mmol) in 1,4-dioxane (1.1 L), and stirred for 5 minutes. N-isopropylpropan-2-amine (370 mL, 2.64 mol) was then added. The reaction mixture was purged with nitrogen for ~15 minutes, then iodocopper (3.7 g, 19.4 mmol) and PdCl₂ (12.5 g, 17.8 mmol) were added. The resulting reaction mixture was warmed to 50 °C, and stirred for 3 hours. The reaction mixture was cooled to room temperature, poured into water (300 mL). Sat. aqueous NH₄Cl solution (~400 mL), followed by ethyl acetate (~2 L) were added, and the mixture stirred for 15 minutes. The organic layer was separated, washed with 1 *N* HCl solution (2 x 200 mL), brine (200 mL), then dried over MgSO₄, filtered and concentrated under reduced pressure.

Purified by silica gel chromatography (Gradient: 0-50% ethyl acetate in heptane) afforded the product as a yellow solid. 4-(2-benzyloxy-6-bromo-phenyl)-2,2-dimethyl-but-3-yn-1-ol (130 g, 88%). ¹H NMR (400 MHz, Chloroform-d) δ 7.48 (ddt, J = 7.4, 1.5, 0.7 Hz, 2H), 7.44 - 7.37 (m, 2H), 7.36 - 7.29 (m, 1H), 7.19 (dd, J = 8.1, 1.0 Hz, 1H), 7.08 (t, J = 8.2 Hz, 1H), 6.86 (dd, J = 8.3, 1.0 Hz, 1H), 5.13 (s, 2H), 3.48 (d, J = 7.2 Hz, 2H), 2.12 (t, J = 7.2 Hz, 1H), 1.33 (s, 6H). LCMS *m*/*z* 359.02 [M+1]⁺.

### Step 2. Synthesis of [4-(2-benzyloxy-6-bromo-phenyl)-2,2-dimethyl-but-3-ynoxy]-tert-butyldimethyl-silane (C22)

A 3 L 3-neck RB flask with overhead stirrer, temperature probe, reflux condenser and nitrogen inlet was charged with 4-(2-benzyloxy-6-bromo-phenyl)-2,2-dimethyl-but-3-yn-1-ol **C21** (130 g, 361.9 mmol) in DMF (850 mL). The mixture was stirred for 5 minutes at ambient temperature and then imidazole (64 g, 940.1 mmol) and TBSCl (64 g, 424.6 mmol) were added (observed Tmax = 31 °C). The reaction mixture was poured into ice/water (~1 L), and extracted with MTBE (2 x 1 L). The organic phase was washed with 1 *N* HCl (2 x 200 mL), and brine (200 mL), then dried over MgSO₄, filtered and concentrated under reduced pressure. The residue was purified by silica gel chromatography (Column: 1.5 kg Isco. Gradient, 0-50% ethyl acetate in heptane) afforded the product as a clear, light yellow color oil. [4-(2-benzyloxy-6-bromophenyl)-2,2-dimethyl-but-3-ynoxy]-tert-butyl-dimethyl-silane (164 g, 96%). ¹H NMR (400 MHz, Chloroform-*d*) δ 7.55 - 7.44 (m, 2H), 7.42 - 7.35 (m, 2H), 7.35 - 7.28 (m, 1H), 7.19 (dd, J = 8.1, 1.0 Hz, 1H), 7.04 (t, J = 8.2 Hz, 1H), 6.83 (dd, J = 8.4, 1.0 Hz, 1H), 5.12 (s, 2H), 3.59 (s, 2H), 1.31 (s, 6H), 0.90 (s, 9H), 0.05 (s, 6H).

### Step 3. Synthesis of 3-benzyloxy-2-[4-[tert-butyl(dimethyl)silyl]oxy-3,3-dimethyl-but-1-ynyl]-N-(3,4-difluorophenyl)aniline (C23)

To a solution of [4-(2-benzyloxy-6-bromo-phenyl)-2,2-dimethyl-but-3-ynoxy]-tert-butyl-dimethyl-silane **C22** (11 g, 23.2 mmol) and 3,4-difluoroaniline (3.27 g, 25.33 mmol) in xylene (60 mL) under nitrogen was added NaOtBu (6 g, 62.4 mmol) followed by tBuXPhos Pd G3 (315 mg, 0.40 mmol). The reaction mixture was stirred at room temperature overnight. The reaction was diluted with water and sat aq. NH₄Cl and extracted with EtOAc (x 2). The combined organics were concentrated to dryness and purified by silica gel chromatography (Column: 220g Silica. Gradient: 0-50% EtOAc in heptane) to afford the product as a yellow oil. 3-benzyloxy-2-[4-[tert-butyl(dimethyl)silyl]oxy-3,3-dimethyl-but-1-ynyl]-N-(3,4-difluorophenyl)aniline (11.6 g, 96%). ¹H NMR (400 MHz, Chloroform-d) δ 7.49 (ddt, J = 7.4, 1.3, 0.7 Hz, 2H), 7.38 - 7.32 (m, 2H), 7.31 - 7.25 (m, 1H), 7.10 - 6.96 (m, 3H), 6.86 - 6.80 (m, 1H), 6.70 (dd, J = 8.3, 0.8 Hz, 1H), 6.43 - 6.39 (m, 2H), 5.11 (s, 2H), 3.53 (s, 2H), 1.28 (s, 6H), 0.84 (s, 9H), 0.00 (s, 6H). LCMS *m*/*z* 522.52 [M+1]⁺.

### Step 4. Synthesis of 2-[4-benzyloxy-1-(3, 4-difluorophenyl)indol-2-yl]-2-methyl-propan-1-ol (C24)

A solution of 3-benzyloxy-2-[4-[tert-butyl(dimethyl)silyl]oxy-3,3-dimethyl-but-1-ynyl]-N-(3,4-difluorophenyl)aniline **C23** (11.6 g, 22.2 mmol) in MeOH (100 mL) and EtOAc (50.7 mL) was purged with nitrogen for 1 hour. PdCl₂(CH₃CN)₂ (336 mg, 1.30 mmol) was added and the mixture heated to 60 °C overnight. The reaction was concentrated under reduced pressure and then purified by silica gel chromatography (Gradient: 0-75% EtOAc in heptane) to afford a white solid. 2-[4-benzyloxy-1-(3,4-difluorophenyl)indol-2-yl]-2-methyl-propan-1-ol (8.2 g, 91%). ¹H NMR (400 MHz, Chloroform-d) δ 7.55 (dt, J = 6.3, 1.4 Hz, 2H), 7.48 - 7.41 (m, 2H), 7.41 - 7.31 (m, 2H), 7.31 - 7.24 (m, 3H), 7.22 - 7.15 (m, 1H), 7.02 (t, J = 8.0 Hz, 1H), 6.74 (d, J = 0.8 Hz, 1H), 6.63 (d, J = 7.8 Hz, 1H), 6.33 (d, J = 8.2 Hz, 1H), 5.26 (s, 2H), 3.53 (dd, J = 6.0, 1.6 Hz, 2H), 1.28 (s, 3H), 1.27 (s, 3H). LCMS *m*/*z* 408.37 [M+1]⁺.

### Step 5. Synthesis of 4-benzyloxy-1-(3, 4-difluorophenyl)-2-(2-methoxy-1,1-dimethyl-ethyl)indole (C25)

At 0 °C, 2-[4-benzyloxy-1-(3,4-difluorophenyl)indol-2-yl]-2-methyl-propan-1-ol **C24** (500 mg, 1.23 mmol) and MeI (120 µL, 1.928 mmol) in THF (5 mL) was added NaH (60 mg of 60% w/w, 1.5 mmol) in one portion and allowed to warm to room temperature. After 1 hour, the reaction was complete. Water (5 mL) and sat NH4Cl (5 mL) were added to the reaction mixture followed by extraction with EtOAc (3 x 5 mL). Combined organic fractions were washed with brine (1 x 2 mL), dried over MgSO₄ and concentrated 4-benzyloxy-1-(3,4-difluorophenyl)-2-(2-methoxy-1,1-dimethyl-ethyl)indole (509 mg, 99%). ¹H NMR (400 MHz, Chloroform-d) δ 7.52 - 7.45 (m, 2H), 7.42 - 7.34 (m, 2H), 7.33 - 7.17 (m, 3H), 7.13 (dddd, J = 8.6, 4.0, 2.5, 1.6 Hz, 1H), 6.92 (t, J = 8.0 Hz, 1H), 6.63 (d, J = 0.9 Hz, 1H), 6.54 (dd, J = 7.8, 0.6 Hz, 1H), 6.25 (dt, J = 8.3, 0.7 Hz, 1H), 5.18 (s, 2H), 3.20 (s, 3H), 3.14 (s, 2H), 1.26 - 1.20 (m, 6H). LCMS m/z calc. 421.18533, found 422.37 [M+H]⁺.

### Step 6. 4-benzyloxy-1-(3,4-difluorophenyl)-3-iodo-2-(2-methoxy-1,1-dimethyl-ethyl)indole (S5)

4-benzyloxy-1-(3,4-difluorophenyl)-2-(2-methoxy-1,1-dimethyl-ethyl)indole **C25** (518.4 mg, 1.23 mmol) in dichloromethane (5 mL) was added to NIS (290 mg, 1.29 mmol) and stirred for 2 hours. The mixture was concentrated and dissolved in dichloromethane. Purification by silica gel chromatography (Gradient: 0-20% EtOAc in hexanes) provided the product as white solid. 4-benzyloxy-1-(3,4-difluorophenyl)-3-iodo-2-(2-methoxy-1,1-dimethyl-ethyl)indole (675 mg, 98%) ¹H NMR (400 MHz, Chloroform-*d*) δ 7.65 (ddt, J = 7.4, 1.3, 0.7 Hz, 2H), 7.48 - 7.39 (m, 2H), 7.39 - 7.35 (m, 1H), 7.34 - 7.28 (m, 1H), 7.26 - 7.18 (m, 1H), 7.13 (dddd, J = 8.5, 4.0, 2.5, 1.6 Hz, 1H), 6.97 (dd, J = 8.3, 7.9 Hz, 1H), 6.63 (dd, J = 7.9, 0.8 Hz, 1H), 6.28 (dd, J = 8.3, 0.7 Hz, 1H), 5.26 (s, 2H), 3.79 (d, J = 9.1 Hz, 1H), 3.71 (d, J = 9.1 Hz, 1H), 1.59 (s, 3H), 1.42 (s, 3H), 1.36 (s, 3H). LCMS *m*/*z* 547.28 [M+H]⁺.

### Preparation of S6

### 1-(3-chloro-4-fluoro-phenyl)-3-iodo-4-(methoxymethoxy)-2-tetrahydropyran-4-yl-indole (S6)

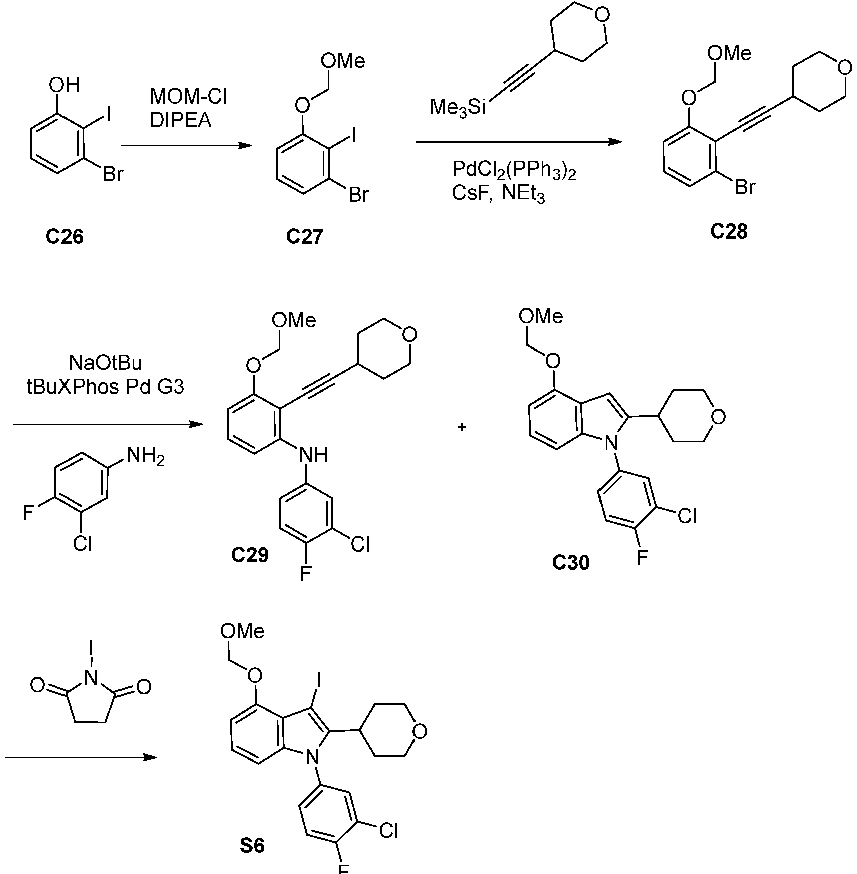

### Step 1. Synthesis of 1-bromo-2-iodo-3-(methoxymethoxy)benzene (C27)

To a solution of 3-bromo-2-iodo-phenol **C26** (5.2 g, 17.40 mmol) and DIPEA (4.5 mL, 25.8 mmol) in dichloromethane (50 mL) at 0 °C was added dropwise chloro(methoxy)methane (1.6 mL, 21.1 mmol) in dichloromethane (10 mL). The reaction was allowed to warm to room temperature and stirred for 2 hours. Aqueous NH₄Cl (20 mL) and water (10 mL) were added, and stirred the mixture was stirred for 5 minutes. The organic layer was separated, dried over sodium sulfate and concentrated to give the product as a brown liquid, which was used directly in the next step. 1-bromo-2-iodo-3-(methoxymethoxy)benzene (6.1 g, 100%). ¹H NMR (400 MHz, Chloroform-*d*) δ 7.34 (dd, J = 8.0, 1.3 Hz, 1H), 7.18 (t, J = 8.1 Hz, 1H), 6.99 (dd, J = 8.3, 1.3 Hz, 1H), 5.26 (s, 2H), 3.53 (s, 3H). LCMS *m*/*z* 342.22 [M+H]⁺.

### Step 2. Synthesis of 4-[2-[2-bromo-6-(methoxymethoxy)phenyl]ethynyl]tetrahydropyran (C28)

Water (250 µL, 13.9 mmol) was added to a solution of 1-bromo-2-iodo-3-(methoxymethoxy)benzene **27** (2 g, 5.8 mmol) and trimethyl(2-tetrahydropyran-4-ylethynyl)silane (1.28 g, 7.02 mmol) in DMF (11 mL) and triethylamine (11 mL) and the mixture was purged with nitrogen for 15 minutes. CsF (2 g, 13.17 mmol), PdCl₂(PPh₃)₂ (245 mg, 0.35 mmol) and CuI (115 mg, 0.60 mmol) were added and the reaction heated to 90 °C overnight. The mixture was cooled, and the triethylamine was removed under vacuum. Ice-water (100 mL) was added and the mixture extracted with ether (3 x 100 mL). The organic layer was washed with water (200 mL), then brine (200 mL), and concentrated. Purification by silica gel chromatography (Gradient: 0-30% ethyl acetate in heptane) afforded the product as a light yellow oil. 4-[2-[2-bromo-6-(methoxymethoxy)phenyl]-ethynyl]tetrahydropyran (1.43 g, 75%). ¹H NMR (400 MHz, Chloroform-d) δ 7.24 (dd, J = 7.2, 1.8 Hz, 1H), 7.11 - 7.01 (m, 2H), 5.23 (s, 2H), 4.02 (ddd, J = 11.5, 7.0, 3.4 Hz, 2H), 3.62 (ddd, J = 11.1, 7.2, 3.3 Hz, 2H), 3.51 (s, 3H), 3.01 (tt, J = 7.7, 4.2 Hz, 1H), 2.01 - 1.91 (m, 2H), 1.87 - 1.77 (m, 2H). LCMS *m*/*z* 325.09 [M+H]⁺.

### Step 3. N-(3-chloro-4-fluoro-phenyl)-3-(methoxymethoxy)-2-(2-tetrahydropyran-4-ylethynyl)aniline (C29) and 1-(3-chloro-4-fluoro-phenyl)-4-(methoxymethoxy)-2-tetrahydropyran-4-yl-indole (C30)

A solution of 4-[2-[2-bromo-6-(methoxymethoxy)phenyl]ethynyl]tetrahydro-pyran **C28** (1.5 g, 4.61 mmol), 3-chloro-4-fluoroaniline (940 mg, 6.46 mmol) in xylene (30 mL) was degassed for 5 minutes. Sodium t-Butoxide (1.33 g, 13.8 mmol) was added and the solution was degassed for another 5 minutes. *t*BuXPhos Pd G3 (529.6 mg, 0.66 mmol) was added and the reaction stirred at room temperature for 3 hours. Ice water (50 mL) and EtOAc (50 mL) were added. Upon stirring for 5 minutes, the aqueous layer was isolated and washed with EtOAc (20 mL). The combined organic layers were washed with brine, dried over MgSO₄ and concentrated. Purification by silica gel chromatography (Gradient: 0-40% EtOAc in heptane) gave products **C29** and **C30.** N-(3-chloro-4-fluoro-phenyl)-3-(methoxymethoxy)-2-(2-tetrahydropyran-4-ylethynyl)aniline **C29** (1.39 g, 75%). ¹H NMR (400 MHz, Chloroform-*d*) δ 7.25 (dd, J = 6.3, 2.7 Hz, 1H), 7.11 (t, J = 8.5 Hz, 2H), 7.05 (ddd, J = 8.9, 4.2, 2.7 Hz, 1H), 6.78 (dd, J = 8.3, 0.8 Hz, 1H), 6.63 (dd, J = 8.3, 0.8 Hz, 1H), 6.36 (s, 1H), 5.27 (s, 2H), 4.00 (ddd, J = 11.6, 6.0, 3.6 Hz, 2H), 3.62 (ddd, J = 11.5, 8.3, 3.0 Hz, 2H), 3.55 (s, 3H), 3.02 (tt, J = 8.3, 4.1 Hz, 1H), 2.05 - 1.94 (m, 2H), 1.82 (dtd, J = 13.4, 8.3, 3.6 Hz, 2H). LCMS *m*/*z* 390.27 [M+H]⁺.

1-(3-chloro-4-fluoro-phenyl)-4-(methoxymethoxy)-2-tetrahydropyran-4-yl-indole **C30** (0.37 g, 19%). ¹H NMR (400 MHz, Chloroform-d) δ 7.21 (dd, J = 6.3, 2.7 Hz, 1H), 7.14 - 7.01 (m, 4H), 6.97 (ddt, J = 8.9, 4.0, 2.2 Hz, 1H), 6.79 (dd, J = 8.3, 0.8 Hz, 1H), 6.68 (ddd, J = 8.7, 4.1, 2.6 Hz, 1H), 6.58 (dd, J = 8.3, 0.9 Hz, 1H), 6.34 (s, 1H), 5.79 (d, J = 2.7 Hz, 1H), 5.26 (s, 2H), 4.00 (ddd, J = 11.6, 6.0, 3.6 Hz, 2H), 3.61 (ddd, J = 11.5, 8.2, 3.1 Hz, 2H), 3.55 (s, 3H), 3.02 (tt, J = 8.3, 4.1 Hz, 1H), 2.06 - 1.93 (m, 2H), 1.89 - 1.77 (m, 2H).

### Step 4. Synthesis of 1-(3-chloro-4-fluoro-phenyl)-3-iodo-4-(methoxymethoxy)-2-tetrahydropyran-4-yl-indole (S6)

To a solution of 1-(3-chloro-4-fluoro-phenyl)-4-(methoxymethoxy)-2-tetrahydropyran-4-yl-indole **C30** in dichloromethane (20 mL) was added N-iodosuccinimide (755 mg, 3.36 mmol). The reaction mixture was stirred for 30 minutes. The mixture was then diluted with water, and the organic layer was concentrated to dryness. Purification by silica gel chromatography (Gradient: 0-25% EtOAc in heptane) yielded the product as a white solid which was used in the next step. 1-(3-chloro-4-fluoro-phenyl)-3-iodo-4-(methoxymethoxy)-2-tetrahydropyran-4-yl-indole (1.38 g, 83%) ¹H NMR (400 MHz, Chloroform-*d*) δ 7.43 - 7.37 (m, 1H), 7.35 (dd, J = 8.5, 1.5 Hz, 1H), 7.21 (ddd, J = 8.7, 4.2, 2.4 Hz, 1H), 7.05 (td, J = 8.1, 2.4 Hz, 1H), 6.81 (ddd, J = 7.9, 4.4, 0.8 Hz, 1H), 6.53 (ddd, J = 14.5, 8.3, 0.7 Hz, 1H), 5.36 (s, 2H), 4.02 (dd, J = 11.5, 4.6 Hz, 2H), 3.63 (d, J = 3.9 Hz, 3H), 3.39 (tdd, J = 11.9, 5.6, 2.0 Hz, 2H), 3.11 (tt, J = 12.5, 3.6 Hz, 1H), 2.55 - 2.17 (m, 2H), 1.60 (s, 2H). LCMS *m*/*z* 515.32 [M+H]⁺.

### Preparation of S7

### 4-benzyloxy-6-fluoro-1-(4-fluoro-3-methyl-phenyl)-3-iodo-2-tetrahydropyran-4-yl-indole (S7)

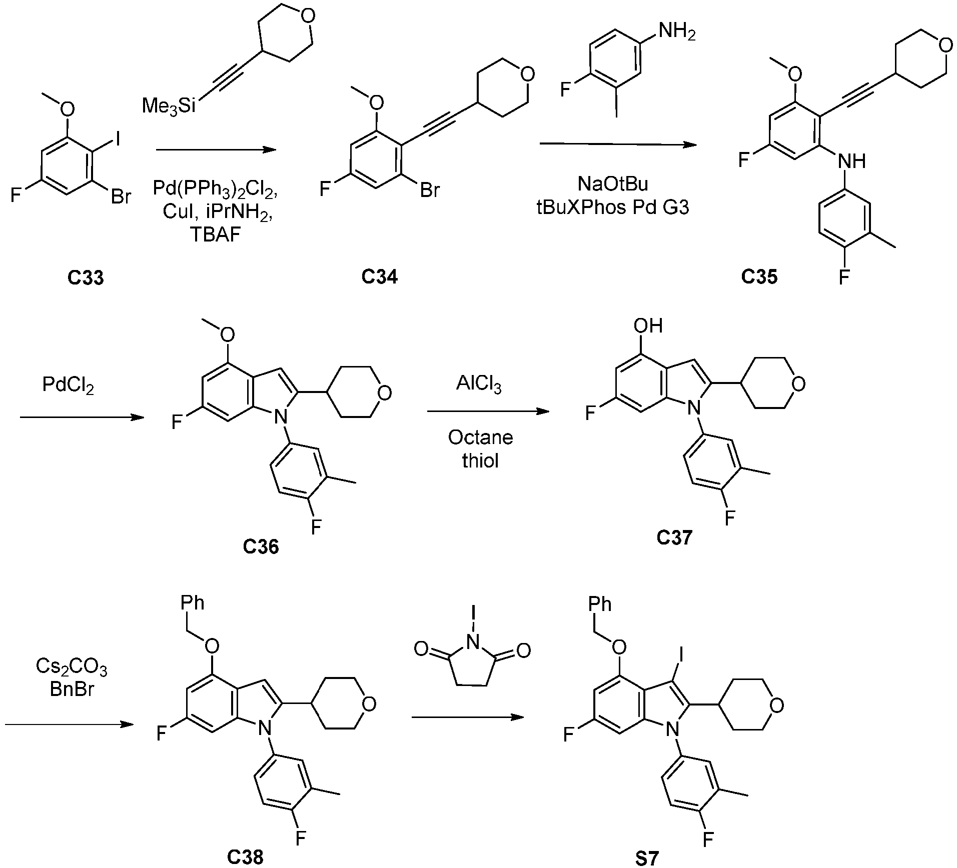

### Step 1. Synthesis of 4-[2-(2-bromo-4-fluoro-6-methoxy-phenyl)ethynyl]tetrahydropyran (C34)

A 5 L 3-neck RB flask with overhead stirrer, temperature probe, and nitrogen inlet was charged with 1-bromo-5-fluoro-2-iodo-3-methoxy-benzene C33 (264 g, 797.8 mmol) and trimethyl(2-tetrahydropyran-4-ylethynyl)silane (250 g, 1.3 mol) in 1,4-dioxane (1.5 L). The mixture was stirred for 5 minutes, and then iPrNH₂ (650 mL, 4.64 mol) was added, followed by CuI (6 g, 31.5 mmol), PdCl₂(PPh₃)₂ (22 g, 31.3 mmol) and TBAF hydrate (300 g, 950.8 mmol). The reaction mixture was warmed to 60 °C for 12 hours. The reaction mixture was cooled to room temperature, poured onto a mixture of water (~1L), sat. aqueous NH₄Cl solution (~2 L) and ethyl acetate (~3 L), then stirred for 10 minutes. The organic phase was separated, washed successively with a 1 *N* HCl solution (2 x 500 mL), brine (500 mL). The organic phase was dried over MgSO₄, filtered and concentrated under reduced pressure. Purification by silica gel chromatography (Gradient: 0- 30% ethyl acetate in heptane) afforded the product as a yellow solid (151 g, 60% yield).

Mixed fractions were purified by an additional silica gel chromatography column (Gradient: 0-50% ethyl acetate in heptane) to afford additional product (50 g, 20% yield). Purified product batches were combined (151 g + 50 g) and dried under vacuum to afford 4-[2-(2-bromo-4-fluoro-6-methoxy-phenyl)ethynyl]tetrahydropyran (200 g, 80%) as a yellow solid. ¹H NMR (400 MHz, DMSO-*d*₆Chloroform-*d*) δ 6.95 (dd, J = 8.0, 2.4 Hz, 1H), 6.57 (dd, J = 10.4, 2.4 Hz, 1H), 4.01 (ddd, J = 11.6, 6.9, 3.4 Hz, 2H), 3.86 (s, 3H), 3.61 (ddd, J = 11.2, 7.3, 3.3 Hz, 2H), 3.00 (dq, J = 7.7, 3.8 Hz, 1H), 2.03 - 1.90 (m, 2H), 1.81 (dtd, J = 13.1, 7.4, 3.1 Hz, 2H). ¹⁹F NMR (376 MHz, Chloroform-d) δ -108.45. LCMS *m*/*z* 314.97 [M+1]⁺.

### Step 2. Synthesis of 5-fluoro-N-(4-fluoro-3-methyl-phenyl)-3-methoxy-2-(2-tetrahydro-pyran-4-ylethynyl)aniline (C35)

To a solution of 4-[2-(2-bromo-4-fluoro-6-methoxy-phenyl)ethynyl]tetra-hydropyran **C34** (92 g, 293.8 mmol), 4-fluoro-3-methyl-aniline (55.2 g, 441.1 mmol), tBuXPhos (3.75 g, 8.83 mmol) and NaOtBu (70.6 g, 734.6 mmol) in m-xylene (1.3 L) was purged with nitrogen for 10 minutes. tBuXPhos Pd G3 (7.01 g, 8.83 mmol) was added, and the mixture heated to 60 °C for 3 hours. The mixture was cooled, then sat. NH₄Cl (1 L) and HCl (64 mL of 6 M, 384.0 mmol) was added and the mixture was allowed to stir overnight. The mixture was extracted with EtOAc (x 2) and the organic layer was concentrated. Purification by silica gel chromatography (Gradient: 0-90% EtOAc in heptanes) to afford the product as a black oil 5-fluoro-N-(4-fluoro-3-methyl-phenyl)-3-methoxy-2-(2-tetrahydropyran-4-ylethynyl)aniline (98 g, 93%). ¹H NMR (300 MHz, Chloroform-d) δ 6.87 - 6.76 (m, 3H), 6.24 (s, 1H), 6.13 (dd, J = 11.2, 2.3 Hz, 1H), 5.90 (dd, J = 10.7, 2.3 Hz, 1H), 3.80 (ddd, J = 11.6, 5.6, 3.6 Hz, 2H), 3.68 (s, 3H), 3.40 (ddd, J = 11.5, 8.4, 3.0 Hz, 2H), 2.82 (tt, J = 8.4, 4.1 Hz, 1H), 2.11 (d, J = 1.8 Hz, 3H), 1.86 - 1.73 (m, 2H), 1.71 - 1.54 (m, 2H). LCMS *m*/*z* 358.22 [M+1]⁺.

### Step 3. Synthesis of 6-fluoro-1-(4-fluoro-3-methyl-phenyl)-4-methoxy-2-tetrahydropyran-4-yl-indole (C36)

To a solution of 5-fluoro-N-(4-fluoro-3-methyl-phenyl)-3-methoxy-2-(2-tetrahydropyran-4-ylethynyl)aniline C35 (98 g) in MeCN (1 L) was added PdCl₂ (2.08 g, 11.7 mmol). The mixture was heated to 60 °C for overnight. The mixture was then concentrated to dryness and MBTE (300 mL) was added. Upon stirring for 10 minutes, the mixture was filtered to afford solid product (40 g). The black filtrate was purified by silica gel chromatography (Gradient: 0-60% EtOAc in dichloromethane) to afford an additional 33g of product. The two product batches were combined to afford a single batch of product. 6-fluoro-1-(4-fluoro-3-methyl-phenyl)-4-methoxy-2-tetrahydropyran-4-yl-indole (73 g, 70%). ¹H NMR (300 MHz, Chloroform-*d*) δ 7.15 (td, J = 8.2, 7.6, 4.2 Hz, 3H), 6.49 (s, 1H), 6.42 - 6.24 (m, 2H), 3.97 (s, 5H), 3.35 (td, J = 11.6, 2.7 Hz, 2H), 2.76 (ddd, J = 15.4, 11.2, 4.4 Hz, 1H), 2.38 (d, J = 1.8 Hz, 3H), 1.90 - 1.64 (m, 4H). LCMS *m*/*z* 358.12 [M+1]⁺.

### Step 4. Synthesis of 6-fluoro-1-(4-fluoro-3-methyl-phenyl)-2-tetrahydropyran-4-yl-indol-4-ol (C37)

A 1 L 3-neck RB flask was charged 6-fluoro-1-(4-fluoro-3-methyl-phenyl)-4-methoxy-2-tetrahydropyran-4-yl-indole **C36** (15.1 g, 42.3 mmol) in dichloromethane (250 mL), stirred for 5 minutes and then cooled to 0 °C with an ice/water bath. AlCl₃ (20.4 g, 153.0 mmol) was added, stirred for 10 minutes, and then octane-1-thiol (31 mL, 178.6 mmol) was added and the mixture was stirred at room temperature for 5 hours. The reaction mixture was then poured into ice/water (~120 mL). 2 *N* HCl (~120 mL) and ethyl acetate (400 mL) were added, and the mixture stirred for ~20 minutes. The organic phase was separated, washed with brine (~300 mL), dried over Na₂SO₄, filtered and then concentrated under reduced pressure. The residue was triturated with ~10% MTBE in heptane (~400 mL), dried under vacuum to afford the product as a tan solid. 6-fluoro-1-(4-fluoro-3-methyl-phenyl)-2-tetrahydropyran-4-yl-indol-4-ol (13.2 g, 91%). ¹H NMR (300 MHz, DMSO-*d*₆) δ 9.98 (s, 1H), 7.35 (t, J = 8.8 Hz, 2H), 7.27 (ddd, J = 8.2, 4.6, 2.7 Hz, 1H), 6.45 (s, 1H), 6.26 (dd, J = 11.5, 2.1 Hz, 1H), 6.10 (dd, J = 9.8, 1.6 Hz, 1H), 3.82 (d, J = 10.9 Hz, 2H), 3.22 (td, J = 11.2, 3.2 Hz, 3H), 2.76 (dt, J = 10.3, 5.4 Hz, 1H), 2.32 (d, J = 1.6 Hz, 4H), 1.74 - 1.49 (m, 2H). LCMS *m*/*z* 344.14 [M+1]⁺.

### Step 5. Synthesis of 4-benzyloxy-6-fluoro-1-(4-fluoro-3-methyl-phenyl)-2-tetrahydropyran-4-yl-indole (C38)

A solution of 6-fluoro-1-(4-fluoro-3-methyl-phenyl)-2-tetrahydropyran-4-yl-indol-4-ol **C37** (15 g, 43.69 mmol) in THF (163 mL) and DMF (32 mL) was stirred for 5 minutes, until a clear, light brown colored solution formed. Cs₂CO₃ (29.1 g, 89.3 mmol) and benzyl bromide (6.3 mL, 53.0 mmol) was added and the resulting reaction mixture (white suspension) was stirred at room temperature overnight. The reaction mixture was diluted with ethyl acetate (130 mL), stirred for 10 minutes, and then the resulting white suspension was filtered through a medium fritted funnel, washing with ethyl acetate (50 mL). The combined filtrate was washed with water (~200 mL), brine (~200 mL), dried over MgSO₄, filtered and concentrated under reduced pressure. The residue was triturated with 10% MTBE in heptane (~300 mL), and dried under vacuum to afford the product as a white solid. 4-benzyloxy-6-fluoro-1-(4-fluoro-3-methylphenyl)-2-tetrahydropyran-4-yl-indole (16.1 g, 85%). ¹H NMR (300 MHz, Chloroform-d) δ 7.57 - 7.49 (m, 2H), 7.49 - 7.33 (m, 3H), 7.24 - 7.08 (m, 3H), 6.55 (s, 1H), 6.43 (dd, J = 11.6, 2.0 Hz, 1H), 6.32 (dd, J = 9.5, 1.9 Hz, 1H), 5.22 (s, 2H), 4.03 - 3.91 (m, 2H), 3.35 (td, J = 11.6, 2.7 Hz, 2H), 2.83 - 2.69 (m, 1H), 2.38 (d, J = 1.9 Hz, 3H), 1.90 - 1.64 (m, 4H). LCMS *m*/*z* 434.15 [M+1]⁺.

### Step 6. Synthesis of 4-benzyloxy-6-fluoro-1-(4-fluoro-3-methyl-phenyl)-3-iodo-2-tetrahydropyran-4-yl-indole (S7)

A solution of 4-benzyloxy-6-fluoro-1-(4-fluoro-3-methyl-phenyl)-2-tetrahydropyran-4-yl-indole **C38** (64.5 g, 148.8 mmol) in dichloromethane (1 L) was stirred for 5 minutes to afford a clear, colorless solution. The reaction mixture was cooled to 0 °C with an ice/water bath, then 1-iodopyrrolidine-2,5-dione (36.3 g, 156.5 mmol) was added in three portions over 15 minutes. The resulting reaction mixture was stirred from 0 °C to room temperature over 1 hour. The reaction was then washed with an aqueous mixture of 1*N* Na₂S₂O₃ solution (~300 mL), saturated NaHCO₃ (150 mL), then brine (~300 mL). The mixture was dried over MgSO₄, filtered, and concentrated under reduced pressure. The residue was concentrated to afford the product as a white solid. 4-benzyloxy-6-fluoro-1-(4-fluoro-3-methyl-phenyl)-3-iodo-2-tetrahydropyran-4-yl-indole (83 g, 100%). ¹H NMR (300 MHz, Chloroform-d) δ 7.64 (d, J = 7.0 Hz, 2H), 7.50 - 7.32 (m, 3H), 7.19 (t, J = 8.7 Hz, 1H), 7.09 (td, J = 8.5, 3.7 Hz, 2H), 6.43 (dd, J = 11.6, 2.0 Hz, 1H), 6.13 (dd, J = 9.2, 2.1 Hz, 1H), 5.23 (s, 2H), 3.99 (dd, J = 11.5, 4.1 Hz, 2H), 3.36 (td, J = 11.9, 1.9 Hz, 2H), 3.09 (tt, J = 12.5, 3.5 Hz, 1H), 2.38 (d, J = 1.9 Hz, 3H), 2.26 (ddt, J = 16.9, 12.7, 6.4 Hz, 2H), 1.53 (m, 2H). LCMS *m*/*z* 559.99 [M+1]⁺.

### Preparation of S8

### 4-benzyloxy-5-fluoro-1-(4-fluoro-3-methyl-phenyl)-3-iodo-2-tetrahydropyran-4-yl-indole (S8)

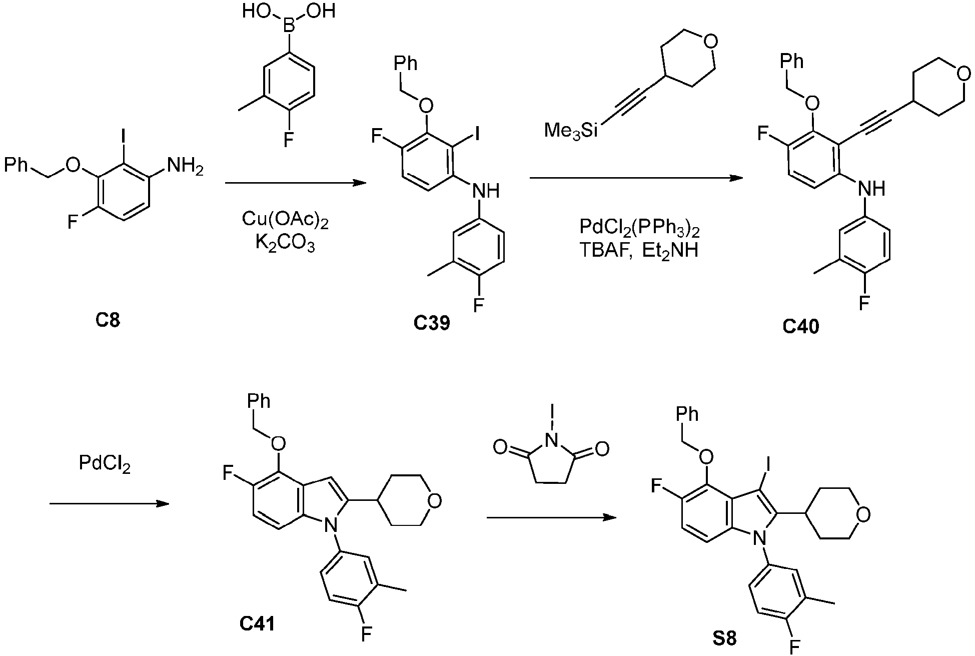

### Step 1. Synthesis of 3-benzyloxy-4-fluoro-N-(4-fluoro-3-methyl-phenyl)-2-iodo-aniline (C39)

3-benzyloxy-4-fluoro-2-iodo-aniline **C8** (7.1 g, 20.7 mmol), (4-fluoro-3-methylphenyl)boronic acid (6.4 g, 41.6 mmol), copper (II) acetate (5.6 g, 30.8 mmol) and K₂CO₃ (6.0 g, 43.4 mmol) were suspended in DMSO (100 mL) and the reaction was stirred at room temperature for 1 week. The reaction was combined with another batch of this reaction run on 3-benzyloxy-4-fluoro-2-iodo-aniline (1.6 g, 4.66 mmol) for workup and purification. The combined reactions were diluted with EtOAc, and filtered through a plug of Celite^{®}. The filtrate was washed with water, brine, and then the organic layer was concentrated to dryness. Purification by silica gel chromatography (Eluent: EtOAc in heptane) afforded the product as a dark brown oil which was used in the subsequent step without further purification. 3-benzyloxy-4-fluoro-N-(4-fluoro-3-methyl-phenyl)-2-iodo-aniline (5.76 g, 62%). LCMS *m*/*z* 452.0 [M+1]⁺. *Step 2 & 3. Synthesis of 4-benzyloxy-5-fluoro-1-(4-fluoro-3-methyl-phenyl)-2-tetrahydropyran-4-yl-indole (**C40**) and 4-benzyloxy-5-fluoro-1-(4-fluoro-3-methyl-phenyl)-2-tetrahydropyran-4-yl-indole (**C41**)*

A mixture of 3-benzyloxy-4-fluoro-N-(4-fluoro-3-methyl-phenyl)-2-iodo-aniline **C39** (5.76 g, 12.76 mmol), PdCl₂(PPh₃)₂ (538 mg, 0.77 mmol), and CuI (243 mg, 1.28 mmol) in DMF (40 mL) was degassed for 10 minutes. Trimethyl(2-tetrahydropyran-4-ylethynyl)silane (2.68 g, 14.7 mmol) and Et₂NH (1.52 mL, 14.7 mmol) were added, followed by TBAF (19.1 mL of 1 M, 19.1 mmol), and the reaction mixture was stirred overnight at 80 °C. The reaction mixture was diluted with water and extracted with EtOAc. The organic layer was concentrated to dryness and purified by silica gel chromatography (Gradient: 0-40% EtOAc in heptane) to afford the product **C40.** The product was dissolved in MeCN (40 mL) and PdCl₂ (200 mg, 1.13 mmol) was then added. The reaction mixture was then stirred overnight at 65 °C. The solvent was removed *in vacuo,* and the resulting material was triturated in MeCN, filtered, washed with heptane. The product was dried to give 4-benzyloxy-5-fluoro-1-(4-fluoro-3-methyl-phenyl)-2-tetrahydropyran-4-yl-indole as a tan solid (2.68 g, 48%). ¹H NMR (400 MHz, Chloroform-d) δ 7.60 - 7.54 (m, 2H), 7.44 (ddt, J = 8.0, 6.4, 1.0 Hz, 2H), 7.41 - 7.35 (m, 1H), 7.22 - 7.11 (m, 3H), 6.88 (dd, J = 11.8, 8.8 Hz, 1H), 6.59 (ddd, J = 8.8, 3.4, 0.8 Hz, 1H), 6.49 (t, J = 0.8 Hz, 1H), 5.34 (s, 2H), 3.99 (ddd, J = 11.7, 4.5, 1.9 Hz, 2H), 3.36 (td, J = 11.7, 2.4 Hz, 2H), 2.76 (tt, J = 11.5, 3.9 Hz, 1H), 2.38 (d, J = 2.0 Hz, 3H), 1.85 - 1.71 (m, 4H). LCMS *m*/*z* 434.0 [M+1]⁺.

### Step 3. Synthesis of 4-benzyloxy-5-fluoro-1-(4-fluoro-3-methyl-phenyl)-3-iodo-2-tetrahydropyran-4-yl-indole (S8)

To a solution of 4-benzyloxy-5-fluoro-1-(4-fluoro-3-methyl-phenyl)-2-tetrahydropyran-4-yl-indole **C40** (2.68 g, 6.18 mmol) in dichloromethane (30 mL) was added N-iodosuccinimide (1.46 g, 6.5 mmol). The reaction mixture was stirred at room temperature for 20 minutes, then washed with water and concentrated to dryness. Purification by silica gel chromatography (Gradient: 0-45% EtOAc in heptane) afforded the product as light yellow solid. 4-benzyloxy-5-fluoro-1-(4-fluoro-3-methyl-phenyl)-3-iodo-2-tetrahydropyran-4-yl-indole (2.84 g, 82%). ¹H NMR (400 MHz, Chloroform-d) δ 7.71 - 7.62 (m, 2H), 7.47 - 7.42 (m, 2H), 7.42 - 7.35 (m, 1H), 7.20 (td, J = 8.8, 1.5 Hz, 1H), 7.16 - 7.05 (m, 2H), 6.95 - 6.88 (m, 1H), 6.49 (dd, J = 8.9, 3.6 Hz, 1H), 5.26 (d, J = 2.7 Hz, 2H), 4.01 (dd, J = 11.6, 4.6 Hz, 2H), 3.38 (td, J = 11.9, 2.0 Hz, 2H), 3.11 (tt, J = 12.6, 3.6 Hz, 1H), 2.38 (d, J = 2.0 Hz, 3H), 1.59 (s, 4H). LCMS *m*/*z* 559.0 [M+1]⁺.

### Preparation of S9 and S10

### 4-benzyloxy-5-fluoro-1-(4-fluoro-3-methyl-phenyl)-2-tetrahydropyran-4-yl-indole (S9) and 4-benzyloxy-1-(4-fluoro-3-methyl-phenyl)-3-iodo-2-tetrahydropyran-4-yl-indole (S10)

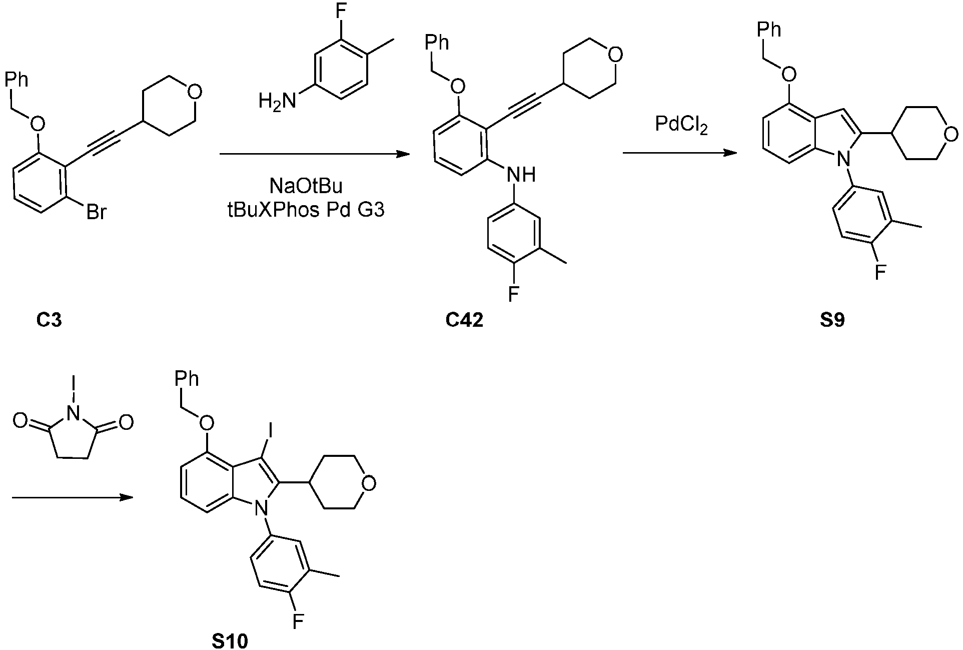

### Step 1. Synthesis of 4-benzyloxy-1-(4-fluoro-3-methyl-phenyl)-2-tetrahydropyran-4-yl-indole (C42)

A solution of 4-[2-(2-benzyloxy-6-bromo-phenyl)ethynyl]tetrahydropyran **C3** (2.85 g, 7.68 mmol) and 4-fluoro-3-methyl-aniline (1.8 g, 14.38 mmol) in m-xylene (40 mL) was degassed for 15 minutes. NaOtBu (2.2 g, 22.89 mmol) was added, followed by tBuXPhos Pd G3 (300 mg, 0.38 mmol). The reaction mixture was stirred at room temperature for 8 hours. The mixture was diluted with ice water (100 mL) and extracted with EtOAc (3 x 50 mL). The combined organics were concentrated to dryness and purified by silica gel chromatography (Gradient: 0-25% EtOAc in heptane) to provide the product. 3-benzyloxy-N-(4-fluoro-3-methylphenyl)-2-(2-tetrahydropyran-4-ylethynyl)aniline (3.21 g, 100%). ¹H NMR (400 MHz, Chloroform-d) δ 7.61 - 7.51 (m, 2H), 7.51 - 7.42 (m, 2H), 7.42 - 7.34 (m, 1H), 7.16 - 6.98 (m, 4H), 6.74 (dd, J = 8.3, 0.8 Hz, 1H), 6.46 (dd, J = 8.3, 0.8 Hz, 1H), 6.41 (s, 1H), 5.19 (s, 2H), 4.00 (ddd, J = 11.6, 6.2, 3.5 Hz, 2H), 3.60 (ddd, J = 11.3, 7.9, 3.1 Hz, 2H), 3.05 (tt, J = 8.1, 4.1 Hz, 1H), 2.33 (d, J = 2.0 Hz, 3H), 2.01 (ddt, J = 13.0, 6.7, 3.7 Hz, 2H), 1.84 (dtd, J = 13.3, 8.0, 3.5 Hz, 2H). LCMS *m*/*z* 416.34 [M+H]⁺.

### Step 2. Synthesis of 4-benzyloxy-1-(4-fluoro-3-methyl-phenyl)-2-tetrahydropyran-4-yl-indole (S9)

To a solution of 3-benzyloxy-N-(4-fluoro-3-methyl-phenyl)-2-(2-tetrahydropyran-4-ylethynyl)aniline **C42** (3.2 g, 7.6 mmol) in acetonitrile (40 mL) was added PdCl₂ (70 mg, 0.4 mmol). The reaction mixture was stirred at 50 °C for 2 hours, then overnight at same temperature. The solution was concentrated to dryness and purified via silica gel chromatography (Gradient: 0-20% EtOAc in heptane) to give the product as light yellow solid. 4-benzyloxy-1-(4-fluoro-3-methyl-phenyl)-2-tetrahydropyran-4-yl-indole (2.7 g, 85%). ¹H NMR (400 MHz, Chloroform-d) δ 7.66 - 7.55 (m, 2H), 7.48 (t, J = 7.4 Hz, 2H), 7.41 (t, J = 7.2 Hz, 1H), 7.27 - 7.16 (m, 3H), 7.07 (t, J = 8.0 Hz, 1H), 6.76 - 6.60 (m, 3H), 5.31 (s, 2H), 4.03 (ddd, J = 11.6, 4.5, 1.8 Hz, 2H), 3.41 (td, J = 11.8, 2.3 Hz, 2H), 2.86 (tt, J = 11.6, 3.9 Hz, 1H), 2.43 (d, J = 1.9 Hz, 3H), 1.97 - 1.84 (m, 2H), 1.83 - 1.78 (m, 2H). LCMS *m*/*z* 416.38 [M+H]⁺.

### Step 3. Synthesis of 4-benzyloxy-1-(4-fluoro-3-methyl-phenyl)-3-iodo-2-tetrahydropyran-4-yl-indole (S10)

To a solution of 4-benzyloxy-1-(4-fluoro-3-methyl-phenyl)-2-tetrahydropyran-4-yl-indole **S9** (2.7 g, 6.5 mmol) in dichloromethane (30 mL) was added N-iodosuccinimide (1.5 g, 6.67 mmol). The solution was stirred at room temperature for 30 minutes. The reaction mixture was diluted with water, and then the organic layer separated and concentrated *in vacuo.* Purification by silica gel chromatography (Gradient: 0-25% EtOAc in heptane) afforded the product as a white solid. 4-benzyloxy-1-(4-fluoro-3-methyl-phenyl)-3-iodo-2-tetrahydropyran-4-yl-indole (3.5 g, 99%). ¹H NMR (400 MHz, Chloroform-*d*) δ 7.70 - 7.63 (m, 2H), 7.47 - 7.40 (m, 2H), 7.40 - 7.33 (m, 1H), 7.19 (t, J = 8.7 Hz, 1H), 7.16 - 7.07 (m, 2H), 7.01 (td, J = 8.1, 4.1 Hz, 1H), 6.69 - 6.61 (m, 1H), 6.50 - 6.43 (m, 1H), 5.27 (s, 2H), 4.00 (dd, J = 11.5, 4.5 Hz, 2H), 3.38 (td, J = 11.9,2.1 Hz, 2H), 3.14 (tt, J = 12.4, 3.5 Hz, 1H), 2.38 (d, J = 2.1 Hz, 3H), 2.30 (td, J = 12.3, 4.4 Hz, 2H), 1.59 - 1.50 (m, 2H). LCMS *m*/*z* 541.33 [M+H]⁺.

### Preparation of S11

### 1-(4-fluoro-3-methyl-phenyl)-2-isopropyl-4-methoxy-indole (S11)

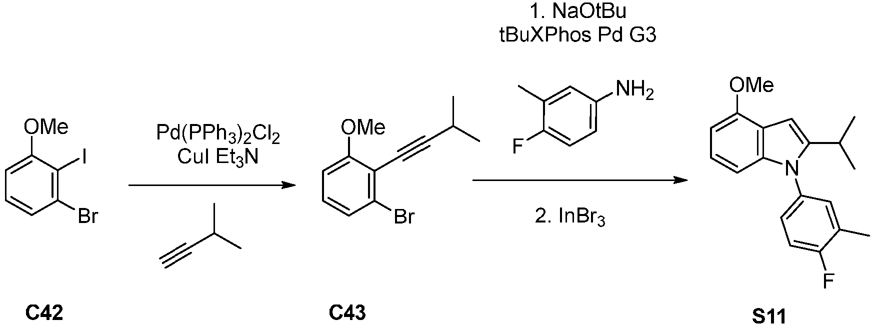

### Step 1. Synthesis of 1-bromo-3-methoxy-2-(3-methylbut-1-ynyl)benzene (C43)

A mixture of 3-methylbut-1-yne (6.6 mL, 63.95 mmol), 1-bromo-2-iodo-3-methoxybenzene **C42** (5 g, 15.98 mmol) in Et₃N (30 mL) and DMF (15 mL) was purged with nitrogen for 5 minutes, Pd(PPh₃)₂Cl₂ (1.1 g, 1.57 mmol), CuI (310 mg, 1.63 mmol) were added and the reaction mixture stirred at room temperature for 60 hours. The reaction mixture was then poured into water (200 mL), extracted with EtOAc (2 x 100 mL), washed with water, then dried over Na₂SO₄. The solvent was removed under reduced pressure. Purification by silica gel chromatography (Gradient: 0-100% EtOAc in heptane), then purified by reversed-phase chromatography (Column: C18. Gradient: 0-100% MeCN in water with 0.1% trifluoroacetic acid) afforded the product 1-bromo-3-methoxy-2-(3-methylbut-1-ynyl)benzene (2.6 g, 62%) as a thick oil. ¹H NMR (400 MHz, Chloroform-d) δ 7.19 (d, J = 1.0 Hz, 1H), 7.09 (t, J = 8.2 Hz, 1H), 6.82 (dd, J = 8.3, 1.0 Hz, 1H), 3.89 (s, 3H), 2.92 (p, J = 6.9 Hz, 1H), 1.35 (d, J = 6.9 Hz, 6H). LCMS *m*/*z* 253.39 [M+H]⁺.

### Step 2. Synthesis of 1-(4-fluoro-3-methyl-phenyl)-2-isopropyl-4-methoxy-indole (S11)

A solution of 4-fluoro-3-methyl-aniline (1.93 g, 15.4 mmol), 1-bromo-3-methoxy-2-(3-methylbut-1-ynyl)benzene **C43** (2.6 g, 10.3 mmol) in t-BuOH (8 mL) and 1,4-dioxane (12 mL) was purged with nitrogen for 10 minutes. Sodium t-butoxide (1.9 g, 19.8 mmol) was added, followed by tBuXPhos Pd G3 (352 mg, 0.51 mmol) then after 5 minutes. The reaction was stirred at room temperature for 18 hours. The reaction mixture was diluted with water (100 mL) and EtOAc (100 mL). The organic layer was separated and aq. layer was extracted with EtOAc (2x 50 mL). The combined organic layers was dried over Na₂SO₄ and the solvent was evaporated under reduced pressure to afford the product N-(4-fluoro-3-methyl-phenyl)-3-methoxy-2-(3-methylbut-1-ynyl)aniline (2.3 g, 75%).

InBr₃ (730 mg, 2.059 mmol) was added to a solution of N-(4-fluoro-3-methylphenyl)-3-methoxy-2-(3-methylbut-1-ynyl)aniline (2.3 g) in toluene (25 mL) and the solution was heated at 110 °C for 3 hours. The solvent was removed under reduced pressure, and the residue dissolved in EtOAc (25 mL), then washed with water. The organic layer was dried and concentrated under reduced pressure. Purification by silica gel chromatography (Gradient: 0-40% EtOAc in heptane) afforded 1-(4-fluoro-3-methyl-phenyl)-2-isopropyl-4-methoxy-indole (2 g, 65%) as yellow solid. ¹H NMR (400 MHz, Chloroform-d) δ 7.20 - 7.06 (m, 2H), 7.03 (t, J = 8.0 Hz, 1H), 6.63 (dt, J = 8.3, 0.8 Hz, 1H), 6.58 - 6.47 (m, 2H), 4.01 (s, 3H), 2.94-2.91 (m, 1H), 2.37 (dd, J = 2.2, 0.7 Hz, 3H), 1.23-1.16 (m, 6H). LCMS *m*/*z* 298.52 [M+1]⁺.

### Preparation of S12

### 4-benzyloxy-1-(4-fluoro-3-methyl-phenyl)-2-isopropyl-indole (S12)

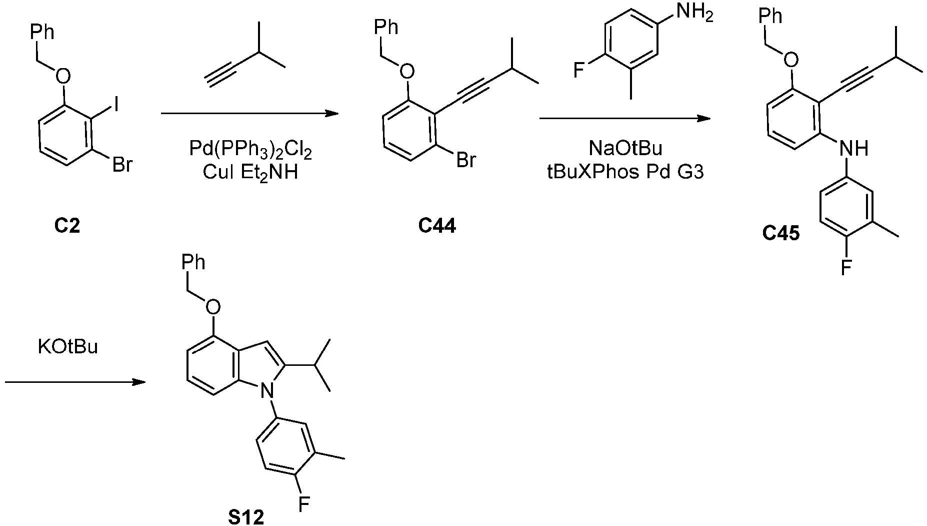

### Step 1. Synthesis of 1-benzyloxy-3-bromo-2-(3-methylbut-1-ynyl)benzene (C44)

A solution of 1-benzyloxy-3-bromo-2-iodo-benzene C2 (7 g, 17.99 mmol), PdCl₂(PPh₃)₂ (757 mg, 1.08 mmol) and CuI (343 mg, 1.80 mmol) in anhydrous DMF (60 mL) was degassed for 10 minutes. 3-methylbut-1-yne (2.76 mL, 26.99 mmol) and diethylamine (2.79 mL, 26.97 mmol) were added and the reaction mixture was stirred overnight at 60 °C in a sealed tube. The reaction mixture was cooled to room temperature, diluted with water (60 mL) and extracted EtOAc (3 x). The organic layer was concentrated to dryness, dissolved in MTBE, and washed with water. Purification by silica gel chromatography (Gradient: 0-35% EtOAc in heptane) yielded the product with was used in the subsequent reaction without further purification. 1-benzyloxy-3-bromo-2-(3-methylbut-1-ynyl)benzene (5.6 g, 95%). ¹H NMR (400 MHz, Chloroform-*d*) δ 7.53 - 7.51 (m, 2H), 7.45 - 7.36 (m, 3H), 7.23 - 7.18 (m, 1H), 7.06 (t, J = 8.2 Hz, 1H), 6.86 (dd, J = 8.3, 1.0 Hz, 1H), 5.17 (s, 2H), 2.92 (hept, J = 6.9 Hz, 1H), 1.34 (d, J = 6.9 Hz, 6H).

### Step 2. Synthesis of 3-benzyloxy-N-(4-fluoro-3-methyl-phenyl)-2-(3-methylbut-1-ynyl)aniline (C45)

A mixture of 1-benzyloxy-3-bromo-2-(3-methylbut-1-ynyl)benzene **C44** (933 mg, 2.83 mmol), 4-fluoro-3-methyl-aniline (413 mg, 3.3 mmol), NaOtBu (817 mg, 8.50 mmol) and tBuXPhos Pd G3 (113 mg, 0.14 mmol) was added to a vial. The vial was sealed and was purged with one vacuum/nitrogen cycle. 1,4-Dioxane (7.1 mL) was added and the mixture was stirred overnight at 50 °C. The reaction was evaporated to dryness and the residue was triturated with EtOAc. The reaction was filtered over a pad of Celite^{®} and rinsed using additional EtOAc. The combined organic layers were dried over Na₂SO₄, filtered and concentrated providing an inseparable mixture of 3-benzyloxy-N-(4-fluoro-3-methyl-phenyl)-2-(3-methylbut-1-ynyl)aniline and 4-benzyloxy-1-(4-fluoro-3-methyl-phenyl)-2-isopropyl-indole (1058 mg, 100%) which was used in the subsequent step without further purification. LCMS *m*/*z* 374.25 [M+1]⁺.

### Step 3. Synthesis of 4-benzyloxy-1-(4-fluoro-3-methyl-phenyl)-2-isopropyl-indole (S12)

In a flask, to a solution of 3-benzyloxy-N-(4-fluoro-3-methyl-phenyl)-2-(3-methylbut-1-ynyl)aniline **C45**(1058 mg, 2.83 mmol) in THF (11.3 mL) was added NaOtBu (973 mg, 8.5 mmol).

The reaction was stirred at 60 °C for 30 minutes. The reaction was cooled to room temperature and was filtered through a pad of Celite^{®}, and rinsed with additional EtOAc. The combined organic phases were evaporated and purified by silica gel chromatography (Gradient: 0-100% EtOAc in heptane) to afford the product. 4-benzyloxy-1-(4-fluoro-3-methyl-phenyl)-2-isopropyl-indole (550 mg, 49%). ¹H NMR (400 MHz, Chloroform-*d*) δ 7.54 (d, J = 7.5 Hz, 2H), 7.41 (t, J = 7.5 Hz, 2H), 7.37 - 7.31 (m, 1H), 7.19 - 7.11 (m, 3H), 6.97 (t, J = 8.0 Hz, 1H), 6.64 - 6.55 (m, 3H), 5.24 (s, 2H), 2.97 - 2.83 (m, 1H), 2.34 (s, 3H), 1.22 - 1.17 (m, 6H). LCMS *m*/*z* 374.25 [M+H]⁺.

### Preparation of S13 and S14

### 4-benzyloxy-1-(4-fluorophenyl)-2-tetrahydropyran-4-yl-indole (S13) and 4-benzyloxy-1-(4-fluorophenyl)-3-iodo-2-tetrahydropyran-4-yl-indole (S14)

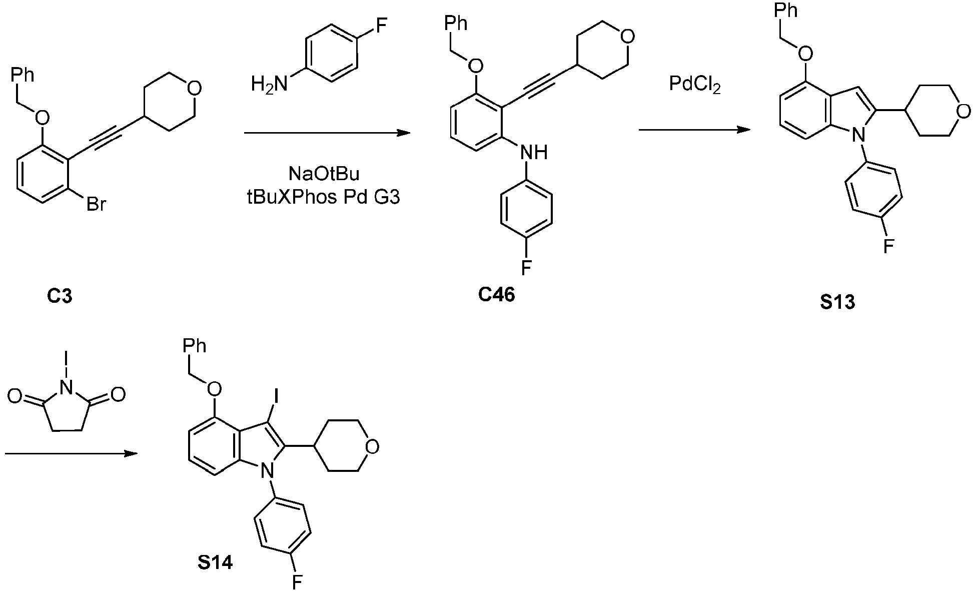

### Step 1. Synthesis of 3-benzyloxy-N-(4-fluorophenyl)-2-(2-tetrahydropyran-4-ylethynyl)aniline (C46)

To a solution of 4-[2-(2-benzyloxy-6-bromo-phenyl)ethynyl]tetrahydropyran **C3** (74.5 g, 200.7 mmol) and 4-fluoroaniline (23.0 mL) in m-xylene (900 mL) was added NaOtBu (58 g, 603.5 mmol) followed by tBuXPhos Pd G3 (8 g, 10.07 mmol). The reaction mixture was stirred at room temperature for 20 hours, then diluted with water (1L) and extracted with EtOAc (3 x 500 mL). The combined organics were concentrated to dryness and purified via silica gel chromatography (Gradient: 0-35% EtOAc in heptane) afforded the product as a brown oil. 3-benzyloxy-N-(4-fluorophenyl)-2-(2-tetrahydropyran-4-ylethynyl)aniline (73 g, 89%). ¹H NMR (300 MHz, Chloroform-*d*) δ 7.57 - 7.47 (m, 2H), 7.46 - 7.31 (m, 3H), 7.18 (ddt, J = 8.2, 5.7, 2.8 Hz, 2H), 7.13 - 6.98 (m, 3H), 6.74 - 6.60 (m, 1H), 6.51 - 6.31 (m, 2H), 5.16 (s, 2H), 3.96 (ddd, J = 11.6, 6.2, 3.5 Hz, 2H), 3.56 (ddd, J = 11.4, 7.9, 3.2 Hz, 2H), 3.02 (tt, J = 8.1, 4.1 Hz, 1H), 2.04 - 1.88 (m, 2H), 1.80 (dtd, J = 13.4, 8.0, 3.5 Hz, 2H). LCMS *m*/*z* 402.15 [M+H]⁺.

### Step 2. Synthesis of 4-benzyloxy-1-(4-fluorophenyl)-2-tetrahydropyran-4-yl-indole (S13)

To a solution of 3-benzyloxy-N-(4-fluorophenyl)-2-(2-tetrahydropyran-4-ylethynyl)aniline **C46** (6.08 g, 15.14 mmol) in acetonitrile (50 mL) was added PdCl₂ (269 mg, 1.52 mmol). The reaction mixture was stirred at 50 °C for 2 hours (significant ppt observed after 10 min) then filtered, washed with heptane, and dried to give 4.8 g light gray solid. The filtrate was concentrated to dryness and purified via silica gel chromatography eluting with 0-30% EtOAc in heptane. Pure fractions were combined and concentrated to give an additional 280 mg of desired product. 4-benzyloxy-1-(4-fluorophenyl)-2-tetrahydropyran-4-yl-indole (5.1 g, 84%). ¹H NMR (400 MHz, Chloroform-d) δ 7.59 - 7.53 (m, 2H), 7.48 - 7.41 (m, 2H), 7.41 - 7.32 (m, 3H), 7.28 - 7.23 (m, 2H), 7.03 (t, J = 8.1 Hz, 1H), 6.69 - 6.60 (m, 3H), 5.27 (s, 2H), 3.99 (ddd, J = 11.6, 4.7, 1.8 Hz, 2H), 3.35 (td, J = 11.9, 2.2 Hz, 2H), 2.80 (tt, J = 11.6, 3.8 Hz, 1H), 1.85 (dtd, J = 13.5, 11.9, 4.3 Hz, 2H), 1.74 (dq, J = 13.1, 2.0 Hz, 2H). LCMS *m*/*z* 402.0 [M+H]⁺.

### Step 3. Synthesis of 4-benzyloxy-1-(4-fluorophenyl)-3-iodo-2-tetrahydropyran-4-yl-indole (S14)

To a solution of 4-benzyloxy-1-(4-fluorophenyl)-2-tetrahydropyran-4-yl-indole **S13** (2 g, 4.98 mmol) in dichloromethane (25 mL) at 0 °C was added N-iodosuccinimide (1.18 g, 5.25 mmol). The reaction mixture was stirred at 0 °C for 1 hour then washed with water. The organic layer was concentrated to dryness and purified via silica gel chromatography (Gradient: 0-30% EtOAc in heptane) to afford the product as a yellow solid. 4-benzyloxy-1-(4-fluorophenyl)-3-iodo-2-tetrahydropyran-4-yl-indole (2.4 g, 91%). ¹H NMR (400 MHz, Chloroform-d) δ 7.67 (ddt, J = 7.5, 1.4, 0.7 Hz, 2H), 7.47 - 7.41 (m, 2H), 7.39 - 7.33 (m, 1H), 7.30 - 7.24 (m, 4H), 7.02 (td, J = 8.1, 4.2 Hz, 1H), 6.65 (ddd, J = 7.9, 3.2, 0.7 Hz, 1H), 6.48 (ddd, J = 15.4, 8.3, 0.7 Hz, 1H), 5.28 (s, 2H), 4.05 - 3.95 (m, 2H), 3.37 (td, J = 11.9, 2.0 Hz, 2H), 3.14 (tt, J = 12.5, 3.6 Hz, 1H), 2.35 (dqd, J = 60.9, 12.7, 4.6 Hz, 2H), 1.58 - 1.54 (m, 2H). LCMS *m*/*z* 527.0 [M+1]+.

### Preparation of S15

### 4-benzyloxy-5-fluoro-1-(4-fluorophenyl)-3-iodo-2-tetrahydropyran-4-yl-indole (S15)

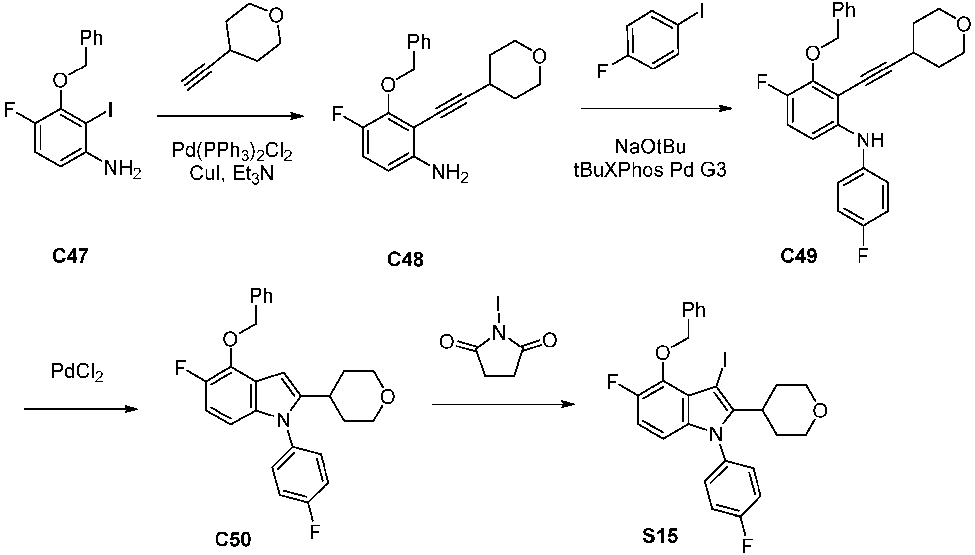

### Step 1. Synthesis of 3-benzyloxy-4-fluoro-2-(2-tetrahydropyran-4-ylethynyl)aniline (C48)

3-benzyloxy-4-fluoro-2-iodo-aniline (670 mg, 1.95 mmol), 4-ethynyltetrahydropyran C47 (280 mg, 2.5 mmol), PdCl₂PPh₃ (210 mg, 0.3 mmol) and CuI (56 mg, 0.3 mmol) were mixed into 1,4-dioxane (10 mL) and Et₃N (8 mL) and the reaction was heated at 60 °C for overnight. The reaction was cooled to room temperature, and filtered through a plug of Celite^{®}. The crude product was diluted with EtOAc and washed with water. The organic layer was dried and concentrated. The crude was purified on silica gel (40 g column, 10-90% Hex: EtOAc) to afford desired product.3-benzyloxy-4-fluoro-2-(2-tetrahydropyran-4-ylethynyl)aniline (480 mg, 72%) LCMS *m*/*z* 326.56 [M+H]⁺.

### Step 2. Synthesis of 3-benzyloxy-4-fluoro-N-(4-fluorophenyl)-2-(2-tetrahydropyran-4-ylethynyl)aniline (C48)

A mixture of 3-benzyloxy-4-fluoro-2-(2-tetrahydropyran-4-ylethynyl)aniline **C47** (450 mg, 1.38 mmol), 1-fluoro-4-iodo-benzene (380 mg, 1.71 mmol), tBuXphos Pd G3 (110 g, 138.5 mmol) and NaOtBu (280 mg, 2.914 mmol) in 1,4- dioxane (3 mL) and t-BuOH (3 mL) was stirred at 60 °C overnight. The reaction was cooled to room temperature, then and diluted with EtOAc and washed with water. The organic layer was dried and concentrated. Purification by silica gel chromatography (Gradient: 10-40% EtOAc in hexane) afforded the desired product. 3-benzyloxy-4-fluoro-N-(4-fluorophenyl)-2-(2-tetrahydropyran-4-ylethynyl)aniline (388 mg, 52%). LCMS *m*/*z* 420.11 [M+H]⁺.

### Step 3. Synthesis of 4-benzyloxy-5-fluoro-1-(4-fluorophenyl)-2-tetrahydropyran-4-yl-indole (C49)

3-benzyloxy-4-fluoro-N-(4-fluorophenyl)-2-(2-tetrahydropyran-4-ylethynyl)aniline **C48** (1.01 g, 2.408 mmol) and PdCl₂ (40 mg, 0.23 mmol) was dissolved into CH₃CN (30 mL) and the reaction was stirred at room temperature for overnight. The reaction was concentrated and diluted with EtOAc and washed with water. The organic layer was dried and concentrated. The crude was purified on silica gel (4 g column, 10-40% Hex: EtOAc) to afford the desired product. 4-benzyloxy-5-fluoro-1-(4-fluorophenyl)-2-tetrahydropyran-4-yl-indole (830 mg, 78%). ¹H NMR (400 MHz, DMSO-*d*₆) δ 7.56 - 7.48 (m, 4H), 7.48 - 7.39 (m, 4H), 7.39 - 7.33 (m, 1H), 6.94 (dd, J = 11.8, 8.8 Hz, 1H), 6.64 - 6.53 (m, 2H), 5.29 (s, 2H), 3.84 (dt, J = 11.3, 3.3 Hz, 2H), 3.27 - 3.13 (m, 2H), 2.76 (tt, J = 10.2, 5.4 Hz, 1H), 1.76 - 1.59 (m, 4H). LCMS *m*/*z* 420.2 [M+H]⁺

### Step 4. Synthesis of 4-benzyloxy-5-fluoro-1-(4-fluorophenyl)-3-iodo-2-tetrahydropyran-4-yl-indole (C50)

1-iodopyrrolidine-2,5-dione (420 mg, 1.867 mmol) was added to a solution of 4-benzyloxy-5-fluoro-1-(4-fluorophenyl)-2-tetrahydropyran-4-yl-indole **C48** (740 mg, 1.76 mmol) was dissolved in dichloromethane (10 mL) and reaction was stirred at room temperature for overnight. The reaction was concentrated and diluted with EtOAc and washed with water. The organic layer was dried and concentrated. The crude was purified on silica gel (Gradient: 10-40% EtOAc in hexane) to afford the product. 4-benzyloxy-5-fluoro-1-(4-fluorophenyl)-3-iodo-2-tetrahydropyran-4-yl-indole (820 mg, 78%). ¹H NMR (400 MHz, DMSO-*d*₆) δ 7.65 - 7.60 (m, 2H), 7.53 (ddt, J = 8.3, 5.5, 2.7 Hz, 2H), 7.50 - 7.41 (m, 4H), 7.41 - 7.35 (m, 1H), 7.04 (dd, J = 11.6, 8.9 Hz, 1H), 6.52 (dd, J = 8.9, 3.7 Hz, 1H), 5.14 (s, 2H), 3.86 (dd, J = 11.5, 4.2 Hz, 2H), 3.25 - 3.11 (m, 2H), 3.02 - 2.81 (m, 1H), 2.18 (qd, J = 12.6, 4.4 Hz, 2H), 1.56 (d, J = 12.3 Hz, 2H). LCMS *m*/*z* 545.04 [M+H]⁺.

### Preparation of S16

### 4-benzyloxy-6-fluoro-1-(4-fluorophenyl)-3-iodo-2-tetrahydropyran-4-yl-indole (S16)

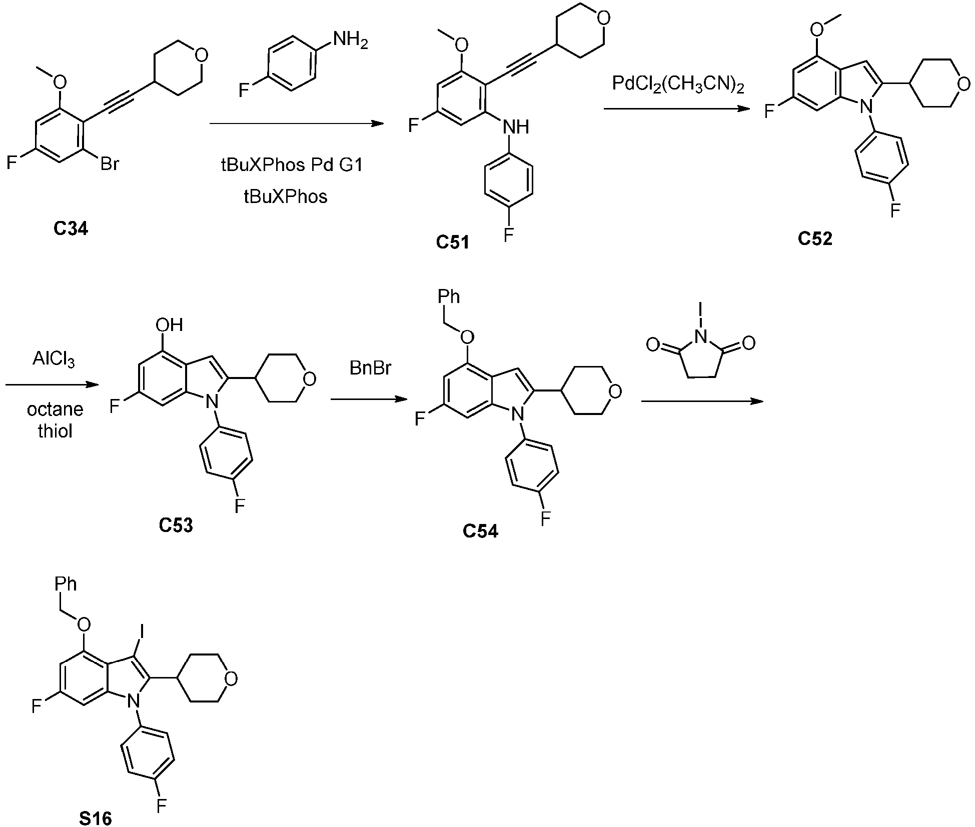

### Step 1.Synthesis of 5-fluoro-N-(4-fluorophenyl)-3-methoxy-2-(2-tetrahydropyran-4-ylethynyl)aniline (C51)

A 2 L 3-neck round bottomed flask with overhead stirrer, temperature probe, and nitrogen inlet was charged with 4-[2-(2-bromo-4-fluoro-6-methoxyphenyl)ethynyl]tetrahydropyran **C34** (53 g, 159.1 mmol), 4-fluoroaniline (26 g, 234 mmol) and NaOtBu (38 g, 395.4 mmol) in THF (750 mL), the mixture was stirred for 5 minutes, and then purged with nitrogen for ~10 minutes. tBuXPhos Pd G1 (3 g, 4.61 mmol), followed by tBu XPhos (2 g, 4.71 mmol) were added. The reaction mixture was purged with nitrogen for an additional 10 minutes. The resulting reaction mixture was warmed to 50 °C (Tₘₐₓ ~62 °C), and stirred at this temperature for 14 hours. The reaction mixture was cooled to room temperature, poured into ice/water (~150 mL). Then sat. aqueous NH₄Cl solution (~200 mL) and ethyl acetate (~500 mL) were added, and the mixture stirred for 10 minutes. The organic phase was separated, washed with brine (~100 mL), dried over MgSO₄, filtered and concentrated under reduced pressure. The residue was purified by passing over a silica gel (~600 g) plug column, (Eluting with 0-30% ethyl acetate in heptane) to afford the product **C51** 5-fluoro-N-(4-fluorophenyl)-3-methoxy-2-(2-tetrahydropyran-4-ylethynyl)aniline (40 g, 73%), mixed with some of the cyclized product **C52,** as a light brown oil. This material was used in the next step without further purification. LCMS *m*/*z* 344.44 [M+H]⁺.

### Step 2. Synthesis of 6-fluoro-1-(4-fluorophenyl)-4-methoxy-2-tetrahydropyran-4-yl-indole (C52)

A 1 L 3-neck RB flask with magnetic stirrer, temperature probe, and nitrogen inlet was charged with methanol (300 mL) and purged with nitrogen for 30 minutes and then warmed to 60 °C for 10 minutes. A separate 2 L 3-neck round bottomed flask with overhead stirrer, temperature probe, and nitrogen inlet was charged PdCl₂(CH₃CN)₂ (1 g, 3.86 mmol) and the degassed methanol was transferred to this flask. The mixture was stirred for 5 minutes, and then a degassed solution of 5-fluoro-N-(4-fluorophenyl)-3-methoxy-2-(2-tetrahydropyran-4-ylethynyl)aniline **C51** (40 g) in ethyl acetate (400 mL) was added. The resulting reaction mixture was warmed to 60 °C, stirred at this temperature for 3 hours. The reaction mixture was cooled to room temperature, concentrated under reduced pressure to afford yellowish light brown solid. The product mixture was purified by silica gel chromatography (Eluent: dichloromethane).The product residue was then treated with EtOAc (~200 mL), heated to reflux, then heptane (~500 mL) was added. The mixture was left to stand at room temperature for 2 hours. The resulting solid was filtered, washed with heptane (~100 mL), then dried under suction to afford 6-fluoro-1-(4-fluorophenyl)-4-methoxy-2-tetrahydropyran-4-yl-indole (27 g, 49%) as white crystals. ¹H NMR (300 MHz, DMSO-*d*₆) δ 7.58 - 7.35 (m, 4H), 6.53 (dd, J = 12.0, 2.0 Hz, 1H), 6.42 (d, J = 0.8 Hz, 1H), 6.26 (ddd, J = 9.7, 2.1, 0.8 Hz, 1H), 3.89 (s, 3H), 3.82 (dt, J = 11.3, 3.2 Hz, 2H), 3.20 (ddd, J = 11.5, 7.8, 5.1 Hz, 2H), 2.73 (q, J = 8.3, 7.7 Hz, 1H), 1.62 (tt, J = 6.8, 3.9 Hz, 4H). ¹⁹F NMR (282 MHz, DMSO-*d*₆) δ -112.99, -118.62. LCMS *m*/*z* 344.24 [M+H]⁺.

### Step 3. Synthesis of 6-fluoro-1-(4-fluorophenyl)-2-tetrahydropyran-4-yl-indol-4-ol (C53)

A 5 L 3-neck RB flask with overhead stirrer, temperature probe and nitrogen inlet was charged with 6-fluoro-1-(4-fluorophenyl)-4-methoxy-2-tetrahydropyran-4-yl-indole C52 (60 g, 166 mmol) in dichloromethane (1 L), stirred for 5 minutes and then cooled to 0 °C with an ice/water bath. AlCl₃ (80 g, 600 mmol) was added, the mixture was then stirred for 10 minutes, and then octane-1-thiol (120 mL, 691.5 mmol) was added. The cooling bath was removed and the resulting mixture was stirred at room temperature for 5 hours. The reaction mixture was poured into ice/water (~500 mL), then 2 *N* HCl (~500 mL) followed by ethyl acetate (~1.5 L) was added, and the mixture stirred for ~20 minutes. The organic phase was separated, washed with brine (~300 mL), dried over MgSO₄, filtered and concentrated under reduced pressure. The residue was triturated with ~10% MTBE in heptane (~1.2 L), and dried under vacuum to afford the product as a tan solid. 6-fluoro-1-(4-fluorophenyl)-2-tetrahydropyran-4-yl-indol-4-ol (46 g, 84%). ¹H NMR (300 MHz, DMSO-*d*₆) δ 10.01 (s, 1H), 7.45 (qdd, J = 9.0, 5.8, 2.5 Hz, 4H), 6.46 (d, J = 0.8 Hz, 1H), 6.27 (dd, J = 11.6, 2.1 Hz, 1H), 6.09 (ddd, J = 9.8, 2.2, 0.7 Hz, 1H), 3.88 - 3.77 (m, 2H), 3.21 (td, J = 11.2, 3.9 Hz, 2H), 2.81 - 2.67 (m, 1H), 1.69 - 1.50 (m, 4H). ¹⁹F NMR (282 MHz, DMSO-*d*₆) δ -113.15, -119.80. LCMS *m*/*z* 330.09 [M+H]⁺.

### Step 4. Synthesis of 4-benzyloxy-6-fluoro-1-(4-fluorophenyl)-2-tetrahydropyran-4-yl-indole (C54)

A 3 L 3-neck round bottom flask with overhead stirrer, temperature probe, reflux condenser and nitrogen inlet was charged 6-fluoro-1-(4-fluorophenyl)-2-tetrahydropyran-4-yl-indol-4-ol **C53** (46 g, 139.7 mmol) in a mixture of THF (500 mL) and DMF (100 mL), was stirred for 5 minutes, Cs₂CO₃ (93 g, 285.4 mmol) was added, followed by benzylbromide (20 mL, 168.2 mmol). The resulting reaction mixture (white suspension) was stirred at room temperature for 7 hours. The reaction mixture was diluted with ethyl acetate (400 mL), stirred for 10 minutes and then the resulting white suspension was filtered through medium fritted funnel, and washed with ethyl acetate (50 mL). The combined filtrate was washed with water (~200 mL), brine (~200 mL), dried over MgSO₄, filtered and concentrated under reduced pressure. The residue was triturated with MTBE (~500 mL), and dried under vacuum to afford 4-benzyloxy-6-fluoro-1-(4-fluorophenyl)-2-tetrahydropyran-4-yl-indole (51 g, 87%) as a white solid. ¹H NMR (300 MHz, DMSO-*d*₆) δ 7.60 - 7.30 (m, 9H), 6.63 (dd, J = 11.9, 2.0 Hz, 1H), 6.46 (s, 1H), 6.35 - 6.19 (m, 1H), 5.25 (s, 2H), 3.81 (dt, J = 11.2, 3.2 Hz, 2H), 3.27 - 3.10 (m, 2H), 2.75 (p, J = 8.2 Hz, 1H), 1.63 (tt, J = 9.1, 3.8 Hz, 4H). ¹⁹F NMR (282 MHz, DMSO-*d*₆) δ - 112.95, -118.69. LCMS *m*/*z* 545.07 [M+H]⁺.

### Step 5. Synthesis of 4-benzyloxy-6-fluoro-1-(4-fluorophenyl)-3-iodo-2-tetrahydropyran-4-yl-indole (S16)

To a solution of 4-benzyloxy-6-fluoro-1-(4-fluorophenyl)-2-tetrahydropyran-4-yl-indole **C54** (10.2 g, 24.3 mmol) in dichloromethane (125 mL) at 0 °C N-iodosuccinimide (5.75 g, 25.6 mmol) was added portionwise over 2 minutes. The reaction mixture was stirred for 10 minutes at 0 °C then washed with water and brine. The organic layer was concentrated to dryness, triturated with EtOAc, filtered, washed with heptane. The resulting solid was dried to afford the product as an off-white solid. The trituration process was repeated with the filtrate to recover an additional 1.3 g of product. The batches of solid were combined to give 12.0 g of 4-benzyloxy-6-fluoro-1-(4-fluorophenyl)-3-iodo-2-tetrahydropyran-4-yl-indole(12 g, 91%). ¹H NMR (400 MHz, Chloroform-d) δ 7.64 (ddt, J = 7.5, 1.4, 0.7 Hz, 2H), 7.48 - 7.41 (m, 2H), 7.40 - 7.34 (m, 1H), 7.27 (dd, J = 6.8, 1.8 Hz, 4H), 6.45 (dd, J = 11.5, 2.1 Hz, 1H), 6.13 (dd, J = 9.2, 2.1 Hz, 1H), 5.23 (s, 2H), 4.02 - 3.96 (m, 2H), 3.36 (td, J = 11.9, 2.0 Hz, 2H), 3.09 (tt, J = 12.6, 3.5 Hz, 1H), 2.25 (qd, J = 12.6, 4.4 Hz, 2H), 1.58 - 1.52 (m, 2H). LCMS *m*/*z* 545.0 [M+H]⁺.

### Preparation of S17

### 4-benzyloxy-6-fluoro-1-(4-fluorophenyl)indole (S17)

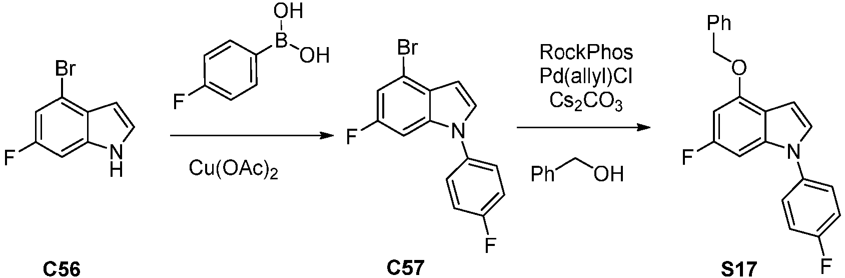

### Step 1. Synthesis of 4-bromo-6-fluoro-1-(4-fluorophenyl)indole (C57)

To a mixture of 4-bromo-6-fluoro-1H-indole **C56** (5 g, 23.4 mmol), (4-fluorophenyl)boronic acid (6.54 g, 46.74 mmol) and copper (II) acetate (8.5 g, 46.8 mmol) in dichloromethane (100 mL) was added triethylamine (6.5 mL, 46.6 mmol) and the mixture stirred vigorously in air. Additional dichloromethane (100 mL), 4-fluorophenyl boronic acid (5.7g), Cu(OAc)₂, and NEt₃ (6 mL) were added and the mixture was stirred vigorously. The reaction mixture was filtered through Celite^{®} with the aid of EtOAc and then concentrated. Purification by column chromatography (Gradient: 0-50% EtOAc in heptane) afforded the product as a white solid. 4-bromo-6-fluoro-1-(4-fluorophenyl)indole (2.84 g, 39%). ¹H NMR (400 MHz, DMSO-*d*₆) δ 7.78 (d, J = 3.3 Hz, 1H), 7.69 - 7.62 (m, 2H), 7.47 - 7.40 (m, 2H), 7.38 (dd, J = 9.1, 2.1 Hz, 1H), 7.31 (ddd, J = 9.9, 2.1, 0.9 Hz, 1H), 6.66 (dd, J = 3.4, 0.8 Hz, 1H). LCMS *m*/*z 308.02* [M+1]⁺.

### Step 2. Synthesis of 4-benzyloxy-6-fluoro-1-(4-fluorophenyl)indole (S17)

A vial was charged with 4-bromo-6-fluoro-1-(4-fluorophenyl)indole **C57** (2.14 g, 6.95 mmol), palladium allyl chloride (38 mg, 0.21 mmol), ditert-butyl-[6-methoxy-3-methyl-2-(2,4,6-triisopropylphenyl)phenyl]phosphane (293 mg, 0.63 mmol), Cs₂CO₃ (4.2 g, 12.9 mmol) then toluene (14 mL) and benzyl alcohol (1.4 mL, 13.53 mmol). The mixture was stirred under nitrogen at 90-100 °C. The mixture filtered through Celite^{®}, and the filtrate concentrated. EtOAc was added, the mixture sonicated and filtered to afford the product as a white solid. 900mg. 4-benzyloxy-6-fluoro-1-(4-fluorophenyl)indole (1.8 g, 77%). ¹H NMR (400 MHz, DMSO-*d*₆) δ 7.65 - 7.58 (m, 2H), 7.55 - 7.49 (m, 3H), 7.46 - 7.32 (m, 5H), 6.85 (ddd, J = 10.0, 2.0, 0.8 Hz, 1H), 6.73 - 6.67 (m, 2H), 5.29 (s, 2H).

### Preparation of S18

### 4-benzyloxy-1-(4-fluorophenyl)indole (S18)

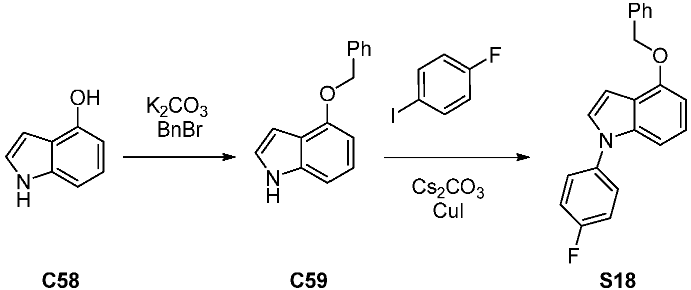

### Step 1. Synthesis of 4-benzyloxy-1H-indole (C59)

To a mixture of 1H-indol-4-ol **C58** (1 g, 7.51 mmol) and K₂CO₃ (2 g, 14.5mmol) in Acetone (10 mL) was added benzyl bromide (1 mL, 8.41 mmol) and refluxed overnight. The mixture was diluted with dichloromethane, filtered though a layer of Celite^{®}, and concentrated. Purification by silica gel chromatography (Gradient: 0-25% EtOAc in heptane) afforded the product 4-benzyloxy-1H-indole (1.4 g, 67%). LCMS *m*/*z* 224.0 [M+H]⁺.

### Step 2. Synthesis of 4-benzyloxy-1-(4-fluorophenyl)indole

A mixture of 4-benzyloxy-1H-indole **C59** (10 g, 44.8 mmol), 1-fluoro-4-iodo-benzene (6.5 mL, 56.4 mmol), CuI (500 mg, 2.63 mmol) and cesium carbonate (25 g, 76.7 mmol) in DMF (50 mL) was bubbled with nitrogen and stirred at 120 °C for 48 hours. The reaction mixture was diluted with water (500 mL) and EtOAc (200 mL). The organic layer was separated and the aqueous layer was extracted with EtOAc (2x 100 mL). The combined organic layers were washed with brine, dried over Na₂SO₄ and concentrated under reduced pressure. The resulting solid was triturated with ether (100 mL), filtered. The solid was washed with ether (25 mL) and dried under high vacuum to afford the product 4-benzyloxy-1-(4-fluorophenyl)indole (10.5 g, 71%) as grey colored solid. ¹H NMR (400 MHz, DMSO-*d*₆) δ 7.66 - 7.58 (m, 2H), 7.55 - 7.50 (m, 4H), 7.45 - 7.37 (m, 5H), 7.36 - 7.27 (m, 1H), 7.09 (d, J = 6.0 Hz, 2H), 6.73 (q, J = 2.7, 2.2 Hz, 2H), 5.28 (s, 2H). LCMS *m*/*z* 318.12 [M+H]⁺.

### Preparation of S19

### [2-[4-benzyloxy-1-(4-fluorophenyl)-3-iodo-indol-2-yl]-2-methyl-propoxy]-tert-butyl-dimethylsilane (S19)

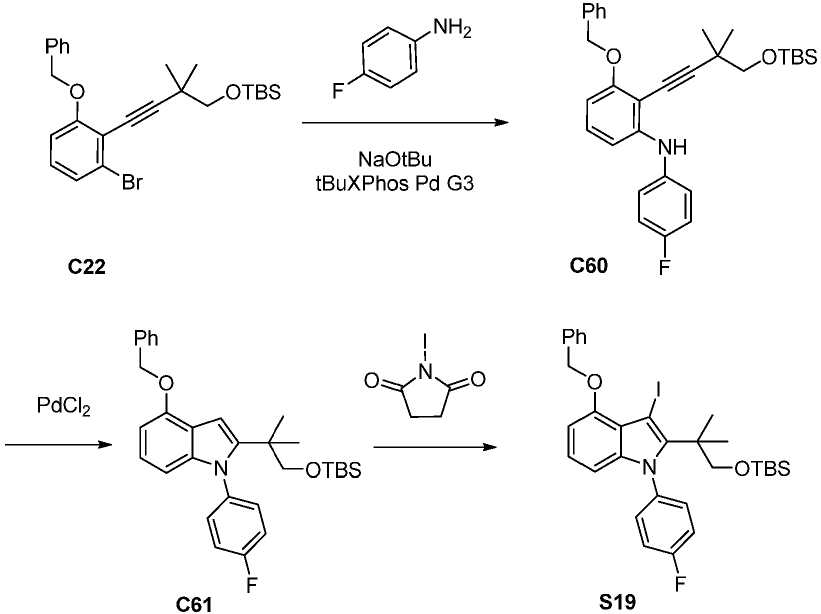

### Step 1. Synthesis of 3-benzyloxy-2-[4-[tert-butyl(dimethyl)silyl]oxy-3,3-dimethyl-but-1-ynyl]-N-(4-fluorophenyl)aniline (C60)

A solution of [4-(2-benzyloxy-6-bromo-phenyl)-2,2-dimethyl-but-3-ynoxy]-tert-butyl-dimethyl-silane **C22** (40.3 g, 85.1 mmol) and 4-fluoroaniline (12.1 mL, 127.7 mmol) in m-xylene (400 mL) was purged with nitrogen for 10 minutes. NaOtBu (24.5 g, 254.9 mmol) and tBuXPhos Pd G3 (2.03 g, 2.56 mmol) were then added in one portion and the reaction mixture was stirred at 35 °C for 4 hours, then filtered over Celite^{®}. The filtered solids were rinsed with xylene and the filtrate was concentrated. The filtered solids were washed with 1:1 EtOAc and water, and the organic layer of the filtrate was combined and concentrated with the xylene filtrate to give a dark brown oil. Purification by silica gel chromatography (Gradient: 0-20% EtOAc in heptane) afforded the product as a light yellow oil. 3-benzyloxy-2-[4-[tert-butyl(dimethyl)silyl]oxy-3,3-dimethyl-but-1-ynyl]-N-(4-fluorophenyl)aniline (40.3 g, 94%). ¹H NMR (400 MHz, Chloroform-d) δ 7.53 (ddt, J = 7.4, 1.3, 0.7 Hz, 2H), 7.41 - 7.35 (m, 2H), 7.34 - 7.28 (m, 1H), 7.18 - 7.13 (m, 2H), 7.06 - 6.99 (m, 3H), 6.63 (dd, J = 8.3, 0.8 Hz, 1H), 6.42 (s, 1H), 6.39 (dd, J = 8.3, 0.8 Hz, 1H), 5.14 (s, 2H), 3.57 (s, 2H), 1.32 (s, 6H), 0.87 (s, 9H), 0.03 (s, 6H). LCMS *m*/*z* 504.0 [M+H]⁺.

### Step 2. Synthesis of [2-[4-benzyloxy-1-(4-fluorophenyl)indol-2-yl]-2-methyl-propoxy]-tert-butyldimethyl-silane (C61)

To a solution of 3-benzyloxy-2-[4-[tert-butyl(dimethyl)silyl]oxy-3,3-dimethyl-but-1-ynyl]-N-(4-fluorophenyl)aniline **C60** (40.3 g, 80.0 mmol) in MeCN (400 mL) was added PdCl₂ (567 mg, 3.2 mmol). The reaction mixture was stirred at 60 °C overnight, then filtered. The filtrate was concentrated to dryness, triturated with MeCN, and filtered again. The process was repeated 3-4 times and all solids were combined, and dried under vacuum to afford the product as a tan solid. [2-[4-benzyloxy-1-(4-fluorophenyl)indol-2-yl]-2-methyl-propoxy]-tert-butyldimethyl-silane (38.1 g, 95%). ¹H NMR (400 MHz, Chloroform-*d*) δ 7.60 - 7.55 (m, 2H), 7.48 - 7.43 (m, 2H), 7.42 - 7.35 (m, 3H), 7.25 - 7.18 (m, 2H), 6.98 (t, J = 8.0 Hz, 1H), 6.69 (d, J = 0.8 Hz, 1H), 6.64 - 6.59 (m, 1H), 6.32 (dt, J = 8.3, 0.7 Hz, 1H), 5.28 (s, 2H), 3.54 (s, 2H), 1.24 (s, 6H), 0.88 (s, 9H), 0.00 (s, 6H). LCMS *m*/*z* 504.0 [M+H]⁺.

### Step 3. Synthesis of [2-[4-benzyloxy-1-(4-fluorophenyl)-3-iodo-indol-2-yl]-2-methyl-propoxy]-tert-butyl-dimethyl-silane (S19)

To a solution of [2-[4-benzyloxy-1-(4-fluorophenyl)indol-2-yl]-2-methyl-propoxy]-tert-butyl-dimethyl-silane **C61** (500 mg, 0.99 mmol) in dichloromethane (6 mL) was added N-iodosuccinimide (240 mg, 1.07 mmol). The reaction was stirred at room temperature for 10 minutes then washed with water. The organic layer was concentrated to dryness and purified via silica gel chromatography (Gradient: 0-20% EtOAc in heptane) to afford the product. [2-[4-benzyloxy-1-(4-fluorophenyl)-3-iodo-indol-2-yl]-2-methyl-propoxy]-tert-butyl-dimethyl-silane (390 mg, 62%). ¹H NMR (400 MHz, Chloroform-d) δ 7.65 - 7.59 (m, 2H), 7.43 - 7.38 (m, 2H), 7.35 - 7.26 (m, 2H), 7.18 - 7.11 (m, 2H), 6.95 - 6.89 (m, 1H), 6.60 (dd, J = 7.8, 0.8 Hz, 1H), 6.24 (ddd, J = 8.3, 2.3, 0.8 Hz, 1H), 5.24 (d, J = 2.4 Hz, 2H), 4.01 (s, 2H), 1.27 (dd, J = 2.7, 1.5 Hz, 6H), 0.84 (d, J = 3.0 Hz, 9H), -0.00 (s, 6H). LCMS *m*/*z* 450.58 [M+H]⁺.

### Preparation of S20

### 4-benzyloxy-1-(4-fluorophenyl)-3-iodo-2-(2-methoxy-1,1-dimethyl-ethyl)indole (S20)

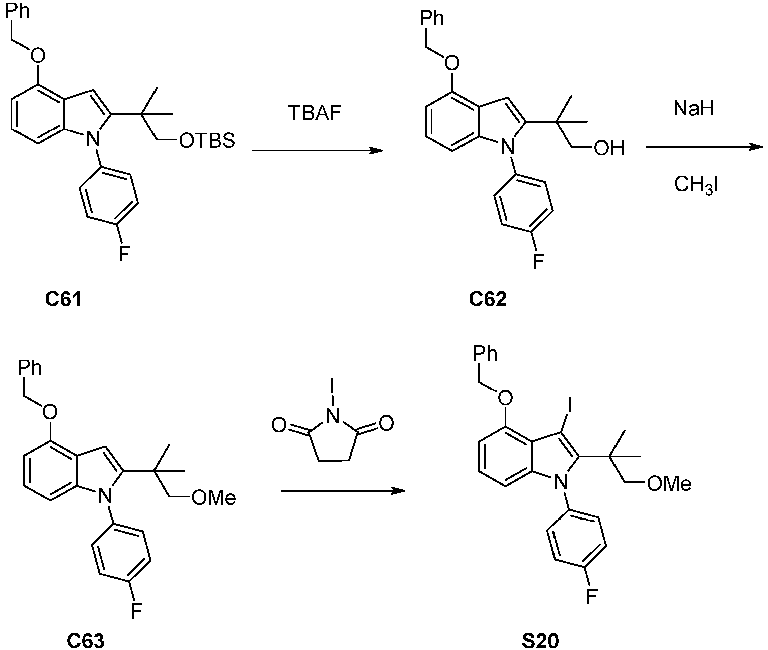

### Step 1. Synthesis of 2-[4-benzyloxy-1-(4-fluorophenyl)indol-2-yl]-2-methyl-propan-1-ol (C62)

To a solution of [2-[4-benzyloxy-1-(4-fluorophenyl)indol-2-yl]-2-methyl-propoxy]-tert-butyl-dimethyl-silane **C62** (4.8 g, 9.53 mmol) in THF (40 mL) was added TBAF (40 mL of 1 M, 40.0 mmol). The mixture was stirred for 4 hours at 55 °C then concentrated, and purified by silica gel chromatography (Gradient: 0-50% EtOAc in heptane) to afford the product 2-[4-benzyloxy-1-(4-fluorophenyl)indol-2-yl]-2-methyl-propan-1-ol (3.15 g, 85%) as an off white solid. ¹H NMR (400 MHz, Chloroform-d) δ 7.51 - 7.17 (m, 7H), 7.08 (q, J = 8.3, 7.9 Hz, 2H), 6.88 (t, J = 7.9 Hz, 1H), 6.62 (s, 1H), 6.50 (d, J = 7.8 Hz, 1H), 6.21 (d, J = 8.3 Hz, 1H), 5.13 (s, 2H), 3.35 (s, 2H), 1.12 (s, 6H). LCMS *m*/*z* 390.0 [M+H]⁺.

### Step 2. Synthesis of 4-benzyloxy-1-(4-fluorophenyl)-2-(2-methoxy-1,1-dimethyl-ethyl)indole

*(**C63**)To* a mixture of 2-[4-benzyloxy-1-(4-fluorophenyl)indol-2-yl]-2-methyl-propan-1-ol **C62** (2.07 g, 5.32 mmol) and MeI (520 µL, 8.35 mmol) in THF (30 mL) at 0 °C was added NaH (260 mg of 60% w/w, 6.50 mmol) . The mixture was stirred for 2 hours at room temperature. A few drops of ice water and HCl were added, and the mixture concentrated. The residue was dissolved in dichloromethane, filtered and concentrated to give the product. 4-benzyloxy-1-(4-fluorophenyl)-2-(2-methoxy-1,1-dimethyl-ethyl)indole (2.25 g, 97%). ¹H NMR (400 MHz, Chloroform-d) δ 7.60 - 7.52 (m, 2H), 7.48 - 7.34 (m, 5H), 7.26 - 7.18 (m, 2H), 6.96 (t, J = 8.0 Hz, 1H), 6.59 (dd, J = 7.8, 0.7 Hz, 1H), 6.30 (dt, J = 8.2, 0.7 Hz, 1H), 5.25 (s, 2H), 3.25 (s, 3H), 3.21 (s, 2H), 1.30 (s, 6H). LCMS *m*/*z* 403.33 [M+H]⁺.

### Step 3. Synthesis of 4-benzyloxy-1-(4-fluorophenyl)-3-iodo-2-(2-methoxy-1,1-dimethylethyl)indole (S20)

To a solution of 4-benzyloxy-1-(4-fluorophenyl)-2-(2-methoxy-1,1-dimethylethyl)indole **C63** (2.25 g, 5.55 mmol) in dichloromethane (30 mL) was added N-iodosuccinimide (1.5 g, 6.68 mmol) and stirred for 1 hour at room temperature. The solvent was removed and the product purified by silica gel chromatography (Gradient: 0-25% EtOAc in Hexanes) afforded the product as a light yellow solid. 4-benzyloxy-1-(4-fluorophenyl)-3-iodo-2-(2-methoxy-1,1-dimethyl-ethyl)indole (2.9 g, 97%). ¹H NMR (400 MHz, Chloroform-d) δ 7.70 - 7.59 (m, 2H), 7.43 (ddd, J = 7.7, 6.5, 1.5 Hz, 2H), 7.39 - 7.30 (m, 3H), 7.18 (ddt, J = 8.6, 6.4, 1.8 Hz, 2H), 6.95 (td, J = 8.1, 6.2 Hz, 1H), 6.63 (dd, J = 7.9, 4.7 Hz, 1H), 6.26 (dd, J = 8.3, 2.7 Hz, 1H), 5.26 (d, J = 2.8 Hz, 2H), 3.75 (s, 2H), 3.27 (d, J = 2.4 Hz, 3H), 1.38 (s, 6H). LCMS *m*/*z* 529.47 [M+H]⁺.

### Preparation of S21

### [3-[4-benzyloxy-1-(4-fluorophenyl)-3-iodo-indol-2-yl]-3-methyl-butoxy]-tert-butyl-dimethylsilane (S21)

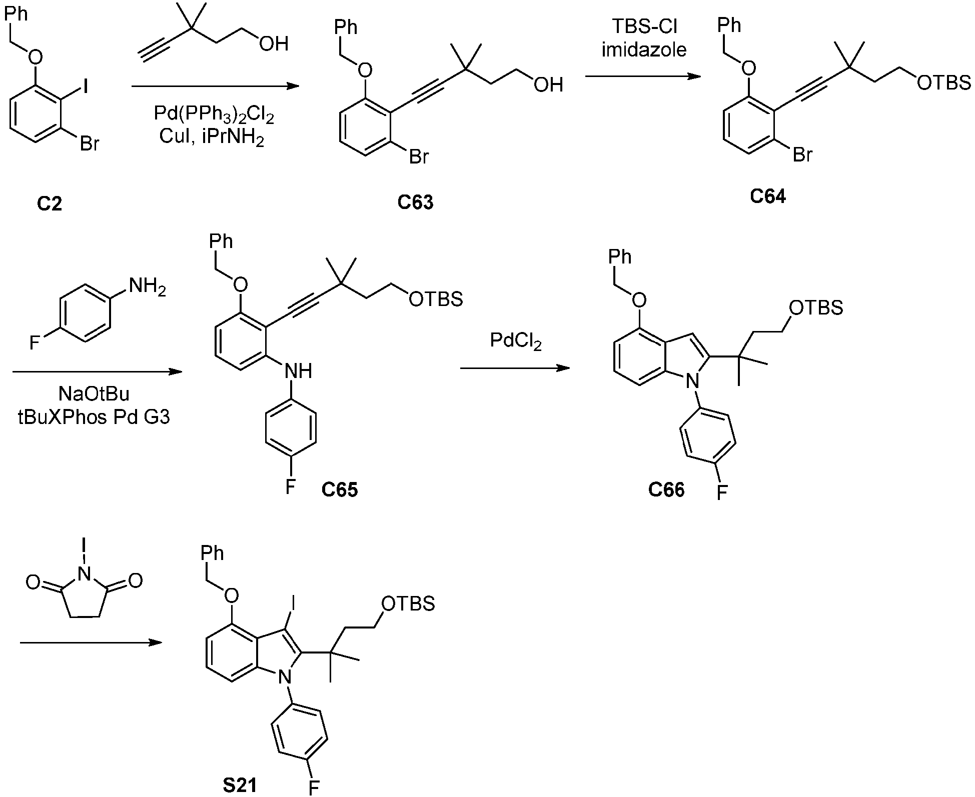

### Step 1. Synthesis of 5-(2-benzyloxy-6-bromo-phenyl)-3, 3-dimethyl-pent-4-yn-1-ol (C63)

A solution of 1-benzyloxy-3-bromo-2-iodo-benzene **C2** (60 g, 154.2 mmol), 3,3-dimethylpent-4-yn-1-ol (23 g, 205.0 mmol) and N-isopropylpropan-2-amine (140 mL, 998.9 mmol) in 1,4-dioxane (400 mL) was purged with nitrogen for 10 minutes, then CuI (1.38 g, 7.25 mmol) and Pd(PPh₃)₂Cl₂ (4.65 g, 6.63 mmol) were added. The reaction mixture was stirred at 50 °C for 4 hours, then cooled to room temperature and filtered to remove a light tan solid. The filtrate was concentrated to dryness then partitioned between water and EtOAc. The mixture was filtered over Celite^{®} to aid separation of the layers. The organic layer was concentrated to dryness and purified via silica gel chromatography (Gradient: 0-50% EtOAc in heptane) afforded the product as an orange oil. 5-(2-benzyloxy-6-bromo-phenyl)-3,3-dimethyl-pent-4-yn-1-ol (47 g, 82%). ¹H NMR (400 MHz, Chloroform-*d*) δ 7.52 - 7.48 (m, 2H), 7.44 - 7.39 (m, 2H), 7.38 - 7.32 (m, 1H), 7.20 (dd, J = 8.1, 1.0 Hz, 1H), 7.07 (t, J = 8.2 Hz, 1H), 6.85 (dd, J = 8.4, 0.9 Hz, 1H), 5.15 (s, 2H), 3.89 (q, J = 6.1 Hz, 2H), 2.23 (t, J = 5.9 Hz, 1H), 1.82 (t, J = 6.3 Hz, 2H), 1.39 (s, 6H). LCMS *m*/*z* 373.0 [M+H]⁺.

### Step 2. Synthesis of [5-(2-benzyloxy-6-bromo-phenyl)-3,3-dimethyl-pent-4-ynoxy]-tert-butyldimethyl-silane (C64)

To a solution of 5-(2-benzyloxy-6-bromo-phenyl)-3,3-dimethyl-pent-4-yn-1-ol **C63** (47 g, 125.9 mmol) in dichloromethane (500 mL) was added TBS-Cl (19.9 g, 132.0 mmol) and imidazole (9.0 g, 132.2 mmol). The reaction mixture was stirred at room temperature over the weekend. A tan precipitate was removed by filtration and the filtrate was washed with water (2x). The organic layer was dried over magnesium sulfate, filtered, and concentrated to afford the product as light yellow oil. [5-(2-benzyloxy-6-bromo-phenyl)-3,3-dimethyl-pent-4-ynoxy]-tert-butyl-dimethyl-silane (59.3 g, 97%). ¹H NMR (400 MHz, Chloroform-d) δ 7.50 (ddq, J = 6.8, 1.5, 0.7 Hz, 2H), 7.41 - 7.36 (m, 2H), 7.35 - 7.30 (m, 1H), 7.19 (dd, J = 8.1, 1.0 Hz, 1H), 7.05 (t, J = 8.2 Hz, 1H), 6.84 (dd, J = 8.3, 1.0 Hz, 1H), 5.13 (s, 2H), 3.98 - 3.90 (m, 2H), 1.85 - 1.77 (m, 2H), 1.36 (s, 6H), 0.89 (s, 9H), 0.05 (s, 6H). LCMS *m*/*z* 487.0 [M+H]⁺.

### Step 3. Synthesis of 3-benzyloxy-2-[5-[tert-butyl(dimethyl)silyl]oxy-3,3-dimethyl-pent-1-ynyl]-N-(4-fluorophenyl)aniline (C65)

A solution of [5-(2-benzyloxy-6-bromo-phenyl)-3,3-dimethyl-pent-4-ynoxy]-tert-butyl-dimethyl-silane **C64** (59.3 g, 121.7 mmol) and 4-fluoroaniline (17.3 mL, 182.6 mmol) in m-xylene (500 mL) was degassed with nitrogen for 10 minutes and then NaOtBu (35.1 g, 365.2 mmol) and tBuXPhos Pd G3 (2.9 g, 3.65 mmol) were added in one portion. The reaction mixture was stirred at 35 °C for 1 hour, and then filtered over Celite^{®}. The filter pad was washed with 1:1 EtOAc / water, and then the organic layer of the filtrate was combined with the xylene and concentrated to dryness. The resulting brown oil was purified via silica gel chromatography (Gradient: 0-25% EtOAc in heptane) to afford the desired product as an amber oil. 3-benzyloxy-2-[5-[tert-butyl(dimethyl)silyl]oxy-3,3-dimethyl-pent-1-ynyl]-N-(4-fluorophenyl)aniline (56.12 g, 89%). ¹H NMR (400 MHz, Chloroform-d) δ 7.52 (ddq, J = 7.0, 1.5, 0.8 Hz, 2H), 7.42 - 7.37 (m, 2H), 7.34 - 7.29 (m, 1H), 7.19 - 7.14 (m, 2H), 7.07 - 7.00 (m, 3H), 6.68 (dd, J = 8.3, 0.8 Hz, 1H), 6.40 (dd, J = 8.3, 0.8 Hz, 1H), 6.38 (s, 1H), 5.15 (s, 2H), 3.94 - 3.86 (m, 2H), 1.85 - 1.77 (m, 2H), 1.38 (s, 6H), 0.86 (s, 9H), 0.00 (s, 6H). LCMS *m*/*z* 518.0 [M+H]⁺.

### Step 4. Synthesis of [3-[4-benzyloxy-1-(4-fluorophenyl)indol-2-yl]-3-methyl-butoxy]-tert-butyldimethyl-silane (C66)

To a solution of 3-benzyloxy-2-[5-[tert-butyl(dimethyl)silyl]oxy-3,3-dimethyl-pent-1-ynyl]-N-(4-fluorophenyl)aniline **C65** (56.1 g, 108.4 mmol) in MeCN (500 mL) was added PdCl₂ (965 mg, 5.44 mmol). The reaction mixture was stirred at 65 °C overnight, then cooled to room temperature and filtered. The filtrate was concentrated to dryness, triturated with MeCN, and filtered again. The solids were combined and rinsed with cold MeCN, then dried under vacuum to afford the product as a white solid. [3-[4-benzyloxy-1-(4-fluorophenyl)indol-2-yl]-3-methyl-butoxy]-tert-butyl-dimethyl-silane (48.65 g, 87%). ¹H NMR (400 MHz, Chloroform-d) δ 7.54 (ddt, J = 7.5, 1.4, 0.7 Hz, 2H), 7.45 - 7.39 (m, 2H), 7.35 (tdd, J = 5.8, 3.9, 2.6 Hz, 3H), 7.21 - 7.14 (m, 2H), 6.94 (t, J = 8.0 Hz, 1H), 6.61 - 6.56 (m, 2H), 6.27 (dt, J = 8.2, 0.7 Hz, 1H), 5.24 (s, 2H), 3.57 - 3.49 (m, 2H), 1.76 - 1.66 (m, 2H), 1.27 (s, 6H), 0.83 (s, 9H), -0.04 (s, 6H). LCMS *m*/*z* 518.0 [M+H]⁺.

### Step 5. Synthesis of [3-[4-benzyloxy-1-(4-fluorophenyl)-3-iodo-indol-2-yl]-3-methyl-butoxy]-tert-butyl-dimethyl-silane (S21)

To a solution of [3-[4-benzyloxy-1-(4-fluorophenyl)indol-2-yl]-3-methyl-butoxy]-tert-butyl-dimethyl-silane **C66** (5 g, 9.65 mmol) in dichloromethane (50 mL) at 0 °C was added N-iodosuccinimide (2.3 g, 10.2 mmol). The reaction mixture was stirred at 0 °C for 30 minutes then washed with water. The organic layer was concentrated to dryness, dry loaded onto loose Celite^{®}, and purified via silica gel chromatography (Gradient: 0-20% EtOAc in heptane) to afford the product as a white solid. [3-[4-benzyloxy-1-(4-fluorophenyl)-3-iodo-indol-2-yl]-3-methyl-butoxy]-tert-butyl-dimethyl-silane (5.85 g, 94%). ¹H NMR (400 MHz, Chloroform-d) δ 7.65 - 7.60 (m, 2H), 7.40 (tt, J = 6.7, 0.9 Hz, 2H), 7.35 - 7.30 (m, 1H), 7.27 - 7.22 (m, 2H), 7.19 - 7.12 (m, 2H), 6.95 - 6.89 (m, 1H), 6.61 (ddd, J = 7.9, 4.1, 0.8 Hz, 1H), 6.21 (ddd, J = 8.3, 3.3, 0.7 Hz, 1H), 5.24 (d, J = 2.7 Hz, 2H), 3.61 - 3.54 (m, 2H), 2.31 (t, J = 7.2 Hz, 2H), 1.33 (d, J = 12.6 Hz, 6H), 0.84 (d, J = 2.2 Hz, 9H), -0.01 (d, J = 4.1 Hz, 6H). LCMS *m*/*z* 643.0 [M+H]⁺.

### Preparation of S22

### 4-benzyloxy-6-fluoro-1-(4-fluorophenyl)-3-iodo-2-(2-methoxy-1,1-dimethyl-ethyl)indole (S22)

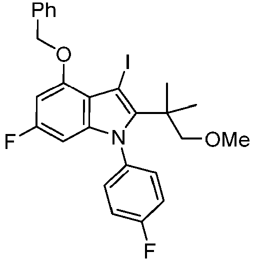

Compound **S22** was prepared in three steps from **C18** and 4-fluoroaniline using the methods used in the preparation of compounds **S4.** Purification by silica gel chromatography (Gradient: 0-10% EtOAc in Hexanes) afforded the product as a white solid. 4-benzyloxy-6-fluoro-1-(4-fluorophenyl)-3-iodo-2-(2-methoxy-1,1-dimethyl-ethyl)indole (402.8 mg, 97%). ¹H NMR (400 MHz, Chloroform-d) δ 7.64 (ddt, J = 7.5, 1.4, 0.7 Hz, 2H), 7.48 - 7.40 (m, 2H), 7.40 - 7.33 (m, 1H), 7.30 - 7.25 (m, 2H), 7.24 - 7.12 (m, 2H), 6.41 (dd, J = 11.5, 2.2 Hz, 1H), 5.92 (dd, J = 9.4, 2.1 Hz, 1H), 5.22 (s, 2H), 3.70 (s, 2H), 3.27 (s, 3H), 1.36 (s, 6H). LCMS *m*/*z* 547.37 [M+H]⁺.

### B. Synthesis of Compounds 1-457

All the specific and generic compounds, and the intermediates disclosed for making those compounds, are considered to be part of the disclosure disclosed herein.

### Compound 1

### 3-cyano-4-[1-(3,4-difluorophenyl)-4-hydroxy-2-tetrahydropyran-4-yl-indol-3-yl]benzoic acid (1)

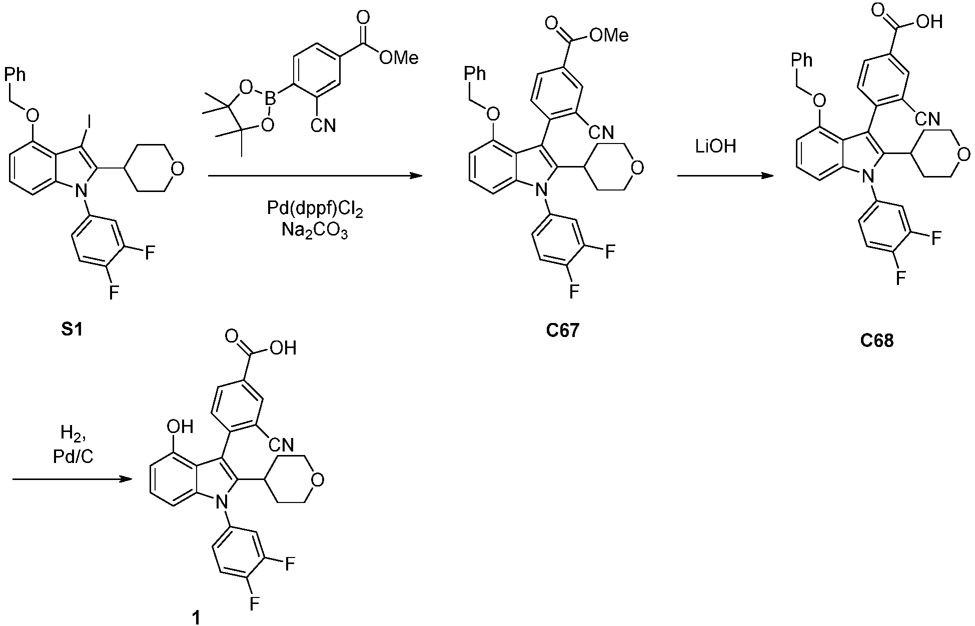

### Step 1. Synthesis of methyl 4-[4-benzyloxy-1-(3,4-difluorophenyl)-2-tetrahydropyran-4-yl-indol-3-yl]-3-cyano-benzoate (C67)

A mixture of 4-benzyloxy-1-(3,4-difluorophenyl)-3-iodo-2-tetrahydropyran-4-yl-indole **S1** (157 mg, 0.263 mmol) in dimethyl formamide (2 mL), methyl 3-cyano-4-(4,4,5,5-tetramethyl-1,3,2-dioxaborolan-2-yl)benzoate (90 mg, 0.31 mmol), sodium carbonate (400 µL of 2 M, 0.8 mmol), and Pd(dppf)Cl₂ (24 mg, 0.03 mmol) was heated in a microwave at 100 °C for 1 hour. The mixture was diluted with water and extracted with EtOAc (2x). The combined organics were washed with water (2x), brine, dried over sodium sulfate, and concentrated under reduced pressure. Purification by silica gel chromatography (Gradient: 0-40% EtOAc in heptane) afforded the product. Methyl 4-[4-benzyloxy-1-(3,4-difluorophenyl)-2-tetrahydropyran-4-yl-indol-3-yl]-3-cyano-benzoate (15 mg, 10%). ¹H NMR (400 MHz, DMSO-*d*₆) δ 8.18 - 8.11 (m, 1H), 8.07 (dd, J = 9.8, 1.9 Hz, 1H), 7.87 - 7.79 (m, 1H), 7.72 (dd, J = 11.1, 8.0 Hz, 2H), 7.40 (d, J = 9.0 Hz, 1H), 7.21 (t, J = 7.4 Hz, 1H), 7.17 - 7.04 (m, 3H), 6.85 (d, J = 7.9 Hz, 2H), 6.71 (d, J = 7.9 Hz, 1H), 6.58 (dd, J = 8.2, 4.9 Hz, 1H), 4.89 (q, J = 11.5 Hz, 2H), 3.95 (s, 3H), 3.66 (t, J = 10.5 Hz, 2H), 3.06 (d, J = 10.4 Hz, 2H), 2.81 (d, J = 14.4 Hz, 1H), 1.68 (d, J = 14.3 Hz, 1H), 1.50 (d, J = 25.1 Hz, 3H). LCMS *m*/*z* 579.55 [M+H]⁺.

### Step 2. Synthesis of 4-[4-benzyloxy-1-(3, 4-difluorophenyl)-2-tetrahydropyran-4-yl-indol-3-yl]-3-cyano-benzoic acid (C68)

LiOH (500 µL of 1 M, 0.5 mmol) was added to a solution of methyl 4-[4-benzyloxy-1-(3,4-difluorophenyl)-2-tetrahydropyran-4-yl-indol-3-yl]-3-cyano-benzoate **C67** (15 mg, 0.03 mmol) in tetrahydrofuran (1.5 mL) / methanol (1.5 mL) and the reaction was warmed to 50 °C for 90 minutes. The mixture was neutralized by the addition of 10% citric acid, and extracted with EtOAc (2x). The combined organics were washed with brine, dried over sodium sulfate, and concentrated under reduced pressure to afford the product. 4-[4-benzyloxy-1-(3,4-difluorophenyl)-2-tetrahydropyran-4-yl-indol-3-yl]-3-cyano-benzoic acid (14 mg, 91%). LCMS *m*/*z* 565.06 [M+H]⁺.

### Step 3. Synthesis of 3-cyano-4-[1-(3,4-difluorophenyl)-4-hydroxy-2-tetrahydropyran-4-yl-indol-3-yl]benzoic acid (1)

To a solution of 4-[4-benzyloxy-1-(3,4-difluorophenyl)-2-tetrahydropyran-4-yl-indol-3-yl]-3-cyano-benzoic acid **C68** (14 mg, 0.024 mmol) in ethanol (2 mL) was added a mixture of palladium on carbon catalyst (2 mg, 0.02 mmol) in ethanol (300 µL). The reaction mixture was placed under a hydrogen atmosphere (balloon pressure) and allowed to stir overnight. The reaction mixture was filtered and purified by reversed-phase chromatography (Column: C18. Gradient: 5-100% MeCN in water with 0.1% trifluoroacetic acid). The product was concentrated and azeotroped with CH₃CN to give desired product as an off-white solid. 3-cyano-4-[1-(3,4-difluorophenyl)-4-hydroxy-2-tetrahydropyran-4-yl-indol-3-yl]benzoic acid (2.0 mg, 17%). ¹H NMR (400 MHz, Chloroform-d) δ 8.49 (s, 1H), 8.34 (d, J = 8.1 Hz, 1H), 7.75 (d, J = 8.1 Hz, 1H), 7.47 - 7.32 (m, 2H), 7.23 (d, J = 6.5 Hz, 1H), 7.02 (t, J = 8.0 Hz, 1H), 6.53 (d, J = 8.1 Hz, 1H), 6.47 (d, J = 7.7 Hz, 1H), 3.90 (t, J = 11.4 Hz, 2H), 3.23 (s, 2H), 2.84 (s, 1H), 1.75 (s, 2H), 1.66 (s, 2H). LCMS *m*/*z* 475.32 [M+H]⁺.

### Compound 2

### 4-[1-(3,4-difluorophenyl)-4-hydroxy-2-tetrahydropyran-4-yl-indol-3-yl]-2-fluoro-benzoic acid (2)

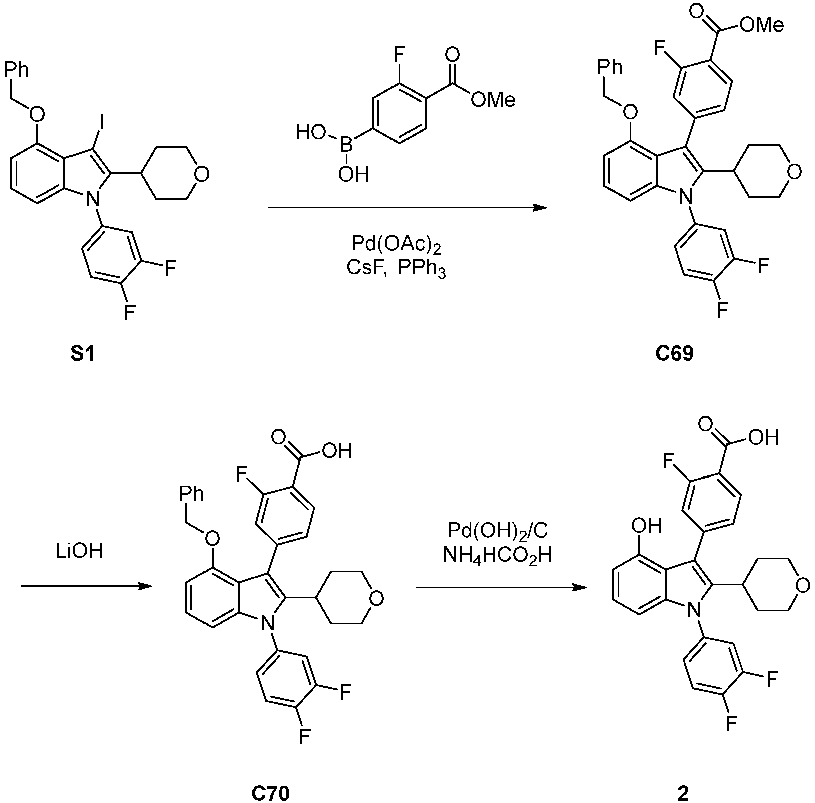

### Step 1. Synthesis of methyl 4-[4-benzyloxy-1-(3,4-difluorophenyl)-2-tetrahydropyran-4-yl-indol-3-yl]-2-fluoro-benzoate (C69)

A suspension of 4-benzyloxy-1-(3,4-difluorophenyl)-3-iodo-2-tetrahydropyran-4-yl-indole **S1** (130 g, 238.4 mmol), (3-fluoro-4-methoxycarbonyl-phenyl)boronic acid (93.9 g, 474.3 mmol), PPh₃ (12.6 g, 48.0 mmol) and CsF (163 g, 1.1 mol) in dimethoxyethane (3.64 L) in a 12 L round bottom flask was purged with nitrogen gas (via gas dispersion tube) for 15 minutes. Pd(OAc)₂ (5.2 g, 23.1 mmol) was added, and nitrogen gas was bubbled through for an additional 15 minutes. The mixture was heated gradually to 80 °C, under a positive pressure of nitrogen. After 45 minutes, internal temp reached 80 °C, and maintained this temperature for 2 hours. The mixture was cooled to room temperature, then partitioned between EtOAc (4 L) and water (4 L). The organic layer was separated, washed with saturated aqueous NaHCO₃ (3 L), followed by brine (3 L), dried (MgSO₄), filtered and concentrated. The residue was dissolved in dichloromethane (250 mL) and purified by silica gel chromatography (Combiflash (3 kg silica gel). Gradient: 0-40% EtOAc in heptane. 900 mL/min flow rate). The product was concentrated and the residue was dissolved in EtOAc (1.0 L, at 72 °C). The resulting solution was treated with heptane (4 L), then spun on rotovap (no vacuum, ice-water bath) for 90 minutes. The resulting crystals were collected via filtration, washing with heptane (2 L) and dried under suction for 1 hour. The resulting product (118 g pale yellow fluffy crystals) was dissolved in hot EtOAc (1.0 L, at 70 °C), then treated with Biotage MP-TMT resin (52 g) was added. The mixture was heated at 70 °C for 1 hour. The mixture was filtered, and the resin was washed with EtOAc (80 mL). The filtrate was treated with heptane (4 L) and allowed to stand at room temperature for 16 hours. The resulting crystals were isolated by filtration, washed with heptane (1 L) then dried under suction for 1 hour to afford the product methyl 4-[4-benzyloxy-1-(3,4-difluorophenyl)-2-tetrahydropyran-4-yl-indol-3-yl]-2-fluoro-benzoate (114 g, 84%) as white crystals. ¹H NMR (400 MHz, Chloroform-d) δ 7.85 (t, J = 7.9 Hz, 1H), 7.41 (dt, J = 9.8, 8.6 Hz, 1H), 7.34 - 7.26 (m, 2H), 7.26 - 7.16 (m, 5H), 7.14 - 7.07 (m, 1H), 6.96 - 6.89 (m, 2H), 6.63 (dd, J = 7.9, 0.7 Hz, 1H), 6.55 (dd, J = 8.3, 0.7 Hz, 1H), 4.95 (s, 2H), 4.01 (s, 3H), 3.84 (dd, J = 12.0, 4.2 Hz, 2H), 3.19 (tdd, J = 11.8, 3.7, 2.1 Hz, 2H), 2.84 (tt, J = 12.3, 3.5 Hz, 1H), 1.71 (qd, J = 12.4, 4.2 Hz, 2H), 1.60 - 1.52 (m, 2H). ¹H NMR shows presence of residual EtOAc. ¹⁹F NMR (376 MHz, Chloroform-d) δ -110.98, -133.71 (d, J = 21.5 Hz), -135.49 (d, J = 21.7 Hz). LCMS *m*/*z* 572.04 [M+H]⁺.

### Step 2. 4-[4-benzyloxy-1-(3,4-difluorophenyl)-2-tetrahydropyran-4-yl-indol-3-yl]-2-fluorobenzoic acid (C70)

To a solution of methyl 4-[4-benzyloxy-1-(3,4-difluorophenyl)-2-tetrahydropyran-4-yl-indol-3-yl]-2-fluoro-benzoate **C69** (4.25 g, 7.45 mmol) in THF (170 mL) at room temperature was added LiOH (3.5 g, 146.1 mmol), MeOH (35 mL), water (35 mL). The mixture was heated to 50 °C, then quenched with 1 M aq HCl (170 mL), and extracted with 2-MeTHF (200 mL). The combined organic extracts were washed with brine (200 mL), dried over MgSO₄, then filtered and concentrated to afford the product 4-[4-benzyloxy-1-(3,4-difluorophenyl)-2-tetrahydropyran-4-yl-indol-3-yl]-2-fluoro-benzoic acid (4.1 g, 99%) as a white solid. ¹H NMR (300 MHz, Chloroform-d) δ 7.93 (t, J = 7.9 Hz, 1H), 7.41 (dt, J = 9.9, 8.6 Hz, 1H), 7.34 - 7.16 (m, 7H), 7.11 (t, J = 8.0 Hz, 1H), 6.98 - 6.89 (m, 2H), 6.64 (d, J = 7.8 Hz, 1H), 6.56 (dd, J = 8.3, 0.6 Hz, 1H), 4.94 (s, 2H), 3.89 (dd, J = 11.6, 4.0 Hz, 2H), 3.21 (t, J = 11.8 Hz, 2H), 2.85 (ddd, J = 12.2, 8.7, 3.5 Hz, 1H), 1.76 (qd, J = 12.6, 4.2 Hz, 2H), 1.59 (d, J = 12.9 Hz, 2H). LCMS *m*/*z* 557.95 [M+H]⁺.

### Step 3. Synthesis of 4-[1-(3,4-difluorophenyl)-4-hydroxy-2-tetrahydropyran-4-yl-indol-3-yl]-2-fluoro-benzoic acid (2)

A 5 L 3-neck RBF equipped with mechanical stirrer, heating jacket, temp probe and condenser, was charged with a solution of 4-[4-benzyloxy-1-(3,4-difluorophenyl)-2-tetrahydropyran-4-yl-indol-3-yl]-2-fluoro-benzoic acid **C70** (89 g, 159.6 mmol) in THF (1.6 L) and MeOH (1.3 L). Ammonium formate (121 g, 1.92 mol) was added and the reaction vessel was purged with nitrogen for 5 minutes. 20% Pd(OH)₂ on carbon (3.33 g of 20% w/w, 4.7 mmol) was added and the reaction mixture was heated to 58 °C for 2 hours. The mixture was filtered through a pad of Celite^{®}, and then washed with 1:1 THF:MeOH (1 L) to give a pale yellow filtrate. The filtrate was concentrated and the residue was concentrated from MeOH (2 x 500 mL) to remove residual ammonium formate. The residue was slurried in 1:1 MeOH:water (1 L) for 16 hours. The mixture was filtered, washing with 1:1 MeOH:water (100 mL), then dried under suction for 20 minutes. The solid was dissolved in 1:1 MeOH:THF (2 L), then treated with Biotage MP-TMT resin (20 g) and activated charcoal (10 g), heated at 55 °C for 45 minutes, then filtered. The filtrate was concentrated, and the residue was treated with MeOH (500 mL), spun on rotary evaporator at 65 °C (no vacuum) for 45 minutes, then concentrated. The MeOH (500 mL) treatment was repeated and the resulting yellow solid was dried in vacuum oven at 55 °C for 20 hours to afford the product 4-[1-(3,4-difluorophenyl)-4-hydroxy-2-tetrahydropyran-4-yl-indol-3-yl]-2-fluoro-benzoic acid (50 g, 66%) as a pale yellow solid. ¹H NMR (300 MHz, DMSO-*d*₆) δ 9.31 (s, 1H), 7.86 - 7.59 (m, 3H), 7.38 (ddt, J = 8.6, 3.9, 1.7 Hz, 1H), 7.28 - 7.03 (m, 2H), 6.86 (t, J = 7.9 Hz, 1H), 6.42 (dd, J = 7.7, 0.8 Hz, 1H), 6.27 (dd, J = 8.1, 0.8 Hz, 1H), 3.70 (d, J = 11.3 Hz, 2H), 3.18 - 2.94 (m, 2H), 2.81 (dq, J = 10.0, 5.6 Hz, 1H), 1.71 - 1.40 (m, 4H). ¹⁹F NMR (376 MHz, DMSO-*d*₆) δ -114.56, -135.67 (d, J = 23.1 Hz), - 137.46 (d, J = 22.8 Hz).¹³C NMR (101 MHz, DMSO-*d*₆) δ 167.16, 161.03, 158.53, 151.92, 151.33, 151.24, 151.11, 148.87, 148.76, 148.63, 140.74, 139.90, 138.61, 135.06, 130.21, 127.38, 127.22, 123.44, 119.81, 119.63, 119.51, 119.28, 118.86, 118.68, 116.28, 113.72, 105.55, 101.64, 67.88, 34.96, 32.79, 32.72. LCMS *m*/*z* 468.23 [M+H]⁺. XRPD shows material is crystalline, DSC shows melting point = 303 °C.

### Compound 3

### 4-[1-(3,4-difluorophenyl)-4-hydroxy-2-tetrahydropyran-4-yl-indol-3-yl]-3-fluoro-benzoic acid (3)

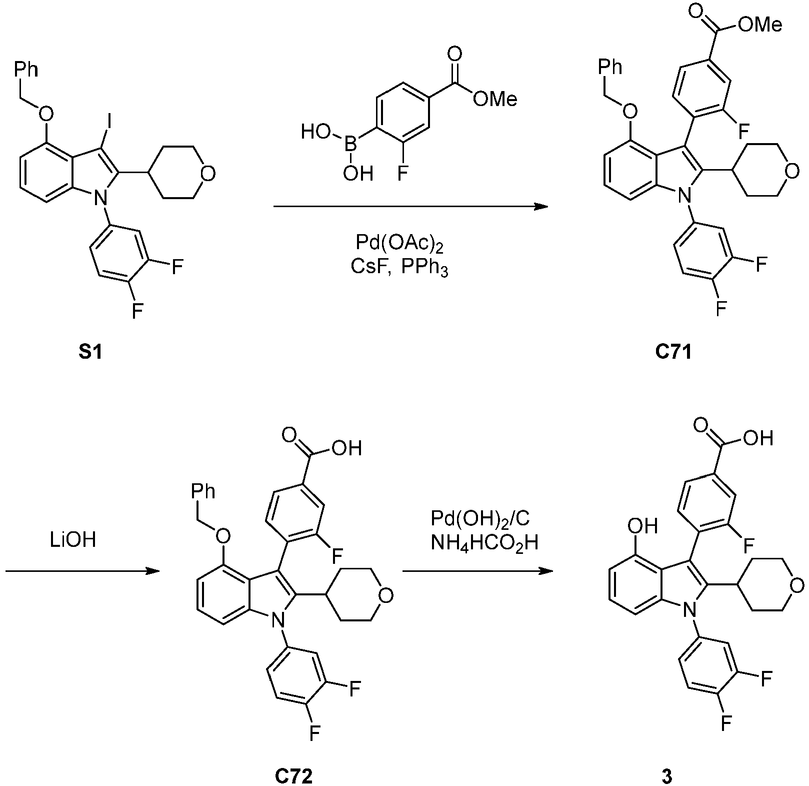

### Step 1. Synthesis of methyl 4-[4-benzyloxy-1-(3,4-difluorophenyl)-2-tetrahydropyran-4-yl-indol-3-yl]-3-fluoro-benzoate (C71)

A suspension of 4-benzyloxy-1-(3,4-difluorophenyl)-3-iodo-2-tetrahydropyran-4-yl-indole **S1** (137 g, 251.2 mmol), (2-fluoro-4-methoxycarbonyl-phenyl)boronic acid (100 g, 505.2 mmol), triphenyl phosphine (13.2 g, 50.3 mmol) and CsF (172 g, 1.13 mol) in DME (3.84 L) was bubbled through with nitrogen (via gas dispersion tube) for 15 minutes. Pd(OAc)₂ (5.5 g, 24.4 mmol) was added, and N₂ bubbling was continued for 15 minutes, then heating was commenced (target temp = 80 °C), while a positive pressure of N₂ was maintained. After 45 minutes, internal temp has reached 80 °C, and this was maintained for 1 hour. EtOAc (4 L) and water (4 L) were added. The organic layer was separated, washed with saturated aqueous NaHCO₃ (3 L) followed by brine (3 L), dried (MgSO₄), filtered and concentrated. The residue was dissolved in dichloromethane (400 mL) and purified by silica gel chromatography (3 kg silica gel. Gradient: 0-40% EtOAc in heptane. 900 mL/min flow rate.) The product solution (in chromatography solvent) was allowed to stand at room temperature for 1 week. The resulting crystals were filtered off. The filtrate was concentrated and combined with the crystals. The entire mixture was suspended in EtOAc (1 L, 60 °C), and heptane (4 L) was added. The mixture was cooled to 0 °C (ice-water bath), held at 0 °C for 30 minutes, then filtered. The collected solid was washed with heptane (2 L), and dried under suction. The collected crystals (~114 g) were dissolved in EtOAc (1 L) and THF (500 mL) at 75 °C (rotovap bath). The resulting solution was treated with Biotage MP-TMT resin (53 g) and heated at 75 °C for 45 minutes, then filtered while hot, washing with THF (200 mL). The combined filtrate was concentrated and the residue was dissolved/suspended in hot EtOAc (500 mL), heated at 75 °C for 30 minutes, then treated with heptane (1.5 L). Upon standing at room temperature overnight, the mixture was filtered, washing with heptane (200 mL). The product was dried under suction for 30 minutes, then on rotovap (1 mbar, 65 °C) for 45 minutes to afford the product methyl 4-[4-benzyloxy-1-(3,4-difluorophenyl)-2-tetrahydropyran-4-yl-indol-3-yl]-3-fluoro-benzoate (103.4 g, 72%) as a white powder. ¹H NMR (300 MHz, Chloroform-*d*) δ 7.77 (dd, J = 7.9, 1.7 Hz, 1H), 7.62 (dd, J = 9.6, 1.7 Hz, 1H), 7.53 - 7.12 (m, 7H), 7.08 (t, J = 8.0 Hz, 1H), 6.95 - 6.85 (m, 2H), 6.61 (d, J = 7.8 Hz, 1H), 6.56 (d, J = 8.2 Hz, 1H), 4.92 (s, 2H), 3.99 (s, 3H), 3.83 (dd, J = 11.0, 5.2 Hz, 2H), 3.27 - 3.10 (m, 2H), 2.80 (t, J = 12.0 Hz, 1H), 1.87 - 1.48 (m, 4H). ¹⁹F NMR (282 MHz, Chloroform-*d*) δ -111.00, -111.10, -133.79 (d, J = 21.5 Hz), -133.87 (d, J = 21.5 Hz), -135.70 (d, J = 21.5 Hz), -135.71 (d, J = 21.5 Hz). LCMS *m*/*z* 571.44 [M+H]⁺. Melting point = 182 °C.

### Step 2. Synthesis of 4-[4-benzyloxy-1-(3, 4-difluorophenyl)-2-tetrahydropyran-4-yl-indol-3-yl]-3-fluoro-benzoic acid (C72)

THF (1.9 L) and MeOH (400 mL) was added to methyl 4-[4-benzyloxy-1-(3,4-difluorophenyl)-2-tetrahydropyran-4-yl-indol-3-yl]-3-fluoro-benzoate **C71** (103 g, 180.2 mmol) in a 5 L 3-neck flask equipped with heating jacket, mechanical stirrer and a temp probe. LiOH (43 g, 1.8 mol) and H₂O (400 mL) were added at room temperature. The mixture was heated to 50 °C for 2 hours. The mixture was then cooled to 22 °C (ice-water bath), then neutralized with 2 M aq HCl (1 L). The temperature rose to 35 °C. The mixture was partitioned between 2-MeTHF and brine (500 mL each).The organic layer was washed with brine (1 L), dried (MgSO₄), filtered and concentrated. The aqueous layer was re-extracted with 2-MeTHF (1 L), and the organic extract was washed with brine (1 L), dried (MgSO₄), filtered, and concentrated to afford the product 4-[4-benzyloxy-1-(3,4-difluorophenyl)-2-tetrahydropyran-4-yl-indol-3-yl]-3-fluoro-benzoic acid (101 g, 101%) as a white solid. ¹H NMR (300 MHz, DMSO-*d*₆) δ 13.22 (s, 1H), 7.94 - 7.64 (m, 3H), 7.65 - 7.51 (m, 2H), 7.42 (dddd, J = 18.0, 9.0, 4.1, 2.0 Hz, 1H), 7.24 - 7.09 (m, 3H), 7.05 (t, J = 8.1 Hz, 1H), 6.83 - 6.75 (m, 2H), 6.68 (d, J = 7.9 Hz, 1H), 6.55 (dd, J = 8.2, 2.2 Hz, 1H), 5.02 - 4.84 (m, 2H), 3.68 (dt, J = 11.1, 5.0 Hz, 2H), 3.14 - 2.97 (m, 2H), 2.75 (td, J = 10.2, 8.4, 6.0 Hz, 1H), 1.74 - 1.29 (m, 4H). ¹⁹F NMR (282 MHz, DMSO-*d*₆) δ -110.76, - 110.89, -135.40 (d, J = 22.9 Hz), -135.44 (d, J = 22.9 Hz), -137.26 (d, J = 22.9 Hz), -137.31 (d, J = 22.9 Hz). LCMS *m*/*z* 557.16 [M+H]⁺.

### Step 3. Synthesis of 4-[1-(3,4-difluorophenyl)-4-hydroxy-2-tetrahydropyran-4-yl-indol-3-yl]-3-fluoro-benzoic acid (3)

In a 500 mL flask, 4-[1-(3,4-difluorophenyl)-4-hydroxy-2-tetrahydropyran-4-yl-indol-3-yl]-3-fluoro-benzoic acid **C72** (40.3 g, 84.7 mmol) was suspended in AcOH (80 mL) and water (160 mL). The flask was wrapped in foil to block ambient light and stirred at room temperature for 20 hours. The reaction mixture was filtered, and the solid collected, washed with water (100 mL), dried under suction for 45 minutes, then on a rotovap (75 °C, at 2 mbar pressure) for 2 hours to afford the product 4-[1-(3,4-difluorophenyl)-4-hydroxy-2-tetrahydropyran-4-yl-indol-3-yl]-3-fluoro-benzoic acid (39.5 g, 100%) as a slightly off-white solid. ¹H NMR (300 MHz, DMSO-*d*₆) δ 13.23 (s, 1H), 9.16 (s, 1H), 7.92 - 7.62 (m, 4H), 7.55 (q, J = 7.5 Hz, 1H), 7.48 - 7.29 (m, 1H), 6.87 (t, J = 7.9 Hz, 1H), 6.47 - 6.25 (m, 2H), 3.68 (p, J = 4.6 Hz, 2H), 3.05 (tt, J = 11.7, 8.4, 3.6 Hz, 2H), 2.75 (tt, J = 12.4, 3.6 Hz, 1H), 1.74 - 1.29 (m, 4H). ¹⁹F NMR (282 MHz, DMSO-*d*₆) δ -110.64, -110.79, -135.53 (d, J = 23.0 Hz), -135.57 (d, J = 23.0 Hz), -137.45 (d, J = 23.0 Hz), -137.50 (d, J = 23.0 Hz). LCMS *m*/*z* 468.17 [M+H]⁺. Melting point = 296 °C (DSC).

### Compound 4

### 4-[1-(3,4-difluorophenyl)-4-hydroxy-2-tetrahydropyran-4-yl-indol-3-yl]benzoic acid (4)

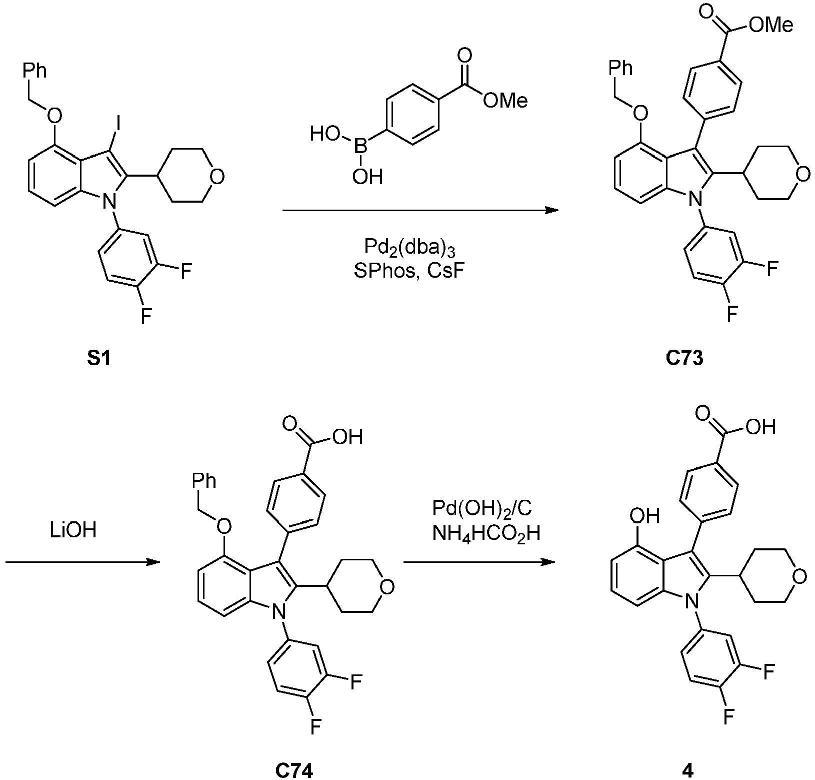

### Step 1. Synthesis of methyl 4-[4-benzyloxy-1-(3,4-difluorophenyl)-2-tetrahydropyran-4-yl-indol-3-yl]benzoate (C73)

A 2 L 5 neck flask fitted with a mechanical stirrer, a heating mantle, a J-Kem temperature probe, a water cooled reflux condenser and a nitrogen inlet/outlet was charged 4-benzyloxy-1-(3,4-difluorophenyl)-3-iodo-2-tetrahydropyran-4-yl-indole **S1** (80 g, 144.6 mmol), (4-methoxycarbonylphenyl)boronic acid (35 g, 194.5 mmol), CsF (105 g, 691.2 mmol) and degassed DME (1.6 L). The solution was bubbled through with nitrogen for 5 minutes and then Pd₂(dba)₃ (6.4 g, 6.99 mmol) and dicyclohexyl-[2-(2,6-dimethoxyphenyl)phenyl]phosphane (5.6 g, 13.64 mmol) were added. The resulting reaction mixture was bubbled through with nitrogen for 20 minutes , warmed to 80 °C, and stirred at this temperature for 8 hours.

The reaction mixture was cooled to room temperature, partitioned between dichloromethane (400 mL), EtOAc (800 mL) and water (400 mL). The organic phase was separated and washed successively with water (400 mL), sat. aqueous NaHCO₃ solution (400 mL) and brine (2 x 200 mL). The organic phase was then dried over Na₂SO₄, filtered and concentrated under reduced pressure. The residue was dissolved/suspended in MTBE (~500 mL), refluxed for 30 seconds and the resulting suspension was allowed to stand for 2 hours at room temperature. The mixture was filtered and the collected solid was washed with MTBE (~100 mL), dried under suction to afford ~60 g white crystals which contained ~5% deiodination starting material and also ~7% boroxine. A 5 L 3 neck flask was fitted with a mechanical stirrer, a heating mantle, a J-Kem temperature probe, a water cooled reflux condenser was charged with ~60 g white crystals, suspended in EtOAc (800 mL) then heated to reflux. Additional EtOAc was added portionwise until a clear solution was attained (total amount of EtOAc required = ~1.6 L). The mixture was refluxed for 30 minutes, then heat was switched off, flask was left in heating mantle to cool down slowly. After approximately 14 hours, crystals were filtered, and dried under suction to afford the product 62 g which contained ~6 wt% boroxine. This material when recrystallized an additional time using above conditions to afford the product methyl 4-[4-benzyloxy-1-(3,4-difluorophenyl)-2-tetrahydropyran-4-yl-indol-3-yl]benzoate (40 g, 50%) as a white crystalline solid. ¹H NMR (300 MHz, DMSO-*d*₆) δ 8.01 - 7.89 (m, 2H), 7.88 - 7.75 (m, 1H), 7.75 - 7.63 (m, 1H), 7.54 (d, J = 7.9 Hz, 2H), 7.42 (ddd, J = 9.6, 4.2, 2.2 Hz, 1H), 7.24 - 7.13 (m, 1H), 7.13 - 6.97 (m, 3H), 6.81 - 6.71 (m, 2H), 6.67 (d, J = 7.8 Hz, 1H), 6.51 (d, J = 8.2 Hz, 1H), 4.92 (s, 2H), 3.92 (s, 3H), 3.66 (d, J = 10.7 Hz, 2H), 3.03 (td, J = 11.7, 3.5 Hz, 2H), 2.85 - 2.67 (m, 1H), 1.53 (q, J = 13.0, 12.3 Hz, 4H).¹⁹F NMR (282 MHz, DMSO-*d*₆) δ -135.48, - 135.56, -137.29, -137.37. LCMS *m*/*z* 554.07 [M+H]⁺. Melting point = 201 °C.

### Step 2. Synthesis of 4-[4-benzyloxy-1-(3, 4-difluorophenyl)-2-tetrahydropyran-4-yl-indol-3-yl]benzoic acid (C74)

A 5 L 3 neck flask fitted with overhead stirrer, a heating mantle, a J-Kem temperature probe, and a water cooled reflux condenser was charged with methyl 4-[4-benzyloxy-1-(3,4-difluorophenyl)-2-tetrahydropyran-4-yl-indol-3-yl]benzoate **C73** (52 g, 93.9 mmol) in a mixture of THF (1.5 L) and methanol (220 mL). LiOH (15 g, 626.3 mmol) in water (450 mL) was added and the resulting reaction mixture was warmed to 50 °C. The reaction was stirred at this temperature for 14 hours. The mixture was then cooled to 0 °C with ice/water bath, acidified with 2 *N* HCl to pH = 1, and a white suspension was obtained. The resulting reaction mixture was concentrated under reduced pressure to remove THF and methanol (~1.7 L). The white precipitate formed was filtered through a medium fritted funnel, washed with water (2 x 500 mL), dried in a convection oven at 80 °C for 14 hours to afford 4-[4-benzyloxy-1-(3,4-difluorophenyl)-2-tetrahydropyran-4-yl-indol-3-yl]benzoic acid (49 g, 95%) as a white sold which contained traces of ethyl acetate and THF. This material was taken into the next step without further purification. ¹H NMR (300 MHz, DMSO-*d*₆) δ 12.92 (s, 1H), 7.99 - 7.89 (m, 2H), 7.87 - 7.75 (m, 1H), 7.70 (dd, J = 10.6, 8.9 Hz, 1H), 7.52 (d, J = 7.9 Hz, 2H), 7.46 - 7.36 (m, 1H), 7.20 - 6.98 (m, 4H), 6.76 - 6.64 (m, 3H), 6.51 (d, J = 8.2 Hz, 1H), 4.94 (s, 2H), 3.65 (t, J = 9.1 Hz, 2H), 3.03 (dd, J = 13.4, 10.1 Hz, 2H), 2.85 - 2.68 (m, 1H), 1.53 (q, J = 12.8, 12.2 Hz, 4H). ¹⁹F NMR (282 MHz, DMSO-*d*₆) δ -135.49, -135.57, -137.31, -137.39. Melting point is 272 °C. LCMS *m*/*z* 540.12 [M+H]⁺.

### Step 3. Synthesis of 4-[1-(3,4-difluorophenyl)-4-hydroxy-2-tetrahydropyran-4-yl-indol-3-yl]benzoic acid (4)

In a 250 mL flask, 4-[4-benzyloxy-1-(3,4-difluorophenyl)-2-tetrahydropyran-4-yl-indol-3-yl]benzoic acid **C74** (2.4 g, 4.45 mmol) was dissolved in THF (60 mL) and EtOH (40 mL). Pd on C, wet, Degussa (565 mg of 5% w/w, 0.27 mmol) was added, then reaction mixture was placed under vacuum for 5 minutes, then placed under H₂ (balloon pressure) for 1 hour. The reaction mixture was bubbled through with N₂ for 5 minutes, then transferred to a 500 mL flask. Ammonium formate (3.4 g, 53.9 mmol) was added, and the resulting mixture was refluxed for 2.5 hours. Pd(OH)₂ (628 mg of 20% w/w, 0.9 mmol) was added and the reaction mixture was refluxed for an additional 1 hour. The reaction mixture was filtered through Celite^{®} while hot, washing with EtOH (30 mL). The filtrate was concentrated and the residue was concentrated from EtOH (80 mL) twice to remove excess ammonium formate. The residue was dissolved in 1:1 EtOH:2-MeTHF (100 mL), treated with Biotage MP-TMT resin (2.4 g, washed with hot EtOH) and heated at 75 °C for 45 minutes. The mixture was filtered while hot, and the resin was washed with EtOH (30 mL) filtrate was concentrated. The residue was re-concentrated from EtOH (50 mL) and the resulting residue was treated with EtOH (50 mL), and heated to 75 °C for 20 minutes. Water (20 mL) was added, the mixture was sonicated for 2 minutes, and then heated at 75 °C for 5 minutes. The resulting suspension was allowed to stand at room temperature for 90 minutes. The mixture was filtered and the collected solid was washed with water (10 mL), then dried under suction for 30 minutes, then transferred to a 100 mL flask and dried on rotovap (2 mbar, 70 °C) for 1 hour to afford the product 4-[1-(3,4-difluorophenyl)-4-hydroxy-2-tetrahydropyran-4-yl-indol-3-yl]benzoic acid (1.46 g, 72%) as an off-white crystalline solid. ¹H NMR (400 MHz, DMSO-*d*₆) δ 12.89 (s, 1H), 9.17 (s, 1H), 7.96 (d, J = 8.4 Hz, 2H), 7.79 (ddd, J = 11.1, 7.2, 2.5 Hz, 1H), 7.70 (dt, J = 10.5, 8.9 Hz, 1H), 7.57 - 7.47 (m, 2H), 7.39 (dq, J = 8.9, 2.6, 2.2 Hz, 1H), 6.86 (t, J = 7.9 Hz, 1H), 6.40 (dd, J = 7.7, 0.8 Hz, 1H), 6.29 (dd, J = 8.2, 0.8 Hz, 1H), 3.68 (dt, J = 11.3, 2.9 Hz, 2H), 3.04 (tdd, J = 11.2, 5.4, 2.6 Hz, 2H), 2.81 (tt, J = 11.6, 4.1 Hz, 1H), 1.64 - 1.43 (m, 4H). ¹³C NMR (101 MHz, DMSO-*d*₆) δ 167.54, 151.40, 151.17 - 150.46 (m), 148.69 - 147.89 (m), 141.57, 139.51, 138.18, 134.56 (d, J = 4.6 Hz), 131.59, 128.52, 128.06, 126.95, 123.00, 119.28 (d, J = 17.7 Hz), 118.32 (d, J = 18.0 Hz), 115.90, 113.88, 105.02, 101.31, 67.43, 34.52, 32.38, 32.31. ¹⁹F NMR (376 MHz, DMSO-*d*₆) δ -135.65 (d, J = 23.0 Hz), -137.44 (d, J = 22.8 Hz). LCMS *m*/*z* 450.22 [M+H]⁺.

### Compound 5

### 4-[6-amino-1-(3,4-difluorophenyl)-4-hydroxy-2-tetrahydropyran-4-yl-indol-3-yl]benzoic acid (5)

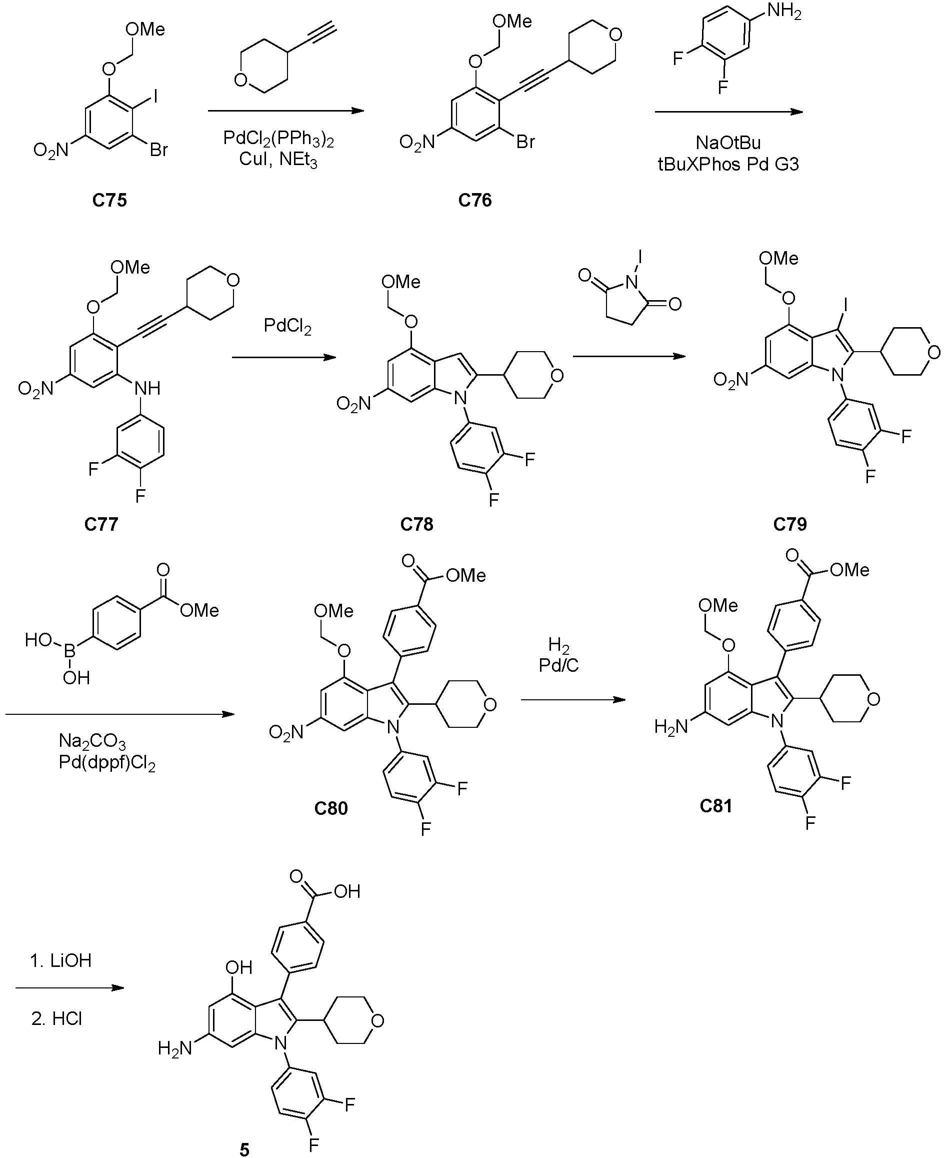

### Step 1. Synthesis of 4-[2-[2-bromo-6-(methoxymethoxy)-4-nitro-phenyl]ethynyl]-tetrahydropyran (C76)

In a 5 mL microwave tube, under nitrogen, 1-bromo-2-iodo-3-(methoxy-methoxy)-5-nitro-benzene **C75** (1.54 g, 3.81 mmol) and 4-ethynyltetrahydropyran (440 mg, 3.99 mmol) were combined in 1,4-dioxane (2.1 mL) and then Pd(PPh₃)₂Cl₂ (275 mg, 0.4 mmol), CuI (85 mg, 0.45 mmol) and NEt₃ (2.1 mL) were added. The mixture was purged with nitrogen, and the reaction was heated at 60 °C overnight. Upon cooling to room temperature, the mixture was diluted with EtOAc and washed successively with water (x 2), brine, dried over sodium sulfate, and concentrated under reduced pressure. Purification by silica gel chromatography (Gradient: 0-35% EtOAc in heptane) afforded the product 4-[2-[2-bromo-6-(methoxymethoxy)-4-nitrophenyl]ethynyl]tetrahydropyran (860 mg, 59%). ¹H NMR (400 MHz, DMSO-*d*₆) δ 8.15 (d, J = 2.2 Hz, 1H), 7.95 (d, J = 2.1 Hz, 1H), 5.43 (s, 2H), 3.89 - 3.78 (m, 2H), 3.57 - 3.48 (m, 2H), 3.44 (s, 3H), 3.16 - 3.04 (m, 1H), 1.96 - 1.81 (m, 2H), 1.73 - 1.53 (m, 2H). LCMS *m*/*z* 370.03 [M+H]⁺.

### Step 2. Synthesis of N-(3, 4-difluorophenyl)-3-(methoxymethoxy)-5-nitro-2-(2-tetrahydropyran-4-ylethynyl)aniline (C76) and 1-(3,4-difluorophenyl)-4-(methoxymethoxy)-6-nitro-2-tetrahydropyran-4-yl-indole(C77)

To a solution of 4-[2-[2-bromo-6-(methoxymethoxy)-4-nitrophenyl]ethynyl]tetrahydropyran (857 mg, 2.24 mmol) and 3,4-difluoroaniline (240 µL, 2.42 mmol) in m-xylene (11 mL) was added NaOtBu (645 mg, 6.71 mmol) followed by tBuXPhos Pd G3 (70 mg, 0.09 mmol). The reaction mixture was stirred at room temperature overnight. The mixture was diluted with water and extracted with EtOAc (2x). The combined organics were concentrated to dryness and purified by silica gel chromatography (Gradient: 0-30% EtOAc in heptane) to afford the desired product N-(3,4-difluorophenyl)-3-(methoxymethoxy)-5-nitro-2-(2-tetrahydropyran-4-ylethynyl)aniline (mixed with ~10% of the cyclized indole product 1-(3,4-difluorophenyl)-4-(methoxymethoxy)-6-nitro-2-tetrahydropyran-4-yl-indole). The N-(3,4-difluorophenyl)-3-(methoxymethoxy)-5-nitro-2-(2-tetrahydropyran-4-ylethynyl)aniline product mixture was dissolved in acetonitrile (10 mL) and PdCl₂ (40 mg, 0.23 mmol) was added. The mixture was warmed to 50 °C and allowed to stir for 3 hours. The mixture was diluted with EtOAc, washed with water, brine, dried over sodium sulfate, and then concentrated under reduced pressure to afford the product. 1-(3,4-difluorophenyl)-4-(methoxymethoxy)-6-nitro-2-tetrahydropyran-4-yl-indole (576 mg, 60%). ¹H NMR (400 MHz, DMSO-*d*₆) δ 7.92 - 7.83 (m, 1H), 7.80 - 7.69 (m, 1H), 7.62 (d, J = 1.8 Hz, 1H), 7.54 (dd, J = 1.9, 0.8 Hz, 1H), 7.51 - 7.44 (m, 1H), 6.72 (d, J = 0.7 Hz, 1H), 5.47 (s, 2H), 3.84 (d, J = 8.5 Hz, 2H), 3.47 (s, 3H), 3.33 - 3.19 (m, 2H), 2.93 - 2.79 (m, 1H), 1.78 - 1.62 (m, 4H). LCMS *m*/*z* 419.16 [M+H]⁺.

### Step 3. Synthesis of 1-(3, 4-difluorophenyl)-3-iodo-4-(methoxymethoxy)-6-nitro-2-tetrahydropyran-4-yl-indole (C79)

To an ice-cold solution of 1-(3,4-difluorophenyl)-4-(methoxymethoxy)-6-nitro-2-tetrahydropyran-4-yl-indole **C78** (575 mg, 1.34 mmol) in dichloromethane (10 mL) was added 1-iodopyrrolidine-2,5-dione (325 mg, 1.45 mmol) and allowed to stir for 1 hour. The reaction was by the addition of 1N sodium thiosulfate, passed through a phase separator, and concentrated to give the product as a yellow solid. 1-(3,4-difluorophenyl)-3-iodo-4-(methoxymethoxy)-6-nitro-2-tetrahydropyran-4-yl-indole (700 mg, 93%). ¹H NMR (400 MHz, DMSO-*d*₆) δ 7.92 - 7.83 (m, 1H), 7.82 - 7.70 (m, 1H), 7.57 (d, J = 1.9 Hz, 1H), 7.48 (d, J = 8.8 Hz, 1H), 7.37 (d, J = 1.9 Hz, 1H), 5.46 (s, 2H), 3.88 (dd, J = 11.3, 4.1 Hz, 2H), 3.52 (s, 3H), 3.31 - 3.17 (m, 2H), 2.99 (t, J = 12.3 Hz, 1H), 2.29 - 2.15 (m, 2H), 1.60 (t, J = 12.7 Hz, 2H). LCMS *m*/*z* 544.95 [M+H]⁺.

### Step 4. Synthesis of methyl 4-[1-(3, 4-difluorophenyl)-4-(methoxymethoxy)-6-nitro-2-tetrahydropyran-4-yl-indol-3-yl]benzoate (C80)

A mixture of 1-(3,4-difluorophenyl)-3-iodo-4-(methoxymethoxy)-6-nitro-2-tetrahydropyran-4-yl-indole **C79** (200 mg, 0.36 mmol), (4-methoxycarbonylphenyl)boronic acid (80 mg, 0.44 mmol), sodium carbonate (380 µL of 2 M, 0.76 mmol), and Pd(dppf)Cl₂ (17 mg, 0.02 mmol) in N,N-dimethylformamide (2.2 mL) was heated at 90 °C for 1 hour, then heated in a microwave at 110 °C for 30 minutes. The mixture was diluted with ethyl acetate and washed successively with water (3 x), brine, dried over sodium sulfate, and concentrated under reduced pressure. Silica gel chromatography (Gradient: 0-35% EtOAc / heptane) afforded the product. methyl 4-[1-(3,4-difluorophenyl)-4-(methoxymethoxy)-6-nitro-2-tetrahydropyran-4-yl-indol-3-yl]benzoate (87 mg, 44%). ¹H NMR (400 MHz, DMSO-*d*₆) δ 8.07 - 8.00 (m, 2H), 8.00 - 7.91 (m, 1H), 7.83 - 7.73 (m, 1H), 7.56 (dd, J = 14.9, 7.8 Hz, 3H), 7.49 (d, J = 1.9 Hz, 1H), 7.45 (d, J = 1.9 Hz, 1H), 5.05 (s, 2H), 3.90 (s, 3H), 3.67 (d, J = 11.5 Hz, 2H), 3.11 (s, 3H), 3.09 - 2.98 (m, 2H), 2.84 (t, J = 11.9 Hz, 1H), 1.65 - 1.45 (m, 4H). LCMS *m*/*z* 553.14 [M+H]⁺.

### Step 5. Synthesis of methyl 4-[6-amino-1-(3,4-difluorophenyl)-4-(methoxymethoxy)-2-tetrahydropyran-4-yl-indol-3-yl]benzoate (C81)

Palladium on carbon (45 mg of 10% w/w, 0.04 mmol), followed by methanol (8 mL) was added to methyl 4-[1-(3,4-difluorophenyl)-4-(methoxymethoxy)-6-nitro-2-tetrahydropyran-4-yl-indol-3-yl]benzoate **C80** (180 mg, 0.33 mmol) The reaction mixture was placed under a hydrogen atmosphere via balloon and allowed to stir for 3 hours. The catalyst was filtered washing with ethyl acetate and the filtrate was concentrated. The residue was purified by silica gel chromatography (Gradient: 0-50% EtOAc in heptane) to afford the product methyl 4-[6-amino-1-(3,4-difluorophenyl)-4-(methoxymethoxy)-2-tetrahydropyran-4-yl-indol-3-yl]benzoate (110 mg, 63%). ¹H NMR (400 MHz, DMSO-*d*₆) δ 7.99 - 7.92 (m, 2H), 7.79 - 7.64 (m, 2H), 7.55 - 7.48 (m, 2H), 7.40 - 7.31 (m, 1H), 6.06 (d, J = 1.7 Hz, 1H), 5.66 (d, J = 1.6 Hz, 1H), 4.88 (s, 2H), 4.77 (s, 2H), 3.88 (s, 3H), 3.66 (d, J = 11.1 Hz, 2H), 3.07 (s, 3H), 3.06 - 2.95 (m, 2H), 2.77 - 2.68 (m, 1H), 1.57 - 1.38 (m, 4H). LCMS *m*/*z* 523.22 [M+H]⁺.

### Step 6 & 7. Synthesis of 4-[6-amino-1-(3,4-difluorophenyl)-4-hydroxy-2-tetrahydropyran-4-yl-indol-3-yl]benzoic acid (5)

To a solution of methyl 4-[6-amino-1-(3,4-difluorophenyl)-4-(methoxymethoxy)-2-tetrahydropyran-4-yl-indol-3-yl]benzoate **C81** (110 mg, 0.20 mmol) in THF (8 mL) / methanol (4 mL) was added LiOH (2.0 mL of 1 M, 2.0 mmol). The mixture was warmed to 50 °C and allowed to stir for 2 hours. 1N HCl was added until the mixture attained pH~5. The mixture was extracted with EtOAc (2x), and the combined organics were washed with brine, dried over sodium sulfate, and concentrated under reduced pressure. To the resulting residue was added hydrogen chloride (2.0 mL of 4 M, 8.0 mmol) in 1,4-dioxane, and allowed to stir overnight. The mixture was then concentrated under reduced pressure. Purification by reversed-phase chromatography (Column: C18. Gradient: 0-100% MeCN in water with 0.1% trifluoroacetic acid) afforded the product. The combined product fractions were concentrated under reduced pressure, and then dissolved in acetonitrile (5 mL) and hydrogen chloride (500 µL of 6 M, 3.0 mmol). The mixture was stirred for 20 minutes, then concentrated under reduced pressure. This latter HCl step was repeated to afford the product as a tan solid 4-[6-amino-1-(3,4-difluorophenyl)-4-hydroxy-2-tetrahydropyran-4-yl-indol-3-yl]benzoic acid (Hydrochloride salt) (60 mg, 56%). ¹H NMR (400 MHz, DMSO-*d*₆) δ 12.92 (s, 1H), 9.76 (d, J = 66.7 Hz, 3H), 8.00 - 7.91 (m, 2H), 7.93 - 7.83 (m, 1H), 7.80 - 7.69 (m, 1H), 7.55 - 7.36 (m, 3H), 6.41 (s, 1H), 6.26 (s, 1H), 3.68 (d, J = 11.2 Hz, 2H), 3.05 (t, J = 8.0 Hz, 2H), 2.80 (t, J = 12.0 Hz, 1H), 1.64 - 1.43 (m, 4H). LCMS *m*/*z* 465.18 [M+H]⁺.

### Compounds 6-10

Compounds **6-10** (Table 1) were prepared from intermediates **S1-S3** and the appropriate boronic acid or boronic ester according to the methods described for the preparation of Compounds **1** and **2**. Any modifications to the procedures are noted in the table footnotes.

**Table 1. Method of preparation, structure and physicochemical data for Compounds 6-10**

| **Compound** | **Method/ Product** | **Boronic acid or ester** | ¹H NMR**; LCMS m/z** [M+H]⁺ |
|---|---|---|---|
| **6** | From **S2**^{1,2,3} | 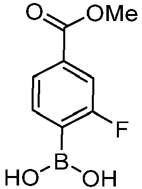 | ¹H NMR (400 MHz, DMSO-*d*₆) δ 13.34 (s, 1H), 9.28 (s, 1H), 7.92 - 7.76 (m, 2H), 7.70 (tdd, J = 9.5, 8.5, 1.9 Hz, 2H), 7.56 (dt, J = 10.8, 7.6 Hz, 1H), 7.42 (ddd, J = 21.8, 7.8, 3.6 Hz, 1H), 6.92 (dd, J = 11.2, 8.8 Hz, 1H), 6.30 (dt, J = 8.8, 3.3 Hz, 1H), 3.68 (t, J = 9.7 Hz, 2H), 3.05 (tt, J = 7.2, 4.7 Hz, 2H), 2.73 (t, J = 12.3 Hz, 1H), 1.66 - 1.48 (m, 3H), 1.43 - 1.33 (m, 1H). LCMS *m*/*z* 485.97 [M+H]⁺. |
| | Compound **2** | | |
| | 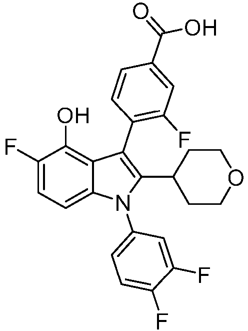 | | |
| **7** | From **S2** | 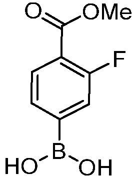 | ¹H NMR (400 MHz, DMSO-*d*₆) δ 7.88 (t, J = 8.0 Hz, 1H), 7.71 (dt, J = 10.6, 8.9 Hz, 1H), 7.40 (ddt, J = 8.4, 3.9, 1.8 Hz, 1H), 7.37 - 7.23 (m, 2H), 6.94 (dd, J = 11.2, 8.9 Hz, 1H), 6.26 (dd, J = 8.9, 3.4 Hz, 1H), 3.76 - 3.66 (m, 2H), 3.06 (tdd, J = 11.6, 5.6, 2.2 Hz, 2H), 2.81 (tt, J = 12.1, 3.7 Hz, 1H), 1.69 - 1.41 (m, 4H). LCMS *m*/*z* 486.15 [M+H]⁺. |
| | See footnotes^{4,2,3} | | |
| | 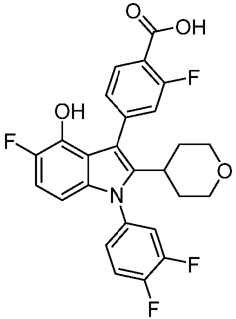 | | |
| **8** | From **S2**^{1,2,3} | 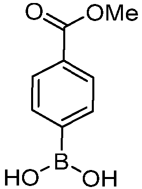 | ¹H NMR (400 MHz, Chloroform-*d*) δ 8.11 (dq, J = 8.3, 1.7 Hz, 2H), 7.60 - 7.52 (m, 2H), 7.40 (dt, J = 9.6, 8.6 Hz, 1H), 7.27 (ddd, J = 10.0, 6.9, 2.5 Hz, 1H), 7.19 (ddd, J = 8.4, 3.4, 1.9 Hz, 1H), 6.93 - 6.81 (m, 1H), 6.32 - 6.24 (m, 1H), 3.87 - 3.77 (m, 2H), 3.18 (ddd, J = 11.8, 9.5, 2.9 Hz, 2H), 2.94 - 2.81 (m, 1H), 1.71 (qd, J = 12.4, 4.2 Hz, 2H). LCMS *m*/*z* 468.02 [M+H]⁺. |
| | Compound **2** | | |
| | 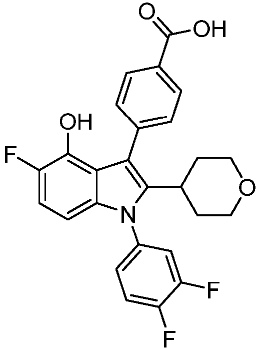 | | |
| **9** | From **S3**^{5,2,3} | 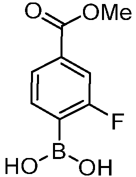 | ¹H NMR (400 MHz, DMSO-*d*₆) δ 13.26 (s, 1H), 9.73 (d, J = 1.7 Hz, 1H), 7.96 - 7.63 (m, 4H), 7.54 (dt, J = 10.6, 7.7 Hz, 1H), 7.47 - 7.31 (m, 1H), 6.22 (dd, J = 11.4, 2.2 Hz, 1H), 6.12 (ddd, J = 9.6, 3.8, 2.1 Hz, 1H), 3.77 - 3.57 (m, 2H), 3.13 - 2.94 (m, 2H), 2.71 (t, J = 12.2 Hz, 1H), 1.63 (d, J = 13.0 Hz, 1H), 1.52 (dd, J = 12.8, 4.2 Hz, 2H), 1.37 (qd, J = 12.5, 4.2 Hz, 1H). LCMS *m*/*z* 486.12 [M+H]⁺. |
| | 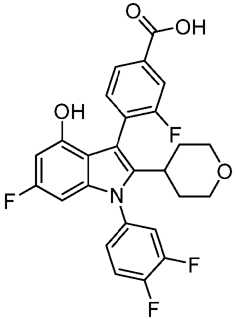 | | |
| | From **S3**^{6,2,3,7} | | ¹H NMR (400 MHz, DMSO-*d*₆) δ 13.22 (s, 1H), 9.84 (s, 1H), 7.87 (t, J = 8.0 Hz, 1H), 7.79 (ddd, J = 11.0, 7.2, 2.5 Hz, 1H), 7.71 (dt, J = 10.5, 8.9 Hz, 1H), 7.43 - 7.35 (m, 1H), 7.35 - 7.24 (m, 2H), 6.30 - 6.24 (m, 1H), 6.08 (dd, J = 9.5, 2.2 Hz, 1H), 3.69 (d, J = 11.2 Hz, 2H), 3.05 (ddd, J = 11.7, 9.7, 5.6 Hz, 2H), 2.78 (ddt, J = 12.1, 7.2, 3.6 Hz, 1H), 1.67 - 1.36 (m, 4H). LCMS *m*/*z 486.01* [M+H]⁺. |
| | Compound **1** | | |
| **10** | 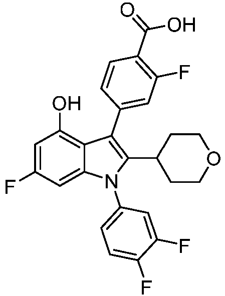 | 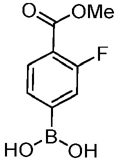 | |

| | | | |
|---|---|---|---|
| **^{1.}** Suzuki Conditions: Pd(OAc)₂, PPh₃, CsF, in DME at 100 °C. **^{2.}** Hydrolysis conditions: LiOH **^{3.}** Hydrogenation: H₂, Pd/C in EtOH **^{4.}** Suzuki Conditions: Pd(PPh₃)₄, CsF in DME at 100 °C. **^{5·}** Suzuki Conditions: Pd₂(dba)₃, SPhos, K₃PO₄ in THF at 80 °C. **^{6.}** Suzuki Conditions: Pd(dppf)Cl₂, Na₂CO₃, in DMF at 100 °C. **^{7.}** Purification by reversed-phase chromatography (Column: C18. Gradient: 0-100% MeCN in water with 0.1% formic acid) afforded the product. | | | |

### Compound 11

### 4-[1-(3, 4-difluorophenyl)-6-fluoro-4-hydroxy-2-tetrahydropyran-4-yl-indol-3-yl]benzoic acid (11)

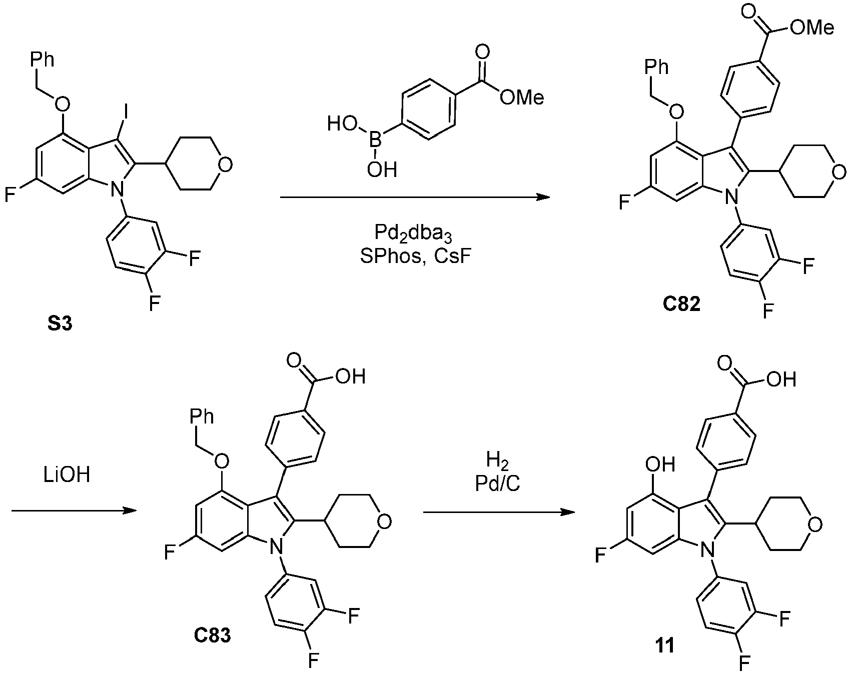

### Step 1. Synthesis of methyl 4-[4-benzyloxy-1-(3, 4-difluorophenyl)-6-fluoro-2-tetrahydropyran-4-yl-indol-3-yl]benzoate (C82)

In a 3 L 4-neck RBF equipped with mechanical stirrer, heating jacket and a temp probe, a solution/suspension of 4-benzyloxy-1-(3,4-difluorophenyl)-6-fluoro-3-iodo-2-tetrahydropyran-4-yl-indole **S3** (42.0 g, 74.6 mmol), (4-methoxycarbonylphenyl)boronic acid (26.8 g, 148.9 mmol), Pd₂(dba)₃ (1.36 g, 1.49 mmol), dicyclohexyl-[2-(2,6-dimethoxyphenyl)phenyl]phosphane (2.47 g, 6.02 mmol) and CsF (51 g, 335.7 mmol) in DME (1.0 L) was bubbled through with nitrogen for 10 minutes via gas dispersion tube. The reaction was placed under a positive pressure of nitrogen then heated to 80 °C for 3 hours. The reaction was slowly allowed to cool to room temperature, without removing the heating jacket. After 16 hours, dichloromethane (1.3 L) was added (slight exotherm 22 → 34 °C), the mixture was stirred for 10 minutes, then filtered. The solid was collected, washed with dichloromethane (200 mL). The combined filtrate was concentrated and the residue was partitioned between water and dichloromethane (1.5 L each). The organic layer was separated, washed with brine (1 L), dried (MgSO₄), filtered and concentrated. The residue was dissolved/suspended in MTBE (400 mL), spun on rotovap (no vacuum) at 60 °C for 3 minutes, then allowed to stand at room temperature for 40 minutes. The solid was isolated via filtration, washing with MTBE (100 mL) and dried under suction. The material was dissolved in refluxing EtOAc (1.4 L), refluxed for 30 minutes, then the heat was switched off and the solution was slowly allowed to cool to room temperature. After 18 hours, the suspension was filtered, and the collected solid was washed with EtOAc (50 mL) and dried under suction to afford the product. Methyl 4-[4-benzyloxy-1-(3,4-difluorophenyl)-6-fluoro-2-tetrahydropyran-4-yl-indol-3-yl]benzoate (29.14 g, 68%) as a white solid. ¹H NMR (400 MHz, DMSO-*d*₆) δ 7.96 - 7.91 (m, 2H), 7.82 (ddd, J = 11.0, 7.2, 2.5 Hz, 1H), 7.72 (dt, J = 10.5, 8.8 Hz, 1H), 7.53 (d, J = 7.8 Hz, 2H), 7.45 - 7.38 (m, 1H), 7.23 - 7.15 (m, 1H), 7.13 - 7.05 (m, 2H), 6.78 - 6.72 (m, 2H), 6.63 (dd, J = 12.0, 2.1 Hz, 1H), 6.32 (dd, J = 9.5, 2.0 Hz, 1H), 4.95 (s, 2H), 3.92 (s, 3H), 3.65 (dd, J = 11.5, 3.6 Hz, 2H), 3.01 (ddt, J = 13.7, 7.3, 3.7 Hz, 2H), 2.80 - 2.67 (m, 1H), 1.63 - 1.37 (m, 4H). ¹⁹F NMR (376 MHz, DMSO-*d*₆) δ - 117.67, -135.24 (d, J = 23.0 Hz), -137.03 (d, J = 22.8 Hz). LCMS *m*/*z* 572.19 [M+H]⁺.

### Step 2. Synthesis of 4-[4-benzyloxy-1-(3, 4-difluorophenyl)-6-fluoro-2-tetrahydropyran-4-yl-indol-3-yl]benzoic acid (C83)

In a 2 L RBF, to a solution/suspension of methyl 4-[4-benzyloxy-1-(3,4-difluorophenyl)-6-fluoro-2-tetrahydropyran-4-yl-indol-3-yl]benzoate **C82** (25.9 g, 45.3 mmol) in THF (470 mL) at room temperature was added MeOH (100 mL), LiOH (10.9 g, 455.2 mmol) and water (100 mL). The resulting suspension was heated at 60 °C for 16 hours. The mixture was concentrated to dryness. The resulting white solid was treated with 1 M aq HCl (1 L), spun on rotovap at 70 °C for 30 minutes, then the suspension was filtered. The collected solid was washed with water (500 mL), then dried under suction for 1 hour. Solid was then dried on rotovap (2 mbar, 75 °C) for 30 minutes to afford 4-[4-benzyloxy-1-(3,4-difluorophenyl)-6-fluoro-2-tetrahydropyran-4-yl-indol-3-yl]benzoic acid (24.7 g, 98%) as a white solid. ¹H NMR (400 MHz, DMSO-*d*₆) δ 13.12 (s, 1H), 7.99 - 7.91 (m, 2H), 7.82 (ddd, J = 11.1, 7.2, 2.6 Hz, 1H), 7.72 (dt, J = 10.5, 8.8 Hz, 1H), 7.50 (d, J = 7.7 Hz, 2H), 7.42 (ddt, J = 10.0, 3.9, 1.7 Hz, 1H), 7.21 - 7.15 (m, 1H), 7.11 (tt, J = 6.9, 1.8 Hz, 2H), 6.75 - 6.68 (m, 2H), 6.63 (dd, J = 12.0, 2.1 Hz, 1H), 6.32 (dd, J = 9.5, 2.0 Hz, 1H), 4.96 (s, 2H), 3.71 - 3.62 (m, 2H), 3.02 (ddd, J = 12.1, 10.0, 5.5 Hz, 2H), 2.73 (tt, J = 12.0, 3.6 Hz, 1H), 1.62 - 1.39 (m, 4H). ¹⁹F NMR (376 MHz, DMSO-*d*₆) δ -117.78, -135.26 (d, J = 22.8 Hz), -137.07 (d, J = 23.0 Hz). LCMS *m*/*z* 558.1 [M+H]⁺.

### Step 3. Synthesis of 4-[1-(3,4-difluorophenyl)-6-fluoro-4-hydroxy-2-tetrahydropyran-4-yl-indol-3-yl]benzoic acid (11)

To a solution of 4-[4-benzyloxy-1-(3,4-difluorophenyl)-6-fluoro-2-tetrahydropyran-4-yl-indol-3-yl]benzoic acid **C83** (24.7 g, 44.30 mmol) in THF (667 mL) and EtOH (333 mL) under nitrogen was added Pd on C, wet, Degussa (2.25 g of 10% w/w, 2.1 mmol). The reaction vessel was subjected to vacuum, needle to pump for 3 minutes, then placed under H₂ (balloon) for 3 hours. The crude reaction mixture was combined with another 1 g scale batch of this reaction. The mixture was filtered through a Celite^{®} pad, washing with THF: EtOH (2:1, 200 mL). The filtrate was concentrated and the residue was dissolved in THF (500 mL), treated with Biotage MP-TMT resin (15 g) then refluxed for 3 hours. The resin was removed via filtration and the filtrate was concentrated. The residue was treated with EtOAc (100 mL), refluxed for 2 hours, then allowed to cool. Upon standing overnight for 16 hours, the solid was collected via filtration, washing with EtOAc (20 mL). The solid was dried under suction for 30 minutes, then on rotovap (75 °C, 3 mbar) for 3 hours. The solid was dried in vacuum oven (100 °C) for 5 days to afford the product. 4-[1-(3,4-difluorophenyl)-6-fluoro-4-hydroxy-2-tetrahydropyran-4-yl-indol-3-yl]benzoic acid (18.9 g, 86%) as an off-white solid. ¹H NMR (400 MHz, DMSO-*d*₆) δ 13.02 (s, 1H), 9.83 (s, 1H), 7.97 - 7.91 (m, 2H), 7.80 (ddd, J = 11.0, 7.3, 2.5 Hz, 1H), 7.70 (dt, J = 10.5, 8.8 Hz, 1H), 7.51 - 7.45 (m, 2H), 7.40 (ddq, J = 8.4, 3.9, 1.6 Hz, 1H), 6.26 (dd, J = 11.4, 2.2 Hz, 1H), 6.07 (dd, J = 9.6, 2.2 Hz, 1H), 3.66 (dd, J = 11.7, 3.3 Hz, 2H), 3.02 (dddd, J = 11.6, 8.0, 5.9, 3.4 Hz, 2H), 2.77 (tt, J = 11.9, 3.8 Hz, 1H), 1.62 - 1.41 (m, 4H). LCMS *m*/*z* 468.08 [M+H]⁺.

### Compound 12

### 4-[6-chloro-1-(3,4-difluorophenyl)-4-hydroxy-2-tetrahydropyran-4-yl-indol-3-yl]benzoic acid (12)

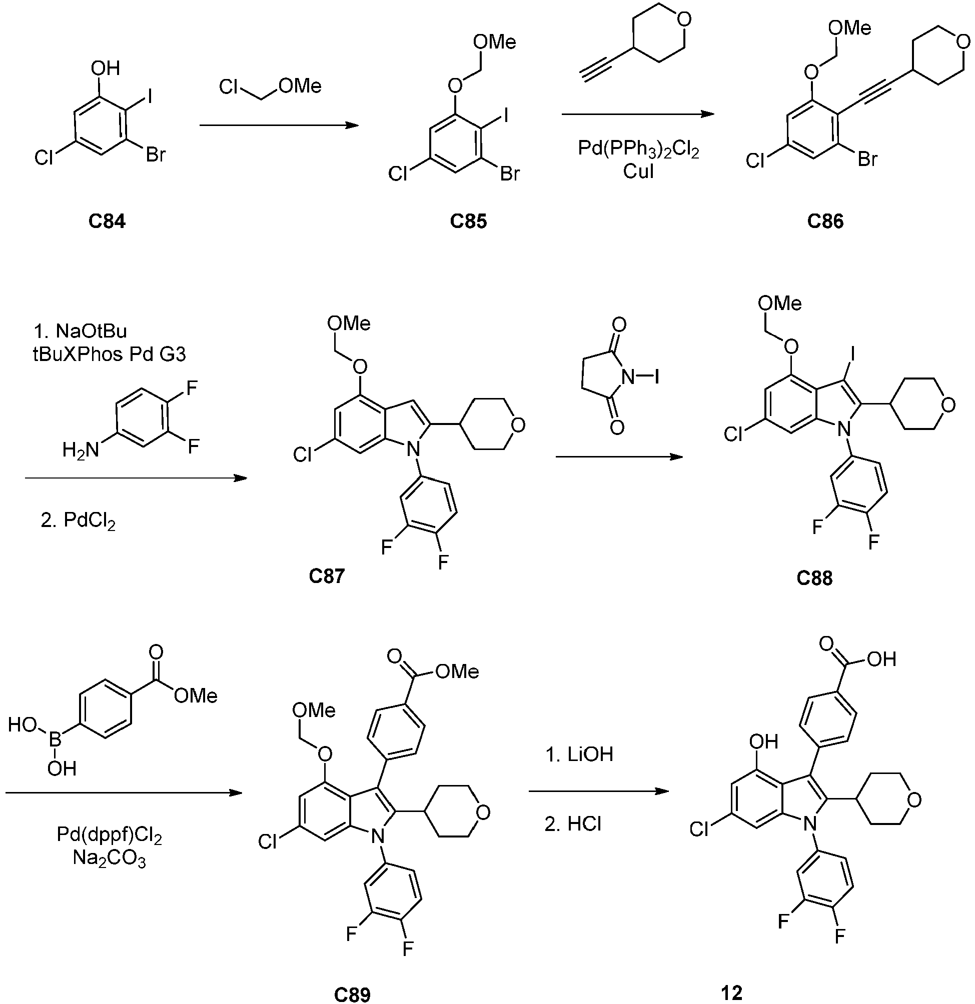

### Step 1. Synthesis of 1-bromo-5-chloro-2-iodo-3-(methoxymethoxy)benzene (C84)

To an ice-cold suspension of 3-bromo-5-chloro-2-iodo-phenol (1.43 g, 4.24 mmol) in dichloromethane (14 mL) was added N,N-diisopropylethylamine (970 µL, 5.57 mmol) followed by chloro(methoxy)methane (400 µL, 5.27 mmol). The mixture was allowed to stir for 30 minutes at room temperature. The reaction mixture was diluted with dichloromethane and washed with 10% citric acid. The organic phase was passed through a phase separator and concentrated. 1-bromo-5-chloro-2-iodo-3-(methoxymethoxy)benzene (1.6 g, 94%). ¹H NMR (400 MHz, DMSO-*d*₆) δ 7.52 (d, J = 2.1 Hz, 1H), 7.16 (d, J = 2.2 Hz, 1H), 5.34 (s, 2H), 3.41 (s, 3H). LCMS *m*/*z* 374.49 [M+1]⁺;

### Step 2. Synthesis of 4-[2-[2-bromo-4-chloro-6-(methoxymethoxy)phenyl]ethynyl]tetrahydropyran (C85)

In a 5 mL microwave tube, 1-bromo-5-chloro-2-iodo-3-(methoxymethoxy)-benzene **C84** (1.6 g, 4.24 mmol) and 4-ethynyltetrahydropyran (490 mg, 4.45 mmol) were combined under nitrogen in 1,4-dioxane (2.2 mL) and Pd(PPh₃)₂Cl₂ (304 mg, 0.43 mmol), CuI (94 mg, 0.49 mmol) and triethyl amine (2.2 mL). The mixture was purged with nitrogen and the reaction was stirred at 60 °C, overnight. The reaction was cooled to room temperature, diluted with EtOAc and washed with water (2x), brine, dried over sodium sulfate, and concentrated under reduced pressure. Silica gel chromatography (Gradient: 0-35% EtOAc in heptane) afforded the product 4-[2-[2-bromo-4-chloro-6-(methoxymethoxy)phenyl]ethynyl]tetrahydropyran (1.27 g, 82%). ¹H NMR (400 MHz, DMSO-*d*₆) δ 7.47 (d, J = 1.9 Hz, 1H), 7.28 (d, J = 1.9 Hz, 1H), 5.31 (s, 2H), 3.89 - 3.78 (m, 2H), 3.56 - 3.44 (m, 2H), 3.41 (s, 3H), 3.07 - 2.95 (m, 1H), 1.93 - 1.80 (m, 2H), 1.68 - 1.55 (m, 2H). LCMS *m*/*z* 358.02 [M+H]⁺.

### Step 3. Synthesis of 6-chloro-1-(3, 4-difluorophenyl)-4-(methoxymethoxy)-2-tetrahydropyran-4-yl-indole (C86)

To a solution of 4-[2-[2-bromo-4-chloro-6-(methoxymethoxy)phenyl]ethynyl]-tetrahydropyran **C85** (615 mg, 1.67 mmol) and 3,4-difluoroaniline (182 µL) in m-xylene (8.1 mL) was added NaOtBu (487 mg, 5.07 mmol), followed by tBuXPhos Pd G3 (55 mg, 0.07 mmol). The reaction mixture was stirred at room temperature overnight. (Note: The product of double addition of the aniline to **C85** was also observed, resulting in a mixture of N1,N3-bis(3,4-difluorophenyl)-5-(methoxymethoxy)-4-(2-tetrahydropyran-4-ylethynyl)benzene-1,3-diamine and the desired 5-chloro-N-(3,4-difluorophenyl)-3-(methoxymethoxy)-2-(2-tetrahydropyran-4-ylethynyl)aniline) The mixture was diluted with water and extracted EtOAc (x 2). The combined organic layers were washed with brine, dried over sodium sulfate, and concentrated to dryness. Silica gel chromatography (Gradient: 0-30% EtOAc in heptane) afforded the product 5-chloro-N-(3,4-difluorophenyl)-3-(methoxymethoxy)-2-(2-tetrahydropyran-4-ylethynyl)aniline which was used directly in the next step. 5-chloro-N-(3,4-difluorophenyl)-3-(methoxymethoxy)-2-(2-tetrahydropyran-4-ylethynyl)aniline was dissolved in acetonitrile (7.5 mL) and PdCl₂ (30 mg, 0.17 mmol). The mixture was warmed to 50 °C and allowed to stir overnight. The mixture was then diluted with EtOAc, then washed with water, brine, and dried over sodium sulfate, then concentrated under reduced pressure to afford the product 6-chloro-1-(3,4-difluorophenyl)-4-(methoxymethoxy)-2-tetrahydropyran-4-yl-indole (59 mg, 9%). LCMS *m*/*z* 408.14 [M+H]⁺.

### Step 4. Synthesis of 6-chloro-1-(3, 4-difluorophenyl)-3-iodo-4-(methoxymethoxy)-2-tetrahydropyran-4-yl-indole (C87)

To an ice-cold solution of 6-chloro-1-(3,4-difluorophenyl)-4-(methoxymethoxy)-2-tetrahydropyran-4-yl-indole **C86** (59 mg, 0.14 mmol) in dichloromethane (2 mL) was added 1-iodopyrrolidine-2,5-dione (35 mg, 0.16 mmol) and allowed to stir for 90 minutes. The mixture was quenched with Na₂S₂O₃, passed through a phase separator, and concentrated to give the product as an off-white solid. 6-chloro-1-(3,4-difluorophenyl)-3-iodo-4-(methoxymethoxy)-2-tetrahydropyran-4-yl-indole (77 mg, 95%). ¹H NMR (400 MHz, DMSO-*d*₆) δ 7.81 - 7.62 (m, 2H), 7.41 - 7.30 (m, 1H), 6.77 (d, J = 1.7 Hz, 1H), 6.52 (d, J = 1.7 Hz, 1H), 5.34 (s, 2H), 3.86 (dd, J = 11.4, 4.0 Hz, 2H), 3.50 (s, 3H), 3.21 (q, J = 10.5 Hz, 2H), 2.96 - 2.85 (m, 1H), 2.23 - 2.10 (m, 2H), 1.56 (t, J = 11.3 Hz, 2H). LCMS *m*/*z* 532.98 [M+H]⁺.

### Step 5. Synthesis of methyl 4-[6-chloro-1-(3, 4-difluorophenyl)-4-(methoxymethoxy)-2-tetrahydropyran-4-yl-indol-3-yl]benzoate (C88)

A mixture of 6-chloro-1-(3,4-difluorophenyl)-3-iodo-4-(methoxymethoxy)-2-tetrahydropyran-4-yl-indole **C87** (75 mg, 0.13 mmol), (4-methoxycarbonylphenyl)boronic acid (24 mg, 0.13 mmol), sodium carbonate (200 µL of 2 M, 0.4 mmol), and Pd(dppf)Cl₂ (11 mg, 0.013 mmol) in dimethylformamide (2 mL) was heated in the microwave at 90 °C for 40 minutes. The mixture was diluted with EtOAc and washed with water (3 x), brine, dried over sodium sulfate, and concentrated under reduced pressure. Purification by silica gel chromatography (Gradient: 0-35% EtOAc in heptane) afforded the product. Methyl 4-[6-chloro-1-(3,4-difluorophenyl)-4-(methoxymethoxy)-2-tetrahydropyran-4-yl-indol-3-yl]benzoate (30 mg, 41%). ¹H NMR (400 MHz, DMSO-*d*₆) δ 8.05 - 7.96 (m, 2H), 7.83 (q, J = 11.8, 10.5 Hz, 1H), 7.72 (q, J = 9.4 Hz, 1H), 7.55 (d, J = 7.7 Hz, 2H), 7.44 (d, J = 8.6 Hz, 1H), 6.69 (d, J = 1.7 Hz, 1H), 6.56 (d, J = 1.7 Hz, 1H), 4.93 (s, 2H), 3.89 (d, J = 1.5 Hz, 3H), 3.66 (d, J = 10.7 Hz, 2H), 3.12 - 2.94 (m, 5H), 2.77 (t, J = 12.2 Hz, 1H), 1.64 - 1.40 (m, 4H). LCMS *m*/*z* 542.12 [M+H]⁺.

### Step 6. Synthesis of 4-[6-chloro-1-(3, 4-difluorophenyl)-4-hydroxy-2-tetrahydropyran-4-yl-indol-3-yl]benzoic acid (12)

To a solution of methyl 4-[6-chloro-1-(3,4-difluorophenyl)-4-(methoxymethoxy)-2-tetrahydropyran-4-yl-indol-3-yl]benzoate **C89** (30 mg, 0.06 mmol) in tetrahydrofuran (2.7 mL) / methanol (1.3 mL) was added LiOH (600 µL of 1 M, 0.6 mmol). The reaction was warmed to 50 °C and allowed to stir for 90 minutes, whereupon it was acidified with 1N HCl and extracted with EtOAc. The organic layer was washed with brine, dried over sodium sulfate, and concentrated under reduced pressure. The resulting white solid was dissolved in 1,4-dioxane (2 mL), hydrochloric acid (300 µL of 4 M, 1.2 mmol) was added and the mixture was allowed to stir overnight. Additional hydrogen chloride (300 µL of 4 M, 1.2 mmol) was added and allowed to stir for 3 hours. Purification by reversed-phase chromatography (Column: C18. Gradient: 30-100% MeCN in water with 0.2% trifluoroacetic acid) afforded the product. 4-[6-chloro-1-(3,4-difluorophenyl)-4-hydroxy-2-tetrahydropyran-4-yl-indol-3-yl]benzoic acid (11.2 mg, 42%). ¹H NMR (400 MHz, DMSO-*d*₆) δ 9.78 (s, 1H), 7.96 (d, J = 7.7 Hz, 2H), 7.83 (t, J = 8.4 Hz, 1H), 7.71 (q, J = 9.4 Hz, 1H), 7.50 (dd, J = 6.8, 3.8 Hz, 2H), 7.42 (s, 1H), 6.43 (d, J = 1.9 Hz, 1H), 6.31 (d, J = 2.1 Hz, 1H), 3.67 (d, J = 11.1 Hz, 2H), 3.02 (d, J = 13.2 Hz, 2H), 2.76 (q, J = 12.4 Hz, 1H), 1.64 - 1.41 (m, 4H). LCMS *m*/*z* 484.12 [M+H]⁺.

### Compound 13

### 4-[1-(3,4-difluorophenyl)-6-fluoro-4-hydroxy-2-(2-methoxy-1,1-dimethyl-ethyl)indol-3-yl]-2-fluoro-benzoic acid (13)

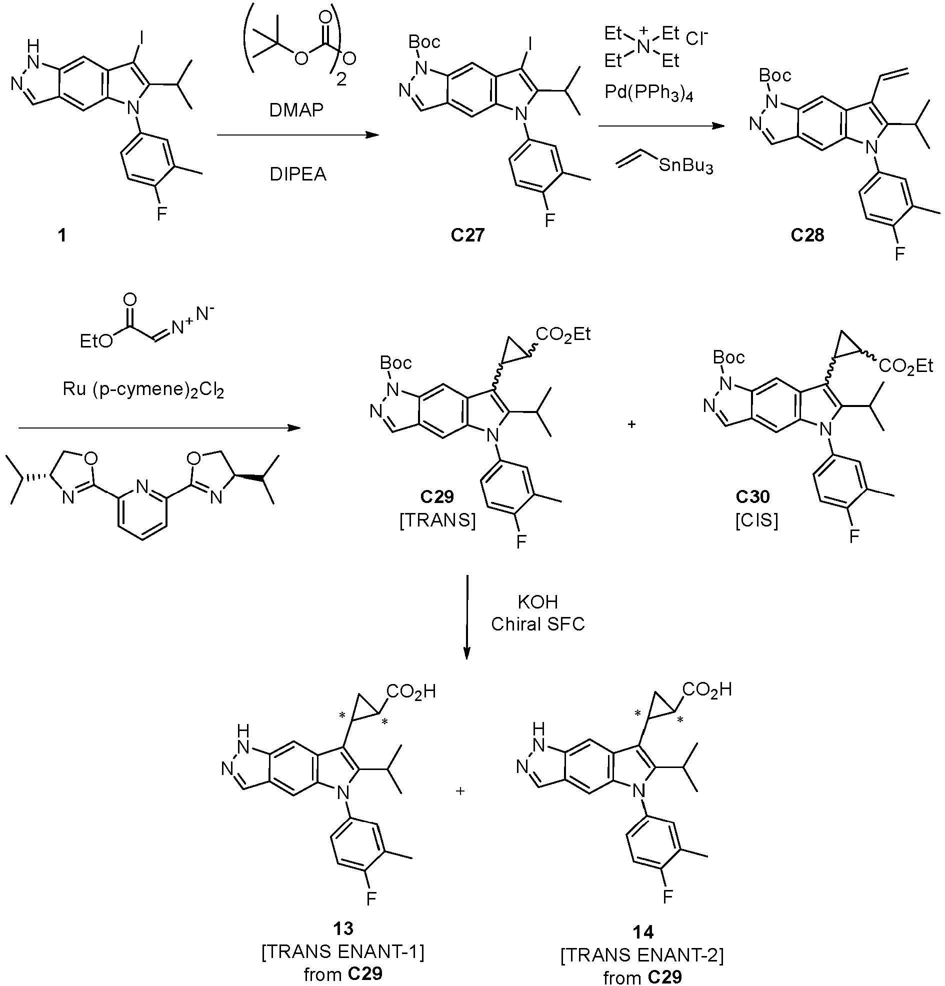

Compound **13** was prepared in 3 steps from **S4** according to the method described for synthesis of compound 1. 4-[1-(3,4-difluorophenyl)-6-fluoro-4-hydroxy-2-(2-methoxy-1,1-dimethyl-ethyl)indol-3-yl]-2-fluoro-benzoic acid (145.3 mg, 92%). ¹H NMR (400 MHz, Chloroform-*d*/CD3OD) δ 7.97 (s, 1H), 7.40 (d, J = 25.5 Hz, 5H), 6.21 (d, J = 10.9 Hz, 1H), 5.87 (s, 1H), 3.15 (s, 3H), 3.02 (s, 2H), 1.10 (s, 6H). LCMS *m*/*z* 488.08 [M+H]⁺

### Compound 14

### 4-[1-(3,4-difluorophenyl)-6-fluoro-4-hydrody-2-(2-methoxy-1,1-dimethyl-ethyl)indol-3-yl]benzoic acid (14)

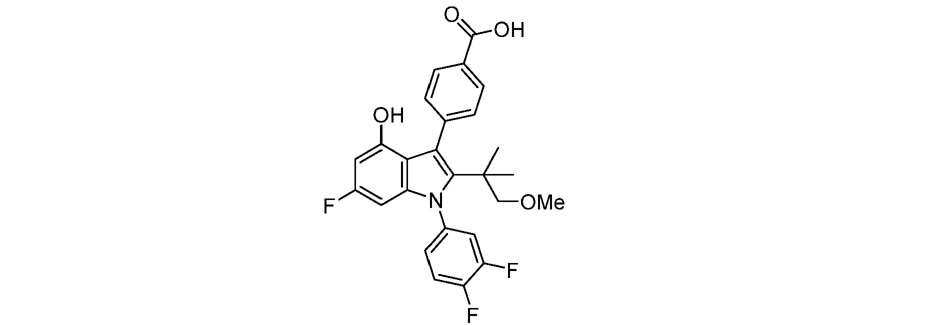

Compound **14** was prepared in 3 steps from **S4** according to the method described for synthesis of compound 1. 4-[1-(3,4-difluorophenyl)-6-fluoro-4-hydroxy-2-(2-methoxy-1,1-dimethyl-ethyl)indol-3-yl]benzoic acid (124 mg, 96%). ¹H NMR (400 MHz, Chloroform-*d*) δ 8.33 - 8.18 (m, 2H), 7.74 (ddd, J = 8.8, 4.6, 2.0 Hz, 2H), 7.45 - 7.31 (m, 2H), 7.28 (dt, J = 8.9, 3.3 Hz, 1H), 6.28 (dd, J = 10.8, 2.2 Hz, 1H), 5.95 (dd, J = 9.4, 2.2 Hz, 1H), 3.16 (s, 3H), 3.02 (s, 2H), 1.10 (d, J = 2.3 Hz, 6H). LCMS *m*/*z* 470.4 [M+H]⁺.

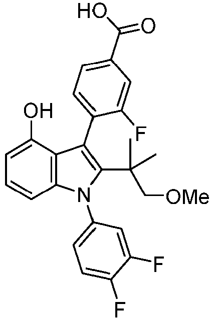

Compound **15** was prepared in 3 steps from **S4** and (2-fluoro-4-methoxy-carbonylphenyl)boronic acid according to the method described for synthesis of compound 1. ¹H NMR (400 MHz, Chloroform-d) δ 8.12 (td, J = 7.8, 1.8 Hz, 1H), 7.49 (dddd, J = 21.0, 11.3, 4.4, 1.6 Hz, 2H), 7.42 - 7.32 (m, 2H), 7.32 - 7.22 (m, 1H), 6.97 (t, J = 8.0 Hz, 1H), 6.48 (d, J = 7.6 Hz, 1H), 6.25 (d, J = 8.2 Hz, 1H), 3.27 - 3.11 (m, 3H), 3.05 (s, 2H), 1.12 (s, 6H). LCMS *m*/*z* 470.19 [M+H]⁺.

### Compound 16

### 4-[1-(3,4-difluorophenyl)-4-hydroxy-2-(2-methoxy-1,1-dimethyl-ethyl)indol-3-yl]benzoic acid (16)

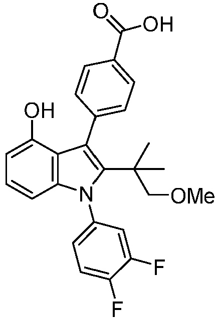

Compound **16** was prepared in 3 steps from **S4** and (4-methoxycarbonyl-phenyl)boronic acid according to the method described for synthesis of compound 1.4-[1-(3,4-difluorophenyl)-4-hydroxy-2-(2-methoxy-1,1-dimethyl-ethyl)indol-3-yl]benzoic acid (91.5 mg, 81%). ¹H NMR (400 MHz, Chloroform-*d*) δ 8.12 - 8.04 (m, 2H), 7.65 - 7.56 (m, 2H), 7.31 - 7.22 (m, 2H), 7.18 - 7.11 (m, 1H), 6.89 - 6.79 (m, 1H), 6.36 (dd, J = 7.8, 0.8 Hz, 1H), 6.13 (dd, J = 8.3, 0.8 Hz, 1H), 3.02 (s, 3H), 2.91 (s, 2H), 0.99 (d, J = 2.2 Hz, 6H). LCMS *m*/*z* 452.3 [M+H]⁺.

### Compound 17

### 4-[1-(3,4-difluorophenyl)-4-hydroxy-2-[2-methoxy-1-(methoxymethyl)ethyl]indol-3-yl]benzoic acid (17)

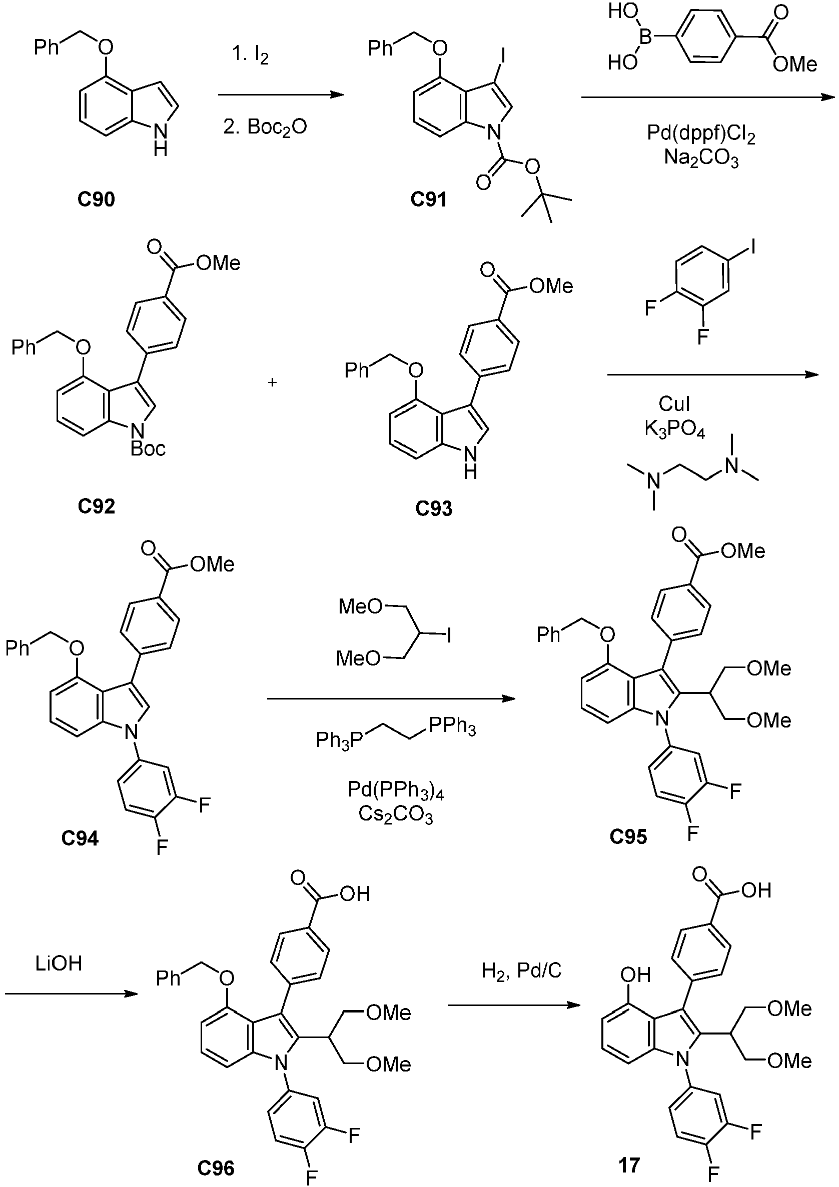

### Step 1. Synthesis of tert-butyl 4-benzyloxy-3-iodo-indole-1-carboxylate (C91)

4-benzyloxy-1H-indole **C90** (2.5 g, 11.2 mmol) in DMF (25 mL) at rt. was added **KOH** (1.6 g, 28.5 mmol) and the mixture was heated to 75 °C for 15 minutes. Upon cooling to room temperature, a solution of I₂ (3.1 g, 12.2 mmol) in DMF (5 mL) was added dropwise. The reaction mixture was stirred for 45 minutes. The mixture was then poured into ice water containing 1% v/v NH₄OH and 0.2% w/v sodium metabisulfite and stirred for 20 minutes. A pink solid was collected by filtration and dried to give 4-benzyloxy-3-iodo-1H-indole (3 g, 77%) which was used without further purification. To a solution of 4-benzyloxy-3-iodo-1H-indole (3 g, 77%) and boc anhydride (2.5 g, 11.5 mmol) in dichloromethane (25 mL) was added DMAP (150 mg, 1.23 mmol) and the solution was stirred at room temperature for 2 hours. The solvent was removed under reduced pressure. Purification by silica gel chromatography (Gradient: 10-50%) EtOAc in heptane) afforded tert-butyl 4-benzyloxy-3-iodo-indole-1-carboxylate (3.3 g, 63%) as light brown solid. ¹H NMR (400 MHz, DMSO-*d*₆) δ 7.72 (d, J = 2.0 Hz, 2H), 7.63-7.61 (m, 2H), 7.45 - 7.38 (m, 2H), 7.35 - 7.21 (m, 2H), 6.95 (m, 1H), 5.28 (s, 2H), 1.61 (s, 9H). LCMS *m*/*z* 450.16 [M+H]⁺.

### Step 2. Synthesis of tert-butyl 4-benzyloxy-3-(4-methoxycarbonylphenyl)indole-1-carboxylate (C92) and methyl 4-(4-benzyloxy-1H-indol-3-yl)benzoate (C93)

A mixture of tert-butyl 4-benzyloxy-3-iodo-indole-1-carboxylate **C91** (1.4 g, 3.11 mmol), (4-methoxycarbonylphenyl)boronic acid (800 mg, 4.45 mmol), (4-methoxycarbonylphenyl)boronic acid (800 mg, 4.45 mmol) and Na₂CO₃ (3.2 mL of 3 M, 9.6 mmol) in DMF (10 mL) and water (3.2 mL) was degassed for 5 minutes. Pd(dppf)Cl₂ (160 mg, 0.20 mmol) was added and the mixture degassed for another 5 minutes, then microwaved at 100° C for 20 minutes. The mixture was diluted with ice-water and the precipitate was collected. Purification via silica gel chromatography (Gradient: 0-40% EtOAc in heptane) afforded the N-Boc and NH indole products tert-butyl 4-benzyloxy-3-(4-methoxycarbonylphenyl)indole-1-carboxylate **(C92)** and methyl 4-(4-benzyloxy-1H-indol-3-yl)benzoate **(C93).**

N-Boc Product **(C92):** tert-butyl 4-benzyloxy-3-(4-methoxycarbonylphenyl)-indole-1-carboxylate (433 mg, 30%). ¹H NMR (400 MHz, Chloroform-d) δ 7.98 - 7.91 (m, 3H), 7.70 - 7.62 (m, 2H), 7.59 (s, 1H), 7.33 (t, J = 8.2 Hz, 1H), 7.31 - 7.22 (m, 3H), 7.13 - 7.06 (m, 2H), 6.83 (dd, J = 8.1, 0.7 Hz, 1H), 5.08 (s, 2H), 3.99 (s, 3H), 1.73 (s, 9H). LCMS *m*/*z* 458.4 [M+H]⁺.

NH Product **(C93):** methyl 4-(4-benzyloxy-1H-indol-3-yl)benzoate (480 mg, 43%). ¹H NMR (400 MHz, Chloroform-*d*) δ 8.36 (s, 1H), 7.94 - 7.85 (m, 2H), 7.73 - 7.61 (m, 2H), 7.27 - 7.23 (m, 3H), 7.21 - 7.15 (m, 4H), 7.07 (dd, J = 8.2, 0.8 Hz, 1H), 6.67 (dd, J = 7.8, 0.8 Hz, 1H), 5.11 (s, 2H), 3.94 (s, 3H). LCMS *m*/*z* 358.27 [M+H]⁺.

### Step 3. Synthesis of methyl 4-[4-benzyloxy-1-(3,4-difluorophenyl)indol-3-yl]benzoate (C94)

CuI (45 mg, 0.24 mmol), methyl 4-(4-benzyloxy-1H-indol-3-yl)benzoate **C93** (420 mg, 1.2 mmol), K₃PO₄ (525 mg, 2.5 mmol) were added to a vial fitted with a rubber septum. The vessel was evacuated and back-filled with argon, and this sequence was repeated an additional time. Toluene (6 mL) was added, followed by the successive addition of 1,2-difluoro-4-iodo-benzene (280 µL, 2.32 mmol) and N,N'-dimethylethane-1,2-diamine (50 µL, 0.47 mmol) by syringe under a stream of argon. The reaction tube was sealed and the contents were stirred with heating from an oil bath at 110 °C for 24 hours. The reaction mixture was cooled to ambient temperature, diluted with ethyl acetate (2-3 mL), filtered through a plug of silica gel, eluting with additional ethyl acetate (10-20 mL). The filtrate was concentrated and the resulting residue was purified by column chromatography (Gradient: 0-80% Ethyl acetate in hexane) to provide the product as a white solid. methyl 4-[4-benzyloxy-1-(3,4-difluorophenyl)indol-3-yl]benzoate (0.45 g, 82%). ¹H NMR (400 MHz, Chloroform-*d*) δ 7.99 - 7.91 (m, 2H), 7.76 - 7.67 (m, 2H), 7.47 - 7.40 (m, 1H), 7.40 - 7.34 (m, 1H), 7.32 - 7.26 (m, 5H), 7.25 - 7.13 (m, 4H), 6.77 (dd, J = 7.7, 0.9 Hz, 1H), 5.15 (s, 2H), 3.98 (s, 3H). LCMS *m*/*z* 470.35 [M+H]⁺.

### Step 4. methyl 4-[4-benzyloxy-1-(3, 4-difluorophenyl)-2-[2-methoxy-1-(methoxymethyl)ethyl]indol-3-yl]benzoate (C95)

A vial charged with methyl 4-[4-benzyloxy-1-(3,4-difluorophenyl)indol-3-yl]benzoate **C94** (105 mg, 0.22 mmol), Pd(PPh₃)₄ (25 mg, 0.02 mmol), 3-diphenylphosphanylpropyl(diphenyl)phosphane (13 mg, 0.032 mmol) and Cs₂CO₃ (140 mg, 0.43 mmol) was evacuated and refilled with nitrogen (x 2), then 2-iodo-1,3-dimethoxy-propane (52 mg, 0.23 mmol) in dry 1',1',1'-trifluorotoluene (1.5 mL) was added. After stirring at room temperature for 2 minutes, the mixture was vigorously stirred at 110 °C for 24 hours. The reaction mixture was cooled and concentrated on a rotary evaporator. The resulting residue was subjected to silica gel chromatography (Gradient: 0-20% EtOAc in Hexanes) to provide the product **C95** and unreacted starting material. Methyl 4-[4-benzyloxy-1-(3,4-difluorophenyl)-2-[2-methoxy-1-(methoxymethyl)ethyl]indol-3-yl]benzoate (7.5 mg, 6%). ¹H NMR (400 MHz, Chloroform-d) δ 7.94 - 7.87 (m, 2H), 7.51 - 7.42 (m, 2H), 7.34 - 7.24 (m, 2H), 7.12 - 7.02 (m, 4H), 7.02 - 6.96 (m, 1H), 6.75 - 6.66 (m, 2H), 6.52 (ddd, J = 10.2, 8.1, 0.7 Hz, 2H), 4.84 (s, 2H), 3.91 (s, 3H), 3.32 - 3.27 (m, 1H), 3.26 - 3.15 (m, 4H), 3.06 (d, J = 5.1 Hz, 6H). LCMS *m*/*z* 572.17 [M+H]⁺.

### Step 5. Synthesis of 4-[4-benzyloxy-1-(3, 4-difluorophenyl)-2-[2-methoxy-1-(methoxymethyl)ethyl]indol-3-yl]benzoic acid (C96)

To a solution of methyl 4-[4-benzyloxy-1-(3,4-difluorophenyl)-2-[2-methoxy-1-(methoxymethyl)ethyl]indol-3-yl]benzoate **C95** (18 mg, 0.03 mmol) in THF (250 µL) and MeOH (100 µL) was added LiOH (100 µL of 1 M, 0.1 mmol) in water (100 µL). The solution was stirred at 50 °C for 1 hour. HCl (0.2 mL) was added to neutralized the reaction and conc. EtOAc (2 mL) was added and washed with water (0.5 mL) and brine (0.5 mL), dried to afford the product which used in the next step without further purification. 4-[4-benzyloxy-1-(3,4-difluorophenyl)-2-[2-methoxy-1-(methoxymethyl)ethyl]indol-3-yl]benzoic acid (15.6 mg, 86%). LCMS *m*/*z* 558.44 [M+H]⁺.

### Step 6. Synthesis of 4-[1-(3,4-difluorophenyl)-4-hydroxy-2-[2-methoxy-1-(methoxymethyl)ethyl]indol-3-yl]benzoic acid (17)

To a solution of 4-[4-benzyloxy-1-(3,4-difluorophenyl)-2-[2-methoxy-1-(methoxymethyl)ethyl]indol-3-yl]benzoic acid **C96** (15 mg, 0.03 mmol) in THF (1 mL) and EtOH (1 mL) was added Pd (4. mg of 10% w/w, 0.004 mmol) on carbon. The reaction mixture was subjected to hydrogenation (H₂ at balloon pressure) for 3 hours. The mixture was filtered through a pad of Celite^{®} to remove the catalyst. The filtrate was concentrated to afford and purified by silica gel chromatography (Gradient: 0-10% MeOH in dichloromethane) to provide product as white solid. 4-[1-(3,4-difluorophenyl)-4-hydroxy-2-[2-methoxy-1-(methoxymethyl)ethyl]indol-3-yl]benzoic acid (11 mg, 81%). ¹H NMR (300 MHz, Chloroform-*d)* δ 8.15 (d, J = 8.1 Hz, 2H), 7.68 - 7.58 (m, 2H), 7.36 - 7.23 (m, 2H), 7.17 - 7.11 (m, 1H), 6.94 (t, J = 8.0 Hz, 1H), 6.46 (dd, J = 8.0, 1.8 Hz, 2H), 3.35 (dt, J = 8.1, 6.7 Hz, 1H), 3.30 - 3.14 (m, 4H), 3.09 (d, J = 2.4 Hz, 6H). LCMS *m*/*z* 468.37 [M+H]⁺.

### Compound 18

### 4-[1-(3,4-difluorophenyl)-4-hydroxy-2-(trifluoromethyl)indol-3-yl]benzoic acid (18)

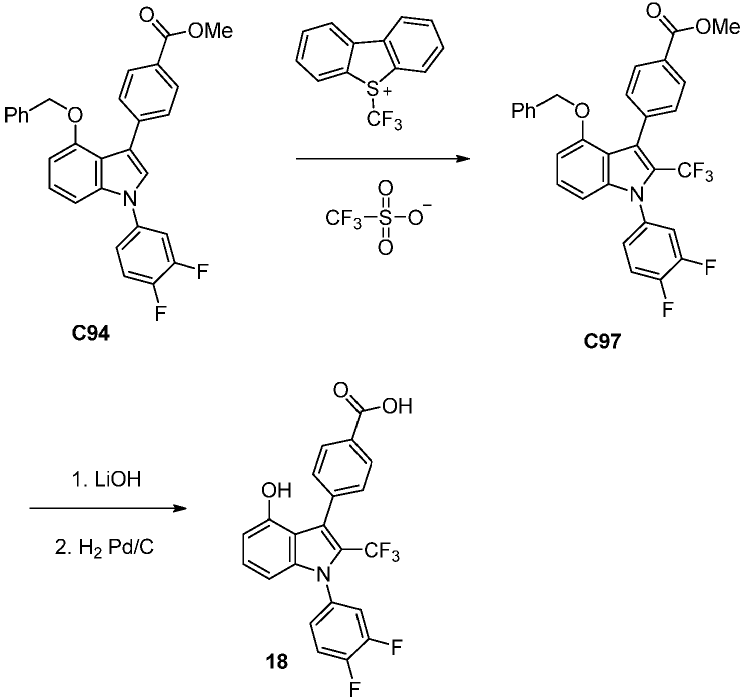

### Step 1. Synthesis of methyl 4-[4-benzyloxy-1-(3, 4-difluorophenyl)-2-(trifluoromethyl)indol-3-yl]benzoate (C97)

To a vial was charged with methyl 4-[4-benzyloxy-1-(3,4-difluorophenyl)indol-3-yl]benzoate **C94** (145 mg, 0.31 mmol) and trifluoromethanesulfonate;5-(trifluoromethyl)dibenzothiophen-5-ium (250 mg, 0.62 mmol) followed by DMF (1.5 mL) and NMM (80 µL, 0.73 mmol) methyl 4-[4-benzyloxy-1-(3,4-difluorophenyl)indol-3-yl]benzoate (145 mg, 0.31 mmol) and stirred at 50 °C overnight. 1M HCl (3 mL) was added, and the aqueous was extracted with dichloromethane (3 mL x 3). Combined organic layers were dried and purification silica gel chromatography (Gradient: 0-10% EtOAc in hexanes) to give the product as a white solid. Methyl 4-[4-benzyloxy-1-(3,4-difluorophenyl)-2-(trifluoromethyl)indol-3-yl]benzoate (68 mg, 40%) LCMS *m*/*z* 538.41 [M+H]⁺.

### Steps 2 & 3. Synthesis of 4-[1-(3,4-difluorophenyl)-4-hydroxy-2-(trifluoromethyl)indol-3-yl]benzoic acid (18)

Methyl 4-[4-benzyloxy-1-(3,4-difluorophenyl)-2-(trifluoromethyl)indol-3-yl]benzoate **C97** (20 mg, 0.03721 mmol) in THF (300 µL) and MeOH (150 µL) was added LiOH (120 µL of 1 M, 0.1200 mmol) in water (120 µL). The solution was stirred at 50 °C for 1 hour. The reaction was neutralized by the addition of 1M HCl (0.2 mL) and then concentrated. EtOAc (2 mL) was added. The mixture was washed with water (0.5 mL), brine (0.5 mL), dried and concentrated to give the product 4-[4-benzyloxy-1-(3,4-difluorophenyl)-2-(trifluoromethyl)indol-3-yl]benzoic acid (16 mg, 78%) which was used in the next step without further purification. 4-[4-benzyloxy-1-(3,4-difluorophenyl)-2-(trifluoromethyl)indol-3-yl]benzoic acid (18 mg, 0.03 mmol) in THF (500 µL) and EtOH (500 µL) was added Pd (5 mg of 10% w/w, 0.005 mmol) on carbon, then hydrogenated using a balloon of H₂ (5 mg, 2.5 mmol) for 3 hours. The mixture was filtered through a pad of Celite^{®} to remove the catalyst and the filtrate was concentrated. Silica gel chromatography (Gradient: 0-8% MeOH in dichloromethane) to provide the desired product as white solid. 4-[1-(3,4-difluorophenyl)-4-hydroxy-2-(trifluoromethyl)indol-3-yl]benzoic acid (14 mg, 99%). ¹H NMR (400 MHz, Chloroform-d) δ 8.24 - 8.17 (m, 2H), 7.67 - 7.59 (m, 2H), 7.36 - 7.24 (m, 2H), 7.18 (d, J = 4.4 Hz, 1H), 7.13 (d, J = 8.1 Hz, 1H), 6.55 (d, J = 8.1 Hz, 2H). LCMS *m*/*z* 434.52 [M+H]⁺.

### Compounds 19-34

Compounds **19-34** (Table 2) were prepared from **S6, S7** or **S8** and the appropriate boronic acid or ester using a Suzuki coupling, benzyl group removal via hydrogenation, or MOM group removal as appropriate, and an ester hydrolysis where appropriate, as described in the preparation of compounds **1-6** or compound **12.** Any modifications to these methods are noted in the table footnotes.

**Table 2. Method of preparation, structure and physicochemical data for compounds 19-34**

| **Compound** | **Method/Product** | **Boronic acid or ester** | ¹H NMR; **LCMS m/z** [M+H]⁺ |
|---|---|---|---|
| **19** | From **S6** Compound **12¹** | 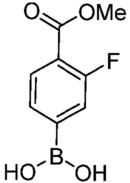 | ¹H NMR (400 MHz, Chloroform-*d*) δ 8.02 (t, J = 7.9 Hz, 1H), 7.49 (dd, J = 6.5, 2.5 Hz, 1H), 7.35 (dd, J = 8.2, 6.5 Hz, 2H), 7.31 - 7.25 (m, 2H), 7.03 - 6.90 (m, 1H), 6.50 (dd, J = 7.7, 0.8 Hz, 1H), 6.40 (dd, J = 8.3, 0.8 Hz, 1H), 3.84 (dt, J = 12.8, 3.0 Hz, 2H), 3.22 (ddd, J = 11.7, 4.0, 2.2 Hz, 2H), 2.87 (tt, J = 12.3, 3.5 Hz, 1H), 1.70 (qd, J = 12.4, 4.3 Hz, 2H), 1.63 - 1.53 (m, 2H). LCMS *m*/*z* 484.35 [M+H]⁺. |
| | 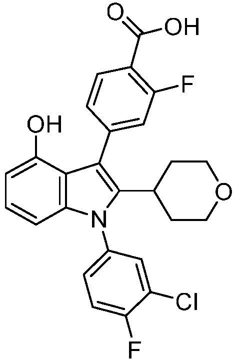 | | |
| **20** | From **S6** Compound **12¹** | 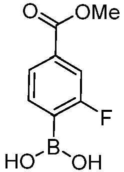 | ¹H NMR (400 MHz, Chloroform-*d*) δ 7.85 (d, J = 7.9 Hz, 1H), 7.78 (d, J = 9.6 Hz, 1H), 7.45 (dt, J = 21.0, 7.1 Hz, 2H), 7.29 (dt, J = 8.1, 4.4 Hz, 2H), 6.90 (t, J = 7.9 Hz, 1H), 6.39 (dd, J = 14.8, 8.0 Hz, 2H), 3.76 (dd, J = 12.2, 5.9 Hz, 2H), 3.11 (td, J = 11.4, 4.3 Hz, 2H), 2.71 (t, J = 12.0 Hz, 1H), 1.68 - 1.47 (m, 4H). LCMS *m*/*z* 434.52 [M+H]⁺ |
| | 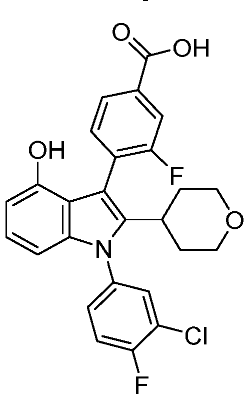 | | |
| **21** | From **S6.** As for compound **12¹** | 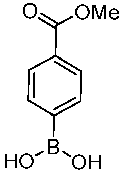 | ¹H NMR (400 MHz, Chloroform-*d*) δ 8.21 (d, J = 7.8 Hz, 2H), 7.64 (d, J = 7.9 Hz, 2H), 7.51 (d, J = 6.3 Hz, 1H), 7.37 (dd, J = 19.0, 10.8 Hz, 2H), 7.03 (t, J = 8.1 Hz, 1H), 6.55 (d, J = 7.7 Hz, 1H), 6.47 (d, J = 8.1 Hz, 1H), 4.14 (q, J = 7.2 Hz, 1H), 3.85 (d, J = 11.5 Hz, 2H), 3.18 (d, J = 12.6 Hz, 2H), 2.86 (d, J = 13.6 Hz, 1H), 1.84 - 1.63 (m, 1H), 1.59 (d, J = 13.1 Hz, 2H). LCMS *m*/*z* 466.3 [M+H]⁺. |
| | 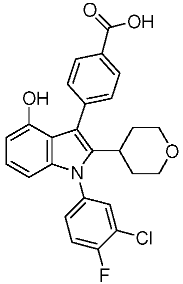 | | |
| **22** | From **S7.** See footnote for method^{2,3} | 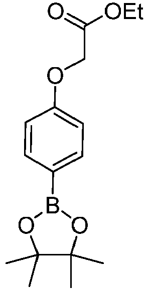 | ¹H NMR (300 MHz, DMSO-*d*₆) δ 13.06 (s, 1H), 9.56 (d, J = 1.3 Hz, 1H), 7.36 (dddd, J = 28.3, 17.4, 8.3, 5.1 Hz, 3H), 6.90 - 6.73 (m, 2H), 6.17 (dd, J = 11.5, 2.2 Hz, 1H), 5.99 (dt, J = 9.5, 1.7 Hz, 1H), 4.75 (s, 2H), 3.67 (d, J = 11.3 Hz, 2H), 3.01 (s, 2H), 2.70 (d, J = 15.4 Hz, 1H), 2.33 (s, 3H), 1.65 - 1.21 (m, 4H). LCMS *m*/*z* 512 [M+H]⁺. |
| | 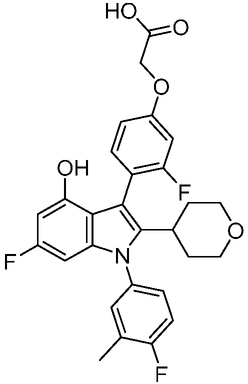 | | |
| **23** | From **S7.** See footnote for method^{2,3} | 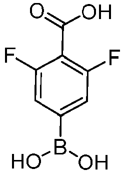 | ¹H NMR (400 MHz, DMSO-*d*₆) δ 13.94 (s, 1H), 9.91 (s, 1H), 7.45 - 7.37 (m, 2H), 7.32 (ddd, J = 8.2, 4.6, 2.7 Hz, 1H), 7.18 (d, J = 9.3 Hz, 2H), 6.26 (dd, J = 11.4, 2.2 Hz, 1H), 5.97 (dd, J = 9.5, 2.2 Hz, 1H), 3.71 (d, J = 11.5 Hz, 2H), 3.06 (td, J = 11.7, 2.2 Hz, 2H), 2.80 (ddt, J = 11.8, 7.5, 3.9 Hz, 1H), 2.34 (d, J = 1.9 Hz, 3H), 1.64 - 1.45 (m, 4H). LCMS *m*/*z* 500.0 [M+H]⁺. |
| | 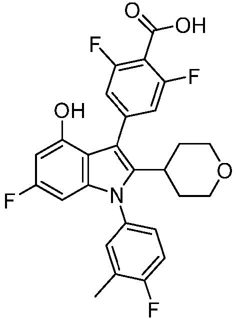 | | |
| **24** | From **S7.** See footnote for method² | 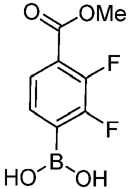 | ¹H NMR (300 MHz, DMSO-*d*₆) δ 9.80 (s, 1H), 7.54 - 7.25 (m, 4H), 7.05 (s, 1H), 6.23 (dd, J = 11.6, 2.2 Hz, 1H), 5.99 (dt, J = 9.6, 1.8 Hz, 1H), 3.69 (d, J = 11.3 Hz, 2H), 3.02 (q, J = 10.3, 8.6 Hz, 2H), 2.72 (s, 1H), 2.33 (s, 3H), 1.65 - 1.40 (m, 4H). LCMS *m*/*z* 500.0 [M+H]⁺. |
| | 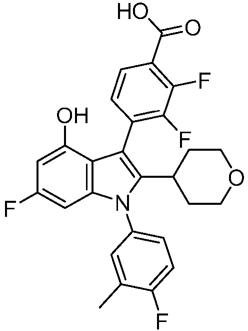 | | |
| **25** | From **S7.** See footnote for method² | 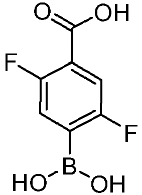 | ¹H NMR (300 MHz, DMSO-*d*₆) δ 9.80 (s, 1H), 7.43 - 7.28 (m, 4H), 7.13 (s, 1H), 6.23 (dd, J = 11.5, 2.2 Hz, 1H), 5.99 (dt, J = 9.6, 1.6 Hz, 1H), 3.69 (s, 2H), 3.03 (t, J = 11.2 Hz, 2H), 2.72 (s, 1H), 2.33 (d, J = 1.9 Hz, 3H), 1.53 (dd, J = 34.6, 9.6 Hz, 4H). LCMS *m*/*z* 500.0 [M+H]⁺. |
| | 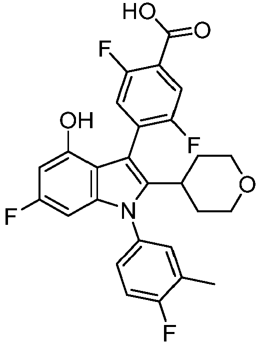 | | |
| **26** | From **S7.** See footnote for method² | 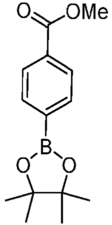 | ¹H NMR (400 MHz, DMSO-*d*₆) δ 9.84 (s, 1H), 7.93 (d, J = 7.9 Hz, 2H), 7.46 (dd, J = 6.8, 3.5 Hz, 3H), 7.42 - 7.32 (m, 2H), 6.25 (dd, J = 11.5, 2.2 Hz, 1H), 5.95 (dd, J = 9.6, 2.2 Hz, 1H), 3.70 - 3.62 (m, 2H), 3.07 - 2.94 (m, 2H), 2.84 - 2.72 (m, 1H), 2.34 (d, J = 1.9 Hz, 3H), 1.59 - 1.45 (m, 4H). LCMS *m*/*z* 464.0 [M+H]⁺. |
| | 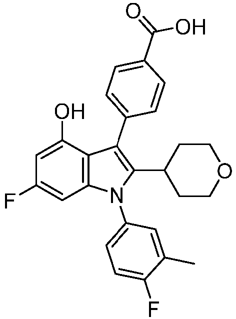 | | |
| **27** | From **S7.** See footnote for method^{2,3} | 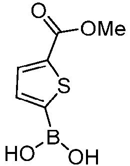 | ¹H NMR (400 MHz, DMSO-*d*₆) δ 9.88 (s, 1H), 7.59 (s, 1H), 7.49 - 7.33 (m, 3H), 7.04 (s, 1H), 6.26 (dd, J = 11.4, 2.2 Hz, 1H), 5.97 (dd, J = 9.5, 2.2 Hz, 1H), 3.72 (d, J = 11.2 Hz, 2H), 3.10 - 3.00 (m, 2H), 2.90 - 2.68 (m, 1H), 2.33 (d, J = 1.9 Hz, 3H), 1.61 (q, J = 10.7, 10.1 Hz, 4H). LCMS *m*/*z* 470.0 [M+H]⁺. |
| | 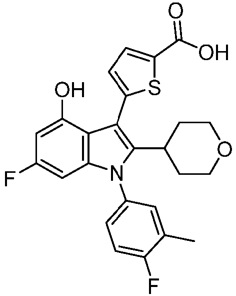 | | |
| **28** | From **S7.** See footnote for method^{4,5} | 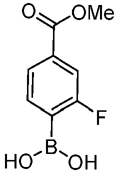 | ¹H NMR (400 MHz, DMSO-*d*₆) δ 9.75 (s, 1H), 7.77 (d, J = 7.8 Hz, 1H), 7.66 (d, J = 9.9 Hz, 1H), 7.53 - 7.32 (m, 4H), 6.21 (dd, J = 11.4, 2.2 Hz, 1H), 6.00 (dt, J = 9.7, 2.2 Hz, 1H), 3.66 (s, 2H), 3.01 (dt, J = 11.5, 9.2 Hz, 2H), 2.76 - 2.65 (m, 1H), 2.34 (s, 3H), 1.66 - 1.36 (m, 4H). LCMS *m*/*z* 482.1 [M+H]⁺ |
| | 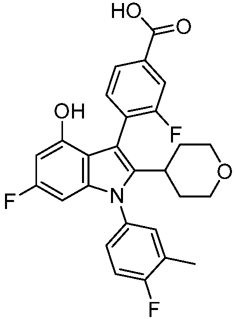 | | |
| **29** | From **S7.** See footnote for method² | 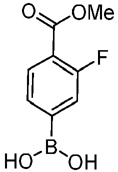 | ¹H NMR (400 MHz, DMSO-*d*₆) δ 9.90 (s, 1H), 7.80 (t, J = 7.9 Hz, 1H), 7.48 - 7.32 (m, 3H), 7.30 - 7.18 (m, 2H), 6.26 (dd, J = 11.4, 2.2 Hz, 1H), 5.96 (dd, J = 9.6, 2.2 Hz, 1H), 3.73 - 3.64 (m, 2H), 3.03 (td, J = 11.4, 3.0 Hz, 2H), 2.85 - 2.74 (m, 1H), 2.34 (d, J = 1.9 Hz, 3H), 1.61 - 1.46 (m, 4H). LCMS *m*/*z* 482.0 [M+H]⁺ |
| | 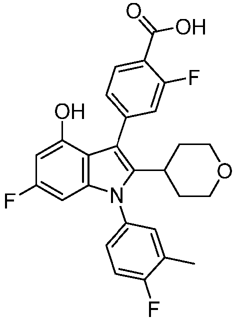 | | |
| **30** | See footnote for method^{6,7,8} | 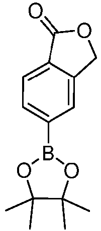 | ¹H NMR (300 MHz, Methanol-*d*₄) δ 7.86 (dd, J = 7.8, 0.8 Hz, 1H), 7.72 - 7.56 (m, 2H), 7.40 - 7.12 (m, 3H), 6.17 (dd, J = 11.2, 2.2 Hz, 1H), 5.97 (dd, J = 9.6, 2.2 Hz, 1H), 5.45 (s, 2H), 3.74 (d, J = 11.6 Hz, 2H), 3.14 (td, J = 11.4, 3.0 Hz, 2H), 2.94 - 2.81 (m, 1H), 2.38 (d, J = 2.0 Hz, 3H), 1.77 - 1.49 (m, 4H). LCMS *m*/*z* 476.22 [M+H]⁺ |
| | 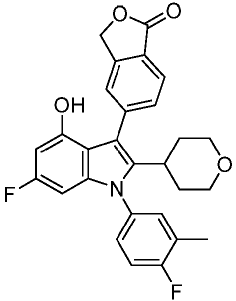 | | |
| **31** | From S8^{2,9} | 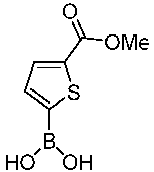 | ¹H NMR (400 MHz, DMSO-*d*₆) δ 9.34 (s, 1H), 7.61 (d, J = 3.7 Hz, 1H), 7.46 (dd, J = 6.9, 2.4 Hz, 1H), 7.43 - 7.32 (m, 2H), 7.08 (d, J = 3.7 Hz, 1H), 6.91 (dd, J = 11.2, 8.8 Hz, 1H), 6.19 (dd, J = 8.8, 3.5 Hz, 1H), 3.77 - 3.68 (m, 2H), 3.05 (td, J = 11.6, 2.5 Hz, 2H), 2.87 - 2.77 (m, 1H), 2.33 (d, J = 1.9 Hz, 3H), 1.72 - 1.55 (m, 4H). LCMS *m*/*z* 470.0 [M+H]⁺ |
| | 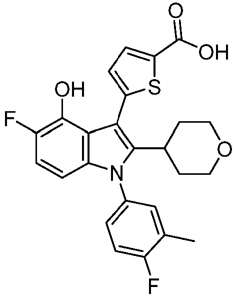 | | |
| **32** | 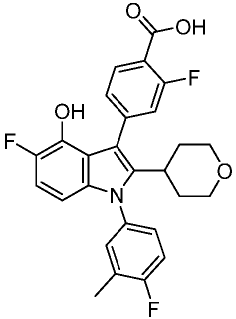 | 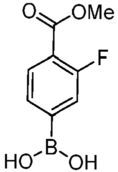 | ¹H NMR (400 MHz, DMSO-*d*₆) δ 9.39 - 9.25 (m, 1H), 7.85 (t, J = 7.9 Hz, 1H), 7.49 - 7.23 (m, 5H), 6.90 (dd, J = 11.2, 8.9 Hz, 1H), 6.19 (dd, J = 8.8, 3.5 Hz, 1H), 3.74 - 3.61 (m, 2H), 3.04 (td, J = 11.4, 3.1 Hz, 2H), 2.82 (tt, J = 9.9, 4.4 Hz, 1H), 2.33 (d, J = 1.8 Hz, 3H), 1.58 (s, 2H), 1.26 - 1.22 (m, 2H). LCMS *m*/*z* 482.0 [M+H]⁺ |
| **33** | 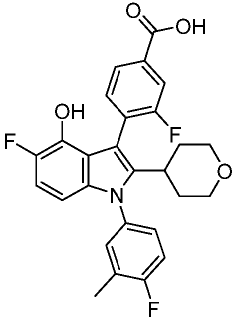 | 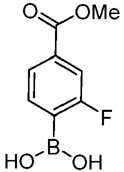 | ¹H NMR (400 MHz, DMSO-*d*₆) δ 9.16 (s, 1H), 7.73 (d, J = 7.8 Hz, 1H), 7.63 (d, J = 10.2 Hz, 1H), 7.51 - 7.30 (m, 4H), 6.88 (dd, J = 11.2, 8.8 Hz, 1H), 6.21 (ddd, J = 8.8, 3.5, 1.9 Hz, 1H), 3.68 (d, J = 13.6 Hz, 3H), 3.02 (t, J = 11.1 Hz, 2H), 2.78 - 2.63 (m, 0H), 2.35 - 2.28 (m, 3H), 1.64 - 1.42 (m, 4H). LCMS *m*/*z* 482.0 [M+H]⁺ |
| **34** | 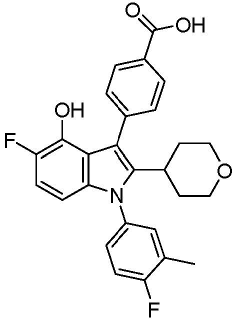 | 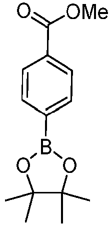 | ¹H NMR (400 MHz, DMSO-*d*₆) δ 9.19 (s, 1H), 7.98 - 7.92 (m, 2H), 7.55 - 7.49 (m, 2H), 7.46 (dd, J = 6.9, 2.4 Hz, 1H), 7.42 - 7.34 (m, 2H), 6.89 (dd, J = 11.2, 8.9 Hz, 1H), 6.18 (dd, J = 8.9, 3.5 Hz, 1H), 3.71 - 3.63 (m, 2H), 3.02 (td, J = 11.7, 11.2, 4.0 Hz, 2H), 2.85 - 2.73 (m, 1H), 2.33 (d, J = 1.8 Hz, 3H), 1.54 (dt, J = 12.3, 6.7 Hz, 4H). LCMS *m*/*z* 464.0 [M+H]⁺ |

| | | | |
|---|---|---|---|
| **^{1.}** Purification by silica gel chromatography (Eluent: 0-100% MeOH in dichloromethane) afforded the product. **^{2.}** Suzuki Conditions: Pd(dppf)Cl₂, Na₂CO₃, in DMF at 90 °C. **^{3.}** Purification by reversed-phase chromatography (Column: C18. Gradient: 5-95% MeCN in water with 0.1% trifluoroacetic acid) afforded the product. **^{4.}** Suzuki Conditions: Pd₂(dba)₃, SPhos, CsF in DME at 80 °C. **^{5.}** Purification by reversed-phase chromatography (Column: C18. Gradient: 10-100% MeCN in water with 0.1% formic acid) afforded the product. **^{6.}** Compound **30** was prepared from the MOM protected variant of S7 6-fluoro-1-(4-fluoro-3-methylphenyl)-4-(methoxymethoxy)-2-(tetrahydro-2H-pyran-4-yl)-1H-indole. 6-fluoro-1-(4-fluoro-3-methylphenyl)-4-(methoxymethoxy)-2-(tetrahydro-2H-pyran-4-yl)-1H-indole was prepared from C37 by MOM protection then iodination. **^{7.}** Purification by silica gel chromatography (Gradient: 0-100% EtOAc in heptane) yielded the product. **^{8.}** Suzuki Conditions: Pd(dppf)Cl₂, Na₂CO₃, in DMF at 90 °C. MOM group removal with HCl in 1,4-dioxane at 60 °C. **^{9.}** The product was triturated with 9:1 Heptane:EtOAc, then filtered and dried. | | | |

### Compound 35

### 6-[1-(4-fluoro-3-methyl-phenyl)-4-hydroxy-2-tetrahydropyran-4-yl-indol-3-yl]spiro[3.3]heptane-2-carboxylic acid [RAC](35)

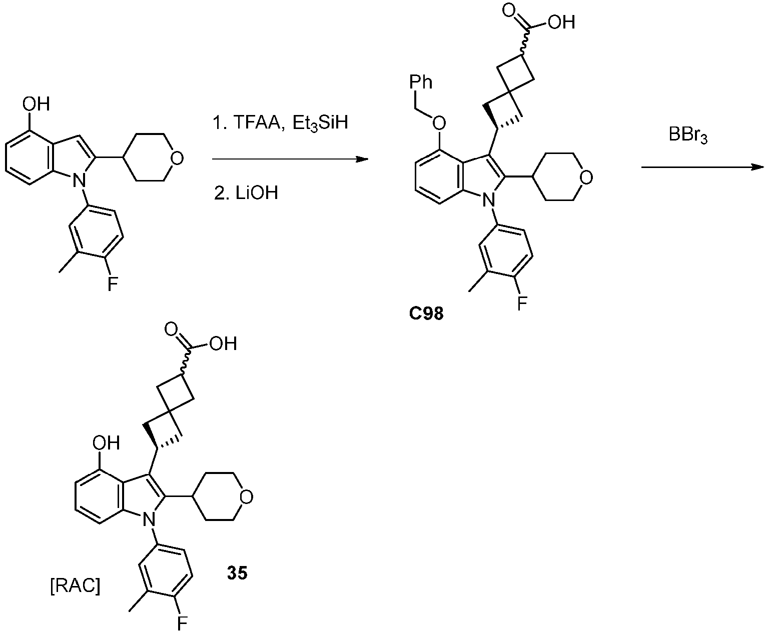

### Step 1. Synthesis of 6-[4-benzyloxy-1-(4-fluoro-3-methyl-phenyl)-2-tetrahydropyran-4-yl-indol-3-yl]spiro[3.3]heptane-2-carboxylic acid (C98)

To a solution of 4-benzyloxy-1-(4-fluoro-3-methyl-phenyl)-2-tetrahydropyran-4-yl-indole (500 mg, 1.203 mmol) and methyl 2-oxospiro[3.3]heptane-6-carboxylate (1.21 g, 7.2 mmol) in toluene (3 mL) was added Et₃SiH (1.15 mL, 7.2 mmol) followed by TFA (556 µL, 7.2 mmol). The reaction mixture was stirred overnight at 85 °C in a sealed tube. The reaction mixture was stirred at 85 °C for an additional 3 days. The mixture was then diluted with water and extracted with EtOAc. The organic layer was concentrated to dryness, dissolved in THF (2 mL), MeOH (1 mL), and water (1 mL) and treated with LiOH (265 mg, 11.07 mmol). The reaction mixture was stirred at room temperature for 2 hours, acidified with 1 M aq. HCl, and extracted with EtOAc. The organic layer was concentrated to dryness and purified via silica gel chromatography (Gradient: 0-10% MeOH in dichloromethane). 6-[4-benzyloxy-1-(4-fluoro-3-methyl-phenyl)-2-tetrahydropyran-4-yl-indol-3-yl]spiro[3.3]heptane-2-carboxylic acid (463 mg, 70%). ¹H NMR (400 MHz, Chloroform-d) δ 7.52 - 7.48 (m, 2H), 7.44 - 7.38 (m, 2H), 7.37 - 7.31 (m, 1H), 7.16 (t, J = 8.8 Hz, 1H), 7.07 (dddd, J = 15.7, 7.5, 5.3, 2.6 Hz, 2H), 6.92 (t, J = 8.0 Hz, 1H), 6.52 (dd, J = 7.9, 0.8 Hz, 1H), 6.43 (dd, J = 8.2, 0.7 Hz, 1H), 5.35 (d, J = 3.5 Hz, 2H), 4.07 - 4.00 (m, 2H), 3.32 (t, J = 11.7 Hz, 2H), 3.25 - 3.19 (m, 1H), 3.05 (p, J = 8.4 Hz, 1H), 2.89 (ddt, J = 20.8, 15.8, 7.3 Hz, 3H), 2.67 - 2.60 (m, 3H), 2.55 - 2.45 (m, 4H), 2.35 (d, J = 1.9 Hz, 3H), 2.31 - 2.23 (m, 1H), 1.96 (ddd, J = 11.6, 8.6, 3.0 Hz, 1H), 1.63 (d, J = 12.9 Hz, 2H). LCMS *m*/*z* 554.0 [M+1]⁺.

### Step 2. 6-[1-(4-fluoro-3-methyl-phenyl)-4-hydroxy-2-tetrahydropyran-4-yl-indol-3-yl]spiro[3.3]heptane-2-carboxylic acid [RAC](35)

To a solution of 6-[4-benzyloxy-1-(4-fluoro-3-methyl-phenyl)-2-tetrahydropyran-4-yl-indol-3-yl]spiro[3.3]heptane-2-carboxylic acid (60 mg, 0.11 mmol) in dichloromethane (1 mL) was added dropwise BBr₃ (120 µL of 1 M, 0.12 mmol) in dichloromethane. The reaction mixture was stirred at room temperature. The mixture was diluted with water, then the organic layer was removed, concentrated to dryness, and purified by reversed phase chromatography (Column: C18. 5-80% MeCN in water with 0.1% TFA). Fractions containing the desired product were combined, diluted with water, and extracted with EtOAc. The organic layer was concentrated to dryness and purified via silica gel chromatography (Gradient: 0-8% MeOH in dichloromethane) to afford the product. 6-[1-(4-fluoro-3-methyl-phenyl)-4-hydroxy-2-tetrahydropyran-4-yl-indol-3-yl]spiro[3.3]heptane-2-carboxylic acid (5 mg, 9%). ¹H NMR (400 MHz, Chloroform-*d*) δ 7.21 - 7.00 (m, 2H), 6.96 - 6.88 (m, 1H), 6.47 - 6.41 (m, 2H), 4.03 (dd, J = 11.5, 4.2 Hz, 2H), 3.94 (q, J = 9.4 Hz, 1H), 3.32 (t, J = 11.7 Hz, 2H), 3.16 (p, J = 8.4 Hz, 1H), 2.99 - 2.77 (m, 3H), 2.60 - 2.27 (m, 5H), 2.27 - 2.05 (m, 2H), 1.62 (d, J = 14.1 Hz, 4H), 1.28 (s, 2H). LCMS *m*/*z* 464.0 [M+1]⁺.

### Compounds 36-41

Compounds **36-41** were prepared from **S9** by reductive alkylation with triethylsilane in trifluoroacetic acid, as described in the preparation of compound **65.** Ester hydrolysis and benzyl group removal by hydrogenation afforded the product. Benzyl group removal was preformed either with hydrogen gas, or using ammonium formate as the hydrogen source.

**Table 3. Method of preparation, structure and physicochemical data for compounds 36-41**

| **Compound** | **Method/ Product** | **Aldehyde or ketone** | ¹H NMR; **LCMS m/z** [M+H]⁺ |
|---|---|---|---|
| **36** | From **S9¹** | | ¹H NMR (400 MHz, Methanol-*d*₄) δ 7.27 - 7.15 (m, 2H), 7.08 (ddd, J = 8.1, 4.5, 2.6 Hz, 1H), 6.82 (t, J = 7.9 Hz, 1H), 6.46 (dd, J = 7.6, 0.9 Hz, 1H), 6.26 (dd, J = 8.1, 0.8 Hz, 1H), 4.02 - 3.87 (m, 3H), 3.38 - 3.20 (m, 3H), 3.11 - 2.97 (m, 2H), 2.97 - 2.89 (m, 1H), 2.89 - 2.81 (m, 1H), 2.52 - 2.33 (m, 7H), 2.24 (td, J = 10.1, 9.4, 2.9 Hz, 1H), 2.14 - 2.04 (m, 2H), 1.68 - 1.57 (m, 2H). LCMS *m*/*z* 464.0 [M+H]⁺ |
| | 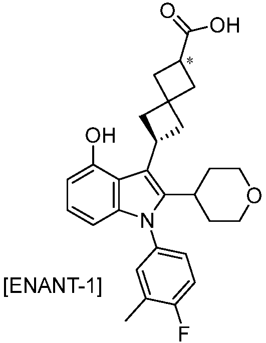 | | |
| **37** | From S9¹ | | ¹H NMR (400 MHz, Methanol-*d*₄) δ 7.22 - 7.10 (m, 2H), 7.04 (ddd, J = 8.1, 4.6, 2.6 Hz, 1H), 6.80 (t, J = 7.9 Hz, 1H), 6.42 (d, J = 7.5 Hz, 1H), 6.23 (d, J = 8.1 Hz, 1H), 4.00 - 3.88 (m, 3H), 3.36 - 3.23 (m, 3H), 3.08 - 2.97 (m, 2H), 2.97 - 2.88 (m, 1H), 2.83 (tt, J = 12.5, 3.7 Hz, 1H), 2.50 - 2.31 (m, 7H), 2.21 (ddt, J = 11.1, 8.9, 4.0 Hz, 1H), 2.08 (dddt, J = 16.7, 12.7, 8.6, 3.9 Hz, 2H), 1.66 - 1.54 (m, 2H). LCMS *m*/*z* 464.0 [M+H]⁺ |
| | 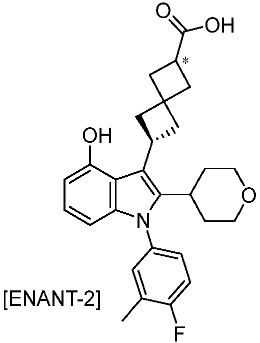 | | |
| **38** | From S9² | 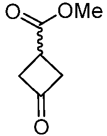 | ¹H NMR (400 MHz, Methanol-*d*₄) δ 7.12 (t, J = 9.0 Hz, 1H), 7.09 - 7.03 (m, 1H), 7.02 - 6.96 (m, 1H), 6.71 (ddd, J = 13.6, 8.2, 7.6 Hz, 1H), 6.33 (ddd, J = 20.5, 7.6, 0.8 Hz, 1H), 6.13 (ddd, J = 15.8, 8.2, 0.8 Hz, 1H), 3.85 (dd, J = 11.6, 4.2 Hz, 2H), 3.51 (q, J = 7.0 Hz, 1H), 3.17 - 2.98 (m, 3H), 2.89 - 2.67 (m, 1H), 2.45 - 2.37 (m, 2H), 2.24 (d, J = 1.9 Hz, 3H), 1.54 (t, J = 12.9 Hz, 2H), 1.14 (s, 4H). LCMS *m*/*z* 424.0 [M+H]⁺ |
| | 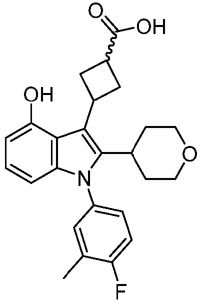 | | |
| **39** | From S9² | 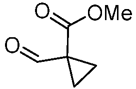 | ¹H NMR (400 MHz, DMSO-*d*₆) δ 7.35 - 7.28 (m, 2H), 7.21 (dd, J = 8.2, 4.4 Hz, 1H), 6.73 (t, J = 7.9 Hz, 1H), 6.36 (d, J = 7.5 Hz, 1H), 6.09 (d, J = 8.1 Hz, 1H), 3.80 (d, J = 10.5 Hz, 2H), 3.54 (s, 2H), 3.19 (t, J = 11.6 Hz, 2H), 2.92 (t, J = 12.4 Hz, 1H), 2.31 (d, J = 1.8 Hz, 3H), 1.73 (t, J = 13.0 Hz, 2H), 1.55 (d, J = 12.6 Hz, 2H), 0.91 (d, J = 3.2 Hz, 2H), 0.66 (s, 2H). LCMS *m*/*z* 424.0 [M+H]⁺ |
| | 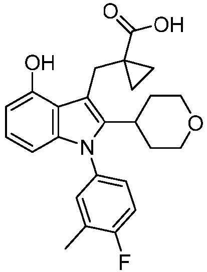 | | |
| ***40*** | From S9³ | 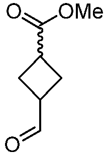 | LCMS *m*/*z* 438.0 [M+H]⁺ |
| | 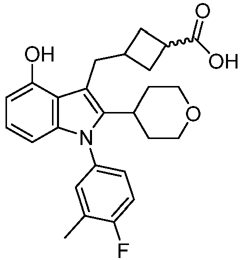 | | |
| | | | No NMR-need flow |
| ***41*** | From S9³ | 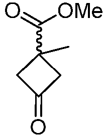 | ¹H NMR (400 MHz, Chloroform-*d*) δ 7.21 - 7.04 (m, 2H), 6.92 (dd, J = 8.2, 7.6 Hz, 1H), 6.51 - 6.39 (m, 2H), 4.08 - 4.03 (m, 3H), 3.52 - 3.43 (m, 1H), 3.35 (q, J = 11.2, 9.6 Hz, 2H), 2.92 (t, J = 10.3 Hz, 2H), 2.85 - 2.79 (m, 2H), 2.56 (t, J = 6.3 Hz, 1H), 2.42 - 2.37 (m, 1H), 2.36 (d, J = 2.0 Hz, 3H), 2.19 - 2.01 (m, 2H), 1.82 - 1.75 (m, 2H), 1.64 (d, J = 13.4 Hz, 2H). LCMS *m*/*z* 438.0 [M+H]⁺ |
| | 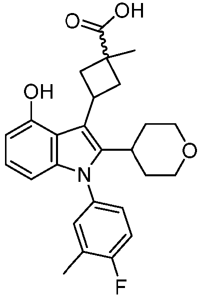 | | |

| | | | |
|---|---|---|---|
| ^{1.} Compounds **36** and **37** were prepared from **S9** as described for compound **35.** Compound **35** was separated into its constituent isomers by chiral SFC to afford compound **36** and **37.** ^{2.} Benzyl group remove with Pd/C and ammonium formate in EtOH ^{3.} Removal of benzyl group with BBr₃ as described for the preparation of compound **35.** | | | |

### Compounds 42-46

Compounds **42-46** were prepared from **S10** according to the method described for the preparation of compound 1.

**Table 4. Method of preparation, structure and physicochemical data for compounds 42-46**

| **Compound** | **Method/Product** | **Boronic acid or ester** | ¹H NMR; **LCMS m/z** [M+H]⁺ |
|---|---|---|---|
| **42** | From **S10** as for compound **1** | 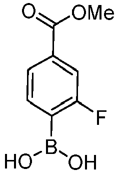 | ¹H NMR (400 MHz, Chloroform-*d*) δ 7.81 (d, J = 7.4 Hz, 1H), 7.73 (d, J = 9.1 Hz, 1H), 7.45 (q, J = 6.9, 6.4 Hz, 1H), 7.14 (tt, J = 12.8, 5.9 Hz, 3H), 6.84 (q, J = 7.3, 6.7 Hz, 1H), 6.34 (dt, J = 14.1, 6.5 Hz, 2H), 3.71 (s, 2H), 3.10 (t, J = 11.7 Hz, 2H), 2.71 (d, J = 11.6 Hz, 1H), 2.29 (d, J = 5.2 Hz, 3H), 1.58 (qd, J = 13.0, 7.1 Hz, 4H). LCMS *m*/*z* 464.37 [M+H]⁺. |
| | 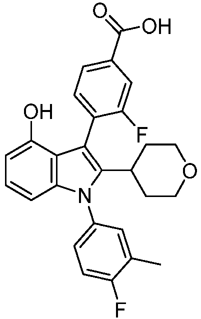 | | |
| **43** | From **S10** as for compound **1** | 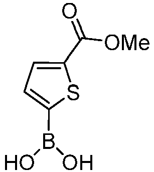 | ¹H NMR (400 MHz, DMSO-*d*₆) δ 9.25 (s, 1H), 7.49 - 7.29 (m, 4H), 6.97 (d, J = 3.6 Hz, 1H), 6.84 (t, J = 7.9 Hz, 1H), 6.40 (d, J = 7.6 Hz, 1H), 6.21 (d, J = 8.2 Hz, 1H), 3.73 (d, J = 11.6 Hz, 2H), 3.05 (td, J = 11.7, 2.2 Hz, 2H), 2.85 (td, J = 11.9, 5.9 Hz, 1H), 2.33 (d, J = 1.9 Hz, 3H), 1.69 (qd, J = 12.3, 4.3 Hz, 2H), 1.57 (t, J = 10.3 Hz, 2H). LCMS *m*/*z* 452.0 [M+H]⁺. |
| | 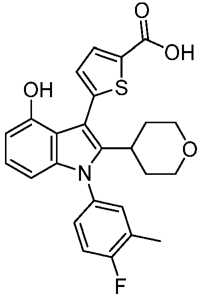 | | |
| **44** | From **S10** as for compound **1** | 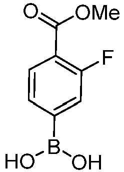 | ¹H NMR (400 MHz, Chloroform-*d*) δ 7.92 (q, J = 7.5 Hz, 1H), 7.33 - 7.20 (m, 3H), 7.22 - 7.08 (m, 2H), 6.89 (dt, J = 15.1, 7.9 Hz, 1H), 6.47 - 6.36 (m, 1H), 6.38 - 6.28 (m, 1H), 3.75 (d, J = 11.5 Hz, 2H), 3.32 (d, J = 11.8 Hz, 2H), 3.13 (q, J = 11.5 Hz, 2H), 2.90 - 2.76 (m, 1H), 2.31 (d, J = 11.0 Hz, 3H), 1.65 (qt, J = 12.3, 6.5 Hz, 2H), 1.50 (s, 2H). LCMS *m*/*z* 464.37 [M+H]⁺. |
| | 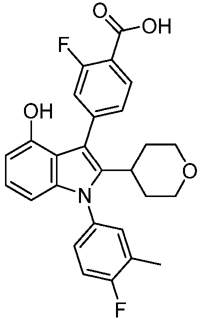 | | |
| **45** | From **S10** as for compound **1** | 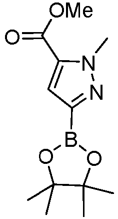 | LCMS *m*/*z* 450.0 [M+H]⁺. |
| | 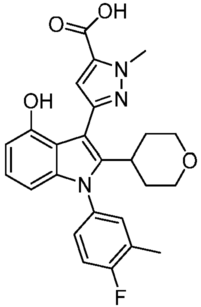 | | |
| **46** | From **S10** as for compound 1 | 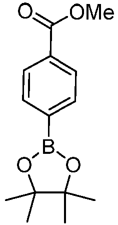 | LCMS *m*/*z* 446.1 [M+H]⁺. |
| | 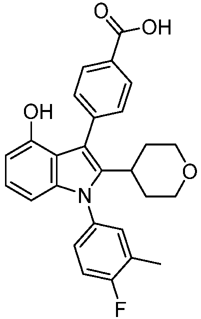 | | |

### Compound 47

### 4-(6-chloro-1-(4fluoro-3-methylphenyl)-4-hydroxy-2-(tetrahydro-2Hpyran-4yl)-1H-indol-3-yl)benzoic acid (47)

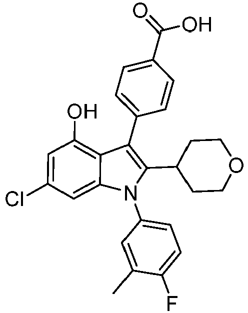

Compound **47** was prepared from 6-chloro-1-(4-fluoro-3-methyl-phenyl)-4-methoxy-2-tetrahydropyran-4-yl-indole. 6-chloro-1-(4-fluoro-3-methyl-phenyl)-4-methoxy-2-tetrahydropyran-4-yl-indole was prepared in three steps from 1-bromo-5-chloro-2-iodo-3-methoxy-benzene as described for the synthesis of **C36** in the preparation of **S7.** Iodination with N-iodosuccinimide, Suzuki coupling, and finally removal of the methyl ester and methoxy groups using AlCl₃ and octane-thiol, as described using the method described in the preparation **of C37.** Purification by reversed-phase chromatography (Column: C18. Gradient: 40-100% MeCN in water with 0.1% formic acid) afforded compound **47.** ¹H NMR (400 MHz, DMSO-*d*₆) δ 12.87 (s, 1H), 9.70 (s, 1H), 8.00 - 7.88 (m, 2H), 7.55 - 7.45 (m, 2H), 7.45 - 7.32 (m, 2H), 6.40 (d, J = 1.8 Hz, 1H), 6.20 (d, J = 1.7 Hz, 1H), 3.66 (d, J = 11.2 Hz, 2H), 3.50 - 3.38 (m, 1H), 3.01 (t, J = 10.4 Hz, 2H), 2.85 - 2.72 (m, 1H), 2.34 (d, J = 1.9 Hz, 3H), 1.61 - 1.40 (m, 4H). LCMS *m*/*z* 479.99 [M+H]⁺.

### Compound 48

### 4-[1-(4-fluoro-3-methyl-phenyl)-4-hydroxy-2-isopropylsulfonyl-indol-3-yl]benzoic acid (48)

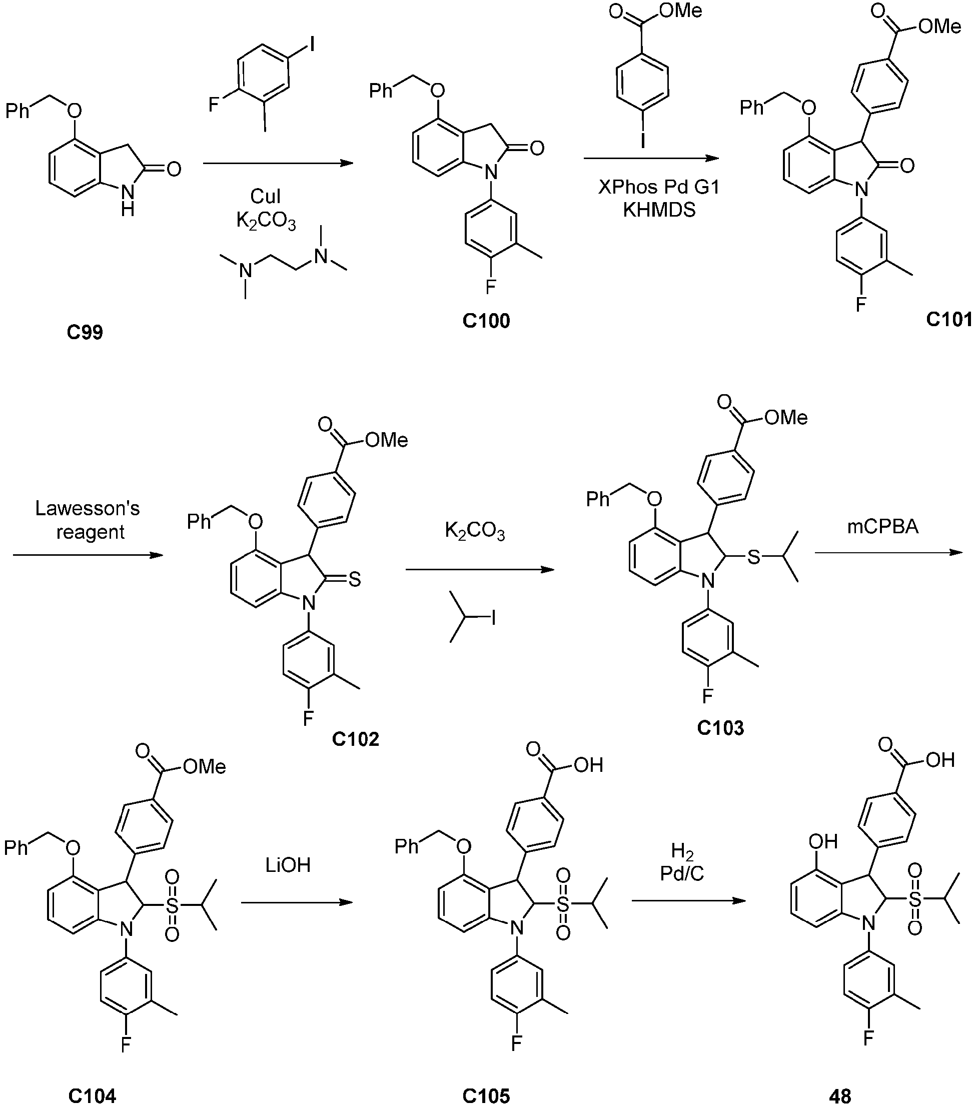

### Step 1. Synthesis of 4-benzyloxy-1-(4-fluoro-3-methyl-phenyl)indolin-2-one (C100)

A vial was charged with 4-benzyloxyindolin-2-one **C99** (1 g, 4.18 mmol), CuI (80 mg, 0.42 mmol), 1-fluoro-4-iodo-2-methyl-benzene (1.18 g, 5.0 mmol), N,N'-dimethylethane-1,2-diamine (90 µL, 0.85 mmol), K₂CO₃ (1.27 g, 9.19 mmol) and MeCN (20 mL). The reaction was heated to 80°C under a nitrogen atmosphere overnight. The mixture was diluted with dichloromethane and filtered through Celite^{®} (washing with dichloromethane) and then concentrated. The filtrate was purified by silica gel chromatography (Gradient: 0-20% MeOH in dichloromethane). Heptane was added to the product and a pale orange solid collected by filtration to afford the product. 4-benzyloxy-1-(4-fluoro-3-methyl-phenyl)indolin-2-one (900 mg, 62%). The product was then further purified by reversed-phase chromatography (Column: C18. Gradient: 0-100% MeCN in water with 0.2% trifluoroacetic acid) to afford 4-benzyloxy-1-(4-fluoro-3-methyl-phenyl)indolin-2-one. ¹H NMR (400 MHz, Chloroform-*d*) δ 7.56 - 7.34 (m, 5H), 7.27 - 7.11 (m, 4H), 6.71 (d, J = 8.3 Hz, 1H), 6.44 - 6.36 (m, 1H), 5.19 (s, 2H), 3.69 (s, 2H), 2.35 (d, J = 2.1 Hz, 3H).

### Step 2. Synthesis of methyl 4-[4-benzyloxy-1-(4-fluoro-3-methyl-phenyl)-2-oxo-indolin-3-yl]benzoate (C101)

A vial was charged with 4-benzyloxy-1-(4-fluoro-3-methyl-phenyl)indolin-2-one **C100** (80 mg, 0.23 mmol), methyl 4-bromobenzoate (55 mg, 0.26 mmol), XPhos Pd G1 (10 mg, 0.014 mmol) and THF (2 mL). KHMDS (500 µL of 0.5 M, 0.25 mmol) was added at room temperature. The reaction was heated to 80°C. Aqueous saturated NH₄Cl and water were added and the layers separated. The aqueous layer was extracted with EtOAc (x 2) and the combined organics were concentrated to give the product which was used in the subsequent step without further purification. Methyl 4-[4-benzyloxy-1-(4-fluoro-3-methyl-phenyl)-2-oxo-indolin-3-yl]benzoate (110.9 mg, 100%). LCMS *m*/*z* 482.07 [M+H]⁺.

### Step 3. Synthesis of methyl 4-[4-benzyloxy-1-(4-fluoro-3-methyl-phenyl)-2-thioxo-indolin-3-yl]benzoate (C102)

To a solution of methyl 4-[4-benzyloxy-1-(4-fluoro-3-methyl-phenyl)-2-oxo-indolin-3-yl]benzoate **C101** (63 mg, 0.13 mmol) in THF (2 mL) was added Lawesson's reagent (58 mg, 0.14 mmol) and heated at 50 °C, then 70 °C. The reaction mixture was concentrated, and purified by silica gel chromatography (Gradient: 0-75% EtOAc in heptane) to afford the product. Methyl 4-[4-benzyloxy-1-(4-fluoro-3-methyl-phenyl)-2-thioxo-indolin-3-yl]benzoate (52 mg, 80%). LCMS *m*/*z* 498.09 [M+H]⁺.

### Step 4. Synthesis of methyl 4-[4-benzyloxy-1-(4-fluoro-3-methyl-phenyl)-2-isopropylsulfanyl-indol-3-yl]benzoate (C103)

To a solution of methyl 4-[4-benzyloxy-1-(4-fluoro-3-methyl-phenyl)-2-thioxo-indolin-3-yl]benzoate **C102** (52 mg, 0.10 mmol) in acetone (4 mL) was added potassium carbonate (17 mg, 0.12 mmol) followed by 2-iodopropane (11 µL, 0.11 mmol) at room temperature. The reaction was concentrated, then diluted with EtOAc and water. The layers were separated and the aqueous layer was re-extracted with EtOAc. The combined organics were dried (Na₂SO₄), filtered and concentrated and used directly in the next step without further purification. Methyl 4-[4-benzyloxy-1-(4-fluoro-3-methyl-phenyl)-2-isopropylsulfanyl-indol-3-yl]benzoate (56 mg, 99%). LCMS *m*/*z* 540.12 [M+H]⁺.

### Step 5. Synthesis of methyl 4-[4-benzyloxy-1-(4-fluoro-3-methyl-phenyl)-2-isopropylsulfonyl-indol-3-yl]benzoate (C104)

To a solution of methyl 4-[4-benzyloxy-1-(4-fluoro-3-methyl-phenyl)-2-isopropylsulfanyl-indol-3-yl]benzoate **C103** (56 mg, 0.10 mmol) in dichloromethane (2 mL) was added 3-chlorobenzenecarboperoxoic acid (56 mg, 0.25 mmol). A solution of saturated aqueous sodium bicarbonate was added, and the layers separated. The aqueous layer was extracted with dichloromethane, and the combined organic layers were concentrated. Purification by column chromatography (Gradient: 0-75% EtOAc in heptane) to afford the product as a yellow solid. Methyl 4-[4-benzyloxy-1-(4-fluoro-3-methyl-phenyl)-2-isopropylsulfonyl-indol-3-yl]benzoate (10 mg, 17%). LCMS *m*/*z* 572.07 [M+H]⁺.

### Step 6. Synthesis of 4-[4-benzyloxy-1-(4-fluoro-3-methyl-phenyl)-2-isopropylsulfonyl-indol-3-yl]benzoic acid (C105)

To a solution of methyl 4-[4-benzyloxy-1-(4-fluoro-3-methyl-phenyl)-2-isopropylsulfonyl-indol-3-yl]benzoate **C104** (10 mg, 0.017 mmol) in THF (1.5 mL), MeOH (0.25 mL) was added LiOH (500 µL of 1 M, 0.5 mmol) and the mixture heated at 80 °C. HCl (400 µL of 2 M, 0.8 mmol) was added, and then extracted with EtOAc (x 3). The organic layer was concentrated to dryness and used in the next step without purification. 4-[4-benzyloxy-1-(4-fluoro-3-methyl-phenyl)-2-isopropylsulfonyl-indol-3-yl]benzoic acid (9.7 mg, 99%) LCMS *m*/*z* 558.07 [M+H]⁺.

### Step 7. Synthesis of 4-[1-(4-fluoro-3-methyl-phenyl)-4-hydroxy-2-isopropylsulfonyl-indol-3-yl]benzoic acid (48)

A solution of 4-[4-benzyloxy-1-(4-fluoro-3-methyl-phenyl)-2-isopropylsulfonyl-indol-3-yl]benzoic acid **C105** (9.7 mg, 0.02 mmol) in MeOH (3 mL) was stirred in the presence of 10% Pd/C (10 mg, Degussa, wet) under an atmosphere of hydrogen gas for 30 minutes. The reaction mixture was filtered through Celite^{®}, washing with MeOH. The filtrate was concentrated, and then purified by reversed-phase chromatography (Column: C18. Gradient: 0-100% MeCN in water with 0.2% trifluoroacetic acid) to afford the product as a white solid. 4-[1-(4-fluoro-3-methyl-phenyl)-4-hydroxy-2-isopropylsulfonyl-indol-3-yl]benzoic acid (1.5 mg, 18%). ¹H NMR (400 MHz, Methanol-*d*₄) δ 8.01 (d, J = 8.4 Hz, 2H), 7.58 (d, J = 8.0 Hz, 2H), 7.39 - 7.34 (m, 1H), 7.31 (dd, J = 8.5, 4.1 Hz, 1H), 7.23 (t, J = 8.9 Hz, 1H), 7.13 (dd, J = 8.5, 7.7 Hz, 1H), 6.47 (dd, J = 7.7, 0.7 Hz, 1H), 6.40 (dd, J = 8.4, 0.7 Hz, 1H), 2.73 (sept, J = 6.8 Hz, 1H), 2.36 (d, J = 2.0 Hz, 3H), 1.05 (d, J = 6.8 Hz, 6H). LCMS *m*/*z* 468.12 [M+H]⁺.

### Compound 49 and Compound 50

### 3-[1-(4-fluoro-3-methyl-phenyl)-4-hydroxy-2-(2-hydroxy-2-methyl-propyl)indol-3-yl]cyclobutanecarboxylic acid [CIS] (49) and 3-[1-(4-fluoro-3-methyl-phenyl)-4-hydroxy-2-(2-hydroxy-2-methyl-propyl)indol-3-yl]cyclobutanecarboxylic acid [TRANS] (50)

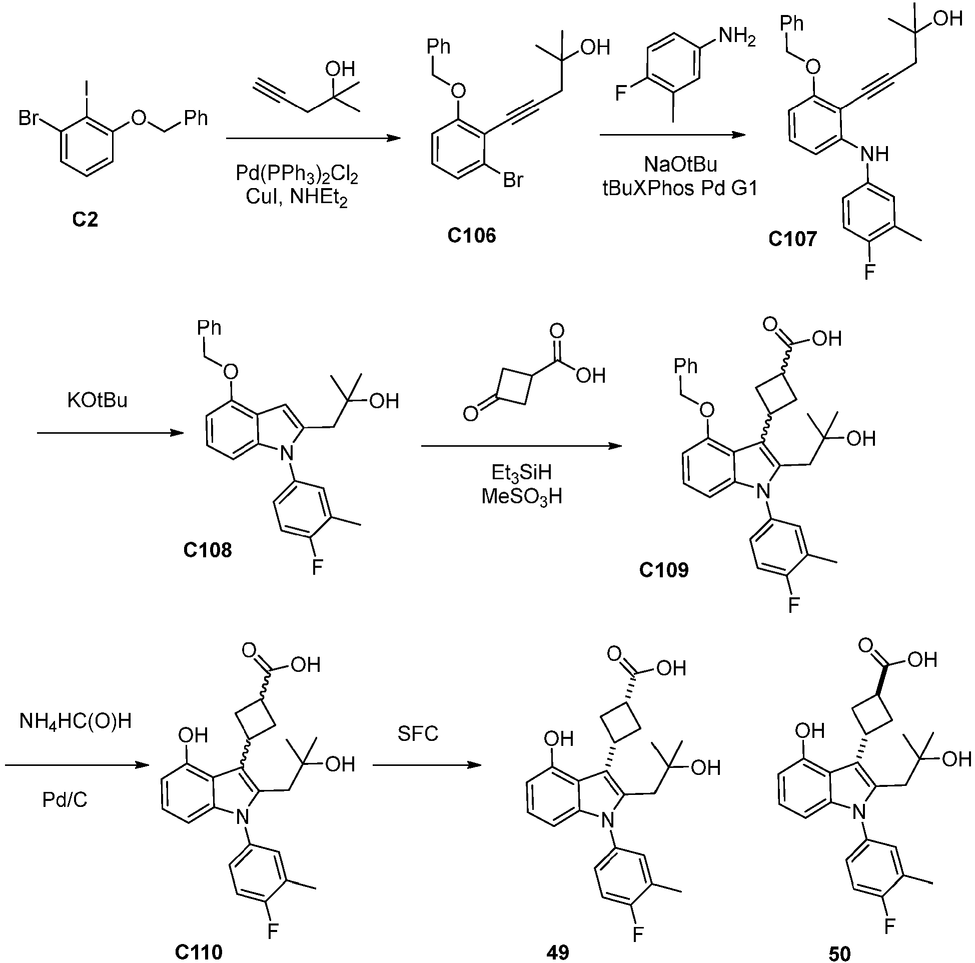

### Step 1. Synthesis of 5-(2-benzyloxy-6-bromo-phenyl)-2-methyl-pent-4-yn-2-ol (C106)

Compound ***C106** was* prepared from 1-benzyloxy-3-bromo-2-iodo-benzene C2 (3.51 g, 9.02 mmol) and 2-methylpent-4-yn-ol using Sonagashira coupling conditions as described in the synthesis of **C17** in the synthesis of **S4**. Diethylamine was used as the base. The product was purified by reversed-phase chromatography (Column: C18. Gradient: 5-95% MeCN in water with 0.1% trifluoroacetic acid). The product solution in water was extracted with ethyl acetate (2 x 100 mL). The organic layers were combined and dried over sodium sulfate and then concentrated under reduced pressure to afford the product as a yellowish brown solid. 5-(2-benzyloxy-6-bromo-phenyl)-2-methyl-pent-4-yn-2-ol (2.01 g, 62%). ¹H NMR (400 MHz, Chloroform-d) δ 7.40 - 7.20 (m, 5H), 7.11 (dd, J = 8.1, 1.0 Hz, 1H), 6.98 (t, J = 8.2 Hz, 1H), 6.77 (dd, J = 8.4, 1.0 Hz, 1H), 5.06 (s, 2H), 2.61 (s, 2H), 2.20 (s, 1H), 1.27 (s, 6H).

### Step 2. Synthesis of 5-[2-benzyloxy-6-(4-fluoro-3-methyl-anilino)phenyl]-2-methyl-pent-4-yn-2-of (C107)

Compound **C107** was prepared by coupling of 5-(2-benzyloxy-6-bromo-phenyl)-2-methyl-pent-4-yn-2-ol (2.01 g, 5.60 mmol) **C106** with 4-fluoro-2-methyl-aniline using the method described for the synthesis of **S7.** tBuXPhos Pd G1 catalyst was used. Purification by silica gel chromatography (Gradient: 0-25% EtOAc in heptane) yielded the product **C107** as a mixture with the cyclized indole product **C108.** The mixture was advanced to the next step without further purification.

### Step 3. Synthesis of 1-[4-benzyloxy-1-(4-fluoro-3-methyl-phenyl)indol-2-yl]-2-methyl-propan-2-of (C108)

To a mixture of **C107** and **C108** (2.26 g, 5.60 mmol) from the previous reaction in 2-MeTHF (20 mL) was added KOtBu (5.6 mL of 1 M, 5.6 mmol) and allowed to stir overnight at room temperature. Ethyl acetate and water were added, followed by brine (20 mL) and saturated ammonium chloride. The organic layer was dried over sodium sulfate and concentrated under reduced pressure. Silica gel chromatography (0-100% ethyl acetate in heptane) afforded the product (~ 80% purity by NMR). The product was used in the subsequent reaction without additional purification. 1-[4-benzyloxy-1-(4-fluoro-3-methyl-phenyl)indol-2-yl]-2-methyl-propan-2-ol (980 mg, 43%). ¹H NMR (400 MHz, Chloroform-d) δ 7.56 - 7.51 (m, 2H), 7.44 - 7.31 (m, 3H), 7.13 (td, J = 5.6, 3.0 Hz, 2H), 7.02 (t, J = 8.0 Hz, 1H), 6.75 - 6.71 (m, 1H), 6.67 (d, J = 8.3 Hz, 1H), 6.63 (d, J = 7.8 Hz, 1H), 5.25 (s, 2H), 2.84 (s, 2H), 2.34 (d, J = 2.0 Hz, 3H), 2.19 (d, J = 2.0 Hz, 1H), 1.71 (s, 1H), 1.12 (d, J = 1.6 Hz, 6H). LCMS *m*/*z* 404.27 [M+H]⁺.

### Step 4. Synthesis of 3-[4-benzyloxy-1-(4-fluoro-3-methyl-phenyl)-2-(2-hydroxy-2-methylpropyl)indol-3-yl]cyclobutanecarboxylic acid (C109)

To a mixture of 1-[4-benzyloxy-1-(4-fluoro-3-methyl-phenyl)indol-2-yl]-2-methyl-propan-2-ol **(C108)** (95 mg, 0.24 mmol) and 3-oxocyclobutanecarboxylic acid (67 mg, 0.59 mmol) in dichloroethane (500 µL) was added methanesulfonic acid (26 µL, 0.40 mmol) then triethylsilane (94 µL, 0.59 mmol) and the resulting dark solution stirred at room temperature for 1 hour. Purification by silica gel chromatography (12g column. Gradient: 10-100% EtOAc in heptane) afforded the product **C109** 3-[4-benzyloxy-1-(4-fluoro-3-methyl-phenyl)-2-(2-hydroxy-2-methyl-propyl)indol-3-yl]cyclobutanecarboxylic acid (58 mg, 49%) as a straw colored oil, which was advanced to the next step without further purification. LCMS *m*/*z 502.56* [M+H]⁺. A spirocyclic by-product 9-benzyloxy-5-(4-fluoro-3-methyl-phenyl)-3,3-dimethyl-spiro[4H-pyrano[4,3-b]indole-1,3'-cyclobutane]-1'-carboxylic acid (58 mg, 49%) was also observed. LCMS *m*/*z* 500.58 [M+H]⁺. *Step 5. Synthesis of 3-[1-(4-fluoro-3-methyl-pheny)-4-hydroxy-2-(2-hydroxy-2-methylpropyl)indol-3-yl]cyclobutanecarboxylic acid **(49)*** and *3-[1-(4-fluoro-3-methyl-phenyl)-4-hydroxy-2-(2-hydroxy-2-methyl-propyl)indol-3-yl [cyclobutanecarboxylic acid **(50)***

A flask was charged with 3-[4-benzyloxy-1-(4-fluoro-3-methyl-phenyl)-2-(2-hydroxy-2-methyl-propyl)indol-3-yl]cyclobutanecarboxylic acid **C109** (58 mg, 0.12 mmol), ammonium formate (100 mg, 1.58 mmol), 10% Pd/C (wet, Degussa type, around 50 mg) and EtOH (4 mL) were stirred at room temperature for 30 minutes, with a balloon attached to avoid overpressurizing.

The reaction mixture was filtered through Celite^{®} with the aid of MeOH and then concentrated. Water and dichloromethane were added and the layers separated. Purification by column chromatography (4g column. Gradient: 0-10% MeOH in dichloromethane) afforded racemic mixture **C110.** The mixture was purified by SFC chromatography. Column: Phenomenex Lux^{®} Cellulose-2, 20 x 250 mm. Mobile phase: 40% MeOH (containing 5 mM Ammonia), 60% CO₂. Flow: 75 mL/min. SFC chromatography afforded cis product **49** and trans product **50.** 3-[1-(4-fluoro-3-methyl-phenyl)-4-hydroxy-2-(2-hydroxy-2-methylpropyl)indol-3-yl]cyclobutanecarboxylic acid **49** (3.2 mg, 6%). ¹H NMR (400 MHz, Methanol-*d*₄) δ 7.22 - 7.06 (m, 3H), 6.84 - 6.79 (m, 1H), 6.42 (td, J = 7.9, 0.8 Hz, 2H), 3.79 - 3.67 (m, 1H), 3.20 - 3.08 (m, 3H), 2.94 (s, 2H), 2.54 - 2.45 (m, 2H), 2.32 (d, J = 1.9 Hz, 3H), 1.00 (s, 3H), 0.99 (s, 3H). LCMS *m*/*z* 412.15 [M+H]⁺. 3-[1-(4-fluoro-3-methyl-phenyl)-4-hydroxy-2-(2-hydroxy-2-methyl-propyl)indol-3-yl]cyclobutanecarboxylic acid **50** (2.3 mg, 4%). ¹H NMR (400 MHz, Methanol-*d*₄) δ 7.21 - 7.07 (m, 3H), 6.87 - 6.82 (m, 1H), 6.45 (ddd, J = 17.5, 7.9, 0.9 Hz, 2H), 4.17 - 4.04 (m, 1H), 3.24 - 3.07 (m, 3H), 2.91 (s, 2H), 2.49 (tt, J = 9.5, 2.4 Hz, 2H), 2.32 (d, J = 1.9 Hz, 3H), 0.99 (s, 3H), 0.98 (s, 3H). LCMS *m*/*z* 412.19 [M+H]⁺.

### Compound 51 and Compound 52

### 2-[3-[1-(4-fluoro-3-methyl-phenyl)-4-hydroxy-2-isopropyl-indol-3-yl]cyclohexyl]acetic acid [TRANS-RAC] (51) and 2-[3-[1-(4-fluoro-3-methyl-phenyl)-2-isopropyl-4-methoxy-indol-3-yl]cyclohexyl]acetic acid [CIS-RAC] (52)

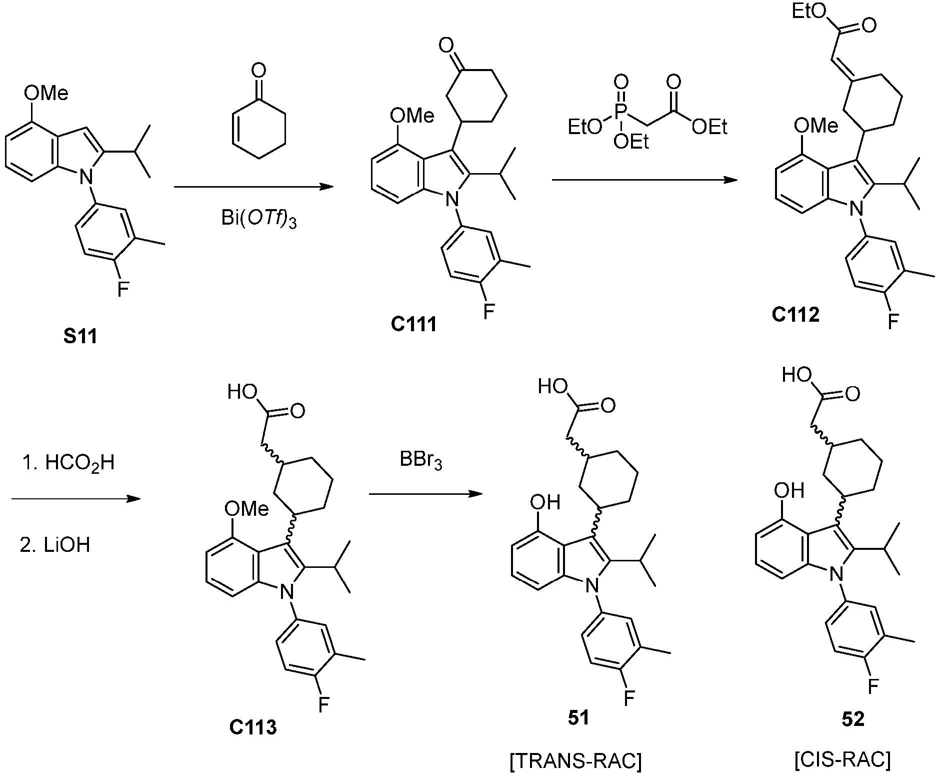

### Step 1. Synthesis of 3-[1-(4-fluoro-3-methyl-phenyl)-2-isopropyl-4-methoxy-indol-3-yl]cyclohexanone (C111)

Bismuth 2-methylpropane-2-sulfonate (315 mg, 0.51 mmol) was added to a stirred suspension of 1-(4-fluoro-3-methyl-phenyl)-2-isopropyl-4-methoxy-indole S11 (630 mg, 2.10 mmol) and cyclohex-2-en-1-one (310 mg, 3.23 mmol) in CH₃CN (10 mL) at room temperature for 1.5 hours. The solution was stirred at room temperature for 3 hours. The solvent was removed under reduced pressure, the crude product was dissolved in EtOAc (10 mL) and washed with water. The organic layer was dried and concentrated under reduced pressure. The crude product was purified by silica gel column chromatography (Gradient: 0-40% EtOAc/heptanes) to afford 3-[1-(4-fluoro-3-methyl-phenyl)-2-isopropyl-4-methoxy-indol-3-yl]cyclohexanone (560 mg, 67%) as a white foam. ¹H NMR (400 MHz, Chloroform-*d*) δ 7.20 - 7.02 (m, 3H), 7.03 (t, J = 8.0 Hz, 1H), 6.59 (dd, J = 7.9, 0.8 Hz, 1H), 6.52 (dd, J = 8.2, 0.7 Hz, 1H), 4.02 (s, 3H), 3.54 - 3.32 (m, 2H), 3.05 - 2.87 (m, 1H), 2.63-2.62 (m, 1H), 2.56 - 2.47 (m, 3H), 2.40 - 2.27 (s, 3H), 2.27 - 2.12 (m, 1H), 1.91 - 1.70 (m, 2H), 1.28 (dt, J = 7.3, 1.8 Hz, 6H). LCMS *m*/*z* 394.51 [M+H]⁺.

### Step 2. Synthesis of ethyl (2E)-2-[3-[1-(4-fluoro-3-methyl-phenyl)-2-isopropyl-4-methoxy-indol-3-yl]cyclohexylidene]acetate (C112)

KOtBu (300 mg, 2.67 mmol) was added to a solution of ethyl 2-diethoxyphosphorylacetate (570 µL, 2.87 mmol) in THF (10 mL) at room temperature for 30 minutes. A solution of 3-[1-(4-fluoro-3-methyl-phenyl)-2-isopropyl-4-methoxy-indol-3-yl]cyclohexanone **C111** (480 mg, 1.21 mmol) in THF (5 mL) was added dropwise. The reaction mixture was stirred at room temperature for 3 hours. The solvent was evaporated under reduced pressure and the residue was dissolved in water (10 mL). The aqueous layer was extracted with EtOAc (2 x 10 mL) and the combined organic phases were dried over Na₂SO₄, filtered and evaporated. The crude product was purified by flash chromatography. Purification by silica gel chromatography (Gradient: 0-40% EtOAc in heptane) yielded the product ethyl (2E)-2-[3-[1-(4-fluoro-3-methyl-phenyl)-2-isopropyl-4-methoxy-indol-3-yl]cyclohexylidene]acetate (504 mg, 88%) as a white foam. ¹H NMR (400 MHz, Chloroform-*d*) δ 7.18 - 7.08 (m, 3H), 7.03 - 6.95 (m, 1H), 6.59 - 6.55 (m, 1H), 6.51 (d, J = 8.1 Hz, 1H), 5.70 (d, J = 4.1 Hz, 1H), 4.27 - 4.10 (m, 2H), 3.99 (s, 3H), 3.28 (dt, J = 39.3, 12.7 Hz, 2H), 3.04 - 2.82 (m, 1H), 2.49-2.47 (m, 1H), 2.36 (m, , 2H), 2.29 (d, J = 12.6 Hz, 1H), 2.16 - 1.91 (m, 2H), 1.78 (d, J = 13.1 Hz, 1H), 1.56-1.53 (m, 1H), 1.37 - 1.22 (m, 9H). LCMS *m*/*z* 463.68 [M+H]⁺.

### Step 3. Synthesis of 2-[3-[1-(4-fluoro-3-methyl-phenyl)-2-isopropyl-4-methoxy-indol-3-yl]cyclohexyl]acetic acid (C113)

**Part A.** Ammonium formate (625 mg, 9.91 mmol) was added to stirred solution of ethyl (2E)-2-[3-[1-(4-fluoro-3-methyl-phenyl)-2-isopropyl-4-methoxy-indol-3-yl]cyclohexylidene]acetate **C112** (460 mg, 0.99 mmol) in EtOH (10 mL) nitrogen purged. The solution was heated at 60 °C for 1 hr. The reaction mixture was filtered. The filtrate was concentrated and the crude product was dissolved in EtOAc (10 mL) and washed with water. The organic layer was dried over sodium sulfate, filtered and concentrated to afford ethyl 2-[3-[1-(4-fluoro-3-methyl-phenyl)-2-isopropyl-4-methoxy-indol-3-yl]cyclohexyl]acetate (440 mg, 95%) (~1: 1 (cis/trans) ratio by LCMS).

**Part B.** LiOH (240 mg, 10.0 mmol) was added to a stirred solution of ethyl 2-[3-[1-(4-fluoro-3-methyl-phenyl)-2-isopropyl-4-methoxy-indol-3-yl]cyclohexyl]acetate (440 mg) in MeOH (7 mL), THF (2 mL) and H₂O (1 mL).The reaction was stirred at room temperature for 18 hours. The solvent was removed under reduced pressure. The crude product was dissolved in water (5 mL) and acidified with 6 M HCl. The aqueous layer was extracted with EtOAc (2 x 5 mL). The combined organic layers were dried and concentrated to afford 2-[3-[1-(4-fluoro-3-methyl-phenyl)-2-isopropyl-4-methoxy-indol-3-yl]cyclohexyl]acetic acid (400 mg, 81%) as white solid (as cis and trans (1:1) mixture).

¹H NMR (400 MHz, Chloroform-d) δ 7.08 - 6.94 (m, 3H), 6.89 (t, J = 8.0 Hz, 1H), 6.44 (dd, J = 7.8, 2.1 Hz, 1H), 6.39 (d, J = 8.2 Hz, 1H), 4.13 - 3.96 (m, 2H), 3.88 (d, J = 8.2 Hz, 3H), 3.00 -2.97(m, J = 7.4 Hz, 1H), 2.86-2.84(m, 1H), 2.58 - 2.41 (m, 2H), 2.25 (d, J = 2.0 Hz, 3H), 2.21 - 2.04 (m, 2H), 1.98 - 1.68 (m, 2H), 1.65 - 1.32 (m, 4H), 1.25-1.16 (m, 9H). LCMS *m*/*z* 438.61 [M+H]⁺. *Step 4. Synthesis of 2-[(1S, 3R)-3-[1-(4-fluoro-3-methyl-phenyl)-4-hydroxy-2-isopropyl-indol-3-yl]cyclohexyl]acetic acid and 2-[(1R, 3R)-3-[1-(4-fluoro-3-methyl-phenyl)-4-hydroxy-2-isopropyl-indol-3-yl]cyclohexyl]acetic acid*

BBr₃ (3 mL of 1 M, 3.0 mmol) was added to a stirred solution of mixture of 2-[3-[1-(4-fluoro-3-methyl-phenyl)-2-isopropyl-4-methoxy-indol-3-yl]cyclohexyl]acetic acid **C113** (454 mg, 0.91 mmol) (cis/trans) in dichloromethane (6 mL) at 0 °C and the solution was stirred at room temperature for 6 hours. The mixture was washed with water, dried and concentrated. Purification by reversed-phase HPLC. Method: C18 Waters Sunfire column (30 x 150 mm, 5 micron). Gradient: MeCN in H₂O with 0.1% trifluoroacetic acid afforded racemic trans isomer 51 and racemic cis isomer **52.**

Trans isomer compound **51** was the more polar product. 2-[3-[1-(4-fluoro-3-methylphenyl)-4-hydroxy-2-isopropyl-indol-3-yl]cyclohexyl]acetic acid [TRANS-RAC] (62 mg, 16%). ¹H NMR (400 MHz, DMSO-*d*₆) δ 12.02 (s, 1H), 9.59 (s, 1H), 7.40 - 7.26 (m, 2H), 7.19-7.17 (m, 1H), 6.74 (t, J = 7.9 Hz, 1H), 6.38 (dt, J = 7.7, 1.3 Hz, 1H), 6.17 (d, J = 8.1 Hz, 1H), 3.13 (brs, 1H), 2.85 (brs, 1H), 2.53-2.51(m,2H), 2.49 - 2.32 (m, 2H), 2.31(d, J = 4 Hz, 3H), 2.1 (s, 2H), 1.59-1.41 (m, 4H), 1.27-1.23 (m, 6H). LCMS *m*/*z* 424.66 [M+H]⁺.

Cis isomer compound **52** as the less polar product 2-[3-[1-(4-fluoro-3-methylphenyl)-2-isopropyl-4-methoxy-indol-3-yl]cyclohexyl]acetic acid [CIS-RAC] (68 mg, 17%). ¹H NMR (400 MHz, DMSO-*d*₆) δ 11.97 (s, 1H), 9.57 (s, 1H), 7.38 - 7.25 (m, 2H), 7.21 - 6.93 (m, 1H), 6.74 (t, J = 7.9 Hz, 1H), 6.37 (d, J = 7.5 Hz, 1H), 6.17 (d, J = 8.0 Hz, 1H), 2.99 (brs, 1H), 2.87 (brs, 1H), 2.32-2.31 (m, 4H), 2.18 - 2.05 (m, 4H), 1.85-1.77 (m, 3H), 1.50-1.42 (m,, 3H), 1.22-1.25 (m, 6H), 1.10 - 0.93 (m, 1H). LCMS *m*/*z* 424.66 [M+H]⁺.

### Compound 53

### 3-[1-(4-fluoro-3-methyl-phenyl)-4-hydroxy-2-isopropyl-indol-3-yl]-1-(trifluoromethyl)cyclobutanecarboxylic acid (53)

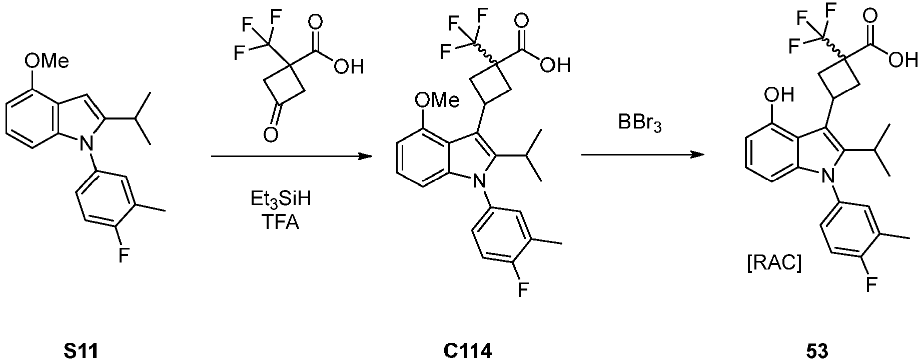

### Step 1. Synthesis of 3-[1-(4fluoro-3-methyl-phenyl)-2-isopropyl-4-methoxy-indol-3-yl]-1-(trifluoromethyl)cyclobutanecarboxylic acid (C114)

Triethylsilane (500 µL, 3.13 mmol) was added to a stirred solution of 1-(4-fluoro-3-methyl-phenyl)-2-isopropyl-4-methoxy-indole **S11** (260 mg, 0.87 mmol), 3-oxo-1-(trifluoromethyl)cyclobutanecarboxylic acid (250 mg, 1.37 mmol) and trifluoroacetic acid (200 µL, 2.60 mmol) in dichloromethane (5 mL). The solution was heated at 50 °C for 72 hours. The reaction mixture was washed with water and dried over Na₂SO₄. The solvent was removed under reduced pressure and purified by silica gel chromatography (Gradient: 0-50% EtOAc/heptane) to afford 3-[1-(4-fluoro-3-methyl-phenyl)-2-isopropyl-4-methoxy-indol-3-yl]-1-(trifluoromethyl)cyclobutanecarboxylic acid (278 mg, 69%) as a yellow solid.

### Step 2. Synthesis of 3-[1-(4-fluoro-3-methyl-phenyl)-4-hydroxy-2-isopropyl-indol-3-yl]-1-(trifluoromethyl)cyclobutanecarboxylic acid (53)

BBr₃ (3 mL of 1 M, 3.0 mmol) was (278 mg) in added to a stirred solution of 3-[1-(4-fluoro-3-methyl-phenyl)-2-isopropyl-4-methoxy-indol-3-yl]-1-(trifluoromethyl)cyclobutanecarboxylic acid **C114** in dichloromethane (5 mL) at 0 °C. The solution was warmed to room temperature and stirred for 3 hours. The reaction was washed with water, dried over Na₂SO₄ and concentrated. Purification by reversed-phase chromatography (Column: C18. Gradient: 0-100% MeCN in water with 0.1% trifluoroacetic acid) afforded the product 3-[1-(4-fluoro-3-methyl-phenyl)-4-hydroxy-2-isopropyl-indol-3-yl]-1-(trifluoromethyl)cyclobutanecarboxylic acid (160 mg, 40%). ¹H NMR (400 MHz, DMSO-*d*₆) δ 13.55 (s, 1H), 9.84 (s, 1H), 7.45 - 7.29 (m, 2H), 7.20-7.18 (m, 1H), 6.78 (t, J = 7.9 Hz, 1H), 6.54 - 6.38 (m, 1H), 6.17 (dd, J = 8.1, 0.9 Hz, 1H), 4.03-4.02 (m, 1H), 3.28-3.26 (m, 1H), 2.97 - 2.82 (m, 1H), 2.59 (dd, J = 8.0, 2.6 Hz, 1H), 2.29 (d, J = 1.9 Hz, 3H), 1.23 (d, J = 8 Hz, 6H). LCMS *m*/*z* 450.58 [M+H]⁺.

### Compound 54

### 6-[6-fluoro-1-(4-fluoro-3-methyl-phenyl)-4-hydroxy-2-isopropyl-indol-3-yl]spiro[3.3]heptane-2-carboxylic acid (54)

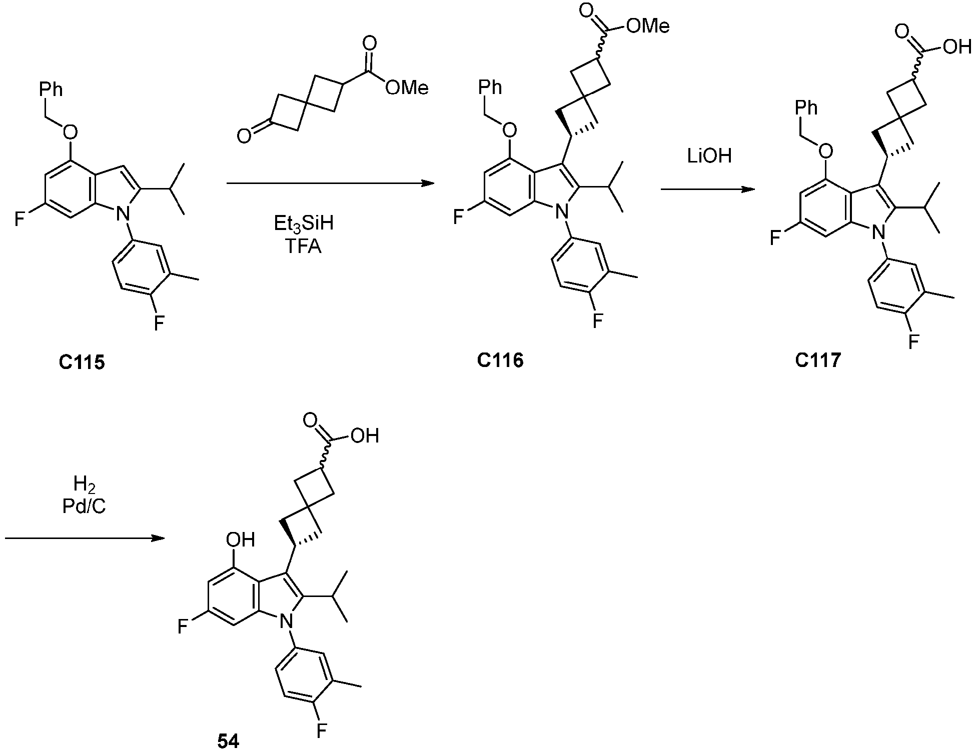

### Step 1. Synthesis of methyl 6-[4-benzyloxy-6-fluoro-1-(4-fluoro-3-methyl-phenyl)-2-isopropyl-indol-3-yl]spiro[3.3]heptane-2-carboxylate (C116)

4-(benzyloxy)-6-fluoro-1-(4-fluoro-3-methylphenyl)-2-isopropyl-1H-indole **C115** (160 mg, 0.40 mmol), methyl 2-oxospiro[3.3]heptane-6-carboxylate (135 mg, 0.80 mmol), triethylsilane (142 mg, 1.2 mmol) and TFA (1.15 g, 10.07 mmol) were mixed into dichloromethane (5 mL) and the reaction was heated at reflux overnight. The reaction was cooled to room temperature and diluted with EtOAc and washed with water. The organic layer was dried and concentrated. Purification by silica gel chromatography (4 g column, 10-40% EtOAc in hexane) afforded the product. Methyl 6-[4-benzyloxy-6-fluoro-1-(4-fluoro-3-methylphenyl)-2-isopropyl-indol-3-yl]spiro[3.3]heptane-2-carboxylate (120 mg, 16%). ¹H NMR (400 MHz, Chloroform-d) δ 7.52 - 7.46 (m, 2H), 7.42 (ddd, J = 7.6, 6.8, 1.3 Hz, 2H), 7.38 - 7.31 (m, 1H), 7.14 (t, J = 8.7 Hz, 1H), 7.11 - 7.00 (m, 2H), 6.32 (dd, J = 11.8, 2.2 Hz, 1H), 6.12 (dd, J = 9.3, 2.1 Hz, 1H), 5.33 (s, 1H), 5.29 (d, J = 3.7 Hz, 2H), 3.94 - 3.75 (m, 1H), 3.68 (s, 3H), 3.11 - 2.91 (m, 2H), 2.86 - 2.70 (m, 2H), 2.41 (dd, J = 8.4, 1.6 Hz, 2H), 2.35 (d, J = 2.0 Hz, 4H), 1.35 - 1.19 (m, 8H). LCMS *m*/*z* 544.35 [M+H]⁺.

### Step 2. Synthesis of 6-[4-benzyloxy-6-fluoro-1-(4-fluoro-3-methyl-phenyl)-2-isopropyl-indol-3-yl]spiro[3.3]heptane-2-carboxylic acid (C117)

Methyl 6-[4-benzyloxy-6-fluoro-1-(4-fluoro-3-methyl-phenyl)-2-isopropyl-indol-3-yl]spiro[3.3]heptane-2-carboxylate **C116** (116 mg, 0.21 mmol) and LiOH hydrate (28 mg, 0.67 mmol) were mixed into THF (3 mL), MeOH (1 mL) and water (1 mL). The reaction was heated at 60 °C for 2 hours. 1 N aq. HCl was added to adjust pH to 2. The reaction was extracted with dichloromethane (10 mL). The organic layer was dried and concentrated to afford the product 6-[4-benzyloxy-6-fluoro-1-(4-fluoro-3-methyl-phenyl)-2-isopropyl-indol-3-yl]spiro[3.3]heptane-2-carboxylic acid (76 mg, 56%) which was used in the next step without further purification. LCMS *m*/*z* 440.28 [M+H]⁺.

### Step 3. Synthesis of 6-[6-fluoro-1-(4-fluoro-3-methyl-phenyl)-4-hydroxy-2-isopropyl-indol-3-yl]spiro[3.3]heptane-2-carboxylic acid (54)

Pd on carbon (3 mg, 0.03 mmol) was placed into a 20 mL vial under nitrogen and EtOH (5 mL) was added. 6-[4-benzyloxy-6-fluoro-1-(4-fluoro-3-methyl-phenyl)-2-isopropyl-indol-3-yl]spiro[3.3]heptane-2-carboxylic acid **C117** (42 mg, 0.08 mmol) was added. The reaction was stirred under H₂ (100 mg, 49.6 mmol) with an attached balloon for 2 hours. The reaction was filtered, concentrated. The crude mixture was purified on silica gel (4 g column, Gradient: 10-90% EtOAc in hexane) to afford the product. ¹H NMR (400 MHz, Chloroform*-d*) δ 7.05 (t, J = 8.8 Hz, 1H), 6.98 (td, J = 8.9, 8.1, 3.5 Hz, 2H), 6.15 (dd, J = 10.3, 2.1 Hz, 1H), 6.02 (dd, J = 9.5, 2.2 Hz, 1H), 3.81 - 3.69 (m, 1H), 3.05 (q, J = 8.5 Hz, 1H), 2.87 (p, J = 7.3 Hz, 1H), 2.75 (dt, J = 24.9, 10.4 Hz, 2H), 2.43 (dd, J = 8.5, 2.9 Hz, 2H), 2.39 - 2.24 (m, 6H), 2.24 - 2.17 (m, 1H), 1.18 (d, J = 2.1 Hz, 6H). LCMS *m*/*z* 440.28 [M+H]⁺.

### Compounds 55-58

Compounds **55-58** were prepared in three steps from **S12** or the appropriate indole core by reductive alkylation, ester hydrolysis and benzyl removal by hydrogenation as described for the preparation of compound **54.** Any modifications are noted in the accompanying footnotes.

**Table 5. Method of preparation, structure and physicochemical data for compounds 55-58**

| **Compound** | **Method/Product** | **Aldehyde or Ketone** | **¹H NMR; LCMS m/z [M+H]⁺** |
|---|---|---|---|
| **55** | See footnote¹ | 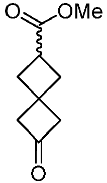 | ¹H NMR (400 MHz, Chloroform*-d*) δ 7.14 (s, 0H), 7.05 - 6.87 (m, 4H), 6.41 (dd, J = 11.6, 8.3 Hz, 1H), 6.10 (dd, J = 8.3, 3.1 Hz, 1H), 3.73 (p, J = 9.3 Hz, 1H), 2.99 (p, J = 8.5 Hz, 1H), 2.81 - 2.64 (m, 3H), 2.36 (dd, J = 8.5, 2.8 Hz, 2H), 2.32 - 2.23 (m, 1H), 2.27 - 2.04 (m, 7H), 2.08 - 1.99 (m, 0H), 1.12 (dt, J = 7.3, 2.8 Hz, 7H). LCMS *m*/*z* 440.24 [M+H]⁺ |
| | 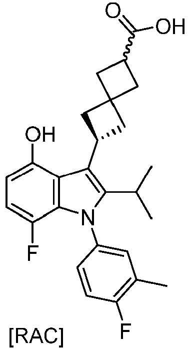 | | |
| **56** | From S12 | 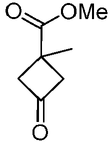 | ¹H NMR (400 MHz, DMSO-*d*₆) δ 12.07 (s, 1H), 9.82 (s, 1H), 7.37 - 7.23 (m, 2H), 7.21 - 7.12 (m, 1H), 6.77 (t, J = 7.9 Hz, 1H), 6.46 (d, J = 7.6 Hz, 1H), 6.17 (d, J = 8.1 Hz, 1H), 4.07 - 3.93 (m, 1H), 2.91 - 2.79 (m, 1H), 2.50 (DMSO overlap, m, 2H), 2.32 - 2.26 (m, 3H), 1.43 (s, 3H), 1.26 - 1.19 (m, 6H). LCMS *m*/*z* 396.2 [M+H]⁺. |
| | As for Compound **54** | | |
| | 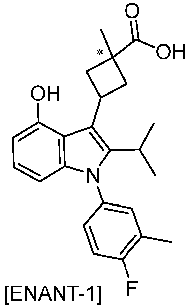 | | |
| **57** | From S12 | 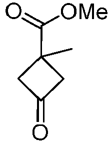 | ¹H NMR (400 MHz, DMSO-*d*₆) δ 11.75 (s, 1H), 9.63 (s, 1H), 7.38 - 7.25 (m, 2H), 7.23 - 7.13 (m, 1H), 6.74 (t, J = 7.9 Hz, 1H), 6.45 (d, J = 7.6 Hz, 1H), 6.13 (d, J = 8.0 Hz, 1H), 3.97 - 3.83 (m, 1H), 3.14 - 3.04 (m, 2H), 3.01 - 2.90 (m, 1H), 2.29 (s, 3H), 2.04 - 1.94 (m, 2H), 1.50 (s, 3H), 1.23 (d, J = 7.2 Hz, 4H). LCMS *m*/*z* 396.2 [M+H]⁺. |
| | As for Compound **54** | | |
| | 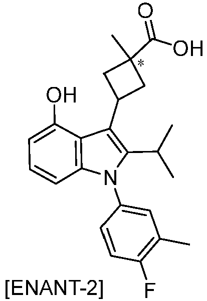 | | |
| **58** | From S12 | 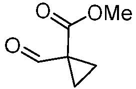 | ¹H NMR (400 MHz, DMSO-*d*₆) δ 9.41 (s, 1H), 7.35 - 7.27 (m, 2H), 7.24 - 7.16 (m, 1H), 6.72 (t, J = 7.9 Hz, 1H), 6.34 (d, J = 7.6 Hz, 1H), 6.10 (d, J = 7.9 Hz, 1H), 3.54 - 3.52 (m, 2H), 3.08 - 2.98 (m, 1H), 2.29 (s, 3H), 1.17 - 1.06 (m, 6H), 0.94 - 0.89 (m, 2H), 0.70 - 0.64 (m, 2H). LCMS *m*/*z* 382.2 [M+H]⁺. |
| | As for Compound **54** | | |
| | 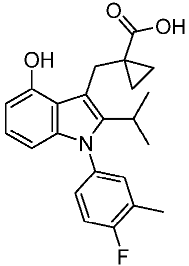 | | |

| | | | |
|---|---|---|---|
| **^{1.}** Prepared from 7-fluoro-1-(4-fluoro-3-methylphenyl)-2-isopropyl-4-methoxy-1H-indole according to the method described for the preparation of compound **54.** Methoxy group deprotection was performed by treatment with BBr₃. **^{2.}** Purification by reverse phase chromatography Purification by reversed-phase HPLC. Method: C18 Waters Sunfire column (30 x 150 mm, 5 micron). Gradient: MeCN in H₂O with 0.2% formic acid. | | | |

### Compound 59

### 4-[5,7-difluoro-1-(4-fluorophenyl)-4-hydroxy-2-tetrahydropyran-4-yl-indol-3-yl]benzoic acid (59)

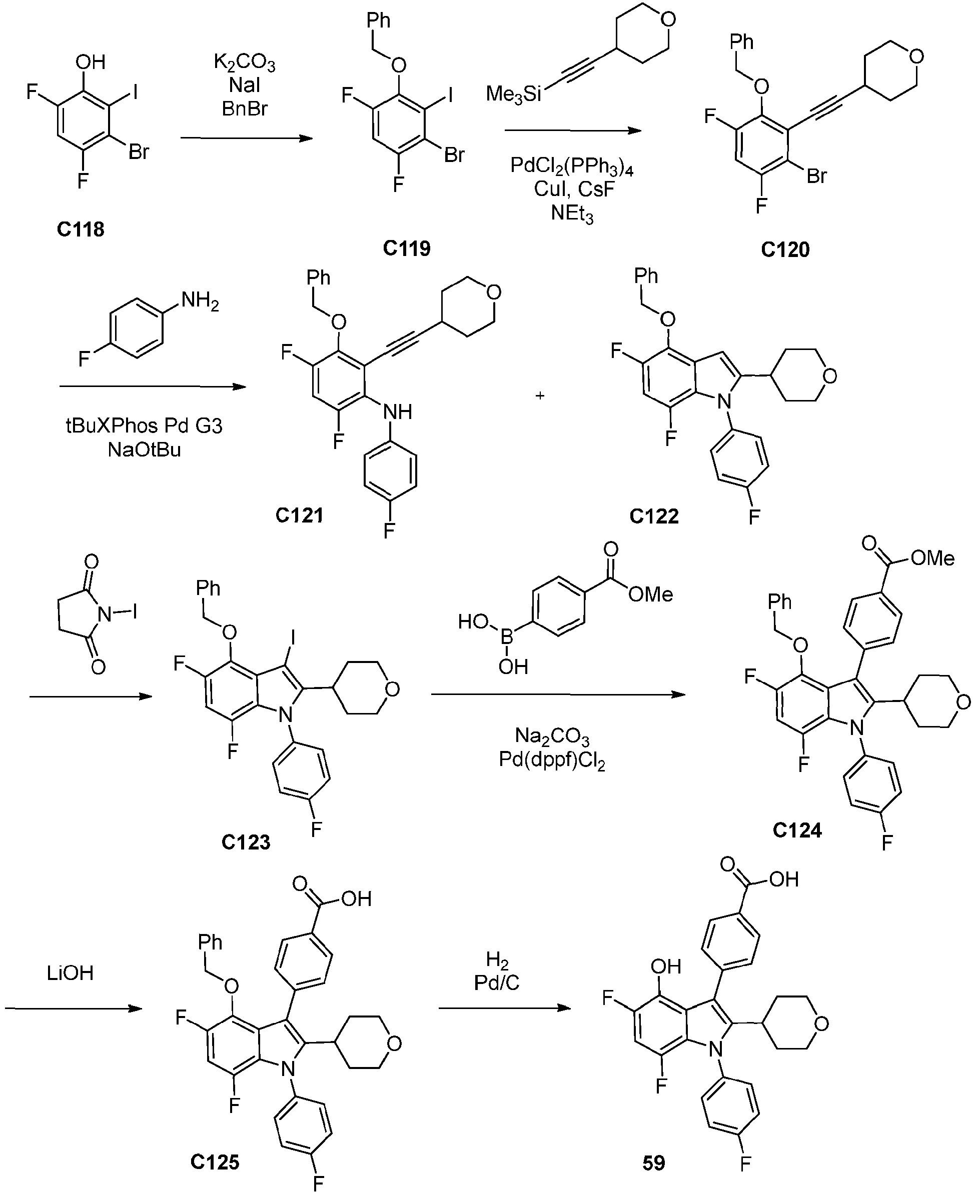

### Step 1. Synthesis of 2-benzyloxy-4-bromo-1,5-difluoro-3-iodo-benzene (C119)

Compound **C119** was prepared using the method described for **C2** in preparation of **S1.** 2-benzyloxy-4-bromo-1,5-difluoro-3-iodo-benzene (157 mg, 93%). ¹H NMR (400 MHz, Chloroform-d) δ 7.60 - 7.51 (m, 2H), 7.46 - 7.35 (m, 3H), 7.07 (dd, J = 10.3, 8.0 Hz, 1H), 5.08 (d, J = 5.9 Hz, 2H). LCMS *m*/*z* 425.65 [M+H]⁺.

### Step 2. Synthesis of 4-[2-(2-benzyloxy-6-bromo-3,5-difluoro-phenyl)ethynyl]tetrahydropyran (C120)

Compound **C120** was prepared from **C119** using a Sonagashira coupling as described for the synthesis of **C3** in the preparation of **S1.** 4-[2-(2-benzyloxy-6-bromo-3,5-difluorophenyl)ethynyl]tetrahydropyran (105 mg, 75%). ¹H NMR (400 MHz, Chloroform-d) δ 7.54 - 7.45 (m, 2H), 7.45 - 7.34 (m, 3H), 6.93 (dd, J = 10.4, 8.2 Hz, 1H), 5.16 (s, 2H), 3.98 (ddd, J = 11.6, 6.4, 3.5 Hz, 2H), 3.60 (ddd, J = 11.4, 7.8, 3.2 Hz, 2H), 3.00 (tt, J = 8.1, 4.2 Hz, 1H), 2.02 - 1.90 (m, 2H), 1.80 (dtd, J = 13.4, 7.9, 3.5 Hz, 2H). LCMS *m*/*z* 409.04 [M+H]⁺. *Step 3. Synthesis of 3-benzyloxy-4, 6-difluoro-N-(4-fluorophenyl)-2-(2-tetrahydropyran-4-ylethynyl)aniline **(C121)** and 4-benzyloxy-5, 7-difluoro-1-(4-fluorophenyl)-3-iodo-2-tetrahydropyran-4-yl-indole **(C122)***

A mixture of compound **C121** and **C122** was prepared from **C120** by coupling of 4-fluroaniline using the method described for the synthesis of **C4** in the preparation of **S1.** Purification by silica gel chromatography (Gradient: 0-25% EtOAc in heptane) afforded the products. 3-benzyloxy-4,6-difluoro-N-(4-fluorophenyl)-2-(2-tetrahydropyran-4-ylethynyl)aniline **C121** (0.54 g, 50%). ¹H NMR (400 MHz, Chloroform-*d*) δ 7.57 - 7.47 (m, 2H), 7.45 - 7.34 (m, 3H), 6.94 (dt, J = 15.3, 9.5 Hz, 3H), 6.76 (ddd, J = 8.7, 4.4, 1.5 Hz, 2H), 5.69 (s, 1H), 5.20 (s, 2H), 3.81 (ddd, J = 11.5, 5.7, 3.7 Hz, 2H), 3.48 (ddd, J = 11.6, 8.5, 2.9 Hz, 2H), 2.82 (tt, J = 8.4, 4.1 Hz, 1H), 1.78 (ddt, J = 13.3, 6.1, 3.8 Hz, 2H), 1.65 - 1.52 (m, 2H). LCMS *m*/*z* 438.35 [M+H]⁺. 4-benzyloxy-5,7-difluoro-1-(4-fluorophenyl)-2-tetrahydropyran-4-yl-indole **C122** (98 mg, 9%) ¹H NMR (400 MHz, Chloroform-*d*) δ 7.59 - 7.52 (m, 2H), 7.44 (ddt, J = 7.9, 6.3, 1.1 Hz, 2H), 7.40 - 7.31 (m, 3H), 7.25 - 7.14 (m, 2H), 6.66 (t, J = 11.5 Hz, 1H), 6.49 (dd, J = 2.2, 0.7 Hz, 1H), 5.26 (s, 2H), 4.05 - 3.90 (m, 2H), 3.33 (td, J = 11.8, 2.2 Hz, 2H), 2.67 (tt, J = 11.7, 3.9 Hz, 1H), 1.81 (dtd, J = 13.4, 11.8, 4.4 Hz, 2H), 1.74 - 1.61 (m, 2H). LCMS *m*/*z* 438.35 [M+H]⁺.

### Step 4. Synthesis of 4-benzyloxy-5,7-difluoro-1-(4-fluorophenyl)-3-iodo-2-tetrahydropyran-4-yl-indole (C123)

Compound **C123** was prepared from compound **C122** using the iodination method as described for the preparation of **S1**.4-benzyloxy-5,7-difluoro-1-(4-fluorophenyl)-3-iodo-2-tetrahydropyran-4-yl-indole (125.2 mg, 100%). ¹H NMR (400 MHz, Chloroform-d) δ 7.71 - 7.61 (m, 2H), 7.50 - 7.42 (m, 2H), 7.42 - 7.35 (m, 1H), 7.33 (td, J = 4.7, 2.4 Hz, 2H), 7.26 - 7.15 (m, 2H), 6.68 (t, J = 11.3 Hz, 1H), 5.20 (s, 2H), 4.05 - 3.94 (m, 2H), 3.33 (td, J = 11.9, 2.0 Hz, 2H), 3.01 (tt, J = 12.5, 3.6 Hz, 1H), 2.35 (qd, J = 12.6, 4.4 Hz, 2H), 1.54 (ddd, J = 12.9, 3.8, 1.8 Hz, 2H). LCMS *m*/*z* 564.29 [M+H]⁺.

### Step 5. methyl 4-[4-benzyloxy-5, 7-difluoro-1-(4-fluorophenyl)-2-tetrahydropyran-4-yl-indol-3-yl]benzoate (C124)

Compound **C124** was prepared from **C123** and (4-methoxycarbonylphenyl)-boronic acid by Suzuki coupling as described in the method used to synthesize compound **1.**

Methyl 4-[4-benzyloxy-5,7-difluoro-1-(4-fluorophenyl)-2-tetrahydropyran-4-yl-indol-3-yl]benzoate (126 mg, 96%). ¹H NMR (400 MHz, Chloroform-*d*) δ 8.05 - 7.93 (m, 2H), 7.59 - 7.48 (m, 2H), 7.48 - 7.40 (m, 2H), 7.27 - 7.12 (m, 5H), 6.88 - 6.78 (m, 2H), 6.70 (t, J = 11.3 Hz, 1H), 4.62 (s, 2H), 4.00 (s, 3H), 3.84 - 3.74 (m, 2H), 3.12 (td, J = 11.8, 2.0 Hz, 2H), 2.77 (tt, J = 12.3, 3.5 Hz, 1H), 1.74 - 1.61 (m, 2H), 1.52 (ddd, J = 12.6, 3.6, 1.6 Hz, 2H). LCMS *m*/*z* 570.14 [M+H]⁺.

### Step 6. Synthesis of 4-[4-benzyloxy-5, 7-difluoro-1-(4-fluorophenyl)-2-tetrahydropyran-4-yl-indol-3-yl]benzoic acid (C125)

Compound **C125** was prepared by hydrolysis of **C124** using LiOH as described for preparation **of C68** in the synthesis of compound 1. 4-[4-benzyloxy-5,7-difluoro-1-(4-fluorophenyl)-2-tetrahydropyran-4-yl-indol-3-yl]benzoic acid (118 mg, 99%). ¹H NMR (400 MHz, Chloroform-*d*) δ 8.09 - 7.91 (m, 2H), 7.56 - 7.47 (m, 2H), 7.46 - 7.37 (m, 2H), 7.27 - 7.13 (m, 5H), 6.84 - 6.74 (m, 2H), 6.68 (t, J = 11.3 Hz, 1H), 4.59 (s, 2H), 3.86 - 3.71 (m, 2H), 3.11 (td, J = 11.8, 2.0 Hz, 2H), 2.75 (tt, J = 12.2, 3.4 Hz, 1H), 1.78 - 1.59 (m, 2H), 1.59 - 1.46 (m, 2H). LCMS *m*/*z* 558.48 [M+H]⁺.

### Step 7.Synthesis of 4-[5, 7-difluoro-1-(4-fluorophenyl)-4-hydroxy-2-tetrahydropyran-4-yl-indol-3-yl]benzoic acid (59)

Compound **59** was palladium on carbon catalyzed hydrogenation of **C125** as described for preparation of compound **1.** The catalyst was filtered and concentrated to give the product as a pure white solid. 4-[5,7-difluoro-1-(4-fluorophenyl)-4-hydroxy-2-tetrahydropyran-4-yl-indol-3-yl]benzoic acid (86.2 mg, 80%). ¹H NMR (400 MHz, Chloroform-*d*/CD3OD) δ 8.13 - 7.98 (m, 2H), 7.57 - 7.38 (m, 2H), 7.38 - 7.27 (m, 2H), 7.17 - 7.07 (m, 2H), 6.60 (t, J = 11.0 Hz, 1H), 3.72 (dd, J = 11.4, 4.1 Hz, 2H), 3.04 (td, J = 11.8, 2.0 Hz, 2H), 2.68 (ddt, J = 12.3, 8.8, 3.6 Hz, 1H), 1.60 (qd, J = 12.4, 4.3 Hz, 2H), 1.51 - 1.38 (m, 2H). LCMS *m*/*z* 468.37 [M+H]⁺.

### Compound 60

### 4-[[1-(4-fluorophenyl)-4-hydroxy-2-tetrahydropyran-4-yl-indol-3-yl]methyl]benzoic acid (60)

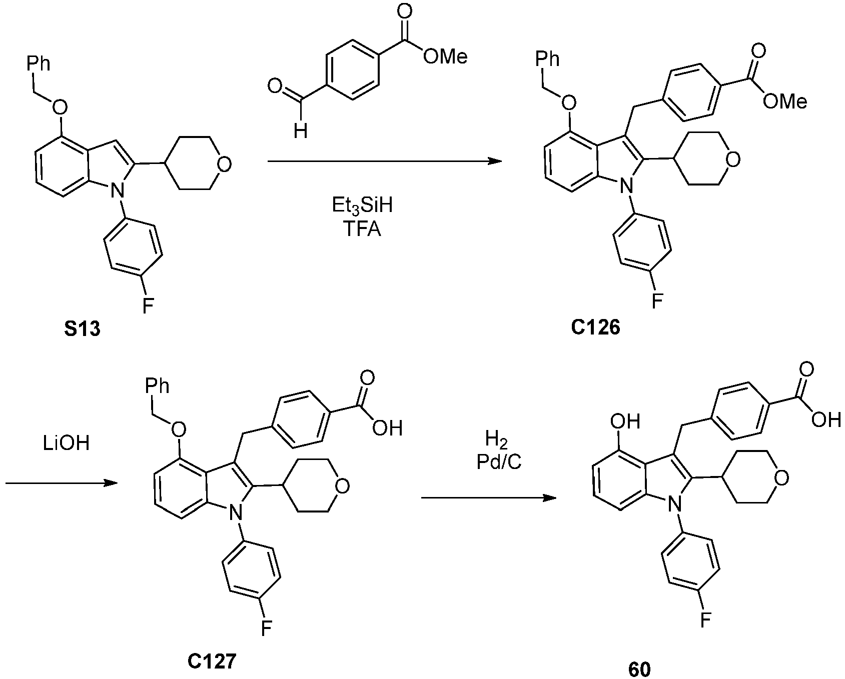

### Synthesis of 4-[[1-(4-fluorophenyl)-4-hydroxy-2-tetrahydropyran-4-yl-indol-3-yl]methyl]benzoic acid (60)

Compound 60 was prepared in three steps from **S13** using the method described for the preparation of **54.** Purification by silica gel chromatography (Gradient: 0-10% MeOH in dichloromethane) afforded the product as a white solid. 4-[[1-(4-fluorophenyl)-4-hydroxy-2-tetrahydropyran-4-yl-indol-3-yl]methyl]benzoic acid (25 mg, 94%). ¹H NMR (400 MHz, Methanol-d/CDCl₃) δ 7.97 - 7.83 (m, 2H), 7.43 - 7.19 (m, 6H), 6.92 - 6.76 (m, 1H), 6.45 - 6.36 (m, 1H), 6.35 - 6.22 (m, 1H), 4.59 (s, 2H), 3.85 (dd, J = 11.5, 4.1 Hz, 2H), 3.29 - 3.19 (m, 2H), 3.00 - 2.82 (m, 1H), 1.86 (qd, J = 12.7, 4.3 Hz, 2H), 1.58 - 1.44 (m, 2H). LCMS *m*/*z* 446.21 [M+H]⁺.

### Compounds 61-102

Compounds **61-102** (Table 6) were prepared from **S14** or **S15** according to the method described for compound **1.** Any modifications are noted in the table footnotes. In some examples, the benzyl protecting group was removed by hydrogenation with ammonium formate instead of hydrogen gas (as described in the preparation of compound **2).**

**Table 6. Method of preparation, structure and physicochemical data for compounds 61-102**

| **Compound** | **Method/Product** | **Boronic acid or ester** | **¹H NMR; LCMS m/z [M+H]⁺** |
|---|---|---|---|
| **61** | From **S14** | 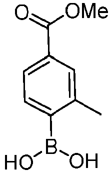 | ¹H NMR (400 MHz, DMSO-*d*₆) δ 12.84 (s, 1H), 8.97 (s, 1H), 7.83 (d, J = 1.8 Hz, 1H), 7.76 (dd, J = 7.7, 1.8 Hz, 1H), 7.53 (dd, J = 8.3, 4.8 Hz, 2H), 7.50 - 7.41 (m, 2H), 7.38 (d, J = 7.8 Hz, 1H), 6.83 (t, J = 7.9 Hz, 1H), 6.33 (d, J = 7.6 Hz, 1H), 6.27 (d, J = 8.1 Hz, 1H), 3.70 - 3.55 (m, 2H), 3.04 - 2.87 (m, 2H), 2.65 (s, 1H), 2.17 (s, 3H), 1.66 - 1.54 (m, 2H), 1.45 (d, J = 13.0 Hz, 1H), 1.29 - 1.21 (m, 1H). LCMS *m*/*z 446.2* [M+H]⁺. |
| | As for compound **1^{1,2}** | | |
| | 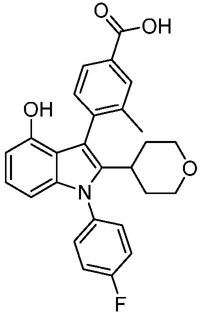 | | |
| **62** | From **S14** | 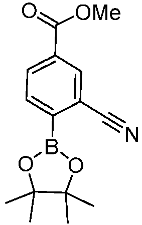 | ¹H NMR (400 MHz, Methanol-*d*₄) δ 8.36 (d, J = 1.7 Hz, 1H), 8.27 (dd, J = 8.1, 1.8 Hz, 1H), 7.73 (d, J = 8.0 Hz, 1H), 7.52 - 7.43 (m, 2H), 7.37 (t, J = 8.6 Hz, 2H), 6.92 - 6.85 (m, 1H), 6.39 (dd, J = 7.7, 0.8 Hz, 1H), 6.34 (dd, J = 8.3, 0.8 Hz, 1H), 3.76 (t, J = 11.0 Hz, 2H), 3.20 - 3.09 (m, 2H), 2.91 - 2.81 (m, 1H), 1.76 - 1.52 (m, 4H). LCMS *m*/*z 457.18* [M+H]⁺. |
| | As for compound **1^{1,3}** | | |
| | 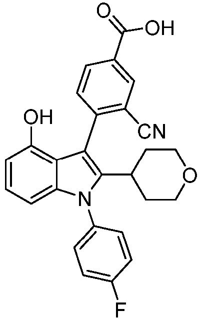 | | |
| **63** | From **S14** | 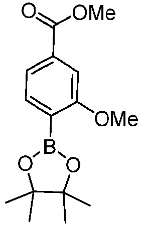 | ¹H NMR (400 MHz, DMSO-*d*₆) δ 8.91 (s, 1H), 7.59 - 7.42 (m, 6H), 7.36 (d, J = 7.6 Hz, 1H), 6.80 (t, J = 7.9 Hz, 1H), 6.31 (dd, J = 7.7, 0.8 Hz, 1H), 6.23 (dd, J = 8.2, 0.8 Hz, 1H), 3.76 (s, 3H), 3.65 (t, J = 11.8 Hz, 2H), 2.99 (t, J = 11.4 Hz, 2H), 2.73 - 2.63 (m, 1H), 1.62 - 1.38 (m, 4H). LCMS *m*/*z* 462.0 [M+H]⁺. |
| | As for compound 1^{1,4} 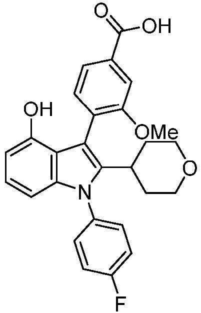 | | |
| **64** | From **S14** | 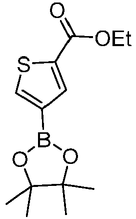 | ¹H NMR (400 MHz, DMSO-*d*₆) δ 13.03 (s, 1H), 9.22 (s, 1H), 7.71 (d, J = 6.6 Hz, 2H), 7.57 - 7.42 (m, 4H), 6.83 (t, J = 8.0 Hz, 1H), 6.38 (d, J = 7.7 Hz, 1H), 6.21 (d, J = 8.1 Hz, 1H), 3.71 (d, J = 11.2 Hz, 2H), 3.12 - 2.98 (m, 2H), 2.82 (p, J = 8.0 Hz, 1H), 1.58 (d, J = 7.3 Hz, 4H). LCMS *m*/*z* 438.0 [M+H]⁺. |
| | As for compound **1¹,⁴** | | |
| | 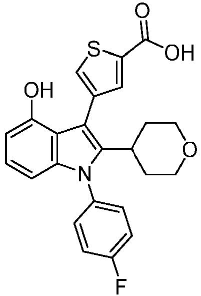 | | |
| **65** | From **S14** | 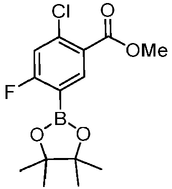 | ¹H NMR (400 MHz, DMSO-*d*₆) δ 13.43 (s, 1H), 9.20 (s, 1H), 7.86 (s, 1H), 7.56 (s, 2H), 7.51 - 7.40 (m, 2H), 6.85 (t, J = 7.9 Hz, 1H), 6.36 (dd, J = 7.7, 0.8 Hz, 1H), 6.30 - 6.23 (m, 1H), 3.70 (s, 2H), 3.03 (q, J = 10.4 Hz, 2H), 2.72 (t, J = 12.4 Hz, 1H), 1.72 - 1.34 (m, 5H). LCMS *m*/*z* 484.31 [M+H]⁺. |
| | As for compound **1^{1,5}** | | |
| | 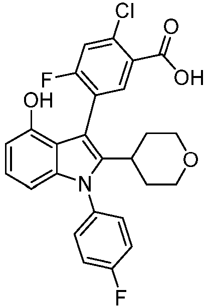 | | |
| **66** | From **S14** | 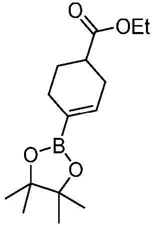 | ¹H NMR (400 MHz, Methanol-*d*₄) δ 7.45 (dt, J = 8.8, 3.7 Hz, 1H), 7.30 - 7.22 (m, 2H), 7.17 (t, J = 8.1 Hz, 3H), 6.78 (q, J = 7.4, 7.0 Hz, 1H), 6.37 (dd, J = 7.6, 2.0 Hz, 1H), 6.24 (d, J = 8.2 Hz, 1H), 5.74 (s, 1H), 3.83 (d, J = 10.6 Hz, 3H), 3.20 (s, 2H), 2.90 - 2.59 (m, 3H), 1.97 (q, J = 34.6, 30.8 Hz, 6H), 1.55 (t, J = 14.6 Hz, 4H). LCMS *m*/*z* 436.37 [M+H]⁺. |
| | As for compound **1^{1,5}** | | |
| | 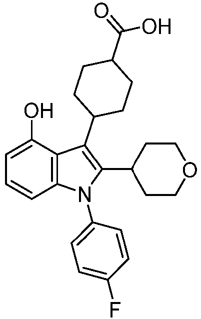 | | |
| **67** | From **S14** | 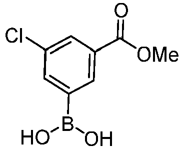 | ¹H NMR (400 MHz, Chloroform-*d*) δ 7.88 (t, J = 1.6 Hz, 1H), 7.83 (t, J = 1.8 Hz, 1H), 7.50 (t, J = 1.8 Hz, 1H), 7.18 (dd, J = 8.7, 4.8 Hz, 2H), 7.08 (t, J = 8.4 Hz, 2H), 6.79 - 6.69 (m, 1H), 6.27 (d, J = 7.6 Hz, 1H), 6.19 (d, J = 8.3 Hz, 1H), 3.62 (dd, J = 11.4, 4.0 Hz, 2H), 2.99 (td, J = 11.7, 2.1 Hz, 2H), 2.67 (tt, J = 12.2, 3.6 Hz, 1H), 1.49 (qd, J = 12.3, 4.1 Hz, 2H), 1.44 - 1.35 (m, 2H). LCMS *m*/*z* 466.35 [M+H]⁺. |
| | As for compound **1^{1,5}** | | |
| | 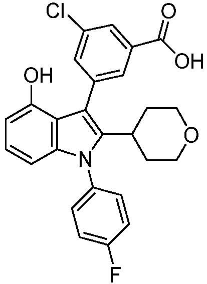 | | |
| **68** | From **S14** | 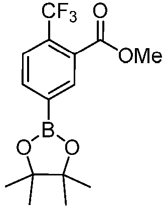 | LCMS *m*/*z* 500.58 [M+H]⁺. |
| | As for compound **1^{1,5}** | | |
| | 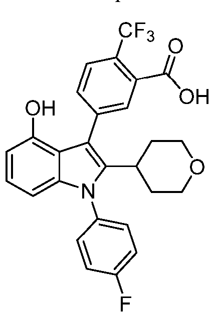 | | |
| **69** | From **S14** | 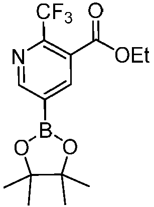 | ¹H NMR (400 MHz, DMSO-*d*₆) δ 14.05 (s, 1H), 9.50 (s, 1H), 8.82 (s, 1H), 8.15 (s, 1H), 7.64 - 7.51 (m, 2H), 7.53 - 7.41 (m, 2H), 6.94 - 6.82 (m, 1H), 6.43 (dd, J = 7.7, 0.8 Hz, 1H), 6.24 (dd, J = 8.2, 0.7 Hz, 1H), 3.70 (dd, J = 11.2, 4.0 Hz, 2H), 3.07 (t, J = 11.4 Hz, 2H), 2.85 (t, J = 12.3 Hz, 1H), 1.64 (d, J = 12.9 Hz, 2H), 1.58 - 1.41 (m, 2H). LCMS *m*/*z* 501.32 [M+H]⁺. |
| | As for compound **1^{1,5}** | | |
| | 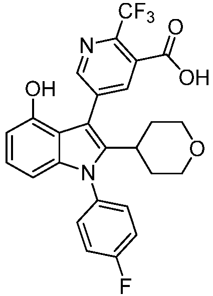 | | |
| **70** | From S14 | 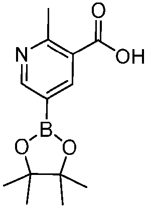 | LCMS *m*/*z* 447.35 [M+H]⁺. |
| | As for compound 1^{1,5} | | |
| | 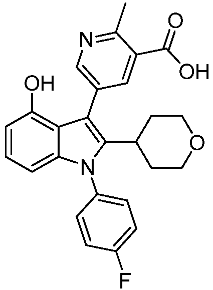 | | |
| **71** | From S14 | 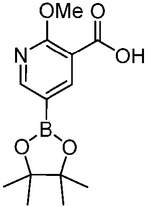 | LCMS *m*/*z* 463.38 [M+H]⁺. |
| | As for compound 1^{1,5} | | |
| | 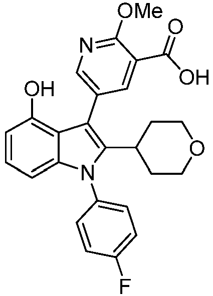 | | |
| **72** | From **S14** | 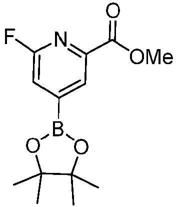 | LCMS *m*/*z* 451.0 [M+H]⁺. |
| | As for compound 1^{1,6} | | |
| | 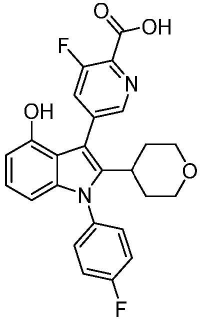 | | |
| **73** | From **S14.** | 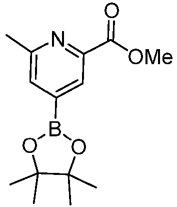 | ¹H NMR (400 MHz, DMSO-*d*₆) δ 9.30 (d, J = 14.0 Hz, 1H), 8.51 (d, J = 2.0 Hz, 1H), 7.79 (d, J = 2.0 Hz, 1H), 7.57 - 7.45 (m, 4H), 6.87 (t, J = 7.9 Hz, 1H), 6.41 (d, J = 7.6 Hz, 1H), 6.21 (dd, J = 17.3, 8.2 Hz, 1H), 3.74 - 3.65 (m, 2H), 3.05 (dd, J = 12.6, 10.4 Hz, 2H), 2.83 - 2.69 (m, 1H), 2.55 (s, 3H), 1.64 - 1.39 (m, 4H). LCMS *m*/*z* 447.0 [M+H]⁺. |
| | As for compound **1^{1,6}** | | |
| | 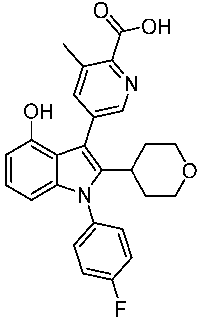 | | |
| **74** | From **S14** | 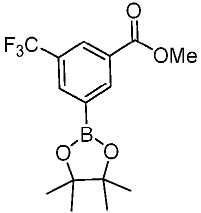 | ¹H NMR (400 MHz, Chloroform-*d*) δ 7.80 (t, J = 1.6 Hz, 1H), 7.68 (dd, J = 2.1, 1.5 Hz, 1H), 7.41 (t, J = 1.8 Hz, 1H), 7.23 - 7.12 (m, 2H), 7.12 - 6.97 (m, 5H), 6.84 (t, J = 8.0 Hz, 1H), 6.74 - 6.66 (m, 2H), 6.46 - 6.37 (m, 1H), 6.31 (dd, J = 8.3, 0.7 Hz, 1H), 4.70 (d, J = 5.4 Hz, 2H), 3.68 (s, 3H), 3.65 - 3.54 (m, 2H), 2.94 (td, J = 11.7, 2.0 Hz, 2H), 2.62 (tt, J = 12.2, 3.5 Hz, 1H), 1.56 - 1.40 (m, 2H), 1.39 - 1.32 (m, 2H). LCMS *m*/*z* 500.38 [M+H]⁺ |
| | As for compound 1¹ | | |
| | 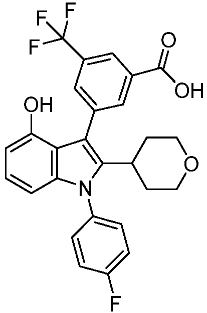 | | |
| **75** | From **S14** | 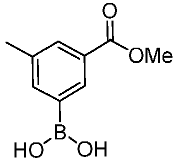 | ¹H NMR (400 MHz, Chloroform-*d*) δ 7.90 (d, J = 1.8 Hz, 1H), 7.82 (t, J = 1.3 Hz, 1H), 7.46 - 7.37 (m, 1H), 7.18 (dd, J = 8.8, 4.8 Hz, 2H), 7.08 (t, J = 8.4 Hz, 2H), 6.80 (t, J = 8.0 Hz, 1H), 6.33 (dd, J = 7.7, 0.8 Hz, 1H), 6.27 (dd, J = 8.2, 0.8 Hz, 1H), 3.72 - 3.61 (m, 2H), 2.96 (t, J = 11.7 Hz, 2H), 2.72 - 2.54 (m, 1H), 2.42 - 2.31 (m, 3H), 1.56 - 1.35 (m, 4H). LCMS *m*/*z* 446.36 [M+H]⁺. |
| | As for compound **1** | | |
| | 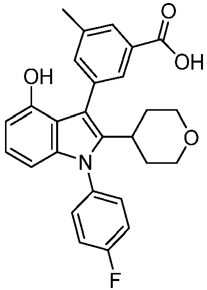 | | |
| **76** | From **S14** | 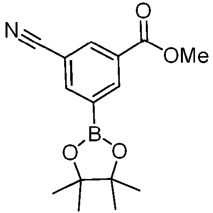 | ¹H NMR (400 MHz, Chloroform-*d*) δ 8.20 (q, J = 1.6 Hz, 1H), 8.12 (q, J = 1.6 Hz, 1H), 7.79 (q, J = 1.6 Hz, 1H), 7.27 - 7.16 (m, 2H), 7.08 (td, J = 8.5, 1.7 Hz, 2H), 6.75 (td, J = 8.0, 1.6 Hz, 1H), 6.26 (dd, J = 7.5, 1.4 Hz, 1H), 6.18 (dd, J = 8.0, 1.4 Hz, 1H), 3.69 - 3.60 (m, 2H), 2.99 (t, J = 11.3 Hz, 2H), 2.68 (td, J = 11.5, 5.9 Hz, 1H), 1.44 (q, J = 13.6, 13.1 Hz, 4H). LCMS *m*/*z* 457.21 [M+H]⁺. |
| | As for compound **1^{1,5}** | | |
| | 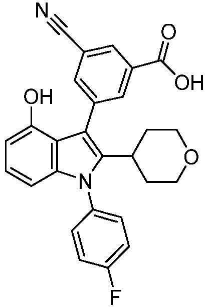 | | |
| **77** | As for compound **66** | 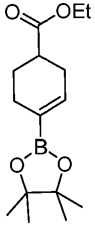 | ¹H NMR (400 MHz, Methanol-*d*₄) δ 7.45 (dt, J = 8.8, 3.7 Hz, 1H), 7.30 - 7.22 (m, 2H), 7.17 (t, J = 8.1 Hz, 3H), 6.78 (q, J = 7.4, 7.0 Hz, 1H), 6.37 (dd, J = 7.6, 2.0 Hz, 1H), 6.24 (d, J = 8.2 Hz, 1H), 5.74 (s, 1H), 3.83 (d, J = 10.6 Hz, 3H), 3.20 (s, 2H), 2.90 - 2.59 (m, 3H), 1.97 (q, J = 34.6, 30.8 Hz, 6H), 1.55 (t, J = 14.6 Hz, 4H). LCMS *m*/*z* 436.37 [M+H]⁺. |
| | 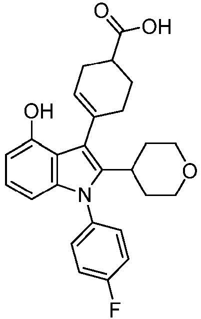 | | |
| **78** | From **S14** | 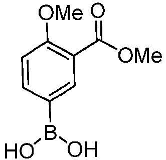 | ¹H NMR (400 MHz, DMSO-*d*₆) δ 12.51 (s, 1H), 9.07 (s, 1H), 7.67 (d, J = 2.3 Hz, 1H), 7.59 - 7.37 (m, 5H), 7.13 (d, J = 8.6 Hz, 1H), 6.82 (t, J = 7.9 Hz, 1H), 6.35 (dd, J = 7.7, 0.8 Hz, 1H), 6.21 (dd, J = 8.2, 0.8 Hz, 1H), 3.88 (s, 3H), 3.68 (d, J = 11.2 Hz, 2H), 3.07 - 2.95 (m, 2H), 2.82 - 2.72 (m, 1H), 1.63 - 1.44 (m, 4H). LCMS *m*/*z* 462.16 [M+H]⁺ |
| | As for compound **1**^{1,3} | | |
| | 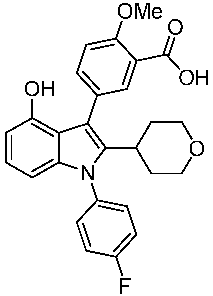 | | |
| **79** | From **S14** | 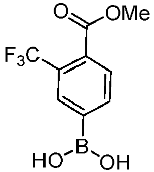 | ¹H NMR (400 MHz, DMSO-*d*₆) δ 13.55 (s, 1H), 9.35 (s, 1H), 7.89 - 7.74 (m, 3H), 7.59 - 7.51 (m, 2H), 7.52 - 7.42 (m, 2H), 6.87 (t, J = 7.9 Hz, 1H), 6.41 (dd, J = 7.7, 0.8 Hz, 1H), 6.22 (dd, J = 8.2, 0.8 Hz, 1H), 3.74 - 3.64 (m, 2H), 3.04 (t, J = 11.4 Hz, 2H), 2.83 (t, J = 12.2 Hz, 1H), 1.65 - 1.42 (m, 4H). LCMS *m*/*z* 500.11 [M+H]⁺ |
| | As for compound **1**^{1,3} | | |
| | 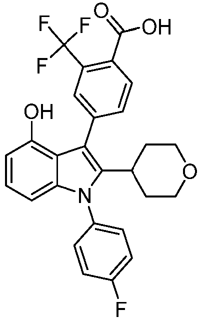 | | |
| **80** | From **S14** | 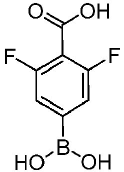 | ¹H NMR (400 MHz, DMSO-*d*₆) δ 13.88 (s, 1H), 9.34 (s, 1H), 7.58 - 7.41 (m, 4H), 7.20 (d, J = 9.3 Hz, 2H), 6.92 - 6.80 (m, 1H), 6.41 (dd, J = 7.8, 0.8 Hz, 1H), 6.21 (dd, J = 8.3, 0.8 Hz, 1H), 3.72 (d, J = 9.3 Hz, 2H), 3.06 (t, J = 11.0 Hz, 2H), 2.88 - 2.78 (m, 1H), 1.65 - 1.44 (m, 4H). LCMS *m*/*z* 468.12 [M+H]⁺. |
| | As for compound **1**^{1,8} | | |
| | 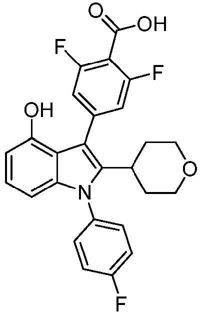 | | |
| **81** | From **S14** | 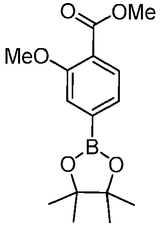 | ¹H NMR (400 MHz, DMSO-*d*₆) δ 12.51 (s, 1H), 9.19 (s, 1H), 7.66 (d, J = 7.9 Hz, 1H), 7.58 - 7.40 (m, 4H), 7.13 (d, J = 1.4 Hz, 1H), 7.04 (dd, J = 7.8, 1.4 Hz, 1H), 6.89 - 6.77 (m, 1H), 6.40 (dd, J = 7.7, 0.8 Hz, 1H), 6.20 (dd, J = 8.2, 0.8 Hz, 1H), 3.82 (s, 3H), 3.70 (d, J = 10.7 Hz, 2H), 3.04 (t, J = 12.8 Hz, 2H), 2.92 - 2.82 (m, 1H), 1.57 (s, 4H). LCMS *m*/*z* 462.19 [M+H]⁺ |
| | As for compound **1**^{1,8} | | |
| | 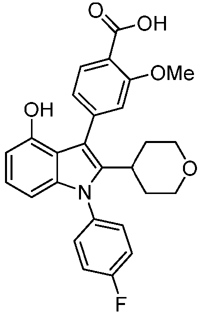 | | |
| **82** | From **S14** | 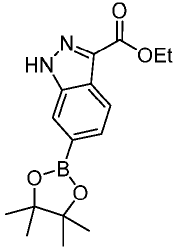 | LCMS *m*/*z* 472.0 [M+H]⁺. |
| | As for compound **1**^{1,7} | | |
| | 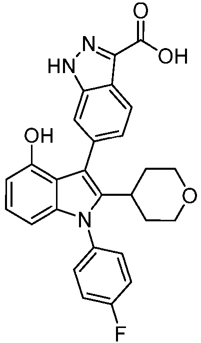 | | |
| **83** | From **S14** | 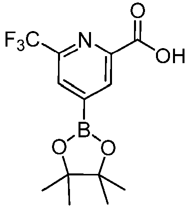 | ¹H NMR (400 MHz, DMSO-*d*₆) δ 9.68 (s, 1H), 8.29 (s, 1H), 8.09 (s, 1H), 7.57 (dd, J = 8.7, 5.1 Hz, 2H), 7.49 (t, J = 8.6 Hz, 2H), 6.92 (t, J = 8.0 Hz, 1H), 6.47 (d, J = 7.7 Hz, 1H), 6.23 (d, J = 8.2 Hz, 1H), 3.72 (dd, J = 12.0, 4.0 Hz, 2H), 3.08 (t, J = 11.4 Hz, 2H), 2.92 (t, J = 12.3 Hz, 1H), 1.65 (d, J = 12.7 Hz, 2H), 1.56 - 1.44 (m, 2H). LCMS *m*/*z* 501.0 [M+H]⁺. |
| | As for compound **1**^{1,7} | | |
| | 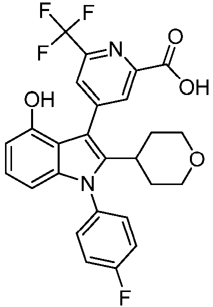 | | |
| 84 | From **S14** | 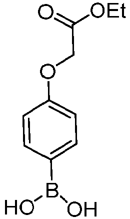 | ¹H NMR (400 MHz, DMSO-*d*₆) δ 9.01 (d, J = 1.9 Hz, 1H), 7.53 - 7.41 (m, 4H), 7.32 - 7.26 (m, 2H), 6.90 (d, J = 8.5 Hz, 2H), 6.80 (t, J = 7.9 Hz, 1H), 6.34 (d, J = 7.7 Hz, 1H), 6.19 (d, J = 8.1 Hz, 1H), 4.70 (s, 2H), 3.67 (d, J = 11.4 Hz, 2H), 3.00 (q, J = 8.2, 7.7 Hz, 2H), 2.77 (s, 1H), 1.52 (d, J = 7.9 Hz, 4H). LCMS *m*/*z* 462.0 [M+H]⁺. |
| | As for compound **1**^{1,7} | | |
| | 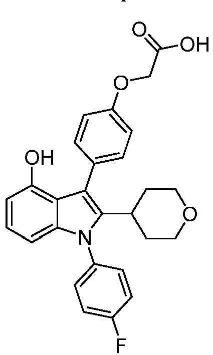 | | |
| **85** | From **S14** | 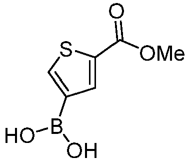 | ¹H NMR (400 MHz, DMSO-*d*₆) δ 9.33 (s, 1H), 7.67 (d, J = 3.7 Hz, 1H), 7.59 - 7.52 (m, 2H), 7.47 (t, J = 8.7 Hz, 2H), 7.09 (d, J = 3.7 Hz, 1H), 6.87 (t, J = 7.9 Hz, 1H), 6.42 (d, J = 7.7 Hz, 1H), 6.22 (d, J = 8.2 Hz, 1H), 3.79 - 3.67 (m, 2H), 3.11 - 3.01 (m, 2H), 2.94 - 2.70 (m, 1H), 1.62 (dt, J = 15.8, 11.4 Hz, 4H). LCMS *m*/*z* 438.0 [M+H]⁺. |
| | As for compound **1**^{1,7} | | |
| | 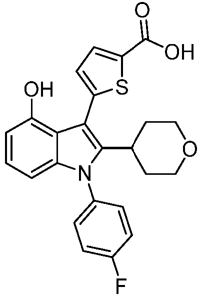 | | |
| **86** | From **S14** | 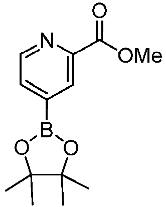 | LCMS *m*/*z* 433.0 [M+H]⁺. |
| | As for compound **1**^{1,7} | | |
| | 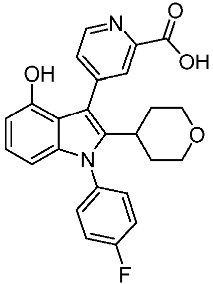 | | |
| **87** | From **S14** | 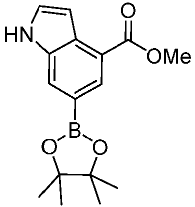 | ¹H NMR (400 MHz, DMSO-*d*₆) δ 11.34 (s, 1H), 8.97 (s, 1H), 7.79 (d, J = 1.5 Hz, 1H), 7.64 (d, J = 1.2 Hz, 1H), 7.55 (dd, J = 8.5, 5.1 Hz, 2H), 7.52 - 7.44 (m, 3H), 6.99 (t, J = 2.4 Hz, 1H), 6.83 (t, J = 7.9 Hz, 1H), 6.35 (d, J = 7.6 Hz, 1H), 6.24 (d, J = 8.1 Hz, 1H), 3.64 (d, J = 11.1 Hz, 2H), 3.01 - 2.93 (m, 2H), 2.79 (q, J = 8.2, 7.6 Hz, 1H), 1.56 (s, 5H). LCMS *m*/*z* 471.0 [M+H]⁺ |
| | As for compound **1**^{1,7} | | |
| | 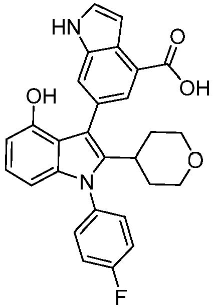 | | |
| **88** | From **S14** | 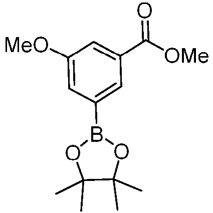 | ¹H NMR (400 MHz, Chloroform-*d*) δ 7.81 (dq, J = 4.2, 2.5, 2.0 Hz, 1H), 7.63 (dd, J = 4.3, 2.6 Hz, 1H), 7.35 (ddt, J = 9.3, 4.5, 2.2 Hz, 2H), 7.24 - 7.16 (m, 2H), 6.95 (td, J = 8.0, 2.5 Hz, 1H), 6.49 (dd, J = 7.6, 2.7 Hz, 1H), 6.47 - 6.38 (m, 1H), 3.92 - 3.83 (m, 3H), 3.81 - 3.68 (m, 2H), 3.13 (t, J = 11.4 Hz, 2H), 2.88 - 2.76 (m, 1H), 1.79 - 1.62 (m, 2H), 1.62 - 1.50 (m, 3H). LCMS *m*/*z* 462.6 [M+H]⁺. |
| | As for compound **1**. ^{1,7} | | |
| | 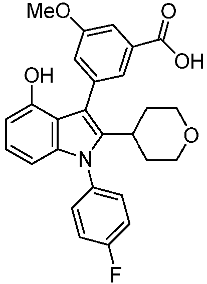 | | |
| **89** | From **S14** | 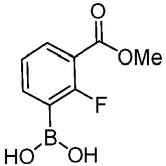 | ¹H NMR (400 MHz, DMSO-*d*₆) δ 9.12 (s, 1H), 7.83 (s, 1H), 7.66 - 7.40 (m, 4H), 7.28 (t, J = 7.6 Hz, 1H), 6.84 (t, J = 7.9 Hz, 1H), 6.34 (d, J = 7.6 Hz, 1H), 6.26 (d, J = 8.1 Hz, 1H), 3.67 (d, J = 11.2 Hz, 3H), 3.01 (d, J = 9.3 Hz, 2H), 2.76 - 2.66 (m, 1H), 1.62 - 1.40 (m, 4H). LCMS *m*/*z* 450.0 [M+H]⁺. |
| | As for compound **1**^{1,7} | | |
| | 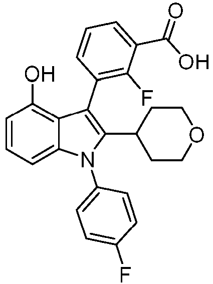 | | |
| **90** | From S14 | 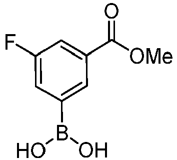 | ¹H NMR (400 MHz, Chloroform*-d*) δ 7.97 (t, J = 1.5 Hz, 1H), 7.72 (ddd, J = 9.0, 2.6, 1.4 Hz, 1H), 7.39 (ddd, J = 9.1, 2.6, 1.5 Hz, 1H), 7.33 (ddt, J = 8.2, 5.5, 2.7 Hz, 2H), 7.26 - 7.19 (m, 2H), 6.98 - 6.88 (m, 1H), 6.44 (dd, J = 7.7, 0.8 Hz, 1H), 6.37 (dd, J = 8.2, 0.8 Hz, 1H), 3.83 - 3.71 (m, 2H), 3.13 (td, J = 11.8, 2.1 Hz, 2H), 2.83 (dt, J = 12.2, 3.5 Hz, 1H), 1.64 (qd, J = 12.4, 4.3 Hz, 2H), 1.57 - 1.49 (m, 2H). LCMS *m*/*z* 450.32 [M+H]⁺. |
| | As for compound **1**^{1,9} | | |
| | 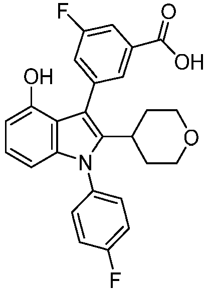 | | |
| **91** | From **S14** | 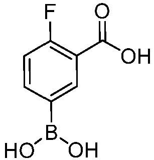 | ¹H NMR (400 MHz, Chloroform-*d*) δ 8.04 - 7.93 (m, 1H), 7.56 (d, J = 7.0 Hz, 1H), 7.28 (s, 2H), 7.17 (t, J = 8.3 Hz, 2H), 7.14 - 7.04 (m, 1H), 6.83 (t, J = 7.9 Hz, 1H), 6.36 (d, J = 7.6 Hz, 1H), 6.29 (d, J = 8.1 Hz, 1H), 3.27 (d, J = 3.3 Hz, 2H), 3.07 (t, J = 11.4 Hz, 2H), 2.84 - 2.68 (m, 1H), 1.58 (qd, J = 12.4, 4.7 Hz, 2H), 1.47 (d, J = 13.4 Hz, 2H). LCMS *m*/*z* 450.23 [M+H]⁺. |
| | As for compound **1**^{1,8} | | |
| | 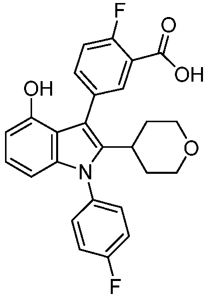 | | |
| **92** | From **S14** | 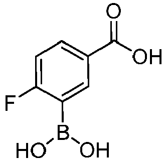 | ¹H NMR (400 MHz, Chloroform-*d*) δ 8.11 (dd, J = 7.2, 2.3 Hz, 1H), 8.01 (ddd, J = 8.5, 4.9, 2.3 Hz, 1H), 7.39 - 7.27 (m, 2H), 7.19 (ddd, J = 9.0, 7.9, 1.5 Hz, 2H), 7.12 (t, J = 8.7 Hz, 1H), 6.85 (t, J = 7.9 Hz, 1H), 6.35 (ddd, J = 12.7, 8.0, 0.9 Hz, 2H), 3.79 - 3.66 (m, 2H), 3.09 (tt, J = 11.2, 2.2 Hz, 2H), 2.80 - 2.64 (m, 1H), 1.74 - 1.42 (m, 4H). LCMS *m*/*z* 450.28 [M+H]⁺. |
| | As for compound **1**^{1,8} | | |
| | 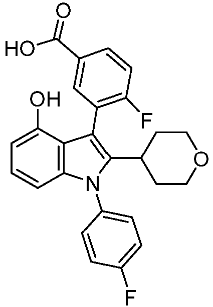 | | |
| **93** | **From S14** | 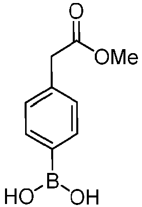 | LCMS *m*/*z* 446.0 [M+H]⁺. |
| | As for compound **1**^{1,7} | | |
| | 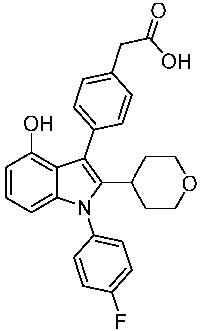 | | |
| **94** | From S14 | 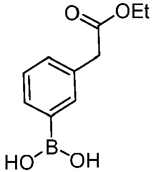 | LCMS *m*/*z* 446.0 [M+H]⁺. |
| | As for compound **1**^{1,7} | | |
| | 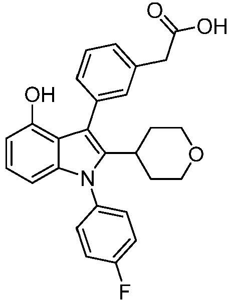 | | |
| **95** | From S14 | 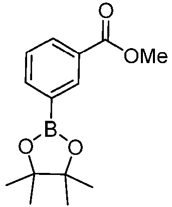 | LCMS *m*/*z* 432.0 [M+H]⁺. |
| | As for compound **1**^{1,7} | | |
| | 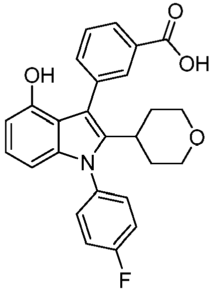 | | |
| **96** | From **S14** | 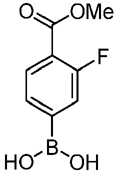 | ¹H NMR (400 MHz, DMSO-*d*₆) δ 9.29 (s, 1H), 7.85 (t, J = 8.0 Hz, 1H), 7.53 (ddd, J = 8.3, 5.3, 2.7 Hz, 2H), 7.51 - 7.43 (m, 2H), 7.34 - 7.25 (m, 2H), 6.86 (t, J = 7.9 Hz, 1H), 6.41 (d, J = 7.5 Hz, 1H), 6.25 - 6.18 (m, 1H), 3.73 - 3.64 (m, 2H), 3.04 (td, J = 11.4, 2.9 Hz, 2H), 2.83 (ddt, J = 11.5, 8.6, 4.3 Hz, 1H), 1.62 - 1.48 (m, 4H). LCMS *m*/*z* 450.0 [M+H]⁺ |
| | As for compound **1**¹ | | |
| | 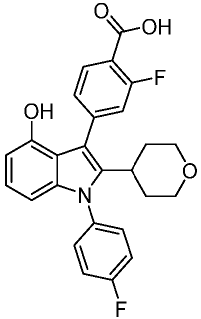 | | |
| **97** | From **S14** | 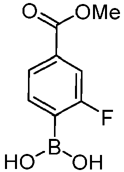 | ¹H NMR (400 MHz, DMSO-*d*₆) δ 9.15 (s, 1H), 7.78 (dd, J = 7.9, 1.7 Hz, 1H), 7.67 (dd, J = 9.8, 1.6 Hz, 1H), 7.60 - 7.50 (m, 3H), 7.50 - 7.43 (m, 2H), 6.85 (t, J = 7.9 Hz, 1H), 6.36 (d, J = 7.6 Hz, 1H), 6.25 (d, J = 8.1 Hz, 1H), 3.67 (td, J = 9.4, 8.6, 3.1 Hz, 2H), 3.01 (dt, J = 11.1, 9.0 Hz, 2H), 2.78 - 2.67 (m, 1H), 1.64 - 1.34 (m, 4H). LCMS *m*/*z* 450.0 [M+H]⁺. |
| | As for compound **1**¹ | | |
| | 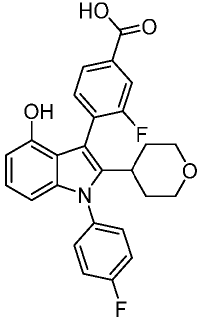 | | |
| **98** | From **S14** | 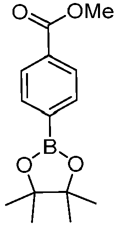 | ¹H NMR (400 MHz, DMSO-*d*₆) δ 9.17 (s, 1H), 7.99 - 7.89 (m, 2H), 7.59 - 7.39 (m, 6H), 6.90 - 6.77 (m, 1H), 6.39 (dd, J = 7.7, 0.8 Hz, 1H), 6.21 (dd, J = 8.2, 0.7 Hz, 1H), 3.67 (dt, J = 11.4, 3.0 Hz, 2H), 3.01 (td, J = 11.3, 4.5 Hz, 2H), 2.81 (tt, J = 10.7, 5.7 Hz, 1H), 1.64 - 1.43 (m, 4H). LCMS *m*/*z* 431.89 [M+H]⁺. |
| | As for compound **1**¹ | | |
| | 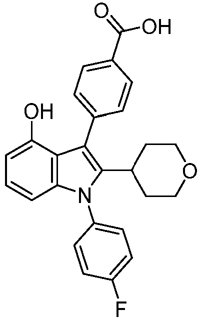 | | |
| **99** | From **S15** | 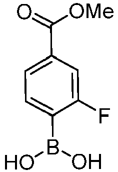 | ¹H NMR (300 MHz, DMSO-*d*₆) δ 13.24 (s, 1H), 9.25 (d, J = 1.6 Hz, 1H), 7.81 (dd, J = 7.8, 1.6 Hz, 1H), 7.71 (dd, J = 9.8, 1.7 Hz, 1H), 7.65 - 7.51 (m, 3H), 7.47 (ddd, J = 9.1, 7.5, 1.9 Hz, 2H), 6.91 (dd, J = 11.2, 8.8 Hz, 1H), 6.22 (dd, J = 8.8, 3.4 Hz, 1H), 3.77 - 3.56 (m, 2H), 3.15 - 2.90 (m, 2H), 2.82 - 2.61 (m, 1H), 1.71 - 1.47 (m, 3H), 1.41 (tt, J = 12.5, 6.3 Hz, 1H). LCMS *m*/*z* 468.06 [M+H]⁺. |
| | As for compound **1**¹ | | |
| | 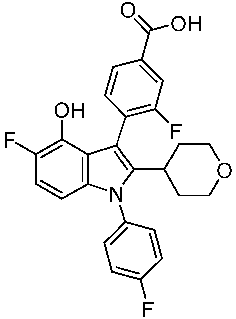 | | |
| **100** | From **S15** | 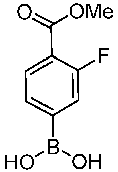 | ¹H NMR (400 MHz, DMSO-*d*₆) δ 9.36 (d, J = 1.8 Hz, 1H), 7.88 (t, J = 8.0 Hz, 1H), 7.55 (ddt, J = 8.3, 5.6, 2.8 Hz, 2H), 7.51 - 7.43 (m, 2H), 7.39 - 7.22 (m, 2H), 6.96 - 6.83 (m, 1H), 6.18 (dd, J = 8.8, 3.4 Hz, 1H), 4.38 (t, J = 5.0 Hz, 1H), 3.74 - 3.61 (m, 2H), 3.04 (td, J = 11.5, 2.5 Hz, 2H), 2.89 - 2.80 (m, 1H), 1.69 - 1.43 (m, 4H). |
| | As for compound **1**^{1,10} | | |
| | 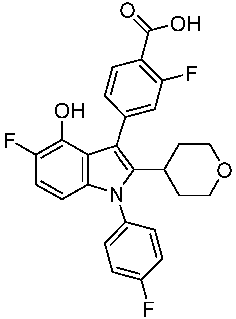 | | |
| **101** | From **S15** | 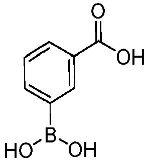 | ¹H NMR (400 MHz, Methanol-*d*₄) δ 8.10 (t, J = 1.8 Hz, 1H), 7.97 (dt, J = 7.8, 1.5 Hz, 1H), 7.62 (dt, J = 7.6, 1.5 Hz, 1H), 7.41 (t, J = 7.7 Hz, 1H), 7.30 (td, J = 4.7, 2.4 Hz, 2H), 7.25 - 7.14 (m, 2H), 6.77 (dd, J = 11.0, 8.8 Hz, 1H), 6.18 (dd, J = 8.8, 3.5 Hz, 1H), 3.78 - 3.66 (m, 2H), 3.09 (td, J = 11.7, 2.1 Hz, 2H), 2.78 (tt, J = 12.2, 3.4 Hz, 1H), 1.61 (qd, J = 12.3, 4.3 Hz, 2H), 1.50 (d, J = 12.9 Hz, 2H). LCMS *m*/*z* 450.37 [M+H]⁺. |
| | As for compound **1**^{1,10} | | |
| | 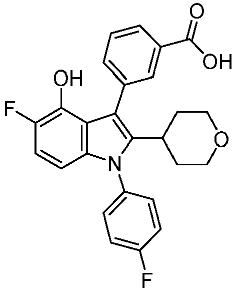 | | |
| | From **S15** | | ¹H NMR (400 MHz, DMSO-*d*₆) δ 12.89 (s, 1H), 9.22 (d, J = 1.9 Hz, 1H), 8.02 - 7.89 (m, 2H), 7.63 - 7.51 (m, 4H), 7.51 - 7.40 (m, 2H), 6.90 (dd, J = 11.2, 8.8 Hz, 1H), 6.17 (dd, J = 8.8, 3.5 Hz, 1H), 3.73 - 3.57 (m, 2H), 3.01 (td, J = 11.2, 3.0 Hz, 2H), 2.86 - 2.67 (m, 1H), 1.62 - 1.44 (m, 4H). LCMS *m*/*z* 450.28 [M+H]⁺ |
| | As for compound **1**^{1,10} | | |
| **102** | 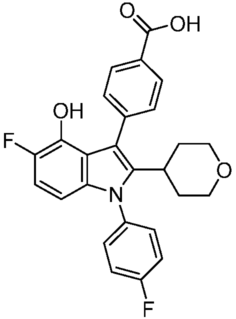 | 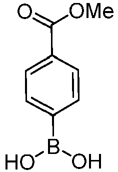 | |

| | | | |
|---|---|---|---|
| **1**. Suzuki Conditions: Pd(dppf)Cl₂, Na₂CO₃ or NaHCO₃, in DMF at 130 °C **2.** Purification by silica gel chromatography (Gradient: 0-7% MeOH in dichloromethane) yielded the product. **3.** Purification by reversed-phase chromatography (Column: C18. Gradient: 5-100% MeCN in water with 0.1% trifluoroacetic acid) afforded the product. **4.** Purification by reversed-phase chromatography (Column: C18. Gradient: 5-95% MeCN in water with 0.1% trifluoroacetic acid) afforded the product. **5.** Purification by silica gel chromatography (Gradient: 0-10% MeOH in dichloromethane) yielded the product. **6.** Purification by silica gel chromatography (Gradient: 0-20% MeOH in dichloromethane) yielded the product. **7.** Benzyl group remove with Pd/C and ammonium formate in EtOH **8.** Hydrogenation was performed with Pd(OH)₂ on carbon and hydrogen gas. Purification by reversed-phase HPLC. Method: C18 Waters Sunfire column (30 x 150 mm, 5 micron). Gradient: MeCN in H₂O with 0.1% trifluoroacetic acid. **9.** Benzyl group deprotection was performed by treatment with AlBr₃. **10**. Purification by silica gel chromatography (Gradient: 10-90% EtOAc in heptane) yielded the product. | | | |

### Compound 103

### 6-[1-(4-fluorophenyl)-4-hydroxy-2-tetrahydropyran-4-yl-indol-3-yl]spiro[3.3]heptane-2-carboxylic acid (103)

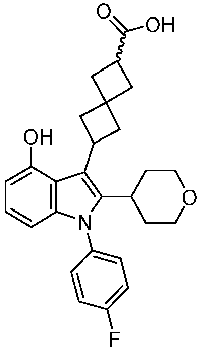

Compound **103** was prepared from 4-allyloxy-1-(4-fluorophenyl)-2-tetrahydropyran-4-yl-indole and methyl 2-oxospiro[3.3]heptane-6-carboxylate (55 mg, 0.33 mmol) by a reductive alkylation as described for **C116** in the preparation of compound 54. Ester hydrolysis using LiOH, then allyl group removal using standard allyl group deprotection conditions (Pd(PPh₃)₄ and 1,3-dimethylbarbituric acid in 1,4-dioxane and water) afforded the product. Purification was performed using reverse-phase HPLC and then SFC. ¹H NMR (400 MHz, Chloroform-d) δ 7.14 (dt, J = 7.9, 4.1 Hz, 4H), 6.80 (t, J = 7.9 Hz, 1H), 6.39 (d, J = 7.5 Hz, 1H), 6.24 (d, J = 8.1 Hz, 1H), 3.92 (dd, J = 11.6, 4.2 Hz, 2H), 3.84 (q, J = 9.3 Hz, 1H), 3.25 - 3.15 (m, 2H), 3.08 - 2.89 (m, 2H), 2.85 (t, J = 10.4 Hz, 1H), 2.71 (tt, J = 12.5, 3.8 Hz, 1H), 2.48 - 2.30 (m, 3H), 2.27 (dd, J = 8.6, 2.4 Hz, 2H), 2.18 (ddd, J = 11.8, 8.9, 3.7 Hz, 1H), 2.09 - 1.98 (m, 2H), 1.53 (d, J = 13.4 Hz, 2H). LCMS *m*/*z* 450.37 [M+H]⁺

### Compound 104

### 4-[5-cyano-1-(4-fluorophenyl)-4-hydroxy-2-tetrahydropyran-4-yl-indol-3-yl]benzoic acid (104)

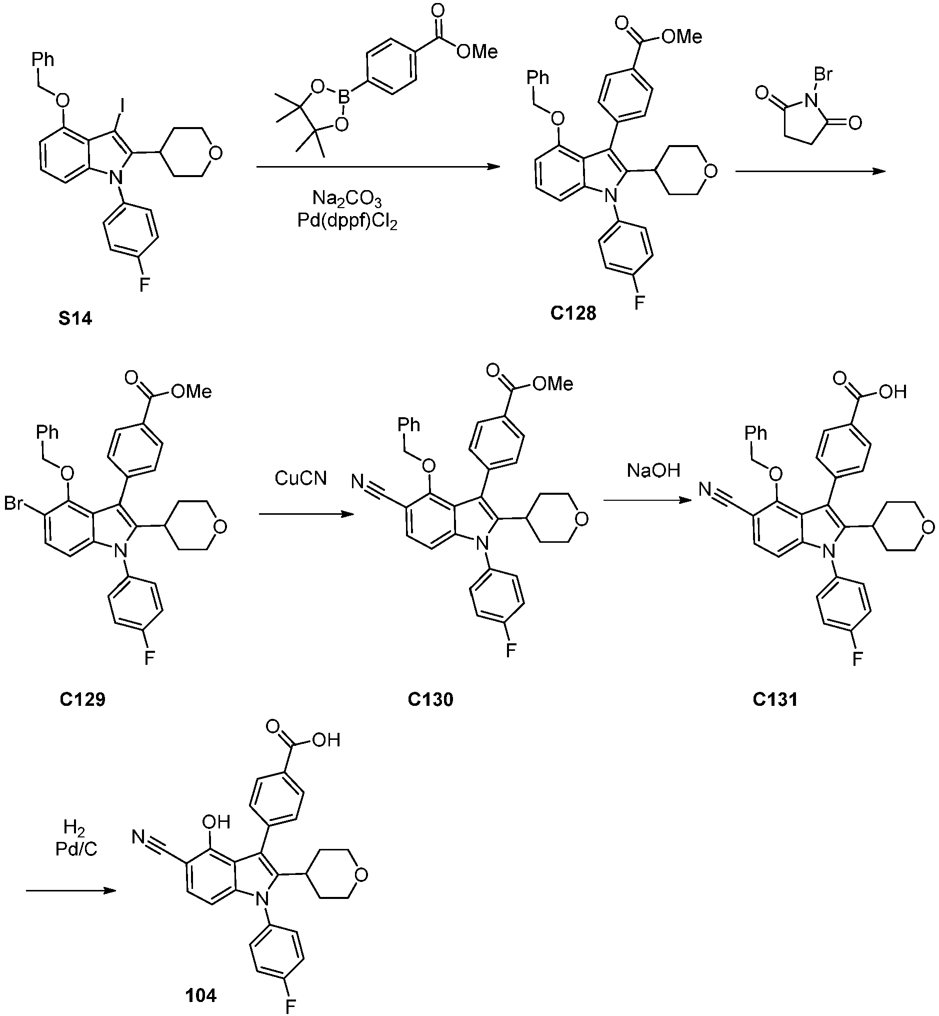

### Step 1. Synthesis of methyl 4-[4-benzyloxy-1-(4-fluorophenyl)-2-tetrahydropyran-4-yl-indol-3-yl]benzoate (C128)

Compound **128** was prepared from **S4** indole (20 g, 37.9 mmol) and methyl 4-(4,4,5,5-tetramethyl-1,3,2-dioxaborolan-2-yl)benzoate using the method described in the synthesis of compound 1. Silica gel chromatography (Gradient: 0-2% MeOH-dichloromethane) afforded the product (10.7g ~90% pure) which was used directly in the subsequent step without further purification. Methyl 4-[4-benzyloxy-1-(4-fluorophenyl)-2-tetrahydropyran-4-yl-indol-3-yl]benzoate (10.7 g, 47%). LCMS *m*/*z* 536.11 [M+1]+;

### Step 2. Synthesis of methyl 4-[4-benzyloxy-5-bromo-1-(4-fluorophenyl)-2-tetrahydropyran-4-yl-indol-3-yl]benzoate (C129)

To a solution of methyl 4-[4-benzyloxy-1-(4-fluorophenyl)-2-tetrahydropyran-4-yl-indol-3-yl]benzoate (50 mg, 0.093 mmol) in dichloromethane (1 mL) at 0 °C was added dropwise a solution of N-bromosuccinimide (17 mg, 0.10 mmol) in dichloromethane (400 µL). The reaction mixture was stirred at 0 °C for 10 minutes, then washed with water. The organic layer was concentrated to dryness and purified via silica gel chromatography (Gradient: 0-35% EtOAc in heptane) to afford the product as a white solid. methyl 4-[4-benzyloxy-5-bromo-1-(4-fluorophenyl)-2-tetrahydropyran-4-yl-indol-3-yl]benzoate (44 mg, 73%). ¹H NMR (400 MHz, Chloroform-d) δ 8.00 - 7.95 (m, 2H), 7.51 - 7.46 (m, 2H), 7.42 - 7.37 (m, 2H), 7.25 - 7.11 (m, 6H), 6.81 - 6.74 (m, 2H), 6.47 (d, J = 8.4 Hz, 1H), 4.87 (s, 2H), 4.00 (s, 3H), 3.80 - 3.73 (m, 2H), 3.07 (td, J = 11.8, 2.0 Hz, 2H), 2.67 (tt, J = 12.3, 3.4 Hz, 1H), 1.66 - 1.60 (m, 2H), 1.53 - 1.43 (m, 2H). LCMS *m*/*z* 614.0 [M+H]⁺.

### Step 3. Synthesis of methyl 4-[4-benzyloxy-5-cyano-1-(4-fluorophenyl)-2-tetrahydropyran-4-yl-indol-3-yl]benzoate (C130)

Methyl 4-[4-benzyloxy-5-bromo-1-(4-fluorophenyl)-2-tetrahydropyran-4-yl-indol-3-yl]benzoate (442 mg, 0.72 mmol) and cyanocopper (0.13 g, 1.43 mmol) were placed in a vial under nitrogen. The vial was purged with one cycle of vacuum then backfilling with nitrogen. NMP (8 mL) The mixture was then heated thermally at 180 °C overnight. The mixture was diluted into aqueous saturated NH₄Cl solution and extracted with EtOAc (x 2). The organic phase was dried (MgSO₄), filtered and concentrated *in vacuo.* The reaction mixture was purified by reversed-phase chromatography (Column: C18. Gradient: 60-100% MeCN in water with 0.1% trifluoroacetic acid). The desired fractions were concentrated *in vacuo,* diluted with dichloromethane and neutralized with aqueous saturated NaHCO₃ solution. The mixture was passed through a phase separator and resulting organic phase concentrated *in vacuo* to afford the product as a tan solid (43% yield). methyl 4-[4-benzyloxy-5-cyano-1-(4-fluorophenyl)-2-tetrahydropyran-4-yl-indol-3-yl]benzoate (52 mg, 33%) . LCMS *m*/*z* 1056.97 [M+H]⁺.

### Step 4. Synthesis of 4-[4-benzyloxy-5-cyano-1-(4-fluorophenyl)-2-tetrahydropyran-4-yl-indol-3-yl]benzoic acid (C131)

Compound **C131** was prepared from **C130** methyl 4-[4-benzyloxy-5-cyano-1-(4-fluorophenyl)-2-tetrahydropyran-4-yl-indol-3-yl]benzoate (135 mg, 0.16 mmol) by hydrolysis using the method described in the synthesis of compound **1.** NaOH was used instead of LiOH. 4-[4-benzyloxy-5-cyano-1-(4-fluorophenyl)-2-tetrahydropyran-4-yl-indol-3-yl]benzoic acid (41 mg, 46%) ¹H NMR (400 MHz, DMSO-*d*₆) δ 7.96 - 7.87 (m, 2H), 7.70 - 7.61 (m, 2H), 7.56 (d, J = 8.3 Hz, 1H), 7.45 (dd, J = 9.9, 7.7 Hz, 4H), 7.20 - 7.06 (m, 3H), 6.83 (d, J = 8.5 Hz, 1H), 6.76 - 6.65 (m, 2H), 5.05 (s, 2H), 3.64 (d, J = 10.4 Hz, 2H), 2.95 (t, J = 11.1 Hz, 2H), 2.71 - 2.60 (m, 1H), 1.61 - 1.36 (m, 4H). LCMS *m*/*z* 547.46 [M+H]⁺.

### Step 5. Synthesis of 4-[5-cyano-1-(4-fluorophenyl)-4-hydroxy-2-tetrahydropyran-4-yl-indol-3-yl]benzoic acid

Compound **104** was prepared from 4-[4-benzyloxy-5-cyano-1-(4-fluorophenyl)-2-tetrahydropyran-4-yl-indol-3-yl]benzoic acid **(C131)** by benzyl group removal by hydrogenation using the conditions described for the preparation of 4-[5-cyano-1-(4-fluorophenyl)-4-hydroxy-2-tetrahydropyran-4-yl-indol-3-yl]benzoic acid (15 mg, 42%). ¹H NMR (400 MHz, DMSO-*d*₆) δ 11.02 (s, 1H), 7.93 (d, J = 7.9 Hz, 2H), 7.68 - 7.60 (m, 2H), 7.43 (td, J = 8.4, 1.7 Hz, 4H), 7.34 (d, J = 8.2 Hz, 1H), 6.57 (d, J = 8.2 Hz, 1H), 3.64 (dd, J = 8.6, 5.6 Hz, 2H), 2.96 (td, J = 11.1, 3.6 Hz, 2H), 2.73 - 2.66 (m, 1H), 1.51 (q, J = 4.5, 4.1 Hz, 4H). LCMS *m*/*z* 457.0 [M+H]⁺.

### Compound 105

### 4-[1-(4-fluorophenyl)-4-hydroxy-5-methyl-2-tetrahydropyran-4-yl-indol-3-yl]benzoic acid (105)

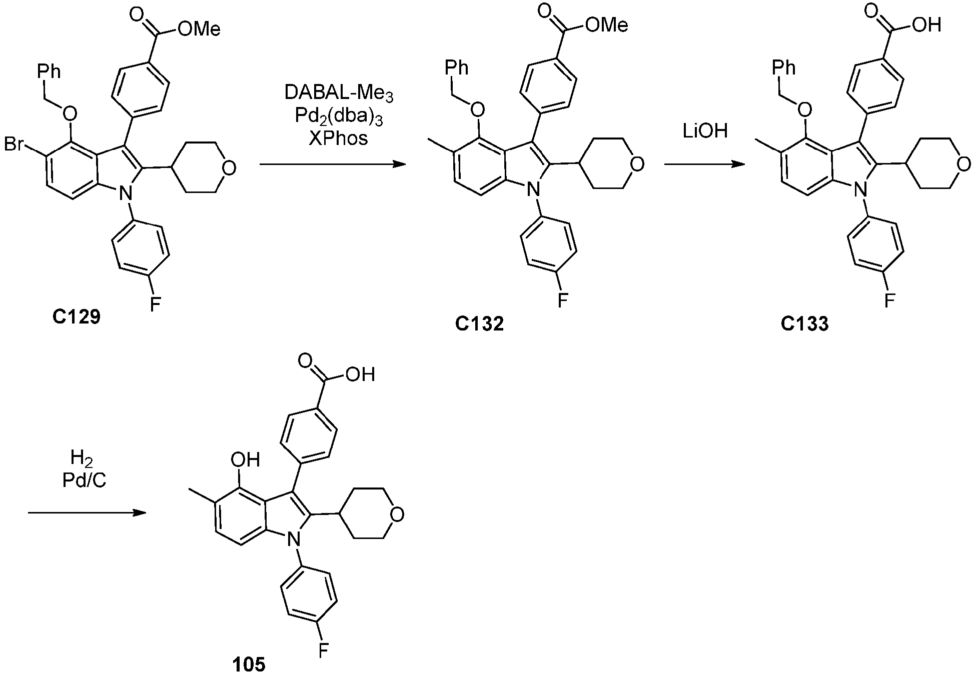

### Step 1.Synthesis of methyl 4-[4-benzyloxy-1-(4-fluorophenyl)-5-methyl-2-tetrahydropyran-4-yl-indol-3-yl]benzoate (C132)

A mixture of methyl 4-[4-benzyloxy-5-bromo-1-(4-fluorophenyl)-2-tetrahydropyran-4-yl-indol-3-yl]benzoate **C129** (160 mg, 0.26 mmol), DABAL-Me₃ (100 mg, 0.39 mmol), Pd₂(dba)₃ (10 mg, 0.011 mmol) and XPhos (10 mg, 0.021 mmol) were dissolved in THF (2 mL) and stirred at 85 °C in a sealed tube for 1 hour. The reaction mixture was treated carefully with 5 mL 2 M aq. HCl and extracted twice with MTBE. The combined organic layers were concentrated to dryness and purified via silica gel chromatography (Gradient: 0-35% EtOAc in heptane) to afford the product as a white solid. methyl 4-[4-benzyloxy-1-(4-fluorophenyl)-5-methyl-2-tetrahydropyran-4-yl-indol-3-yl]benzoate (125 mg, 87%). ¹H NMR (400 MHz, Chloroform-d) δ 8.01 - 7.92 (m, 2H), 7.54 - 7.49 (m, 2H), 7.49 - 7.39 (m, 2H), 7.25 - 7.10 (m, 5H), 6.84 - 6.75 (m, 3H), 6.50 (d, J = 7.9 Hz, 1H), 4.88 (s, 2H), 4.00 (s, 3H), 3.76 (dd, J = 11.4, 4.1 Hz, 2H), 3.14 - 3.02 (m, 2H), 2.69 - 2.59 (m, 2H), 1.79 (d, J = 0.9 Hz, 3H), 1.64 (dd, J = 12.7, 4.2 Hz, 1H), 1.48 (d, J = 13.1 Hz, 2H). LCMS *m*/*z* 550.0 [M+H]⁺.

### Step 2. Synthesis of 4-[4-benzyloxy-1-(4-fluorophenyl)-5-methyl-2-tetrahydropyran-4-yl-indol-3-yl]benzoic acid (C133)

Compound **C133** was prepared from methyl 4-[4-benzyloxy-1-(4-fluorophenyl)-5-methyl-2-tetrahydropyran-4-yl-indol-3-yl]benzoate **C132** (125 mg, 0.23 mmol) by hydrolysis with LiOH using the method described in the preparation of compound 1. 4-[4-benzyloxy-1-(4-fluorophenyl)-5-methyl-2-tetrahydropyran-4-yl-indol-3-yl]benzoic acid (98 mg, 80%). ¹H NMR (400 MHz, DMSO-*d*₆) δ 7.95 - 7.91 (m, 2H), 7.63 - 7.59 (m, 2H), 7.52 - 7.49 (m, 2H), 7.44 - 7.41 (m, 1H), 7.36 - 7.30 (m, 1H), 7.17 - 7.12 (m, 1H), 7.10 - 7.05 (m, 2H), 6.75 (dd, J = 7.8, 1.0 Hz, 1H), 6.69 - 6.66 (m, 2H), 6.53 (d, J = 8.0 Hz, 1H), 4.87 (s, 2H), 3.67 - 3.59 (m, 2H), 2.97 - 2.86 (m, 2H), 2.61 - 2.51 (m, 1H), 1.70 (d, J = 0.9 Hz, 3H), 1.46 (d, J = 12.4 Hz, 4H). LCMS *m*/*z* 536.0 [M+H]⁺.

### Step 3. Synthesis of 4-[1-(4-fluorophenyl)-4-hydroxy-5-methyl-2-tetrahydropyran-4-yl-indol-3-yl]benzoic acid (105)

Compound **105** was prepared from **C133** 4-[4-benzyloxy-1-(4-fluorophenyl)-5-methyl-2-tetrahydropyran-4-yl-indol-3-yl]benzoic acid (98 mg, 0.18 mmol) by hydrogenation using the method described for the preparation of compound **1.** 4-[1-(4-fluorophenyl)-4-hydroxy-5-methyl-2-tetrahydropyran-4-yl-indol-3-yl]benzoic acid (10 mg, 12%). Product contains ~5% des-methyl compound. ¹H NMR (400 MHz, DMSO-*d*₆) δ 8.81 (s, 1H), 7.96 - 7.88 (m, 2H), 7.62 - 7.54 (m, 2H), 7.51 - 7.45 (m, 2H), 7.38 (t, J = 8.7 Hz, 2H), 6.58 (d, J = 2.3 Hz, 1H), 6.24 (d, J = 7.7 Hz, 1H), 3.64 (d, J = 11.6 Hz, 2H), 2.67 - 2.52 (m, 1H), 1.66 - 1.60 (m, 3H), 1.48 (d, J = 8.2 Hz, 6H). LCMS *m*/*z* 446.0 [M+H]⁺.

### Compound 106

### 4-[5-chloro-1-(4-fluorophenyl)-4-hydroxy-2-tetrahydropyran-4-yl-indol-3-yl]benzoic acid (106)

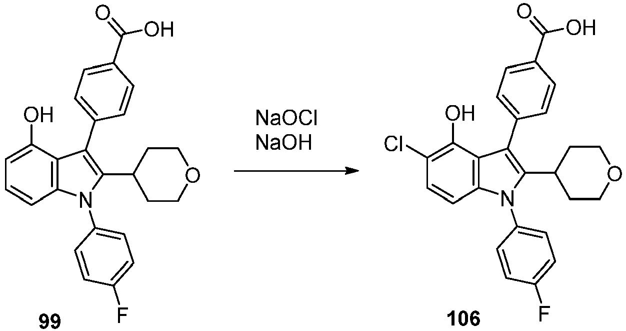

4-[1-(4-fluorophenyl)-4-hydroxy-2-tetrahydropyran-4-yl-indol-3-yl]benzoic acid **99** (100 mg, 0.24 mmol) was suspended in NaOH (3 mL of 1 M, 3.0 mmol) and sodium hypochlorite (518 mg of 5% w/w, 0.35 mmol) was added. The reaction was stirred at room temperature overnight. The reaction was concentrated, diluted with EtOAc and washed with water. The organic layer was dried and concentrated. Purification by silica gel chromatography (4 g column, Gradient: 10-40% EtOAc in hexane) to afford the desired product. 4-[5-chloro-1-(4-fluorophenyl)-4-hydroxy-2-tetrahydropyran-4-yl-indol-3-yl]benzoic acid (40 mg, 35%). ¹H NMR (400 MHz, Methanol-*d*₄) δ 8.11 - 7.96 (m, 2H), 7.60 - 7.50 (m, 2H), 7.50 - 7.42 (m, 2H), 7.42 - 7.32 (m, 2H), 6.99 (d, J = 8.7 Hz, 1H), 6.34 (d, J = 8.7 Hz, 1H), 4.12 (q, J = 7.1 Hz, 2H), 3.86 - 3.69 (m, 2H), 3.15 (td, J = 11.7, 2.1 Hz, 2H), 2.90 (tt, J = 12.2, 3.6 Hz, 1H), 2.03 (s, 3H), 1.71 (qd, J = 12.4, 11.9, 4.2 Hz, 2H), 1.64 - 1.52 (m, 2H). LCMS *m*/*z* 466.26 [M+H]⁺.

### Compound 107-110

Compounds **107-110** were prepared from **S16** as described for the preparation of compound **1.** Any exceptions to this method are noted in the table footnotes.

**Table 7. Method of preparation, structure, physicochemical data for compounds 107-110.**

| **Compound** | **Method/Product** | **Boronic acid or ester** | **¹H NMR; LCMS m/z [M+H]⁺** |
|---|---|---|---|
| **107** | From **S16** | 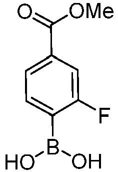 | ¹H NMR (400 MHz, DMSO-*d*₆) δ 13.25 (s, 1H), 9.71 (s, 1H), 7.80 (dd, J = 7.9, 1.6 Hz, 1H), 7.69 (dd, J = 9.8, 1.6 Hz, 1H), 7.63 - 7.51 (m, 3H), 7.47 (tt, J = 8.6, 1.3 Hz, 2H), 6.21 (dd, J = 11.4, 2.2 Hz, 1H), 6.01 (dd, J = 9.6, 2.2 Hz, 1H), 3.66 (dd, J = 19.4, 3.6 Hz, 2H), 3.08 - 2.92 (m, 2H), 2.78 - 2.62 (m, 1H), 1.69 - 1.43 (m, 3H), 1.38 (qd, J = 12.1, 4.3 Hz, 1H). LCMS *m*/*z* 468.06 [M+H]⁺. |
| | As for compound **1**. ^{1,2} | | |
| | 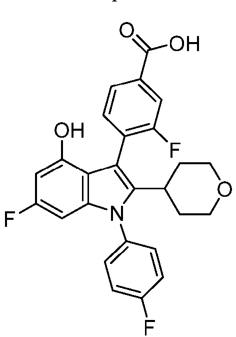 | | |
| **108** | From **S16** | 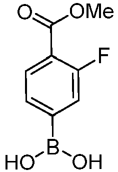 | ¹H NMR (400 MHz, Chloroform-*d*) δ 7.87 (t, J = 7.9 Hz, 1H), 7.26 (dd, J = 5.8, 2.9 Hz, 3H), 7.23 - 7.13 (m, 3H), 6.17 (dd, J = 11.1, 2.2 Hz, 1H), 5.93 (dd, J = 9.5, 2.1 Hz, 1H), 3.79 - 3.62 (m, 2H), 3.18 - 3.04 (m, 2H), 2.85 - 2.69 (m, 1H), 1.69 - 1.55 (m, 2H), 1.55 - 1.39 (m, 2H). LCMS *m*/*z* 468.33 [M+H]⁺. |
| | As for compound **1.** ^{3,2} | | |
| | 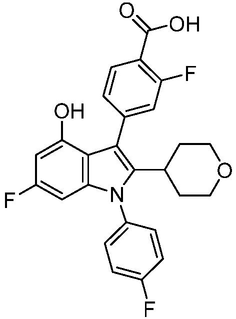 | | |
| **109** | From **S16** | 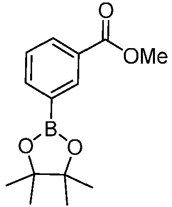 | ¹H NMR (400 MHz, Methanol-*d*₄) δ 8.08 (td, J = 1.8, 0.5 Hz, 1H), 7.99 (ddd, J = 7.8, 1.8, 1.3 Hz, 1H), 7.65 (ddd, J = 7.6, 1.8, 1.2 Hz, 1H), 7.48 (dd, J = 7.7, 0.5 Hz, 1H), 7.46 - 7.40 (m, 2H), 7.38 - 7.31 (m, 2H), 6.17 (dd, J = 11.3, 2.1 Hz, 1H), 5.97 (dd, J = 9.6, 2.2 Hz, 1H), 3.80 - 3.64 (m, 2H), 3.12 (td, J = 11.6, 2.4 Hz, 2H), 2.84 (tt, J = 11.9, 3.8 Hz, 1H), 1.73 - 1.61 (m, 2H), 1.61 - 1.51 (m, 2H). LCMS *m*/*z* 450.54 [M+H]⁺. |
| | As for compound **1**^{1,2} | | |
| | 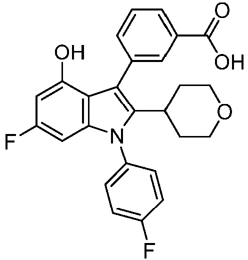 | | |
| **110** | From **S16** | 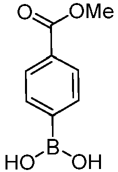 | ¹H NMR (400 MHz, DMSO-*d*₆) δ 12.89 (s, 1H), 9.72 (s, 1H), 8.04 - 7.88 (m, 2H), 7.61 - 7.36 (m, 6H), 6.24 (dd, J = 11.4, 2.2 Hz, 1H), 5.96 (dd, J = 9.6, 2.2 Hz, 1H), 3.72 - 3.60 (m, 2H), 3.00 (td, J = 11.3, 2.8 Hz, 2H), 2.77 (ddt, J = 11.5, 8.4, 4.2 Hz, 1H), 1.62 - 1.42 (m, 4H). LCMS *m*/*z* 450.31 [M+H]⁺. |
| | As for compound **1**^{1,2} | | |
| | 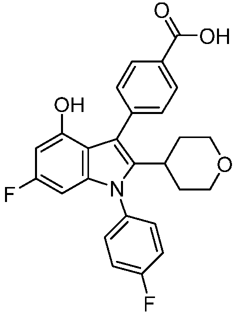 | | |

| | | | |
|---|---|---|---|
| **1.** Suzuki Conditions: Pd₂(dba)₃, SPhos, K₃PO₄ in THF at 80 °C. **2.** Purification by silica gel chromatography (Gradient: 10-90% EtOAc in heptane) yielded the product. **3.** Suzuki Conditions: Pd(PPh₃)₄, CsF in DME at 100 °C. | | | |

### Compound 111

### 4-[1-(4-fluorophenyl)-4-hydroxy-2-tetrahydropyran-4-yl-6-(trifluoromethyl)indol-3-yl]benzoic acid (111)

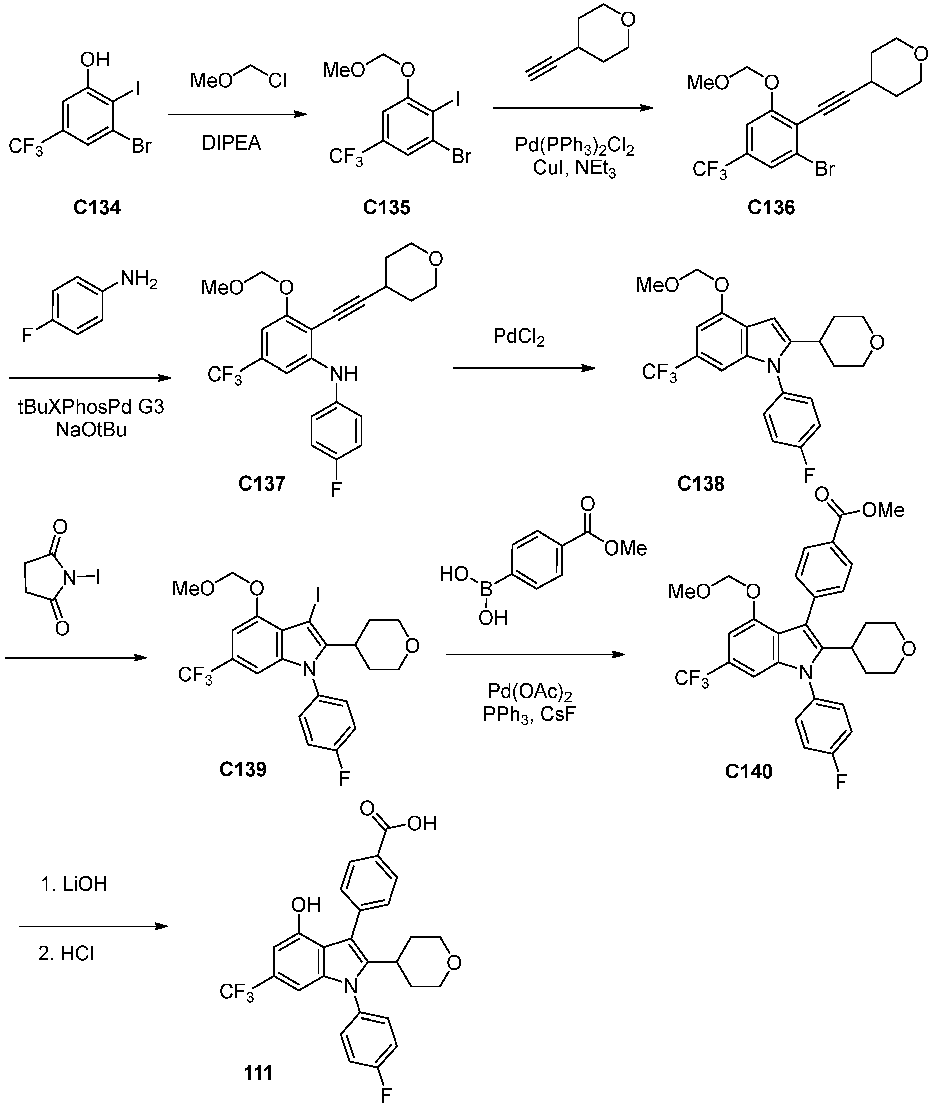

Compound 111 was prepared from C134 using the method described for the preparation of compound 12. The Suzuki cross coupling step for the conversion of C139 to C140 was performed using Pd(OAc)₂, PPh₃ and CsF, as described for synthesis of C73 in the preparation of compound 3. 4-[1-(4-fluorophenyl)-4-hydroxy-2-tetrahydropyran-4-yl-6-(trifluoromethyl)indol-3-yl]benzoic acid (41.5 mg, 66%). ¹H NMR (400 MHz, DMSO-*d*₆) δ 12.94 (s, 1H), 9.89 (s, 1H), 8.01 - 7.90 (m, 2H), 7.68 - 7.56 (m, 2H), 7.57 - 7.44 (m, 4H), 6.64 (d, J = 1.5 Hz, 1H), 6.49 (s, 1H), 3.67 (d, J = 11.1 Hz, 2H), 3.07 - 2.95 (m, 2H), 2.88 - 2.75 (m, 1H), 1.62 - 1.42 (m, 4H). LCMS *m*/*z* 500.05 [M+H]⁺.

### Compound 112

### 4-[6-cyano-1-(4-fluorophenyl)-4-hydroxy-2-tetrahydropyran-4-yl-indol-3-yl]benzoic acid (112)

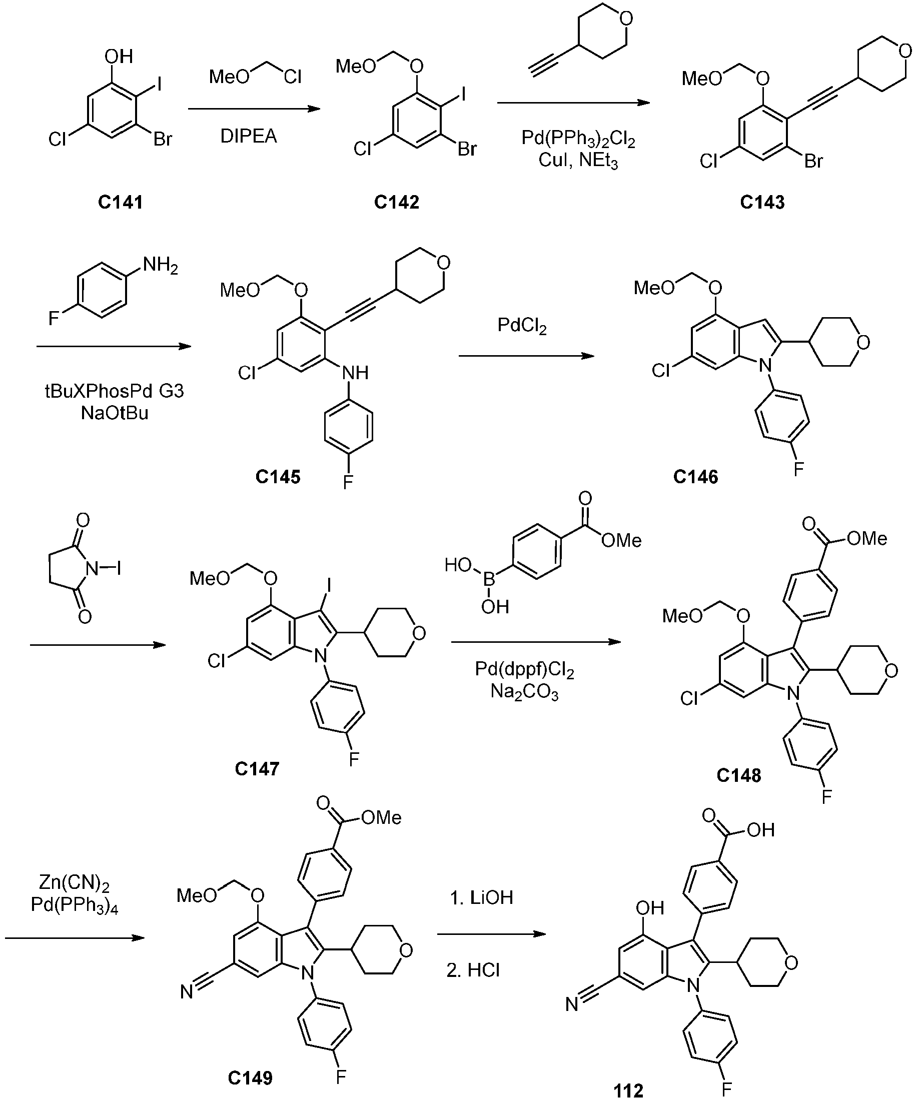

### Synthesis of methyl 4-[6-chloro-1-(4-fluorophenyl)-4-(methoxymethoxy)-2-tetrahydropyran-4-yl-indol-3-yl]benzoate (C148)

Compound **C148** was prepared in 6 steps from **C141** using the method described for the synthesis of **C89** in the preparation of compound **12.** Methyl 4-[6-chloro-1-(4-fluorophenyl)-4-(methoxymethoxy)-2-tetrahydropyran-4-yl-indol-3-yl]benzoate (190 mg, 77%). ¹H NMR (400 MHz, DMSO-*d*₆) δ 8.04 - 7.96 (m, 2H), 7.63 - 7.53 (m, 4H), 7.53 - 7.46 (m, 2H), 6.68 (d, J = 1.7 Hz, 1H), 6.43 (d, J = 1.7 Hz, 1H), 4.94 (s, 2H), 3.89 (s, 3H), 3.66 (dd, J = 11.3, 3.8 Hz, 2H), 3.08 (s, 3H), 3.00 (dd, J = 12.2, 9.9 Hz, 2H), 2.83 - 2.72 (m, 1H), 1.62 - 1.40 (m, 4H). LCMS *m*/*z* 524.17 [M+H]⁺.

### Step 6. Synthesis of methyl 4-[6-cyano-1-(4-fluorophenyl)-4-(methoxymethoxy)-2-tetrahydropyran-4-yl-indol-3-yl]benzoate (C149)

A mixture of methyl 4-[6-chloro-1-(4-fluorophenyl)-4-(methoxymethoxy)-2-tetrahydropyran-4-yl-indol-3-yl]benzoate C**148** (260 mg, 0.49 mmol), dicyanozinc (120 mg, 1.0 mmol), and Pd(PPh₃)₄ (115 mg, 0.10 mmol) in DMF (3 mL) was heated to 95 °C and allowed to stir for 36 hours. The mixture was diluted with EtOAc and washed with water (3 x), brine, dried over sodium sulfate and concentrated under reduced pressure. Purification by silica gel chromatography (0-35% EtOAc in heptane) afforded the product. Methyl 4-[6-cyano-1-(4-fluorophenyl)-4-(methoxymethoxy)-2-tetrahydropyran-4-yl-indol-3-yl]benzoate (37 mg, 14%). ¹H NMR (400 MHz, DMSO-*d*₆) δ 8.05 - 7.99 (m, 2H), 7.63 (dd, J = 8.9, 5.0 Hz, 2H), 7.60 - 7.55 (m, 3H), 7.49 (dd, J = 5.4, 3.2 Hz, 1H), 6.98 - 6.93 (m, 2H), 4.99 (s, 2H), 3.90 (s, 3H), 3.66 (d, J = 9.8 Hz, 2H), 3.06 (s, 3H), 3.00 (t, J = 11.1 Hz, 2H), 2.80 (d, J = 13.0 Hz, 1H), 1.61 - 1.50 (m, 4H). LCMS *m*/*z* 510.22 [M+H]⁺.

### Step 7. Synthesis of 4-[6-cyano-1-(4-fluorophenyl)-4-hydroxy-2-tetrahydropyran-4-yl-indol-3-yl]benzoic acid (112)

Compound **112** was prepared from **C149** by ester hydrolysis using LiOH, then MOM deprotection using HCl as described in the preparation of compound **12.** 4-[6-cyano-1-(4-fluorophenyl)-4-hydroxy-2-tetrahydropyran-4-yl-indol-3-yl]benzoic acid (13 mg, 43%). ¹H NMR (400 MHz, DMSO-*d*₆) δ 12.95 (s, 1H), 9.97 (s, 1H), 8.00 - 7.92 (m, 2H), 7.65 - 7.57 (m, 2H), 7.56 - 7.45 (m, 4H), 6.71 (d, J = 1.3 Hz, 1H), 6.62 (d, J = 1.3 Hz, 1H), 3.72 - 3.61 (m, 2H), 3.08 - 2.95 (m, 2H), 2.88 - 2.77 (m, 1H), 1.62 - 1.44 (m, 4H). LCMS *m*/*z* 457.13 [M+H]⁺.

### Compound 113

### 4-[6-chloro-1-(4-fluorophenyl)-4-hydroxy-2-tetrahydropyran-4-yl-indol-3-yl]benzoic acid (113)

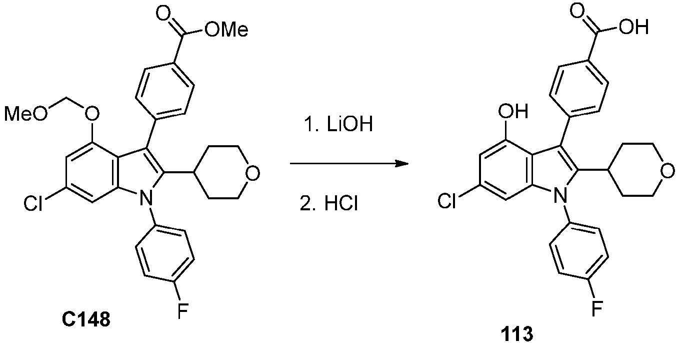

Compound 113 was prepared from **C148** methyl 4-[6-chloro-1-(4-fluorophenyl)-4-(methoxymethoxy)-2-tetrahydropyran-4-yl-indol-3-yl]benzoate (70 mg, 0.13 mmol) as described for the preparation of compound 12. 4-[6-chloro-1-(4-fluorophenyl)-4-hydroxy-2-tetrahydropyran-4-yl-indol-3-yl]benzoic acid (23.7 mg, 38%). ¹H NMR (400 MHz, DMSO-*d*₆) δ 12.91 (s, 1H), 9.71 (s, 1H), 7.99 - 7.90 (m, 2H), 7.62 - 7.54 (m, 2H), 7.54 - 7.43 (m, 4H), 6.41 (d, J = 1.8 Hz, 1H), 6.20 (d, J = 1.7 Hz, 1H), 3.66 (d, J = 11.1 Hz, 2H), 3.06 - 2.94 (m, 2H), 2.83 - 2.72 (m, 1H), 1.60 - 1.42 (m, 4H). LCMS *m*/*z* 465.86 [M+H]⁺.

### Compound 114

### 4-[6-fluoro-1-(4-fluorophenyl)-4-hydroxy-2-(trifluoromethyl)indol-3-yl]benzoic acid (114)

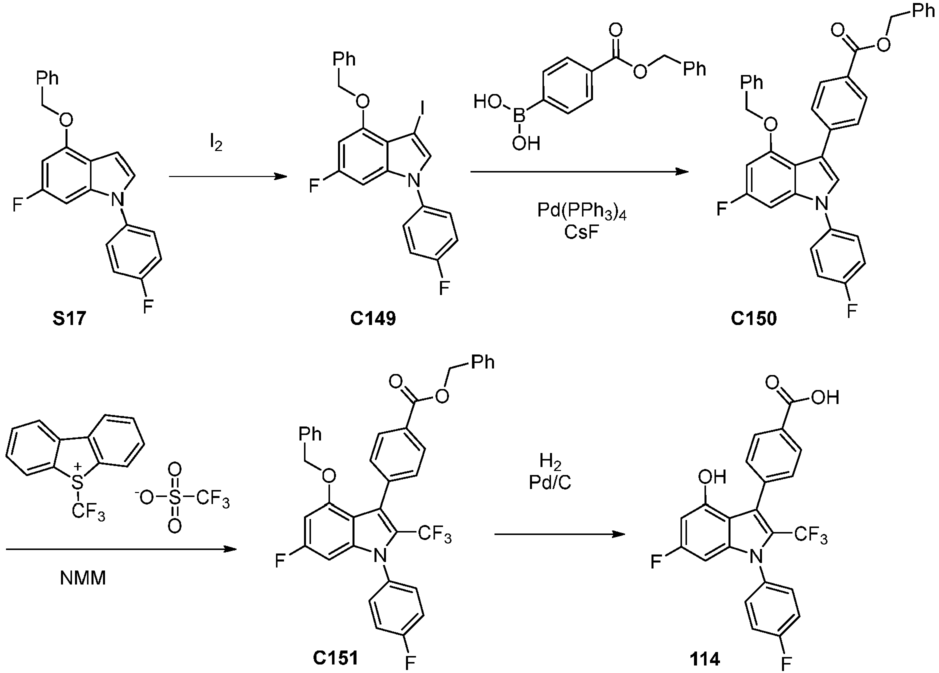

### Step 1. Synthesis 4-benzyloxy-6-fluoro-1-(4-fluorophenyl)-3-iodo-indole (C149)

To a solution of 4-benzyloxy-6-fluoro-1-(4-fluorophenyl)indole S17 (5 mg, 0.02 mmol) in DMF (0.1 mL) was added I₂ (10 mg, 0.04 mmol) . The solution was stirred at room temperature for 1 hour. NaS₂O₃ solution was added. The solid precipitate was collected and used directly in the subsequent reaction without further purification. 4-benzyloxy-6-fluoro-1-(4-fluorophenyl)-3-iodo-indole (7 mg, 102%). LCMS *m*/*z* 461.17 [M+H]⁺.

### Step 2. Synthesis of benzyl 4-[4-benzyloxy-6-fluoro-1-(4-fluorophenyl)indol-3-yl]benzoate (C150)

A mixture of 4-benzyloxy-6-fluoro-1-(4-fluorophenyl)-3-iodo-indole **C149** (140 mg, 0.3 mmol), (4-benzyloxycarbonylphenyl)boronic acid (105 mg, 0.41 mmol) and CsF (130 mg, 0.86 mmol) in DME (2.5 mL) was purged with nitrogen for 5 minutes, and then Pd(PPh₃)₄ (33 mg, 0.03 mmol) was added and the mixture purged with nitrogen for another 5 minutes, then heated at 100 °C over 36 hours. The mixture was diluted EtOAc (5 mL) and the precipitate was filtered. The filtrate was conc and purified by silica gel chromatography (12g column, Gradient: 0-10% EtOAc in Hexanes) to afford the product as a white solid. Benzyl 4-[4-benzyloxy-6-fluoro-1-(4-fluorophenyl)indol-3-yl]benzoate (61 mg, 38%). ¹H NMR (400 MHz, Chloroform-d) δ 8.01 - 7.89 (m, 2H), 7.75 - 7.60 (m, 2H), 7.55 - 7.47 (m, 4H), 7.47 - 7.39 (m, 3H), 7.27 - 7.22 (m, 6H), 7.18 (dd, J = 7.7, 2.0 Hz, 2H), 6.80 (dd, J = 9.3, 2.0 Hz, 1H), 6.54 (dd, J = 11.4, 2.1 Hz, 1H), 5.42 (s, 2H), 5.11 (s, 2H). LCMS *m*/*z* 546.47 [M+H]⁺.

### Step 3. Synthesis of benzyl 4-[4-benzyloxy-6-fluoro-]-(4-fluorophenyl)-2-(trifluoromethyl)indol-3-yl]benzoate (C151)

A vial was charged with benzyl 4-[4-benzyloxy-6-fluoro-1-(4-fluorophenyl)indol-3-yl]benzoate **C150** (62 mg, 0.11 mmol) and 5-(trifluoromethyl)dibenzothiophen-5-ium trifluoromethanesulfonate (95 mg, 0.24 mmol) followed by DMF (700 µL), NMM (30 µL, 0.27 mmol) and stirred at 50 °C overnight. After addition of HCl (1N 3 mL), the aqueous layer was extracted with dichloromethane (3 mL x 3). The combined organic layers were dried, and purified by silica gel chromatography (Gradient: 0-10% EtOAc in Hexanes) to give product as a white solid. Benzyl 4-[4-benzyloxy-6-fluoro-1-(4-fluorophenyl)-2-(trifluoromethyl)indol-3-yl]benzoate (20.5 mg, 30%). LCMS *m*/*z* 614.23 [M+H]⁺.

### Step 4. Synthesis of 4-[6-fluoro-1-(4-fluorophenyl)-4-hydroxy-2-(trifluoromethyl)indol-3-yl]benzoic acid (114)

To a solution of benzyl 4-[4-benzyloxy-6-fluoro-1-(4-fluorophenyl)-2-(trifluoromethyl)indol-3-yl]benzoate **C151** (20.5 mg, 0.033 mmol) in THF (0.5 mL) and EtOH (0.5 mL) (flushed with N₂) was added Pd (4 mg of 10% w/w, 0.004 mmol) on carbon, then hydrogenated under a balloon of H₂ (6 mg, 2.98 mmol) for 3 hours. The mixture was filtered and concentrated to afford the desired product as a white solid. 4-[6-fluoro-1-(4-fluorophenyl)-4-hydroxy-2-(trifluoromethyl)indol-3-yl]benzoic acid (11.9 mg, 82%). ¹H NMR (400 MHz, Chloroform-*d*/MeOD) δ 8.35 - 8.14 (m, 2H), 7.83 - 7.65 (m, 2H), 7.65 - 7.53 (m, 2H), 7.46 - 7.30 (m, 2H), 6.46 (ddd, J = 11.0, 4.4, 2.5 Hz, 1H), 6.31 (ddd, J = 9.3, 4.7, 2.8 Hz, 1H). LCMS *m*/*z* 433.92 [M+H]⁺

### Compound 115

### 2-fluoro-4-[1-(4-fluorophenyl)-4-hydroxy-2-(trifluoromethyl)indol-3-yl]benzoic acid (115)

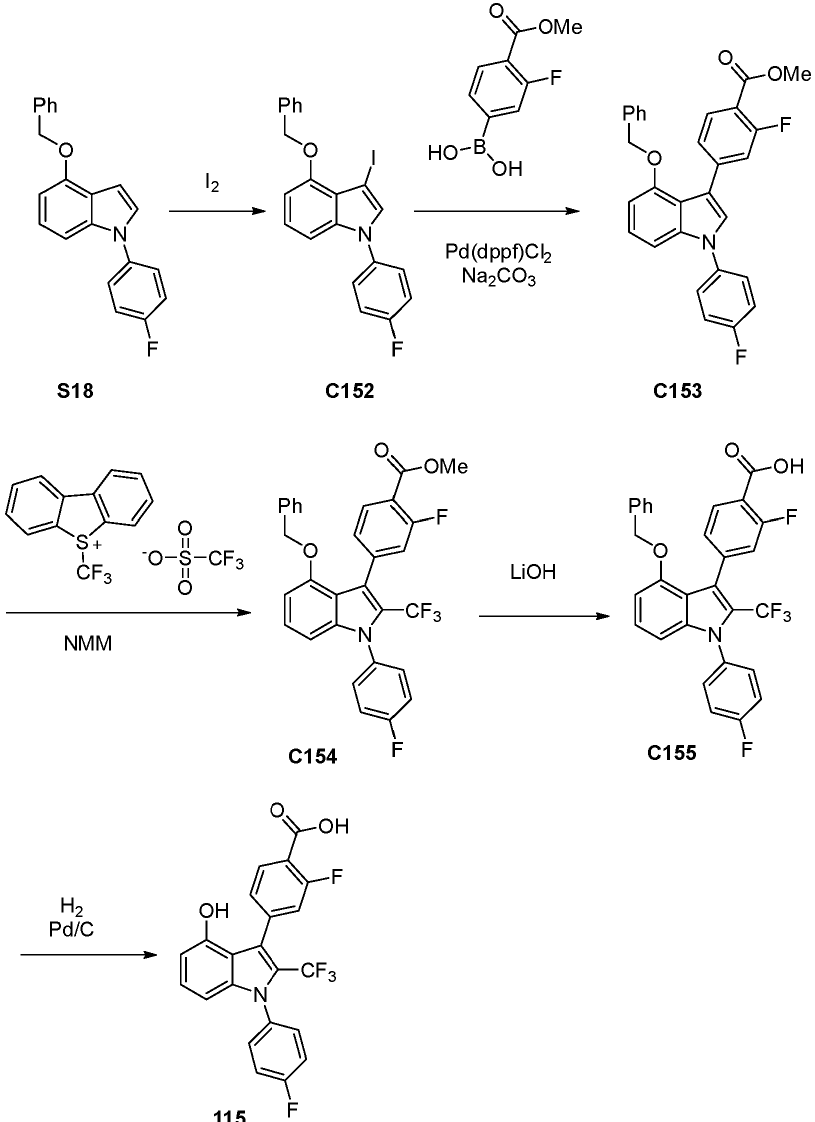

Compound **115** was prepared from **S18** using the method described for the preparation of compound **114**. Silica gel chromatography (Gradient: 0-70% EtOAc in heptane) afforded the product.2-fluoro-4-[1-(4-fluorophenyl)-4-hydroxy-2-(trifluoromethyl)indol-3-yl]benzoic acid (12 mg, 54%). ¹H NMR (400 MHz, Methanol-*d*₄) δ 7.91 (t, J = 7.9 Hz, 1H), 7.51-7.48 (m,, 2H), 7.37 - 7.22 (m, 4H), 7.10 (t, J = 8.0 Hz, 1H), 6.50 (s, 1H), 6.45 (d, J = 8.4 Hz, 1H). LCMS *m*/*z* 434.05 [M+H]⁺.

### Compound 116

### 4-[1-(4-fluorophenyl)-4-hydroxy-2-(trifluoromethyl)indol-3-yl]benzoic acid (116)

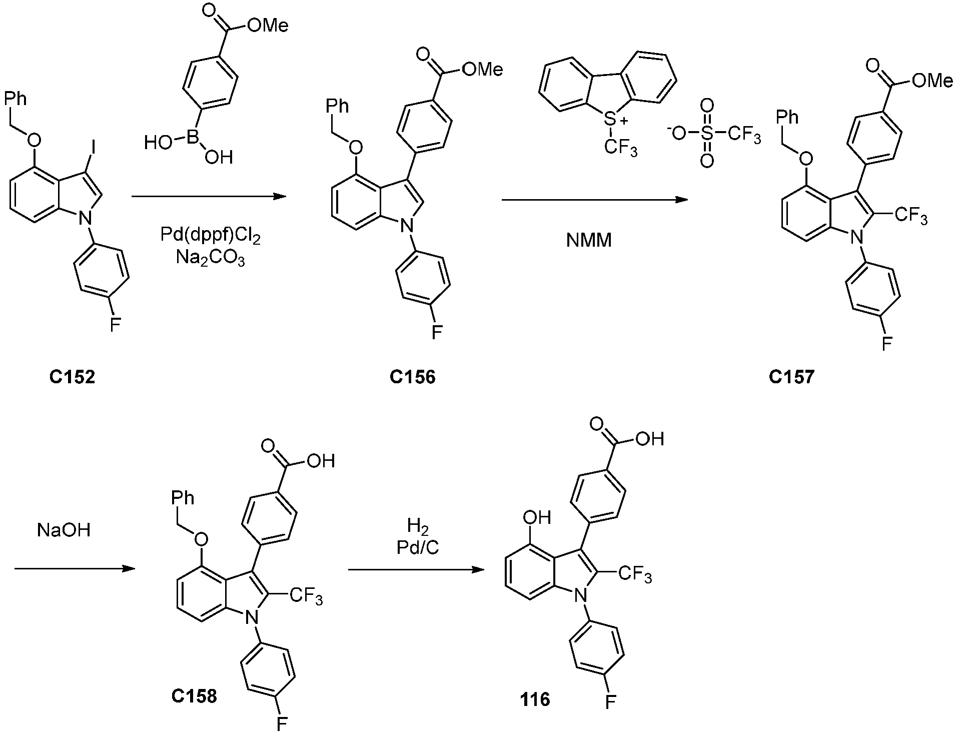

Compound 116 was prepared in 4 steps from compound C152 using the method described for Compound 114. The crude product was purified by flash column chromatography eluting with EtOAc/heptane (0-70%) to afford the product. 4-[1-(4-fluorophenyl)-4-hydroxy-2-(trifluoromethyl)indol-3-yl]benzoic acid (35 mg, 87%). ¹H NMR (400 MHz, Acetone-d6) δ 8.08 (d, J = 7.9 Hz, 2H), 7.69 - 7.58 (m, 4H), 7.44 (t, J = 8.7 Hz, 2H), 7.15 (t, J = 8.1 Hz, 1H), 6.60 (d, J = 7.7 Hz, 1H), 6.52 (d, J = 8.4 Hz, 1H). LCMS *m*/*z* 416.19 [M+H]⁺.

### Compound 117

### 3-[1-(4-fluorophenyl)-4-hydroxy-2-(trifluoromethyl)indol-3-yl]-1-methyl-cyclobutanecarboxylic acid (117)

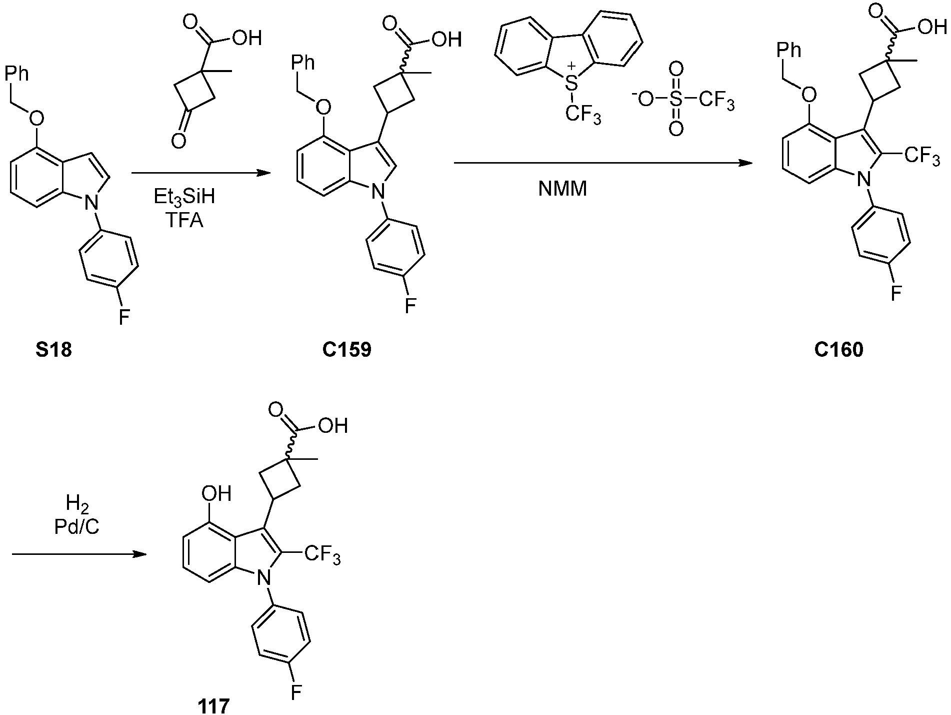

### Step 1. 3-[4-benzyloxy-1-(4-fluorophenyl)indol-3-yl]-1-methyl-cyclobutanecarboxylic acid (C159)

Triethylsilane (900 µL, 5.635 mmol) was added to a stirred solution of 4-benzyloxy-1-(4-fluorophenyl)indole S18 (500 mg, 1.52 mmol), 1-methyl-3-oxo-cyclobutanecarboxylic acid (360 mg, 2.81 mmol) and trifluoroacetic acid (350 µL, 4.54 mmol) in dichloromethane (10 mL). The solution was heated at 50 °C for 18 hours. The reaction mixture was washed with water and dried over Na₂SO₄. The solvent was removed under reduced pressure and purified by silica gel chromatography (Gradient: 0-70% EtOAc in heptane) to afford the product. 3-[4-benzyloxy-1-(4-fluorophenyl)indol-3-yl]-1-methyl-cyclobutanecarboxylic acid (447 mg, 67%) as a off white solid. LCMS *m*/*z* 430.18 [M+H]⁺. ¹H NMR indicated cis/trans mixture.

### Steps 2-3: Synthesis of 3-[1-(4-fluorophenyl)-4-hydroxy-2-(trifluoromethyl)indol-3-yl]-1-methyl-cyclobutane carboxylic acid (117)

Compound **117** was prepared from **C159** in two steps using a trifluoromethylation and then a hydrogenation as described in the synthesis of compound **114.** The product was purified by silica gel column chromatography (Gradient: 0-70% EtOAc heptane) to afford the product as a white solid. 3-[1-(4-fluorophenyl)-4-hydroxy-2-(trifluoromethyl)indol-3-yl]-1-methyl-cyclobutanecarboxylic acid (26 mg, 44%). ¹H NMR (400 MHz, Acetone-d6) δ 7.52 - 7.45 (m, 2H), 7.41 - 7.30 (m, 2H), 7.10 (t, J = 8.0 Hz, 1H), 6.69 (dd, J = 7.7, 0.8 Hz, 1H), 6.40 (dd, J = 8.3, 0.8 Hz, 1H), 4.38 - 4.19 (m, 1H), 2.95-2.92 (m, 4H), 2.79-2.73 (m, 2H), 1.49 (s, 3H). LCMS *m*/*z* 408.32 [M+H]⁺.

### Compound 118

### 6-[1-(4-fluorophenyl)-4-hydroxy- 2-(trifluoromethyl)indol-3-yl)spiro[3.3]heptane-2-carboxylic acid (118)

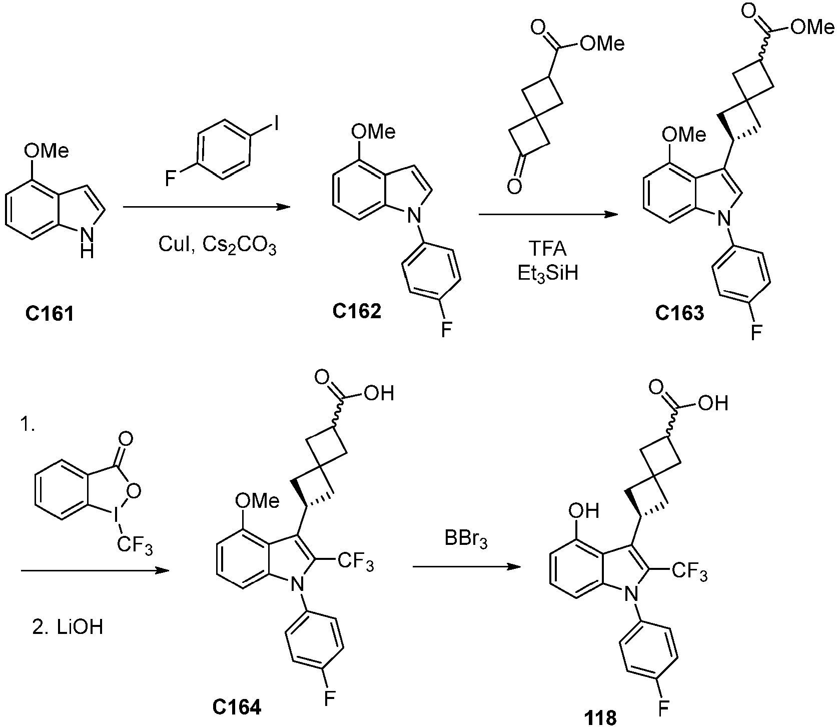

### Step 1. Synthesis of 1-(4-fluorophenyl)-4-methoxy-indole (C162)

A mixture of 4-methoxy-1H-indole (10 g, 67.95 mmol), 1-fluoro-4-iodo-benzene **C161** (9.5 mL, 82.4 mmol), CuI (760 mg, 3.99 mmol) and cesium carbonate (40 g, 122.8 mmol) in DMF (50 mL) was bubbled with nitrogen and stirred overnight at 120 °C. The reaction mixture was diluted with EtOAc (200 ml) and H₂O (200 ml). The organic layer was separated and the aqueous layer was extracted with EtOAc (2 x 100 mL). The combined organic layers were washed with H₂O, dried over Na₂SO₄ and concentrated. Purified by chromatography on silica gel (Gradient: 0-15% EtOAc in Heptane) to afford the product as a white product. 1-(4-fluorophenyl)-4-methoxy-indole (5.8 g, 34%) 1-(4-fluorophenyl)-4-methoxy-indole (5.8 g, 34%). ¹H NMR (400 MHz, Chloroform-d) δ 7.54 - 7.35 (m, 2H), 7.25 - 7.21 (m, 2H), 7.18 (t, J = 0.5 Hz, 1H), 7.12-7.10 (m,, 1H), 6.81 (dd, J = 3.2, 0.8 Hz, 1H), 6.62 (dd, J = 7.7, 0.8 Hz, 1H), 4.02 (s, 3H). LCMS *m*/*z* 242.5 [M+H]⁺.

### Step 2. Synthesis of methyl 6-[1-(4-fluorophenyl)-4-methoxy-indol-3-yl]spiro[3.3]heptane-2-carboxylate (C163)

A solution of methyl 2-oxospiro[3.3]heptane-6-carboxylate **C162** (543 mg, 3.23 mmol), 1-(4-fluorophenyl)-4-methoxy-indole (500 mg, 2.0 mmol) trifluoroacetic acid (500 µL, 6.5 mmol) and triethylsilane (1.2 mL, 7.5 mmol) in dichloromethane (8 mL) was stirred at 50 °C for 72 hours, The reaction mixture was washed with water and dried over Na₂SO₄. The solvent was removed under reduced pressure and crude product was purified by silica gel chromatography (Gradient: 0-50% EtOAc in heptane) to afford the product as a yellow solid. Methyl 6-[1-(4-fluorophenyl)-4-methoxy-indol-3-yl]spiro[3.3]heptane-2-carboxylate (655 mg, 83%). ¹H NMR (400 MHz, Chloroform-d) δ 7.51 - 7.36 (m, 2H), 7.25 - 7.20 (m, 2H), 7.12 (t, J = 8.0 Hz, 1H), 7.05 (d, J = 0.8 Hz, 1H), 6.92 (d, J = 1.2 Hz, 1H), 6.56 (d, J = 0.8 Hz, 1H), 3.95 (s, 3H), 3.90 - 3.83 (m, 1H), 3.70 (s, 3H), 3.16 - 2.98 (m, 1H), 2.58-2.55 (m, 1H), 2.50 - 2.37 (m, 3H), 2.35 - 2.13 (m, 4H).

### Steps 3-5: Synthesis of 6-[1-(4-fluorophenyl)-4-hydroxy-2-(trifluoromethyl)indol-3-yl]spiro[3.3]heptane-2-carboxylic acid (118)

Step 3. 1-(trifluoromethyl)-1λ³-benzo[d][1,2]iodaoxol-3(1H)-one (1.4 g, 2.66 mmol) (Togni's reagent) was added to a stirred nitrogen purged solution of methyl 6-[1-(4-fluorophenyl)-4-methoxy-indol-3-yl]spiro[3.3]heptane-2-carboxylate **C163** (655 mg, 1.67 mmol) in CH₃CN (20 mL). The solution was heated at 80 °C for 3 hours to form dark black solution and solvent was removed under reduced pressure. The product was dissolved in EtOAc (10 mL) and washed with water. The organic layer was dried and concentrated under reduced pressure. The crude product was purified by silica gel chromatography (Gradient: 0-50%) EtOAc heptanes) to afford the product as an oil. Methyl 6-[1-(4-fluorophenyl)-4-methoxy-2-(trifluoromethyl)indol-3-yl]spiro[3.3]heptane-2-carboxylate (220 mg, 26%). LCMS *m*/*z 462.3* [M+H]⁺.

Step 4. LiOH (200 mg, 8.4 mmol) was added to stirred solution of methyl 6-[1-(4-fluorophenyl)-4-methoxy-2-(trifluoromethyl)indol-3-yl]spiro[3.3]heptane-2-carboxylate (75 mg) in MeOH (7 mL), THF (2 mL) and H₂O (1 mL). The solution was stirred at room temperature for 3 hours. The solvent was removed under reduced pressure and the crude product was dissolved in water (5 mL) and acidified with 6N HCl. The aqueous layer was extracted with EtOAc (3 x 5 mL), dried over Na₂SO₄. The solvent was removed under reduced pressure to afford 6-[1-(4-fluorophenyl)-4-methoxy-2-(trifluoromethyl)indol-3-yl]spiro[3.3]heptane-2-carboxylic acid (160 mg, 21%) as a white solid. LCMS *m*/*z* 447.75 [M+H]⁺.

Step 5. BBr₃ (1 mL of 1 M, 1.0 mmol) was added to a nitrogen purged solution of 6-[1-(4-fluorophenyl)-4-methoxy-2-(trifluoromethyl)indol-3-yl]spiro[3.3]heptane-2-carboxylic acid **C164** (160 mg) in dichloromethane (5 mL) at room temperature and stirred for 2 hours. The reaction mixture was poured into water. The dichloromethane layer was separated, dried and concentrated. The crude product was purified by reversed-phase chromatography (Column: C18. Gradient: 0-100% MeCN in water with 0.1% trifluoroacetic acid) afforded the product. 6-[1-(4-fluorophenyl)-4-hydroxy-2-(trifluoromethyl)indol-3-yl]spiro[3.3]heptane-2-carboxylic acid (45 mg, 6%).

¹H NMR (400 MHz, Methanol-*d*₄) δ 7.26 - 7.04 (m, 4H), 6.94 (d, J = 8.0 Hz, 1H), 6.46 - 6.30 (m, 1H), 6.22 (d, J = 8.1 Hz, 1H), 4.04 - 3.72 (m, 1H), 3.00 - 2.65 (m, 3H), 2.41 - 2.27 (m, 3H), 2.25 - 2.12 (m, 3H). LCMS *m*/*z* 434.52 [M+H]⁺.

### Compound 119

### 4-[1-(4-fluorophenyl)-4-hydroxy-2-(2-hydroxy-1,1-dimethyl-propyl)indol-3-yl]benzoic acid (119)

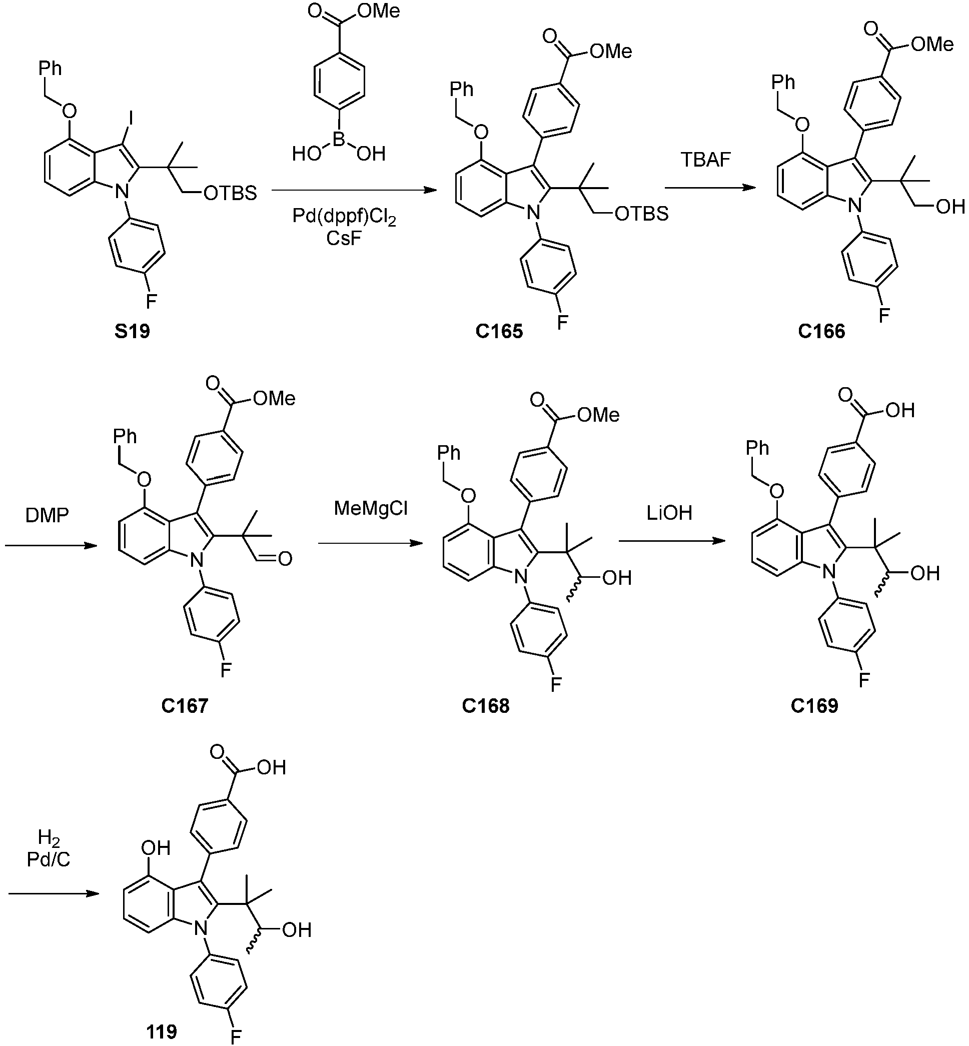

### Step 1. Synthesis of methyl 4-[4-benzyloxy-2-[2-[tert-butyl(dimethyl)silyl]oxy-1,1-dimethylethyl]-1-(4-fluorophenyl)indol-3-yl]benzoate (C165)

A mixture of [2-[4-benzyloxy-1-(4-fluorophenyl)-3-iodo-indol-2-yl]-2-methyl-propoxy]-tert-butyl-dimethyl-silane S19 (7.7 g, 12.2 mmol), (4-methoxycarbonylphenyl)boronic acid (4.4 g, 24.5 mmol), PdCl₂(dppf) (1 g, 1.23 mmol), and CsF (7.4 g, 48.7 mmol) in DME (60 mL) was heated at 90 °C in a sealed flask for 4 hours. The reaction mixture was cooled to room temperature, diluted with water and extracted with EtOAc. The organic layer was concentrated to dryness and purified via silica gel chromatography (Gradient: 0-40% EtOAc in heptane) to afford the product as a light orange solid which was used in the subsequent step without additional purification. Methyl 4-[4-benzyloxy-2-[2-[tert-butyl(dimethyl)silyl]oxy-1,1-dimethyl-ethyl]-1-(4-fluorophenyl)indol-3-yl]benzoate (6.6 g, 85%). LCMS *m*/*z* 638.0 [M+H]⁺.

### Step 2. Synthesis of methyl 4-[4-benzyloxy-1-(4-fluorophenyl)-2-(2-hydroxy-1,1-dimethylethyl)indol-3-yl]benzoate (C166)

To a solution of methyl 4-[4-benzyloxy-2-[2-[tert-butyl(dimethyl)silyl]oxy-1,1-dimethyl-ethyl]-1-(4-fluorophenyl)indol-3-yl]benzoate **C165** (6.6 g, 10.4 mmol) in DME (60 mL) was added TBAF (35 mL of 1 M, 35.0 mmol). The reaction mixture was stirred at room temperature for 2 hours then diluted with water and extracted with EtOAc. The organic layer was concentrated to dryness and purified via silica gel chromatography (Gradient: 0-40% EtOAc in heptane) to afford the product as a white solid. Methyl 4-[4-benzyloxy-1-(4-fluorophenyl)-2-(2-hydroxy-1, 1-dimethyl-ethyl)indol-3-yl]benzoate (2.7 g, 50%). ¹H NMR (400 MHz, Chloroform-d) δ 7.94 - 7.89 (m, 2H), 7.56 - 7.52 (m, 2H), 7.48 - 7.42 (m, 2H), 7.28 - 7.22 (m, 2H), 7.20 - 7.12 (m, 3H), 7.03 (t, J = 8.1 Hz, 1H), 6.82 - 6.77 (m, 2H), 6.55 (dd, J = 7.9, 0.7 Hz, 1H), 6.31 (dd, J = 8.3, 0.7 Hz, 1H), 4.84 (s, 2H), 3.99 (s, 3H), 3.32 (d, J = 6.5 Hz, 2H), 1.06 (s, 6H). LCMS *m*/*z* 524.0 [M+H]⁺.

### Step 3. Synthesis of methyl 4-[4-benzyloxy-2-(1,1-dimethyl-2-oxo-ethyl)-1-(4-fluorophenyl)indol-3-yl]benzoate (C167)

To a solution of methyl 4-[4-benzyloxy-1-(4-fluorophenyl)-2-(2-hydroxy-1,1-dimethyl-ethyl)indol-3-yl]benzoate **C166** (700 mg, 1.34 mmol) in dichloromethane (7 mL) was added Dess Martin periodinane (596 mg, 1.41 mmol). The reaction was stirred for 10 minutes, then diluted with dichloromethane and washed with water. The organic layer was concentrated to dryness and purified via silica gel chromatography (Gradient: 0-35% EtOAc in heptane) to afford the product as a white solid. Methyl 4-[4-benzyloxy-2-(1,1-dimethyl-2-oxo-ethyl)-1-(4-fluorophenyl)indol-3-yl]benzoate (630 mg, 90%). LCMS *m*/*z* 522.0 [M+H]⁺.

### Step 4. Synthesis of methyl 4-[4-benzyloxy-1-(4-fluorophenyl)-2-(2-hydroxy-1,1-dimethylpropyl)indol-3-yl]benzoate (C168)

To a solution of methyl 4-[4-benzyloxy-2-(1,1-dimethyl-2-oxo-ethyl)-1-(4-fluorophenyl)indol-3-yl]benzoate **C167** (100 mg, 0.19 mmol) in THF (1.5 mL) at 0 °C was added MeMgCl (101 µL of 2 M, 0.20 mmol). The reaction was stirred allowed to warm up to room temperature while stirring for 1 hour. The mixture was quenched with water and extracted with EtOAc. The organic layer was concentrated to dryness and purified by silica gel chromatography (Gradient: 0-40% EtOAc in heptane) to afford the product as a white solid. Methyl 4-[4-benzyloxy-1-(4-fluorophenyl)-2-(2-hydroxy-1,1-dimethyl-propyl)indol-3-yl]benzoate (74 mg, 72%). ¹H NMR (400 MHz, Chloroform-*d*) δ 7.92 - 7.86 (m, 2H), 7.54 - 7.48 (m, 2H), 7.48 - 7.38 (m, 2H), 7.27 - 7.21 (m, 2H), 7.21 - 7.13 (m, 3H), 7.02 (dd, J = 8.3, 7.8 Hz, 1H), 6.82 - 6.77 (m, 2H), 6.54 (dd, J = 7.8, 0.7 Hz, 1H), 6.25 (dd, J = 8.3, 0.7 Hz, 1H), 4.82 (s, 2H), 4.02 (t, J = 6.4 Hz, 1H), 3.98 (s, 3H), 1.29 (t, J = 7.1 Hz, 6H), 1.03 (s, 3H). LCMS m/z 538.0 [M+H]⁺.

### Step 5. Synthesis of 4-[4-benzyloxy-1-(4fluorophenyl)-2-(2-hydroxy-1,1-dimethylpropyl)indol-3-yl]benzoic acid (C169)

To a solution of methyl 4-[4-benzyloxy-1-(4-fluorophenyl)-2-(2-hydroxy-1,1-dimethyl-propyl)indol-3-yl]benzoate **C168** (74 mg, 0.14 mmol) in THF (3 mL), MeOH (1 mL), and water (1 mL) was added LiOH (35 mg, 1.46 mmol). The reaction mixture was stirred at room temperature for 3 hours. The reaction was heated to 75 °C for 30 minutes, then cooled to room temperature, acidified using 1 M aq. HCl, and extracted with EtOAc. The organic layer was dried over magnesium sulfate, filtered, and concentrated to afford the product. 4-[4-benzyloxy-1-(4-fluorophenyl)-2-(2-hydroxy-1,1-dimethyl-propyl)indol-3-yl]benzoic acid (67 mg, 93%). ¹H NMR (400 MHz, Chloroform-d) δ 7.98 - 7.92 (m, 2H), 7.58 - 7.51 (m, 2H), 7.48 - 7.39 (m, 3H), 7.26 - 7.18 (m, 5H), 7.06 - 7.01 (m, 1H), 6.83 (ddd, J = 7.7, 3.2, 2.0 Hz, 2H), 6.56 (dd, J = 7.8, 0.7 Hz, 1H), 6.26 (dd, J = 8.3, 0.7 Hz, 1H), 4.83 (s, 2H), 4.02 (q, J = 6.3 Hz, 1H), 1.06 - 1.02 (m, 6H), 0.97 (s, 3H). LCMS *m*/*z* 524.0 [M+H]⁺.

### Step 6. Synthesis of 4-[1-(4-fluorophenyl)-4-hydroxy-2-(2-hydroxy-1,1-dimethyl-propyl)indol-3-yl]benzoic acid (119)

To a slurry of Pd on carbon (50 mg, 0.05 mmol) in EtOH (5 mL) was added a solution of 4-[4-benzyloxy-1-(4-fluorophenyl)-2-(2-hydroxy-1,1-dimethyl-propyl)indol-3-yl]benzoic acid **C169** (67 mg, 0.13 mmol) in EtOAc (5 mL). The reaction was stirred at room temperature under 1 atm hydrogen for 10 minutes, then filtered over Celite^{®} and concentrated to dryness. The crude material was purified via silica gel chromatography (Gradient: 0-10% MeOH in dichloromethane) to afford the product as a white solid. 4-[1-(4-fluorophenyl)-4-hydroxy-2-(2-hydroxy-1,1-dimethyl-propyl)indol-3-yl]benzoic acid (30 mg, 51%). ¹H NMR (400 MHz, Chloroform-d) δ 8.23 (ddd, J = 7.9, 3.7, 1.7 Hz, 2H), 7.74 (ddd, J = 12.0, 7.9, 1.4 Hz, 2H), 7.50 - 7.39 (m, 2H), 7.28 - 7.23 (m, 2H), 6.98 (t, J = 8.0 Hz, 1H), 6.49 (dd, J = 7.7, 0.7 Hz, 1H), 6.20 (dd, J = 8.3, 0.8 Hz, 1H), 4.04 (q, J = 6.3 Hz, 1H), 1.07 (d, J = 6.5 Hz, 6H), 0.99 (s, 3H). LCMS *m*/*z* 434.0 [M+H]⁺.

### Compound 120 and Compound 121

Compounds **120-121** were prepared from **S20** and the appropriate boronic acid. Compound **120** was prepared by Suzuki coupling, hydrogenation, and then methyl group was removed by treatment with boron tribromide.

**Table 8. Method of preparation, structure, physicochemical data for Compounds 120-121**

| **Compound** | **Method/Product** | **Boronic acid or ester** | **¹H NMR; LCMS m/z [M+H]⁺** |
|---|---|---|---|
| ***120*** | From **S20**^{1, 2} | 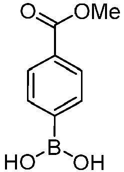 | ¹H NMR (400 MHz, Chloroform-d) δ 8.01 (dd, J = 8.3, 1.9 Hz, 2H), 7.60 - 7.51 (m, 2H), 7.30 - 7.22 (m, 2H), 7.06 - 6.99 (m, 2H), 6.81 - 6.73 (m, 1H), 6.30 - 6.25 (m, 1H), 6.06 - 6.01 (m, 1H), 3.17 (s, 2H), 0.86 (s, 6H). LCMS *m*/*z* 420.25 [M+H]⁺ |
| | 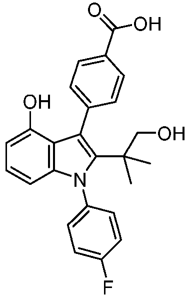 | | |
| ***121*** | From **S20**³ | 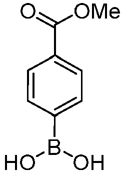 | ¹H NMR (400 MHz, Chloroform-*d*/CD30D) δ 8.19 - 8.07 (m, 2H), 7.75 - 7.62 (m, 2H), 7.49 - 7.38 (m, 2H), 7.23 (t, J = 8.5 Hz, 2H), 6.90 (t, J = 8.0 Hz, 1H), 6.48 - 6.37 (m, 1H), 6.19 (d, J = 8.2 Hz, 1H), 3.07 (s, 3H), 2.99 (s, 2H), 1.06 (s, 6H). LCMS *m*/*z* 434.34 [M+H]⁺. |
| | 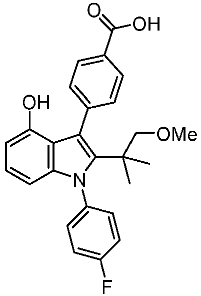 | | |

| | | | |
|---|---|---|---|
| **1.** Suzuki Conditions: Pd₂(dba)₃, SPhos, K₃PO₄ in THF at 80 °C. **2.** Purification by silica gel chromatography (Gradient: 0-10% MeOH in dichloromethane) yielded the product. **3.** Compound **121** was prepared as for compound **120,** omitting the final methoxy deprotection step. | | | |

### Compound 122

### 4-[2-(1,1-dimethyl-3-methylsulfonyl-propyl)-1-(4-fluorophenyl)-4-hydroxy-indol-3-yl]benzoic acid (122)

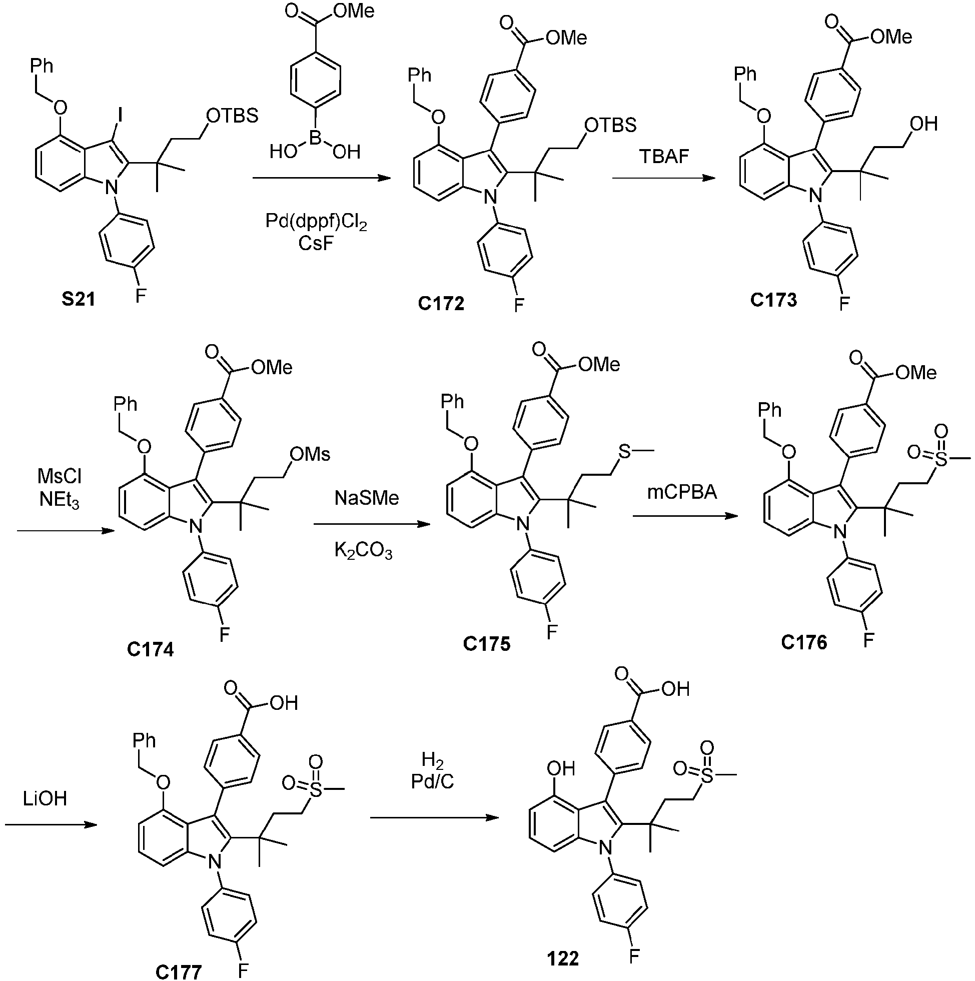

### Step 1. Synthesis of methyl 4-[4-benzyloxy-2-[3-[tert-butyl(dimethyl)silyl]oxy-1,1-dimethylpropyl]-1-(4-fluorophenyl)indol-3-yl]benzoate (C172)

A mixture of [3-[4-benzyloxy-1-(4-fluorophenyl)-3-iodo-indol-2-yl]-3-methyl-butoxy]-tert-butyl-dimethyl-silane S21 (10 g, 15.5 mmol), (4-methoxycarbonylphenyl)boronic acid (8.3 g, 46.1 mmol), CsF (9.4 g, 61.9 mmol), and PdCl₂(dppf) (1.27 g, 1.56 mmol) in DME (80 mL) was stirred at 85 °C for 4 hours then cooled to room temperature, partitioned with water and EtOAc, filtered over Celite^{®}, and separated. The organic layer was concentrated to dryness and purified by silica gel chromatography (Gradient: 0-30% EtOAc in heptane) afforded the product as an off-white solid (~70% pure) which was advanced to the next step without further purification. methyl 4-[4-benzyloxy-2-[3-[tert-butyl(dimethyl)silyl]oxy-1,1-dimethyl-propyl]-1-(4-fluorophenyl)indol-3-yl]benzoate (8.3 g, 82%). LCMS *m*/*z* 652.0 [M+H]⁺.

### Step 2. Synthesis of methyl 4-[4-benzyloxy-1-(4-fluorophenyl)-2-(3-hydroxy-1,1-dimethylpropyl)indol-3-yl]benzoate (C173)

To a solution of 4-[4-benzyloxy-2-[3-[tert-butyl(dimethyl)silyl]oxy-1,1-dimethylpropyl]-1-(4-fluorophenyl)indol-3-yl]benzoate **C172** (1.94 g, 2.98 mmol) in THF (15 mL) was added TBAF (15 mL of 1 M, 15.00 mmol) in THF. The reaction mixture was stirred at room temperature for 90 minutes then diluted with water and extracted with EtOAc. The organic layer was dried over magnesium sulfate, filtered, and concentrated to dryness. The crude product was purified by silica gel chromatography (Gradient: 0-60% EtOAc in heptane) to afford the product as a white solid. methyl 4-[4-benzyloxy-1-(4-fluorophenyl)-2-(3-hydroxy-1,1-dimethyl-propyl)indol-3-yl]benzoate (1.34 g, 84%). ¹H NMR (400 MHz, Chloroform-d) δ 7.93 - 7.87 (m, 2H), 7.54 - 7.46 (m, 4H), 7.29 - 7.24 (m, 2H), 7.21 - 7.14 (m, 3H), 7.05 - 6.99 (m, 1H), 6.82 - 6.77 (m, 2H), 6.55 (dd, J = 7.9, 0.7 Hz, 1H), 6.27 (dd, J = 8.3, 0.7 Hz, 1H), 4.83 (s, 2H), 3.99 (s, 3H), 3.65 (t, J = 7.3 Hz, 2H), 1.70 (t, J = 7.3 Hz, 2H), 1.60 (s, 1H), 1.06 (s, 6H). LCMS *m*/*z* 538.0 [M+H]⁺.

### Step 3. Synthesis of methyl 4-[4-benzyloxy-2-(1,1-dimethyl-3-methylsulfonyloxypropyl)-1-(4-fluorophenyl)indol-3-yl]benzoate (C174)

To a solution of methyl 4-[4-benzyloxy-1-(4-fluorophenyl)-2-(3-hydroxy-1,1-dimethyl-propyl)indol-3-yl]benzoate **C173** (400 mg, 0.75 mmol) in dichloromethane (10 mL) was added Et₃N (130 µL, 0.93 mmol) followed by MsCl (70 µL, 0.90 mmol). The reaction mixture was stirred at room temperature for 2 hours then washed with water. The organic layer was dried over magnesium sulfate, filtered and concentrated to dryness to afford the product as a colorless oil. Methyl 4-[4-benzyloxy-2-(1,1-dimethyl-3-methylsulfonyloxy-propyl)-1-(4-fluorophenyl)indol-3-yl]benzoate (460 mg, 100%). ¹H NMR (400 MHz, Chloroform-d) δ 7.89 - 7.85 (m, 2H), 7.50 - 7.45 (m, 2H), 7.45 - 7.39 (m, 2H), 7.26 (d, J = 2.6 Hz, 2H), 7.18 - 7.10 (m, 3H), 7.03 - 6.96 (m, 1H), 6.80 - 6.75 (m, 2H), 6.52 (dd, J = 7.8, 0.7 Hz, 1H), 6.25 (dd, J = 8.3, 0.7 Hz, 1H), 4.80 (s, 2H), 4.16 (dd, J = 8.0, 7.0 Hz, 2H), 3.96 (s, 3H), 2.85 (s, 3H), 1.82 (t, J = 7.5 Hz, 2H), 1.07 (s, 6H). LCMS *m*/*z* 616.0 [M+H]⁺.

### Step 4. Methyl 4-[4-benzyloxy-2-(1,1-dimethyl-3-methylsulfanyl-propyl)-1-(4-fluorophenyl)indol-3-yl]benzoate (C175)

To a solution of methyl 4-[4-benzyloxy-2-(1,1-dimethyl-3-methylsulfonyloxy-propyl)-1-(4-fluorophenyl)indol-3-yl]benzoate **C174** (340 mg, 0.55 mmol) in DMF (3 mL) was added CH₃NaS (79 mg, 1.13 mmol) and K₂CO₃ (152 mg, 1.10 mmol). The reaction mixture was stirred in a sealed vial at 70 °C for 1 hour, then cooled to room temperature, diluted with water, and extracted with EtOAc. The organic layer was concentrated to dryness and purified by silica gel chromatography (Gradient: 0-40% EtOAc in heptane) to afford the product as a white solid. Methyl 4-[4-benzyloxy-2-(1,1-dimethyl-3-methylsulfanyl-propyl)-1-(4-fluorophenyl)indol-3-yl]benzoate (216 mg, 69%) LCMS *m*/*z* 568.0 [M+H]⁺.

### Step 5. Synthesis of methyl 4-[4-benzyloxy-2-(1,1-dimethyl-3-methylsulfonyl-propyl)-1-(4-fluorophenyl)indol-3-yl]benzoate (C176)

To a solution of methyl 4-[4-benzyloxy-2-(1,1-dimethyl-3-methylsulfanyl-propyl)-1-(4-fluorophenyl)indol-3-yl]benzoate **C175** (180 mg, 0.32 mmol) in dichloromethane (3 mL) was added m-CPBA (58 mg, 0.34 mmol). The reaction mixture was stirred at room temperature for 5 minutes.
A: An aliquot was removed was washed with water. The organic layer was concentrated to dryness then purified by silica gel chromatography (Gradient: 0-8% MeOH in dichloromethane). to afford the sulfoxide as a white foam. methyl 4-[4-benzyloxy-2-(1,1-dimethyl-3-methylsulfinyl-propyl)-1-(4-fluorophenyl)indol-3-yl]benzoate (43 mg, 46%). ¹H NMR (400 MHz, Chloroform-d) δ 7.92 - 7.85 (m, 2H), 7.53 - 7.40 (m, 4H), 7.26 (ddd, J = 9.2, 6.7, 2.4 Hz, 2H), 7.20 - 7.11 (m, 3H), 7.03 (dd, J = 8.3, 7.8 Hz, 1H), 6.82 - 6.77 (m, 2H), 6.55 (dd, J = 7.8, 0.7 Hz, 1H), 6.27 (dd, J = 8.3, 0.7 Hz, 1H), 4.83 (s, 2H), 3.98 (s, 3H), 2.67 - 2.54 (m, 2H), 2.50 (s, 3H), 1.95 - 1.72 (m, 2H), 1.10 (d, J = 4.0 Hz, 6H). LCMS *m*/*z* 584.0 [M+H]⁺.
B: To the remaining half reaction was added an additional 30 mg mCPBA and the reaction mixture was monitored for completion by TLC. After 20 minutes at room temperature, the mixture was washed with water and the organic layer was concentrated to dryness then purified by silica gel chromatography (Gradient: 0-35% EtOAc in heptane) to afford the sulfone as a white solid. (38 mg, 40%). ¹H NMR (400 MHz, Chloroform-d) δ 7.91 - 7.85 (m, 2H), 7.51 - 7.44 (m, 4H), 7.29 - 7.26 (m, 2H), 7.21 - 7.13 (m, 3H), 7.07 - 7.00 (m, 1H), 6.82 - 6.77 (m, 2H), 6.55 (d, J = 7.8 Hz, 1H), 6.27 (dd, J = 8.3, 0.7 Hz, 1H), 4.82 (s, 2H), 3.98 (s, 3H), 2.97 - 2.88 (m, 2H), 2.82 (s, 3H), 2.00 - 1.90 (m, 2H), 1.08 (s, 6H). LCMS *m*/*z* 600.0 [M+H]⁺.

### Step 6. Synthesis of 4-[4-benzyloxy-2-(1,1-dimethyl-3-methylsulfonyl-propyl)-1-(4-fluorophenyl)indol-3-yl]benzoic acid (C177)

To a solution of methyl 4-[4-benzyloxy-2-(1,1-dimethyl-3-methylsulfonyl-propyl)-1-(4-fluorophenyl)indol-3-yl]benzoate **C176** (38 mg, 0.063 mmol) in THF (3 mL), MeOH (1 mL), and water (1 mL) was added LiOH (20 mg, 0.84 mmol). The reaction mixture was stirred at room temperature overnight then acidified with aq. 1 M HCl and extracted with EtOAc. The organic layer was dried over magnesium sulfate, filtered and dried to give the product as a white solid. 4-[4-benzyloxy-2-(1,1-dimethyl-3-methylsulfonyl-propyl)-1-(4-fluorophenyl)indol-3-yl]benzoic acid (35 mg, 94%). LCMS *m*/*z* 586.0 [M+H]⁺.

### Step 7. Synthesis of 4-[2-(1,1-dimethyl-3-methylsulfonyl-propyl)-1-(4-fluorophenyl)-4-hydroxy-indol-3-yl]benzoic acid (122)

To a slurry of Pd on carbon (approximately 12.7 mg, 0.012 mmol) in EtOH was added a solution of 4-[4-benzyloxy-2-(1,1-dimethyl-3-methylsulfonyl-propyl)-1-(4-fluorophenyl)indol-3-yl]benzoic acid **C177** (35 mg, 0.06 mmol) in EtOAc. The reactions were stirred for 60 minutes under hydrogen (1 atm pressure), then filtered over Celite^{®} and concentrated to dryness. 4-[2-(1,1-dimethyl-3-methylsulfonyl-propyl)-1-(4-fluorophenyl)-4-hydroxy-indol-3-yl]benzoic acid (18 mg, 58%).¹H NMR (400 MHz, DMSO-*d*₆) δ 12.82 (s, 1H), 8.88 (s, 1H), 7.93 - 7.86 (m, 2H), 7.63 - 7.55 (m, 2H), 7.55 - 7.47 (m, 2H), 7.45 - 7.38 (m, 2H), 6.78 (t, J = 8.0 Hz, 1H), 6.26 (dd, J = 7.6, 0.8 Hz, 1H), 5.93 (dd, J = 8.2, 0.8 Hz, 1H), 3.03 - 2.97 (m, 2H), 2.95 (s, 3H), 1.78 - 1.70 (m, 2H), 1.01 (s, 6H). LCMS *m*/*z* 496.0 [M+H]⁺.

### Compound 123

### 4-[2-tert-butyl-1-(4-fluorophenyl)-4-hydroxy-indol-3-yl]-2-fluoro-benzoic acid (123)

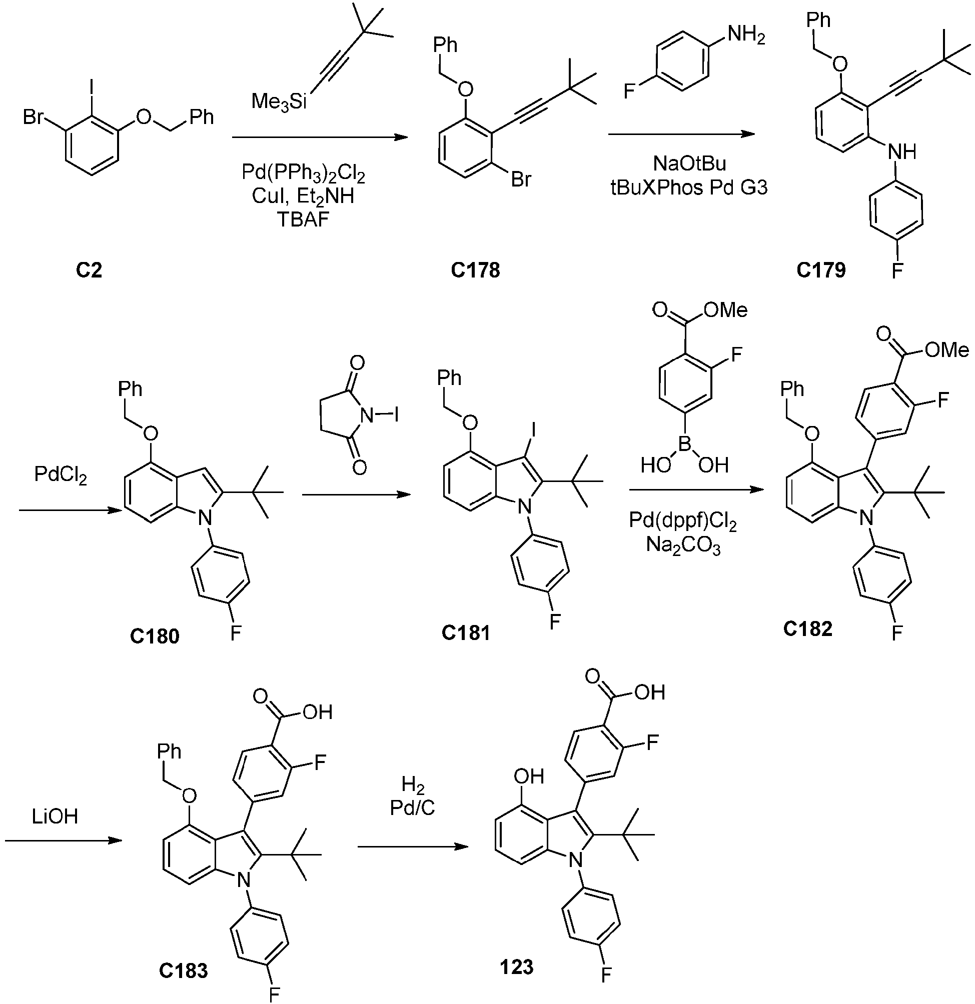

### Steps 1-4: Synthesis of 4-benzyloxy-2-tert-butyl-1-(4-fluorophenyl)-3-iodo-indole (C181)

Compound C181 was prepared from C2 using the method described for the preparation of S1. 3,3-dimethylbut-1-ynyl(trimethyl)silane was used in the Sonagashira coupling in step 1. 4-fluoroaniline was the coupling in step 2. 4-benzyloxy-2-tert-butyl-1-(4-fluorophenyl)-3-iodo-indole (2.3 g, 86%). ¹H NMR (400 MHz, Chloroform-d) δ 7.67 - 7.59 (m, 2H), 7.41 (ddd, J = 7.7, 6.3, 1.5 Hz, 2H), 7.37 - 7.30 (m, 1H), 7.26 - 7.22 (m, 2H), 7.18 (ddt, J = 8.9, 6.5, 1.9 Hz, 2H), 6.94 (td, J = 8.1, 5.9 Hz, 1H), 6.61 (dd, J = 7.8, 4.4 Hz, 1H), 6.25 (dd, J = 8.3, 0.9 Hz, 1H), 5.24 (d, J = 2.9 Hz, 2H), 1.45 - 1.34 (m, 9H). LCMS *m*/*z* 499.0 [M+H]⁺.

### Steps 5-8. Synthesis of 4-[2-tert-butyl-1-(4-fluorophenyl)-4-hydroxy-indol-3-yl]-2-fluoro-benzoic acid (123)

Compound **123** was prepared in three steps from **C181** using the method described for the synthesis of compound 1. 4-[2-tert-butyl-1-(4-fluorophenyl)-4-hydroxy-indol-3-yl]-2-fluorobenzoic acid (12 mg) ¹H NMR (400 MHz, Chloroform-d) δ 8.14 (t, J = 7.8 Hz, 1H), 7.49 (dd, J = 7.9, 1.6 Hz, 1H), 7.46 - 7.40 (m, 3H), 7.28 - 7.23 (m, 2H), 6.96 (dd, J = 8.3, 7.7 Hz, 1H), 6.49 (dd, J = 7.7, 0.8 Hz, 1H), 6.24 (dd, J = 8.3, 0.8 Hz, 1H), 1.13 (s, 9H). LCMS *m*/*z* 422.0 [M+H]⁺.

### Compound 124

### 4-[1-(4-fluorophenyl)-4-hydroxy-2-(3-hydroxy-1,1-dimethyl-propyl)indol-3-yl]benzoic acid (124)

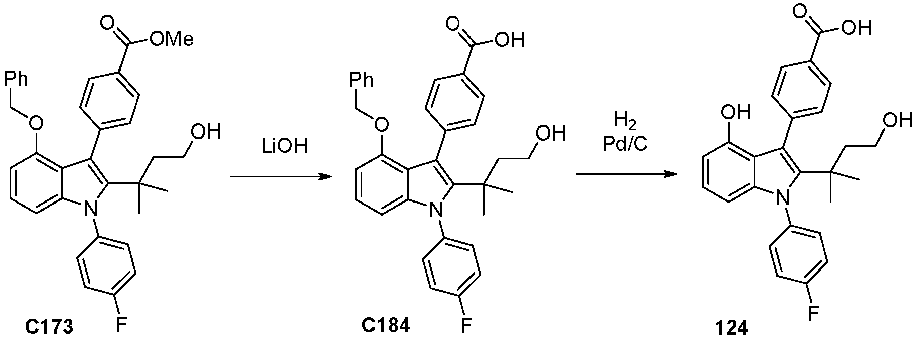

Compound **124** was prepared from **C173** according to the method the described in the synthesis of compound **1.** The resulting material was triturated in EtOAc in heptane (3:1), filtered, and dried to afford the product compound **124.** 4-[1-(4-fluorophenyl)-4-hydroxy-2-(3-hydroxy-1,1-dimethyl-propyl)indol-3-yl]benzoic acid (40 mg, 51%). ¹H NMR (400 MHz, DMSO-*d*₆) δ 8.83 (s, 1H), 7.90 - 7.84 (m, 2H), 7.55 - 7.39 (m, 6H), 6.79 - 6.73 (m, 1H), 6.25 (dd, J = 7.7, 0.8 Hz, 1H), 5.92 (dd, J = 8.2, 0.8 Hz, 1H), 4.26 (s, 1H), 3.34 (s, 2H), 1.54 (t, J = 7.7 Hz, 2H), 0.96 (s, 6H). LCMS *m*/*z* 434.0 [M+H]⁺.

### Compound 125

### 4-[2-[2-(difluoromethoxy)-1,1-dimethyl-ethyl]-1-(4-fluorophenyl)-4-hydroxy-indol-3-yl]benzoic acid (125)

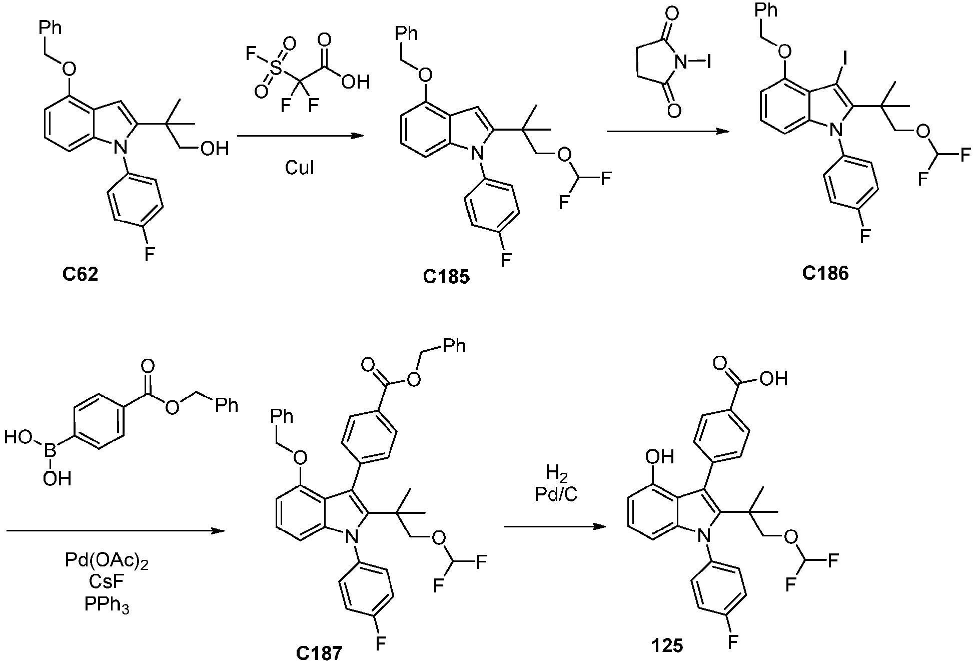

### Step 1. Synthesis of 4-benzyloxy-2-[2-(difluoromethoxy)-1,1-dimethyl-ethyl]-1-(4-fluorophenyl)indole (C185)

To a solution of 2-[4-benzyloxy-1-(4-fluorophenyl)indol-2-yl]-2-methyl-propan-1-ol C62 (510 mg, 1.309 mmol) and CuI (50 mg, 0.26 mmol) in MeCN (10 mL) was heated at 50 °C and 2,2-difluoro-2-fluorosulfonyl-acetic acid (200 µL, 1.94 mmol) in MeCN (3 mL) was added dropwise over 30 minutes, then stirred for 1 hour at the same temperature. The reaction was cooled down to room temperature and concentrated to give a light yellow solid which was used without further purification. 4-benzyloxy-2-[2-(difluoromethoxy)-1,1-dimethyl-ethyl]-1-(4-fluorophenyl)indole (574 mg, 98%). ¹H NMR (400 MHz, Chloroform-*d*) δ 7.60 - 7.52 (m, 2H), 7.50 - 7.42 (m, 3H), 7.40 - 7.36 (m, 3H), 7.26 - 7.21 (m, 2H), 6.99 (t, J = 8.0 Hz, 1H), 6.71 (d, J = 0.8 Hz, 1H), 6.61 (d, J = 7.7 Hz, 1H), 6.31 (dd, J = 8.2, 0.7 Hz, 1H), 5.26 (s, 2H), 3.76 (s, 2H), 1.33 (s, 6H). LCMS *m*/*z* 440.22 [M+H]⁺.

### Steps 2-4. Synthesis of 4-[2-[2-(difluoromethoxy)-1,1-dimethyl-ethyl]-1-(4-fluorophenyl)-4-hydroxy-indol-3-yl]benzoic acid (125)

Compound **125** 4-[2-[2-(difluoromethoxy)-1,1-dimethyl-ethyl]-1-(4-fluorophenyl)-4-hydroxy-indol-3-yl]benzoic acid was prepared in three steps from compound **C185** using the method described for the preparation of compound **1,** omitting the ester hydrolysis step. Pd(OAc)₂, PPh₃ and CsF were used in the Suzuki coupling step. **C187** was converted to compound **125** by hydrogenation. 4-[2-[2-(difluoromethoxy)-1,1-dimethyl-ethyl]-1-(4-fluorophenyl)-4-hydroxy-indol-3-yl]benzoic acid (16.7 mg, 80%). ¹H NMR (400 MHz, Chloroform-d) δ 8.16 (d, J = 8.1 Hz, 2H), 7.71 - 7.62 (m, 2H), 7.41 - 7.31 (m, 2H), 7.18 (d, J = 8.4 Hz, 2H), 6.89 (t, J = 8.0 Hz, 1H), 6.41 (dd, J = 7.7, 0.8 Hz, 1H), 6.25 - 5.80 (m, 2H), 3.53 (s, 2H), 1.01 (s, 6H). LCMS *m*/*z* 470.15 [M+H]⁺.

### Compound 126

### 4-[1-(4-fluorophenyl)-4-hydroxy-2-(3-methoxy-1,1-dimethyl-propyl)indol-3-yl]benzoic acid (126)

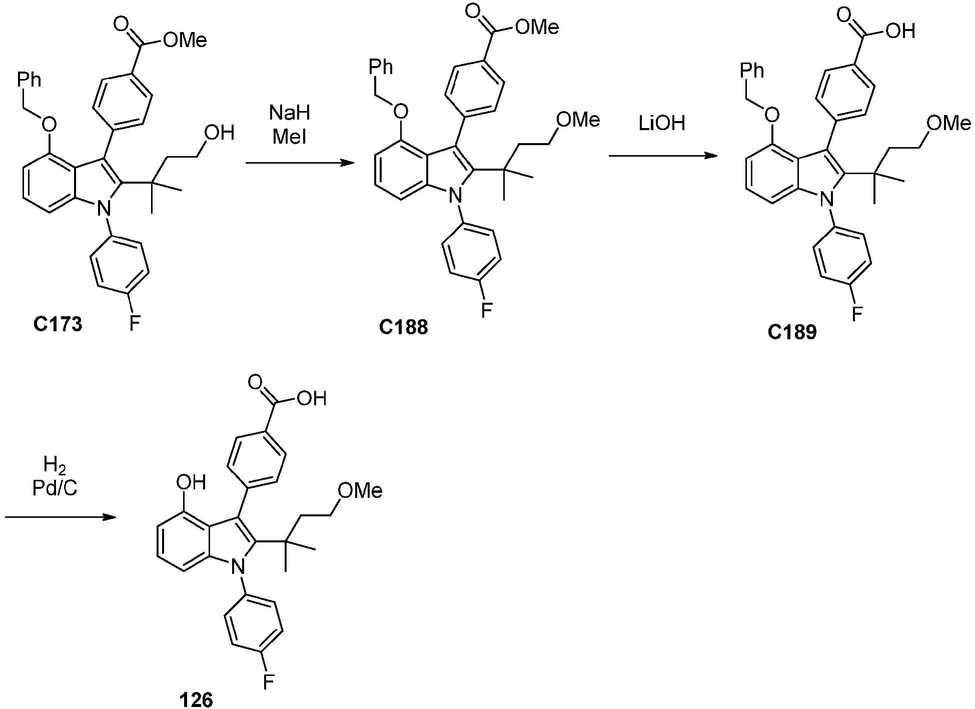

### Step 1. Synthesis of methyl 4-[4-benzyloxy-1-(4-fluorophenyl-2-(3-methoxy-1,1-dimethylpropyl)indol-3-yl]benzoate (C188)

To a solution of methyl 4-[4-benzyloxy-1-(4-fluorophenyl)-2-(3-hydroxy-1,1-dimethyl-propyl)indol-3-yl]benzoate **C173**(100 mg, 0.19 mmol) and MeI (60 µL, 0.96 mmol) in THF (1 mL) was added NaH (12 mg, 0.3 mmol). The reaction mixture was stirred at room temperature overnight then quenched with water, and extracted with EtOAc. The organic layer was concentrated to dryness and purified via silica gel chromatography (Gradient: 0-40% EtOAc in heptane) to afford the product as a white solid. Methyl 4-[4-benzyloxy-1-(4-fluorophenyl)-2-(3-methoxy-1,1-dimethyl-propyl)indol-3-yl]benzoate (18 mg, 18%). ¹H NMR (400 MHz, Chloroform-d) δ 7.92 - 7.87 (m, 2H), 7.52 - 7.47 (m, 2H), 7.45 - 7.40 (m, 2H), 7.28 - 7.23 (m, 2H), 7.20 - 7.13 (m, 3H), 7.04 - 6.99 (m, 1H), 6.81 - 6.77 (m, 2H), 6.54 (dd, J = 7.9, 0.7 Hz, 1H), 6.27 (dd, J = 8.3, 0.7 Hz, 1H), 4.83 (s, 2H), 3.98 (s, 3H), 3.32 (dd, J = 8.2, 7.0 Hz, 2H), 3.26 (s, 3H), 1.73 - 1.67 (m, 2H), 1.05 (s, 6H). LCMS *m*/*z* 552.0 [M+H]⁺.

### Steps 2-3. Synthesis of 4-[1-(4-fluorophenyl)-4-hydroxy-2-(3-methoxy-1,1-dimethylpropyl)indol-3-yl]benzoic acid (126)

Compound **126** was prepared from **C188** in two steps by ester hydrolysis and hydrogenation as described in the preparation of compound 1. 4-[1-(4-fluorophenyl)-4-hydroxy-2-(3-methoxy-1,1-dimethyl-propyl)indol-3-yl]benzoic acid (5 mg, 32%). ¹H NMR (400 MHz, DMSO-*d*₆) δ 8.85 (s, 1H), 7.89 (d, J = 8.1 Hz, 2H), 7.54 - 7.39 (m, 6H), 6.76 (t, J = 7.9 Hz, 1H), 6.26 (d, J = 7.6 Hz, 1H), 5.93 (d, J = 8.2 Hz, 1H), 3.64 - 3.56 (m, 2H), 3.14 (s, 3H), 1.57 (t, J = 7.3 Hz, 2H), 0.99 (s, 6H). LCMS *m*/*z* 448.0 [M+H]⁺.

### Compound 127

### 4-[2-(1, 1-difluoro-2-hydroxy-ethyl)-1-(4-fluorophenyl)-4-hydroxy-indol-3-yl]benzoic acid (127)

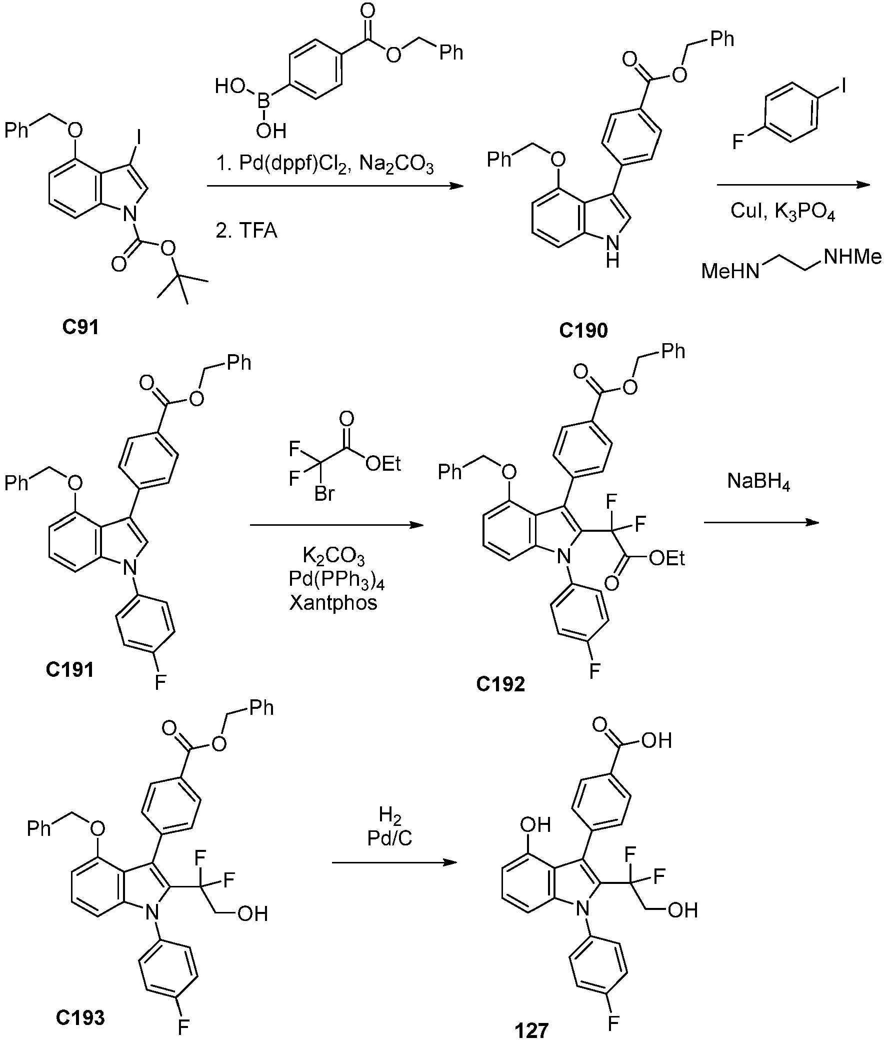

### Step 1 and 2: Synthesis of benzyl 4-(4-benzyloxy-]H-indol-3-yl)benzoate (C190)

To a solution of tert-butyl 4-benzyloxy-3-iodo-indole-1-carboxylate C91 (3.3 g, 7.1 mmol) and (4-benzyloxycarbonylphenyl)boronic acid (2.5 g, 9.76 mmol) in DMF (22 mL) was bubbled with nitrogen for 20 minutes, then added Pd(dppf)Cl₂ (580 mg, 0.71 mmol) and pre-degassed Na₂CO₃ (11 mL of 2 M, 22 mmol) in water (11 mL). The suspension was heated to 100 °C on a hot bath and reacted for 5 hours. The reaction was cooled to room temperature, and ice (100 g) was added. The solid was filtered off and washed with water (20 mL x 2). EtOAc (100 mL) was added to the filtrate, and the filter funnel, further washed with EtOAc (50 mL). The organic solution was washed with water (20 mL) and brine (20 mL), then dried over MgSO₄, concentrated to give the product as a dark brown liquid, which turned into a dark solid upon drying under vacuum which was used in the subsequent step without further purification. tert-butyl 4-benzyloxy-3-(4-benzyloxycarbonylphenyl)indole-1-carboxylate (4.2 g, 107%). LCMS *m*/*z* 534.54 [M+H]⁺.

To a solution of tert-butyl 4-benzyloxy-3-(4-benzyloxycarbonylphenyl)indole-1-carboxylate (4.3 g, 7.71 mmol) in dichloromethane (20 mL) was added TFA (12 mL, 155.8 mmol) dropwise, and stirred for 1 hour. The solvent was removed and the product was purified by silica gel chromatography (40g column, Gradient: 0-50% EtOAc in hexanes) to afford the product as a white solid. benzyl 4-(4-benzyloxy-1H-indol-3-yl)benzoate (2.8 g, 80%). ¹H NMR (400 MHz, Chloroform-d) δ 8.00 - 7.93 (m, 3H), 7.67 - 7.58 (m, 3H), 7.55 - 7.50 (m, 2H), 7.47 - 7.31 (m, 5H), 7.22 - 7.14 (m, 3H), 7.11 - 7.03 (m, 2H), 6.87 (d, J = 8.0 Hz, 1H), 5.43 (s, 2H), 5.07 (s, 2H). LCMS *m*/*z* 434.3 [M+H]⁺.

### Step 3. Synthesis of benzyl 4-[4-benzyloxy-1-(4-fluorophenyl)indol-3-yl]benzoate (C191)

To a vial was added CuI (56 mg, 0.29 mmol), benzyl 4-(4-benzyloxy-1H-indol-3-yl)benzoate **C190** (660 mg, 1.46 mmol), K₃PO₄ (650 mg, 3.06 mmol). The vessel was evacuated and back-filled with argon, and this sequence was repeated an additional time. Toluene (7 mL) was added, followed by the successive addition of 1-fluoro-4-iodo-benzene (350 µL, 3.04 mmol) and N,N'-dimethylethane-1,2-diamine (62 µL, 0.58 mmol) under a stream of argon. The reaction tube was sealed and the contents were stirred with heating at 110 °C for 24 hours. The reaction mixture was cooled to ambient temperature, diluted with ethyl acetate (2-3 mL), filtered through a plug of silica gel, eluting with additional ethyl acetate (10-20 mL). The filtrate was concentrated and the resulting residue was purified by column silica gel chromatography (Gradient: 0-80% EtOAc in hexane) to provide the desired product as a white solid. benzyl 4-[4-benzyloxy-1-(4-fluorophenyl)indol-3-yl]benzoate (170 mg, 22%). ¹H NMR (400 MHz, Chloroform-*d*) δ 8.01 - 7.93 (m, 2H), 7.78 - 7.66 (m, 2H), 7.59 - 7.36 (m, 7H), 7.36 - 7.17 (m, 9H), 7.14 (dd, J = 8.3, 0.8 Hz, 1H), 6.75 (dd, J = 7.7, 0.8 Hz, 1H), 5.43 (s, 2H), 5.15 (s, 2H). LCMS *m*/*z* 528.37 [M+H]⁺.

### Step 4. Synthesis of benzyl 4-[4-benzyloxy-2-(2-ethoxy-1,1-difluoro-2-oxo-ethyl)-1-(4-fluorophenyl)indol-3-yl]benzoate (C192)

A 5 mL microwave vial was charged with benzyl 4-[4-benzyloxy-1-(4-fluorophenyl)indol-3-yl]benzoate **C191** (167 mg, 0.32 mmol), ethyl 2-bromo-2,2-difluoro-acetate (130 mg, 0.64 mmol), K₂CO₃ (90 mg, 0.65 mmol), Xantphos (18.5 mg, 0.03 mmol) (10 mol%) and 1,4- dioxane (3 mL). The solution was bubbled with nitrogen for 5 minutes, then Pd(PPh₃)₄ (18.3 mg, 0.02 mmol) was added and the reaction was heated at 110 °C for 2 hour. The mixture was allowed to cool down to room temperature and diluted with ethyl acetate (10 mL). The reaction mixture was washed with saturated aqueous NaHSO₃ solution (5 mL) and brine (5 mL, x 2), then concentrated under reduced pressure. Purification by silica gel chromatography (12g column, Gradient: 0-30% EtOAc in hexanes) afforded the product as a white solid. benzyl 4-[4-benzyloxy-2-(2-ethoxy-1,1-difluoro-2-oxo-ethyl)-1-(4-fluorophenyl)indol-3-yl]benzoate (37.5 mg, 18%). ¹H NMR (400 MHz, Chloroform-d) δ 7.89 - 7.85 (m, 2H), 7.58 - 7.55 (m, 2H), 7.44 - 7.39 (m, 4H), 7.39 - 7.30 (m, 4H), 7.22 - 7.15 (m, 3H), 7.14 - 7.10 (m, 3H), 7.10 - 7.06 (m, 2H), 6.84 (d, J = 1.3 Hz, 1H), 5.32 (d, J = 2.8 Hz, 3H), 5.06 (s, 2H), 4.20 (q, J = 7.2 Hz, 2H), 1.21 (d, J = 7.1 Hz, 3H). LCMS *m*/*z* 650.23 [M+H]⁺.

### Step 5. Synthesis of benzyl 4-[4-benzyloxy-2-(1,1-difluoro-2-hydroxy-ethyl)-1-(4-fluorophenyl)indol-3-yl]benzoate (C193)

Benzyl 4-[4-benzyloxy-2-(2-ethoxy-1,1-difluoro-2-oxo-ethyl)-1-(4-fluorophenyl)indol-3-yl]benzoate **C192** (37.5 mg, 0.06 mmol) in MeOH (2 mL) and THF (1 mL) (THF was added because of poor solubility of **C192** in MeOH) were added NaBH₄ (8 mg, 0.21 mmol) at room temperature for 1 hour. Additional NaBH₄ (8 mg, 0.21 mmol) was added and the reaction allowed to stir overnight. An additional portion of NaBH₄ (8 mg, 0.22 mmol) was added to complete the reaction. Water (2 mL) was added to the reaction mixture, followed by extraction with EtOAc (3 x 5 mL). The combined organic fractions were washed with brine (20 mL), water (2 x 20 mL), dried over sodium sulfate and concentrated to dryness. Purification by silica gel chromatography (Gradient: 0-30% EtOAc in hexanes) afforded the product as a white solid. benzyl 4-[4-benzyloxy-2-(1,1-difluoro-2-hydroxy-ethyl)-1-(4-fluorophenyl)indol-3-yl]benzoate (19.2 mg, 54%). ¹H NMR (400 MHz, Chloroform-d) δ 7.95 - 7.86 (m, 2H), 7.49 - 7.41 (m, 4H), 7.40 - 7.30 (m, 5H), 7.18 - 7.10 (m, 2H), 7.07 (d, J = 8.1 Hz, 1H), 7.02 - 6.94 (m, 3H), 6.71 - 6.63 (m, 2H), 6.52 (d, J = 7.8 Hz, 1H), 6.48 (d, J = 8.3 Hz, 1H), 5.34 (s, 2H), 4.82 (s, 2H), 3.50 (td, J = 13.6, 7.2 Hz, 2H), 1.59 (t, J = 7.3 Hz, 1H). LCMS *m*/*z* 608.2 [M+H]⁺.

### Step 6. Synthesis of 4-[2-(1,1-difluoro-2-hydroxy-ethyl)-fluorophenyl)-4-hydroxy-indol-3-yl]benzoic acid (127)

To a mixture of benzyl 4-[4-benzyloxy-2-(1,1-difluoro-2-hydroxy-ethyl)-1-(4-fluorophenyl)indol-3-yl]benzoate **C193** (6 mg, 0.01 mmol) and Pd on carbon (1.0 mg of 10% w/w) was added THF (250 µL) and EtOH (250 µL). The mixture was then bubbled with H₂ (balloon for 1 min), then sealed for 2 hours. The catalyst was removed by filtration and the mixture concentrated to afford the desired product as a light yellow solid. 4-[2-(1,1-difluoro-2-hydroxy-ethyl)-1-(4-fluorophenyl)-4-hydroxy-indol-3-yl]benzoic acid (2.4 mg, 58%). ¹H NMR (400 MHz, Chloroform-d) δ 8.08 - 7.95 (m, 2H), 7.57 (d, J = 7.8 Hz, 2H), 7.37 (dd, J = 8.0, 4.7 Hz, 2H), 7.25 (q, J = 6.0, 3.9 Hz, 1H), 7.14 (t, J = 7.4 Hz, 2H), 6.98 (t, J = 8.0 Hz, 1H), 6.43 (d, J = 7.7 Hz, 1H), 6.37 (d, J = 8.3 Hz, 1H), 3.53 - 3.42 (m, 4H). LCMS *m*/*z* 427.98 [M+H]⁺.

### Compound 128

### 4-[2-(1,1-difluoro-2-methoxy-ethyl)-1-(4-fluorophenyl)-4-hydroxy-indol-3-yl]benzoic acid (128)

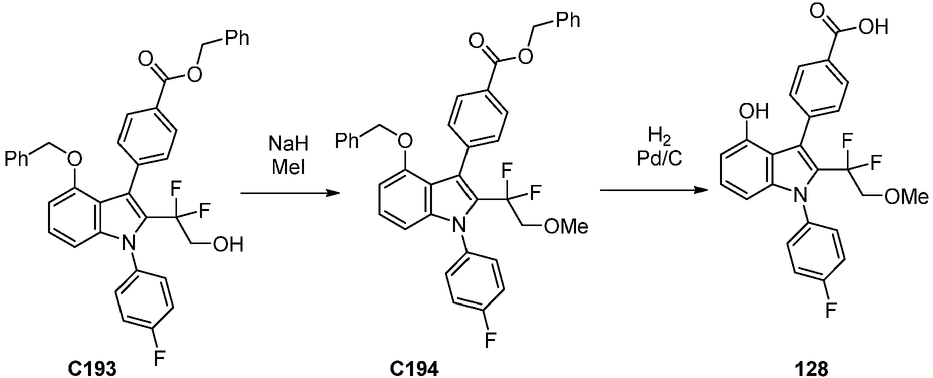

Compound **128** was prepared in two steps from compound C193 by methylation and then hydrogenation using the methods described in the preparation of compound 126. Purification by reversed-phase chromatography (Column: C18. Gradient: 10-100% MeCN in water with 0.2% formic acid) afforded the product as a white solid. 4-[2-(1,1-difluoro-2-methoxy-ethyl)-1-(4-fluorophenyl)-4-hydroxy-indol-3-yl]benzoic acid (5.8 mg, 68%). ¹H NMR (400 MHz, Acetonitrile-d3) δ 8.03 (dd, J = 8.2, 1.6 Hz, 2H), 7.64 - 7.58 (m, 2H), 7.50 (dd, J = 8.2, 5.0 Hz, 2H), 7.34 (td, J = 8.6, 1.6 Hz, 2H), 7.13 - 7.03 (m, 1H), 7.03 - 6.77 (m, 1H), 6.51 (dd, J = 7.7, 1.4 Hz, 1H), 6.46 - 6.38 (m, 1H), 3.46 (td, J = 14.0, 1.5 Hz, 2H), 3.16 (d, J = 1.4 Hz, 3H). LCMS *m*/*z* 442.02 [M+H]⁺.

### Compound 129

### 3-fluoro-5-[1-(4-fluorophenyl)-4-hydroxy-2-isopropyl-indol-3-yl]pyridine-2-carboxylic acid (129)

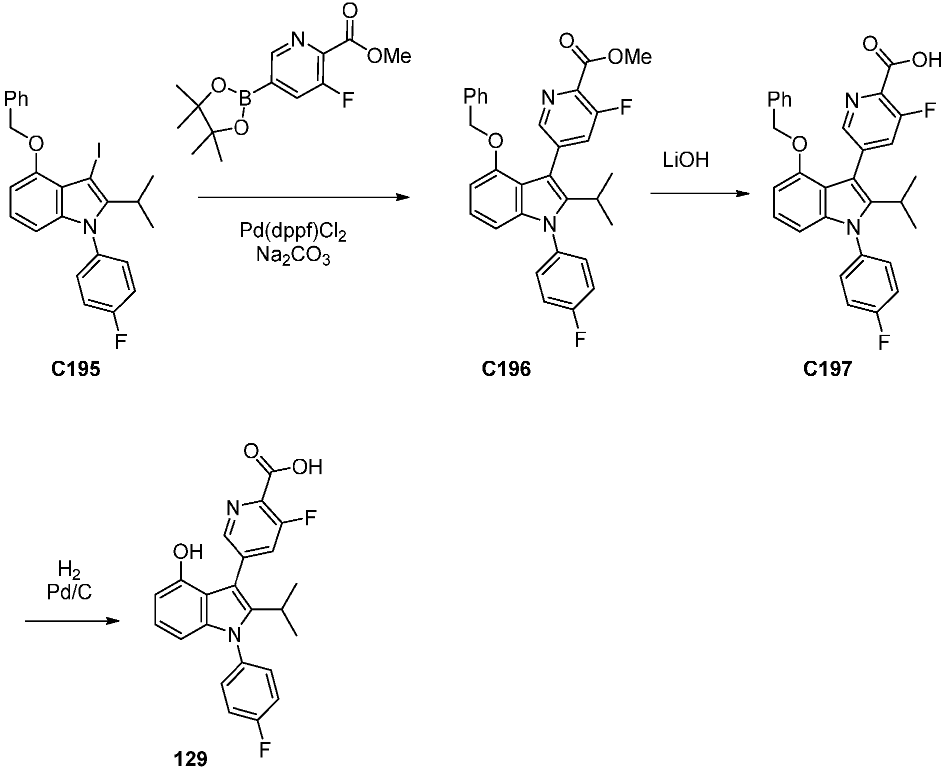

Compound 129 was prepared in two steps from C195 according to the method described in the synthesis of compound 1. Purification by reversed-phase chromatography (Column: C18. Gradient: 5-95% MeCN in water with 0.1% trifluoroacetic acid) afforded the product. 3-fluoro-5-[1-(4-fluorophenyl)-4-hydroxy-2-isopropyl-indol-3-yl]pyridine-2-carboxylic acid (54 mg, 70%) ¹H NMR (400 MHz, DMSO-*d*₆) δ 9.47 (s, 1H), 8.53 (s, 1H), 7.87 (d, J = 11.5 Hz, 1H), 7.54 (ddt, J = 8.3, 5.5, 2.7 Hz, 2H), 7.51 - 7.43 (m, 2H), 6.88 (t, J = 7.9 Hz, 1H), 6.49 - 6.41 (m, 1H), 6.23 (d, J = 8.1 Hz, 1H), 3.01 (p, J = 7.1 Hz, 1H), 1.04 (d, J = 7.2 Hz, 6H). LCMS *m*/*z* 409.0 [M+H]⁺.

### Compound 130

### 4-[2-[1,1-dimethyl-2-(methylamino)-2-oxo-ethyl]-1-(4-fluorophenyl)-4-hydroxy-indol-3-yl]benzoic acid (130)

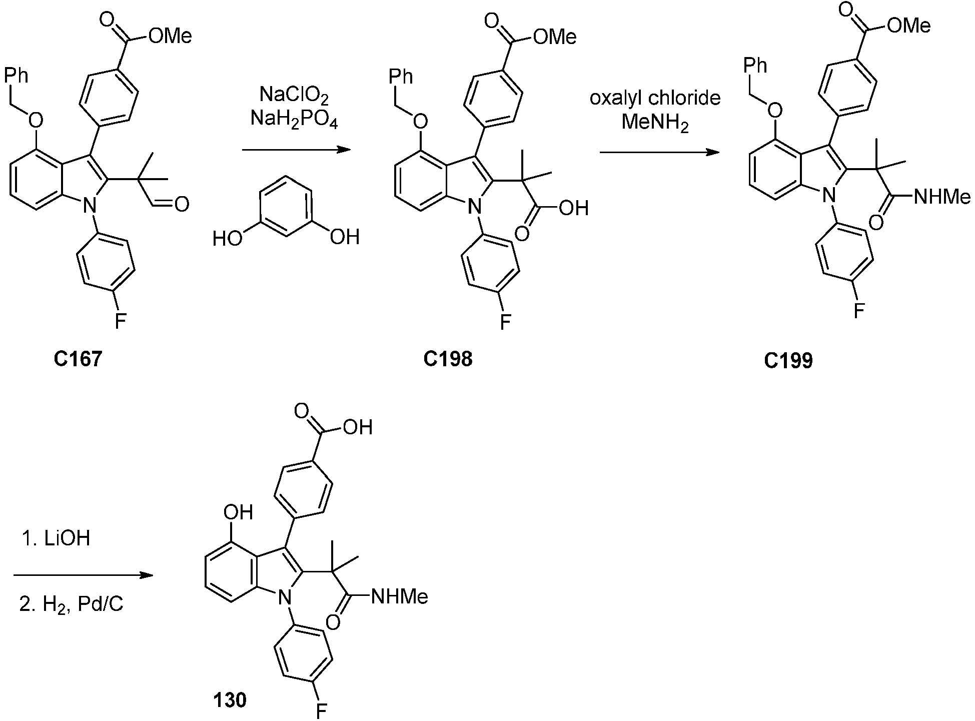

### Step 1. Synthesis of 2-[4-benzyloxy-1-(4-fluorophenyl)-3-(4-methoxycarbonylphenyl)indol-2-yl]-2-methyl-propanoic acid (C198)

To a suspension of methyl 4-[4-benzyloxy-2-(1,1-dimethyl-2-oxo-ethyl)-1-(4-fluorophenyl)indol-3-yl]benzoate C167 (132 mg, 0.25 mmol) and benzene-1,3-diol (60 mg, 0.55 mmol) in t-BuOH (4 mL) was added a solution of sodium dihydrogen phosphate (66 mg, 0.55 mmol) and sodium chlorite (47 mg, 0.52 mmol) in water (1 mL). The reaction mixture was stirred at room temperature overnight. Additional portions of sodium chlorite and sodium dihydrogen phosphate (10 eq. each) in water (10 mL) were added and the reaction was stirred at room temperature for 30 minutes. The reaction mixture was diluted with water and washed with EtOAc. The organic layer was dried over magnesium sulfate, filtered, and concentrated. The residue was purification by reversed-phase chromatography (Column: C18. Gradient: 10-90% MeCN in water with 0.1% trifluoroacetic acid). Fractions were combined, diluted with water, and extracted with EtOAc. The organic layer was dried over magnesium sulfate, filtered, and concentrated to afford the product as an off-white solid. 2-[4-benzyloxy-1-(4-fluorophenyl)-3-(4-methoxycarbonylphenyl)indol-2-yl]-2-methyl-propanoic acid (43 mg, 32%). ¹H NMR (300 MHz, Chloroform-d) δ 7.85 - 7.78 (m, 2H), 7.47 - 7.43 (m, 2H), 7.32 - 7.24 (m, 2H), 7.07 - 6.98 (m, 4H), 6.93 (t, J = 8.1 Hz, 1H), 6.69 - 6.63 (m, 2H), 6.44 (d, J = 7.8 Hz, 1H), 6.25 (d, J = 8.2 Hz, 1H), 4.75 (s, 2H), 3.86 (s, 3H), 1.22 (s, 6H). LCMS *m*/*z* 538.0 [M+H]⁺.

### Step 2. Synthesis of methyl 4-[4-benzyloxy-2-[1,1-dimethyl-2-(methylamino)-2-oxo-ethyl]-1-(4-fluorophenyl)indol-3-yl]benzoate (C199)

To a solution of 2-[4-benzyloxy-1-(4-fluorophenyl)-3-(4-methoxycarbonyl-phenyl)indol-2-yl]-2-methyl-propanoic acid **C198** (43 mg, 0.08 mmol) in dichloromethane (400 µL) was added oxalyl chloride (52 µL of 2 M, 0.10 mmol) followed by DMF (2 µL, 0.026 mmol) (exotherm). The reaction was stirred for 15 minutes then concentrated to dryness, taken up in THF (400 µL), and treated with methylamine (120 µL of 2 M, 0.24 mmol). The mixture was stirred for 45 minutes, and then diluted with EtOAc and washed with water. The organic layer was concentrated to dryness and purified by silica gel chromatography (Gradient: 0-40% EtOAc in heptane) to afford the product as a white solid. methyl 4-[4-benzyloxy-2-[1,1-dimethyl-2-(methylamino)-2-oxo-ethyl]-1-(4-fluorophenyl)indol-3-yl]benzoate (43 mg, 98%). ¹H NMR (400 MHz, Chloroform-d) δ 7.85 - 7.80 (m, 2H), 7.47 - 7.42 (m, 2H), 7.23 (ddt, J = 8.2, 5.5, 2.8 Hz, 2H), 7.15 - 7.02 (m, 5H), 6.97 (t, J = 8.1 Hz, 1H), 6.72 - 6.67 (m, 2H), 6.48 (dd, J = 7.8, 0.7 Hz, 1H), 6.27 (dd, J = 8.3, 0.7 Hz, 1H), 5.42 (d, J = 4.9 Hz, 1H), 4.77 (s, 2H), 3.89 (s, 3H), 2.56 (d, J = 4.8 Hz, 3H), 1.13 (s, 6H). LCMS *m*/*z* 551.0 [M+H]⁺.

### Step 3 and 4: Synthesis of 4-[2-[1,1-dimethyl-2-(methylamino)-2-oxo-ethyl]-1-(4-fluorophenyl)-4-hydroxy-indol-3-yl]benzoic acid (130)

Compound **130** was prepared from **C199** in two steps by ester hydrolysis then hydrogenation as described in the synthesis of compound **1.** Purification by reversed-phase chromatography (Column: C18. Gradient: 20-95% MeCN in water with 0.1% trifluoroacetic acid) afforded the product. 4-[2-[1,1-dimethyl-2-(methylamino)-2-oxo-ethyl]-1-(4-fluorophenyl)-4-hydroxy-indol-3-yl]benzoic acid (8 mg, 29%). ¹H NMR (400 MHz, Chloroform-d) δ 8.28 - 8.21 (m, 2H), 7.79 - 7.73 (m, 2H), 7.42 - 7.36 (m, 2H), 7.26 (t, J = 8.5 Hz, 2H), 7.04 (t, J = 8.0 Hz, 1H), 6.53 (dd, J = 7.8, 0.8 Hz, 1H), 6.32 (dd, J = 8.3, 0.8 Hz, 1H), 5.80 (d, J = 5.0 Hz, 1H), 2.67 (d, J = 4.7 Hz, 3H), 1.33 (s, 6H). LCMS *m*/*z* 447.0 [M+H]⁺.

### Compound 131

### 4-[1-(4-fluorophenyl)-4-hydroxy-2-(pyrrolidine-1-carbonyl)indol-3-yl]benzoic acid (131)

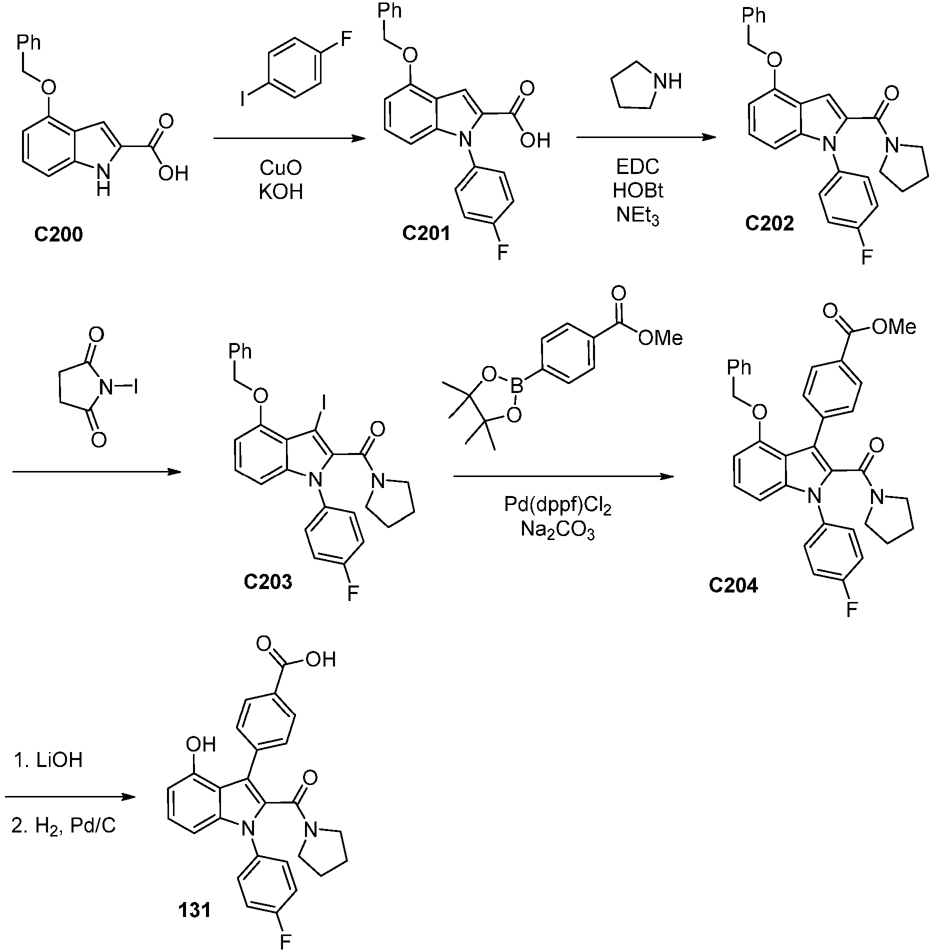

### Step 1. Synthesis of 4-benzyloxy-1-(4-fluorophenyl)indole-2-carboxylic acid (C201)

To a suspension of 4-benzyloxy-1H-indole-2-carboxylic acid **C200** (1 g, 3.74 mmol), CuO (300 mg, 3.77 mmol) and KOH (525 mg, 9.36 mmol) in DMF (50 mL) was added 1-fluoro-4-iodo-benzene (863 µL, 7.48 mmol). The reaction mixture was stirred at 155 °C for 1 hour, cooled to room temperature, then acidified with 1 M aq. HCl. The mixture was extracted with EtOAc and the organic layer was concentrated to dryness. The residue was purified by reversed-phase chromatography (Column: C18. Gradient: 5-90% MeCN in water with 0.1% trifluoroacetic acid). The combined product fractions were diluted with water, and extracted with EtOAc. The organic layer was dried over magnesium sulfate, filtered, and concentrated to afford the product as a brown solid. 4-benzyloxy-1-(4-fluorophenyl)indole-2-carboxylic acid (790 mg, 58%). ¹H NMR (400 MHz, DMSO-*d*₆) δ 7.56 - 7.52 (m, 2H), 7.47 - 7.40 (m, 4H), 7.40 - 7.31 (m, 4H), 7.22 - 7.15 (m, 1H), 6.76 (d, J = 7.8 Hz, 1H), 6.59 (d, J = 8.4 Hz, 1H), 5.30 (s, 2H). LCMS *m*/*z* 362.0 [M+H]⁺.

### Step 2. Synthesis of [4-benzyloxy-1-(4-fluorophenyl)indol-2-yl]-pyrrolidin-1-yl-methanone (C202)

To a solution of 4-benzyloxy-1-(4-fluorophenyl)indole-2-carboxylic acid ***C201*** (700 mg, 1.94 mmol), EDC (560 mg, 2.92 mmol) and HOBt (450 mg, 2.94 mmol) in DMF (8.4 mL) was added pyrrolidine (340 µL, 4.07 mmol) and Et₃N (820 µL, 5.88 mmol). The reaction mixture was stirred overnight at room temperature then diluted with water and extracted with EtOAc. The organic layer was concentrated to dryness and purified by silica gel chromatography (Gradient: 0-40% EtOAc in heptane). Pure fractions were combined and concentrated to give 642 mg yellow solid. [4-benzyloxy-1-(4-fluorophenyl)indol-2-yl]-pyrrolidin-1-yl-methanone (642 mg, 80%). ¹H NMR (400 MHz, Chloroform-d) δ 7.58 - 7.53 (m, 2H), 7.48 - 7.37 (m, 5H), 7.23 - 7.16 (m, 3H), 7.13 (d, J = 0.8 Hz, 1H), 6.88 (dt, J = 8.3, 0.7 Hz, 1H), 6.68 (dd, J = 7.9, 0.6 Hz, 1H), 5.28 (s, 2H), 3.57 (dt, J = 15.0, 6.1 Hz, 4H), 1.95 - 1.80 (m, 4H). LCMS *m*/*z* 415.0 [M+H]⁺.

### Step 3. Synthesis of [4-benzyloxy-1-(4-fluorophenyl)-3-iodo-indol-2-yl]-pyrrolidin-1-yl-methanone (C203)

Compound **C203** was prepared from **C202** (642 mg, 1.55 mmol) by iodination with N-iodosuccinimide as described in the preparation of **S1.** Purification by silica gel chromatography (Gradient: 0-40% EtOAc in heptane) to afford the product. [4-benzyloxy-1-(4-fluorophenyl)-3-iodo-indol-2-yl]-pyrrolidin-1-yl-methanone (626 mg, 75%) ¹H NMR (400 MHz, Chloroform-d) δ 7.65 (ddt, J = 8.1, 1.2, 0.6 Hz, 2H), 7.43 (td, J = 6.3, 5.9, 1.9 Hz, 4H), 7.38 - 7.35 (m, 1H), 7.23 - 7.18 (m, 2H), 7.18 - 7.12 (m, 1H), 6.86 (dt, J = 7.8, 0.9 Hz, 1H), 6.69 (dd, J = 7.9, 0.7 Hz, 1H), 5.29 (d, J = 2.0 Hz, 2H), 3.70 - 3.48 (m, 2H), 3.37 (dt, J = 12.9, 6.8 Hz, 1H), 3.04 (dt, J = 10.7, 6.4 Hz, 1H), 1.98 - 1.69 (m, 4H). LCMS *m*/*z* 541.0 [M+H]⁺

### Step 4-6. Synthesis of 4-[1-(4-fluorophenyl)-4-hydroxy-2-(pyrrolidine-1-carbonyl)indol-3-yl]benzoic acid (131)

Compound **131** was prepared in three steps from **C203** by Suzuki coupling, ester hydrolysis and then hydrogenation as described in the synthesis of compound 1 to afford the product as a white solid. 4-[1-(4-fluorophenyl)-4-hydroxy-2-(pyrrolidine-1-carbonyl)indol-3-yl]benzoic acid (80 mg, 52%). ¹H NMR (400 MHz, DMSO-*d*₆) δ 9.89 (s, 1H), 7.94 - 7.89 (m, 2H), 7.59 - 7.53 (m, 2H), 7.51 - 7.46 (m, 2H), 7.40 (t, J = 8.8 Hz, 2H), 7.09 - 7.03 (m, 1H), 6.70 - 6.65 (m, 1H), 6.60 (d, J = 7.6 Hz, 1H), 3.15 (t, J = 6.9 Hz, 2H), 2.77 - 2.53 (m, 2H), 1.54 (d, J = 39.6 Hz, 4H). LCMS *m*/*z* calc. 444.14853, found 445.0 [M+H]⁺.

### Compound 132

### 4-[1-(4-fluorophenyl)-4-hydroxy-2-(1-methyl-1-oxazol-5-yl-ethyl)indol-3-yl]benzoic acid (132)

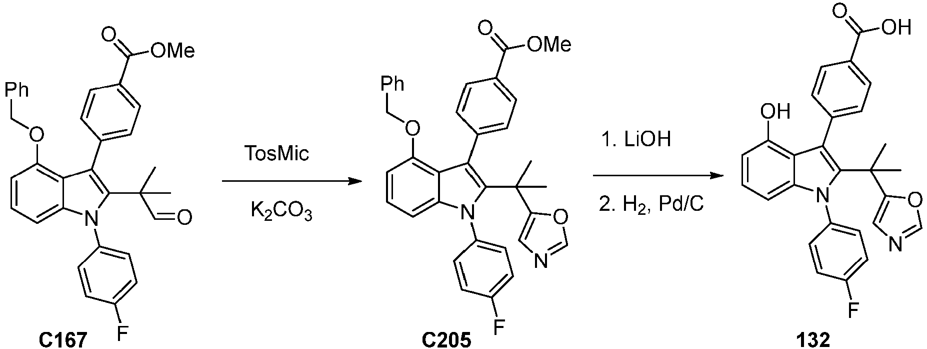

### Step 1. Synthesis of methyl 4-[4-benzyloxy-1-(4-fluorophenyl)-2-(1-methyl-1-oxazol-5-yl-ethyl)indol-3-yl]benzoate (C205)

A suspension of methyl 4-[4-benzyloxy-2-(1,1-dimethyl-2-oxo-ethyl)-1-(4-fluorophenyl)indol-3-yl]benzoate **C167** (400 mg, 0.77 mmol), TosMIC (192 mg, 0.98 mmol), and K₂CO₃ (208 mg, 1.51 mmol) in MeOH (8 mL) was heated overnight at 100 °C in a sealed tube. The reaction mixture was cooled to room temperature, diluted with aq. 1 M HCl, and extracted with EtOAc. The organic layer was concentrated to dryness and purified by silica gel chromatography (Gradient: 0-40% EtOAc in heptane) afforded the product. Methyl 4-[4-benzyloxy-1-(4-fluorophenyl)-2-(1-methyl-1-oxazol-5-yl-ethyl)indol-3-yl]benzoate (9 mg, 2%). LCMS *m*/*z* 561.0 [M+H]⁺.

### Steps 2 & 3. Synthesis of 4-[1-(4-fluorophenyl)-4-hydroxy-2-(1-methyl-1-oxazol-5-yl-ethyl)indol-3-yl]benzoic acid (132)

Compound **132** was prepared in two steps from **C205** by ester hydrolysis then hydrogenation using the methods described in preparation of compound 1. 4-[1-(4-fluorophenyl)-4-hydroxy-2-(1-methyl-1-oxazol-5-yl-ethyl)indol-3-yl]benzoic acid (4.8 mg, 57%). LCMS *m*/*z* 457.0 [M+H]⁺

### Compound 133

### 4-[2-[3-(dimethylamino)-1,1-dimethyl-3-oxo-propyl]-1-(4-fluorophenyl)-4-hydroxy-indol-3-yl]benzoic acid (133)

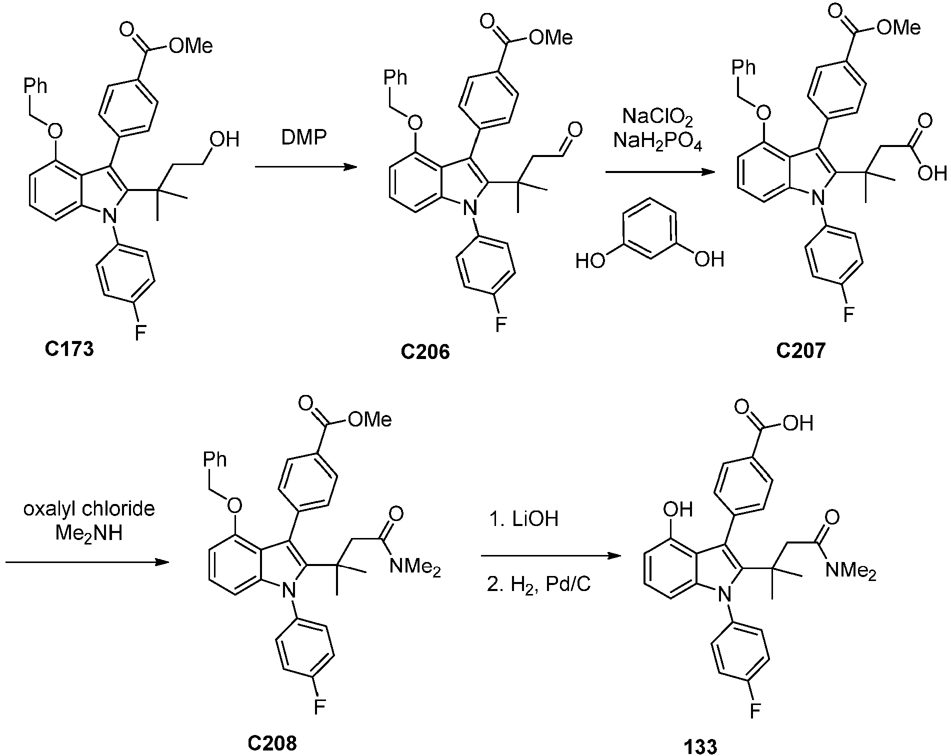

### Step 1. Methyl 4-[4-benzyloxy-2-(1,1-dimethyl-3-oxo-propyl)-1-(4-fluorophenyl)indol-3-yl]benzoate (C206)

Compound **C206** was prepared from compound **C173** (1 g, 1.86 mmol) by oxidation with Dess-Martin periodinane (829 mg, 1.96 mmol) as described in the preparation of intermediate **C167** in the synthesis of compound **119.** Methyl 4-[4-benzyloxy-2-(1,1-dimethyl-3-oxo-propyl)-1-(4-fluorophenyl)indol-3-yl]benzoate (810 mg, 81%). ¹H NMR (400 MHz, Chloroform-d) δ 9.60 (t, J = 2.4 Hz, 1H), 7.93 - 7.87 (m, 2H), 7.51 - 7.40 (m, 4H), 7.29 - 7.23 (m, 2H), 7.20 - 7.12 (m, 3H), 7.06 - 6.99 (m, 1H), 6.79 (dt, J = 7.3, 1.0 Hz, 2H), 6.55 (dd, J = 7.8, 0.7 Hz, 1H), 6.29 (dd, J = 8.3, 0.7 Hz, 1H), 4.83 (s, 2H), 3.98 (s, 3H), 2.40 (d, J = 2.4 Hz, 2H), 1.20 (s, 6H). LCMS *m*/*z* 536.0 [M+H]⁺.

### Steps 2-5. Synthesis of 4-[2-[3-(dimethylamino)-1,1-dimethyl-3-oxo-propyl]-1-(4-fluorophenyl)-4-hydroxy-indol-3-yl]benzoic acid (133)

Compound **133** was prepared in four steps from **C173** using the method described for the preparation of compound **130.** Dimethyl amine was used in step 3. 4-[2-[3-(dimethylamino)-1,1-dimethyl-3-oxo-propyl]-1-(4-fluorophenyl)-4-hydroxy-indol-3-yl]benzoic acid (21 mg, 68%).¹H NMR (300 MHz, DMSO-*d*₆) δ 12.78 (s, 1H), 8.74 (s, 1H), 7.91 - 7.84 (m, 2H), 7.51 - 7.39 (m, 6H), 6.72 (t, J = 7.9 Hz, 1H), 6.24 (d, J = 7.6 Hz, 1H), 5.93 (d, J = 8.1 Hz, 1H), 2.74 (s, 3H), 2.51 (s, 3H), 2.22 (s, 2H), 1.10 (s, 6H). LCMS *m*/*z* 475.0 [M+H]⁺.

### Compound 134

### 4-[2-[1,1-dimethyl-3-(methylamino)-3-oxo-propyl]-1-(4-fluorophenyl)-4-hydroxy-indol-3-yl]benzoic acid (134)

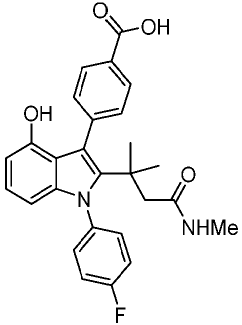

### Preparation of 4-[2-[1,1-dimethyl-3-(methylamino)-3-oxo-propyl]-1-(4-fluorophenyl)-4-hydroxy-indol-3-yl]benzoic acid (134)

Compound **134** was prepared in three steps from **C207** and methyl amine as described for the synthesis of compound **133.** 4-[2-[1,1-dimethyl-3-(methylamino)-3-oxo-propyl]-1-(4-fluorophenyl)-4-hydroxy-indol-3-yl]benzoic acid (22 mg, 81%). ¹H NMR (400 MHz, DMSO-*d*₆) δ 12.74 (s, 1H), 8.78 (s, 1H), 7.88 - 7.82 (m, 2H), 7.66 - 7.60 (m, 2H), 7.55 (d, J = 4.7 Hz, 1H), 7.46 - 7.39 (m, 4H), 6.78 - 6.70 (m, 1H), 6.24 (dd, J = 7.7, 0.8 Hz, 1H), 5.93 (dd, J = 8.2, 0.8 Hz, 1H), 2.54 (d, J = 4.6 Hz, 3H), 2.18 (s, 2H), 1.00 (s, 6H). LCMS *m*/*z* 461.0 [M+H]⁺.

### Compound 135

### 4-[2-(3-amino-1,1-dimethyl-3-oxo-propyl)-1-(4-fluorophenyl)-4-hydroxy-indol-3-yl]benzoic acid (135)

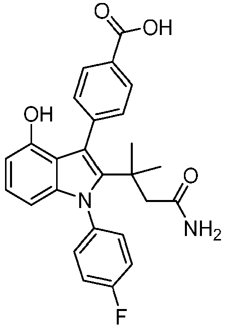

Compound **135** was prepared in three steps from **C207** and ammonia as described for the synthesis of compound **133.** 4-[2-(3-amino-1,1-dimethyl-3-oxo-propyl)-1-(4-fluorophenyl)-4-hydroxy-indol-3-yl]benzoic acid (20 mg, 76%). ¹H NMR (400 MHz, DMSO-*d*₆) δ 12.78 (s, 1H), 8.78 (s, 1H), 7.88 - 7.83 (m, 2H), 7.63 - 7.56 (m, 2H), 7.51 - 7.47 (m, 2H), 7.46 - 7.38 (m, 2H), 7.05 (d, J = 2.6 Hz, 1H), 6.80 - 6.69 (m, 2H), 6.24 (dd, J = 7.6, 0.8 Hz, 1H), 5.92 (dd, J = 8.2, 0.8 Hz, 1H), 2.17 (s, 2H), 1.02 (s, 6H). LCMS *m*/*z* 447.0 [M+H]⁺.

### Compound 136

### 2-fluoro-4-[6-fluoro-1-(4-fluorophenyl)-4-hydroxy-2-(2-methoxy-1,1-dimethyl-ethyl)indol-3-yl]benzoic acid (136)

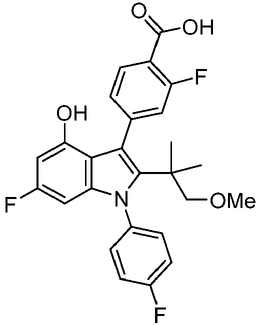

Compound **136** was prepared in three steps from **S22** by Suzuki coupling with (3-fluoro-4-methoxycarbonyl-phenyl)boronic acid using Pd(PPh₃)₄ and CsF, followed by hydrolysis and hydrogenation as described in the preparation of compound 1. Purification by silica gel chromatography afforded the product (Gradient: 0-10% MeOH in dichloromethane) to give a light yellow solid. 2-fluoro-4-[6-fluoro-1-(4-fluorophenyl)-4-hydroxy-2-(2-methoxy-1,1-dimethyl-ethyl)indol-3-yl]benzoic acid (112.7 mg, 86%). ¹H NMR (400 MHz, Chloroform-d) δ 7.99 (td, J = 7.8, 1.8 Hz, 1H), 7.46 - 7.30 (m, 4H), 7.21 - 7.13 (m, 2H), 6.15 (dd, J = 10.8, 2.1 Hz, 1H), 5.87 - 5.77 (m, 1H), 3.05 (s, 3H), 2.92 (d, J = 1.6 Hz, 2H), 1.04 - 0.91 (m, 6H). LCMS *m*/*z* 470.19 [M+H]⁺.

### Compound 137

### 4-[6-fluoro-1-(4-fluorophenyl)-4-hydroxy-2-(2-methoxy-1,1-dimethyl-ethyl)indol-3-yl]benzoic acid (137)

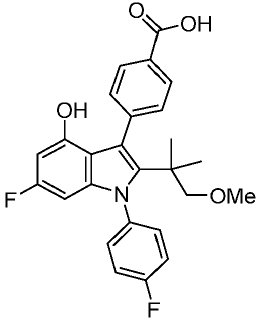

Compound **137** was prepared in two steps from **S22** by Suzuki coupling with (4-benzyloxycarbonylphenyl)boronic acid using Pd(PPh₃)₄ and CsF, followed by hydrogenation as described in the preparation of compound **1.** Silica gel chromatography (Gradient: 0-8% MeOH in dichloromethane) afforded the desired product as white solid. 4-[6-fluoro-1-(4-fluorophenyl)-4-hydroxy-2-(2-methoxy-1,1-dimethyl-ethyl)indol-3-yl]benzoic acid (157 mg, 104%). ¹H NMR (400 MHz, Chloroform-d) δ 8.31 - 8.18 (m, 2H), 7.83 - 7.70 (m, 2H), 7.51 - 7.40 (m, 2H), 7.28 (d, J = 7.8 Hz, 2H), 6.28 (dd, J = 10.8, 2.2 Hz, 1H), 5.92 (dd, J = 9.6, 2.2 Hz, 1H), 3.13 (s, 3H), 3.00 (s, 2H), 1.09 (s, 6H). LCMS *m*/*z* 452.39 [M+H]⁺.

### Compound 138

### 3-fluoro-4-[1-(4-fluorophenyl)-4-hydroxy-2-(2-methoxy-1,1-dimethyl-ethyl)indol-3-yl]benzoic acid (138)

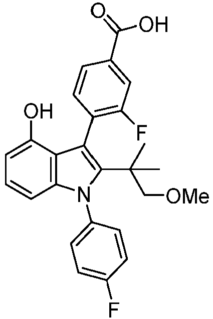

Compound **138** was prepared in three steps from **S20** by Suzuki coupling with (2-fluoro-4-methoxycarbonyl-phenyl)boronic acid using Pd(dppf)Cl₂ and Na₂CO₃, followed by ester hydrolysis and hydrogenation as described in the preparation of compound 1. The product was purified by reverse phase HPLC and then by silica gel chromatography (Gradient: 0-10% MeOH in dichloromethane) to afford the product as a light yellow solid. 3-fluoro-4-[1-(4-fluorophenyl)-4-hydroxy-2-(2-methoxy-1,1-dimethyl-ethyl)indol-3-yl]benzoic acid (2.1 mg, 27%). ¹H NMR (400 MHz, Chloroform-d) δ 7.72 (dd, J = 9.3, 6.8 Hz, 1H), 7.19 (dd, J = 8.0, 1.8 Hz, 2H), 7.13 (dd, J = 11.7, 2.7 Hz, 1H), 7.05 (t, J = 8.2 Hz, 3H), 6.68 (td, J = 7.9, 2.4 Hz, 1H), 6.20 (dd, J = 7.8, 2.6 Hz, 1H), 5.96 (dd, J = 8.4, 2.6 Hz, 1H), 2.92 (d, J = 2.3 Hz, 3H), 2.83 (d, J = 2.8 Hz, 2H), 1.08 (s, 6H). LCMS *m*/*z* 452.35 [M+H]⁺

### Compound 139

### 2-fluoro-4-[1-(4-fluorophenyl)-4-hydroxy-2-(2-methoxy-1,1-dimethyl-ethyl)indol-3-yl]benzoic acid (139)

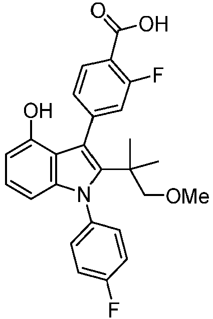

Compound **139** was prepared in three steps from **S20** and (3-fluoro-4-methoxycarbonyl-phenyl)boronic acid by Suzuki coupling, ester hydrolysis and then hydrogenation using the method described for the preparation of compound 1. 2-fluoro-4-[1-(4-fluorophenyl)-4-hydroxy-2-(2-methoxy-1,1-dimethyl-ethyl)indol-3-yl]benzoic acid (35 mg, 89%). ¹H NMR (400 MHz, Chloroform-d/CD3OD) δ 7.94 (t, J = 7.8 Hz, 1H), 7.42 - 7.30 (m, 4H), 7.14 (t, J = 8.5 Hz, 2H), 6.82 (t, J = 7.9 Hz, 1H), 6.34 (d, J = 7.6 Hz, 1H), 6.10 (d, J = 8.2 Hz, 1H), 3.02 (s, 3H), 2.92 (s, 2H), 1.05 - 0.95 (m, 6H). LCMS *m*/*z* 451.9 [M+H]⁺.

### Compound 140

### 4-[1-[3-(difluoromethyl)-4-fluoro-phenyl]-4-hydroxy-2-tetrahydropyran-4-yl-indol-3-yl]benzoic acid (140)

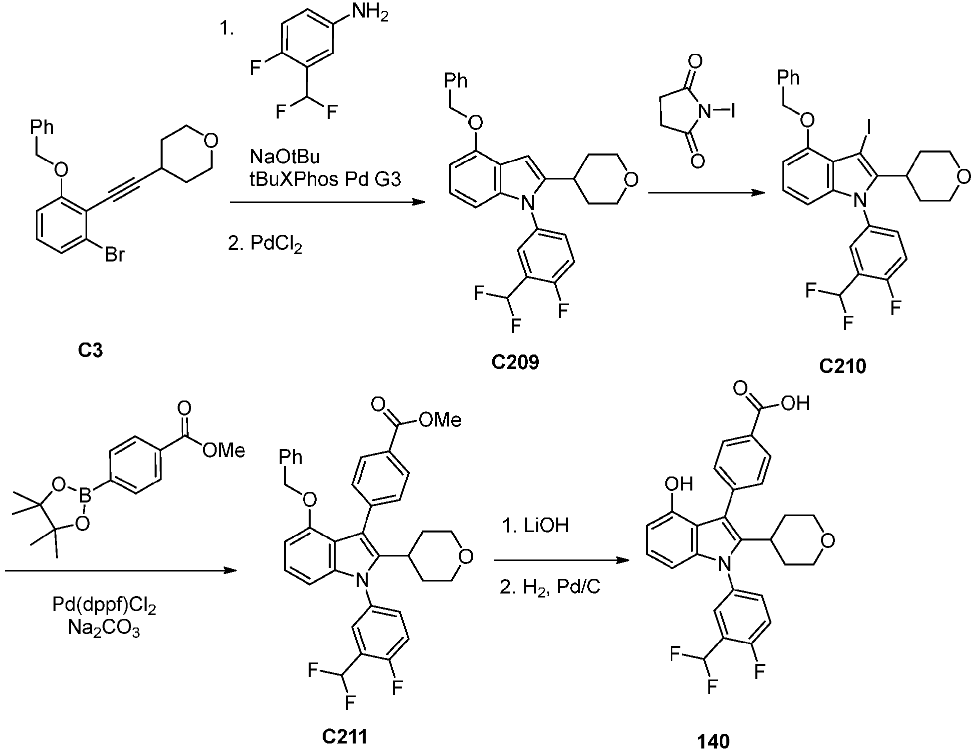

### Step 1-3: Synthesis of 4-benzyloxy-1-[3-(difluoromethyl)-4-fluoro-phenyl]-3-iodo-2-tetrahydropyran-4-yl-indole (C210)

**C210** was prepared in three steps from **C3** and 3-(difluoromethyl)-4-fluoro-aniline as described for the preparation of compound **S1.** Purification by silica gel chromatography (Gradient: 0-45% EtOAc in heptane), followed by combining, concentrating, and trituration with heptane, then filtering and drying gave the product as a white solid. 4-benzyloxy-1-[3-(difluoromethyl)-4-fluoro-phenyl]-3-iodo-2-tetrahydropyran-4-yl-indole (2 g, 90%). ¹H NMR (400 MHz, Chloroform-d) δ 7.66 (ddt, J = 7.5, 1.4, 0.7 Hz, 2H), 7.57 (dd, J = 6.1, 2.5 Hz, 1H), 7.45 - 7.41 (m, 3H), 7.40 - 7.32 (m, 2H), 7.07 - 6.99 (m, 2H), 6.69 - 6.63 (m, 1H), 6.44 (dd, J = 8.3, 0.7 Hz, 1H), 5.28 (s, 2H), 4.01 (dd, J = 11.5, 4.4 Hz, 2H), 3.38 (tdd, J = 11.8, 3.6, 2.1 Hz, 2H), 3.12 (tt, J = 12.5, 3.5 Hz, 1H), 2.33 - 2.19 (m, 2H), 1.59 (s, 2H). LCMS *m*/*z* 577.0 [M+H]⁺.

### Steps 4-6. Synthesis of 4-[1-[3-(difluoromethyl)-4-fluoro-phenyl]-4-hydroxy-2-tetrahydropyran-4-yl-indol-3-yl]benzoic acid (140)

Compound **140** was prepared in three steps from compound **C210** using the method described in the preparation of compound 1. Methyl 4-(4,4,5,5-tetramethyl-1,3,2-dioxaborolan-2-yl)benzoate was used in the Suzuki coupling step. The product mixture was filtered over Celite^{®}. The filtrate was concentrated to dryness and purified via silica gel chromatography (Gradient: 0-15% MeOH in dichloromethane). Purified fractions were combined, concentrated, triturated in EtOAc / heptane, filtered, and dried to afford the product as a white solid. 4-[1-[3-(difluoromethyl)-4-fluoro-phenyl]-4-hydroxy-2-tetrahydropyran-4-yl-indol-3-yl]benzoic acid (100 mg, 56%). ¹H NMR (400 MHz, DMSO-*d*₆) δ 12.88 (s, 1H), 9.19 (s, 1H), 7.99 - 7.93 (m, 2H), 7.82 - 7.74 (m, 2H), 7.66 (t, J = 9.3 Hz, 1H), 7.56 - 7.49 (m, 2H), 7.30 (t, J = 54.0 Hz, 1H), 6.90 - 6.84 (m, 1H), 6.40 (dd, J = 7.7, 0.8 Hz, 1H), 6.23 (dd, J = 8.2, 0.8 Hz, 1H), 3.73 - 3.62 (m, 2H), 3.07 - 2.96 (m, 2H), 2.79 (ddt, J = 12.0, 7.4, 3.7 Hz, 1H), 1.65 - 1.32 (m, 4H). LCMS *m*/*z* 482.0 [M+H]⁺.

### Compound 141

### 4-[1-(4-fluoro-3-methoxy-phenyl)-4-hydroxy-2-tetrahydropyran-4-yl-indol-3-yl]benzoic acid (141)

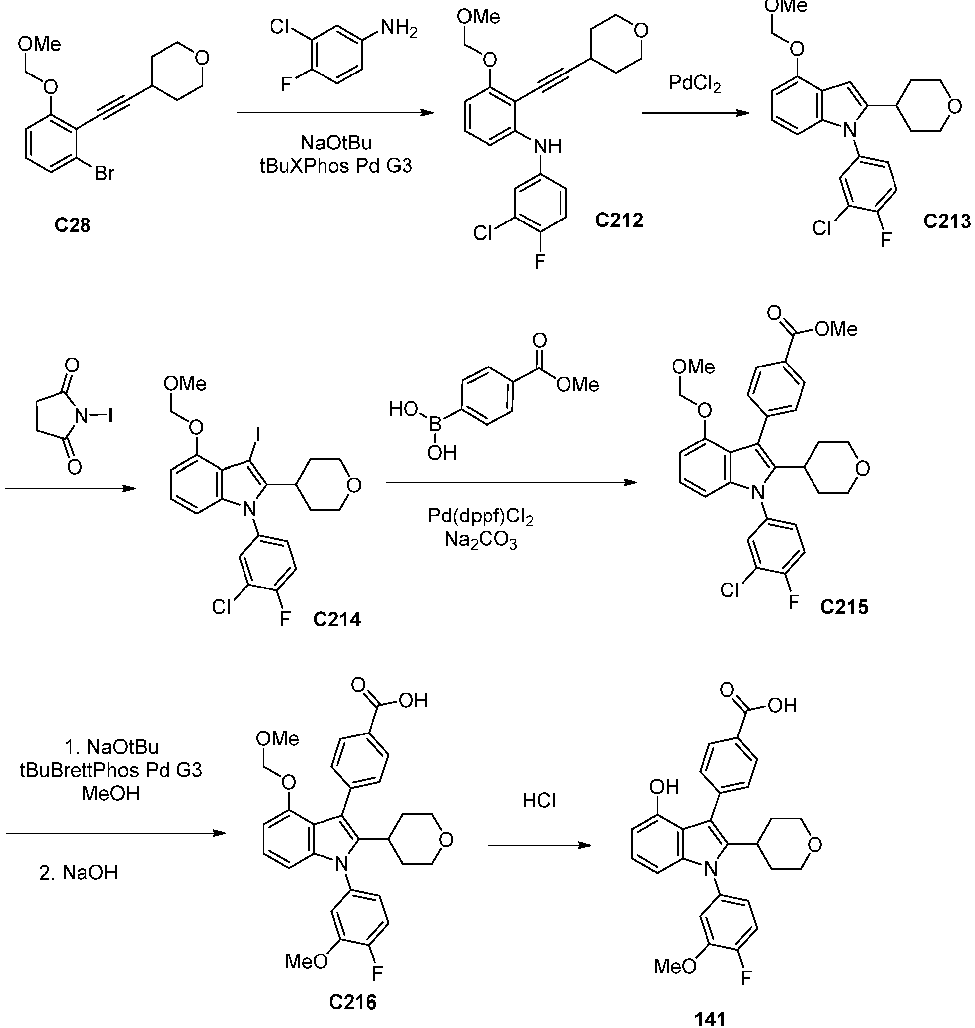

### Steps 1-4. Synthesis of methyl 4-[1-(3-chloro-4-fluoro-phenyl)-4-(methoxymethoxy)-2-tetrahydropyran-4-yl-indol-3-yl]benzoate (C215)

Compound **C215** was prepared in four steps from **C28** and (4-methoxycarbonylphenyl)boronic acid using the methods described in the preparation of **C80** in the preparation of compound **5.**

Methyl 4-[1-(3-chloro-4-fluoro-phenyl)-4-(methoxymethoxy)-2-tetrahydropyran-4-yl-indol-3-yl]benzoate (264 mg, 58%). ¹H NMR (400 MHz, Chloroform-d) δ 8.14 - 8.05 (m, 2H), 7.59 - 7.54 (m, 2H), 7.51 (dd, J = 6.5, 2.4 Hz, 1H), 7.41 - 7.30 (m, 2H), 7.07 (t, J = 8.1 Hz, 1H), 6.72 (dd, J = 7.9, 0.8 Hz, 1H), 6.57 (dd, J = 8.3, 0.7 Hz, 1H), 4.91 (s, 2H), 4.00 (s, 3H), 3.83 (dt, J = 11.9, 2.7 Hz, 2H), 3.23 (s, 3H), 3.17 (ddd, J = 11.8, 4.0, 2.2 Hz, 2H), 2.87 (tt, J = 12.3, 3.4 Hz, 1H), 1.79 - 1.69 (m, 2H), 1.63 - 1.51 (m, 2H). LCMS *m*/*z* 524.41 [M+H]⁺.

### Step 5. Synthesis of 4-[1-(4fluoro-3-methoxy-phenyl)-4-(methoxymethoxy)-2-tetrahydropyran-4-yl-indol-3-yl]benzoic acid (C216)

A mixture of methyl 4-[1-(3-chloro-4-fluoro-phenyl)-4-(methoxymethoxy)-2-tetrahydropyran-4-yl-indol-3-yl]benzoate **C215** (50 mg, 0.095 mmol), NaOtBu (13 mg, 0.14 mmol) and tBuBrettPhos Pd G3 (4.0 mg, 0.005 mmol) in a reaction vial was evacuated and flushed with nitrogen (x 3), then MeOH (20 µL, 0.5 mmol) and 1,4-dioxane (0.5 mL) were added, and the reaction vial sealed and heated to 50 °C for 20 hours. NaOH (200 µL of 1 M, 0.2 mmol) in water and more MeOH (0.2 mL) were added and the mixture was stirred for another 2 hours at 50 °C. The mixture was neutralized with 1 M HCl (300 µL), extracted with EtOAc (2 x 2 mL) and concentrated.

Purification by silica gel chromatography (Gradient: 0-10% MeOH in dichloromethane) provided the product as a white solid. 4-[1-(4-fluoro-3-methoxy-phenyl)-4-(methoxymethoxy)-2-tetrahydropyran-4-yl-indol-3-yl]benzoic acid (43.8 mg, 86%). ¹H NMR (400 MHz, Chloroform-d) δ 8.09 (d, J = 7.8 Hz, 2H), 7.54 (d, J = 7.7 Hz, 2H), 7.35 - 7.22 (m, 2H), 7.11 - 6.92 (m, 2H), 6.68 (d, J = 7.7 Hz, 1H), 6.56 (dd, J = 20.4, 8.3 Hz, 1H), 4.88 (s, 2H), 3.91 (d, J = 11.5 Hz, 3H), 3.80 (dt, J = 10.1, 4.6 Hz, 2H), 3.20 (s, 3H), 3.18 - 3.07 (m, 2H), 1.83 - 1.62 (m, 2H), 1.56 (t, J = 12.5 Hz, 2H). LCMS *m*/*z* 506.41 [M+H]⁺.

### Step 6. Synthesis of 4-[1-(4fluoro-3-methoxy-phenyl)-4-hydroxy-2-tetrahydropyran-4-yl-indol-3-yl]benzoic acid (141)

To a solution of 4-[1-(4-fluoro-3-methoxy-phenyl)-4-(methoxymethoxy)-2-tetrahydropyran-4-yl-indol-3-yl]benzoic acid **C216** (43 mg, 0.09 mmol) in 1,4-dioxane (1 mL) was added HCl (500 µL of 4 M, 2.0 mmol) in 1,4-dioxane and the reaction mixture allowed to stir for 2 hours. The solvent was removed under reduced pressure. Purification by reversed-phase chromatography (Column: C18. Gradient: 0-100% MeCN in water with 0.1% formic acid) and then SFC chromatography (Column: Daicel Chiralpak ^{®} AD-H, 10 x 250 mm; Mobile Phase: 20% Methanol (containing 5 mM Ammonia), 70% carbon dioxide) afforded the product as a white solid. 4-[1-(4-fluoro-3-methoxy-phenyl)-4-hydroxy-2-tetrahydropyran-4-yl-indol-3-yl]benzoic acid (6.3 mg, 16%) ¹H NMR (400 MHz, Chloroform-*d*) δ 8.06 (d, J = 7.7 Hz, 2H), 7.50 (d, J = 7.4 Hz, 2H), 7.18 (t, J = 9.6 Hz, 1H), 6.98 - 6.82 (m, 3H), 6.41 (t, J = 9.1 Hz, 2H), 3.81 (s, 3H), 3.71 (d, J = 11.4 Hz, 2H), 3.08 (t, J = 11.6 Hz, 2H), 2.79 (d, J = 12.2 Hz, 1H), 1.62 (ddt, J = 17.2, 11.9, 6.1 Hz, 2H), 1.47 (t, J = 13.6 Hz, 2H). LCMS *m*/*z* 462.39 [M+H]⁺.

### Compound 142

### 4-[4-hydroxy-1-(2-methyl-4-pyridyl)-2-tetrahydropyran-4-yl-indol-3-yl]benzoic acid (142)

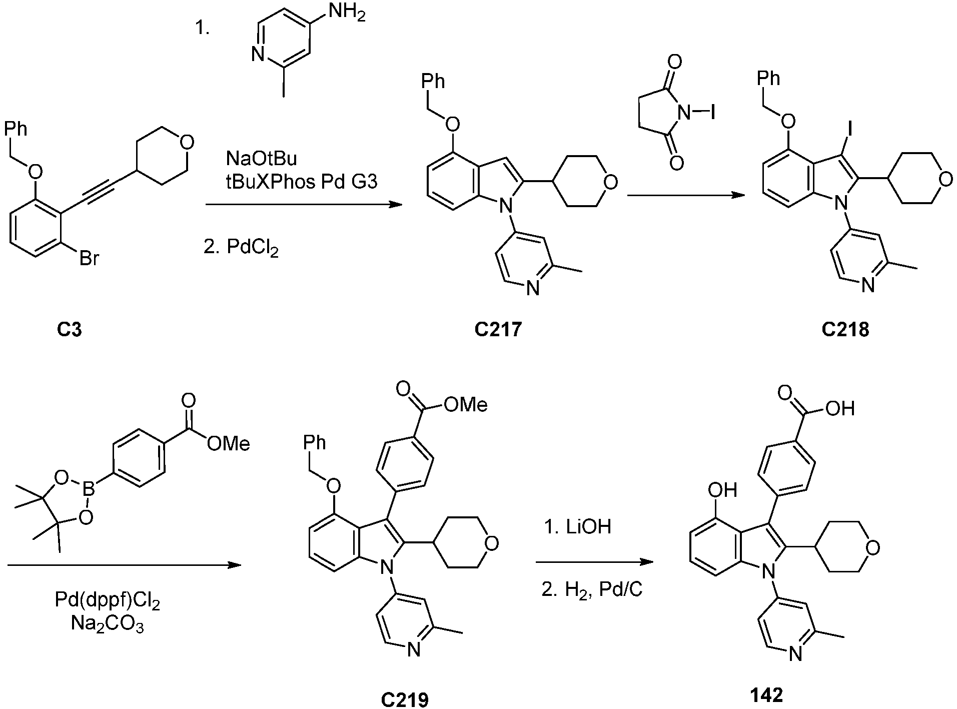

Compound **142** was prepared from **C3** in six steps using the methods described in the preparation of S1 and compound 1. 2-Methylpyridin-4-amine was used in the aryl amination step (step 1). methyl 4-(4,4,5,5-tetramethyl-1,3,2-dioxaborolan-2-yl)benzoate was used in the Suzuki coupling step (step 4). The final product was purified by reversed-phase chromatography (Column: C18. Gradient: 0-90% MeCN in water with 0.1% TFA) afforded the product. Pure fractions were combined, diluted with water, and extracted with EtOAc. The organic layer was dried over magnesium sulfate, filtered, and concentrated to give the product as a yellow solid.4-[4-hydroxy-1-(2-methyl-4-pyridyl)-2-tetrahydropyran-4-yl-indol-3-yl]benzoic acid (20 mg, 23%). ¹H NMR (400 MHz, DMSO-*d*₆) δ 9.22 (s, 1H), 8.78 (d, J = 5.5 Hz, 1H), 7.99 - 7.93 (m, 2H), 7.64 (d, J = 2.0 Hz, 1H), 7.56 - 7.49 (m, 3H), 6.91 (t, J = 8.0 Hz, 1H), 6.47 (d, J = 8.2 Hz, 1H), 6.42 (d, J = 7.6 Hz, 1H), 3.71 - 3.63 (m, 2H), 3.05 (td, J = 11.7, 2.2 Hz, 2H), 2.88 - 2.77 (m, 1H), 2.66 (s, 3H), 1.62 - 1.48 (m, 4H). LCMS *m*/*z* 429.0 [M+H]⁺

### Compound 143

### 4-[1-(3,4-difluorophenyl)-6-fluoro-4-phosphonooxy-2-tetrahydropyran-4-yl-indol-3-yl]benzoic acid (143)

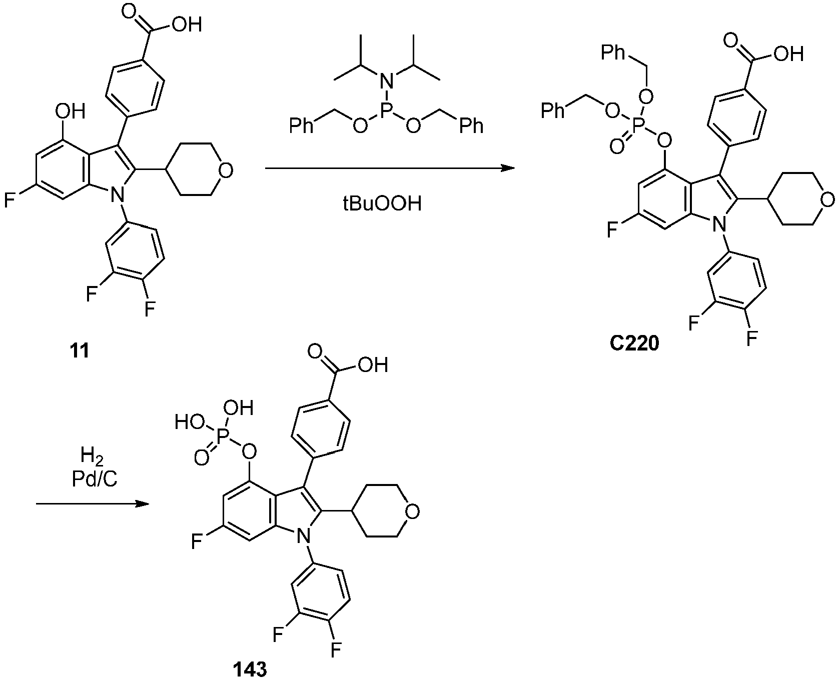

### Step 1. 4-[4-dibenzyloxyphosphoryloxy-1-(3,4-difluorophenyl)-6-fluoro-2-tetrahydropyran-4-yl-indol-3-yl]benzoic acid (C220)

Tetrazole (approximately 1.63 mL of 0.45 M, 0.73 mmol) was added to a mixture of compound **11** (132 mg, 0.28 mmol) and N-dibenzyloxyphosphanyl-N-isopropyl-propan-2-amine (227 µL, 0.68 mmol) in THF (4 mL) and the reaction was stirred at room temperature overnight. t-Butyl hydroperoxide (1.5 mL of 5.5 M, 8.3 mmol) was added and the mixture stirred at room temperature for 15 minutes. The reaction mixture was then partitioned between ethyl acetate and water. The organic layer was dried (MgSO₄), filtered and concentrated *in vacuo.*

The product mixture was purified by reversed-phase chromatography (Column: C18. Gradient: 0-100% MeCN in water with 0.1% formic acid). The desired fractions concentrated *in vacuo,* diluted with dichloromethane and water. The mixture was passed through a phase separator and the resulting organic phase concentrated *in vacuo* to afford the product containing an impurity (~10%). The product was carried onto next step of the synthesis without further purification. 4-[4-dibenzyloxyphosphoryloxy-1-(3,4-difluorophenyl)-6-fluoro-2-tetrahydropyran-4-yl-indol-3-yl]benzoic acid (56 mg, 26%). ¹H NMR (400 MHz, DMSO-*d*₆) δ 12.96 (s, 1H), 8.04 - 7.94 (m, 2H), 7.86 (ddd, J = 10.6, 7.3, 2.6 Hz, 1H), 7.74 (dt, J = 10.5, 8.9 Hz, 1H), 7.52 (d, J = 8.1 Hz, 2H), 7.49 - 7.41 (m, 1H), 7.35 - 7.27 (m, 6H), 7.24 - 7.08 (m, 4H), 6.92 (ddd, J = 10.7, 2.2, 1.0 Hz, 1H), 6.63 (dd, J = 9.2, 2.2 Hz, 1H), 4.75 - 4.53 (m, 4H), 3.65 (d, J = 11.4 Hz, 2H), 3.01 (td, J = 11.3, 5.9 Hz, 2H), 2.84 - 2.65 (m, 1H), 1.61 - 1.33 (m, 4H). LCMS *m*/*z* 660.58 [M+H]⁺.

### Step 2. Synthesis of 4-[1-(3,4-difluorophenyl)-6-fluoro-4-phosphonooxy-2-tetrahydropyran-4-yl-indol-3-yl]benzoic acid (143)

To a flask containing palladium on carbon (2 mg, 0.02 mmol) under nitrogen was added EtOH (1 mL), a solution of 4-[4-dibenzyloxyphosphoryloxy-1-(3,4-difluorophenyl)-6-fluoro-2-tetrahydropyran-4-yl-indol-3-yl]benzoic acid **C220** (52 mg, 0.07 mmol) in THF (1 mL) and ethyl acetate (1 mL). The mixture was purged with hydrogen and then stirred under atmosphere of hydrogen (balloon) for 3 hours. The mixture was filtered through a pad of Florosil^{®}, rinsing with 35% MeOH/EtOAc solution. The filtrate was concentrated *in vacuo.* Purification by reversed-phase chromatography (Column: C18. Gradient: 10-100% MeCN in water with 0.1% formic acid) afforded the product. 4-[1-(3,4-difluorophenyl)-6-fluoro-4-phosphonooxy-2-tetrahydropyran-4-yl-indol-3-yl]benzoic acid (10 mg, 26%). ¹H NMR (400 MHz, DMSO-*d*₆) δ 12.19 (s, 3H), 8.00 - 7.91 (m, 2H), 7.84 (ddd, J = 11.0, 7.2, 2.5 Hz, 1H), 7.72 (dt, J = 10.5, 8.9 Hz, 1H), 7.57 - 7.37 (m, 3H), 7.04 - 6.84 (m, 1H), 6.43 (dd, J = 9.0, 2.1 Hz, 1H), 3.67 (d, J = 11.3 Hz, 2H), 3.03 (ddd, J = 11.7, 9.5, 5.3 Hz, 2H), 2.80 (ddt, J = 11.9, 7.4, 3.7 Hz, 1H), 1.66 - 1.35 (m, 4H). LCMS *m*/*z* 547.97 [M+H]⁺.

### Compound 144 and Compound 145

### 4-[1-(4-fluoro-3-methoxy-phenyl)-4-hydroxy-2-(2-methoxy-1,1-dimethyl-ethyl)indol-3-yl]benzoic acid (144) and 4-[1-(4-fluoro-3-methoxy-phenyl)-4-hydroxy-2-(2-hydroxy-1,1-dimethyl-ethyl)indol-3-yl]benzoic acid (145)

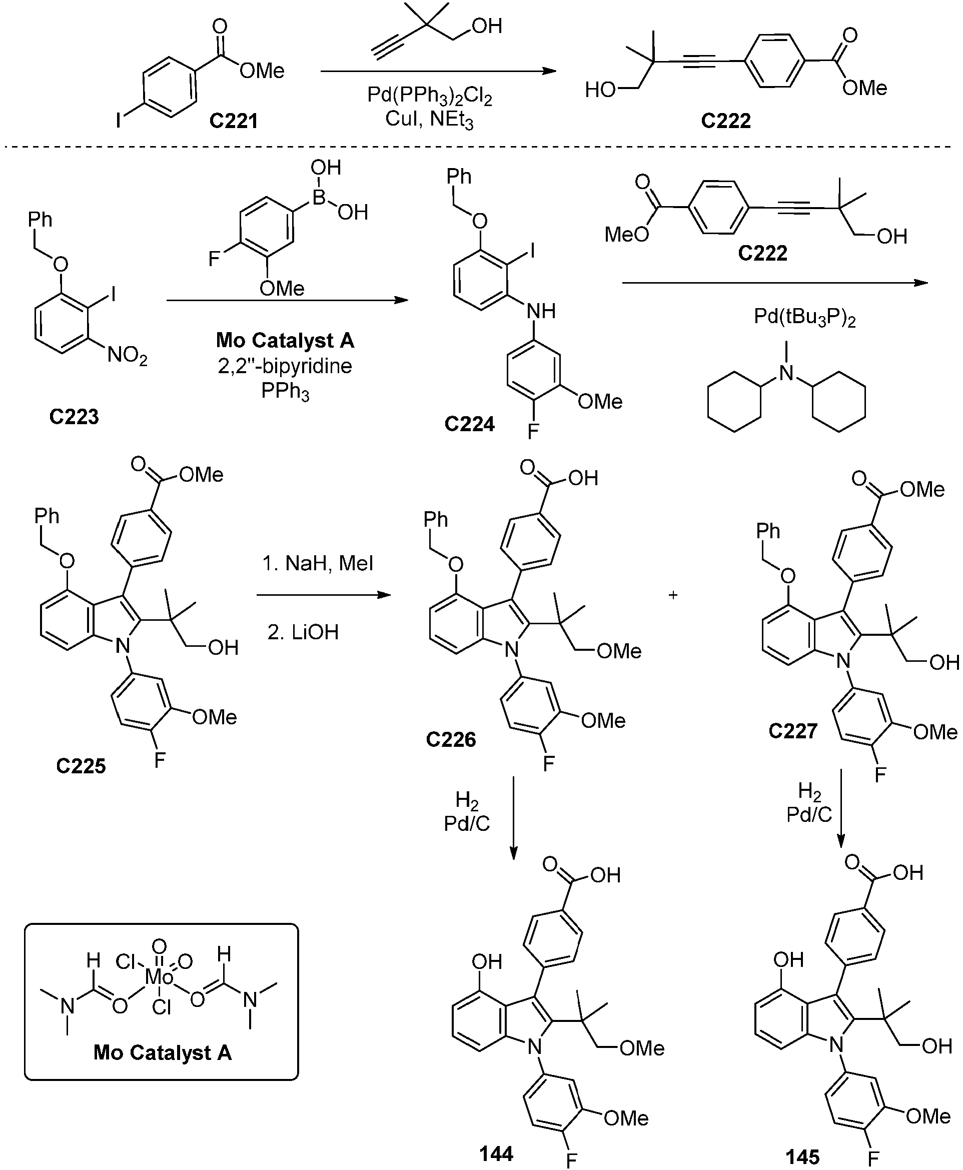

### Step 1. Synthesis of methyl 4-(4-hydroxy-3, 3-dimethyl-but-1-ynyl)benzoate (C222)

In a 250 mL round bottom flask under nitrogen were introduced 2,2-dimethylbut-3-yn-1-ol **C221** (2.8 g, 28.5 mmol), methyl 4-iodobenzoate (5 g, 19.1 mmol). The vial was purged with nitrogen (x 3) and triethylamine (40 mL) and 1,4-dioxane (40 mL) were added. Pd(PPh₃)₂Cl₂ (670 mg, 0.95 mmol) and CuI (360 mg, 1.9 mmol) were added. The reaction mixture was stirred at room temperature for 1 hour. The mixture was concentrated to dryness. EtOAc (50 mL) and Water (20 mL) were added. The aqueous layer was isolated and washed with EtOAc (20 mL). The combined organic layers were washed with sat. NH₄OH, brine, dried over MgSO₄, filtered and concentrated. Purification by silica gel chromatography (Gradient: 0-80% ethyl acetate in heptane) to give the product as a light yellow solid. Methyl 4-(4-hydroxy-3,3-dimethyl-but-1-ynyl)benzoate (4.4 g, 99%). ¹H NMR (400 MHz, Chloroform-d) δ 8.03 - 7.90 (m, 2H), 7.52 - 7.44 (m, 2H), 3.93 (s, 3H), 3.54 (s, 2H), 1.83 (s, 1H), 1.34 (s, 6H). LCMS *m*/*z* 233.09 [M+H]⁺.

### Step 2. Synthesis of 3-benzyloxy-N-(4-fluoro-3-methoxy-phenyl)-2-iodo-aniline (C224)

To a solution of 1-benzyloxy-2-iodo-3-nitro-benzene **C222** (400 mg, 1.05 mmol) 2,2'-bipyridine (18 mg, 0.12 mmol) and dichlorobis(N,N-dimethylformamide-κO)dioxomolybdenum (36 mg, 0.10 mmol) in anhydrous toluene (8 mL) was added (4-fluoro-3-methoxyphenyl)boronic acid (300 mg, 1.77 mmol) and PPh₃ (660 mg, 2.52 mmol). The resulting suspension was irradiated in the microwave cavity at 150 °C for 60 minutes, The crude product was loaded directly onto silica gel and purified by silica gel chromatography (Gradient: 0-50% EtOAc in heptane) to afford the product. 3-benzyloxy-N-(4-fluoro-3-methoxy-phenyl)-2-iodoaniline (276 mg, 58%). ¹H NMR (400 MHz, Chloroform-d) δ 7.43 (ddt, J = 7.4, 1.3, 0.7 Hz, 2H), 7.35 - 7.27 (m, 2H), 7.27 - 7.18 (m, 1H), 7.00 (t, J = 8.2 Hz, 1H), 6.93 (dd, J = 11.0, 8.6 Hz, 1H), 6.70 (dd, J = 7.5, 2.6 Hz, 1H), 6.63 - 6.53 (m, 2H), 6.30 (dd, J = 8.1, 1.2 Hz, 1H), 5.96 (s, 1H), 5.07 (s, 2H), 3.75 (s, 3H). LCMS *m*/*z* 449.94 [M+H]⁺.

### Step 3. Synthesis of methyl 4-[4-benzyloxy-1-(4-fluoro-3-methoxy-phenyl)-2-(2-hydroxy-1,1-dimethyl-ethyl)indol-3-yl]benzoate (C225)

A mixture of methyl 4-(4-hydroxy-3,3-dimethyl-but-1-ynyl)benzoate **C224** (220 mg, 0.95 mmol), 3-benzyloxy-N-(4-fluoro-3-methoxy-phenyl)-2-iodo-aniline (286 mg, 0.63 mmol) and N-cyclohexyl-N-methyl-cyclohexanamine (350 µL, 1.63 mmol) in a reaction vial was placed under vacuum and flushed with nitrogen.1,4-Dioxane (3 mL) was added and the mixture was evacuated and flushed with nitrogen. Pd(*t*Bu₃P)₂ (17 mg, 0.03 mmol) was added and the vessel was sealed. The mixture was heated to 60 °C for 12 hours. Solvent was removed and the mixture was purified by silica gel chromatography (Gradient: 60% EtOAc in heptane) to afford the product as a light yellow solid. Methyl 4-[4-benzyloxy-1-(4-fluoro-3-methoxy-phenyl)-2-(2-hydroxy-1,1-dimethyl-ethyl)indol-3-yl]benzoate (192 mg, 55%). ¹H NMR (400 MHz, Chloroform-d) δ 7.96 - 7.87 (m, 2H), 7.59 - 7.51 (m, 2H), 7.27 - 7.22 (m, 1H), 7.17 (tddd, J = 8.8, 6.2, 2.9, 1.6 Hz, 3H), 7.11 - 7.00 (m, 3H), 6.79 (dt, J = 7.2, 1.0 Hz, 2H), 6.55 (dd, J = 7.8, 0.7 Hz, 1H), 6.37 (dd, J = 8.3, 0.7 Hz, 1H), 4.84 (s, 2H), 3.99 (s, 3H), 3.91 (s, 3H), 3.42 - 3.26 (m, 2H), 1.08 (d, J = 0.9 Hz, 6H). LCMS *m*/*z* 554.2 [M+H]⁺.

### Step 4. Synthesis of methyl 4-[4-benzyloxy-1-(4-fluoro-3-methoxy-phenyl)-2-(2-methoxy-1,1-dimethyl-ethyl)indol-3-yl]benzoate (C226) and 4-[4-benzyloxy-1-(4-fluoro-3-methoxy-phenyl)-2-(2-hydroxy-1,1-dimethyl-ethyl)indol-3-yl]benzoic acid (C227)

Part A. methyl 4-[4-benzyloxy-1-(4-fluoro-3-methoxy-phenyl)-2-(2-hydroxy-1,1-dimethyl-ethyl)indol-3-yl]benzoate (105 mg, 0.19 mmol) and MeI (35 µL, 0.56 mmol) in THF (1.5 mL) was added NaH (15 mg of 60% w/w, 0.38 mmol) at room temperature and the mixture was stirred for several hours. Solvent was removed *in vacuo* and the crude product was advanced to part B.

Part B. THF (1.5 mL) and MeOH (600 mL) were added to the product of part A, followed by LiOH (approximately 569 µL of 1 M, 0.57 mmol) solution. The solution was heated at 50 °C for 2 hours. The mixture was quenched with HCl (2 N, 1 mL). The mixture was extracted with EtOAc and 4-Me THF solution (2 x 2 mL). Combined organic layers were dried and purified by reverse phase chromatography (C18 column. Gradient: 0-100% MeCN in water, 0.2% formic acid modifier) to afford two products. **C226** is the methylated product and **C227** is the hydroxyl product.

**C226:** 4-[4-benzyloxy-1-(4-fluoro-3-methoxy-phenyl)-2-(2-methoxy-1,1-dimethylethyl)indol-3-yl]benzoic acid (20 mg, 19%). ¹H NMR (400 MHz, Chloroform-d) δ 8.01 - 7.95 (m, 2H), 7.62 - 7.55 (m, 2H), 7.25 - 7.15 (m, 4H), 7.10 (dd, J = 7.7, 2.4 Hz, 1H), 7.05 (ddd, J = 8.4, 4.0, 2.4 Hz, 1H), 6.99 (d, J = 8.1 Hz, 1H), 6.84 - 6.79 (m, 2H), 6.53 (d, J = 7.8 Hz, 1H), 6.34 (dd, J = 8.3, 0.6 Hz, 1H), 4.83 (s, 2H), 3.89 (s, 3H), 3.09 (s, 3H), 3.01 (s, 2H), 1.08 (d, J = 3.3 Hz, 6H). LCMS *m*/*z* 554.11 [M+H]⁺.

**C227:** 4-[4-benzyloxy-1-(4-fluoro-3-methoxy-phenyl)-2-(2-hydroxy-1,1-dimethylethyl)indol-3-yl]benzoic acid (10 mg, 10%). ¹H NMR (400 MHz, Chloroform-*d*) δ 7.96 - 7.87 (m, 2H), 7.56 - 7.45 (m, 2H), 7.17 - 7.07 (m, 4H), 7.03 - 6.88 (m, 3H), 6.81 - 6.68 (m, 2H), 6.47 (dd, J = 7.9, 0.7 Hz, 1H), 6.28 (dd, J = 8.3, 0.6 Hz, 1H), 4.75 (s, 2H), 3.82 (s, 3H), 3.33 - 3.18 (m, 2H), 1.01 (d, J = 1.1 Hz, 6H). LCMS *m*/*z* 540.12 [M+H]⁺.

### Step 4. Synthesis of 4-[1-(4fluoro-3-methoxy-phenyl)-4-hydroxy-2-(2-methoxy-1,1-dimethylethyl)indol-3-yl]benzoic acid (144)

To a mixture of 4-[4-benzyloxy-1-(4-fluoro-3-methoxy-phenyl)-2-(2-methoxy-1,1-dimethyl-ethyl)indol-3-yl]benzoic acid **C226** (20 mg, 0.04 mmol) in THF (500 µL) and ethanol (500 µL) was added Pd on carbon (4 mg, 0.004 mmol). The subjected to hydrogenation under a balloon pressure of H₂ for 2 hours. The reaction was filtered and dried to provide the product. 4-[1-(4-fluoro-3-methoxy-phenyl)-4-hydroxy-2-(2-methoxy-1,1-dimethyl-ethyl)indol-3-yl]benzoic acid (15.3 mg, 86%). ¹H NMR (400 MHz, Chloroform-d) δ 8.26 - 8.18 (m, 2H), 7.80 - 7.70 (m, 2H), 7.24 - 7.18 (m, 1H), 7.10 (dd, J = 7.6, 2.4 Hz, 1H), 7.04 (ddd, J = 8.4, 3.9, 2.4 Hz, 1H), 6.94 (t, J = 8.0 Hz, 1H), 6.46 (dd, J = 7.7, 0.9 Hz, 1H), 6.27 (dd, J = 8.2, 0.8 Hz, 1H), 3.90 (s, 3H), 3.11 (s, 3H), 3.03 (s, 2H), 1.09 (d, J = 3.6 Hz, 6H). LCMS *m*/*z* 464.12 [M+H]⁺.

### Step 4. 4-[1-(4-fluoro-3-methoxy-phenyl)-4-hydroxy-2-(2-hydroxy-1,1-dimethyl-ethyl)indol-3-yl]benzoic acid (145)

Compound **145** was prepared from **C227** (10 mg, 0.019 mmol) by hydrogenation as described for the synthesis of compound **144.** 4-[1-(4-fluoro-3-methoxy-phenyl)-4-hydroxy-2-(2-hydroxy-1,1-dimethyl-ethyl)indol-3-yl]benzoic acid (7.5 mg, 82%). ¹H NMR (400 MHz, Chloroform-d) δ 8.26 - 8.17 (m, 2H), 7.75 - 7.70 (m, 2H), 7.25 - 7.19 (m, 1H), 7.11 - 7.02 (m, 2H), 6.99 - 6.91 (m, 1H), 6.47 (dd, J = 7.8, 0.8 Hz, 1H), 6.28 (dd, J = 8.3, 0.8 Hz, 1H), 3.89 (s, 3H), 3.43 - 3.33 (m, 2H), 1.08 (d, J = 2.3 Hz, 6H). LCMS *m*/*z* 450.03 [M+H]⁺.

### Compound 146

### 4-[1-(3,4-difluorophenyl)-5-fluoro-4-hydroxy-2-(2-methoxy-1,1-dimethyl-ethyl)indol-3-yl]benzoic acid (146)

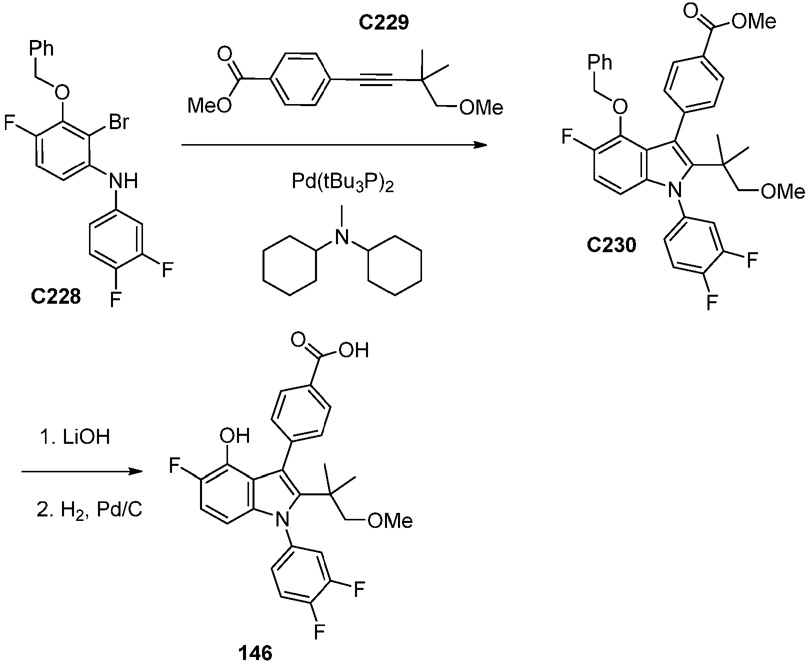

### Synthesis of methyl 4-(4-methoxy-3, 3-dimethyl-but-1-ynyl)benzoate (C229)

Compound **C229** was prepared from methyl 4-bromobenzoate (1.23 g, 5.7 mmol) and 4-methoxy-3,3-dimethyl-but-1-yne (970 mg, 8.65 mmol) by Sonagashira coupling as described for the synthesis of **C222.** Silica gel chromatography (Gradient: 0-10% EtOAc in heptane) afforded the product as a clear, slightly yellow liquid. Methyl 4-(4-methoxy-3,3-dimethyl-but-1-ynyl)benzoate (1.3285 g, 89%). ¹H NMR (400 MHz, Chloroform-d) δ 7.97 (d, J = 7.6 Hz, 2H), 7.48 (d, J = 7.6 Hz, 2H), 3.93 (s, 3H), 3.47 (s, 3H), 3.37 (s, 2H), 1.34 (s, 6H). LCMS *m*/*z 247.13* [M+H]⁺.

### Step 1. Synthesis of methyl 4-[4-benzyloxy-1-(3, 4-difluorophenyl)-5-fluoro-2-(2-methoxy-1,1-dimethyl-ethyl)indol-3-yl]benzoate (C230)

Compound **C230** was prepared in a single step from **C228** using a one-pot alkyne amine coupling, following by cyclization, as described for the preparation of compound **C225.** N-cyclohexyl-N-methyl-cyclohexanamine and Pd(*t*Bu₃P)₂ were used as the catalyst system for this transformation. Silica gel chromatography (Gradient: 10% to 90% EtOAc in hexane) afforded the product. Methyl 4-[4-benzyloxy-1-(3,4-difluorophenyl)-5-fluoro-2-(2-methoxy-1,1-dimethyl-ethyl)indol-3-yl]benzoate (145 mg, 44%) LCMS *m*/*z* 574.15 [M+H]⁺. Steps 2 & 3. *4-[1-(3,4-difluorophenyl)-5-fluoro-4-hydroxy-2-(2-methoxy-1,1-dimethyl-ethyl)indol-3-yl]benzoic acid **(146)***

Compound **146** was prepared from **C230** in two steps (ester hydrolysis and hydrogenation) as described in the synthesis of compound **1.** 4-[1-(3,4-difluorophenyl)-5-fluoro-4-hydroxy-2-(2-methoxy-1,1-dimethyl-ethyl)indol-3-yl]benzoic acid (38 mg, 73%). ¹H NMR (400 MHz, Chloroform-d) δ 8.18 - 8.06 (m, 2H), 7.67 - 7.54 (m, 2H), 7.32 (ddd, J = 10.3, 7.8, 2.3 Hz, 2H), 7.24 (tdd, J = 6.3, 3.5, 1.8 Hz, 1H), 6.83 (dd, J = 10.9, 8.9 Hz, 1H), 6.07 (dd, J = 8.9, 3.5 Hz, 1H), 3.09 (s, 3H), 2.99 (s, 2H), 1.07 (d, J = 2.2 Hz, 6H). LCMS *m*/*z* 470.13 [M+H]⁺.

### Compound 147

### 4-[2-(2-cyano-1,1-dimethyl-ethyl)-1-(3,4-difluorophenyl)-5-fluoro-4-hydroxy-indol-3-yl]benzoic acid (147)

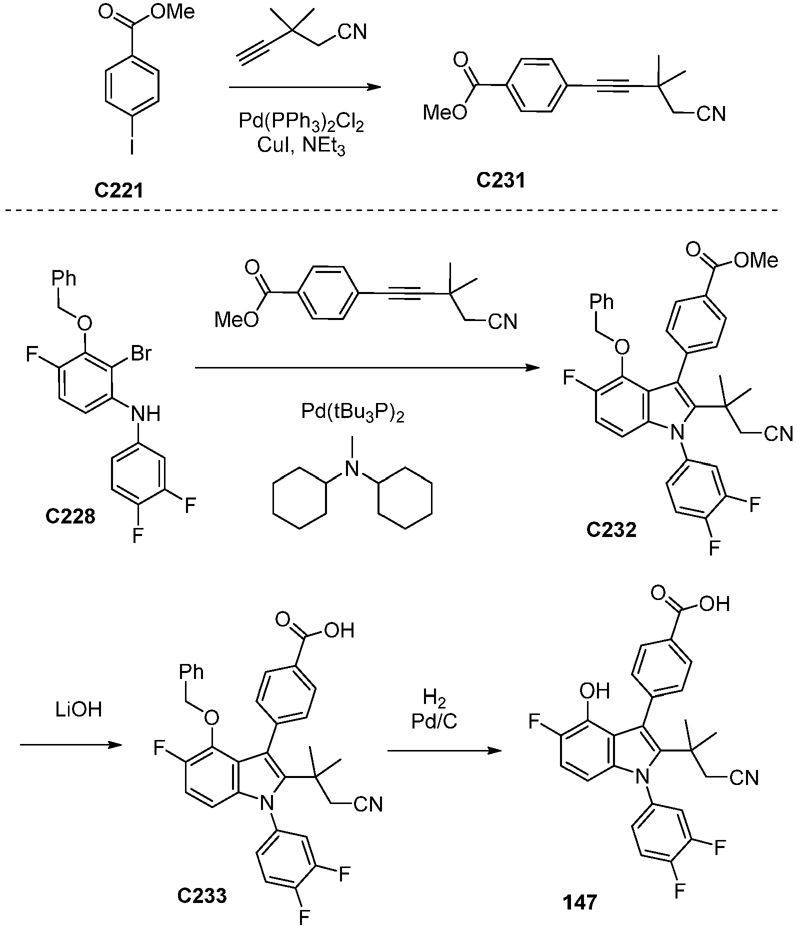

### Step 1. Synthesis of methyl 4-(4-cyano-3,3-dimethyl-but-1-ynyl)benzoate (C231)

Compound **231** was prepared from methyl 4-iodobenzoate (1.8 g, 6.87 mmol), 3,3-dimethylpent-4-ynenitrile (810 mg, 7.56 mmol) by Sonagashira coupling as described for the synthesis of **C222.** Silica gel chromatography (4 g column, 10-40% EtOAc in hexane) afforded the product. Methyl 4-(4-cyano-3,3-dimethyl-but-1-ynyl)benzoate (1.5 g, 88%). LCMS m/z 242.13 [M+H]⁺.

### Step 4. 4-[2-(2-cyano-1,1-dimethyl-ethyl)-1-(3,4-difluoropheny)-5-fluoro-4-hydroxy-indol-3-yl]benzoic acid (147)

Compound **147** was prepared in three steps from **C228** and **C231** using the method described for the preparation of compound **146.** Silica gel chromatography (4 g column, 10-90% EtOAc in hexane) afforded the product. 4-[2-(2-cyano-1,1-dimethyl-ethyl)-1-(3,4-difluorophenyl)-5-fluoro-4-hydroxy-indol-3-yl]benzoic acid (45 mg, 44%). ¹H NMR (400 MHz, DMSO-*d*₆) δ 12.95 (s, 1H), 9.14 (d, J = 2.0 Hz, 1H), 8.03 - 7.92 (m, 2H), 7.82 (ddd, J = 11.0, 7.3, 2.6 Hz, 1H), 7.71 (dt, J = 10.5, 8.9 Hz, 1H), 7.57 (tt, J = 8.2, 1.9 Hz, 2H), 7.50 - 7.36 (m, 1H), 6.91 (dd, J = 11.1, 8.9 Hz, 1H), 6.04 (dd, J = 8.9, 3.4 Hz, 1H), 2.53 (s, 2H), 1.15 (d, J = 7.1 Hz, 6H). LCMS *m*/*z* 465.09 [M+H]⁺.

### Compound 148

### 4-[2-(2-cyano-1,1-dimethyl-ethy)-5-fluoro-1-(4-fluoro-3-methyl-pheny)-4-hydroxy-indol-3-yl]benzoic acid (148)

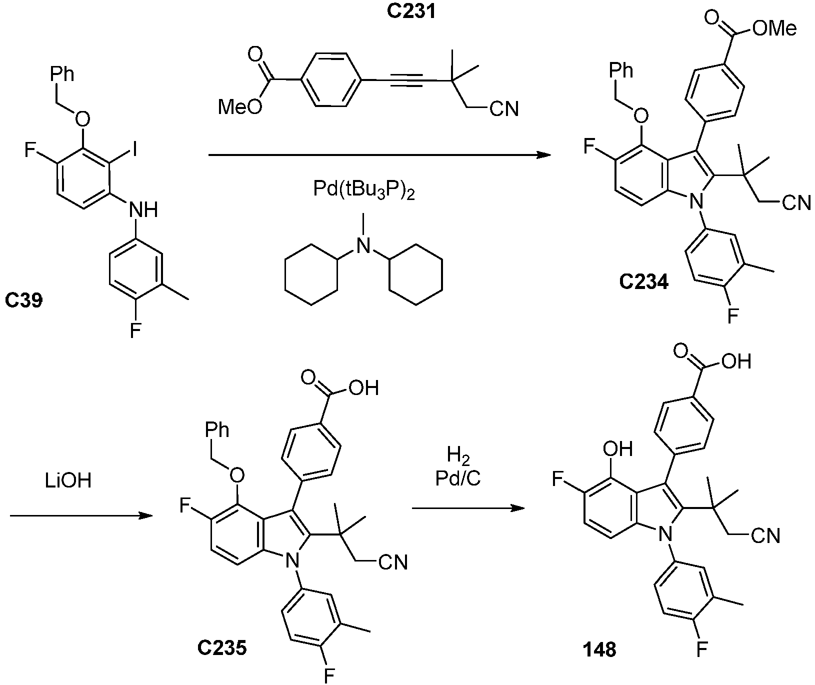

### Synthesis of 4-[2-(2-cyano-1,1-dimethyl-ethyl)-5-fluoro-1-(4-fluoro-3-methyl-phenyl)-4-hydroxy-indol-3-yl]benzoic acid (148)

Compound **148** was prepared in three steps from **C39** and **C231** using the method described for the preparation of compound **147.** Silica gel chromatography (4 g column, 10-90% EtOAc in hexane) afforded the product. 4-[2-(2-cyano-1,1-dimethyl-ethyl)-5-fluoro-1-(4-fluoro-3-methyl-phenyl)-4-hydroxy-indol-3-yl]benzoic acid (42 mg, 75%). ¹H NMR (400 MHz, DMSO-*d*₆) δ 12.92 (s, 1H), 9.09 (d, J = 1.9 Hz, 1H), 7.98 - 7.90 (m, 2H), 7.57 (dq, J = 8.6, 1.8 Hz, 2H), 7.48 (dd, J = 6.9, 2.3 Hz, 1H), 7.44 - 7.35 (m, 2H), 6.89 (dd, J = 11.1, 8.9 Hz, 1H), 5.98 (dd, J = 8.9, 3.5 Hz, 1H), 2.33 (d, J = 1.8 Hz, 3H), 1.14 (d, J = 3.3 Hz, 6H). LCMS *m*/*z* 461.14 [M+H]⁺.

### Compound 149

### 4-[5-fluoro-1-(4-fluoro-3-methyl-phenyl)-4-hydroxy-2-(2-methoxy-1,1-dimethyl-ethyl)indol-3-yl]benzoic acid (149)

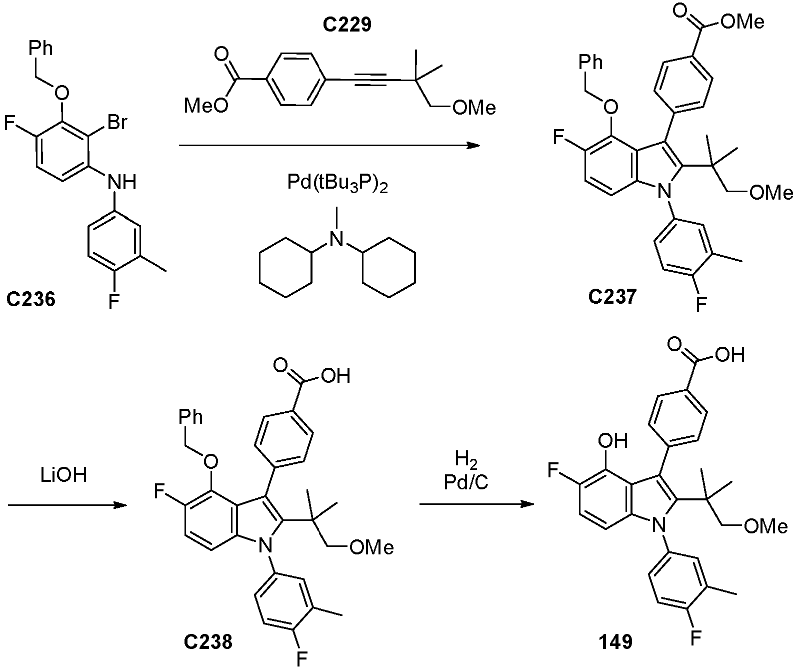

### Synthesis of 4-[5-fluoro-1-(4-fluoro-3-methyl-phenyl)-4-hydroxy-2-(2-methoxy-1,1-dimethylethyl)indol-3-yl]benzoic acid (149)

Compound **149** was prepared in three steps from **C236** and **C229** using the method described for the preparation of compound **146**.Silica gel chromatography (4 g column, 10-90% EtOAc in hexane) afforded the product.4-[5-fluoro-1-(4-fluoro-3-methyl-phenyl)-4-hydroxy-2-(2-methoxy-1,1-dimethyl-ethyl)indol-3-yl]benzoic acid (58 mg, 91%). ¹H NMR (400 MHz, Chloroform-d) δ 8.20 - 7.98 (m, 2H), 7.65 - 7.57 (m, 2H), 7.24 - 7.14 (m, 2H), 7.08 (t, J = 8.7 Hz, 1H), 6.74 (dd, J = 10.8, 8.9 Hz, 1H), 6.02 (dd, J = 8.9, 3.6 Hz, 1H), 3.01 (s, 3H), 2.91 (s, 2H), 2.29 (d, J = 2.0 Hz, 3H), 1.01 (d, J = 2.4 Hz, 6H). LCMS *m*/*z* 466.17 [M+H]⁺.

### Compound 150

### 4-[5-fluoro-1-(4-fluorophenyl)-4-hydroxy-2-(2-hydroxy-1,1-dimethyl-ethyl)indol-3-yl]benzoic acid (150)

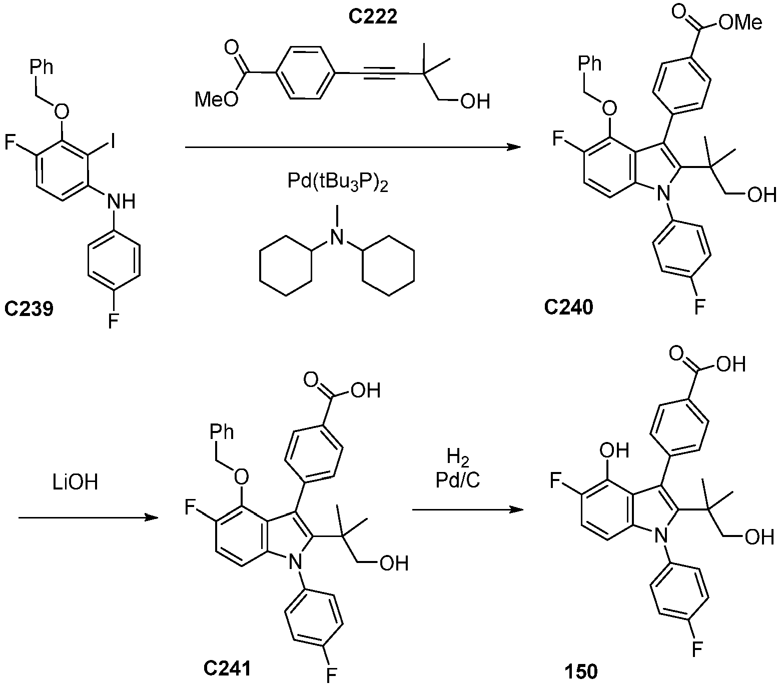

### Synthesis of 4-[5-fluoro-1-(4-fluorophenyl)-4-hydroxy-2-(2-hydroxy-1,1-dimethyl-ethyl)indol-3-yl]benzoic acid (150)

Compound 150 was prepared from **C222** and **C239** in three steps using the method described for the preparation of **146.** Silica gel chromatography (4 g column, 10-90% EtOAc in hexane) afforded the product. 4-[5-fluoro-1-(4-fluorophenyl)-4-hydroxy-2-(2-hydroxy-1,1-dimethyl-ethyl)indol-3-yl]benzoic acid (30 mg, 79%). ¹H NMR (300 MHz, DMSO-*d*₆) δ 8.91 (s, 1H), 7.89 (d, J = 7.8 Hz, 2H), 7.53 (d, J = 8.1 Hz, 4H), 7.42 (t, J = 8.3 Hz, 2H), 6.81 (t, J = 10.0 Hz, 1H), 5.98 - 5.82 (m, 1H), 5.76 (s, 1H), 4.67 (s, 1H), 3.21 (d, J = 5.3 Hz, 2H), 0.90 (s, 6H). LCMS *m*/*z* 438.15 [M+H]⁺.

### Compound 151

### 4-[2-(2-cyano-1,1-dimethyl-ethyl)-5-fluoro-1-(4-fluorophenyl)-4-hydroxy-indol-3-yl]benzoic acid (151)

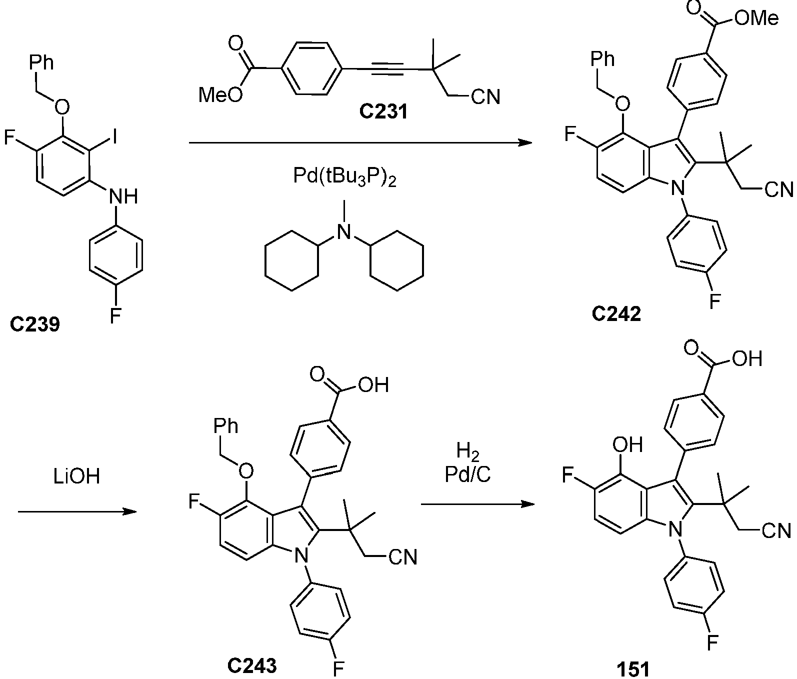

### Synthesis of 4-[2-(2-cyano-1,1-dimethyl-ethyl)-5-fluoro-1-(4-fluorophenyl)-4-hydroxy-indol-3-yl]benzoic acid

Compound **151** was prepared in three steps from **C239** and **C231** using the method described for the preparation of compound **147.** Silica gel chromatography (4 g column, 10-90% EtOAc in hexane) afforded the product. 4-[2-(2-cyano-1,1-dimethyl-ethyl)-5-fluoro-1-(4-fluorophenyl)-4-hydroxy-indol-3-yl]benzoic acid (85 mg, 88%). ¹H NMR (400 MHz, DMSO-*d*₆) δ 12.89 (s, 1H), 9.06 (d, J = 1.9 Hz, 1H), 8.00 - 7.86 (m, 2H), 7.65 - 7.53 (m, 3H), 7.48 (t, J = 8.7 Hz, 2H), 6.89 (dd, J = 11.2, 8.9 Hz, 1H), 5.96 (dd, J = 8.9, 3.4 Hz, 1H), 3.57 (s, 2H), 1.14 (s, 6H). LCMS *m*/*z* 447.14 [M+H]⁺.

### Compound 152

### 4-[2-(2-cyano-1,1-dimethyl-ethyl)-1-(4-fluorophenyl)-4-hydroxy-indol-3-yl]benzoic acid (152)

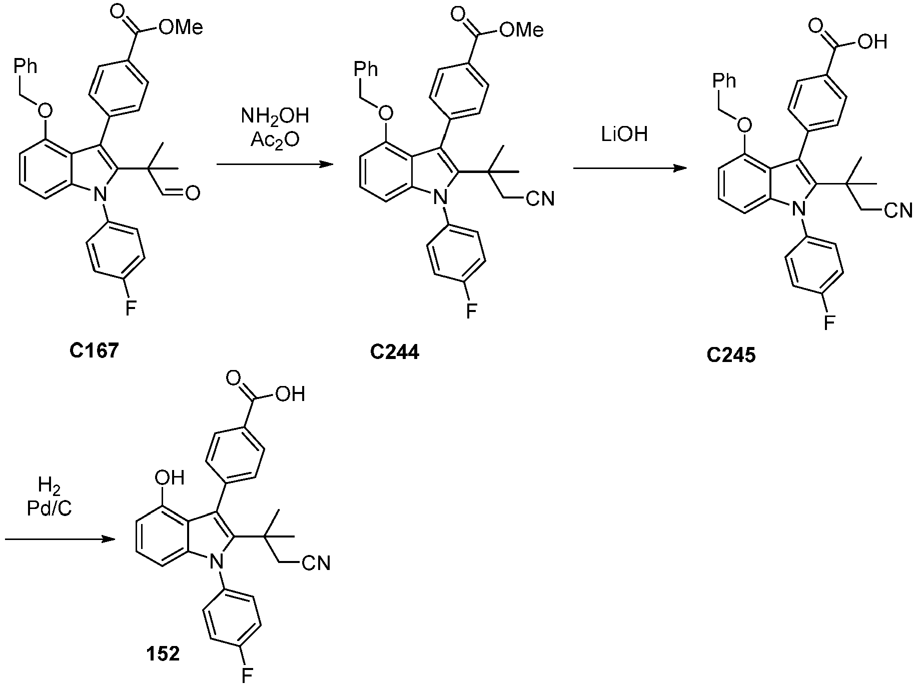

### Step 1. Synthesis of methyl 4-[4-benzyloxy-2-(2-cyano-1,1-dimethyl-ethyl)-1-(4-fluorophenyl)indol-3-yl]benzoate (C244)

A mixture of methyl 4-[4-benzyloxy-2-(1,1-dimethyl-3-oxo-propyl)-1-(4-fluorophenyl)indol-3-yl]benzoate **C167** (500 mg, 0.93 mmol) and hydroxylamine Hydrochloride salt (130 mg, 1.87 mmol) in pyridine (5 mL) was stirred at 110 °C in a sealed tube for 1 hour. Ac₂O (710 µL, 7.53 mmol) was added. The reaction mixture was stirred at 110 °C for 7 hours, then diluted with water and extracted with EtOAc. The organic layer was concentrated to dryness and purified by silica gel chromatography (Gradient: 0-50% EtOAc in heptane) to afford the product as a white solid.

Methyl 4-[4-benzyloxy-2-(2-cyano-1,1-dimethyl-ethyl)-1-(4-fluorophenyl)indol-3-yl]benzoate (280 mg, 56%). ¹H NMR (400 MHz, Chloroform-d) δ 7.94 - 7.86 (m, 2H), 7.60 - 7.55 (m, 2H), 7.49 (ddd, J = 9.7, 4.9, 2.5 Hz, 2H), 7.32 - 7.29 (m, 2H), 7.21 - 7.12 (m, 3H), 7.08 - 7.01 (m, 1H), 6.82 - 6.79 (m, 2H), 6.56 (dd, J = 7.8, 0.7 Hz, 1H), 6.30 (dd, J = 8.4, 0.7 Hz, 1H), 4.83 (d, J = 4.7 Hz, 2H), 3.99 (s, 3H), 2.37 (s, 2H), 1.25 (s, 6H). LCMS *m*/*z* 533.0 [M+H]⁺.

### Step 2 & 3: 4-[2-(2-cyano-1,1-dimethyl-ethyl)-1-(4-fluorophenyl)-4-hydroxy-indol-3-yl]benzoic acid (152)

Compound **152** was prepared in two steps from **C244** in two steps (ester hydrolysis and hydrogenation) using the method described in the synthesis of compound **1.** 4-[2-(2-cyano-1,1-dimethyl-ethyl)-1-(4-fluorophenyl)-4-hydroxy-indol-3-yl]benzoic acid (67 mg, 77%). ¹H NMR (400 MHz, DMSO-*d*₆) δ 12.87 (s, 1H), 8.95 (s, 1H), 7.95 - 7.90 (m, 2H), 7.59 - 7.53 (m, 4H), 7.51 - 7.44 (m, 2H), 6.85 - 6.78 (m, 1H), 6.30 (dd, J = 7.7, 0.8 Hz, 1H), 6.00 (dd, J = 8.2, 0.8 Hz, 1H), 3.30 (s, 2H), 1.14 (s, 6H). LCMS *m*/*z* 429.0 [M+H]⁺.

### Compound 153

### 4-[2-(2-cyano-2-methyl-propyl)-1-(4-fluorophenyl)-4-hydroxy-indol-3-yl]benzoic acid (153)

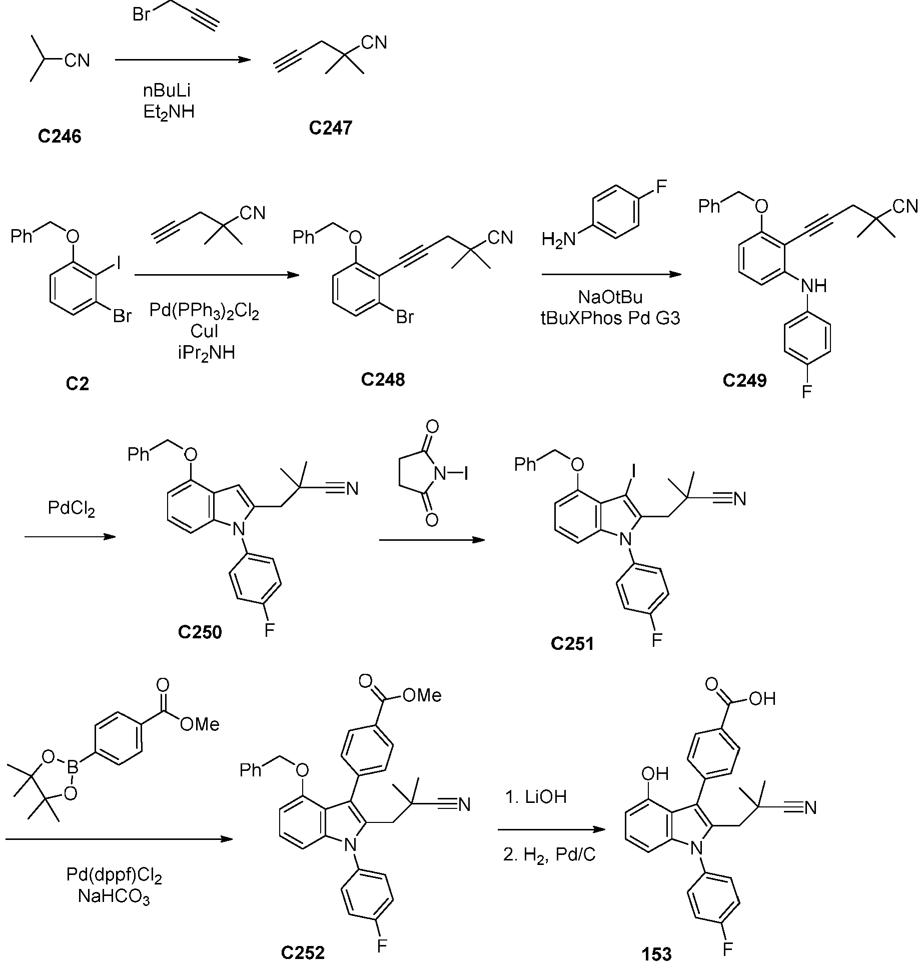

### Synthesis of 2, 2-dimethylpent-4-ynenitrile (C247)

nBuLi (6.3 mL of 2.5 M, 15.75 mmol) was added to a solution of N-ethylethanamine **C246** (1.4 mL, 13.5 mmol) in THF (40 mL) at 0 °C. The mixture was stirred for 1 hour, then 2-methylpropanenitrile (1 g, 14.5 mmol) was added and stirred for another 1 hour at 0 °C. The solution was cooled down to -78 °C and a solution of 3-bromoprop-1-yne (1.4 mL, 15.7 mmol) in THF (8 mL) was added slowly. After completion, the reaction was cooled to 0 °C and quenched with ice water. The mixture was extracted with ether and the ether layer was washed with brine, dried over Na₂SO₄ and concentrated to give 2,2-dimethylpent-4-ynenitrile (1.03 g, 66%). ¹H NMR (400 MHz, Chloroform-d) δ 2.51 (d, J = 2.7 Hz, 2H), 2.20 (t, J = 2.7 Hz, 1H), 1.47 (s, 6H).

### Synthesis of 4-[2-(2-cyano-2-methyl-propyl)-1-(4-fluorophenyl)-4-hydroxy-indol-3-yl]benzoic acid (153)

Compound **153** was prepared in 7 steps form compound **C2** and alkyne **C247.** Intermediate **C251** was prepared from **C2** and **C247** according to the method described for the preparation of **S1. C251** was converted to compound **153** by Suzuki coupling with methyl 4-(4,4,5,5-tetramethyl-1,3,2-dioxaborolan-2-yl)benzoate, then ester hydrolysis and debenzylation by hydrogenation as described in the synthesis of compound **1.** The resulting product was triturated with 9:1 heptane / EtOAc, filtered, and dried to white solid. 4-[2-(2-cyano-2-methylpropyl)-1-(4-fluorophenyl)-4-hydroxy-indol-3-yl]benzoic acid (27 mg, 62%). ¹H NMR (400 MHz, DMSO-*d*₆) δ 12.87 (s, 1H), 9.45 (s, 1H), 7.98 - 7.93 (m, 2H), 7.65 - 7.60 (m, 2H), 7.60 - 7.53 (m, 2H), 7.51 - 7.43 (m, 2H), 6.97 - 6.91 (m, 1H), 6.50 (ddd, J = 13.1, 8.0, 0.8 Hz, 2H), 3.16 (s, 2H), 0.81 (s, 6H). LCMS *m*/*z* 429.0 [M+H]⁺.

### Compound 154

### 4-[2-(1-acetyl-4-piperidyl)-1-(4-fluorophenyl)-4-hydroxy-indol-3-yl]benzoic acid (154)

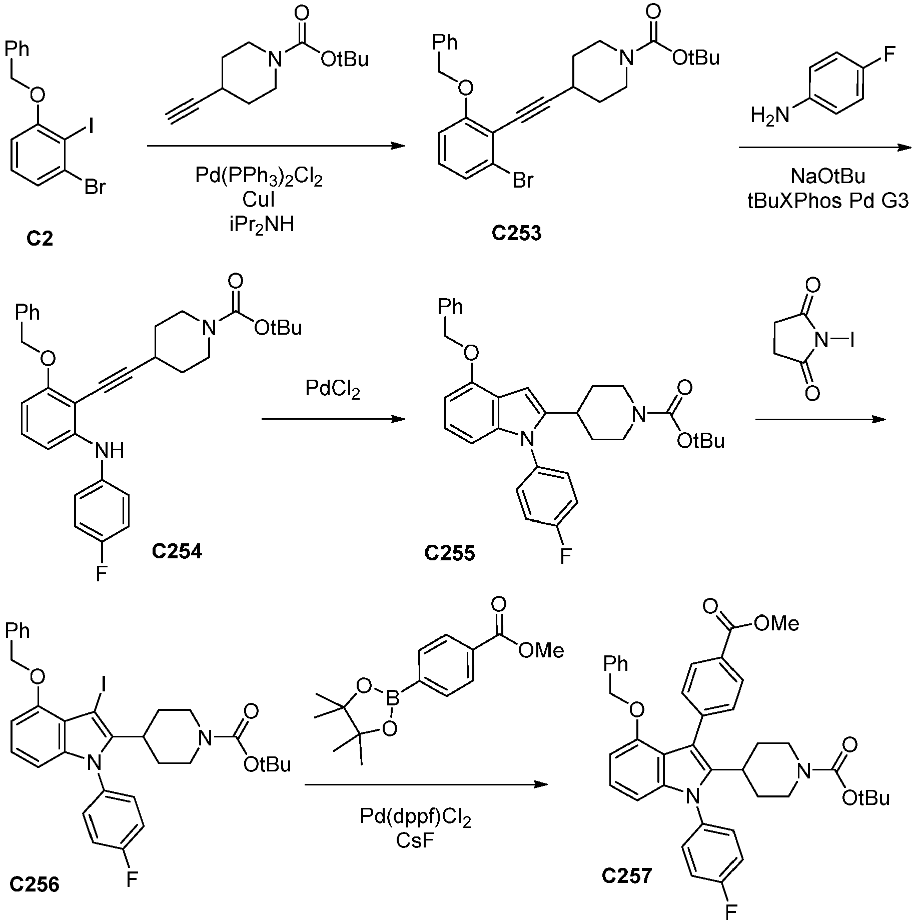

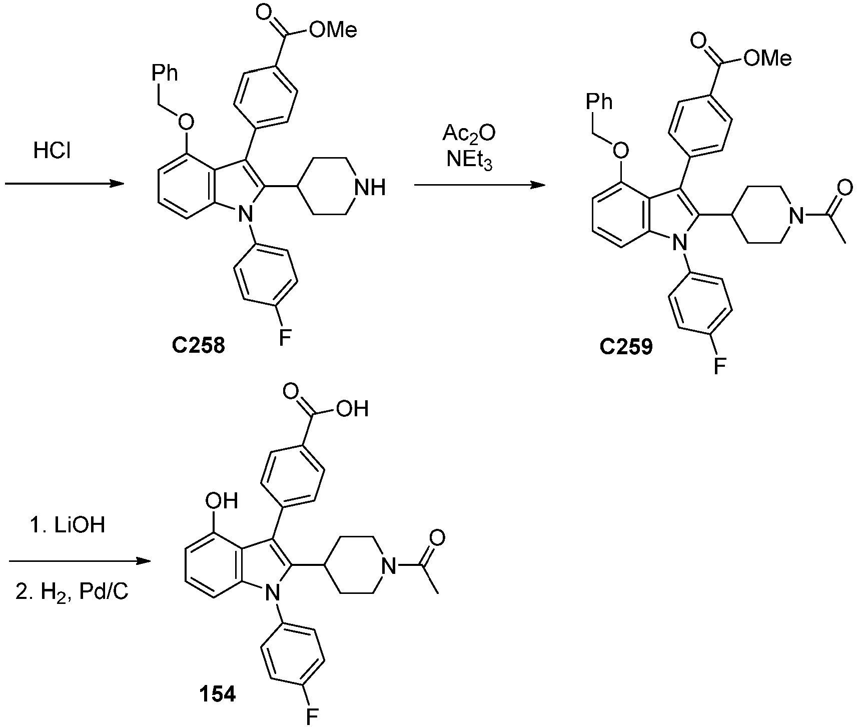

### Steps 1-4: tert-butyl 4-[4-benzyloxy-1-(4-fluorophenyl)-3-iodo-indol-2-yl]piperidine-1-carboxylate (C256)

Compound **C256** was prepared in four steps from **C2** and tert-butyl 4-ethynylpiperidine-1-carboxylate using the methods described for the preparation of compound **C255.** tert-butyl 4-[4-benzyloxy-1-(4-fluorophenyl)-3-iodo-indol-2-yl]piperidine-1-carboxylate (1.0 g, 60%). ¹H NMR (400 MHz, DMSO-*d*₆) δ 7.67 - 7.55 (m, 2H), 7.51 - 7.37 (m, 6H), 7.32 (dd, J = 8.3, 6.4 Hz, 1H), 6.98 (dt, J = 10.1, 8.0 Hz, 1H), 6.73 (t, J = 7.4 Hz, 1H), 6.38 (dd, J = 18.9, 8.2 Hz, 1H), 5.25 (d, J = 2.7 Hz, 2H), 3.94 (s, 4H), 2.97 (d, J = 12.5 Hz, 1H), 1.94 (dd, J = 27.4, 13.1 Hz, 2H), 1.62 (t, J = 15.0 Hz, 2H), 1.36 (s, 9H). LCMS *m*/*z* 626.0 [M+H]⁺.

### Step 5. tert-butyl 4-[4-benzyloxy-1-(4-fluorophenyl)-3-(4-methoxycarbonylphenyl)indol-2-yl]piperidine-1-carboxylate (C257)

A mixture of tert-butyl 4-[4-benzyloxy-1-(4-fluorophenyl)-3-iodo-indol-2-yl]piperidine-1-carboxylate **C256** (1 g, 1.6 mmol), methyl 4-(4,4,5,5-tetramethyl-1,3,2-dioxaborolan-2-yl)benzoate (1.26 g, 4.8 mmol), CsF (970 mg, 6.4 mmol), and PdCl₂(dppf) (130 mg, 0.16 mmol) in DME (8 mL) was stirred overnight at 85 °C. The mixture was cooled to room temperature, diluted with water, and extracted with EtOAc. The organic layer was concentrated to dryness and purified via silica gel chromatography (Gradient: 0-45% EtOAc in heptane). tert-butyl 4-[4-benzyloxy-1-(4-fluorophenyl)-3-(4-methoxycarbonylphenyl)indol-2-yl]piperidine-1-carboxylate (480 mg, 47%). ¹H NMR (400 MHz, Chloroform-d) δ 7.54 - 7.49 (m, 2H), 7.42 - 7.37 (m, 2H), 7.28 (d, J = 2.4 Hz, 2H), 7.21 - 7.12 (m, 3H), 7.10 - 7.04 (m, 1H), 6.91 - 6.86 (m, 1H), 6.84 - 6.79 (m, 2H), 6.61 (dd, J = 7.8, 0.7 Hz, 1H), 6.54 (dd, J = 8.3, 0.7 Hz, 1H), 4.94 (s, 2H), 3.95 (d, J = 35.8 Hz, 6H), 2.73 (tt, J = 12.4, 3.3 Hz, 1H), 2.43 (s, 2H), 1.67 - 1.42 (m, 2H), 1.38 (s, 10H). LCMS *m*/*z* 635.0 [M+H]⁺.

### Step 6. Synthesis of methyl 4-[4-benzyloxy-1-(4fluorophenyl)-2-(4piperidyl)indol-3-yl]benzoate (C258)

To a suspension of tert-butyl 4-[4-benzyloxy-1-(4-fluorophenyl)-3-(4-methoxycarbonylphenyl)indol-2-yl]piperidine-1-carboxylate **C257** (480 mg, 0.76 mmol) in MeOH (5 mL) was added HCl in 1,4-dioxane (15 mL of 4 M, 60 mmol). The reaction mixture was stirred at room temperature for 2 then concentrated to dryness. The residue was triturated in a 9:1 mixture of Et₂O and MeOH, then filtered and dried to afford the product as a white solid. Methyl 4-[4-benzyloxy-1-(4-fluorophenyl)-2-(4-piperidyl)indol-3-yl]benzoate (Hydrochloride salt) (320 mg, 74%). ¹H NMR (400 MHz, DMSO-*d*₆) δ 7.95 - 7.91 (m, 2H), 7.58 - 7.51 (m, 4H), 7.51 - 7.43 (m, 2H), 7.20 - 7.15 (m, 1H), 7.12 - 7.06 (m, 2H), 7.03 (t, J = 8.0 Hz, 1H), 6.79 - 6.75 (m, 2H), 6.69 - 6.64 (m, 1H), 6.44 (dd, J = 8.3, 0.6 Hz, 1H), 4.92 (s, 2H), 3.93 (s, 3H), 3.08 (d, J = 12.5 Hz, 2H), 2.85 - 2.75 (m, 1H), 2.61 (t, J = 12.6 Hz, 2H), 1.82 (d, J = 13.6 Hz, 2H), 1.62 (qd, J = 13.1, 3.8 Hz, 2H). LCMS *m*/*z* 535.0 [M+H]⁺.

### Step 7. Synthesis of methyl 4-[2-(1-acetyl-4-piperidyl)-4-benzyloxy-]-(4fluorophenyl)indol-3-yl]benzoate (C259)

To a suspension of methyl 4-[4-benzyloxy-1-(4-fluorophenyl)-2-(4-piperidyl)indol-3-yl]benzoate (Hydrochloride salt) **C258** (75 mg, 0.13 mmol) in dichloromethane (600 µL) was added Et₃N (37 µL, 0.27 mmol) followed by Ac₂O (15 µL, 0.16 mmol). The reaction mixture was stirred at room temperature for 30 minutes. The mixture was washed with water, dried over magnesium sulfate, filtered, and concentrated to afford the product as a colorless film. Methyl 4-[2-(1-acetyl-4-piperidyl)-4-benzyloxy-1-(4-fluorophenyl)indol-3-yl]benzoate (68 mg, 90%). ¹H NMR (400 MHz, Chloroform-*d*) δ 8.02 - 7.95 (m, 2H), 7.53 - 7.48 (m, 2H), 7.43 - 7.37 (m, 2H), 7.32 - 7.26 (m, 2H), 7.20 - 7.12 (m, 3H), 7.10 - 7.05 (m, 1H), 6.83 - 6.78 (m, 2H), 6.61 (dd, J = 7.9, 0.7 Hz, 1H), 6.54 (dd, J = 8.3, 0.7 Hz, 1H), 4.94 (s, 2H), 4.52 (d, J = 13.2 Hz, 1H), 4.01 (s, 3H), 3.70 - 3.60 (m, 1H), 3.14 (qd, J = 7.3, 4.8 Hz, 0H), 2.85 - 2.69 (m, 2H), 1.95 (s, 3H), 1.70 (s, 2H), 1.54 - 1.45 (m, 2H). LCMS *m*/*z* 577.0 [M+H]⁺

### Step 8 & 9: Synthesis of 4-[2-(1-acetyl-4-piperidyl)-]-(4-fluoropheny)-4-hydroxy-indol-3-yl]benzoic acid (154)

Compound **154** was prepared in two steps form **C259** by ester hydrolysis and hydrogenation as using the methods described in the preparation of compound **1.** The final product was triturated with heptane, filtered, and dried to give the product as a white solid. 4-[2-(1-acetyl-4-piperidyl)-1-(4-fluorophenyl)-4-hydroxy-indol-3-yl]benzoic acid (40 mg, 72%). ¹H NMR (400 MHz, DMSO-*d*₆) δ 12.83 (s, 1H), 9.16 (s, 1H), 7.95 - 7.90 (m, 2H), 7.57 - 7.42 (m, 6H), 6.89 - 6.79 (m, 1H), 6.38 (dd, J = 7.7, 0.8 Hz, 1H), 6.21 (dd, J = 8.2, 0.8 Hz, 1H), 4.22 (d, J = 13.0 Hz, 1H), 3.64 (d, J = 13.5 Hz, 1H), 2.85 - 2.65 (m, 2H), 2.18 (t, J = 12.7 Hz, 1H), 1.79 (s, 3H), 1.67 (t, J = 14.0 Hz, 2H), 1.44 - 1.32 (m, 1H), 1.26 (dd, J = 12.9, 7.1 Hz, 1H). LCMS *m*/*z* 473.0 [M+H]⁺.

### Compound 155

### 4-[1-(4-fluorophenyl)-4-hydroxy-2-(1-methoxycarbonyl-4-piperidyl)indol-3-yl]benzoic acid (155)

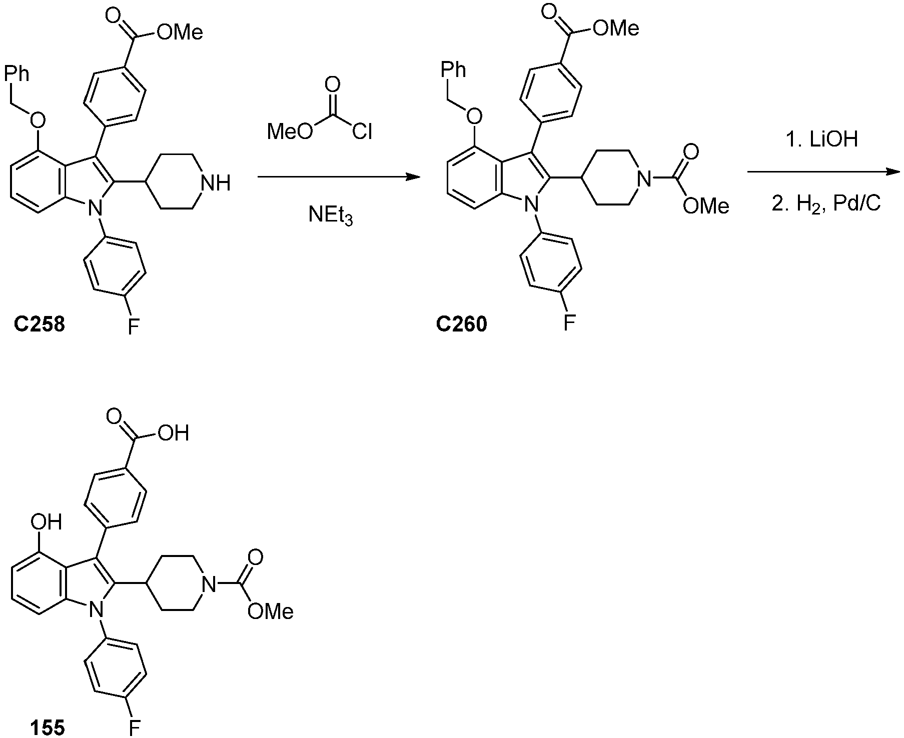

### Step 1. methyl 4-[4-benzyloxy-1-(4-fluorophenyl)-3-(4-methoxycarbonylphenyl)indol-2-yl]piperidine-1-carboxylate (C260)

To a suspension of 4-[2-(1-acetyl-4-piperidyl)-1-(4-fluorophenyl)-4-hydroxy-indol-3-yl]benzoic acid (Hydrochloride salt) **C258** (75 mg, 0.13 mmol) in dichloromethane (1 mL) was added Et₃N (40 µL, 0.28 mmol) followed by methyl carbonochloridate (15 µL, 0.19 mmol). The reaction mixture was stirred at room temperature for 30 minutes, then washed with 1 M HCl, dried over magnesium sulfate, filtered and concentrated to afford the product. Methyl 4-[4-benzyloxy-1-(4-fluorophenyl)-3-(4-methoxycarbonylphenyl)indol-2-yl]piperidine-1-carboxylate (57 mg, 73%). ¹H NMR (400 MHz, Chloroform-*d*) δ 8.02 - 7.97 (m, 2H), 7.55 - 7.49 (m, 2H), 7.40 (ddt, J = 8.2, 5.4, 2.7 Hz, 2H), 7.33 - 7.26 (m, 2H), 7.21 - 7.13 (m, 3H), 7.11 - 7.05 (m, 1H), 6.84 - 6.79 (m, 2H), 6.61 (dd, J = 7.8, 0.7 Hz, 1H), 6.55 (dd, J = 8.2, 0.7 Hz, 1H), 4.94 (s, 2H), 4.01 (s, 3H), 3.62 (s, 3H), 3.14 (qd, J = 7.3, 4.8 Hz, 1H), 2.73 (tt, J = 12.3, 3.2 Hz, 1H), 2.48 (s, 2H), 1.65 (s, 2H), 1.48 (d, J = 15.8 Hz, 2H). LCMS *m*/*z* 593.0 [M+H]⁺.

### Steps 2 & 3: Synthesis of 4-[1-(4-fluorophenyl)-4-hydroxy-2-(1-methoxycarbonyl-4-piperidyl)indol-3-yl]benzoic acid (155)

Compound **155** was prepared in two steps from **C259** as described in the preparation of compound **1.** 4-[1-(4-fluorophenyl)-4-hydroxy-2-(1-methoxycarbonyl-4-piperidyl)indol-3-yl]benzoic acid (39 mg, 88%). ¹H NMR (400 MHz, DMSO-*d*₆) δ 12.80 (s, 1H), 9.16 (s, 1H), 7.96 - 7.90 (m, 2H), 7.57 - 7.41 (m, 6H), 6.84 (t, J = 7.9 Hz, 1H), 6.38 (dd, J = 7.7, 0.8 Hz, 1H), 6.21 (dd, J = 8.2, 0.8 Hz, 1H), 3.78 (s, 2H), 3.46 (s, 3H), 2.80 - 2.71 (m, 1H), 1.65 (d, J = 12.8 Hz, 2H), 1.40 - 1.21 (m, 4H). LCMS *m*/*z* 489.0 [M+H]⁺.

### Compound 156

### 4-[5-fluoro-1-(4-fluorophenyl)-4-hydroxy-2-[2-hydroxy-1-(hydroxymethyl)-1-methyl-ethyl]indol-3-yl]benzoic acid (156)

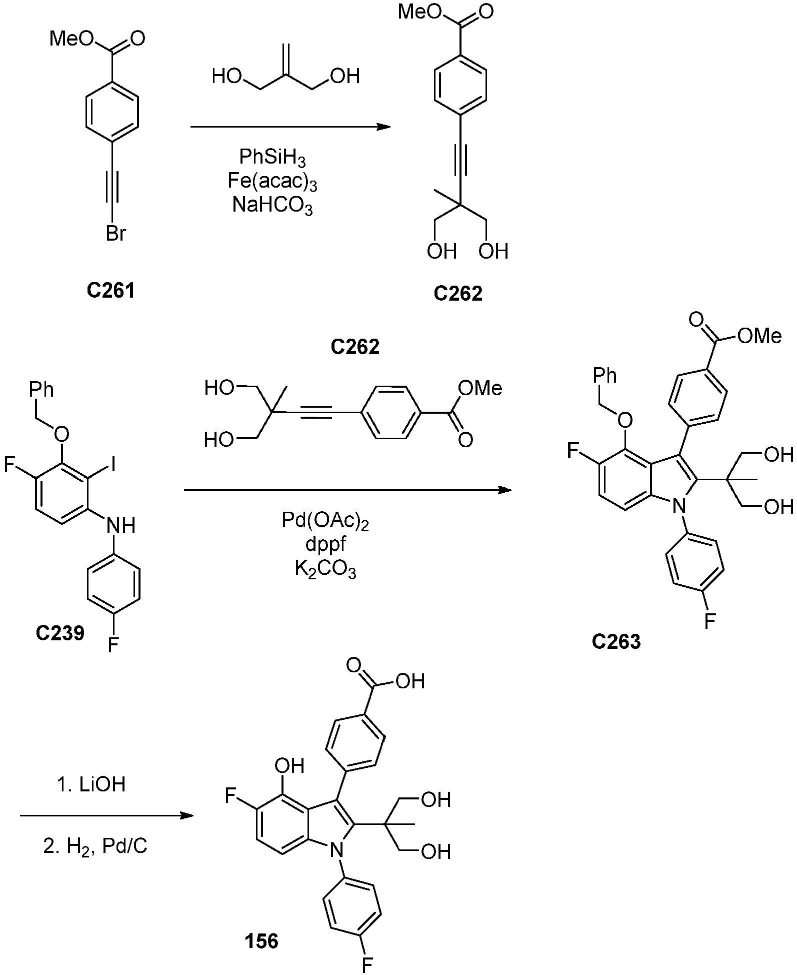

### Step 1. Synthesis of methyl 4-[4-hydroxy-3-(hydroxymethyl)-3-methyl-but-1-ynyl]benzoate (C262)

To a flask was added methyl 4-(2-bromoethynyl)benzoate **C261** (2 g, 8.09 mmol), tris[(Z)-1-methyl-3-oxo-but-1-enoxy]iron (1.5 g, 4.25 mmol) and NaHCO₃ (1.4 g, 16.7 mmol), evacuated and purged with Argon (x 3). A solution of 2-Methylenepropane-1,3-diol (2.2 g, 24.97 mmol) in EtOH (30 mL) was added via syringe. The solution was warmed to 60 °C, then phenylsilane (2 mL, 16.2 mmol) in EtOH (5 mL) was added over 1 hour, then kept for 12 hours. Additional PhSiH₃ (2 mL) was added and the mixture was allowed to stir for an additional 2 hours. The solution was filtered to remove the red solid, and the filtrate was concentrated under vacuum. Purification by silica gel chromatography (Gradient: 0-50% ethyl acetate/heptane) afforded the product as a white solid. methyl 4-[4-hydroxy-3-(hydroxymethyl)-3-methyl-but-1-ynyl]benzoate (0.7 g, 35%). ¹H NMR (400 MHz, Methanol-*d*₄) δ 7.98 - 7.89 (m, 2H), 7.55 - 7.45 (m, 2H), 3.88 (s, 3H), 3.67 - 3.57 (m, 4H), 1.25 (s, 3H). LCMS *m*/*z* 249.06 [M+H]⁺.

### Step 2-4. 4-[5-fluoro-1-(4-fluorophenyl)-4-hydroxy-2-[2-hydroxy-1-(hydroxymethyl)-1-methylethyl]indol-3-yl]benzoic acid (156)

Compound **156** was prepared from **C239** and **C262** using the method described for the preparation of compound **146.** 4-[5-fluoro-1-(4-fluorophenyl)-4-hydroxy-2-[2-hydroxy-1-(hydroxymethyl)-1-methyl-ethyl]indol-3-yl]benzoic acid (10 mg, 100%). ¹H NMR (400 MHz, Chloroform-d) δ 7.90 (dd, J = 8.3, 1.6 Hz, 2H), 7.55 - 7.46 (m, 2H), 7.42 - 7.31 (m, 2H), 7.15 - 7.04 (m, 2H), 6.65 (td, J = 9.6, 8.9, 1.3 Hz, 1H), 5.93 - 5.83 (m, 1H), 3.29 (q, J = 11.2 Hz, 4H), 0.91 (s, 3H). LCMS *m*/*z* 453.43 [M+H]⁺.

### Compound 157

### 4-[5-fluoro-1-(4-fluoropheny)-4-hydroxy-2-[2-methoxy-1-(methoxymethy)-1-methylethyl]indol-3-yl]benzoic acid (157)

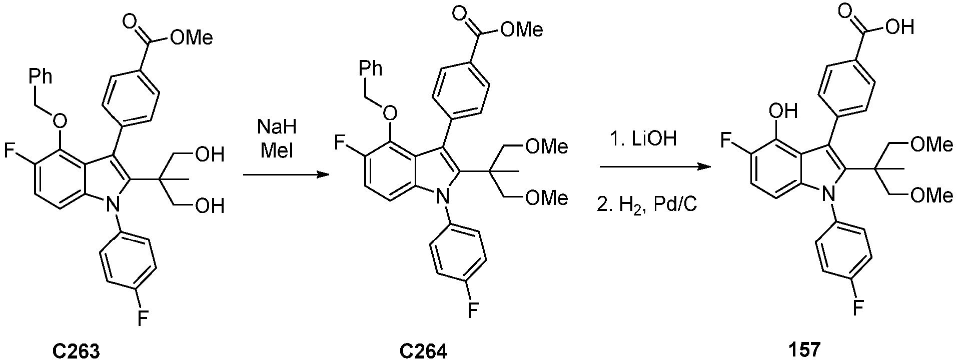

### Step 1. Synthesis of methyl 4-[4-benzyloxy-5-fluoro-1-(4-fluorophenyl)-2-[2-methoxy-1-(methoxymethyl)-1-methyl-ethyl]indol-3-yl]benzoate (C264)

To a solution of methyl 4-[4-benzyloxy-5-fluoro-1-(4-fluorophenyl)-2-[2-hydroxy-1-(hydroxymethyl)-1-methyl-ethyl]indol-3-yl]benzoate **C263** (28 mg, 0.05 mmol) and iodomethane (500 µL of 1 M, 0.5 mmol) in THF (1 mL) was added NaH (6 mg of 60% w/w, 0.15 mmol) and stirred at 40 °C for 2 hours. Additional iodomethane (500 µL of 1 M, 0.5 mmol) and NaH (6 mg of 60% w/w, 0.15 mmol) were added and the reaction stirred overnight. The mixture was heated to 60 °C for 12 hours. The reaction was quenched by the addition of HCl and then concentrated. The crude product was purified by chromatography (Gradient: 0-40% EtOAc in hexanes) to afford the product as a white solid. methyl 4-[4-benzyloxy-5-fluoro-1-(4-fluorophenyl)-2-[2-methoxy-1-(methoxymethyl)-1-methyl-ethyl]indol-3-yl[benzoate (16 mg, 58%). ¹H NMR (400 MHz, Chloroform-d) δ 7.94 - 7.85 (m, 2H), 7.60 - 7.52 (m, 2H), 7.50 - 7.41 (m, 2H), 7.25 - 7.11 (m, 5H), 6.89 - 6.79 (m, 3H), 6.25 (dd, J = 8.9, 3.5 Hz, 1H), 4.64 (d, J = 1.0 Hz, 2H), 3.94 (s, 3H), 3.20 (d, J = 9.1 Hz, 2H), 3.07 (d, J = 9.9 Hz, 8H), 1.05 (s, 3H). LCMS *m*/*z* 586.11 [M+H]⁺.

### Step 2 & 3: Synthesis of 4-[5-fluoro-1-(4-fluorophenyl)-4-hydroxy-2-[2-methoxy-1-(methoxymethyl)-1-methyl-ethyl]indol-3-yl]benzoic acid (157)

Compound **157** was prepared from **C264** in two steps (ester hydrolysis and benzyl group removal by hydrogenation) as described in the synthesis of compound **1.** 4-[5-fluoro-1-(4-fluorophenyl)-4-hydroxy-2-[2-methoxy-1-(methoxymethyl)-1-methyl-ethyl]indol-3-yl]benzoic acid (10 mg, 75%). ¹H NMR (400 MHz, Chloroform-d) δ 8.18 - 8.06 (m, 2H), 7.69 - 7.61 (m, 2H), 7.44 - 7.33 (m, 2H), 7.18 - 7.11 (m, 2H), 6.75 (dd, J = 10.8, 8.9 Hz, 1H), 5.99 (dd, J = 8.9, 3.6 Hz, 1H), 3.16 (d, J = 9.1 Hz, 2H), 3.01 (s, 8H), 1.00 (s, 3H). LCMS *m*/*z* 482.07 [M+H]⁺.

### Compound 158

### 4-[1-(4-fluorophenyl)-4-hydroxy-2-[2-methoxy-1-(methoxymethyl)-1-methyl-ethyl]indol-3-yl]benzoic acid (158)

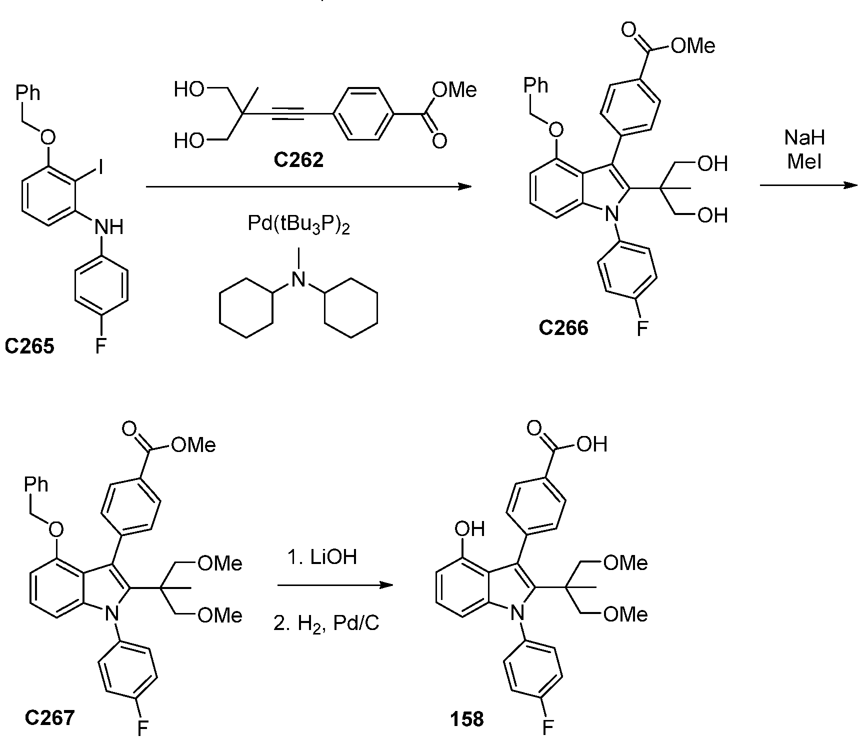

### Step 1. Synthesis of methyl 4-[4-benzyloxy-1-(4-fluorophenyl)-2-[2-hydroxy-1-(hydroxymethyl)-1-methyl-ethyl]indol-3-yl]benzoate (C266)

A mixture of methyl 4-[4-hydroxy-3-(hydroxymethyl)-3-methyl-but-1-ynyl]-benzoate (200 mg, 0.81 mmol), 3-benzyloxy-N-(4-fluorophenyl)-2-iodo-aniline **C265** (240 mg, 0.55 mmol) and N-cyclohexyl-N-methyl-cyclohexanamine (300 µL, 1.40 mmol) under and inert atmosphere (place under vacuum and then flushed with nitrogen). 1,4-dioxane (3 mL) and N-cyclohexyl-N-methyl-cyclohexanamine (300 µL, 1.40 mmol) were added. The mixture was pulled vacuum and flushed with nitrogen, then Pd(tBu₃P)₂ (15 mg, 0.03 mmol) was added. The reaction was sealed and heated at 60 °C for 12 hours. The mixture was then diluted with EtOAc (20 mL) and washed with water. The organic layer was dried and concentrated to dryness under reduced pressure. Silica gel chromatography (Gradient: 0-70% EtOAc in heptane) afforded the product as a white solid. Methyl 4-[4-benzyloxy-1-(4-fluorophenyl)-2-[2-hydroxy-1-(hydroxymethyl)-1-methyl-ethyl]indol-3-yl]benzoate (44 mg, 15%). ¹H NMR (400 MHz, Chloroform-*d*) δ 7.98 - 7.89 (m, 2H), 7.65 - 7.57 (m, 2H), 7.57 - 7.50 (m, 2H), 7.27 - 7.22 (m, 2H), 7.22 - 7.11 (m, 3H), 7.04 (dd, J = 8.3, 7.8 Hz, 1H), 6.86 - 6.77 (m, 2H), 6.55 (dd, J = 7.8, 0.7 Hz, 1H), 6.32 (dd, J = 8.3, 0.7 Hz, 1H), 4.83 (s, 2H), 3.99 (s, 3H), 3.52 (ddd, J = 12.0, 7.6, 1.7 Hz, 2H), 3.38 (dd, J = 11.5, 5.4 Hz, 2H), 2.21 (dd, J = 7.2, 5.5 Hz, 2H), 1.08 (s, 3H). LCMS *m*/*z* 540.07 [M+H]⁺.

### Step 2-4: Synthesis of 4-[1-(4-fluorophenyl)-4-hydroxy-2-[2-methoxy-1-(methoxymethyl)-1-methyl-ethyl]indol-3-yl]benzoic acid

Compound **158** was prepared in two steps from **C266** as described for the synthesis of compound **157.** 4-[1-(4-fluorophenyl)-4-hydroxy-2-[2-methoxy-1-(methoxymethyl)-1-methylethyl]indol-3-yl]benzoic acid (18.3 mg, 96%). ¹H NMR (400 MHz, Chloroform-*d*) δ 8.21 (d, J = 7.9 Hz, 2H), 7.76 (d, J = 7.9 Hz, 2H), 7.46 (dd, J = 8.6, 4.9 Hz, 2H), 7.23 (t, J = 8.3 Hz, 2H), 6.92 (t, J = 8.0 Hz, 1H), 6.45 (d, J = 7.6 Hz, 1H), 6.19 (d, J = 8.2 Hz, 1H), 3.23 (d, J = 9.1 Hz, 2H), 3.10 (d, J = 11.5 Hz, 8H), 1.08 (s, 3H). LCMS *m*/*z* 464.21 [M+H]⁺.

### Compound 159

### 4-[1-(4-fluoro-3-methyl-phenyl)-4-hydroxy-2-tetrahydropyran-4-yl-indol-3-yl]-2-hydroxybenzoic acid (159)

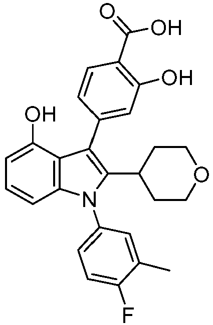

Compound **159** was prepared in three steps from **S3** and methyl 2-hydroxy-4-(4,4,5,5-tetramethyl-1,3,2-dioxaborolan-2-yl)benzoate as described in the preparation of compounds 9-11. Pd(OAc)₂, PPh₃, CsF was used in the Suzuki coupling step. Purification by reversed phase chromatography (C18 column. Gradient: 20-100% MeCN in water with a formic acid modifier) afforded the product. 4-[1-(4-fluoro-3-methyl-phenyl)-4-hydroxy-2-tetrahydropyran-4-yl-indol-3-yl]-2-hydroxy-benzoic acid (20 mg, 54%). ¹H NMR (400 MHz, DMSO-*d*₆) δ 9.07 (s, 1H), 7.70 (d, J = 8.0 Hz, 1H), 7.43 - 7.19 (m, 3H), 6.93 - 6.83 (m, 2H), 6.81 - 6.69 (m, 1H), 6.31 (dd, J = 7.7, 0.8 Hz, 1H), 6.15 (dd, J = 8.2, 0.8 Hz, 1H), 3.71 - 3.56 (m, 2H), 2.96 (td, J = 11.4, 4.9 Hz, 2H), 2.85 - 2.67 (m, 1H), 2.26 (d, J = 1.9 Hz, 3H), 1.51 (dt, J = 17.1, 4.8 Hz, 4H). LCMS *m*/*z* 462.12 [M+H]⁺.

### Compound 160

### 1-(3,4-difluorophenyl)-6-fluoro-3-(3-methylsulfonylphenyl)-2-tetrahydropyran-4-yl-indol-4-ol (160)

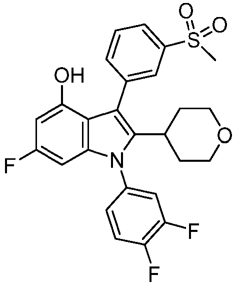

Compound **160** was prepared from **S10** in two steps, by Suzuki coupling with (3-methylsulfonylphenyl)boronic acid then benzyl group deprotection by hydrogenation. Suzuki coupling was performed with Pd(OAc)₂, PPh₃, and CsF. 1-(3,4-difluorophenyl)-6-fluoro-3-(3-methylsulfonylphenyl)-2-tetrahydropyran-4-yl-indol-4-ol (60 mg, 82%). ¹H NMR (400 MHz, Chloroform-*d*/CD₃OD) δ 7.99 (d, J = 2.6 Hz, 1H), 7.83 (d, J = 7.7 Hz, 1H), 7.72 - 7.64 (m, 1H), 7.54 (td, J = 7.6, 2.5 Hz, 1H), 7.40 - 7.28 (m, 2H), 7.18 (ddd, J = 10.0, 7.1, 3.1 Hz, 1H), 7.13 - 7.07 (m, 1H), 6.19 (dq, J = 11.1, 2.1 Hz, 1H), 5.99 (dq, J = 9.4, 2.0 Hz, 1H), 3.85 - 3.64 (m, 2H), 3.18 - 3.07 (m, 2H), 3.04 (d, J = 2.7 Hz, 3H), 2.79 (tt, J = 11.7, 3.5 Hz, 1H), 1.65 - 1.49 (m, 4H). LCMS *m*/*z* 502.05 [M+H]⁺.

### Compound 161

### 4-[5-fluoro-1-(4-fluorophenyl)-4-hydroxy-2-(2-methoxy-1,1-dimethyl-ethyl)indol-3-yl]benzoic acid (161)

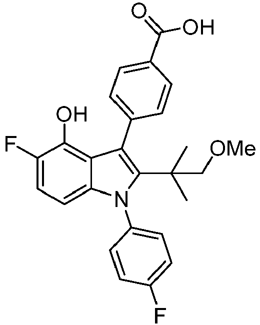

Compound **161** was prepared from **C229** and **C239** 3-benzyloxy-4-fluoro-N-(4-fluorophenyl)-2-iodo-aniline according to the method described for compound **146.** 4-[5-fluoro-1-(4-fluorophenyl)-4-hydroxy-2-(2-methoxy-1,1-dimethyl-ethyl)indol-3-yl]benzoic acid (88 mg). ¹H NMR (400 MHz, DMSO-*d*₆) δ 12.87 (s, 1H), 8.95 (d, J = 2.0 Hz, 1H), 7.97 - 7.83 (m, 2H), 7.62 - 7.46 (m, 4H), 7.46 - 7.30 (m, 2H), 6.82 (dd, J = 11.1, 8.8 Hz, 1H), 5.91 (dd, J = 8.8, 3.5 Hz, 1H), 2.99 (s, 3H), 2.93 (s, 2H), 0.98 (s, 6H). LCMS *m*/*z* 452.14 [M+1]⁺.

### Compound 162

### 4-[2-(2-cyano-1,1-dimethyl-ethyl)-5-fluoro-1-(4-fluorophenyl)-4-hydroxy-indol-3-yl]-3-fluoro-benzoic acid (162)

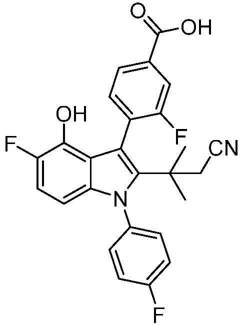

Compound **162** was prepared from **C229** and **C231** according to the method described for the preparation of compound **151.** 4-[2-(2-cyano-1,1-dimethyl-ethyl)-5-fluoro-1-(4-fluorophenyl)-4-hydroxy-indol-3-yl]-3-fluoro-benzoic acid (51 mg). ¹H NMR (400 MHz, DMSO-*d*₆) δ 13.27 (s, 1H), 9.25 (d, J = 1.8 Hz, 1H), 7.80 (dd, J = 7.9, 1.6 Hz, 1H), 7.75 - 7.57 (m, 3H), 7.51 (dddd, J = 15.1, 9.0, 7.4, 4.0 Hz, 3H), 6.91 (dd, J = 11.2, 8.9 Hz, 1H), 5.97 (dd, J = 8.9, 3.4 Hz, 1H), 2.60 - 2.53 (m, 2H), 1.20 - 1.14 (m, 6H). LCMS *m*/*z* 465.05 [M+1]+;

### Compound 163

### 4-[2-(2-cyano-1,1-dimethyl-ethyl)-1-(3,4-difluorophenyl)-5-fluoro-4-hydroxy-indol-3-yl]-3-fluoro-benzoic acid (163)

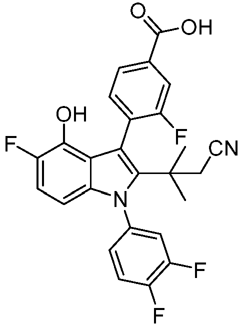

Compound **163** was prepared from **C228** and methyl 4-(4-cyano-3,3-dimethylbut-1-yn-1-yl)-3-fluorobenzoate according to the method described for compound **147.** 4-[2-(2-cyano-1,1-dimethyl-ethyl)-1-(3,4-difluorophenyl)-5-fluoro-4-hydroxy-indol-3-yl]-3-fluoro-benzoic acid (48 mg). ¹H NMR (400 MHz, Chloroform-*d*) δ 7.89 (dq, J = 7.8, 1.1 Hz, 1H), 7.80 (dt, J = 9.6, 1.2 Hz, 1H), 7.61 (td, J = 7.6, 1.9 Hz, 1H), 7.39 (dddd, J = 20.4, 10.0, 7.1, 2.7 Hz, 3H), 7.27 (d, J = 11.8 Hz, 1H), 6.87 (dd, J = 10.8, 8.9 Hz, 1H), 6.09 - 6.01 (m, 1H), 2.49 - 2.42 (m, 2H), 1.30 - 1.24 (m, 6H). LCMS *m*/*z* 483.04 [M+H]⁺.

### Compound 164

### 4-(2-(1-cyano-2-methylpropan-2-yl)-5-fluoro-1-(4-fluoro-3-methylphenyl)-4-hydroxy-1H-indol-3-yl)-3-fluorobenzoic acid (164)

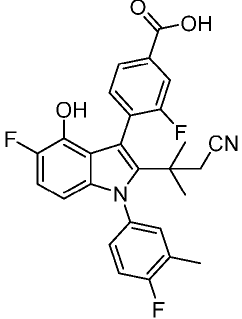

Compound **164** was prepared from **C39** and methyl 4-(4-cyano-3,3-dimethylbut-1-yn-1-yl)-3-fluorobenzoate according to the method described for the preparation of compound **148.** 4-[2-(2-cyano-1,1-dimethyl-ethyl)-5-fluoro-1-(4-fluoro-3-methyl-phenyl)-4-hydroxy-indol-3-yl]-3-fluoro-benzoic acid (98 mg, 87%) ¹H NMR (400 MHz, DMSO-d6) δ 13.26 (s, 1H), 9.23 (t, J = 1.7 Hz, 1H), 7.79 (dt, J = 7.9, 1.8 Hz, 1H), 7.70 (dd, J = 9.6, 1.7 Hz, 1H), 7.66 - 7.53 (m, 2H), 7.50 - 7.27 (m, 3H), 6.90 (dd, J = 11.2, 8.9 Hz, 1H), 6.05 - 5.97 (m, 1H), 2.56 (dd, J = 6.1, 2.0 Hz, 2H), 1.17 (dd, J = 5.8, 3.3 Hz, 6H). LCMS m/z 479.09 [M+H]+.

### Compound 165

### 4-[2-(2-cyano-1,1-dimethyl-ethyl)-1-(3,4-difluorophenyl)-6-fluoro-4-hydroxy-indol-3-yl]benzoic acid (165)

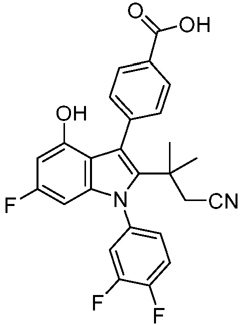

Compound **165** was prepared by Suzuki coupling of 3-[4-benzyloxy-3-bromo-1-(3,4-difluorophenyl)-6-fluoro-indol-2-yl]-3-methyl-butanenitrile with methyl 4-(4,4,5,5-tetramethyl-1,3,2-dioxaborolan-2-yl)benzoate using the method described for the preparation of compound **1.** 4-[2-(2-cyano-1,1-dimethyl-ethyl)-1-(3,4-difluorophenyl)-6-fluoro-4-hydroxy-indol-3-yl]benzoic acid (37 mg). ¹H NMR (400 MHz, DMSO-*d*₆) δ 12.89 (s, 1H), 9.61 (s, 1H), 7.96 - 7.90 (m, 2H), 7.80 (tdd, J = 8.8, 4.4, 2.5 Hz, 1H), 7.71 (dt, J = 10.5, 8.9 Hz, 1H), 7.56 - 7.49 (m, 2H), 7.41 (ddd, J = 10.0, 4.2, 2.3 Hz, 1H), 6.17 (dd, J = 11.5, 2.2 Hz, 1H), 5.86 (dd, J = 9.7, 2.2 Hz, 1H), 2.49 (s, 2H), 1.13 (d, J = 7.7 Hz, 6H). LCMS *m*/*z* 465.0 [M+1]+;

### Compound 166

### 4-[2-(2-cyano-1-methyl-ethyl)-1-(3,4-difluorophenyl)-6-fluoro-4-hydroxy-indol-3-yl]benzoic acid (166)

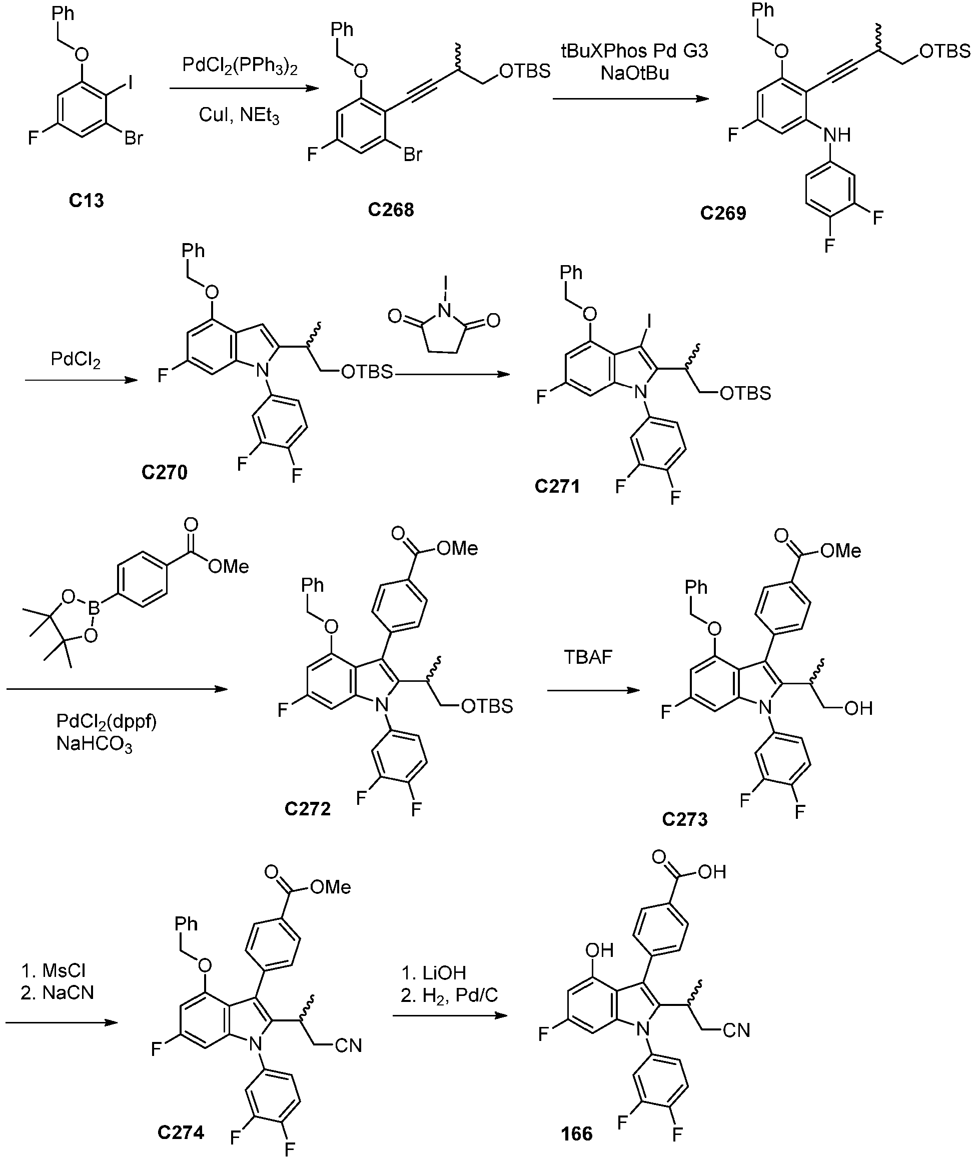

### Steps 1-6. Synthesis of methyl 4-(4-(benzyloxy)-1-(3,4-difluorophenyl)-6-fluoro-2-(1-hydroxypropan-2-yl)-1H-indol-3-yl)benzoate (C273)

Compound **C273** was prepared in six steps from compound **C13** using the method described for the preparation of **C166** in the synthesis of compound **119.**

### Step 7 & 8. Synthesis of methyl 4-[4-benzyloxy-1-(3,4-difluorophenyl)-6-fluoro-2-(1-methyl-2-methylsulfonyloxy-ethyl)indol-3-yl]benzoate (C274)

To a solution of methyl 4-(4-(benzyloxy)-1-(3,4-difluorophenyl)-6-fluoro-2-(1-hydroxypropan-2-yl)-1H-indol-3-yl)benzoate **C273** (250 mg, 0.46 mmol) in dichloromethane (2 mL) was added Et₃N (68 µL, 0.5 mmol) followed by MsCl (38 µL, 0.5 mmol). The reaction mixture was stirred for 10 minutes then washed with water. The organic layer was concentrated to dryness and purified via silica gel chromatography (Gradient: 0-55% EtOAc in heptane) to afford the product methyl 4-[4-benzyloxy-1-(3,4-difluorophenyl)-6-fluoro-2-(1-methyl-2-methylsulfonyloxy-ethyl)indol-3-yl]benzoate (190 mg, 66%). ¹H NMR (400 MHz, Chloroform-*d*) δ 8.08 - 8.01 (m, 2H), 7.60 - 7.54 (m, 2H), 7.51 - 7.35 (m, 2H), 7.32 - 7.16 (m, 4H), 6.85 - 6.80 (m, 2H), 6.49 - 6.43 (m, 1H), 6.32 (ddd, J = 9.2, 3.5, 2.0 Hz, 1H), 4.98 - 4.91 (m, 2H), 4.13

### (ddd, J = 9.7, 8.2, 1.3 Hz, 1H), 4.04 (s, 3H), 4.00 (ddd, J = 9.7, 7.4, 1.9 Hz, 1H), 3.35 - 3.23 (m, 1H), 2.91 (d, J = 5.0 Hz, 3H), 1.17 (dd, J = 7.2, 1.0 Hz, 3H). LCMS m/z 624.0 [M+1]⁺.

To a solution of methyl 4-[4-benzyloxy-1-(3,4-difluorophenyl)-6-fluoro-2-(1-methyl-2-methylsulfonyloxy-ethyl)indol-3-yl]benzoate **C273** (175 mg, 0.28 mmol) in NMP (2 mL) was added NaCN (69 mg, 1.408 mmol). The reaction mixture was stirred in a sealed vial at 90 °C for 4 hours. The mixture was diluted with sat. aq. NaHCO₃ and extracted with EtOAc. The organic layer was concentrated to dryness and purified via silica gel chromatography eluting with 0-40% EtOAc in heptane. Pure fractions were combined and concentrated to give 36 mg white solid. methyl 4-[4-benzyloxy-2-(2-cyano-1-methyl-ethyl)-1-(3,4-difluorophenyl)-6-fluoro-indol-3-yl]benzoate (36 mg, 23%) ¹H NMR (400 MHz, Chloroform-d) δ 8.07 - 7.97 (m, 2H), 7.60 - 7.32 (m, 4H), 7.27 - 7.10 (m, 4H), 6.81 - 6.77 (m, 2H), 6.42 (dd, J = 11.5, 2.1 Hz, 1H), 6.28 (ddd, J = 9.1, 3.2, 2.0 Hz, 1H), 4.90 (s, 2H), 4.01 (s, 3H), 3.28 - 3.12 (m, 1H), 2.37 - 2.22 (m, 2H), 1.27 - 1.21 (m, 3H). LCMS *m*/*z* 555.0 [M+1]⁺.

Compound **166** was prepared from **C274** by ester hydrolysis and benzyl group removal using the method described for the preparation of compound **166.** 4-[2-(2-cyano-1-methyl-ethyl)-1-(3,4-difluorophenyl)-6-fluoro-4-hydroxy-indol-3-yl]benzoic acid (10 mg, 36%). ¹H NMR (400 MHz, DMSO-*d*₆) δ 9.85 (s, 1H), 7.99 - 7.94 (m, 2H), 7.81 - 7.67 (m, 2H), 7.51 (dd, J = 8.1, 1.5 Hz, 2H), 7.40 (d, J = 9.0 Hz, 1H), 6.28 (dt, J = 11.4, 1.9 Hz, 1H), 6.11 (dd, J = 9.6, 2.2 Hz, 1H), 3.15 (p, J = 8.2 Hz, 1H), 2.77 (d, J = 7.6 Hz, 1H), 2.42 - 2.30 (m, 1H), 1.12 (dd, J = 7.2, 4.8 Hz, 3H). LCMS *m*/*z* 451.0 [M+H]⁺.

### Compound 167

### 4-[1-(3,4-difluorophenyl)-6-fluoro-4-hydroxy-2-tetrahydropyran-4-yl-indol-3-yl]-2-hydroxybenzoic acid (167)

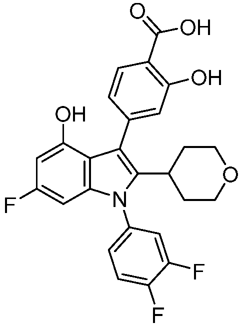

Compound **167** was prepared from **S3** according to the method described for preparation of compound **159.** Purification by reversed-phase chromatography (Column: C18. Gradient: 20-100% MeCN in water with 0.1% formic acid) afforded the product.4-[1-(3,4-difluorophenyl)-6-fluoro-4-hydroxy-2-tetrahydropyran-4-yl-indol-3-yl]-2-hydroxy-benzoic acid (12 mg). ¹H NMR (400 MHz, DMSO-*d*₆) δ 9.70 (s, 1H), 7.84 - 7.58 (m, 3H), 7.47 - 7.33 (m, 1H), 6.96 - 6.81 (m, 3H), 6.66 (s, 1H), 6.24 (dd, J = 11.5, 2.2 Hz, 1H), 6.06 (dd, J = 9.6, 2.2 Hz, 1H), 3.76 - 3.63 (m, 2H), 3.03 (t, J = 9.4 Hz, 2H), 2.84 - 2.71 (m, 1H), 1.60 - 1.44 (m, 4H). LCMS *m*/*z* 484.12 [M+H]⁺

### Compound 168

### 4-[1-(4-fluoro-3-methyl-phenyl)-4-hydroxy-2-(2-hydroxy-1,1-dimethyl-ethyl)indol-3-yl]benzoic acid (168)

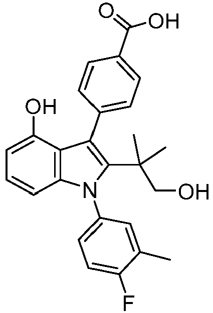

Compound **168** was prepared from 3-benzyloxy-2-bromo-N-(4-fluoro-3-methylphenyl)aniline and **C222** as described for compound **150.** 3-benzyloxy-2-bromo-N-(4-fluoro-3-methyl-phenyl)aniline was prepared from **C2.** 4-[1-(4-fluoro-3-methyl-phenyl)-4-hydroxy-2-(2-hydroxy-1,1-dimethyl-ethyl)indol-3-yl]benzoic acid (20.3 mg). ¹H NMR (400 MHz, Chloroform-d) δ 8.28 - 8.08 (m, 2H), 7.81 - 7.66 (m, 2H), 7.31 - 7.26 (m, 2H), 7.17 (t, J = 8.7 Hz, 1H), 6.95 (t, J = 8.0 Hz, 1H), 6.46 (d, J = 7.7 Hz, 1H), 6.24 (d, J = 8.3 Hz, 1H), 3.38 (s, 2H), 2.36 (d, J = 1.9 Hz, 3H), 1.06 (s, 6H). LCMS *m*/*z* 434.1 [M+H]⁺.

### Compound 169

### 4-[2-[2-(dimethylcarbamoyloxy)-1,1-dimethyl-ethyl]-1-(4-fluorophenyl)-4-hydroxy-indol-3-yl]benzoic acid (169)

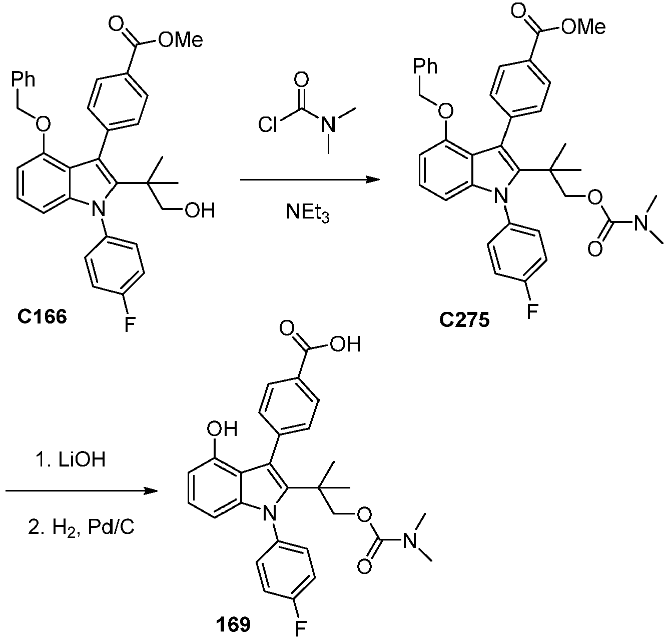

### Step 1. Synthesis of methyl 4-[4-benzyloxy-2-[2-[tert-butyl(dimethyl)silyl]oxy-1,1-dimethylethyl]-1-(4-fluorophenyl)indol-3-yl]benzoate (C275)

A mixture of [2-[4-benzyloxy-1-(4-fluorophenyl)-3-iodo-indol-2-yl]-2-methyl-propoxy]-tert-butyl-dimethyl-silane **C166** (7.7 g, 12.2 mmol), (4-methoxycarbonylphenyl)boronic acid (4.4 g, 24.5 mmol), PdCl₂(dppf) (1 g, 1.23 mmol), and CsF (7.4 g, 48.7 mmol) in DME (60 mL) was heated at 90 °C in a sealed flask for 4 hours. The reaction mixture was cooled to room temperature, diluted with water and extracted with EtOAc. The organic layer was concentrated to dryness and purified via silica gel chromatography (gradient: 0-40% EtOAc in heptane) to afford the product which was carried to the next step without further purification. Methyl 4-[4-benzyloxy-2-[2-[tert-butyl(dimethyl)silyl]oxy-1,1-dimethyl-ethyl]-1-(4-fluorophenyl)indol-3-yl]benzoate (6.6 g, 85%). LCMS *m*/*z* 638.0 [M+H]⁺.

### Step 2 & 3. Synthesis of methyl 4-[4-benzyloxy-2-[2-(dimethylcarbamoyloxy)-1,1-dimethylethyl]-1-(4-fluorophenyl)indol-3-yl]benzoate (169)

Compound **169** was prepared in two steps from **C275** by ester hydrolysis with LiOH, then hydrogenation as described in the preparation of compound **1.** 4-[4-benzyloxy-2-[2-(dimethylcarbamoyloxy)-1,1-dimethyl-ethyl]-1-(4-fluorophenyl)indol-3-yl]benzoic acid (43 mg). ¹H NMR (400 MHz, Chloroform-d) δ 7.86 (d, J = 7.8 Hz, 2H), 7.43 (d, J = 7.9 Hz, 2H), 7.35 (dd, J = 8.7, 4.8 Hz, 2H), 7.19 - 7.05 (m, 5H), 6.93 (t, J = 8.0 Hz, 1H), 6.75 - 6.67 (m, 2H), 6.45 (d, J = 7.8 Hz, 1H), 6.19 (d, J = 8.3 Hz, 1H), 4.73 (s, 2H), 3.81 (s, 2H), 2.78 (s, 6H), 1.00 (s, 6H). LCMS *m*/*z* 581.0 [M+1]+

### Compound 170

### 4-[1-(3,4-difluorophenyl)-6-fluoro-4-hydroxy-2-(2-methoxy-1-methyl-ethyl)indol-3-yl]benzoic acid (170)

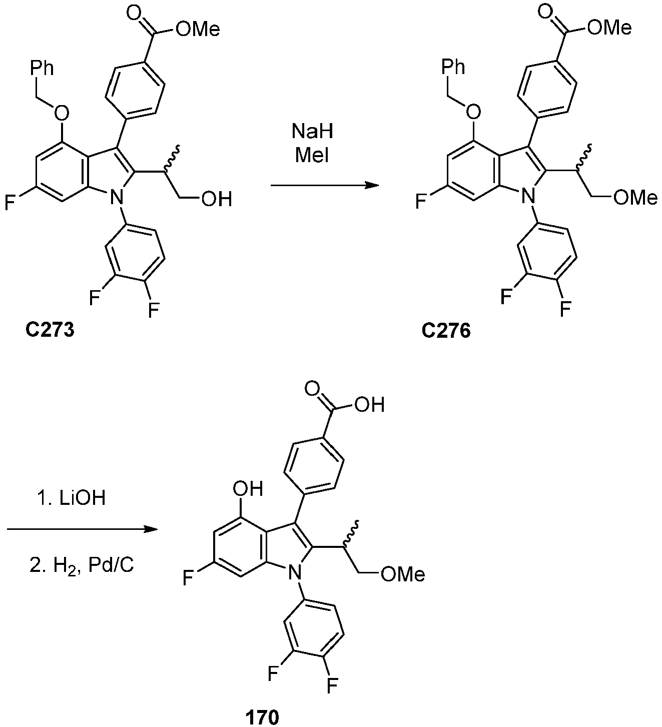

### Step 1. Synthesis of methyl 4-[4-benzyloxy-1-(3, 4-difluorophenyl)-6-fluoro-2-(2-methoxy-1-methyl-ethyl)indol-3-yl]benzoate (C276)

To a solution of **C273** (250 mg, 0.46 mmol) in NMP (3 mL) was added NaH. After 5 minutes, MeI (86 µL) was added and the mixture was heated to 90 °C for 4 hours. The mixture was diluted with water and extracted with EtOAc. The organic layer was concentrated to dryness and purified via silica gel chromatography (Gradient: 0-50% EtOAc in heptane) to afford the product

methyl 4-[4-benzyloxy-1-(3,4-difluorophenyl)-6-fluoro-2-(2-methoxy-1-methylethyl)indol-3-yl]benzoate (105 mg, 41%). ¹H NMR (400 MHz, Chloroform-*d*) δ 8.00 - 7.93 (m, 2H), 7.51 (d, J = 7.7 Hz, 2H), 7.39 - 7.21 (m, 3H), 7.20 - 7.10 (m, 3H), 6.79 - 6.73 (m, 2H), 6.38 (dd, J = 11.5, 2.0 Hz, 1H), 6.25 (dt, J = 9.2, 2.1 Hz, 1H), 4.88 (s, 2H), 3.98 (s, 3H), 3.22 - 3.02 (m, 6H), 1.04 - 0.98 (m, 3H). LCMS *m*/*z* 560.0 [M+H]⁺.

### Steps 2 & 3. Synthesis of 4-[1-(3,4-difluorophenyl)-6-fluoro-4-hydroxy-2-(2-methoxy-1-methylethyl)indol-3-yl]benzoic acid (170)

Compound **170** was prepared in two steps from compound **C276** by ester hydrolysis and then benzyl group removal by hydrogenation as described for compound **1.** The product was triturated in 9:1 heptane / EtOAc then filtered and dried to afford 4-[1-(3,4-difluorophenyl)-6-fluoro-4-hydroxy-2-(2-methoxy-1-methyl-ethyl)indol-3-yl]benzoic acid (42 mg). ¹H NMR (400 MHz, DMSO-*d*₆) δ 12.91 (s, 1H), 9.76 (s, 1H), 7.98 - 7.90 (m, 2H), 7.80 - 7.64 (m, 2H), 7.49 (dd, J = 8.3, 1.7 Hz, 2H), 7.42 - 7.31 (m, 1H), 6.26 (dd, J = 11.4, 2.2 Hz, 1H), 6.08 (ddd, J = 9.6, 2.2, 0.9 Hz, 1H), 3.18 - 2.98 (m, 6H), 0.97 (dd, J = 6.6, 3.6 Hz, 3H). LCMS *m*/*z* 456.0 [M+H]⁺.

### Compound 171

### 4-[1-(4-fluoro-3-methyl-pheny)-4-hydroxy-2-(2-methoxy-1,1-dimethyl-ethy)indol-3-yl]benzoic acid (171)

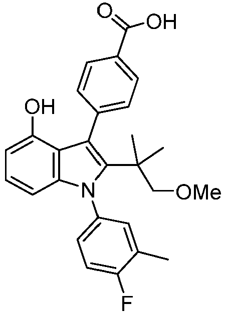

Compound **171** was prepared from **C222** and 3-benzyloxy-2-bromo-N-(4-fluoro-3-methyl-phenyl)aniline using the method described for the preparation of compound **144.** 3-benzyloxy-2-bromo-N-(4-fluoro-3-methyl-phenyl)aniline was prepared by coupling (4-fluoro-3-methyl-phenyl)boronic acid with 1-benzyloxy-2-bromo-3-nitro-benzene using the Mo catalysis method used in the preparation of **C224.** 4-[1-(4-fluoro-3-methyl-phenyl)-4-hydroxy-2-(2-methoxy-1,1-dimethyl-ethyl)indol-3-yl]benzoic acid (47 mg). ¹H NMR (400 MHz, Chloroform-*d)* δ 8.19 - 8.07 (m, 2H), 7.74 - 7.62 (m, 2H), 7.26 - 7.15 (m, 2H), 7.09 (t, J = 8.7 Hz, 1H), 6.90 - 6.81 (m, 1H), 6.38 (dd, J = 7.7, 0.8 Hz, 1H), 6.16 (dd, J = 8.3, 0.8 Hz, 1H), 3.02 (s, 3H), 2.92 (s, 2H), 2.29 (d, J = 1.9 Hz, 3H), 1.00 (d, J = 2.6 Hz, 6H). LCMS *m*/*z* 448.14 [M+H]⁺.

### Compound 172

### (2S, 3S, 4S, 5R)-6-[4-[1-(3,4-difluorophenyl)-6-fluoro-4-hydroxy-2-tetrahydropyran-4-yl-indol-3-yl]benzoyl]oxy-3,4,5-trihydroxy-tetrahydropyran-2-carboxylic acid (172)

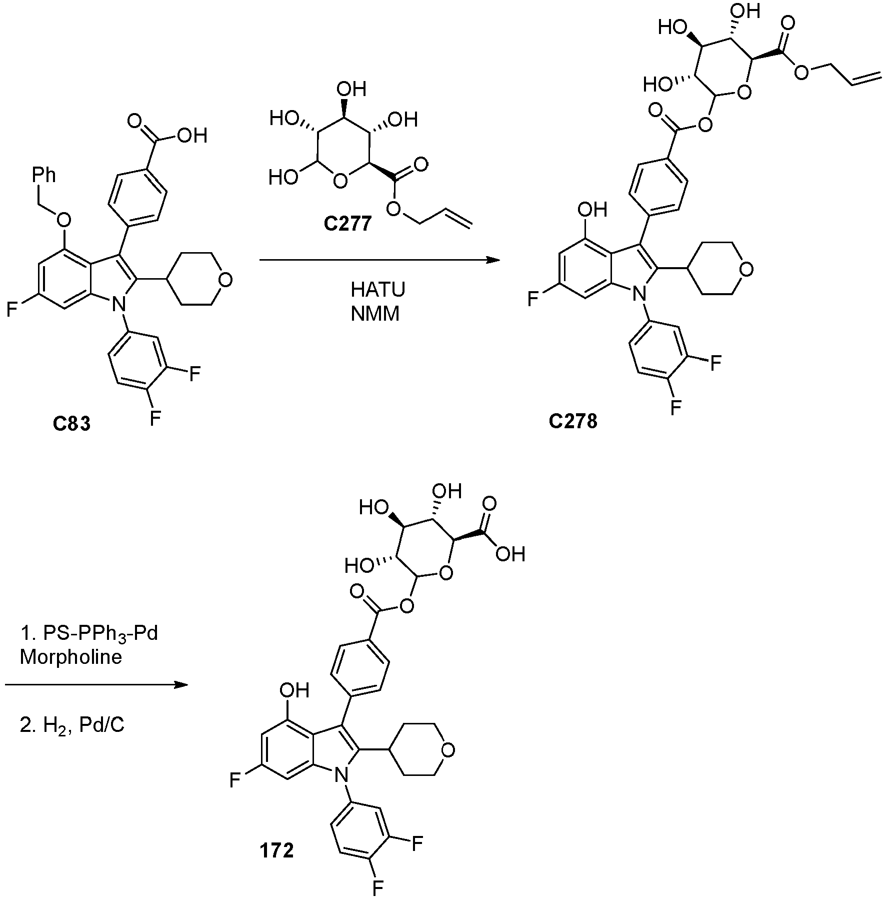

### Step 1. Synthesis of allyl (2S,3S,4S,5R)-6-[4-[4-benzyloxy-1-(3,4-difluorophenyl)-6-fluoro-2-tetrahydropyran-4-yl-indol-3-yl]benzoyl]oxy-3,4,5-trihydroxy-tetrahydropyran-2-carboxylate (C278)

DMF (10 mL) followed by NMM (493 µL, 4.48 mmol) was added to a mixture of 4-[4-benzyloxy-1-(3,4-difluorophenyl)-6-fluoro-2-tetrahydropyran-4-yl-indol-3-yl]benzoic acid (500 mg, 0.90 mmol), allyl (2S,3S,4S,5R)-3,4,5,6-tetrahydroxytetrahydropyran-2-carboxylate (526 mg, 2.246 mmol), and HATU (1.02 g, 2.68 mmol). The mixture was allowed to stir at room temperature for 24 hours. DMF (8 mL) was added and the mixture heated to 80 °C for 30 minutes. Upon cooling, the mixture was diluted into brine, and extracted with EtOAc (x 2). The combined organic phases were washed with brine (x 2). Combined organic phases were dried (MgSO₄), filtered and concentrated *in vacuo.* Purification via silica gel chromatography (Gradient: 0-30% MeOH in CH₂Cl₂). The product was used in subsequent steps without further purification. Allyl (2S,3S,4S,5R)-6-[4-[4-benzyloxy-1-(3,4-difluorophenyl)-6-fluoro-2-tetrahydropyran-4-yl-indol-3-yl]benzoyl]oxy-3,4,5-trihydroxy-tetrahydropyran-2-carboxylate (56 mg, 7%). LCMS *m*/*z* 793.27 [M+H]⁺.

### Step 2. Synthesis of (2S, 3S, 4S,5R)-6-[4-[1-(3,4-difluorophenyl)-6-fluoro-4-hydroxy-2-tetrahydropyran-4-yl-indol-3-yl]benzoyl]oxy-3,4,5-trihydroxy-tetrahydropyran-2-carboxylic acid (172)

To a solution of allyl (2S,3S,4S,5R)-6-[4-[4-benzyloxy-1-(3,4-difluorophenyl)-6-fluoro-2-tetrahydropyran-4-yl-indol-3-yl]benzoyl]oxy-3,4,5-trihydroxy-tetrahydropyran-2-carboxylate **C278** (52 mg, 0.06 mmol) in dichloromethane (3 mL) at room temperature was added morpholine (11 µL, 0.13 mmol). The solution was purged with nitrogen for 5 minutes, then PS-PPh₃-Pd (polymer supported palladium (tertakis)triphenylphosphine) (31 mg, 0.12 mmol) was added. The reaction mixture was stirred at room temperature for 16 hours. The mixture was then filtered, washed with EtOAc. A small quantity of methanol was added to aid solubilization. The mixture was concentrated to dryness under reduced pressure. Purification by reversed-phase chromatography (Column: C18. Gradient: 0-80% MeCN in water with 0.1% formic acid) afforded allyl (2S,3S,4S,5R)-6-[4-[4-benzyloxy-1-(3,4-difluorophenyl)-6-fluoro-2-tetrahydropyran-4-yl-indol-3-yl]benzoyl]oxy-3,4,5-trihydroxy-tetrahydropyran-2-carboxylate. To a flask containing palladium on carbon (5 mg, 0.005 mmol) under nitrogen, was added ethanol (1.0 mL). A solution of allyl (2S,3S,4S,5R)-6-[4-[4-benzyloxy-1-(3,4-difluorophenyl)-6-fluoro-2-tetrahydropyran-4-yl-indol-3-yl]benzoyl]oxy-3,4,5-trihydroxy-tetrahydropyran-2-carboxylate (52 mg, 0.06 mmol) in THF (1.0 mL) and ethyl acetate (1 mL) was added to the mixture. The mixture was purged with hydrogen and then stirred under an atmosphere of hydrogen (balloon) for 2 hours, then for a further 3 days. The reaction was filtered through a pad of florosil^{®}, washed with 10% MeOH in EtOAc, then a mixture of THF-MeOH . The resulting filtrate was concentrated *in vacuo.* Purification by reversed-phase chromatography (Column: C18. Gradient: 20-100% MeCN in water with 0.1% formic acid) afforded the product. (2S,3S,4S,5R)-6-[4-[1-(3,4-difluorophenyl)-6-fluoro-4-hydroxy-2-tetrahydropyran-4-yl-indol-3-yl]benzoyl]oxy-3,4,5-trihydroxy-tetrahydropyran-2-carboxylic acid (7 mg, 50%) ¹H NMR (400 MHz, DMSO-*d*₆) δ 9.77 (s, 1H), 8.08 - 8.00 (m, 2H), 7.80 (t, J = 9.2 Hz, 1H), 7.76 - 7.65 (m, 1H), 7.61 - 7.52 (m, 2H), 7.40 (d, J = 8.9 Hz, 1H), 6.87 (s, 1H), 6.66 (s, 1H), 6.25 (dd, J = 11.5, 2.2 Hz, 1H), 6.07 (dd, J = 9.6, 2.2 Hz, 1H), 5.64 (d, J = 6.8 Hz, 1H), 5.53 (s, 1H), 5.28 (s, 1H), 3.67 (d, J = 11.0 Hz, 2H), 3.03 (s, 2H), 2.77 (t, J = 11.7 Hz, 1H), 1.60 - 1.40 (m, 4H). LCMS *m*/*z* 643.93 [M+H]⁺.

### Compound 173

### 4-[1-(4-fluorophenyl)-4-hydroxy-2-(4-hydroxy-4-methyl-cyclohexyl)indol-3-yl]benzoic acid [TRANS](173)

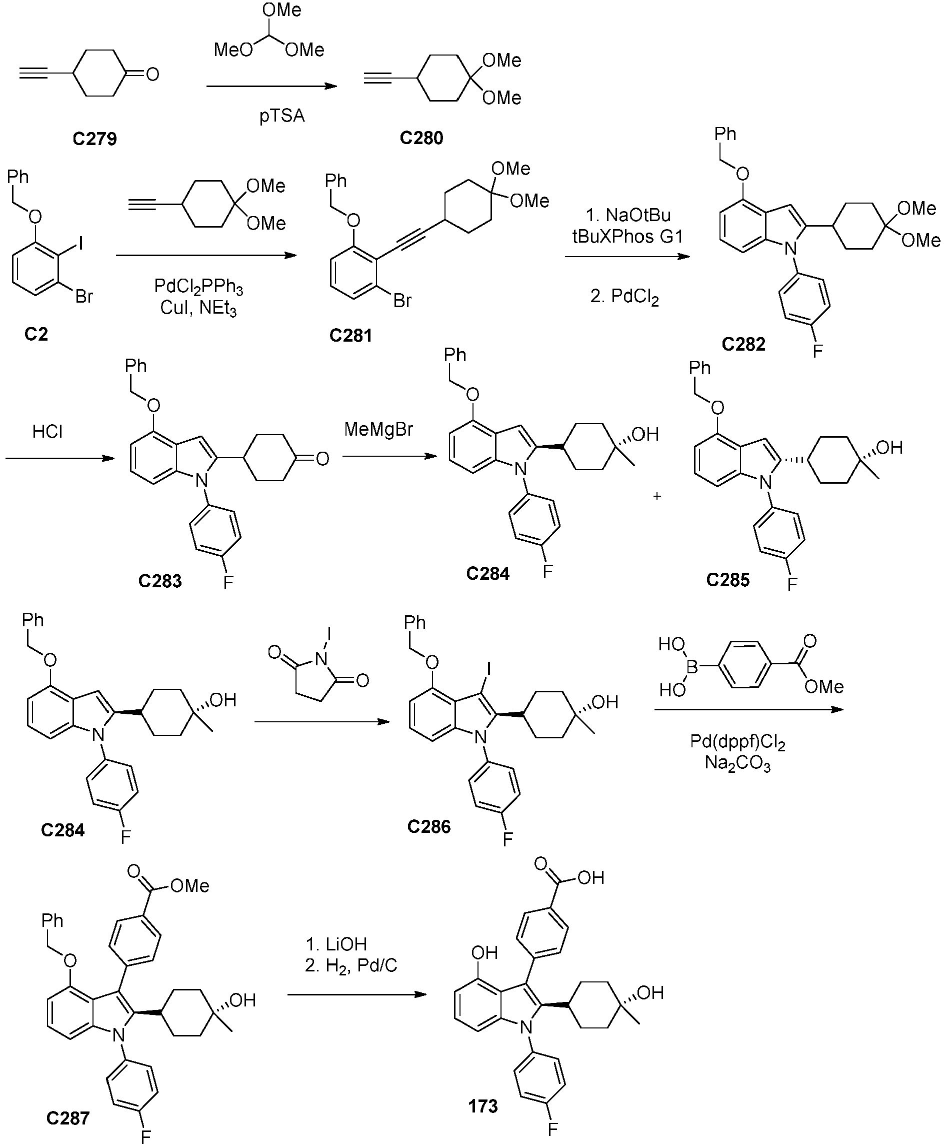

### Step 1. Synthesis of 4-ethynyl-1,1-dimethoxy-cyclohexane (C280)

A 5 mL microwave tube, a mixture of 4-ethynylcyclohexanone (305 mg, 2.5 mmol), trimethoxymethane (1.4 mL, 12.8 mmol), and p-Toluenesulfonic acid (hydrate) (24 mg, 0.13 mmol) in methanol (500 µL) was heated to 80 °C (heating block temperature) and allowed to stir for three days. The mixture was concentrated under reduced pressure. The mixture was then diluted with dichloromethane, and washed successively with saturated NaHCO₃, water, brine, and dried over sodium sulfate. The mixture was concentrated to afford the product, which was used in the subsequent step without further purification. 4-ethynyl-1,1-dimethoxy-cyclohexane (387 mg, 83%). ¹H NMR (400 MHz, DMSO-*d*₆) δ 3.05 (d, J = 1.9 Hz, 6H), 2.87 (d, J = 2.4 Hz, 1H), 2.43 (s, 1H), 1.76 (q, J = 9.0 Hz, 2H), 1.72 - 1.61 (m, 2H), 1.51 - 1.38 (m, 4H).

### Steps 2-3. Synthesis of 4-benzyloxy-2-(4, 4-dimethoxycyclohexyl)-1-(4-fluorophenyl)indole (C282)

Compound **C282** was prepared from **C2** and **C280** in three steps according to the method described for the preparation of **C5** in the preparation of **S1.** Silica gel chromatography (Column: 80g Combiflash Isco. Gradient: 0-30% EtOAc in heptane) afforded the product. 4-benzyloxy-2-(4,4-dimethoxycyclohexyl)-1-(4-fluorophenyl)indole (1.18 g) ¹H NMR (300 MHz, DMSO-*d*₆) δ 7.57 - 7.49 (m, 2H), 7.49 - 7.39 (m, 6H), 7.38 - 7.30 (m, 1H), 6.98 - 6.90 (m, 1H), 6.66 (d, J = 7.8 Hz, 1H), 6.51 (d, J = 8.2 Hz, 1H), 6.42 (s, 1H), 5.23 (s, 2H), 3.05 (d, J = 1.8 Hz, 6H), 2.58 (d, J = 11.8 Hz, 1H), 1.94 (d, J = 13.1 Hz, 2H), 1.70 (d, J = 12.0 Hz, 2H), 1.53 (q, J = 12.0 Hz, 2H), 1.24 - 1.10 (m, 2H).

### Step 4. Synthesis of 4-[4-benzyloxy-1-(4-fluorophenyl)indol-2-yl]cyclohexanone (C283)

To a solution of 4-benzyloxy-2-(4,4-dimethoxycyclohexyl)-1-(4-fluorophenyl)indole **C282** (1.18 g, 2.6 mmol) in tetrahydrofuran (12 mL) was added aqueous hydrogen chloride (12 mL of 3 M, 36.0 mmol). The mixture was warmed to 50 °C and allowed to stir for 5 hours. The mixture was diluted with dichloromethane, and washed with brine, dried over magnesium sulfate, filtered and concentrated to give desired product. 4-[4-benzyloxy-1-(4-fluorophenyl)indol-2-yl]cyclohexanone (600 mg, 56%). ¹H NMR (400 MHz, DMSO-*d*₆) δ 7.57 - 7.38 (m, 8H), 7.38 - 7.30 (m, 1H), 6.95 (t, J = 8.0 Hz, 1H), 6.67 (d, J = 7.7 Hz, 1H), 6.55 - 6.50 (m, 2H), 5.23 (s, 2H), 3.09 - 2.97 (m, 1H), 2.45 - 2.29 (m, 2H), 2.23 - 2.04 (m, 4H), 1.94 - 1.78 (m, 2H). LCMS *m*/*z* 414.17 [M+H]⁺.

### Step 5. Synthesis of 4-[4-benzyloxy-1-(4-fluorophenyl)indol-2-yl]-1-methyl-cyclohexanol [TRANS] (C284) and 4-[4-benzyloxy-1-(4-fluorophenyl)indol-2-yl]-1-methyl-cyclohexanol[CIS] (C285)

A solution of 4-[4-benzyloxy-1-(4-fluorophenyl)indol-2-yl]cyclohexanone **C283** (150 mg, 0.36 mmol) in 2-MeTHF (1.6 mL) under nitrogen was cooled to 0 °C with ice bath. Bromo(methyl)magnesium (160 µL of 3.4 M, 0.54 mmol) was added, and after 5 minutes, the ice bath was removed and allowed to stir at room temperature for 1 hour. The mixture was then quenched with sat NH₄Cl (aq) and extracted with 2-MeTHF (2x). The combined organic layers were washed with brine, dried over sodium sulfate, and concentrated under reduced pressure. Silica gel chromatography (Column: 12g Combiflash Isco. Gradient: 0-30% EtOAc in heptane) afforded two product, cis and trans isomers.

4-[4-benzyloxy-1-(4-fluorophenyl)indol-2-yl]-1-methyl-cyclohexanol [TRANS] **C284** (35 mg, 45%). ¹H NMR (400 MHz, DMSO-*d*₆) δ 7.56 - 7.50 (m, 2H), 7.49 - 7.38 (m, 6H), 7.38 - 7.31 (m, 1H), 6.93 (t, J = 8.0 Hz, 1H), 6.66 (d, J = 7.8 Hz, 1H), 6.50 (d, J = 8.2 Hz, 1H), 6.43 (s, 1H), 5.23 (s, 2H), 4.06 (s, 1H), 1.77 (dd, J = 12.9, 10.0 Hz, 2H), 1.53 (t, J = 13.3 Hz, 4H), 1.27 - 1.07 (m, 3H), 1.05 (s, 3H). LCMS *m*/*z* 430.14 [M+H]⁺.

4-[4-benzyloxy-1-(4-fluorophenyl)indol-2-yl]-1-methyl-cyclohexanol [CIS] **C285** (40 mg, 50%). ¹H NMR (400 MHz, DMSO-*d*₆) δ 7.55 - 7.50 (m, 2H), 7.49 - 7.38 (m, 6H), 7.38 - 7.32 (m, 1H), 6.93 (t, J = 8.0 Hz, 1H), 6.65 (d, J = 7.8 Hz, 1H), 6.53 - 6.46 (m, 2H), 5.22 (s, 2H), 4.24 (s, 1H), 1.75 - 1.67 (m, 2H), 1.50 (q, J = 12.1 Hz, 4H), 1.31 - 1.21 (m, 3H), 1.13 (s, 3H). LCMS *m*/*z* 430.23 [M+H]⁺.

### Steps 6-8. Synthesis of 4-[1-(4-fluorophenyl)-4-hydroxy-2-(4-hydroxy-4-methylcyclohexyl)indol-3-yl]benzoic acid [TRANS] (173)

Compound **173** was prepared in 4 steps from **C284** using the method described for the preparation of compound **1.** 4-[1-(4-fluorophenyl)-4-hydroxy-2-(4-hydroxy-4-methylcyclohexyl)indol-3-yl]benzoic acid (14.8 mg, 95%). ¹H NMR (400 MHz, DMSO-*d*₆) δ 12.86 (s, 1H), 9.15 (s, 1H), 7.97 - 7.89 (m, 2H), 7.58 - 7.39 (m, 6H), 6.83 (t, J = 7.9 Hz, 1H), 6.38 (d, J = 7.6 Hz, 1H), 6.20 (d, J = 8.1 Hz, 1H), 3.41 (s, 2H), 1.58 (d, J = 12.7 Hz, 2H), 1.40 (dd, J = 17.4, 12.4 Hz, 4H), 1.09 (t, J = 12.4 Hz, 2H), 0.67 (s, 3H). LCMS *m*/*z* 460.2 [M+H]⁺.

### Compound 174

### 4-[1-(4-fluoro-3-methyl-phenyl)-4-hydroxy-2-(3-methyl-1,1-dioxo-thietan-3-yl)indol-3-yl]benzoic acid (174)

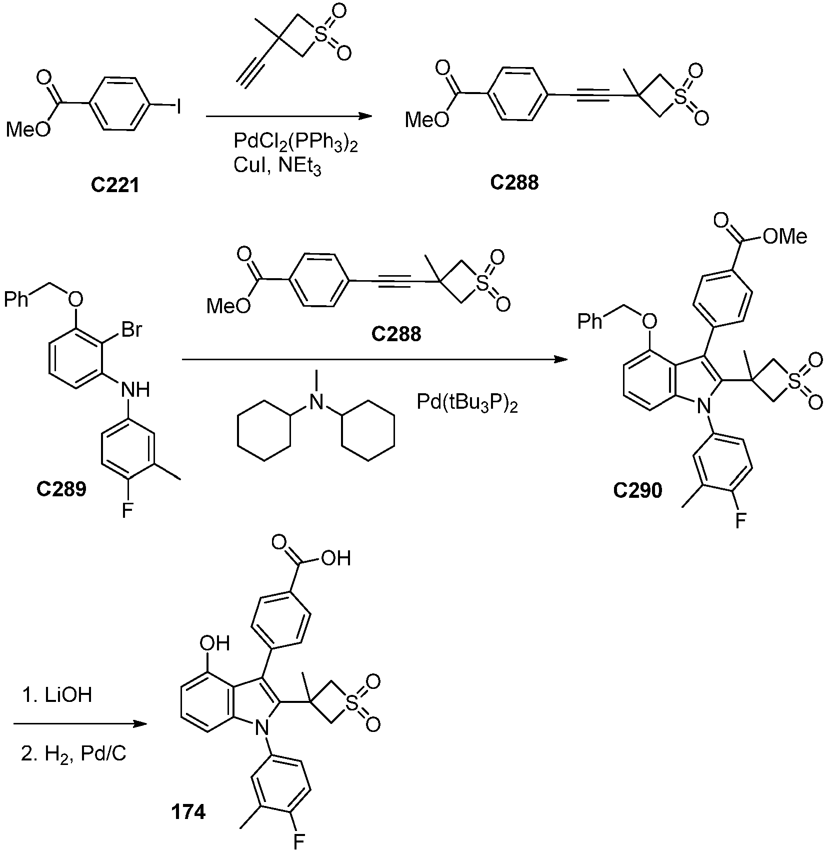

### Step 1. Synthesis of methyl 4-[2-(3-methyl-1,1-dioxo-thietan-3-yl)ethynyl]benzoate (C288)

Compound **C228** was prepared by Sonagashira coupling of methyl 4-iodobenzoate **C221** (790 mg, 3.02 mmol) and 3-ethynyl-3-methyl-thietane 1,1-dioxide (500 mg, 3.47 mmol) according to the method described in the preparation of C222. Methyl 4-[2-(3-methyl-1,1-dioxothietan-3-yl)ethynyl]benzoate (788 mg, 94%). ¹H NMR (400 MHz, Chloroform-d) δ 8.07 - 7.96 (m, 2H), 7.53 - 7.46 (m, 2H), 4.61 - 4.52 (m, 2H), 4.20 - 4.10 (m, 2H), 3.94 (s, 3H), 1.89 (s, 3H).

### Steps 2-5. Synthesis of 4-[1-(4-fluoro-3-methyl-phenyl)-4-hydroxy-2-(3-methyl-1,1-dioxothietan-3-yl)indol-3-yl]benzoic acid (174)

Compound **174** was prepared in three steps from **C289** according to the methods used in the preparation of compound **146.** The reaction mixture was concentrated to dryness, triturated with a 9:1 mixture of heptane and EtOAc. The resulting precipitate was filtered and dried to afford the product as an off-white solid. 4-[1-(4-fluoro-3-methyl-phenyl)-4-hydroxy-2-(3-methyl-1,1-dioxo-thietan-3-yl)indol-3-yl]benzoic acid (87 mg, 79%). ¹H NMR (400 MHz, DMSO-*d*₆) δ 13.02 (s, 1H), 9.30 (s, 1H), 7.99 - 7.92 (m, 2H), 7.58 (dd, J = 7.0, 2.6 Hz, 1H), 7.55 - 7.45 (m, 3H), 7.36 (t, J = 9.0 Hz, 1H), 6.92 - 6.84 (m, 1H), 6.41 (dd, J = 7.7, 0.8 Hz, 1H), 6.16 (dd, J = 8.3, 0.8 Hz, 1H), 4.12 (dd, J = 13.9, 5.5 Hz, 2H), 3.29 - 3.21 (m, 2H), 2.32 (d, J = 1.8 Hz, 3H), 1.93 (s, 3H). LCMS *m*/*z* 480.0 [M+H]⁺.

### Compound 175

### 4-[1-(4-fluorophenyl)-4-hydroxy-2-(4-methoxynorbornan-1-yl)indol-3-yl[benzoic acid (175)

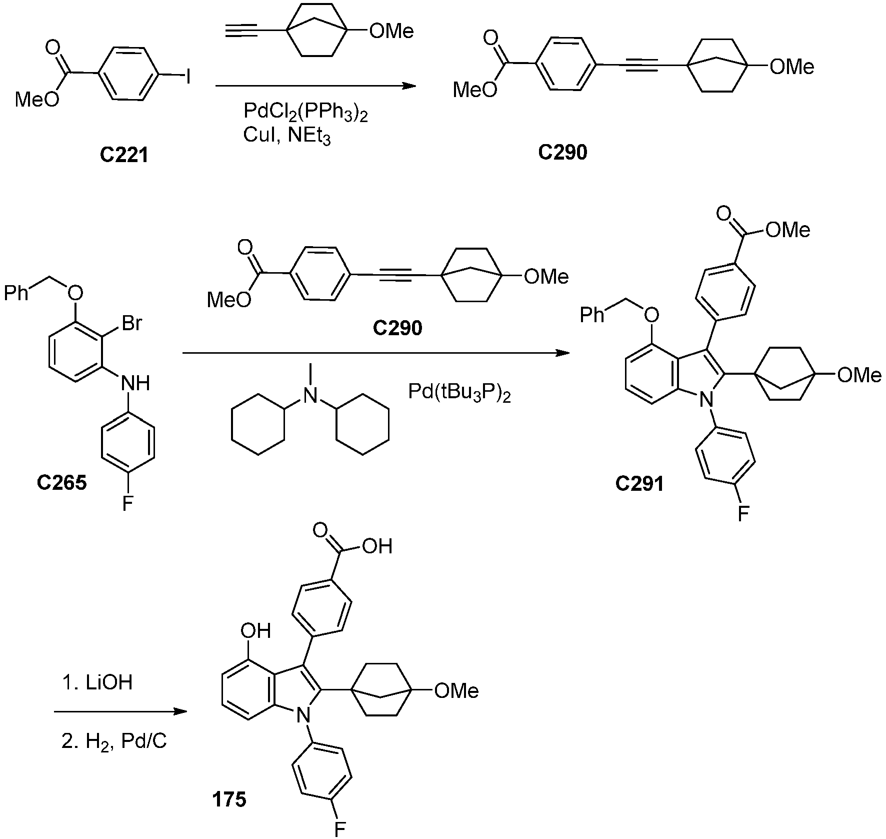

### Synthesis of 4-[1-(4-fluorophenyl)-4-hydroxy-2-(4-methoxynorbornan-1-yl)indol-3-yl]benzoic acid (175)

Compound **175** was prepared from **C265** and **C290** according to the method described in the preparation of compound **146.** Compound **C290** was prepared by Sonagashira coupling from **C221** according to the method described for the preparation of **C222.** 4-[1-(4-fluorophenyl)-4-hydroxy-2-(4-methoxynorbornan-1-yl)indol-3-yl]benzoic acid (171 mg, 83%). ¹H NMR (400 MHz, DMSO-*d*₆) δ 12.88 (s, 1H), 8.94 (s, 1H), 7.93 - 7.87 (m, 2H), 7.55 - 7.46 (m, 4H), 7.46 - 7.38 (m, 2H), 6.79 (t, J = 7.9 Hz, 1H), 6.30 (dd, J = 7.7, 0.8 Hz, 1H), 6.08 (dd, J = 8.2, 0.8 Hz, 1H), 2.93 (s, 3H), 1.88 - 1.78 (m, 1H), 1.47 - 1.30 (m, 6H), 1.12 (s, 2H). LCMS *m*/*z* 472.0 [M+H]⁺.

### Compound 176

### 4-[1-(4-fluorophenyl)-4-hydroxy-2-(1-methoxycarbonyl-3-methyl-azetidin-3-yl)indol-3-yl]benzoic acid (176)

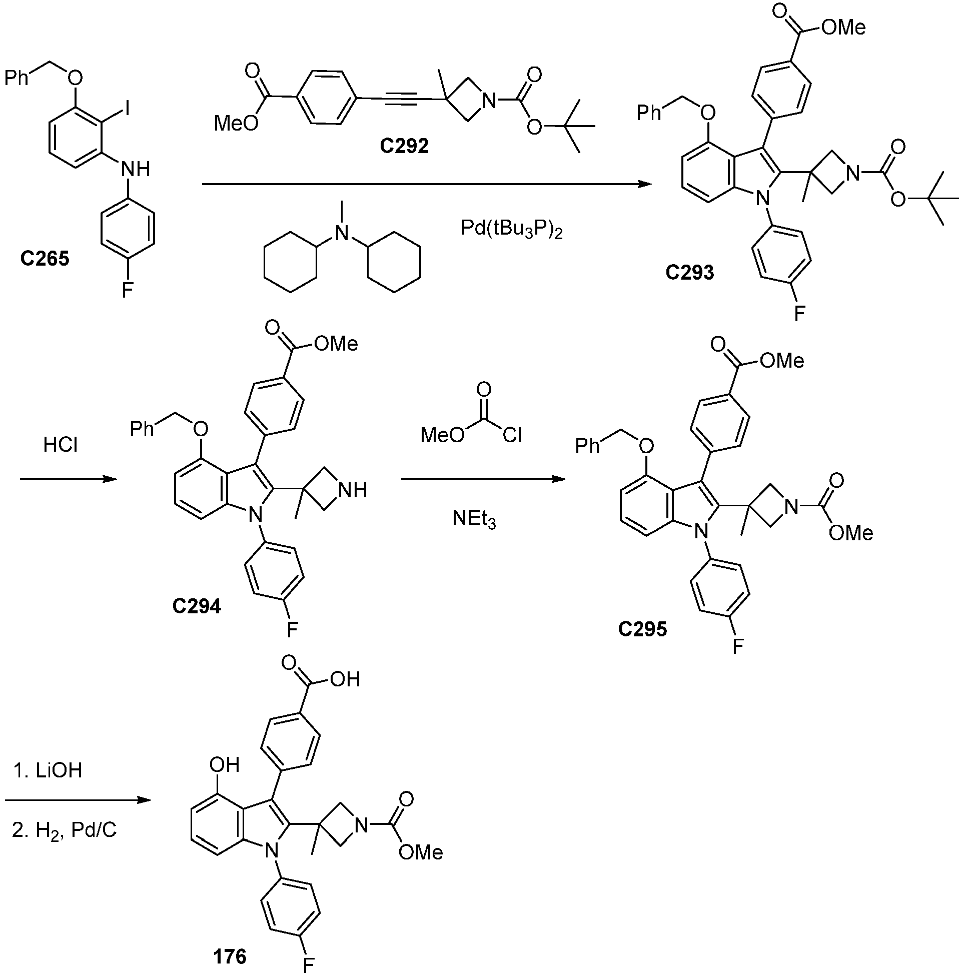

### Step 1. Synthesis of tert-butyl 3-[4-benzyloxy-1-(4-fluorophenyl)-3-(4-methoxycarbonylphenyl)indol-2-yl]-3-methyl-azetidine-1-carboxylate (C293)

Compound **C293** was prepared from **C265** and **C292** using the method described for the preparation of compound **146.** Tert-butyl 3-[4-benzyloxy-1-(4-fluorophenyl)-3-(4-methoxycarbonylphenyl)indol-2-yl]-3-methyl-azetidine-1-carboxylate (245 mg, 67%). ¹H NMR (400 MHz, Chloroform-d) δ 7.99 - 7.94 (m, 2H), 7.54 - 7.50 (m, 2H), 7.48 - 7.43 (m, 2H), 7.28 - 7.13 (m, 5H), 7.11 - 7.05 (m, 1H), 6.87 - 6.82 (m, 2H), 6.63 (dd, J = 7.8, 0.7 Hz, 1H), 6.50 (dd, J = 8.3, 0.6 Hz, 1H), 4.96 (s, 2H), 4.00 (s, 3H), 3.77 (d, J = 8.3 Hz, 2H), 3.02 - 2.96 (m, 2H), 1.84 (s, 3H), 1.33 (s, 9H). LCMS *m*/*z* 620.0 [M+H]⁺.

### Step 2. Synthesis of methyl 4-[4-benzyloxy-1-(4-fluorophenyl)-2-(3-methylazetidin-3-yl)indol-3-yl]benzoate (C294)

A solution of tert-butyl 3-[4-benzyloxy-1-(4-fluorophenyl)-3-(4-methoxycarbonylphenyl)indol-2-yl]-3-methyl-azetidine-1-carboxylate (245 mg, 0.39 mmol) in HCl in dioxane (10 mL of 4 M, 40.0 mmol) was stirred at room temperature for 1 hour. The mixture was concentrated to dryness, dissolved in minimal MeOH, dropped into a solution of cold Et₂O, filtered and dried to afford the product as a white solid. Methyl 4-[4-benzyloxy-1-(4-fluorophenyl)-2-(3-methylazetidin-3-yl)indol-3-yl]benzoate (Hydrochloride salt) (196 mg, 89%). ¹H NMR (400 MHz, DMSO-*d*₆) δ 9.45 (s, 1H), 8.58 (s, 1H), 7.96 - 7.90 (m, 2H), 7.70 - 7.64 (m, 2H), 7.60 - 7.56 (m, 2H), 7.49 (dd, J = 9.7, 7.7 Hz, 2H), 7.22 - 7.16 (m, 1H), 7.13 - 7.07 (m, 3H), 6.85 - 6.80 (m, 2H), 6.73 (d, J = 7.9 Hz, 1H), 6.42 (d, J = 8.2 Hz, 1H), 4.98 (s, 2H), 3.92 (s, 3H), 3.73 - 3.65 (m, 2H), 3.53 - 3.40 (m, 2H), 1.88 (s, 3H). LCMS *m*/*z* 521.0 [M+H]⁺.

### Step 3. Synthesis of methyl 3-[4-benzyloxy-1-(4-fluorophenyl)-3-(4-methoxycarbonylphenyl)indol-2-yl]-3-methyl-azetidine-1-carboxylate (C295)

To a solution of methyl 4-[4-benzyloxy-1-(4-fluorophenyl)-2-(3-methylazetidin-3-yl)indol-3-yl]benzoate (Hydrochloride salt) (98 mg, 0.18 mmol) and Et₃N (30 µL, 0.22 mmol) in dichloromethane (1 mL) was added methylchloroformate (15 µL, 0.19 mmol). The reaction mixture was stirred at room temperature for 30 minutes, then washed with water and concentrated to dryness. The compound was purified via silica gel chromatography (Gradient: 0-40% EtOAc in heptane) to afford methyl 3-[4-benzyloxy-1-(4-fluorophenyl)-3-(4-methoxycarbonylphenyl)indol-2-yl]-3-methyl-azetidine-1-carboxylate (90 mg, 88%). ¹H NMR (400 MHz, Chloroform-d) δ 7.98 - 7.94 (m, 2H), 7.54 - 7.49 (m, 2H), 7.47 - 7.42 (m, 2H), 7.28 (d, J = 2.2 Hz, 2H), 7.22 - 7.13 (m, 3H), 7.11 - 7.06 (m, 1H), 6.87 - 6.82 (m, 2H), 6.63 (dd, J = 7.9, 0.7 Hz, 1H), 6.50 (dd, J = 8.3, 0.7 Hz, 1H), 4.97 (s, 2H), 4.00 (s, 3H), 3.84 (d, J = 8.1 Hz, 2H), 3.54 (s, 3H), 3.10 - 3.02 (m, 2H), 1.86 (s, 3H). LCMS *m*/*z* 579.0 [M+H]⁺.

### Steps 4-5. Synthesis of 4-[1-(4-fluorophenyl)-4-hydroxy-2-(1-methoxycarbonyl-3-methyl-azetidin-3-yl)indol-3-yl]benzoic acid (176)

Compound **176** was prepared from **C295** by ester hydrolysis then hydrogenation according to the methods described for the preparation of compound 1. 4-[1-(4-fluorophenyl)-4-hydroxy-2-(1-methoxycarbonyl-3-methyl-azetidin-3-yl)indol-3-yl]benzoic acid (37 mg, 50%). ¹H NMR (400 MHz, DMSO-*d*₆) δ 12.86 (s, 1H), 9.31 (s, 1H), 7.96 - 7.91 (m, 2H), 7.68 - 7.60 (m, 2H), 7.56 - 7.50 (m, 2H), 7.46 (t, J = 8.7 Hz, 2H), 6.87 (t, J = 7.9 Hz, 1H), 6.43 (d, J = 7.6 Hz, 1H), 6.17 (d, J = 8.1 Hz, 1H), 3.68 (s, 2H), 3.40 (s, 3H), 2.92 (d, J = 7.9 Hz, 2H), 1.84 (s, 3H). LCMS *m*/*z* 475.0 [M+H]⁺.

### Compound 177

### 4-[2-(1,3-dimethylazetidin-3-yl)-1-(4-fluorophenyl)-4-hydroxy-indol-3-yl]benzoic acid (177)

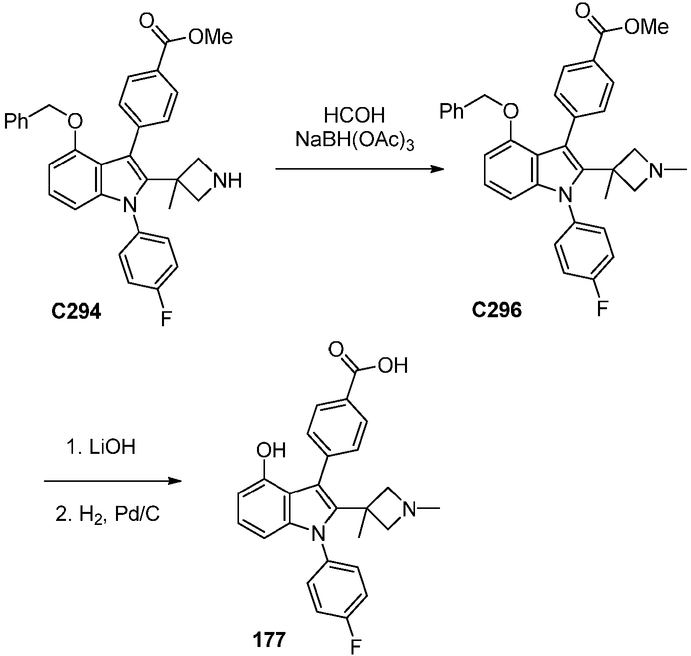

### Step 1. Synthesis of methyl 4-[4-benzyloxy-2-(1, 3-dimethylazetidin-3-yl)-1-(4-fluorophenyl)indol-3-yl]benzoate (C296)

To a solution of Methyl 4-[4-benzyloxy-1-(4-fluorophenyl)-2-(3-methylazetidin-3-yl)indol-3-yl]benzoate (Hydrochloride salt) (98 mg, 0.18 mmol) in dichloromethane (3 mL) was added formaldehyde (100 µL of 37% w/v, 1.23 mmol) in water, followed by AcOH (5 µL, 0.09 mmol) and one spatula of magnesium sulfate. The mixture was stirred at room temperature for 5 minutes and then Na(OAc)₃BH (440 mg, 2.08 mmol) was added. The suspension was stirred at room temperature for 20 minutes. The mixture was washed with water, concentrated to dryness. Purification by silica gel chromatography (Gradient: 0-80% EtOAc in heptane then flushing with MeOH in dichloromethane to afford the product. Methyl 4-[4-benzyloxy-2-(1,3-dimethylazetidin-3-yl)-1-(4-fluorophenyl)indol-3-yl]benzoate (78 mg, 83%). ¹H NMR (400 MHz, Chloroform-d) δ 7.99 - 7.95 (m, 2H), 7.56 - 7.51 (m, 2H), 7.47 - 7.41 (m, 2H), 7.27 - 7.12 (m, 5H), 7.08 (t, J = 8.0 Hz, 1H), 6.87 - 6.82 (m, 2H), 6.63 (dd, J = 7.9, 0.7 Hz, 1H), 6.50 (dd, J = 8.3, 0.6 Hz, 1H), 4.97 (s, 2H), 4.00 (s, 3H), 2.98 - 2.88 (m, 4H), 2.17 (s, 3H), 1.89 (s, 3H). LCMS *m*/*z* 535.0 [M+H]⁺.

### Step 2. Synthesis of 4-[2-(1,3-dimethylazetidin-3-yl)-1-(4-fluorophenyl)-4-hydroxy-indol-3-yl]benzoic acid (177)

Compound **177** was prepared from **C296** by ester hydrolysis and benzyl group removal by hydrogenation, according to the methods described in the preparation of compound **1.** 4-[2-(1,3-dimethylazetidin-3-yl)-1-(4-fluorophenyl)-4-hydroxy-indol-3-yl]benzoic acid (24 mg, 66%). ¹H NMR (400 MHz, DMSO-*d*₆) δ 8.01 - 7.94 (m, 2H), 7.67 - 7.60 (m, 2H), 7.59 - 7.55 (m, 2H), 7.52 - 7.45 (m, 2H), 6.95 - 6.87 (m, 1H), 6.48 (dd, J = 7.7, 0.8 Hz, 1H), 6.22 (dd, J = 8.2, 0.8 Hz, 1H), 3.77 (d, J = 9.1 Hz, 2H), 3.20 - 3.13 (m, 2H), 2.53 (s, 3H), 1.91 (s, 3H). LCMS *m*/*z* 431.0 [M+H]⁺.

### Compound 178

### 4-[2-dimethylphosphoryl-1-(4-fluorophenyl)-4-hydroxy-indol-3-yl]benzoic acid (178)

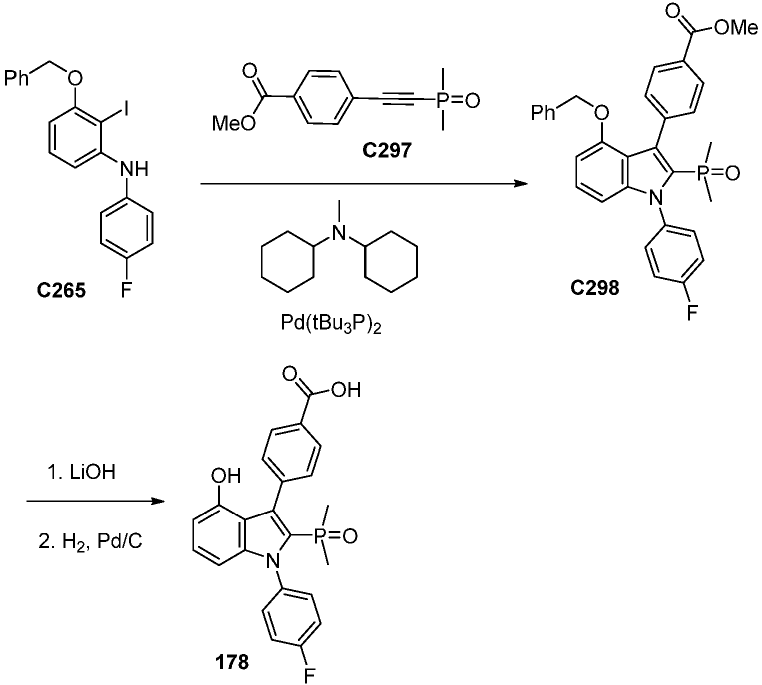

Compound **178** was prepared from **C265** and **C297** using the method described in the preparation of compound **146.** 4-[2-dimethylphosphoryl-1-(4-fluorophenyl)-4-hydroxy-indol-3-yl]benzoic acid (1.5 mg, 10%). ¹H NMR (400 MHz, Methanol-*d*₄) δ 8.48 (s, 2H), 8.05 (d, J = 8.0 Hz, 2H), 7.72 - 7.55 (m, 4H), 7.35 (t, J = 8.6 Hz, 2H), 7.07 (s, 1H), 6.50 (d, J = 8.3 Hz, 1H), 6.43 (d, J = 7.6 Hz, 1H), 1.22 (d, J = 13.7 Hz, 6H). LCMS *m*/*z* 424.0 [M+H]⁺.

### Compound 179

### 4-[2-(2-cyano-1,1-dimethyl-ethyl)-6-fluoro-1-(4-fluorophenyl)-4-hydroxy-indol-3-yl]benzoic acid (179)

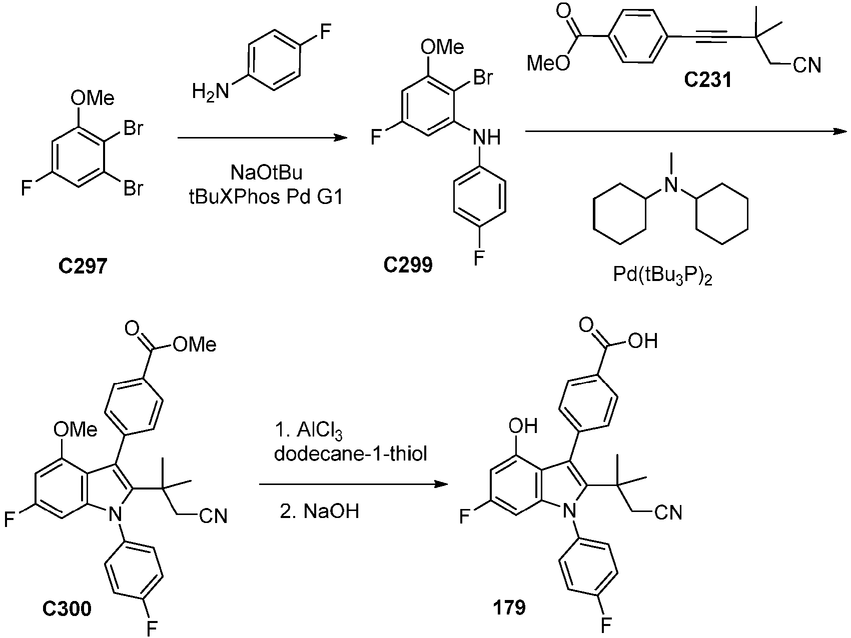

### Synthesis of methyl 4-[2-(2-cyano-1,1-dimethyl-ethyl)-6-fluoro-1-(4-fluorophenyl)-4-methoxy-indol-3-yl]benzoate (C300)

Compound **C300** was prepared from **C299** and **C231** using the described for the preparation of **C232** in the synthesis of compound **147.** Methyl 4-[2-(2-cyano-1,1-dimethylethyl)-6-fluoro-1-(4-fluorophenyl)-4-methoxy-indol-3-yl]benzoate (470 mg, 61%). ¹H NMR (300 MHz, Chloroform-d) δ 8.11 - 8.02 (m, 2H), 7.61 - 7.53 (m, 2H), 7.51 - 7.42 (m, 2H), 7.34 - 7.25 (m, 2H), 6.23 (dd, J = 11.6, 2.1 Hz, 1H), 5.94 (dd, J = 9.4, 2.1 Hz, 1H), 3.99 (s, 3H), 3.48 (s, 3H), 2.35 (s, 2H), 1.24 (s, 6H). LCMS *m*/*z* 475.25 [M+H]⁺

### Synthesis of 4-[2-(2-cyano-1,1-dimethyl-ethyl)-6-fluoro-1(4-fluorophenyl)-4-hydroxy-indol-3-yl]benzoic acid (179)

To a solution of methyl 4-[2-(2-cyano-1,1-dimethyl-ethyl)-6-fluoro-1-(4-fluorophenyl)-4-methoxy-indol-3-yl]benzoate **C300** (200 mg, 0.42 mmol) in dichloromethane (5 mL) was added AlCl₃ (196 mg, 1.47 mmol), followed by dodecane-1-thiol (250 µL, 1.04mmol). The reaction was allowed to stir at room temperature for 1 hour. The mixture was concentrated *in vacuo.* Purification by silica gel chromatography (0-60% EtOAc in heptane) to afford methyl 4-[2-(2-cyano-1,1-dimethyl-ethyl)-6-fluoro-1-(4-fluorophenyl)-4-hydroxy-indol-3-yl]benzoate (140 mg, 72%). THF (2 mL) and MeOH (1 mL) was added to methyl 4-[2-(2-cyano-1,1-dimethyl-ethyl)-6-fluoro-1-(4-fluorophenyl)-4-hydroxy-indol-3-yl]benzoate (140 mg). NaOH (1.3 mL of 1 M, 1.30 mmol) was added to the solution and the reaction was heated at 40 °C for 1 hour. The mixture was concentrated to remove solvent. The pH was adjusted to pH 1 by addition of HCl. The yellow solid that precipitated was filtered and the filter cake was washed with water (x 3), then heptane (x 2) and then minimal TBME. The solid was dried under vacuum to afford the product. 4-[2-(2-cyano-1,1-dimethyl-ethyl)-6-fluoro-1-(4-fluorophenyl)-4-hydroxy-indol-3-yl]benzoic acid (85 mg, 44%). ¹H NMR (400 MHz, DMSO-*d*₆) δ 12.94 (s, 1H), 9.59 (s, 1H), 7.96 - 7.87 (m, 2H), 7.63 - 7.52 (m, 4H), 7.52 - 7.41 (m, 2H), 6.15 (dd, J = 11.4, 2.2 Hz, 1H), 5.74 (dd, J = 9.7, 2.2 Hz, 1H), 2.48 (s, 2H), 1.12 (s, 6H). LCMS *m*/*z* 446.91 [M+H]⁺.

### Compound 180

### 4-(2-(1-cyano-2-methylpropan-2-yl)-6-fluoro-1-(4-fluoro-3-methylphenyl)-4-hydroxy-1H-indol-3-yl)benzoic acid (180)

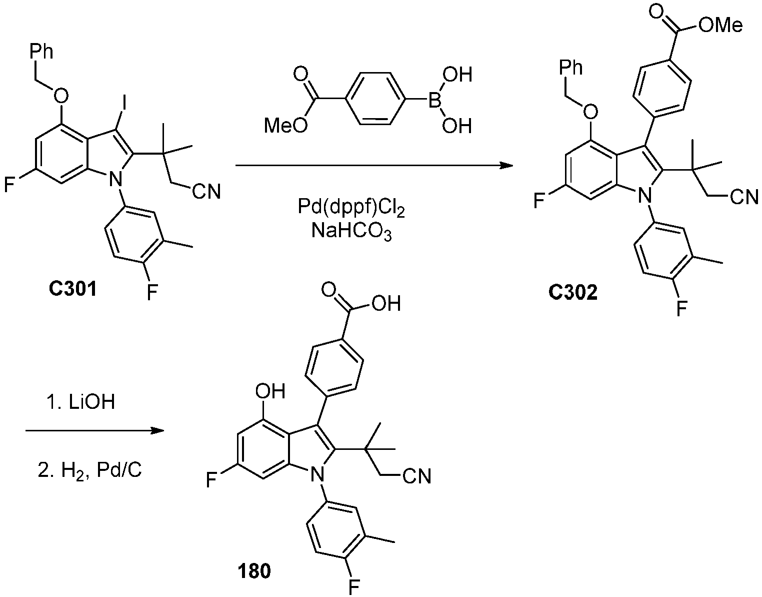

### 4-(2-(1-cyano-2-methylpropan-2-yl)-6-fluoro-1-(4-jluoro-3-methylphenylj-4-hydroxy-1H-indol-3-yl)benzoic acid (180)

Compound **180** was prepared from **C301** as described for compound **153.** 4-[2-(2-cyano-1,1-dimethyl-ethyl)-6-fluoro-1-(4-fluoro-3-methyl-phenyl)-4-hydroxy-indol-3-yl]benzoic acid (13.2 mg, 62%). ¹H NMR (400 MHz, Chloroform-d) δ 8.09 - 8.02 (m, 2H), 7.65 - 7.53 (m, 2H), 7.23 (d, J = 2.5 Hz, 1H), 7.20 - 7.16 (m, 1H), 7.12 (t, J = 8.7 Hz, 1H), 6.15 (dd, J = 10.9, 2.2 Hz, 1H), 5.80 (dd, J = 9.5, 2.2 Hz, 1H), 2.30 (d, J = 1.9 Hz, 3H), 2.28 (s, 2H), 1.14 (s, 6H). LCMS *m*/*z* 461.15 [M+H]⁺.

### Compound 181

### 4-[2-(4-cyanocyclohexyl)-1-(4-fluorophenyl)-4-hydroxy-indol-3-yl]benzoic acid [CIS](181)

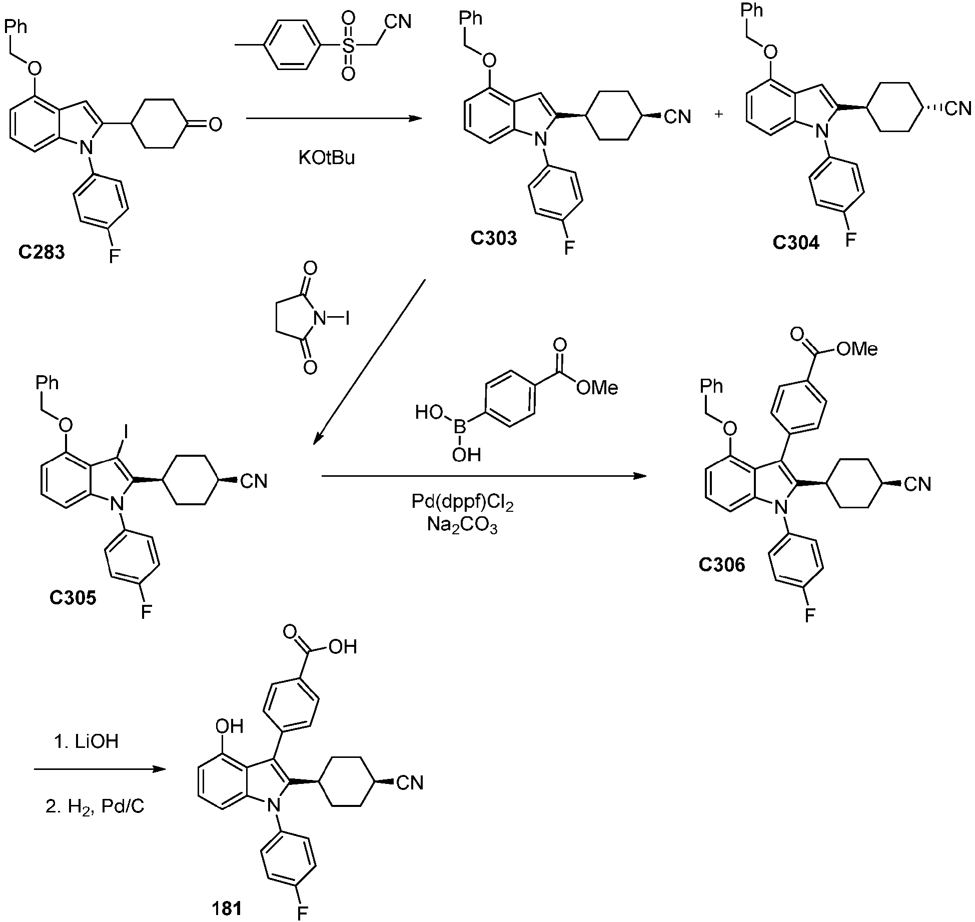

### Synthesis of 4-[4-benzyloxy-1-(4-fluorophenyl)indol-2-yl]cyclohexanecarbonitrile [CIS](C303) and 4-[4-benzyloxy-1-(4-fluorophenyl)indol-2-yl]cyclohexanecarbonitrile [TRANS] (C304)

To an ice-cold mixture of 4-[4-benzyloxy-1-(4-fluorophenyl)indol-2-yl]cyclohexanone **C283** (65 mg, 0.16 mmol), 1-(isocyanomethylsulfonyl)-4-methyl-benzene (39 mg, 0.20 mmol), and ethanol (11 µL, 0.19 mmol) in 1,2-dimethoxyethane (510 µL) was added potassium tert-butoxide (45 mg, 0.40 mmol). The ice bath was removed and the mixture was allowed to stir overnight. The reaction was then diluted with water and extracted with ethyl acetate (x 2). The combined organics were washed with brine, dried over sodium sulfate, and concentrated under reduced pressure. Purification by silica gel chromatography (Column: 12g Combiflash Isco. Gradient: 0-25% EtOAc in heptane) afforded the cis and trans products **C303** and **C304.**

4-[4-benzyloxy-1-(4-fluorophenyl)indol-2-yl]cyclohexanecarbonitrile [CIS] **C303** (24 mg, 73%). ¹H NMR (400 MHz, DMSO-*d*₆) δ 7.55 - 7.50 (m, 2H), 7.50 - 7.38 (m, 6H), 7.38 - 7.32 (m, 1H), 6.94 (t, J = 8.0 Hz, 1H), 6.66 (d, J = 7.8 Hz, 1H), 6.51 (d, J = 8.2 Hz, 1H), 6.44 (d, J = 0.8 Hz, 1H), 5.23 (s, 2H), 2.71 (t, J = 7.6 Hz, 2H), 2.01 (d, J = 11.7 Hz, 2H), 1.84 (d, J = 12.0 Hz, 2H), 1.55 - 1.35 (m, 4H). LCMS *m*/*z* 425.28 [M+H]⁺.

4-[4-benzyloxy-1-(4-fluorophenyl)indol-2-yl]cyclohexanecarbonitrile [TRANS] **C304** (32 mg, 97%). ¹H NMR (400 MHz, DMSO-*d*₆) δ 7.55 - 7.51 (m, 2H), 7.52 - 7.38 (m, 6H), 7.38 - 7.31 (m, 1H), 6.95 (t, J = 8.0 Hz, 1H), 6.67 (d, J = 7.7 Hz, 1H), 6.51 (d, J = 8.2 Hz, 1H), 6.47 (d, J = 0.8 Hz, 1H), 5.24 (s, 2H), 3.12 (s, 1H), 1.85 (t, J = 12.6 Hz, 4H), 1.69 - 1.43 (m, 5H). LCMS *m*/*z* 425.19 [M+H]⁺.

### Synthesis of 4-[2-(4-cyanocyclohexyl)-1-(4-fluorophenyl)-4-hydroxy-indol-3-yl]benzoic acid [CIS](181)

Compound **181** was prepared from **C303** in four steps by iodination, Suzuki coupling, ester hydrolysis, and then benzyl group removal by hydrogenation as described for compound **173.**

Purification by reversed-phase chromatography (Column: C18. Gradient: 30-100% MeCN in water with 0.1% formic acid) afforded the product as a white solid. 4-[2-(4-cyanocyclohexyl)-1-(4-fluorophenyl)-4-hydroxy-indol-3-yl]benzoic acid *[CIS]* (21 mg). ¹H NMR (400 MHz, DMSO-*d*₆) δ 12.90 (s, 1H), 9.14 (s, 1H), 7.97 - 7.88 (m, 2H), 7.59 - 7.35 (m, 6H), 6.83 (t, J = 7.9 Hz, 1H), 6.41 - 6.33 (m, 1H), 6.23 - 6.16 (m, 1H), 2.59 (d, J = 10.9 Hz, 1H), 2.27 (d, J = 3.9 Hz, 1H), 1.88 (d, J = 9.8 Hz, 2H), 1.75 (d, J = 10.1 Hz, 2H), 1.25 (q, J = 9.9, 9.4 Hz, 4H). LCMS *m*/*z* 455.21 [M+H]⁺.

### Compound 182

### 4-[2-(4-cyanocyclohexyl)-1-(4-fluorophenyl)-4-hydroxy-indol-3-yl]benzoic acid [TRANS](182)

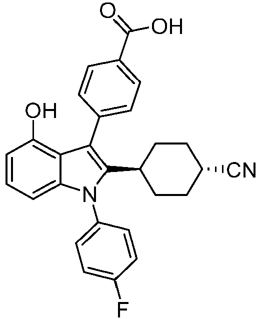

### Synthesis of 4-[2-(4-cyanocyclohexyl)-]-(4-fluorophenyl)-4-hydroxy-indol-3-yl]benzoic acid [TRANS](182)

Compound **182** was prepared from **C304** as described for the preparation of compound **181.**

Purification by reversed-phase chromatography (Column: C18. Gradient: 30-100% MeCN in water with 0.1% formic acid) afforded the product as a white solid. 4-[2-(4-cyanocyclohexyl)-1-(4-fluorophenyl)-4-hydroxy-indol-3-yl]benzoic acid *[TRANS](*15 mg). ¹H NMR (400 MHz, DMSO-*d*₆) δ 12.84 (s, 1H), 9.18 (s, 1H), 7.96 - 7.87 (m, 2H), 7.58 - 7.46 (m, 4H), 7.47 - 7.39 (m, 2H), 6.84 (t, J = 7.9 Hz, 1H), 6.39 (d, J = 7.5 Hz, 1H), 6.19 (d, J = 8.1 Hz, 1H), 2.93 (s, 1H), 2.63 (d, J = 12.5 Hz, 1H), 1.77 - 1.52 (m, 6H), 1.29 (t, J = 13.5 Hz, 2H). LCMS *m*/*z* 455.12 [M+H]⁺.

### Compound 183

### 4-[1-(4-fluorophenyl)-4-hydroxy-2-tetrahydrofuran-3-yl-indol-3-yl]benzoic acid (183)

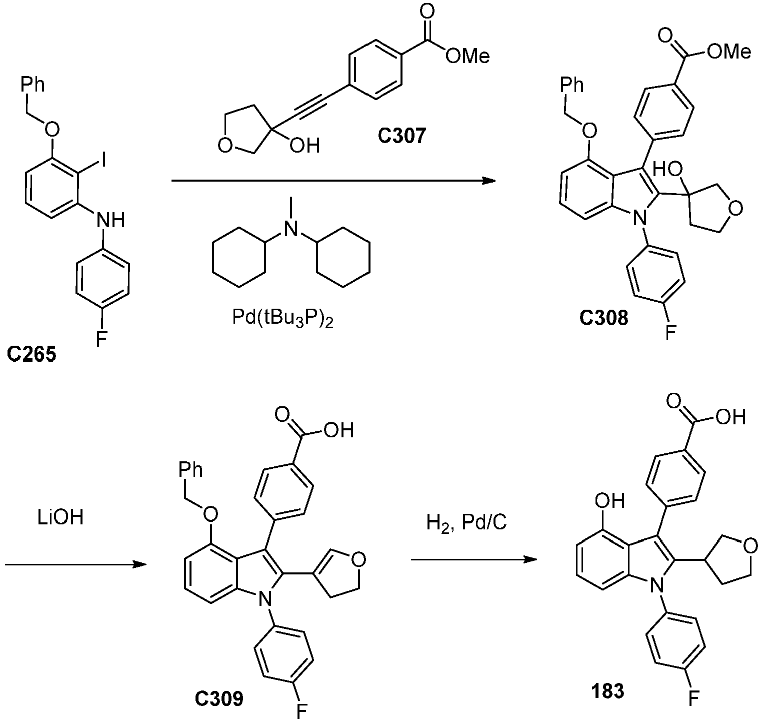

Compound **183** was prepared from C265 and C307 using the method described for the proportion of compound **146.** Compound **307** was prepared from C221 and the 3-ethynyltetrahydrofuran-3-ol using the method described for the preparation of compound C222. 4-[1-(4-fluorophenyl)-4-hydroxy-2-tetrahydrofuran-3-yl-indol-3-yl]benzoic acid (40 mg, 61%) ¹H NMR (400 MHz, DMSO-*d*₆) δ 12.88 (s, 1H), 9.21 (s, 1H), 7.95 - 7.91 (m, 2H), 7.60 - 7.50 (m, 4H), 7.49 - 7.41 (m, 2H), 6.86 (t, J = 7.9 Hz, 1H), 6.40 (d, J = 7.5 Hz, 1H), 6.28 - 6.23 (m, 1H), 3.72 (t, J = 8.4 Hz, 1H), 3.48 (dt, J = 13.5, 7.9 Hz, 2H), 3.42 - 3.34 (m, 1H), 3.26 (td, J = 8.5, 4.9 Hz, 1H), 1.95 - 1.77 (m, 2H). LCMS *m*/*z* 418.0 [M+H]⁺

### Compound 184

### 4-[1-(4-fluorophenyl)-4-hydroxy-2-(4-methyltetrahydropyran-4-yl)indol-3-yl]benzoic acid (184)

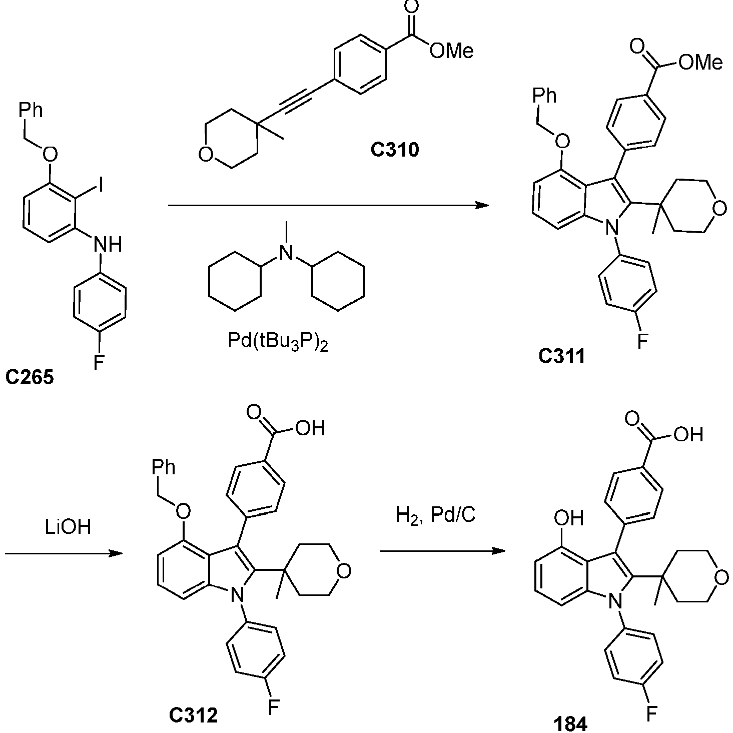

Compound **184** was prepared from **C265** and C310 using the method described for the proportion of compound **146.** Compound 310 was prepared from **C221** and the 4-ethynyl-4-methyl-tetrahydropyran using the method described for the preparation of compound **C222.** 4-[1-(4-fluorophenyl)-4-hydroxy-2-(4-methyltetrahydropyran-4-yl)indol-3-yl]benzoic acid (6 mg, 8%). ¹H NMR (400 MHz, DMSO-*d*₆) δ 12.89 (s, 1H), 8.94 (s, 1H), 7.91 (s, 2H), 7.55 - 7.50 (m, 4H), 7.44 (d, J = 8.5 Hz, 2H), 6.79 (t, J = 8.0 Hz, 1H), 6.29 (d, J = 7.6 Hz, 1H), 6.03 (d, J = 8.3 Hz, 1H), 3.28 (s, 4H), 1.61 - 1.52 (m, 2H), 1.42 (s, 3H), 1.06 (s, 2H). LCMS *m*/*z* 446.0 [M+H]⁺.

### Compound 185

### 4-[2-(3-ethyloxetan-3-yl)-1-(4-fluorophenyl)-4-hydroxy-indol-3-yl]benzoic acid (185)

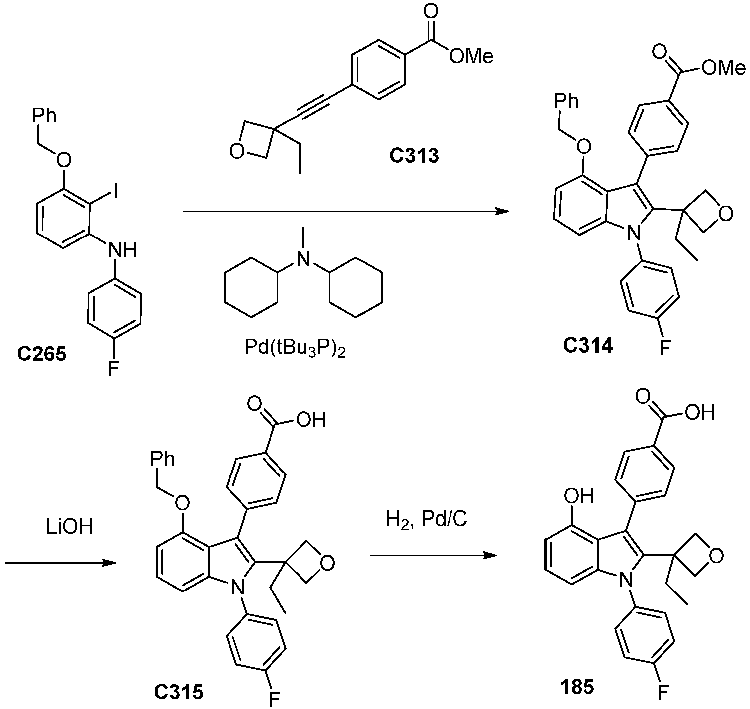

Compound **185** was prepared from **C265** and **C314** using the method described for the preparation of compound 146. Compound 314 was prepared by C221 and 3-ethyl-3-ethynyl-oxetane using the method described for the preparation of C222. 4-[2-(3-ethyloxetan-3-yl)-1-(4-fluorophenyl)-4-hydroxy-indol-3-yl]benzoic acid (70 mg, 73%). ¹H NMR (400 MHz, DMSO-*d*₆) δ 12.91 (s, 1H), 9.25 (s, 1H), 7.94 - 7.89 (m, 2H), 7.57 - 7.51 (m, 2H), 7.51 - 7.46 (m, 2H), 7.45 - 7.38 (m, 2H), 6.88 - 6.83 (m, 1H), 6.41 (dd, J = 7.7, 0.8 Hz, 1H), 6.13 (dd, J = 8.2, 0.8 Hz, 1H), 4.46 (d, J = 5.9 Hz, 2H), 3.60 (d, J = 6.0 Hz, 2H), 2.05 - 1.95 (m, 2H), 1.05 (t, J = 7.4 Hz, 3H). LCMS *m*/*z* 432.0 [M+H]⁺.

### Compound 186

### 4-[1-(4-fluoro-3-methyl-phenyl)-4-hydroxy-2-(3-methoxy-1-methyl-cyclobutyl)indol-3-yl]benzoic acid (186)

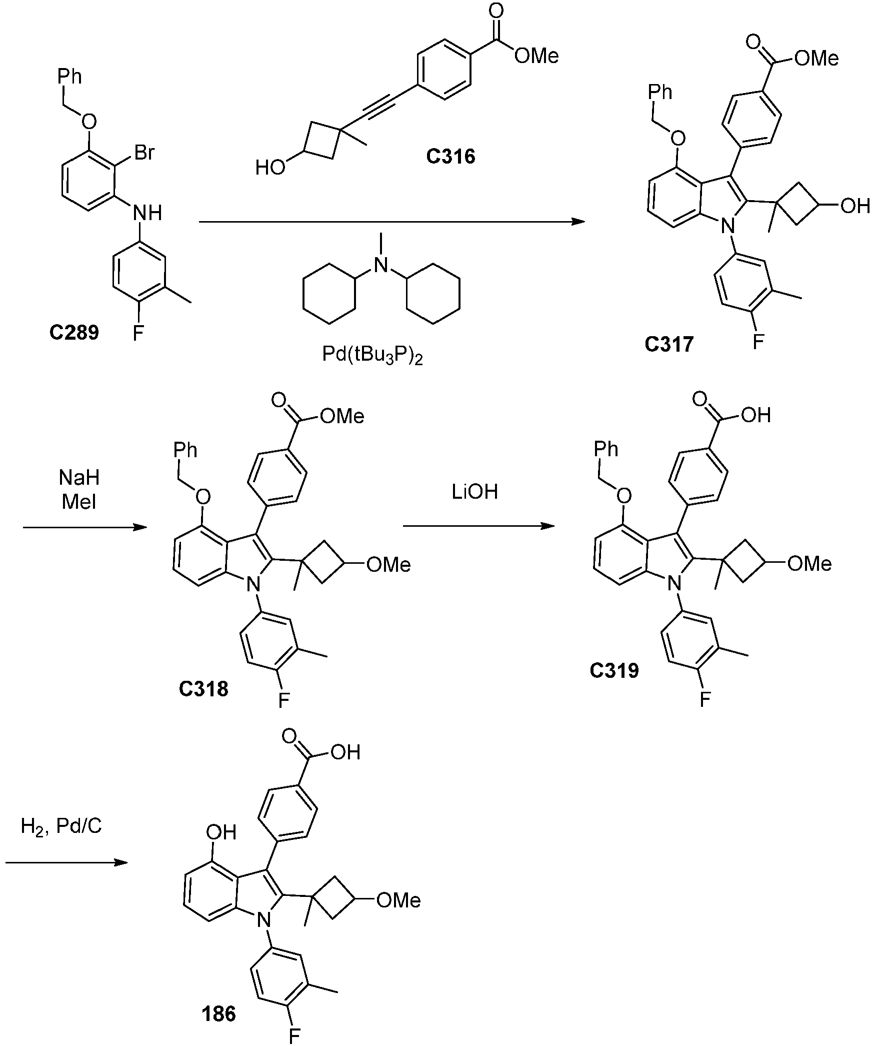

Compound **186** was prepared from **C289** and **C316** using the method described for the preparation of compound 146. 4-[1-(4-fluoro-3-methyl-phenyl)-4-hydroxy-2-(3-methoxy-1-methyl-cyclobutyl)indol-3-yl]benzoic acid (10 mg, 47%). ¹H NMR (400 MHz, Chloroform-d) δ 7.99 (d, J = 7.6 Hz, 2H), 7.51 (dd, J = 7.7, 2.4 Hz, 2H), 7.22 - 7.11 (m, 2H), 7.06 (t, J = 8.8 Hz, 1H), 6.85 (t, J = 8.0 Hz, 1H), 6.39 (d, J = 7.6 Hz, 1H), 6.28 (d, J = 8.2 Hz, 1H), 3.29 (d, J = 3.2 Hz, 1H), 2.99 - 2.82 (m, 3H), 2.26 (d, J = 2.3 Hz, 3H), 1.76 (q, J = 8.6, 7.1 Hz, 2H), 1.60 (dd, J = 11.3, 6.2 Hz, 2H), 1.53 (d, J = 2.8 Hz, 3H). LCMS *m*/*z* 460.16 [M+H]⁺

### Compound 187

### 4-[1-(4-fluorophenyl)-4-hydroxy-2-(3-hydroxycyclobutyl)indol-3-yl]benzoic acid [CIS](187)

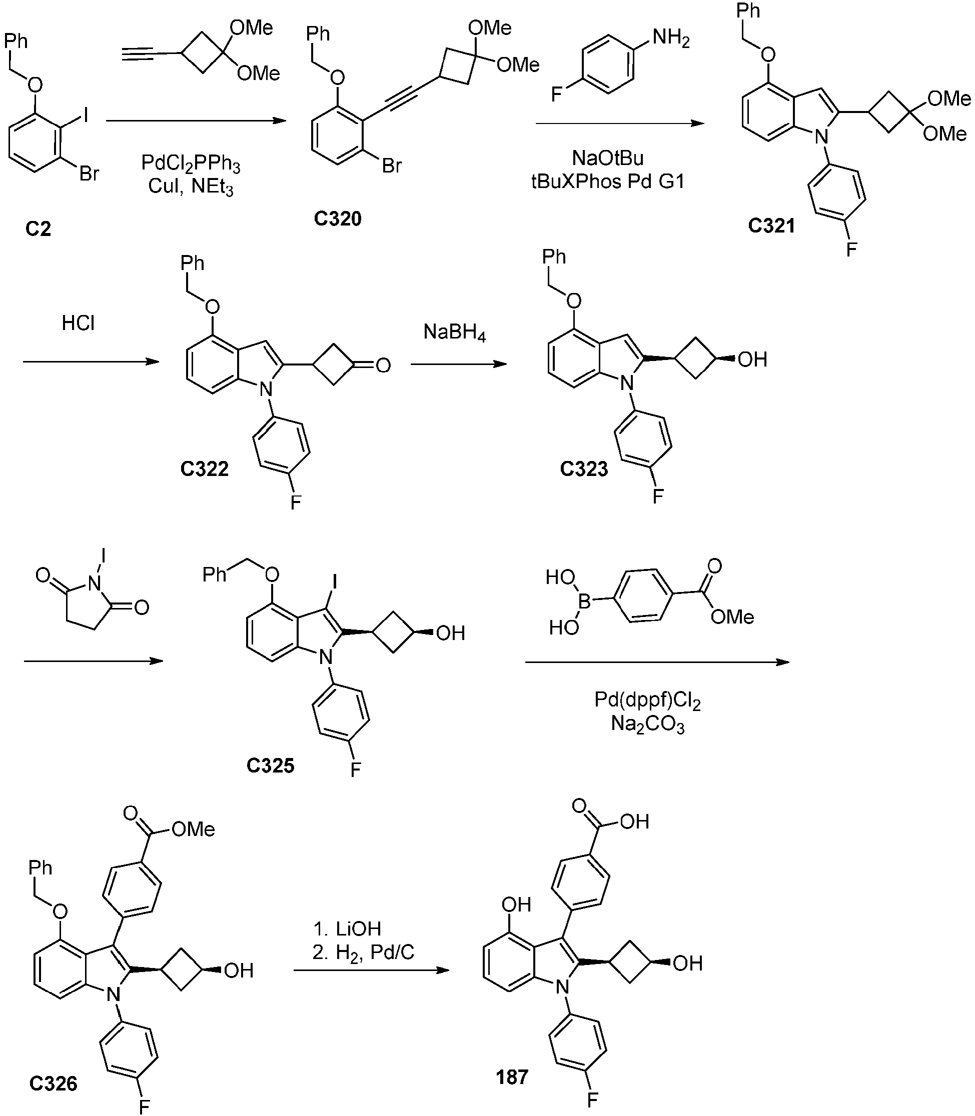

Compound **187** was prepared from C2 and 3-ethynyl-1,1-dimethoxy-cyclobutane using the method described for the preparation of compound 173. C323 was prepared by reduction of C322 with sodium borohydride in step 4. Purification by reversed-phase chromatography (Column: C18. Gradient: 20-100% MeCN in water with 0.1% formic acid) afforded the product as a white solid. 4-[1-(4-fluorophenyl)-4-hydroxy-2-(3-hydroxycyclobutyl)indol-3-yl]benzoic acid (4.7 mg, 55%). ¹H NMR (400 MHz, DMSO-*d*₆) δ 13.00-12.77 (s, 1H), 9.25 (s, 1H), 7.93 - 7.82 (m, 2H), 7.59 - 7.45 (m, 4H), 7.46 - 7.37 (m, 2H), 6.85 (t, J = 7.9 Hz, 1H), 6.42 (dd, J = 7.9, 3.4 Hz, 2H), 4.73 (d, J = 6.3 Hz, 1H), 3.61 (d, J = 5.3 Hz, 1H), 3.25 - 3.18 (m, 1H), 1.85 - 1.73 (m, 2H), 1.32 - 1.22 (m, 2H). LCMS *m*/*z 418.17* [M+1]⁺.

### Compound 188

### 4-[1-(4-fluorophenyl)-4-hydroxy-2-(3-hydroxycyclobutyl)indol-3-yl]benzoic acid (188)

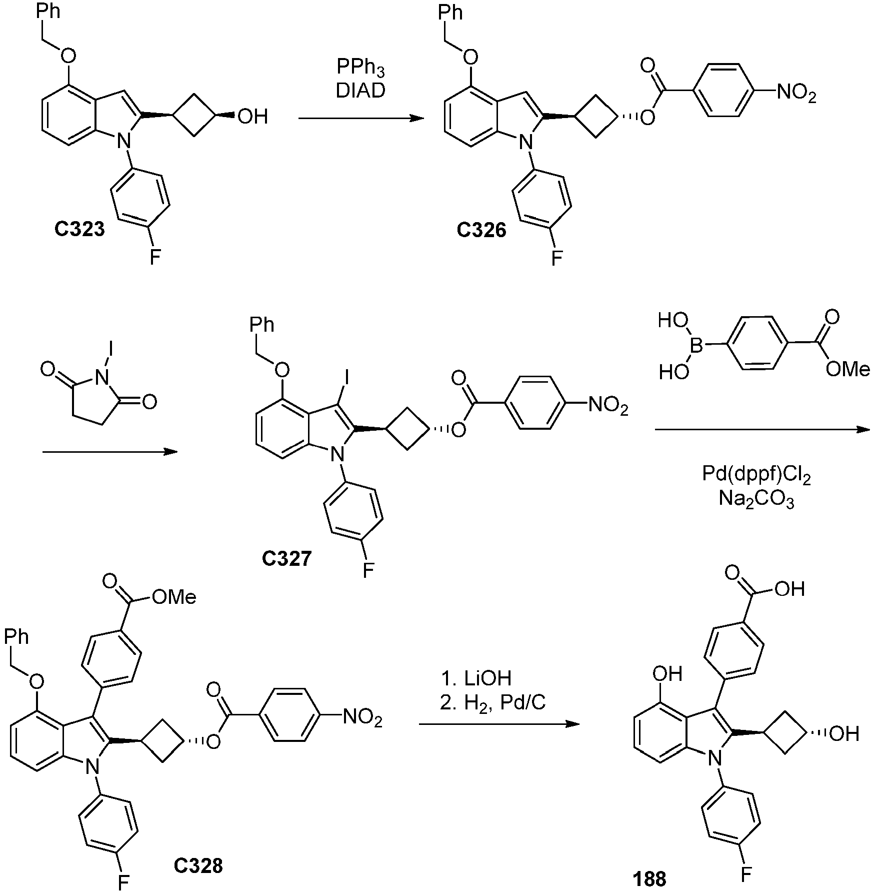

### Step 1. Synthesis of [3-[4-benzyloxy-1-(4-fluorophenyl)indol-2-yl]cyclobutyl] 4-nitrobenzoate C326

To a solution of 3-[4-benzyloxy-1-(4-fluorophenyl)indol-2-yl]cyclobutanol **C323** (120 mg, 0.31 mmol), 4-nitrobenzoic acid (62 mg, 0.37 mmol), triphenyl phosphine (100 mg, 0.38 mmol) in tetrahydrofuran (2 mL) was added DIAD (72 µL, 0.37 mmol). The reaction was allowed to stir overnight. The mixture was concentrated under reduced pressure followed by silica gel chromatography (Gradient: 0-30% EtOAc in heptane) which to afford the product. [3-[4-benzyloxy-1-(4-fluorophenyl)indol-2-yl]cyclobutyl] 4-nitrobenzoate (120 mg, 71%). ¹H NMR (400 MHz, DMSO-*d*₆) δ 8.38 - 8.31 (m, 2H), 8.22 - 8.15 (m, 2H), 7.58 - 7.52 (m, 2H), 7.49 - 7.38 (m, 6H), 7.39 - 7.32 (m, 1H), 6.98 (t, J = 8.0 Hz, 1H), 6.72 - 6.67 (m, 2H), 6.57 (d, J = 8.2 Hz, 1H), 5.40 - 5.31 (m, 1H), 5.27 (s, 2H), 3.68 - 3.59 (m, 1H), 2.71 - 2.63 (m, 2H), 2.48 - 2.41 (m, 2H). LCMS *m*/*z* 537.15 [M+1]+.

### Step 2-4.Synthesis of 4-[1-(4-fluorophenyl)-4-hydroxy-2-(3-hydroxycyclobutyl)indol-3-yl]benzoic acid [trans](188)

Compound **188** was prepared from **C326** by iodination, Suzuki coupling, ester hydrolysis and hydrogenation. Purification by reversed-phase chromatography (Column: C18. Gradient: 20-100% MeCN in water with 0.1% formic acid) afforded the product. 4-[1-(4-fluorophenyl)-4-hydroxy-2-(3-hydroxycyclobutyl)indol-3-yl]benzoic acid (16.3 mg, 36%). ¹H NMR (400 MHz, CDCl₃ / Methanol-*d*₄) δ 8.08 - 7.99 (m, 2H), 7.65 - 7.56 (m, 2H), 7.45 - 7.36 (m, 2H), 7.30 - 7.18 (m, 2H), 6.94 (t, J = 8.0 Hz, 1H), 6.56 (dd, J = 8.2, 0.8 Hz, 1H), 6.48 (dd, J = 7.7, 0.8 Hz, 1H), 4.14 - 4.05 (m, 1H), 3.94 - 3.84 (m, 1H), 2.05 - 1.96 (m, 2H), 1.82 - 1.66 (m, 2H). LCMS *m*/*z* 418.17 [M+1]+.

### Compound 189

### 4-[1-(4-fluorophenyl)-2-(4-hydroxycyclohexyl)-4-methoxy-indol-3-yl]benzoic acid [CIS] (189)

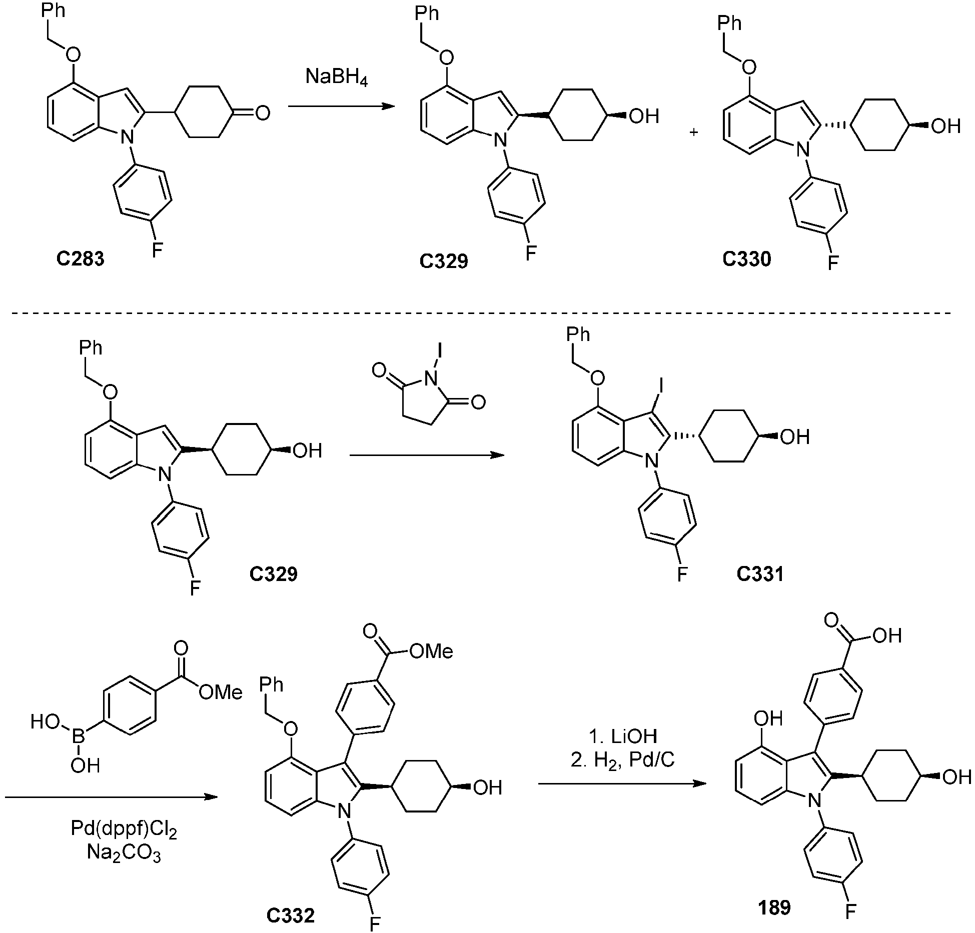

Compound **189** was prepared from **C283** using the method described for the preparation of compound **187** from C325. Purification by reversed-phase chromatography (Column: C18. Gradient: 20-100% MeCN in water with 0.1% formic acid) afforded the product. NMR taken in CDCl₃ as well as DMSO-*d*₆ to show methine is under DMSO peak. 4-[1-(4-fluorophenyl)-4-hydroxy-2-(4-hydroxycyclohexyl)indol-3-yl]benzoic acid (8.8 mg, 54%). ¹H NMR (400 MHz, DMSO-*d*₆) δ 12.86 (s, 1H), 9.11 (s, 1H), 7.96 - 7.86 (m, 2H), 7.54 - 7.46 (m, 4H), 7.46 - 7.39 (m, 2H), 6.86 - 6.76 (m, 1H), 6.37 (dd, J = 7.7, 0.8 Hz, 1H), 6.18 (dd, J = 8.2, 0.8 Hz, 1H), 3.95 (d, J = 2.2 Hz, 1H), 3.64 (s, 1H), 2.57 (d, J = 12.5 Hz, 1H), 1.72 (q, J = 13.8 Hz, 2H), 1.50 (d, J = 13.5 Hz, 2H), 1.36 (d, J = 12.5 Hz, 2H), 1.10 (t, J = 13.3 Hz, 2H). LCMS *m*/*z* 446.2 [M+1]⁺.

### Compound 190

### 4-[1-(4-fluorophenyl)-4-hydroxy-2-(4-hydroxycyclohexyl)indol-3-yl]benzoic acid [TRANS](190)

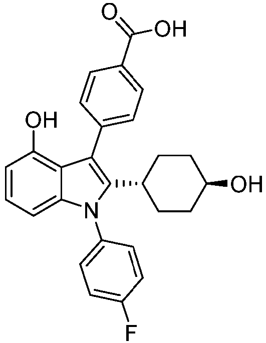

Compound **190** was prepared from **C329** as described for the preparation of compound **189.** Purification by reversed-phase chromatography (Column: C18. Gradient: 20-100% MeCN in water with 0.1% formic acid) afforded the product. NMR taken in CDCl₃ as well as DMSO-*d*₆ to show methine is under DMSO peak. 4-[1-(4-fluorophenyl)-4-hydroxy-2-(4-hydroxycyclohexyl)indol-3-yl]benzoic acid (14 mg, 75%). ¹H NMR (400 MHz, DMSO-*d*₆) δ 12.86 (s, 1H), 9.13 (s, 1H), 7.97 - 7.89 (m, 2H), 7.57 - 7.41 (m, 6H), 6.82 (t, J = 7.9 Hz, 1H), 6.37 (dd, J = 7.7, 0.8 Hz, 1H), 6.20 (dd, J = 8.2, 0.8 Hz, 1H), 4.39 (d, J = 4.8 Hz, 1H), 2.93 (s, 1H), 1.66 (d, J = 11.6 Hz, 4H), 1.28 (q, J = 12.5 Hz, 2H), 0.86 (q, J = 11.6 Hz, 2H). LCMS *m*/*z* 446.11 [M+1]⁺.

### Compound 191

### 4-[6-fluoro-1-(4-fluoro-3-methyl-phenyl)-4-hydroxy-2-(3-hydroxy-1-methyl-cyclobutyl)indol-3-yl]benzoic acid (191)

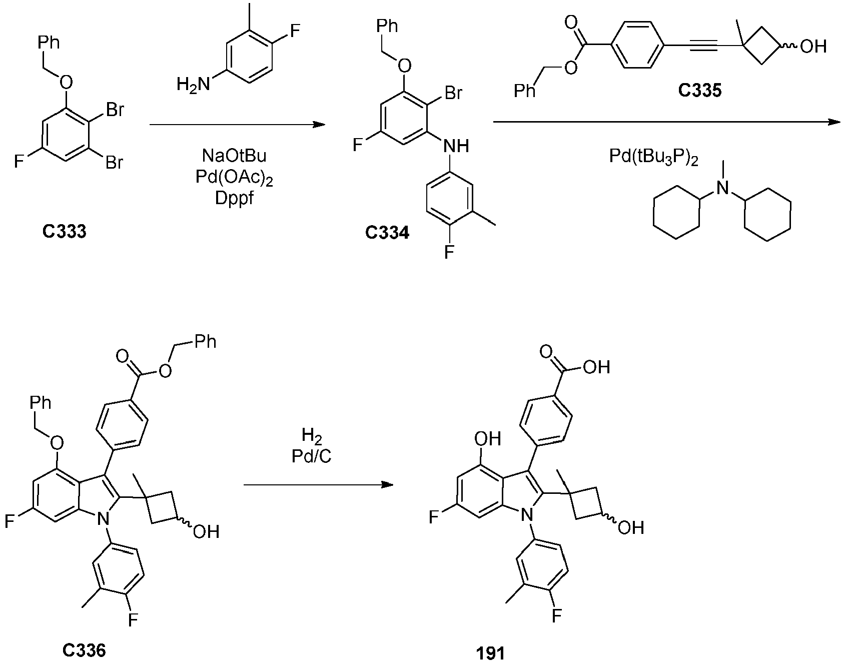

### Step 1. 3-benzyloxy-2-bromo-5-fluoro-N-(4-fluoro-3-methyl-phenyl)aniline (C334)

A mixture of 1-benzyloxy-2,3-dibromo-5-fluoro-benzene (17 g, 47.22 mmol), 4-fluoro-3-methyl-aniline (8.86 g, 70.80 mmol), ferrous cyclopenta-1,4-dien-1-yl(diphenyl)phosphane (1.31 g, 2.363 mmol) and NaOtBu (7.3 g, 75.96 mmol) in 1,4-dioxane (200 mL) were purged with nitrogen for 10 minutes. Pd(OAc)₂ (530 mg, 2.361 mmol) was added and the mixture purged with nitrogen for 10 minutes, the heated to 80 °C overnight. The mixture was cooled and sat. NH₄Cl (100 mL), EtOAc (150 mL) and HCl (10 mL of 6 M, 60.00 mmol), pH = 2 was added. The two layers were separated, and the aqueous layer was back washed with EtOAc (100 mL). Combined organic layers were washed with brine (100 mL), dried over Na₂SO₄, and concentrated. Purification by silica gel chromatography (0-50% dichloromethane in heptane) afforded the product as a yellow solid. Heptane (80 mL) was added, and the mixture stirred for 5 minutes. The mixture was filtered, washed with heptane, and dried under vacuum to afford the product as a white solid. 3-benzyloxy-2-bromo-5-fluoro-N-(4-fluoro-3-methyl-phenyl)aniline (15.5 g, 81%). LCMS *m*/*z* 403.87 [M+H]⁺

### Step 2. benzyl 4-[4-benzyloxy-6-fluoro-1-(4-fluoro-3-methyl-phenyl)-2-(3-hydroxy-1-methyl-cyclobutyl)indol-3-yl]benzoate (C336)

A mixture of benzyl 4-[2-(3-hydroxy-1-methyl-cyclobutyl)ethynyl]benzoate ***C336*** (110 mg, 0.3433 mmol), 3-benzyloxy-2-bromo-5-fluoro-N-(4-fluoro-3-methyl-phenyl)aniline (145 mg, 0.3464 mmol) and N-cyclohexyl-N-methyl-cyclohexanamine (400 µL, 1.867 mmol) was purged with nitrogen. 1,4-dioxane (2 mL) was added and the mixture placed under vacuum and flushed with nitrogen. Palladium tri-tbutylphosphane (12 mg, 0.024 mmol) (white crystal solid) was added. The reaction vessel was sealed, and the mixture was heated to 90 °C over 12 hours. The temperature was raised to 110 °C and reacted for over the weekend. The mixture was concentrated to dryness. Purification by silica gel chromatography (Gradient: 0-60% EtOAc in heptane) to afford the product. Benzyl 4-[4-benzyloxy-6-fluoro-1-(4-fluoro-3-methyl-phenyl)-2-(3-hydroxy-1-methyl-cyclobutyl)indol-3-yl]benzoate (126 mg, 55%). LCMS *m*/*z* 644.47 [M+H]⁺

### Step 3. 4-[6-fluoro-1-(4-fluoro-3-methyl-phenyl)-4-hydroxy-2-(3-hydroxy-1-methyl-cyclobutyl)indol-3-yl]benzoic acid (191)

To a mixture of benzyl 4-[4-benzyloxy-6-fluoro-1-(4-fluoro-3-methyl-phenyl)-2-(3-hydroxy-1-methyl-cyclobutyl)indol-3-yl]benzoate (25.5 mg, 0.03961 mmol) in THF (500 µL) and ethanol (500 µL) was added Pd on carbon (5 mg, 0.005 mmol). The mixture was subjected to hydrogenation with H₂ gas (10 mg, 4.96 mmol) at balloon pressure for 2 hours. The reaction was filtered and concentrated. The residue was purified by reverse phase HPLC (C18 column. Gradient: 0-70% MeCN in water (0.1% FA as modifier) to give the product as a white solid.

4-[6-fluoro-1-(4-fluoro-3-methyl-phenyl)-4-hydroxy-2-(3-hydroxy-1-methylcyclobutyl)indol-3-yl]benzoic acid (15.6 mg, 85%). ¹H NMR (400 MHz, Chloroform-d) δ 7.99 (dd, *J* = 8.2, 2.5 Hz, 2H), 7.50 (dd, *J* = 8.2, 4.6 Hz, 2H), 7.17 (ddd, *J* = 15.1, 5.8, 2.3 Hz, 2H), 7.09 (t, *J* = 8.7 Hz, 1H), 6.25 - 6.17 (m, 1H), 5.98 (dd, *J* = 9.6, 2.3 Hz, 1H), 4.01 - 3.91 (m, 1H), 2.30 (d, *J =* 2.1 Hz, 3H), 1.79 (d, *J =* 8.2 Hz, 3H), 1.71 - 1.58 (m, 2H), 1.52 (s, 2H). LCMS *m*/*z* 464.25 [M+H]⁺

### Compound 192

### 4-[2-(1,1-dimethyl-2-methylsulfonyl-ethyl)-1-(4-fluorophenyl)-4-hydroxy-indol-3-yl]benzoic acid (192)

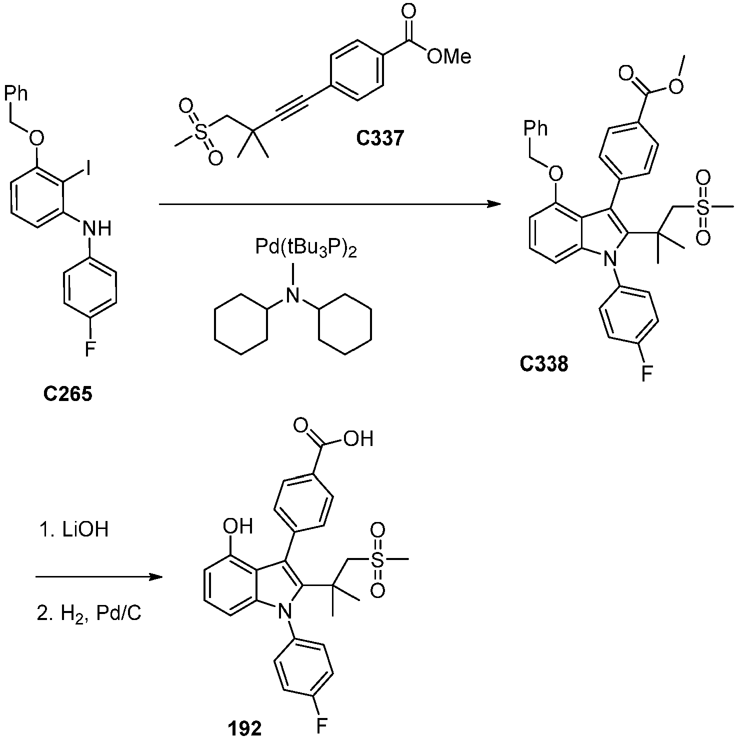

Compound **192** was prepared from **C265** and **C337** according to the method described for the preparation of compound **147.** 4-[2-(1,1-dimethyl-2-methylsulfonyl-ethyl)-1-(4-fluorophenyl)-4-hydroxy-indol-3-yl]benzoic acid (43 mg, 77%). ¹H NMR (400 MHz, DMSO-*d*₆) δ 12.66 (s, 1H), 8.88 (s, 1H), 7.94 - 7.86 (m, 2H), 7.65 - 7.57 (m, 2H), 7.57 - 7.51 (m, 2H), 7.51 - 7.42 (m, 2H), 6.82 - 6.74 (m, 1H), 6.26 (dd, J = 7.7, 0.8 Hz, 1H), 5.93 (dd, J = 8.2, 0.8 Hz, 1H), 3.34 (s, 2H), 2.83 (s, 3H), 1.21 - 1.18 (m, 6H). LCMS *m*/*z* 482.0 [M+H]⁺

### Compound 193

### 4-[1-(4-fluoro-3-methyl-phenyl)-4-hydroxy-2-(3-hydroxy-1-methyl-cyclobutyljindol-3-yl]benzoic acid (193)

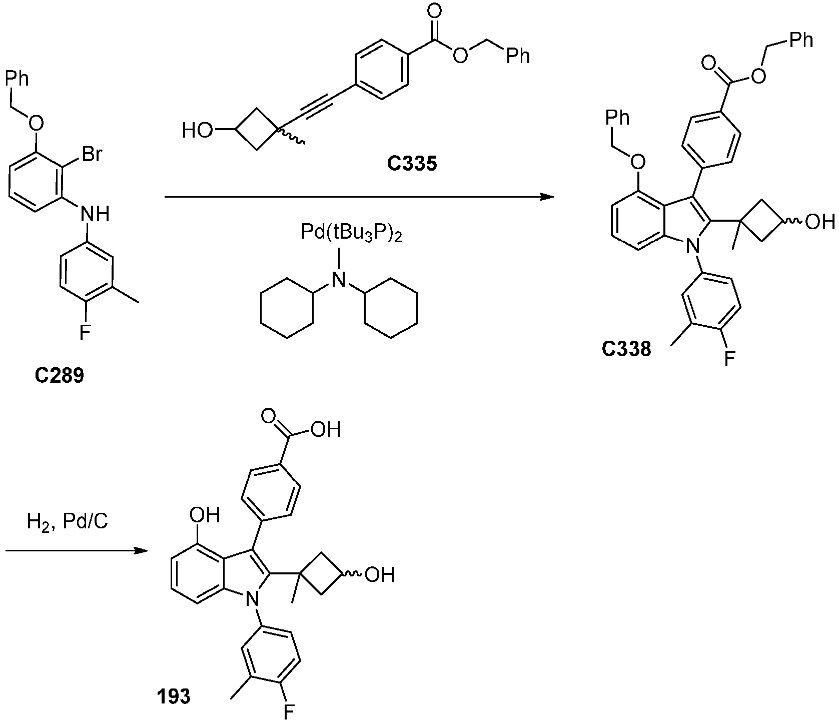

Compound 193 was prepared from C289 and C335 using the method described for the preparation of compound 191. 4-[1-(4-fluoro-3-methyl-phenyl)-4-hydroxy-2-(3-hydroxy-1-methyl-cyclobutyl)indol-3-yl]benzoic acid (13.2 mg, 74%). ¹H NMR (400 MHz, Chloroform-d) δ 8.21 (d, J = 7.7 Hz, 2H), 7.67 (d, J = 7.8 Hz, 2H), 7.24 (s, 2H), 7.17 (t, J = 8.8 Hz, 1H), 6.99 (t, J = 8.0 Hz, 1H), 6.55 (d, J = 7.5 Hz, 1H), 6.44 (d, J = 8.1 Hz, 1H), 4.09 (s, 1H), 2.37 (s, 5H), 1.91 (s, 3H), 1.42 (d, J = 12.5 Hz, 2H). LCMS *m*/*z* 446.21 [M+H]⁺.

### Compound 194

### 4-[1-(4-fluorophenyl)-4-hydroxy-2-(3-methyloxetan-3-yl)indol-3-yl]benzoic acid (194)

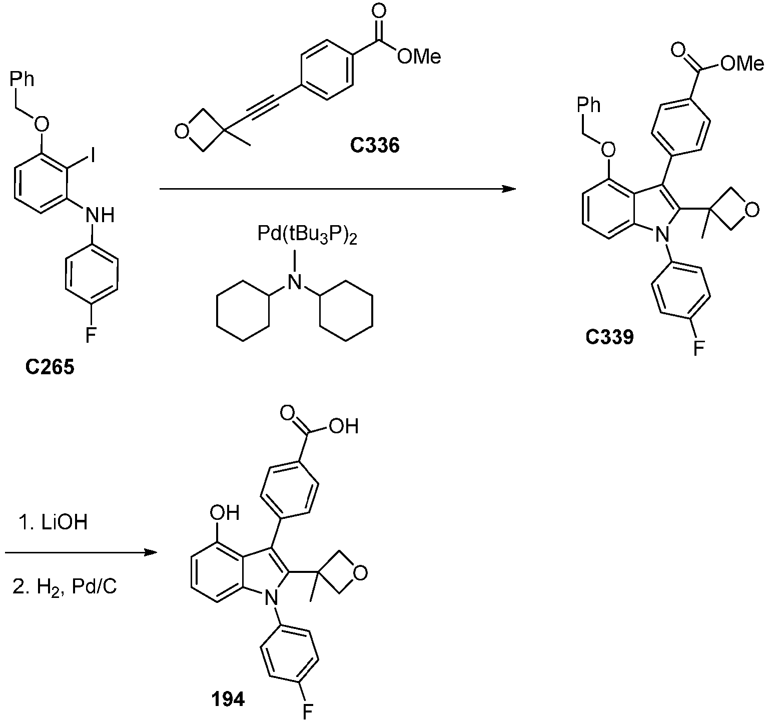

Compound **194** was prepared from **C265** and **C336** using the method described for the preparation of compound **147.** Methyl 4-[4-benzyloxy-1-(4-fluorophenyl)-2-(3-methyloxetan-3-yl)indol-3-yl]benzoate (25 mg, 17%). ¹H NMR (400 MHz, Chloroform-d) δ 7.98 - 7.93 (m, 2H), 7.53 - 7.49 (m, 2H), 7.45 - 7.41 (m, 2H), 7.28 - 7.13 (m, 5H), 7.09 (dd, J = 8.3, 7.8 Hz, 1H), 6.86 (ddt, J = 6.8, 1.8, 0.8 Hz, 2H), 6.64 (dd, J = 7.9, 0.8 Hz, 1H), 6.51 (dd, J = 8.3, 0.7 Hz, 1H), 4.98 (s, 2H), 4.63 - 4.55 (m, 2H), 3.99 (s, 3H), 3.58 - 3.54 (m, 2H), 1.96 (s, 3H). LCMS *m*/*z* 522.0 [M+H]⁺

### Compound 195

### 4-[2-(1-acetylpyrrolidin-3-yl)-1-(4-fluorophenyl)-4-hydroxy-indol-3-yl]benzoic acid (195)

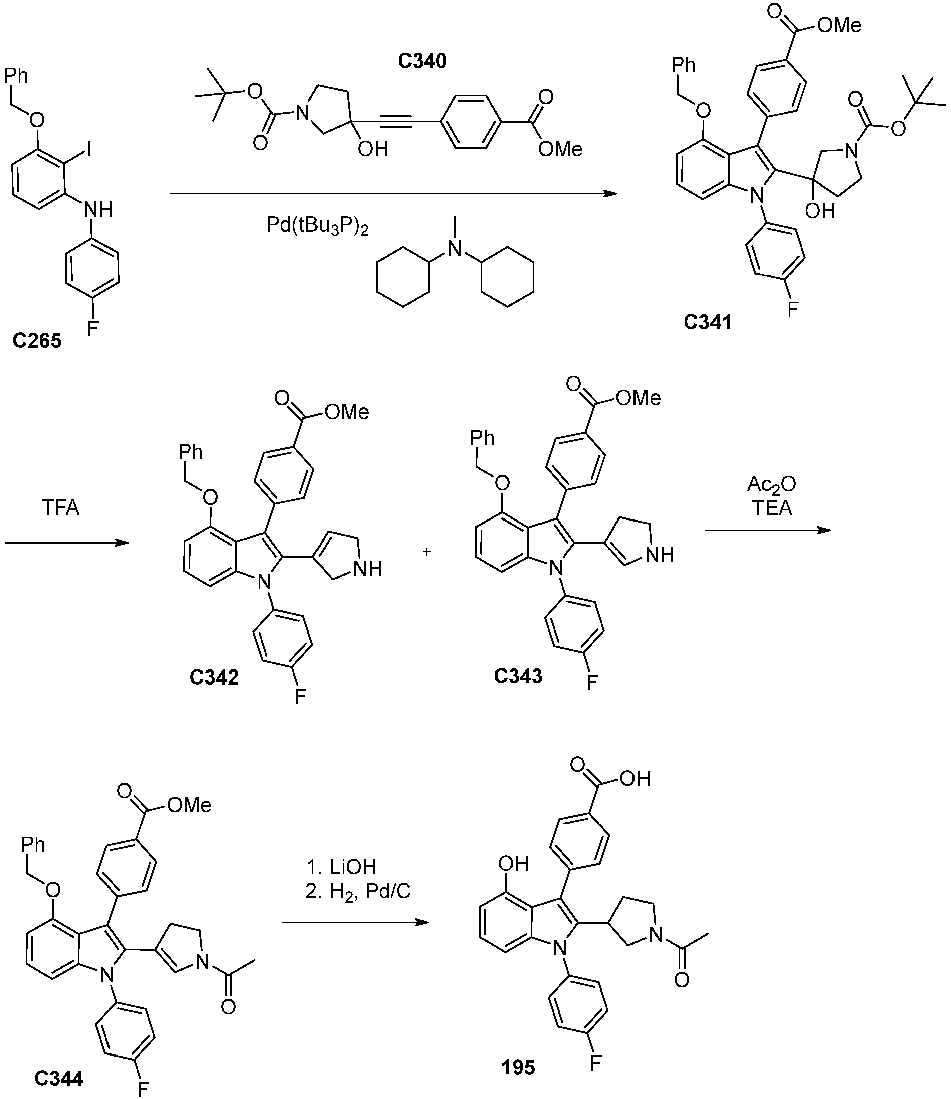

### Step 1. tert-butyl 3-[4-benzyloxy-1-(4-fluorophenyl)-3-(4-methoxycarbonylphenyl)indol-2-yl]-3-hydroxy-pyrrolidine-1-carboxylate (C341)

A sealed vial containing 3-benzyloxy-N-(4-fluorophenyl)-2-iodo-aniline (525 mg, 1.252 mmol), tert-butyl 3-hydroxy-3-[2-(4-methoxycarbonylphenyl)ethynyl]pyrrolidine-1-carboxylate (498 mg, 1.442 mmol), and palladium;tritert-butylphosphane (32 mg, 0.06262 mmol) was evacuated and back-filled with nitrogen. A solution of N-cyclohexyl-N-methyl-cyclohexanamine (672 µL, 3.137 mmol) in anhydrous 1,4-dioxane (7.5 mL) was added and the reaction mixture was stirred at 80 °C overnight. The mixture was diluted with water and extracted with EtOAc. The organic layer was concentrated to dryness and Silica gel chromatography (Column: 24g Combiflash Isco. Gradient: 0-40% EtOAc in heptane) afforded the product as an off-white solid. tert-butyl 3-[4-benzyloxy-1-(4-fluorophenyl)-3-(4-methoxycarbonylphenyl)indol-2-yl]-3-hydroxy-pyrrolidine-1-carboxylate (638 mg, 80%) ¹H NMR (400 MHz, DMSO-*d*₆) δ 7.94 - 7.84 (m, 2H), 7.71 - 7.55 (m, 4H), 7.49 - 7.38 (m, 2H), 7.17 (t, J = 7.4 Hz, 1H), 7.07 (tt, J = 8.1, 4.3 Hz, 3H), 6.73 (d, J = 7.5 Hz, 2H), 6.66 (t, J = 7.1 Hz, 1H), 6.41 (dd, J = 18.5, 8.3 Hz, 1H), 5.65 (s, 1H), 4.90 (d, J = 12.2 Hz, 2H), 3.92 (d, J = 7.3 Hz, 3H), 3.09 - 2.92 (m, 3H), 2.77 (d, J = 11.3 Hz, 1H), 1.67 (q, J = 12.4, 10.9 Hz, 1H), 1.48 - 1.35 (m, 1H), 1.23 (d, J = 38.0 Hz, 9H). LCMS *m*/*z* 636.19 [M+H]⁺ *Step 2. Methyl 4-[4-benzyloxy-2-(2,3-dihydro-1H-pyrrol-4-yl)-1-(4-fluoropheny)indol-3-yl]benzoate **(C343)** and methyl 4-[4-benzyloxy-2-(2,5-dihydro-1H-pyrrol-3-yl)-1-(4-fluorophenyl)indol-3-yl]benzoate **(C342)***

To a mixture of tert-butyl 3-[4-benzyloxy-1-(4-fluorophenyl)-3-(4-methoxycarbonylphenyl)indol-2-yl]-3-hydroxy-pyrrolidine-1-carboxylate (1.28 g, 1.917 mmol) in dichloromethane (20 mL) was added trifluoroacetic acid (800 µL, 10.38 mmol). The reaction turned dark and homogeneous, allowed to stir overnight. Saturated aqueous sodium bicarbonate was slowly added until the reaction pH were approximately 8. The solution was extracted with DCM (2x), the combined organics were washed with brine, passed through a phase separator, and concentrated under reduced pressure. Silica gel chromatography (Column: 4g Combiflash Isco. Gradient: 0-8% MeOH in DCM) afforded the product as an off-white solid. methyl 4-[4-benzyloxy-2-(2,3-dihydro-1H-pyrrol-4-yl)-1-(4-fluorophenyl)indol-3 -yl]benzoate. *Step 3. Methyl 4-[2-(1-acetyl-2, 3-dihydropyrrol-4 yl)-4-benzyloxy-1-(4-fluorophenyl)indol-3-yl]benzoate and methyl 4-[2-(1-acetyl-2,5-dihydropyrrol-3-yl)-4-benzyloxy-1-(4-fluorophenyl)indol-3-yl]benzoate*

To a solution of the mixture of regioisomers, methyl 4-[4-benzyloxy-2-(2,3-dihydro-1H-pyrrol-4-yl)-1-(4-fluorophenyl)indol-3-yl]benzoate (40 mg, 0.07564 mmol) / methyl 4-[4-benzyloxy-2-(2,5-dihydro-1H-pyrrol-3-yl)-1-(4-fluorophenyl)indol-3-yl]benzoate (10 mg, 0.01928 mmol) and triethylamine (27 µL, 0.1937 mmol) in tetrahydrofuran (1 mL) under nitrogen was added acetic anhydride (14 µL, 0.1484 mmol). Allowed to stir for 1 hour, the reaction was complete based on LCMS. Methanol was added and stirred for 5min then concentrated under reduced pressure. Will use as is, olefinic mixture will be hydrogenated in final step. Methyl 4-[2-(1-acetyl-2,3-dihydropyrrol-4-yl)-4-benzyloxy-1-(4-fluorophenyl)indol-3-yl]benzoate (40 mg, 89%) ¹H NMR (400 MHz, Chloroform-d) δ 7.97 - 7.89 (m, 2H), 7.54 - 7.45 (m, 3H), 7.39 - 7.28 (m, 3H), 7.22 - 7.12 (m, 4H), 6.87 (t, J = 6.9 Hz, 2H), 6.74 (dd, J = 9.1, 8.2 Hz, 1H), 6.63 (d, J = 7.9 Hz, 1H), 5.40 - 5.31 (m, 1H), 4.98 (d, J = 4.0 Hz, 2H), 4.11 - 4.03 (m, 2H), 4.01 - 3.94 (m, 3H), 3.86 (s, 1H), 3.83 - 3.78 (m, 1H), 1.94 (s, 2H), 1.74 (s, 1H). LCMS *m*/*z* 561.46 [M+H]⁺

### Step 4. 4-[2-(1-acetylpyrrolidin-3-yl)-1-(4-fluorophenyl)-4-hydroxy-indol-3-yl]benzoic acid (195)

To a solution of methyl 4-[2-(1-acetyl-2,3-dihydropyrrol-4-yl)-4-benzyloxy-1-(4-fluorophenyl)indol-3-yl]benzoate (39 mg, 0.06744 mmol) in tetrahydrofuran (5 mL) / methanol (1.5 mL) / Water (950 µL) was added lithium hydroxide (35 mg, 1.461 mmol) and allowed to stir overnight, reaction complete by lcms. Acidified reaction with 10% citric acid and extracted with EtOAc. The organic phase was washed with brine, dried over sodium sulfate and concentrated under reduced pressure.

The resulting solid was added as a solution ethyl acetate (3.8 mL) in to a slurry of palladium (15 mg, 0.1410 mmol) in ethanol (2.4 mL). The reaction mixture was stirred under 1 atm hydrogen overnight. the catalyst was filtered off and chromatographed over 4g silica gel using 010% methanol / DCM as eluant. 5.4mg obtained as a white solid. 4-[2-(1-acetylpyrrolidin-3-yl)-1-(4-fluorophenyl)-4-hydroxy-indol-3-yl]benzoic acid (5.4 mg, 17%) ¹H NMR (400 MHz, DMSO-*d*₆) δ 12.88 (s, 1H), 9.19 (d, J = 13.6 Hz, 1H), 7.92 (d, J = 8.0 Hz, 2H), 7.82 - 7.25 (m, 6H), 6.95 - 6.77 (m, 1H), 6.39 (dd, J = 7.8, 4.5 Hz, 1H), 6.25 (d, J = 8.4 Hz, 1H), 3.57 - 3.45 (m, 1H), 3.27 - 3.08 (m, 2H), 3.03 (t, J = 10.9 Hz, 1H), 2.90 (d, J = 9.6 Hz, 1H), 2.03 - 1.62 (m, 5H). LCMS *m*/*z* 459.38 [M+H]⁺

### Compound 196

### 4-[1-(4-fluorophenyl)-4-hydroxy-2-(1-methoxycarbonyl-3-piperidyl)indol-3-yl]benzoic acid (196)

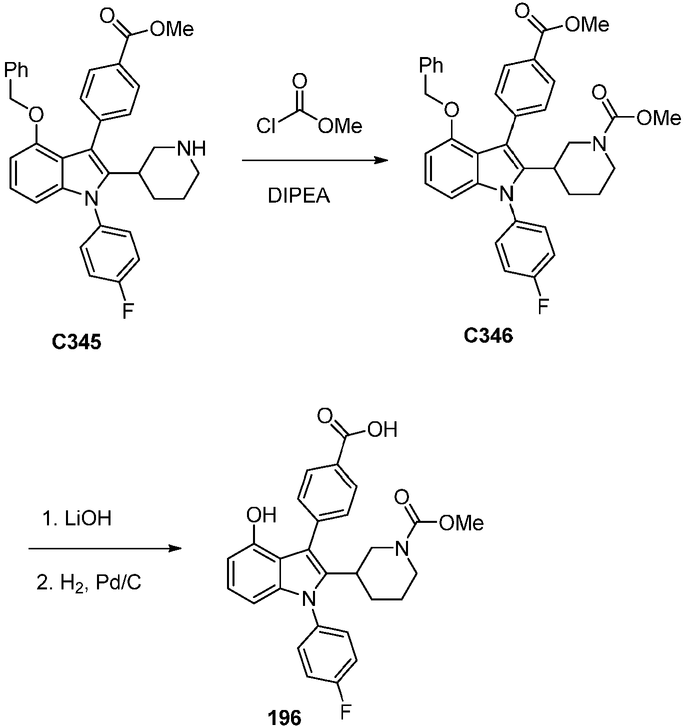

Intermediate **C345** was prepared from **C2** and tert-butyl 3-ethynylpiperidine-1-carboxylate using the method described for the preparation on intermediate **C258** (see compound **154).** Compound **196** was prepared from **C345** according to the method described for the preparation of compound **155.** Purification by reverse-phase chromatography (Column: C18. Gradient: 15-100% MeCN in water with 0.2% formic acid) afforded the product as a white solid. 4-[1-(4-fluorophenyl)-4-hydroxy-2-(1-methoxycarbonyl-3-piperidyl)indol-3-yl]benzoic acid (18.6 mg, 53%) ¹H NMR (400 MHz, Methanol/CDCl3-d4) δ 8.14 - 8.07 (m, 2H), 7.60 (d, J = 7.7 Hz, 2H), 7.44 (s, 1H), 7.38 - 7.33 (m, 1H), 7.29 (t, J = 7.7 Hz, 2H), 7.00 - 6.92 (m, 1H), 6.51 - 6.40 (m, 2H), 4.07 (s, 4H), 3.60 (d, J = 2.0 Hz, 3H), 2.69 (d, J = 10.0 Hz, 2H), 2.35 (s, 1H), 1.82 (d, J = 13.0 Hz, 1H), 1.56 - 1.35 (m, 2H), 1.31 - 1.12 (m, 1H). LCMS *m*/*z* 489.39 [M+H]⁺

### Compound 197

### 4-[2-(1-acetyl-3-piperidyl)-1-(4-fluorophenyl)-4-hydroxy-indol-3-yl]benzoic acid (197)

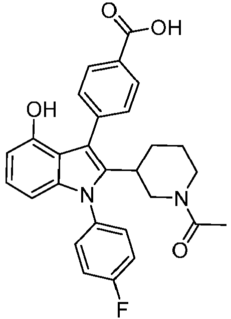

Compound **197** was prepared from **C345** and Ac₂O as described for compound **154.** Purification by reverse-phase chromatography (Column: C18. Gradient: 15-100% MeCN in water with 0.2% formic acid) afforded the product as a white solid. Rotomers observed in both ¹H and F NMR. 4-[2-(1-acetyl-3-piperidyl)-1-(4-fluorophenyl)-4-hydroxy-indol-3-yl]benzoic acid (12.5 mg, 35%) ¹H NMR (400 MHz, Chloroform-d) δ 8.26 - 8.05 (m, 2H), 7.62 (s, 2H), 7.48 - 7.38 (m, 1H), 7.38 - 7.28 (m, 2H), 7.25 - 7.14 (m, 1H), 7.06 - 6.92 (m, 1H), 6.51 (dd, J = 26.2, 7.9 Hz, 2H), 5.31 (s, 2H), 4.75 - 4.38 (m, 1H), 3.69 - 3.57 (m, 1H), 3.02 - 2.72 (m, 1H), 2.71 - 2.58 (m, 1H), 2.48 - 2.03 (m, 1H), 1.92 (d, J = 78.2 Hz, 4H), 1.64 - 1.37 (m, 2H), 1.24 - 1.15 (m, 1H). LCMS *m*/*z* 473.37 [M+H]⁺

### Compound 198

### 4-[1-(4-fluorophenyl)-4-hydroxy-2-[1-(2-methoxyacetyl)-3-piperidyl]indol-3-yl]benzoic acid (198)

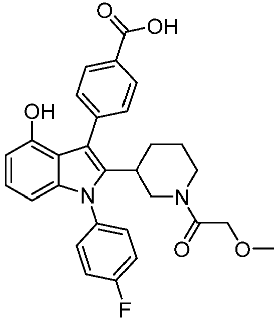

Compound **198** was prepared from intermediate **C345** and 2-methoxyacetyl chloride using the method described for the preparation of compound **155.** Purification by reverse-phase chromatography (Column: C18. Gradient: 10-100% MeCN in water with 0.2% formic acid) afforded the product as a white solid. Rotomers observed in both ¹H and F NMR's. 4-[1-(4-fluorophenyl)-4-hydroxy-2-[1-(2-methoxyacetyl)-3-piperidyl]indol-3-yl]benzoic acid (5 mg, 12%). ¹H NMR (400 MHz, Chloroform-d) δ 8.24 - 7.98 (m, 2H), 7.65 (s, 2H), 7.45 (s, 1H), 7.39 - 7.27 (m, 2H), 7.23 - 7.09 (m, 1H), 7.09 - 6.96 (m, 1H), 6.57 (dd, J = 21.8, 7.6 Hz, 1H), 6.52 - 6.44 (m, 1H), 4.53 (s, 1H), 3.80 (d, J = 3.9 Hz, 1H), 3.65 (d, J = 12.9 Hz, 1H), 3.29 (d, J = 39.9 Hz, 3H), 2.89 (t, J = 12.4 Hz, 1H), 2.81 - 2.67 (m, 2H), 2.59 (d, J = 13.1 Hz, 1H), 2.46 (d, J = 10.5 Hz, 1H), 2.13 (t, J = 13.0 Hz, 1H), 1.85 (d, J = 13.7 Hz, 1H), 1.55 (t, J = 15.6 Hz, 2H), 1.24 (d, J = 13.7 Hz, 1H). LCMS *m*/*z* 503.34 [M+H]⁺

### Compound 199

### 4-[1-(4-fluorophenyl)-4-hydroxy-2-[1-(2-methoxyacetyl)pyrrolidin-3-yl]indol-3-yl]benzoic acid (199)

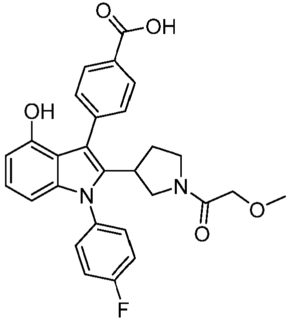

Compound **199** was prepared from methyl 4-[4-benzyloxy-2-(2,5-dihydro-1H-pyrrol-3-yl)-1-(4-fluorophenyl)indol-3-yl]benzoate **(C343)** and 2-methoxyacetyl chloride using the method described for the preparation of compound **195.** purification by reverse-phase chromatography (Column: C18.(50g) Gradient: 5-100% MeCN in water with 0.2% formic acid) afforded the product. 4-[1-(4-fluorophenyl)-4-hydroxy-2-[1-(2-methoxyacetyl)pyrrolidin-3-yl]indol-3-yl]benzoic acid (2.9 mg, 14%). ¹H NMR (400 MHz, Methanol-*d*₄) δ 8.05 - 7.96 (m, 2H), 7.56 - 7.43 (m, 4H), 7.40 - 7.30 (m, 2H), 6.92 - 6.84 (m, 1H), 6.40 - 6.29 (m, 2H), 5.49 (s, 2H), 3.88 (d, J = 3.6 Hz, 1H), 3.83 - 3.61 (m, 2H), 3.51 (d, J = 7.2 Hz, 1H), 3.43 - 3.35 (m, 2H), 3.26 (s, 3H), 3.20 - 3.06 (m, 1H), 2.09 - 1.97 (m, 2H). LCMS *m*/*z* 489.39 [M+H]⁺

### Compound 200

### 4-[1-(4-fluorophenyl)-4-hydroxy-2-(1-methoxycarbonylpyrrolidin-3-yl)indol-3-yl]benzoic acid (200)

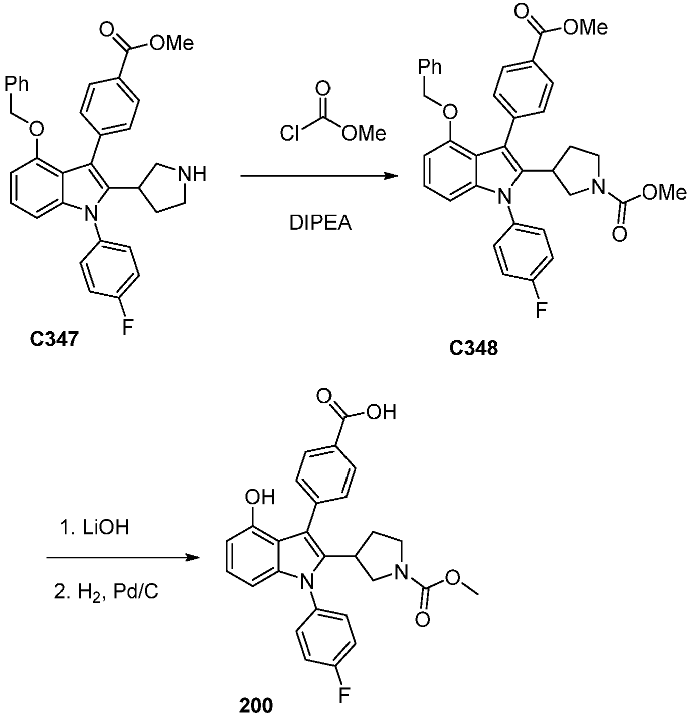

Intermediate **C347** was prepared from C2 and tert-butyl 3-ethynylpyrrolidine-1-carboxylate using the method described for the preparation of intermediate **C258** (see compound **154).** Compound **200** was prepared from intermediate **C347** using the method described for the preparation on compound **155.** 4-[1-(4-fluorophenyl)-4-hydroxy-2-(1-methoxycarbonylpyrrolidin-3-yl)indol-3-yl]benzoic acid (19.1 mg, 46%).¹H NMR (400 MHz, DMSO-*d*₆) δ 9.21 (s, 1H), 7.93 (d, J = 8.0 Hz, 2H), 7.61 - 7.54 (m, 2H), 7.51 (d, J = 7.8 Hz, 2H), 7.49 - 7.41 (m, 2H), 6.87 (t, J = 7.9 Hz, 1H), 6.40 (d, J = 7.7 Hz, 1H), 6.26 (d, J = 8.2 Hz, 1H), 3.20 - 3.04 (m, 3H), 3.00 (s, 1H), 2.51 (s, 3H), 1.91 (d, J = 9.2 Hz, 1H), 1.80 (s, 1H). LCMS *m*/*z* 475.31 [M+H]⁺

### Compound 201

### 4-[1-(4-fluorophenyl)-4-hydroxy-2-(1-methylsulfonyl-3-piperidyl)indol-3-yl]benzoic acid (201)

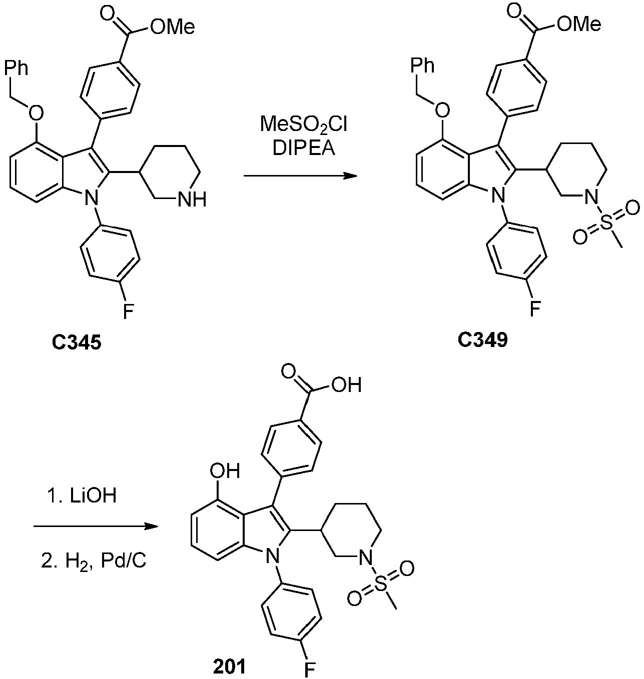

### Step 1. Synthesis of methyl 4-[4-benzyloxy-1-(4-fluorophenyl)-2-(1-methylsulfonyl-3-piperidyl)indol-3-yl]benzoate (C349)

To a mixture of methyl 4-[4-benzyloxy-1-(4-fluorophenyl)-2-(3-piperidyl)indol-3-yl]benzoate (Hydrochloride salt) (70 mg, 0.12 mmol) and diisopropyl ethylamine (120 µL, 0.69 mmol) in dichloromethane (1 mL) / N,N-dimethylformamide (0.1 mL) was added methanesulfonyl chloride (14 µL, 0.18 mmol) and allowed to stir overnight. Additional diisopropyl ethylamine (120 µL, 0.69 mmol) was added and the mixture became homogeneous. The reaction was allowed to stir for 4 hours and was then diluted with EtOAc and washed with water (2x), brine, dried over sodium sulfate, and concentrated. Silica gel chromatography (Column: 12g Combiflash Isco. Gradient: 0-30% EtOAc in heptane) afforded the product (55 mg, 74%). ¹H NMR (400 MHz, Chloroform-d) δ 7.96 (s, 2H), 7.49 (s, 2H), 7.44 - 7.38 (m, 1H), 7.38 - 7.26 (m, 3H), 7.21 - 7.03 (m, 4H), 6.78 - 6.72 (m, 2H), 6.61 - 6.54 (m, 2H), 4.90 (s, 2H), 3.99 (s, 3H), 3.74 (d, J = 11.2 Hz, 1H), 3.60 (d, J = 12.0 Hz, 1H), 2.82 (d, J = 11.6 Hz, 1H), 2.52 (s, 4H), 2.22 (t, J = 10.6 Hz, 1H), 1.84 (d, J = 9.2 Hz, 1H), 1.59 (d, J = 10.9 Hz, 1H), 1.35 (t, J = 9.9 Hz, 2H). LCMS *m*/*z* 613.38 [M+H]⁺

### Step 2. Synthesis of 4-[4-benzyloxy-1-(4-fluorophenyl)-2-(1-methylsulfonyl-3-piperidyl)indol-3-yl]benzoic acid

To a solution of methyl 4-[4-benzyloxy-1-(4-fluorophenyl)-2-(1-methylsulfonyl-3-piperidyl)indol-3-yl]benzoate (55 mg, 0.09 mmol) in tetrahydrofuran (6.8 mL) /methanol (1.7 mL) / water (1.2 mL) was added lithium hydroxide (45 mg, 1.88 mmol) and warmed to 50 °C and allowed to stir for 3 hours. The mixture cooled to room temperature, acidified with 10% citric acid, and extracted with EtOAc. The organics were washed with brine, dried over sodium sulfate, and concentrated under reduced pressure to afford the product. LCMS *m*/*z* 599.21 [M+H]⁺

### Step 3. Synthesis of 4-[1-(4fluorophenyl)-4-hydroxy-2-(1-methylsulfonyl-3-piperidyl)indol-3-yl]benzoic acid (201)

To a mixture of 4-[4-benzyloxy-1-(4-fluorophenyl)-2-(1-methylsulfonyl-3-piperidyl)indol-3-yl]benzoic acid (53 mg, 0.08 mmol) and dihydroxypalladium (4 mg, 0.028 mmol) was added methanol (10 mL). The mixture was placed under a hydrogen atmosphere (balloon pressure) and allowed to stir for 4 hours. The catalyst was filtered off and the filtrate was concentrated. Purification by reverse-phase chromatography (Column: C18. Gradient: 20-100% MeCN in water with 0.2% formic acid) afforded the product as a white solid. 4-[1-(4-fluorophenyl)-4-hydroxy-2-(1-methylsulfonyl-3-piperidyl)indol-3-yl]benzoic acid (17.1 mg, 38%). ¹H NMR (400 MHz, DMSO-*d*₆) δ 12.90 (s, 1H), 9.19 (s, 1H), 7.97 - 7.90 (m, 2H), 7.62 - 7.50 (m, 4H), 7.50 - 7.42 (m, 2H), 6.90 - 6.84 (m, 1H), 6.39 (dd, J = 7.7, 0.8 Hz, 1H), 6.24 (dd, J = 8.2, 0.8 Hz, 1H), 3.57 (d, J = 11.2 Hz, 1H), 3.46 - 3.35 (m, 2H), 2.77 (d, J = 12.0 Hz, 1H), 2.67 (s, 3H), 2.21 (t, J = 11.1 Hz, 1H), 1.77 (d, J = 9.7 Hz, 1H), 1.56 (d, J = 9.2 Hz, 1H), 1.26 - 1.16 (m, 2H). LCMS *m*/*z* 509.23 [M+H]⁺

### Compound 202

### 4-[1-(4-fluorophenyl)-4-hydroxy-2-[1-(trifluoromethylsulfonyl)-3-piperidyl]indol-3-yl]benzoic acid (202)

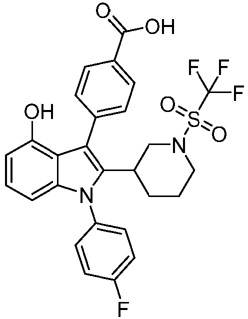

Compound **202** was prepared from **C345** and trifluoromethanesulfonyl chloride according to the method described for the preparation of compound **201.** Purification by reverse-phase chromatography (Column: C18. Gradient: 20-100% MeCN in water with 0.2% formic acid) afforded the product as a white solid. 4-[1-(4-fluorophenyl)-4-hydroxy-2-[1-(trifluoromethylsulfonyl)-3-piperidyl]indol-3-yl]benzoic acid (22.4 mg, 90%) ¹H NMR (400 MHz, DMSO-*d*₆) δ 12.93 (s, 1H), 9.19 (s, 1H), 7.94 (d, J = 8.2 Hz, 2H), 7.65 - 7.35 (m, 6H), 6.92 - 6.83 (m, 1H), 6.39 (dd, J = 7.7, 0.8 Hz, 1H), 6.25 (dd, J = 8.3, 0.8 Hz, 1H), 3.78 (d, J = 12.1 Hz, 1H), 3.60 (d, J = 13.0 Hz, 1H), 2.98 (s, 1H), 2.80 (d, J = 12.0 Hz, 2H), 1.84 (d, J = 13.1 Hz, 1H), 1.63 (d, J = 13.4 Hz, 1H), 1.35 (d, J = 13.8 Hz, 1H), 1.17 (d, J = 16.1 Hz, 1H). LCMS *m*/*z* 563.17 [M+H]⁺

### Compound 203

### 4-[2-(1-ethylsulfonyl-3-piperidyl)-1-(4-fluorophenyl)-4-hydroxy-indol-3-yl]benzoic acid (203)

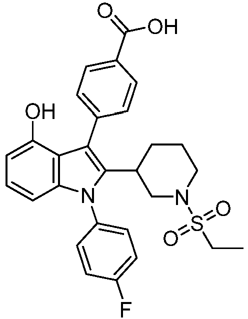

Compound **203** was prepared from **C345** and ethanesulfonyl chloride according to the method described for the preparation of compound 201. Purification by reverse-phase chromatography (Column: C18. Gradient: 20-100% MeCN in water with 0.2% formic acid) afforded the product as a white solid. 4-[2-(1-ethylsulfonyl-3-piperidyl)-1-(4-fluorophenyl)-4-hydroxy-indol-3-yl]benzoic acid (28.2 mg, 71%) ¹H NMR (400 MHz, DMSO-*d*₆) δ 12.91 (s, 1H), 9.18 (s, 1H), 7.98 - 7.90 (m, 2H), 7.61 - 7.49 (m, 4H), 7.46 (dd, J = 9.7, 7.8 Hz, 2H), 6.91 - 6.82 (m, 1H), 6.39 (dd, J = 7.7, 0.8 Hz, 1H), 6.24 (dd, J = 8.2, 0.8 Hz, 1H), 3.60 (d, J = 11.5 Hz, 1H), 3.42 (d, J = 12.3 Hz, 1H), 2.91 - 2.70 (m, 3H), 2.64 (t, J = 11.7 Hz, 1H), 2.32 (t, J = 10.9 Hz, 1H), 1.78 (d, J = 11.1 Hz, 1H), 1.54 (d, J = 11.1 Hz, 1H), 1.21 (d, J = 10.8 Hz, 2H), 1.00 (t, J = 7.3 Hz, 3H). LCMS *m*/*z* 523.22 [M+H]⁺

### Compound 204

### 4-[1-(4-fluorophenyl)-4-hydroxy-2-(1-methylsulfonylpyrrolidin-3-yl)indol-3-yl]benzoic acid (204)

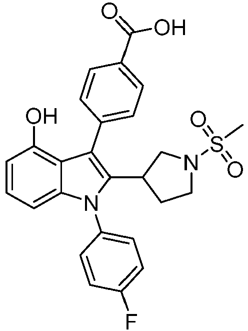

Compound **204** was prepared from **C347** and methane sulfonyl chloride as described for the preparation of compound **201.** 4-[1-(4-fluorophenyl)-4-hydroxy-2-(1-methylsulfonylpyrrolidin-3-yl)indol-3-yl]benzoic acid (19.8 mg, 64%). ¹H NMR (400 MHz, DMSO-*d*₆) δ 9.26 (s, 1H), 7.96 (d, J = 8.0 Hz, 2H), 7.65 - 7.53 (m, 4H), 7.52 - 7.43 (m, 2H), 6.89 (t, J = 7.9 Hz, 1H), 6.41 (d, J = 7.6 Hz, 1H), 6.27 (d, J = 8.1 Hz, 1H), 3.14 - 2.95 (m, 4H), 2.61 (s, 3H), 1.92 (s, 1H), 1.81 (p, J = 10.1, 9.7 Hz, 1H). LCMS *m*/*z* 495.13 [M+H]⁺

### Compound 205

### 4-[1-(4-fluorophenyl)-4-hydroxy-2-[1-(trifluoromethylsulfonyl)pyrrolidin-3-yl]indol-3-yl]benzoic acid (205)

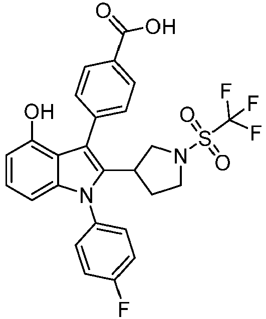

Compound **205** was prepared from **C347** and trifluoromethyl sulfonyl chloride as described for the preparation of compound 201. ¹H NMR (400 MHz, DMSO-*d*₆) δ 12.91 (s, 1H), 9.27 (s, 1H), 7.96 (d, J = 7.9 Hz, 2H), 7.62 (dd, J = 8.1, 4.3 Hz, 2H), 7.55 (d, J = 7.9 Hz, 2H), 7.52 - 7.41 (m, 2H), 6.90 (t, J = 8.0 Hz, 1H), 6.41 (d, J = 7.7 Hz, 1H), 6.28 (d, J = 8.2 Hz, 1H), 3.61 (t, J = 8.7 Hz, 1H), 3.57 - 3.43 (m, 0H), 2.53 - 2.49 (m, 4H), 2.07 (dd, J = 13.1, 6.8 Hz, 1H), 1.98 - 1.84 (m, 1H). LCMS *m*/*z* 548.73 [M+H]⁺

### Compound 206

### 4-[2-(1-ethylsulfonylpyrrolidin-3-yl)-1-(4-fluorophenyl)-4-hydroxy-indol-3-yl]benzoic acid (206)

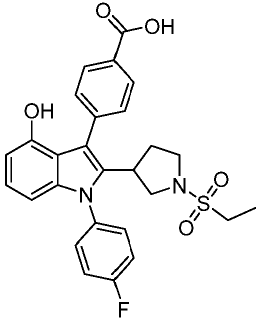

Compound **206** prepared from **C347** and trifluoromethyl sulfonyl chloride as described for the preparation of compound **201.** 4-[2-(1-ethylsulfonylpyrrolidin-3-yl)-1-(4-fluorophenyl)-4-hydroxy-indol-3-yl]benzoic acid. ¹H NMR (400 MHz, DMSO-*d*₆) δ 9.26 (s, 1H), 7.95 (d, J = 7.9 Hz, 2H), 7.60 (q, J = 5.2, 4.6 Hz, 2H), 7.55 (d, J = 8.0 Hz, 2H), 7.48 (t, J = 9.0 Hz, 2H), 6.88 (t, J = 7.9 Hz, 1H), 6.41 (d, J = 7.6 Hz, 1H), 6.27 (d, J = 8.2 Hz, 1H), 3.16 - 2.97 (m, 4H), 2.86 - 2.69 (m, 3H), 1.95 (d, J = 11.7 Hz, 1H), 1.84 (q, J = 10.3, 9.7 Hz, 1H), 0.99 (t, J = 7.4 Hz, 3H). LCMS *m*/*z* 509.14 [M+H]⁺

### Compound 207

### 4-[1-(4-fluorophenyl)-4-hydroxy-2-(1-tetrahydrofuran-3-ylethyl)indol-3-yl]benzoic acid (207)

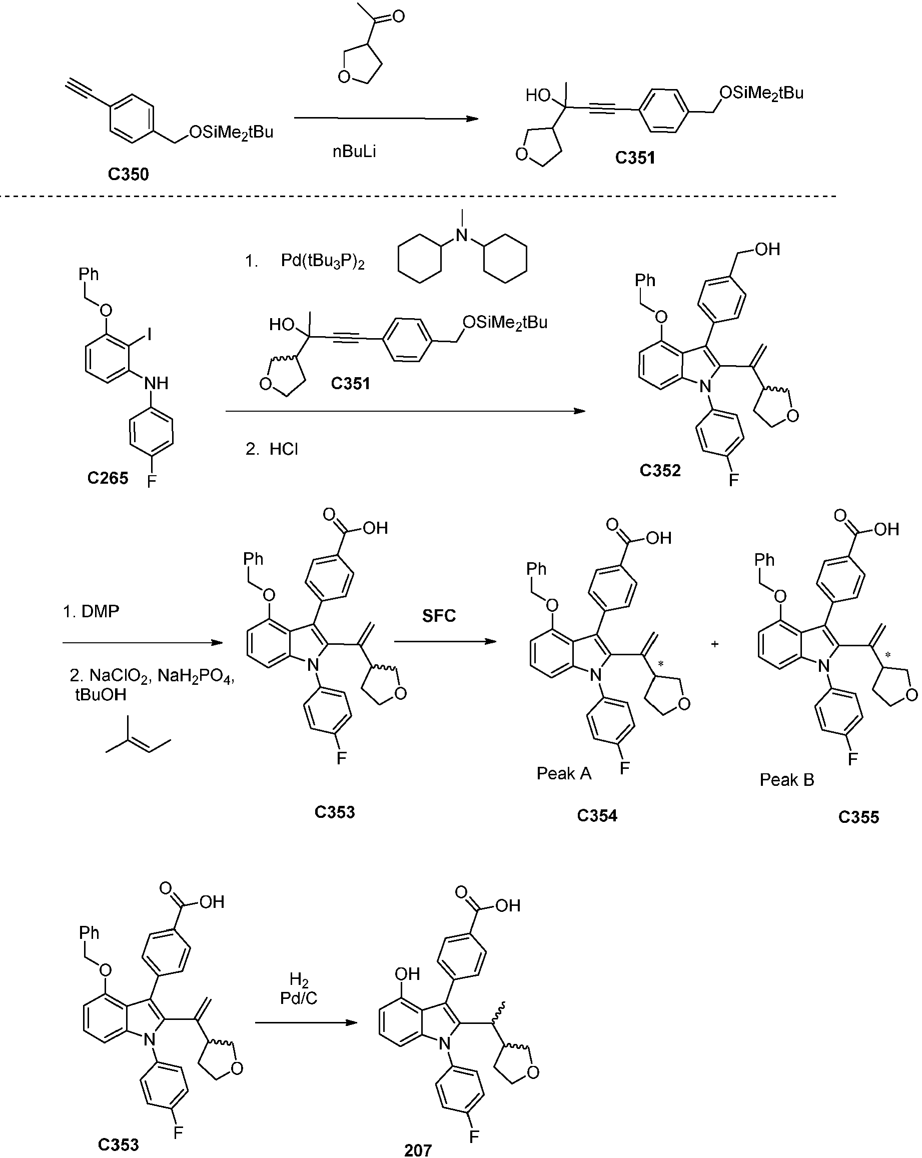

### Step 1. Preparation of Compound C352

Compound **C352** was prepared from **C265** and **C351** using the method described for the preparation of compound **147.**

### Step 2-3. Preparation of 4-[4-benzyloxy-1-(4-fluorophenyl)-2-(1-tetrahydrofuran-3-ylvinyl)indol-3-yl]benzoic acid (C353)

To a solution of **C352** (450 mg, 0.86 mmol) in dichloromethane (5 mL) was added DMP (405 mg, 0.95 mmol). The reaction mixture was stirred at room temperature for 20 minutes and quenched with 1 M aq. sodium thiosulfate solution. After stirring for 5 minutes, the organic layer was concentrated to dryness and purified via silica gel chromatography eluting with 0-50% EtOAc in heptane. Pure fractions were combined and concentrated to give 265 mg white solid. 4-[4-benzyloxy-1-(4-fluorophenyl)-2-(1-tetrahydrofuran-3-ylvinyl)indol-3-yl]benzaldehyde (265 mg, 59%) ¹H NMR (400 MHz, Chloroform-d) δ 10.00 (s, 1H), 7.75 - 7.71 (m, 2H), 7.62 - 7.59 (m, 2H), 7.43 - 7.37 (m, 2H), 7.27 - 7.20 (m, 3H), 7.20 - 7.13 (m, 3H), 6.93 (ddt, *J* = 7.4, 1.5, 0.7 Hz, 2H), 6.84 (dd, *J* = 8.3, 0.7 Hz, 1H), 6.70 (dd, *J* = 7.9, 0.7 Hz, 1H), 5.36 (t, *J* = 1.1 Hz, 1H), 5.21 (t, *J* = 0.8 Hz, 1H), 5.02 (s, 2H), 3.73 (td, *J* = 8.3, 4.8 Hz, 1H), 3.61 (dt, *J* = 8.4, 7.4 Hz, 1H), 3.51 (dd, *J* = 8.3, 7.3 Hz, 1H), 3.31 (dd, *J* = 8.3, 7.6 Hz, 1H), 2.55 - 2.44 (m, 1H), 1.75 (dtd, *J =* 12.4, 7.6, 4.9 Hz, 1H), 1.68 - 1.60 (m, 1H). LCMS *m*/*z* 518.0 [M+H]⁺

### 4-[4-benzyloxy-1-(4-fluorophenyl)-2-(1-tetrahydrofuran-3-ylvinyl)indol-3-yl]benzoic acid (C353)

To a suspension of 4-[4-benzyloxy-1-(4-fluorophenyl)-2-(1-tetrahydrofuran-3-ylvinyl)indol-3-yl]benzaldehyde (250 mg, 0.48 mmol) and 2-methylbut-2-ene in THF (7.92 mL of 2 M, 15.84 mmol) in t-BuOH (5.5 mL) and water (5.5 mL) was added NaClO₂ (Sodium salt) (1.25 g, 13.82 mmol) and dihydrogen phosphate (Sodium salt) (1.67 g, 13.92 mmol). The reaction mixture was stirred at room temperature for 15 minutes then diluted with water and extracted with EtOAc. The organic layer was dried over magnesium sulfate, filtered, concentrated, and purified via silica gel chromatography eluting with 0-10% MeOH in DCM. Pure fractions were combined, concentrated, triturated with 9:1 heptane / EtOAc, filtered, and dried to give 213 mg off-white solid. 4-[4-benzyloxy-1-(4-fluorophenyl)-2-(1-tetrahydrofuran-3-ylvinyl)indol-3-yl]benzoic acid (213 mg, 83%). ¹H NMR (400 MHz, DMSO-*d*₆) δ 12.84 (s, 1H), 7.88 - 7.83 (m, 2H), 7.55 - 7.49 (m, 4H), 7.45 - 7.40 (m, 2H), 7.22 - 7.09 (m, 4H), 6.91 - 6.86 (m, 2H), 6.75 (d, *J* = 7.9 Hz, 1H), 6.72 (d, *J* = 8.3 Hz, 1H), 5.37 (d, *J =* 1.3 Hz, 1H), 5.26 (s, 1H), 5.03 (s, 2H), 3.55 (td, *J* = 8.3, 4.8 Hz, 1H), 3.44 (dt, *J* = 8.3, 7.4 Hz, 1H), 3.37 - 3.34 (m, 1H), 3.09 (t, *J* = 7.9 Hz, 1H), 2.40 (t, *J* = 7.7 Hz, 1H), 1.66 (dtd, *J* = 12.4, 7.6, 4.8 Hz, 1H), 1.54 - 1.42 (m, 1H). LCMS *m*/*z* 534.0 [M+H]⁺

### Preparation of 4-[4-benzyloxy-1-(4-fluorophenyl)-2-(1-tetrahydrofuran-3-ylvinyl)indol-3-yl]benzoic acid (C354) and 4-[4-benzyloxy-1-(4-fluorophenyl)-2-(1-tetrahydrofuran-3-ylvinyl)indol-3-yl]benzoic acid (C355)

4-[4-benzyloxy-1-(4-fluorophenyl)-2-(1-tetrahydrofuran-3-ylvinyl)indol-3-yl]benzoic acid (**C353**) (190 mg, 0.36 mmol) was separated into its constituent stereoisomers by SFC.
**Peak A: Intermediate C354** 4-[4-benzyloxy-1-(4-fluorophenyl)-2-(1-tetrahydrofuran-3-ylvinyl)indol-3-yl]benzoic acid (40 mg, 42%) ¹H NMR (400 MHz, Chloroform-d) δ 8.04 - 7.98 (m, 2H), 7.60 - 7.54 (m, 2H), 7.45 - 7.38 (m, 2H), 7.27 - 7.13 (m, 6H), 6.99 - 6.92 (m, 2H), 6.85 (d, J = 8.3 Hz, 1H), 6.70 (d, J = 7.8 Hz, 1H), 5.36 (t, J = 1.2 Hz, 1H), 5.22 (s, 1H), 5.04 (s, 2H), 3.75 (td, J = 8.3, 4.8 Hz, 1H), 3.63 (q, J = 7.7 Hz, 1H), 3.53 (dd, J = 8.3, 7.3 Hz, 1H), 3.33 (t, J = 8.0 Hz, 1H), 2.52 (p, J = 7.7 Hz, 1H), 1.77 (dtd, J = 12.4, 7.6, 4.8 Hz, 1H), 1.62 (dq, J = 12.3, 8.0 Hz, 1H). LCMS *m*/*z* 534.0 [M+H]⁺
**Peak B: Intermediate C355** 4-[4-benzyloxy-1-(4-fluorophenyl)-2-(1-tetrahydrofuran-3-ylvinyl)indol-3-yl]benzoic acid (40 mg, 42%). ¹H NMR (400 MHz, Chloroform-d) δ 7.93 - 7.88 (m, 2H), 7.50 - 7.45 (m, 2H), 7.34 - 7.28 (m, 2H), 7.17 - 7.04 (m, 6H), 6.88 - 6.82 (m, 2H), 6.75 (dd, J = 8.3, 0.6 Hz, 1H), 6.60 (d, J = 7.8 Hz, 1H), 5.27 (d, J = 1.2 Hz, 1H), 5.12 (t, J = 0.8 Hz, 1H), 4.94 (s, 2H), 3.65 (td, J = 8.3, 4.9 Hz, 1H), 3.53 (dt, J = 8.4, 7.4 Hz, 1H), 3.43 (dd, J = 8.3, 7.3 Hz, 1H), 3.27 - 3.19 (m, 1H), 2.42 (p, J = 7.7 Hz, 1H), 1.67 (dtd, J = 12.3, 7.5, 4.8 Hz, 1H), 1.52 (dq, J = 12.2, 8.0 Hz, 1H). LCMS *m*/*z* 534.0 [M+H]⁺

### Step 3. 4-[1-(4-fluorophenyl)-4-hydroxy-2-(1-tetrahydrofuran-3-ylethyl)indol-3-yl]benzoic acid (207)

To a slurry of Pd / C (25 mg, 0.02349 mmol) in EtOH (5 mL) was added a solution of 4-[4-benzyloxy-1-(4-fluorophenyl)-2-(1-tetrahydrofuran-3-ylvinyl)indol-3-yl]benzoic acid (25 mg, 0.047 mmol) in EtOAc (5 mL). The reaction mixture was stirred under 1 atm hydrogen for 20 minutes. The mixture was stirred for another 40 minutes, filtered over Celite^{®}, and concentrated to dryness. The residue was then triturated with heptane, filtered and dried. 4-[1-(4-fluorophenyl)-4-hydroxy-2-(1-tetrahydrofuran-3-ylethyl)indol-3-yl]benzoic acid (6 mg, 26%). ¹H NMR (400 MHz, DMSO-*d*₆) δ 9.29 - 9.15 (m, 1H), 7.93 (d, *J* = 7.8 Hz, 2H), 7.52 - 7.44 (m, 6H), 6.83 (td, *J* = 7.9, 1.7 Hz, 1H), 6.40 (dd, *J* = 7.6, 2.9 Hz, 1H), 6.19 (dd, *J* = 8.2, 4.4 Hz, 1H), 3.55 - 3.26 (m, 3H), 3.02 (dt, *J* = 32.0, 7.4 Hz, 1H), 2.62 (dt, *J* = 19.5, 7.7 Hz, 1H), 2.44 - 2.22 (m, 1H), 1.93 - 1.58 (m, 2H), 1.17 (dd, *J* = 28.3, 7.0 Hz, 3H). LCMS *m*/*z* 446.0 [M+H]⁺

### Compound 208 and Compound 209

### 4-[1-(4-fluorophenyl)-4-hydroxy-2-(1-tetrahydrofuran-3-ylethyl)indol-3-yl]benzoic acid (208) and 4-[1-(4-fluorophenyl)-4-hydroxy-2-(1-tetrahydrofuran-3-ylethyl)indol-3-yl]benzoic acid (209)

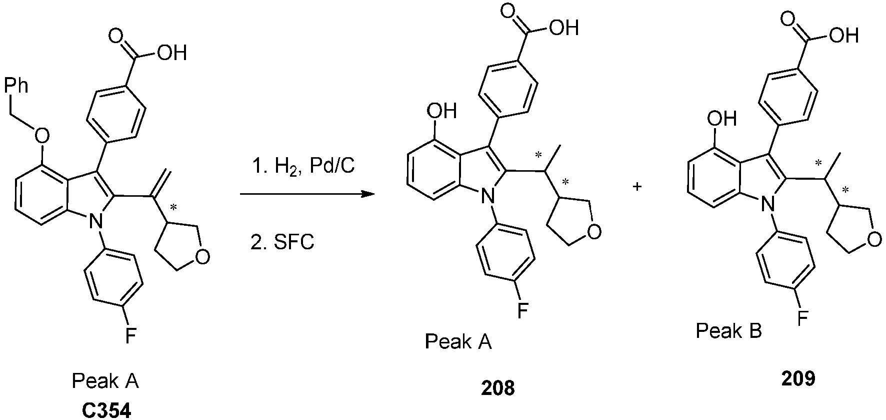

To a slurry of Pd / C (25 mg, 0.02349 mmol) in EtOH (10 mL) was added a solution of 4-[4-benzyloxy-1-(4-fluorophenyl)-2-(1-tetrahydrofuran-3-ylvinyl)indol-3-yl]benzoic acid (40 mg, 0.07496 mmol) in EtOAc (10 mL). The reaction mixture was stirred under 1 atm hydrogen for 2 hours then filtered over Celite^{®}, and concentrated to dryness. The mixture was separated into its constituent stereoisomers by SFC.
**Peak A:** 4-[1-(4-fluorophenyl)-4-hydroxy-2-(1-tetrahydrofuran-3-ylethyl)indol-3-yl]benzoic acid **208** (11 mg, 61%) ¹H NMR (400 MHz, DMSO-*d*₆) δ 12.87 (s, 1H), 9.17 (s, 1H), 7.97 - 7.91 (m, 2H), 7.57 - 7.42 (m, 6H), 6.84 (t, J = 7.9 Hz, 1H), 6.39 (d, J = 7.6 Hz, 1H), 6.19 (d, J = 8.2 Hz, 1H), 3.56 - 3.36 (m, 3H), 2.97 (dd, J = 8.7, 7.3 Hz, 1H), 2.61 (dd, J = 11.2, 7.1 Hz, 1H), 1.89 (dt, J = 11.1, 7.5 Hz, 1H), 1.82 - 1.71 (m, 1H), 1.21 (dq, J = 8.0, 4.8 Hz, 4H). LCMS *m*/*z* 446.0 [M+H]⁺
**Peak B:** 4-[1-(4-fluorophenyl)-4-hydroxy-2-(1-tetrahydrofuran-3-ylethyl)indol-3-yl]benzoic acid **209** (10 mg, 59%) ¹H NMR (400 MHz, DMSO-*d*₆) δ 12.88 (s, 1H), 9.17 (s, 1H), 7.97 - 7.92 (m, 2H), 7.51 (ddd, J = 12.4, 8.7, 4.5 Hz, 6H), 6.84 (t, J = 7.9 Hz, 1H), 6.41 - 6.37 (m, 1H), 6.19 (d, J = 8.2 Hz, 1H), 3.56 - 3.37 (m, 3H), 2.97 (dd, J = 8.6, 7.2 Hz, 1H), 2.67 - 2.53 (m, 1H), 1.89 (dt, J = 11.2, 7.5 Hz, 1H), 1.82 - 1.70 (m, 1H), 1.26 - 1.16 (m, 4H). LCMS *m*/*z* 446.0 [M+H]⁺

### Compound 210 and Compound 211

### 4-[1-(4-fluorophenyl)-4-hydroxy-2-(1-tetrahydrofuran-3-ylethyl)indol-3-yl]benzoic acid (210) and 4-[1-(4-fluorophenyl)-4-hydroxy-2-(1-tetrahydrofuran-3-ylethyl)indol-3-yl]benzoic acid (211)

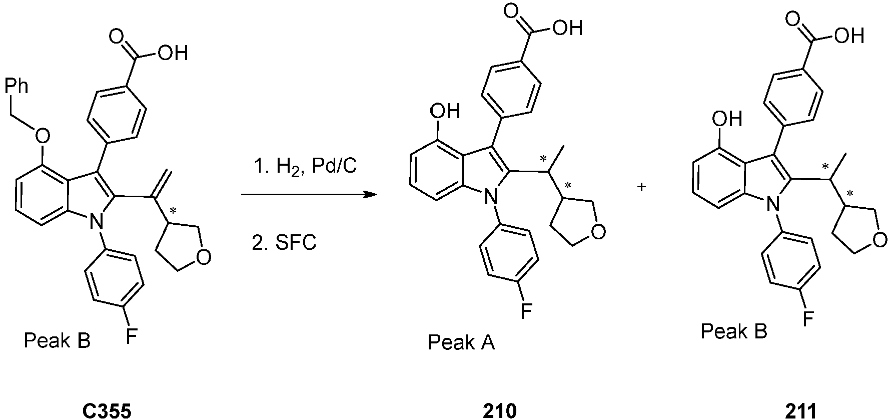

Compound **210** and **211** were prepared from **C355** using the method described for the preparation of compounds **208** and **209.**
**Peak A:** 4-[1-(4-fluorophenyl)-4-hydroxy-2-(1-tetrahydrofuran-3-ylethyl)indol-3-yl]benzoic acid 210 (10 mg, 59%) ¹H NMR (400 MHz, DMSO-*d*₆) δ 12.88 (s, 1H), 9.17 (s, 1H), 7.97 - 7.92 (m, 2H), 7.51 (ddd, *J* = 12.4, 8.7, 4.5 Hz, 6H), 6.84 (t, *J* = 7.9 Hz, 1H), 6.41 - 6.37 (m, 1H), 6.19 (d, *J* = 8.2 Hz, 1H), 3.56 - 3.37 (m, 3H), 2.97 (dd, *J* = 8.6, 7.2 Hz, 1H), 2.67 - 2.53 (m, 1H), 1.89 (dt, *J* = 11.2, 7.5 Hz, 1H), 1.82 - 1.70 (m, 1H), 1.26 - 1.16 (m, 4H). LCMS *m*/*z* 446.0 [M+H]⁺
**Peak B:** 4-[1-(4-fluorophenyl)-4-hydroxy-2-(1-tetrahydrofuran-3-ylethyl)indol-3-yl]benzoic acid **211** (8 mg, 47%) ¹H NMR (400 MHz, DMSO-*d*₆) δ 12.86 (s, 1H), 9.17 (s, 1H), 7.98 - 7.93 (m, 2H), 7.56 - 7.46 (m, 6H), 6.84 *(t, J* = 7.9 Hz, 1H), 6.42 - 6.37 (m, 1H), 6.20 (d, *J* = 8.1 Hz, 1H), 3.53 - 3.50 (m, 1H), 3.41 - 3.35 (m, 2H), 3.06 (dt, *J* = 8.5, 5.9 Hz, 1H), 2.69 - 2.58 (m, 1H), 1.85 (dp, *J* = 13.6, 7.0 Hz, 1H), 1.67 (ddd, *J* = 10.8, 7.5, 5.4 Hz, 1H), 1.16 (dd, *J* = 17.6, 7.1 Hz, 4H). LCMS *m*/*z* 446.0 [M+H]⁺

### Compound 212

### 4-[1-(4-fluorophenyl)-4-hydroxy-2-isopropyl-indol-3-yl]benzoic acid (212)

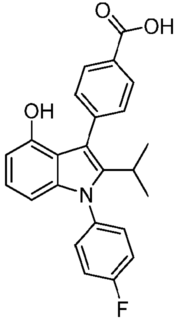

Compound **212** was prepared from **C195** as described for compound **129.** 4-[1-(4-fluorophenyl)-4-hydroxy-2-isopropyl-indol-3-yl]benzoic acid (66 mg, 89%). ¹H NMR (400 MHz, DMSO-*d*₆) δ 12.83 (s, 1H), 9.15 (s, 1H), 7.99 - 7.90 (m, 2H), 7.60 - 7.48 (m, 4H), 7.48 - 7.32 (m, 2H), 6.83 (t, J = 7.9 Hz, 1H), 6.39 (dd, J = 7.7, 0.9 Hz, 1H), 6.20 (dd, J = 8.2, 0.8 Hz, 1H), 2.98 (h, J = 7.3 Hz, 1H), 1.00 (d, J = 7.2 Hz, 6H). LCMS *m*/*z* 390.23 [M+H]⁺

### Compound 213

### 4-[1-(4-fluorophenyl)-4-hydroxy-2-isopropyl-indol-3-yl]sulfonylbenzoic acid (213)

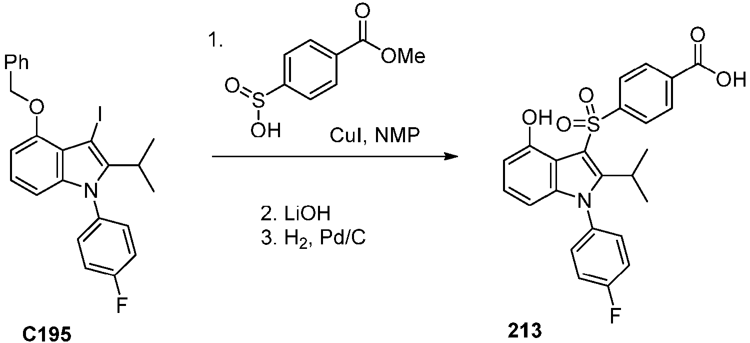

### Step 1. Synthesis of methyl 4-[4-benzyloxy-1-(4-fluorophenyl)-2-isopropyl-indol-3-ylJsulfonylbenzoate

A mixture of 4-benzyloxy-1-(4-fluorophenyl)-3-iodo-2-isopropyl-indole (300 mg, 0.6181 mmol), 4-methoxycarbonylbenzenesulfinic acid (Sodium salt) (410 mg, 1.837 mmol) and CuI (480 mg, 2.520 mmol) in NMP (3 mL) was heated at 130 oC in a sealed tube for 2 hours. The reaction mixture was diluted with EtOAc and washed with water and brine then concentrated to dryness. The mixture was purified by reverse phase chromatography eluting with 5-90% MeCN in water with 0.1% TFA. The fractions were combined, diluted with water, and extracted with EtOAc. The organic layer was concentrated to dryness and purified via silica gel chromatography eluting with 0-35% EtOAc in heptane. Pure fractions were combined and concentrated to give 122 mg off-white solid. methyl 4-[4-benzyloxy-1-(4-fluorophenyl)-2-isopropyl-indol-3-yl]sulfonylbenzoate (122 mg, 35%). ¹H NMR (400 MHz, Chloroform-d) δ 7.95 - 7.90 (m, 2H), 7.79 - 7.74 (m, 2H), 7.41 - 7.30 (m, 7H), 7.26 (dd, J = 7.1, 2.6 Hz, 2H), 6.99 (t, J = 8.1 Hz, 1H), 6.53 (d, J = 7.9 Hz, 1H), 6.33 (d, J = 8.1 Hz, 1H), 4.97 (s, 2H), 3.95 (s, 3H), 1.32 (d, J = 8.2 Hz, 6H). LCMS *m*/*z* 558.0 [M+H]⁺

### Step 2. 4-[4-benzyloxy-1-(4-fluorophenyl)-2-isopropyl-indol-3-yl]sulfonylbenzoic acid

To a solution of methyl 4-[4-benzyloxy-1-(4-fluorophenyl)-2-isopropyl-indol-3-yl]sulfonylbenzoate (122 mg, 0.2188 mmol) in THF (6 mL), MeOH (2 mL), and water (2 mL) was added LiOH (70 mg, 2.923 mmol). The reaction mixture was stirred at room temperature for 1 hour then was acidified using 1 M aq. HCl and extracted with EtOAc. The organic layer was dried over magnesium sulfate, filtered, and concentrated to give 81 mg off-white solid. 4-[4-benzyloxy-1-(4-fluorophenyl)-2-isopropyl-indol-3-yl]sulfonylbenzoic acid (81 mg, 68%). ¹H NMR (400 MHz, Chloroform-d) δ 7.97 - 7.92 (m, 2H), 7.78 - 7.74 (m, 2H), 7.42 - 7.30 (m, 7H), 7.26 (dd, J = 7.0, 2.6 Hz, 2H), 7.00 (t, J = 8.1 Hz, 1H), 6.55 (dd, J = 8.1, 0.7 Hz, 1H), 6.34 (dd, J = 8.3, 0.8 Hz, 1H), 4.96 (s, 2H), 1.34 (d, J = 7.2 Hz, 6H). LCMS *m*/*z* 544.0 [M+H]⁺

### Step 3. 4-[1-(4-fluorophenyl)-4-hydroxy-2-isopropyl-indol-3-yl]sulfonylbenzoic acid (213)

To a suspension of Pd / C (50 mg, 0.04698 mmol) in EtOH (5 mL) was added a solution of 4-[4-benzyloxy-1-(4-fluorophenyl)-2-isopropyl-indol-3-yl]sulfonylbenzoic acid (76 mg, 0.1398 mmol) in EtOH (10 mL). The reaction mixture was stirred for 1 hour under 1 atm hydrogen then filtered over Celite^{®}. The filtrate was concentrated to dryness and purified via silica gel chromatography eluting with 0-15% MeOH in dichloromethane. Pure fractions were combined and concentrated to afford 15 mg white solid. 4-[1-(4-fluorophenyl)-4-hydroxy-2-isopropyl-indol-3-yl]sulfonylbenzoic acid (15 mg, 22%). ¹H NMR (400 MHz, DMSO-*d*₆) δ 13.49 (s, 1H), 9.63 (s, 1H), 8.17 - 8.11 (m, 2H), 8.07 - 7.99 (m, 2H), 7.69 - 7.60 (m, 2H), 7.53 - 7.44 (m, 2H), 7.02 (t, J = 8.0 Hz, 1H), 6.65 - 6.58 (m, 1H), 6.17 (dd, J = 8.3, 0.9 Hz, 1H), 4.03 (q, J = 7.2 Hz, 1H), 1.08 (d, J = 7.2 Hz, 6H). LCMS *m*/*z* 454.0 [M+H]⁺

### Compound 214

### 4-[2-[2-(difluoromethoxy)-1,1-dimethyl-ethyl]-61luoro-1-(4-fluorophenyl)-4-hydroxy-indol-3-yl]benzoic acid (214)

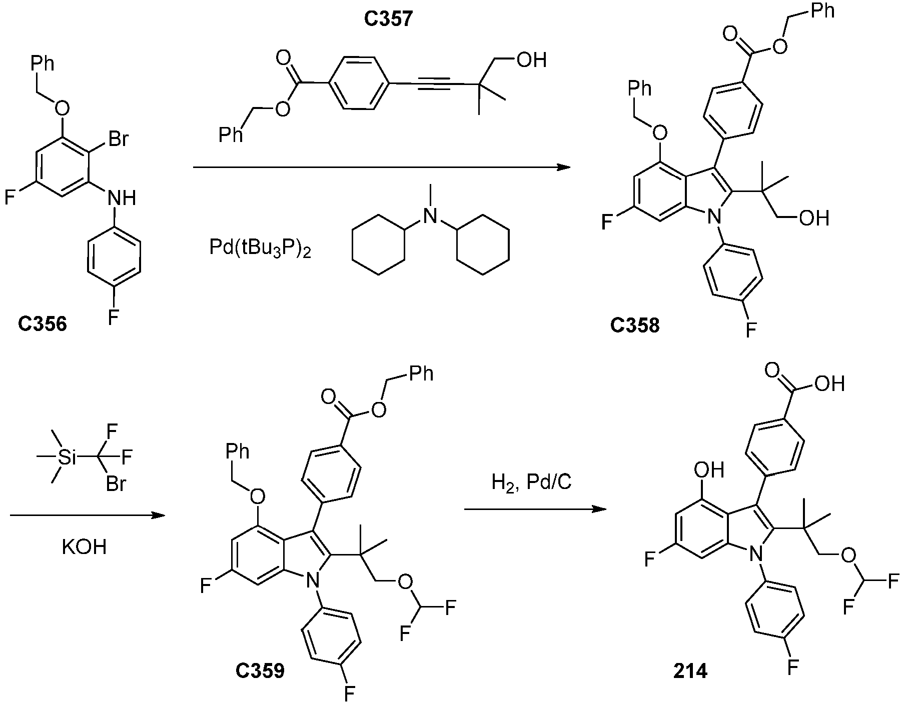

### Step 1. Synthesis of benzyl 4-[4-benzyloxy-6-fluoro-1-(4-fluorophenyl)-2-(2-hydroxy-1,1-dimethyl-ethyl)indol-3-yl]benzoate (C358)

A mixture of 3-benzyloxy-2-bromo-5-fluoro-N-(4-fluorophenyl)aniline (265 mg, 0.68 mmol), benzyl 4-(4-hydroxy-3,3-dimethyl-but-1-ynyl)benzoate (255 mg, 0.82 mmol) and N-cyclohexyl-N-methyl-cyclohexanamine (450 µL, 2.1 mmol) in a 5 ml vial. The mixture was flushed with nitrogen. Dioxane (3 mL) was added and bubbled with nitrogen for 5 minutes. Pd(tBu₃P)₂ (18 mg, 0.035 mmol). The mixture was heated to 100 °C over 12 hours, then to 120 °C for 6 hours. Concentration and purification by silica gel chromatography (0-50% EtOAc in heptane) afforded the product as a light yellow solid. Benzyl 4-[4-benzyloxy-6-fluoro-1-(4-fluorophenyl)-2-(2-hydroxy-1,1-dimethyl-ethyl)indol-3-yl]benzoate (288 mg, 69%). ¹H NMR (400 MHz, Chloroform-d) δ 7.88 - 7.79 (m, 2H), 7.48 - 7.38 (m, 4H), 7.38 - 7.25 (m, 6H), 7.13 (ddt, J = 8.8, 6.6, 2.1 Hz, 2H), 7.00 - 6.88 (m, 3H), 6.66 - 6.56 (m, 2H), 6.21 (dd, J = 11.5, 2.1 Hz, 1H), 5.87 (dd, J = 9.4, 2.1 Hz, 1H), 5.32 (s, 2H), 4.65 (s, 2H), 3.18 (s, 2H), 0.91 (s, 6H). LCMS *m*/*z* 618.37 [M+H]⁺

### Step 2. Synthesis of benzyl 4-[4-benzyloxy-2-[2-(difluoromethoxy)-1,1-dimethyl-ethyl]-6-fluoro-1-(4-fluorophenyl)indol-3-yl]benzoate (C359)

A solution of 4-[4-benzyloxy-6-fluoro-1-(4-fluorophenyl)-2-(2-hydroxy-1,1-dimethylethyl)indol-3-yl]benzoate (70 mg, 0.1133 mmol) in dichloromethane (354 µL) was cooled down to 0 oC. KOH (160 µL of 20% w/w, 0.6787 mmol) was added and the reaction was stirred vigorously for 5 minutes. [Bromo(difluoro)methyl]-trimethyl-silane (35.2 µL, 0.2263 mmol) in dichloromethane (88.4 µL) was added and the reaction was stirred for 4 hours. Water and dichloromethane were added and organic layer collected through phase separator. Purified by normal phase chromatography (0 - 60% dichloromethane/heptane) to give benzyl 4-[4-benzyloxy-2-[2-(difluoromethoxy)-1,1-dimethyl-ethyl]-6-fluoro-1-(4-fluorophenyl)indol-3-yl]benzoate (31 mg, 38%). ¹H NMR (400 MHz, DMSO-*d*₆) δ 7.91 - 7.86 (m, 2H), 7.58 - 7.37 (m, 11H), 7.06 - 6.95 (m, 3H), 6.75 - 6.54 (m, 4H), 5.90 (dd, J = 9.5, 2.1 Hz, 1H), 5.41 (s, 2H), 4.84 (s, 2H), 3.54 (s, 2H), 0.97 (s, 6H). LCMS *m*/*z* 668.37 [M+H]⁺

### Step 3. Synthesis of 4-[2-[2-(difluoromethoxy)-1,1-dimethyl-ethyl]-6-fluoro-1-(4-fluorophenyl)-4-hydroxy-indol-3-yl]benzoic acid (214)

A solution of benzyl 4-[4-benzyloxy-2-[2-(difluoromethoxy)-1,1-dimethyl-ethyl]-6-fluoro-1-(4-fluorophenyl)indol-3-yl]benzoate (31 mg, 0.04 mmol) in THF (878 µL) and EtOH (878 µL) was added Pd on carbon (12.5 mg of 10% w/w, 0.012 mmol) and stirred under H₂ (1.3 mg, 0.65 mmol) (balloon) until completion. The mixture was filtered through Celite^{®}. Purification by reverse phase chromatography (0-100% MeCN + 0.2% formic acid) gave 4-[2-[2-(difluoromethoxy)-1,1-dimethyl-ethyl]-6-fluoro-1-(4-fluorophenyl)-4-hydroxy-indol-3-yl]benzoic acid (12.4 mg, 58%) LCMS *m*/*z* 488.28 [M+H]⁺

### Compounds 215-221

### Step 2. Synthesis of benzyl 4-[4-benzyloxy-2-[2-(difluoromethoxy)-1,1-dimethyl-ethyl]-6-fluoro-1-(4-fluorophenyl)indol-3-yl]benzoate (C359)

Compounds **215-221** (Table 9) were prepared by Larock indole cyclization between aryl anilines (Reactant A) and an alkyne (Reactant B) using the method described for the preparation of compound **147.** The phenol moiety in reactant A was protected with a benzyl group or a MOM group. The appropriate reagent for removal of the ether protecting group was used in each case. MOM group removal was performed as described with HCl as used in the preparation of compound **141.** Benzyl group removal was performed according to the hydrogenation procedure as described for the preparation of compound **147 or 191.**

**Table 9. Method of preparation, structure and physicochemical data for compounds 215-221**

| **Compound** | **Structure** | **Reactant A** | **Reactant B** |
|---|---|---|---|
| **215** | 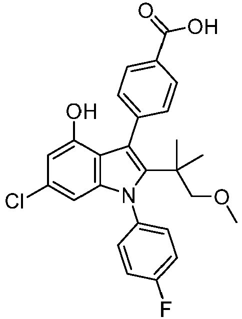 | 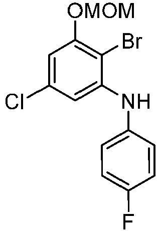 | 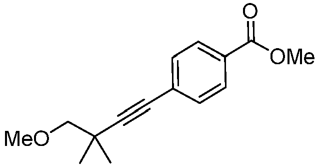 |
| **216** | 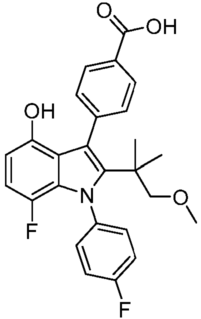 | 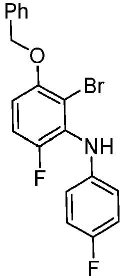 | 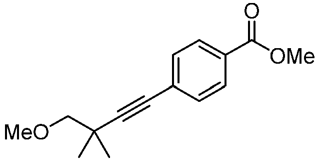 |
| **217** | 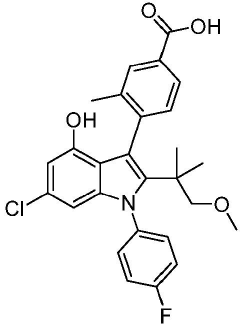 | 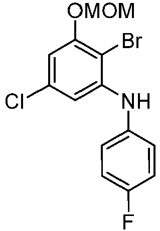 | 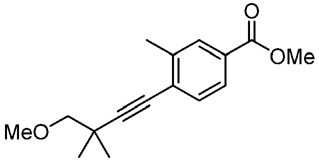 |
| **218** | 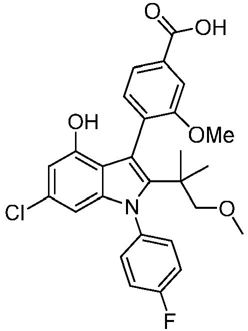 | 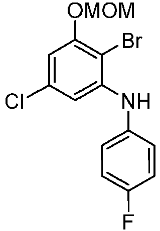 | 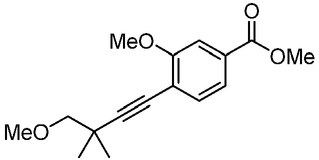 |
| **219¹** | 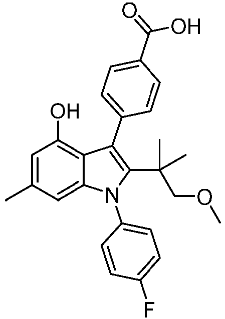 | 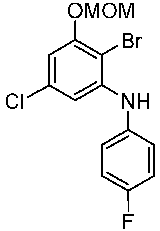 | 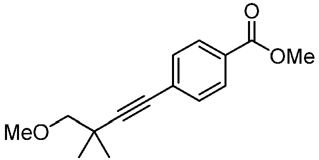 |
| **220** | 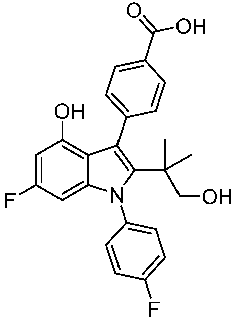 | 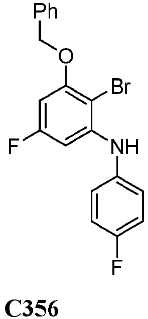 | 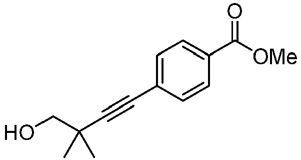 |
| **221** | 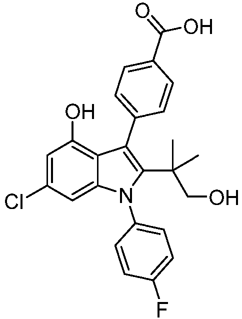 | 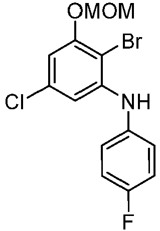 | 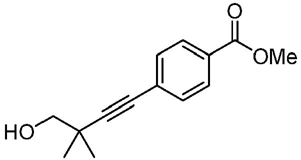 |

| | | | |
|---|---|---|---|
| 1. A Suzuki reaction with trimethyl boroxine was used to install the methyl group before the final deprotection steps. | | | |

**Table 10. NMR and LCMS Data for compounds 215-221**

| **Compound** | **¹H NMR; *LCMS m*/*z* [M+H]⁺** |
|---|---|
| **215** | ¹H NMR (400 MHz, DMSO-*d*₆) δ 12.87 (s, 1H), 9.44 (s, 1H), 7.96 - 7.81 (m, 2H), 7.60 - 7.39 (m, 6H), 6.29 (d, J = 1.8 Hz, 1H), 5.94 (d, J = 1.8 Hz, 1H), 2.99 (s, 3H), 2.91 (s, 2H), 0.97 (s, 6H). LCMS *m*/*z* [M+H]⁺ 468.29 |
| **216** | ¹H NMR (400 MHz, DMSO-*d*₆) δ 8.82 (s, 1H), 7.90 - 7.84 (m, 2H), 7.62 - 7.56 (m, 2H), 7.45 - 7.40 (m, 2H), 7.36 - 7.29 (m, 2H), 6.54 (dd, J = 12.5, 8.4 Hz, 1H), 6.13 (dd, J = 8.4, 3.0 Hz, 1H), 2.99 (s, 3H), 2.87 (s, 2H), 0.96 (s, 6H). LCMS *m*/*z* [M+H]⁺ 452 |
| **217** | ¹H NMR (400 MHz, Chloroform-d) δ 8.08 (s, 1H), 8.01 (dd, J = 8.0, 1.8 Hz, 1H), 7.62 (d, J = 7.8 Hz, 1H), 7.49 - 7.36 (m, 2H), 7.26 - 7.21 (m, 2H), 6.45 (d, J = 1.7 Hz, 1H), 6.17 (d, J = 1.7 Hz, 1H), 4.44 (s, 1H), 3.08 (s, 3H), 2.97 (s, 2H), 2.33 (s, 3H), 1.03 (d, J = 1.7 Hz, 7H). LCMS *m*/*z* [M+H]⁺ 482.33 |
| **218** | ¹H NMR (400 MHz, Chloroform-d) δ 7.86 (dd, J = 7.7, 1.6 Hz, 1H), 7.76 (d, J = 1.6 Hz, 1H), 7.63 (d, J = 7.7 Hz, 1H), 7.58 - 7.47 (m, 1H), 7.46 - 7.39 (m, 1H), 7.27 - 7.21 (m, 1H), 6.48 (d, J = 1.7 Hz, 1H), 6.21 (d, J = 1.7 Hz, 1H), 3.93 (s, 3H), 3.09 (s, 3H), 3.06 - 2.95 (m, 2H), 1.08 (d, J = 3.7 Hz, 6H). LCMS *m*/*z* [M+H]⁺ 498.4 |
| **219** | ¹H NMR (400 MHz, Chloroform-d) δ 8.10 (d, J = 7.8 Hz, 2H), 7.56 (d, J = 7.7 Hz, 2H), 7.31 (dd, J = 8.5, 4.9 Hz, 2H), 7.13 (t, J = 8.4 Hz, 2H), 6.19 (s, 1H), 5.88 (s, 1H), 2.96 (s, 3H), 2.86 (s, 2H), 2.14 (s, 3H), 0.93 (s, 7H). LCMS *m*/*z* [M+H]⁺ 448.36 |
| **220** | ¹H NMR (400 MHz, Chloroform-*d*/CD₃OD) δ 7.99 (dq, J = 8.3, 1.8 Hz, 2H), 7.59 - 7.51 (m, 2H), 7.41 - 7.31 (m, 2H), 7.22 - 7.10 (m, 3H), 6.16 - 6.07 (m, 1H), 5.76 (dd, J = 9.6, 2.3 Hz, 1H), 3.24 (s, 2H), 0.93 (d, J = 2.2 Hz, 6H). LCMS *m*/*z* [M+H]⁺ 438.2 |
| **221** | ¹H NMR (400 MHz, Methanol-*d*₄/CDCl₃) δ 8.08 - 8.03 (m, 2H), 7.62 - 7.58 (m, 2H), 7.48 - 7.43 (m, 2H), 7.29 - 7.21 (m, 2H), 6.38 (d, J = 1.7 Hz, 1H), 6.12 (d, J = 1.7 Hz, 1H), 3.33 (s, 2H), 1.01 (s, 6H). LCMS *m*/*z* [M+H]⁺ 454.26 |

### Compound 222

### 4-[2-(3-cyano-1,1[-dimethyl-propyl)-1-(4-fluorophenyl)-4-hydroxy-indol-3-yl]benzoic acid (222)

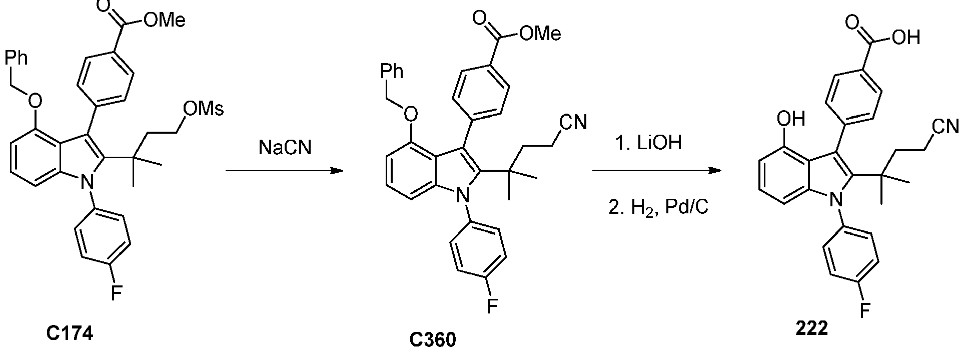

### Step 1. Synthesis of methyl 4-[4-benzyloxy-2-(3-cyano-1,1-dimethyl-propyl)-1-(4-fluorophenyl)indol-3-yl]benzoate (C360)

To a solution of methyl 4-[4-benzyloxy-2-(1,1-dimethyl-3-methylsulfonyloxy-propyl)-1-(4-fluorophenyl)indol-3-yl]benzoate (220 mg, 0.36 mmol) in NMP (3 mL) at 90 oC was added NaCN (145 mg, 2.96 mmol). The reaction mixture was stirred at 90 oC for 3 hour then diluted with sat. aq. sodium bicarbonate and extracted with EtOAc. The organic layer was concentrated to dryness and purified via silica gel chromatography eluting with 0-35% EtOAc in heptane. Pure fractions were combined and concentrated to give 112 mg white solid. methyl 4-[4-benzyloxy-2-(3-cyano-1,1-dimethyl-propyl)-1-(4-fluorophenyl)indol-3-yl]benzoate (112 mg, 57%). ¹H NMR (400 MHz, Chloroform-d) δ 7.91 - 7.85 (m, 2H), 7.47 - 7.42 (m, 2H), 7.42 - 7.35 (m, 2H), 7.30 - 7.26 (m, 2H), 7.19 - 7.10 (m, 3H), 7.02 (t, J = 8.1 Hz, 1H), 6.79 - 6.74 (m, 2H), 6.53 (dd, J = 7.9, 0.7 Hz, 1H), 6.26 (dd, J = 8.3, 0.7 Hz, 1H), 4.80 (s, 2H), 3.96 (s, 3H), 2.23 - 2.17 (m, 2H), 1.73 - 1.67 (m, 2H), 1.06 (s, 6H). LCMS *m*/*z* 547.0 [M+H]⁺

Compound **222** was prepared in two steps from **C360** by ester hydrolysis and hydrogenation described for the preparation of compound **147.** 4-[2-(3-cyano-1,1-dimethylpropyl)-1-(4-fluorophenyl)-4-hydroxy-indol-3-yl]benzoic acid (13 mg, 17%) ¹H NMR (400 MHz, DMSO-*d*₆) δ 12.86 (s, 1H), 8.90 (s, 1H), 7.93 - 7.87 (m, 2H), 7.59 - 7.54 (m, 2H), 7.53 - 7.49 (m, 2H), 7.48 - 7.42 (m, 2H), 6.81 - 6.74 (m, 1H), 6.27 (dd, J = 7.6, 0.8 Hz, 1H), 5.96 (dd, J = 8.2, 0.8 Hz, 1H), 2.37 - 2.29 (m, 2H), 1.56 (dd, J = 9.1, 7.0 Hz, 2H), 1.01 (s, 6H). LCMS *m*/*z* 443.0 [M+H]⁺

### Compound 223

### 4-[6-chloro-2-(2-cyano-1,1-dimethyl-ethyl)-1-(4-fluorophenyl)-4-hydroxy-indol-3-yl]benzoic acid (223)

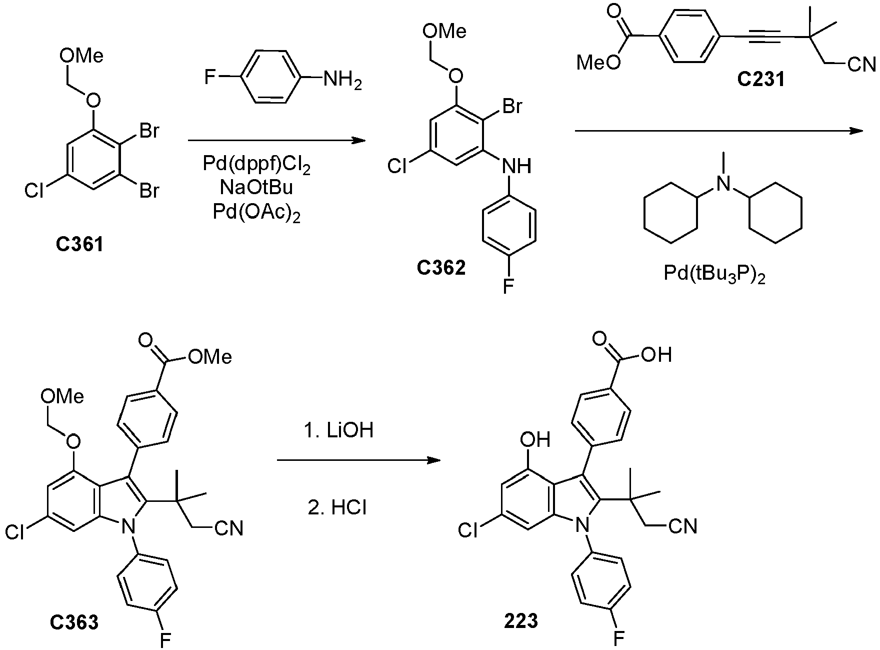

Compound 223 was prepared from C361 and C362 using the method described for the preparation of compound 191. In this example, HCl was used to remove the MOM protecting group, using the method described for the preparation of compound 214. 4-[6-chloro-2-(2-cyano-1,1-dimethyl-ethyl)-1-(4-fluorophenyl)-4-hydroxy-indol-3-yl]benzoic acid (26.8 mg, 47%). ¹H NMR (400 MHz, DMSO-*d*₆) δ 12.95 (s, 1H), 9.59 (s, 1H), 7.96 - 7.89 (m, 2H), 7.64 - 7.56 (m, 2H), 7.57 - 7.44 (m, 4H), 6.33 (d, J = 1.7 Hz, 1H), 5.99 (d, J = 1.7 Hz, 1H), 2.47 - 2.38 (m, 2H), 1.14 (d, J = 14.4 Hz, 6H). LCMS *m*/*z* 463.25 [M+H]⁺

### Compounds 224-241

Compounds **224-241** (Table 11) were prepared from the appropriate disubstituted alkyne and halogen aryl halide as described in the preparation of compounds **214** and **223,** and as shown in table 9 for the preparation of compounds **215-221.** Alkynes were protected with a methyl or benzyl ester.

**Table 11. Method of preparation, structure, physicochemical data for compounds 224-241**

| **Compound** | **Structure** | **¹H NMR; LCMS m/z [M+H]⁺** |
|---|---|---|
| ***224*** | 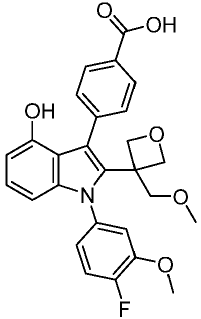 | ¹H NMR (400 MHz, DMSO-*d*₆) δ 12.86 (s, 1H), 9.28 (s, 1H), 7.91 - 7.85 (m, 2H), 7.64 - 7.57 (m, 2H), 7.47 - 7.35 (m, 2H), 7.10 (ddd, J = 8.5, 3.9, 2.4 Hz, 1H), 6.91 - 6.85 (m, 1H), 6.43 (dd, J = 7.7, 0.8 Hz, 1H), 6.24 (dd, J = 8.2, 0.8 Hz, 1H), 4.49 (d, J = 5.9 Hz, 1H), 4.31 (d, J = 5.9 Hz, 1H), 3.90 - 3.81 (m, 5H), 3.72 (d, J = 5.9 Hz, 1H), 3.65 (d, J = 5.8 Hz, 1H), 3.38 (s, 3H). LCMS *m*/*z* 478.31 [M+H]⁺ |
| ***225*** | 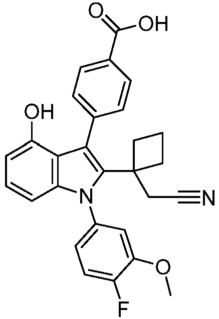 | ¹H NMR (400 MHz, Chloroform-*d*) δ 8.15 (d, J = 8.7 Hz, 2H), 7.76 (d, J = 8.8 Hz, 2H), 7.16 (d, J = 8.5 Hz, 1H), 7.00 (dd, J = 7.7, 2.6 Hz, 1H), 6.98 - 6.91 (m, 2H), 6.48 (d, J = 7.6 Hz, 1H), 6.33 (d, J = 8.2 Hz, 1H), 3.83 (d, J = 2.9 Hz, 3H), 2.79 (d, J = 3.6 Hz, 2H), 2.24 (q, J = 10.3, 9.8 Hz, 2H), 1.81 (d, J = 10.7 Hz, 1H), 1.60 (d, J = 10.2 Hz, 1H), 1.48 (d, J = 9.8 Hz, 2H). LCMS *m*/*z* 471.2 [M+H]⁺ |
| ***226*** | 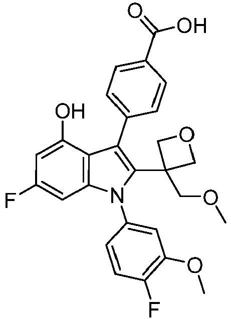 | ¹H NMR (400 MHz, DMSO-*d*₆) δ 12.88 (s, 1H), 9.87 (s, 1H), 7.93 - 7.85 (m, 2H), 7.64 - 7.56 (m, 2H), 7.47 - 7.36 (m, 2H), 7.11 (ddd, J = 8.5, 4.0, 2.5 Hz, 1H), 6.28 (dd, J = 11.4, 2.2 Hz, 1H), 6.00 (dd, J = 9.6, 2.2 Hz, 1H), 4.48 (d, J = 5.9 Hz, 1H), 4.31 (d, J = 5.9 Hz, 1H), 3.90 - 3.77 (m, 5H), 3.71 (d, J = 5.9 Hz, 1H), 3.64 (d, J = 5.7 Hz, 1H), 3.39 (s, 3H). LCMS *m*/*z* 496.24 [M+H]⁺ |
| ***227*** | 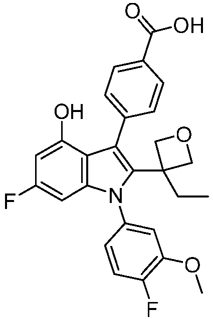 | ¹H NMR (400 MHz, DMSO-*d*₆) δ 12.90 (s, 1H), 9.79 (s, 1H), 7.94 - 7.88 (m, 2H), 7.46 (d, J = 8.1 Hz, 2H), 7.41 (dd, J = 11.2, 8.5 Hz, 1H), 7.34 (dd, J = 7.8, 2.5 Hz, 1H), 7.06 (ddd, J = 8.5, 3.9, 2.4 Hz, 1H), 6.25 (dd, J = 11.4, 2.2 Hz, 1H), 5.96 (dd, J = 9.6, 2.2 Hz, 1H), 4.58 (d, J = 5.7 Hz, 1H), 4.39 (d, J = 5.8 Hz, 1H), 3.85 (s, 3H), 3.62 (dd, J = 9.0, 5.8 Hz, 2H), 2.00 (q, J = 8.2, 7.7 Hz, 2H), 1.05 (t, J = 7.4 Hz, 3H). LCMS *m*/*z* 480.22 [M+H]⁺ |
| ***228*** | 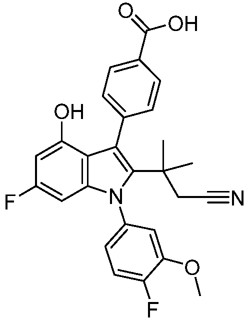 | ¹H NMR (400 MHz, Chloroform-*d*) δ 8.10 (d, J = 8.1 Hz, 2H), 7.72 - 7.57 (m, 2H), 7.36 - 7.15 (m, 1H), 7.13 - 6.96 (m, 2H), 6.20 (dd, J = 11.0, 2.1 Hz, 1H), 5.89 (dd, J = 9.5, 2.1 Hz, 1H), 3.89 (s, 3H), 2.33 (d, J = 2.2 Hz, 2H), 1.22 (d, J = 10.4 Hz, 6H). LCMS *m*/*z* 477.25 [M+H]⁺ |
| ***229*** | 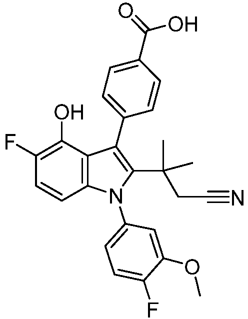 | ¹H NMR (400 MHz, Chloroform-*d*/CD3OD) δ 8.04 (d, J = 8.0 Hz, 2H), 7.57 (t, J = 9.6 Hz, 2H), 7.31 - 7.12 (m, 2H), 7.00 (ddd, J = 18.4, 8.0, 3.2 Hz, 2H), 6.80 (t, J = 9.9 Hz, 1H), 6.05 (dd, J = 8.9, 3.5 Hz, 1H), 3.84 (s, 3H), 2.30 (d, J = 3.5 Hz, 2H), 1.19 (d, J = 8.3 Hz, 6H). LCMS *m*/*z* 478.02 [M+H]⁺ |
| ***230*** | 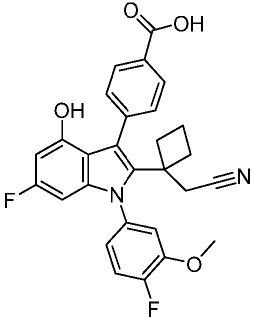 | ¹H NMR (400 MHz, Chloroform-*d*) δ 8.34 - 8.22 (m, 2H), 7.85 (dd, J = 8.2, 2.2 Hz, 2H), 7.28 (dd, J = 10.7, 8.4 Hz, 1H), 7.12 - 6.97 (m, 2H), 6.37 (dd, J = 10.8, 2.1 Hz, 1H), 6.11 (dd, J = 9.3, 2.1 Hz, 1H), 3.94 (s, 3H), 2.87 (d, J = 3.3 Hz, 2H), 2.32 (q, J = 10.5 Hz, 2H), 1.90 (h, J = 10.1 Hz, 1H), 1.70 (dt, J = 11.4, 9.2 Hz, 1H), 1.63 - 1.48 (m, 2H). LCMS *m*/*z* 489.29 [M+H]⁺ |
| **231** | 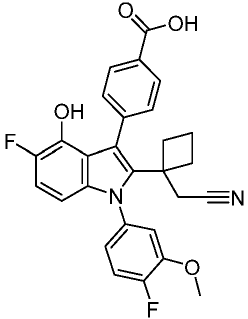 | ¹H NMR (400 MHz, Chloroform-*d*) δ 8.21 - 8.04 (m, 2H), 7.77 - 7.64 (m, 2H), 7.22 - 7.17 (m, 1H), 7.00 (dd, J = 7.5, 2.4 Hz, 1H), 6.94 (ddd, J = 8.4, 3.9, 2.4 Hz, 1H), 6.85 (dd, J = 10.6, 8.9 Hz, 1H), 6.19 (dd, J = 8.9, 3.6 Hz, 1H), 3.83 (s, 3H), 2.80 (d, J = 2.2 Hz, 2H), 2.24 (q, J = 9.7 Hz, 2H), 1.89 - 1.73 (m, 1H), 1.67 - 1.55 (m, 1H), 1.55 - 1.35 (m, 2H). LCMS *m*/*z* 489.2 [M+H]⁺ |
| **232** | 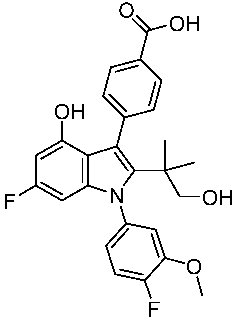 | ¹H NMR (400 MHz, Chloroform-*d*) δ 8.13 - 7.99 (m, 2H), 7.66 - 7.55 (m, 2H), 7.17 - 7.10 (m, 1H), 7.03 - 6.97 (m, 1H), 6.97 - 6.89 (m, 1H), 6.18 (dd, J = 10.8, 2.2 Hz, 1H), 5.89 (dd, J = 9.5, 2.1 Hz, 1H), 3.83 (s, 3H), 3.30 (d, J = 1.4 Hz, 2H), 0.98 (d, J = 2.8 Hz, 6H). LCMS *m*/*z* 468.23 [M+H]⁺ |
| **233** | 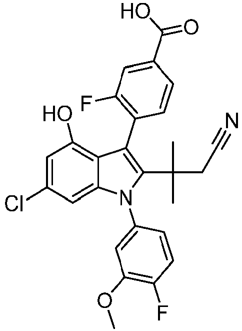 | ¹H NMR (400 MHz, DMSO-*d*₆) δ 13.28 (s, 1H), 9.69 (d, J = 4.7 Hz, 1H), 7.79 (ddd, J = 7.9, 4.7, 1.7 Hz, 1H), 7.69 (dt, J = 9.6, 1.7 Hz, 1H), 7.59 (q, J = 7.7 Hz, 1H), 7.55 - 7.48 (m, 1H), 7.48 - 7.28 (m, 1H), 7.15 (dddd, J = 48.7, 8.5, 3.9, 2.5 Hz, 1H), 6.35 (t, J = 1.5 Hz, 1H), 6.10 (dd, J = 4.6, 1.7 Hz, 1H), 3.89 (d, J = 8.7 Hz, 3H), 2.56 (dd, J = 7.1, 5.6 Hz, 2H), 1.22 - 1.14 (m, 6H). LCMS *m*/*z* 511 [M+H]⁺ |
| **234** | 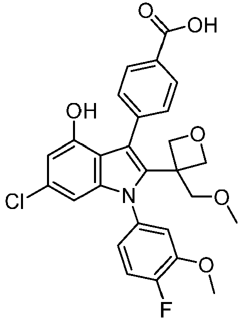 | ¹H NMR (400 MHz, DMSO-*d*₆) δ 12.90 (s, 1H), 9.85 (s, 1H), 7.95 - 7.84 (m, 2H), 7.62 - 7.57 (m, 2H), 7.49 - 7.34 (m, 2H), 7.11 (ddd, J = 8.5, 3.9, 2.5 Hz, 1H), 6.45 (d, J = 1.8 Hz, 1H), 6.23 (d, J = 1.7 Hz, 1H), 4.49 (d, J = 5.9 Hz, 1H), 4.31 (d, J = 5.9 Hz, 1H), 3.91 - 3.78 (m, 5H), 3.70 (d, J = 5.8 Hz, 1H), 3.64 (d, J = 5.8 Hz, 1H), 3.38 (s, 3H). LCMS *m*/*z* 512.24 [M+H]⁺ |
| **235** | 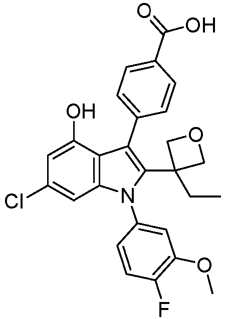 | ¹H NMR (400 MHz, DMSO-*d*₆) δ 12.92 (s, 1H), 9.78 (s, 1H), 7.96 - 7.89 (m, 2H), 7.49 - 7.39 (m, 3H), 7.36 (dd, J = 7.8, 2.5 Hz, 1H), 7.08 (ddd, J = 8.5, 3.9, 2.4 Hz, 1H), 6.43 (d, J = 1.8 Hz, 1H), 6.20 (d, J = 1.7 Hz, 1H), 4.60 (d, J = 5.7 Hz, 1H), 4.40 (d, J = 5.8 Hz, 1H), 3.85 (s, 3H), 3.63 (t, J = 6.1 Hz, 2H), 2.06 - 1.96 (m, 2H), 1.05 (t, J = 7.4 Hz, 3H). LCMS *m*/*z* 496.23 [M+H]⁺ |
| **236** | 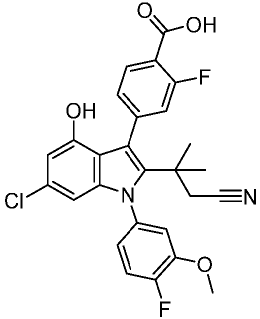 | ¹H NMR (400 MHz, DMSO-*d*₆) δ 13.27 (s, 1H), 9.69 (s, 1H), 7.83 (td, J = 8.2, 1.8 Hz, 1H), 7.49 (ddd, J = 11.2, 8.5, 1.5 Hz, 1H), 7.39 - 7.24 (m, 3H), 7.12 (ddd, J = 8.6, 3.9, 2.5 Hz, 1H), 6.35 (d, J = 1.7 Hz, 1H), 6.09 (t, J = 1.8 Hz, 1H), 3.88 (d, J = 2.2 Hz, 3H), 2.56 - 2.53 (m, 2H), 1.23 - 1.09 (m, 6H). LCMS *m*/*z* 511.25 [M+H]⁺ |
| **237** | 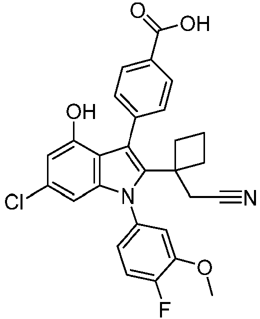 | ¹H NMR (400 MHz, Chloroform-*d)* δ 8.08 (d, J = 7.8 Hz, 2H), 7.69 (t, J = 5.3 Hz, 2H), 7.26 (tq, J = 6.7, 3.9 Hz, 1H), 7.07 (dt, J = 7.6, 3.6 Hz, 1H), 7.01 (dq, J = 8.5, 3.4 Hz, 1H), 6.55 - 6.44 (m, 1H), 6.40 - 6.25 (m, 1H), 3.92 (d, J = 2.9 Hz, 3H), 2.88 (d, J = 6.2 Hz, 2H), 2.41 - 2.19 (m, 2H), 1.87 (dh, J = 19.0, 10.4, 9.5 Hz, 1H), 1.75 - 1.59 (m, 1H), 1.52 (t, J = 12.5 Hz, 2H). LCMS *m*/*z* 505.18 [M+H]⁺ |
| **238** | 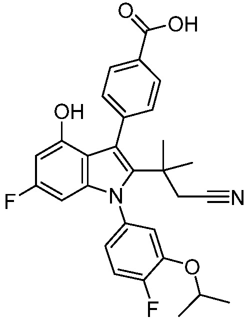 | ¹H NMR (400 MHz, Chloroform-*d*) δ 8.36 - 8.23 (m, 2H), 7.84 - 7.74 (m, 2H), 7.31 - 7.23 (m, 1H), 7.11 (dd, J = 7.3, 2.5 Hz, 1H), 7.05 (ddd, J = 8.5, 3.9, 2.5 Hz, 1H), 6.30 (dd, J = 10.8, 2.2 Hz, 1H), 5.98 (dd, J = 9.4, 2.1 Hz, 1H), 4.61 (p, J = 6.1 Hz, 1H), 2.42 (s, 2H), 1.43 (d, J = 6.0 Hz, 6H), 1.29 (d, J = 8.0 Hz, 6H). LCMS *m*/*z* 505.27 [M+H]⁺ |
| **239** | 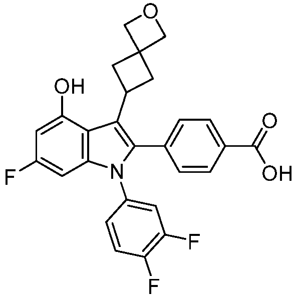 | ¹H NMR (400 MHz, Methanol-*d*₄) δ 7.97 (d, J = 7.6 Hz, 2H), 7.37 - 7.23 (m, 3H), 7.19 - 7.11 (m, 1H), 6.97 (d, J = 8.8 Hz, 1H), 6.35 (d, J = 10.4 Hz, 2H), 4.80 (s, 2H), 4.71 (s, 2H), 3.62 - 3.54 (m, 1H), 2.94 (t, J = 10.9 Hz, 2H), 2.57 (t, J = 10.4 Hz, 2H). LCMS *m*/*z* 480.21 [M+H]⁺ |
| **240** | 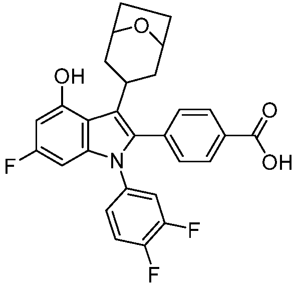 | ¹H NMR (400 MHz, Methanol-*d*₄) δ 7.97 (d, J = 7.7 Hz, 2H), 7.34 (d, J = 7.8 Hz, 2H), 7.31 - 7.19 (m, 1H), 7.17 - 7.07 (m, 1H), 6.97 (s, 1H), 6.32 (t, J = 9.3 Hz, 2H), 4.40 (s, 2H), 3.48 (s, 2H), 2.60 (t, J = 12.7 Hz, 2H), 1.89 (s, 2H), 1.67 (d, J = 7.6 Hz, 2H), 1.46 (d, J = 13.0 Hz, 2H). LCMS *m*/*z* 494.34 [M+H]⁺ |
| **241** | 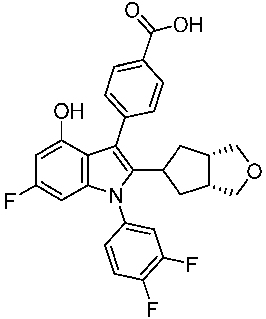 | ¹H NMR (400 MHz, DMSO-*d*₆) δ 12.92 (s, 1H), 9.79 (d, J = 2.6 Hz, 1H), 7.99 - 7.92 (m, 2H), 7.92 - 7.79 (m, 1H), 7.70 (td, J = 9.7, 9.1, 7.9 Hz, 1H), 7.54 - 7.49 (m, 2H), 7.48 - 7.39 (m, 1H), 6.26 (dd, J = 11.4, 2.2 Hz, 1H), 6.08 (dd, J = 9.6, 2.2 Hz, 1H), 3.79 (d, J = 10.1 Hz, 1H), 3.60 - 3.52 (m, 1H), 3.44 (q, J = 6.6 Hz, 1H), 2.99 (dd, J = 10.8, 6.9 Hz, 1H), 2.81 (ddd, J = 10.5, 7.0, 3.3 Hz, 1H), 1.93 - 1.74 (m, 3H), 1.63 - 1.51 (m, 1H), 1.47 - 1.25 (m, 2H). LCMS *m*/*z* 494.24 [M+H]⁺ |

### Compound 242-243

Compounds **242-243** (Table 12) were prepared as described for the preparation of compound **12.** A methyl ether protecting group was used instead of a MOM protecting group. The methyl ester and methyl ethers were simultaneously deprotected with AlCl₃ and octane-1-thiol (as described in the preparation of compound **179).**

**Table 12. Method of preparation, structure, physicochemical data for compounds 242-243**

| **Compound** | **Structure** | Method | ¹H NMR*;* ***LCMS m*/*z*** [M+H]⁺ |
|---|---|---|---|
| **242** | 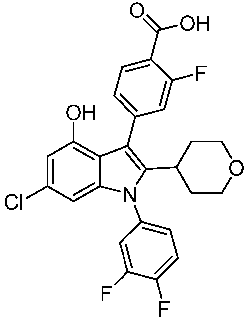 | As for compound 12 | ¹H NMR (400 MHz, DMSO-*d*₆) δ 13.24 (s, 1H), 9.84 (s, 1H), 7.91 - 7.77 (m, 2H), 7.77 - 7.65 (m, 1H), 7.41 (d, J = 9.0 Hz, 1H), 7.34 - 7.21 (m, 2H), 6.44 (d, J = 1.7 Hz, 1H), 6.31 (d, J = 1.7 Hz, 1H), 3.69 (d, J = 10.9 Hz, 2H), 3.12 - 2.97 (m, 2H), 2.79 (t, J = 12.1 Hz, 1H), 1.63 - 1.42 (m, 4H). LCMS *m*/*z* 501.9 [M+H]⁺ |
| **243** | 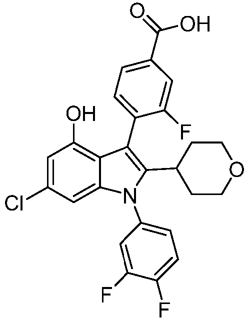 | As for compound 12 | ¹H NMR (400 MHz, DMSO-*d*₆) δ 13.29 (s, 1H), 9.72 (d, J = 1.8 Hz, 1H), 7.91 - 7.83 (m, 1H), 7.82 - 7.77 (m, 1H), 7.75 - 7.65 (m, 2H), 7.52 (q, J = 8.0 Hz, 1H), 7.42 (dd, J = 22.4, 8.8 Hz, 1H), 6.40 (d, J = 1.7 Hz, 1H), 6.35 (dd, J = 3.3, 1.7 Hz, 1H), 3.67 (s, 2H), 3.08 - 2.98 (m, 2H), 2.73 (d, J = 12.1 Hz, 1H), 1.63 (d, J = 12.9 Hz, 1H), 1.52 (s, 1H), 1.38 (t, J = 12.2 Hz, 2H). LCMS *m*/*z* 501.9 [M+H]⁺ |

### Compounds 244-250

Compound **244** (Table 13) was prepared from **S3** by Suzuki coupling with (2-fluoro-4-methoxycarbonyl-phenyl)boronic acid, then HATU coupling with ethanolamine, and finally benzyl group removal by hydrogenolysis. Compounds **245-250** (Table 14) were prepared from **S3** by Suzuki coupling with the appropriate boronic acid or ester as described for the preparation of compound **9.** Any modifications are noted in the table footnotes.

**Table 13. Method of preparation, structure, physicochemical data for compounds 244-250**

| **Compound** | **Structure** | **Method** | ¹H NMR*;* ***LCMS m*/*z*** [M+H]⁺ |
|---|---|---|---|
| **244¹** | 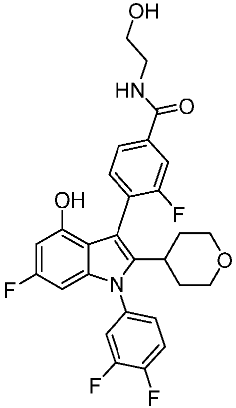 | From S3 See footnote for method. | ¹H NMR (400 MHz, Chloroform-*d*/CD3OD) δ 7.71 - 7.60 (m, 2H), 7.50 (t, J = 7.6 Hz, 1H), 7.44 (d, J = 9.2 Hz, 1H), 7.36 - 7.14 (m, 2H), 6.25 (dd, J = 11.2, 2.2 Hz, 1H), 6.09 (d, J = 9.6 Hz, 1H), 3.89 - 3.70 (m, 4H), 3.58 (t, J = 5.3 Hz, 2H), 3.19 (t, J = 11.8 Hz, 2H), 2.77 (t, J = 11.9 Hz, 1H), 1.80 - 1.49 (m, 4H). LCMS *m*/*z* 529.14 [M+H]⁺ |
| **245²** | 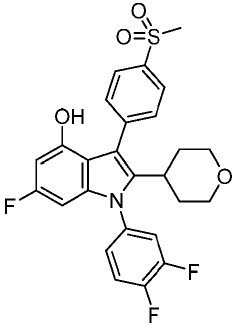 | From S3 As for Compound 9 | ¹H NMR (400 MHz, Chloroform-*d*/CD3OD) δ 7.88 - 7.76 (m, 2H), 7.62 - 7.48 (m, 2H), 7.34 - 7.27 (m, 1H), 7.15 (ddd, J = 10.0, 6.9, 2.5 Hz, 1H), 7.10 - 7.03 (m, 1H), 6.23 - 6.10 (m, 1H), 6.00 - 5.87 (m, 1H), 3.70 (d, J = 11.6 Hz, 2H), 3.14 - 2.98 (m, 5H), 2.73 (tt, J = 12.1, 3.6 Hz, 1H), 1.55 (qd, J = 12.4, 11.8, 4.2 Hz, 2H), 1.47 (s, 2H). LCMS *m*/*z* 501.99 [M+H]⁺ |
| **246²** | 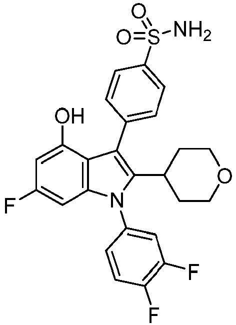 | From **S3** As for Compound **9** | ¹H NMR (400 MHz, Chloroform-*d*/CD3OD) δ 7.82 (dd, J = 8.3, 1.7 Hz, 2H), 7.54 - 7.44 (m, 2H), 7.36 - 7.27 (m, 1H), 7.15 (t, J = 8.8 Hz, 1H), 7.10 - 7.00 (m, 1H), 6.15 (dd, J = 11.1, 2.1 Hz, 1H), 5.95 (dd, J = 9.3, 2.0 Hz, 1H), 3.72 - 3.63 (m, 2H), 3.13 - 2.98 (m, 2H), 2.71 (td, J = 12.3, 6.3 Hz, 1H), 1.64 - 1.49 (m, 2H), 1.45 (d, J = 13.5 Hz, 2H). LCMS *m*/*z* 503.13 [M+H]⁺ |
| **247²** | 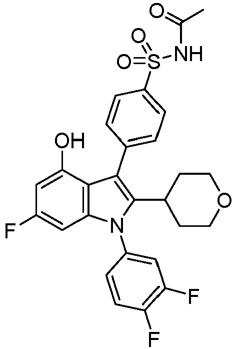 | From **S3** As for Compound **9** | ¹H NMR (400 MHz, Chloroform-*d*) δ 8.12 - 7.99 (m, 2H), 7.70 - 7.59 (m, 2H), 7.42 (t, J = 8.7 Hz, 1H), 7.39 - 7.32 (m, 1H), 7.28 (td, J = 9.4, 8.3, 5.3 Hz, 1H), 7.20 (s, 1H), 6.34 - 6.21 (m, 1H), 6.16 - 6.01 (m, 1H), 3.82 (d, J = 10.9 Hz, 2H), 3.27 - 3.13 (m, 2H), 2.86 (td, J = 10.4, 9.0, 5.2 Hz, 1H), 2.16 - 1.97 (m, 3H), 1.63 (s, 3H). LCMS *m*/*z* 544.93 [M+H]⁺ |
| ***248³*** | 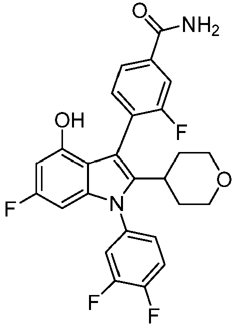 | From **S3.** See footnote for method. | ¹H NMR (400 MHz, Chloroform-*d*) δ 7.62 - 7.50 (m, 2H), 7.38 (dd, J = 8.3, 7.0 Hz, 1H), 7.36 - 7.27 (m, 1H), 7.22 - 7.13 (m, 1H), 7.13 - 7.03 (m, 1H), 6.13 (dd, J = 11.1, 2.1 Hz, 1H), 5.96 (ddd, J = 9.4, 2.2, 0.9 Hz, 1H), 3.73 - 3.61 (m, 2H), 3.06 (ddd, J = 14.8, 10.9, 2.8 Hz, 2H), 2.79 - 2.60 (m, 1H), 1.67 - 1.34 (m, 5H). LCMS *m*/*z* 485.13 [M+H]⁺ |
| ***249²*** | 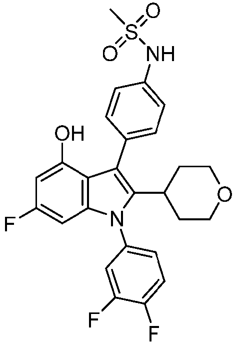 | From **S3** As for Compound **9** | ¹H NMR (400 MHz, DMSO-*d*₆) δ 9.80 (s, 1H), 9.65 - 9.56 (m, 1H), 7.80 - 7.58 (m, 3H), 7.40 - 7.25 (m, 3H), 7.25 - 7.14 (m, 2H), 6.27 - 5.97 (m, 1H), 3.67 (dd, J = 10.9, 3.0 Hz, 2H), 3.09 - 2.97 (m, 5H), 2.74 (tt, J = 10.0, 4.9 Hz, 1H), 1.51 (td, J = 11.7, 10.5, 4.4 Hz, 4H). LCMS *m*/*z* 517.21 [M+H]⁺ |
| ***250²*** | 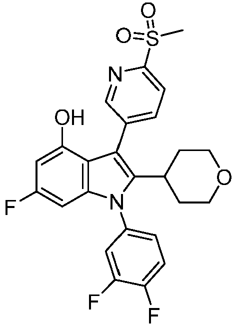 | From **S3** Compound **9** | ¹H NMR (400 MHz, Chloroform-*d*) δ 8.53 (dd, J = 1.9, 1.0 Hz, 1H), 7.91 - 7.79 (m, 2H), 7.19 (dt, J = 9.7, 8.6 Hz, 1H), 7.04 (ddd, J = 10.0, 6.9, 2.4 Hz, 1H), 6.95 (ddd, J = 8.4, 3.3, 1.8 Hz, 1H), 6.06 - 5.90 (m, 1H), 5.76 (dd, J = 9.3, 2.1 Hz, 1H), 3.63 - 3.51 (m, 2H), 3.06 - 3.00 (m, 4H), 3.00 - 2.90 (m, 2H), 2.61 (tt, J = 11.2, 4.9 Hz, 1H), 1.44 - 1.29 (m, 4H). LCMS *m*/*z* 503.04 [M+H]⁺ |

| | | | |
|---|---|---|---|
| ^{1.} Prepared from **S3** by Suzuki coupling with (2-fluoro-4-methoxycarbonyl-phenyl)boronic acid. The resulting intermediate was coupled with ethanolamine using HATU, DIPEA in dichloromethane at room temperature. ^{2.} No ester hydrolysis step required. ^{3.} Prepared by Suzuki coupling of **S3** with (4-cyano-2-fluoro-phenyl)boronic acid. The nitrile group was subjected to oxidation with H₂O₂ and K₂CO₃ to afford 4-[4-benzyloxy-1-(3,4-difluorophenyl)-6-fluoro-2-tetrahydropyran-4-yl-indol-3-yl]-3-fluoro-benzamide. Hydrogenolysis afforded the product. | | | |

### Compound 251 and Compound 252

Compounds **251** and **252** (Table 14) were prepared from compound **4** by HATU coupling with the ammonia and hydroxylamine respectively.

**Table 14. Structure and physicochemical data for compounds 251-252**

| **Compound** | **Structure** | **¹H NMR; *LCMS m*/*z* [M+H]⁺** |
|---|---|---|
| **251** | 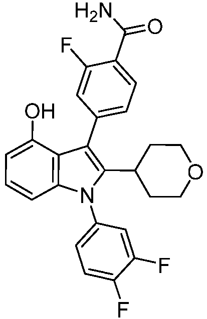 | ¹H NMR (300 MHz, Chloroform-d) δ 7.88 (t, J = 8.0 Hz, 1H), 7.64 - 7.45 (m, 2H), 7.40 (dd, J = 8.0, 1.5 Hz, 1H), 7.36 - 7.28 (m, 2H), 6.94 (dd, J = 8.2, 7.7 Hz, 1H), 6.41 (ddd, J = 21.7, 8.0, 0.8 Hz, 2H), 3.83 (d, J = 11.4 Hz, 2H), 3.22 (t, J = 11.3 Hz, 2H), 3.03 - 2.88 (m, 1H), 1.84 - 1.62 (m, 4H). LCMS *m*/*z* 467.31 [M+H]⁺ |
| **252** | 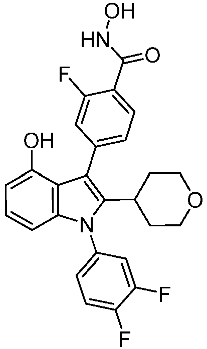 | ¹H NMR (300 MHz, Methanol-*d*₄) δ 7.72 (t, J = 7.7 Hz, 1H), 7.62 - 7.44 (m, 2H), 7.38 (dd, J = 7.9, 1.5 Hz, 1H), 7.34 - 7.24 (m, 2H), 6.97 - 6.86 (m, 1H), 6.39 (ddd, J = 21.0, 8.0, 0.8 Hz, 2H), 3.81 (d, J = 11.3 Hz, 2H), 3.20 (t, J = 11.4 Hz, 2H), 2.99 - 2.87 (m, 1H), 1.71 (dt, J = 23.2, 8.5 Hz, 4H). LCMS *m*/*z* 483.24 [M+H]⁺ |

### Compounds 253-278

Compounds **253-278** (Table 15) were prepared from the appropriate disubstituted alkyne and aniline aryl halide as described in the preparation of compound **146.** Any exceptions to this preparation are noted in the table footnote.

**Table 15. Structure and physicochemical data for compounds 253-278**

| **Compound** | **Structure** | ¹H **NMR; LCMS m/z [M+H]⁺** |
|---|---|---|
| **253** | 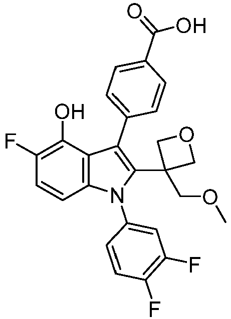 | ¹H NMR (400 MHz, DMSO-*d*₆) δ 12.92 (s, 1H), 9.41 (s, 1H), 7.94 - 7.88 (m, 2H), 7.84 (ddd, J = 11.2, 7.2, 2.5 Hz, 1H), 7.72 - 7.63 (m, 1H), 7.64 - 7.58 (m, 2H), 7.45 (dq, J = 7.4, 3.1, 2.5 Hz, 1H), 6.96 (dd, J = 11.2, 8.9 Hz, 1H), 6.21 (dd, J = 8.9, 3.4 Hz, 1H), 4.45 (d, J = 5.8 Hz, 1H), 4.35 (d, J = 5.9 Hz, 1H), 3.91 - 3.77 (m, 2H), 3.69 (dd, J = 11.5, 5.8 Hz, 2H), 3.39 (s, 3H). LCMS *m*/*z* 484.23 [M+H]⁺ |
| **254** | 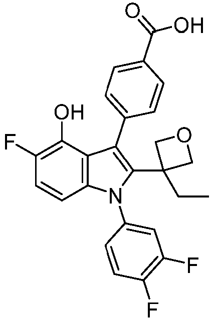 | ¹H NMR (400 MHz, DMSO-*d*₆) δ 12.95 (s, 1H), 9.35 (d, J = 1.9 Hz, 1H), 7.99 - 7.90 (m, 2H), 7.84 (ddd, J = 10.8, 7.2, 2.5 Hz, 1H), 7.67 (dt, J = 10.5, 8.9 Hz, 1H), 7.53 - 7.46 (m, 2H), 7.45 - 7.37 (m, 1H), 6.94 (dd, J = 11.2, 8.9 Hz, 1H), 6.18 (dd, J = 8.9, 3.4 Hz, 1H), 4.54 (d, J = 5.7 Hz, 1H), 4.47 (d, J = 5.8 Hz, 1H), 3.67 - 3.62 (m, 2H), 2.01 (q, J = 7.3 Hz, 2H), 1.07 (t, J = 7.4 Hz, 3H). LCMS *m*/*z* 468.26 [M+H]⁺ |
| **255** | 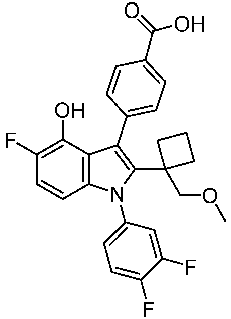 | ¹H NMR (400 MHz, DMSO-*d*₆) δ 12.83 (s, 1H), 9.20 (s, 1H), 7.87 (d, J = 8.0 Hz, 2H), 7.83 - 7.75 (m, 1H), 7.67 - 7.58 (m, 3H), 7.41 (d, J = 9.7 Hz, 1H), 6.89 (t, J = 10.1 Hz, 1H), 6.14 (d, J = 8.6 Hz, 1H), 3.64 (s, 2H), 3.35 (s, 3H), 2.05 - 1.97 (m, 1H), 1.97 - 1.87 (m, 1H), 1.80 - 1.68 (m, 1H), 1.45 - 1.26 (m, 3H). LCMS *m*/*z* 482.02 [M+H]⁺ |
| **256** | 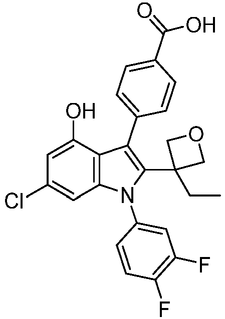 | ¹H NMR (400 MHz, DMSO-*d*₆) δ 12.95 (s, 1H), 9.83 (s, 1H), 7.93 (d, J = 8.5 Hz, 2H), 7.85 (ddd, J = 10.3, 7.2, 2.5 Hz, 1H), 7.72 - 7.61 (m, 1H), 7.49 - 7.39 (m, 3H), 6.45 (d, J = 1.7 Hz, 1H), 6.24 (d, J = 1.7 Hz, 1H), 4.51 (d, J = 5.8 Hz, 1H), 4.44 (d, J = 5.8 Hz, 1H), 3.64 (d, J = 5.9 Hz, 2H), 1.99 (q, J = 7.3 Hz, 2H), 1.06 (t, J = 7.4 Hz, 3H). LCMS *m*/*z* 484.27 [M+H]⁺ |
| **257** | 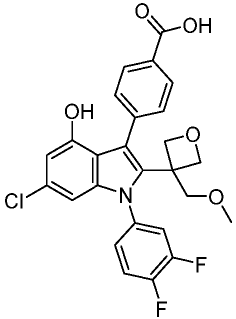 | ¹H NMR (400 MHz, DMSO-*d*₆) δ 12.94 (s, 1H), 9.93 (s, 1H), 7.92 - 7.88 (m, 2H), 7.85 (ddd, J = 11.2, 7.3, 2.6 Hz, 1H), 7.67 (dt, J = 10.6, 8.9 Hz, 1H), 7.61 - 7.57 (m, 2H), 7.45 (dt, J = 7.7, 2.8 Hz, 1H), 6.48 (d, J = 1.8 Hz, 1H), 6.26 (d, J = 1.7 Hz, 1H), 4.43 (d, J = 5.8 Hz, 1H), 4.33 (d, J = 5.8 Hz, 1H), 3.88 - 3.78 (m, 2H), 3.68 (dd, J = 10.7, 5.8 Hz, 2H), 3.39 (s, 3H). LCMS *m*/*z* 500.25 [M+H]⁺ |
| **258** | 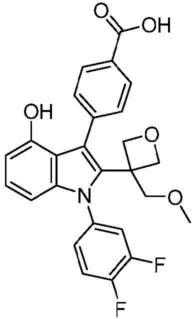 | ¹H NMR (400 MHz, DMSO-*d*₆) δ 12.86 (s, 1H), 9.33 (s, 1H), 7.91 - 7.88 (m, 2H), 7.82 (ddd, J = 11.0, 7.3, 2.6 Hz, 1H), 7.66 (dt, J = 10.6, 8.9 Hz, 1H), 7.62 - 7.59 (m, 2H), 7.47 - 7.40 (m, 1H), 6.90 (t, J = 7.9 Hz, 1H), 6.45 (d, J = 7.6 Hz, 1H), 6.23 (d, J = 8.1 Hz, 1H), 4.44 (d, J = 5.8 Hz, 1H), 4.33 (d, J = 5.8 Hz, 1H), 3.91 - 3.81 (m, 2H), 3.69 (dd, J = 12.6, 5.8 Hz, 2H), 3.39 (s, 3H). LCMS *m*/*z* 466.23 [M+H]⁺ |
| **259** | 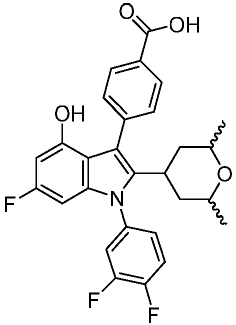 | ¹H NMR (400 MHz, Chloroform-*d*/CD3OD) δ 8.04 (d, J = 7.8 Hz, 2H), 7.49 (d, J = 7.9 Hz, 2H), 7.34 (q, J = 9.0 Hz, 1H), 7.27 - 7.17 (m, 1H), 7.11 (d, J = 8.4 Hz, 1H), 6.22 (d, J = 10.5 Hz, 1H), 6.02 (d, J = 9.1 Hz, 1H), 3.20 (t, J = 8.2 Hz, 2H), 2.93 - 2.75 (m, 1H), 1.61 - 1.43 (m, 2H), 1.16 (q, J = 12.3, 11.9 Hz, 2H), 0.98 (d, J = 7.3 Hz, 6H). LCMS *m*/*z* 496.24 [M+H]⁺ |
| **260** | 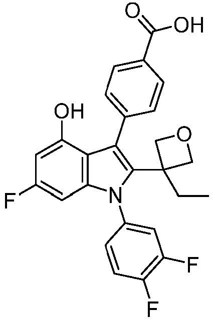 | ¹H NMR (400 MHz, DMSO-*d*₆) δ 12.93 (s, 1H), 9.83 (s, 1H), 7.97 - 7.86 (m, 2H), 7.83 (ddd, J = 11.1, 7.2, 2.5 Hz, 1H), 7.66 (dt, J = 10.4, 8.9 Hz, 1H), 7.50 - 7.44 (m, 2H), 7.44 - 7.37 (m, 1H), 6.27 (dd, J = 11.5, 2.2 Hz, 1H), 6.00 (dd, J = 9.5, 2.2 Hz, 1H), 4.47 (dd, J = 27.7, 5.7 Hz, 2H), 3.63 (d, J = 5.0 Hz, 2H), 1.99 (q, J = 7.3 Hz, 2H), 1.06 (t, J = 7.4 Hz, 3H). LCMS *m*/*z* 468.26 [M+H]⁺ |
| **261¹** | 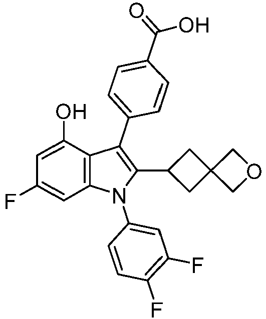 | ¹H NMR (400 MHz, Methanol-*d*₄) δ 7.99 (d, J = 7.5 Hz, 2H), 7.55 - 7.43 (m, 4H), 7.32 - 7.22 (m, 1H), 6.21 (d, J = 10.2 Hz, 2H), 4.56 (s, 2H), 4.21 (s, 2H), 3.64 (p, J = 9.0 Hz, 1H), 2.03 - 1.96 (m, 2H), 1.85 (t, J = 10.5 Hz, 2H). LCMS *m*/*z* 480.2 [M+H]⁺ |
| **262** | 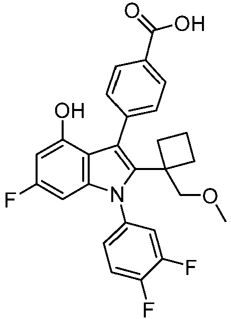 | ¹H NMR (400 MHz, Methanol-*d*₄) δ 7.94 (d, J = 7.8 Hz, 2H), 7.60 (d, J = 7.7 Hz, 2H), 7.54 - 7.39 (m, 2H), 7.32 (d, J = 6.9 Hz, 1H), 6.16 (d, J = 11.3 Hz, 1H), 5.91 (d, J = 9.7 Hz, 1H), 3.69 (s, 2H), 3.41 (s, 3H), 2.14 - 2.00 (m, 2H), 1.86 - 1.72 (m, 1H), 1.56 - 1.35 (m, 3H). LCMS *m*/*z* 482.29 [M+H]⁺ |
| **263** | 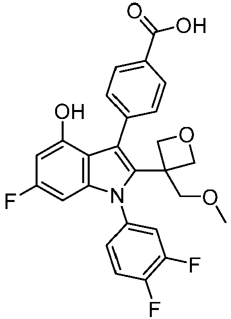 | ¹H NMR (400 MHz, DMSO-*d*₆) δ 12.92 (s, 1H), 9.91 (s, 1H), 7.92 - 7.86 (m, 2H), 7.82 (ddd, J = 11.2, 7.2, 2.6 Hz, 1H), 7.65 (dt, J = 10.5, 8.9 Hz, 1H), 7.59 (d, J = 8.4 Hz, 2H), 7.47 - 7.35 (m, 1H), 6.29 (dd, J = 11.4, 2.2 Hz, 1H), 6.01 (dd, J = 9.5, 2.2 Hz, 1H), 4.41 (d, J = 5.8 Hz, 1H), 4.31 (d, J = 5.9 Hz, 1H), 3.87 - 3.77 (m, 2H), 3.67 (dd, J = 11.6, 5.8 Hz, 2H), 3.38 (s, 3H). LCMS *m*/*z* 484.18 [M+H]⁺ |
| **264** | 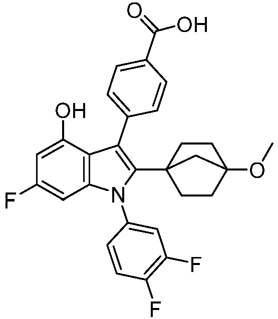 | ¹H NMR (400 MHz, DMSO-*d*₆) δ 12.97 (s, 1H), 9.60 (s, 1H), 7.93 - 7.88 (m, 2H), 7.77 (ddd, J = 11.1, 7.3, 2.6 Hz, 1H), 7.66 (dt, J = 10.5, 8.8 Hz, 1H), 7.46 (td, J = 8.3, 1.7 Hz, 2H), 7.38 (dd, J = 8.6, 4.2 Hz, 1H), 6.18 (dd, J = 11.4, 2.2 Hz, 1H), 5.92 (dd, J = 9.7, 2.2 Hz, 1H), 2.93 (s, 3H), 1.83 (s, 2H), 1.48 - 1.21 (m, 8H). LCMS *m*/*z* 508 [M+H]⁺ |
| **265** | 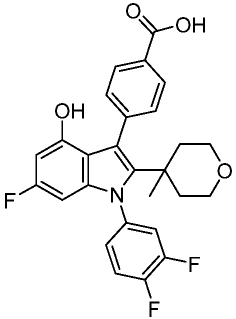 | ¹H NMR (400 MHz, DMSO-*d*₆) δ 9.66 (s, 1H), 7.92 - 7.87 (m, 2H), 7.80 (ddd, J = 11.1, 7.2, 2.5 Hz, 1H), 7.66 (dt, J = 10.4, 8.9 Hz, 1H), 7.45 (td, J = 8.2, 1.6 Hz, 2H), 6.18 (dd, J = 11.4, 2.2 Hz, 1H), 5.86 (dd, J = 9.8, 2.2 Hz, 1H), 3.40 - 3.21 (m, 5H), 1.55 (d, J = 14.4 Hz, 2H), 1.41 (s, 3H), 1.09 - 1.03 (m, 2H). LCMS *m*/*z* 482 [M+H]⁺ |
| **266** | 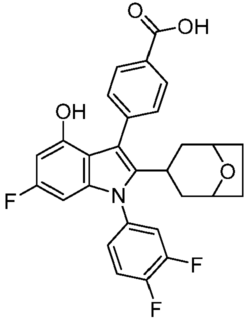 | ¹H NMR (400 MHz, Methanol-*d*₄) δ 8.03 (d, J = 8.2 Hz, 2H), 7.60 - 7.24 (m, 5H), 6.19 (dd, J = 11.3, 2.1 Hz, 1H), 6.02 (dd, J = 9.5, 2.1 Hz, 1H), 4.18 (s, 2H), 3.22 (tt, J = 12.7, 4.7 Hz, 1H), 1.90 - 1.71 (m, 4H), 1.50 - 1.32 (m, 4H). LCMS *m*/*z* 494.17 [M+H]⁺ |
| **267** | 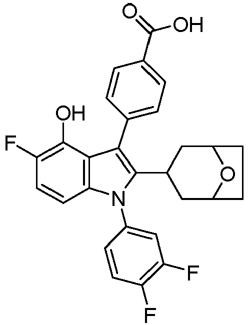 | ¹H NMR (400 MHz, DMSO-*d*₆) δ 12.94 (s, 1H), 9.23 (d, J = 1.8 Hz, 1H), 7.98 - 7.93 (m, 2H), 7.83 (ddd, J = 10.6, 7.4, 2.5 Hz, 1H), 7.77 - 7.67 (m, 1H), 7.51 (d, J = 8.1 Hz, 2H), 7.41 (d, J = 9.2 Hz, 1H), 6.92 (dd, J = 11.2, 8.9 Hz, 1H), 6.27 (dd, J = 8.8, 3.4 Hz, 1H), 4.10 (s, 2H), 3.22 - 3.01 (m, 1H), 1.73 - 1.60 (m, 4H), 1.39 (d, J = 12.9 Hz, 2H), 1.28 - 1.11 (m, 2H). LCMS *m*/*z* 494.25 [M+H]⁺ |
| **268** | 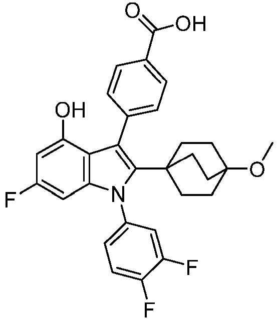 | ¹H NMR (400 MHz, Methanol-*d*₄) δ 7.98 (d, J = 7.8 Hz, 2H), 7.55 - 7.40 (m, 4H), 7.26 (d, J = 7.7 Hz, 1H), 6.08 (d, J = 11.2 Hz, 1H), 5.73 (d, J = 9.7 Hz, 1H), 3.02 (s, 3H), 1.98 - 1.91 (m, 1H), 1.88 - 1.81 (m, 5H), 1.51 - 1.47 (m, 1H), 1.46 - 1.40 (m, 5H). LCMS *m*/*z* 522.28 [M+H]⁺ |
| **269** | 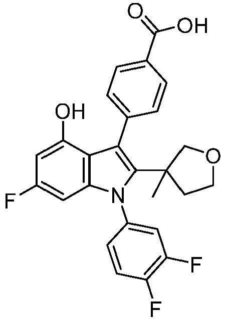 | ¹H NMR (400 MHz, DMSO-*d*₆) δ 12.93 (s, 1H), 9.65 (s, 1H), 7.96 - 7.80 (m, 3H), 7.68 (dt, J = 10.4, 8.8 Hz, 1H), 7.56 - 7.39 (m, 3H), 6.20 (dd, J = 11.4, 2.2 Hz, 1H), 5.95 (ddd, J = 9.7, 5.3, 2.2 Hz, 1H), 3.51 (dd, J = 9.3, 4.9 Hz, 2H), 3.32 - 3.19 (m, 1H), 2.81 (dd, J = 11.0, 7.9 Hz, 1H), 1.82 (dq, J = 21.8, 10.3 Hz, 1H), 1.45 - 1.36 (m, 3H), 1.18 - 1.07 (m, 1H). LCMS *m*/*z* 468.23 [M+H]⁺ |
| **270** | 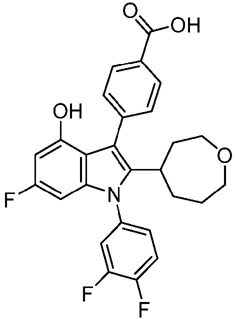 | LCMS *m*/*z* 482 [M+H]⁺ |
| **271** | 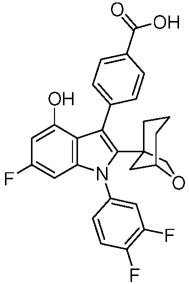 | ¹H NMR (400 MHz, DMSO-*d*₆) δ 12.94 (s, 1H), 9.61 (d, J = 1.3 Hz, 1H), 7.95 - 7.89 (m, 2H), 7.84 (dtd, J = 13.8, 7.4, 7.0, 3.6 Hz, 1H), 7.74 - 7.62 (m, 1H), 7.56 - 7.48 (m, 2H), 7.47 - 7.38 (m, 1H), 6.18 (dd, J = 11.4, 2.2 Hz, 1H), 5.95 - 5.88 (m, 1H), 4.05 - 3.98 (m, 1H), 3.57 - 3.46 (m, 1H), 3.10 (dd, J = 11.0, 7.8 Hz, 1H), 2.09 (d, J = 11.3 Hz, 1H), 1.89 - 1.77 (m, 1H), 1.66 (dd, J = 19.3, 8.9 Hz, 1H), 1.50 - 1.30 (m, 3H), 1.03 (dd, J = 15.0, 11.0 Hz, 2H). LCMS *m*/*z* 494.2 [M+H]⁺ |
| **272** | 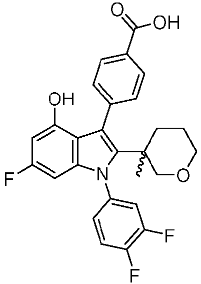 | ¹H NMR (400 MHz, DMSO-*d*₆) δ 12.91 (s, 1H), 9.52 (d, J = 2.2 Hz, 1H), 7.95 - 7.87 (m, 2H), 7.87 - 7.62 (m, 2H), 7.51 (ddt, J = 8.6, 6.8, 1.5 Hz, 2H), 7.45 - 7.34 (m, 1H), 6.15 (dd, J = 11.4, 2.2 Hz, 1H), 5.86 (dd, J = 9.8, 2.2 Hz, 1H), 3.37 (d, J = 4.5 Hz, 2H), 3.32 - 3.24 (m, 1H), 2.86 (dd, J = 11.7, 6.2 Hz, 1H), 1.65 (d, J = 13.4 Hz, 1H), 1.46 - 1.30 (m, 1H), 1.25 (d, J = 3.3 Hz, 5H). LCMS *m*/*z* 482.23 [M+H]⁺ |
| **273²** | 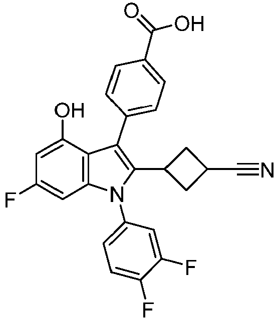 | ¹H NMR (400 MHz, Methanol-*d*₄) δ 8.01 (d, J = 7.5 Hz, 2H), 7.60 - 7.47 (m, 4H), 7.31 (d, J = 8.1 Hz, 1H), 6.23 (d, J = 10.2 Hz, 2H), 4.30 (p, J = 9.4 Hz, 1H), 2.75 (t, J = 9.2 Hz, 1H), 2.14 (q, J = 10.2 Hz, 2H), 1.98 - 1.88 (m, 2H). LCMS *m*/*z* 463.37 [M+H]⁺ |
| **274** | 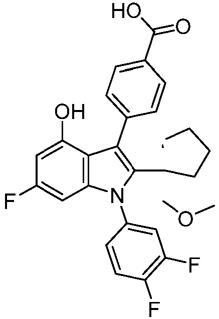 | ¹H NMR (400 MHz, DMSO-*d*₆) δ 12.86 (s, 1H), 9.50 (s, 1H), 7.95 - 7.84 (m, 2H), 7.73 (ddd, J = 11.4, 7.4, 2.6 Hz, 1H), 7.65 (dt, J = 10.6, 8.9 Hz, 1H), 7.54 (ddt, J = 10.2, 8.1, 1.5 Hz, 2H), 7.42 - 7.35 (m, 1H), 6.15 (dd, J = 11.5, 2.2 Hz, 1H), 5.83 (dd, J = 9.8, 2.2 Hz, 1H), 3.28 (s, 3H), 3.26 - 3.16 (m, 2H), 1.55 - 1.15 (m, 8H). LCMS *m*/*z* 496.22 [M+H]⁺ |
| **275** | 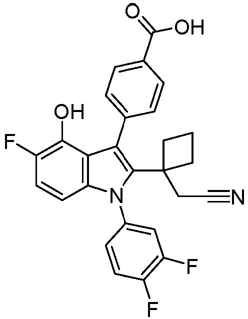 | ¹H NMR (400 MHz, Methanol-*d*₄) δ 7.99 (d, J = 8.5 Hz, 2H), 7.66 (d, J = 8.1 Hz, 2H), 7.59 - 7.46 (m, 2H), 6.87 (dd, J = 11.2, 8.9 Hz, 1H), 6.17 (dd, J = 8.9, 3.4 Hz, 1H), 3.04 (d, J = 3.2 Hz, 2H), 2.32 (dq, J = 20.8, 10.3 Hz, 2H), 2.00 - 1.86 (m, 1H), 1.60 (dt, J = 12.1, 9.4 Hz, 1H), 1.48 (d, J = 8.3 Hz, 2H). LCMS *m*/*z* 477.29 [M+H]⁺ |
| **276** | 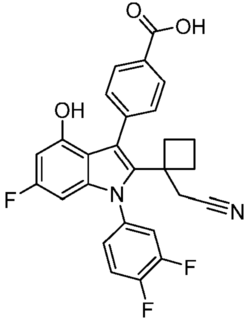 | ¹H NMR (400 MHz, Chloroform-*d*) δ 8.26 (d, J = 8.2 Hz, 2H), 7.85 (t, J = 7.1 Hz, 2H), 7.47 - 7.30 (m, 2H), 7.28 - 7.23 (m, 1H), 6.38 (dd, J = 10.7, 2.1 Hz, 1H), 6.07 (dd, J = 9.2, 2.1 Hz, 1H), 2.86 (d, J = 2.1 Hz, 2H), 2.42 - 2.22 (m, 2H), 1.91 (dt, J = 19.2, 9.6 Hz, 1H), 1.71 (q, J = 9.9 Hz, 1H), 1.63 - 1.44 (m, 2H). LCMS *m*/*z* 477.29 [M+H]⁺ |
| **277** | 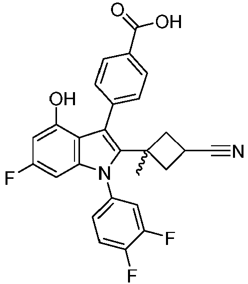 | ¹H NMR (400 MHz, Methanol-*d*₄) δ 8.01 (d, J = 7.5 Hz, 2H), 7.52 (d, J = 7.5 Hz, 4H), 7.38 - 7.29 (m, 1H), 6.19 (d, J = 11.3 Hz, 1H), 5.95 (d, J = 9.5 Hz, 1H), 2.89 (t, J = 11.4 Hz, 1H), 2.62 - 2.50 (m, 2H), 1.97 (s, 3H), 1.60 - 1.50 (m, 2H). LCMS *m*/*z* 477.1 [M+H]⁺ |
| ***278*** | 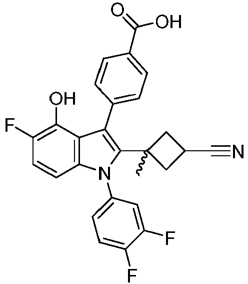 | ¹H NMR (400 MHz, Methanol-*d*₄) δ 8.02 (d, J = 7.5 Hz, 2H), 7.59 - 7.46 (m, 4H), 7.34 (d, J = 8.2 Hz, 1H), 6.86 (t, J = 9.9 Hz, 1H), 6.20 (d, J = 8.6 Hz, 1H), 2.89 (t, J = 10.4 Hz, 1H), 2.58 (q, J = 11.8 Hz, 2H), 2.03 (s, 3H), 1.61 - 1.53 (m, 2H). LCMS *m*/*z* 477.24 [M+H]⁺ |

| | | |
|---|---|---|
| ^{1.} Compound **261** was prepared by Suzuki coupling from 4-benzyloxy-1-(3,4-difluorophenyl)-6-fluoro-3-iodo-2-(2-oxaspiro[3.3]heptan-6-yl)indole. ^{2.} Compound 273 was prepared by Suzuki coupling from 3-[4-benzyloxy-1-(3,4-difluorophenyl)-6-fluoro-3-iodo-indol-2-yl]cyclobutanecarbonitrile | | |

### Compound 279 and Compound 280

### 4-[1-(3,4-difluorophenyl)-6-fluoro-4-hydroxy-2-(2-methoxy-1-methyl-ethyl)indol-3-yl]benzoic acid [ENANT-1] (279) and 4-[1-(3, 4-difluorophenyl)-6-fluoro-4-hydroxy-2-(2-methoxy-1-methyl-ethyl)indol-3-yl]benzoic acid [ENANT]-2 (280)

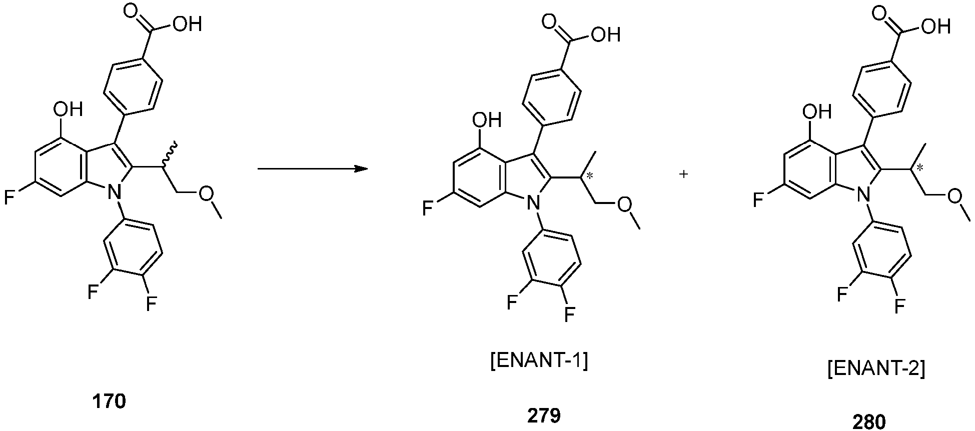

Compounds 279 and **280** were prepared by separation of compound 170 into its constituent isomers by SFC. Column: Daicel Chiralpak ^{®} OJ-H. Mobile Phase: 10% MeOH (containing 5 mM Ammonia), 90% carbon dioxide. Compound 279 was the first eluting peak. Compound 280 was the second eluting peak.
**Compound 279.** ¹H NMR (400 MHz, DMSO-*d*₆) δ 12.91 (s, 1H), 9.76 (s, 1H), 7.98 - 7.90 (m, 2H), 7.80 - 7.64 (m, 2H), 7.49 (dd, J = 8.3, 1.7 Hz, 2H), 7.42 - 7.31 (m, 1H), 6.26 (dd, J = 11.4, 2.2 Hz, 1H), 6.08 (ddd, J = 9.6, 2.2, 0.9 Hz, 1H), 3.18 - 2.98 (m, 6H), 0.97 (dd, J = 6.6, 3.6 Hz, 3H). LCMS *m*/*z* 456 [M+H]⁺
**Compound 280.** ¹H NMR (400 MHz, DMSO-*d*₆) δ 12.91 (s, 1H), 9.76 (s, 1H), 7.98 - 7.90 (m, 2H), 7.80 - 7.64 (m, 2H), 7.49 (dd, J = 8.3, 1.7 Hz, 2H), 7.42 - 7.31 (m, 1H), 6.26 (dd, J = 11.4, 2.2 Hz, 1H), 6.08 (ddd, J = 9.6, 2.2, 0.9 Hz, 1H), 3.18 - 2.98 (m, 6H), 0.97 (dd, J = 6.6, 3.6 Hz, 3H). LCMS *m*/*z* 456 [M+H]⁺

### Compound 281

### 4-[1-(3,4-difluorophenyl)-6-fluoro-4-hydroxy-2-(3,3,4,5,5-pentadeuteriotetrahydropyran-4-yl)indol-3-yl]benzoic acid (281)

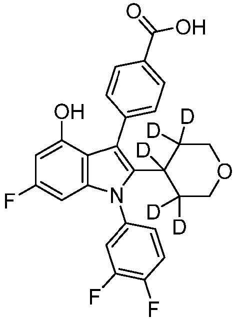

4-[1-(3,4-difluorophenyl)-6-fluoro-4-hydroxy-2-(3,3,4,5,5-pentadeuteriotetrahydropyran-4-yl)indol-3-yl]benzoic acid (9 mg, 95%). ¹H NMR (400 MHz, DMSO-*d*₆) δ 9.75 (s, 1H), 7.97 - 7.91 (m, 2H), 7.80 (ddd, J = 11.1, 7.2, 2.6 Hz, 1H), 7.70 (dt, J = 10.6, 8.9 Hz, 1H), 7.52 - 7.47 (m, 2H), 7.43 - 7.36 (m, 1H), 6.24 (dd, J = 11.4, 2.2 Hz, 1H), 6.07 (dd, J = 9.6, 2.2 Hz, 1H), 3.66 (dd, J = 11.5, 2.5 Hz, 2H), 3.01 (dd, J = 11.5, 5.5 Hz, 2H). LCMS *m*/*z* 473.0 [M+H]⁺

### Compound 282

### 4-[1-(3,4-difluorophenyl)-4-hydroxy-2-isopropyl-indol-3-yl]benzoic acid (282)

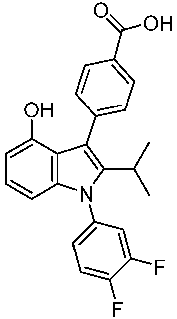

Compound 282 was prepared from 4-benzyloxy-1-(3,4-difluorophenyl)-3-iodo-2-isopropyl-indole and (4-benzyloxycarbonylphenyl)boronic acid using the method described for the preparation of compound 1. ¹H NMR (400 MHz, DMSO-*d*₆) δ 12.84 (s, 1H), 9.16 (s, 1H), 7.99 - 7.88 (m, 2H), 7.82 - 7.74 (m, 1H), 7.69 (dt, J = 10.5, 8.8 Hz, 1H), 7.57 - 7.45 (m, 2H), 7.44 - 7.31 (m, 1H), 6.85 (t, J = 8.0 Hz, 1H), 6.43 - 6.35 (m, 1H), 6.27 (d, J = 8.2 Hz, 1H), 2.98 (p, J = 7.1 Hz, 1H), 1.10 - 0.89 (m, 6H). LCMS *m*/*z* 408.32 [M+H]⁺

### Compound 283-290

Compound **283-290** (Table 16) were prepared by Larock indole cyclization between the appropriate alkynes and aryl anilines according to the procedure described for the preparation of compound **146.** Modifications are notes in the table footnotes.

**Table 16. Method of preparation, structure, physicochemical data for compounds 283-290**

| **Compound** | **Structure** | **¹H NMR; LCMS m/z [M+H]⁺** |
|---|---|---|
| **283¹** | 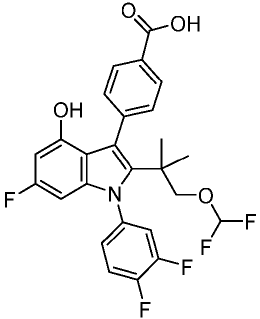 | ¹H NMR (400 MHz, DMSO-*d*₆) δ 12.89 (s, 1H), 9.53 (s, 1H), 7.90 (d, J = 7.7 Hz, 2H), 7.77 - 7.63 (m, 2H), 7.48 (t, J = 7.4 Hz, 2H), 7.38 (d, J = 9.2 Hz, 1H), 6.55 (t, J = 75.5 Hz, 1H), 6.14 (d, J = 11.6 Hz, 1H), 5.83 (d, J = 9.5 Hz, 1H), 3.62 - 3.50 (m, 2H), 0.99 (s, 6H). LCMS *m*/*z* 506.23 [M+H]⁺ |
| **284** | 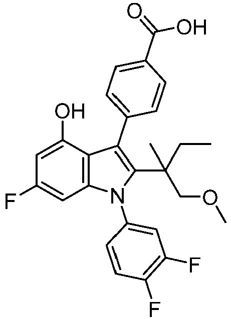 | ¹H NMR (400 MHz, DMSO-*d*₆) δ 12.87 (s, 1H), 9.44 (s, 1H), 7.93 - 7.85 (m, 2H), 7.73 - 7.55 (m, 2H), 7.45 (ddt, J = 7.7, 6.5, 1.6 Hz, 2H), 7.39 - 7.27 (m, 1H), 6.11 (dd, J = 11.5, 2.2 Hz, 1H), 5.77 (dd, J = 9.8, 2.2 Hz, 1H), 3.12 (dd, J = 16.7, 9.1 Hz, 1H), 3.06 (d, J = 3.4 Hz, 3H), 2.89 (dd, J = 9.1, 5.8 Hz, 1H), 1.41 (dt, J = 15.1, 7.8 Hz, 1H), 1.17 - 1.06 (m, 1H), 0.86 (d, J = 2.2 Hz, 3H), 0.70 (td, J = 7.4, 1.8 Hz, 3H). LCMS *m*/*z* 484.25 [M+H]⁺ |
| **285** | 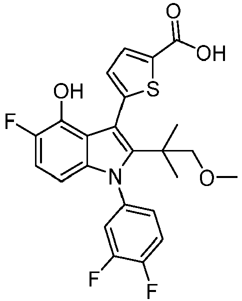 | ¹H NMR (400 MHz, DMSO-*d*₆) δ 12.92 (s, 1H), 9.21 (d, J = 1.8 Hz, 1H), 7.75 (s, 1H), 7.71 - 7.63 (m, 2H), 7.38 (s, 1H), 7.07 (d, J = 3.7 Hz, 1H), 6.87 (dd, J = 11.2, 8.9 Hz, 1H), 5.99 (dd, J = 8.9, 3.5 Hz, 1H), 3.06 (s, 3H), 3.03 (d, J = 5.3 Hz, 2H), 1.09 (s, 6H). LCMS *m*/*z* 476 [M+H]⁺ |
| **286²** | 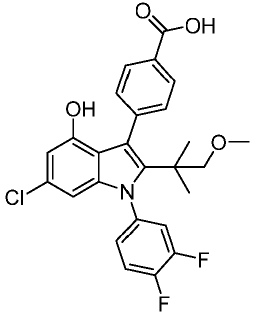 | ¹H NMR (400 MHz, Chloroform-*d*) δ 8.25 - 8.18 (m, 2H), 7.74 - 7.65 (m, 2H), 7.41 - 7.28 (m, 2H), 7.25 - 7.19 (m, 1H), 6.48 (d, J = 1.7 Hz, 1H), 6.21 (d, J = 1.7 Hz, 1H), 4.51 (s, 1H), 3.11 (s, 3H), 2.97 (s, 2H), 1.06 (d, J = 2.5 Hz, 6H). LCMS *m*/*z* 486.21 [M+H]⁺ |
| **287³** | 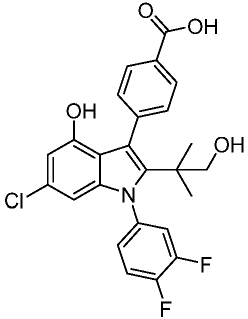 | ¹H NMR (400 MHz, Methanol-*d*₄/ CDCl3) δ 8.09 - 8.02 (m, 2H), 7.64 - 7.55 (m, 2H), 7.40 - 7.32 (m, 2H), 7.30 - 7.24 (m, 1H), 6.39 (d, J = 1.7 Hz, 1H), 6.14 (d, J = 1.7 Hz, 1H), 3.33 (s, 2H), 1.01 (s, 6H). LCMS *m*/*z* 472.29 [M+H]⁺ |
| **288³** | 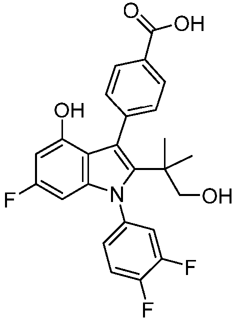 | ¹H NMR (400 MHz, Chloroform-*d*) δ 8.05 (d, J = 7.8 Hz, 2H), 7.59 (t, J = 6.6 Hz, 2H), 7.45 - 7.31 (m, 2H), 7.20 (t, J = 8.4 Hz, 1H), 6.17 (dt, J = 11.0, 2.7 Hz, 1H), 5.83 (ddd, J = 8.7, 6.3, 2.2 Hz, 1H), 3.28 (s, 2H), 0.97 (d, J = 3.5 Hz, 6H). LCMS *m*/*z* 456.24 [M+H]⁺ |
| **289²** | 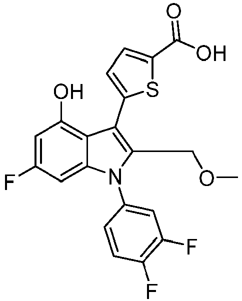 | ¹H NMR (400 MHz, DMSO-*d*₆) δ 12.99 (s, 1H), 9.70 (s, 1H), 7.81 - 7.64 (m, 2H), 7.62 (d, J = 3.8 Hz, 1H), 7.37 (s, 1H), 7.04 (d, J = 3.7 Hz, 1H), 6.19 (dd, J = 11.4, 2.2 Hz, 1H), 5.81 (dd, J = 9.7, 2.2 Hz, 1H), 3.06 (s, 3H), 3.04 - 2.97 (m, 2H), 1.07 (s, 6H). LCMS *m*/*z* 476 [M+H]⁺ |
| **290** | 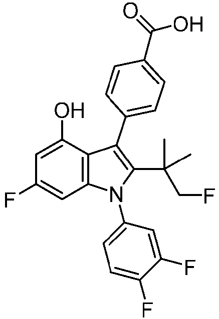 | ¹H NMR (400 MHz, DMSO-*d*₆) δ 12.92 (s, 1H), 9.55 (s, 1H), 7.91 (d, J = 7.8 Hz, 2H), 7.85 - 7.74 (m, 1H), 7.68 (q, J = 9.2, 8.7 Hz, 1H), 7.48 (t, J = 7.3 Hz, 2H), 7.42 - 7.34 (m, 1H), 6.15 (d, J = 11.1 Hz, 1H), 5.85 (d, J = 9.7 Hz, 1H), 4.09 (d, J = 47.5 Hz, 2H), 0.99 (s, 6H). LCMS *m*/*z* 458.26 [M+H]⁺ |

| | | |
|---|---|---|
| ^{1.} CF₂ group was added according to the alcohol using the method described for the preparation of **(C185)** ^{2.} Larock indole cyclization with **C229** ^{3.} Larock indole cyclization **C222** | | |

### Compound 291-301

Compounds **91-301** were prepared by Larock indole cyclization according to the method described for the preparation of compound **17.**

**Table 17. Method of preparation, structure, physicochemical data for compounds 291-301**

| **Compound** | **Structure** | **¹H NMR; LCMS m/z [M+H]⁺** |
|---|---|---|
| **291** | 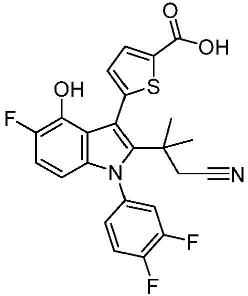 | ¹H NMR (400 MHz, DMSO-*d*₆) δ 13.18 (s, 1H), 9.35 (d, J = 1.8 Hz, 1H), 7.84 (s, 1H), 7.71 (dt, J = 10.5, 8.9 Hz, 1H), 7.65 (d, J = 3.7 Hz, 1H), 7.43 (s, 1H), 7.15 (d, J = 3.7 Hz, 1H), 6.94 (dd, J = 11.2, 8.9 Hz, 1H), 6.04 (dd, J = 8.9, 3.4 Hz, 1H), 2.65 (s, 2H), 1.24 (s, 6H). LCMS *m*/*z* 471 [M+H]⁺ |
| **292** | 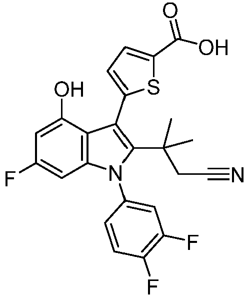 | ¹H NMR (400 MHz, DMSO-*d*₆) δ 13.19 (s, 1H), 9.87 (s, 1H), 7.83 (s, 1H), 7.75 - 7.67 (m, 1H), 7.66 (d, J = 3.7 Hz, 1H), 7.42 (s, 1H), 7.13 (d, J = 3.7 Hz, 1H), 6.23 (dd, J = 11.4, 2.2 Hz, 1H), 5.86 (dd, J = 9.6, 2.2 Hz, 1H), 2.62 (s, 2H), 1.22 (s, 6H). LCMS *m*/*z* 471 [M+H]⁺ |
| **293** | 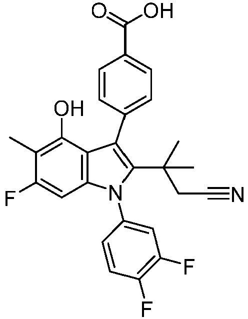 | ¹H NMR (400 MHz, Methanol-*d*₄) δ 8.08 - 8.02 (m, 2H), 7.66 - 7.58 (m, 2H), 7.57 - 7.46 (m, 2H), 7.39 - 7.30 (m, 1H), 5.90 (d, J = 10.1 Hz, 1H), 2.48 (d, J = 2.0 Hz, 2H), 2.05 - 1.99 (m, 4H), 1.23 (d, J = 2.9 Hz, 7H). LCMS *m*/*z* 479.31 [M+H]⁺ |
| **294** | 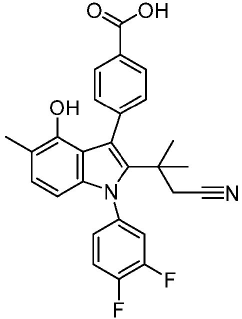 | ¹H NMR (400 MHz, Methanol-*d*₄) δ 8.12 - 7.99 (m, 2H), 7.71 - 7.59 (m, 2H), 7.59 - 7.45 (m, 2H), 7.40 - 7.28 (m, 1H), 6.82 (dd, J = 8.3, 0.7 Hz, 1H), 6.11 (d, J = 8.3 Hz, 1H), 2.49 (d, J = 2.3 Hz, 2H), 2.13 (s, 3H), 1.25 (d, J = 2.8 Hz, 7H). LCMS *m*/*z* 461.27 [M+H]⁺ |
| **295** | 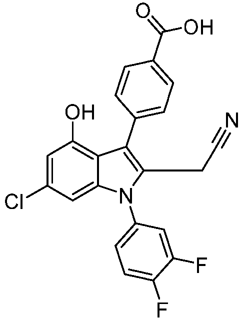 | ¹H NMR (400 MHz, Methanol-*d*₄/ CDCl3) δ 8.13 - 8.03 (m, 2H), 7.62 (d, J = 8.0 Hz, 2H), 7.47 - 7.36 (m, 2H), 7.27 (d, J = 8.7 Hz, 1H), 6.43 (d, J = 1.8 Hz, 1H), 6.16 (d, J = 1.8 Hz, 1H), 2.37 (s, 2H), 1.25 (d, J = 3.1 Hz, 6H). LCMS *m*/*z* 481.16 [M+H]⁺ |
| **296** | 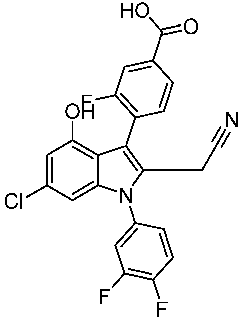 | ¹H NMR (400 MHz, CD3OD) δ 7.83 (d, J = 7.7 Hz, 1H), 7.71 (d, J = 9.5 Hz, 1H), 7.56 (d, J = 10.7 Hz, 4H), 7.39 (d, J = 31.4 Hz, 1H), 6.35 (d, J = 4.2 Hz, 1H), 6.08 (s, 1H), 2.64 - 2.44 (m, 2H), 1.27 (s, 9H). LCMS *m*/*z* 499 [M+H]⁺ |
| **297** | 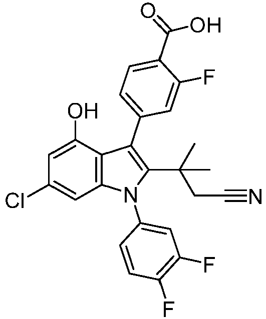 | ¹H NMR (400 MHz, DMSO-*d*₆) δ 13.25 (s, 1H), 9.71 (d, J = 1.4 Hz, 1H), 7.88 - 7.79 (m, 2H), 7.72 (dtd, J = 10.5, 8.9, 1.6 Hz, 1H), 7.47 - 7.39 (m, 1H), 7.38 - 7.25 (m, 2H), 6.36 (d, J = 1.8 Hz, 1H), 6.11 (dd, J = 2.8, 1.7 Hz, 1H), 2.56 (d, J = 1.8 Hz, 2H), 1.15 (dd, J = 7.9, 6.5 Hz, 6H). LCMS *m*/*z* 499.19 [M+H]⁺ |
| **298** | 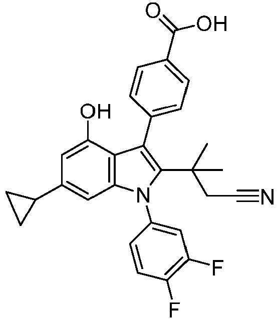 | ¹H NMR (400 MHz, Methanol-*d*₄) δ 8.05 - 7.97 (m, 2H), 7.57 (ddd, J = 8.1, 3.9, 1.7 Hz, 3H), 7.53 (d, J = 1.6 Hz, 1H), 7.38 - 7.29 (m, 1H), 6.05 (d, J = 1.3 Hz, 1H), 5.89 (d, J = 1.4 Hz, 1H), 2.46 (d, J = 2.0 Hz, 2H), 1.76 (ddd, J = 13.5, 8.5, 5.1 Hz, 1H), 1.23 (d, J = 2.9 Hz, 7H), 0.86 - 0.76 (m, 2H), 0.53 - 0.44 (m, 2H). LCMS *m*/*z* 487.21 [M+H]⁺ |
| **299** | 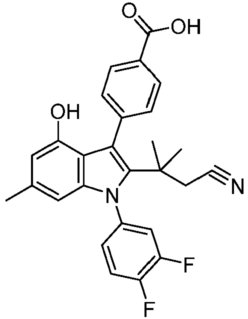 | ¹H NMR (400 MHz, Methanol-*d*₄) δ 8.04 - 7.95 (m, 2H), 7.62 - 7.44 (m, 4H), 7.34 (q, J = 4.1 Hz, 1H), 6.19 (d, J = 1.2 Hz, 1H), 5.92 (t, J = 1.0 Hz, 1H), 2.47 (d, J = 2.2 Hz, 2H), 2.20 (s, 3H), 1.23 (d, J = 3.0 Hz, 6H). LCMS *m*/*z* 461.32 [M+H]⁺ |
| **300** | 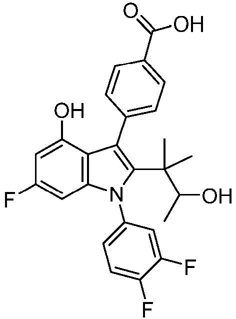 | ¹H NMR (400 MHz, Chloroform-*d*) δ 8.10 (tdd, J = 5.5, 2.7, 1.6 Hz, 2H), 7.65 - 7.55 (m, 2H), 7.34 - 7.05 (m, 3H), 6.18 (dd, J = 10.8, 2.2 Hz, 1H), 5.85 - 5.76 (m, 1H), 3.90 (dp, J = 6.4, 3.5, 3.1 Hz, 1H), 0.96 (td, J = 6.4, 2.7 Hz, 6H), 0.88 (q, J = 3.8, 3.3 Hz, 3H). LCMS *m*/*z* 470.24 [M+H]⁺ |
| **301** | 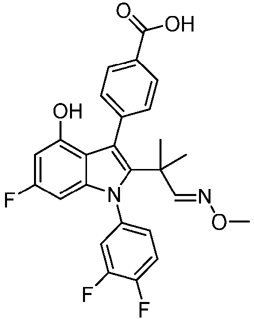 | ¹H NMR (400 MHz, Chloroform-*d*) δ 8.23 - 8.10 (m, 2H), 7.64 - 7.56 (m, 2H), 7.32 - 7.22 (m, 1H), 7.14 (ddd, J = 10.0, 6.9, 2.4 Hz, 1H), 7.10 - 7.02 (m, 2H), 6.23 (dd, J = 10.8, 2.2 Hz, 1H), 5.94 (dd, J = 9.3, 2.1 Hz, 1H), 3.46 (s, 3H), 1.17 (d, J = 1.9 Hz, 6H). LCMS *m*/*z* 483.19 [M+H]⁺ |

### Compound 302-303

Compounds **302** and **303** (Table 18) were prepared by Larock indole cyclization method using the appropriate aryl aniline and disubstituted alkyne.

**Table 18. Method of preparation, structure, physicochemical data for compounds 302-303**

| **Compound** | **Structure** | ¹H **NMR; LCMS m/z** [M+H]⁺ |
|---|---|---|
| **302** | 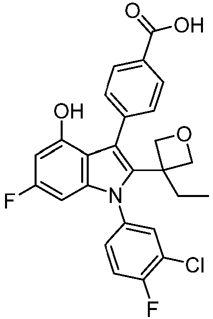 | ¹H NMR (400 MHz, DMSO-*d*₆) δ 12.97 (s, 1H), 9.87 (s, 1H), 7.98 - 7.89 (m, 3H), 7.64 (t, J = 8.8 Hz, 1H), 7.61 - 7.55 (m, 1H), 7.47 (d, J = 7.9 Hz, 2H), 6.28 (d, J = 11.3 Hz, 1H), 6.00 (d, J = 9.5 Hz, 1H), 4.56 - 4.48 (m, 1H), 4.43 (d, J = 5.6 Hz, 1H), 3.63 (d, J = 5.2 Hz, 2H), 1.98 (q, J = 7.5 Hz, 2H), 1.06 (t, J = 7.4 Hz, 3H). LCMS *m*/*z* 484.23 [M+H]⁺ |
| **303** | 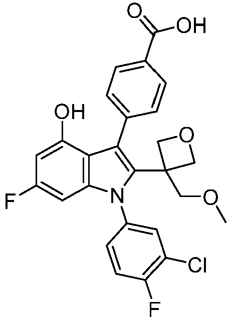 | ¹H NMR (400 MHz, DMSO-*d*₆) δ 12.90 (s, 1H), 9.91 (s, 1H), 7.99 - 7.83 (m, 3H), 7.71 - 7.51 (m, 4H), 6.30 (dd, J = 11.4, 2.2 Hz, 1H), 6.02 (dd, J = 9.5, 2.2 Hz, 1H), 4.41 (d, J = 5.8 Hz, 1H), 4.32 (d, J = 5.9 Hz, 1H), 3.82 (d, J = 2.5 Hz, 2H), 3.66 (dd, J = 14.5, 5.7 Hz, 2H), 3.39 (s, 3H). LCMS *m*/*z* 500.11 [M+H]⁺ |

### Compound 304-376

Compounds **304-376** (Table 19) were prepared by Larock indole cyclization method using the appropriated disubstituted alkyne and aniline.

**Table 19. Method of preparation, structure, physicochemical data for compounds 304-376**

| **Compound** | **Structure** | **¹H NMR; LCMS m/z [M+H]⁺** |
|---|---|---|
| **304** | 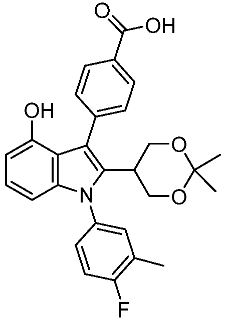 | ¹H NMR (400 MHz, Chloroform-*d*/CD3OD) δ 8.22 - 8.10 (m, 2H), 7.67 - 7.59 (m, 2H), 7.28 - 7.14 (m, 3H), 6.97 (t, J = 7.9 Hz, 1H), 6.46 (dd, J = 7.9, 2.4 Hz, 2H), 3.92 (td, J = 11.7, 7.6 Hz, 2H), 3.66 (s, 2H), 3.26 (s, 1H), 2.38 (d, J = 2.0 Hz, 3H), 1.24 (s, 6H). LCMS *m*/*z* 476.26 [M+H]⁺ |
| **305** | 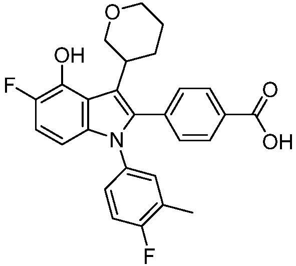 | LCMS *m*/*z* 464.32 [M+H]⁺ |
| **306** | 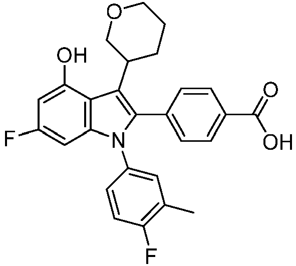 | LCMS *m*/*z* 464.32 [M+H]⁺ |
| **307** | 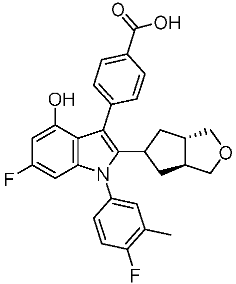 | ¹H NMR (400 MHz, DMSO-*d*₆) δ 12.90 (s, 1H), 9.77 (d, J = 1.0 Hz, 1H), 8.03 - 7.87 (m, 2H), 7.58 - 7.47 (m, 2H), 7.43 - 7.36 (m, 3H), 6.26 (dd, J = 11.4, 2.3 Hz, 1H), 5.99 (dt, J = 9.6, 2.3 Hz, 1H), 3.81 (d, J = 10.4 Hz, 1H), 3.61 - 3.52 (m, 1H), 3.42 (q, J = 7.0 Hz, 1H), 3.04 - 2.94 (m, 1H), 2.84 - 2.74 (m, 1H), 2.34 (d, J = 1.8 Hz, 3H), 1.95 - 1.71 (m, 3H), 1.63 - 1.51 (m, 1H), 1.50 - 1.39 (m, 1H), 1.27 (ddd, J = 19.1, 11.7, 7.1 Hz, 1H). LCMS *m*/*z* 490.24 [M+H]⁺ |
| **308** | 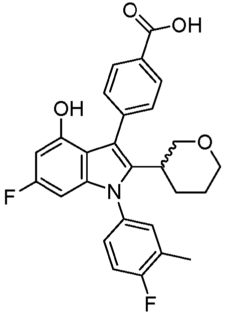 | ¹H NMR (400 MHz, DMSO-*d*₆) δ 12.93 (s, 1H), 9.70 (s, 1H), 7.95 (d, J = 7.8 Hz, 2H), 7.50 (d, J = 8.1 Hz, 2H), 7.46 - 7.25 (m, 2H), 6.22 (d, J = 11.4 Hz, 1H), 5.99 (d, J = 9.6 Hz, 1H), 4.03 (q, J = 7.1, 6.7 Hz, 0H), 3.82 - 3.51 (m, 2H), 3.13 (t, J = 11.3 Hz, 1H), 2.88 - 2.67 (m, 2H), 2.34 (s, 3H), 1.76 (s, 1H), 1.47 - 1.17 (m, 3H). LCMS *m*/*z* [M+H]⁺ |
| **309** | 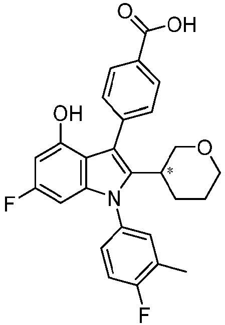 | ¹H NMR (400 MHz, DMSO-*d*₆) δ 12.92 (s, 1H), 9.69 (d, J = 1.6 Hz, 1H), 7.99 - 7.90 (m, 2H), 7.55 - 7.48 (m, 2H), 7.48 - 7.28 (m, 2H), 6.22 (dd, J = 11.4, 2.3 Hz, 1H), 5.98 (dd, J = 9.6, 2.2 Hz, 1H), 3.73 (t, J = 10.3 Hz, 1H), 3.62 (d, J = 11.4 Hz, 1H), 3.13 (td, J = 11.1, 2.8 Hz, 1H), 2.80 (dd, J = 25.1, 13.2 Hz, 3H), 2.34 (d, J = 1.9 Hz, 3H), 1.76 (s, 1H), 1.37 (d, J = 13.9 Hz, 2H). LCMS *m*/*z* 464.28 [M+H]⁺ |
| **310** | 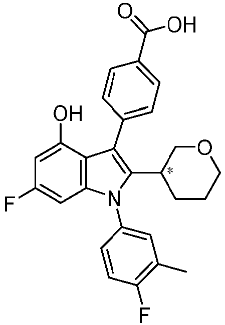 | ¹H NMR (400 MHz, DMSO-*d*₆) δ 12.92 (s, 1H), 9.70 (d, J = 1.6 Hz, 1H), 8.00 - 7.91 (m, 2H), 7.54 - 7.47 (m, 2H), 7.47 - 7.26 (m, 2H), 6.22 (dd, J = 11.4, 2.2 Hz, 1H), 5.98 (dd, J = 9.6, 2.2 Hz, 1H), 3.79 - 3.56 (m, 3H), 3.13 (td, J = 11.2, 2.9 Hz, 1H), 2.94 - 2.60 (m, 2H), 2.34 (d, J = 2.0 Hz, 3H), 1.76 (s, 1H), 1.47 - 1.29 (m, 1H), 0.93 - 0.82 (m, 1H). LCMS *m*/*z* [M+H]⁺ |
| **311** | 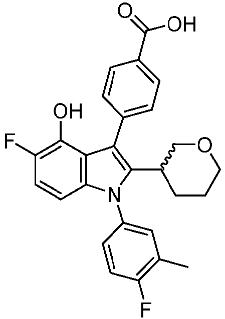 | LCMS *m*/*z* 464.32 [M+H]⁺ |
| **312** | 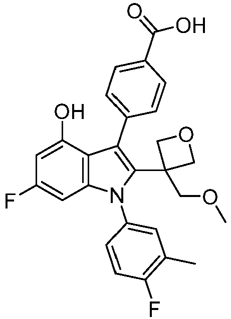 | ¹H NMR (400 MHz, DMSO-*d*₆) δ 12.89 (s, 1H), 9.88 (s, 1H), 7.93 - 7.85 (m, 2H), 7.65 - 7.58 (m, 2H), 7.48 (dd, J = 6.9, 2.5 Hz, 1H), 7.44 - 7.31 (m, 2H), 6.28 (dd, J = 11.4, 2.2 Hz, 1H), 5.92 (dd, J = 9.5, 2.2 Hz, 1H), 4.35 (dd, J = 11.0, 5.9 Hz, 2H), 3.80 (d, J = 1.1 Hz, 2H), 3.65 (dd, J = 11.1, 5.8 Hz, 2H), 3.38 (s, 3H), 2.32 (d, J = 1.8 Hz, 3H). LCMS *m*/*z* 480.22 [M+H]⁺ |
| **313** | 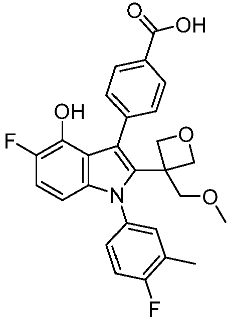 | ¹H NMR (400 MHz, DMSO-*d*₆) δ 12.91 (s, 1H), 9.37 (d, J = 1.8 Hz, 1H), 7.95 - 7.86 (m, 2H), 7.66 - 7.57 (m, 2H), 7.49 (dd, J = 6.9, 2.6 Hz, 1H), 7.43 - 7.30 (m, 2H), 6.94 (dd, J = 11.2, 8.9 Hz, 1H), 6.14 (dd, J = 8.9, 3.5 Hz, 1H), 4.38 (dd, J = 9.8, 5.9 Hz, 2H), 3.82 (d, J = 1.2 Hz, 2H), 3.66 (dd, J = 11.0, 5.8 Hz, 2H), 3.38 (s, 3H), 2.31 (d, J = 1.8 Hz, 3H). LCMS *m*/*z* 480.22 [M+H]⁺ |
| **314** | 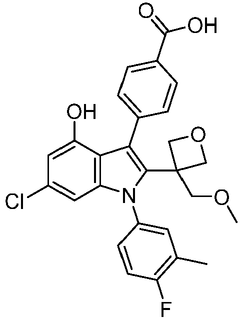 | ¹H NMR (400 MHz, DMSO-*d*₆) δ 12.90 (s, 1H), 9.88 (s, 1H), 7.90 (d, J = 7.8 Hz, 2H), 7.61 (d, J = 7.8 Hz, 2H), 7.49 (d, J = 6.8 Hz, 1H), 7.37 (dd, J = 17.9, 7.9 Hz, 2H), 6.46 (s, 1H), 6.16 (s, 1H), 4.36 (dd, J = 11.3, 5.9 Hz, 2H), 3.81 (s, 2H), 3.65 (dd, J = 11.1, 5.9 Hz, 2H), 3.38 (d, J = 1.8 Hz, 3H), 2.32 (s, 3H). LCMS *m*/*z* 496.24 [M+H]⁺ |
| **315** | 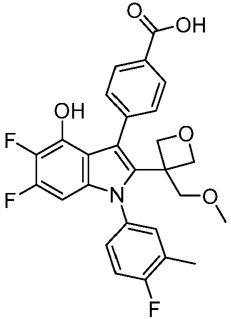 | ¹H NMR (400 MHz, DMSO-*d*₆) δ 12.92 (s, 1H), 10.05 (s, 1H), 7.99 - 7.85 (m, 2H), 7.68 - 7.58 (m, 2H), 7.52 - 7.45 (m, 1H), 7.44 - 7.32 (m, 2H), 6.15 (dd, J = 10.5, 5.8 Hz, 1H), 4.37 (dd, J = 9.7, 5.9 Hz, 2H), 3.80 (d, J = 1.2 Hz, 2H), 3.64 (dd, J = 10.5, 5.9 Hz, 2H), 3.38 (s, 3H), 2.32 (d, J = 1.8 Hz, 3H). LCMS *m*/*z* 498.27 [M+H]⁺ |
| **316** | 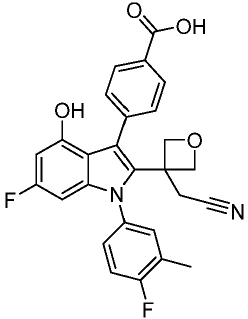 | ¹H NMR (400 MHz, DMSO-*d*₆) δ 9.96 (s, 1H), 7.97 - 7.88 (m, 2H), 7.63 - 7.56 (m, 2H), 7.53 (dd, J = 6.8, 2.6 Hz, 1H), 7.44 (ddd, J = 7.6, 4.5, 2.7 Hz, 1H), 7.37 (t, J = 8.9 Hz, 1H), 6.29 (dd, J = 11.5, 2.2 Hz, 1H), 5.95 (dd, J = 9.5, 2.2 Hz, 1H), 4.48 (t, J = 7.3 Hz, 2H), 3.67 (t, J = 6.4 Hz, 2H), 3.45 (dd, J = 12.6, 5.4 Hz, 2H), 2.32 (d, J = 1.8 Hz, 3H). LCMS *m*/*z* 475.31 [M+H]⁺ |
| **317** | 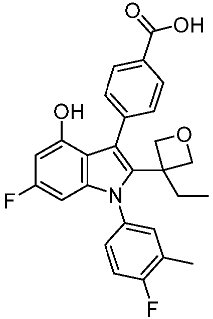 | ¹H NMR (400 MHz, DMSO-*d*₆) δ 12.92 (s, 1H), 9.80 (s, 1H), 7.97 - 7.87 (m, 2H), 7.53 - 7.43 (m, 3H), 7.35 (dd, J = 6.7, 1.8 Hz, 2H), 6.25 (dd, J = 11.5, 2.2 Hz, 1H), 5.90 (dd, J = 9.5, 2.2 Hz, 1H), 4.46 (dd, J = 18.8, 5.8 Hz, 2H), 3.60 (dd, J = 8.4, 5.9 Hz, 2H), 2.34 - 2.29 (m, 3H), 1.98 (q, J = 7.4 Hz, 2H), 1.04 (t, J = 7.4 Hz, 3H). LCMS *m*/*z* 464.21 [M+H]⁺ |
| **318** | 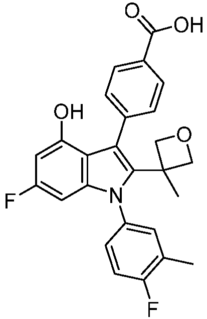 | ¹H NMR (400 MHz, DMSO-*d*₆) δ 10.19 (s, 1H), 7.91 - 7.86 (m, 2H), 7.50 (dd, J = 7.0, 2.6 Hz, 1H), 7.44 - 7.29 (m, 4H), 6.32 (dd, J = 11.5, 2.2 Hz, 1H), 5.91 (dd, J = 9.5, 2.2 Hz, 1H), 4.36 (t, J = 4.9 Hz, 2H), 3.42 (t, J = 5.8 Hz, 2H), 2.31 (d, J = 1.9 Hz, 3H), 1.89 (d, J = 8.3 Hz, 3H). LCMS *m*/*z* 450 [M+H]⁺ |
| **319** | 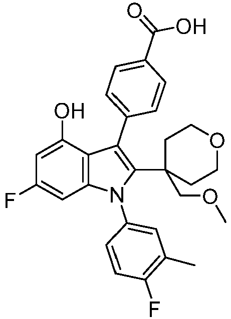 | ¹H NMR (400 MHz, Methanol-*d*₄) δ 8.01 - 7.92 (m, 2H), 7.59 - 7.51 (m, 2H), 7.36 - 7.19 (m, 3H), 6.10 (dd, J = 11.2, 2.2 Hz, 1H), 5.76 (dd, J = 9.8, 2.2 Hz, 1H), 3.51 (d, J = 4.5 Hz, 2H), 3.35 (s, 3H), 2.36 (d, J = 2.0 Hz, 3H), 1.71 (d, J = 14.3 Hz, 2H), 1.42 - 1.29 (m, 3H). LCMS *m*/*z* 508.38 [M+H]⁺ |
| **320** | 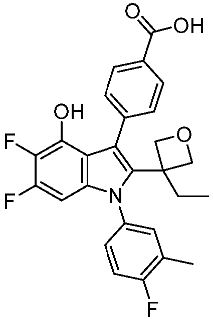 | ¹H NMR (400 MHz, DMSO-*d*₆) δ 12.95 (s, 1H), 9.98 (s, 1H), 8.00 - 7.89 (m, 2H), 7.54 - 7.44 (m, 3H), 7.40 - 7.32 (m, 2H), 6.13 (dd, J = 10.5, 5.7 Hz, 1H), 4.48 (dd, J = 17.5, 5.8 Hz, 2H), 3.60 (dd, J = 7.9, 6.0 Hz, 2H), 2.31 (d, J = 1.8 Hz, 3H), 1.98 (q, J = 7.4 Hz, 2H), 1.03 (t, J = 7.4 Hz, 3H). LCMS *m*/*z* 482.16 [M+H]⁺ |
| **321** | 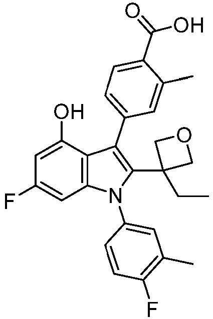 | ¹H NMR (400 MHz, DMSO-*d*₆) δ 12.78 (s, 1H), 9.79 (s, 1H), 7.82 (d, J = 8.0 Hz, 1H), 7.44 (d, J = 6.8 Hz, 1H), 7.34 (dd, J = 6.4, 2.3 Hz, 2H), 7.30 - 7.23 (m, 2H), 6.24 (dd, J = 11.5, 2.2 Hz, 1H), 5.89 (dd, J = 9.5, 2.2 Hz, 1H), 4.49 (d, J = 5.8 Hz, 1H), 4.44 (d, J = 5.9 Hz, 1H), 3.60 (dd, J = 7.9, 6.0 Hz, 2H), 2.57 (s, 3H), 2.31 (d, J = 1.8 Hz, 3H), 1.97 (q, J = 7.4 Hz, 2H), 1.04 (t, J = 7.4 Hz, 3H). LCMS *m*/*z* 478.31 [M+H]⁺ |
| **322** | 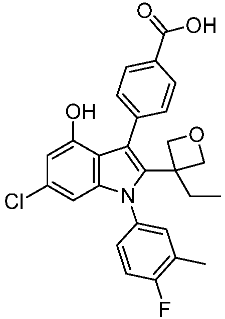 | ¹H NMR (400 MHz, DMSO-*d*₆) δ 12.96 (s, 1H), 9.82 (s, 1H), 7.99 - 7.87 (m, 2H), 7.51 - 7.44 (m, 3H), 7.39 - 7.32 (m, 2H), 6.43 (d, J = 1.8 Hz, 1H), 6.14 (d, J = 1.7 Hz, 1H), 4.49 (d, J = 5.8 Hz, 1H), 4.44 (d, J = 5.9 Hz, 1H), 3.60 (t, J = 6.9 Hz, 2H), 2.32 (d, J = 1.9 Hz, 3H), 1.99 (q, J = 7.3 Hz, 2H), 1.04 (t, J = 7.4 Hz, 3H). LCMS *m*/*z* 480.22 [M+H]⁺ |
| **323** | 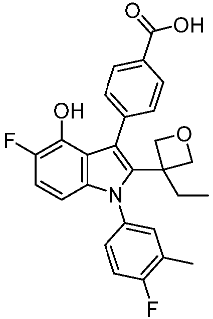 | ¹H NMR (300 MHz, DMSO-*d*₆) δ 12.95 (s, 1H), 9.29 (d, J = 1.8 Hz, 1H), 7.97 - 7.88 (m, 2H), 7.48 (dd, J = 8.0, 6.2 Hz, 3H), 7.38 - 7.31 (m, 2H), 6.91 (dd, J = 11.2, 8.8 Hz, 1H), 6.12 (dd, J = 8.8, 3.5 Hz, 1H), 4.49 (dd, J = 13.0, 5.8 Hz, 2H), 3.61 (t, J = 6.1 Hz, 2H), 2.31 (d, J = 1.9 Hz, 3H), 2.00 (q, J = 7.2 Hz, 2H), 1.05 (t, J = 7.4 Hz, 3H). LCMS *m*/*z* 464.25 [M+H]⁺ |
| **324** | 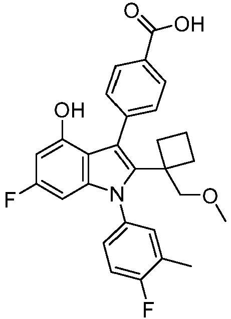 | ¹H NMR (400 MHz, Methanol-*d*₄) δ 7.93 (d, J = 7.8 Hz, 2H), 7.64 (d, J = 7.7 Hz, 2H), 7.36 - 7.17 (m, 3H), 6.15 (d, J = 11.2 Hz, 1H), 5.84 (d, J = 9.6 Hz, 1H), 3.68 (s, 2H), 3.40 (s, 3H), 2.35 (s, 3H), 2.10 - 2.02 (m, 2H), 1.81 - 1.72 (m, 1H), 1.53 - 1.37 (m, 3H). LCMS *m*/*z* 478.29 [M+H]⁺ |
| **325** | 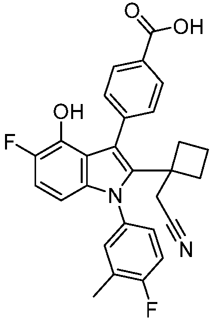 | ¹H NMR (400 MHz, Methanol-*d*₄) δ 8.05 - 7.91 (m, 2H), 7.70 - 7.63 (m, 2H), 7.38 (dd, J = 7.0, 2.6 Hz, 1H), 7.34 - 7.28 (m, 1H), 7.25 (t, J = 8.8 Hz, 1H), 6.84 (dd, J = 11.2, 8.9 Hz, 1H), 6.13 (dd, J = 8.9, 3.5 Hz, 1H), 3.02 (s, 2H), 2.36 (d, J = 2.0 Hz, 3H), 2.28 (dd, J = 12.7, 6.8 Hz, 2H), 1.64 - 1.35 (m, 4H). LCMS *m*/*z* 473.28 [M+H]⁺ |
| **326** | 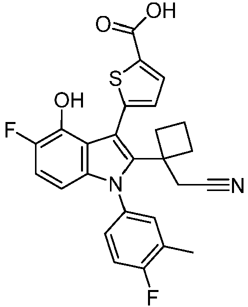 | ¹H NMR (400 MHz, Methanol-*d*₄) δ 7.67 (d, J = 3.7 Hz, 1H), 7.41 - 7.18 (m, 4H), 6.86 (dd, J = 11.2, 8.9 Hz, 1H), 6.14 (dd, J = 8.9, 3.5 Hz, 1H), 3.06 (s, 2H), 2.47 (d, J = 10.8 Hz, 2H), 1.98 (dd, J = 18.4, 8.8 Hz, 1H), 1.73 - 1.47 (m, 3H). LCMS *m*/*z* 479.19 [M+H]⁺ |
| **327** | 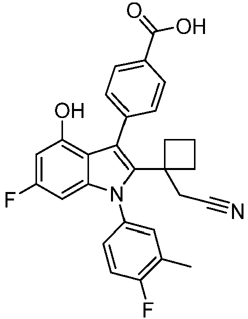 | ¹H NMR (400 MHz, DMSO-*d*₆) δ 12.89 (s, 1H), 9.75 (s, 1H), 7.93 - 7.83 (m, 2H), 7.71 - 7.57 (m, 2H), 7.51 (dd, J = 6.8, 2.5 Hz, 1H), 7.44 - 7.32 (m, 2H), 6.23 (dd, J = 11.4, 2.2 Hz, 1H), 5.87 (dd, J = 9.6, 2.2 Hz, 1H), 3.07 (d, J = 1.7 Hz, 2H), 2.31 (d, J = 1.9 Hz, 3H), 2.11 (q, J = 10.2 Hz, 2H), 1.86 (h, J = 9.7 Hz, 1H), 1.50 - 1.41 (m, 1H), 1.32 (d, J = 6.2 Hz, 2H). LCMS *m*/*z* 473.25 [M+H]⁺ |
| **328** | 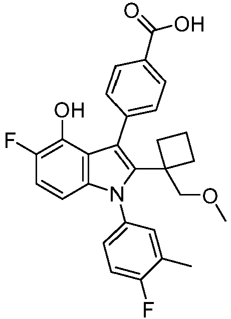 | ¹H NMR (400 MHz, DMSO-*d*₆) δ 12.81 (s, 1H), 9.15 (s, 1H), 7.86 (d, J = 7.5 Hz, 2H), 7.63 (d, J = 7.6 Hz, 2H), 7.45 (d, J = 6.8 Hz, 1H), 7.39 - 7.27 (m, 2H), 6.86 (t, J = 9.7 Hz, 1H), 6.07 (d, J = 8.8 Hz, 1H), 3.63 (s, 2H), 3.31 (s, 3H), 2.30 (s, 3H), 2.01 - 1.89 (m, 2H), 1.77 - 1.67 (m, 1H), 1.45 - 1.39 (m, 1H), 1.34 - 1.24 (m, 2H). LCMS *m*/*z* 478.24 [M+H]⁺ |
| **329** | 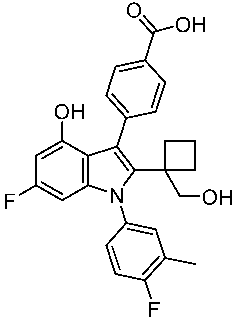 | ¹H NMR (400 MHz, DMSO-*d*₆) δ 12.80 (s, 1H), 9.66 (s, 1H), 7.87 - 7.80 (m, 2H), 7.73 - 7.67 (m, 2H), 7.49 (dd, J = 7.0, 2.6 Hz, 1H), 7.40 (dt, J = 7.7, 4.0 Hz, 1H), 7.32 (t, J = 9.0 Hz, 1H), 6.22 (dd, J = 11.4, 2.3 Hz, 1H), 5.82 (dd, J = 9.6, 2.2 Hz, 1H), 5.15 (t, J = 6.0 Hz, 1H), 3.71 (d, J = 6.0 Hz, 2H), 2.30 (d, J = 1.8 Hz, 3H), 1.87 (q, J = 10.5 Hz, 2H), 1.67 (q, J = 9.6 Hz, 1H), 1.44 - 1.21 (m, 3H). LCMS *m*/*z* 464.23 [M+H]⁺ |
| **330** | 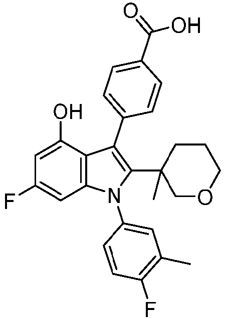 | ¹H NMR (400 MHz, DMSO-*d*₆) δ 12.87 (s, 1H), 9.48 (s, 1H), 7.90 (dq, J = 8.2, 1.6 Hz, 2H), 7.54 - 7.49 (m, 2H), 7.49 - 7.41 (m, 1H), 7.40 - 7.28 (m, 2H), 6.13 (dd, J = 11.4, 2.2 Hz, 1H), 5.76 (dd, J = 9.8, 2.2 Hz, 1H), 3.41 - 3.27 (m, 3H), 2.86 (dd, J = 11.6, 2.2 Hz, 1H), 2.36 - 2.30 (m, 3H), 1.69 - 1.57 (m, 1H), 1.43 - 1.32 (m, 1H), 1.30 - 1.14 (m, 5H). LCMS *m*/*z* 478.27 [M+H]⁺ |
| **331** | 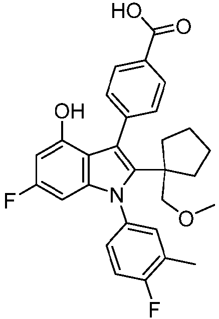 | ¹H NMR (400 MHz, DMSO-*d*₆) δ 12.81 (s, 1H), 9.48 (s, 1H), 7.91 - 7.83 (m, 2H), 7.60 - 7.49 (m, 2H), 7.46 - 7.40 (m, 1H), 7.37 - 7.33 (m, 2H), 6.14 (dd, J = 11.4, 2.2 Hz, 1H), 5.73 (dd, J = 9.7, 2.2 Hz, 1H), 3.27 (s, 3H), 3.20 (q, J = 9.4 Hz, 2H), 2.33 (d, J = 1.8 Hz, 3H), 1.51 - 1.19 (m, 8H). LCMS *m*/*z* 492.26 [M+H]⁺ |
| **332** | 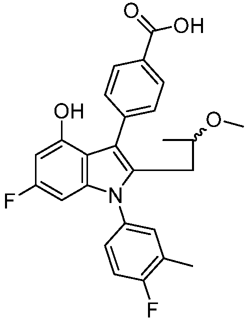 | ¹H NMR (400 MHz, Chloroform-*d*) δ 8.12 - 7.97 (m, 2H), 7.52 (dd, J = 9.1, 6.0 Hz, 2H), 7.25 - 7.01 (m, 3H), 6.22 (dt, J = 10.8, 4.1 Hz, 1H), 6.07 - 5.90 (m, 1H), 3.58 (q, J = 7.2 Hz, 1H), 3.08 - 2.88 (m, 3H), 2.41 - 2.25 (m, 3H), 1.78 (dt, J = 19.4, 7.7 Hz, 2H), 1.64 (dt, J = 12.0, 6.0 Hz, 3H). LCMS *m*/*z* 478.15 [M+H]⁺ |
| **333** | 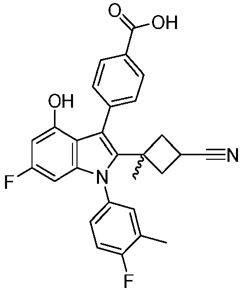 | ¹H NMR (400 MHz, Methanol-*d*₄) δ 8.03 (d, J = 7.7 Hz, 2H), 7.55 (d, J = 7.8 Hz, 2H), 7.40 (d, J = 6.3 Hz, 1H), 7.37 - 7.25 (m, 2H), 6.20 (d, J = 11.4 Hz, 1H), 5.93 (d, J = 9.5 Hz, 1H), 3.14 - 3.03 (m, 1H), 2.40 (s, 3H), 2.36 - 2.27 (m, 2H), 1.72 (s, 3H), 1.66 (t, J = 8.0 Hz, 2H). LCMS *m*/*z* 473.32 [M+H]⁺ |
| **334** | 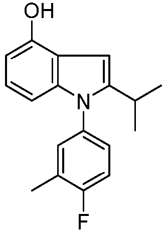 | ¹H NMR (400 MHz, DMSO-*d*₆) δ 9.38 (s, 1H), 7.46 - 7.14 (m, 3H), 6.81 (t, J = 7.9 Hz, 1H), 6.45 (s, 1H), 6.40 (dd, J = 7.7, 0.8 Hz, 1H), 6.36 (dt, J = 8.3, 0.8 Hz, 1H), 3.06 - 2.67 (m, 1H), 2.32 (d, J = 2.0 Hz, 3H), 1.14 (d, J = 6.8 Hz, 6H). LCMS *m*/*z* 284.3 [M+H]⁺ |
| **335** | 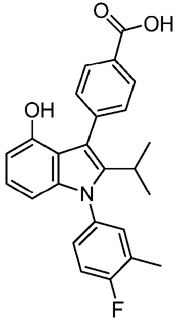 | ¹H NMR (400 MHz, DMSO-*d*₆) δ 12.83 (s, 1H), 9.12 (s, 1H), 7.93 (d, J = 7.9 Hz, 2H), 7.55 - 7.47 (m, 2H), 7.44 (dd, J = 7.1, 2.4 Hz, 1H), 7.39 - 7.26 (m, 2H), 6.82 (td, J = 8.0, 1.5 Hz, 1H), 6.38 (d, J = 7.6 Hz, 1H), 6.21 (d, J = 8.2 Hz, 1H), 2.98 (p, J = 7.2 Hz, 1H), 2.38 - 2.27 (m, 3H), 1.01 (dd, J = 7.2, 5.2 Hz, 6H). LCMS *m*/*z* 404.27 [M+H]⁺ |
| **336** | 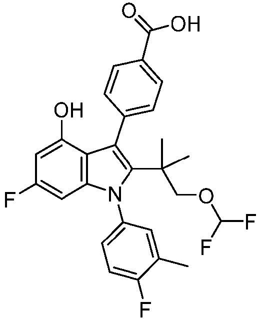 | ¹H NMR (400 MHz, Methanol-*d*₄) δ 8.00 (d, J = 7.8 Hz, 2H), 7.53 (d, J = 7.8 Hz, 2H), 7.38 - 7.24 (m, 3H), 6.48 - 6.07 (m, 2H), 5.75 (d, J = 9.7 Hz, 1H), 3.62 (s, 2H), 2.38 (s, 3H), 1.09 (d, J = 6.2 Hz, 6H). LCMS *m*/*z* 502.27 [M+H]⁺ |
| **337** | 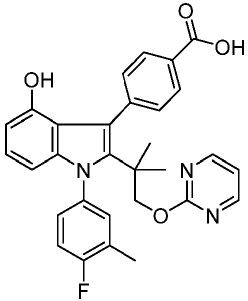 | ¹H NMR (400 MHz, Chloroform-d) δ 8.44 (tt, J = 5.3, 2.3 Hz, 2H), 7.99 (dd, J = 8.5, 3.6 Hz, 2H), 7.76 - 7.59 (m, 2H), 7.38 (d, J = 9.7 Hz, 1H), 7.17 - 7.05 (m, 1H), 6.97 (q, J = 4.8, 3.8 Hz, 1H), 6.89 (q, J = 7.1 Hz, 1H), 6.40 (t, J = 5.8 Hz, 1H), 6.20 (t, J = 6.2 Hz, 1H), 3.73 (d, J = 7.2 Hz, 2H), 2.30 - 2.13 (m, 3H), 1.36 - 1.13 (m, 6H). LCMS *m*/*z* 512.26 [M+H]⁺ |
| **338** | 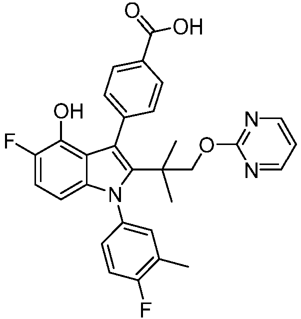 | ¹H NMR (400 MHz, Chloroform-*d*) δ 8.52 - 8.36 (m, 2H), 8.03 - 7.91 (m, 2H), 7.59 (dd, J = 9.1, 4.8 Hz, 2H), 7.25 (dd, J = 8.0, 4.2 Hz, 2H), 7.09 (ddd, J = 11.6, 7.5, 2.8 Hz, 1H), 6.97 (td, J = 4.9, 2.7 Hz, 1H), 6.80 (ddd, J = 13.7, 7.4, 2.8 Hz, 1H), 6.06 (dt, J = 8.9, 3.3 Hz, 1H), 4.05 - 3.97 (m, 2H), 2.22 (p, J = 6.1, 5.0 Hz, 3H), 1.25 (td, J = 8.6, 7.8, 4.3 Hz, 6H). LCMS *m*/*z* 530.26 [M+H]⁺ |
| **339** | 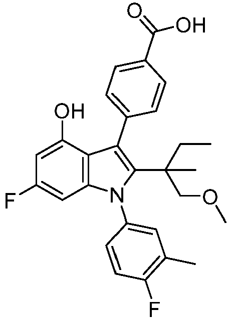 | ¹H NMR (400 MHz, DMSO-*d*₆) δ 12.85 (s, 1H), 9.39 (d, J = 1.6 Hz, 1H), 7.88 (ddd, J = 8.4, 4.0, 1.9 Hz, 2H), 7.48 - 7.42 (m, 2H), 7.42 - 7.22 (m, 3H), 6.09 (dd, J = 11.4, 2.2 Hz, 1H), 5.66 (dd, J = 9.8, 2.2 Hz, 1H), 3.12 (dd, J = 19.5, 9.0 Hz, 1H), 3.05 (d, J = 3.2 Hz, 3H), 2.86 (dd, J = 9.0, 6.0 Hz, 1H), 2.33 (d, J = 1.8 Hz, 3H), 1.49 - 1.37 (m, 1H), 1.10 (dt, J = 13.5, 6.7 Hz, 1H), 0.85 (d, J = 3.3 Hz, 3H), 0.72 - 0.65 (m, 3H). LCMS *m*/*z* 480.25 [M+H]⁺ |
| **340** | 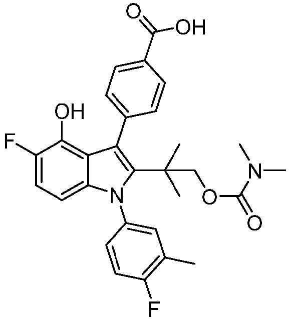 | ¹H NMR (400 MHz, DMSO-*d*₆) δ 8.00 - 7.73 (m, 2H), 7.48 - 7.37 (m, 2H), 7.12 - 6.96 (m, 3H), 6.66 (dd, J = 11.0, 8.9 Hz, 1H), 5.90 (dd, J = 8.8, 3.5 Hz, 1H), 3.74 - 3.56 (m, 2H), 2.69 (s, 6H), 2.19 (d, J = 1.9 Hz, 3H), 0.95 (d, J = 5.2 Hz, 6H). LCMS *m*/*z* 523.29 [M+H]⁺ |
| **341** | 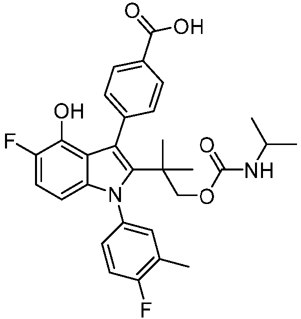 | ¹H NMR (400 MHz, Chloroform-d) δ 8.13 - 8.00 (m, 2H), 7.58 (d, J = 7.6 Hz, 2H), 7.29 - 7.12 (m, 3H), 6.88 - 6.72 (m, 1H), 6.07 (dd, J = 8.8, 3.5 Hz, 1H), 3.38 (p, J = 1.7 Hz, 2H), 2.35 (s, 3H), 1.15 (d, J = 6.5 Hz, 6H), 1.07 (d, J = 3.6 Hz, 6H). LCMS *m*/*z* 537.26 [M+H]⁺ |
| **342** | 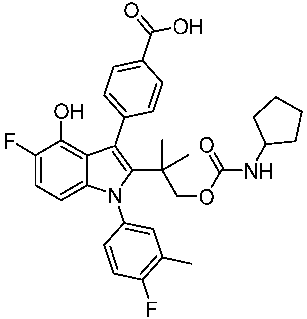 | LCMS *m*/*z* 563.26 [M+H]⁺ |
| **343** | 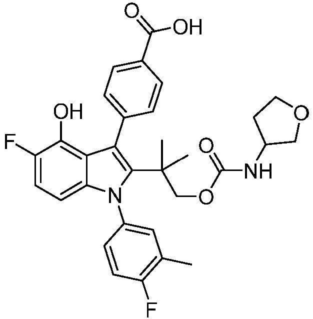 | ¹H NMR (400 MHz, Chloroform-*d*) δ 7.90 - 7.80 (m, 2H), 7.38 (d, J = 7.9 Hz, 2H), 7.10 - 6.93 (m, 3H), 6.62 (dd, J = 10.9, 8.9 Hz, 1H), 5.93 (d, J = 7.2 Hz, 1H), 5.87 (dd, J = 8.9, 3.5 Hz, 1H), 3.68 (dq, J = 35.8, 7.8 Hz, 4H), 3.58 (s, 2H), 3.45 (dd, J = 9.2, 3.2 Hz, 1H), 2.17 (d, J = 1.8 Hz, 3H), 2.02 (dt, J = 14.8, 7.4 Hz, 1H), 0.90 (d, J = 3.0 Hz, 6H). LCMS *m*/*z* 565.29 [M+H]⁺ |
| **344** | 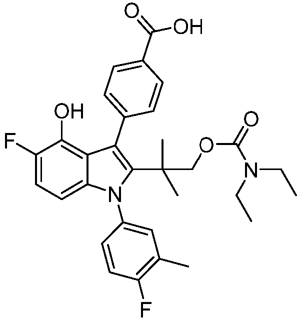 | ¹H NMR (400 MHz, DMSO-*d*₆) δ 12.91 (s, 1H), 8.96 (d, J = 2.0 Hz, 1H), 7.97 - 7.81 (m, 2H), 7.52 - 7.37 (m, 2H), 7.41 - 7.20 (m, 3H), 6.83 (dd, J = 11.1, 8.8 Hz, 1H), 5.93 (dd, J = 8.9, 3.5 Hz, 1H), 3.73 - 3.52 (m, 2H), 2.29 (d, J = 1.8 Hz, 3H), 1.17 - 0.73 (m, 12H). LCMS *m*/*z* 551.25 [M+H]⁺ |
| **345** | 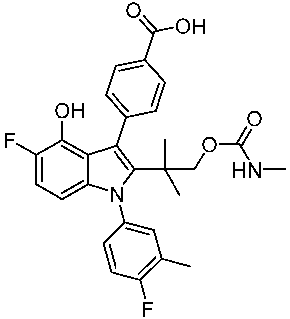 | ¹H NMR (400 MHz, Chloroform-*d*) δ 8.18 (dd, J = 8.1, 5.3 Hz, 2H), 7.68 (dd, J = 17.9, 8.2 Hz, 2H), 7.28 - 7.13 (m, 2H), 6.87 (dd, J = 10.7, 8.9 Hz, 2H), 6.15 - 6.11 (m, 1H), 3.45 (d, J = 55.7 Hz, 3H), 2.38 (d, J = 5.1 Hz, 2H), 2.04 (s, 3H), 1.09 (s, 6H). LCMS *m*/*z* 509.18 [M+H]⁺ |
| **346** | 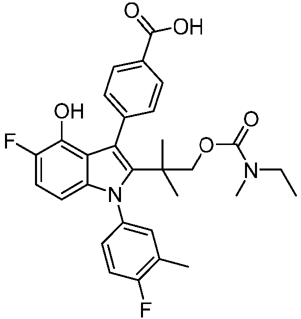 | ¹H NMR (400 MHz, Chloroform-*d*) δ 7.91 - 7.77 (m, 2H), 7.43 - 7.32 (m, 2H), 7.09 - 6.90 (m, 3H), 6.63 (dd, J = 11.0, 8.9 Hz, 1H), 5.87 (dd, J = 8.9, 3.5 Hz, 1H), 3.68 - 3.56 (m, 2H), 3.19 (p, J = 1.6 Hz, 2H), 2.65 (d, J = 16.0 Hz, 3H), 2.17 (d, J = 1.9 Hz, 3H), 0.93 (d, J = 5.6 Hz, 9H). LCMS *m*/*z* 537.21 [M+H]⁺ |
| **347** | 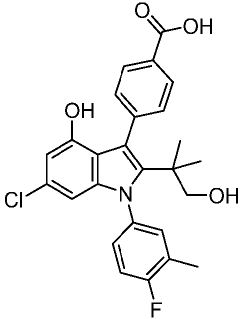 | ¹H NMR (400 MHz, DMSO-*d*₆) δ 12.84 (s, 1H), 9.39 (s, 1H), 7.93 - 7.82 (m, 2H), 7.48 (ddt, J = 10.1, 7.0, 2.2 Hz, 3H), 7.41 - 7.32 (m, 2H), 6.27 (d, J = 1.8 Hz, 1H), 5.94 (d, J = 1.8 Hz, 1H), 4.66 (t, J = 5.7 Hz, 1H), 3.20 (d, J = 5.8 Hz, 2H), 2.32 (d, J = 1.9 Hz, 3H), 0.89 (s, 6H). LCMS *m*/*z* 468 [M+H]⁺ |
| **348** | 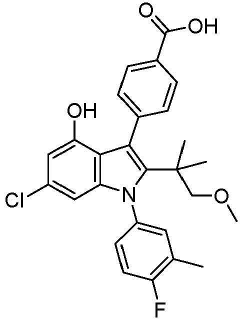 | ¹H NMR (400 MHz, DMSO-*d*₆) δ 9.42 (s, 1H), 7.94 - 7.85 (m, 2H), 7.47 (dq, J = 8.3, 1.8 Hz, 2H), 7.42 (dd, J = 6.9, 2.3 Hz, 1H), 7.38 - 7.32 (m, 2H), 6.28 (d, J = 1.7 Hz, 1H), 5.96 (d, J = 1.7 Hz, 1H), 2.99 (s, 3H), 2.90 (s, 2H), 2.33 (d, J = 1.9 Hz, 3H), 0.98 (d, J = 5.0 Hz, 6H). LCMS *m*/*z* 482 [M+H]⁺ |
| **349** | 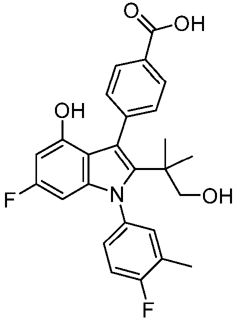 | ¹H NMR (400 MHz, Chloroform-*d*/CD3OD) δ 8.09 (d, J = 8.1 Hz, 2H), 7.63 (d, J = 7.9 Hz, 2H), 7.26 (dd, J = 14.5, 6.0 Hz, 2H), 7.20 - 7.01 (m, 1H), 6.19 (d, J = 11.1 Hz, 1H), 5.87 (d, J = 9.7 Hz, 1H), 3.37 - 3.15 (m, 2H), 2.32 (s, 3H), 1.00 (d, J = 10.5 Hz, 6H). LCMS *m*/*z* 452.28 [M+H]⁺ |
| **350** | 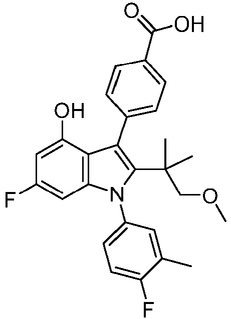 | ¹H NMR (400 MHz, Chloroform-*d*) δ 8.12 - 8.04 (m, 2H), 7.63 - 7.55 (m, 2H), 7.12 (ddd, J = 12.6, 5.8, 2.2 Hz, 2H), 7.03 (t, J = 8.7 Hz, 1H), 6.10 (dd, J = 10.8, 2.2 Hz, 1H), 5.78 (dd, J = 9.6, 2.2 Hz, 1H), 4.55 (s, 1H), 2.96 (s, 3H), 2.84 (s, 2H), 2.23 (d, J = 1.9 Hz, 3H), 0.92 (d, J = 2.7 Hz, 6H). LCMS *m*/*z* 466.28 [M+H]⁺ |
| **351** | 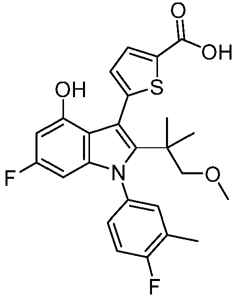 | ¹H NMR (400 MHz, DMSO-*d*₆) δ 12.97 (s, 1H), 9.64 (s, 1H), 7.64 (d, J = 3.7 Hz, 1H), 7.37 (dd, J = 17.6, 9.0 Hz, 3H), 7.05 (d, J = 3.7 Hz, 1H), 6.16 (dd, J = 11.4, 2.2 Hz, 1H), 5.70 (dd, J = 9.7, 2.2 Hz, 1H), 3.04 (s, 3H), 2.97 (s, 2H), 2.32 (d, J = 1.8 Hz, 3H), 1.07 (d, J = 1.3 Hz, 6H). LCMS *m*/*z* 472 [M+H]⁺ |
| **352** | 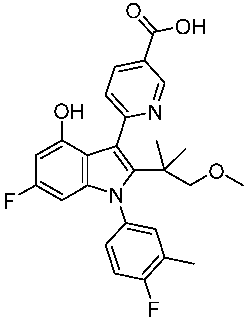 | ¹H NMR (400 MHz, Methanol-*d*₄) δ 9.08 (dd, J = 2.1, 0.9 Hz, 1H), 8.40 (d, J = 8.1 Hz, 1H), 7.76 (d, J = 8.2 Hz, 1H), 7.38 - 7.24 (m, 3H), 6.11 (dd, J = 11.2, 2.1 Hz, 1H), 5.74 (dd, J = 9.7, 2.1 Hz, 1H), 3.09 (s, 3H), 3.02 (s, 2H), 2.41 - 2.34 (m, 3H), 1.05 (d, J = 4.6 Hz, 6H). LCMS *m*/*z* 467.3 [M+H]⁺ |
| **353** | 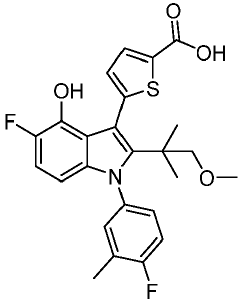 | ¹H NMR (400 MHz, DMSO-*d*₆) δ 13.02 (s, 1H), 9.15 (d, J = 1.9 Hz, 1H), 7.64 (d, J = 3.7 Hz, 1H), 7.38 (dd, J = 19.4, 10.9 Hz, 3H), 7.07 (d, J = 3.7 Hz, 1H), 6.85 (dd, J = 11.2, 8.8 Hz, 1H), 5.93 (dd, J = 8.9, 3.5 Hz, 1H), 3.04 (s, 3H), 3.00 (s, 2H), 2.32 (d, J = 1.8 Hz, 3H), 1.15 - 1.06 (m, 6H). LCMS *m*/*z* 472 [M+H]⁺ |
| **354** | 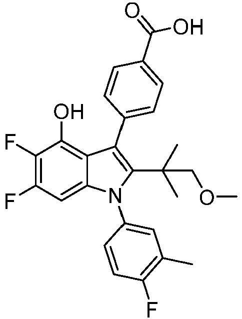 | ¹H NMR (400 MHz, DMSO-*d*₆) δ 9.62 (s, 1H), 7.97 - 7.89 (m, 2H), 7.55 - 7.46 (m, 2H), 7.45 - 7.26 (m, 3H), 5.91 (dd, J = 10.7, 5.8 Hz, 1H), 2.99 (s, 3H), 2.90 (s, 2H), 2.33 (d, J = 1.9 Hz, 3H), 0.98 (d, J = 5.2 Hz, 6H). LCMS *m*/*z* 484 [M+H]⁺ |
| **355** | 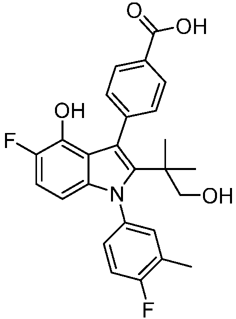 | ¹H NMR (400 MHz, DMSO-*d*₆) δ 12.84 (s, 1H), 8.90 (d, J = 2.0 Hz, 1H), 7.93 - 7.70 (m, 2H), 7.67 - 7.50 (m, 2H), 7.46 (dd, J = 7.2, 2.3 Hz, 1H), 7.43 - 7.25 (m, 2H), 6.80 (dd, J = 11.1, 8.9 Hz, 1H), 5.91 (dd, J = 8.8, 3.5 Hz, 1H), 4.66 (t, J = 5.7 Hz, 1H), 3.22 (d, J = 5.3 Hz, 2H), 2.31 (d, J = 1.8 Hz, 3H), 0.88 (d, J = 19.0 Hz, 6H). LCMS *m*/*z* 452.24 [M+H]⁺ |
| **356** | 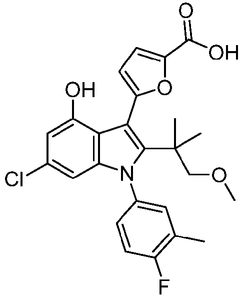 | LCMS *m*/*z* 472 [M+H]⁺ |
| **357** | 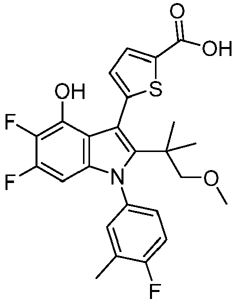 | ¹H NMR (400 MHz, DMSO-*d*₆) δ 9.86 (s, 1H), 7.58 (d, J = 3.7 Hz, 1H), 7.39 (dd, J = 18.9, 10.5 Hz, 3H), 7.04 (d, J = 3.7 Hz, 1H), 5.91 (dd, J = 10.6, 5.8 Hz, 1H), 3.04 (s, 3H), 2.99 (s, 2H), 2.33 (d, J = 1.8 Hz, 3H), 1.09 (s, 6H). LCMS *m*/*z* 490 [M+H]⁺ |
| **358** | 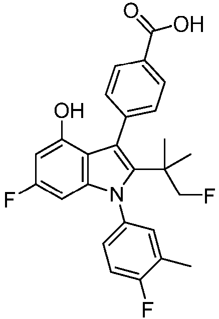 | ¹H NMR (400 MHz, DMSO-*d*₆) δ 12.91 (s, 1H), 9.51 (s, 1H), 7.91 (d, J = 8.5 Hz, 2H), 7.52 - 7.44 (m, 3H), 7.39 - 7.32 (m, 2H), 6.13 (dd, J = 11.4, 2.1 Hz, 1H), 5.74 (dd, J = 9.8, 2.1 Hz, 1H), 4.08 (d, J = 47.5 Hz, 2H), 2.33 (s, 3H), 0.99 (s, 6H). LCMS *m*/*z* 454.17 [M+H]⁺ |
| **359** | 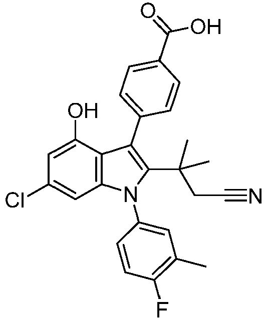 | ¹H NMR (400 MHz, DMSO-*d*₆) δ 9.61 (s, 1H), 7.97 - 7.90 (m, 2H), 7.56 - 7.47 (m, 3H), 7.47 - 7.33 (m, 2H), 6.34 (d, J = 1.8 Hz, 1H), 6.01 (d, J = 1.7 Hz, 1H), 2.72 (s, 2H), 2.35 (d, J = 1.8 Hz, 3H), 1.13 (d, J = 3.4 Hz, 6H). LCMS *m*/*z* 477 [M+H]⁺ |
| **360** | 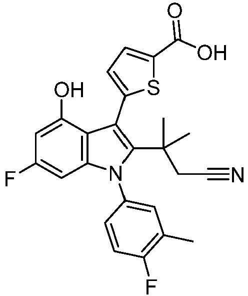 | ¹H NMR (400 MHz, DMSO-*d*₆) δ 13.08 (s, 1H), 9.80 (s, 1H), 7.67 (d, J = 3.7 Hz, 1H), 7.54 - 7.32 (m, 3H), 7.13 (d, J = 3.7 Hz, 1H), 6.21 (dd, J = 11.4, 2.2 Hz, 1H), 5.76 (dd, J = 9.6, 2.2 Hz, 1H), 2.59 (s, 2H), 2.33 (d, J = 1.8 Hz, 3H), 1.22 (s, 6H). LCMS *m*/*z* 467 [M+H]⁺ |
| **361** | 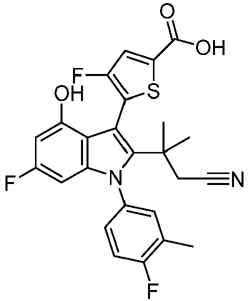 | ¹H NMR (400 MHz, DMSO-*d*₆) δ 13.47 (s, 1H), 9.94 (s, 1H), 7.64 (s, 1H), 7.56 - 7.45 (m, 1H), 7.45 - 7.34 (m, 2H), 6.23 (dd, J = 11.4, 2.2 Hz, 1H), 5.81 - 5.73 (m, 1H), 2.68 - 2.57 (m, 2H), 2.33 (t, J = 2.5 Hz, 3H), 1.26 (d, J = 2.7 Hz, 6H). LCMS *m*/*z* 485 [M+H]⁺ |
| **362** | 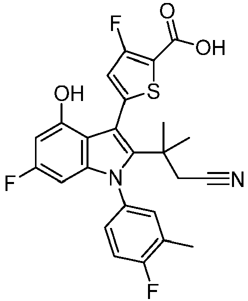 | ¹H NMR (400 MHz, DMSO-*d*₆) δ 9.96 (s, 1H), 7.56 - 7.27 (m, 3H), 7.12 (s, 1H), 6.29 - 6.19 (m, 1H), 5.79 - 5.69 (m, 1H), 2.63 (s, 2H), 2.33 (s, 3H), 1.25 (s, 6H). LCMS *m*/*z* 485 [M+H]⁺ |
| **363** | 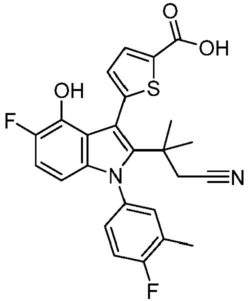 | ¹H NMR (400 MHz, DMSO-*d*₆) δ 13.11 (s, 1H), 9.31 (d, J = 1.9 Hz, 1H), 7.67 (d, J = 3.7 Hz, 1H), 7.48 (s, 1H), 7.40 (d, J = 8.5 Hz, 2H), 7.15 (d, J = 3.7 Hz, 1H), 6.91 (dd, J = 11.2, 8.9 Hz, 1H), 5.98 (dd, J = 8.9, 3.4 Hz, 1H), 2.62 (s, 2H), 2.33 (d, J = 1.8 Hz, 3H), 1.23 (s, 6H). LCMS *m*/*z* 467 [M+H]⁺ |
| **364** | 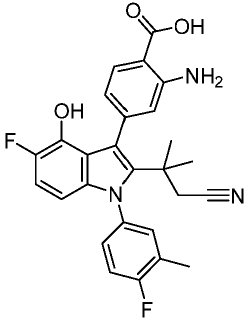 | ¹H NMR (400 MHz, DMSO-*d*₆) δ 9.53 (s, 1H), 7.77 - 7.61 (m, 1H), 7.49 - 7.31 (m, 3H), 6.79 (d, J = 1.9 Hz, 1H), 6.58 (d, J = 8.3 Hz, 1H), 6.15 (dd, J = 11.4, 2.3 Hz, 1H), 5.75 - 5.69 (m, 1H), 2.53 - 2.52 (m, 2H), 2.33 (t, J = 1.6 Hz, 3H), 1.20 - 1.10 (m, 6H). LCMS *m*/*z* 476.34 [M+H]⁺ |
| **365** | 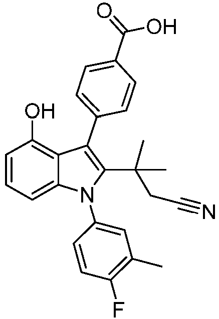 | ¹H NMR (400 MHz, Chloroform-*d*) δ 8.14 (ddt, J = 8.9, 2.8, 1.5 Hz, 2H), 7.78 - 7.62 (m, 2H), 7.34 (d, J = 2.3 Hz, 1H), 7.25 - 7.14 (m, 1H), 7.02 - 6.86 (m, 2H), 6.44 (dd, J = 8.7, 3.2 Hz, 1H), 6.27 - 6.18 (m, 1H), 3.39 (dp, J = 3.3, 1.5 Hz, 2H), 2.67 - 2.62 (m, 2H), 2.38 (d, J = 2.0 Hz, 3H), 1.29 - 1.23 (m, 6H). LCMS *m*/*z* 443.32 [M+H]⁺ |
| **366** | 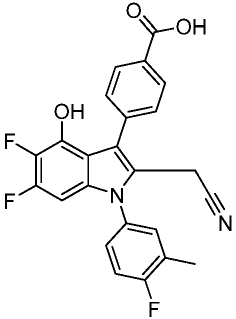 | ¹H NMR (400 MHz, DMSO-*d*₆) δ 9.79 (s, 1H), 7.98 - 7.92 (m, 2H), 7.58 - 7.53 (m, 2H), 7.48 (dd, J = 6.9, 2.5 Hz, 1H), 7.45 - 7.34 (m, 2H), 5.96 (dd, J = 10.6, 5.8 Hz, 1H), 2.49 (s, 2H), 2.34 (d, J = 1.9 Hz, 3H), 1.13 (d, J = 3.2 Hz, 6H). LCMS *m*/*z* 479 [M+H]⁺ |
| **367** | 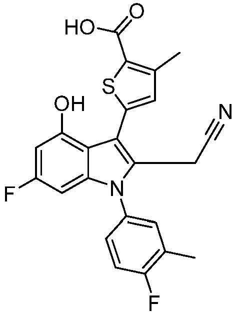 | ¹H NMR (400 MHz, DMSO-*d*₆) δ 12.86 (s, 1H), 9.79 (s, 1H), 7.54 - 7.34 (m, 3H), 7.01 (s, 1H), 6.20 (dd, J = 11.4, 2.2 Hz, 1H), 5.76 (dd, J = 9.6, 2.2 Hz, 1H), 2.61 (s, 2H), 2.34 (d, J = 1.8 Hz, 3H), 1.25 (d, J = 7.7 Hz, 9H). LCMS *m*/*z* 481 [M+H]⁺ |
| **368** | 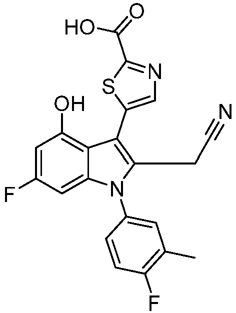 | ¹H NMR (400 MHz, DMSO-*d*₆) δ 9.79 (s, 1H), 9.17 (d, J = 0.8 Hz, 1H), 7.83 (d, J = 0.8 Hz, 1H), 7.48 (d, J = 6.6 Hz, 1H), 7.41 (d, J = 9.0 Hz, 1H), 6.21 (dd, J = 11.5, 2.2 Hz, 1H), 5.77 (dd, J = 9.6, 2.2 Hz, 1H), 2.60 (s, 2H), 2.34 (d, J = 1.9 Hz, 3H), 1.20 (s, 6H). LCMS *m*/*z* 468 [M+H]⁺ |
| **369** | 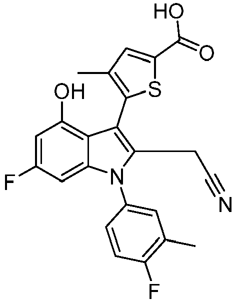 | ¹H NMR (400 MHz, DMSO-*d*₆) δ 12.94 (s, 1H), 9.72 (d, J = 1.1 Hz, 1H), 7.58 (s, 1H), 7.56 - 7.47 (m, 1H), 7.47 - 7.34 (m, 2H), 6.20 (dd, J = 11.5, 2.2 Hz, 1H), 5.76 (ddd, J = 9.6, 3.8, 2.2 Hz, 1H), 2.66 - 2.54 (m, 2H), 2.34 (dd, J = 6.8, 1.8 Hz, 3H), 2.09 (s, 3H), 1.29 - 1.20 (m, 6H). LCMS *m*/*z* 481 [M+H]⁺ |
| **370** | 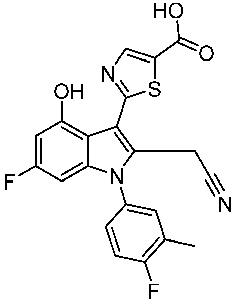 | ¹H NMR (400 MHz, DMSO-*d*₆) δ 10.10 (s, 1H), 8.27 (s, 1H), 7.58 - 7.50 (m, 1H), 7.46 - 7.38 (m, 2H), 6.28 (dd, J = 11.4, 2.2 Hz, 1H), 5.80 (dd, J = 9.5, 2.2 Hz, 1H), 2.72 (s, 2H), 2.34 (d, J = 1.8 Hz, 3H), 1.17 (s, 6H). LCMS *m*/*z* 468 [M+H]⁺ |
| **371** | 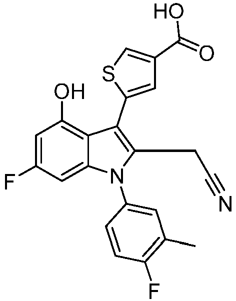 | ¹H NMR (400 MHz, DMSO-*d*₆) δ 12.73 (s, 1H), 9.77 (s, 1H), 8.31 (d, J = 1.4 Hz, 1H), 7.48 (d, J = 6.5 Hz, 1H), 7.41 (q, J = 6.8, 5.0 Hz, 2H), 7.35 (d, J = 1.4 Hz, 1H), 6.21 (dd, J = 11.4, 2.2 Hz, 1H), 5.76 (dd, J = 9.6, 2.2 Hz, 1H), 2.60 (s, 2H), 2.34 (d, J = 1.8 Hz, 3H), 1.22 (s, 6H). LCMS *m*/*z* 467 [M+H]⁺ |
| **372** | 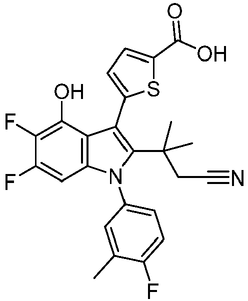 | ¹H NMR (400 MHz, DMSO-*d*₆) δ 13.11 (s, 1H), 10.03 (d, J = 1.6 Hz, 1H), 7.70 (d, J = 3.7 Hz, 1H), 7.55 - 7.33 (m, 3H), 7.18 (d, J = 3.7 Hz, 1H), 5.99 (dd, J = 10.5, 5.8 Hz, 1H), 2.61 (s, 2H), 2.34 (d, J = 1.7 Hz, 3H), 1.23 (s, 6H). LCMS *m*/*z* 485 [M+H]⁺ |
| **373** | 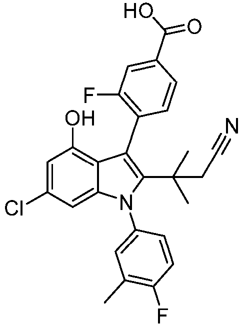 | ¹H NMR (400 MHz, DMSO-*d*₆) δ 13.27 (s, 1H), 9.70 (d, J = 1.8 Hz, 1H), 7.79 (dt, J = 7.9, 1.8 Hz, 1H), 7.69 (dd, J = 9.7, 1.7 Hz, 1H), 7.60 (dd, J = 7.6, 2.5 Hz, 1H), 7.50 - 7.33 (m, 3H), 6.34 (d, J = 1.7 Hz, 1H), 6.02 (t, J = 1.9 Hz, 1H), 2.58 - 2.54 (m, 2H), 2.35 (dd, J = 4.9, 1.8 Hz, 3H), 1.17 (dd, J = 6.2, 4.1 Hz, 6H). LCMS *m*/*z* 495 [M+H]⁺ |
| **374** | 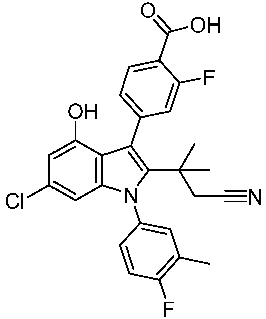 | ¹H NMR (400 MHz, DMSO-*d*₆) δ 13.24 (s, 1H), 9.68 (s, 1H), 7.85 (td, J = 8.2, 1.3 Hz, 1H), 7.52 - 7.47 (m, 1H), 7.45 - 7.26 (m, 4H), 6.34 (d, J = 1.8 Hz, 1H), 6.00 (d, J = 1.7 Hz, 1H), 2.54 (s, 2H), 2.34 (d, J = 2.1 Hz, 3H), 1.15 (dd, J = 7.3, 3.3 Hz, 6H). LCMS *m*/*z* 495.14 [M+H]⁺ |
| **375** | 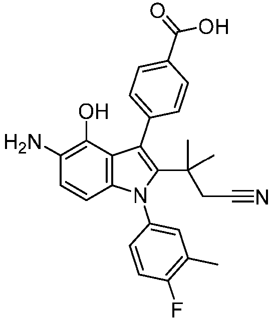 | ¹H NMR (400 MHz, DMSO-*d*₆) δ 8.01 - 7.85 (m, 2H), 7.54 (d, J = 8.4 Hz, 2H), 7.36 (td, J = 17.3, 16.6, 6.9 Hz, 3H), 6.52 (d, J = 8.5 Hz, 1H), 5.90 (d, J = 8.5 Hz, 1H), 2.48 - 2.42 (m, 2H), 2.32 (s, 3H), 1.13 (d, J = 3.5 Hz, 6H). LCMS *m*/*z* 458.26 [M+H]⁺ |
| **376** | 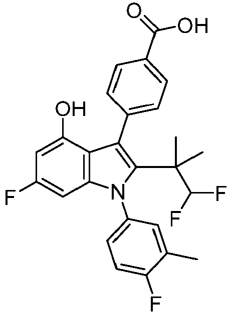 | ¹H NMR (400 MHz, DMSO-*d*₆) δ 12.93 (s, 1H), 9.59 (s, 1H), 7.92 (d, J = 7.8 Hz, 2H), 7.51 - 7.43 (m, 3H), 7.39 - 7.33 (m, 2H), 6.14 (d, J = 11.2 Hz, 1H), 6.00 - 5.70 (m, 2H), 2.33 (s, 3H), 0.98 (s, 6H). LCMS *m*/*z* 472.22 [M+H]⁺ |

### Compound 377

### 4-[3-(4-fluorophenyl)-7-hydroxy-2-tetrahydropyran-4-yl-indol-1-yl]benzoic acid (377)

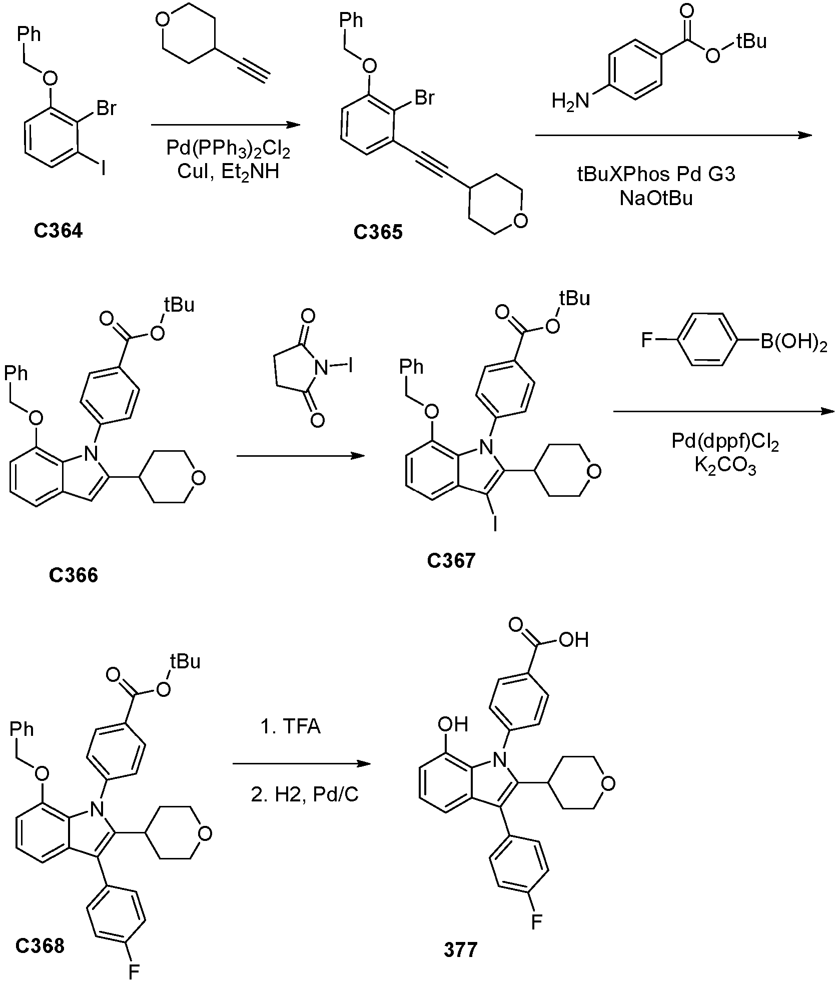

### Step 1. Synthesis of 4-[2-(3-benzyloxy-2-bromo-phenyl)ethynyl]tetrahydropyran (C365)

A solution of 1-benzyloxy-2-bromo-3-iodo-benzene (3.02 g, 7.375 mmol) in DMF (18 mL) was bubbled through with N₂ for 5 minutes. Then diethylamine (1.1 mL, 10.63 mmol) and 4-ethynyltetrahydropyran (1.06 g, 9.623 mmol) were added. The mixture was bubbled through with nitrogen for a further 2 minutes. Copper (I) iodide (211 mg, 1.108 mmol) and Pd(PPh₃)₂Cl₂ (260 mg, 0.3704 mmol) were added. The reaction mixture was heated to 65 °C. After 17 hours, the mixture was partitioned between EtOAc and water. The organic layer was separated, washed twice with brine, dried (MgSO4), filtered and concentrated *in vacuo.* Purification via silica gel chromatography with 80g isco column using 0-50% (EtOAc/Heptanes) afforded 2.4 grams of red oil. 4-[2-(3-benzyloxy-2-bromo-phenyl)ethynyl]tetrahydropyran (2.4 g, 88%). ¹H NMR (400 MHz, DMSO-*d*₆) δ 7.51 - 7.44 (m, 2H), 7.44 - 7.37 (m, 2H), 7.37 - 7.25 (m, 2H), 7.17 (dd, J = 8.4, 1.4 Hz, 1H), 7.09 (dd, J = 7.6, 1.4 Hz, 1H), 5.23 (s, 2H), 3.83 (ddd, J = 11.5, 5.9, 3.6 Hz, 2H), 3.49 (ddd, J = 11.4, 8.3, 2.9 Hz, 2H), 2.97 (tt, J = 8.3, 4.1 Hz, 1H), 1.92 - 1.80 (m, 2H), 1.62 (dtd, J = 13.3, 8.4, 3.6 Hz, 2H).

### Step 2. Synthesis of tert-butyl 4-(7-benzyloxy-2-tetrahydropyran-4-yl-indol-1-yl)benzoate (C366)

To a solution of 4-[2-(3-benzyloxy-2-bromo-phenyl)ethynyl]tetrahydropyran (1.90 g, 5.118 mmol) and tert-butyl 4-aminobenzoate (1.2 g, 6.210 mmol) in xylene (25 mL) was added NaOtBu (1.5 g, 15.61 mmol) followed by tBuXPhos Pd G3 (210 mg, 0.2644 mmol). The reaction mixture was stirred at room temperature for 16 hours. The mixture was diluted with aqueous saturated NH₄Cl solution and extracted twice with EtOAc. The combined organics were washed with brine, dried (MgSO₄), filtered and concentrated *in vacuo.* Purification by ISCO (80g, gold column) eluting with Heptane/ethylacetate 0-30% over 24 minutes afforded the cyclized product. tert-butyl 4-(7-benzyloxy-2-tetrahydropyran-4-yl-indol-1-yl)benzoate (1.12 g, 45%). ¹H NMR (400 MHz, Chloroform-d) δ 7.85 (d, J = 8.7 Hz, 2H), 7.41 - 7.18 (m, 5H), 7.16 - 6.89 (m, 3H), 6.85 - 6.63 (m, 2H), 6.18 (s, 1H), 5.10 (s, 2H), 3.75 (ddd, J = 11.6, 6.1, 3.5 Hz, 2H), 3.44 (ddd, J = 11.4, 8.1, 3.0 Hz, 2H), 2.72 (tt, J = 8.2, 4.1 Hz, 1H), 1.81 - 1.63 (m, 2H), 1.60 (s, 9H), 1.47 (dtd, J = 13.4, 8.2, 3.5 Hz, 2H). LCMS *m*/*z* 484.08 [M+H]⁺

### Step 3. Synthesis of tert-butyl 4-(7-benzyloxy-3-iodo-2-tetrahydropyran-4-yl-indol-1-yl)benzoate (C367)

To a solution of tert-butyl 4-(7-benzyloxy-2-tetrahydropyran-4-yl-indol-1-yl)benzoate (1.10 g, 2.275 mmol) in dichloromethane (25.0 mL) was added N-iodosuccinimide (540.0 mg, 2.400 mmol). The reaction mixture was stirred at room temperature for 30 minutes. The reaction was quenched with saturated NaHCO₃, back extracted with dichloromethane, concentrated and dried and purified using ISCO(40g gold column) eluting with Ethyl acetate/Heptane (0-60% over 18 min) to afford tert-butyl 4-(7-benzyloxy-3-iodo-2-tetrahydropyran-4-yl-indol-1-yl)benzoate (600 mg, 42%). ¹H NMR (400 MHz, Chloroform-d) δ 8.02 - 7.84 (m, 2H), 7.34 - 7.29 (m, 2H), 7.24 - 7.09 (m, 4H), 6.89 - 6.66 (m, 4H), 4.84 (s, 2H), 3.99 (dd, J = 11.5, 4.2 Hz, 2H), 3.27 (t, J = 11.8 Hz, 2H), 2.80 (t, J = 12.5 Hz, 1H), 2.65 - 2.41 (m, 2H), 1.58 (d, J = 1.7 Hz, 9H), 1.51 (d, J = 13.2 Hz, 2H). LCMS *m*/*z* 609.96 [M+H]⁺

### Step 4. tert-butyl 4-[7-benzyloxy-3-(4-fluorophenyl)-2-tetrahydropyran-4-yl-indol-1-yl]benzoate (C368)

A mixture of tert-butyl 4-(7-benzyloxy-3-iodo-2-tetrahydropyran-4-yl-indol-1-yl)benzoate (350.0 mg, 0.5742 mmol), potassium carbonate (400.0 mg, 2.894 mmol), (4-fluorophenyl)boronic acid (96.0 mg, 0.6861 mmol) and PdCl₂(dppf) (50.0 mg, 0.06123 mmol) in DMF (5 mL) in a Teflon sealed vial purged with nitrogen for 5 minutes was added water (0.8 mL). The mixture was heated at 90 °C for 4 hours. The solvent was evaporated and the crude mixture was purified using ISCO (40g gold column) eluting with Ethyl acetate/Heptane (0-45%, 16 CV) to afford tert-butyl 4-[7-benzyloxy-3-(4-fluorophenyl)-2-tetrahydropyran-4-yl-indol-1-yl]benzoate (215 mg, 59%) as white solid. tert-butyl 4-[7-benzyloxy-3-(4-fluorophenyl)-2-tetrahydropyran-4-yl-indol-1-yl]benzoate (215 mg, 59%). ¹H NMR (400 MHz, Chloroform-d) δ 8.09 - 7.86 (m, 2H), 7.44 (dt, J = 8.7, 2.8 Hz,2H), 7.25 - 7.16 (m, 6H), 7.06 (t, J = 7.8 Hz, 2H), 6.97 (d, J = 0.9 Hz, 1H), 6.86 - 6.79 (m, 2H), 6.74 (d, J = 1.0 Hz, 1H), 4.88 (s, 2H), 3.93 - 3.74 (m, 2H), 3.14 (td, J = 11.9, 1.9 Hz, 2H), 2.78 (tt, J = 12.2, 3.4 Hz, 1H), 1.83 - 1.74 (m, 2H), 1.71 (s, 9H), 1.54 (ddd, J = 12.9, 3.7, 1.7 Hz, 2H). LCMS *m*/*z* 577.97 [M+H]⁺

### Step 5. Synthesis of 4-[3-(4-fluorophenyl)-7-hydroxy-2-tetrahydropyran-4-yl-indol-1-yl]benzoic acid (377)

To a solution of tert-butyl 4-[7-benzyloxy-3-(4-fluorophenyl)-2-tetrahydropyran-4-yl-indol-1-yl]benzoate (215 mg, 0.3722 mmol) in dichloromethane (4 mL) at 25 °C was added 1,2,3,4,5-pentamethylbenzene (280.0 mg, 1.889 mmol) (cation scavenger) and trifluoroacetic acid (1000.0 µL, 12.98 mmol), the mixture was allowed to stir at 25 °C for 3 hours. The solvent was evaporated and the product was taken to next step without further purification. 4-[7-benzyloxy-3-(4-fluorophenyl)-2-tetrahydropyran-4-yl-indol-1-yl]benzoic acid (194.0 mg, 74%). LCMS *m*/*z* 522.01 [M+H]⁺

To a flask containing palladium on carbon (0.498 mg, 0.005 mmol) under nitrogen was added a solution of 4-[7-benzyloxy-3-(4-fluorophenyl)-2-tetrahydropyran-4-yl-indol-1-yl]benzoic acid (25 mg, 0.05 mmol) in EtOAc (1.75 mL). The mixture was purged with nitrogen and stirred at room temperature for 2.5 hours. Pd/C catalyst was added, then 1 mL of EtOH was added. The mixture was stirred under hydrogen at room temperature for 29 hours. The reaction mixture was diluted with EtOAc and filtered through a pad of Fluorosil^{®} and washed with EtOAc. The Fluorosil^{®} pad was washed with 10% EtOH/CH₂Cl₂, the filtrate was concentrated. Purification by reverse phase chromatography (Column: C18. Gradient: 0-100% MeCN in water with 0.1% trifluoroacetic acid) afforded the product. Pure fractions were concentrated *in vacuo,* diluted with EtOAc and neutralized with aqueous NaHCO₃ solution. The organic phase was dried (MgSO₄), filtered and concentrated *in vacuo* to afford 9.7 mg product as white solid. 4-[3-(4-fluorophenyl)-7-hydroxy-2-tetrahydropyran-4-yl-indol-1-yl]benzoic acid (9.7 mg, 46%). ¹H NMR (400 MHz, DMSO-*d*₆) δ 13.16 (s, 1H), 9.14 (s, 1H), 8.10 - 7.91 (m, 2H), 7.55 (d, J = 8.3 Hz, 2H), 7.47 - 7.36 (m, 2H), 7.37 - 7.19 (m, 2H), 6.81 (t, J = 7.7 Hz, 1H), 6.60 (d, J = 7.8 Hz, 1H), 6.46 (d, J = 7.5 Hz, 1H), 3.67 (d, J = 11.3 Hz, 2H), 2.99 (t, J = 9.5 Hz, 2H), 2.82 - 2.59 (m, 2H), 1.54 (d, J = 8.1 Hz, 4H). LCMS *m*/*z* 432.23 [M+H]⁺

### Compounds 378-383

Compounds **378-383** (Table 20) was prepared as described for the preparation of compound **377,** substituting the appropriate amine and boronic acid reactants.

**Table 20. Method of preparation, structure, physicochemical data for compounds 378-383**

| **Compound** | **Structure** | **Boronic acid** | **Amine** | **¹H NMR; LCMS m/z [M+H]⁺** |
|---|---|---|---|---|
| **378** | 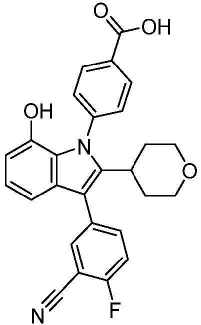 | 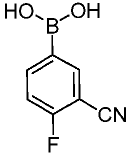 | 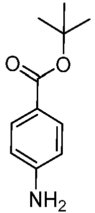 | ¹H NMR (400 MHz, Methanol-*d*₄) δ 8.23 - 8.07 (m, 2H), 7.79 - 7.67 (m, 2H), 7.53 - 7.45 (m, 2H), 7.43 - 7.35 (m, 1H), 6.92 (t, J = 7.8 Hz, 1H), 6.70 (dd, J = 7.9, 1.0 Hz, 1H), 6.57 (dd, J = 7.6, 1.0 Hz, 1H), 4.02 - 3.76 (m, 2H), 3.16 (td, J = 11.6, 2.5 Hz, 2H), 2.80 (tt, J = 11.7, 3.9 Hz, 1H), 1.73 - 1.55 (m, 4H). LCMS *m*/*z* 457 [M+H]⁺ |
| **379** | 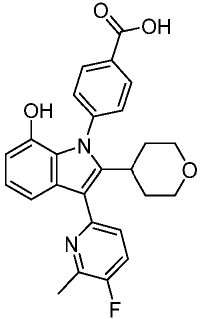 | 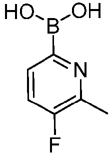 | 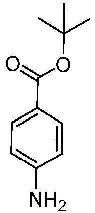 | ¹H NMR (400 MHz, Methanol-*d*₄) δ 8.19 - 8.07 (m, 2H), 7.63 (t, J = 8.8 Hz, 1H), 7.55 - 7.41 (m, 3H), 6.97 - 6.73 (m, 2H), 6.49 (dd, J = 7.5, 1.1 Hz, 1H), 3.77 (dd, J = 11.6, 4.2 Hz, 2H), 3.14 (td, J = 11.9, 1.9 Hz, 2H), 2.95 - 2.77 (m, 1H), 2.57 (d, J = 3.0 Hz, 3H), 1.89 - 1.78 (m, 2H), 1.61 (d, J = 13.0 Hz, 2H). LCMS *m*/*z* 447.14 [M+H]⁺ |
| **380¹** | 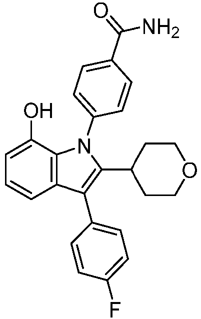 | 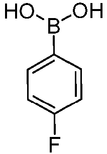 | 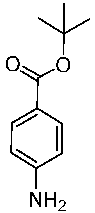 | ¹H NMR (400 MHz, DMSO-*d*₆) δ 9.08 (s, 1H), 8.13 (s, 1H), 8.02 - 7.94 (m, 2H), 7.55 - 7.48 (m, 3H), 7.47 - 7.41 (m, 2H), 7.37 - 7.27 (m, 2H), 6.80 (t, J = 7.8 Hz, 1H), 6.60 (dt, J = 7.9, 1.1 Hz, 1H), 6.45 (ddd, J = 7.6, 2.6, 1.0 Hz, 1H), 3.68 (d, J = 10.8 Hz, 2H), , 3.10 - 2.85 (m, 2H), 2.84 - 2.65 (m, 1H), 1.57 (d, J = 17.7 Hz, 4H). LCMS *m*/*z* 431.04 [M+H]⁺ |
| **381** | 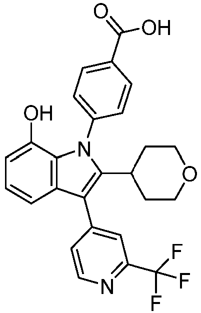 | 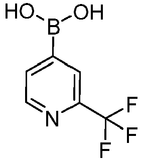 | 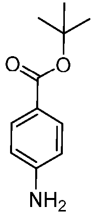 | ¹H NMR (400 MHz, Chloroform-*d*) δ 8.26 (d, J = 5.0 Hz, 1H), 7.62 (d, J = 8.2 Hz, 2H), 7.38 - 7.25 (m, 2H), 7.22 (dd, J = 5.1, 1.5 Hz, 1H), 7.00 (d, J = 8.1 Hz, 2H), 6.40 (t, J = 7.8 Hz, 1H), 6.28 (dd, J = 8.0, 1.0 Hz, 1H), 6.02 (dd, J = 7.6, 1.0 Hz, 1H), 3.27 (d, J = 4.1 Hz, 2H), 2.75 - 2.58 (m, 2H), 2.56 - 2.25 (m, 1H), 1.37 - 0.96 (m, 4H). LCMS *m*/*z* 483 [M+H]⁺ |
| **382** | 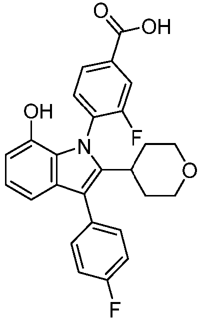 | 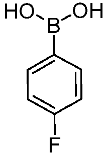 | 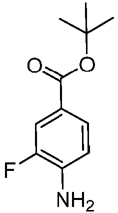 | ¹H NMR (400 MHz, Methanol-*d*₄) δ 8.05 - 7.83 (m, 2H), 7.54 (t, J = 7.8 Hz, 1H), 7.46 - 7.37 (m, 2H), 7.25 - 7.03 (m, 2H), 6.90 (t, J = 7.7 Hz, 1H), 6.78 (dd, J = 7.9, 1.0 Hz, 1H), 6.55 (dd, J = 7.6, 1.1 Hz, 1H), 3.90 - 3.69 (m, 2H), 3.27 - 3.04 (m, 2H), 2.87 -2.64 (m, 1H), 1.83 - 1.68 (m, 2H), 1.67 - 1.46 (m, 2H). LCMS *m*/*z* 450.11 [M+H]⁺ |
| **383** | 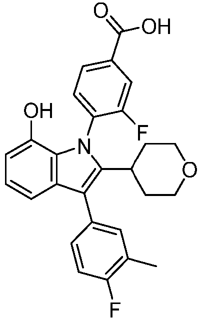 | 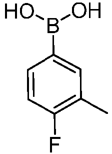 | 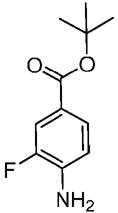 | ¹H NMR (400 MHz, Chloroform-d) δ 7.79 (ddd, J = 23.0, 9.0, 1.8 Hz, 2H), 7.40 (t, J = 7.8 Hz, 1H), 7.17 - 7.02 (m, 2H), 6.96 (dd, J = 9.6, 8.3 Hz, 1H), 6.77 (t, J = 7.8 Hz, 1H), 6.67 (dd, J = 7.9, 1.1 Hz, 1H), 6.41 (dd, J = 7.5, 1.1 Hz, 1H), 3.75 - 3.58 (m, 2H), 3.05 (tdd, J = 11.8, 4.3, 2.1 Hz, 2H), 2.62 (t, J = 12.2 Hz, 1H), 2.22 (d, J = 1.9 Hz, 3H), 1.80 - 1.60 (m, 2H), 1.47 (t, J = 11.7 Hz, 2H). LCMS *m*/*z* 464.19 [M+H]⁺ |

| | | | | |
|---|---|---|---|---|
| ^{1.} Compound **380** was prepared from compound **377** by coupling HATU to ammonia. | | | | |

### Compound 384

### (2S)-1-[3-[3-(4-fluorophenyl)-7-hydroxy-2-tetrahydropyran-4-yl-indol-1-yl]azetidin-1-yl]-2-hydroxy-propan-1-one (384)

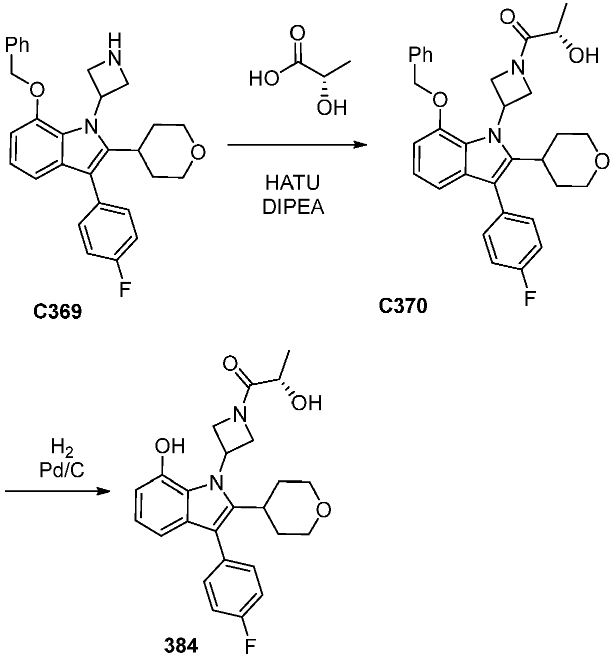

Compound **C369** was prepared as described for the preparation of compound **C377** using tert-butyl 3-aminoazetidine-1-carboxylate. Compound 384 was prepared in two steps from C369.

### Step 1. Synthesis of (2S)-1-[3-[7-benzyloxy-3-(4-fluorophenyl)-2-tetrahydropyran-4-yl-indol-1-yl]azetidin-1-yl]-2-hydroxy-propan-1-one (C370)

A solution of 1-(azetidin-3-yl)-7-benzyloxy-3-(4-fluorophenyl)-2-tetrahydropyran-4-yl-indole (Trifluoroacetate salt) (83 mg, 0.1357 mmol) and (2S)-2-hydroxypropanoic acid (23.0 mg, 0.2553 mmol) in DCM (3.0 mL) was added HATU (Phosphorus Hexafluoride Ion) (68.0 mg, 0.1788 mmol) and DIEA (68.0 µL, 0.3904 mmol). The mixture was stirred overnight, concentrated and purified using ISCO (12 g gold column) 0-60% Ethyl acetate/Heptane, 16 CV) to afford (2S)-1-[3-[7-benzyloxy-3-(4-fluorophenyl)-2-tetrahydropyran-4-yl-indol-1-yl]azetidin-1-yl]-2-hydroxy-propan-1-one (66.0 mg, 86%) LCMS *m*/*z* 529.02 [M+H]⁺

### Step 2. Synthesis of (2S)-1-[3-[3-(4-fluorophenyl)-7-hydroxy-2-tetrahydropyran-4-yl-indol-1-yl]azetidin-1-yl]-2-hydroxy-propan-1-one (384)

To a solution of (2S)-1-[3-[7-benzyloxy-3-(4-fluorophenyl)-2-tetrahydropyran-4-yl-indol-1-yl]azetidin-1-yl]-2-hydroxy-propan-1-one (64.0 mg, 0.1127 mmol) in EtOH (1 mL) and THF (0.5 mL) was added palladium (23.0 mg of 5% w/w, 0.01 mmol) and formic acid (Ammonia (1)) (60.0 mg, 0.95 mmol). The mixture was heated at 50 °C for 30 minutes. The mixture was filtered and purified using ISCO (15.5 g, formic acid modifier) to afford (2S)-1-[3-[3-(4-fluorophenyl)-7-hydroxy-2-tetrahydropyran-4-yl-indol-1-yl]azetidin-1-yl]-2-hydroxy-propan-1-one (25.3 mg, 51%). (2S)-1-[3-[3-(4-fluorophenyl)-7-hydroxy-2-tetrahydropyran-4-yl-indol-1-yl]azetidin-1-yl]-2-hydroxy-propan-1-one (25.3 mg, 51%) ¹H NMR (400 MHz, Methanol-*d*₄) δ 7.39 - 7.23 (m, 2H), 7.20 - 7.10 (m, 2H), 6.96 - 6.80 (m, 1H), 6.76 - 6.61 (m, 2H), 5.84 - 5.73 (m, 1H), 5.09 - 4.89 (m, 1H), 4.83 - 4.76 (m, 1H), 4.48 (dt, J = 21.8, 9.3 Hz, 1H), 4.40 - 4.25 (m, 3H), 4.12 - 3.82 (m, 2H), 3.59 - 3.43 (m, 2H), 3.30 - 3.13 (m, 1H), 2.03 - 1.95 (m, 1H), 1.77 - 1.62 (m, 2H), 1.48 - 1.39 (m, 3H). LCMS *m*/*z* 439.14 [M+H]^{+.}

### Compound 385

### (2R)-1-[3-[3-(4-fluorophenyl)-7-hydroxy-2-tetrahydropyran-4-yl-indol-1-yl]azetidin-1-yl]-2-hydroxy-propan-1-one (385)

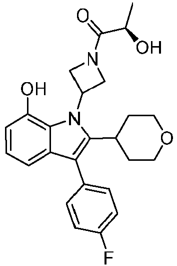

Compound **385** was prepared from **C369** and (2R)-2-hydroxypropanoic acid as described for the preparation of compound **384.** ¹H NMR (400 MHz, Methanol-*d*₄) δ 7.39 - 7.23 (m, 2H), 7.20 - 7.10 (m, 2H), 6.96 - 6.80 (m, 1H), 6.76 - 6.61 (m, 2H), 5.84 - 5.73 (m, 1H), 5.09 - 4.89 (m, 1H), 4.83 - 4.76 (m, 1H), 4.48 (dt, J = 21.8, 9.3 Hz, 1H), 4.40 - 4.25 (m, 3H), 4.12 - 3.82 (m, 2H), 3.59 - 3.43 (m, 2H), 3.30 - 3.13 (m, 1H), 2.03 - 1.95 (m, 1H), 1.77 - 1.62 (m, 2H), 1.48 - 1.39 (m, 3H). LCMS *m*/*z* 439.09 [M+H]⁺

### Compound 386

### 4-[6-fluoro-1-(4-fluoro-2-methoxy-phenyl)-4-hydroxy-2-(2-methoxy-1,1-dimethyl-ethyl)indol-3-yl]benzoic acid (386)

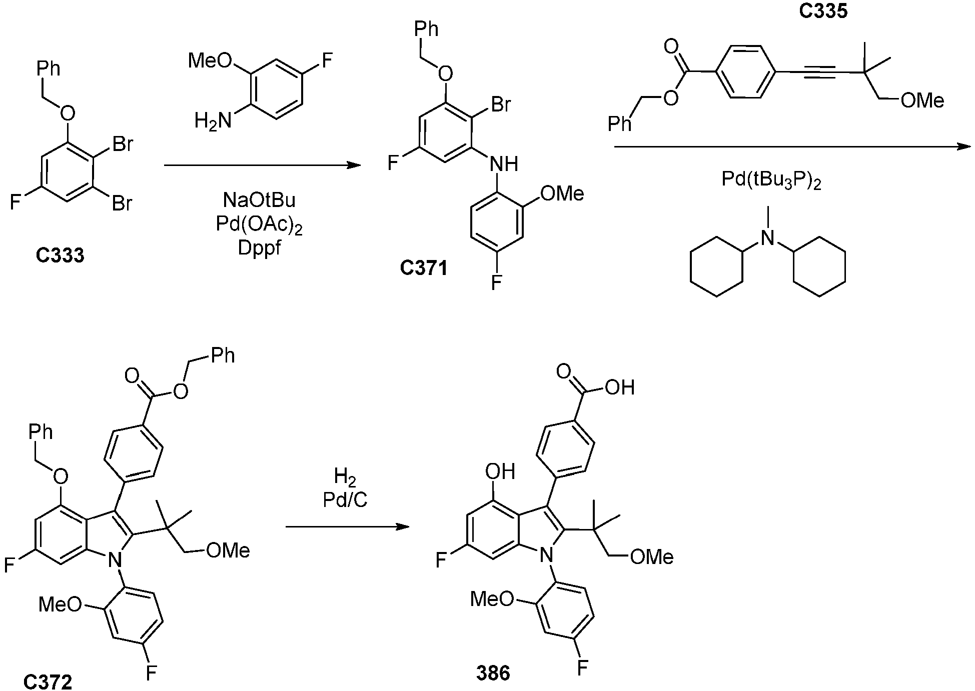

Compound **386** was prepared in three steps from **C333** using the method described for the preparation of compound **191.** ¹H NMR (400 MHz, Chloroform-d) δ 8.25 - 8.17 (m, 2H), 7.82 - 7.72 (m, 2H), 7.36 (dd, J = 9.2, 6.3 Hz, 1H), 6.88 - 6.78 (m, 2H), 6.26 (dd, J = 10.9, 2.2 Hz, 1H), 5.89 (dd, J = 9.6, 2.2 Hz, 1H), 3.81 (s, 3H), 3.11 (s, 4H), 2.99 (d, J = 9.0 Hz, 1H), 1.04 (d, J = 3.8 Hz, 7H). LCMS *m*/*z* 482.32 [M+H]⁺

### Compound 387

### 4-[6-fluoro-1-(4-fluoro-2-methoxy-phenyl)-4-hydroxy-2-(2-methoxy-1,1-dimethyl-ethyl)indol-3-yl]benzoic acid (387)

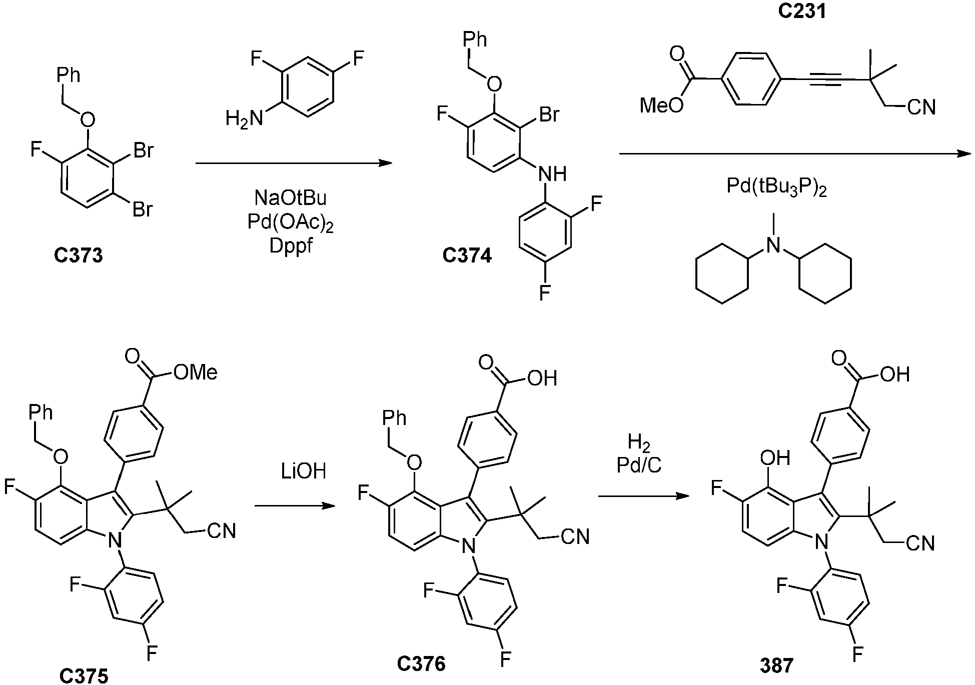

Compound **387** was prepared from 2-(benzyloxy)-3,4-dibromo-1-fluorobenzene using the method described for the preparation of compound 147.4-difluoroaniline was used in the Buchwald amination step. 4-[6-fluoro-1-(4-fluoro-2-methoxy-phenyl)-4-hydroxy-2-(2-methoxy-1,1-dimethyl-ethyl)indol-3-yl]benzoic acid. ¹H NMR (400 MHz, DMSO-*d*₆) δ 9.18 (s, 1H), 7.99 - 7.92 (m, 2H), 7.79 (td, J = 8.9, 6.0 Hz, 1H), 7.69 (ddd, J = 10.2, 8.9, 2.9 Hz, 1H), 7.58 (ddt, J = 10.5, 7.1, 1.7 Hz, 2H), 7.44 - 7.36 (m, 1H), 6.93 (dd, J = 11.2, 8.9 Hz, 1H), 6.01 (dd, J = 8.9, 3.4 Hz, 1H), 2.54 (s, 2H), 1.15 (d, J = 9.3 Hz, 6H). LCMS *m*/*z* 465 [M+H]⁺

### Compound 388

### 4-[1-(4-fluorophenyl)-4-hydroxy-2-(3-hydroxy-3-methyl-cyclobutyl)indol-3-yl]benzoic acid (388)

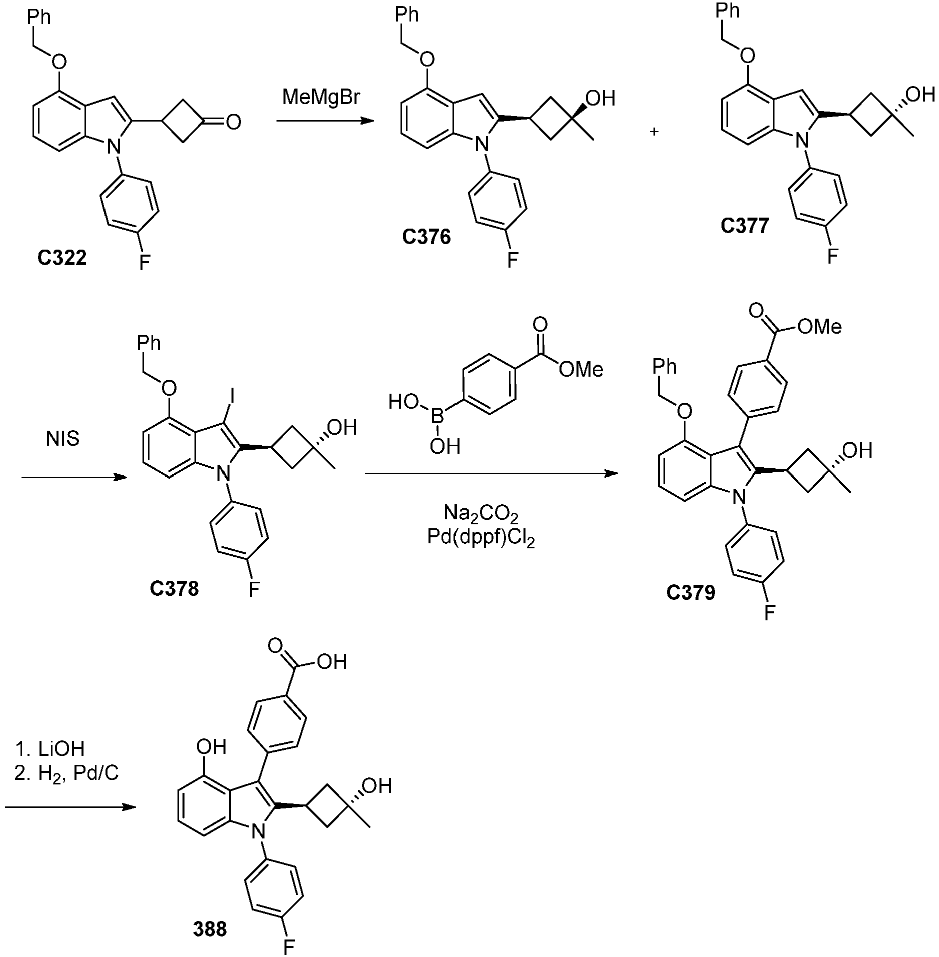

Compounds **C376** [CIS] and C377 [TRANS] were prepared from **C322** using the method described for preparation of compounds **C284** and **C285.** Compound **C377** was used in the preparation of **388** using the method described for the preparation of compound **173.** ¹H NMR (400 MHz, DMSO-*d*₆) δ 12.86 (s, 1H), 9.25 (s, 1H), 7.93 - 7.86 (m, 2H), 7.59 - 7.49 (m, 4H), 7.48 - 7.38 (m, 2H), 6.89 - 6.82 (m, 1H), 6.47 - 6.39 (m, 2H), 4.55 (s, 1H), 4.06 - 3.97 (m, 1H), 1.63 - 1.54 (m, 2H), 1.54 - 1.44 (m, 2H), 0.81 (s, 3H). LCMS *m*/*z* 432.25 [M+H]⁺

### Compound 389

### 4-[4-benzyloxy-1-(4-fluorophenyl)-2-(3-hydroxy-3-methyl-cyclobutyl)indol-3-yl]benzoic acid (389)

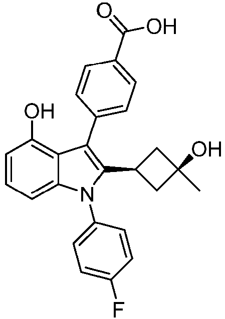

Compound **C376** was used in the preparation of **389** using the method described for the preparation of compound **173.** ¹H NMR (400 MHz, DMSO-*d*₆) δ 12.82 (s, 1H), 9.27 (s, 1H), 7.92 - 7.85 (m, 2H), 7.59 - 7.48 (m, 4H), 7.47 - 7.38 (m, 2H), 6.86 (t, J = 7.9 Hz, 1H), 6.50 - 6.40 (m, 2H), 4.59 (s, 1H), 3.42 (t, J = 8.6 Hz, 1H), 1.58 - 1.41 (m, 4H), 1.00 (s, 3H). LCMS *m*/*z* 432.25 [M+H]⁺

### Compound 390

### 4-[1-(4-fluorophenyl)-4-hydroxy-2-(4-hydroxy-4-methyl-cyclohexyl)indol-3-yl]benzoic acid (390)

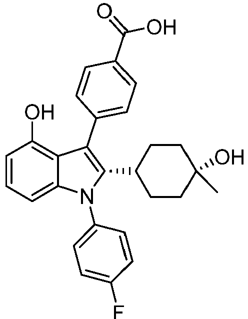

Compound **390** was prepared from **C285** as described for compound **173.** ¹H NMR (400 MHz, DMSO-*d*₆) δ 12.89 (d, J = 5.0 Hz, 1H), 9.09 (s, 1H), 7.89 (d, J = 8.2 Hz, 2H), 7.56 - 7.34 (m, 6H), 6.84 - 6.78 (m, 1H), 6.40 - 6.34 (m, 1H), 6.18 (dd, J = 8.2, 0.8 Hz, 1H), 3.63 (s, 1H), 1.72 (q, J = 11.8 Hz, 3H), 1.43 - 1.31 (m, 4H), 1.03 - 0.89 (m, 5H). LCMS *m*/*z 460.19* [M+H]⁺.

### Compounds 391-399

Compounds **391-392** were prepared from compound **98** by coupling with the appropriate amine using HATU reagent. Compounds **393-398** were prepared from Suzuki coupling between **S14** and the appropriate boronic acid or boronic ester.

**Table 21. Method of preparation, structure, physicochemical data for compounds 391-399**

| **Compound** | **Method/Product** | | **¹H NMR; LCMS m/z [M+H]⁺** |
|---|---|---|---|
| **391** | From compound **98** | | ¹H NMR (400 MHz, Chloroform-d) δ 7.95 - 7.83 (m, 2H), 7.63 - 7.56 (m, 2H), 7.39 - 7.31 (m, 2H), 7.23 (t, J = 8.4 Hz, 2H), 6.91 (td, J = 7.9, 2.8 Hz, 1H), 6.47 - 6.40 (m, 1H), 6.36 (dd, J = 8.2, 0.9 Hz, 1H), 3.81 - 3.69 (m, 2H), 3.38 (s, 3H), 3.11 (dd, J = 12.6, 10.6 Hz, 2H), 2.87 - 2.75 (m, 1H), 1.63 (qd, J = 12.4, 4.4 Hz, 2H), 1.51 (d, J = 13.0 Hz, 2H). LCMS *m*/*z* 509.36 [M+H]⁺ |
| | 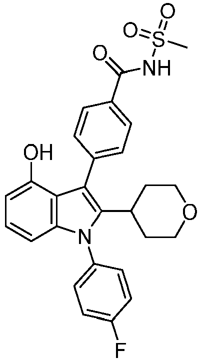 | | |
| **392** | From compound **98** | | ¹H NMR (400 MHz, DMSO-*d*₆) δ 9.42 (s, 1H), 8.50 (t, J = 1.6 Hz, 1H), 8.14 (s, 1H), 7.84 (dd, J = 11.9, 1.7 Hz, 1H), 7.69 (s, 1H), 7.57 - 7.43 (m, 4H), 6.89 (t, J = 8.0 Hz, 1H), 6.44 (dd, J = 7.7, 0.8 Hz, 1H), 6.24 (dd, J = 8.2, 0.8 Hz, 1H), 3.71 (dd, J = 11.3, 4.0 Hz, 2H), 3.13 - 3.06 (m, 2H), 2.90 - 2.79 (m, 1H), 1.69 - 1.44 (m, 4H). LCMS *m*/*z* 450 [M+H]⁺ |
| | 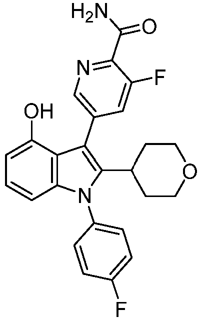 | | |
| ***393*** | From S14 | | ¹H NMR (400 MHz, DMSO-*d*₆) δ 9.34 (s, 1H), 8.61 (s, 2H), 7.57 - 7.41 (m, 4H), 6.86 (dd, J = 8.2, 7.7 Hz, 1H), 6.40 (dd, J = 7.7, 0.8 Hz, 1H), 6.24 (dd, J = 8.2, 0.8 Hz, 1H), 3.99 (s, 3H), 3.71 (dd, J = 11.2, 4.0 Hz, 2H), 3.11 - 3.02 (m, 2H), 2.79 (tt, J = 12.2, 3.5 Hz, 1H), 1.61 (d, J = 12.6 Hz, 2H), 1.51 (qd, J = 12.3, 4.2 Hz, 2H). LCMS *m*/*z 420* [M+H]⁺ |
| | 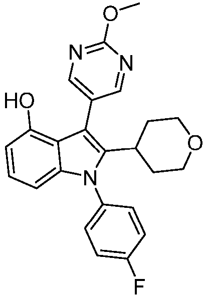 | | |
| ***394*** | From S14 | 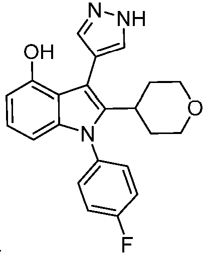 | LCMS *m*/*z* 378 [M+H]⁺ |
| **395** | From S14 | 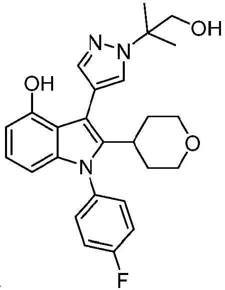 | ¹H NMR (400 MHz, DMSO-*d*₆) δ 9.15 (s, 1H), 7.75 (d, J = 0.7 Hz, 1H), 7.50 - 7.42 (m, 5H), 6.83 - 6.75 (m, 1H), 6.38 (dd, J = 7.6, 0.8 Hz, 1H), 6.14 (dd, J = 8.2, 0.8 Hz, 1H), 5.01 (t, J = 5.5 Hz, 1H), 3.72 (dd, J = 10.7, 3.7 Hz, 2H), 3.63 (d, J = 4.7 Hz, 2H), 3.16 - 3.05 (m, 2H), 2.95 (td, J = 11.6, 3.9 Hz, 1H), 1.52 (s, 10H). LCMS *m*/*z* 450 [M+H]⁺ |
| **396** | From S14 | 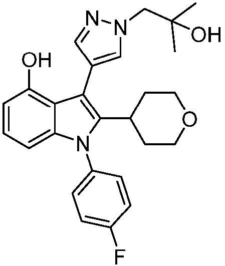 | ¹H NMR (400 MHz, DMSO-*d*₆) δ 9.10 (s, 1H), 7.68 (d, J = 0.8 Hz, 1H), 7.51 - 7.40 (m, 5H), 6.82 - 6.75 (m, 1H), 6.37 (dd, J = 7.6, 0.8 Hz, 1H), 6.16 (dd, J = 8.2, 0.8 Hz, 1H), 4.71 (s, 1H), 4.08 (s, 2H), 3.75 - 3.66 (m, 2H), 3.07 (dd, J = 12.6, 10.4 Hz, 2H), 2.95 - 2.84 (m, 1H), 1.69 - 1.48 (m, 4H), 1.11 (s, 6H). LCMS *m*/*z* 450 [M+H]⁺ |
| **397** | From S14 | 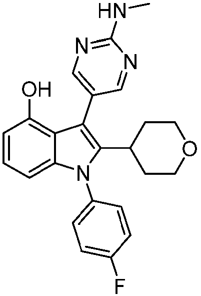 | ¹H NMR (400 MHz, DMSO-*d*₆) δ 8.27 (s, 2H), 7.53 - 7.40 (m, 5H), 7.04 (q, J = 4.8 Hz, 1H), 6.82 (td, J = 7.9, 1.3 Hz, 1H), 6.39 (dd, J = 7.7, 0.8 Hz, 1H), 6.24 - 6.18 (m, 1H), 3.76 - 3.66 (m, 2H), 3.11 - 3.01 (m, 2H), 2.86 (d, J = 4.9 Hz, 3H), 2.79 - 2.71 (m, 1H), 1.58 (td, J = 10.9, 9.3, 3.5 Hz, 4H). LCMS *m*/*z* 419 [M+H]⁺ |
| **398** | From S14 | | ¹H NMR (400 MHz, DMSO-*d*₆) δ 9.53 (s, 1H), 8.80 (t, J = 1.5 Hz, 1H), 8.01 - 7.94 (m, 2H), 7.56 (ddt, J = 8.2, 5.3, 2.7 Hz, 2H), 7.52 - 7.44 (m, 2H), 6.87 (t, J = 7.9 Hz, 1H), 6.41 (d, J = 7.6 Hz, 1H), 6.22 (d, J = 8.1 Hz, 1H), 3.69 (dd, J = 11.2, 4.0 Hz, 2H), 3.09 - 2.99 (m, 2H), 2.88 - 2.76 (m, 1H), 1.73 (d, J = 13.5 Hz, 6H), 1.61 (d, J = 10.8 Hz, 2H), 1.50 (qd, J = 12.2, 4.2 Hz, 2H). LCMS *m*/*z* 465 [M+H]⁺ |
| | 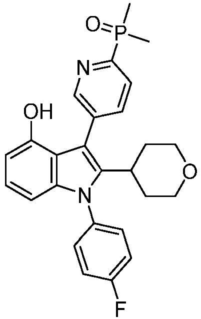 | | |
| **399** | Larock method¹ | | ¹H NMR (400 MHz, DMSO-*d*₆) δ 12.83 (s, 1H), 9.16 (s, 1H), 7.94 (d, J = 7.9 Hz, 2H), 7.56 - 7.45 (m, 6H), 6.84 (t, J = 7.9 Hz, 1H), 6.39 (d, J = 7.6 Hz, 1H), 6.23 (d, J = 8.2 Hz, 1H), 3.47 - 3.37 (m, 1H), 3.31 - 3.20 (m, 1H), 3.04 (t, J = 12.7 Hz, 1H), 1.61 (d, J = 13.2 Hz, 1H), 1.44 - 1.26 (m, 3H), 0.95 (s, 3H), 0.84 (s, 3H). LCMS *m*/*z 460* [M+H]⁺ |
| | 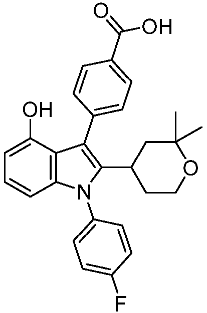 | | |

| | | | |
|---|---|---|---|
| ^{1.} Larock indole cyclization between methyl 4-[2-(4-hydroxy-2,2-dimethyl-tetrahydropyran-4-yl)ethynyl]benzoate. | | | |

### Compound 400-417

Compounds **400-417** were prepared by Larock indole cyclization.

**Table 22. Method of preparation, structure, physicochemical data for compounds 400-417**

| **Compound** | **Structure** | **¹H NMR; LCMS m/z [M+H]⁺** |
|---|---|---|
| **400** | 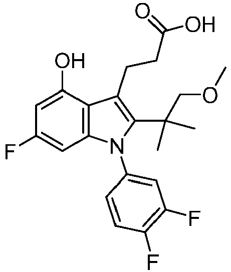 | ¹H NMR (400 MHz, DMSO-*d*₆) δ 12.10 (s, 1H), 10.15 (s, 1H), 7.66 - 7.48 (m, 2H), 7.21 (ddt, J = 8.2, 3.8, 1.8 Hz, 1H), 6.21 (dd, J = 11.4, 2.2 Hz, 1H), 5.70 (dd, J = 9.9, 2.2 Hz, 1H), 3.28 - 3.18 (m, 4H), 3.15 (s, 3H), 2.62 - 2.53 (m, 2H), 1.20 (d, J = 3.5 Hz, 6H). LCMS *m*/*z* 422 [M+H]⁺ |
| **401** | 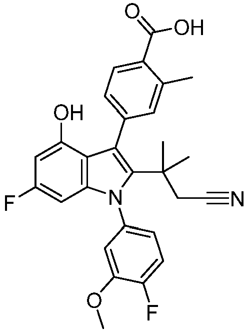 | ¹H NMR (300 MHz, DMSO-*d*₆) δ 12.79 (s, 1H), 9.54 (s, 1H), 7.82 (d, J = 8.1 Hz, 1H), 7.47 (dd, J = 11.3, 8.5 Hz, 1H), 7.33 (td, J = 7.8, 2.6 Hz, 3H), 7.14 - 7.05 (m, 1H), 6.14 (dd, J = 11.4, 2.2 Hz, 1H), 5.83 (dd, J = 9.7, 2.2 Hz, 1H), 3.88 (s, 3H), 2.56 (s, 3H), 2.50 (s, 2H), 1.15 (s, 6H). LCMS *m*/*z* 491 [M+H]⁺ |
| **402** | 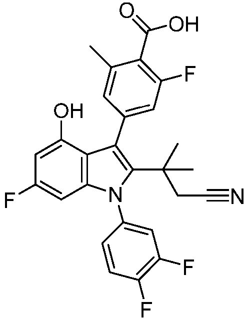 | ¹H NMR (400 MHz, Methanol-*d*₄) δ 7.60 - 7.46 (m, 2H), 7.38 - 7.28 (m, 1H), 7.19 (d, J = 2.9 Hz, 1H), 7.09 (dt, J = 10.4, 2.3 Hz, 1H), 6.15 (dd, J = 11.2, 2.2 Hz, 1H), 5.83 - 5.75 (m, 1H), 2.49 (t, J = 1.8 Hz, 2H), 2.45 (s, 3H), 1.25 (dd, J = 3.0, 1.9 Hz, 6H). LCMS *m*/*z* 497.26 [M+H]⁺ |
| **403** | 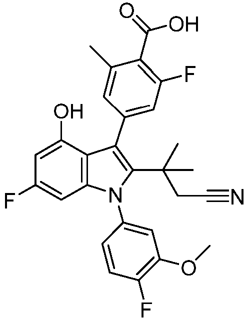 | ¹H NMR (400 MHz, Methanol-*d*₄) δ 7.35 (dd, J = 10.9, 8.5 Hz, 1H), 7.21 (d, J = 7.4 Hz, 2H), 7.08 (ddd, J = 12.0, 6.8, 3.1 Hz, 2H), 6.13 (dd, J = 11.2, 2.2 Hz, 1H), 5.81 (dd, J = 9.6, 2.1 Hz, 1H), 3.90 (s, 3H), 2.49 (dd, J = 9.2, 2.2 Hz, 2H), 2.45 (s, 3H), 2.01 (s, 1H), 1.29 - 1.22 (m, 7H). LCMS *m*/*z* 509.23 [M+H]⁺ |
| **404** | 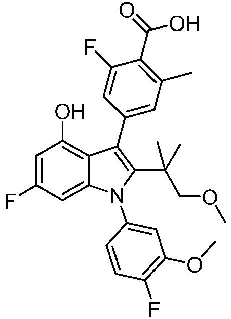 | ¹H NMR (400 MHz, Methanol-*d*₄) δ 7.30 (dd, J = 11.1, 8.5 Hz, 1H), 7.20 - 7.11 (m, 2H), 7.07 - 6.97 (m, 2H), 6.10 (dd, J = 11.2, 2.2 Hz, 1H), 5.77 (dd, J = 9.7, 2.2 Hz, 1H), 3.89 (s, 3H), 3.13 (d, J = 0.6 Hz, 3H), 3.04 (d, J = 2.2 Hz, 2H), 2.45 (d, J = 0.8 Hz, 3H), 1.09 (s, 6H). LCMS *m*/*z* 514.17 [M+H]⁺ |
| **405** | 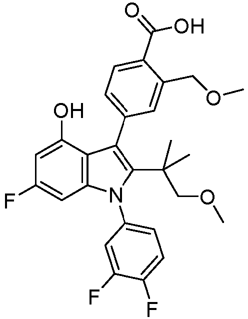 | ¹H NMR (400 MHz, DMSO-*d*₆) δ 9.55 (s, 1H), 7.78 (d, J = 7.9 Hz, 1H), 7.76 - 7.60 (m, 2H), 7.54 (t, J = 2.2 Hz, 1H), 7.41 - 7.35 (m, 1H), 7.35 - 7.25 (m, 1H), 6.14 (dd, J = 11.4, 2.2 Hz, 1H), 5.79 (ddd, J = 9.8, 2.2, 1.1 Hz, 1H), 4.90 - 4.76 (m, 2H), 3.35 (s, 3H), 3.01 (s, 3H), 2.97 - 2.88 (m, 2H), 0.97 (s, 6H). LCMS *m*/*z* 514 [M+H]⁺ |
| **406** | 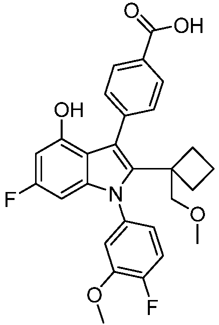 | ¹H NMR (400 MHz, Chloroform-*d*/CD3OD) δ 7.92 (dq, J = 8.4, 2.0 Hz, 2H), 7.64 - 7.54 (m, 2H), 7.17 - 7.07 (m, 1H), 7.04 (ddt, J = 7.7, 4.6, 2.2 Hz, 1H), 6.90 (ddt, J = 8.5, 4.0, 2.3 Hz, 1H), 6.14 (dt, J = 11.1, 2.2 Hz, 1H), 5.93 (dt, J = 9.6, 2.2 Hz, 1H), 3.78 (d, J = 2.1 Hz, 3H), 3.55 (q, J = 2.0 Hz, 2H), 3.28 (d, J = 2.1 Hz, 3H), 1.99 - 1.85 (m, 2H), 1.64 (td, J = 10.6, 8.3 Hz, 1H), 1.47 - 1.26 (m, 3H). LCMS *m*/*z* 494.15 [M+H]⁺ |
| **407** | 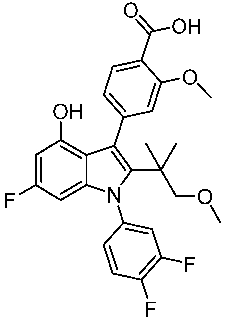 | ¹H NMR (400 MHz, Methanol-*d*₄) δ 7.83 (d, J = 7.9 Hz, 1H), 7.56 - 7.37 (m, 2H), 7.30 (dddd, J = 9.9, 5.8, 3.8, 1.6 Hz, 1H), 7.20 (d, J = 1.4 Hz, 1H), 7.10 (dd, J = 7.9, 1.4 Hz, 1H), 6.11 (dd, J = 11.2, 2.1 Hz, 1H), 5.77 (ddd, J = 9.6, 2.1, 0.7 Hz, 1H), 3.93 (d, J = 1.1 Hz, 3H), 3.12 (s, 3H), 3.06 - 3.00 (m, 2H), 1.08 (d, J = 2.2 Hz, 6H). LCMS *m*/*z* 500.13 [M+H]⁺ |
| **408** | 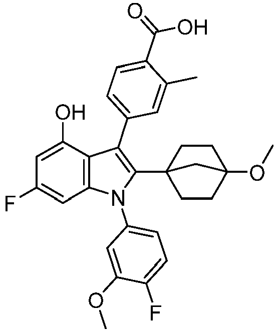 | ¹H NMR (400 MHz, DMSO-*d*₆) δ 12.75 (s, 1H), 9.48 (d, J = 0.7 Hz, 1H), 7.84 - 7.77 (m, 1H), 7.42 (dd, J = 11.3, 8.5 Hz, 1H), 7.33 - 7.23 (m, 3H), 7.04 (dddd, J = 8.5, 6.1, 3.9, 2.4 Hz, 1H), 6.15 (dd, J = 11.4, 2.2 Hz, 1H), 5.90 (dt, J = 9.7, 2.1 Hz, 1H), 3.86 (d, J = 1.2 Hz, 3H), 2.94 (s, 3H), 2.56 (s, 3H), 1.85 (d, J = 13.1 Hz, 2H), 1.40 (dd, J = 27.3, 12.4 Hz, 6H), 1.19 - 1.10 (m, 2H). LCMS *m*/*z* 534 [M+H]⁺ |
| **409** | 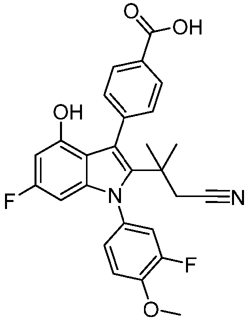 | ¹H NMR (400 MHz, DMSO-*d*₆) δ 12.93 (s, 1H), 9.55 (s, 1H), 7.97 - 7.86 (m, 2H), 7.56 - 7.47 (m, 3H), 7.40 (t, J = 9.1 Hz, 1H), 7.32 (ddd, J = 8.7, 2.5, 1.2 Hz, 1H), 6.14 (dd, J = 11.4, 2.2 Hz, 1H), 5.79 (dd, J = 9.7, 2.2 Hz, 1H), 3.96 (s, 3H), 3.35 (s, 2H), 1.13 (d, J = 8.3 Hz, 6H). LCMS *m*/*z* 477.15 [M+H]⁺ |
| **410** | 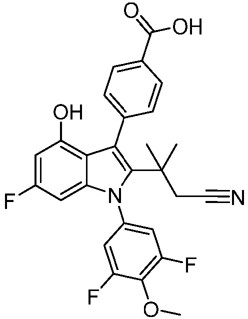 | ¹H NMR (400 MHz, DMSO-*d*₆) δ 12.94 (s, 1H), 9.59 (s, 1H), 7.98 - 7.88 (m, 2H), 7.56 - 7.49 (m, 2H), 7.47 (d, J = 8.6 Hz, 2H), 6.16 (dd, J = 11.4, 2.2 Hz, 1H), 5.95 (dd, J = 9.7, 2.2 Hz, 1H), 4.07 (d, J = 1.1 Hz, 3H), 2.53 (s, 2H), 1.15 (s, 6H). LCMS *m*/*z* 495.12 [M+H]⁺ |
| **411** | 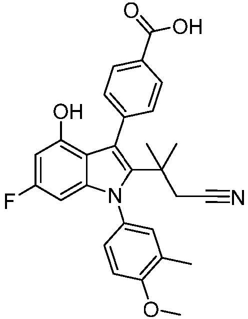 | ¹H NMR (400 MHz, DMSO-*d*₆) δ 12.91 (s, 1H), 9.51 (s, 1H), 7.96 - 7.85 (m, 2H), 7.56 - 7.49 (m, 2H), 7.35 - 7.23 (m, 2H), 7.16 (d, J = 8.6 Hz, 1H), 6.12 (dd, J = 11.4, 2.2 Hz, 1H), 5.72 (dd, J = 9.8, 2.2 Hz, 1H), 3.91 (s, 3H), 2.49 (s, 2H), 2.24 (s, 3H), 1.12 (d, J = 4.0 Hz, 6H). LCMS *m*/*z* 473.17 [M+H]⁺ |
| **412** | 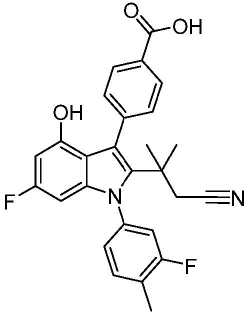 | ¹H NMR (400 MHz, DMSO-*d*₆) δ 12.93 (s, 1H), 9.58 (s, 1H), 7.98 - 7.87 (m, 2H), 7.60 - 7.49 (m, 3H), 7.43 (dd, J = 10.2, 2.1 Hz, 1H), 7.27 (dd, J = 8.0, 2.1 Hz, 1H), 6.15 (dd, J = 11.4, 2.2 Hz, 1H), 5.77 (dd, J = 9.7, 2.2 Hz, 1H), 3.33 (s, 3H), 2.38 (d, J = 1.9 Hz, 2H), 1.13 (d, J = 6.1 Hz, 6H). LCMS *m*/*z* 461.16 [M+H]⁺ |
| **413** | 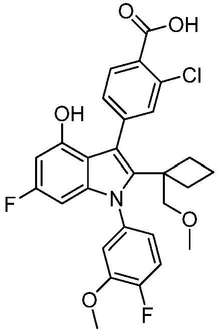 | ¹H NMR (400 MHz, Chloroform-d) δ 7.95 (d, J = 8.0 Hz, 1H), 7.84 (d, J = 1.6 Hz, 1H), 7.56 (dd, J = 8.0, 1.7 Hz, 1H), 7.12 (dd, J = 10.8, 8.5 Hz, 1H), 7.05 - 6.97 (m, 1H), 6.92 (tdd, J = 8.5, 3.9, 2.4 Hz, 1H), 6.25 (dd, J = 10.8, 2.2 Hz, 1H), 6.02 (td, J = 9.9, 9.4, 2.1 Hz, 1H), 3.82 (d, J = 1.8 Hz, 4H), 3.59 (t, J = 2.1 Hz, 2H), 3.36 (s, 3H), 1.98 (s, 2H), 1.77 - 1.68 (m, 1H), 1.55 - 1.42 (m, 2H). LCMS *m*/*z* 528.17 [M+H]⁺ |
| **414** | 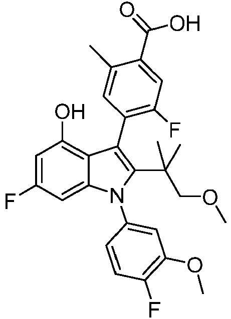 | ¹H NMR (400 MHz, DMSO-*d*₆) δ 9.53 (d, J = 5.0 Hz, 1H), 7.55 (dd, J = 9.9, 3.2 Hz, 1H), 7.43 (ddd, J = 11.2, 8.6, 7.0 Hz, 1H), 7.39 - 7.16 (m, 2H), 7.15 - 6.93 (m, 1H), 6.11 (dt, J = 11.4, 1.9 Hz, 1H), 5.80 (ddd, J = 9.8, 5.2, 2.2 Hz, 1H), 3.87 (d, J = 7.6 Hz, 3H), 3.07 - 2.92 (m, 5H), 2.52 (s, 3H), 1.03 (dd, J = 3.2, 1.8 Hz, 6H). LCMS *m*/*z* 514.17 [M+H]⁺ |
| **415** | 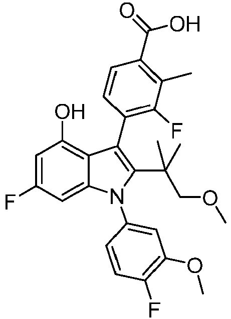 | ¹H NMR (400 MHz, DMSO-*d*₆) δ 9.50 (d, J = 4.0 Hz, 1H), 7.61 (dd, J = 8.0, 3.5 Hz, 1H), 7.43 (dt, J = 11.3, 8.4 Hz, 1H), 7.37 - 7.17 (m, 2H), 7.05 (dddd, J = 49.2, 8.6, 4.0, 2.4 Hz, 1H), 6.10 (dd, J = 11.4, 2.3 Hz, 1H), 5.80 (ddd, J = 9.8, 8.6, 2.2 Hz, 1H), 3.87 (d, J = 10.5 Hz, 3H), 3.07 - 2.88 (m, 5H), 2.46 (d, J = 2.3 Hz, 3H), 1.02 (d, J = 2.9 Hz, 6H). LCMS *m*/*z 514.17* [M+H]⁺ |
| **416** | 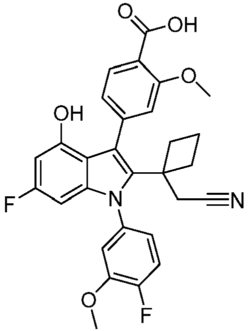 | ¹H NMR (400 MHz, Chloroform-d) δ 8.29 (d, J = 7.9 Hz, 1H), 7.62 (d, J = 10.9 Hz, 1H), 7.50 - 7.38 (m, 1H), 7.34 - 7.22 (m, 1H), 7.02 (dd, J = 7.3, 2.0 Hz, 2H), 6.38 (dd, J = 10.8, 2.2 Hz, 1H), 6.07 (dd, J = 9.2, 2.1 Hz, 1H), 4.15 (s, 3H), 3.93 (d, J = 8.1 Hz, 3H), 2.98 - 2.80 (m, 2H), 2.41 (q, J = 10.0, 8.6 Hz, 1H), 2.22 (q, J = 10.5 Hz, 1H), 1.90 (h, J = 10.1 Hz, 1H), 1.73 (tdd, J = 11.6, 8.3, 4.7 Hz, 1H), 1.67 - 1.44 (m, 2H). LCMS *m*/*z* 519.17 [M+H]⁺ |
| **417** | 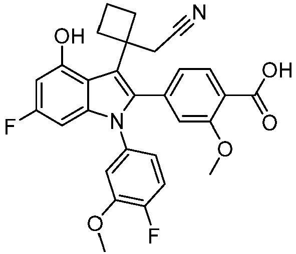 | ¹H NMR (400 MHz, Chloroform-d) δ 7.95 (d, J = 7.8 Hz, 1H), 7.53 - 7.36 (m, 1H), 7.36 - 7.18 (m, 2H), 7.11 (td, J = 7.4, 2.5 Hz, 1H), 6.94 (tdd, J = 8.3, 3.9, 2.5 Hz, 1H), 6.37 (dd, J = 10.9, 2.1 Hz, 1H), 6.07 (dd, J = 9.3, 2.2 Hz, 1H), 5.87 (s, 1H), 5.09 (s, 1H), 3.97 (d, J = 1.1 Hz, 3H), 3.96 (s, 3H), 2.96 - 2.77 (m, 2H), 2.38 (dt, J = 23.0, 10.3 Hz, 1H), 2.26 (q, J = 9.5 Hz, 1H), 1.96 - 1.79 (m, 1H), 1.69 (d, J = 10.0 Hz, 2H), 1.61 - 1.49 (m, 1H). LCMS *m*/*z* 519.17 [M+H]⁺ |

### Compound 418

### 4-(1-(4,4-difluorocyclohexyl)-4-hydroxy-2-(tetrahydro-2H-pyran-4-yl)-lH-indol-3-yl)benzoic acid (418)

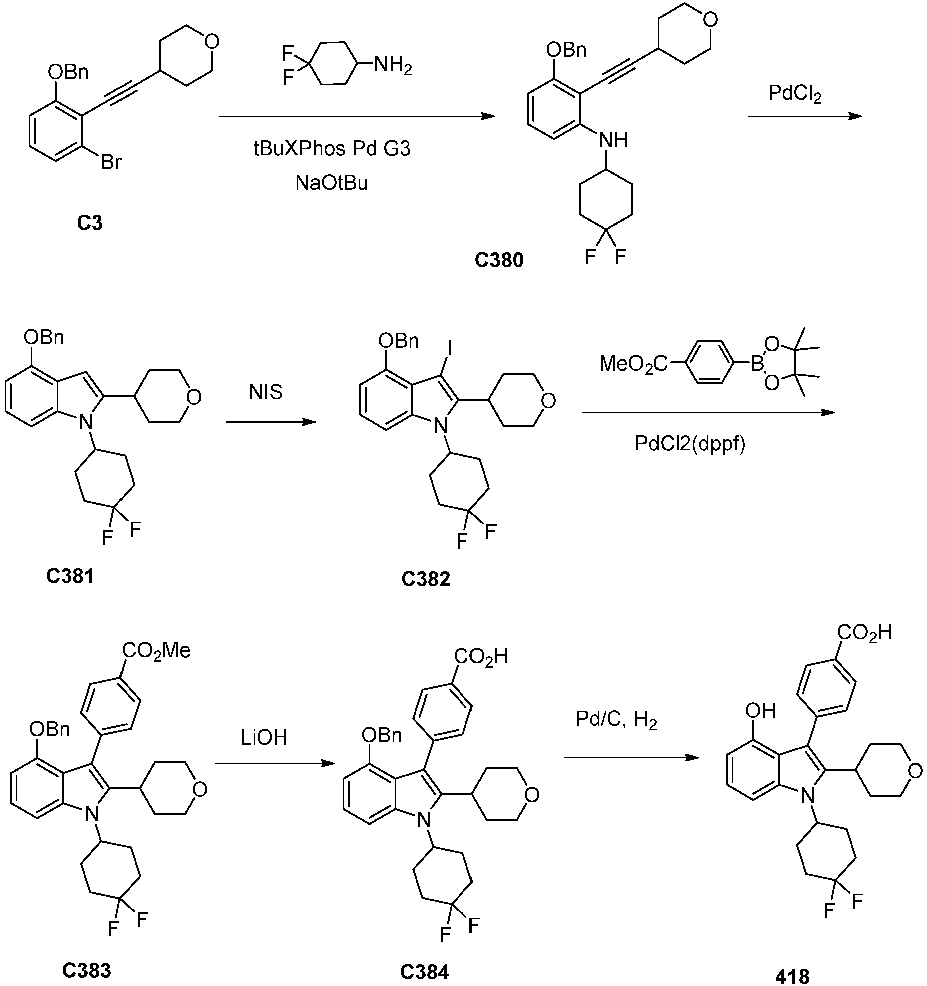

### Step 1: Synthesis of 3-(benzyloxy)-N-(4, 4-difluorocyclohexyl)-2-((tetrahydro-2H-pyran-4-yl)ethynyl)aniline (C380)

To a solution of 4-[2-(2-benzyloxy-6-bromo-phenyl) ethynyl] tetrahydropyran (1.00 g, 2.52 mmol) and 4,4-difluorocyclohexanamine (0.40 g, 2.96 mmol) in m-xylene (10 mL) at 60 °C was added NaOtBu (0.73 g, 7.58 mmol) in one portion followed by [2-(2-aminophenyl)phenyl]palladium di-tert-butyl-[2-(2,4,6-triisopropylphenyl)phenyl]phosphane methanesulfonate (PdtBuXPhos G3, 0.06 g, 0.08 mmol). The reaction was stirred for 20 minutes and diluted with water (10 mL). The solution was extracted three times with EtOAc. The organic phase was dried (MgSO₄), filtered, and concentrated *in vacuo.* The resulting residue was purified by silica gel chromatography (40 g ISCO column) using 0-40% EtOAc/heptanes gradient to afford 490 mg of product. Pure fractions were combined and concentrated to give acyclic product as a white solid (46%). 3-Benzyloxy-N-(4,4-difluorocyclohexyl)-2-(2-1 tetrahydropyran-4-ylethynyl)aniline. H NMR (400 MHz, Chloroform-d) δ 7.47 (ddt, J = 7.5, 1.3, 0.7 Hz, 2H), 7.40 - 7.33 (m, 2H), 7.33 - 7.28 (m, 1H), 7.08 (t, *J* = 8.3 Hz, 1H), 6.28 (td, *J =* 8.5, 0.8 Hz, 2H), 5.11 (s, 2H), 4.57 (d, *J* = 8.0 Hz, 1H), 3.93 (ddd, *J* = 11.6, 6.1, 3.5 Hz, 2H), 3.60 - 3.45 (m, 3H), 2.99 (tt, *J* = 8.2*,* 4.1 Hz, 1H), 2.21 - 2.04 (m, 4H), 2.00 - 1.59 (m, 6H).

### Step 2: Synthesis of 4-(benzyloxy)-1-(4,4-difluorocyclohexyl)-2-(tetrahydro-2H-pyran-4-yl)-1H-indole (C381)

3-benzyloxy-*N*-(4,4-difluorocyclohexyl)-2-(2-tetrahydropyran-4-ylethynyl)aniline **C380** (490mg, 1.152 mmol) was dissolved in MeCN (10 mL) at 60 °C and then PdCl₂ (0.045 g, 0.254 mmol) was added. The mixture was concentrated to dryness. The resulting residue was purified by silica gel chromatography using 0-50% EtOAc/heptanes gradient to afford 190 mg of product. 4-(benzyloxy)-1-(4,4-difluorocyclohexyl)-2-(tetrahydro-2*H*-pyran-4-yl)-1*H*-indole (18%). ¹H NMR (300 MHz, Chloroform*-d*) δ 7.54 - 7.47 (m, 2H), 7.45 - 7.30 (m, 3H), 7.16 - 7.00 (m, 2H), 6.56 (d, *J* = 7.6 Hz, 1H), 6.46 (s, 1H), 5.20 (s, 2H), 4.23 (t, *J =* 13.0 Hz, 1H), 4.10 (dd, *J =* 11.2, 3.6 Hz, 2H), 3.59 (td, *J =* 11.4, 3.0 Hz, 2H), 2.97 - 2.68 (m, 3H), 2.32 (s, 2H), 2.09 - 1.78 (m, 6H). ESI-MS m/z calc. 425.2, found 426.0 (M+1)⁺.

### Step 3: Synthesis of 4-(benzyloxy)-1-(4,4-difluorocyclohexyl)-3-iodo-2-(tetrahydro-2H-pyran-4-yl)-1H-indole (C382)

To a solution of 4-(benzyloxy)-1-(4,4-difluorocyclohexyl)-2-(tetrahydro-2*H*-pyran-4-yl)-1*H*-indole **C381** (0.19 g, 0.45 mmol) in CH₂Cl₂ (5 mL) was added N-iodosuccinimide (0.11 g, 0.47 mmol). The reaction mixture was stirred at room temperature for 5 minutes. The reaction mixture was concentrated to dryness. The resulting residue was purified by silica gel chromatography using 0-50% EtOAc/heptanes gradient to afford 240 mg of product. 4-benzyloxy-1-(4,4-difluorocyclohexyl)-3-iodo-2-tetrahydropyran-4-yl-indole (98%). ¹H NMR (400 MHz, Chloroform-*d*) δ 7.61 (ddt, *J* = 7.7, 1.4, 0.7 Hz, 2H), 7.43 - 7.37 (m, 2H), 7.36 - 7.29 (m, 1H), 7.12 (d, J = 8.5 Hz, 1H), 7.05 (t, *J =* 8.1 Hz, 1H), 6.58 (dd, *J* = 7.8, 3.1 Hz, 1H), 5.21 (s, 2H), 4.64 (t, J = 12.9 Hz, 1H), 4.15 (dd, *J=* 11.5, 4.4 Hz, 2H), 3.72 (s, 1H), 3.67 - 3.56 (m, 2H), 2.80 (q, *J* = 14.6, 13.3 Hz, 2H), 2.32 (s, 2H), 1.98 (dd, J = 50.3, 10.2 Hz, 6H), 1.75 (d, *J* = 13.0 Hz, 2H). ESI-MS m/z calc. 551.1133, found 551.0 (M+1)⁺.

### Step 4: Synthesis of methyl 4-(4-(benzyloxy)-1-(4,4-difluorocyclohexyl)-2-(tetrahydro-2H-pyran-4-yl)-1H-indol-3-yl)benzoate (C383)

A mixture of 4-benzyloxy-1-(4,4-difluorocyclohexyl)-3-iodo-2-tetrahydropyran-4-yl-indole **C382** (0.24 g, 0.44 mmol), methyl 4-(4,4,5,5-tetramethyl-1,3,2-dioxaborolan-2-yl)benzoate (0.13 g, 0.48 mmol), and PdCl₂(dppf) (0.04 g, 0.04 mmol) in DMF (6 mL) and aqueous saturated NaHCO₃ solution (2 mL) was irradiated in a microwave at 90 °C for 20 minutes. The mixture was then diluted into water and extracted with EtOAc. The organic phase was dried (MgSO₄), filtered, and concentrated *in vacuo.* The resulting residue was purified by silica gel chromatography (40 g ISCO column) using 0-60% EtOAc/heptanes gradient to afford 106 mg of product. Methyl 4-[4-benzyloxy-1-(4,4-difluorocyclohexyl)-2-tetrahydropyran-4-yl-indol-3-yl]benzoate (44%). ¹H NMR (400 MHz, Chloroform-*d*) δ 7.96 - 7.90 (m, 2H), 7.44 - 7.38 (m, 2H), 7.24 - 7.10 (m, 5H), 6.81 (d, *J* = 7.3 Hz, 2H), 6.58 (dd, *J =* 7.7, 3.5 Hz, 1H), 4.92 (s, 2H), 4.61 (d, *J* = 13.0 Hz, 1H), 4.04 (dd, *J =* 11.6, 4.2 Hz, 2H), 3.99 (s, 3H), 3.37 (dd, *J* = 12.6, 10.7 Hz, 2H), 3.12 (t, *J* = 12.8 Hz, 1H), 2.93 (d, *J* = 13.4 Hz, 2H), 2.38 (s, 2H), 2.03 (d, *J* = 12.1 Hz, 6H), 1.69 (d, *J* = 13.2 Hz, 2H). ESI-MS m/z calc. 559.2, found 560.0 (M+1)⁺.

### Step 5: Synthesis of 4-(4-(benzyloxy)-1-(4,4-difluorocyclohexyl)-2-(tetrahydro-2H-pyran-4-yl)-1H-indol-3-yl)benzoic acid (C384)

To a solution of methyl 4-[4-benzyloxy-1-(4,4-difluorocyclohexyl)-2-tetrahydropyran-4-yl-indol-3-yl]benzoate **C383** (0.106 g, 0.189 mmol) in 2-methyl THF (6 mL), MeOH (2 mL), and water (2 mL) was added LiOH (0.050 g, 2.088 mmol). The reaction mixture was stirred at room temperature for 1 hour. The reaction was heated to 65 °C, stirred for another 1 hour and went to completion. The solution was acidified with 1M HCl and extracted with EtOAc. The organic layer was dried over magnesium sulfate, filtered, and concentrated to dryness. Crude product was carried forward to the next step. 4-[4-benzyloxy-1-(4,4-difluorocyclohexyl)-2-tetrahydropyran-4-yl-indol-3-yl]benzoic acid (90 mg, 87%). ESI-MS m/z calc. 545.2, found 546.0 (M+1)⁺.

### Step 6: Synthesis of 4-(1-(4,4-difluorocyclohexyl)-4-hydroxy-2-(tetrahydro-2H-pyran-4-yl)-1H-indol-3-yl)benzoic acid (418)

To a slurry of Pd on C (0.050 g, 0.047 mmol) in EtOH (5 mL) was added a solution of 4-[4-benzyloxy-1-(4,4-difluorocyclohexyl)-2-tetrahydropyran-4-yl-indol-3-yl]benzoic acid **C384** (0.090 g, 0.165 mmol) in 2-methyl THF (5 mL). The mixture was stirred at room temperature for 20 minutes, filtered through a bed of Celite^{®}, and the resulting filtrate concentrated to dryness. The resulting residue was purified by silica gel chromatography using 0-10% MeOH/CH₂Cl₂ gradient to afford product. Pure fractions were combined, concentrated, triturated in heptane, filtered, and dried to give 35 mg of product. 4-[1-(4,4-difluorocyclohexyl)-4-hydroxy-2-tetrahydropyran-4-yl-indol-3-yl]benzoic acid (44%). ¹H NMR (400 MHz, DMSO-*d6)* δ 12.84 (s, 1H), 9.01 (s, 1H), 7.92 - 7.87 (m, 2H), 7.44 - 7.38 (m, 2H), 6.94 - 6.82 (m, 2H), 6.35 - 6.26 (m, 1H), 4.63 (s, 1H), 3.91 - 3.79 (m, 2H), 3.29 (d, *J* = 11.6 Hz, 2H), 3.14 (t, *J* = 12.5 Hz, 1H), 2.66 (d, *J=* 10.8 Hz, 2H), 2.25 (d, *J=* 45.8 Hz, 4H), 1.86 (dd, *J* = 30.4, 12.9 Hz, 4H), 1.68 - 1.53 (m, 2H). ESI-MS m/z calc. 455.2, found 456.0 (M+1).

### Compound 419

### 4-(5-fluoro-1-(2-fluorobenzyl)-4-hydroxy-2-(1-methoxy-2-methylpropan-2-yl)-1H-indol-3-yl)benzoic acid (419)

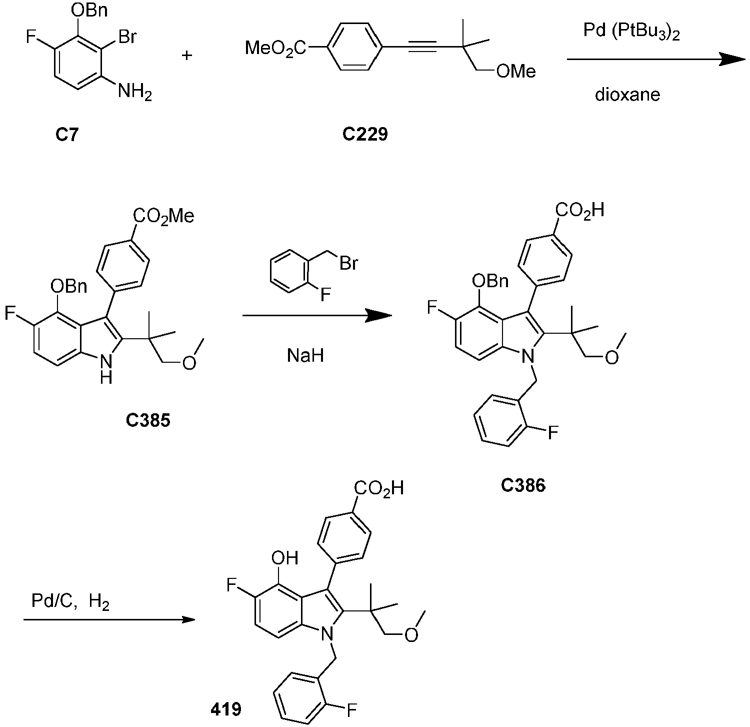

### Step 1: Synthesis of methyl 4-(4-(benzyloxy)-5-fluoro-2-(1-methoxy-2-methylpropan-2-yl)-1H-indol-3-yl)benzoate (C385)

Combined 3-benzyloxy-2-bromo-4-fluoro-aniline **C7** (0.50 g, 1.53 mmol), methyl 4-(4-methoxy-3,3-dimethyl-but-1-ynyl)benzoate **C229** (0.50 g, 2.03 mmol) and *N*-cyclohexyl-*N-*methyl-cyclohexanamine (1.80 mL, 8.40 mmol) in a 20 ml vial with a stir bar. The vial was vacuumed and flushed with nitrogen three times. Dioxane (5.4 mL) was added and the mixture was bubbled with nitrogen for 5 minutes followed by the addition of Pd(P*t*Bu₃)₂ (0.05 g, 0.09 mmol). The vial was sealed and heated to 110 °C overnight. The reaction was incomplete. Temperature was raised to 120 °C and heated for another 5 hours. The reaction was diluted into EtOAc (3 mL) and HCl (2N, 1 mL). The aqueous layer was separated and extracted twice with EtOAc. Combined organic phases was washed with brine, dried (MgSO₄), filtered and concentrated to dryness. The resulting residue was purified by silica gel chromatography (24g ISCO column) using 0-40% EtOAc/heptanes gradient to afford 403 mg of product. Methyl 4-[4-benzyloxy-5-fluoro-2-(2-methoxy-1,1-dimethyl-ethyl)-1*H*-indol-3-yl]benzoate (56%). ¹H NMR (400 MHz, Chloroform*-d*) δ 9.39 (s, 1H), 7.98 - 7.90 (m, 2H), 7.53 - 7.47 (m, 2H), 7.25 - 7.13 (m, 3H), 7.05 (dd, *J* = 8.7, 3.7 Hz, 1H), 6.98 (dd, *J =* 11.6, 8.7 Hz, 1H), 6.88 - 6.75 (m, 2H), 4.69 (d, *J =* 0.8 Hz, 2H), 3.99 (s, 3H), 3.48 (s, 3H), 3.43 (s, 2H), 1.19 (s, 6H). ESI-MS m/z calc. 461.20, found 462.32 (M+1)⁺.

### Step 2: Synthesis of 4-(4-(benzyloxy)-5-fluoro-1-(2-fluorobenzyl)-2-(1-methoxy-2-methylpropan-2-yl)-1H-indol-3-yl)benzoic acid (C386)

To a solution of methyl 4-[4-benzyloxy-5-fluoro-2-(2-methoxy-1,1-dimethyl-ethyl)-1H-indol-3-yl]benzoate **C385** (0.055 g, 0.119 mmol) in THF (0.60 mL) was added NaH (0.006 g of 60% w/w, 0.150 mmol). The reaction mixture was stirred at room temperature for 30 minutes. 1-(bromomethyl)-2-fluoro-benzene (0.020 mL, 0.166 mmol) was added and the mixture was stirred at room temperature overnight. NaOtBu (0.120 mL of 1 M solution, 0.120 mmol) was added and the reaction mixture was heated to 60 °C for 2 hours. The reaction was cooled to room temperature and quenched by addition of HCl (1N, 1.0 mL). The crude residue was purified by reverse phase flash chromatography (RF ISCO, C18 column, 30g) eluting with CH₃CN /water (0-100%, 0.1% formic acid) to afford 32 mg of product. 4-[4-benzyloxy-5-fluoro-1-[(2-fluorophenyl)methyl]-2-(2-methoxy-1,1-dimethyl-ethyl)indol-3-yl]benzoic acid (46%). ESI-MS m/z calc. 555.2, found 556.3 (M+1)⁺.

### Step 3: Synthesis of 4-(5-fluoro-1-(2-fluorobenzyl)-4-hydroxy-2-(1-methoxy-2-methylpropan-2-yl)-1H-indol-3-yl)benzoic acid (419)

To a solution of 4-[4-benzyloxy-5-fluoro-1-[(2-fluorophenyl)methyl]-2-(2-methoxy-1,1-dimethyl-ethyl)indol-3-yl]benzoic acid **C386** (0.035 g, 0.060mmol) in THF (1 mL) and EtOH (1 mL) was added Pd on carbon (0.006 g of 10% w/w, 0.006 mmol). The mixture was purged with hydrogen for 3 minutes and then stirred an atmosphere of hydrogen for 1 hour. The crude mixture was filtered through a pad of Celite^{®} and the filtrate was concentrated *in vacuo.* The crude residue was purified by reverse phase flash chromatography (RF ISCO, C18 column, 30g) eluting with CH₃CN /water (0-100%, 0.1% formic acid) to afford 28 mg of product. 4-[5-fluoro-1-[(2-fluorophenyl)methyl]-4-hydroxy-2-(2-methoxy-1,1-dimethyl-ethyl)indol-3-yl]benzoic acid (100%). ¹H NMR (400 MHz, Chloroform-d) δ 8.12 - 8.04 (m, 2H), 7.61 - 7.53 (m, 2H), 7.21 - 7.11 (m, 1H), 7.04 (ddd, *J=* 10.3, 8.2, 1.2 Hz, 1H), 6.91 (td, *J* = 7.6, 1.2 Hz, 1H), 6.79 (dd, *J=* 10.8, 8.9 Hz, 1H), 6.50 - 6.37 (m, 2H), 5.58 (s, 2H), 3.21 (s, 2H), 2.93 (s, 3H), 1.15 (s, 6H). ESI-MS m/z calc. 465.2, found 466.4 (M+1)⁺.

### Compound 420

### 4-(1-(4,4-difluorocyclohexyl)-5-fluoro-4-hydroxy-2-(1-hydroxy-2-methylpropan-2-yl)-1H-indol-3-yl)benzoic acid (420)

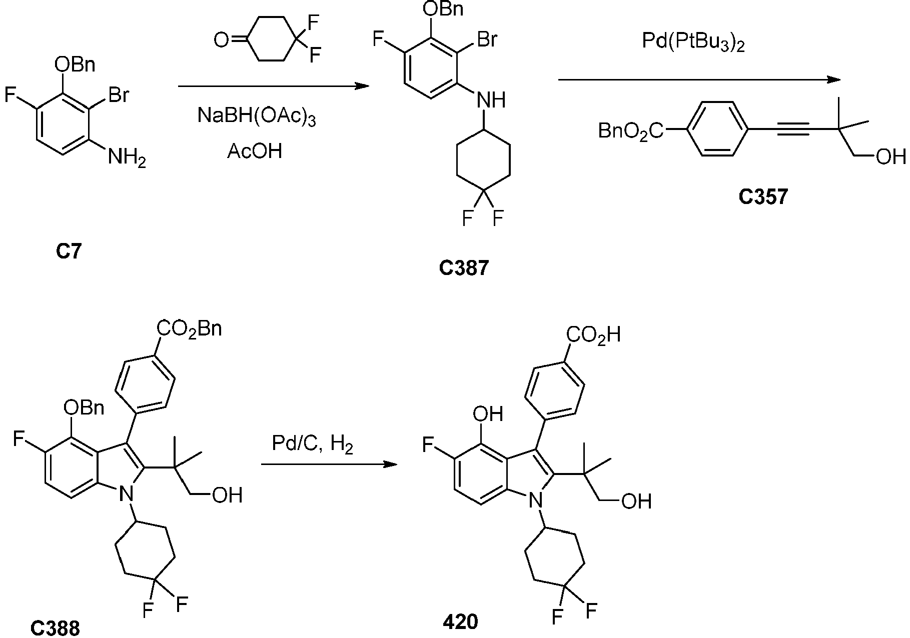

### Step 1: Synthesis of 3-benzyloxy-2-bromo-N-(4,4-difluorocyclohexyl)-4-fluoro-aniline (C387)

To a solution of 4,4-difluorocyclohexanone (0.34 g, 2.54 mmol) and 3-benzyloxy-2-bromo-4-fluoro-aniline C7 (0.50 g, 1.69 mmol) in 1,2-dichloroethane (10 mL) was added AcOH (0.20 mL, 3.52 mmol), followed by NaBH(OAc)₃ (1.10 g, 5.19 mmol). The reaction mixture was stirred at room temperature for 2 days and slowly quenched with aqueous saturated NaHCO₃ solution. The layers were separated. The aqueous layer was extracted three times with CH₂Cl₂. The combined organic phases were adsorbed onto silica, and purified by silica gel flash chromatography (CombiFlash ^{®}, 40 g column, 0-40% EtOAc in hexane) to afford 675 of product. 3-benzyloxy-2-bromo-*N*-(4,4-difluorocyclohexyl)-4-fluoro-aniline (89%). ¹H NMR (400 MHz, Chloroform-*d*) δ 7.60 - 7.53 (m, 2H), 7.46 - 7.33 (m, 3H), 7.00 (dd, *J* = 10.6, 9.1 Hz, 1H), 6.40 - 6.31 (m, 1H), 5.14 (d, J = 2.4 Hz, 2H), 4.15 (d, J = 7.8 Hz, 1H), 3.44 (d, *J* = 9.5 Hz, 1H), 2.27 - 2.04 (m, 4H), 2.02 - 1.78 (m, 2H), 1.77 - 1.62 (m, 2H). ESI-MS m/z calc. 413.06, found 414.13 (M+1)⁺.

### Step 2: benzyl 4-[4-benzyloxy-1-(4,4-difluorocyclohexyl)-5-fluoro-2-(2-hydroxy-1,1-dimethylethyl)indol-3-yl]benzoate (C388)

Combined 3-benzyloxy-2-bromo-*N*-(4,4-difluorocyclohexyl)-4-fluoro-aniline **C387** (0.150 g, 0.362 mmol), benzyl 4-(4-hydroxy-3,3-dimethyl-but-1-ynyl)benzoate **C357** (0.195 g, 0.632 mmol) and *N*-cyclohexyl-*N*-methyl-cyclohexanamine (0.400 mL, 1.867 mmol) in a 20 ml vial with a stir bar. The vial was vacuumed and flushed with nitrogen. 1,4-dioxane (2 mL). was added. The solution was bubbled with nitrogen for 3 minutes. Pd(PtBu₃) (0.013 g, 0.025 mmol) was added and the vial was sealed and heated to 110 °C for 3 days. The mixture was concentrated *in vacuo.* The resulting residue was purified by silica gel chromatography (12 g ISCO column) using 0-60% EtOAc/heptanes gradient to afford 113 mg of product. Benzyl 4-[4-benzyloxy-1-(4,4-difluorocyclohexyl)-5-fluoro-2-(2-hydroxy-1,1-dimethyl-ethyl)indol-3-yl]benzoate (33%). ¹H NMR (400 MHz, Chloroform-*d*) δ 7.92 - 7.84 (m, 2H), 7.56 - 7.49 (m, 2H), 7.49 - 7.37 (m, 5H), 7.20 (dd, *J* = 9.1, 3.4 Hz, 1H), 7.12 - 7.03 (m, 3H), 7.03 - 6.93 (m, 1H), 6.90 - 6.77 (m, 2H), 5.39 (s, 2H), 4.77 (t, *J* = 12.6 Hz, 1H), 4.61 (d, *J* = 1.0 Hz, 2H), 3.61 (d, *J* = 5.6 Hz, 2H), 2.98 - 2.80 (m, 2H), 2.38 (s, 2H), 2.10 - 1.84 (m, 4H), 1.32 (s, 6H). ESI-MS m/z calc. 641.3, found 642.5 (M+1)⁺.

### Step 4: Synthesis of 4-[]-(4,4-difluorocyclohexyl)-5-fluoro-4-hydroxy-2-(2-hydroxy-1,1-dimethyl-ethyl)indol-3-yl]benzoic acid (420)

Benzyl 4-[4-benzyloxy-1-(4,4-difluorocyclohexyl)-5-fluoro-2-(2-hydroxy-1,1-dimethyl-ethyl)indol-3-yl]benzoate (0.045 g, 0.069 mmol) in THF (1 mL) and EtOH (1 mL) was added Pd/C (0.007 g of 10% w/w, 0.006 mmol). The mixture was flushed with hydrogen (balloon) for 3 minutes. The reaction mixture was stirred under a hydrogen atmosphere for 12 hours. The crude mixture was filtered through a pad of Celite^{®} and the filtrate was concentrated *in vacuo* to afford 30 mg of product. 4-[1-(4,4-Difluorocyclohexyl)-5-fluoro-4-hydroxy-2-(2-hydroxy-1,1-dimethyl-ethyl)indol-3-yl]benzoic acid (88%). ¹H NMR (400 MHz, Chloroform-d) δ 7.96 - 7.79 (m, 2H), 7.45 - 7.32 (m, 2H), 6.88 - 6.69 (m, 2H), 4.72 - 4.57 (m, 1H), 3.55 - 3.46 (m, 2H), 2.72 (t, J = 13.2 Hz, 2H), 2.26 - 2.10 (m, 2H), 1.98 - 1.71 (m, 4H), 1.17 - 1.11 (m, 6H). ESI-MS m/z calc. 461.18, found 462.23 (M+1)⁺.

### Compound 421

### 4-[2-(2-cyano-1, 1-dimethyl-ethyl)-1-(4, 4-difluorocyclohexyl)-5-fluoro-4-hydroxy-indol-3-yl]benzoic acid (421)

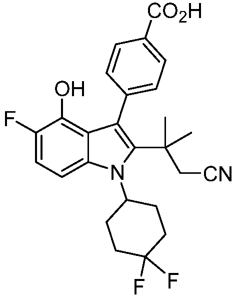

Compound **421** was prepared in same fashion as **420** using methyl 4-(4-cyano-3,3-dimethyl-but-1-ynyl)benzoate **C231** as described in the synthesis of **C388.** Lithium hydroxide induced hydrolysis of the methyl ester followed by removal of the benzyl protecting group with Pd/C under hydrogen atmosphere afforded the product. ¹H NMR (400 MHz, Chloroform-*d*) δ 7.97 - 7.87 (m, 2H), 7.47 - 7.35 (m, 2H), 6.84 (dd, *J =* 6.9, 3.5 Hz, 2H), 4.45 (s, 1H), 2.82 (d, *J* = 14.1 Hz, 2H), 2.56 (d, *J* = 4.6 Hz, 2H), 2.26 (s, 2H), 1.99 - 1.74 (m, 4H), 1.49 - 1.33 (m, 6H). ESI-MS m/z calc. 470.18, found 471.27 (M+1)⁺.

### Compound 422

### 4-[1-(4,4-difluorocyclohexyl)-5-fluoro-4-hydroxy-2-(2-methoxy-1,1-dimethyl-ethyl)indol-3-yl]benzoic acid (422)

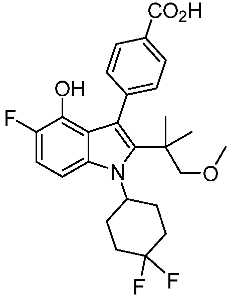

Compound **422** was prepared in same fashion as **420** using benzyl 4-(4-hydroxy-3,3-dimethyl-but-1-ynyl)benzoate **C222** as described in the synthesis of **C388.** Lithium hydroxide induced hydrolysis of the methyl ester followed by removal of the benzyl protecting group with Pd/C under hydrogen atmosphere afforded the product. ¹H NMR (400 MHz, Chloroform-*d*) δ 8.09 - 8.00 (m, 2H), 7.55 - 7.46 (m, 2H), 6.95 - 6.80 (m, 2H), 4.78 (t, *J =* 12.8 Hz, 1H), 4.49 (s, 1H), 3.28 (s, 2H), 3.11 (s, 3H), 2.86 - 2.64 (m, 2H), 2.27 (s, 3H), 2.02 - 1.72 (m, 4H), 1.24 (s, 6H). ESI-MS m/z calc. 475.2, found 476.3 (M+1)⁺.

### Compound 423

### 4-[6-fluoro-4-hydroxy-2-(1-hydroxy-2-methylpropan-2-yl)-1-(2,2,2-trifluoroethyl)indol-3-yl]benzoic acid (423)

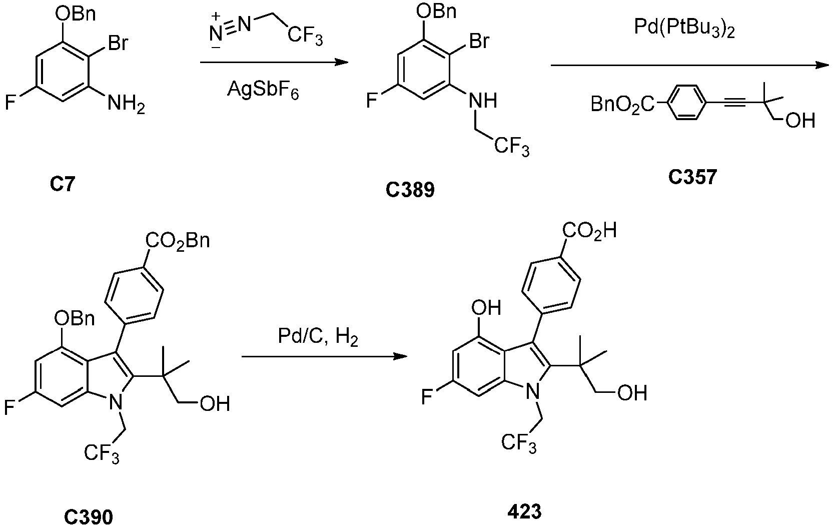

### Step 1: Synthesis of 3-benzyloxy-2-bromo-5-fluoro-N-(2,2,2-trifluoroethyl)aniline (C389)

To a solution of 3-benzyloxy-2-bromo-5-fluoro-aniline **C7** (0.50 g, 1.69 mmol) and silver (I) hexafluoroantimony (0.03 g, 0.09 mmol) in 1,2-dichloroethane (15 mL) was added 2-diazo-1,1,1-trifluoro-ethane (3.00 mL of 0.85 M in toluene, 2.55 mmol). The reaction mixture was heated to 50 °C for 2 hours. Solvent was removed under reduced pressure. The resulting residue was purified by silica gel chromatography (24 g ISCO column) using 0-40% EtOAc/heptanes gradient to afford 611 mg of product. 3-benzyloxy-2-bromo-5-fluoro-*N*-(2,2,2-trifluoroethyl)aniline (64%). ¹H NMR (400 MHz, Chloroform-*d*) δ 7.62 - 7.52 (m, 2H), 7.46 - 7.36 (m, 3H), 7.04 (dd, *J* = 10.5, 9.1 Hz, 1H), 6.46 (dd, *J* = 9.1, 4.1 Hz, 1H), 5.16 (d, J = 0.8 Hz, 2H), 4.62 (t, *J* = 7.0 Hz, 1H), 3.82 (qd, *J =* 8.8, 6.9 Hz, 2H). ESI-MS m/z calc. 377.0, found 378.0 (M+1)⁺.

### Step 2: Synthesis of benzyl 4-[4-benzyloxy-6-fluoro-2-(1-hydroxy-2-methylpropan-2-yl)-1-(2,2,2-trifluoroethyl)indol-3-yl]benzoate (C390)

Combined 3-benzyloxy-2-bromo-5-fluoro-*N*-(2,2,2-trifluoroethyl)aniline **C389** (0.24 g, 0.63 mmol), benzyl 4-(4-hydroxy-3,3-dimethyl-but-1-ynyl)benzoate **C357** (0.30 g, 0.972mmol) and *N*-cyclohexyl-*N*-methyl-cyclohexanamine (0.60 mL, 2.80 mmol) in a 20ml vial with a stir bar. The vial was vacuumed and flushed with nitrogen. 1,4-dioxane (2 mL) was added. The solution was purged with nitrogen for 3 minutes and Pd(P*t*Bu₃)₂ (0.02 g, 0.04 mmol) was added. The reaction vial was sealed and heated to 120 °C for 3 days. Solvent was removed to near dryness and the residue was diluted into EtOAc (10 mL) and HCl (2mL). The organic layer was separated and concentrated *in vacuo.* The resulting residue was purified by silica gel chromatography (12 g ISCO column) using 0-40% EtOAc/heptanes gradient to afford 110 mg of product. Benzyl 4-[4-benzyloxy-6-fluoro-2-(1-hydroxy-2-methylpropan-2-yl)-1-(2,2,2-trifluoroethyl)indol-3-yl]benzoate (28%). ¹H NMR (400 MHz, Chloroform-*d*) δ 7.94 - 7.84 (m, 2H), 7.59 - 7.50 (m, 2H), 7.50 - 7.37 (m, 5H), 7.13 - 7.03 (m, 5H), 6.92 - 6.74 (m, 2H), 5.41 (s, 2H), 5.13 (q, *J =* 8.2 Hz, 2H), 4.65 (d, *J =* 1.0 Hz, 2H), 3.59 (s, 2H), 1.30 (s, 6H). ESI-MS m/z calc. 605.22, found 606.31 (M+1)⁺.

### Step 3: Synthesis of 4-[6-fluoro-4-hydroxy-2-(1-hydroxy-2-methylpropan-2-yl)-1-(2,2,2-trifluoroethyl)indol-3-yl]benzoic acid (423)

To a solution of benzyl 4-[4-benzyloxy-6-fluoro-2-(1-hydroxy-2-methylpropan-2-yl)-1-(2,2,2-trifluoroethyl)indol-3-yl]benzoate **C390** (0.045 g, 0.074 mmol) in THF (1 mL) and EtOH (1 mL) was added Pd/C (0.007 g of 10% w/w, 0.007 mmol). The reaction mixture was stirred under an atmosphere of hydrogen for 5 hours. The mixture was filtered through a microfilter cartridge and the solvent was removed under reduced pressure to afford 31 mg of product. 4-[6-fluoro-4-hydroxy-2-(1-hydroxy-2-methylpropan-2-yl)-1-(2,2,2-trifluoroethyl)indol-3-yl]benzoic acid (96%). ¹H NMR (400 MHz, Chloroform-*d*) δ 8.02 - 7.92 (m, 2H), 7.43 (dt, *J =* 7.5, 3.6 Hz, 2H), 7.27 (d, *J =* 11.6 Hz, 1H), 6.90 (ddt, *J* = 11.0, 5.2, 3.0 Hz, 1H), 6.72 (d, *J = 9.1* Hz, 1H), 5.04 (d, *J =* 12.2 Hz, 2H), 3.50 (q, *J* = 2.9 Hz, 3H), 1.20 (q, *J* = 2.5, 2.1 Hz, 6H); ESI-MS m/z calc. 425.13, found 426.14 (M+1)⁺.

### Compound 424

### 4-[6-fluoro-4-hydroxy-2-(1-methoxy-2-methylpropan-2-yl)-1-(2,2,2-trifluoroethyl)indol-3-yl]benzoic acid (424)

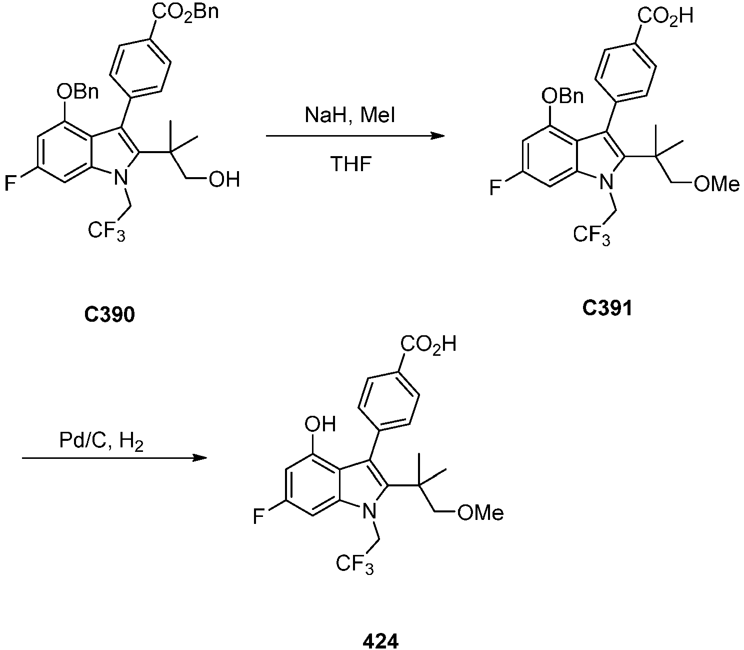

### Step 1: Synthesis of 4-[4-benzyloxy-5-fluoro-2-(1-methoxy-2methylpropan-2yl)-1-(2,2,2-trifluoroethyl)indol-3-yl]benzoic acid (C391)

To a solution of benzyl 4-[4-benzyloxy-6-fluoro-2-(1-hydroxy-2-methylpropan-2-yl)-1-(2,2,2-trifluoroethyl)indol-3-yl]benzoate **C390** (0.075 g, 0.123 mmol) in THF (1.5 mL) was added MeI (0.025 mL, 0.402 mmol) followed by NaH (0.015 g of 60% w/w, 0.375 mmol). The reaction was heated at 50 °C overnight. The reaction mixture was quenched with a few drops of HCl and concentrated to dryness. Purification by reverse phase MPLC (10-90% ACN in Water and 0.2% FA as modifier) afforded 40 mg of product. 4-[4-benzyloxy-6-fluoro-2-(1-methoxy-2-methylpropan-2-yl)-1-(2,2,2-trifluoroethyl)indol-3-yl]benzoic acid (61%);. ESI-MS m/z calc. 529.19, found 530.26 (M+1)⁺.

### Step 2: 4-[6-fluoro-4-hydroxy-2-(1-methoxy-2-methylpropan-2-yl)-1-(2,2,2-trifluoroethyl)indol-3-yl]benzoic acid (424)

To a solution of 4-[4-benzyloxy-6-fluoro-2-(1-methoxy-2-methylpropan-2-yl)-1-(2,2,2-trifluoroethyl)indol-3-yl]benzoic acid **C391** (0.038 g, 0.071 mmol) in THF (1.5 mL) and EtOH (1.5 mL) was added Pd/C (0.010 g of 10% w/w, 0.009 mmol). The mixture was flushed with hydrogen (balloon) for 3 minutes and the reaction was then stirred under an atmosphere of hydrogen for 5 hours. After filtering through a microfilter cartridge, the solvent was removed under reduced pressure. Purification by reverse phase MPLC (10-90% ACN in water with 0.2% FA as modifier) afforded 30 mg of product (92%). ¹H NMR (400 MHz, Chloroform-*d*) δ 8.06 - 7.90 (m, 2H), 7.44 (d, *J* = 7.8 Hz, 2H), 6.90 (ddd, *J* = 10.6, 9.0, 1.1 Hz, 1H), 6.73 (dd, *J* = 9.1*,* 3.1 Hz, 1H), 5.04 (q, *J* = 8.3 Hz, 2H), 3.24 (s, 2H), 3.14 (d, *J =* 1.1 Hz, 3H), 1.20 (s, 6H); ESI-MS m/z calc. 439.14, found 440.22 (M+1)⁺.

### Compound 425

### 4-[5-fluoro-1-(4-fluorophenyl)-4-hydroxy-2-isopropyl-1H-pyrrolo[2,3-c]pyridin-3-yl]benzoic acid (425)

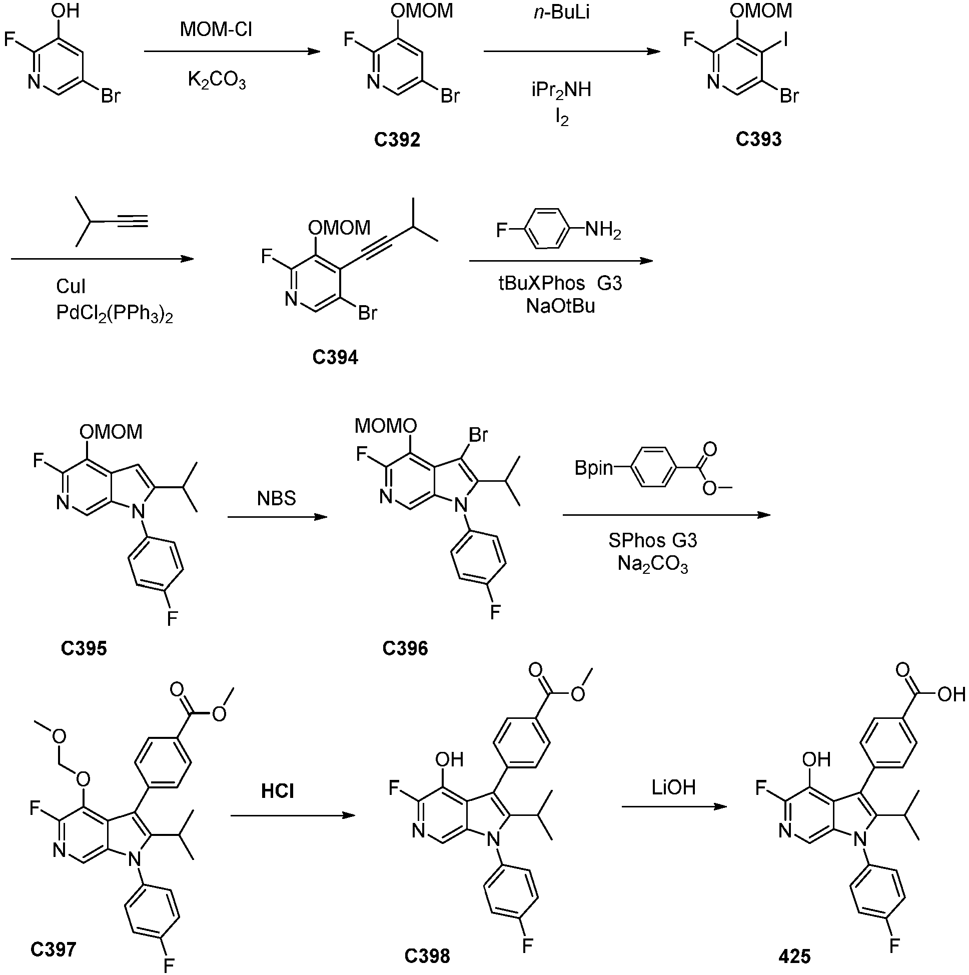

### Step 1. Synthesis of 5-bromo-2-fluoro-3-(methoxymethoxy)pyridine (C392)

A solution of 5-bromo-2-fluoro-pyridin-3-ol (3.10 g, 16.15 mmol) in acetone (20 mL) was treated with K₂CO₃ (2.68 g, 19.39 mmol) and chloro(methoxy)methane (1.50 mL, 19.75 mmol). The mixture was heated at 60 °C for 2 hrs. After cooling to room temperature, the reaction mixture was filtered to remove inorganic salts. The filtrate was concentrated under reduced pressure. The residue was purified by flash column chromatography (0- 10% EtOAc/hexanes) to afford 1.4 g of product. 5-bromo-2-fluoro-3-(methoxymethoxy)pyridine (35%). ESI-MS m/z calc. 234.96, found 235.88 (M+1)⁺.

### Step 2. Synthesis of 5-bromo-2-fluoro-4-iodo-3-(methoxymethoxy)pyridine (C393)

To a cold (-78 °C) solution of diisopropylamine (0.39 mL, 2.783 mmol) in THF (6 mL) was added dropwise n-butyllithium (1.12 mL of 2.5 M in hexanes, 2.80 mmol). The reaction mixture was stirred at -78 °C for 30 minutes and 0 °C for 30 minutes. A solution of 5-bromo-2-fluoro-3-(methoxymethoxy)pyridine **C392** (0.70 g, 2.80 mmol) in THF (3 mL) was added dropwise at -78 °C and the reaction was stirred at -78 °C for 2 hours. Iodine (0.71 g, 2.80 mmol) in 3 mL THF was added dropwise at -78 °C and the reaction was stirred at -78 °C for 2 hours and warmed to room temperature before quenching with aqueous saturated Na₂S₂O₄. The product was extracted with EtOAc. The organic solution was dried with Na₂SO₄, filtered and concentrated *in vacuo.* The resulting residue was purified by silica gel chromatography (40 g ISCO column) using 0-30% EtOAc/heptanes gradient to afford 0.80 g of product. 5-bromo-2-fluoro-4-iodo-3-(methoxymethoxy)pyridine (79%). ¹H NMR (300 MHz, Chloroform-d) δ 8.07 (d, *J=* 1.6 Hz, 1H), 5.30 (d, *J=* 1.0 Hz, 2H), 3.66 (s, 3H).

### Step 3. Synthesis of 5-bromo-2-fluoro-3-(methoxymethoxy)-4-(3-methylbut-1-ynyl)pyridine (C394)

A solution of 5-bromo-2-fluoro-4-iodo-3-(methoxymethoxy)pyridine **C393** (0.60 g, 1.66 mmol) in 1,4-dioxane (3.2 mL) and diisopropylamine (1.40 mL, 9.99 mmol) was degassed with nitrogen for 10 minutes. 3-methylbut-1-yne (0.26 mL, 2.52 mmol) was then added, followed by CuI (0.02 g, 0.08 mmol) and PdCl₂(PPh₃)₂ (0.06 g, 0.08 mmol). The reaction was heated overnight at 55 °C in a sealed flask. The reaction mixture was diluted with water and extracted with EtOAc. The organic layer was concentrated to dryness. The resulting residue was purified by silica gel chromatography using 0-40% EtOAc/heptanes gradient to afford 300 mg of product. 5-bromo-2-fluoro-3-(methoxymethoxy)-4-(3-methylbut-1-ynyl)pyridine (60%). ¹H NMR (300 MHz, Chloroform-*d*) δ 7.97 (d, *J =* 1.5 Hz, 1H), 5.20 (d, *J =* 0.5 Hz, 2H), 3.55 (s, 3H), 2.84 (dt, *J =* 13.8, 6.9 Hz, 1H), 1.27 - 1.23 (m, 6H).

### Step 4. Synthesis of 5-fluoro-1-(4-fluorophenyl)-2-isopropyl-4-(methoxymethoxy)pyrrolo[2, 3-c]pyridine (C395)

To a 20 mL scintillation vial was added 5-bromo-2-fluoro-3-(methoxymethoxy)-4-(3-methylbut-1-ynyl)pyridine **C394** (0.300 g, 0.993 mmol) and 2-methylpropan-2-olate (Sodium salt) (0.283 g, 2.945 mmol) followed by 4-fluoroaniline (0.113 mL, 1.190 mmol). tBuOH (3.75 mL) was added and the mixture was degassed with nitrogen for 10 minutes. Ditert-butyl-[2-(2,4,6-triisopropylphenyl)phenyl]phosphane; dichloromethane; methane sulfonate; *N*-methyl-2-phenyl-aniline; palladium (tBuXPhos G3) (0.094 g, 0.105 mmol) was added and the reaction mixture was heated at 90 °C for overnight. The reaction was quenched by addition of aqueous saturated NH₄Cl solution and extracted with EtOAc. The organic phase was dried (MgSO₄), filtered, and concentrated *in vacuo.* The resulting residue was purified by silica gel chromatography using 0-10% EtOAc/heptanes gradient to afford 140 mg of product. 5-fluoro-1-(4-fluorophenyl)-2-isopropyl-4-(methoxymethoxy)-pyrrolo[2,3-c]pyridine (42%). ¹H NMR (300 MHz, Methanol-*d*4) δ 7.52 - 7.43 (m, 3H), 7.42 - 7.31 (m, 2H), 6.67 (d, *J=* 0.8 Hz, 1H), 5.32 (s, 2H), 3.60 (s, 3H), 2.95 (pd, *J* = 6.9, 0.7 Hz, 1H), 1.24 (d, *J=* 6.9 Hz, 6H).

### Step 5. Synthesis of 3-bromo-5-fluoro-1-(4-fluorophenyl)-2-isopropyl-4-(methoxymethoxy)pyrrolo[2,3-c]pyridine (C396)

To a cold (0 °C) solution of 5-fluoro-1-(4-fluorophenyl)-2-isopropyl-4-(methoxymethoxy)pyrrolo[2,3-c]pyridine **C395** (0.140 g, 0.403 mmol) in DMF (4 mL) was added 1-bromopyrrolidine-2,5-dione (0.094 g, 0.524 mmol). The reaction mixture was stirred at 0 °C for 1 hour. The reaction was quenched with the addition of 1N sodium thiosulfate, washed with water, and extracted with EtOAc. The resulting residue was purified by silica gel chromatography (12 g ISCO column) using 0-10% MeOH/CH₂Cl₂ gradient to afford 160 mg of product. 3-bromo-5-fluoro-1-(4-fluorophenyl)-2-isopropyl-4-(methoxymethoxy)pyrrolo[2,3-c]pyridine (94%). ESI-MS m/z calc. 410.04, found 411.36 (M+1)⁺.

### Step 6. Synthesis of methyl 4-[5-fluoro-1-(4-fluorophenyl)-2-isopropyl-4-(methoxymethoxy)pyrrolo[2,3-c]pyridin-3-yl]benzoate (C397)

To a vial was added 3-bromo-5-fluoro-1-(4-fluorophenyl)-2-isopropyl-4-(methoxymethoxy)-pyrrolo[2,3-c]pyridine **C396** (0.040 g, 0.083 mmol), methyl 4-(4,4,5,5-tetramethyl-1,3,2-dioxaborolan-2-yl)benzoate (0.028 g, 0.107 mmol), [2-(2-aminophenyl)phenyl]-sulfooxy-palladium;dicyclohexyl-[2-(2,6-dimethoxyphenyl)phenyl]phosphane (SPhos G3) (0.007 g, 0.008 mmol). The vial was sealed and flushed with nitrogen. To the vial was added dioxane (0.80 mL) and Na₂CO₃ (0.167 mL of 2 M, 0.334 mmol). The reaction mixture was heated at 80 °C for 2 hours. The reaction was quenched with aqueous saturated NH₄Cl solution and extracted with EtOAc. The organic solution was dried with Na₂SO₄, filtered and concentrated *in vacuo.* The product was purified by ISCO (24 g silica gel, 20% to 50% EtOAc in heptane) to afford 36 mg of product. Methyl 4-[5-fluoro-1-(4-fluorophenyl)-2-isopropyl-4-(methoxymethoxy)pyrrolo[2,3-c]pyridin-3-yl]benzoate (92%). ESI-MS m/z calc. 466.17, found 467.13 (M+1)⁺.

### Step 7. Synthesis of methyl 4-[5-fluoro-1-(4-fluorophenyl)-4-hydroxy-2-isopropyl-pyrrolo[2, 3-c]pyridin-3-yl]benzoate (C398)

To methyl 4-[5-fluoro-1-(4-fluorophenyl)-2-isopropyl-4-(methoxymethoxy)-pyrrolo[2,3-c]pyridin-3-yl]benzoate **C397** (0.036 g, 0.077 mmol) was added HCl (2 mL of 4 M, 8.000 mmol) in dioxane. The reaction was stirred at room temperature for 1 hour. The reaction was diluted with water and basified by 1 N NaOH to pH~4. The product was extracted with EtOAc and concentrated. The product was purified by silica gel chromatography (12 g silica gel, 0% to 40% EtOAc/ heptanes gradient) to afford 24 mg of product. Methyl 4-[5-fluoro-1-(4-fluorophenyl)-4-hydroxy-2-isopropyl-pyrrolo[2,3-c]pyridin-3-yl]benzoate (59%). ESI-MS m/z calc. 422.14, found 423.47 (M+1)⁺.

### Step 8. Synthesis of methyl 4-[5-fluoro-1-(4-fluorophenyl)-4-hydroxy-2-isopropyl-pyrrolo[2, 3-c]pyridin-3-yl]benzoic acid (425)

To a solution of methyl 4-[5-fluoro-1-(4-fluorophenyl)-4-hydroxy-2-isopropyl-pyrrolo[2,3-c]pyridin-3-yl]benzoate **C398** (0.024 g, 0.057 mmol) in THF (0.96 mL)/MeOH (0.32 mL)/water (0.32 mL) was added LiOH (0.010 g, 0.417 mmol). The reaction was stirred at room temperature overnight. The reaction was diluted with water and acidified by 1N HCl to pH~4. The product was extracted with EtOAc and concentrated to afford 21 mg of product. 4-[5-fluoro-1-(4-fluorophenyl)-4-hydroxy-2-isopropyl-pyrrolo[2,3-c]pyridin-3-yl]benzoic acid (60%). ¹H NMR (300 MHz, Methanol-*d*4) δ 8.09 - 7.98 (m, 2H), 7.59 - 7.47 (m, 5H), 7.45 - 7.33 (m, 2H), 7.17 (d, *J* = 2.0 Hz, 1H), 3.09 (hept, *J* = 7.2 Hz, 1H), 1.10 (d, *J =* 7.2 Hz, 7H). ESI-MS m/z calc. 408.19, found 409.42 (M+1)⁺.

### 3-bromo-1-(4-fluorophenyl)-2-isopropyl-4-(methoxymethoxy)-1H-pyrrolo[2,3-c]pyridine (S23)

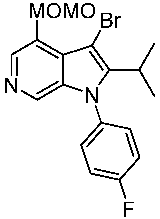

Intermediate **S23** was prepared in same fashion as **C396** using 5-chloropyridin-3-ol instead of 5-bromo-2-fluoro-pyridin-3-ol as described in the synthesis of **C392.** Iodination with nBuLi, Sonogashira coupling with alkyne, Pd-catalyzed Buchwald coupling with 4-fluoroaniline and bromination with NBS afforded the product. ESI-MS m/z calc. 392.06, found 392.94 (M+1)⁺.

### Compound 426

### 4-[5-fluoro-1-(4-fluorophenyl)-4-hydroxy-2-isopropyl-pyrrolo[2,3-c]pyridin-3-yl]benzoic acid (426)

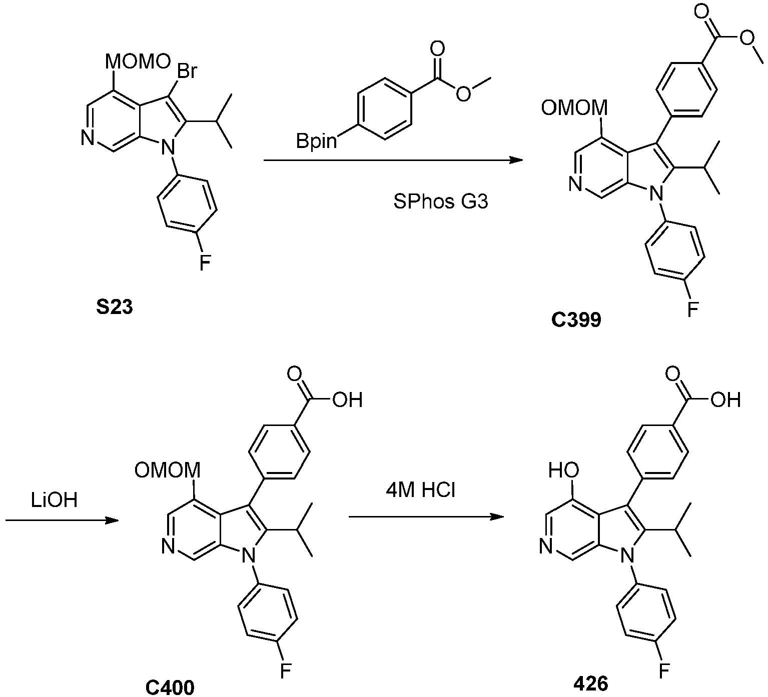

### Step 1. Synthesis of methyl 4-[1-(4-fluorophenyl)-2-isopropyl-4-(methoxymethoxy)-pyrrolo[2,3-c]pyridin-3-yl]benzoate (C399)

To a vial was added 3-bromo-5-fluoro-1-(4-fluorophenyl)-2-isopropyl-4-(methoxymethoxy)pyrrolo[2,3-c]pyridine **S23** (0.050 g, 0.109 mmol), methyl 4-(4,4,5,5-tetramethyl-1,3,2-dioxaborolan-2-yl)benzoate (0.046 g, 0.175 mmol), [2-(2-aminophenyl)phenyl]-sulfooxy-palladium;dicyclohexyl-[2-(2,6-dimethoxyphenyl)phenyl] phosphane (0.009 g, 0.011 mmol). The vial was sealed and flushed by nitrogen. To the vial was added dioxane (1 mL) and Na₂CO₃ (0.218 mL of 2 M, 0.436 mmol). The reaction was stirred at 80 °C for 2 hours. The reaction was quenched with aqueous saturated NH₄Cl solution and extracted with EtOAc. The organic solution was dried with Na₂SO₄, filtered and concentrated *in vacuo.* The product was purified by silica gel chromatography (24 g silica gel column, 20% to 50% EtOAc/ heptanes gradient) to afford 34 mg of product. Methyl 4-[1-(4-fluorophenyl)-2-isopropyl-4-(methoxymethoxy)pyrrolo[2,3-c]pyridin-3-yl]benzoate (62%). ESI-MS m/z calc. 448.18, found 448.77 (M+1)⁺.

### Step 2. Synthesis of 4-[1-(4-fluorophenyl)-2-isopropyl-4-(methoxymethoxy)pyrrolo[2,3-c]pyridin-3-yl]benzoic acid (C400)

To a solution of methyl 4-[1-(4-fluorophenyl)-2-isopropyl-4-(methoxymethoxy)pyrrolo[2,3-c]pyridin-3-yl]benzoate **C399** (0.034 g, 0.076 mmol) in THF (1.2 mL)/MeOH (0.4 mL)/H₂O (0.4 mL) was added LiOH (0.013 g, 0.543 mmol). The reaction was stirred at room temperature overnight. The reaction was diluted with water and acidified by 1N HCl to pH~4. The product was extracted with EtOAc and concentrated to afford 20 mg of product. 4-[1-(4-fluorophenyl)-2-isopropyl-4-(methoxymethoxy)pyrrolo[2,3-c]pyridin-3-yl]benzoic acid (40%). ESI-MS m/z calc. 434.16, found 435.08 (M+1)⁺.

### Step 3. Synthesis of 4-[1-(4-fluorophenyl)-4-hydroxy-2-isopropyl-pyrrolo[2,3-c]pyridin-3-yl]benzoic acid (426)

To 4-[1-(4-fluorophenyl)-2-isopropyl-4-(methoxymethoxy)pyrrolo-[2,3-c]pyridin-3-yl]benzoic acid **C400** (0.020 g, 0.046 mmol) was added HCl (0.545 mL of 4 M solution in dioxane, 2.180 mmol). The reaction mixture was stirred at room temperature for 2 hours. The reaction was diluted with water and basified by 1N NaOH to pH~4. The product was extracted with EtOAc and concentrated to afford 15 mg of product. 4-[1-(4-fluorophenyl)-4-hydroxy-2-isopropyl-pyrrolo[2,3-c]pyridin-3-yl]benzoic acid (35%). ¹H NMR (300 MHz, DMSO-*d*6) δ 9.57 (s, 1H), 7.99 - 7.92 (m, 2H), 7.70 - 7.59 (m, 4H), 7.57 - 7.43 (m, 4H), 3.03 (p, *J* = 7.1 Hz, 1H), 1.03 (d, *J =* 7.2 Hz, 6H). ESI-MS m/z calc. 390.14, found 391.05 (M+1)⁺.

### Compound 427

### 4-[7-chloro-1-(4-fluorophenyl)-4-hydroxy-2-isopropyl-pyrrolo[2,3-c]pyridin-3-yl]benzoic acid (427)

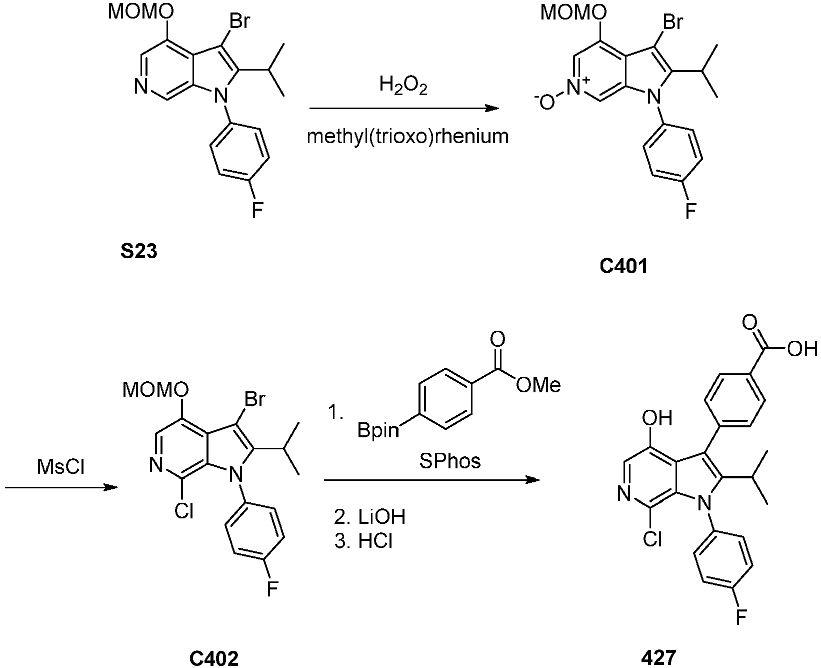

### Step 1. Synthesis of 3-bromo-1-(4-fluorophenyl)-2-isopropyl-4-(methoxymethoxy)-6-oxido-pyrrolo[2, 3-c]pyridin-6-ium (C401)

To a vial containing 3-bromo-1-(4-fluorophenyl)-2-isopropyl-4-(methoxymethoxy)pyrrolo[2,3-c]pyridine **S23** (0.130 g, 0.280 mmol) was added H₂O₂ (0.064 mL of 30% w/w, 0.564 mmol). The reaction vessel was charged with CH₂Cl₂ (1 mL), followed by methyl(trioxo)rhenium (0.007 g, 0.028 mmol) and the mixture was stirred at room temperature overnight. The reaction was quenched by the addition of manganese(IV) oxide (0.010 g, 0.120 mmol). After the evolution of gas had ceased (20 min), the mixture was concentrated *in vacuo.* The resulting residue was purified by silica gel chromatography (40 g ISCO column) using 0-10% MeOH/ CH₂Cl₂ gradient to afford 110 mg of product. 3-bromo-1-(4-fluorophenyl)-2-isopropyl-4-(methoxymethoxy)-6-oxido-pyrrolo[2,3-c]pyridin-6-ium (85%). ¹H NMR (300 MHz, Chloroform-*d*) δ 7.96 (d, *J =* 1.4 Hz, 1H), 7.76 (d, *J =* 1.4 Hz, 1H), 7.32 - 7.23 (m, 5H), 5.31 (s, 2H), 3.59 (s, 3H), 3.04 (h, *J* = 7.2 Hz, 1H), 1.36 (d, *J* = 7.2 Hz, 6H). ESI-MS m/z calc. 408.05, found 408.97 (M+1)⁺.

### Step 2. Synthesis of 3-bromo-7-chloro-1-(4-fluorophenyl)-2-isopropyl-4-(methoxymethoxy)-pyrrolo[2,3-c]pyridine (C402)

A cold (0 °C) solution of MsCl (0.057 mL, 0.736 mmol) in CH₂Cl₂ (2 mL) was added into a solution of 3-bromo-1-(4-fluorophenyl)-2-isopropyl-4-(methoxymethoxy)-6-oxido-pyrrolo[2,3-c]pyridin-6-ium **C401** (0.150 g, 0.367 mmol) in CH₂Cl₂ (3.75 mL). The reaction was gradually warmed to room temperature over 2 hours, and then stirred for 2 hours. The reaction was quenched by aqueous saturated NaHCO₃ solution. The product was extracted with EtOAc. The organic solution was dried with Na₂SO₄, filtered and concentrated *in vacuo.* The resulting residue was purified by silica gel chromatography (12 g ISCO column) using 0-30% EtOAc/heptanes gradient to afford 50 mg of product. 3-Bromo-7-chloro-1-(4-fluorophenyl)-2-isopropyl-4-(methoxymethoxy)pyrrolo[2,3-c]pyridine (32%). ¹H NMR (300 MHz, Chloroform-*d*) δ 7.83 (s, 1H), 7.35 - 7.14 (m, 4H), 5.34 (s, 2H), 3.62 (s, 3H), 2.95 (hept, *J* = 7.2 Hz, 1H), 1.36 (d, *J* = 7.2 Hz, 7H).

### Step 3. Synthesis of 4-[7-chloro-1-(4-fluorophenyl)-4-hydroxy-2-isopropyl-pyrrolo[2,3-c]pyridin-3-yl]benzoic acid (427)

To a vial was added 3-bromo-7-chloro-1-(4-fluorophenyl)-2-isopropyl-4-(methoxymethoxy)-pyrrolo[2,3-c]pyridine **C402** (0.028 g, 0.056 mmol), boronic acid (methyl 4-(4,4,5,5-tetramethyl-1,3,2-dioxaborolan-2-yl)benzoate (0.024 g, 0.092 mmol), [2-(2-aminophenyl)phenyl]-sulfooxy-palladium;dicyclohexyl-[2-(2,6-dimethoxyphenyl)phenyl] phosphane (0.004 g, 0.006 mmol). The vial was sealed and flushed with nitrogen. To the vial was added dioxane (0.560 mL) and Na₂CO₃ (0.112 mL of 2 M aq solution, 0.224 mmol). The reaction was stirred at 80 °C for 2 hours. After cooling to room temperature, the reaction was quenched with aqueous saturated NH₄Cl solution and extracted with EtOAc. The organic solution was dried with Na₂SO₄, filtered and concentrated *in vacuo.* The resulting residue was purified by silica gel chromatography (24 g ISCO column) using 0-100% EtOAc/heptanes to afford 3-aryl azaindole product. Methyl 4-[7-chloro-1-(4-fluorophenyl)-2-isopropyl-4-(methoxymethoxy)pyrrolo[2,3-c]pyridin-3-yl]benzoate. To a solution of the intermediate in THF (0.60 mL)/MeOH (0.20 mL)/H₂O (0.20 mL) was added LiOH (0.007 g, 0.278 mmol). The reaction was stirred at room temperature for 4 hours. The reaction was diluted with water and acidified with 1N HCl to pH~3. The product was extracted with EtOAc and the organic solution was concentrated to afford 4-[7-chloro-1-(4-fluorophenyl)-2-isopropyl-4-(methoxymethoxy)-pyrrolo[2,3-c]pyridin-3-yl]benzoic acid. To this intermediate was added HCl (0.28 mL of 4 M, 1.12 mmol). The reaction was stirred at room temperature for 2 hours. The reaction was diluted with water and basicified with 1N NaOH to pH~3. The product was extracted with EtOAc and concentrated to afford 3.2 mg of product. 4-[7-Chloro-1-(4-fluorophenyl)-4-hydroxy-2-isopropyl-pyrrolo[2,3-c]pyridin-3-yl]benzoic acid (12%). ¹H NMR (400 MHz, Methanol-*d*4) δ 8.07 - 8.01 (m, 2H), 7.55 - 7.46 (m, 4H), 7.38 (s, 1H), 7.35 - 7.26 (m, 2H), 3.76 - 3.54 (m, 2H), 2.96 (hept, *J* = 7.1 Hz, 1H), 1.05 (d, *J=* 7.2 Hz, 6H). ESI-MS m/z calc. 424.1, found 425.0 (M+1)⁺.

### Compound 428

### 4-(5-chloro-1-(4-fluorophenyl)-4-hydroxy-2-isopropyl-1H-pyrrolo[2,3-c]pyridin-3-yl)benzoic acid (428)

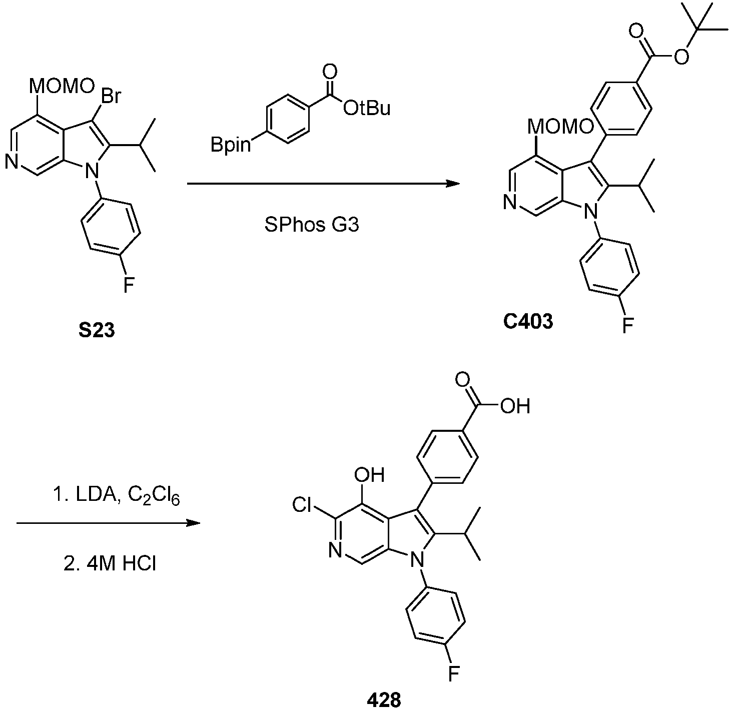

### Step 1. Synthesis of tert-butyl 4-[1-(4-fluorophenyl)-2-isopropyl-4-(methoxymethoxy)-pyrrolo[2,3-c]pyridin-3-yl]benzoate (C403)

To a vial was added 3-bromo-1-(4-fluorophenyl)-2-isopropyl-4-(methoxymethoxy)pyrrolo[2,3-c]pyridine **S23** (0.150 g, 0.327 mmol), *tert*-butyl 4-(4,4,5,5-tetramethyl-1,3,2-dioxaborolan-2-yl)benzoate (0.159 g, 0.523 mmol) and [2-(2-aminophenyl)phenyl]-sulfooxy-palladium-dicyclohexyl-[2-(2,6-dimethoxyphenyl)phenyl]phosphane (0.026 g, 0.0327 mmol). The vial was sealed and flushed with nitrogen. To the vial was added dioxane (3 mL) and Na₂CO₃ (0.655 mL of 2 M aq solution, 1.310 mmol). The reaction was stirred at 70 °C for 2 hours. After cooling to room temperature, the reaction was quenched with aqueous saturated NH₄Cl solution and extracted with EtOAc. The organic solution was dried with Na₂SO₄, filtered and concentrated *in vacuo.* The resulting residue was purified by silica gel chromatography (24 g ISCO column) using 0-40% EtOAc/heptanes gradient to afford 150 mg of product tert-butyl 4-[1-(4-fluorophenyl)-2-isopropyl-4-(methoxymethoxy)pyrrolo[2,3-c]pyridin-3-yl]benzoate (86%). ESI-MS m/z calc. 490.23, found 491.17 (M+1)⁺.

### Step 2. Synthesis of 4-[5-chloro-1-(4-fluorophenyl)-4-hydroxy-2-isopropyl-pyrrolo[2,3-c]pyridin-3-yl]benzoic acid (428)

To a cold (-78 °C) solution of *tert*-butyl 4-[1-(4-fluorophenyl)-2-isopropyl-4-(methoxymethoxy)-pyrrolo[2,3-c]pyridin-3-yl]benzoate **C403** (0.030 g, 0.0612 mmol) in THF (1 mL) was added dropwise LDA (0.061 mL of 2 M, 0.1220 mmol). The reaction was stirred for 2 hours at the same temperature, then C₂Cl₆ (0.036 g, 0.153 mmol) in 0.4 mL THF at -78 °C was added. The reaction was gradually warmed to room temperature and stirred overnight. The reaction was quenched with aqueous saturated NH₄Cl solution and extracted with EtOAc. The organic solution was dried with Na₂SO₄, filtered and concentrated *in vacuo.* The resulting residue was purified by silica gel chromatography (24 g ISCO column) using 0-50% EtOAc/heptanes gradient to afford product. *tert-*Butyl 4-[5-chloro-1-(4-fluorophenyl)-2-isopropyl-4-(methoxymethoxy)pyrrolo[2,3-c]pyridin-3-yl]benzoate. This product was dissolved in HCl (1 mL of 4 M, 4.000 mmol). The solution was stirred at room temperature for 1 hour, 40 °C for 1 hour, and then 50 °C for 2 hours. The reaction was diluted with water and basified with 1N NaOH to pH~4. The product was extracted with EtOAc and the organic solution was concentrated to afford 4.6 mg of product. 4-[5-chloro-1-(4-fluorophenyl)-4-hydroxy-2-isopropyl-pyrrolo[2,3-c]pyridin-3-yl]benzoic acid (17%). ¹H NMR (300 MHz, DMSO-*d*6) δ 8.02 - 7.93 (m, 4H), 7.72 (d, J = 10.2 Hz, 3H), 7.70 - 7.62 (m, 2H), 7.55 - 7.48 (m, 3H), 3.02 (p, *J* = 7.1 Hz, 1H), 1.04 (dd, *J* = 7.1, 2.2 Hz, 9H). ESI-MS *m*/*z* calc. 424.1, found 425.0 (M+1)⁺.

### Benzyl 4-(4-methoxy-3,3-dimethylbut-1-yn-1-yl)benzoate (S24)

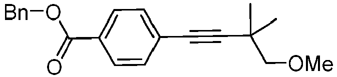

Intermediate **S24** was prepared in same fashion as **C222** using benzyl 4-iodobenzoate instead of methyl 4-iodobenzoate and 4-methoxy-3,3-dimethyl-but-1-yne instead of 2,2-dimethylbut-3-yn-1-ol. ¹H NMR (400 MHz, Chloroform-*d*) δ 8.07 - 7.98 (m, 2H), 7.54 - 7.35 (m, 7H), 5.38 (s, 2H), 3.47 (s, 3H), 3.38 (s, 2H), 1.36 (s, 6H). LCMS *m*/*z* 323.16 [M+H]⁺.

### Benzyl 4-(4-methoxy-3,3-dimethylbut-1-yn-1-yl)benzoate (S25)

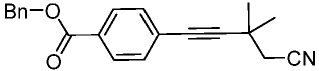

Intermediate **S25** was prepared in same fashion as **C222** using benzyl 4-iodobenzoate instead of methyl 4-iodobenzoate and 3,3-dimethylpent-4-ynenitrile instead of 2,2-dimethylbut-3-yn-1-ol. ESI-MS m/z calc. 392.06, found 392.94 (M+1)⁺. ¹H NMR (400 MHz, Chloroform-d) δ 8.07 - 7.98 (m, 2H), 7.54 - 7.35 (m, 7H), 5.38 (s, 2H), 3.47 (s, 3H), 3.38 (s, 2H), 1.36 (s, 6H). LCMS *m*/*z* 323.16 [M+H]⁺. ¹H NMR (400 MHz, Chloroform-d) δ8.14 - 7.95 (m, 2H), 7.57 - 7.32 (m, 7H), 5.39 (s, 2H), 2.64 (s,2H), 1.52 (s, 6H). LCMS *m*/*z* 318.12 [M+H]⁺.

### Compound 429

### 4-(4-amino-6-fluoro-1-(4-fluorophenyl)-2-(1-methoxy-2-methylpropan-2-yl)-1H-indol-3-yl)benzoic acid (429)

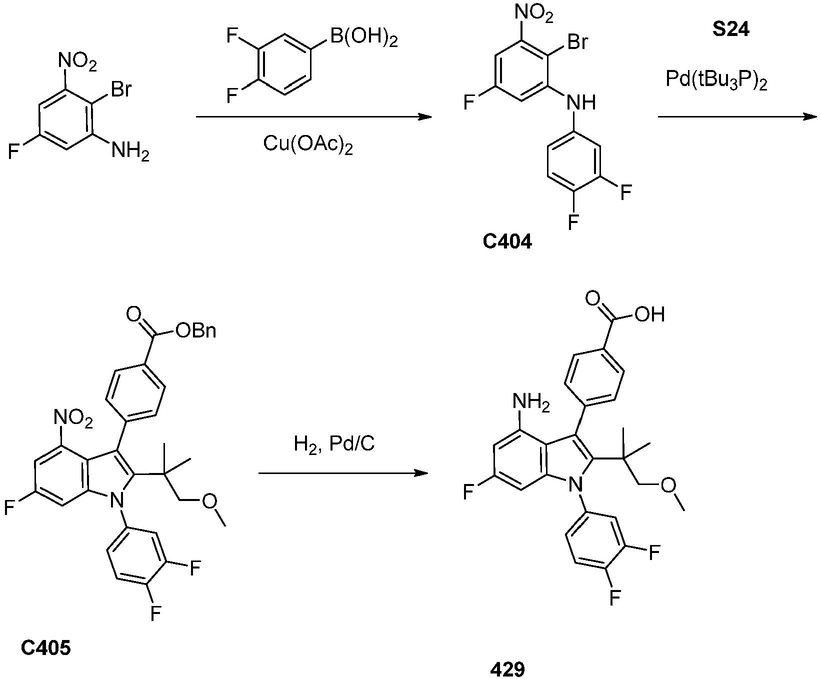

### Step 1. Synthesis of 2-bromo-5-fluoro-N-(4-fluorophenyl)-3-nitroaniline (C404)

A solution of 2-bromo-5-fluoro-3-nitro-aniline (0.53 g, 2.26 mmol), (3,4-difluorophenyl)boronic acid (1.40 g, 8.87 mmol), copper (II) acetate (0.77 g, 4.24 mmol) and 4Å Sieves in dichloromethane (15.5 mL) was stirred for 15 minutes. Triethylamine (0.82 mL, 5.88 mmol) was added dropwise at ambient temperature and the resulting mixture was stirred at room temperature open to the air for 72 hours. The reaction mixture was filtered through Celite^{®} and diluted with dichloromethane. The filtrate was washed successively with aqueous saturated NH₄Cl (2x), brine, dried over MgSO₄, filtered and concentrated *in vacuo.* Purification by silica gel chromatography (0- 30% ethyl acetate in heptane) afforded 300 mg of product. 2-Bromo-5-fluoro-*N*-(4-fluorophenyl)-3-nitroaniline (33%). LCMS *m*/*z* 347.3 [M+H]⁺.

### Step 2. benzyl 4-(1-(3,4-difluorophenyl)-6-fluoro-2-(1-methoxy-2-methylpropan-2-yl)-4-nitro-1H-indol-3-yl)benzoate (C405)

A solution of 2-bromo-N-(3,4-difluorophenyl)-5-fluoro-3-nitro-aniline **C404** (0.200 g, 0.576 mmol), benzyl 4-(4-methoxy-3,3-dimethyl-but-1-ynyl)benzoate **S24** (0.278 g, 0.862 mmol), *N*-cyclohexyl-*N*-methyl-cyclohexanamine (0.300 mL, 1.401 mmol) and dioxane (4 mL) was degassed with nitrogen for 10 minutes. Pd(tBu₃P)₂ (0.012 g, 0.023 mmol) was added and the solution was degassed for an additional 5 minutes at room temperature, then the reaction was heated to 105 °C and stirred for 1 hour. The reaction was heated to 120 °C and stirred for 16 hours. The reaction was cooled to room temperature and Pd(tBu₃P)₂ (0.018 g, 0.035 mmol) was added, then the reaction was heated at 120 °C for 4 hours. The reaction was cooled to room temperature, diluted with water, and extracted with EtOAc. The organic layer was dried (MgSO₄), filtered, and concentrated *in vacuo.* Purification by silica gel chromatography (0-60% ethyl acetate in heptane) afforded 66 mg of product. Benzyl 4-(1-(3,4-difluorophenyl)-6-fluoro-2-(1-methoxy-2-methylpropan-2-yl)-4-nitro-1H-indol-3-yl)benzoate (19%). LCMS *m*/*z* 589.34 [M+H]⁺.

### Step 3. 4-(4-amino-1-(3,4-difluorophenyl)-6-fluoro-2-(1-methoxy-2-methylpropan-2-yl)-1H-indol-3-yl)benzoic acid (429)

A solution of 4-[1-(3,4-difluorophenyl)-6-fluoro-2-(2-methoxy-1,1-dimethyl-ethyl)-4-nitro-indol-3-yl]benzoate **C405** (0.020 g, 0.034 mmol) and palladium on carbon (0.005 g, 0.002 mmol) in EtOH (2 mL) and EtOAc (2 mL) was evacuated and backfilled with hydrogen gas three times. The reaction was stirred at room temperature for 15 minutes, then filtered through Celite^{®}, diluted with EtOAc and EtOH and the filtrate was concentrated *in vacuo.* Purification by reversed-phase HPLC on a C18 Waters Sunfire column (30x150 mm, 5 micron) (5-95% MeCN in H₂O with 0.1% trifluoroacetic acid) afforded 4.4 mg of product. 4-[4-amino-1-(3,4-difluorophenyl)-6-fluoro-2-(2-methoxy-1,1-dimethyl-ethyl)indol-3-yl]benzoic acid (28%). ¹H NMR (400 MHz, DMSO-*d*6) δ 8.05 - 7.97 (m, 2H), 7.76 - 7.52 (m, 4H), 7.41 - 7.29 (m, 1H), 5.97 (dd, *J=* 12.0, 2.3 Hz, 1H), 5.55 (dd, *J=* 9.9, 2.3 Hz, 1H), 3.01 (s, 3H), 2.96 - 2.84 (m, 2H), 0.96 (s, 6H). LCMS *m*/*z* 469.24 [M+1]⁺.

### Compounds 430-433

Compounds **430-433** were prepared by Larock indole cyclization.

**Table 23. Method of preparation, structure, physicochemical data for compounds 430-433.**

| **Compound** | **Method/Product** | **Boronic acid or ester** | **¹H NMR; LCMS m/z [M+H]⁺** |
|---|---|---|---|
| **430** | From**S24**^{1,2} | 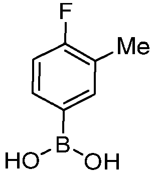 | ¹H NMR (400 MHz, Methanol-*d*4) δ 8.12 - 8.04 (m, 2H), 7.66 - 7.59 (m, 2H), 7.35 - 7.21 (m, 3H), 5.97 (dd, *J*= 11.6, 2.2 Hz, 1H), 5.56 (dd, *J=* 9.9, 2.2 Hz, 1H), 3.09 (s, 3H), 2.98 (s, 2H), 2.41 - 2.33 (m, 3H), 1.04 (d, *J=* 3.4 Hz, 7H). LCMS *m*/*z* 465.31 [M+H]⁺. |
| | 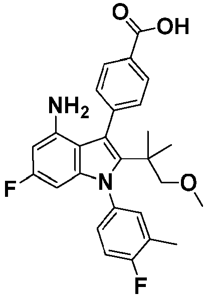 | | |
| **431** | From**S25**^{3,2} | 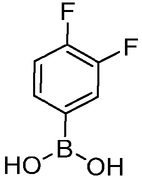 | ¹H NMR (400 MHz, DMSO-*d*6) δ 13.17 (s, 1H), 8.09 - 7.98 (m, 2H), 7.83 - 7.57 (m, 4H), 7.39 (dq, *J=* 7.8, 3.2, 2.5 Hz, 1H), 6.01 (dd, *J=* 12.0, 2.3 Hz, 1H), 5.59 (dd, *J=* 9.8, 2.3 Hz, 1H), 4.30 (s, 2H), 3.34 (s, 2H), 1.11 (d, *J=* 7.7 Hz, 6H). LCMS *m*/*z* 464.31 [M+H]⁺. |
| | 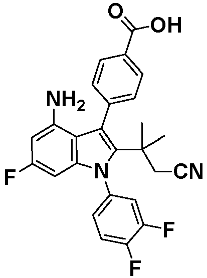 | | |
| **432** | From**S25**^{1,2} | 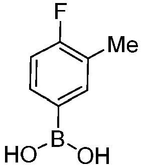 | ¹H NMR (400 MHz, DMSO-d6) δ 13.17 (s, 1H), 8.08 - 7.96 (m, 2H), 7.65 (dq, *J=* 8.4, 2.0 Hz, 2H), 7.52 - 7.27 (m, 3H), 5.99 (dd, *J=* 12.1, 2.3 Hz, 1H), 5.49 (dd, *J=* 9.8, 2.3 Hz, 1H), 4.28 (s, 2H), 3.34 (s, 1H), 2.34 (d, *J=* 2.0 Hz, 3H), 1.10 (d, *J=* 3.6 Hz, 6H). LCMS *m*/*z* 460.31 [M+H]⁺. |
| | 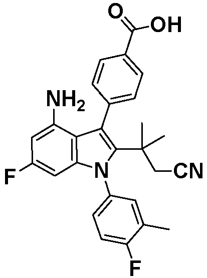 | | |
| | From**S24**^{4,2} | | |
| **433** | 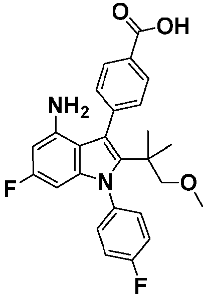 | 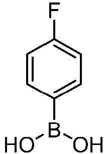 | ¹H NMR (400 MHz, DMSO-*d6)* δ 8.02 - 7.96 (m, 2H), 7.61 - 7.57 (m, 2H), 7.51 - 7.39 (m, 4H), 6.72 - 6.67 (m, 1H), 6.10 (d, *J=* 7.5 Hz, 1H), 5.77 (d, *J=* 5.7 Hz, 1H), 3.01 (s, 3H), 2.93 (s, 2H), 0.97 (s, 6H). LCMS *m*/*z* 433.0 [M+H]⁺. |

| | | | |
|---|---|---|---|
| **^{1.}** Larock indole cyclization using 2-bromo-5-fluoro-*N*-(4-fluoro-3-methylphenyl)-3-nitroaniline **^{2.}** Nitro reduction and benzyl ether hydrolysis using hydrogen, Pd/C, EtOH, EtOAc **^{3.}** Larock indole cyclization using **C404** **^{4.}** Larock indole cyclization using 2-bromo-5-fluoro-*N*-(4-fluorophenyl)-3-nitroaniline | | | |

### Compound 434

### 4-(4-amino-6-fluoro-1-(4-fluorophenyl)-2-(1-methoxy-2-methylpropan-2-yl)-1H-indol-3-yl)benzoic acid (434)

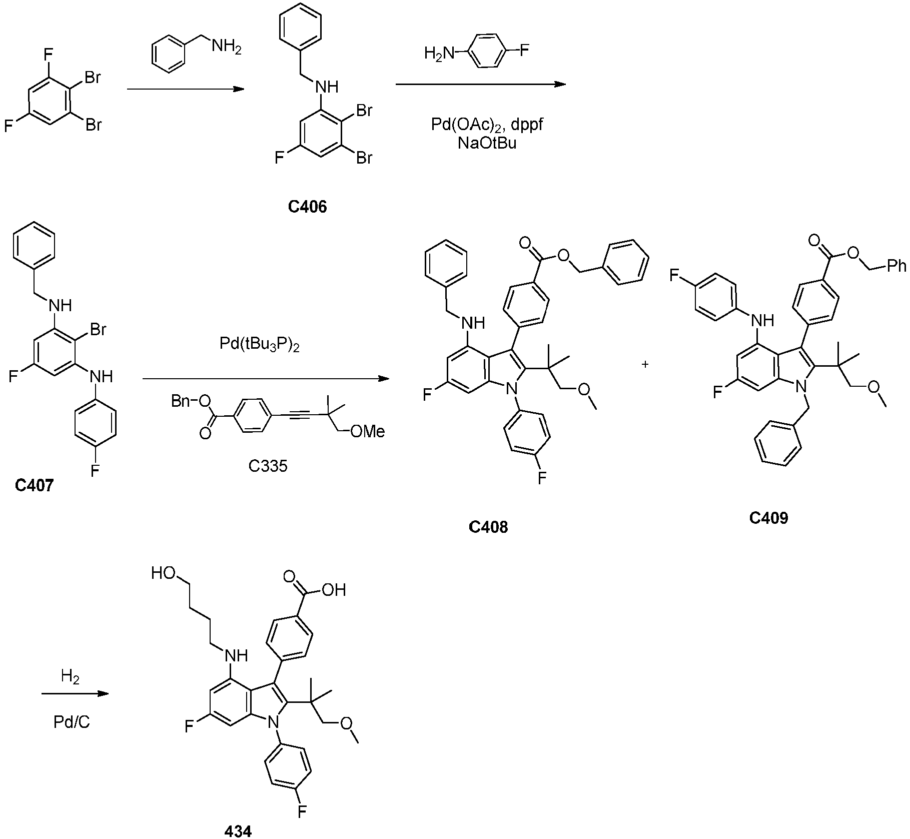

### Step 1. Synthesis of N-benzyl-2,3-dibromo-5-fluoro-aniline (C406)

A solution of 1,2-dibromo-3,5-difluoro-benzene (5.00 g, 18.39 mmol) and benzyl amine (5.00 mL, 45.78 mmol) in NMP (12 mL) was heated to 80 °C overnight. The reaction was cooled to room temperature, diluted with water, and extracted with ethyl acetate, then concentrated *in vacuo.* Purification by silica gel chromatography (0-50% CH₂Cl₂/heptanes) afforded 3.3 g of product. N-benzyl-2,3-dibromo-5-fluoro-aniline (50%). ¹H NMR (300 MHz, Chloroform-d) δ 7.47 - 7.29 (m, 5H), 6.76 (dd, *J=* 8.0, 2.8 Hz, 1H), 6.28 (dd, *J=* 10.9, 2.8 Hz, 1H), 5.13 (s, 1H), 4.39 (d, *J=* 5.5 Hz, 2H). LCMS *m*/*z* 359.63 [M+H]⁺.

### Step 2. N1-benzyl-2-bromo-5-fluoro-N3-(4-fluorophenyl)benzene-1,3-diamine (C407)

A solution of *N*-benzyl-2,3-dibromo-5-fluoro-aniline **C406** (3.30 g, 9.19 mmol), 4-fluoroaniline (1.53 g, 13.77 mmol), dppf (0.255 g, 0.460 mmol) and NaO^{t}Bu (1.77 g, 18.42 mmol) in dioxane (40 mL) was purged with nitrogen for 10 minutes. To the reaction was added Pd(OAc)₂ (0.46 mmol) and the reaction solution was purged with nitrogen for an additional 10 minutes. The mixture was heated to 70 °C overnight. The reaction was cooled to room temperature and diluted with ethyl acetate, then washed sequentially with aqueous saturated NH₄Cl solution and 6M aqueous HCl. The aqueous layers were extracted with ethyl acetate. Combined organic phases were washed twice with brine, then dried over sodium sulfate, filtered, and concentrated *in vacuo.* Purification by silica gel chromatography (0-60% dichloromethane in heptane) afforded 3.0 g product. *N*1-benzyl-2-bromo-5-fluoro-*N*3-(4-fluorophenyl)benzene-1,3-diamine (84%). ¹H NMR (300 MHz, Chloroform-*d*) δ 7.47 - 7.29 (m, 5H), 7.23 - 7.11 (m, 2H), 7.11 - 6.99 (m, 2H), 6.13 (dd, *J=* 11.0, 2.7 Hz, 1H), 6.05 (s, 1H), 5.93 (dd, *J=* 11.0, 2.7 Hz, 1H), 4.89 (s, 1H), 4.41 (d, *J=* 5.5 Hz, 2H). LCMS *m*/*z* 388.86 [M+H]⁺.

### Step 3. 4-[4-(benzylamino)-6-fluoro-1-(4-fluorophenyl)-2-(2-methoxy-1,1-dimethyl-ethyl)indol-3-yl]benzoate (C408)

A solution of *N*1-benzyl-2-bromo-5-fluoro-N3-(4-fluorophenyl)benzene-1,3-diamine **C407** (1.25 g, 3.21 mmol), benzyl 4-(4-methoxy-3,3-dimethyl-but-1-ynyl)benzoate **C335** (1.60 g, 4.81 mmol) and *N*-cyclohexyl-*N*-methyl-cyclohexanamine (1.72 mL, 8.03 mmol) in 1,4-dioxane (15 mL) was purged with nitrogen for 10 minutes. Pd(tBu₃P)₂ (0.20 g, 0.39 mmol) was added and the solution was degassed for an additional 5 minutes at room temperature. The reaction mixture was heated to 105 °C and stirred overnight. The reaction was cooled to room temperature, filtered through Celite^{®}, and washed with ethyl acetate. Purification by silica gel chromatography (0-100% CH₂Cl₂/heptanes gradient) afforded a mixture of two regioisomers: benzyl 4-(1-benzyl-6-fluoro-4-((4-fluorophenyl)amino)-2-(1-methoxy-2-methylpropan-2-yl)-1*H*-indol-3-yl)benzoate **C409** and 4-[4-(benzylamino)-6-fluoro-1-(4-fluorophenyl)-2-(2-methoxy-1,1-dimethyl-ethyl)indol-3-yl]benzoate **C408** (830 mg, 41%). LCMS *m*/*z* 631*.*24 [M+H]⁺. The mixture was taken into the next step without further purification.

### Step 4. 4-[6-fluoro-1-(4-fluorophenyl)-4-(4-hydroxybutylamino)-2-(2-methoxy-1,1-dimethylethyl)indol-3-yl]benzoic acid (434)

To a solution of a mixture of benzyl 4-[4-(benzylamino)-6-fluoro-1-(4-fluorophenyl)-2-(2-methoxy-1,1-dimethyl-ethyl)indol-3-yl]benzoate **C408** (0.32 g, 0.51 mmol) and benzyl 4-(1-benzyl-6-fluoro-4-((4-fluorophenyl)amino)-2-(1-methoxy-2-methylpropan-2-yl)-1*H*-indol-3-yl)benzoate **C409)** in THF (5 mL) was added palladium on carbon (0.05 g of 10% w/w, 0.05 mmol). The solution was purged with hydrogen gas for 1 minute, then stirred at room temperature under 1 atm of hydrogen overnight. Purification by C18 reverse phase chromatography afforded 100 mg of product. 4-[6-fluoro-1-(4-fluorophenyl)-4-(4-hydroxybutylamino)-2-(2-methoxy-1,1-dimethyl-ethyl)indol-3-yl]benzoic acid (37%). ¹H NMR (300 MHz, DMSO-*d*6) δ 13.04 (s, 1H), 8.02 (d, *J=* 8.2 Hz, 2H), 7.61 (d, *J=* 8.2 Hz, 2H), 7.55 - 7.36 (m, 4H), 5.84 (dd, *J=* 12.7, 2.1 Hz, 1H), 5.48 (dd, *J=* 9.8, 2.1 Hz, 1H), 3.22 (t, *J=* 5.7 Hz, 2H), 3.01 (s, 3H), 2.91 (s, 2H), 2.77 (d, *J=* 6.0 Hz, 2H), 1.17 - 1.01 (m, 4H), 095 (s, 6H). LCMS *m*/*z* 523.22 [M+H]⁺.

### Compound 435

### 4-(4-amino-6-fluoro-1-(4-fluorophenyl)-2-(1-methoxy-2-methylpropan-2-yl)-1H-indol-3-yl)benzoic acid (435)

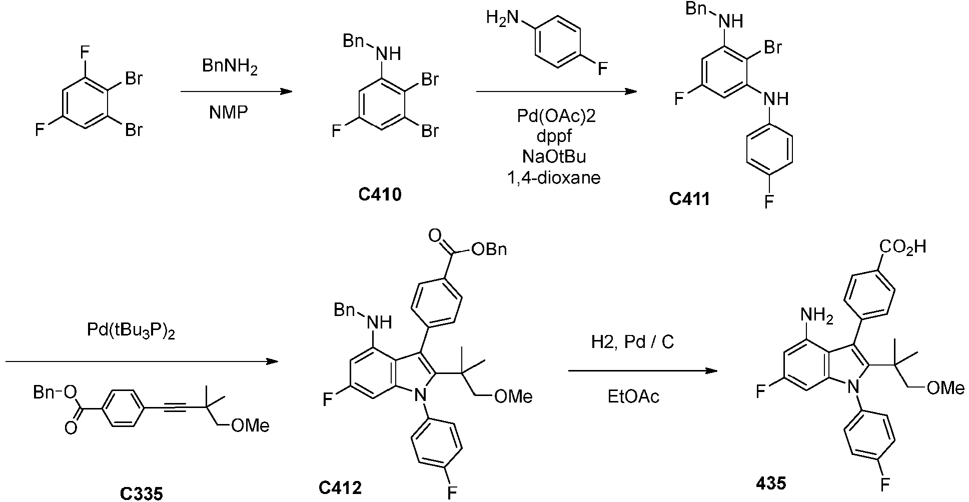

### Step 1. Synthesis of N-benzyl-2,3-dibromo-5-fluoroaniline (C410)

A solution of 1,2-dibromo-3,5-difluorobenzene (5.0 g, 18.4 mmol) and benzylamine (5 mL, 45.8 mmol) in NMP (12 mL) was stirred overnight at 80 °C. The reaction mixture was diluted with water and extracted with EtOAc. The organic layer was dried over magnesium sulfate, filtered, concentrated to dryness, and purified *via* silica gel chromatography eluting with CH₂Cl₂ in heptanes. Pure fractions were combined and concentrated to afford a colorless oil (3.3 g, 50%). ¹H NMR (300 MHz, Chloroform-*d*) δ 7.47 - 7.29 (m, 5H), 6.76 (dd, *J* = 8.0, 2.8 Hz, 1H), 6.28 (dd, *J* = 10.9, 2.8 Hz, 1H), 5.13 (s, 1H), 4.39 (d, J = 5.5 Hz, 2H). LCMS *m*/*z* 359.6 [M+1]⁺.

### Step 2. Synthesis of N¹-fluoro-N³-(4-fluorophenyl)benzene-1,3-diamine (C411)

A suspension of *N*-benzyl-2,3-dibromo-5-fluoroaniline **C410** (3.3 g, 9.2 mmol), 4-fluoroaniline (1.3 mL, 13.8 mmol), dppf (0.3 g, 0.46 mmol), and NaOtBu (1.8 g, 18.4 mmol) in 1,4-dioxane (40 mL) was degassed with nitrogen for 10 minutes. Pd(OAc)₂ (0.10 g, 0.46 mmol) was added, then mixture was degassed with nitrogen for an additional 10 minutes and heated overnight at 70 °C. The reaction mixture was diluted with saturated ammonium chloride and 6 M aq. HCl then extracted with EtOAc. The organic layer was washed with brine, dried over sodium sulfate, filtered, and concentrated to dryness. The crude residue was purified *via* silica gel chromatography eluting with CH₂Cl₂ in heptanes to afford 3 g of product (84%). ¹H NMR (300 MHz, Chloroform-*d*) δ 7.47 - 7.29 (m, 5H), 7.23 - 7.11 (m, 2H), 7.11 - 6.99 (m, 2H), 6.13 (dd, *J* = 11.0, 2.7 Hz, 1H), 6.05 (s, 1H), 5.93 (dd, J = 11.0, 2.7 Hz, 1H), 4.89 (s, 1H), 4.41 (d, *J =* 5.5 Hz, 2H). LCMS *m*/*z* 388.9 [M+1]⁺.

### Step 3. Synthesis of benzyl 4-(4-(benzylamino)-6-fluoro-1-(4-fluorophenyl)-2-(1-methoxy-2-methylpropan-2-yl)-1H-indol-3-yl)benzoate (C412)

A mixture of *N*¹-benzyl-2-bromo-5-fluoro-*N*³-(4-fluorophenyl)benzene-1,3-diamine **C411** (1.3 g, 3.2 mmol), benzyl 4-(4-methoxy-3,3-dimethylbut-1-yn-1-yl)benzoate **C335** (1.6 g, 4.8 mmol), and *N,N*-dicyclohexylmethylamine (1.7 mL, 8.0 mmol) in 1,4-dioxane (15 mL) was degassed with nitrogen for 10 minutes. Pd(t-Bu₃P)₂ (0.2 g, 0.4 mmol) was added, then the mixture was degassed with nitrogen for 5 minutes and stirred overnight at 105 °C. The reaction mixture was filtered through Celite^{®} and the filter pad was rinsed with EtOAc. The filtrate was concentrated to dryness and purified *via* silica gel chromatography eluting with CH₂Cl₂ in heptanes to afford 830 mg of product (41%). ¹H NMR (300 MHz, Chloroform-*d*) δ 7.99 (d, *J=* 8.2 Hz, 2H), 7.61 (d, *J=* 8.2 Hz, 2H), 7.56 - 7.34 (m, 7H), 7.23 (t, J = 8.5 Hz, 2H), 7.10 (dq, *J* = 14.4, 7.1 Hz, 3H), 6.94 (d, *J* = 6.9 Hz, 2H), 5.90 (dd, *J=* 12.3, 2.1 Hz, 1H), 5.68 (dd, J = 9.8, 2.1 Hz, 1H), 5.41 (s, 2H), 4.00 (d, *J* = 4.9 Hz, 2H), 3.55 (s, 1H), 3.08 (s, 3H), 2.95 (s, 2H), 1.01 (s, 6H). LCMS *m*/*z* 631.3 [M+1]⁺.

### Step 4. Synthesis of 4-(4-amino-6-fluoro-1-(4-fluorophenyl)-2-(1-methoxy-2-methylpropan-2-yl)-1H-indol-3-yl)benzoic acid (435)

To a solution of benzyl 4-(4-(benzylamino)-6-fluoro-1-(4-fluorophenyl)-2-(1-methoxy-2-methylpropan-2-yl)-1*H*-indol-3-yl)benzoate **C412** (0.83 g, 1.30 mmol) in EtOAc (10 mL) was added palladium on carbon (0.10 g, 0.10 mmol, 10% w/w). The reaction mixture was stirred overnight under 1 atmosphere of hydrogen. The mixture was filtered through Celite^{®} and resulting filtrate concentrated to dryness. The crude product was purified *via* reverse phase chromatography eluting with MeCN in water with 0.1% TFA. Pure fractions were combined, neutralized with saturated sodium bicarbonate solution, and extracted with EtOAc. The organic layer was dried over magnesium sulfate, filtered, and concentrated to afford 130 mg of product (21%). ¹H NMR (300 MHz, Methanol-*d*4) δ 8.09 (d, *J=* 8.2 Hz, 2H), 7.63 (d, *J=* 8.2 Hz, 2H), 7.45 (dd, *J* = 8.9, 5.0 Hz, 2H), 7.32 (t, *J =* 8.6 Hz, 2H), 5.97 (dd, *J =* 11.7, 2.2 Hz, 1H), 5.55 (dd, *J =* 9.8, 2.2 Hz, 1H), 3.09 (s, 3H), 2.98 (s, 2H), 1.04 (s, 6H). LCMS *m*/*z* 451.2 [M+1]⁺.

### Compound 436

### 4-(4-amino-2-(1-(cyanomethyl)cyclobutyl)-5-fluoro-1-(4-fluoro-3-methylphenyl)-1H-indol-3-yl)benzoic acid (436)

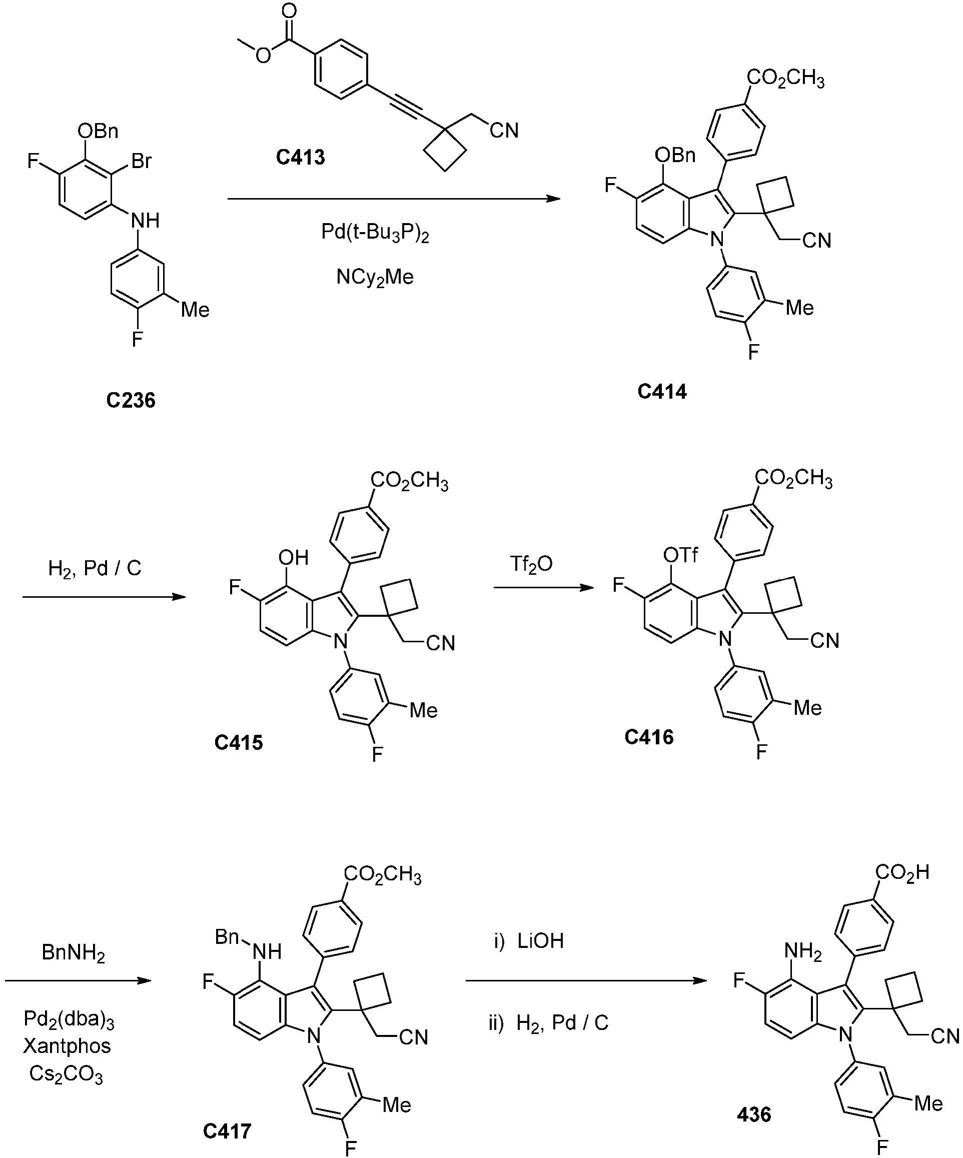

### Step 1. Synthesis of methyl 4-(4-(benzyloxy)-2-(1-(cyanomethyl)cyclobutyl)-5-fluoro-1-(4-fluoro-3-methylphenyl)-1H-indol-3-yl)benzoate (C414)

Compound **C413** was prepared from methyl 4-iodobenzoate by Sonagashira coupling as described for the synthesis of **C222.** A solution of 3-(benzyloxy)-2-bromo-4-fluoro-N-(4-fluoro-3-methylphenyl)aniline **C236** (0.26 g, 0.64 mmol), methyl 4-((1-(cyanomethyl)cyclobutyl)ethynyl)benzoate **C413** (0.15 g, 0.60 mmol), and *N,N-*dicyclohexylmethylamine (0.50 mL, 2.30 mmol) was degassed with nitrogen for 5 minutes. Pd(t-Bu₃P)₂ was added, then the mixture was degassed again with nitrogen for 5 minutes and stirred overnight at 105 °C. The reaction mixture was diluted with water and extracted with EtOAc. The organic layer was washed with brine, passed through a phase separator, and concentrated to dryness. The crude product was purified *via* silica gel chromatography eluting with EtOAc/heptanes to afford 296 mg of product (80%). LCMS *m*/*z* 577.3 [M+1]⁺.

### Step 2. Synthesis of methyl 4-(2-(1-(cyanomethyl)cyclobutyl)-5-fluoro-1-(4-fluoro-3-methylphenyl)-4-hydroxy-1H-indol-3-yl)benzoate (C415)

To a solution of methyl 4-(4-(benzyloxy)-2-(1-(cyanomethyl)cyclobutyl)-5-fluoro-1-(4-fluoro-3-methylphenyl)-1*H*-indol-3-yl)benzoate **C414** (0.30 g, 0.51 mmol) in EtOAc (2.5 mL) and EtOH (2.5 mL) was added palladium on carbon (0.04 g, 0.02 mmol, 5% wt/wt). The reaction mixture was stirred under 1 atm hydrogen for 48 hours then filtered through Celite^{®}. The filter pad was rinsed with EtOAc and EtOH, then the filtrate was concentrated to dryness to afford 230 mg of product (64%). LCMS *m*/*z* 487.2 [M+1]⁺.

### Step 3. Synthesis of methyl 4-(2-(1-(cyanomethyl)cyclobutyl)-5-fluoro-1-(4-fluoro-3-methylphenyl)-4-(((trifluoromethyl)sulfonyl)oxy)-1H-indol-3-yl)benzoate (C416)

To a cold (0 °C) solution of methyl 4-(2-(1-(cyanomethyl)cyclobutyl)-5-fluoro-1-(4-fluoro-3-methylphenyl)-4-hydroxy-1*H*-indol-3-yl)benzoate **C415** (0.23 g, 0.47 mmol) in CH₂Cl₂ (3.5 mL) and triethylamine (150 mL, 1.1 mmol) was added triflic anhydride (150 mL, 0.9 mmol). The reaction mixture was warmed to room temperature and stirred for 1 hour then diluted with CH₂Cl₂ and washed successively with aqueous saturated sodium bicarbonate solution and brine. The organic layer was passed through a phase separator, concentrated to dryness, then purified *via* silica gel chromatography eluting with EtOAc/heptanes to afford 154 mg of product (53%). LCMS *m*/*z* 619.2 [M+1]⁺.

### Step 4. Synthesis of methyl 4-(4-(benzylamino)-2-(1-(cyanomethyl)cyclobutyl)-5-fluoro-1-(4-fluoro-3-methylphenyl)-1H-indol-3-yl)benzoate (C417)

To a solution of methyl 4-(2-(1-(cyanomethyl)cyclobutyl)-5-fluoro-1-(4-fluoro-3-methylphenyl)-4-(((trifluoromethyl)sulfonyl)oxy)-1*H-*indol-3-yl)benzoate **C416** (0.154 g, 0.250 mmol), Pd₂(dba)₃ (0.002 g, 1.600 mmol), Xantphos (0.002 mg, 3.800 mmol), and cesium carbonate (0.110 g, 0.340 mmol) was added a solution of benzylamine (50 mL, 0.46 mmol) in 1,4-dioxane (1 mL). The reaction mixture was purged with nitrogen for 5 minutes then stirred overnight at 100 °C. The reaction mixture was diluted with CH₂Cl₂, filtered, concentrated, and purified *via* silica gel chromatography eluting with EtOAc in heptane to afford 43 mg of product (30%). LCMS *m*/*z* 576.4 [M+1]⁺.

### Step 5. Synthesis of 4-(4-amino-2-(1-(cyanomethyl)cyclobutyl)-5-fluoro-1-(4-fluoro-3-methylphenyl)-1H-indol-3-yl)benzoic acid (436)

To a solution of methyl 4-(4-(benzylamino)-2-(1-(cyanomethyl)cyclobutyl)-5-fluoro-1-(4-fluoro-3-methylphenyl)-1*H*-indol-3-yl)benzoate **C417** (0.043 g, 0.075 mmol) in THF (1 mL), water (0.5 mL), and MeOH (0.5 mL) was added lithium hydroxide (0.006 g, 0.250 mmol). The reaction mixture was stirred at room temperature for 1 hour then acidified with 6 M aq. HCl, extracted with EtOAc, filtered through a phase separator and concentrated to dryness. The resulting crude material was redissolved in EtOAc (1 mL) and EtOH (1 mL) then treated with palladium on carbon (10 mg, 0.005 mmol, 5% w/w) and stirred under 1 atm hydrogen for 30 minutes. The reaction mixture was filtered over Celite^{®}, concentrated to dryness, and purified via reverse phase chromatography eluting with MeCN in water with 0.1% TFA. Pure fractions were combined and concentrated to dryness to afford 14 mg of product (33%). ¹H NMR (400 MHz, Methanol-*d*4) δ 8.16 - 8.06 (m, 2H), 7.84 - 7.75 (m, 2H), 7.42 - 7.21 (m, 3H), 6.85 (dd, *J* = 11.3, 8.9 Hz, 1H), 6.16 (dd, *J =* 8.9, 3.7 Hz, 1H), 3.00 (s, 2H), 2.36 (s, 5H), 1.97 - 1.84 (m, 1H), 1.61 (dt, *J =* 11.5, 9.2 Hz, 1H), 1.47 - 1.38 (m, 2H). LCMS *m*/*z* 472.3 [M+1]⁺.

### Compound 437

### 4-(4-amino-1-(4-fluorophenyl)-2-isopropyl-1H-indol-3-yl)benzoic acid (437)

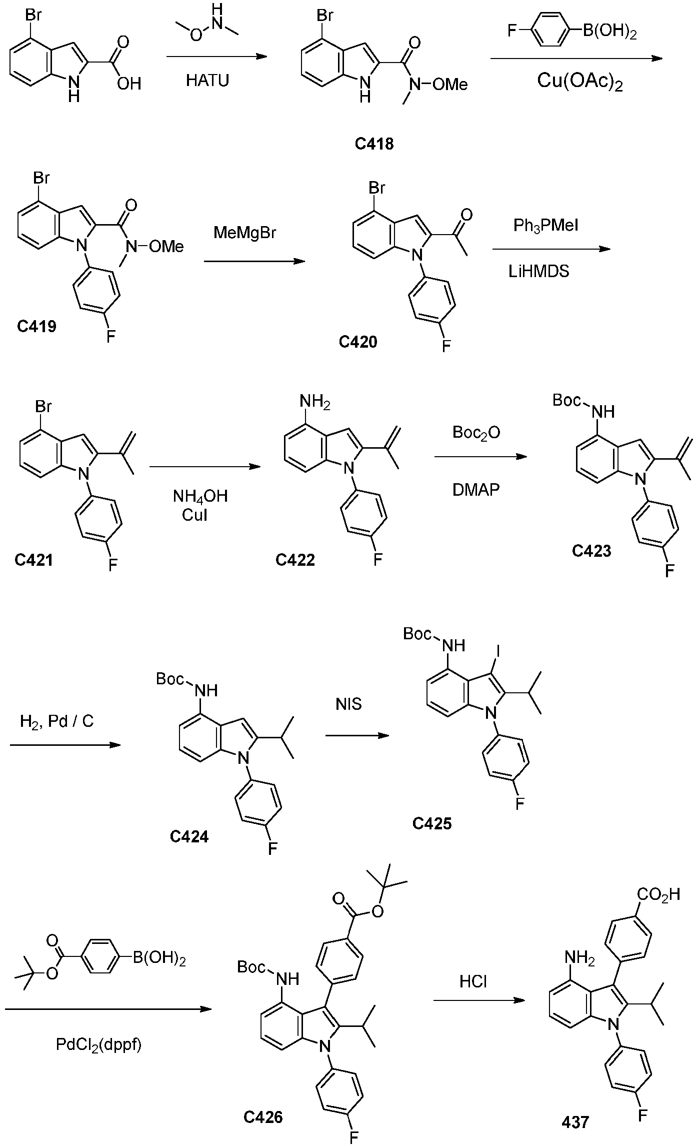

### Step 1. Synthesis of 4-bromo-N-methoxy-N-methyl-1H-indole-2-carboxamide (C418)

To a cold (10 °C) solution of 4-bromo-1*H*-indole-2-carboxylic acid (5.0 g, 20.8 mmol) in DMF (50 mL) was added *N,O*-dimethylhydroxylamine hydrochloride (2.8 g, 28.2 mmol) and HATU (9.5 g, 25.0 mmol) followed by slow addition of diisopropylethylamine (8.0 mL, 45.9 mmol). The reaction mixture was stirred overnight with gradual warming to room temperature. The mixture was diluted with water. The resulting white precipitate was filtered, washed with water, and dried overnight under vacuum at 40 °C to afford 5.6 g of product (90%). ¹H NMR (300 MHz, Chloroform-*d*) δ 9.43 (s, 1H), 7.40 (dt, *J =* 8.2, 0.9 Hz, 1H), 7.34 (dd, *J =* 7.6, 0.8 Hz, 1H), 7.26 (dd, *J =* 2.3, 1.0 Hz, 1H), 7.17 (dd, *J =* 8.2, 7.6 Hz, 1H), 3.90 (s, 3H), 3.46 (s, 3H).

### Step 2. Synthesis of 4-bromo-1-(4-fluorophenyl)-N-methoxy-N-methyl-1H-indole-2-carboxamide (C419)

To a suspension of 4 A molecular sieves (10 g) in CH₂Cl₂ (200 mL) was added 4-fluorophenylboronic acid (3.2 g, 22.9 mmol), 4-bromo-N-methoxy-N-methyl-1H-indole-2-carboxamide **C418** (1.6 g, 5.7 mmol), copper (II) acetate (1.5 g, 8.3 mmol) and pyridine (2.7 mL, 33.4 mmol). The reaction mixture was stirred for 5 hours at 40 °C then filtered. The filtrate was washed successively with water, saturated ammonium chloride, and brine then was dried over sodium sulfate, filtered, and concentrated to dryness. The crude material was purified *via* silica gel chromatography eluting with EtOAc in heptanes. Pure fractions were combined and concentrated to dryness (1.9 g, 89%). ¹H NMR (300 MHz, Chloroform-^{d}) δ 7.42 - 7.31 (m, 3H), 7.25 - 7.16 (m, 3H), 7.16 - 7.09 (m, 2H), 3.70 (s, 3H), 3.28 (s, 3H).

### Step 3. Synthesis of 1-(4-bromo-1-(4-fluorophenyl)-1H-indol-2-yl)ethan-1-one (C420)

To a cold (-20 °C) solution of 4-bromo-1-(4-fluorophenyl)-N-methoxy-N-methyl-1H-indole-2-carboxamide **C419** (1.8 g, 4.8 mmol) in THF (40 mL) under nitrogen was added methylmagnesium bromide (2.8 mL, 9.5 mmol, 3.4 M in THF). The reaction mixture was stirred for 90 minutes with gradual warming to 0 °C then quenched with 0.5 M aqueous HCl and extracted with EtOAc. The organic layer was washed successively with aqueous saturated sodium bicarbonate and brine then dried over sodium sulfate, filtered, and concentrated to dryness (1.65 g, 94%). ¹H NMR (400 MHz, Chloroform-*d*) δ 7.47 (d, *J =* 0.9 Hz, 1H), 7.41 (dd, *J =* 7.5, 0.8 Hz, 1H), 7.28 - 7.14 (m, 5H), 7.00 (dt, *J =* 8.4, 0.8 Hz, 1H), 2.63 (s, 3H).

### Step 4. Synthesis of 4-bromo-1-(4-fluorophenyl)-2-(prop-1-en-2-yl)-1H-indole (C421)

To a cold (-20 °C) solution of methyltriphenylphosphonium iodide (3.1 g, 7.7 mmol) in THF (65 mL) was added LiHMDS (7.0 mL, 7.0 mmol, 1 M in THF). The reaction mixture was stirred for 1 hour with gradual warming to 0 °C and then a solution of 1-(4-bromo-1-(4-fluorophenyl)-1*H*-indol-2-yl)ethan-1-one **C420** (1.6 g, 4.7 mmol) in THF (20 mL) was added *via* cannula. The reaction mixture was stirred at 0 °C for 3 hours, quenched with 0.5 M aqueous HCl, and extracted with diethyl ether. The organic layer was washed with brine, dried over sodium sulfate, filtered and concentrated to dryness. The crude residue was purified *via* silica gel chromatography eluting with EtOAc in heptanes to afford the desired product (1.33 g, 82%). ¹H NMR (400 MHz, Chloroform-*d*) δ 7.27 - 7.20 (m, 3H), 7.18 - 7.08 (m, 2H), 6.96 - 6.89 (m, 2H), 6.63 (s, 1H), 5.01 (t, *J* = 1.5 Hz, 1H), 4.77 (t, *J* = 1.1 Hz, 1H), 1.90 (dd, *J* = 1.5, 0.8 Hz, 3H).

### Step 5. Synthesis of 1-(4-fluorophenyl)-2-(prop-l-en-2-yl)-1H-indol-4-amine (C422)

To a suspension of 4-bromo-1-(4-fluorophenyl)-2-(prop-1-en-2-yl)-1*H*-indole *C421* (0.16 g, 0.47 mmol) in polyethylene glycol (1 mL) was added ammonium hydroxide (3.00 mL, 28% w/v) and DMSO (1.5 mL). Copper (I) iodide (0.01 g, 0.05 mmol) and *N,N'-*dimethylethylenediamine (15.00 mL, 0.14 mmol) were added and the reaction mixture was heated for 10 hours. The reaction mixture was diluted with water and extracted with EtOAc. The organic layer was washed with brine, dried over sodium sulfate, filtered, concentrated to dryness, and purified *via* silica gel chromatography eluting with EtOAc in heptanes. Pure fractions were combined and concentrated to afford the desired product (45 mg, 34%). ¹H NMR (300 MHz, Chloroform-*d*) δ 7.37 - 7.29 (m, 2H), 7.25 - 7.14 (m, 2H), 6.99 (dd, J = 8.3, 7.5 Hz, 1H), 6.60 (d, J = 0.8 Hz, 1H), 6.55 (dt, J = 8.2, 0.8 Hz, 1H), 6.45 (dd, J = 7.5, 0.8 Hz, 1H), 5.02 (p, J = 1.5 Hz, 1H), 4.79 (dd, J = 1.6, 0.8 Hz, 1H), 3.98 (s, 2H), 1.98 (dd, J = 1.5, 0.8 Hz, 3H).

### Step 6. Synthesis of tert-butyl (1-(4-fluorophenyl)-2-(prop-1-en-2-yl)-1H-indol-4-yl)carbamate (C423)

To a solution of 1-(4-fluorophenyl)-2-(prop-1-en-2-yl)-1*H*-indol-4-amine **C422** (0.04 g, 0.11 mmol) in EtOH (6 mL) was added Boc anhydride (0.06 g, 0.27 mmol) and DMAP (0.002 g, 0.020 mmol). The reaction mixture was concentrated to dryness and purified *via* silica gel chromatography eluting with EtOAc in heptanes. Pure fractions were combined and concentrated to afford the desired product (35 mg, 96%). ¹H NMR (300 MHz, Chloroform-d) δ 7.63 (d, J = 7.8 Hz, 1H), 7.40 - 7.29 (m, 2H), 7.26 - 7.17 (m, 2H), 7.13 (t, J = 8.0 Hz, 1H), 6.81 (dt, J = 8.3, 0.9 Hz, 1H), 6.72 (s, 1H), 6.61 (d, J = 0.8 Hz, 1H), 5.11 - 5.02 (m, 1H), 4.87 - 4.81 (m, 1H), 1.98 (dd, J = 1.5, 0.8 Hz, 3H), 1.59 (s, 9H).

### Step 7. Synthesis of tert-butyl (1-(4-fluorophenyl)-2-isopropyl-1H-indol-4-yl)carbamate (C424)

To a slurry of palladium on carbon (0.005 g, 0.050 mmol, 10% w/w) in EtOAc (15 mL) was added *tert*-butyl (1-(4-fluorophenyl)-2-(prop-1-en-2-yl)-1*H*-indol-4-yl)carbamate **C423** (0.042 g, 0.110 mmol). The reaction mixture was stirred under 1 atm hydrogen for 30 minutes then filtered through Celite^{®} and concentrated to dryness (37 mg, 86%). ¹H NMR (400 MHz, Chloroform-*d*) δ 7.52 (s, 1H), 7.27 - 7.19 (m, 2H), 7.19 - 7.09 (m, 2H), 6.97 (t, *J =* 8.0 Hz, 1H), 6.68 - 6.56 (m, 2H), 6.28 (d, J = 0.9 Hz, 1H), 2.83 (pd, J = 6.8, 0.7 Hz, 1H), 1.49 (s, 9H), 1.12 (d, *J =* 6.8 Hz, 6H).

### Step 8. Synthesis of tert-butyl (1-(4-fluorophenyl)-3-iodo-2-isopropyl-1H-indol-4-yl) carbamate (C425)

To a cold (0 °C) solution of tert-butyl (1-(4-fluorophenyl)-2-isopropyl-1*H*-indol-4-yl)carbamate **C424** (0.037 g, 0.100 mmol) in CH₂Cl₂ (5 mL) was added *N*-iodosuccinimide (0.035 g, 0.150 mmol). The reaction mixture was diluted with CH₂Cl₂ then washed successively with aqueous sodium thiosulfate and brine, then dried over sodium sulfate, filtered, and concentrated to dryness. The crude residue was purified *via* silica gel chromatography eluting with EtOAc in heptanes. Pure fractions were combined and concentrated to afford the desired product (42 mg, 72%). ¹H NMR (400 MHz, Chloroform-d) δ 8.15 (d, *J =* 51.3 Hz, 1H), 7.71 (dd, *J =* 43.3, 7.9 Hz, 1H), 7.32 - 7.21 (m, 4H), 7.07 (t, *J =* 8.1 Hz, 1H), 6.57 (d, *J =* 8.2 Hz, 1H), 3.38 - 2.96 (m, 1H), 1.60 (d, *J =* 1.9 Hz, 9H), 1.31 (dd, *J =* 19.9, 7.2 Hz, 6H).

### Step 9. Synthesis of tert-butyl 4-(4-((tert-butoxycarbonyl)amino)-1-(4-fluorophenyl)-2-isopropyl-1H-indol-3-yl)benzoate (C426)

To tert-butyl (1-(4-fluorophenyl)-3-iodo-2-isopropyl-1*H*-indol-4-yl)carbamate **C425** (0.041 g, 0.080 mmol) in water (2 mL) was added sodium carbonate (0.090 g, 0.850 mmol), DME (6 mL), (4-(*tert*-butoxycarbonyl)phenyl)boronic acid (0.028 g, 0.120 mmol), and PdCl₂(dppf) (0.008 g, 0.010 mmol). The reaction mixture was purged with nitrogen for 15 minutes then heated for 3 hours at 100 °C. The reaction mixture was diluted with aqueous ammonium chloride and water then extracted with diethyl ether. The organic layer was washed with brine, dried over sodium sulfate, filtered, and concentrated to dryness. The crude residue was purified *via* silica gel chromatography eluting with EtOAc in heptanes. Pure fractions of two isomers were combined and concentrated to afford the desired product (41 mg, 77%).

Peak A: ¹H NMR (400 MHz, Chloroform-*d*) δ 8.13 - 7.90 (m, 2H), 7.63 - 7.38 (m, 3H), 7.33 - 7.27 (m, 1H), 7.22 - 7.15 (m, 2H), 7.04 - 6.88 (m, 1H), 6.65 - 6.42 (m, 1H), 6.14 (d, J = 116.3 Hz, 1H), 3.07 - 2.77 (m, 1H), 1.55 (d, J = 2.1 Hz, 9H), 1.26 (s, 9H), 1.05 (dd, J = 63.0, 7.0 Hz, 6H). ¹⁹ F NMR (376 MHz, Chloroform-*d*) δ -112.34.

Peak B: ¹H NMR (400 MHz, Chloroform-d) δ 8.09 - 8.01 (m, 2H), 7.52 - 7.42 (m, 3H), 7.32 - 7.29 (m, 1H), 6.99 (t, J = 8.0 Hz, 1H), 6.63 - 6.46 (m, 1H), 6.1 4 (d, J = 116.3 Hz, 1H), 2.88 (p, J = 7.1 Hz, 1H), 1.55 (s, 9H), 1.50 (s, 1H), 1.26 (s, 10H), 0.97 (d, J = 7.2 Hz, 7H). 19 F NMR (376 MHz, Chloroform-*d*) δ -112.34.

### Step 10. Synthesis of 4-(4-amino-1-(4-fluorophenyl)-2-isopropyl-1H-indol-3-yl)benzoic acid (437)

To a cold (-10 °C) solution of *tert*-butyl 4-(4-((tert-butoxycarbonyl)amino)-1-(4-fluorophenyl)-2-isopropyl-1*H*-indol-3-yl)benzoate **C426** (0.048 g, 0.090 mmol) in diethyl ether (3 mL) was added dropwise HCl (4.0 mL, 16.0 mmol, 4 M in 1,4-dioxane). The reaction mixture was heated for 4 hours at 60 °C then concentrated to dryness and purified via reverse phase chromatography eluting with MeCN in water with 0.2% formic acid. Pure fractions were combined, diluted with water, and extracted with CH₂Cl₂. The organic layer was dried over sodium sulfate, filtered, and concentrated to dryness to afford the title compound as a light yellow solid (10 mg, 28%). ¹H NMR (400 MHz, Chloroform-*d*) δ 8.16 - 8.06 (m, 2H), 7.64 - 7.53 (m, 2H), 7.37 - 7.28 (m, 2H), 7.16 (d, *J =* 8.6 Hz, 2H), 6.85 (t, *J =* 7.9 Hz, 1H), 6.24 (d, *J =* 7.9 Hz, 2H), 2.89 (p, *J =* 7.1 Hz, 1H), 0.96 (d, *J =* 7.1 Hz, 6H). LCMS *m*/*z* 389.3 [M+1]⁺.

### Compounds 438-457

Compounds **438-457** were prepared in a single step by Larock indole cyclization using the corresponding alkyne and bromo-amines, as described for the preparation of compound **C225.** *N*-cyclohexyl-*N*-methyl-cyclohexanamine and Pd(*t*Bu₃P)₂ were typically used as the catalyst system for this transformation.

**Table 24. Structure and physicochemical data for compounds 438-457**

| **Compound** | **Structure** | ¹H NMR; **LCMS m/z** [M+H]⁺ |
|---|---|---|
| **438** | 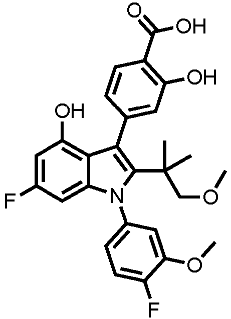 | ¹H NMR (400 MHz, DMSO-*d*6) δ 11.22 (s, 1H), 9.43 (s, 1H), 7.76 - 7.68 (m, 1H), 7.42 (ddd, *J=* 11.3, 8.5, 1.0 Hz, 1H), 7.27 (ddd, *J=* 8.1, 5.9, 2.4 Hz, 1H), 7.04 (dt, *J=* 6.6, 4.2 Hz, 1H), 6.98 - 6.86 (m, 2H), 6.12 (dd, *J=* 11.4, 2.2 Hz, 1H), 5.78 (dd, *J=* 9.8, 2.2 Hz, 1H), 3.87 (d, *J=* 1.8 Hz, 3H), 3.04 (s, 3H), 3.02 - 2.92 (m, 2H), 1.02 (d, *J=* 1.9 Hz, 6H). LCMS *m*/*z* 498.25 [M+H]⁺. |
| **439** | 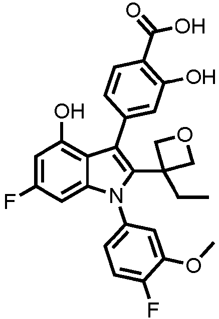 | ¹H NMR (400 MHz, DMSO-d6) δ 9.79 (s, 1H), 7.75 (d, *J=* 8.1 Hz, 1H), 7.40 (dd, *J=* 11.2, 8.5 Hz, 1H), 7.33 (dd, *J=* 7.8, 2.5 Hz, 1H), 7.05 (ddd, *J=* 8.5, 3.9, 2.5 Hz, 1H), 6.96 - 6.86 (m, 2H), 6.26 (dd, *J=* 11.5, 2.2 Hz, 1H), 5.95 (dd, *J=* 9.6, 2.2 Hz, 1H), 4.63 (d, *J=* 5.8 Hz, 1H), 4.47 (d, *J=* 5.9 Hz, 1H), 3.85 (s, 3H), 3.67 (dd, *J=* 12.8, 5.8 Hz, 2H), 1.04 (t, *J=* 7.4 Hz, 3H), 0.88 - 0.81 (m, 2H). LCMS *m*/*z* 496.22 [M+H]⁺. |
| **440¹** | 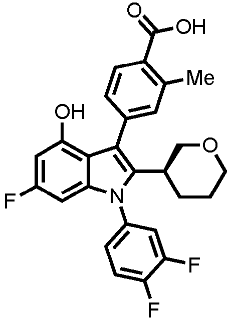 | 1H NMR (400 MHz, DMSO-d6) δ 9.69 (s, 1H), 7.89 - 7.64 (m, 3H), 7.44 - 7.21 (m, 2H), 6.23 (dd, J = 11.4, 2.2 Hz, 1H), 6.07 (dt, J = 9.6, 2.5 Hz, 1H), 3.84 - 3.49 (m, 2H), 3.14 (td, J = 11.1, 1.8 Hz, 1H), 2.91 - 2.67 (m, 2H), 2.58 (s, 3H), 1.87 - 1.70 (m, 1H), 1.44 - 1.18 (m, 3H). LCMS *m*/*z* 482.2 [M+H]⁺. |
| **441¹** | 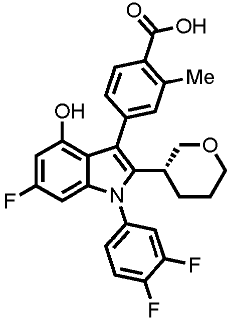 | 1H NMR (400 MHz, DMSO-d6) δ 9.69 (s, 1H), 7.85 (d, J = 7.9 Hz, 1H), 7.82 - 7.66 (m, 2H), 7.45 - 7.34 (m, 1H), 7.33 - 7.23 (m, 2H), 6.23 (dd, J = 11.4, 2.2 Hz, 1H), 6.07 (dt, J = 9.6, 2.5 Hz, 1H), 3.81 - 3.58 (m, 2H), 3.14 (td, J = 11.1, 1.8 Hz, 1H), 2.91 - 2.67 (m, 2H), 2.58 (s, 3H), 1.84 - 1.71 (m, 1H), 1.46 - 1.14 (m, 3H). LCMS *m*/*z* 482.2 [M+H]⁺. |
| **442** | 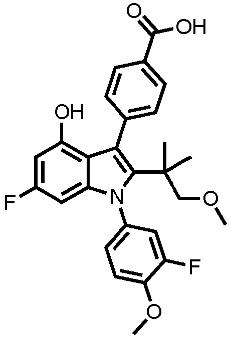 | ¹H NMR (400 MHz, DMSO-d6) δ 9.41 (s, 1H), 7.90 - 7.84 (m, 2H), 7.48 - 7.32 (m, 4H), 7.26 (ddd, *J*= 8.8, 2.5, 1.2 Hz, 1H), 6.11 (dd, *J=* 11.4, 2.2 Hz, 1H), 5.74 (dd, *J=* 9.8, 2.2 Hz, 1H), 3.96 (s, 3H), 3.00 (s, 3H), 2.99 - 2.87 (m, 2H), 0.97 (d, *J=* 2.8 Hz, 6H). LCMS *m*/*z* 482.16 [M+H]⁺. |
| **443** | 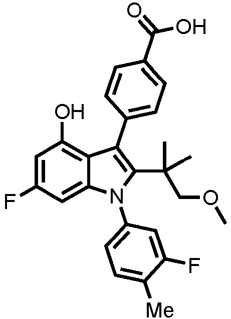 | ¹H NMR (400 MHz, DMSO-d6) δ 9.42 (s, 1H), 7.94 - 7.83 (m, 2H), 7.56 - 7.43 (m, 3H), 7.33 (dd, *J=* 10.3, 2.1 Hz, 1H), 7.22 (dd, *J=* 8.0, 2.0 Hz, 1H), 6.64 (d, *J=* 8.3 Hz, 0H), 6.11 (dd, *J=* 11.4, 2.2 Hz, 1H), 5.73 (dd, *J=* 9.8, 2.2 Hz, 1H), 3.00 (s, 3H), 2.98 - 2.89 (m, 2H), 2.37 (d, *J=* 1.8 Hz, 3H), 0.97 (s, 6H). LCMS *m*/*z* 466.13 [M+H]⁺. |
| **444** | 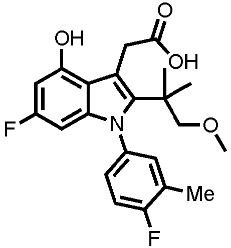 | ¹H NMR (400 MHz, DMSO-*d*6) δ 11.98 (s, 1H), 10.01 (s, 1H), 7.36 - 7.22 (m, 2H), 7.19 (ddd, *J=* 8.1, 4.6, 2.7 Hz, 1H), 6.17 (dd, *J=* 11.4, 2.2 Hz, 1H), 5.62 (dd, *J=* 9.8, 2.2 Hz, 1H), 4.11 (s, 2H), 3.13 (s, 2H), 3.12 (s, 3H), 2.30 (d, *J=* 1.9 Hz, 3H), 1.18 (d, *J=* 6.0 Hz, 6H). LCMS *m*/*z* 404.18 [M+H]⁺. |
| **445** | 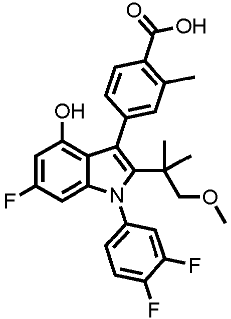 | ¹H NMR (400 MHz, DMSO-*d*6) δ 12.72 (s, 1H), 9.44 (s, 1H), 7.80 (d, *J =* 8.1 Hz, 1H), 7.74 - 7.53 (m, 2H), 7.43 - 7.32 (m, 1H), 7.32 - 7.21 (m, 2H), 6.24 - 6.02 (m, 1H), 5.80 (ddd, *J =* 9.7, 2.2, 0.8 Hz, 1H), 3.00 (d, *J =* 1.0 Hz, 3H), 2.97 - 2.82 (m, 2H), 2.55 (s, 3H), 0.98 (d, *J =* 3.2 Hz, 6H). ESI-MS *m*/*z* calc. 483.2, found 484.3 (M+1)⁺ |
| **446** | 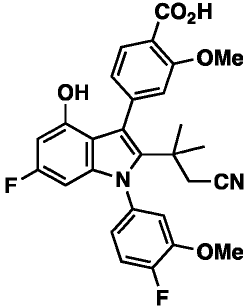 | ¹H NMR (400 MHz, DMSO-*d*6) δ 12.54 (s, 1H), 9.56 (s, 1H), 7.64 (d, *J =* 7.8 Hz, 1H), 7.47 (dd, *J =* 11.2, 8.5 Hz, 1H), 7.31 (ddd, *J =* 21.7, 7.8, 2.5 Hz, 1H), 7.18 - 7.02 (m, 3H), 6.16 (dd, *J* = 11.4, 2.2 Hz, 1H), 5.84 (dt, *J =* 9.7, 2.1 Hz, 1H), 3.88 (d, J = 1.1 Hz, 3H), 3.81 (s, 3H), 2.53 (d, *J* = 6.8 Hz, 2H), 1.21 - 1.15 (m, 6H). LCMS *m*/*z* 507 [M+H]⁺ |
| **447** | 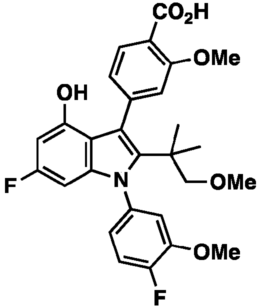 | ¹H NMR (400 MHz, DMSO-*d*6) δ 12.46 (s, 1H), 9.41 (d, *J =* 1.2 Hz, 1H), 7.61 (d, *J =* 7.8 Hz, 1H), 7.43 (ddd, *J* = 11.2, 8.5, 1.0 Hz, 1H), 7.25 (ddd, *J =* 24.7, 7.8, 2.5 Hz, 1H), 7.12 - 6.97 (m, 3H), 6.13 (dd, J = 11.4, 2.2 Hz, 1H), 5.79 (ddd, *J =* 9.8, 2.3, 1.3 Hz, 1H), 3.87 (s, 3H), 3.80 (d, *J* = 0.9 Hz, 3H), 3.04 (s, 3H), 3.00 (d, *J* = 2.4 Hz, 2H), 1.06 - 0.98 (m, 6H). LCMS *m*/*z* 512 [M+H]⁺ |
| **448** | 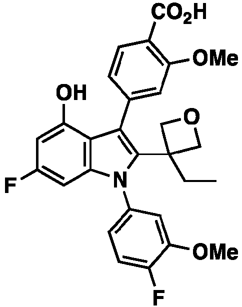 | ¹H NMR (400 MHz, DMSO-*d*6) δ 12.52 (s, 1H), 9.79 (s, 1H), 7.64 (d, *J =* 7.9 Hz, 1H), 7.41 (dd, *J =* 11.2, 8.5 Hz, 1H), 7.31 (dd, *J =* 7.8, 2.5 Hz, 1H), 7.09 - 7.01 (m, 2H), 6.97 (dd, *J =* 7.9, 1.4 Hz, 1H), 6.27 (dd, J = 11.4, 2.2 Hz, 1H), 5.96 (dd, *J* = 9.5, 2.2 Hz, 1H), 4.61 (d, *J =* 5.7 Hz, 1H), 4.45 (d, *J =* 5.9 Hz, 1H), 3.85 (m, 6H), 3.69 (dd, *J =* 13.3, 5.8 Hz, 2H), 2.06 - 1.97 (m, 2H), 1.07 (t, *J =* 7.4 Hz, 3H). LCMS *m*/*z* 510 [M+H]⁺ |
| **449** | 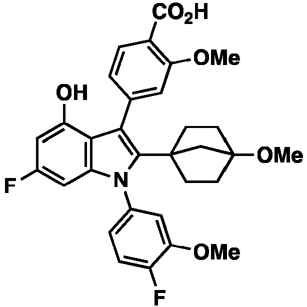 | ¹H NMR (400 MHz, DMSO-*d*6) δ 12.50 (s, 1H), 9.50 (d, *J =* 1.7 Hz, 1H), 7.62 (d, *J* = 7.8 Hz, 1H), 7.42 (dd, *J =* 11.2, 8.6 Hz, 1H), 7.28 (ddd, *J =* 23.2, 7.8, 2.4 Hz, 1H), 7.12 - 6.98 (m, 3H), 6.17 (dd, *J* = 11.4, 2.2 Hz, 1H), 5.91 (dt, J = 9.7, 2.0 Hz, 1H), 3.86 (s, 3H), 3.81 (d, *J =* 1.4 Hz, 3H), 2.96 (s, 3H), 1.89 (t, *J =* 11.7 Hz, 2H), 1.54 - 1.13 (m, 8H). LCMS *m*/*z* 550 [M+H]⁺ |
| **450** | 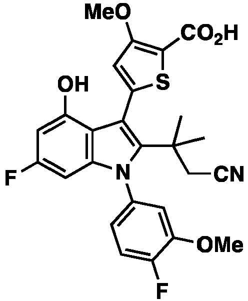 | ¹H NMR (400 MHz, DMSO-*d*6) δ 12.78 (s, 1H), 9.73 (d, *J* = 1.6 Hz, 1H), 7.55 (d, *J =* 13.8 Hz, 1H), 7.47 (ddd, *J =* 11.2, 9.9, 8.5 Hz, 1H), 7.29 (ddd, J = 58.8, 7.7, 2.5 Hz, 1H), 7.08 (dddd, *J =* 58.4, 8.5, 3.9, 2.5 Hz, 1H), 6.17 (ddd, *J =* 11.3, 2.1, 0.9 Hz, 1H), 5.81 (ddd, *J =* 9.6, 8.8, 2.2 Hz, 1H), 3.88 (d, J = 15.9 Hz, 3H), 3.78 (d, *J =* 7.8 Hz, 3H), 2.63 - 2.54 (m, 2H), 1.19 (dd, *J =* 6.9, 5.8 Hz, 6H). LCMS *m*/*z* 513 [M+H]⁺ |
| **451** | 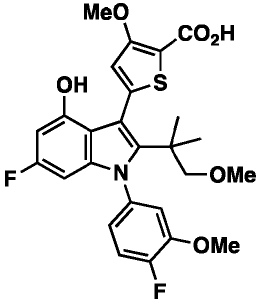 | ¹H NMR (400 MHz, DMSO-*d*6) δ 12.68 (s, 1H), 9.59 (d, *J =* 1.6 Hz, 1H), 7.48 (d, *J =* 15.1 Hz, 1H), 7.45 - 7.37 (m, 1H), 7.24 (ddd, *J =* 58.9, 7.8, 2.5 Hz, 1H), 7.01 (dddd, *J =* 60.7, 8.5, 4.0, 2.4 Hz, 1H), 6.13 (ddd, *J =* 11.5, 2.2, 0.9 Hz, 1H), 5.77 (ddd, *J =* 9.7, 7.4, 2.2 Hz, 1H), 3.86 (d, *J* = 16.5 Hz, 3H), 3.78 (d, *J =* 6.6 Hz, 3H), 3.06 (d, *J =* 3.1 Hz, 3H), 3.05 - 3.01 (m, 2H), 1.13 - 1.00 (m, 6H). LCMS *m*/*z* 518 [M+H]⁺ |
| **452** | 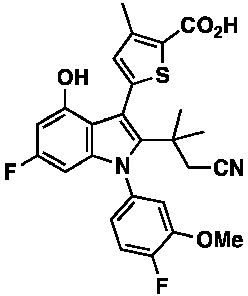 | ¹H NMR (400 MHz, DMSO-*d*6) δ 12.82 (s, 1H), 9.75 (s, 1H), 7.46 (dd, *J =* 11.2, 8.5 Hz, 1H), 7.34 (s, 1H), 7.09 (d, *J =* 10.1 Hz, 1H), 6.99 (s, 1H), 2.51 - 2.50 (m, 3H), 6.20 (dd, *J =* 11.5, 2.2 Hz, 1H), 5.83 (dd, *J* = 9.6, 2.2 Hz, 1H), 3.87 (s, 3H), 2.62 (d, *J =* 2.5 Hz, 2H), 1.24 (d, J = 2.4 Hz, 6H). LCMS *m*/*z* 497 [M+H]⁺ |
| **453** | 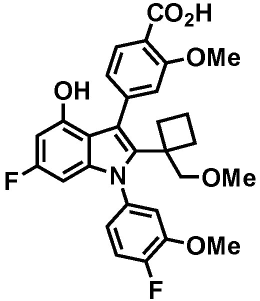 | ¹H NMR (400 MHz, Chloroform-*d*) δ 10.70 (s, 1H), 8.27 (d, J = 8.0 Hz, 1H), 7.68 (s, 1H), 7.44 (d, J = 7.9 Hz, 1H), 7.23 (dd, J = 10.8, 8.5 Hz, 1H), 7.03 (d, J = 24.1 Hz, 2H), 6.39 (dd, J = 10.8, 2.2 Hz, 1H), 6.09 (dd, J = 9.3, 2.2 Hz, 1H), 5.28 (d, J = 32.1 Hz, 1H), 4.14 (s, 3H), 3.92 (s, 3H), 3.76 - 3.66 (m, 2H), 3.44 (s, 3H), 2.18 (s, 1H), 2.01 - 1.88 (m, 1H), 1.88 - 1.74 (m, 1H), 1.74 - 1.53 (m, 2H), 1.50 - 1.34 (m, 1H). LCMS *m*/*z* 524.17 [M+1]⁺. |
| **454** | 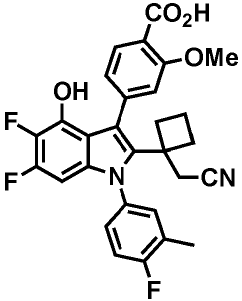 | ¹H NMR (400 MHz, Chloroform-*d*) δ 8.10 (d, J = 7.9 Hz, 1H), 7.39 (d, J = 1.4 Hz, 1H), 7.27 (dd, J = 8.0, 1.4 Hz, 1H), 7.16 (d, J = 32.5 Hz, 3H), 5.96 (dd, J = 10.2, 5.8 Hz, 1H), 3.92 (s, 3H), 2.77 (d, J *=* 14.5 Hz, 2H), 2.29 (s, 3H), 2.22 - 2.14 (m, 1H), 2.13 (d, J = 17.3 Hz, 1H), 1.83 - 1.73 (m, 1H), 1.60 (q, J= 10.2, 9.6 Hz, 1H), 1.52 - 1.28 (m, 2H). LCMS *m*/*z* 521.2 [M+1]^{+.} |
| **455** | 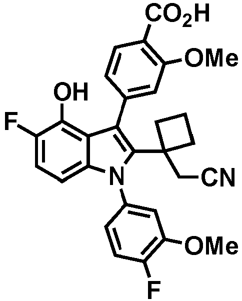 | ¹H NMR (400 MHz, Chloroform-d) δ 10.68 (s, 1H), 8.14 (d, J = 8.0 Hz, 1H), 7.32 (dd, J = 8.0, 1.5 Hz, 1H), 7.22 - 7.12 (m, 1H), 6.94 (ddd, J = 9.6, 7.6, 3.1 Hz, 2H), 6.85 (dd, J = 10.7, 8.9 Hz, 1H), 6.16 (dd, J = 8.9, 3.6 Hz, 1H), 4.03 (s, 3H), 3.83 (s, 3H), 2.81 (s, 2H), 2.24 - 2.12 (m, 1H), 1.89 - 1.72 (m, 2H), 1.62 (dtt, J = 11.3, 9.2, 2.0 Hz, 1H), 1.51 (s, 3H). LCMS *m*/*z* 519.2 [M+1]⁺. |
| **456** | 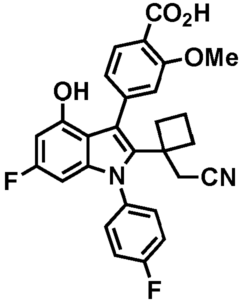 | ¹H NMR (400 MHz, Chloroform-*d*) δ 8.18 (d, *J* = 7.9 Hz, 1H), 7.53 (d, *J =* 1.4 Hz, 1H), 7.34 (dt, *J* = 8.5, 2.9 Hz, 3H), 7.19 (s, 2H), 6.28 (dd,*J* = 10.8, 2.2 Hz, 1H), 5.92 (dd, *J=* 9.2, 2.1 Hz, 1H), 4.05 (s, 3H), 2.89 - 2.61 (m, 2H), 2.30 (d, *J*= 11.0 Hz, 1H), 2.12 (q, *J =* 10.0, 8.9 Hz, 1H), 1.91 - 1.80 (m, 2H), 1.70 - 1.56 (m, 2H). LCMS *m*/*z* 489.2 [M+H]⁺ |
| **457** | 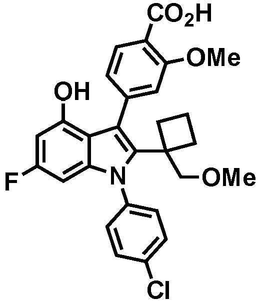 | ¹H NMR (400 MHz, Chloroform-*d*) δ 10.46 (s, 1H), 8.17 (d, J = 7.9 Hz, 1H), 7.59 (d, J = 1.4 Hz, 1H), 7.48 - 7.40 (m, 2H), 7.35 (dd, J = 8.0, 1.5 Hz, 1H), 7.27 (s, 2H), 6.28 (dd, J = 10.9, 2.2 Hz, 1H), 5.94 (dd, J = 9.3, 2.1 Hz, 1H), 5.13 (s, 1H), 4.05 (s, 3H), 3.57 (d, J *=* 11.9 Hz, 2H), 3.33 (s, 3H), 2.16 - 1.94 (m, 2H), 1.90 - 1.77 (m, 1H), 1.77 - 1.67 (m, 1H), 1.50 (dt, J = 11.3, 9.3 Hz, 2H). LCMS *m*/*z* 510.1 [M+H]⁺ |

| | | |
|---|---|---|
| **^{1.}** Final compound is single stereoisomer of unknown absolute configuration | | |

### Preparation of C427-C439

Intermediates **C427-C439** (Table **24**) were prepared from corresponding methyl 4-bromobenzoates and alkynes by Sonagashira coupling as described for the synthesis of **C222.**

**Table 25. Method of preparation, structure, physicochemical data for compounds C427-C439**

| **Compound** | **Structure** | ¹H NMR; **LCMS m/z** [M+H]⁺ |
|---|---|---|
| **C427** | 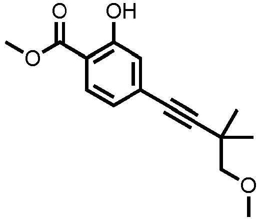 | LCMS m/z 263.23 [M+H]⁺. |
| **C428** | 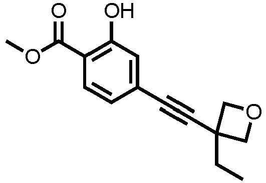 | LCMS m/z 261.16 [M+H]⁺. |
| **C429^{1,2}** | 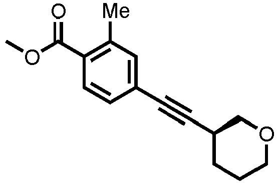 | LCMS m/z 259.27 [M+H]⁺. |
| **C430^{1,2}** | 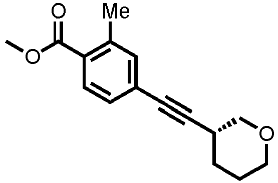 | LCMS m/z 259.27 [M+H]⁺. |
| **C431** | 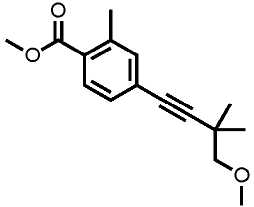 | LCMS m/z 261.25 [M+H]⁺. |
| **C432** | 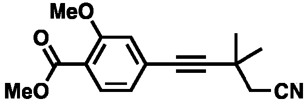 | ¹H NMR (400 MHz, Chloroform-d) δ 7.76 (d, J = 8.0 Hz, 1H), 7.04 (dd, J = 8.0, 1.4 Hz, 1H), 7.01 (d, J = 1.3 Hz, 1H), 3.93 (s, 3H), 3.90 (s, 3H), 2.64 (s, 2H), 1.52 (s, 6H). |
| **C433** | 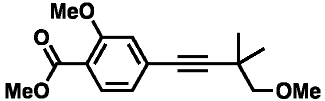 | ¹H NMR (400 MHz, Chloroform-d) δ 7.75 (d, J = 7.9 Hz, 1H), 7.03 (dd, J = 7.9, 1.4 Hz, 1H), 7.01 (d, J = 1.4 Hz, 1H), 3.92 (s, 3H), 3.90 (s, 3H), 3.47 (s, 3H), 3.37 (s, 2H), 1.34 (s, 6H). |
| **C434** | 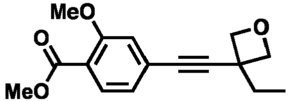 | ¹H NMR (400 MHz, Chloroform-d) δ 7.77 (d, J = 8.0 Hz, 1H), 7.06 (dd, J = 7.9, 1.4 Hz, 1H), 7.03 (d, J = 1.3 Hz, 1H), 4.90 (d, J = 5.5 Hz, 2H), 4.52 (d, J = 5.5 Hz, 2H), 3.94 (s, 3H), 3.91 (s, 3H), 2.06 (q, J = 7.4 Hz, 2H), 1.10 (t, J = 7.4 Hz, 3H). |
| **C435** | 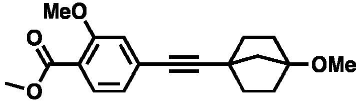 | ¹H NMR (400 MHz, Chloroform-d) δ 7.75 - 7.70 (m, 1H), 7.02 - 6.97 (m, 2H), 3.90 (s, 3H), 3.88 (s, 3H), 3.33 (s, 3H), 2.06 (td, J = 12.6, 5.1 Hz, 2H), 1.90 - 1.83 (m, 6H), 1.64 (dt, J = 12.8, 8.1 Hz, 2H). |
| **C436** | 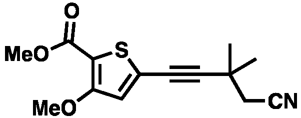 | ¹H NMR (400 MHz, Chloroform-d) δ 7.44 (s, 1H), 4.10 (s, 3H), 3.86 (s, 3H), 2.59 (s, 2H), 1.48 (s, 6H). |
| **C437** | 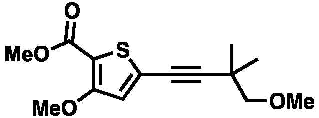 | ¹H NMR (400 MHz, Chloroform-d) δ 7.39 (s, 1H), 4.11 (s, 3H), 3.85 (s, 3H), 3.42 (s, 3H), 3.32 (s, 2H), 1.29 (s, 6H). |
| **C438** | 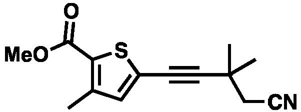 | ¹H NMR (400 MHz, Chloroform-d) δ 6.98 (d, J = 0.5 Hz, 1H), 3.87 (s, 3H), 2.62 (s, 2H), 2.51 (s, 3H), 1.50 (s, 6H). |
| **C439** | 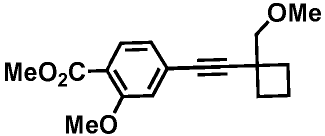 | ¹H NMR (400 MHz, Chloroform-d) δ 7.75 (d, J = 7.9 Hz, 1H), 7.05 (d, J = 9.2 Hz, 2H), 3.92 (d, J = 1.6 Hz, 3H), 3.91 - 3.85 (m, 3H), 3.55 (s, 2H), 3.52 - 3.45 (m, 3H), 2.38 (q, J = 9.4, 8.8 Hz, 2H), 2.25 (q, J = 10.7, 9.7 Hz, 2H), 2.11 (ddt, J = 18.8, 8.5, 4.8 Hz, 1H), 1.99 (h, J *=* 8.1 Hz, 1H) |

| | | |
|---|---|---|
| **^{1.}** SFC chiral separation to obtain individual stereoisomer. **^{2.}** Final compound is single stereoisomer of unknown absolute configuration | | |

### Preparation of C440

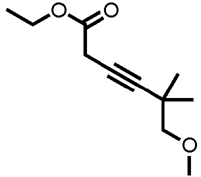

### Synthesis of ethyl 6-methoxy-3,5-dimethyl-hex-3-ynoate (C440)

To a solution of CuI (0.09 g, 0.46 mmol) in acetonitrile (10.0 mL) under nitrogen atmosphere at ambient temperature was added 4-methoxy-3,3-dimethyl-but-1-yne (2.80 g, 23.71 mmol) in a single charge. The solution was stirred for 5 minutes and ethyl 2-diazoacetate (1.00 mL, 9.51 mmol) (w/v ≥13% dichloromethane) was added dropwise and the solution was stirred at ambient temperature for 16 hours. The crude reaction mixture was filtered through Florisil, washed with dichloromethane, and the filtrate was concentrated *in vacuo.* Purification by silica gel chromatography (0-25% *tert*-butyl methyl ether in heptanes) afforded 1.02 g of product (51%). ¹H NMR (400 MHz, CDCl₃) δ 4.16 (q, *J=* 7.1 Hz, 2H), 3.40 (s, 3H), 3.24 (apparent d, 4H), 1.26 (t, *J=* 7.1 Hz, 3H), 1.20 (s, 6H).

### Compounds 458-514

Compounds **458-514** were prepared from the corresponding halo-anilines and alkynes by Larock cyclization as for compound **146.** Halo-anilines were prepared via Buchwald amination of the corresponding di-halo anilines. The disubstituted alkynes in Table 26 were prepared via Sonogashira coupling between the corresponding aryl halide and alkyne.

**Table 26. Structure and physicochemical data for compounds 458-514**

| **Compound** | **Structure** | ¹H NMR; **LCMS m/z** [M+H]⁺ |
|---|---|---|
| **458** | 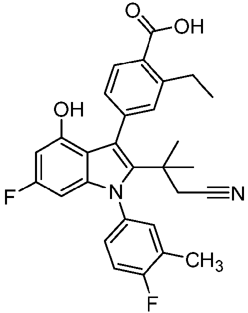 | ¹H NMR (400 MHz, Chloroform-d) δ 8.09 (d, J = 7.9 Hz, 1H), 7.56 (d, J = 1.6 Hz, 1H), 7.49 (dt, J = 8.0, 1.6 Hz, 1H), 7.22 (td, J = 8.5, 7.6, 3.0 Hz, 2H), 7.14 (t, J = 8.5 Hz, 1H), 6.21 (dd, J = 10.9, 2.1 Hz, 1H), 5.84 (dd, J = 9.4, 2.1 Hz, 1H), 4.58 (s, 1H), 3.08 (dt, J = 9.1, 6.5 Hz, 2H), 2.31 (d, J = 4.1 Hz, 5H), 1.25 (td, J = 7.5, 1.6 Hz, 4H), 1.18 (d, J = 1.8 Hz, 6H). ESI-MS m/z 489.29 (M+1)+ |
| **459** | 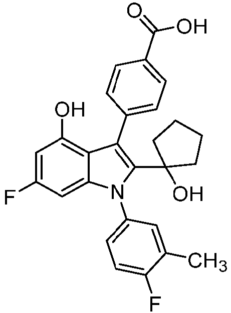 | ¹H NMR (400 MHz, DMSO) δ 12.84 (s, 1H), 9.67 (s, 1H), 7.92 (d, J = 8.0 Hz, 2H), 7.49 (d, J = 8.0 Hz, 2H), 7.46 - 7.42 (m, 1H), 7.40 - 7.30 (m, 2H), 6.23 (dd, J = 11.4, 2.2 Hz, 1H), 5.97 (dd, J = 9.6, 2.1 Hz, 1H), 2.95 (p, J = 9.3 Hz, 1H), 2.32 (d, J = 1.8 Hz, 3H), 1.68 (s, 2H), 1.53 (s, 2H), 1.24 (p, J = 8.6, 7.5 Hz, 4H). ESI-MS m/z 464.32 (M+1) |
| **460** | 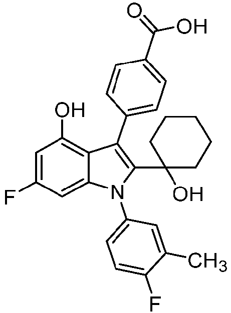 | ¹H NMR (400 MHz, DMSO-d6) δ 12.77 (s, 1H), 10.00 (s, 1H), 7.88 (d, J = 8.0 Hz, 2H), 7.53 (d, J = 8.1 Hz, 2H), 7.39 - 7.33 (m, 1H), 7.30 (d, J = 9.1 Hz, 1H), 7.27 - 7.20 (m, 1H), 6.33 (ddd, J = 9.4, 7.8, 2.2 Hz, 2H), 5.59 (s, 1H), 2.30 (d, J = 1.9 Hz, 3H), 2.07 (s, 2H), 1.85 (s, 2H), 1.70 (s, 2H), 1.35 (s, 4H). ESI-MS 476.33 (M+1) |
| **461** | 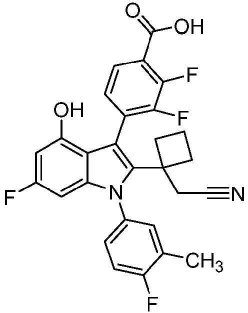 | ¹H NMR (400 MHz, Chloroform-d) δ 7.81 (s, 1H), 7.53 (t, J = 7.1 Hz, 1H), 7.28 - 7.13 (m, 3H), 6.32 - 6.22 (m, 1H), 6.05 (dt, J = 9.2, 1.9 Hz, 1H), 2.93 (d, J = 17.1 Hz, 1H), 2.78 (dd, J = 17.1, 3.9 Hz, 1H), 2.44 (d, J = 10.4 Hz, 1H), 2.38 (dd, J = 8.9, 1.8 Hz, 3H), 2.26 (q, J = 10.4 Hz, 1H), 1.95 (q, J = 9.9 Hz, 1H), 1.72 (d, J = 10.4 Hz, 1H). ESI-MS m/z 509.18 (M+1) |
| **462** | 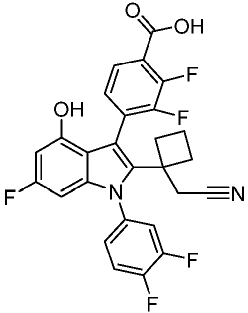 | ¹H NMR (400 MHz, Methanol-d4) δ 7.66 (td, J = 7.4, 6.5, 3.4 Hz, 1H), 7.62 - 7.44 (m, 3H), 7.37 (dd, J = 8.3, 3.4 Hz, 1H), 6.20 (dd, J = 11.2, 2.1 Hz, 1H), 5.94 (dd, J = 9.5, 2.1 Hz, 1H), 3.15 (dd, J = 17.3, 4.1 Hz, 1H), 2.90 (dd, J = 17.5, 11.0 Hz, 1H), 2.51 (p, J = 10.6 Hz, 1H), 2.24 (p, J = 10.1 Hz, 1H), 1.98 (d, J = 9.4 Hz, 1H), 1.70 (d, J = 11.0 Hz, 1H), 1.59 (t, J = 5.8 Hz, 1H), 1.47 (d, J = 7.1 Hz, 1H). ESI-MS m/z 513.1 (M+1)+ |
| **463** | 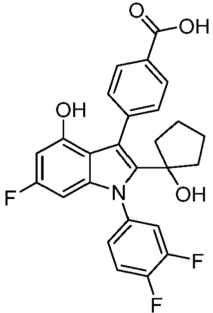 | ¹H NMR (400 MHz, DMSO-d6) δ 12.89 (s, 1H), 9.74 (s, 1H), 7.92 (d, J = 8.0 Hz, 2H), 7.80 (ddd, J = 10.6, 7.3, 2.6 Hz, 1H), 7.68 (q, J = 9.3 Hz, 1H), 7.48 (d, J = 7.9 Hz, 2H), 7.39 (d, J = 8.9 Hz, 1H), 6.25 (dd, J = 11.5, 2.2 Hz, 1H), 6.07 (dd, J = 9.6, 2.2 Hz, 1H), 2.96 (p, J = 9.3 Hz, 1H), 1.69 (s, 2H), 1.52 (s, 2H), 1.35 - 1.22 (m, 4H).ESI-MS m/z 468.28 (M+1) |
| **464** | 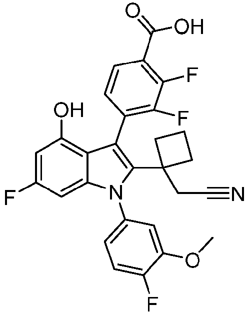 | ¹H NMR (400 MHz, Chloroform-d) δ 7.82 (s, 1H), 7.53 (d, J = 7.5 Hz, 1H), 7.28 - 7.21 (m, 1H), 7.07 (dd, J = 7.6, 2.3 Hz, 1H), 7.01 (d, J = 8.0 Hz, 1H), 6.27 (d, J = 10.1 Hz, 1H), 6.13 - 6.07 (m, 1H), 3.93 (d, J = 6.7 Hz, 3H), 2.95 (d, J = 16.6 Hz, 1H), 2.87 - 2.73 (m, 1H), 2.46 (d, J = 9.3 Hz, 1H), 2.26 (t, J = 10.6 Hz, 1H), 1.95 (q, J = 9.9 Hz, 1H), 1.75 (t, J = 10.4 Hz, 1H), 1.61 (m, 2H). ESI-MS m/z 525.16 (M+1) |
| **465** | 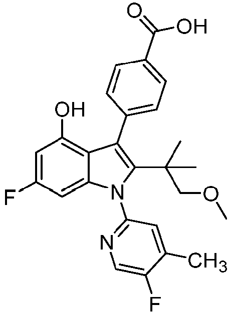 | ¹H NMR (400 MHz, DMSO-d6) δ 12.84 (s, 1H), 9.44 (s, 1H), 8.57 (d, J = 1.1 Hz, 1H), 7.94 - 7.82 (m, 2H), 7.64 (d, J = 5.6 Hz, 1H), 7.48 (s, 2H), 6.14 (dd, J = 11.4, 2.2 Hz, 1H), 5.93 (dd, J = 9.9, 2.2 Hz, 1H), 2.94 (s, 5H), 2.41 (d, J = 1.6 Hz, 3H), 0.96 (d, J = 8.1 Hz, 6H). ESI-MS m/z 467.34 (M+1) |
| **466** | 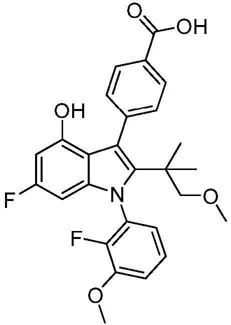 | ¹H NMR (400 MHz, DMSO-d6) δ 12.84 (s, 1H), 9.46 (s, 1H), 7.93 - 7.85 (m, 2H), 7.55 - 7.30 (m, 4H), 7.11 (ddd, J = 8.0, 6.5, 1.7 Hz, 1H), 6.13 (dd, J = 11.5, 2.2 Hz, 1H), 5.72 (dd, J = 9.6, 2.2 Hz, 1H), 3.95 (s, 3H), 3.04 - 2.91 (m, 5H), 0.97 (d, J = 7.8 Hz, 6H). ESI-MS 482.33 (M+1) |
| **467** | 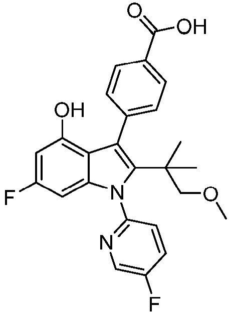 | ¹H NMR (400 MHz, DMSO-d6) δ 12.85 (s, 1H), 9.47 (s, 1H), 8.70 (d, J = 3.1 Hz, 1H), 8.05 (td, J = 8.3, 3.1 Hz, 1H), 7.94 - 7.84 (m, 2H), 7.73 (dd, J = 8.7, 4.0 Hz, 1H), 7.48 (d, J = 7.8 Hz, 2H), 6.14 (dd, J = 11.5, 2.2 Hz, 1H), 5.89 (dd, J = 9.8, 2.2 Hz, 1H), 2.95 (s, 3H), 0.95 (s, 6H). ESI-MS m/z 453.3 (M+1) |
| **468** | 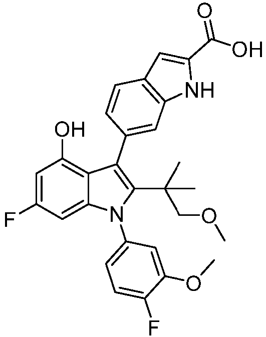 | ¹H NMR (400 MHz, DMSO-d6) δ 12.83 (s, 1H), 11.66 (d, J = 2.2 Hz, 1H), 9.13 (s, 1H), 7.55 (d, J = 8.2 Hz, 1H), 7.48 - 7.34 (m, 2H), 7.29 (ddd, J = 10.6, 7.8, 2.4 Hz, 1H), 7.13 - 6.96 (m, 3H), 6.09 (dd, J = 11.4, 2.3 Hz, 1H), 5.79 (dt, J = 9.9, 2.7 Hz, 1H), 3.88 (s, 3H), 3.02 (d, J = 3.9 Hz, 3H), 2.99 - 2.94 (m, 2H), 1.06 - 0.89 (m, 6H). ESI-MS m/z 521.43 (M+1)+ |
| **469** | 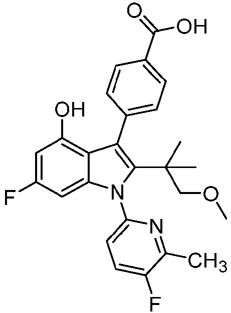 | ¹H NMR (400 MHz, DMSO-d6) δ 12.84 (s, 1H), 9.45 (s, 1H), 7.99 - 7.84 (m, 3H), 7.60 - 7.41 (m, 3H), 6.14 (dd, J = 11.4, 2.2 Hz, 1H), 5.92 (dd, J = 9.9, 2.2 Hz, 1H), 3.10 (qd, J = 7.3, 4.9 Hz, 2H), 2.95 (s, 3H), 1.18 (t, J = 7.3 Hz, 4H), 0.96 (s, 6H). ESI-MS m/z 467.3 (M+1)+; |
| **470** | 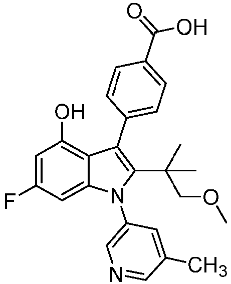 | ¹H NMR (400 MHz, DMSO-d6) δ 12.84 (s, 1H), 9.46 (s, 1H), 8.63 (dd, J = 2.0, 0.9 Hz, 1H), 8.47 (d, J = 2.3 Hz, 1H), 7.95 - 7.85 (m, 2H), 7.83 - 7.76 (m, 1H), 7.48 (td, J = 8.0, 1.7 Hz, 2H), 6.13 (dd, J = 11.4, 2.2 Hz, 1H), 5.71 (dd, J = 9.7, 2.2 Hz, 1H), 2.99 (s, 3H), 2.88 (d, J = 1.8 Hz, 2H), 2.43 (s, 3H), 0.96 (d, J = 4.4 Hz, 6H). ESI-MS m/z 449.28 (M+1)+ |
| **471** | 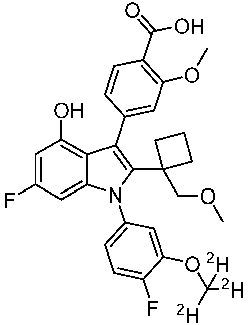 | ¹H NMR (400 MHz, Chloroform-d) δ 8.28 (d, J = 8.0 Hz, 1H), 7.68 (s, 1H), 7.45 (d, J = 8.0 Hz, 1H), 7.24 (dd, J = 10.7, 8.5 Hz, 1H), 7.09 - 6.93 (m, 2H), 6.38 (dd, J = 10.8, 2.1 Hz, 1H), 6.09 (dd, J = 9.4, 2.2 Hz, 1H), 4.14 (s, 3H), 3.70 (s, 2H), 3.43 (s, 3H), 2.17 (d, J = 12.4 Hz, 1H), 1.93 (t, J = 14.8 Hz, 1H), 1.82 (p, J = 9.9 Hz, 1H), 1.69 - 1.53 (m, 2H), 1.43 (d, J = 24.6 Hz, 1H). ESI-MS m/z 527.44 (M+1)+ |
| **472** | 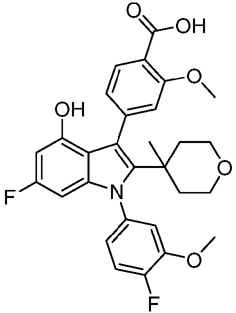 | ¹H NMR (400 MHz, DMSO-d6) δ 9.50 (s, 1H), 7.63 (d, J = 7.8 Hz, 1H), 7.42 (dd, J = 11.2, 8.5 Hz, 1H), 7.29 (ddd, J = 21.4, 7.8, 2.5 Hz, 1H), 7.14 - 6.96 (m, 3H), 6.16 (dd, J = 11.4, 2.2 Hz, 1H), 5.87 (dt, J = 9.8, 2.3 Hz, 1H), 3.85 (s, 3H), 3.80 (d, J = 1.2 Hz, 3H), 3.54 - 3.13 (m, 4H), 1.61 (t, J = 8.2 Hz, 2H), 1.45 (s, 3H), 1.15 - 1.02 (m, 2H). ESI-MS m/z 524.37 (M+1)+ |
| **473** | 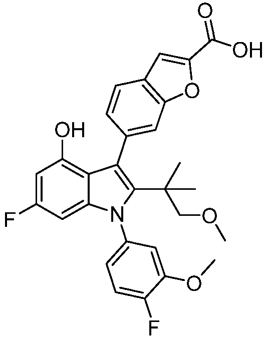 | ¹H NMR (400 MHz, DMSO-d6) δ 13.47 (s, 1H), 9.31 (d, J = 1.4 Hz, 1H), 7.73 - 7.66 (m, 2H), 7.61 (dq, J = 16.1, 1.0 Hz, 1H), 7.43 (dd, J = 11.2, 8.5 Hz, 1H), 7.36 (td, J = 8.3, 1.3 Hz, 1H), 7.29 (ddd, J = 8.1, 6.3, 2.4 Hz, 1H), 7.06 (dddd, J = 8.5, 6.1, 4.0, 2.3 Hz, 1H), 6.11 (dd, J = 11.4, 2.2 Hz, 1H), 5.80 (ddd, J = 9.8, 2.2, 1.1 Hz, 1H), 3.88 (s, 3H), 3.02 (s, 3H), 2.97 (d, J = 1.7 Hz, 2H), 0.99 (d, J = 1.9 Hz, 6H). ESI-MS m/z 522.38 (M+1)+; |
| **474** | 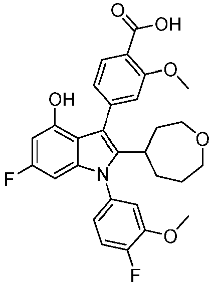 | ¹H NMR (400 MHz, DMSO-d6) δ 12.50 (s, 1H), 9.77 (s, 1H), 7.65 (d, J = 7.8 Hz, 1H), 7.45 (ddd, J = 10.8, 8.5, 1.8 Hz, 1H), 7.30 (td, J = 8.0, 2.4 Hz, 1H), 7.13 - 6.95 (m, 3H), 6.27 (dd, J = 11.4, 2.2 Hz, 1H), 6.03 (dt, J = 9.5, 1.9 Hz, 1H), 3.86 (d, J = 1.7 Hz, 3H), 3.82 (s, 3H), 3.59 - 3.17 (m, 6H), 2.92 (t, J = 10.7 Hz, 1H), 1.89 (td, J = 14.6, 13.7, 5.7 Hz, 2H), 1.75 - 1.45 (m, 2H). ESI-MS m/z 524.44 (M+1)+; |
| **475** | 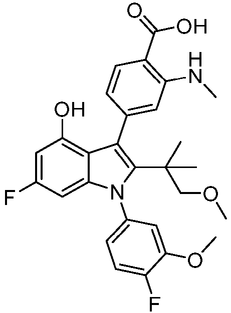 | ¹H NMR (400 MHz, DMSO-d6) δ 9.35 (s, 1H), 7.72 (d, J = 8.1 Hz, 1H), 7.42 (ddd, J = 11.1, 8.6, 2.5 Hz, 1H), 7.27 (ddd, J = 15.2, 7.9, 2.4 Hz, 1H), 7.04 (dddd, J = 12.6, 8.5, 4.0, 2.4 Hz, 1H), 6.69 (dd, J = 6.7, 1.5 Hz, 1H), 6.60 (ddd, J = 8.7, 7.3, 1.5 Hz, 1H), 6.12 (dd, J = 11.4, 2.2 Hz, 1H), 5.78 (ddd, J = 9.9, 2.3, 1.4 Hz, 1H), 3.86 (s, 3H), 3.04 (d, J = 1.1 Hz, 3H), 3.02 (t, J = 2.6 Hz, 1H), 2.83 (s, 3H), 2.54 (s, 2H), 1.04 (d, J = 6.1 Hz, 6H). ESI-MS m/z 511.36 (M+1)+; |
| **476** | 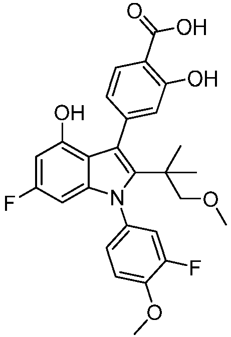 | ¹H NMR (400 MHz, DMSO-d6) δ 9.45 (d, J = 1.2 Hz, 1H), 7.72 (d, J = 7.9 Hz, 1H), 7.45 - 7.19 (m, 3H), 6.98 - 6.84 (m, 2H), 6.11 (dd, J = 11.4, 2.2 Hz, 1H), 5.74 (dd, J = 9.7, 2.2 Hz, 1H), 3.95 (s, 3H), 3.03 (s, 3H), 2.99 - 2.89 (m, 2H), 1.01 (t, J = 2.0 Hz, 6H). ESI-MS m/z 498.36 (M+1) |
| **477** | 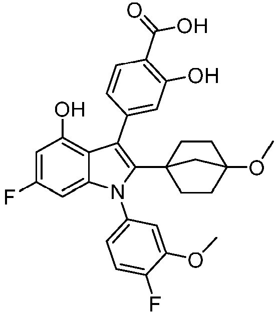 | ¹H NMR (400 MHz, DMSO-d6) δ 11.24 (s, 1H), 9.55 (s, 1H), 7.74 (dd, J = 8.2, 1.5 Hz, 1H), 7.45 - 7.36 (m, 1H), 7.31 (ddd, J = 8.3, 6.1, 2.4 Hz, 1H), 7.09 - 7.00 (m, 1H), 6.98 - 6.86 (m, 2H), 6.17 (dd, J = 11.4, 2.2 Hz, 1H), 5.90 (dd, J = 9.7, 2.2 Hz, 1H), 3.86 (d, J = 1.5 Hz, 3H), 2.96 (s, 3H), 1.86 (s, 2H), 1.60 - 1.30 (m, 6H), 1.26 - 1.14 (m, 2H). ESI-MS m/ 536.34 (M+1)+ |
| **478** | 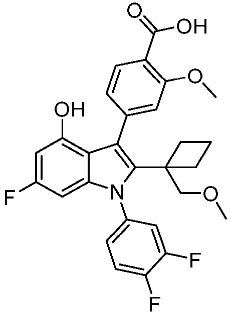 | ¹H NMR (400 MHz, Chloroform-d) δ 8.15 (d, J = 7.9 Hz, 1H), 7.54 (d, J = 1.5 Hz, 1H), 7.33 (dd, J = 8.0, 1.4 Hz, 1H), 7.30 - 7.20 (m, 2H), 7.13 (s, 1H), 6.29 (dd, J = 10.8, 2.1 Hz, 1H), 5.95 (dd, J = 9.2, 2.1 Hz, 1H), 5.34 (s, 1H), 4.04 (s, 3H), 3.63 - 3.51 (m, 2H), 3.34 (s, 3H), 2.04 (s, 1H), 1.85 (s, 1H), 1.81 - 1.60 (m, 2H), 1.61 - 1.48 (m, 2H). ESI-MS m/z 512.33 (M+1)+; |
| **479** | 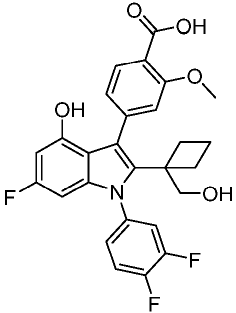 | ¹H NMR (400 MHz, Chloroform-d/CD3OD) δ 8.01 (dd, J = 8.2, 3.8 Hz, 1H), 7.64 - 7.47 (m, 1H), 7.46 - 7.31 (m, 3H), 7.29 - 7.22 (m, 1H), 6.29 (dt, J = 11.1, 2.7 Hz, 1H), 6.06 - 5.88 (m, 1H), 4.06 (d, J = 3.9 Hz, 3H), 3.87 (d, J = 5.9 Hz, 2H), 2.08 - 1.92 (m, 2H), 1.79 (p, J = 9.4 Hz, 1H), 1.69 - 1.35 (m, 3H). ESI-MS m/z calc. 497.14502, found 498.47 (M+1)+ |
| **480** | 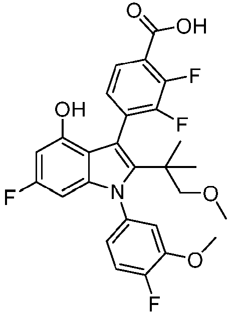 | ¹H NMR (400 MHz, Methanol-d4) δ 7.70 (d, J = 24.5 Hz, 1H), 7.50 (d, J = 12.6 Hz, 1H), 7.39 - 7.04 (m, 1H), 6.82 - 6.74 (m, 2H), 6.68 - 5.78 (m, 2H), 3.94 - 3.70 (m, 6H), 2.40 (s, 2H), 1.37 - 1.19 (m, 6H). ESI-MS m/z 518.32 (M+1)+; |
| **481** | 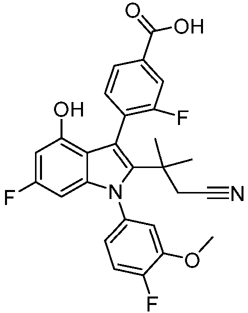 | ¹H NMR (400 MHz, Methanol-d4) δ 7.83 (d, J = 7.9 Hz, 1H), 7.71 (d, J = 9.7 Hz, 1H), 7.58 (t, J = 7.6 Hz, 1H), 7.41 - 7.28 (m, 2H), 7.24 - 7.03 (m, 1H), 6.12 (d, J = 11.0 Hz, 1H), 5.81 (d, J = 11.0 Hz, 1H), 3.90 (s, 3H), 2.64 - 2.42 (m, 2H), 1.32 - 1.20 (m, 6H). ESI-MS m/z 495.29 (M+1)+ |
| **482** | 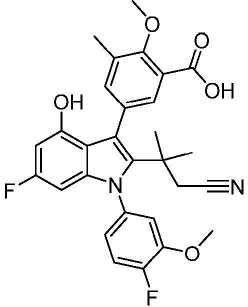 | ¹H NMR (400 MHz, Methanol-d4) δ 7.73 (dd, J = 5.0, 2.2 Hz, 1H), 7.51 (d, J = 2.2 Hz, 1H), 7.34 (ddd, J = 10.7, 8.5, 1.9 Hz, 1H), 7.22 (dt, J = 7.6, 2.1 Hz, 1H), 7.07 (ddd, J = 8.4, 3.7, 2.3 Hz, 1H), 6.12 (dd, J = 11.2, 2.1 Hz, 1H), 5.81 (dt, J = 9.7, 2.0 Hz, 1H), 3.93 - 3.86 (m, 6H), 2.54 - 2.41 (m, 2H), 2.36 (s, 3H), 1.25 (dd, J = 5.7, 4.1 Hz, 6H). ESI-MS m/z 521.3 (M+1)+ |
| **483** | 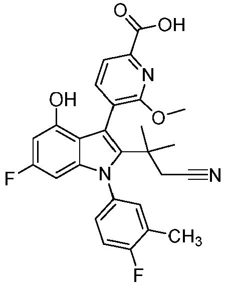 | ¹H NMR (400 MHz, Methanol-d4) δ 7.88 - 7.74 (m, 2H), 7.50 - 7.37 (m, 1H), 7.37 - 7.26 (m, 2H), 6.09 (dd, J = 11.2, 2.0 Hz, 1H), 5.74 (dd, J = 9.7, 2.0 Hz, 1H), 3.99 (d, J = 1.5 Hz, 3H), 2.60 - 2.44 (m, 2H), 2.38 (d, J = 7.2 Hz, 3H), 1.22 (d, J = 17.1 Hz, 6H). ESI-MS m/z 492.31 (M+1)+ |
| **484** | 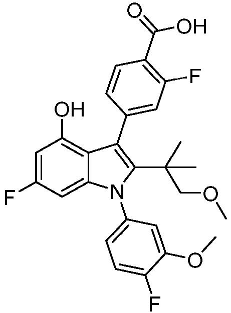 | ¹H NMR (400 MHz, Methanol-d4) δ 7.88 (t, J = 7.9 Hz, 1H), 7.36 - 7.22 (m, 3H), 7.17 (dd, J = 7.8, 2.4 Hz, 1H), 7.06 - 6.99 (m, 1H), 6.77 (dd, J = 11.2, 8.9 Hz, 1H), 6.04 (dd, J = 9.0, 3.4 Hz, 1H), 3.88 (s, 3H), 3.12 (s, 3H), 3.07 (s, 2H), 1.11 (s, 6H). ESI-MS m/z 500.23 (M+1)+ |
| **485** | 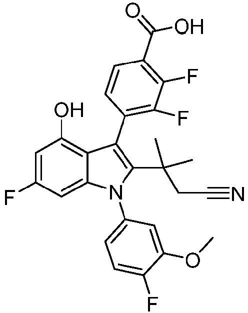 | ¹H NMR (400 MHz, Methanol-d4) δ 7.69 (t, J = 7.4 Hz, 1H), 7.43 - 7.28 (m, 3H), 7.23 - 7.13 (m, 1H), 6.15 (dd, J = 11.3, 2.1 Hz, 1H), 5.82 (dd, J = 9.6, 2.2 Hz, 1H), 3.91 (d, J = 3.5 Hz, 3H), 2.66 - 2.45 (m, 2H), 1.36 - 1.22 (m, 6H). ESI-MS m/z 513.19 (M+1)+; |
| **486** | 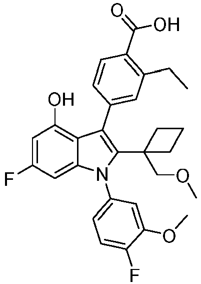 | ¹H NMR (400 MHz, Methanol-d4) δ 7.76 (d, J = 8.0 Hz, 1H), 7.49 (s, 1H), 7.40 (d, J = 8.0 Hz, 1H), 7.30 - 7.22 (m, 2H), 7.06 - 6.99 (m, 1H), 6.15 (dd, J = 11.3, 2.2 Hz, 1H), 5.92 (dd, J = 9.7, 2.1 Hz, 1H), 3.89 (s, 3H), 3.72 (s, 2H), 3.41 (s, 3H), 3.08 - 2.97 (m, 2H), 2.17 - 2.04 (m, 2H), 1.85 - 1.72 (m, 1H), 1.56 - 1.41 (m, 3H), 1.27 (t, J = 7.4 Hz, 3H). ESI-MS m/z 522.34 (M+1)+ |
| **487** | 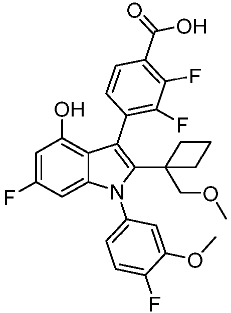 | ¹H NMR (400 MHz, Methanol-d4) δ 7.62 (q, J = 7.2, 6.5 Hz, 1H), 7.50 - 7.37 (m, 1H), 7.35 - 7.19 (m, 2H), 7.11 - 6.98 (m, 1H), 6.17 (dd, J = 11.2, 2.1 Hz, 1H), 5.95 (ddd, J = 9.7, 5.4, 2.2 Hz, 1H), 3.89 (d, J = 12.3 Hz, 3H), 3.84 - 3.60 (m, 2H), 3.39 (d, J = 11.1 Hz, 3H), 2.32 (dq, J = 38.5, 10.2 Hz, 1H), 2.01 - 1.76 (m, 2H), 1.65 - 1.49 (m, 2H), 1.44 - 1.26 (m, 1H). ESI-MS m/z 530.29 (M+1)+ |
| **488** | 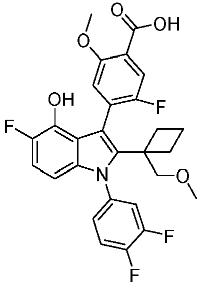 | ¹H NMR (400 MHz, DMSO) δ 12.78 (s, 1H), 9.26 (d, J = 1.7 Hz, 1H), 7.90 (t, J = 9.2 Hz, 1H), 7.80 - 7.55 (m, 1H), 7.55 - 7.15 (m, 2H), 6.89 (dd, J = 11.2, 8.8 Hz, 1H), 6.13 (dt, J = 8.7, 2.9 Hz, 1H), 3.81 (s, 2H), 3.70 (d, J = 9.3 Hz, 1H), 3.65 - 3.51 (m, 1H), 2.28 (dd, J = 39.4, 9.4 Hz, 1H), 1.82 (s, 1H), 1.57 (d, J = 31.2 Hz, 1H), 1.35 - 0.92 (m, 1H). ESI-MS m/z 530.24 (M+1)+ |
| **489** | 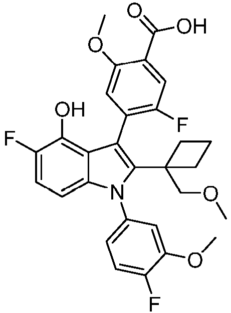 | ¹H NMR (400 MHz, DMSO) δ 12.78 (s, 1H), 9.26 (d, J = 1.7 Hz, 1H), 7.90 (t, J = 9.2 Hz, 1H), 7.80 - 7.55 (m, 1H), 7.55 - 7.15 (m, 2H), 6.89 (dd, J = 11.2, 8.8 Hz, 1H), 6.13 (dt, J = 8.7, 2.9 Hz, 1H), 3.81 (s, 2H), 3.70 (d, J = 9.3 Hz, 1H), 3.65 - 3.51 (m, 1H), 2.28 (dd, J = 39.4, 9.4 Hz, 1H), 1.82 (s, 1H), 1.57 (d, J = 31.2 Hz, 1H), 1.35 - 0.92 (m, 1H).ESI-MS *m*/*z* 530.24 (M+1) ⁺ |
| **490** | 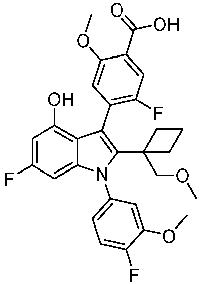 | ¹H NMR (400 MHz, DMSO) δ 12.73 (s, 1H), 9.67 (d, *J =* 3.6 Hz, 1H), 7.53 - 7.30 (m, 4H), 7.00 (d, *J=* 8.6 Hz, 1H), 6.20 (dd, *J* = 11.4, 2.2 Hz, 1H), 5.92 (d, *J* = 9.6 Hz, 1H), 5.76 (s, 2H), 3.96 - 3.76 (m, 7H), 3.76 - 3.47 (m, 2H), 2.43 - 2.16 (m, 1H), 1.91 - 1.69 (m, 2H), 1.69 - 1.31 (m, 2H), 1.31 - 0.91 (m, 1H). ESI-MS *m*/*z* 542.35 (M+1) ⁺ |
| **491** | 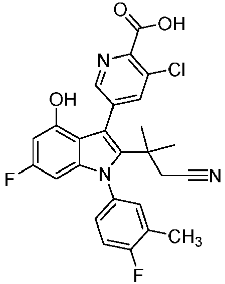 | ¹H NMR (400 MHz, DMSO-d6) δ 13.83 (s, 1H), 9.85 (s, 1H), 8.59 (d, J = 14.2 Hz, 1H), 8.05 (d, J = 25.9 Hz, 1H), 7.49 (dd, J = 11.2, 8.5 Hz, 1H), 7.31 (t, J = 9.8 Hz, 1H), 7.10 (d, J = 8.2 Hz, 1H), 6.18 (d, J = 11.3 Hz, 1H), 5.86 (d, J = 9.5 Hz, 1H), 3.88 (s, 3H), 2.61 (d, J = 4.7 Hz, 2H), 1.17 (d, J = 3.8 Hz, 6H). ESI-MS m/z 496.27 (M+1)+; |
| **492** | 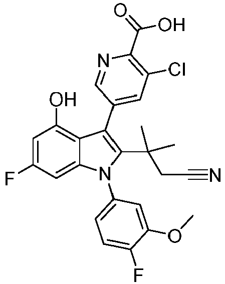 | ¹H NMR (400 MHz, DMSO-d6) δ 13.83 (s, 1H), 9.85 (s, 1H), 8.59 (d, J = 14.2 Hz, 1H), 8.05 (d, J = 25.9 Hz, 1H), 7.49 (dd, J = 11.2, 8.5 Hz, 1H), 7.31 (t, J = 9.8 Hz, 1H), 7.10 (d, J = 8.2 Hz, 1H), 6.18 (d, J = 11.3 Hz, 1H), 5.86 (d, J = 9.5 Hz, 1H), 3.88 (s, 3H), 2.61 (d, J = 4.7 Hz, 2H), 1.17 (d, J = 3.8 Hz, 6H). ESI-MS m/z calc. 495.11612, found 496.27 (M+1)+; |
| **493** | 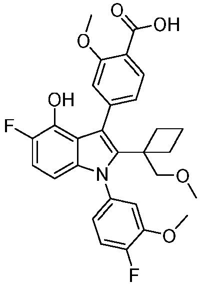 | ¹H NMR (400 MHz, DMSO-d6) δ 12.42 (s, 1H), 9.14 (s, 1H), 7.59 (d, J = 7.9 Hz, 1H), 7.52 - 7.22 (m, 3H), 7.22 - 6.96 (m, 2H), 6.88 (dd, J = 11.1, 8.9 Hz, 1H), 6.12 (dd, J = 8.8, 3.4 Hz, 1H), 3.84 (d, J = 4.8 Hz, 6H), 3.77 - 3.59 (m, 2H), 2.28 - 0.75 (m, 6H). ESI-MS m/z 524.35 (M+1)+; |
| **494** | 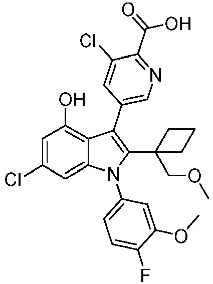 | ¹H NMR (400 MHz, DMSO-d6) δ 13.68 (s, 1H), 10.00 (s, 1H), 8.71 (s, 1H), 8.20 (s, 1H), 7.48 - 7.27 (m, 2H), 7.08 (d, J = 8.5 Hz, 1H), 6.46 (d, J = 1.7 Hz, 1H), 6.19 (d, J = 1.6 Hz, 1H), 3.86 (s, 3H), 3.68 (q, J = 9.6 Hz, 2H), 2.15 - 1.71 (m, 3H), 1.61 - 1.28 (m, 3H). ESI-MS m/z 545.27 (M+1)+; |
| **495** | 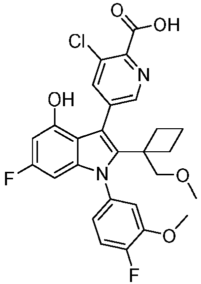 | ¹H NMR (400 MHz, DMSO-d6) δ 13.66 (s, 1H), 10.01 (s, 1H), 8.72 (s, 1H), 8.20 (s, 1H), 7.41 (dd, J = 11.2, 8.5 Hz, 1H), 7.37 - 7.26 (m, 1H), 7.07 (dd, J = 7.9, 4.0 Hz, 1H), 6.28 (dd, J = 11.4, 2.1 Hz, 1H), 5.94 (dd, J = 9.5, 2.1 Hz, 1H), 3.86 (s, 3H), 3.77 - 3.57 (m, 2H), 3.38 (s, 3H), 2.18 - 1.72 (m, 3H), 1.66 - 1.07 (m, 3H). ESI-MS m/z 529.25 (M+1)+; |
| **496** | 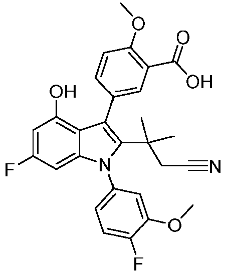 | ¹H NMR (400 MHz, DMSO-d6) δ 12.52 (s, 1H), 9.52 (d, J = 1.2 Hz, 1H), 7.68 (dd, J = 2.3, 1.2 Hz, 1H), 7.57 - 7.43 (m, 2H), 7.31 (ddd, J = 7.9, 5.7, 2.5 Hz, 1H), 7.16 - 7.04 (m, 2H), 6.14 (dd, J = 11.4, 2.2 Hz, 1H), 5.83 (dd, J = 9.8, 2.2 Hz, 1H), 3.87 (d, J = 3.5 Hz, 6H), 2.49 (s, 2H), 1.20 - 1.12 (m, 6H). ESI-MS m/z 507.0 (M+1)+; |
| **497** | 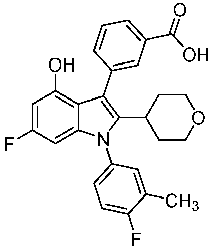 | ¹H NMR (400 MHz, DMSO-d6) δ 12.91 (s, 1H), 9.65 (s, 1H), 7.96 (t, J = 1.7 Hz, 1H), 7.91 (dt, J = 7.7, 1.5 Hz, 1H), 7.63 (dt, J = 7.6, 1.5 Hz, 1H), 7.51 (t, J = 7.7 Hz, 1H), 7.46 - 7.31 (m, 3H), 6.21 (dd, J = 11.4, 2.2 Hz, 1H), 5.98 (dd, J = 9.7, 2.2 Hz, 1H), 3.66 (dd, J = 11.1, 2.7 Hz, 2H), 2.99 (td, J = 11.5, 2.7 Hz, 2H), 2.75 (ddt, J = 11.7, 8.2, 4.1 Hz, 1H), 2.34 (d, J = 1.9 Hz, 3H), 1.60 - 1.40 (m, 4H). ESI-MS m/z 464.0 (M+1)+; |
| **498** | 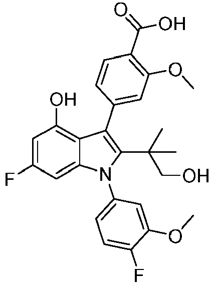 | ¹H NMR (300 MHz, DMSO-d6) δ 9.47 (s, 1H), 7.55 (d, J = 7.8 Hz, 1H), 7.46 - 7.26 (m, 2H), 7.15 - 6.97 (m, 3H), 6.15 (dd, J = 11.5, 2.2 Hz, 1H), 5.76 (dd, J = 9.9, 2.1 Hz, 1H), 3.85 (s, 3H), 3.78 (s, 3H), 3.26 (s, 2H), 0.93 (s, 6H). ESI-MS m/z 498.0 (M+1)+; |
| **499** | 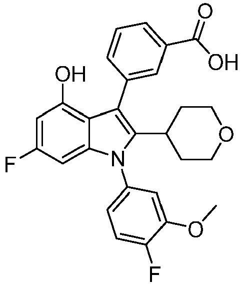 | ¹H NMR (400 MHz, DMSO-d6) δ 12.92 (s, 1H), 9.64 (s, 1H), 7.97 (t, J = 1.7 Hz, 1H), 7.91 (dt, J = 7.7, 1.5 Hz, 1H), 7.63 (dt, J = 7.6, 1.5 Hz, 1H), 7.55 - 7.41 (m, 2H), 7.32 (dd, J = 7.8, 2.4 Hz, 1H), 7.07 (ddd, J = 8.5, 3.9, 2.4 Hz, 1H), 6.21 (dd, J = 11.4, 2.2 Hz, 1H), 6.06 (dd, J = 9.6, 2.2 Hz, 1H), 3.87 (s, 3H), 3.67 (dd, J = 11.2, 3.7 Hz, 2H), 3.07 - 2.95 (m, 2H), 2.78 (tt, J = 11.8, 3.8 Hz, 1H), 1.63 - 1.45 (m, 4H). ESI-MS m/z 480.0 (M+1)+; |
| **500** | 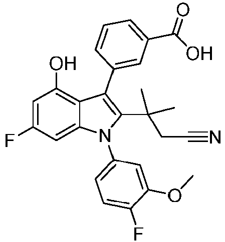 | ¹H NMR (400 MHz, DMSO-d6) δ 12.92 (s, 1H), 9.53 (d, J = 0.9 Hz, 1H), 7.99 (t, J = 1.7 Hz, 1H), 7.92 (dt, J = 7.8, 1.5 Hz, 1H), 7.66 (ddt, J = 9.0, 7.6, 1.5 Hz, 1H), 7.52 - 7.43 (m, 2H), 7.33 (td, J = 7.7, 2.5 Hz, 1H), 7.12 (dddd, J = 8.4, 6.1, 3.9, 2.5 Hz, 1H), 6.14 (dd, J = 11.4, 2.2 Hz, 1H), 5.84 (dd, J = 9.7, 2.2 Hz, 1H), 3.88 (d, J = 1.0 Hz, 3H), 2.48 (d, J = 3.1 Hz, 2H), 1.32 - 1.21 (m, 6H). ESI-MS m/z 477.0 (M+1)+; |
| **501** | 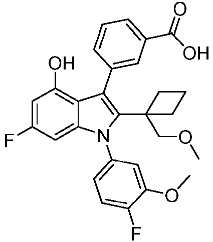 | ¹H NMR (400 MHz, DMSO-d6) δ 12.77 (s, 1H), 9.62 (s, 1H), 8.12 (s, 1H), 7.83 (dt, J = 7.7, 1.4 Hz, 1H), 7.75 - 7.69 (m, 1H), 7.44 - 7.33 (m, 3H), 7.08 (ddd, J = 8.5, 3.9, 2.4 Hz, 1H), 6.20 (dd, J = 11.5, 2.2 Hz, 1H), 5.93 (dd, J = 9.7, 2.2 Hz, 1H), 3.86 (s, 3H), 3.63 (t, J = 7.7 Hz, 2H), 3.35 (s, 3H), 2.10 - 1.98 (m, 1H), 1.91 (q, J = 10.2 Hz, 1H), 1.71 (h, J = 9.6 Hz, 1H), 1.42 (t, J = 10.0 Hz, 1H), 1.37 - 1.26 (m, 2H). ESI-MS m/z 494.0 (M+1)+; |
| **502** | 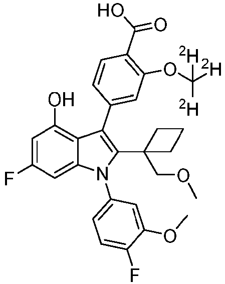 | ¹H NMR (400 MHz, DMSO-d6) δ 9.84 (s, 1H), 7.52 (d, J = 7.9 Hz, 1H), 7.43 - 7.25 (m, 4H), 7.10 (d, J = 8.1 Hz, 1H), 6.26 (dd, J = 11.5, 2.3 Hz, 1H), 5.89 (dd, J = 9.6, 2.2 Hz, 1H), 3.85 (s, 3H), 3.67 (d, J = 2.8 Hz, 2H), 3.36 (s, 3H), 2.34 - 1.16 (m, 6H). ESI-MS m/z found 527.0 (M+1)+; |
| **503** | 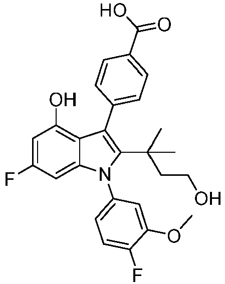 | ¹H NMR (400 MHz, DMSO) δ 12.86 (s, 1H), 9.39 (s, 1H), 7.88 (d, J = 8.0 Hz, 2H), 7.44 (q, J = 10.9, 9.4 Hz, 3H), 7.27 (dd, J = 7.7, 2.4 Hz, 1H), 7.07 (dd, J = 7.6, 3.9 Hz, 1H), 6.10 (dd, J = 11.5, 2.2 Hz, 1H), 5.75 (dd, J = 9.7, 2.2 Hz, 1H), 3.86 (s, 3H), 3.36 (t, J = 7.9 Hz, 2H), 1.55 (q, J = 7.0 Hz, 2H), 0.97 (d, J = 20.9 Hz, 6H). ESI-MS m/z 482.14 (M+1)+; |
| **504** | 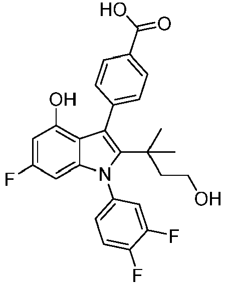 | ¹H NMR (400 MHz, DMSO) δ 12.87 (s, 1H), 9.43 (s, 1H), 7.92 - 7.85 (m, 2H), 7.78 - 7.61 (m, 2H), 7.45 (ddd, J = 6.2, 4.7, 1.6 Hz, 2H), 7.38 (dd, J = 8.5, 4.1 Hz, 1H), 6.11 (dd, J = 11.5, 2.2 Hz, 1H), 5.77 (dd, J = 9.8, 2.2 Hz, 1H), 4.27 (t, J = 4.9 Hz, 1H), 2.55 - 2.52 (m, 2H), 1.52 (dt, J = 7.4, 4.3 Hz, 2H), 0.95 (s, 6H). ESI-MS m/z 470.21 (M+1)+ |
| **505** | 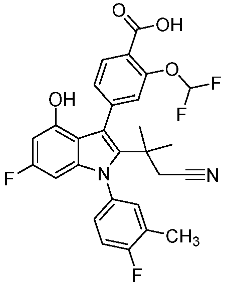 | ¹H NMR (400 MHz, DMSO) δ 13.16 (s, 1H), 9.66 (s, 1H), 7.82 (d, J = 7.9 Hz, 1H), 7.50 - 6.94 (m, 6H), 6.16 (dd, J = 11.4, 2.2 Hz, 1H), 5.79 - 5.72 (m, 1H), 2.60 - 2.51 (m, 2H), 2.34 (s, 3H), 1.14 (t, J = 2.7 Hz, 6H). ESI-MS m/z 527.09 (M+1)+; |
| **506** | 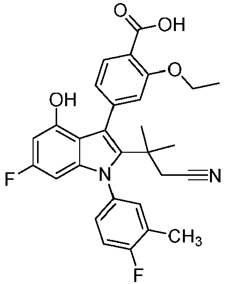 | ¹H NMR (400 MHz, DMSO) δ 12.80 - 12.04 (m, 1H), 9.56 (s, 1H), 7.62 (d, J = 7.8 Hz, 1H), 7.50 - 7.32 (m, 3H), 7.14 (d, J = 3.1 Hz, 1H), 7.05 (dd, J = 7.5, 2.2 Hz, 1H), 6.16 (dd, J = 11.4, 2.2 Hz, 1H), 5.75 (dt, J = 9.7, 2.4 Hz, 1H), 4.10 (q, J = 6.9 Hz, 2H), 2.56 - 2.52 (m, 2H), 2.34 (s, 3H), 1.33 (t, J = 6.9 Hz, 3H), 1.19 - 1.12 (m, 6H). ESI-MS m/z 505.49 (M+1)+; |
| **507** | 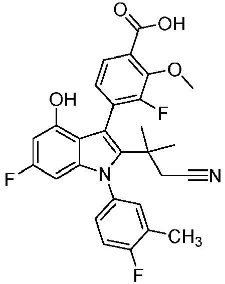 | ¹H NMR (400 MHz, DMSO) δ 13.09 (s, 1H), 9.71 (d, J = 1.9 Hz, 1H), 7.57 - 7.30 (m, 4H), 7.25 (td, J = 8.1, 7.4, 2.8 Hz, 1H), 6.16 (dd, J = 11.4, 2.2 Hz, 1H), 5.76 (ddd, J = 9.7, 3.9, 2.2 Hz, 1H), 3.87 (s, 3H), 2.60 - 2.54 (m, 2H), 2.37 - 2.31 (m, 3H), 1.25 - 1.14 (m, 6H). ESI-MS 509.27 (M+1)+ |
| **508¹** | 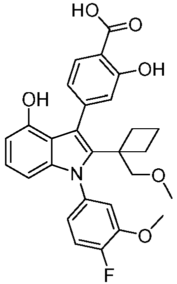 | ¹H NMR (400 MHz, Chloroform-d) δ 10.59 (s, 1H), 7.99 (d, J = 8.1 Hz, 1H), 7.39 (s, 1H), 7.29 - 7.11 (m, 4H), 7.11 - 6.98 (m, 2H), 6.57 (dd, J = 7.8, 0.8 Hz, 1H), 6.44 (dd, J = 8.1, 0.8 Hz, 1H), 3.91 (s, 3H), 3.72 (s, 2H), 3.43 (s, 3H), 2.15 (dt, J = 28.4, 9.9 Hz, 2H), 1.90 - 1.79 (m, 1H), 1.61 (dd, J = 10.2, 8.0 Hz, 3H).ESI-MS *m*/*z* 492.35 (M+1) ⁺ ; |
| **509** | 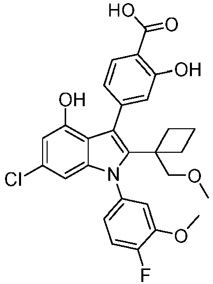 | ¹H NMR (400 MHz, Chloroform-d) δ 10.58 (s, 1H), 7.99 (d, J = 8.1 Hz, 1H), 7.37 (s, 1H), 7.32 - 7.16 (m, 2H), 7.13 (s, 1H), 7.02 (s, 1H), 6.58 (d, J = 1.7 Hz, 1H), 6.41 (d, J = 1.7 Hz, 1H), 3.93 (s, 3H), 3.70 (s, 2H), 3.43 (s, 3H), 2.23 - 2.02 (m, 2H), 1.93 - 1.69 (m, 1H), 1.71 - 1.39 (m, 3H). ESI-MS m/z 526.28 (M+1)+; |
| **510¹** | 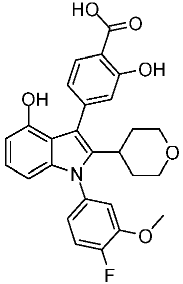 | ¹H NMR (400 MHz, Chloroform-d) δ 11.01 (s, 1H), 7.98 (d, J = 8.1 Hz, 1H), 7.29 (d, J = 8.4 Hz, 2H), 7.23 (d, J = 1.6 Hz, 1H), 7.13 (dd, J = 8.0, 1.6 Hz, 1H), 7.09 - 6.92 (m, 3H), 6.63 - 6.47 (m, 2H), 3.99 (d, J = 12.1 Hz, 2H), 3.93 (s, 3H), 3.29 (t, J = 11.9 Hz, 2H), 2.98 - 2.82 (m, 1H), 1.92 (s, 2H), 1.66 (t, J = 16.7 Hz, 2H). ESI-MS 478.32 (M+1)+ |
| **511** | 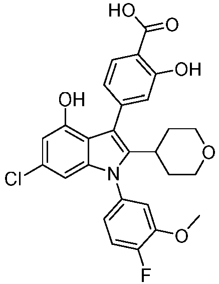 | ¹H NMR (400 MHz, Chloroform-d/CD3OD) δ 7.97 - 7.81 (m, 1H), 7.21 (dd, J = 10.9, 8.2 Hz, 1H), 7.02 (s, 1H), 7.01 - 6.82 (m, 3H), 6.43 (s, 1H), 6.35 (s, 1H), 3.86 (d, J = 3.5 Hz, 3H), 3.76 (dd, J = 11.6, 4.3 Hz, 3H), 3.13 (t, J = 11.6 Hz, 2H), 2.81 (tt, J = 12.3, 3.7 Hz, 1H), 1.69 (qdd, J = 12.3, 7.9, 4.3 Hz, 2H), 1.51 (t, J = 11.7 Hz, 2H). ESI-MS m/z 512.29 (M+1)+; |
| **512¹** | 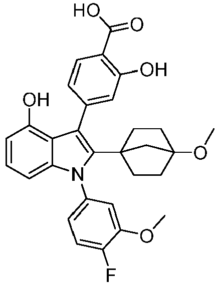 | ¹H NMR (400 MHz, Chloroform-d) δ 11.01 (s, 1H), 10.74 (s, 1H), 7.95 (d, J = 7.9 Hz, 1H), 7.76 (d, J = 8.1 Hz, 1H), 7.25 - 7.18 (m, 2H), 7.06 - 6.98 (m, 3H), 6.76 (dd, J = 8.2, 1.7 Hz, 1H), 6.53 (d, J = 7.7 Hz, 1H), 6.38 (dt, J = 8.2, 0.8 Hz, 1H), 3.96 (s, 3H), 3.23 (s, 3H), 2.61 - 2.53 (m, 2H), 1.98 - 1.82 (m, 8H). ESI-MS m/z 518.5 (M+1)+ |
| **513** | 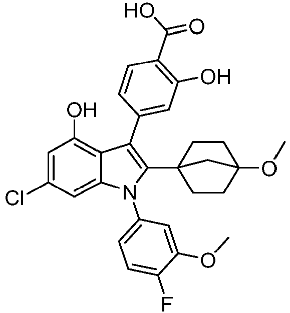 | ¹H NMR (400 MHz, Chloroform-d) δ 10.89 (s, 1H), 7.77 (d, J = 8.0 Hz, 1H), 7.11 - 7.05 (m, 1H), 7.02 (d, J = 1.6 Hz, 1H), 6.94 (dt, J = 8.0, 1.8 Hz, 1H), 6.89 - 6.77 (m, 2H), 6.37 (d, J = 1.7 Hz, 1H), 6.20 (d, J = 1.7 Hz, 1H), 3.77 (d, J = 2.1 Hz, 3H), 3.10 (s, 3H), 1.78 (d, J = 10.4 Hz, 2H), 1.59 - 1.35 (m, 8H). ESI-MS m/z calc. 551.1511, found 552.39 (M+1)+ |
| **514¹** | 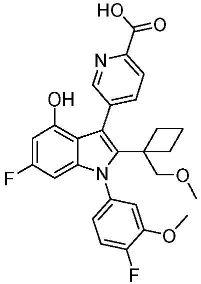 | ¹H NMR (400 MHz, DMSO-d6) δ 9.85 (s, 1H), 8.83 (s, 1H), 8.08 (d, J = 8.3 Hz, 1H), 7.98 (d, J = 8.0 Hz, 1H), 7.48 - 7.27 (m, 2H), 7.18 - 6.81 (m, 1H), 6.26 (dd, J = 11.3, 2.2 Hz, 1H), 5.94 (dd, J = 9.7, 2.1 Hz, 1H), 3.86 (s, 3H), 3.74 - 3.55 (m, 2H), 3.37 (s, 3H), 2.02 (t, J = 10.3 Hz, 1H), 1.83 (dq, J = 53.1, 9.8 Hz, 2H), 1.56 - 1.19 (m, 3H). ESI-MS m/z calc. 494.1653, found 495.23 (M+1)+ |

| | | |
|---|---|---|
| **^{1.}** Formed during debenzylation of the corresponding Cl (i.e., compounds **495, 509, 511, 513**). | | |

### Compounds 515-518

All compounds in Table 27 prepared via Suzuki coupling of the corresponding aryl halide (such as **S3**) with the corresponding boronic acid, followed by ester hydrolysis and debenzylation, as shown for compound **1.** Aryl halides were prepared using similar procedures to the preparation of **S3.**

**Table 27. Method of preparation, structure, and physicochemical data for compounds 515-518**

| **Cmpd.** | **Structure** | **Aryl Halide** | **Boronic Acid** | ¹H NMR; **LCMS m/z** [M+H]⁺ |
|---|---|---|---|---|
| **515** | 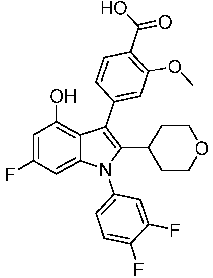 | S3 | 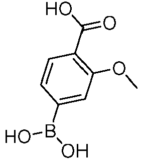 | ¹H NMR (400 MHz, Chloroform-d) δ 8.02 (dd, J = 7.8, 3.5 Hz, 1H), 7.36 (qd, J = 9.0, 3.5 Hz, 1H), 7.26 - 7.01 (m, 4H), 6.37 - 6.16 (m, 1H), 6.13 - 5.89 (m, 1H), 3.99 (d, J = 3.4 Hz, 3H), 3.78 (d, J = 11.5 Hz, 2H), 3.14 (t, J = 11.7 Hz, 2H), 2.83 (ddt, J = 12.6, 9.1, 3.8 Hz, 1H), 1.75 - 1.59 (m, 2H), 1.52 (d, J = 13.3 Hz, 2H). ESI-MS m/z 498.29 (M+1)+; |
| **516** | 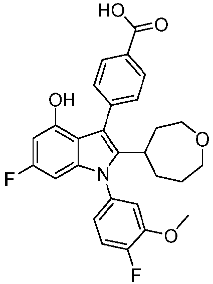 | 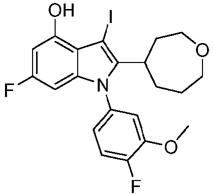 | 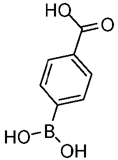 | ¹H NMR (400 MHz, DMSO-d6) δ 12.85 (s, 1H), 9.69 (s, 1H), 7.94 (d, J = 7.9 Hz, 2H), 7.46 (dd, J = 20.5, 8.9 Hz, 3H), 7.37 - 7.25 (m, 1H), 7.07 (dt, J = 8.8, 3.5 Hz, 1H), 6.24 (dd, J = 11.4, 2.1 Hz, 1H), 6.05 (d, J = 9.5 Hz, 1H), 3.86 (d, J = 1.6 Hz, 3H), 3.46 (tdd, J = 19.5, 17.5, 10.6, 4.7 Hz, 3H), 3.29 - 3.20 (m, 1H), 2.87 (t, J = 11.2 Hz, 1H), 1.86 (s, 2H), 1.75 - 1.41 (m, 3H), 1.27 (d, J = 25.1 Hz, 1H). ESI-MS m/z 494.51 (M+1)+ |
| **517** | 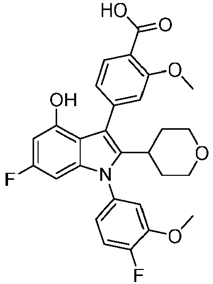 | 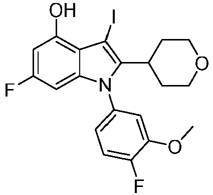 | 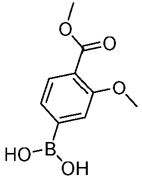 | ¹H NMR (400 MHz, DMSO-d6) δ 12.50 (s, 1H), 9.70 (s, 1H), 7.67 (d, J = 7.9 Hz, 1H), 7.46 (dd, J = 11.3, 8.5 Hz, 1H), 7.29 (dd, J = 7.8, 2.4 Hz, 1H), 7.10 (d, J = 1.4 Hz, 1H), 7.09 - 6.96 (m, 2H), 6.25 (dd, J = 11.4, 2.2 Hz, 1H), 6.04 (dd, J = 9.6, 2.2 Hz, 1H), 3.87 (s, 3H), 3.82 (s, 3H), 3.76 - 3.65 (m, 2H), 3.05 (ddd, J = 13.9, 11.4, 6.7 Hz, 2H), 2.85 (p, J = 8.4, 7.9 Hz, 1H), 1.68 - 1.49 (m, 4H). ESI-MS m/z 510.49 (M+1)+; |
| **518** | 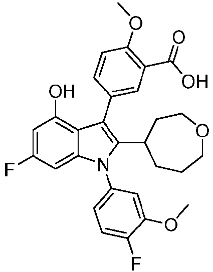 | 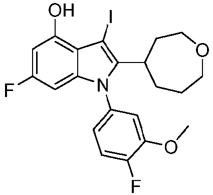 | 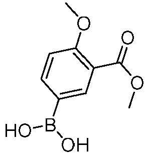 | ¹H NMR (400 MHz, DMSO-d6) δ 12.48 (s, 1H), 9.64 (s, 1H), 7.66 (d, J = 2.1 Hz, 1H), 7.55 - 7.39 (m, 2H), 7.36 - 7.23 (m, 1H), 7.13 (d, J = 8.6 Hz, 1H), 7.08 - 6.98 (m, 1H), 6.21 (dd, J = 11.4, 2.1 Hz, 1H), 6.10 - 5.99 (m, 1H), 4.02 - 3.66 (m, 6H), 3.57 - 3.38 (m, 2H), 3.28 (d, J = 11.7 Hz, 2H), 2.86 (t, J = 10.8 Hz, 1H), 1.85 (d, J = 13.7 Hz, 2H), 1.73 - 1.42 (m, 3H), 1.31 (d, J = 11.2 Hz, 1H). ESI-MS m/z 524.44 (M+1)+ |

### Compound 519

### 4-[2-(3,3a,4,5,6,6a-hexahydro-1H-cyclopenta[c]furan-5-yl)-6-fluoro-1-(4-fluoro-3-methoxy-phenyl)-4-hydroxy-indol-3-yl]-2-hydroxy-benzoic acid (519)

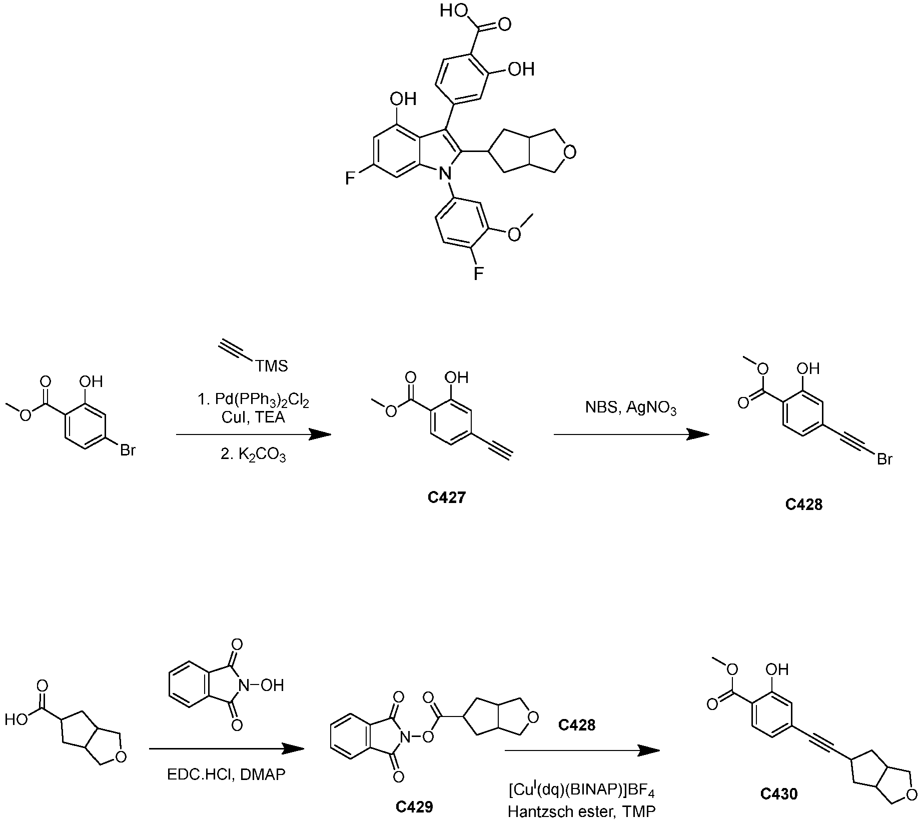

### Step 1: methyl 4-ethynyl-2-hydroxybenzoate (C427)

A reaction vessel was charged with methyl 4-bromo-2-methoxybenzoate (2.978 g, 12.89 mmol) and TEA (30 mL), and the solution was degassed with an N₂ stream for 15 minutes. Then, Pd(PPh₃)₂Cl₂ (445 mg, 0.6340 mmol) and CuI (247 mg, 1.297 mmol) were added in one portion, followed by ethynyl(trimethyl)silane (2.7 mL, 19.11 mmol), and the reaction was heated to 60 °C overnight. The reaction was allowed to cool to room temperature, diluted with EtOAc (100 mL) and extracted with water (2 x 150 mL). The organic phase was collected, and the solution was concentrated *in vacuo.* The crude residue was taken up in MeOH (30 mL) and K₂CO₃ (2.70 g, 19.54 mmol) was added. The reaction was allowed to stir at room temperature for 3 hours. The solvent was evaporated *in vacuo,* and the crude material was partitioned between water (200 mL) and EtOAc (200 mL). The organic phase was collected and concentrated *in vacuo.* Purification by silica gel chromatography (0-30% EtOAc in heptane) afforded methyl 4-ethynyl-2-hydroxybenzoate **C427** (761 mg, 34%) as a light tan solid. ¹H NMR (400 MHz, DMSO) δ 10.54 (s, 1H), 7.75 (d, J = 8.1 Hz, 1H), 7.07 (d, J = 1.5 Hz, 1H), 7.02 (dd, J = 8.1, 1.6 Hz, 1H), 4.45 (s, 1H), 3.88 (s, 3H). ESI-MS m/z calc. 176.04735, found 177.06 (M+1)+.

### Step 2: methyl 4-(bromoethynyl)-2-hydroxybenzoate (C428)

To a solution of methyl 4-ethynyl-2-hydroxybenzoate **C427** (760 mg, 4.314 mmol) in acetone (9 mL), AgNO₃ (74 mg, 0.4356 mmol) was added, and the mixture was stirred at room temperature for 1 hour. Then NBS (1.315 g, 7.388 mmol) was added and the reaction was heated to 50 °C for 1 hour. The mixture was filtered and the solvent was removed *in vacuo.* Purification by silica gel chromatography (0-20% EtOAc in heptane) afforded methyl 4-(bromoethynyl)-2-hydroxybenzoate **C428** (938 mg, 85%) as an off-white solid. ¹H NMR (400 MHz, DMSO) δ 10.55 (s, 1H), 7.74 (dd, J = 8.1, 1.3 Hz, 1H), 7.07 (s, 1H), 7.02 (dd, J = 8.1, 1.6 Hz, 1H), 3.88 (s, 3H). ESI-MS m/z calc. 253.95786, found 255.01 (M+1)+.

### Step 3: hexahydro-1H-cyclopenta[c]furan-5-carboxylic acid--1,3-dioxoisoindolin-2-yl hexahydro-1H-cyclopenta[c]furan-5-carboxylate--2-hydroxyisoindoline-1,3-dione (1/1/1) (C429)

To a solution of hexahydro-1H-cyclopenta[c]furan-5-carboxylic acid--1,3-dioxoisoindolin-2-yl hexahydro-1H-cyclopenta[c]furan-5-carboxylate--2-hydroxyisoindoline-1,3-dione (1/1/1) (1 g, 6.403 mmol), 2-hydroxyisoindoline-1,3-dione (1.61 g, 9.869 mmol) and DMAP (80 mg, 0.6548 mmol) in DCM (65 mL), EDC.HCl (2.42 g, 12.62 mmol) was added, and the reaction was stirred at room temperature overnight. The reaction was then diluted with water (65 mL) and the mixture was passed through a phase separator. The organic phase was collected, and the solvent was evaporated *in vacuo.* Purification by silica gel chromatography (0-10% EtOAc in heptane) afforded hexahydro-1H-cyclopenta[c]furan-5-carboxylic acid--1,3-dioxoisoindolin-2-yl hexahydro-1H-cyclopenta[c]furan-5-carboxylate--2-hydroxyisoindoline-1,3-dione (1/1/1) **C429** (1.419 g, 70%) as a white solid. ¹H NMR (400 MHz, DMSO-d6) δ 8.09 - 7.92 (m, 4H), 3.98 (dtd, J = 10.7, 8.2, 2.3 Hz, 1H), 3.75 (td, J = 6.5, 1.6 Hz, 2H), 3.25 (td, J = 10.3, 7.0 Hz, 2H), 2.31 - 2.12 (m, 3H), 2.04 (ddd, J = 12.4, 6.3, 2.3 Hz, 1H), 1.83 (td, J = 12.1, 10.6 Hz, 1H), 1.70 - 1.57 (m, 1H). ESI-MS m/z calc. 301.09503, found 302.12 (M+1)+.

### Step 4: methyl 4-((hexahydro-1H-cyclopenta[c]furan-5-yl)ethynyl)benzoate (C430)

A reaction vessel was charged with hexahydro-1H-cyclopenta[c]furan-5-carboxylic acid--1,3-dioxoisoindolin-2-yl hexahydro-1H-cyclopenta[c]furan-5-carboxylate--2-hydroxyisoindoline-1,3-dione (1/1/1) **C429** (203 mg, 0.6401 mmol), methyl 4-(bromoethynyl)-2-hydroxybenzoate **C428** (205 mg, 0.8037 mmol), [Cu(dq)(BINAP)]BF₄ (10 mg, 0.009297 mmol), and Hantzsch ester (240 mg, 0.9475 mmol), and DCM (3.2 mL) and TMP (220 µL, 1.304 mmol) were added. The solution was degassed with N₂ stream for 10 minutes, and then the reaction mixture was irradiated by two Kessil Tuna Blue lamps with vigorous stirring and fan cooling overnight. The reaction was diluted with DCM (5 mL) and water (10 mL), and the mixture was passed through a phase separator. The organic phase was collected and the solvent was evaporated *in vacuo.* Purification by silica gel chromatography (0-30% EtOAc in heptane) afforded methyl 4-((hexahydro-1H-cyclopenta[c]furan-5-yl)ethynyl)benzoate **C430** (165 mg, 56%). ¹H NMR (400 MHz, DMSO) δ 10.50 (s, 1H), 7.73 (d, J = 8.2 Hz, 1H), 6.98 - 6.90 (m, 2H), 3.88 (d, J = 1.2 Hz, 3H), 3.72 (ddt, J = 14.9, 8.2, 6.7 Hz, 3H), 3.23 (ddd, J = 21.3, 10.8, 7.1 Hz, 2H), 2.44 - 2.27 (m, 1H), 2.26 - 2.04 (m, 2H), 1.85 - 1.76 (m, 2H), 1.44 (td, J = 11.6, 8.3 Hz, 1H). ESI-MS m/z calc. 286.1205, found 287.13 (M+1)+.

Compound **519** was prepared via Larock cyclization with the corresponding halo-aniline as for compound **146,** followed by deprotection. Halo-anilines were prepared *via* Buchwald amination of the corresponding di-halo anilines. 4-[2-(3,3a,4,5,6,6a-hexahydro-1H-cyclopenta[c]furan-5-yl)-6-fluoro-1-(4-fluoro-3-methoxy-phenyl)-4-hydroxy-indol-3-yl]-2-hydroxy-benzoic acid (18.9 mg, 55%) ¹H NMR (400 MHz, MeOD) δ 7.83 (d, J = 7.9 Hz, 1H), 7.32 (ddd, J = 11.1, 8.5, 2.8 Hz, 1H), 7.24 - 7.16 (m, 1H), 7.03 - 6.95 (m, 3H), 6.19 (dd, J = 11.2, 2.1 Hz, 1H), 6.03 (dd, J = 9.6, 2.1 Hz, 1H), 3.99 - 3.86 (m, 5H), 3.72 - 3.68 (m, 1H), 3.58 (t, J = 6.6 Hz, 1H), 3.16 - 3.08 (m, 1H), 3.01 - 2.99 (m, 1H), 2.05 - 1.78 (m, 3H), 1.73 - 1.48 (m, 3H). ESI-MS *m*/*z* 522.14 (M+1) ⁺.

### Compounds 520 and 521

### 4-(6-fluoro-1-(4-fluoro-3-methoxyphenyl)-4-hydroxy-2-(3-hydroxy-1-methylcyclobutyl)-1H-indol-3-yl)-2-methoxybenzoic acid (520/521)

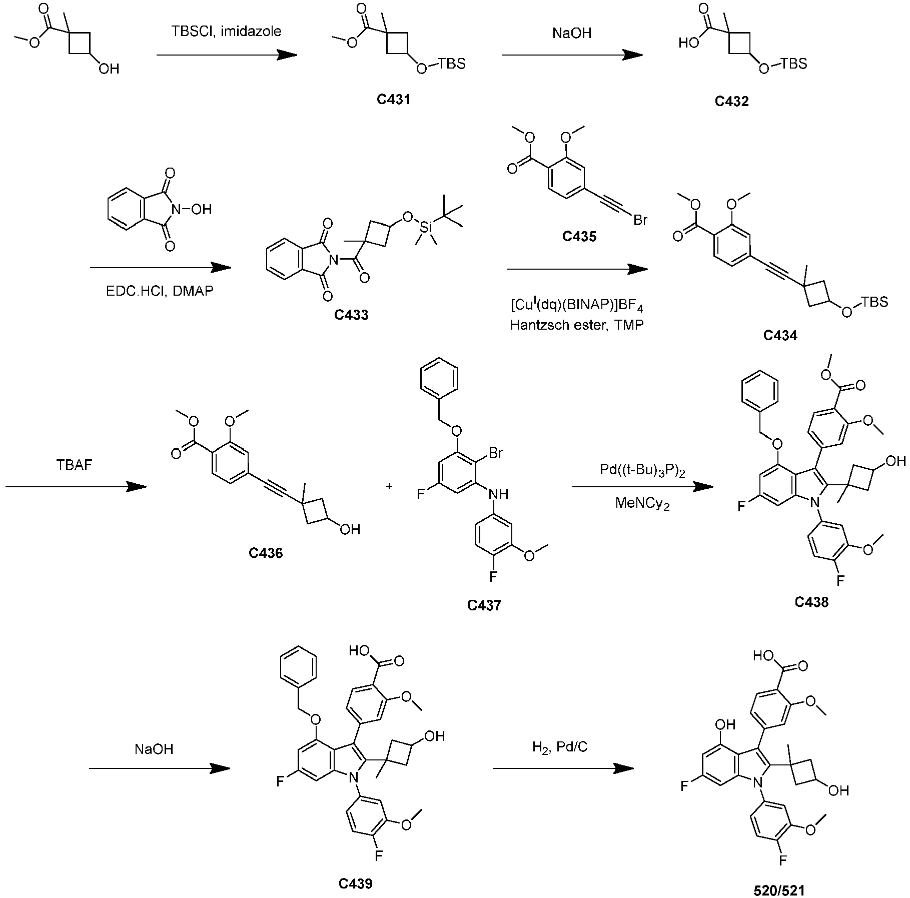

### Step 1: methyl 3-((tert-butyldimethylsilyl)oxy)-1-methylcyclobutane-1-carboxylate (C431)

To a solution of methyl 3-hydroxy-1-methylcyclobutane-1-carboxylate (5 g, 34.68 mmol) in DCM (74 mL) was added TBSCl (6.3 g, 41.80 mmol) and imidazole (2.8 g, 41.13 mmol), and the reaction mixture was stirred at room temperature overnight. The reaction mixture was washed with water, and the organic phase was concentrated *in vacuo.* Purification by silica gel chromatography (0-35% EtOAc in Heptane) afforded methyl 3-((tertbutyldimethylsilyl)oxy)-1-methylcyclobutane-1-carboxylate **C431** (6.7 g, 75%). ¹H NMR (400 MHz, Chloroform-d) δ 4.37 - 4.16 (m, 1H), 3.66 (dd, J = 4.5, 1.3 Hz, 3H), 2.71 (ddd, J = 10.0, 5.7, 2.1 Hz, 1H), 2.34 (ddd, J = 9.8, 7.7, 2.8 Hz, 1H), 2.17 (ddd, J = 9.5, 7.0, 2.8 Hz, 1H), 1.85 (ddd, J = 9.9, 7.1, 2.8 Hz, 1H), 1.36 (d, J = 1.4 Hz, 1H), 1.32 (s, 1H), 0.84 (s, 9H).

### Step 2: 3-((tert-butyldimethylsilyl)oxy)-1-methylcyclobutane-1-carboxylic acid (C432)

To a solution of methyl 3-((tert-butyldimethylsilyl)oxy)-1-methylcyclobutane-1-carboxylate **C431** (4.6 g, 17.80 mmol) in MeOH (50 mL) and THF (100 mL) was added NaOH (50 mL of 1 M, 50.00 mmol), and the reaction was heated at 60 °C for 3 hours. The reaction was allowed to cool to room temperature, and was concentrated *in vacuo.* Purification by silica gel chromatography (0-100% EtOAc in Heptane with 1% AcOH) afforded 3-((tertbutyldimethylsilyl)oxy)-1-methylcyclobutane-1-carboxylic acid **C432** (3.9 g, 90%) ESI-MS m/z calc. 244.14948, found 245.39 (M+1)+.

### Step 3: 2-(3-((tert-butyldimethylsilyl)oxy)-1-methylcyclobutane-1-carbonyl)isoindoline-1,3-dione (C433)

To solution of 3-((tert-butyldimethylsilyl)oxy)-1-methylcyclobutane-1-carboxylic acid **C432** (3100 mg, 12.68 mmol) and DMAP (21 mg, 0.1719 mmol) in DCM (45 mL) was added EDC.HCl (885 mg, 4.617 mmol) followed by 2-hydroxyisoindoline-1,3-dione (610 mg, 3.739 mmol), and the reaction was allowed to stir overnight at room temperature. The reaction was diluted with water and DCM, and the phases were separated. The organic phase was washed with EtOAc, and the combined organic phases were dried over anhydrous Na₂SO₄, filtered, and concentrated *in vacuo* to afford 2-(3-((tert-butyldimethylsilyl)oxy)-1-methylcyclobutane-1-carbonyl)isoindoline-1,3-dione **C433** (4.736 g), which was telescoped directly into the next reaction.

### Step 4: methyl 4-((3-((tert-butyldimethylsilyl)oxy)-1-methylcyclobutyl)ethynyl)-2-methoxybenzoate (C434)

A reaction vessel was charged with 2-(3-((tert-butyldimethylsilyl)oxy)-1-methylcyclobutane-1-carbonyl)isoindoline-1,3-dione **C433** (505 mg, 1.352 mmol), **C435** (447 mg, 1.661 mmol), [Cu(dq)(BINAP)]BF₄ (18 mg, 0.01674 mmol) and Hatnzsch ester (496 mg, 1.958 mmol), and DCM (6.4 mL) and TMP (450 µL, 2.666 mmol) were added. The solution was degassed with N₂ stream for 10 minutes, and then the reaction was irradiated by two Kessil Tuna Blue lamps with vigorous stirring and fan cooling overnight. The reaction was diluted with DCM (5 mL) and was washed with water (15 mL). The solution was passed through a phase separator, the organic phase was collected and concentrated *in vacuo.* Purification by silica gel chromatography (0-20% EtOAc in heptane) afforded methyl 4-((3-((tertbutyldimethylsilyl)oxy)-1-methylcyclobutyl)ethynyl)-2-methoxybenzoate **C434** (342 mg, 67%) as a clear colorless liquid. ¹H NMR (400 MHz, DMSO) δ 7.60 - 7.53 (m, 1H), 7.09 - 7.03 (m, 1H), 7.01 - 6.94 (m, 1H), 4.46 - 4.31 (m, 1H), 3.81 - 3.76 (m, 3H), 3.73 (s, 3H), 2.63 - 2.36 (m, 2H), 2.28 - 1.85 (m, 2H), 1.44 - 1.36 (m, 3H), 0.82 (s, 9H), 0.11 - -0.13 (m, 6H). ESI-MS m/z calc. 388.207, found 389.31 (M+1)+.

### Step 5: methyl 4-((3-hydroxy-1-methylcyclobutyl)ethynyl)-2-methoxybenzoate (C436)

To a solution of methyl 4-((3-((tert-butyldimethylsilyl)oxy)-1-methylcyclobutyl)ethynyl)-2-methoxybenzoate **C434** (340 mg, 0.8690 mmol) in THF (17 mL) was added TBAF (1000 µL of 1 M, 1.000 mmol) in THF dropwise, and the reaction was stirred for 30 minutes at room temperature. The reaction was diluted with water (50 mL) and DCM (50 mL). The mixture was passed through a phase separator, and the organic phase was collected and concentrated *in vacuo* to afford methyl 4-((3-hydroxy-1-methylcyclobutyl)ethynyl)-2-methoxybenzoate **C436** (238.4 mg, 99%) ESI-MS m/z calc. 274.1205, found 275.12 (M+1)+.

### Step 6: methyl 4-(4-(benzyloxy)-6-fluoro-1-(4-fluoro-3-methoxyphenyl)-2-(3-hydroxy-1-methylcyclobutyl)-1H-indol-3-yl)-2-methoxybenzoate (C438)

A reaction vessel was charged with **C437** (293 mg, 0.6902 mmol) and **C436** (240 mg, 0.8666 mmol), and dioxane (3.5 mL) and MeNCy₂ (740 µL, 3.455 mmol) were added. The solution was degassed with N₂ stream for 10 minutes, followed by addition of Pd((t-Bu)₃P)₂ (36 mg, 0.07044 mmol), and the reaction was heated to 110 °C overnight. The reaction was allowed to cool to room temperature, and was then diluted with water (10 mL) and DCM (10 mL). The mixture was passed through a phase separator, and the organic phase was collected and concentrated *in vacuo.* Purification by silica gel chromatography (0-60% EtOAc in heptane) afforded methyl 4-(4-(benzyloxy)-6-fluoro-1-(4-fluoro-3-methoxyphenyl)-2-(3-hydroxy-1-methylcyclobutyl)-1H-indol-3-yl)-2-methoxybenzoate **C438** (273 mg, 63%) as a light yellow solid. ESI-MS m/z calc. 613.2276, found 614.44 (M+1)+.

### Step 7: 4-(4-(benzyloxy)-6-fluoro-1-(4-fluoro-3-methoxyphenyl)-2-(3-hydroxy-1-methylcyclobutyl)-1H-indol-3-yl)-2-methoxybenzoic acid (C439)

To a solution of methyl 4-(4-(benzyloxy)-6-fluoro-1-(4-fluoro-3-methoxyphenyl)-2-(3-hydroxy-1-methylcyclobutyl)-1H-indol-3-yl)-2-methoxybenzoate **C438** (273 mg, 63%) in THF (5.8 mL) and MeOH (2.9 mL) was added aqueous NaOH (4 mL of 1 M, 4.000 mmol), and the reaction was heated to 50 °C for 3 hours. The reaction was allowed to cool to room temperature, and was concentrated *in vacuo.* The crude residue was taken up in minimal water, and aqueous HCl (2 mL of 2 M, 4.000 mmol) was added, and the solution was concentrated *in vacuo.* Purification by reversed-phase chromatography (Column: C18. Gradient: 0-100% MeCN in water with 0.1% TFA) afforded 4-(4-(benzyloxy)-6-fluoro-1-(4-fluoro-3-methoxyphenyl)-2-(3-hydroxy-1-methylcyclobutyl)-1H-indol-3-yl)-2-methoxybenzoic acid **C439** (126 mg, 30%) as an off-white solid. ESI-MS m/z calc. 599.2119, found 600.36 (M+1)+.

### Step 8: 4-(6-fluoro-1-(4-fluoro-3-methoxyphenyl)-4-hydroxy-2-(3-hydroxy-1-methylcyclobutyl)-1H-indol-3-yl)-2-methoxybenzoic acid (520, 521)

A reaction vessel was charged with Pd/C (40 mg of 10% w/w, 0.03759 mmol) under N₂ atmosphere, and a solution of 4-(4-(benzyloxy)-6-fluoro-1-(4-fluoro-3-methoxyphenyl)-2-(3-hydroxy-1-methylcyclobutyl)-1H-indol-3-yl)-2-methoxybenzoic acid **C439** (126 mg, 0.2097 mmol) in MeOH (4.4 mL) was added. The system was evacuated and refilled with N₂ (3x), followed by H₂ (3x, balloon). The reaction was allowed to stir at room temperature under H₂ atmosphere for 3 hours. The reaction system was evacuated and backfilled with N₂, and then quenched by addition of DCM. The solution was filtered through a pad of Celite^{®} and washed with DCM, and the filtrate was evaporated *in vacuo.* Purification of **520/521** (racemic mixture of *trans* isomers) by SFC separation : prepped at 20% MeOH(5 mM Ammonia) on AS-H afforded **520** (42 mg, 39%) as a white solid. ¹H NMR (400 MHz, MeOD) δ 7.65 (d, J = 7.8 Hz, 1H), 7.29 (dd, J = 11.1, 8.5 Hz, 1H), 7.19 (dd, J = 7.6, 2.5 Hz, 1H), 7.13 (s, 1H), 7.03 (dd, J = 13.1, 7.5 Hz, 2H), 6.17 (dd, J = 11.4, 2.3 Hz, 1H), 5.94 (dd, J = 9.7, 2.3 Hz, 1H), 3.96 (d, J = 6.0 Hz, 1H), 3.91 (s, 3H), 3.89 (s, 3H), 2.43 (dd, J = 13.7, 7.0 Hz, 2H), 1.91 (s, 3H), 1.40 (d, J = 12.6 Hz, 2H). ESI-MS m/z calc. 509.165, found 510.31 (M+1)+.

### Step 9: Separation of isomers: 20% MeOH (5 mM Ammonia) on AS-H

First eluting **C520:** ¹H NMR (400 MHz, MeOD) δ 7.61 (d, J = 7.7 Hz, 1H), 7.29 (dd, J = 11.1, 8.5 Hz, 1H), 7.18 (d, J = 7.5 Hz, 1H), 7.11 (s, 1H), 7.02 (t, J = 7.8 Hz, 2H), 6.20 - 6.14 (m, 1H), 5.95 (d, J = 9.6 Hz, 1H), 3.99 (p, J = 7.5 Hz, 1H), 3.92 - 3.87 (m, 6H), 3.68 (d, J = 5.2 Hz, 1H), 1.93 (dt, J = 13.3, 7.6 Hz, 2H), 1.74 (s, 2H), 1.64 (s, 3H). ESI-MS *m*/*z* 510.35 (M+1)⁺.

Second eluting **C521:** 4-[6-fluoro-1-(4-fluoro-3-methoxy-phenyl)-4-hydroxy-2-(3-hydroxy-1-methyl-cyclobutyl)indol-3-yl]-2-methoxy-benzoic acid (42 mg, 39%) ¹H NMR (400 MHz, MeOD) δ 7.65 (d, J = 7.8 Hz, 1H), 7.29 (dd, J = 11.1, 8.5 Hz, 1H), 7.19 (dd, J = 7.6, 2.5 Hz, 1H), 7.13 (s, 1H), 7.03 (dd, J = 13.1, 7.5 Hz, 2H), 6.17 (dd, J = 11.4, 2.3 Hz, 1H), 5.94 (dd, J = 9.7, 2.3 Hz, 1H), 3.96 (d, J = 6.0 Hz, 1H), 3.91 (s, 3H), 3.89 (s, 3H), 2.43 (dd, J = 13.7, 7.0 Hz, 2H), 1.91 (s, 3H), 1.40 (d, J = 12.6 Hz, 2H). ESI-MS *m*/*z* calc. 509.165, found 510.31 (M+1)⁺.

### Compounds 522 and 523

Compounds in Table 28 made via Larock cyclization using procedures analogous to the procedure used to prepare compound **519.** Alkynes were prepared via photochemical mediated Br-alkyne coupling (such as **C430**).

**Table 28. Structure and physicochemical data for compounds 522 and 523**

| **Compound** | **Structure** | ¹H NMR; **LCMS m/z** [M+H]⁺ |
|---|---|---|
| **522** | 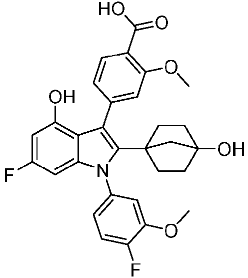 | ¹H NMR (400 MHz, DMSO) δ 12.48 (s, 1H), 9.50 (s, 1H), 7.63 (d, J = 7.8 Hz, 1H), 7.42 (dd, J = 11.2, 8.5 Hz, 1H), 7.27 (ddd, J = 24.1, 7.9, 2.5 Hz, 1H), 7.12 - 6.96 (m, 3H), 6.16 (dd, J = 11.4, 2.2 Hz, 1H), 5.89 (dt, J = 9.6, 2.1 Hz, 1H), 3.86 (s, 3H), 3.81 (s, 3H), 1.93 - 1.77 (m, 2H), 1.52 - 1.35 (m, 4H), 1.33 - 1.21 (m, 2H), 1.14 (s, 2H). ESI-MS *m*/*z* 536.18 (M+1) ⁺ |
| **523** | 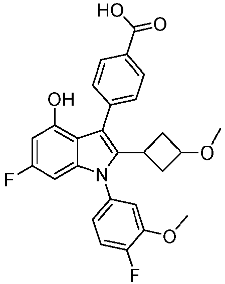 | ¹H NMR (400 MHz, DMSO) δ 9.89 (s, 1H), 8.02 - 7.72 (m, 1H), 7.58 - 7.21 (m, 2H), 7.07 (d, *J =* 8.1 Hz, 1H), 6.35 - 6.07 (m, 1H), 3.99 (t, *J* = 8.9 Hz, 1H), 3.89 (d, *J* = 1.7 Hz, 2H), 3.42 (d, *J =* 7.6 Hz, 1H), 2.94 (s, 1H), 2.86 (s, 1H), 1.86 (s, 1H), 1.67 (t, *J =* 10.9 Hz, 1H), 1.36 (d, *J* = 6.6 Hz, 1H). -MS *m*/*z* 480.43 (M+1) ⁺ |

### Compound S522

### 5-[6-fluoro-1-(4-fluoro-3-methyl-phenyl)-4-hydroxy-2-isopropyl-indol-3-yl]pyridine-3-carboxylic acid (S522)

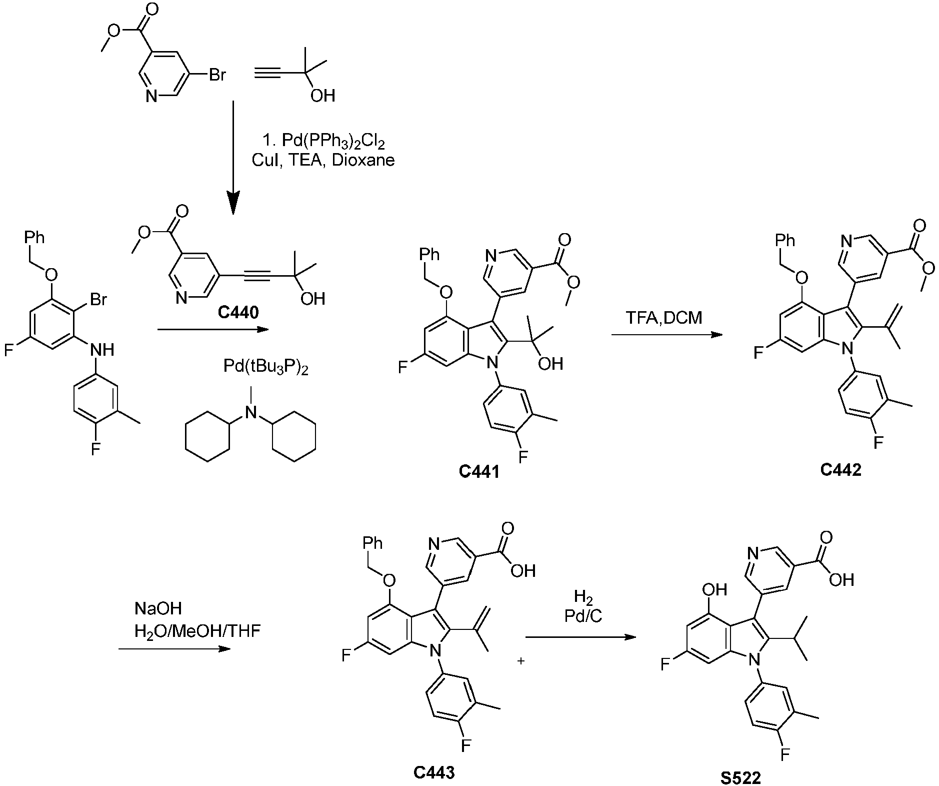

### Step 1: Methyl 5-(3-hydroxy-3-methyl-but-1-ynyl)pyridine-3-carboxylate (C440)

To a solution of methyl 5-bromopyridine-3-carboxylate (500 mg, 2.314 mmol) and 2-methylbut-3-yn-2-ol (224 µL, 2.311 mmol) in 1,4-dioxane (2.6 mL) and Et₃N (2.6 mL) was added, in one portion, dichloropalladium;triphenylphosphane (80.3 mg, 0.1144 mmol) and iodocopper (22.4 mg, 0.1176 mmol). The reaction was stirred under nitrogen at 75 °C overnight. Water and DCM were added. The phases were separated on a phase separator. Purification was done on reverse phase chromatography (ACN/Water + 0.2% FA) to give methyl 5-(3-hydroxy-3-methyl-but-1-ynyl)pyridine-3-carboxylate **C440** (149 mg, 27%) ESI-MS m/z 220.21 (M+1)⁺.

### Step 2: Methyl 5-[4-benzyloxy-6-fluoro-1-(4-fluoro-3-methyl-phenyl)-2-(1-hydroxy-1-methylethyl)indol-3-yl]pyridine-3-carboxylate (C441)

A vial containing 3-benzyloxy-2-bromo-5-fluoro-N-(4-fluoro-3-methylphenyl)aniline (160 mg, 0.3958 mmol) and methyl 5-(3-hydroxy-3-methyl-but-1-ynyl)pyridine-3-carboxylate **C440** (86.7 mg, 0.3955 mmol) in anhydrous 1,4-dioxane (2.4 mL) and N-cyclohexyl-N-methyl-cyclohexanamine (474 µL, 2.213 mmol) was degassed with N₂ for 5 minutes. Palladium;tritert-butylphosphane (23.2 mg, 0.04540 mmol) was added and the mixture was stirred at 110 °C. The reaction was allowed to stir overnight. Water and DCM were added. The phases were separated on a phase separator. Purification was done on silica gel (Eluent: Ethyl acetate/Heptanes) to give methyl 5-[4-benzyloxy-6-fluoro-1-(4-fluoro-3-methyl-phenyl)-2-(1-hydroxy-1-methyl-ethyl)indol-3-yl]pyridine-3-carboxylate **C441** (125 mg, 49%) ESI-MS m/z 543.25 (M+1)+.

### Step 3: Methyl 5-[4-benzyloxy-6-fluoro-1-(4-fluoro-3-methyl-phenyl)-2-isopropenyl-indol-3-yl]pyridine-3-carboxylate (C442)

Methyl 5-[4-benzyloxy-6-fluoro-1-(4-fluoro-3-methyl-phenyl)-2-(1-hydroxy-1-methyl-ethyl)indol-3-yl]pyridine-3-carboxylate **C441** (125 mg, 0.2304 mmol) was dissolved in DCM (2.4 mL) and cooled down to 0 °C. 2,2,2-trifluoroacetic acid (53.0 µL, 0.6879 mmol) added and stirred for 2 hours. NaHCO₃ and DCM were added, and the organic layer was collected through phase separator to give methyl 5-[4-benzyloxy-6-fluoro-1-(4-fluoro-3-methylphenyl)-2-isopropenyl-indol-3-yl]pyridine-3-carboxylate **C442** (82 mg, 57%) ESI-MS m/z 525.25 (M+1)+.

### Step 4: 5-[4-benzyloxy-6-fluoro-1-(4-fluoro-3-methyl-phenyl)-2-isopropenyl-indol-3-yl]pyridine-3-carboxylic acid (C443)

To a solution of methyl 5-[4-benzyloxy-6-fluoro-1-(4-fluoro-3-methyl-phenyl)-2-isopropenyl-indol-3-yl]pyridine-3-carboxylate **C442** (82 mg, 0.1563 mmol) in MeOH (602 µL) and THF (1.1 mL) was added sodium hydroxide (781 µL of 1 M, 0.7810 mmol). The mixture was heated at 50 °C for 1 hour. LCMS showed the reaction went to completion. HCl (725 µL of 2 M, 1.450 mmol) and DCM were added. The organic layer was collected through a phase separator and concentrated to give 5-[4-benzyloxy-6-fluoro-1-(4-fluoro-3-methyl-phenyl)-2-isopropenyl-indol-3-yl]pyridine-3-carboxylic acid **C443** (38 mg, 38%) ESI-MS m/z 511.21 (M+1)⁺.

### Step 5: 5-[6-fluoro-1-(4-fluoro-3-methyl-phenyl)-4-hydroxy-2-isopropyl-indol-3-yl]pyridine-3-carboxylic acid (S522)

5-[4-benzyloxy-6-fluoro-1-(4-fluoro-3-methyl-phenyl)-2-isopropenyl-indol-3-yl]pyridine-3-carboxylic acid **C443** (48 mg, 0.09402 mmol) was suspended in MeOH (2.5 mL) and dihydroxypalladium (26 mg of 20% w/w, 0.03703 mmol) under N₂ atmosphere. The system was evacuated and refilled with N₂ 3x, followed by H2 3x. The reaction was allowed to stir at room temperature. After 2 hours, the reaction mixture was filtered over Celite^{®} and concentrated. Purified by reverse phase chromatography (ACN/0.1% TFA) to give 5-[6-fluoro-1-(4-fluoro-3-methyl-phenyl)-4-hydroxy-2-isopropyl-indol-3-yl]pyridine-3-carboxylic acid **S522** (5.5 mg, 13%) ¹H NMR (300 MHz, Methanol-d4) δ 9.10 (s, 1H), 8.87 (s, 1H), 8.63 (s, 1H), 7.34 (d, J = 6.9 Hz, 1H), 7.28 (d, J = 7.4 Hz, 2H), 6.23 (d, J = 12.6 Hz, 1H), 6.00 (d, J = 9.5 Hz, 1H), 3.11 - 3.02 (m, 1H), 2.38 (s, 3H), 1.11 (d, J = 7.1 Hz, 6H). ESI-MS m/z 423.28 (M+1)⁺.

### Compound 524

### 4-[6-fluoro-1-(4-fluoro-3-methoxy-phenyl)-4-hydroxy-2-isopropyl-indol-3-yl]-2-methoxybenzoic acid (524)

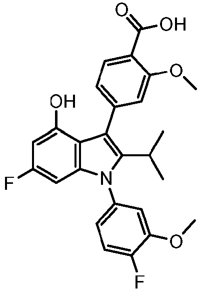

4-[6-fluoro-1-(4-fluoro-3-methoxy-phenyl)-4-hydroxy-2-isopropyl-indol-3-yl]-2-methoxy-benzoic acid **524** was made using the same method as for compound **S522** from methyl 4-bromo-2-methoxybenzoate (29.7 mg, 36%). ¹H NMR (400 MHz, Methanol-d4) δ 7.85 (d, J = 7.9 Hz, 1H), 7.31 (dd, J = 11.1, 8.5 Hz, 1H), 7.24 - 7.17 (m, 1H), 7.17 - 7.08 (m, 2H), 7.03 - 6.93 (m, 1H), 6.19 (dd, J = 11.2, 2.3 Hz, 1H), 6.01 (dd, J = 9.6, 2.3 Hz, 1H), 3.94 (s, 3H), 3.89 (s, 3H), 3.14 - 3.02 (m, 1H), 1.11 (d, J = 7.1 Hz, 6H).ESI-MS m/z 468.34 (M+1)+.

### Compounds 525-531

Compounds from Table 29 were prepared by Larock cyclization with disubstituted alkynes, as for compound **146.** The disubstituted alkynes in Table 29 were prepared via Sonogashira coupling between the corresponding aryl halide and the indicated terminal alkyne.

**Table 29. Method of preparation, structure, and physicochemical data for compounds 525-531**

| **Compound** | **Structure** | **Terminal Alkyne** | **Disubstituted alkyne** | ¹H NMR; **LCMS m/z** [M+H]⁺ |
|---|---|---|---|---|
| **525** | 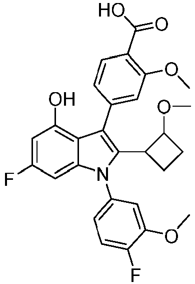 | 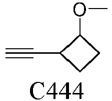 | | |
| **526** | 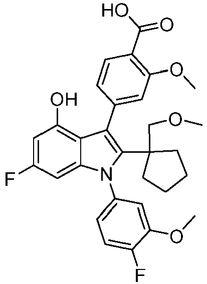 | 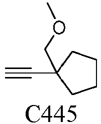 | | ¹H NMR (400 MHz, DMSO-d6) δ 12.48 (s, 1H), 9.48 (s, 1H), 7.59 (d, J = 7.8 Hz, 1H), 7.48 - 7.39 (m, 1H), 7.36 - 7.18 (m, 2H), 7.16 - 6.96 (m, 2H), 6.15 (dd, J = 11.4, 2.3 Hz, 1H), 5.80 (dt, J = 9.7, 2.4 Hz, 1H), 3.86 (d, J = 2.5 Hz, 3H), 3.81 (d, J = 1.6 Hz, 3H), 3.33 - 3.16 (m, 5H), 1.69 - 1.48 (m, 1H), 1.39 (d, J = 27.9 Hz, 6H), 1.19 (q, J = 13.6, 12.3 Hz, 1H). ESI-MS m/z 538.48 (M+1)+ |
| **527** | 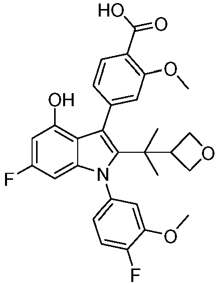 | | 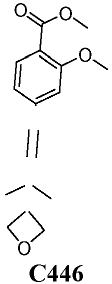 | ¹H NMR (400 MHz, DMSO-d6) δ 12.52 (s, 1H), 9.43 (s, 1H), 7.65 (dd, J = 7.8, 2.6 Hz, 1H), 7.45 (dd, J = 11.4, 8.5 Hz, 1H), 7.32 (dd, J = 7.7, 2.5 Hz, 1H), 7.17 - 6.92 (m, 3H), 6.11 (dt, J = 11.4, 2.4 Hz, 1H), 5.85 - 5.64 (m, 1H), 4.26 (td, J = 8.8, 4.3 Hz, 4H), 3.88 (t, J = 1.8 Hz, 3H), 3.82 (t, J = 3.1 Hz, 3H), 3.56 - 3.38 (m, 1H), 1.21 - 1.06 (m, 6H). ESI-MS m/z 524.48 (M+1)+ |
| **528** | 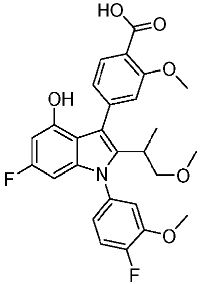 | 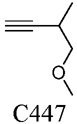 | | ¹H NMR (300 MHz, DMSO-d6) δ 9.97 (s, 1H), 7.58 - 7.39 (m, 2H), 7.27 (ddd, J = 26.2, 7.7, 2.4 Hz, 1H), 7.11 - 6.94 (m, 3H), 6.29 (dd, J = 11.5, 2.2 Hz, 1H), 6.02 (ddd, J = 8.3, 6.1, 2.2 Hz, 1H), 3.87 (d, J = 2.7 Hz, 3H), 3.79 (s, 3H), 3.29 - 3.12 (m, 3H), 3.07 (d, J = 6.2 Hz, 3H), 1.00 (dd, J = 8.6, 6.1 Hz, 3H). ESI-MS m/z 498.0 (M+1)+ |
| **529** | 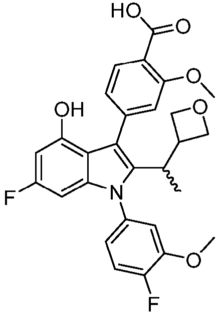 | | 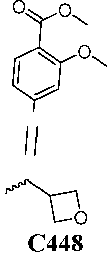 | ¹H NMR (400 MHz, DMSO-d6) δ 12.58 (s, 1H), 10.52 - 9.63 (m, 1H), 7.71 - 7.59 (m, 1H), 7.48 (ddd, J = 11.6, 8.5, 3.4 Hz, 1H), 7.25 (td, J = 22.8, 21.5, 8.7 Hz, 1H), 7.11 - 6.88 (m, 3H), 6.41 - 5.98 (m, 2H), 4.69 - 4.19 (m, 2H), 4.05 - 3.61 (m, 7H), 3.26 - 2.59 (m, 1H), 1.27 - 1.04 (m, 3H). ESI-MS m/z calc. 509.165, found 510.0 (M+1)+ |
| **530** | 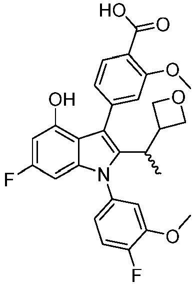 | | 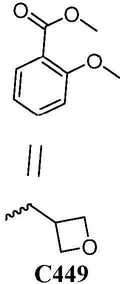 | ¹H NMR (400 MHz, DMSO-d6) δ 12.60 (s, 1H), 10.52 - 9.61 (m, 1H), 7.65 (dd, J = 30.3, 7.8 Hz, 1H), 7.52 - 6.87 (m, 4H), 6.41 - 5.98 (m, 2H), 4.71 - 3.62 (m, 9H), 3.27 - 2.57 (m, 1H), 1.38 - 1.06 (m, 4H). ESI-MS m/z510.0 (M+1)+ |
| **531** | 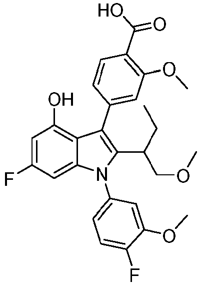 | | 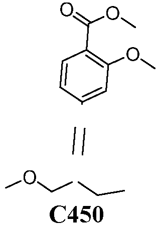 | ¹H NMR (400 MHz, DMSO-d6) δ 12.53 (s, 1H), 9.73 (d, J = 8.7 Hz, 1H), 7.65 (d, J = 7.9 Hz, 1H), 7.46 (dd, J = 11.3, 8.5 Hz, 1H), 7.30 - 7.14 (m, 1H), 7.10 (t, J = 2.9 Hz, 1H), 7.07 - 6.94 (m, 2H), 6.25 (dt, J = 11.5, 2.1 Hz, 1H), 6.05 (ddd, J = 15.5, 9.7, 2.2 Hz, 1H), 3.86 (d, J = 4.3 Hz, 3H), 3.81 (s, 3H), 3.32 (d, J = 16.7 Hz, 2H), 3.13 (d, J = 8.7 Hz, 3H), 2.93 (h, J = 8.3, 7.5 Hz, 1H), 1.41 - 1.09 (m, 2H), 0.65 (td, J = 7.3, 5.3 Hz, 3H). ESI-MS m/z c 512.0 (M+1)+ |

### Compound 532

### 4-[6-fluoro-1-(4-fluoro-3-methoxy-phenyl)-4-hydroxy-2-isopropyl-indol-3-yl]benzoic acid

4-[6-fluoro-1-(4-fluoro-3-methoxy-phenyl)-4-hydroxy-2-isopropyl-indol-3-yl]benzoic acid **532** is prepared using the same method as **S522** from methyl 4-bromobenzoate. ¹H NMR (400 MHz, Methanol-d4) δ 8.02 (d, J = 7.8 Hz, 2H), 7.54 (d, J = 8.0 Hz, 2H), 7.31 (dd, J = 11.1, 8.5 Hz, 1H), 7.15 (dd, J = 7.7, 2.4 Hz, 1H), 7.04 - 6.95 (m, 1H), 6.82 (dd, J = 11.1, 8.8 Hz, 1H), 6.26 (dd, J = 8.9, 3.4 Hz, 1H), 3.88 (s, 3H), 3.14 - 3.00 (m, 1H), 1.10 (d, J = 7.1 Hz, 6H). ESI-MS m/z c438.22 (M+1)+.

### Preparation of C444

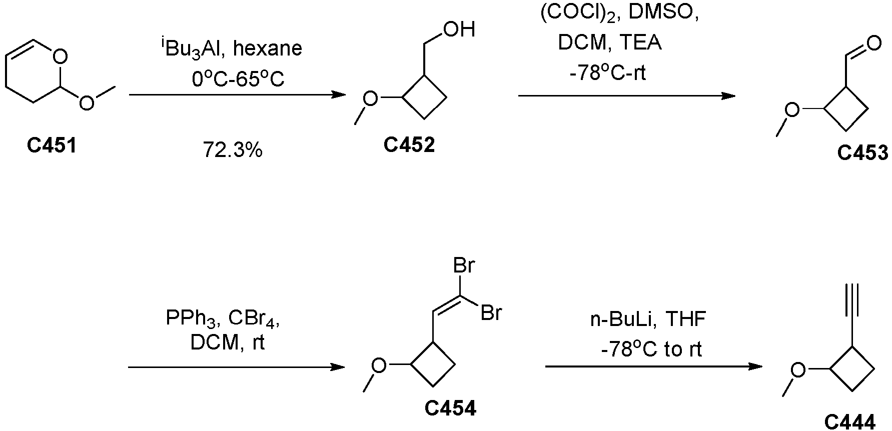

### Step 1: 2-methoxy-3,4-dihydro-2H-pyran (C452)

To a solution of i-Bu₃Al (500 mL, 500.0 mmol, 2.0 eq, 1M) in hexane (500 mL) at 0 °C, **C451** (28.5 g, 250.0 mmol, 1.0 eq) in hexane (50 mL) was added slowly under N₂. After stirring for 30 minutes, the reaction mixture was refluxed for overnight. Then the reaction was quenched by adding of 10% H₂SO₄ (30 mL) at 0 °C and diluted with 500 mL of water. The aqueous phase was extracted with EtOAc (500 mL x 3). The combined organic phase was dried over Na₂SO₄, then concentrated to give a crude material, which was purified by vacuum distillation to give compound **C452** (21.0 g, 72.3%) as a colorless oil. ¹H NMR (400 MHz, CDCl₃):δ 3.69-3.59 (m, 3H), 3.25 (s, 3H), 2.45-2.34 (m, 1H), 2.17-2.09 (m, 1H), 1.91 (s, 1H), 1.83-1.69 (m, 2H), 1.34-1.23 (m, 1H).

### Step 2: (2-methoxycyclobutyl)methanol (C453)

A solution of oxalyl chloride (26.0 g, 206.0 mmol, 2.0 eq) in DCM (70 mL) was cooled to -78 °C, and DMSO (32.0 g, 412.0 mmol, 4.0 eq) in DCM (70 mL) was added dropwise. After 10 minutes of stirring, **C452** (12.0 g, 103.0 mmol, 1.0 eq) was added dropwise. The reaction was stirred for 30 minutes at -78 °C. Then TEA (62.0 g, 618.0 mmol, 6.0 eq) was added, followed by adding 200 mL of water. After extraction with DCM (150 mL, x2), the organic layers **(C453** in DCM (450 mL)) were dried over Na₂SO₄, which was not further purified prior to the next step.

### Step 3: 1-(2,2-dibromovinyl)-2-methoxycyclobutane (C454)

To a mixture of **C453** (DCM solution, 1.0 eq, 500 mL), PPh₃ (217.0 g, 824 mmol, 8.0 eq) and CBr₄ (137.0 g, 412 mmol, 4.0 eq) were added. The resulting mixture was stirred at room temperature for overnight. Water (500 mL) was added to the mixture, and the solution was extracted with DCM (500 mL, x2). The combined organic phase was washed with brine, dried over Na₂SO₄, and concentrated *in vacuo.* The obtained crude oil was purified by column chromatography (petroleum ether/EtOAc = 10: 1) to give **C454** (15.5 g, two step yield 55.6%) as a clear yellow oil. ¹H NMR (400 MHz, CDCl₃): δ 6.47 (d, J = 9.0 Hz, 1H), 3.73-3.61 (m, 1H), 3.20 (s, 3H), 3.06-2.94 (m, 1H), 2.25-2.11 (m, 1H), 2.01 (m, 1H), 1.87-1.73 (m, 1H), 1.47-1.30 (m, 1H).

### Step 4: Synthesis of 1-ethynyl-2-methoxycyclobutane (C444)

To a mixture of **C454** (15.5 g, 57.6 mmol, 1.0 eq) in THF (150 mL) at -78 °C, n-BuLi (50.0 mL, 126.7 mmol, 2.2 eq) was added dropwise. After stirring for 1.5 hours, the reaction was quenched with saturated aqueous solution of sodium potassium tartrate at -78 °C. The aqueous phase was extracted with EtOAc (100 mL, x2), and the combined organic phase was washed with brine, dried over Na₂SO₄, and concentrated to give **C444** (6.2 g) as a clear yellow oil, which was used without further purification for the next step.
¹H NMR (400 MHz, CDCl₃): δ 7.72 (d, J = 7.9 Hz, 1H), 7.01-6.98 (m, 2H), 3.95-3.83 (m, 7H), 3.38 (s, 3H), 3.09-2.93 (m, 1H), 2.23-2.03 (m, 2H), 1.89-1.78 (m, 1H), 1.69-1.52 (m, 1H). LCMS: 275.1 ([M+H]+).

### Preparation of C445

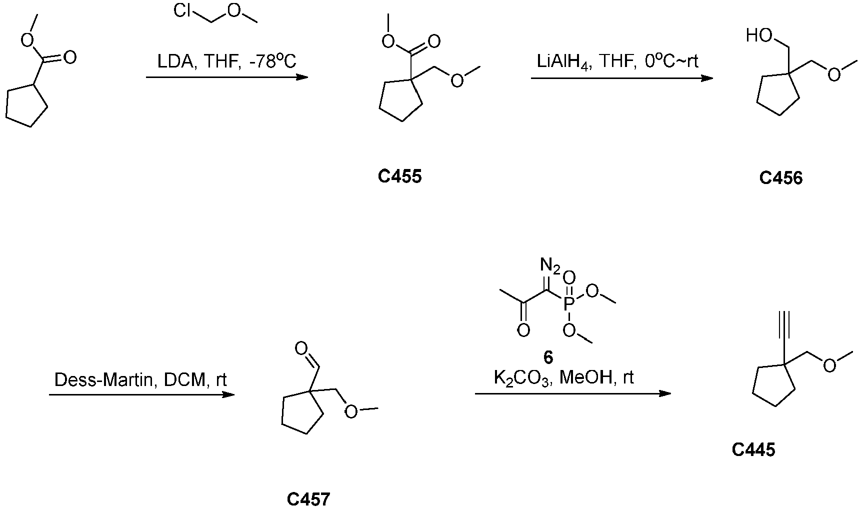

### Step 1: Methyl 1-(methoxymethyl)cyclopentane-1-carboxylate (C455)

To a solution of methyl cyclopentanecarboxylate (50.0 g, 0.39 mol, 1.0 eq) in THF (500 mL) was added LDA (205 mL, 0.39 mol, 1.0 eq, 2 M) at -78 °C under N₂ atmosphere. The resulting mixture was stirred at -40 °C for 30 minutes. Then MOM-Cl (47.1 g, 0.59 mol, 1.5 eq.) was added at -40 °C. Then the reaction mixture was stirred at room temperature for overnight. The reaction was quenched by H₂O, and the solvent was removed under reduced pressure. The residue was extracted with EtOAc and H₂O. The organic layer was concentrated to give the crude product (70.1 g, crude) as a yellow oil, which was used directly without further purification. ¹H NMR (300 MHz, CDCl₃): δ 3.66 (s, 3H), 3.41 (s, 2H), 3.29 (s, 3H), 2.02-1.98 (m, 2H), 1.59-1.51 (m, 6H).

### Step 2: (1-(methoxymethyl)cyclopentyl)methanol (C456)

To a solution of **C455** (70.1 g, 0.41 mol, 1 eq., crude) in THF (700 mL) was added LiAlH₄ (23.2 g, 0.62 mol, 1.5 eq) slowly at 0 °C under N₂ atmosphere. The resulting mixture was stirred at 0 °C for 1 hour. The reaction mixture was successively quenched by H₂O (23 mL), then 15% aqueous NaOH (23 mL) and H₂O (70 mL), dried over Na₂SO₄, and filtered. The filtrates were concentrated and purified by silica gel column chromatography (Petroleum ether/EtOAc = 10:1) to afford the desired product **C456** (29.5 g, 52.5% for two steps) as a light yellow oil. ¹H NMR (300 MHz, CDCl₃): δ 3.44 (s, 2H), 3.34-3.26 (m, 5H), 2.98 (brs, 1H), 1.66-1.20 (m, 8H).

### Step 3: 1-(methoxymethyl)cyclopentane-1-carbaldehyde (C457)

To a solution of **C456** (29.5 g, 0.20 mol, 1.0 eq.) in DCM (1000 mL) was added Dess-Martin periodinane (170.0 g, 0.40 mol, 2.0 eq.) at 0 °C. The reaction mixture was quenched by aqueous NaHCO₃ and extracted with DCM (300 mL, x2). The organic layer was concentrated. The residue was purified by silica gel column chromatography (Petroleum ether/EtOAc = 5:1) to afford **C457** (23.0 g, crude, QNMR~69% purity) as a yellow oil. ¹H NMR (400 MHz, CDCl₃): δ 9.54 (s, 1H), 3.57 (s, 2H), 3.47 (s, 3H), 1.96-1.94 (m, 2H), 1.67-1.60 (m, 4H), 1.55-1.50 (m, 2H).

### Step 4: Ethynyl-1-(methoxymethyl)cyclopentane (C445)

To solution of **C457** (23.0 g, 0.12 mol, 1.0 eq, 69% purity) and K₂CO₃ (33.3 g, 0.24 mol, 2.0 eq.) in MeOH (250 mL) was added dimethyl (1-diazo-2-oxopropyl)phosphonate (26.8 g, 0.14 mol, 1.2 eq.) at 0 °C under N₂ atmosphere. The resulting mixture was stirred at room temperature for 2 hours. The reaction mixture was diluted with diethyl ether (100 mL) and washed with saturated aqueous NH₄Cl. The organic phase was concentrated and purified by column chromatography (Petroleum ether/EtOAc=100:1) to give **C445** (12.5 g) as a yellow liquid. ¹H NMR (400 MHz, CDCl₃): δ 3.41 (s, 3H), 3.31 (s, 2H), 2.15 (s, 1H), 1.93-1.61(m, 8H).

### Preparation of C446

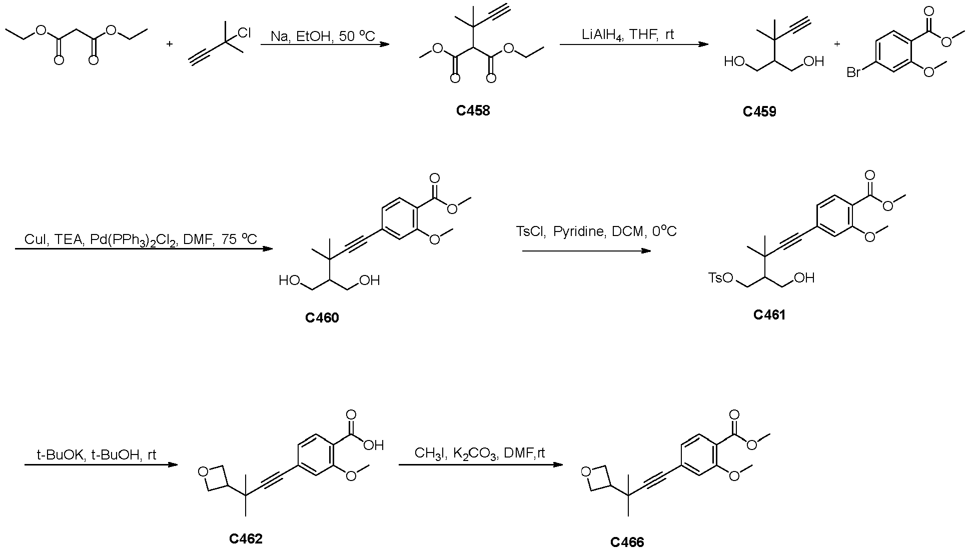

### Step 1: 1-ethyl 3-methyl 2-(2-methylbut-3-yn-2-yl)malonate (C458)

Diethyl malonate (178.2 g, 1.1 mol, 1.0 eq) was added to a freshly prepared solution of sodium ethoxide [Na (25.6 g, 1.1 mol, 1.0 eq)] in EtOH (2800 mL) warmed to 60 °C. After 1 hour, 3-chloro-3-methylbut-1-yne (114.1 g, 1.1 mol, 1.0 eq.) was added to the mixture. The mixture was stirred at 65 °C for 15 hours. The reaction mixture was cooled to room temperature and filtered, then the filtrate was concentrated. The residue was acidified with 2 N HCl and extracted with EtOAc (300 mL, x2). The organic layer was washed with brine, dried over Na₂SO₄ and concentrated to afford the crude material, which was purified using a silica gel column (eluted with petroleum ether/EtOAc = 100:1) to give **C468** (84.4 g, 33.9%) as a yellow oil. ¹H NMR (400MHz, CDCl₃): δ 4.20 (q, J = 7.1 Hz, 4H), 3.43 (s, 1H), 2.18 (s, 1H), 1.44 (s, 6H), 1.26 (t, J = 7.1 Hz, 6H). LCMS: 227.1 ([M+H]+).

### Step 2: 1-ethyl 3-methyl 2-(2-methylbut-3-yn-2-yl)malonate (C459)

To a solution of **C458** (84.4 g, 373.0 mmol, 1.0 eq.) in THF (1.0 L) was added portion-wise LiAlH₄ (42.5 g, 1.12 mol, 3.0 eq) at 0 °C under N₂. Then the reaction mixture was stirred at room temperature for 2 hours. The mixture was diluted with DCM (300 mL) and cooled to -20 °C, then H₂O (42.5 mL) was slowly added dropwise to the mixture. 15% NaOH aqueous solution (42.5 mL) and H₂O (127.5 mL) was added to the mixture. The mixture was warmed to room temperature and stirred for 15 minutes. Then Na₂SO₄ (100 g) was added and stirred for another 15 minutes. The mixture was filtered, and the filtrate was concentrated to afford the crude product, which was purified using a silica gel column (eluted with petroleum ether/EtOAc = 50:1) to give **C459** (36.5 g, 68.9%) as a yellow oil. ¹H NMR (400MHz, CDCl₃): δ 4.02 (dd, J = 10.9, 4.0 Hz, 2H), 3.92 (dd, J = 10.9, 8.0 Hz, 2H), 2.65 (s, 2H), 2.16 (s, 1H), 1.76-1.70 (m, 1H), 1.26 (s, 6H). MS: 143.1 ([M+H]+).

### Step 3: methyl 4-(5-hydroxy-4-(hydroxymethyl)-3, 3-dimethylpent-1-yn-1-yl)-2-methoxybenzoate (C460)

Under N₂, to a mixture of **C459** (36.5 g, 256.7 mmol, 1.0 eq.), methyl 4-bromo-2-methoxybenzoate (69.2 g, 282.4 mmol, 1.1 eq.), TEA (51.9 g, 513.4 mmol, 2.0 eq.) and CuI (4.9 g, 25.7 mmol, 0.1 eq.) in DMF (400 mL), Pd(PPh₃)₂Cl₂ (18.0 g, 25.7 mmol, 0.1 eq.) was added at room temperature. The resulting reaction mixture with N₂ for 10 minutes. Then the reaction mixture was heated to 75 °C for 2 hours. The mixture was cooled to room temperature, then diluted with H₂O (300 mL) and extracted with EtOAc (200 mL, x2). The organic layer was washed with brine, dried over Na₂SO₄, and concentrated to afford a crude product which was purified using a silica gel column (eluted with DCM/ MeOH = 100:1) to give **C460** (31.0 g, 39.4%) as a yellow oil. ¹H NMR (400MHz, CDCl₃): δ 7.72 (d, J = 8.0 Hz, 1H), 6.98-6.83 (m, 2H), 4.11 (dd, J = 10.8, 3.9 Hz, 2H), 3.98 (dd, J = 10.8, 8.3 Hz, 2H), 3.90 (s, 3H), 3.87 (s, 3H), 2.29 (brs, 2H), 1.89-1.84 (m, 1H), 1.35 (s, 6H). LCMS: 307.2 ([M+H]+).

### Step 4: methyl 4-(5-hydroxy-3, 3-dimethyl-4-((tosyloxy)methyl)pent-1-yn-1-yl)-2-methoxybenzoate (C461)

To a solution of **C459** (31.0 g, 101.2 mmol, 1.0 eq) in DCM (300 mL) was added pyridine (24.0 g, 303.6 mmol, 3.0 eq) at 0 °C. TsCl (69.5 g, 364.3 mmol, 3.6 eq) in DCM (300 mL) was then added dropwise, and the reaction was stirred for 1 hour at 0 °C. The mixture was diluted with H₂O (200 mL), acidified with 2 N HCl, and extracted with DCM (300 mL). The organic layer was washed with brine, dried over Na₂SO₄, and concentrated to afford a crude product which was purified using a silica gel column (eluted with petroleum ether/ EtOAc = 3:1) to give **C461** (18.0 g, 38.6%) as a red oil. ¹H NMR (400MHz, CDCl₃): δ 7.80 (d, J = 8.3 Hz, 2H), 7.71 (d, J = 8.0 Hz, 1H), 7.31 (d, J = 8.0 Hz, 1H), 6.95-6.89 (m, 2H), 4.45 (dd, J = 10.3, 4.3 Hz, 1H), 4.36 (dd, J = 10.3, 5.9 Hz, 1H), 3.95-3.84 (m, 8H), 2.42 (s, 3H), 1.88-1.85 (m, 1H), 1.72 (brs, 1H), 1.34 (d, J = 2.2 Hz, 6H). LCMS: 460.8 ([M+H]+).

### Step 5: 2-methoxy-4-(3-methyl-3-(oxetan-3-yl)but-1-yn-1-yl)benzoic acid (C462)

To a solution of **C461** (18.0 g, 39.1 mmol, 1.0 eq) in t-BuOH (900 mL) was added t-BuOK (13.2 g, 117.3 mmol, 3.0 eq) at room temperature, and the mixture was stirred for 12 hours. The mixture was diluted with EtOAc (500 mL) and extracted with H₂O (300 mL, x2). The aqueous layer was neutralized to pH 3 with 2 N HCl and extracted with EtOAc (300 mL, x2). The organic layer was washed with brine, dried over Na₂SO₄, and concentrated to give **C462** (13.0 g, crude), which was used directly for the next step without further purification. LCMS: 274.9 ([M+H]+).

### Step 6: Methyl 2-methoxy-4-(3-methyl-3-(oxetan-3-yl)but-1-yn-1-yl)benzoate (C466)

To a solution of **C462** (13.0 g, crude) and K₂CO₃ (13.5 g, 97.8 mmol, 2.5 eq) in DMF (130 mL) was added CH₃I (8.3 g, 58.7 mmol, 1.5 eq) at room temperature. Then the reaction mixture was stirred at room temperature for 2 hours. The mixture was diluted with H₂O (100 mL) and extracted with EtOAc (150 mL, x2). The organic layer was washed with brine, dried over Na₂SO₄, and concentrated to afford a crude product, which was purified using a silica gel column (eluted with petroleum ether/ EtOAc = 10:1) to give **C466** (5.0 g, 44.2% for two steps) as a yellow oil. ¹H NMR (400MHz, CDCl₃): δ 7.74 (d, J = 8.0 Hz, 1H), 7.06-7.01 (m, 2H), 4.78-4.71 (m, 4H), 3.91 (s, 3H), 3.88 (s, 3H), 3.06-3.02 (m, 1H), 1.18 (s, 6H). LCMS: 289.2 ([M+H]+).

### Preparation of C447

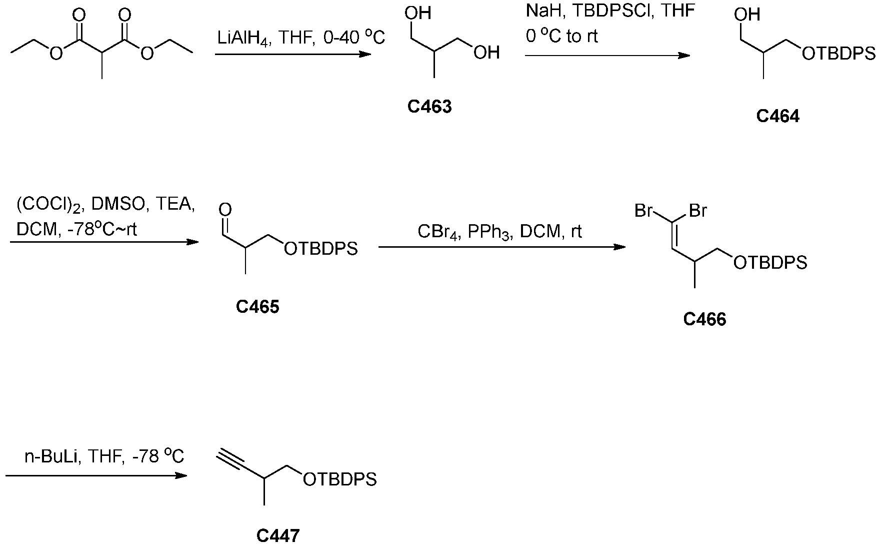

### Step 1: 2-methylpropane-1,3-diol (C463)

To a solution of diethyl 2-methylmalonate (80.0 g, 458.0 mmol, 1.0 eq) in THF (1.0 L) was added LiAlH₄ (52.4 g, 1.38 mol, 3.0 eq) in portions at 0 °C. Then the mixture was heated to 40 °C overnight. The mixture was cooled to 0 °C, quenched with H₂O (52.4 mL), 15% aqueous NaOH (52.4 mL), and H₂O (157.2 mL). Then Na₂SO₄ (220 g) was added to the mixture. Then the mixture was filtered and concentrated under reduced pressure to afford the crude product. The crude product was purified by silica gel column chromatography (Petroleum ether/EtOAc = 1:1) to give **C464** (21.6 g, 52.2%) as a colorless oil. ¹H NMR (400 Hz, CDCl₃): δ 3.70-3.63 (m, 2H), 3.59-3.54 (m, 2H), 3.24 (s, 2H), 1.95-1.88 (m, 1H), 0.84 (d, J = 6.8 Hz, 3H).

### Step 2: 3-((tert-butyldiphenylsilyl)oxy)-2-methylpropan-1-ol (C464)

To a solution of **C463** (20.0 g, 221.9 mol, 1.0 eq) in THF (300 mL) was added NaH (60% in oil, 9.8g, 244.1 mmol, 1.1 eq) in portions at 0 °C, then the mixture was stirred at 0 °C for 10 minutes. TBDPSCl (67.1 g, 244.1 mmol, 1.1 eq) was added dropwise into the reaction, which was stirred at room temperature overnight. TLC showed that the reaction went to completion. The mixture was cooled to 0 °C, quenched with saturated aqueous NH₄Cl (200 mL) and extracted with EtOAc (200 mL, x3). The organic phase was combined, washed with brine (100 mL), dried over Na₂SO₄, and concentrated. The crude product was purified by silica gel column chromatography (Petroleum ether/EtOAc = 10:1) to give **C464** (70.0 g, 96.1%) as a colorless oil. ¹H NMR (400 Hz, CDCl₃): δ 7.75-7.68 (m, 4H), 7.46-7.38 (m, 6H), 3.74 (dd, J = 10.0, 4.8 Hz, 1H), 3.68 (d, J = 6.0 Hz, 2H), 3.61 (dd, J = 10.0, 8.0 Hz, 1H), 2.49 (s, 1H), 2.05-1.96 (m, 1H), 1.09 (s, 9H), 0.84 (d, J = 6.8 Hz, 3H).

### Step 3: 3-((tert-butyldiphenylsilyl)oxy)-2-methylpropanal (C465)

To a solution of (COCl)₂ (54.2 g, 426.8 mmol, 2.0 eq) in DCM (900 mL), DMSO (138.5 g, 853.6 mmol, 4.0 eq) was added dropwise at -78 °C. Then the mixture was stirred at - 60 °C for 30 minutes. **C464** (70.0 g, 213.4 mmol, 1.0 eq) dissolved in DCM (200 mL) was added, and the reaction mixture was stirred at -78 °C for another 1 hour. Then TEA (129.3 g, 1.28 mol, 6.0 eq) was added, the reaction mixture was stirred at -60 °C for 1 hour and room temperature for 1 hour. The reaction was cooled to 0 °C, quenched with water (500 mL), and extracted with DCM (500 mL, x3). The layer was dried over anhydrous Na₂SO₄ and then concentrated under reduced pressure to give crude **C465** (41.0 g, 58.9%), which used in the next step without any further purification. ¹H NMR (400 Hz, CDCl₃): δ 9.77 (s, 1H), 7.66-7.63 (m, 4H), 7.66-7.37 (m, 6H), 3.92-3.82 (m, 1H), 2.85-2.78 (m, 1H), 2.59-2.53 (m, 1H), 1.24 (d, J = 7.2 Hz, 3H), 1.11 (s, 9H).

### Step 4: tert-butyl((4,4-dibromo-2-methylbut-3-en-1-yl)oxy)diphenylsilane (C466)

To a mixture of **C465** (41.0 g, 126.0 mmol, 1.0 eq) in DCM (400 mL) were added triphenylphosphine (132.0 g, 504.0 mmol, 4.0 eq) and carbon tetrabromide (83.4 g, 252.0 mmol, 2.0 eq). The resulting mixture was stirred at room temperature for 1 hour. Water (500 mL) was added to the mixture, which was extracted with DCM (500 mL, x2). The combined organic phase was washed with brine, dried over Na₂SO₄, and concentrated. The obtained crude oil was purified by column chromatography (Petroleum ether/EtOAc = 50:1) to give **C466** (50.0 g, 82.8%) as a clear yellow oil. ¹H NMR (400 Hz, CDCl₃): δ 7.68-7.65 (m, 4H), 7.44-7.37 (m, 6H), 6.28 (d, J = 9.2 Hz, 1H), 3.60-3.52 (m, 2H), 2.74-2.67 (m, 1H), 1.07 (s, 9H), 1.05 (d, J = 6.8 Hz, 3H).

### Step 5: tert-butyl((2-methylbut-3-yn-1-yl)oxy)diphenylsilane (C447)

To a solution of **C466** (50.0 g, 104.2 mmol, 1.0 eq) in THF (500 mL) at -78 °C, n-BuLi (2.5 M in hexanes, 92 mL, 229.2 mmol, 2.2 eq) was added slowly. After stirring for 1.5 hours, the reaction was quenched with a saturated aqueous solution of sodium potassium tartrate (500 mL) at -78 °C. Phases were separated, and the aqueous phase was extracted with EtOAc (500 mL, x2). The combined organic layers were then dried over anhydrous sodium sulfate, filtered, and concentrated. The obtained crude oil was purified by column chromatography (Petroleum ether/EtOAc = 50: 1) to give **C447** (25.0 g, 74.5%) as a clear yellow oil. ¹H NMR (400 Hz, CDCl₃): δ 7.72-7.69 (m, 4H), 7.44-7.26 (m, 6H), 3.79-3.74 (m, 1H), 3.60-3.54 (m, 1H), 2.74-2.67 (m, 1H), 2.05 (t, J = 6.4 Hz, 1H), 1.26 (d, J = 5.2 Hz, 3H), 1.09 (s, 9H).

### Preparation of C448 and C449

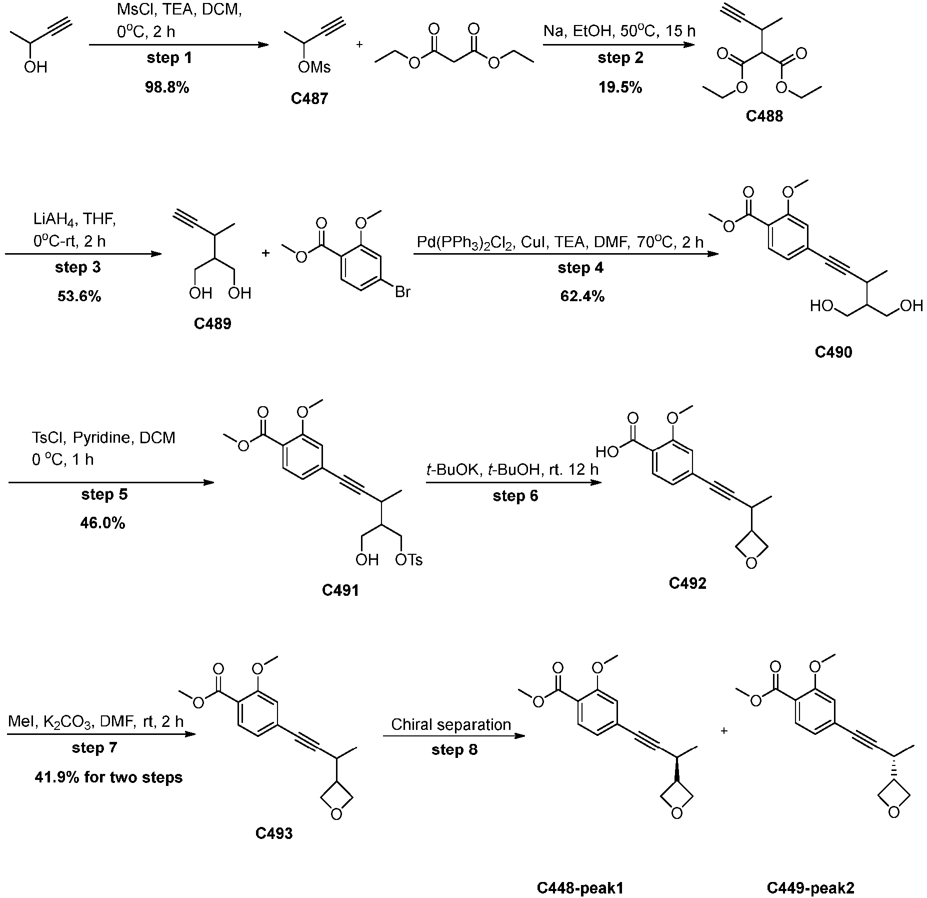

### Step 1: diethyl 2-(but-3-yn-2-yl)malonate (C487)

To a stirred solution of but-3-yn-2-ol (120.0 g, 1.71 mol, 1.0 eq) in DCM (1.2 L) was added triethylamine (260.0 g, 2.57 mol, 1.5 eq) at 0 °C. MsCl (235.0 g, 2.05 mol, 1.2 eq) was then added dropwise over 30 minutes to the solution. The mixture was stirred for a further 2 hours before warming to room temperature. Water (1 L) was added. After separation, the aqueous phase was extracted with DCM (500 mL, x2). The combined organics were washed with brine (500 mL) before drying over Na₂SO₄. The solvent was removed *in vacuo* to afford a crude compound **C487** (250.0 g, 98.8%) as a red oil, which was used in the next step without further purification. ¹H NMR (400MHz, CDCl₃): δ 5.30-5.24 (m, 1H), 3.11 (s, 3H), 2.70 (s, 1H), 1.65 (d, J = 6.8 Hz, 3H).

### Step 2: diethyl 2-(but-3-yn-2-yl)malonate (C488)

**C487** (270.2 g, 1.69 mol, 1.0 eq) was added to a freshly prepared solution of sodium ethylate [Na (38.9 g, 1.69 mol, 1.0 eq) in EtOH (4 L)] warmed to 60 °C. After 1 hour, diethyl malonate was added (250.0 g, 1.69 mol, 1.0 eq) to the mixture. The reaction mixture was stirred at 65 °C for 1 hour, then stirred at 80 °C for 30 minutes, and then stirred at 50 °C for 15 hours. The reaction mixture was cooled to room temperature and filtered; then the filtrate was concentrated. The residue was acidified with 2N HCl and extracted with EtOAc (2 L, x2). The organic layer was washed with brine, dried over Na₂SO₄, and concentrated to afford the crude product which was purified using a silica gel column (eluted with petroleum ether/ EtOAc = 100:1) to give **C488** (70.0 g, 19.5%) as a yellow oil. ¹H NMR (400MHz, CDCl₃): δ 4.24-4.18 (m, 4H), 3.39-3.34 (m, 1H), 3.26-3.20 (m, 1H), 2.10 (s, 1H), 1.28-1.24 (m, 9H). LCMS: 213.1 ([M+H]+).

### Step 3: 2-(but-3-yn-2-yl)propane-1,3-diol (C489)

To a solution of **C488** (70.0 g, 329.8 mmol, 1.0 eq) in THF (1.0 L) was added LiAlH₄ (37.6 g, 989.4 mmol, 3.0 eq) portionwise at 0 °C. Then the reaction mixture was stirred at room temperature for 2 hours. The mixture was diluted with THF (300 mL) and cooled to -20 °C. The mixture was slowly quenched with water (37.6 mL), followed by adding an aqueous solution of NaOH (15%, 37.6 mL). Water (112.8 mL) was then added again, and the mixture was stirred at room temperature for 15 minutes, followed by the addition of Na₂SO₄. The mixture was filtered and the filtrate was concentrated to afford a crude product which was purified using a silica gel column (eluted with petroleum ether/ EtOAc =2:1) to afford **C489** (22.7 g, 53.6%) as a yellow oil. ¹H NMR (400MHz, CDCl₃): δ 3.90-3.73 (m, 4H), 3.34 (s, 2H), 2.68-2.61 (m, 1H), 2.08 (d, J = 2.4 Hz, 1H), 1.71-1.64 (m, 1H), 1.22 (d, J = 6.8 Hz, 3H). LCMS: 129.1 ([M+H]+).

### Step 4: methyl 4-(5-hydroxy-4-(hydroxymethyl)-3-methylpent-1-yn-1-yl)-2-methoxybenzoate (C490)

To a solution of **C489** (26.0 g, 202.8 mmol, 1.0 eq) in DMF (300 mL) were added methyl 4-bromo-2-methoxybenzoate (54.7 g, 223.1 mmol, 1.1 eq), TEA (41.0 g, 405.6 mmol, 2.0 eq), CuI (3.9 g, 20.3 mmol, 0.1 eq), and (PPh₃)₂PdCl₂ (14.2 g, 20.3 mmol, 0.1 eq) successively, degassing the resulting reaction mixture with argon for 10 minutes. The reaction mixture was heated to 75 °C and stirred for 2 hours under argon atmosphere. After cooling to room temperature, the reaction mixture was diluted with H₂O (300 mL) and extracted with EtOAc (200 mL, x2). The organic layer was washed with brine, dried, and concentrated to afford the crude material, which was purified using a silica gel column (eluted with DCM/ MeOH = 100:1) to give **C490** (37.0 g, 62.4%) as a yellow oil. ¹H NMR (400MHz, CDCl₃) δ 7.69 (dd, J = 7.6, 1.2 Hz, 1H), 6.97-6.94 (m, 2H), 3.98-3.83 (m, 10H), 3.00-2.88 (m, 2H), 2.16-2.14 (m, 1H), 1.83-1.79 (m, 1H), 1.30 (d, J = 6.8 Hz, 3H). LCMS: 293.1 ([M+H]+).

### Step 5: methyl 4-(5-hydroxy-3-methyl-4-((tosyloxy)methyl)pent-1-yn-1-yl)-2-methoxybenzoate (C491)

To a solution of **C490** (37.0 g, 126.6 mmol, 1.0 eq) in DCM (300 mL) was added pyridine (30.0 g, 379.7 mmol, 3.0 eq) at 0 °C. A solution of TsCl (86.9 g, 455.8 mmol, 3.6 eq) in DCM (300 mL) was then added dropwise, and the reaction mixture was stirred at 0 °C for 1 hour. The reaction mixture was diluted with H₂O (200 mL), acidified with 2 N HCl, and extracted with DCM (300 mL). The organic layer was washed with brine, dried, and concentrated to afford the crude material, which was purified using a silica gel column (eluted with petroleum ether/ EtOAc = 2:1) to give **C491** (26.0 g, 46.0%) as an oily liquid. LCMS: 447.1 ([M+H]+).

### Step 6: 2-methoxy-4-(3-(oxetan-3-yl)but-1-yn-1-yl)benzoic acid (C492)

To a solution of **C491** (26.0 g, 58.2 mmol, 1.0 eq) in t-BuOH (1 L) was added t-BuOK (19.6 g, 174.6 mmol, 3.0 eq) at room temperature. The mixture was stirred for 12 hours. The reaction mixture was diluted with EtOAc (500 mL) and extracted with H₂O (300 mL, x2). The aqueous layer was acidified to pH 3 with 2 N HCl and extracted with EtOAc (200 mL, x2). The organic layer was washed with brine, dried, and concentrated to give crude compound **C492** (12.0 g, crude), which was used in the next step without further purification. ¹H NMR (400MHz, DMSO-d₆): δ 7.60 (d, J = 7.6 Hz, 1H), 7.07 (s, 1H), 7.00 (dd, J = 8.0, 1.2 Hz, 1H), 4.69-4.62 (m, 2H), 4.50-4.40 (m, 2H), 3.82 (s, 3H), 3.07-3.05 (m, 2H), 1.12 (d, J = 6.4 Hz, 3H). LCMS: 261.2 ([M+H]+).

### Step 7: 2-methoxy-4-(3-(oxetan-3-yl)but-1-yn-1-yl)benzoate C493 (C448, C449)

To a solution of **C492** (12.0 g, 46.1 mmol, 1.0 eq) and K₂CO₃ (15.9 g, 115.3 mmol, 2.5 eq) in DMF (120 mL), CH₃I (6.5 g, 46.1 mmol, 1.0 eq) was added at room temperature. The reaction mixture was then stirred at room temperature for 2 hours. The reaction mixture was diluted with H₂O (100 mL) and extracted with EtOAc (150 mL, x2). The organic layer was washed with brine, dried, and concentrated to afford a crude product, which was purified using a silica gel column (eluted with petroleum ether/ EtOAc = 2:1) to give **C493** (6.7 g, 41.9% for two steps) as an oily liquid. ¹H NMR (400MHz, CDCl₃): δ 7.72 (d, J = 8.0 Hz, 1H), 7.01-6.97 (m, 2H), 4.83-4.76 (m, 2H), 4.61 (t, J = 9.6 Hz, 1H), 4.51 (t, J = 10.0 Hz, 1H), 3.89 (s, 3H), 3.87 (s, 3 H), 3.10-2.97 (m, 1H), 1.19 (d, J = 6.4 Hz, 3H). LCMS: 275.1 ([M+H]+).

### Chiral HPLC separation method:

A Shimadzu LC-20AT CP-HPLC-06 was employed for chiral HPLC. A CHIRALPAKIG(IG00CE-XL022) column (column size: 0.46 cm I.D. × 25 cm L) was used in the separation method, along with a 0.5 µL injection with a MeOH = 100% mobile phase and a 1.0 mL/min flow rate. The wavelength used was UV 254 nm, and the temperature was 35 °C.

### C448-peak 1 (absolute stereochemistry unknown)

¹H NMR (400MHz, CDCl₃): δ 7.72 (d, J = 8.0 Hz, 1H), 7.01-6.98 (m, 2H), 4.83-4.76 (m, 2H), 4.65 (t, J = 10.0 Hz, 1H), 4.52 (t, J = 10.0 Hz, 1H), 3.91-3.87 (m, 6H), 3.10-2.99 (m, 1H), 1.19 (d, J = 6.8 Hz, 3H). LCMS: 275.1 ([M+H]+).

### C449-peak 1 (absolute stereochemistry unknown)

¹H NMR (400MHz, CDCl₃): δ 7.72 (d, J = 8.0 Hz, 1H), 7.02-6.99 (m, 2H), 4.84-4.77 (m, 2H), 4.66 (t, J = 10.0 Hz, 1H), 4.52 (t, J = 10.0 Hz, 1H), 3.90 (s, 3H), 3.88 (s, 3H), 3.09-3.00 (m, 1H), 1.20 (d, J = 6.8 Hz, 3H). LCMS: 275.1 ([M+H]+).

### Preparation of C450

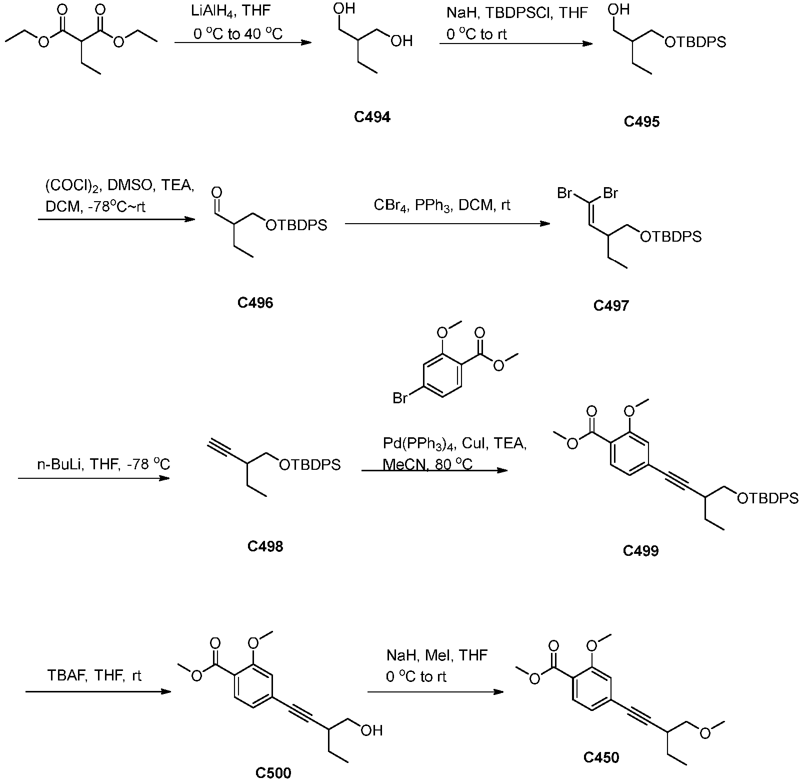

### Step 1: 2-ethylpropane-1,3-diol (C494)

To a solution of diethyl 2-ethylmalonate (80.0 g, 425.0 mmol, 1.0 eq) in THF (1 L) was added LiAlH₄ (48.4 g, 1.28 mol, 3.0 eq) in portions at 0 °C. The mixture was then heated to 40 °C overnight. HPLC showed completion. The reaction was then cooled to 0 °C, quenched with H₂O (48.4 mL), then 15% aqueous NaOH (48.4 mL), then H₂O (145.2 mL). Na₂SO₄ (200 g) was then added. The mixture was filtered and concentrated under reduced pressure to afford the crude product. The crude product was purified by silica gel column chromatography (Petroleum ether/EtOAc = 1:1) to give **C494** (25.0 g, 56.4%) as a colorless oil. ¹H NMR (400 Hz, CDCl₃): δ 3.80-3.76 (m, 2H), 3.64-3.60 (m, 2H), 3.16 (s, 2H), 1.68-1.62 (m, 1H), 1.30-1.23 (m, 2H), 0.98-0.87 (m, 3H).

### Step 2: 2-(((tert-butyldiphenylsilyl)oxy)methyl)butan-1-ol (C495)

To a solution of **C494** (20.0 g, 192.0 mmol, 1.0 eq) in THF (300 mL) was added NaH (60% in oil, 8.5 g, 211.5 mmol, 1.1 eq) in portions at 0 °C, then the mixture was stirred at 0 °C for 10 minutes. TBDPSCl (58.1 g, 211.5 mmol, 1.1 eq) was added dropwise into the reaction, which was stirred at room temperature overnight. TLC showed the reaction was completed. The reaction was cooled to 0 °C, quenched with saturated aqueous NH₄Cl (200 mL), and extracted with EtOAc (200 mL, x3). The organic phase was combined, washed with brine (100 mL), dried over Na₂SO₄, and concentrated. The crude product was purified by silica gel column chromatography (Petroleum ether/EtOAc = 10: 1) to give **C495** (42.5 g, 67.4%) as a colorless oil. ¹H NMR (400 Hz, CDCl₃): δ 7.69-7.67 (m, 4H), 7.45-7.38 (m, 6H), 3.81-3.62 (m, 4H), 1.84-1.76 (m, 1H), 1.32-1.18 (m, 3H), 1.07 (s, 9H), 0.88-0.83 (m, 3H).

### Step 3: 2-(((tert-butyldiphenylsilyl)oxy)methyl)butanal (C496)

To a solution of (COCl)₂ (29.7 g, 233.9 mmol, 2.0 eq) in DCM (600 mL) was added DMSO (36.5 g, 467.8 mmol, 4.0 eq) at -78 °C. The mixture was then stirred at -60 °C for 30 minutes, and **C495** (40.0 g, 116.9 mmol, 1.0 eq) in DCM (200 mL) was added. The reaction mixture was stirred at -78 °C for another 1 hour. TEA (70.8 g, 701.4 mmol, 6.0 eq) was then added, the reaction mixture was stirred at -60 °C for 1 hour, and then the reaction mixture was warmed to room temperature for another 1 hour. HPLC showed completion. The reaction was cooled to 0 °C, quenched with water (500 mL), and extracted with DCM (500 mL, x3). The layer was dried over anhydrous Na₂SO₄ and then concentrated under reduced pressure to give crude compound **C496** (40.0 g crude, 99.0%), which was used in the next step without any further purification. ¹H NMR (400 Hz, CDCl₃): δ 9.74 (d, J = 2.4 Hz, 1H), 7.65-7.63 (m, 4H), 7.46-7.26 (m, 6H), 3.90-3.88 (m, 2H), 2.39-2.35 (m, 1H), 1.76-1.69 (m, 1H), 1.56-1.49 (m, 2H), 1.08 (s, 9H), 0.89-0.81 (m, 3H).

### Step 4: tert-butyl((4,4-dibromo-2-ethylbut-3-en-1-yl)oxy)diphenylsilane (C497)

To a mixture of **C496** (40.0 g, 117.0 mmol, 1.0 eq) in DCM (400 mL) were added triphenylphosphine (122.6 g, 468.0 mmol, 4.0 eq) and carbon tetrabromide (77.9 g, 235.0 mmol, 2.0 eq). The resulting mixture was stirred at room temperature for 1 hour. Water (500 mL) was added to the mixture, and the mixture was extracted twice with DCM (500 mL, x2). The combined organic phase was washed with brine, dried (Na₂SO₄), and concentrated. The obtained crude oil was purified by column chromatography (Petroleum ether/EtOAc = 50:1) to give **C497** (20.0 g, 34.3%) as a clear yellow oil. ¹H NMR (400 Hz, CDCl₃): δ 7.77-7.65 (m, 4H), 7.45-7.26 (m, 6H), 6.27 (d, J =10.0 Hz, 1H), 3.62-3.58 (m, 2H), 2.58-2.49 (m, 1H), 1.73-1.64 (m, 1H), 1.42-1.36 (m, 1H), 1.08 (s, 9H), 0.91-0.88 (m, 3H).

### Step 5: tert-butyl((2-ethylbut-3-yn-1-yl)oxy)diphenylsilane (C498)

To a solution of **C497** (20.0 g, 40.4 mmol, 1.0 eq) in THF (200 mL) at -78°C, n-BuLi (2.5 M in hexanes, 36.0 mL, 88.9 mmol, 2.2 eq) was added slowly. After stirring for 1.5 hours, the reaction was quenched with a saturated aqueous solution of sodium potassium tartrate (200 mL) at -78 °C. Phases were separated and the aqueous phase was extracted with EtOAc (300 mL x 2). The combined organic layers were then dried over anhydrous sodium sulfate, filtered, and concentrated. The obtained crude oil was purified by column chromatography (Petroleum ether/EtOAc = 50:1) to give **C498** (12.0 g, 88.4%) as a clear yellow oil. ¹H NMR (400 Hz, CDCl₃): δ 7.73-7.67 (m, 4H), 7.43-7.26 (m, 6H), 3.77-3.73 (m, 1H), 3.64-3.60 (m, 1H), 2.53-2.47 (m, 1H), 2.04 (t, J = 2.4 Hz, 1H), 1.79-1.71 (m, 1H), 1.64-1.58 (m, 1H), 1.28-1.25 (m, 1H), 1.08 (s, 9H), 1.03-0.99 (m, 3H).

### Step 6: methyl 4-(3-(((tert-butyldiphenylsilyl)oxy)methyl)pent-1-yn-1-yl)-2-methoxybenzoate (C499)

To a solution of **C498** (12.0 g, 35.7 mmol, 1.0 eq) in MeCN (300 mL), methyl 4-bromo-2-methoxybenzoate (8.8 g, 35.7 mmol, 1.0 eq), CuI (678.3 mg, 3.57 mmol, 0.1 eq), Pd(PPh₃)₄ (2.1 g, 1.79 mmol, 0.05 eq) and TEA (10.8 g, 107.1 mmol, 3.0 eq) were added, and the mixture was stirred at 80 °C for 2 hours under argon. HPLC showed completion. The mixture was cooled to room temperature, poured into water (300 mL), and extracted with EtOAc (300 mL x 3). The organic phase was then washed with brine (100 mL x 2), dried over anhydrous Na₂SO₄ and concentrated. The crude product was purified by silica gel column chromatography (Petroleum ether/EtOAc = 5:1) to give **C499** (15.5 g, 94.7%) as a yellow oil. ¹H NMR (400 Hz, CDCl₃): δ 7.74-7.67 (m, 5H), 7.42-7.35 (m, 6H), 7.00-6.97 (m, 2H), 3.90-3.82 (m, 3H), 3.81-3.71 (m, 3H), 2.81-2.73 (m, 1H), 1.83-1.75 (m, 1H), 1.68-1.55 (m, 2H), 1.09-1.05 (m, 12H). LCMS: 501.3 ([M+H+]).

### Step 7: methyl 4-(3-(hydroxymethyl)pent-1-yn-1-yl)-2-methoxybenzoate (C500)

To a solution of **C499** (15.5 g, 31.0 mmol, 1.0 eq) in THF (200 mL) was added TBAF (1M in THF, 62 mL, 62.0 mmol, 2.0 eq) at room temperature. The reaction was stirred at room temperature for 2 hours. HPLC and LCMS showed the reaction went to completion. The mixture was concentrated and purified by silica gel column chromatography (Petroleum ether/EtOAc = 3:1) to give **C500** (6.1 g, 74.8%) as a yellow solid. LCMS: 263.0 ([M+H]+).

### Step 8: methyl 4-(3-(hydroxymethyl)pent-1-yn-1-yl)-2-methoxybenzoate (C450)

To a solution of **C500** (6.1 g, 23.2 mmol, 1.0 eq) in THF (100 mL) was added NaH (60% in oil, 1.9 g, 46.4 mmol, 2.0 eq) at 0 °C under argon. The mixture was then stirred at 0 °C for 10 minutes. MeI (6.6 g, 46.4 mmol, 2.0 eq) was added and stirred at room temperature for 3 hours. HPLC and LCMS showed no starting material remained. The mixture was cooled to 0 °C, quenched with a saturated aqueous solution of NH₄Cl (80 mL), extracted with EtOAc (80 mL x 3), the aqueous phase was adjusted pH 3 with 1N HCl, and extracted with EtOAc (80 mL x 3). The organic phases were combined, washed with brine, dried over Na₂SO₄, and concentrated. The crude product was dissolved in MeOH (80 mL), concentrated H₂SO₄ (1 mL) was added, and the mixture was heated to reflux for 2 hours. HPLC and LCMS showed the reaction went to completion. The mixture was cooled to room temperature and concentrated. The residue was dissolved in EtOAc (100 mL), washed with brine (20 mL x 3), dried over Na₂SO₄, and concentrated. The crude product was purified by silica gel column chromatography (Petroleum ether/EtOAc = 10: 1) to afford **C450** (4.7 g, 72.9%) as a yellow oil. ¹H NMR (400 Hz, CDCl₃): δ 7.73 (d, J = 8.0 Hz, 1H), 7.07-6.97 (m, 2H), 3.89 (d, J = 8.4 Hz, 6H), 3.54 (dd, J = 9.2, 6.4 Hz, 1H), 3.46 (dd, J = 9.2, 6.4 Hz, 1H), 3.41 (s, 3H), 2.87-2.74 (m, 1H), 1.81-1.63 (m, 1H), 1.63-1.48 (m, 1H), 1.09 (t, J = 7.2 Hz, 3H). LCMS: 276.9 ([M+H]+).

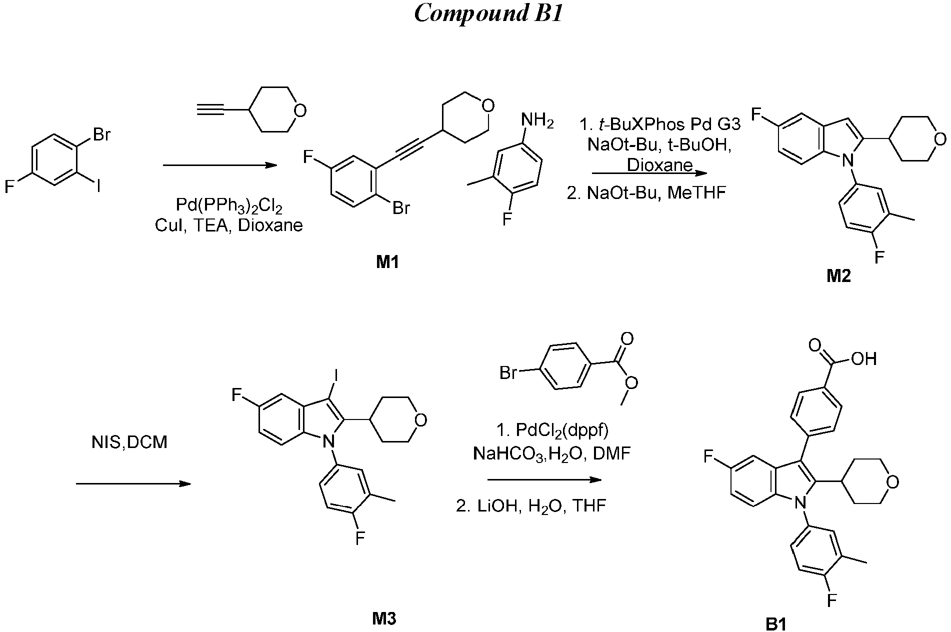

### Step 1. Synthesis of 4-[2-(2-bromo-5-fluoro-phenyl)ethynyl]tetrahydropyran (M1)

1-bromo-4-fluoro-2-iodo-benzene (3 g, 9.970 mmol) and 4-ethynyltetrahydropyran (1.22 g, 10.96 mmol) were dissolved in dioxane (12 mL) and DIEA (4.3 mL, 24.69 mmol), and the solution was purged with N₂ for 5-10 minutes. PdCl₂(PPh₃)₂ (350 mg, 0.4986 mmol) was added, followed by CuI (190 mg, 0.9976 mmol). The reaction mixture was stirred at room temperature under nitrogen overnight. The reaction was filtered off with the aid of EtOAc, concentrated, and purified by column chromatography (80g column; 0-100% EtOAc in heptane). 4-[2-(2-bromo-5-fluoro-phenyl)ethynyl]tetrahydropyran (2.3 g, 81%) ¹H NMR (400 MHz, CDCl₃) δ 7.53 (dd, J = 8.9, 5.3 Hz, 1H), 7.17 (dd, J = 8.9, 3.1 Hz, 1H), 6.91 (ddd, J = 8.8, 7.9, 3.0 Hz, 1H), 4.01 (ddd, J = 11.6, 6.5, 3.5 Hz, 2H), 3.62 (ddd, J = 11.3, 7.7, 3.2 Hz, 2H), 2.97 (tt, J = 7.9, 4.2 Hz, 1H), 2.02 - 1.92 (m, 2H), 1.87 - 1.75 (m, 2H).

### Step 2. Synthesis of 5fluoro-1-(4-fluoro-3-methyl-phenyl)-2-tetrahydropyran-4-yl-indole (M2)

4-[2-(2-bromo-5-fluoro-phenyl)ethynyl]tetrahydropyran **M1** (1.49 g, 5.262 mmol), 4-fluoro-3-methyl-aniline (730 mg, 5.833 mmol), sodium t-butoxide (1.15 g, 11.97 mmol) were suspended/dissolved in dioxane (8 mL) and t-BuOH (8 mL). The reaction purged with N₂ for several minutes. During the purge, tBuXphosPalladacycle (180 mg, 0.2621 mmol) was added, and the reaction mixture was stirred overnight at room temperature. The reaction mixture was filtered through Celite^{®} with the aid of EtOAc, concentrated, and purified by column chromatography (80g GOLD column; 0-100% EtOAc in heptane. MeTHF (5 mL) and 2-methylpropan-2-olate (Potassium Ion (1)) (5.8 mL of 1 M, 5.800 mmol) were added to the mixture. The mixture was stirred at room temperature for 3 hours. Water and DCM were added. The layers were separated with the aid of a phase separator. The aqueous layer was reextracted with DCM, and the layers were separated through a phase separator again and the combined organics concentrated. 5-fluoro-1-(4-fluoro-3-methyl-phenyl)-2-tetrahydropyran-4-yl-indole **(M2)** (1 g, 58%) ¹H NMR (400 MHz, Methanol-d₄) δ 7.29 - 7.16 (m, 4H), 6.87 - 6.76 (m, 2H), 6.41 (t, J = 0.7 Hz, 1H), 3.92 (dt, J = 11.4, 3.2 Hz, 2H), 3.40 - 3.32 (m, 2H), 2.90 - 2.80 (m, 1H), 2.35 (d, J = 2.1 Hz, 3H), 1.81 - 1.71 (m, 4H). ESI-MS m/z 328.19 (M+1)+.

### Step 3. 5-fluoro-1-(4-fluoro-3-methyl-phenyl)-3-iodo-2-tetrahydropyran-4-yl-indole (M3)

To a solution of **M2** (200 mg, 0.6109 mmol) in DCM (5 mL) at 0 °C was added NIS (155 mg, 0.6889 mmol). The reaction mixture was stirred 0 °C for 1 hour, then washed with water. The organic layer was concentrated to dryness and purified via silica gel chromatography, eluting with 0-20% EtOAc in heptane, to yield 5-fluoro-1-(4-fluoro-3-methylphenyl)-3-iodo-2-tetrahydropyran-4-yl-indole **M3** (244 mg, 88%). ¹H NMR (400 MHz, CDCl₃) δ 7.26 - 7.07 (m, 4H), 6.90 (ddd, J = 10.1, 8.4, 2.5 Hz, 1H), 6.77 (dd, J = 8.9, 4.3 Hz, 1H), 4.07 - 3.99 (m, 2H), 3.36 (td, J = 11.9, 2.1 Hz, 2H), 2.98 (tt, J = 12.4, 3.7 Hz, 1H), 2.53 - 2.31 (m, 5H), 1.60 (dd, J = 3.8, 1.8 Hz, 1H), 1.57 (dd, J = 3.9, 2.0 Hz, 1H). ESI-MS m/z 453.0 (M+1)+.

### Step 4. Synthesis of 4-[5-fluoro-1-(4-fluoro-3-methyl-phenyl)-2-tetrahydropyran-4-yl-indol-3-yl]benzoic acid (B1)

A suspension of **M3** (120 mg, 0.2647 mmol), methyl 4-(4,4,5,5-tetramethyl-1,3,2-dioxaborolan-2-yl)benzoate (83 mg, 0.3167 mmol), PdCl₂(dppf) (22 mg, 0.02694 mmol) and NaHCO₃ (45 mg, 0.5357 mmol) in DMF (750 µL) and water (250 µL) was microwaved at 90 °C for 20 minutes. The mixture was diluted with water and extracted with EtOAc. The organic layer was concentrated to dryness and purified via silica gel chromatography, eluting with 0-30% EtOAc in heptane. Methyl 4-[5-fluoro-1-(4-fluoro-3-methyl-phenyl)-2-tetrahydropyran-4-yl-indol-3-yl]benzoate **B1** (90 mg, 74%) ¹H NMR (300 MHz, CDCl₃) δ 8.23 - 8.15 (m, 2H), 7.58 - 7.51 (m, 2H), 7.28 - 7.18 (m, 3H), 7.04 (ddd, J = 9.5, 2.5, 0.5 Hz, 1H), 6.95 - 6.85 (m, 1H), 6.79 (ddd, J = 8.9, 4.5, 0.6 Hz, 1H), 4.00 (s, 3H), 3.86 (ddt, J = 11.3, 4.0, 1.7 Hz, 2H), 3.23 (td, J = 11.8, 2.1 Hz, 2H), 3.01 (tt, J = 12.3, 3.5 Hz, 1H), 2.45 - 2.37 (m, 3H), 1.91 - 1.76 (m, 2H), 1.69 - 1.55 (m, 2H). ESI-MS m/z 462.0 (M+1)+.

To a solution of methyl 4-[5-fluoro-1-(4-fluoro-3-methyl-phenyl)-2-tetrahydropyran-4-yl-indol-3-yl]benzoate (90 mg, 0.1950 mmol) in THF (750 µL) and water (250 µL) was added LiOH (24 mg, 1.002 mmol). The reaction mixture was stirred at room temperature overnight, then acidified with 1 M aqueous HCl and extracted with EtOAc. The organic layer was dried over magnesium sulfate, filtered, and concentrated. The crude residue was purified via silica gel chromatography, eluting with 0-10% MeOH in DCM. Pure fractions were combined and concentrated to give 4-[5-fluoro-1-(4-fluoro-3-methyl-phenyl)-2-tetrahydropyran-4-yl-indol-3-yl]benzoic acid **B1** (50 mg, 54%). ¹H NMR (400 MHz, CDCl₃) δ 8.31 - 8.23 (m, 2H), 7.64 - 7.56 (m, 2H), 7.31 - 7.19 (m, 3H), 7.12 - 7.04 (m, 1H), 6.90 (td, J = 8.9, 2.5 Hz, 1H), 6.81 (dd, J = 8.9, 4.4 Hz, 1H), 3.92 (dd, J = 11.3, 4.0 Hz, 2H), 3.27 (td, J = 11.8, 2.0 Hz, 2H), 3.04 (tt, J = 12.4, 3.4 Hz, 1H), 2.42 (dd, J = 2.0, 0.7 Hz, 3H), 1.95 - 1.80 (m, 2H), 1.71 - 1.59 (m, 2H). ESI-MS m/z found 448.0 (M+1)+.

### Compound B2-B13

Compounds from Table 30 were prepared from the corresponding aryl halides and alkynes as for compound **B1.**

**Table 30. Structure and physicochemical data for compounds B2-B13**

| **Compound** | **Structure** | **¹H NMR; LCMS m/z [M+H]⁺** |
|---|---|---|
| **B2** | 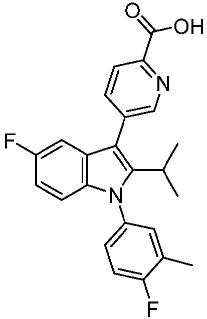 | ESI-MS m/z 407.0 (M+1)+; |
| **B3** | 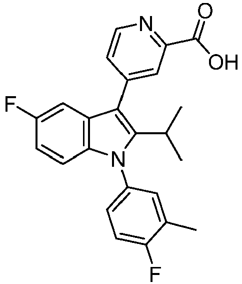 | ESI-MS m/z found 407.0 (M+1)+; |
| **B4** | 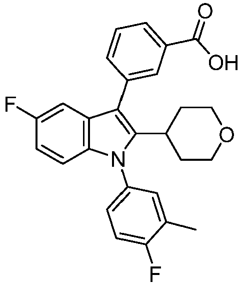 | ¹H NMR (400 MHz, Chloroform-d) δ 8.22 (dt, J = 1.8, 0.9 Hz, 1H), 8.17 (dt, J = 7.8, 1.4 Hz, 1H), 7.72 (dt, J = 7.6, 1.4 Hz, 1H), 7.62 (td, J = 7.7, 0.5 Hz, 1H), 7.28 - 7.25 (m, 1H), 7.24 (t, J = 1.3 Hz, 1H), 7.22 (d, J = 1.5 Hz, 1H), 7.05 - 6.99 (m, 1H), 6.89 (td, J = 8.9, 2.5 Hz, 1H), 6.81 (dd, J = 8.9, 4.4 Hz, 1H), 3.94 - 3.86 (m, 2H), 3.25 (td, J = 11.8, 2.0 Hz, 2H), 3.00 (tt, J = 12.3, 3.4 Hz, 1H), 2.41 (dd, J = 2.0, 0.8 Hz, 3H), 1.92 - 1.77 (m, 2H), 1.72 - 1.60 (m, 2H). ESI-MS m/z 448.0 (M+1)+ |
| **B5** | 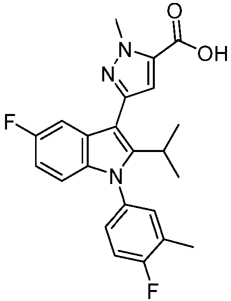 | ¹H NMR (400 MHz, Chloroform-d) δ 7.35 (dd, J = 9.7, 2.4 Hz, 1H), 7.22 - 7.14 (m, 3H), 7.13 (s, 1H), 6.83 (td, J = 8.9, 2.5 Hz, 1H), 6.76 (dd, J = 8.8, 4.4 Hz, 1H), 4.31 (s, 3H), 3.25 (p, J = 7.2 Hz, 1H), 2.39 - 2.32 (m, 3H), 1.22 (dd, J = 7.2, 2.0 Hz, 6H). ESI-MS m/z calc. 409.1602, found 410.0 (M+1)+; |
| **B6** | 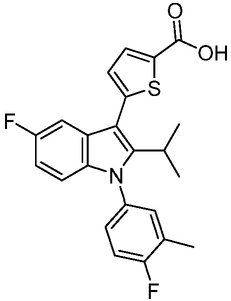 | ¹H NMR (400 MHz, Chloroform-d) δ 7.97 (d, J = 3.8 Hz, 1H), 7.28 - 7.19 (m, 4H), 7.15 (d, J = 3.8 Hz, 1H), 6.89 (td, J = 8.9, 2.4 Hz, 1H), 6.79 (dd, J = 8.9, 4.4 Hz, 1H), 3.30 (hept, J = 7.2 Hz, 1H), 2.42 - 2.35 (m, 3H), 1.22 (dd, J = 7.2, 1.6 Hz, 6H). ESI-MS m/z calc. 411.11044, found 412.0 (M+1)+ |
| **B7** | 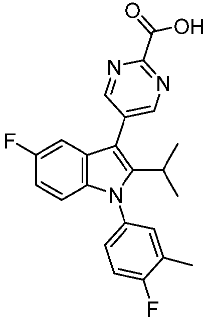 | ¹H NMR (400 MHz, Chloroform-d) δ 7.86 (d, J = 3.8 Hz, 1H), 7.25 - 7.16 (m, 4H), 7.09 (d, J = 3.8 Hz, 1H), 6.86 (td, J = 8.9, 2.5 Hz, 1H), 6.76 (dd, J = 8.9, 4.4 Hz, 1H), 3.92 (s, 3H), 3.25 (hept, J = 7.2 Hz, 1H), 2.37 (dd, J = 2.1, 0.7 Hz, 3H), 1.19 (dd, J = 7.2, 1.7 Hz, 6H ESI-MS m/z calc. 425.1261, found 426.0 (M+1)+ |
| **B8** | 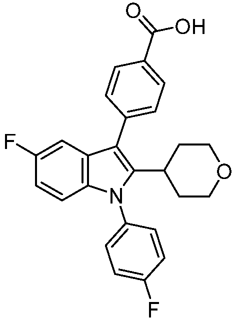 | ¹H NMR (400 MHz, Chloroform-d) δ 8.28 - 8.23 (m, 2H), 7.61 - 7.56 (m, 2H), 7.45 - 7.39 (m, 2H), 7.35 - 7.27 (m, 2H), 7.07 (dd, J = 9.4, 2.4 Hz, 1H), 6.90 (td, J = 8.9, 2.5 Hz, 1H), 6.80 (dd, J = 8.9, 4.3 Hz, 1H), 3.94 - 3.87 (m, 2H), 3.25 (td, J = 11.8, 1.9 Hz, 2H), 3.02 (tt, J = 12.4, 3.4 Hz, 1H), 1.92 - 1.85 (m, 2H), 1.73 - 1.56 (m, 2H). ESI-MS m/z calc. 433.14896, found 434.0 (M+1)+ |
| **B9** | 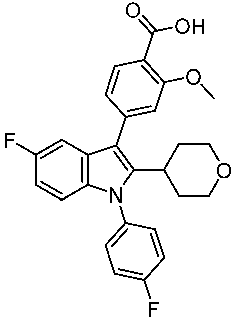 | ¹H NMR (400 MHz, Chloroform-d) δ 8.22 (d, J = 8.0 Hz, 1H), 7.31 (ddt, J = 8.1, 5.5, 2.6 Hz, 2H), 7.26 - 7.20 (m, 2H), 7.15 (dd, J = 8.0, 1.4 Hz, 1H), 7.10 (d, J = 1.4 Hz, 1H), 6.98 (dd, J = 9.4, 2.5 Hz, 1H), 6.83 (td, J = 9.0, 2.5 Hz, 1H), 6.72 (dd, J = 8.9, 4.4 Hz, 1H), 4.05 (s, 3H), 3.83 - 3.76 (m, 2H), 3.15 (td, J = 11.9, 1.9 Hz, 2H), 2.94 (tt, J = 12.3, 3.4 Hz, 1H), 1.81 - 1.71 (m, 2H), 1.60 - 1.49 (m, 2H). ESI-MS m/z 464.0 (M+1)+; |
| **B10** | 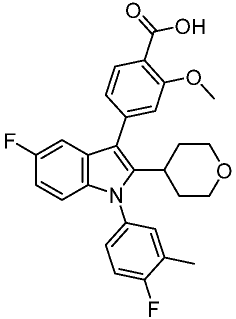 | ¹H NMR (400 MHz, Chloroform-d) δ 8.30 (d, J = 8.0 Hz, 1H), 7.26 - 7.18 (m, 5H), 7.06 (dd, J = 9.4, 2.4 Hz, 1H), 6.90 (td, J = 9.0, 2.5 Hz, 1H), 6.81 (dd, J = 8.9, 4.4 Hz, 1H), 4.14 (s, 3H), 3.89 (dt, J = 11.6, 3.4 Hz, 2H), 3.25 (td, J = 11.8, 2.0 Hz, 2H), 3.04 (tt, J = 12.3, 3.4 Hz, 1H), 2.41 (d, J = 2.0 Hz, 3H), 1.83 (dddd, J = 13.8, 12.2, 3.9, 1.6 Hz, 2H), 1.64 (ddt, J = 15.5, 13.2, 2.4 Hz, 2H). ESI-MS m/z 478.0 (M+1)+; |
| **B11** | 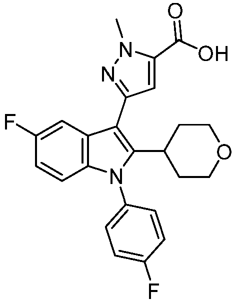 | ¹H NMR (400 MHz, DMSO-d6) δ 13.49 (s, 1H), 7.61 - 7.54 (m, 2H), 7.52 - 7.44 (m, 2H), 7.32 (dd, J = 9.8, 2.5 Hz, 1H), 7.00 - 6.91 (m, 2H), 6.78 (dd, J = 8.9, 4.5 Hz, 1H), 4.19 (s, 3H), 3.79 (dd, J = 11.4, 4.0 Hz, 2H), 3.20 - 3.04 (m, 3H), 1.92 - 1.77 (m, 2H), 1.60 (d, J = 12.6 Hz, 2H). ESI-MS m/z 438.21 (M+1)+; |
| **B12** | 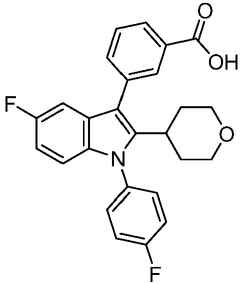 | ¹H NMR (400 MHz, DMSO-d₆) δ 13.08 (s, 1H), 7.99 (dd, J = 7.1, 1.6 Hz, 2H), 7.73 - 7.59 (m, 4H), 7.53 - 7.46 (m, 2H), 7.00 - 6.91 (m, 2H), 6.82 (dd, J = 8.8, 4.4 Hz, 1H), 3.71 (d, J = 10.5 Hz, 2H), 3.11 - 3.01 (m, 2H), 2.96 - 2.83 (m, 1H), 1.69 - 1.51 (m, 4H). ESI-MS m/z 433.57 (M+1)+; |
| **B13** | 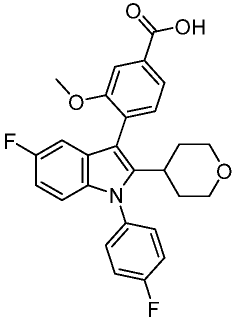 | ¹H NMR (400 MHz, Chloroform-d/CD₃OD) δ 7.68 (d, J = 7.7 Hz, 2H), 7.33 (dt, J = 6.4, 3.6 Hz, 1H), 7.24 (td, J = 9.9, 7.9, 4.4 Hz, 2H), 7.24 - 7.10 (m, 2H), 6.75 - 6.65 (m, 2H), 6.64 (dd, J = 8.5, 4.3 Hz, 1H), 4.05 - 3.94 (m, 1H), 3.75 - 3.64 (m, 5H), 3.05 (td, J = 11.9, 2.2 Hz, 2H), 2.80 - 2.68 (m, 1H), 1.84 - 1.74 (m, 1H), 1.72 - 1.54 (m, 2H). ESI-MS m/z 464.03 (M+1)+; |

### Compound B14

### 4-[2-(2-cyano-1,1-dimethyl-ethyl)-6-fluoro-1-(4-fluoro-3-methoxy-phenyl)indol-3-yl]-2-methoxy-benzoic acid (B14)

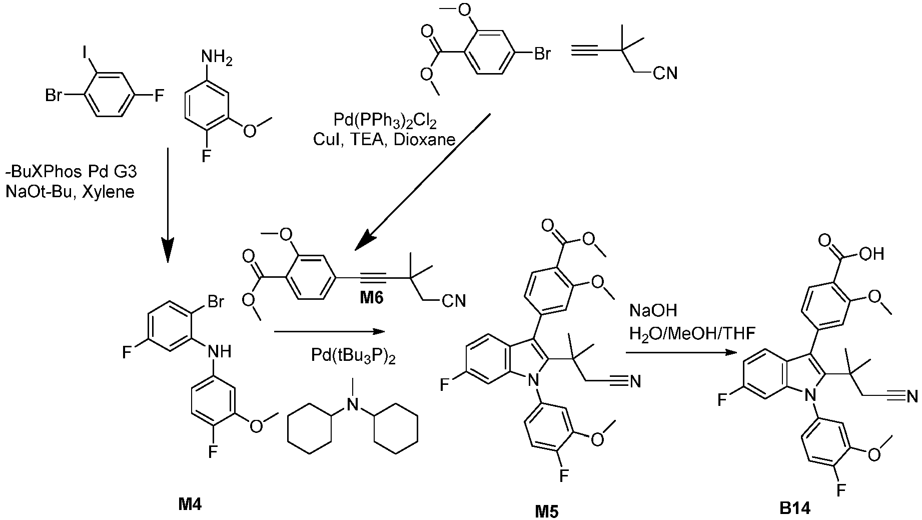

### Step 1. Synthesis of methyl 4-(4-cyano-3, 3-dimethyl-but-1-ynyl)-2-methoxy-benzoate (M6)

To a solution of methyl 4-bromo-2-methoxy-benzoate (2 g, 8.161 mmol) and 3,3-dimethylpent-4-ynenitrile (1.045 g, 9.752 mmol) in 1,4-dioxane (12 mL) and Et₃N (12 mL) was added in one portion dichloropalladium;triphenylphosphane (286 mg, 0.4075 mmol) and iodocopper (78 mg, 0.4096 mmol). The reaction mixture was stirred under nitrogen at 75 °C for 3 hours, then diluted with water and extracted with EtOAc. The organic layer was concentrated to dryness and purified via silica gel chromatography, eluting with 0-40% EtOAc in heptane. Pure fractions were combined and concentrated to give 2.2 g amber oil that solidified upon standing. Methyl 4-(4-cyano-3,3-dimethyl-but-1-ynyl)-2-methoxy-benzoate **(M6)** (2.2 g, 99%) ¹H NMR (400 MHz, Chloroform-d) δ 7.76 (d, J = 8.0 Hz, 1H), 7.04 (dd, J = 8.0, 1.4 Hz, 1H), 7.01 (d, J = 1.3 Hz, 1H), 3.93 (s, 3H), 3.90 (s, 3H), 2.64 (s, 2H), 1.52 (s, 6H).

### Step 2. Synthesis of N-(2-bromo-5-fluoro-phenyl)-4-aniline (M4)

A solution of 1-bromo-4-fluoro-2-iodo-benzene (4.35 mL, 33.25 mmol) and 4-fluoro-3-methoxy-aniline (7.04 g, 49.88 mmol) in xylene (125 mL) was degassed with nitrogen for 10 minutes, then NaOtBu (9.59 g, 99.79 mmol) and tBuXPhos Pd G3 (792 mg, 0.9970 mmol) were added in one portion. The reaction mixture was stirred overnight at room temperature, diluted with 1 M aqueous HCl, and extracted with EtOAc. The organic layer was concentrated to dryness and purified via silica gel chromatography eluting with 0-35% EtOAc in heptane. Pure fractions were combined and concentrated to give 2.58 g yellow oil. N-(2-bromo-5-fluorophenyl)-4-fluoro-3-methoxy-aniline (2.58 g, 25%) ¹H NMR (300 MHz, Chloroform-d) δ 7.45 (dd, J = 8.8, 5.9 Hz, 1H), 7.09 (dd, J = 11.0, 8.6 Hz, 1H), 6.82 (dd, J = 7.5, 2.6 Hz, 1H), 6.79 - 6.70 (m, 2H), 6.46 (ddd, J = 8.6, 7.8, 2.9 Hz, 1H), 6.08 (s, 1H), 3.90 (s, 3H). ESI-MS m/z 314.0 (M+1)+.

### Step 3. Synthesis of methyl 4-[2-(2-cyano-1,1-dimethyl-ethy)-6-fluoro-1-(4-fluoro-3-methoxyphenyl)indol-3-yl]-2-methoxy-benzoate (M5)

N-(2-bromo-5-fluoro-phenyl)-4-fluoro-3-methoxy-aniline **M4** (73 mg, 0.2324 mmol) and methyl 4-(4-cyano-3,3-dimethyl-but-1-ynyl)-2-methoxy-benzoate **M6** (91 mg, 0.3354 mmol) were dissolved in a mixture of 1,4-dioxane (1.5 mL) and N-cyclohexyl-N-methyl-cyclohexanamine (250 µL, 1.167 mmol). The solution was degassed with nitrogen for 15 minutes, followed by addition of Pd(tBu₃P)₂ (12 mg, 0.02348 mmol). The reaction was heated to 110 °C overnight. The reaction was allowed to cool to room temperature, and then diluted with water (5 mL) and DCM (5 mL). The mixture was passed through a phase separator, the organic phase was evaporated, and the crude material was dissolved in minimal DMSO. Purification by reversed-phase chromatography (Column: C18. Gradient: 0-100% MeCN in water with 0.1% TFA) afforded the product as a light yellow viscous oil. methyl 4-[2-(2-cyano-1,1-dimethyl-ethyl)-6-fluoro-1-(4-fluoro-3-methoxy-phenyl)indol-3-yl]-2-methoxy-benzoate **(M5)** (93 mg, 78%) ¹H NMR (400 MHz, DMSO) δ 7.78 (d, J = 7.8 Hz, 1H), 7.50 (dd, J = 11.2, 8.5 Hz, 1H), 7.36 (dd, J = 26.6, 7.7 Hz, 1H), 7.23 - 7.05 (m, 3H), 7.00 (dd, J = 8.7, 5.4 Hz, 1H), 6.90 (ddd, J = 9.5, 8.6, 2.3 Hz, 1H), 6.47 (dd, J = 9.9, 2.3 Hz, 1H), 3.88 (s, 3H), 3.86 - 3.81 (m, 6H), 2.63 (s, 2H), 1.28 - 1.22 (m, 6H). ESI-MS m/z 505.31 (M+1)+.

### Step 4. Synthesis of 4-[2-(2-cyano-1,1-dimethyl-ethyl)-6-fluoro-1-(4-fluoro-3-methoxyphenyl)indol-3-yl]-2-methoxy-benzoic acid (B14)

Methyl 4-[2-(2-cyano-1,1-dimethyl-ethyl)-6-fluoro-1-(4-fluoro-3-methoxyphenyl)indol-3-yl]-2-methoxy-benzoate **M5** was dissolved (92 mg, 0.1788 mmol) in THF (1.8 mL) and MeOH (900 µL), and NaOH (1 mL of 1 M, 1 mmol) was added. The mixture was heated to 50 °C for 1 hour. The solvent was evaporated, followed by neutralization with HCl (500 µL of 2 M, 1.000 mmol). The solvent was evaporated, and the crude material was dissolved in minimal DMSO. C18 RP Column: Purification by reversed-phase chromatography (Column: C18. Gradient: 0-100% MeCN in water with 0.1% formic acid) afforded the product as a white solid. 4-[2-(2-cyano-1,1-dimethyl-ethyl)-6-fluoro-1-(4-fluoro-3-methoxyphenyl)indol-3-yl]-2-methoxy-benzoic acid (**B14**) (65.4 mg, 74%) ¹H NMR (400 MHz, DMSO) δ 12.67 (s, 1H), 7.77 (d, J = 7.8 Hz, 1H), 7.50 (dd, J = 11.2, 8.5 Hz, 1H), 7.36 (dd, J = 26.7, 7.5 Hz, 1H), 7.21 - 7.12 (m, 2H), 7.06 (t, J = 9.1 Hz, 1H), 7.00 (dd, J = 8.7, 5.4 Hz, 1H), 6.90 (ddd, J = 9.5, 8.6, 2.3 Hz, 1H), 6.47 (dd, J = 9.9, 2.3 Hz, 1H), 3.88 (s, 3H), 3.83 (s, 3H), 2.66 - 2.61 (m, 2H), 1.28 - 1.22 (m, 6H). ESI-MS m/z 491.32 (M+1)+.

### Compounds B15-B23

Compounds from Table 31 were prepared from the corresponding aryl halides and alkynes as for compound **B14.**

**Table 31. Structure and physicochemical data for compounds B15-B23**

| **Compound** | **Structure** | ¹H NMR; **LCMS m/z** [M+H]⁺ |
|---|---|---|
| **B15** | 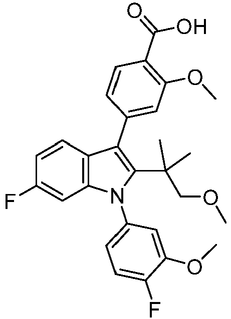 | ¹H NMR (400 MHz, DMSO-d6) δ 12.60 (s, 1H), 7.74 (d, J = 7.8 Hz, 1H), 7.46 (dd, J = 11.3, 8.5 Hz, 1H), 7.31 (d, J = 28.1 Hz, 1H), 7.17 - 6.94 (m, 4H), 6.85 (ddd, J = 9.5, 8.6, 2.3 Hz, 1H), 6.41 (dd, J = 10.0, 2.3 Hz, 1H), 3.88 (s, 3H), 3.83 (s, 3H), 3.09 (d, J = 3.5 Hz, 2H), 3.06 (s, 3H), 1.11 (s, 6H). ESI-MS m/z 496.0 (M+1)+ |
| **B16** | 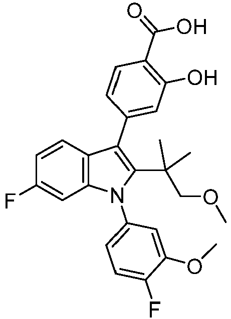 | ¹H NMR (400 MHz, DMSO) δ 10.58 (s, 1H), 7.86 (d, J = 8.2 Hz, 1H), 7.45 (dd, J = 11.3, 8.5 Hz, 1H), 7.33 (d, J = 8.3 Hz, 1H), 7.09 (d, J = 8.3 Hz, 1H), 6.99 - 6.91 (m, 3H), 6.90 - 6.81 (m, 1H), 6.41 (dd, J = 10.0, 2.3 Hz, 1H), 3.93 (s, 3H), 3.87 (s, 3H), 3.07 (d, J = 1.4 Hz, 2H), 3.04 (s, 3H), 1.11 (s, 6H). ESI-MS m/z 496.31 (M+1)+; |
| **B17** | 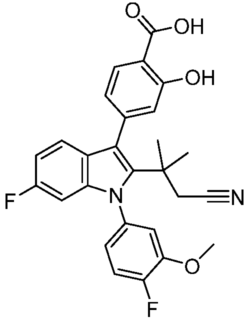 | ¹H NMR (400 MHz, DMSO) δ 14.02 (s, 1H), 11.39 (s, 1H), 7.90 (d, J = 8.0 Hz, 1H), 7.49 (dd, J = 11.2, 8.5 Hz, 1H), 7.38 (dd, J = 7.8, 2.4 Hz, 1H), 7.16 (dd, J = 7.7, 4.0 Hz, 1H), 6.99 (dd, J = 8.8, 5.4 Hz, 3H), 6.90 (td, J = 9.1, 2.3 Hz, 1H), 6.46 (dd, J = 9.9, 2.3 Hz, 1H), 3.88 (s, 3H), 2.64 (s, 2H), 1.26 (s, 6H). ESI-MS m/z 477.19 (M+1)+; |
| **B18** | 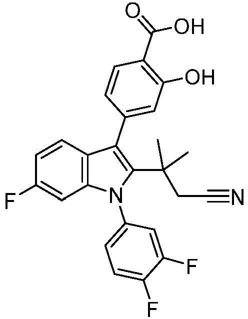 | ¹H NMR (400 MHz, DMSO) δ 11.38 (s, 1H), 7.91 (d, J = 8.0 Hz, 1H), 7.91 - 7.81 (m, 1H), 7.73 (q, J = 9.4 Hz, 1H), 7.46 (d, J = 8.7 Hz, 1H), 7.06 - 6.96 (m, 3H), 6.92 (td, J = 9.2, 8.7, 2.3 Hz, 1H), 6.52 (dd, J = 9.9, 2.3 Hz, 1H), 2.65 (s, 2H), 1.25 (s, 6H). ESI-MS m/z 465.13 (M+1)+; |
| **B19¹** | 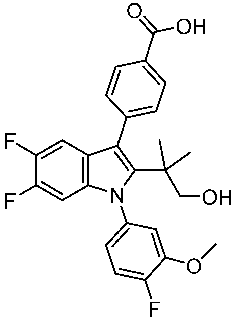 | ¹H NMR (400 MHz, Chloroform-d) δ 8.26 - 8.14 (m, 2H), 7.58 - 7.50 (m, 4H), 7.50 - 7.35 (m, 4H), 7.33 - 7.26 (m, 1H), 7.12 - 7.01 (m, 2H), 6.78 (dd, J = 10.5, 7.7 Hz, 1H), 6.51 (dd, J = 10.6, 6.6 Hz, 1H), 5.44 (s, 2H), 3.93 (s, 3H), 3.50 - 3.30 (m, 2H), 1.58 (s, 1H), 1.14 (s, 6H). ESI-MS m/z calc. 560.35 (M+1)+; |
| **B20¹** | 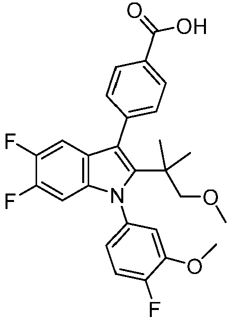 | ¹H NMR (400 MHz, Chloroform-d) δ 8.12 - 7.99 (m, 2H), 7.43 (d, J = 8.0 Hz, 2H), 7.14 (d, J = 8.5 Hz, 2H), 6.94 (dd, J = 7.6, 2.4 Hz, 1H), 6.90 (ddd, J = 8.4, 3.9, 2.4 Hz, 1H), 6.65 (dd, J = 10.5, 7.7 Hz, 1H), 6.35 (dd, J = 10.7, 6.6 Hz, 1H), 3.72 (d, J = 50.7 Hz, 3H), 2.96 (s, 3H), 2.94 (s, 2H), 1.01 (s, 6H). ESI-MS m/z calc. 483.16574, found 483.94 (M+1)+; |
| **B21¹** | 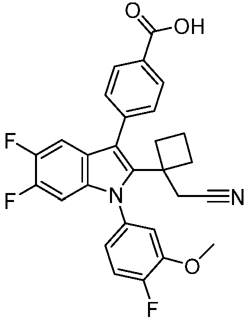 | ¹H NMR (400 MHz, Chloroform-d) δ 8.32 - 8.15 (m, 2H), 7.76 - 7.67 (m, 2H), 7.30 (dd, J = 10.6, 8.3 Hz, 2H), 7.12 (dd, J = 10.4, 7.6 Hz, 1H), 7.09 - 6.99 (m, 2H), 6.69 - 6.54 (m, 1H), 3.94 (s, 3H), 2.92 (d, J = 3.8 Hz, 2H), 2.36 (dd, J = 21.6, 10.0 Hz, 2H), 2.03 - 1.88 (m, 1H), 1.79 - 1.59 (m, 3H). ESI-MS m/z 491.18 (M+1)+; |
| **B22** | 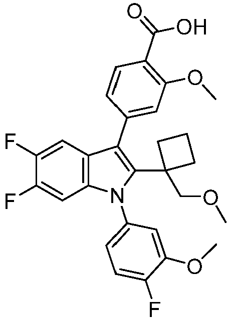 | ¹H NMR (400 MHz, Chloroform-d) δ 10.69 (s, 1H), 8.16 (d, J = 8.0 Hz, 1H), 7.56 (d, J = 1.4 Hz, 1H), 7.30 (dd, J = 8.0, 1.4 Hz, 1H), 7.15 (ddd, J = 10.6, 8.1, 2.5 Hz, 2H), 6.99 - 6.92 (m, 1H), 6.92 - 6.86 (m, 1H), 6.49 (dd, J = 10.5, 6.6 Hz, 1H), 4.04 (s, 3H), 3.83 (s, 3H), 3.66 (s, 2H), 3.35 (s, 3H), 2.04 (q, J = 10.3 Hz, 2H), 1.87 - 1.67 (m, 1H), 1.65 - 1.40 (m, 3H). ESI-MS m/z 526.15 (M+1)+; |
| **B23²** | 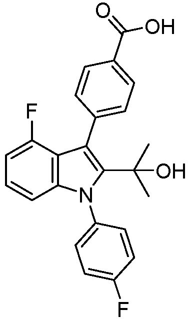 | ¹H NMR (300 MHz, DMSO-d6) δ 12.97 (s, 1H), 8.01 - 7.89 (m, 2H), 7.59 - 7.44 (m, 4H), 7.43 - 7.30 (m, 2H), 7.01 (td, J = 8.1, 5.1 Hz, 1H), 6.73 (dd, J = 11.3, 7.8 Hz, 1H), 6.45 (d, J = 8.2 Hz, 1H), 4.89 (s, 1H), 1.24 (s, 6H). ESI-MS m/z 407.28 (M+1)+ |

| | | |
|---|---|---|
| **^{1.}** Made from benzylester in Larock cyclization. Benzyl deprotection performed at last step instead of ester hydrolysis. **^{2.}** Prepared via Larock cyclization from 4-(3-hydroxy-3-methyl-but-1-ynyl)benzoic acid from 4-(3-hydroxy-3-methyl-but-1-ynyl)benzoic acid. | | |

### Compounds B24 and B25

### 5-[5-fluoro-1-(4-fluoro-3-methyl-phenyl)-2-isopropyl-indol-3-yl]-1,3,4-oxadiazole-2-carboxylic acid (B24) and 2-[5-fluoro-1-(4-fluoro-3-methyl-phenyl)-2-isopropyl-indol-3-yl]-1,3,4-oxadiazole (B25)

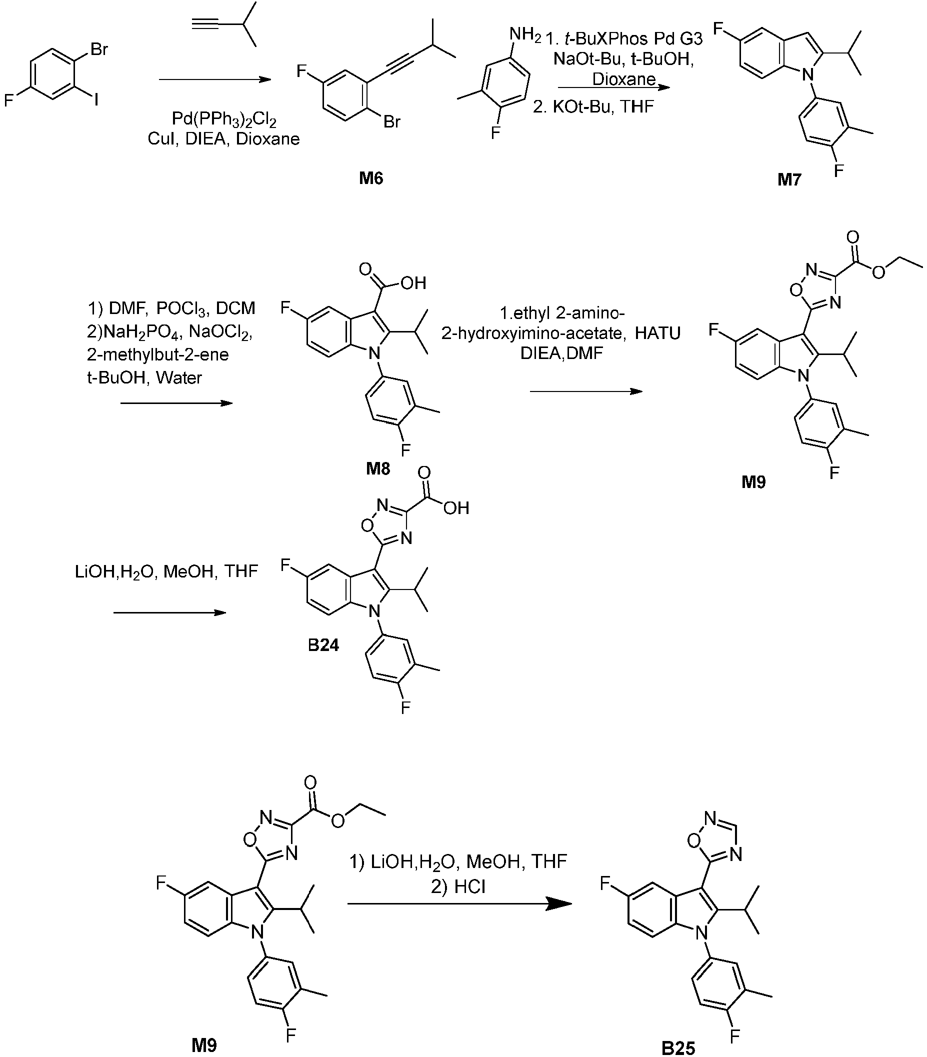

### Step 1. Synthesis of 1-bromo-4-fluoro-2-(3-methylbut-1-ynyl) benzene (M6)

To a solution of 1-bromo-4-fluoro-2-iodo-benzene (21.3 g, 70.79 mmol) and 3-methylbut-1-yne (6.3 g, 92.49 mmol) in 1,4-dioxane (200 mL), DIEA (50 mL, 287.1 mmol), Pd(PPh₃)₂Cl₂ (5.5 g, 7.836 mmol), and CuI (2.97 g, 15.59 mmol) were added. The reaction mixture was stirred overnight at room temperature. The reaction was diluted with water and extracted twice with EtOAc. The organic layer was washed with brine, concentrated to dryness, dissolved in minimal DCM, and dropped into heptane. A light tan solid was filtered. The filtrate was concentrated to dryness and purified via silica gel chromatography. Fractions containing the desired product were combined and concentrated to give 14.1 g amber oil. 1-bromo-4-fluoro-2-(3-methylbut-1-ynyl)benzene **(M6)** (14.1 g, 83%) ¹H NMR (400 MHz, Chloroform-d) δ 7.51 (dd, J = 8.9, 5.3 Hz, 1H), 7.15 (dd, J = 9.0, 3.0 Hz, 1H), 6.87 (ddd, J = 8.9, 7.9, 3.0 Hz, 1H), 2.86 (dq, J = 13.8, 6.9 Hz, 1H), 1.32 (d, J = 6.9 Hz, 6H). ESI-MS m/z calc. 239.995, found 241.0 (M+1)+; Retention time: 0.69 minutes.

### Step 2. Synthesis of 5-fluoro-1-(4-fluoro-3-methyl-phenyl)-2-isopropyl-indole (M7)

To a solution of 1-bromo-4-fluoro-2-(3-methylbut-1-ynyl)benzene (**M6**) (10 g, 41.48 mmol) and 4-fluoro-3-methyl-aniline (6.5 g, 51.94 mmol) in 1,4-dioxane (16 mL) and t-BuOH (160 mL) was added NaOtBu (11.96 g, 124.4 mmol) followed by t-BuXPhos palladacycle G3 (2.6 g, 3.273 mmol). The reaction was stirred overnight at room temperature. The reaction mixture was diluted with water and extracted with EtOAc. The organic layer was concentrated to dryness and purified via silica gel chromatography eluting with EtOAc in heptane. Fractions containing the desired product were combined and concentrated. The resulting material was dissolved in 150 mL THF and treated with KOtBu (10 mL, 80.38 mmol). The mixture was stirred at 50 oC for 30 minutes. The reaction mixture was diluted with water and extracted with EtOAc. The organic layer was concentrated to dryness and purified via silica gel chromatography eluting with 0-20% EtOAc in heptane. Pure fractions were combined and concentrated to give 8 g orange solid. 5-fluoro-1-(4-fluoro-3-methyl-phenyl)-2-isopropyl-indole **(M7)** (8 g, 68%) ¹H NMR (400 MHz, Chloroform-d) δ 7.27 - 7.22 (m, 1H), 7.22 - 7.13 (m, 3H), 6.91 - 6.80 (m, 2H), 6.40 (d, J = 0.8 Hz, 1H), 2.98 - 2.87 (m, 1H), 2.38 (d, J = 2.0 Hz, 3H), 1.25 - 1.21 (m, 6H). ESI-MS m/z calc. 285.1329, found 286.0 (M+1).

### Step 3. Synthesis of 5-fluoro-1-(4-fluoro-3-methyl-phenyl)-2-isopropyl-indole-3-carboxylic acid (M8)

To a solution of **M7** (2.04 g, 7.150 mmol) and dimethyl formamide (765 µL, 9.880 mmol) in dichloromethane (20 mL) at room temperature was added phosphorus oxychloride (1.7 mL, 18.24 mmol). After completion of addition, the reaction was allowed to stir at room temperature for 45 minutes. Aqueous sodium acetate (25.5 mL of 3 M, 76.50 mmol) was added via dropping funnel and allowed to stir for 30 minutes. The organic phase was washed with saturated NaHCO₃, dried over sodium sulfate, and concentrated under reduced pressure. 5-fluoro-1-(4-fluoro-3-methyl-phenyl)-2-isopropyl-indole-3-carbaldehyde was obtained after chromatography (silica gel using 0-20% ethyl acetate / heptane as eluant). Chlorite (Sodium salt) (6.450 g, 71.32 mmol) in water (5 mL) was added to a stirred mixture of 5-fluoro-1-(4-fluoro-3-methyl-phenyl)-2-isopropyl-indole-3-carbaldehyde (1.5 g, 4.746 mmol) and dihydrogen phosphate (sodium salt) (8.56 g, 71.35 mmol) in tert-butanol (26 mL) at room temperature. 2-methylbut-2-ene (24 mL of 2 M, 48.00 mmol) in THF was then added. The pale light brown mixture was stirred at room temperature overnight. The solution was diluted with water and EtOAc, the layers were separated, and the aqueous phase was extracted with EtOAc. The combined organics were washed with brine, dried over sodium sulfate and concentrated under reduced pressure. 5-fluoro-1-(4-fluoro-3-methyl-phenyl)-2-isopropyl-indole-3-carboxylic acid **(M8)** (1.46 g, 93%) ¹H NMR (400 MHz, Chloroform-d) δ 12.42 (s, 1H), 8.02 (dd, J = 10.1, 2.6 Hz, 1H), 7.27 - 7.13 (m, 3H), 6.96 - 6.88 (m, 1H), 6.77 (dd, J = 8.9, 4.5 Hz, 1H), 3.70 - 3.51 (m, 1H), 2.41 (d, J = 2.0 Hz, 3H), 1.40 (dd, J = 7.2, 1.5 Hz, 6H). ESI-MS m/z calc. 329.12274, found 330.43 (M+1)+.

### Step 4. Synthesis of ethyl 5-[5-fluoro-1-(4-fluoro-3-methyl-phenyl)-2-isopropyl-indol-3-yl]-1,2,4-oxadiazole-3-carboxylate (M9)

N,N-Diisopropylethylamine (210 µL, 1.206 mmol) was added to a solution of 5-fluoro-1-(4-fluoro-3-methyl-phenyl)-2-isopropyl-indole-3-carboxylic acid **M8** (200 mg, 0.6023 mmol) and [dimethylamino(triazolo[4,5-b]pyridin-3-yloxy)methylene]-dimethyl-ammonium (Phosphorus Hexafluoride Ion) (345 mg, 0.9073 mmol) in dimethylformamide (3 mL) and allowed to stir for 30 minutes. Ethyl 2-amino-2-hydroxyimino-acetate (120 mg, 0.9083 mmol) was added and allowed to stir overnight. The reaction mixture was diluted with EtOAc and washed with saturated NaHCO₃. The organic phase was washed with water (2x) and brine, dried over sodium sulfate, and concentrated under reduced pressure. The reaction mixture was purified on silica gel using 0-40% EtOAc / heptane as eluant. The resulting white solid was dissolved in dimethylformamide (3 mL) in a 5 mL microwave tube and heated to 160 °C overnight. Ethyl 5-[5-fluoro-1-(4-fluoro-3-methyl-phenyl)-2-isopropyl-indol-3-yl]-1,2,4-oxadiazole-3-carboxylate (**M9**) (89 mg, 33%) ¹H NMR (400 MHz, Chloroform-d) δ 8.07 (dd, J = 9.6, 2.5 Hz, 1H), 7.25 - 7.12 (m, 3H), 7.02 - 6.87 (m, 1H), 6.79 (dd, J = 8.9, 4.4 Hz, 1H), 4.55 (q, J = 7.1 Hz, 2H), 3.76 - 3.63 (m, 1H), 2.39 (d, J = 2.0 Hz, 3H), 1.49 (t, J = 7.1 Hz, 3H), 1.37 (d, J = 7.2 Hz, 6H). ESI-MS m/z 426.51 (M+1)+.

### Step 5a. Synthesis of 5-[5-fluoro-1-(4-fluoro-3-methyl-phenyl)-2-isopropyl-indol-3-yl]-1,2,4-oxadiazole-3-carboxylic acid (B24)

To a solution of ethyl 5-[5-fluoro-1-(4-fluoro-3-methyl-phenyl)-2-isopropyl-indol-3-yl]-1,2,4-oxadiazole-3-carboxylate **M9** (89 mg, 0.1970 mmol) in methanol (4 mL) / tetrahydrofuran (4 mL) was added lithium hydroxide (2 mL of 1 M, 2.000 mmol). The reaction mixture was warmed to 50 °C and allowed to stir for 1 hour. The reaction mixture was concentrated under reduced pressure, acidified with 1N HCl, and extracted with EtOAc (2x). The combined organics were washed with brine, dried over sodium sulfate, and concentrated. 5-[5-fluoro-1-(4-fluoro-3-methyl-phenyl)-2-isopropyl-indol-3-yl]-1,2,4-oxadiazole-3-carboxylic acid (69.8 mg, 88%) (**B24**) ¹H NMR (400 MHz, Chloroform-d/MeOH-d4) δ 8.07 (dd, J = 9.6, 2.6 Hz, 1H), 7.32 - 7.16 (m, 3H), 7.05 - 6.90 (m, 1H), 6.82 (dd, J = 8.9, 4.3 Hz, 1H), 3.73 (q, J = 7.0 Hz, 1H), 2.40 (s, 3H), 1.38 (dd, J = 7.2, 3.6 Hz, 6H). ESI-MS m/z 398.15 (M+1)+.

### Step 5b. Synthesis of 2-[5-fluoro-1-(4-fluoro-3-methyl-phenyl)-2-isopropyl-indol-3-yl]-1,3,4-oxadiazole (B25)

To a solution of ethyl 5-[5-fluoro-1-(4-fluoro-3-methyl-phenyl)-2-isopropyl-indol-3-yl]-1,3,4-oxadiazole-2-carboxylate **M9** (58 mg, 0.1352 mmol) in methanol (3 mL) / tetrahydrofuran (3 mL) was added lithium hydroxide (1.3 mL of 1 M, 1.300 mmol). The reaction mixture was warmed to 50°C and allowed to stir for 1 hour. The reaction mixture was concentrated under reduced pressure, acidified with 1N HCl, and extracted with EtOAc (2x). The combined organics were washed with brine, dried over sodium sulfate, and concentrated giving 2-[5-fluoro-1-(4-fluoro-3-methyl-phenyl)-2-isopropyl-indol-3-yl]-1,3,4-oxadiazole (**B25**) (30.5 mg, 62%). ¹H NMR (400 MHz, DMSO-d6) δ 9.33 (s, 1H), 7.81 (dd, J = 9.8, 2.5 Hz, 1H), 7.57 - 7.48 (m, 1H), 7.48 - 7.38 (m, 2H), 7.13 - 7.01 (m, 1H), 6.89 (dd, J = 8.9, 4.5 Hz, 1H), 3.42 (q, J = 7.2 Hz, 1H), 2.40 - 2.29 (m, 3H), 1.32 (dd, J = 7.2, 0.8 Hz, 6H). ESI-MS m/z calc. 353.13397, found 354.17 (M+1)+.

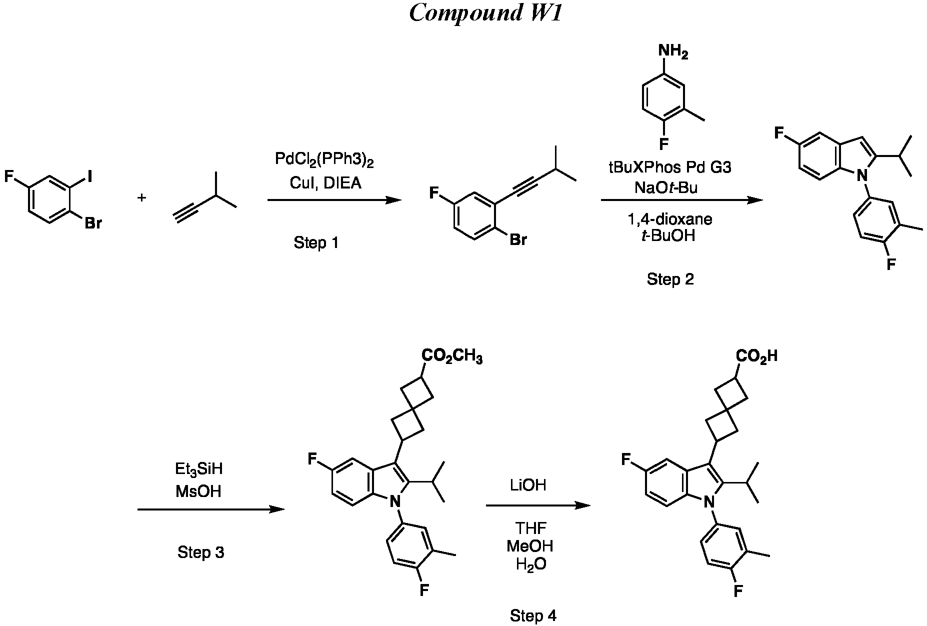

### Step 1. Synthesis of 1-bromo-4-fluoro-2-(3-methylbut-1-ynyl) benzene

A solution of 1-bromo-4-fluoro-2-iodo-benzene (2500 mg, 8.309 mmol) and 3-methylbut-1-yne (736 mg, 10.80 mmol) in 1,4-dioxane (25 mL) and DIEA (5.5 mL, 31.58 mmol) was degassed with nitrogen for 5 minutes. PdCl₂(PPh₃)₂ (670 mg, 0.9518 mmol) and CuI (370 mg, 1.943 mmol) were added and the reaction mixture was stirred at room temperature for 18 hours. The mixture was partitioned between water and DCM, then the organic layer was concentrated to dryness and purified via silica gel chromatography, eluting with EtOAc and heptane. Pure fractions were combined and concentrated to give 1.55 g (77%) of the desired product. ¹H NMR (400 MHz, DMSO) δ 7.78 - 7.61 (m, 1H), 7.38 (dd, J = 9.2, 3.0 Hz, 1H), 7.18 (td, J = 8.6, 3.0 Hz, 1H), 2.87 (hept, J = 6.9 Hz, 1H), 1.24 (dd, J = 6.8, 1.2 Hz, 6H).

### Step 2. Synthesis of 5-fluoro-1-(4-fluoro-3-methyl-phenyl)-2-isopropyl-indole

A solution of 1-bromo-4-fluoro-2-(3-methylbut-1-ynyl)benzene (1.25 g, 5.185 mmol) and 4-fluoro-3-methyl-aniline (815 mg, 6.513 mmol) in t-BuOH (20 mL) and 1,4-dioxane (2 mL) was degassed with nitrogen for 10 minutes. NaOtBu (1.5 g, 15.6 mmol) was added followed by tBuXPhos Pd G3. The reaction mixture was stirred overnight at 80 °C, then diluted with water and extracted with DCM. The organic layer was concentrated to dryness and purified via silica gel chromatography, eluting with EtOAc and heptane. ¹H NMR (400 MHz, Methanol-d4) δ 7.34 - 7.07 (m, 4H), 6.90 - 6.63 (m, 2H), 6.37 (s, 1H), 2.92 (hept, J = 6.8 Hz, 1H), 2.35 (d, J = 2.0 Hz, 3H), 1.20 (d, J = 6.8 Hz, 6H). ESI-MS m/z calc. 285 .1329, found 286.23 (M+1).

### Step 3. Synthesis of methyl 6-[5-fluoro-1-(4-fluoro-3-methyl-phenyl)-2-isopropyl-indol-3-yl]spiro[3. 3]heptane-2-carboxylate

To a solution of 5-fluoro-1-(4-fluoro-3-methyl-phenyl)-2-isopropyl-indole (100 mg, 0.3430 mmol) in DCM (2 mL), methyl 2-oxospiro[3.3]heptane-6-carboxylate (116 mg, 0.6897 mmol), MsOH (72 µL, 1.110 mmol), and Et₃SiH (165 µL, 1.033 mmol) were added. The reaction was stirred for 60 hours at room temperature, then washed with water. The organic layer was concentrated to dryness and purified via silica gel chromatography, eluting with 0-30% EtOAc in heptane. Pure fractions were combined and concentrated to give 102 mg (68%) of the desired product as a colorless oil. ¹H NMR (400 MHz, Chloroform-d) δ 7.46 (ddd, J = 10.3, 2.3, 0.6 Hz, 1H), 7.20 - 7.05 (m, 3H), 6.86 - 6.71 (m, 2H), 3.82 (tt, J = 10.1, 8.4 Hz, 1H), 3.74 (s, 3H), 3.15 (p, J = 8.5 Hz, 1H), 2.99 (hept, J = 7.2 Hz, 1H), 2.82 - 2.68 (m, 2H), 2.53 (dd, J = 8.6, 1.6 Hz, 3H), 2.50 - 2.38 (m, 3H), 2.36 (d, J = 2.0 Hz, 3H), 1.27 (dt, J = 7.2, 1.8 Hz, 6H). ESI-MS m/z calc. 437.21664, found 438.0 (M+1)+.

### Step 4. Synthesis of 6-[5-fluoro-1-(4-fluoro-3-methyl-phenyl)-2-isopropyl-indol-3-yl]spiro[3.3]heptane-2-carboxylic acid

A solution of methyl 6-[5-fluoro-1-(4-fluoro-3-methyl-phenyl)-2-isopropyl-indol-3-yl]spiro[3.3]heptane-2-carboxylate (102 mg, 0.233 mmol) in THF (2 mL) and water (1 mL) was treated with LiOH (30 mg, 1.253 mmol) and stirred at room temperature for 1 hour. The mixture was acidified with 1 M aqueous HCl and extracted with EtOAc. The organic layer was dried over magnesium sulfate, filtered, and concentrated to afford 85 mg (82%) of the desired product. 6-[5-fluoro-1-(4-fluoro-3-methyl-phenyl)-2-isopropyl-indol-3-yl]spiro[3.3]heptane-2-carboxylic acid (85 mg, 82%) ¹H NMR (400 MHz, Chloroform-d) δ 10.80 (s, 1H), 7.46 (dd, J = 10.2, 2.3 Hz, 1H), 7.20 - 7.05 (m, 3H), 6.86 - 6.75 (m, 2H), 3.83 (tt, J = 10.1, 8.4 Hz, 1H), 3.20 (p, J = 8.4 Hz, 1H), 3.00 (p, J = 7.2 Hz, 1H), 2.76 (dt, J = 20.8, 10.7 Hz, 2H), 2.62 - 2.48 (m, 4H), 2.48 - 2.34 (m, 5H), 1.28 (ddd, J = 7.2, 3.6, 1.8 Hz, 6H). ESI-MS m/z calc. 423.201, found 424.0 (M+1)+.

### Compounds W2-W29

Compounds of Table 32 were prepared from the indicated indoles and ketones or aldehydes or acetals as for compound **W1**. Indoles were prepared via Sonogashira coupling followed by Buchwald amination and cyclization of the requisite aryl halides. The ketones and aldehydes in Table 32 were purchased commercially.

**Table 32. Method of preparation, structure, physicochemical data for compounds W2-W29**

| **Compound** | **Structure** | **Indole** | **Ketone, aldehyde or acetal** | ¹H NMR; **LCMS m/z [M+H]⁺** |
|---|---|---|---|---|
| **W2** | 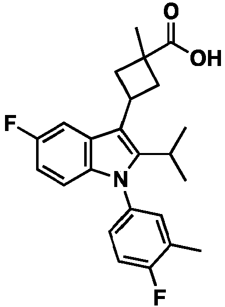 | 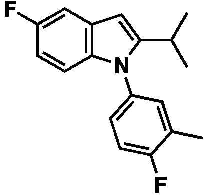 | 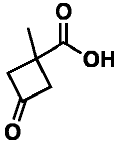 | ¹H NMR (400 MHz, DMSO-d6) δ 12.37 (s, 1H), 7.50 (dd, J = 10.4, 2.4 Hz, 1H), 7.42 - 7.30 (m, 2H), 7.25 (dd, J = 8.6, 4.4 Hz, 1H), 6.87 (dt, J = 9.0, 4.8 Hz, 1H), 6.75 (dd, J = 8.9, 4.7 Hz, 1H), 3.97 (tt, J = 19.3, 9.0 Hz, 1H), 3.08 - 2.76 (m, 1H), 2.70 (dd, J = 11.7, 8.8 Hz, 2H), 2.30 (d, J = 1.9 Hz, 3H), 1.52 (d, J = 12.6 Hz, 3H), 1.22 (dd, J = 12.5, 7.1 Hz, 6H) ESI-MS *m*/*z* 398.38 (M+1) |
| **W3³** | 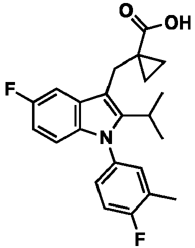 | 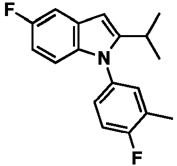 | 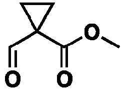 | ¹H NMR (400 MHz, Chloroform-d) δ 7.22 - 7.08 (m, 4H), 6.80 (td, J = 9.0, 2.5 Hz, 1H), 6.71 (dd, J = 8.8, 4.4 Hz, 1H), 3.50 (2H, s) 3.13 (p, J = 7.2 Hz, 1H), 2.36 (d, J = 2.0 Hz, 3H), 1.28 (q, J = 3.8 Hz, 2H), 1.24-1.20 (m, 6H), 0.84 (q, J = 4.0 Hz, 2H) ESI-MS *m*/*z* calc. 383.16968, found 384.24 (M+1) ⁺; Retention time: 0.73 |
| **W4** | 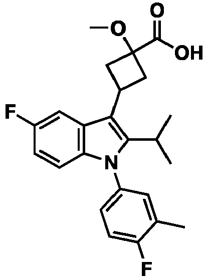 | 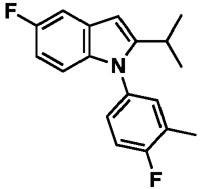 | 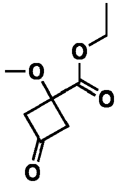 | ¹H NMR (400 MHz, Chloroform-d) δ 7.91 - 7.65 (m, 1H), 7.22 - 7.07 (m, 3H), 6.92 - 6.73 (m, 2H), 4.29 - 3.92 (m, 1H), 3.53 (d, J = 18.8 Hz, 3H), 3.29 (ddt, J = 12.8, 7.9, 2.5 Hz, 1H), 3.07 - 2.93 (m, 2H), 2.71 - 2.62 (m, 1H), 2.37 (d, J = 2.0 Hz, 3H), 1.33 - 1.28 (m, 6H). ESI-MS *m*/*z* 414.0 (M+1)⁺ |
| **W5³** | 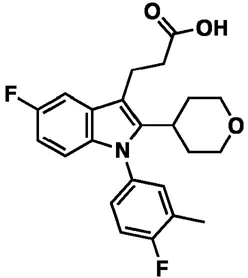 | 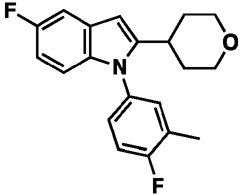 | 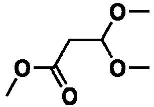 | ¹H NMR (400 MHz, Methanol-d4) δ 7.28 - 7.13 (m, 4H), 6.79 (td, J = 9.0, 2.5 Hz, 1H), 6.71 (dd, J = 8.9, 4.5 Hz, 1H), 3.94 (dd, J = 11.6, 4.2 Hz, 2H), 3.37 - 3.27 (m, 2H), 3.21 - 3.15 (m, 2H), 3.02 - 2.92 (m, 1H), 2.66 - 2.60 (m, 2H), 2.35 (d, J = 2.0 Hz, 3H), 2.04-1.96 (m, 2H), 1.69 (d, J = 12.7 Hz, 2H). ESI-MS *m*/*z* 400.22 (M+1)⁺ |
| **W6³** | 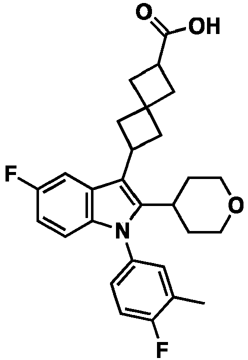 | 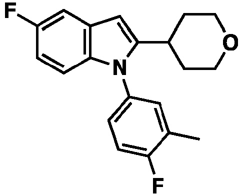 | 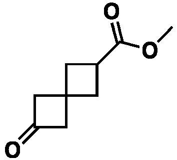 | ¹H NMR (400 MHz, Methanol-d4) δ 7.39 (dd, J = 10.3, 2.3 Hz, 1H), 7.24 (t, J = 8.9 Hz, 1H), 7.21 - 7.17 (m, 1H), 7.12 (dd, J = 8.4, 4.4 Hz, 1H), 6.81 - 6.69 (m, 2H), 3.99 - 3.85 (m, 3H), 3.11 (p, J = 8.4 Hz, 1H), 2.85 (t, J = 12.4 Hz, 1H), 2.69 (dt, J = 29.6, 10.6 Hz, 2H), 2.59 - 2.36 (m, 7H), 2.34 (d, J = 2.0 Hz, 3H), 2.26 - 2.19 (m, 1H), 2.01 (dd, J = 12.7, 3.9 Hz, 2H), 1.64 (d, J = 13.0 Hz, 2H). ESI-MS *m*/*z* 466.26 (M+1) |
| **W7¹** | 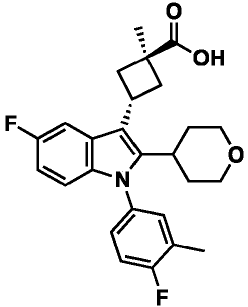 | 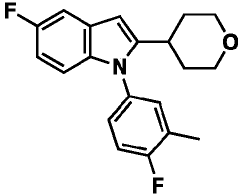 | 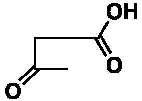 | ¹H NMR (400 MHz, Methanol-d4) δ 7.51 (dd, J = 10.3, 2.3 Hz, 1H), 7.27 - 7.22 (m, 1H), 7.20 (dd, J = 6.8, 2.6 Hz, 1H), 7.12 (ddd, J = 7.9, 4.3, 2.7 Hz, 1H), 6.81 (td, J = 9.0, 2.4 Hz, 1H), 6.74 (dd, J = 8.9, 4.7 Hz, 1H), 4.16 - 4.05 (m, 1H), 3.94 (dd, J = 11.5, 4.2 Hz, 2H), 3.30-3.25 (m, 1H), 2.91 - 2.78 (m, 3H), 2.63 (t, J = 11.4 Hz, 2H), 2.35 (d, J = 2.0 Hz, 3H), 2.11 - 1.95 (m, 3H), 1.64 (d, J = 13.2 Hz, 2H), 1.59 (s, 3H). E SI-MS m/z calc. 439.1959, found 440.28 (M+1) ⁺ ; Retention time: 0.68 minutes |
| **W8¹** | 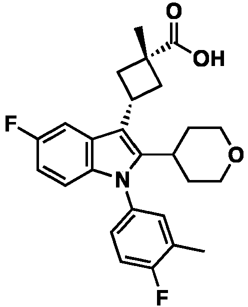 | 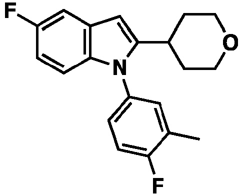 | 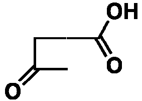 | ¹H NMR (400 MHz, Methanol-d4) δ 7.81 (dd, J = 10.4, 2.3 Hz, 1H), 7.28 - 7.18 (m, 2H), 7.13 (s, 1H), 6.82 - 6.69 (m, 2H), 4.13-4.07 (m, 1H), 3.97 (dd, J = 11.7, 4.2 Hz, 2H), 3.17 (d, J = 10.7 Hz, 2H), 2.86 (d, J = 12.5 Hz, 2H), 2.35 (d, J = 2.0 Hz, 3H), 2.27 (t, J = 7.4 Hz, 1H), 2.20 (dd, J = 11.9, 8.9 Hz, 2H), 2.10-1.95 (m, 2H), 1.69-1.55 (m, 5H) ESI-MS *m*/*z* calc. 439.1959, found 440.28 (M+1) ⁺ ; Retention time: 0.63 minutes |
| **W9^{2, 4}** | 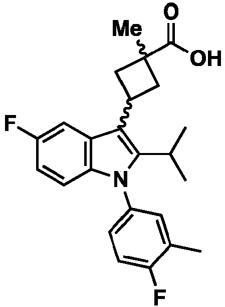 | 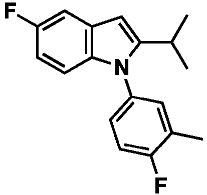 | 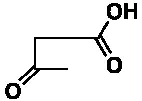 | ¹H NMR (300 MHz, DMSO-d6) δ 7.58 - 7.37 (m, 1H), 7.33 - 7.03 (m, 3H), 6.87 - 6.33 (m, 2H), 4.89 (s, 4H), 4.20 - 3.84 (m, 1H), 2.97 (hept, J = 7.3 Hz, 1H), 2.80 (td, J = 9.2, 2.7 Hz, 2H), 2.68 - 2.48 (m, 2H), 2.35 (d, J = 2.1 Hz, 3H), 1.59 (s, 3H) ESI-MS *m*/*z* calc. 397.18533, found 398.24 (M+1) ⁺ ; Retention time: 0.77 minutes |
| **W10⁴** | 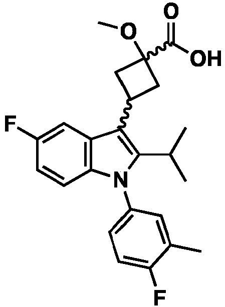 | 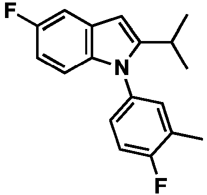 | 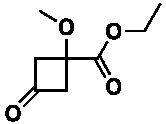 | ¹H NMR (300 MHz, Chloroform-d) δ 7.89 - 7.82 (m, 1H), 7.20 - 7.06 (m, 3H), 6.88 - 6.75 (m, 2H), 4.30 - 4.17 (m, 1H), 3.55 (s, 3H), 3.30 (ddt, J = 11.6, 10.1, 5.0 Hz, 2H), 3.01 (hept, J = 7.3 Hz, 1H), 2.64 (ddd, J = 10.3, 8.8, 3.1 Hz, 2H), 2.36 (d, J = 2.0 Hz, 3H), 1.31 - 1.22 (m, 6H) ESI-MS *m*/*z* calc. 413.18024, found 414.0 (M+1) ⁺ ; Retention time: 0.66 minutes |
| **W11⁴** | 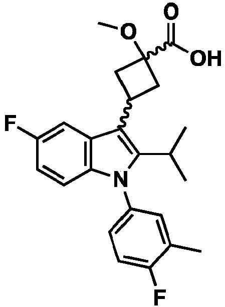 | 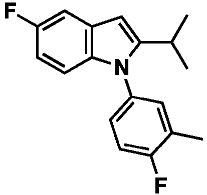 | 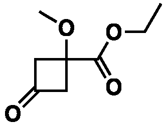 | ¹H NMR (300 MHz, Chloroform-d) δ 7.66 (dd, J = 10.2, 2.3 Hz, 1H), 7.18 - 7.05 (m, 3H), 6.87 - 6.77 (m, 2H), 3.96 (p, J = 9.2 Hz, 1H), 3.48 (s, 3H), 2.97 (q, J = 7.4 Hz, 5H), 2.36 (d, J = 2.0 Hz, 3H), 1.30 - 1.23 (m, 6H). E SI-MS *m*/*z* calc. 413.18024, found 414.0 (M+1) ⁺ ; Retention time: 0.66 minutes |
| **W12³** | 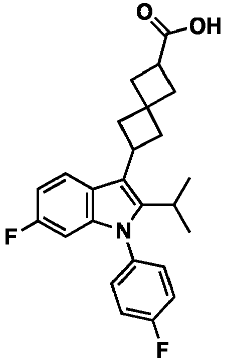 | 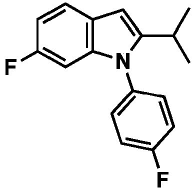 | 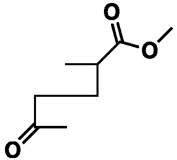 | ¹H NMR (400 MHz, Methanol-d4) δ 7.70 (dd, J = 8.7, 5.2 Hz, 1H), 7.34 - 7.27 (m, 4H), 6.80 (ddd, J = 9.6, 8.7, 2.4 Hz, 1H), 6.42 (dd, J = 9.9, 2.4 Hz, 1H), 3.89 - 3.77 (m, 1H), 3.08 (q, J = 8.5 Hz, 1H), 2.97 (hept, J = 7.3 Hz, 1H), 2.70 (dt, J = 28.2, 10.6 Hz, 2H), 2.57 - 2.30 (m, 6H), 1.24 (dd, J = 7.2, 2.2 Hz, 6H). ESI-MS *m*/*z* calc. 409.18533, found 410.21 (M+1) ⁺ ; Retention time: 0.74 minutes |
| **W13³** | 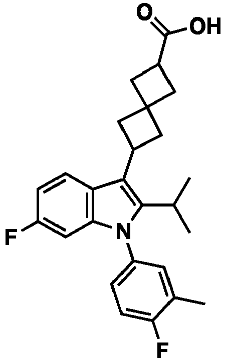 | 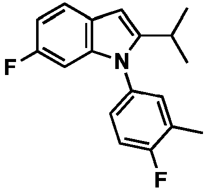 | 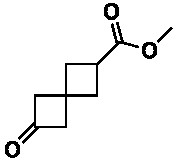 | ¹H NMR (400 MHz, Methanol-d4) δ 7.70 (dd, J = 8.8, 5.2 Hz, 1H), 7.23 (t, J = 8.9 Hz, 1H), 7.18 (dd, J = 6.8, 2.6 Hz, 1H), 7.11 (dt, J = 7.9, 3.6 Hz, 1H), 6.79 (ddd, J = 9.5, 8.7, 2.4 Hz, 1H), 6.42 (dd, J = 10.0, 2.3 Hz, 1H), 3.89 - 3.76 (m, 1H), 3.08 (q, J = 8.5 Hz, 1H), 2.97 (h, J = 7.2 Hz, 1H), 2.69 (dt, J = 28.4, 10.6 Hz, 2H), 2.56 - 2.29 (m, 6H), 1.24 (d, 6H). ESI-MS *m*/*z* calc. 423.201, found 424.26 (M+1) ⁺ ; Retention time: 0.81 minutes |
| **W14¹** | 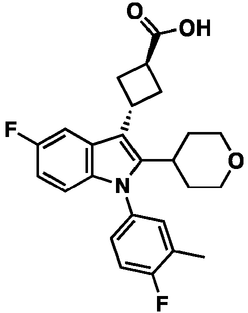 | 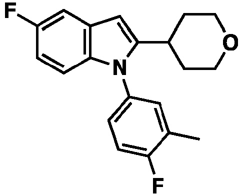 | 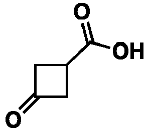 | ¹H NMR (400 MHz, DMSO-d6) δ 12.23 (br s, 1H), 7.67 (dd, J = 10.3, 2.5 Hz, 1H), 7.39 - 7.33 (m, 2H), 7.24 (ddd, J = 8.9, 4.6, 2.8 Hz, 1H), 6.89 (td, J = 9.1, 2.5 Hz, 1H), 6.77 (dd, J = 8.9, 4.7 Hz, 1H), 4.16 (p, J = 9.4 Hz, 1H), 3.87 (dd, J = 11.4, 4.0 Hz, 2H), 3.35 (t, J = 9.9 Hz, 1H), 3.22 - 3.11 (m, 2H), 2.91 - 2.68 (m, 3H), 2.55-2.45 (2H, m), 2.30 (d, J = 1.9 Hz, 3H), 1.93 - 1.78 (m, 2H), 1.62 (d, J = 13.5 Hz, 2H). ESI-MS *m*/*z* calc. 425.18024, found 426.17 (M+1) ⁺ ; Retention time: 4.37 minutes |
| **W15¹** | 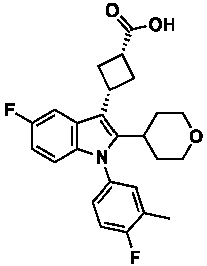 | 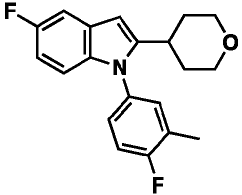 | 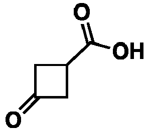 | ¹H NMR (400 MHz, DMSO-d6) δ 12.33 (s, 1H), 7.83 (dd, J = 10.5, 2.5 Hz, 1H), 7.40 - 7.31 (m, 2H), 7.30 - 7.20 (m, 1H), 6.89 (td, J = 9.1, 2.5 Hz, 1H), 6.77 (dd, J = 8.9, 4.7 Hz, 1H), 3.96 (q, J = 9.5 Hz, 1H), 3.87 (d, J = 9.4 Hz, 2H), 3.24 - 3.13 (m, 3H), 2.79 (p, J = 8.6, 7.6 Hz, 3H), 2.55-2.45 (m, 2H), 2.30 (s, 3H), 1.89 (t, J = 12.6 Hz, 2H), 1.62 (m, 2H). ESI-MS *m*/*z* calc. 425.18024, found 426.2 (M+1) ⁺ ; Retention time: 4.27 minute |
| **W16³** | 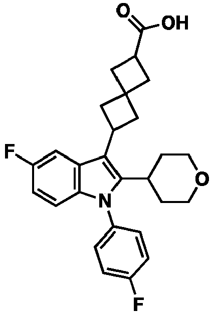 | 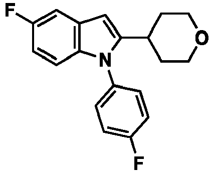 | 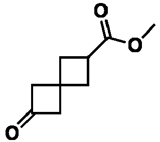 | ¹H NMR (400 MHz, DMSO-d6) δ 7.50 - 7.36 (m, 5H), 6.88 (td, J = 9.1, 2.5 Hz, 1H), 6.73 (dd, J = 8.9, 4.6 Hz, 1H), 3.91 - 3.72 (m, 3H), 3.18 (t, J = 11.6 Hz, 2H), 2.99 (q, J = 8.3 Hz, 1H), 2.82 - 2.40 (m, 5H), 2.38 - 2.23 (m, 4H), 1.90 - 1.75 (m, 2H), 1.60 (d, J = 12.9 Hz, 2H). ESI-MS *m*/*z* calc. 451.1959, found 452.26 (M+1) ⁺ ; Retention time: 0.61 minutes |
| **W17^{3, 4}** | 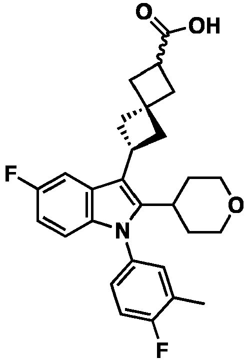 | 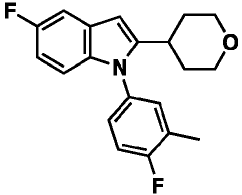 | 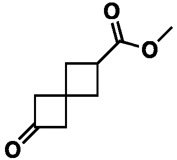 | ¹H NMR (400 MHz, DMSO-d6) δ 7.43 - 7.32 (m, 3H), 7.25 (dd, J = 8.0, 4.4 Hz, 1H), 6.87 (td, J = 9.1, 2.4 Hz, 1H), 6.74 (dd, J = 8.9, 4.7 Hz, 1H), 3.83 (dd, J = 17.8, 9.3 Hz, 3H), 3.18 (t, J = 11.3 Hz, 2H), 3.01 (p, J = 8.3 Hz, 1H), 2.76 (d, J = 12.5 Hz, 1H), 2.69 - 2.54 (m, 2H), 2.53-2.47 (m, 2H), 2.40 - 2.26 (m, 7H), 1.90 - 1.77 (m, 2H), 1.61 (s, 2H) ESI-MS *m*/*z* calc. 465.21155, |
| **W18^{3, 4}** | 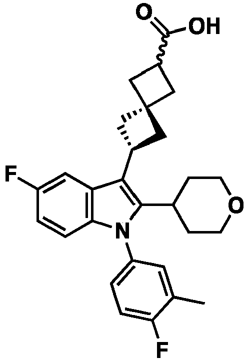 | 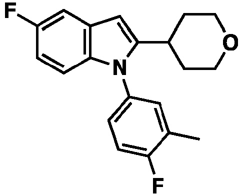 | 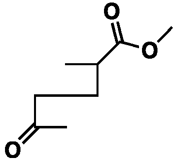 | ¹H NMR (400 MHz, DMSO-d6) δ 7.43 - 7.32 (m, 3H), 7.25 (dd, J = 8.0, 4.4 Hz, 1H), 6.87 (td, J = 9.1, 2.4 Hz, 1H), 6.74 (dd, J = 8.9, 4.7 Hz, 1H), 3.83 (dd, J = 17.8, 9.3 Hz, 3H), 3.18 (t, J = 11.3 Hz, 2H), 3.01 (p, J = 8.3 Hz, 1H), 2.76 (d, J = 12.5 Hz, 1H), 2.69 - 2.54 (m, 2H), 2.53-2.47 (m, 2H), 2.40 - 2.26 (m, 7H), 1.90 - 1.77 (m, 2H), 1.61 (s, 2H). ESI-MS *m*/*z* calc. 465.21155, |
| **W19³** | 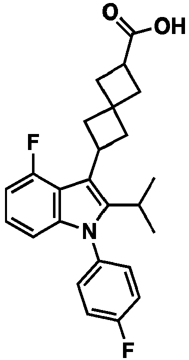 | 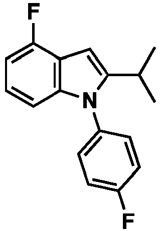 | 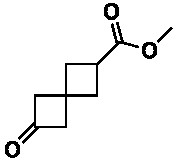 | ¹H NMR (400 MHz, DMSO-d6) δ 12.02 (s, 1H), 7.50 - 7.38 (m, 4H), 6.99 (td, J = 8.0, 5.1 Hz, 1H), 6.88 - 6.81 (m, 1H), 6.55 (d, J = 8.1 Hz, 1H), 3.90 - 3.77 (m, 1H), 3.04 - 2.86 (m, 2H), 2.48 - 2.14 (m, 8H), 1.23 (d, J = 3.5 Hz, 3H), 1.21 (d, J = 3.5 Hz, 3H). ESI-MS *m*/*z* calc. 409.18533, found 410.21 (M+1) ⁺ ; Retention time: 0.74 minutes |
| **W20** | 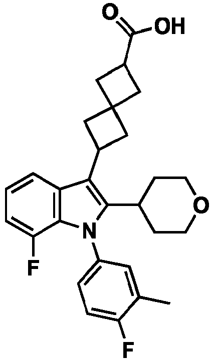 | 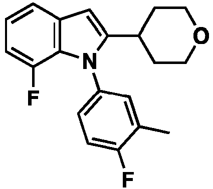 | 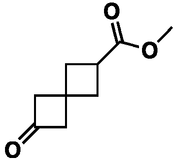 | ¹H NMR (400 MHz, Chloroform-d) δ 10.30 (s, 2H), 7.43 (dd, J = 10.2, 2.4 Hz, 1H), 7.15 (t, J = 8.8 Hz, 1H), 7.08 (dd, J = 6.8, 2.5 Hz, 1H), 7.03 (dt, J = 7.7, 3.7 Hz, 1H), 6.81 (td, J = 8.9, 2.4 Hz, 1H), 6.75 (dd, J = 8.9, 4.7 Hz, 1H), 4.06 (dd, J = 11.6, 4.2 Hz, 2H), 3.84 (tt, J = 9.9, 8.5 Hz, 1H), 3.35 (td, J = 12.0, 2.0 Hz, 2H), 3.18 (p, J = 8.4 Hz, 1H), 2.84 - 2.68 (m, 3H), 2.59 - 2.37 (m, 6H), 2.34 (d, J = 1.9 Hz, 3H), 2.14 - 2.07 (m, 2H), 1.67 - 1.58 (m, 2H). ESI-MS *m*/*z* calc. 465.21155, found 466.0 (M+1) ⁺ ; Retention time: 0.68 minutes |
| **W21³** | 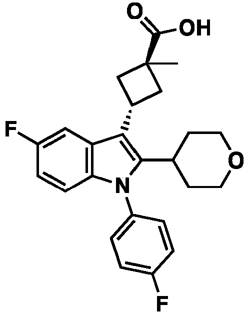 | 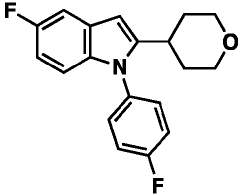 | 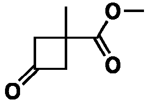 | ¹H NMR (400 MHz, DMSO-d6) δ 12.38 (s, 1H), 7.54 - 7.40 (m, 5H), 6.91 (td, J = 9.1, 2.5 Hz, 1H), 6.76 (dd, J = 8.9, 4.7 Hz, 1H), 3.97 (p, J = 9.7 Hz, 1H), 3.86 (dd, J = 11.5, 4.0 Hz, 2H), 3.16 (t, J = 11.4 Hz, 2H), 2.79 - 2.70 (m, 3H), 2.57-2.53 (m, 2H), 1.83 (td, J = 12.9, 8.6 Hz, 2H), 1.62 (d, J = 11.8 Hz, 2H), 1.51 (s, 3H). ESI-MS *m*/*z* calc. 425.18024, found 426.24 (M+1) ⁺ ; Retention time: 0.97 minutes |
| **W22¹** | 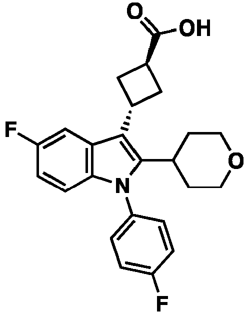 | 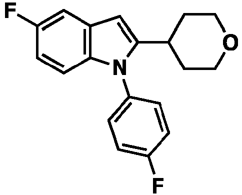 | 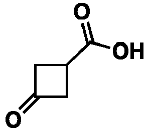 | ¹H NMR (400 MHz, DMSO-d6) δ 12.24 (s, 1H), 7.68 (dd, J = 10.2, 2.2 Hz, 1H), 7.44 (d, J = 6.4 Hz, 4H), 6.90 (dd, J = 10.2, 7.9 Hz, 1H), 6.76 (dd, J = 8.8, 4.5 Hz, 1H), 4.16 (t, J = 9.4 Hz, 1H), 3.92 - 3.80 (m, 2H), 3.35 - 3.26 (m, 2H), 3.17 (t, J = 11.7 Hz, 2H), 2.92 - 2.63 (m, 4H), 1.92 - 1.74 (m, 2H), 1.62 (d, J = 13.2 Hz, 2H). ESI-MS *m*/*z* calc. 411.1646, found 412.19 (M+1) ⁺ ; Retention time: 0.52 minutes |
| **W23¹** | 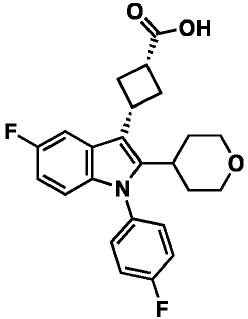 | 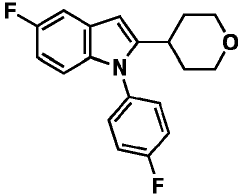 | 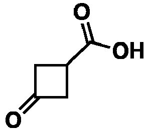 | ¹H NMR (400 MHz, DMSO-d6) δ 12.33 (s, 1H), 7.84 (dd, J = 10.4, 2.1 Hz, 1H), 7.53 - 7.37 (m, 4H), 6.95 - 6.84 (m, 1H), 6.76 (dd, J = 9.2, 4.6 Hz, 1H), 3.95 (t, J = 9.3 Hz, 1H), 3.91 - 3.82 (m, 2H), 3.34 - 3.28 (m, 2H), 3.18 (t, J = 11.7 Hz, 3H), 2.79 (q, J = 10.2, 9.4 Hz, 3H), 1.98 - 1.81 (m, 2H), 1.62 (d, J = 12.6 Hz, 2H). ESI-MS *m*/*z* calc. 411.1646, found 412.51 (M+1) ⁺ ; Retention time: 0.5 minutes |
| **W24** | 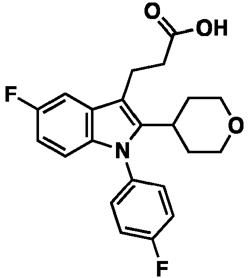 | 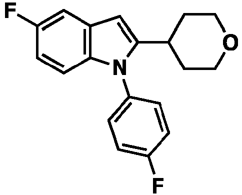 | 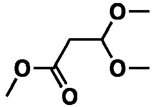 | ¹H NMR (400 MHz, DMSO-d6) δ 12.22 (s, 1H), 7.50 - 7.38 (m, 4H), 7.35 (dd, J = 9.9, 2.5 Hz, 1H), 6.91 - 6.84 (m, 1H), 6.73 (dd, J = 8.9, 4.5 Hz, 1H), 3.85 (dd, J = 11.4, 4.0 Hz, 2H), 3.20 (dd, J = 12.2, 10.3 Hz, 2H), 3.12 - 3.01 (m, 2H), 2.85 (t, J = 12.2 Hz, 1H), 2.58 - 2.52 (m, 2H), 1.90 - 1.75 (m, 2H), 1.64 (d, J = 12.2 Hz, 2H). ESI-MS *m*/*z* calc. 385.14896, found 386.22 (M+1) ⁺ ; Retention time: 0.49 minutes |
| **W25** | 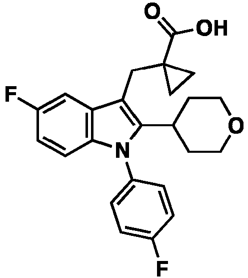 | 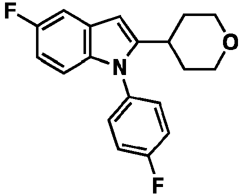 | 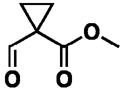 | ¹H NMR (400 MHz, DMSO-d6) δ 12.29 (s, 1H), 7.52 - 7.38 (m, 4H), 7.30 (dd, J = 9.9, 2.5 Hz, 1H), 6.89 - 6.81 (m, 1H), 6.66 (dd, J = 9.0, 4.4 Hz, 1H), 3.81 (dd, J = 11.5, 3.9 Hz, 2H), 3.36 (s, 2H), 3.21 (t, J = 11.4 Hz, 2H), 2.99 (t, J = 12.3 Hz, 1H), 1.80 - 1.65 (m, 2H), 1.59 (d, J = 12.8 Hz, 2H), 1.05 (q, J = 4.0, 3.6 Hz, 2H), 0.67 (t, J = 3.5 Hz, 2H). ESI-MS *m*/*z* calc. 411.1646, found 412.19 (M+1) ⁺ ; Retention time: 0.55 minutes |
| **W26¹** | 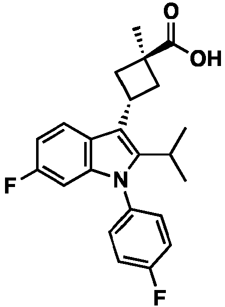 | 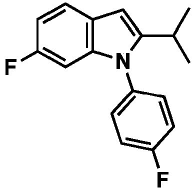 | 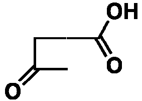 | ¹H NMR (400 MHz, Chloroform-d) δ 7.82 (dd, J = 8.7, 5.2 Hz, 1H), 7.28 - 7.20 (m, 4H), 6.91 (ddd, J = 9.4, 8.7, 2.4 Hz, 1H), 6.55 (dd, J = 9.8, 2.4 Hz, 1H), 4.18 - 4.06 (m, 1H), 3.04 - 2.89 (m, 3H), 2.74 (td, J = 10.2, 2.7 Hz, 2H), 1.67 (s, 3H), 1.27 (s, 3H), 1.25 (s, 3H). ESI-MS *m*/*z* calc. 383.16968, found 384.2 (M+1) ⁺ ; Retention time: 0.71 minutes |
| **W27** | 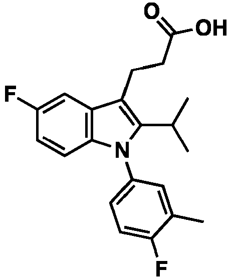 | 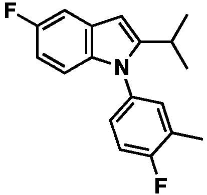 | 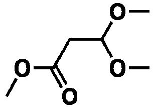 | ¹H NMR (400 MHz, DMSO-d6) δ 12.23 (s, 1H), 7.40 - 7.27 (m, 3H), 7.27 - 7.19 (m, 1H), 6.88 - 6.80 (m, 1H), 6.73 (dd, J = 8.8, 4.5 Hz, 1H), 3.08 - 3.00 (m, 2H), 2.95 (q, J = 7.2 Hz, 1H), 2.54 (s, 2H), 2.31 (d, J = 2.0 Hz, 3H), 1.23 (dd, J = 7.2, 2.3 Hz, 6H). ESI-MS *m*/*z* calc. 357.15402, found 358.51 (M+1) ⁺ ; Retention time: 0.63 minutes |
| **W28** | 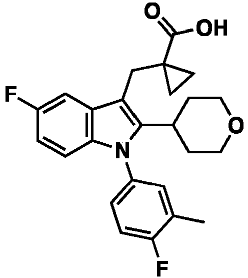 | 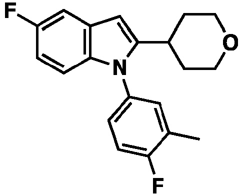 | 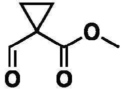 | ¹H NMR (400 MHz, DMSO-d6) δ 12.24 (s, 1H), 7.44 - 7.20 (m, 4H), 6.90 - 6.78 (m, 1H), 6.67 (dd, J = 8.9, 4.5 Hz, 1H), 3.82 (dd, J = 11.5, 3.9 Hz, 2H), 3.36 (s, 2H), 3.21 (t, J = 11.4 Hz, 2H), 3.07 - 2.89 (m, 1H), 2.31 (d, J = 1.9 Hz, 3H), 1.83 - 1.66 (m, 2H), 1.59 (d, J = 12.1 Hz, 2H), 1.04 (q, J = 3.7 Hz, 2H), 0.65 (q, J = 3.9 Hz, 2H). ESI-MS *m*/*z* calc. 425.18024, found 426.28 (M+1) ⁺ ; Retention time: 0.61 minutes |
| **W29** | 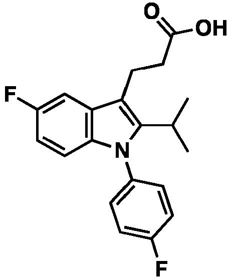 | 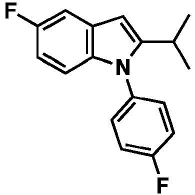 | 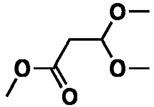 | ¹H NMR (400 MHz, DMSO-d6) δ 12.22 (s, 1H), 7.50 - 7.36 (m, 3H), 7.32 (s, 1H), 6.91 - 6.78 (m, 2H), 6.77 - 6.67 (m, 1H), 4.03 (d, J = 7.2 Hz, 1H), 3.11 - 2.91 (m, 4H), 1.22 (d, J = 7.2 Hz, 6H). ESI-MS *m*/*z* calc. 343.1384, found 342.62 (M+1) ⁺ ; Retention time: 0.86 minutes |

| | | | | |
|---|---|---|---|---|
| **^{1.}** No ester hydrolysis step required. **^{2.}** 5 eq. TFA used instead of MsOH in Step 3. **^{3.}** 1,4-dioxane and water used for Step 4. **^{4.}** SFC purification was used to isolate final compound; absolute stereochemistry unknown. | | | | |

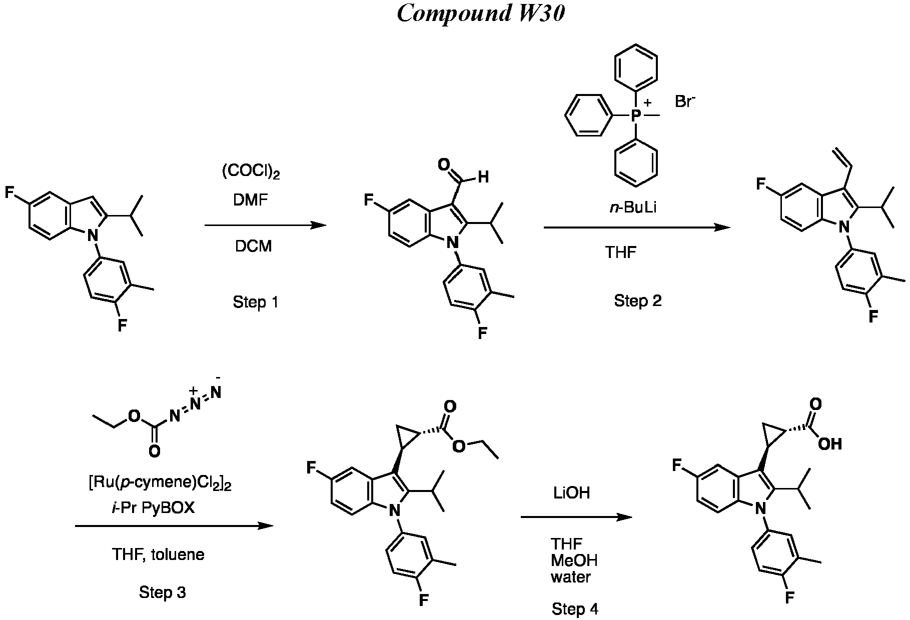

### Step 1. Synthesis of 5-fluoro-1-(4-fluoro-3-methyl-phenyl)-2-isopropyl-indole-3-carbaldehyde

To a solution of oxalyl chloride (6.7 mL of 2 M in DCM, 13.4 mmol) in DCM (20 mL) at 0 °C was added dropwise anhydrous DMF (6.5 mL, 84 mmol). The reaction mixture was stirred at 0 °C and then a solution of 5-fluoro-1-(4-fluoro-3-methyl-phenyl)-2-isopropyl-indole (2 g, 7.01 mmol) in DCM (20 mL) was added dropwise. The reaction mixture was gradually warmed to room temperature and stirred for 3 hours, then treated with saturated aqueous NaHCO₃ and stirred an additional 30 minutes. The organic layer was removed, concentrated to dryness, and purified via silica gel chromatography, eluting with 0-35% EtOAc in heptane. Pure fractions were combined and concentrated to give 1.89 g (86%) of the desired product as a light tan solid. 5-fluoro-1-(4-fluoro-3-methyl-phenyl)-2-isopropyl-indole-3-carbaldehyde (1.89 g, 86%) ¹H NMR (400 MHz, Chloroform-d) δ 10.51 (s, 1H), 8.08 (dd, J = 9.4, 2.5 Hz, 1H), 7.25 (t, J = 8.7 Hz, 1H), 7.21 - 7.12 (m, 2H), 6.94 (td, J = 8.9, 2.6 Hz, 1H), 6.83 - 6.77 (m, 1H), 3.19 (hept, J = 7.2 Hz, 1H), 2.41 (d, J = 2.0 Hz, 3H), 1.48 (dd, J = 7.2, 3.0 Hz, 6H). ESI-MS m/z calc. 313.1278, found 314.0 (M+1)+.

### Step 2. Synthesis of 5-fluoro-1-(4-fluoro-3-methyl-phenyl)-2-isopropyl-3-vinyl-indole

To a suspension of methyl(triphenyl)phosphonium bromide (1.44 g, 4.03 mmol) in THF (25 mL) at 0 °C under nitrogen was added dropwise n-BuLi (1.61 mL of 2.5 M, 4.03 mmol) in hexane. The mixture was stirred at 0 °C for 1 hour and then a solution of 5-fluoro-1-(4-fluoro-3-methyl-phenyl)-2-isopropyl-indole-3-carbaldehyde (900 mg, 2.872 mmol) in THF (6 mL) was added dropwise. The reaction mixture was gradually warmed to room temperature and stirred for 3 hours. The reaction mixture was quenched with water and extracted with EtOAc. The organic layer was washed with brine, concentrated to dryness, and purified via silica gel chromatography, eluting with 0-35% EtOAc in heptane. Pure fractions were combined and concentrated to afford 350 mg (39%) of the desired product as a white solid. ¹H NMR (400 MHz, Chloroform-d) δ 7.57 (dd, J = 10.2, 2.4 Hz, 1H), 7.22 - 7.05 (m, 4H), 6.86 (td, J = 8.9, 2.4 Hz, 1H), 6.79 (dd, J = 8.8, 4.7 Hz, 1H), 5.66 (dd, J = 17.7, 1.6 Hz, 1H), 5.32 (dd, J = 11.5, 1.6 Hz, 1H), 3.04 (hept, J = 7.2 Hz, 1H), 2.38 (d, J = 2.0 Hz, 3H), 1.34 (dd, J = 7.2, 2.6 Hz, 6H). ESI-MS m/z calc. 311.14856, found 312.0 (M+1)+.

### Step 3. Synthesis of ethyl trans-2-[5-fluoro-1-(4-fluoro-3-methyl-phenyl)-2-isopropyl-indol-3-yl]cyclopropanecarboxylate

To a solution of 5-fluoro-1-(4-fluoro-3-methyl-phenyl)-2-isopropyl-3-vinyl-indole (175 mg, 0.562 mmol), i-Pr PyBOX, and [Ru(p-cymene)Cl₂]₂ (14 mg, 0.023 mmol) in THF (4 mL) at 55 °C was added dropwise a solution of ethyl 2-diazoacetate (325 µL, 3.090 mmol) in toluene (1.6 mL) over 60 minutes. The reaction mixture was then stirred at 55 °C for 1 hour, diluted with water, and extracted with EtOAc. The combined organics were concentrated to dryness and purified via reverse phase HPLC eluting with 5-90% MeCN in water with 0.1% TFA. Pure fractions were combined, diluted with water, and extracted with EtOAc. The organic layer was dried over magnesium sulfate, filtered, and concentrated to give 124 mg (56%) of the desired product as a colorless film (approximately 9:1 mixture of trans-enantiomers). ¹H NMR (300 MHz, Chloroform-d) δ 7.32 (dd, J = 9.9, 2.4 Hz, 1H), 7.21 - 7.07 (m, 3H), 6.81 (td, J = 8.9, 2.4 Hz, 1H), 6.74 (dd, J = 8.8, 4.7 Hz, 1H), 4.40 - 4.23 (m, 2H), 3.13 (hept, J = 7.2 Hz, 1H), 2.57 (ddd, J = 9.1, 6.7, 4.3 Hz, 1H), 2.37 (d, J = 2.0 Hz, 3H), 2.13 - 2.03 (m, 1H), 1.73 (ddd, J = 9.1, 5.0, 4.0 Hz, 1H), 1.48 (dddd, J = 8.3, 6.7, 4.0, 1.6 Hz, 1H), 1.41 - 1.30 (m, 9H). ESI-MS m/z calc. 397.46, found 398.0 (M+1)+.

### Step 4. Synthesis of trans-2-[5-fluoro-1-(4-fluoro-3-methyl-phenyl)-2-isopropyl-indol-3-yl]cyclopropanecarboxylic acid

To a solution of ethyl trans-2-[5-fluoro-1-(4-fluoro-3-methyl-phenyl)-2-isopropyl-indol-3-yl]cyclopropanecarboxylate (120 mg, 0.301 mmol) in THF (2 mL), water (1 mL), and MeOH (1 mL) was added LiOH (71 mg, 2.965 mmol). The reaction mixture was stirred overnight then acidified with aqueous 1 M HCl and extracted with EtOAc. The organic layer was dried over magnesium sulfate, filtered, concentrated and purified via SFC to afford 40 mg (35%) of the desired product. ¹H NMR (400 MHz, Chloroform-d) δ 7.34 (dd, J = 9.8, 2.4 Hz, 1H), 7.21 - 7.10 (m, 3H), 6.82 (td, J = 9.0, 2.4 Hz, 1H), 6.75 (dd, J = 8.9, 4.6 Hz, 1H), 3.15 (hept, J = 7.2 Hz, 1H), 2.69 (ddd, J = 9.1, 6.9, 4.2 Hz, 1H), 2.41 - 2.35 (m, 3H), 2.13 - 2.06 (m, 1H), 1.86 - 1.79 (m, 1H), 1.58 (dddd, J = 8.4, 6.4, 4.0, 2.2 Hz, 1H), 1.34 (ddd, J = 7.1, 6.2, 4.8 Hz, 6H). ESI-MS m/z calc. 369.40, found 370.0 (M+1)+.

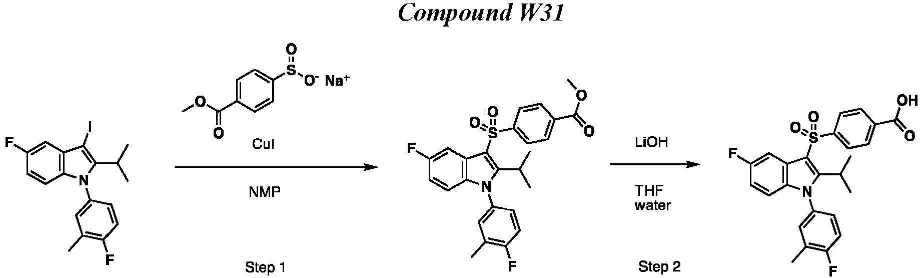

### Step 1. Synthesis of methyl 4-[5-fluoro-1-(4-fluoro-3-methyl-phenyl)-2-isopropyl-indol-3-yl]sulfonylbenzoate

A suspension of 5-fluoro-1-(4-fluoro-3-methyl-phenyl)-3-iodo-2-isopropyl-indole (200 mg, 0.486 mmol), 4-methoxycarbonylbenzenesulfinic acid (sodium salt) (540 mg, 2.419 mmol), and CuI (460 mg, 2.415 mmol) in NMP (3 mL) was stirred in a sealed tube at 130 °C for 2 hours, then diluted with water and extracted with EtOAc. The organic layer was concentrated to dryness and purified via silica gel chromatography, eluting with 0-60% EtOAc in heptane. Pure fractions were combined and concentrated to give 30 mg (13%) of the desired product as an off-white solid. ¹H NMR (400 MHz, Chloroform-d) δ 8.19 - 8.13 (m, 2H), 8.12 - 8.06 (m, 2H), 7.92 (dd, J = 9.6, 2.5 Hz, 1H), 7.23 - 7.11 (m, 3H), 6.93 (td, J = 8.9, 2.5 Hz, 1H), 6.69 (dd, J = 9.0, 4.4 Hz, 1H), 4.13 - 3.99 (m, 1H), 3.96 (s, 3H), 2.37 (d, J = 2.0 Hz, 3H), 1.17 (d, J = 7.2 Hz, 6H). ESI-MS m/z calc. 483.1316, found 484.0 (M+1)+.

### Step 2. Synthesis of 4-[5-fluoro-1-(4-fluoro-3-methyl-phenyl)-2-isopropyl-indol-3-yl]sulfonylbenzoic acid

To a solution of methyl 4-[5-fluoro-1-(4-fluoro-3-methyl-phenyl)-2-isopropyl-indol-3-yl]sulfonylbenzoate (30 mg, 0.062 mmol) in THF (750 µL) and water (250 µL) was added LiOH (10 mg, 0.4176 mmol). The reaction mixture was stirred at room temperature for 30 minutes, then acidified with 1 M aqueous HCl and extracted with EtOAc. The organic layer was dried over magnesium sulfate, filtered, and concentrated to afford 14 mg (43%) of the desired product. ¹H NMR (400 MHz, Chloroform-d) δ 8.23 - 8.18 (m, 2H), 8.11 - 8.06 (m, 2H), 7. 90 (dd, J = 9.6, 2.4 Hz, 1H), 7.20 - 7.08 (m, 3H), 6.91 (td, J = 8.9, 2.5 Hz, 1H), 6.67 (dd, J = 8.9, 4.4 Hz, 1H), 4.02 (p, J = 7.0 Hz, 1H), 2.34 (d, J = 1.9 Hz, 3H), 1.15 (d, J = 7.2 Hz, 6H). ESI-MS m/z calc. 469.11594, found 470.0 (M+1)+.

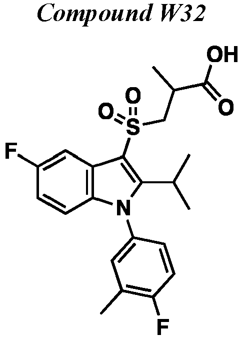

3-[5-fluoro-1-(4-fluoro-3-methyl-phenyl)-2-isopropyl-indol-3-yl]sulfonyl-2-methyl-propanoic acid was prepared according to the procedure of compound **W31,** except for using sodium 3-methoxy-2-methyl-3-oxo-propane-1-sulfinic acid in Step 1. ¹H NMR (400 MHz, Chloroform-d) δ 7.70 (dd, J = 9.6, 2.5 Hz, 1H), 7.23 - 7.11 (m, 3H), 6.88 (td, J = 8.9, 2.5 Hz, 1H), 6.68 (dd, J = 9.0, 4.3 Hz, 1H), 3.86 - 3.73 (m, 2H), 3.24 - 3.13 (m, 2H), 2.37 (t, J = 1.9 Hz, 3H), 1.42 (d, J = 6.9 Hz, 3H), 1.27 (dd, J = 7.2, 2.5 Hz, 6H). ESI-MS m/z calc. 435.1316, found 436.0 (M+1)+.

### Assays for Detecting and Measuring AAT Modulator Properties of Compounds

### A. AAT Function Assay (MSD Assay NL20-SI Cell Line)

Alpha-1 antitrypsin (AAT) is a SERPIN (serine protease inhibitor) that inactivates enzymes by binding to them covalently. This assay measured the amount of functionally active AAT in a sample in the presence of the disclosed Compounds 1-457, Compounds 458-532, Compounds B1-B25, and Compounds W1-W32 by determining the ability of AAT to form an irreversible complex with human neutrophil Elastase (hNE). In practice, the sample (cell supernatant, blood sample, or other) was incubated with excess hNE to allow AAT-Elastase complex to be formed with all functional AAT in the sample. This complex was then captured to a microplate coated with an anti-AAT antibody. The complex captured to the plate was detected with a labeled anti-Elastase antibody and quantitated using a set of AAT standards spanning the concentration range present in the sample. Meso Scale Discovery (MSD) plate reader, Sulfo-tag labeling, and microplates were used to provide high sensitivity and wide dynamic range.

### MATERIALS:

| Reagents/Plates | | **Concentration** |
|---|---|---|
| Goat anti-human Alpha-1-Antitrypsin | | 1 mL @ 1 mg/mL |
| Polyclonal Antibody | | |
| | Use at 5 µg/mL in phosphate buffered saline (PBS) | |
| Human Neutrophil Elastase | | 100 µg lyophilized |
| | Stock at 3.4 µM (0.1 mg + 1 mL PBS) | |
| | Working at 1 µg/mL (34 nm) in MSD Assay buffer (1% bovine serum albumin (BSA)) | |
| Mouse anti-human Neutrophil Elastase Monoclonal Antibody | | 900 µg/mL |
| | Sulfo-tagged @ 12:1 using MSD Gold Sulfo-tag N-hydroxysuccinimide (NHS) ester; use at 0.45 µg/mL in MSD Assay buffer (1% BSA) | |
| M-AAT (Alpha-1-Antitrypsin) | | 5 mg lyophilized |
| MSD Blocker A (BSA) | | 250 mL |
| | 5% solution in PBS for blocking | |
| | 1% solution in PBS for assay buffer | |
| MSD Read Buffer T (4X) with Surfactant | | 1 L or 250 mL |
| MSD 384 high bind plates | | |
| Polypropylene for dilution 384 well plate | | |
| Tissue culture treated black well 384 well plate | | |

### INSTRUMENT(S):

Meso Sector S600
Bravo
Washer dispenser
Multidrop Combi

### ASSAY PROTOCOL

Day 1 Cell Culture
   1. Harvest NL20 human bronchial epithelial cells expressing human Z-AAT in OptiMEM^{™} containing Pen/Strep (P/S).
   2. Seed at 16,000 cells/well in 30 µL (384 well plate).
   3. Centrifuge plates briefly up to speed (1200 rpm) and place into 37°C incubator overnight.
Day 2: Compound Addition and Coating Plates with Capture Antibody
   *Compound Addition:*
      1. Dispense 40 µL of OptiMEM^{™} (P/S) with doxycycline (1:1000 stock = 0.1 µM final) to each well of the compound plate using a multidrop Combi in hood.
      2. Remove cell plate from incubator, flip/blot and take immediately to Bravo to transfer compounds.
      3. Return plates to incubator overnight.
   *Coat MSD Plates*
      1. Dilute capture antibody (Polyclonal Goat anti-AAT) to 5 µg/mL (1:200) in PBS (no BSA).
      2. Dispense 25 µL of diluted capture antibody into all wells of MSD 384-well High Bind plate using the Multidrop equipped with a standard cassette.
      3. Incubate overnight at 4°C.
   Prepare Blocker A (BSA) Solutions
      1. Prepare solution of 5% MSD Blocker A (BSA) following the manufacturer's instructions.
      2. Further dilute the 5% MSD Blocker A in PBS to 1% (Blocker A) as needed.
Day 3: Run MSD Assay
   *Block Plates*
      1. Wash plate 1x with 50 µL Wash buffer (PBS + 0.5% Tween 20), and adds 35 µL 5% Block A buffer to block non-specific binding on washer dispenser.
      2. Rotate plates on shaker for 1 hour at 600 rpm.
   *Prepare M-AAT Standards*
      1. Dilute M-AAT stock to 1.6 µg/mL in 1% BSA Blocker A (Stock in -70 °C); then prepare 12 x 1:2 serial dilutions in 1% Blocker A.
      2. The top starting final concentration on MSD plate is 320 ng/mL. These dilutions correspond to a final concentration of 320, 160, 80, 40, 20, 10, 5, 2.5, 1.25, 0.625, 0.312, 0.156 ng/mL.
   *Dilution plate*
      1. Add 80 µL of 1% Assay buffer to all wells except columns 1/24 (standards) with Multidrop Combi.
      2. Add diluted standards to columns 1 and 24.
      3. Centrifuge dilution plates 1200 rpm briefly.
   *Cell plate*
      1. Aspirate columns which will have the standards from the cell plates in the hood using 16-pin aspirator.
   *Prepare human Neutrophil Elastase (hNE)*
      1. Prepare 1 µg/mL Human Neutrophil Elastase by diluting in 1% Blocker A.
         a. Small 100 µg vial - add 1 mL PBS (100 µg/mL)
            i. This can then be diluted 1:100 in 1% Assay Buffer for a final 1 µg /mL concentration.
   *MSD - add hNE (20 µL*/*well)*
      1. After the MSD plate has blocked for at least 1 hour, wash plate 1x with 50 µL Wash buffer (PBS + 0.5% Tween 20) and then add 20 µL hNE to each well.
   *Bravo - Cell Plate - Dilution Plate - MSD Plate*
Using the Bravo, aspirate 10 µL from the cell plate, transfer to the dilution plate (9-fold dilution)
   1. Mix 25 µL 3x, then aspirate 5 µL, transfer to MSD plate (5-fold dilution).
   2. Mix 10 µL 3x. Total dilution is 45 fold.
   3. Shake plates at 600 rpm for 1.5 hours.

### Add Functional detection hNE antibody

1. Wash plate 1x with wash buffer.
2. Add 25 µL Sulfo-tagged anti-Elastase Monoclonal Mouse anti-Elastase) diluted to 0.45 µg/mL (1:2000) in 1% Blocker A into all wells of the functional activity MSD plates using the washer/dispenser.
   Note: The dilution required for sufficient signal must be determined for each new lot of labeled antibody.
3. Incubate at RT shaking at 600 rpm for 1 hour.

### Final wash and MSD imager read

1. Wash the plate 1x, and add 25 µL of Wash Buffer to the plate.
2. Make 2x Read buffer.
3. Remove wash buffer from MSD plate.
4. Transfer 35 µL 2x Read Buffer to MSD plate using Bravo and take to MSD to read immediately.
Data analysis in MSD Discovery Workbench 4.0 software and EC₅₀ values were determined using Genedata. See Table 33 for data.

### B. Biochemical Assay (Z-AAT Elastase Activity Assay)

This assay measured the modulation of Compounds 1-457, Compounds 458-532, Compounds B1-B25, and Compounds W1-W32 on Z-AAT SERPIN activity using purified Z-AAT protein and purified human neutrophil elastase (hNE). Normally, when active monomeric Z-AAT encounters a protease such as trypsin or elastase, it forms a 1:1 covalent "suicide" complex in which both the AAT and protease are irreversibly inactivated. However, compounds binding to Z-AAT can lead to a decrease in SERPIN activity. In such cases, when a protease encounters compound-bound Z-AAT, the protease cleaves and inactivates Z-AAT without itself being inactivated.

### MATERIALS

*Reagents*
   PBS buffer (media prep) + 0.01% BRIJ35 detergent (Calbiochem catalog #203728) Opti-MEM media (Fisher 11058-021)
   Human neutrophil elastase (hNE, Athens Research #16-14-051200)
      3.4 µM stock (0.1 mg/mL) prepared in 50mM Na Acetate, pH 5.5, 150mM NaCl, stored at -80°C
   Elastase substrate V (ES V, fluorescent peptide substrate MeOSuc-Ala-Ala-Pro-Val-AMC, Calbiochem catalog #324740)
      20 mM stock in DMSO, stored at -20°C
   Purified Z-AAT protein from human plasma;
      12.9 µM (0.67 mg/mL) Z-AAT Vertex Cambridge Sample 4942, from patient #061-SSN, stored at -80C
*Plates*
   Corning 4511 (384 well black low volume)
*Instruments*
   PerkinElmer^{®} EnVision^{™}

### ASSAY PROTOCOL

*Pre-incubation of Z-AAT with Compounds*
   1. 7.5 µL of Z-AAT (20 nM) was incubated with Compounds 1-457, Compounds 458-532, Compounds B1-B25, or Compounds W1-W32 in a GCA plate for 1 hour at room temperature.
*Addition of hNE*
   1. 7.5 µL of HNE solution (3 nM in PBS+0.01% BRIJ35) added into GCA plate
   2. Incubate plate for 30 minutes to allow Z-AAT/HNE suicide complex formation.
*Addition of substrate and read plate on PE Envision*
   1. 7.5 µL of substrate (300 µM solution of elastase substrate (ES V) in
      PBS+0.01% BRIJ35) dispensed per well into GCA plate.
   2. Immediately read on Envision.

### C. EC50 and Z-AAT Elastase Activity Data for Compounds 1 - 457, 458-351, B1-B25, and W1-W32

The compounds of the disclosure are useful as modulators of AAT activity. Table 33 below illustrates the EC₅₀ of the Compounds 1-457, Compounds 458-532, Compounds B1-B25, and Compounds W1-W32 using procedures described in Section A above. Table 33 below also provides the Z-AAT elastase activity using procedures described in Section B above. In Table 33 below, the following meanings apply: For EC₅₀ "+++" means < 0.5 µM; "++" means between 0.5 µM and 2.0 µM; "+" means greater than 2.0 µM. For IC₅₀: "+++" means < 2.0 µM; "++" means between 2.0 µM and 5.0 µM; "+" means greater than 5.0 µM; and "N/A" means activity not assessed. For IC₅₀, "N.D." means activity not detected up to 30 µM.

**Table 33. EC₅₀ and IC₅₀ data for Compounds 1-457, Compounds 458-532, Compounds B1-B25, and Compounds W1-W32**

| **Compound No.** | **NL20 Functional EC₅₀ (µM)** | **Z-AAT Elastase Activity IC₅₀ (µM)** |
|---|---|---|
| 1 | + | + |
| 2 | ++ | + |
| 3 | +++ | + |
| 4 | +++ | + |
| 5 | + | N.D. |
| 6 | +++ | ++ |
| 7 | +++ | ++ |
| 8 | +++ | ++ |
| 9 | +++ | +++ |
| 10 | +++ | +++ |
| 11 | +++ | ++ |
| 12 | +++ | N.D. |
| 13 | +++ | +++ |
| 14 | +++ | +++ |
| 15 | +++ | +++ |
| 16 | +++ | + |
| 17 | ++ | + |
| 18 | ++ | ++ |
| 19 | +++ | ++ |
| 20 | +++ | ++ |
| 21 | +++ | ++ |
| 22 | ++ | N.D. |
| 23 | ++ | ++ |
| 24 | +++ | ++ |
| 25 | +++ | ++ |
| 26 | +++ | +++ |
| 27 | +++ | +++ |
| 28 | +++ | ++ |
| 29 | +++ | +++ |
| 30 | +++ | +++ |
| 31 | +++ | +++ |
| 32 | +++ | +++ |
| 33 | +++ | ++ |
| 34 | +++ | +++ |
| 35 | ++ | + |
| 36 | ++ | + |
| 37 | ++ | + |
| 38 | ++ | N.D. |
| 39 | + | N.D. |
| 40 | + | N.D. |
| 41 | + | N.D. |
| 42 | +++ | ++ |
| 43 | ++ | ++ |
| 44 | +++ | ++ |
| 45 | + | N.D. |
| 46 | +++ | + |
| 47 | +++ | N.D. |
| 48 | ++ | N.D. |
| 49 | + | N.D. |
| 50 | + | N.D. |
| 51 | ++ | +++ |
| 52 | + | ++ |
| 53 | ++ | ++ |
| 54 | ++ | + |
| 55 | + | + |
| 56 | ++ | + |
| 57 | + | N.D. |
| 58 | ++ | + |
| 59 | ++ | + |
| 60 | + | ++ |
| 61 | ++ | + |
| 62 | + | + |
| 63 | + | N.D. |
| 64 | + | + |
| 65 | + | N.D. |
| 66 | + | N.D. |
| 67 | ++ | ++ |
| 68 | + | N.D. |
| 69 | + | N.D. |
| 70 | + | N.D. |
| 71 | + | N.D. |
| 72 | + | N.D. |
| 73 | + | N.D. |
| 74 | ++ | + |
| 75 | ++ | + |
| 76 | + | + |
| 77 | ++ | N.D. |
| 78 | + | N.D. |
| 79 | + | N.D. |
| 80 | + | N.D. |
| 81 | + | N.D. |
| 82 | + | N.D. |
| 83 | + | + |
| 84 | + | N.D. |
| 85 | ++ | + |
| 86 | + | N.D. |
| 87 | + | N.D. |
| 88 | + | N.D. |
| 89 | + | N.D. |
| 90 | ++ | + |
| 91 | + | + |
| 92 | + | + |
| 93 | + | N.D. |
| 94 | + | N.D. |
| 95 | + | + |
| 96 | ++ | + |
| 97 | ++ | + |
| 98 | +++ | + |
| 99 | +++ | +++ |
| 100 | +++ | +++ |
| 101 | +++ | ++ |
| 102 | +++ | + |
| 103 | ++ | N.D. |
| 104 | + | N.D. |
| 105 | + | N.D. |
| 106 | ++ | + |
| 107 | +++ | ++ |
| 108 | +++ | ++ |
| 109 | +++ | ++ |
| 110 | +++ | + |
| 111 | ++ | N.D. |
| 112 | + | N.D. |
| 113 | +++ | N.D. |
| 114 | ++ | +++ |
| 115 | ++ | +++ |
| 116 | +++ | ++ |
| 117 | + | N.D. |
| 118 | ++ | ++ |
| 119 | ++ | + |
| 120 | + | N.D. |
| 121 | +++ | + |
| 122 | + | N.D. |
| 123 | +++ | +++ |
| 124 | +++ | + |
| 125 | +++ | ++ |
| 126 | +++ | ++ |
| 127 | + | + |
| 128 | + | + |
| 129 | ++ | N.D. |
| 130 | + | N.D. |
| 131 | + | N.D. |
| 132 | +++ | +++ |
| 133 | ++ | + |
| 134 | + | + |
| 135 | + | + |
| 136 | +++ | +++ |
| 137 | +++ | +++ |
| 138 | ++ | + |
| 139 | +++ | ++ |
| 140 | ++ | N.D. |
| 141 | +++ | + |
| 142 | + | N.D. |
| 143 | + | N.D. |
| 144 | ++ | + |
| 145 | ++ | N.D. |
| 146 | +++ | +++ |
| 147 | +++ | +++ |
| 148 | +++ | +++ |
| 149 | +++ | +++ |
| 150 | ++ | + |
| 151 | +++ | +++ |
| 152 | +++ | +++ |
| 153 | + | N.D. |
| 154 | + | N/A |
| 155 | ++ | + |
| 156 | + | N.D. |
| 157 | ++ | + |
| 158 | ++ | + |
| 159 | +++ | ++ |
| 160 | ++ | N.D. |
| 161 | +++ | +++ |
| 162 | +++ | +++ |
| 163 | +++ | +++ |
| 164 | +++ | +++ |
| 165 | +++ | +++ |
| 166 | +++ | +++ |
| 167 | +++ | +++ |
| 168 | ++ | + |
| 169 | +++ | + |
| 170 | +++ | +++ |
| 171 | +++ | + |
| 172 | ++ | + |
| 173 | + | N.D. |
| 174 | + | N.D. |
| 175 | +++ | ++ |
| 176 | ++ | + |
| 177 | + | N.D. |
| 178 | + | N.D. |
| 179 | +++ | +++ |
| 180 | +++ | +++ |
| 181 | + | N.D. |
| 182 | ++ | + |
| 183 | + | + |
| 184 | ++ | +++ |
| 185 | ++ | +++ |
| 186 | +++ | ++ |
| 187 | + | N.D. |
| 188 | + | N.D. |
| 189 | + | + |
| 190 | ++ | + |
| 191 | +++ | ++ |
| 192 | + | N.D. |
| 193 | ++ | ++ |
| 194 | ++ | N.D. |
| 195 | N/A | N.D. |
| 196 | ++ | + |
| 197 | + | N.D. |
| 198 | + | N.D. |
| 199 | + | N.D. |
| 200 | + | N.D. |
| 201 | + | + |
| 202 | + | + |
| 203 | + | + |
| 204 | + | N.D. |
| 205 | + | N.D. |
| 206 | + | N.D. |
| 207 | ++ | + |
| 208 | +++ | ++ |
| 209 | ++ | + |
| 210 | +++ | + |
| 211 | ++ | + |
| 212 | +++ | +++ |
| 213 | + | N/A |
| 214 | +++ | +++ |
| 215 | +++ | +++ |
| 216 | ++ | + |
| 217 | ++ | +++ |
| 218 | ++ | + |
| 219 | ++ | + |
| 220 | ++ | ++ |
| 221 | +++ | +++ |
| 222 | +++ | +++ |
| 223 | +++ | +++ |
| 224 | + | N.D. |
| 225 | +++ | + |
| 226 | ++ | + |
| 227 | ++ | ++ |
| 228 | +++ | ++ |
| 229 | +++ | +++ |
| 230 | +++ | +++ |
| 231 | +++ | +++ |
| 232 | ++ | + |
| 233 | +++ | +++ |
| 234 | ++ | N.D. |
| 235 | +++ | + |
| 236 | +++ | +++ |
| 237 | +++ | ++ |
| 238 | ++ | + |
| 239 | ++ | + |
| 240 | + | N.D. |
| 241 | ++ | + |
| 242 | +++ | N.D. |
| 243 | +++ | N.D. |
| 244 | ++ | N.D. |
| 245 | +++ | N.D. |
| 246 | ++ | N.D. |
| 247 | ++ | + |
| 248 | +++ | + |
| 249 | ++ | N.D. |
| 250 | ++ | N.D. |
| 251 | +++ | N.D. |
| 252 | ++ | N.D. |
| 253 | ++ | + |
| 254 | ++ | +++ |
| 255 | +++ | +++ |
| 256 | ++ | +++ |
| 257 | ++ | + |
| 258 | + | N.D. |
| 259 | +++ | ++ |
| 260 | +++ | +++ |
| 261 | ++ | ++ |
| 262 | +++ | +++ |
| 263 | ++ | + |
| 264 | +++ | +++ |
| 265 | +++ | +++ |
| 266 | +++ | +++ |
| 267 | +++ | + |
| 268 | ++ | + |
| 269 | +++ | ++ |
| 270 | +++ | ++ |
| 271 | +++ | ++ |
| 272 | +++ | +++ |
| 273 | ++ | ++ |
| 274 | +++ | +++ |
| 275 | +++ | +++ |
| 276 | +++ | +++ |
| 277 | ++ | ++ |
| 278 | ++ | +++ |
| 279 | +++ | +++ |
| 280 | +++ | +++ |
| 281 | +++ | + |
| 282 | +++ | +++ |
| 283 | +++ | ++ |
| 284 | +++ | +++ |
| 285 | ++ | +++ |
| 286 | +++ | +++ |
| 287 | +++ | +++ |
| 288 | ++ | ++ |
| 289 | ++ | +++ |
| 290 | +++ | +++ |
| 291 | ++ | +++ |
| 292 | ++ | +++ |
| 293 | ++ | + |
| 294 | ++ | + |
| 295 | +++ | +++ |
| 296 | +++ | +++ |
| 297 | +++ | +++ |
| 298 | ++ | + |
| 299 | +++ | +++ |
| 300 | ++ | +++ |
| 301 | +++ | +++ |
| 302 | +++ | +++ |
| 303 | ++ | +++ |
| 304 | + | N.D. |
| 305 | ++ | + |
| 306 | ++ | + |
| 307 | +++ | N.D. |
| 308 | +++ | ++ |
| 309 | +++ | ++ |
| 310 | ++ | + |
| 311 | +++ | ++ |
| 312 | ++ | + |
| 313 | ++ | + |
| 314 | ++ | + |
| 315 | ++ | + |
| 316 | ++ | ++ |
| 317 | +++ | +++ |
| 318 | +++ | ++ |
| 319 | ++ | +++ |
| 320 | +++ | +++ |
| 321 | +++ | N.D. |
| 322 | +++ | ++ |
| 323 | ++ | +++ |
| 324 | +++ | +++ |
| 325 | +++ | +++ |
| 326 | ++ | ++ |
| 327 | +++ | +++ |
| 328 | +++ | ++ |
| 329 | ++ | ++ |
| 330 | +++ | +++ |
| 331 | +++ | +++ |
| 332 | +++ | +++ |
| 333 | +++ | ++ |
| 334 | + | N/A |
| 335 | +++ | +++ |
| 336 | +++ | +++ |
| 337 | +++ | + |
| 338 | +++ | ++ |
| 339 | +++ | +++ |
| 340 | +++ | +++ |
| 341 | ++ | + |
| 342 | + | + |
| 343 | ++ | ++ |
| 344 | +++ | + |
| 345 | +++ | ++ |
| 346 | +++ | + |
| 347 | +++ | + |
| 348 | +++ | +++ |
| 349 | +++ | +++ |
| 350 | +++ | ++ |
| 351 | +++ | +++ |
| 352 | ++ | + |
| 353 | +++ | ++ |
| 354 | +++ | +++ |
| 355 | +++ | ++ |
| 356 | + | + |
| 357 | +++ | +++ |
| 358 | +++ | +++ |
| 359 | +++ | +++ |
| 360 | +++ | +++ |
| 361 | ++ | +++ |
| 362 | +++ | +++ |
| 363 | +++ | +++ |
| 364 | +++ | +++ |
| 365 | +++ | +++ |
| 366 | +++ | +++ |
| 367 | +++ | +++ |
| 368 | +++ | +++ |
| 369 | ++ | +++ |
| 370 | + | +++ |
| 371 | +++ | +++ |
| 372 | +++ | +++ |
| 373 | +++ | +++ |
| 374 | +++ | +++ |
| 375 | + | N.D. |
| 376 | +++ | +++ |
| 377 | +++ | ++ |
| 378 | ++ | + |
| 379 | + | N.D. |
| 380 | ++ | + |
| 381 | + | N.D. |
| 382 | +++ | +++ |
| 383 | +++ | + |
| 384 | + | N.D. |
| 385 | + | N.D. |
| 386 | ++ | + |
| 387 | ++ | ++ |
| 388 | + | N.D. |
| 389 | + | + |
| 390 | + | + |
| 391 | + | N.D. |
| 392 | ++ | N.D. |
| 393 | ++ | N.D. |
| 394 | + | N.D. |
| 395 | + | N.D. |
| 396 | + | N.D. |
| 397 | + | N.D. |
| 398 | + | N.D. |
| 399 | ++ | N.D. |
| 400 | ++ | ++ |
| 401 | +++ | +++ |
| 402 | + | +++ |
| 403 | ++ | +++ |
| 404 | ++ | ++ |
| 405 | +++ | ++ |
| 406 | +++ | + |
| 407 | ++ | ++ |
| 408 | +++ | + |
| 409 | ++ | N.D. |
| 410 | + | + |
| 411 | ++ | N.D. |
| 412 | ++ | + |
| 413 | +++ | ++ |
| 414 | +++ | ++ |
| 415 | +++ | + |
| 416 | +++ | + |
| 417 | + | N.D. |
| 418 | + | N.D. |
| 419 | + | N.D. |
| 420 | + | N.D. |
| 421 | + | N.D. |
| 422 | + | N.D. |
| 423 | + | N.D. |
| 424 | + | N.D. |
| 425 | + | + |
| 426 | + | N.D. |
| 427 | ++ | ++ |
| 428 | + | N/A |
| 429 | +++ | ++ |
| 430 | +++ | ++ |
| 431 | +++ | +++ |
| 432 | +++ | +++ |
| 433 | +++ | + |
| 434 | ++ | N.D. |
| 435 | +++ | + |
| 436 | N/A | +++ |
| 437 | ++ | +++ |
| 438 | N/A | N/A |
| 439 | N/A | N/A |
| 440 | N/A | ++ |
| 441 | N/A | + |
| 442 | ++ | N.D. |
| 443 | + | N.D. |
| 444 | + | N.D. |
| 445 | +++ | +++ |
| 446 | N/A | ++ |
| 447 | N/A | + |
| 448 | N/A | + |
| 449 | N/A | + |
| 450 | N/A | N/A |
| 451 | N/A | N/A |
| 452 | N/A | N/A |
| 453 | +++ | N.D. |
| 454 | +++ | +++ |
| 455 | +++ | ++ |
| 456 | N/A | N/A |
| 457 | N/A | N/A |
| 458 | +++ | +++ |
| 459 | +++ | N.D. |
| 460 | ++ | N.D. |
| 461 | +++ | +++ |
| 462 | + | ++ |
| 463 | +++ | + |
| 464 | ++ | ++ |
| 465 | + | + |
| 466 | ++ | N.D. |
| 467 | + | N.D. |
| 468 | +++ | ++ |
| 469 | + | + |
| 470 | + | N.D. |
| 471 | +++ | N.D. |
| 472 | ++ | + |
| 473 | ++ | + |
| 474 | ++ | + |
| 475 | +++ | ++ |
| 476 | ++ | + |
| 477 | +++ | ++ |
| 478 | +++ | ++ |
| 479 | ++ | + |
| 480 | +++ | + |
| 481 | +++ | +++ |
| 482 | ++ | + |
| 483 | ++ | N.D. |
| 484 | +++ | ++ |
| 485 | ++ | ++ |
| 486 | +++ | +++ |
| 487 | +++ | + |
| 488 | +++ | N.D. |
| 489 | +++ | + |
| 490 | +++ | N.D. |
| 491 | + | N.D. |
| 492 | + | + |
| 493 | +++ | N.D. |
| 494 | ++ | N.D. |
| 495 | +++ | + |
| 496 | ++ | + |
| 497 | +++ | + |
| 498 | + | N.D. |
| 499 | ++ | N.D. |
| 500 | +++ | + |
| 501 | +++ | + |
| 502 | +++ | N.D. |
| 503 | +++ | + |
| 504 | +++ | +++ |
| 505 | +++ | +++ |
| 506 | +++ | +++ |
| 507 | +++ | ++ |
| 508 | +++ | + |
| 509 | +++ | ++ |
| 510 | ++ | ++ |
| 511 | +++ | N.D. |
| 512 | ++ | + |
| 513 | +++ | +++ |
| 514 | ++ | + |
| 515 | ++ | N.D. |
| 516 | +++ | N.D. |
| 517 | ++ | + |
| 518 | + | + |
| 519 | + | + |
| 520 | + | + |
| 521 | + | N.D. |
| 522 | + | N.D. |
| 523 | ++ | + |
| 524 | +++ | + |
| 525 | ++ | N.D. |
| 526 | +++ | + |
| 527 | ++ | + |
| 528 | ++ | + |
| 529 | ++ | N.D. |
| 530 | + | + |
| 531 | +++ | + |
| 532 | +++ | + |
| B1 | ++ | + |
| B2 | + | N.D. |
| B3 | + | N.D. |
| B4 | ++ | N.D. |
| B5 | ++ | + |
| B6 | + | + |
| B7 | + | N.D. |
| B8 | + | N.D. |
| B9 | + | N.D. |
| B10 | ++ | N.D. |
| B11 | + | N.D. |
| B12 | ++ | N.D. |
| B13 | + | N.D. |
| B14 | ++ | N.D. |
| B15 | ++ | N.D. |
| B16 | +++ | + |
| B17 | +++ | ++ |
| B18 | +++ | ++ |
| B19 | ++ | + |
| B20 | +++ | + |
| B21 | +++ | + |
| B22 | ++ | N.D. |
| B23 | + | N.D. |
| B24 | + | + |
| B25 | + | N.D. |
| W1 | + | N.D. |
| W2 | ++ | N.D. |
| W3 | + | N.D. |
| W4 | + | N.D. |
| W5 | + | N.D. |
| W6 | ++ | N.D. |
| W7 | ++ | N.D. |
| W8 | + | N.D. |
| W9 | ++ | N.D. |
| W10 | + | N.D. |
| W11 | + | N.D. |
| W12 | + | + |
| W13 | + | + |
| W14 | + | N.D. |
| W15 | + | N.D. |
| W16 | ++ | N.D. |
| W17 | + | + |
| W18 | + | N.D. |
| W19 | + | + |
| W20 | + | N.D. |
| W21 | + | + |
| W22 | + | N.D. |
| W23 | + | N.D. |
| W24 | + | + |
| W25 | + | N.D. |
| W26 | ++ | + |
| W27 | + | N.D. |
| W28 | + | + |
| W29 | + | N.D. |
| W30 | ++ | N.D. |
| W31 | ++ | + |
| W32 | + | N.D. |

### Other Embodiments

This description provides merely exemplary embodiments of the disclosure. One skilled in the art will readily recognize from the disclosure and accompanying claims, that various changes, modifications and variations can be made therein without departing from the scope of the disclosure as defined in the following claims.

## Claims

1. A compound represented by the following structural formula: 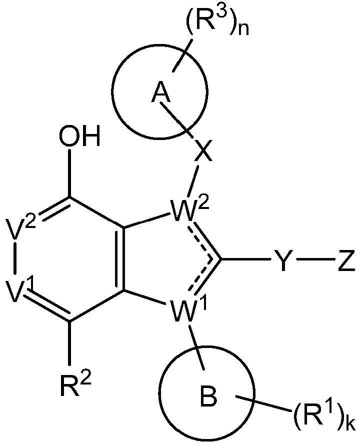 a tautomer thereof, a deuterated derivative of that compound or tautomer, or a pharmaceutically acceptable salt of the foregoing, wherein:
------, for each of the two occurrences, is a single bond or a double bond, provided that one is a single bond and the other is a double bond;
**V¹** and **V²** are each independently N or -C**R²**;
**W¹** and **W²** are each independently N or C, provided that one of W¹ and W² is N and the other is C;
**X** is absent or a bond, -(C**R^{a}R^{b}**)**ₚ-**, or -SO₂-;
**Y** is absent or a bond, -(C**R^{c}R^{d}**)**_{q}**-, -C(=O)-, or -SO₂-;
**R^{a}** and **R^{b}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**R^{c}** and **R^{d}**, for each occurrence, are each independently hydrogen, halogen, -OH, C₁-C₆ alkyl, C₂-C₆ alkenyl, C₁-C₆ haloalkyl, C₁-C₆ alkoxy, or C₁-C₆ haloalkoxy;
**Ring A** is C₃-C₁₂ carbocyclyl, 3 to 12-membered heterocyclyl, C₆ or C₁₀ aryl, or 5 to 10-membered heteroaryl; provided that when **W¹** is N and **W²** is C, **Ring A** is not 1,5,6,7-tetrahydro-4*H*-indol-4-onyl or a tautomer thereof;
**Ring B** is C₄-C₁₂ cycloalkyl, C₆ or C₁₀ aryl, 5 to 10-membered heteroaryl, or benzyl;
Z is 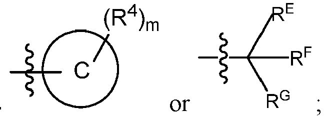 wherein:
**Ring C** is C₃-C₁₂ cycloalkyl, 3 to 12-membered heterocyclyl, C₆ or C₁₀ aryl, or 5 to 10-membered heteroaryl;
provided that when **Ring C** is phenyl, the phenyl is substituted with **R⁴**; provided that when **Ring C** is phenyl, **Y** cannot be -SO₂-; and
provided that when **Ring B** is benzyl, **Ring C** cannot be pyridinyl or indolyl;
**R^{E}**, **R^{F}**, and **R^{G}** are each independently hydrogen, halogen, cyano, C₁-C₆ alkyl, C₂-C₆ alkenyl, C₁-C₆ alkoxy, C₁-C₆ haloalkyl, C₁-C₆ haloalkoxy, -C(=O)**R^{s}**, -C(=O)O**R^{s}**, -C(=O)N**R^{p}R^{q}**, -C**R^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}**, or -OC(=O)N**R^{p}R^{q}**; wherein:
the C₁-C₆ alkyl or the C₂-C₆ alkenyl of any one of **R^{E}**, **R^{F}**, and **R^{G}** is optionally substituted with 1 to 3 groups selected from cyano,
-C(=O)**R^{s}**, -C(=O)O**R^{s}**, -C(=O)N**R^{p}R^{q}**, -N**R^{p}**C(=O)**R^{s}**, -N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, -OC(=O)O**R^{s}**, -OC(=O)N**R^{p}R^{q}**, -S(=O)**ᵣR^{s},** and -S(=O)**ᵣ**N**R^{p}R^{q}**; wherein:
**R^{p}**, **R^{q}**, and **R^{r}**, for each occurrence, are each independently hydrogen, C₁-C₄ alkyl, C₃-C₆ cycloalkyl, or 3 to 6-membered heterocyclyl; wherein:
the C₁-C₄ alkyl of any one of **R^{p}**, **R^{q}**, and **R^{r}** is optionally substituted with 1 to 3 groups selected from halogen, cyano, -OH, C₁-C₃ alkoxy, -C(=O)NH(C₁-C₂ alkyl), and -C(=O)N(C₁-C₂ alkyl)₂; and
the C₃-C₆ cycloalkyl or the 3 to 6-membered heterocyclyl of any one of **R^{p}**, **R^{q}**, and **R^{r}** is optionally substituted with 1 to 3 groups selected from halogen, cyano, -OH, C₁-C₃ alkyl, C₁-C₃ alkoxy, C₁-C₃ haloalkyl, C₁-C₃ haloalkoxy, -C(=O)O(C₁-C₂ alkyl), -C(=O)NH(C₁-C₂ alkyl), and -C(=O)N(C₁-C₂ alkyl)₂;
**R^{s}**, for each occurrence, is independently hydrogen, C₁-C₄ alkyl, C₃-C₆ cycloalkyl, phenyl, or 5 or 6-membered heteroaryl; wherein:
the C₁-C₄ alkyl of **R^{s}** is optionally substituted with 1 to 3 groups selected from halogen, cyano, -OH, -NH₂, -NH(C₁-C₂ alkyl), - N(C₁-C₂ alkyl)₂, C₁-C₃ alkoxy, -C(=O)OH, -C(=O)O(C₁-C₂ alkyl), - C(=O)NH₂, -C(=O)NH(C₁-C₂ alkyl), and -C(=O)N(C₁-C₂ alkyl)₂; and
the C₃-C₆ cycloalkyl, the phenyl, or the 5 or 6-membered heteroaryl of **R^{s}** is optionally substituted with 1 to 3 groups selected from halogen, cyano, -OH, -OCH₃, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
**R¹** is halogen, cyano, C₁-C₃ alkyl, C₁-C₃ haloalkyl, C₁-C₃ alkoxy, C₁-C₃ haloalkoxy, or -O-(C₃-C₆ cycloalkyl);
**R²**, for each occurrence, is independently hydrogen, halogen, cyano, C₁-C₆ alkyl, C₂-C₆ alkenyl, C₁-C₆ alkoxy, C₁-C₆ haloalkyl, C₁-C₆ haloalkoxy, C₃-C₆ cycloalkyl, -N**R^{h}Rⁱ**, phenyl, or 5 or 6-membered heteroaryl; wherein:
the C₁-C₆ alkyl, the C₂-C₆ alkenyl or the C₃-C₆ cycloalkyl of **R²** is optionally substituted with 1 to 3 groups selected from cyano, -C(=O)**R^{k}**, -C(=O)O**R^{k}**, - C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, - N**R^{h}**S(=O)ₛ**R^{k}**, -O**R^{k}**, -OC(=O)**R^{k}**, -OC(=O)O**R^{k}**,-OC(=O)N**R^{h}Rⁱ**, -S(=O)ₛ**R^{k}**, and S(=O)ₛN**R^{h}Rⁱ**; wherein:
**R^{h}**, **Rⁱ**, and **R^{j}**, for each occurrence, are each independently hydrogen, C₁-C₄ alkyl, or C₃-C₆ cycloalkyl; wherein:
the C₁-C₄ alkyl of any one of **R^{h}**, **Rⁱ**, and **R^{j}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
the C₃-C₆ cycloalkyl of any one of **R^{h}**, **Rⁱ**, and **R^{j}** is optionally substituted with 1 to 3 groups selected from halogen, cyano, -OH, - NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
**R^{k},** for each occurrence, is independently hydrogen, C₁-C₄ alkyl, C₃-C₆ cycloalkyl, phenyl, or 5 or 6-membered heteroaryl; wherein:
-O**R^{k}** cannot be -OH;
the C₁-C₄ alkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
the C₃-C₆ cycloalkyl of **R^{k}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁- C₂ alkyl)₂;
**R³** and **R⁴**, for each occurrence, are each independently halogen, cyano, =O, C₁-C₆ alkyl, C₂-C₆ alkenyl, C₁-C₆ alkoxy, C₁-C₆ haloalkyl, C₁-C₆ haloalkoxy, C₃-C₆ cycloalkyl, - C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)**ₜR^{y}**, -N**R^{v}R^{w}**, - N**R^{v}**C(=O)**R^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, - OC(=O)O**R^{y}**, -OC(=O)N**R^{v}R^{w}**, -S(=O)ₜ**R^{y}**, -S(=O)ₜN**R^{v}R^{w},** -S(=O)ₜN**R^{v}**C(=O)**R^{y}**, - P(=O)**R^{z}R^{z},** phenyl, or a 5 or 6-membered heteroaryl; wherein:
the C₁-C₆ alkyl, the C₂-C₆ alkenyl, or the C₃-C₆ cycloalkyl of any one of **R³** and **R⁴** is optionally substituted with 1 to 3 groups selected from cyano, -C(=O)**R^{y}**, -C(=O)O**R^{y}**, -C(=O)N**R^{v}R^{w}**, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -NR^{v}S(=O)ᵣ**R^{y}**, -O**R^{y}**, -OC(=O)**R^{y}**, - OC(=O)O**R^{y}**, -OC(=O)N**R^{v}R^{w}**, -S(=O)**ₜR^{y}**, and -S(=O)**ₜ**N**R^{v}R^{w}**; wherein:
**R^{v}**, **R^{w}**, and **R^{x}**, for each occurrence, are each independently hydrogen, C₁-C₄ alkyl, C₃-C₆ cycloalkyl, 5 or 6-membered heterocyclyl, or 5 or 6-membered heteroaryl; wherein:
the C₁-C₄ alkyl of any one of **R^{v}**, **R^{w}**, and **R^{x}** is optionally substituted with 1 to 3 groups selected from halogen, cyano -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂; and
the C₃-C₆ cycloalkyl, the 5 or 6-membered heterocyclyl, or the 5 or 6-membered heteroaryl of any one of **R^{v}**, **R^{w}**, and **R^{x}** is optionally substituted with 1 to 3 groups selected from halogen, cyano, -OH, -NH₂, -NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, - C(=O)O(C₁-C₂ alkyl), -C(=O)NH₂, -C(=O)NH(C₁-C₂ alkyl), and - C(=O)N(C₁-C₂ alkyl)₂;
**R^{y}**, for each occurrence, is independently hydrogen, C₁-C₄ alkyl, C₃-C₆ cycloalkyl, phenyl, a 5 or 6-membered heterocyclyl, or a 5 or 6-membered heteroaryl; wherein
the C₁-C₄ alkyl of **R^{y}** is optionally substituted with 1 to 3 groups selected from halogen, cyano, -OH, -NH₂, -NH(C₁-C₂ alkyl), - N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, - C(=O)NH(C₁-C₂ alkyl), and -C(=O)N(C₁-C₂ alkyl)₂; and
the C₃-C₆ cycloalkyl, the phenyl, the 5 or 6-membered heterocyclyl, or the 5 or 6-membered heteroaryl of **R^{y}** is optionally substituted with 1 to 3 groups selected from halogen, cyano, -OH, -NH₂, NH(C₁-C₂ alkyl), -N(C₁-C₂ alkyl)₂, C₁-C₃ alkyl, C₁-C₃ alkoxy, C₁-C₃ haloalkyl, C₁-C₃ haloalkoxy, -C(=O)OH, -C(=O)O(C₁-C₂ alkyl), -C(=O)NH₂, -C(=O)NH(C₁-C₂ alkyl), and -C(=O)N(C₁-C₂ alkyl)₂;
**R^{z}**, for each occurrence, is independently C₁-C₂ alkyl, -OH, or
-O(C₁-C₂ alkyl);
**k** is an integer selected from 1, 2, and 3;
**m** and **n** a are each independently an integer selected from 0, 1, 2, and 3;
**p, r, s,** and **t** are each independently an integer selected from 1 and 2; and
**q** is an integer selected from 1, 2, and 3.

2. The compound, tautomer, deuterated derivative, or pharmaceutically acceptable salt according to claim 1, represented by one of the following structural formulae: 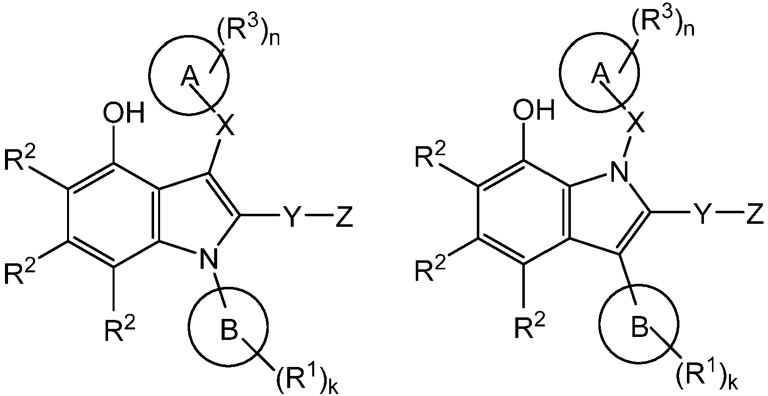 wherein all other variables not specifically defined herein are as defined in claim 1.

3. The compound, tautomer, deuterated derivative, or pharmaceutically acceptable salt according to claim 1, represented by one of the following structural formulae: 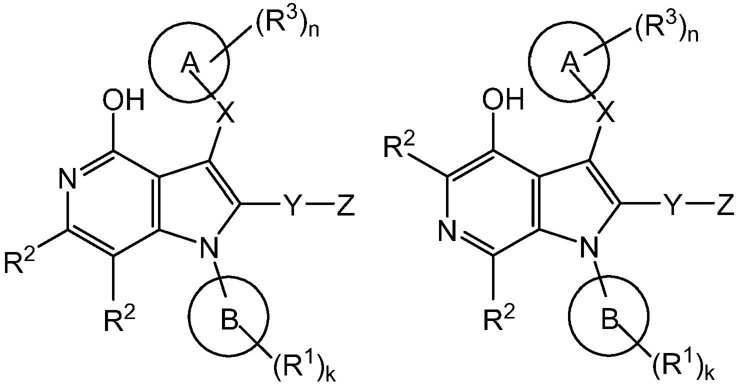 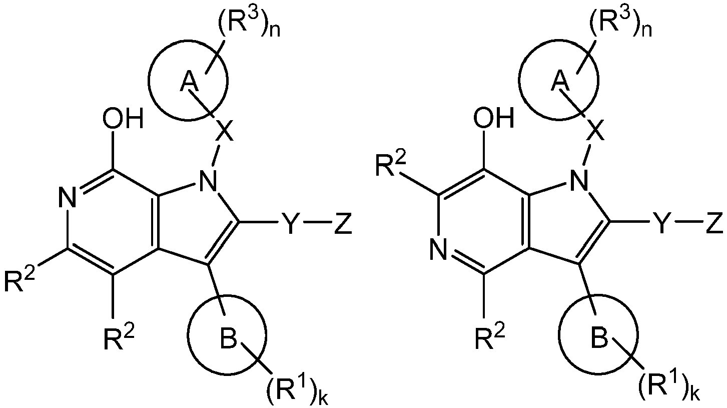 wherein all other variables not specifically defined herein are as defined in claim 1.

4. The compound, tautomer, deuterated derivative, or pharmaceutically acceptable salt according to claim 1 or claim 2, represented by one of the following structural formulae: 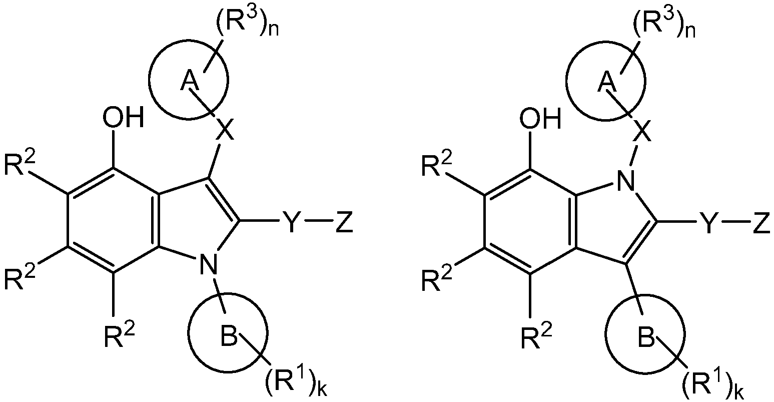 wherein:
**Ring A** is optionally substituted with **R³** and **Ring A** is C₃-C₇ carbocyclyl, 6 to 9-membered heterocyclyl, phenyl, or 5 to 9-membered heteroaryl;
**Ring B** is substituted with **R¹** and **Ring B** is C₄-C₆ cycloalkyl, phenyl, 5 to 6-membered heteroaryl, or benzyl; and
when **Z** is **Ring C** optionally substituted with **R⁴, Ring C** is C₄-C₈ cycloalkyl, 4 to 8-membered heterocyclyl, phenyl, or 5 or 6-membered heteroaryl;
and wherein all other variables not specifically defined herein are as defined in claim 1 or claim 2.

5. The compound, tautomer, deuterated derivative, or pharmaceutically acceptable salt according to any one of claims 1, 2, and 4, represented by one of the following structural formulae: 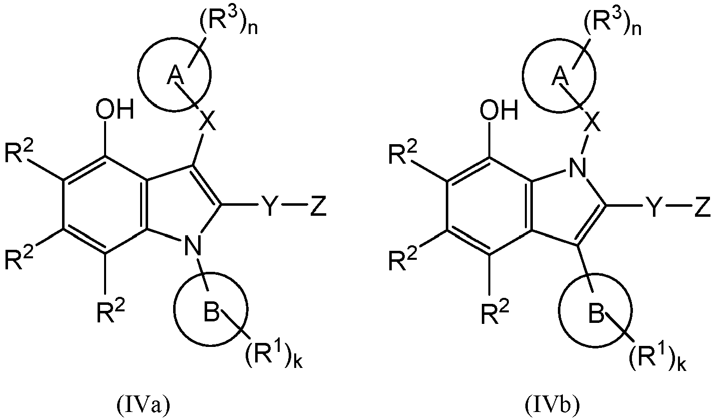 wherein:
**Ring B** is substituted with **R¹** and **Ring B** is cyclohexyl, phenyl, pyridinyl, or benzyl; and wherein all other variables not specifically defined herein are as defined in any one of claims 1, 2, and 4,
optionally wherein the compound, tautomer, deuterated derivative, or pharmaceutically acceptable salt is represented by one of the following structural formulae: 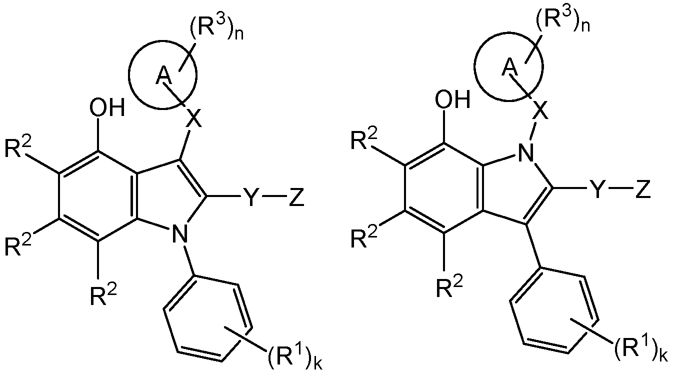 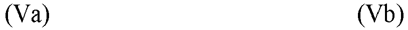 wherein:
**R¹** is halogen, cyano, C₁-C₂ alkyl, C₁-C₂ haloalkyl, or C₁-C₂ alkoxy; and
**k** is an integer selected from 1 and 2.

6. The compound, tautomer, deuterated derivative, or pharmaceutically acceptable salt according to any one of claims 1 to 5, wherein:
(i) **R¹ is** cyano, F, Cl, -CH₃, -CHF₂, -CF₃, -OCH₃, or -OCH(CH₃)₂;
(ii) at least one **R¹** is F;
(iii) **X is** absent or a bond, -(C**R^{a}R^{b}**)-, or -SO₂-;
**R^{a}** and **R^{b},** for each occurrence, are each independently hydrogen, C₁-C₃ alkyl, C₁-C₃ haloalkyl, or C₁-C₃ alkoxy;
(iv) **X** is absent or a bond, -CH₂-, or -SO₂-;
(v) **Y** is absent or a bond, -(C**R^{c}R^{d}**)_{q}-, -C(=O)-, or -SO₂-;
**R^{c}** and **R^{d},** for each occurrence, are each independently hydrogen, C₁-C₃ alkyl, C₁-C₃ haloalkyl, or C₁-C₃ alkoxy;
(vi) **q** is an integer selected from 1 and 2; and/or
(vii) **Y** is absent or a bond, -CH₂-, -CHCH₃-, -C(CH₃)₂-, -C(=O)-, or -SO₂-;
and wherein all other variables not specifically defined herein are as defined in any one of the preceding claims.

7. The compound, tautomer, deuterated derivative, or pharmaceutically acceptable salt according to any one of claims 1 to 6, wherein **Ring A** is optionally substituted with **R³** and **Ring A** is:
(i) C₃-C₇ carbocyclyl, 6 to 9-membered heterocyclyl containing 1 to 3 oxygen atoms, phenyl, or 5 to 9-membered heteroaryl containing 1 to 3 heteroatoms selected from O and N;
(ii) C₃-C₇ carbocyclyl, 6 to 9-membered heterocyclyl containing one or two oxygen atoms, phenyl, or 5 to 9-membered heteroaryl containing one or two nitrogen atoms or one or two oxygen atoms;
(iii) selected from 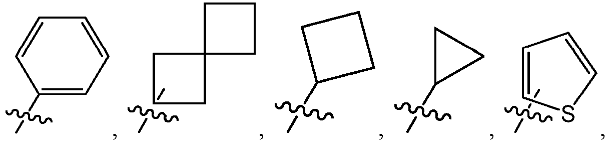 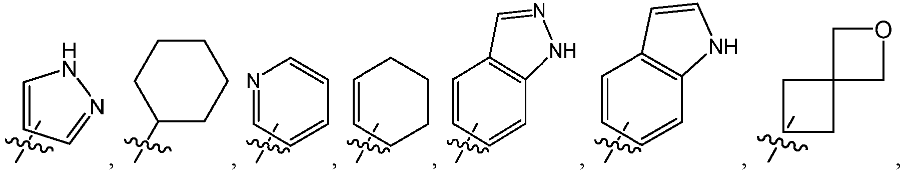 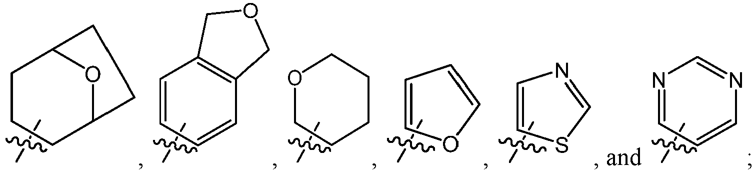 and/or
(iv) selected from 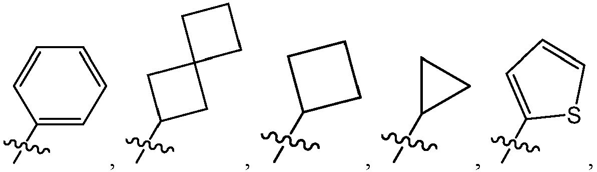 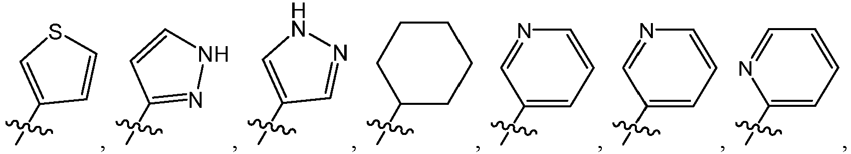 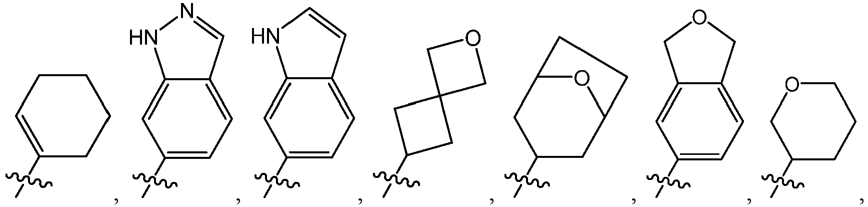 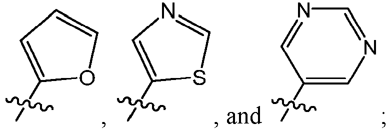
and wherein all other variables not specifically defined herein are as defined in any one of the preceding claims.

8. The compound, tautomer, deuterated derivative, or pharmaceutically acceptable salt according to any one of claims 1 to 7, wherein **Z** is **Ring C** optionally substituted with **R⁴,** and **Ring C** is**:**
(i) C₄-C₈ cycloalkyl, 4 to 8-membered heterocyclyl containing one or two heteroatoms selected from O, N, and S, phenyl, or 5-membered heteroaryl containing one or two heteroatoms selected from O and N;
(ii) selected from 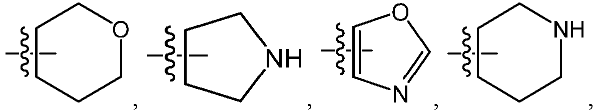 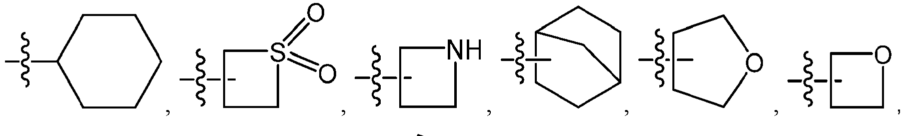 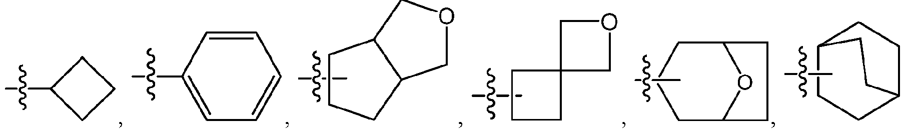 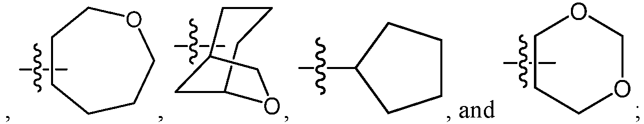 and/or
(iii) selected from 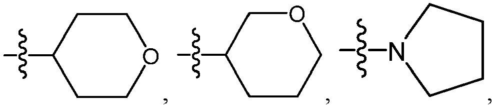 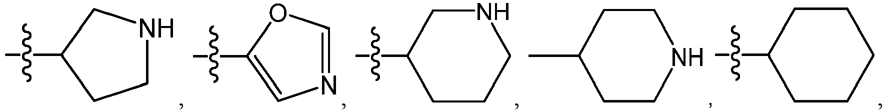 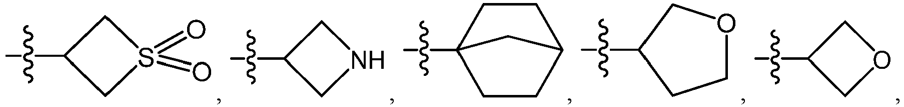 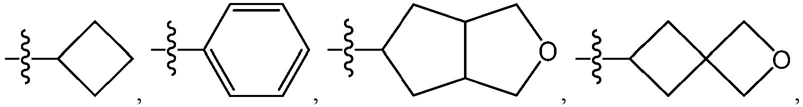 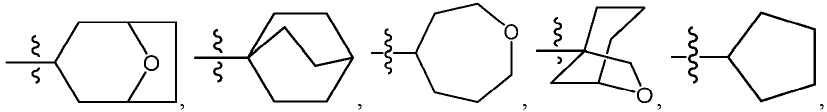 and 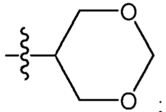
and wherein all other variables not specifically defined herein are as defined in any one of the preceding claims.

9. The compound, tautomer, deuterated derivative, or pharmaceutically acceptable salt according to any one of claims 1 to 8, wherein **R^{E}, R^{F},** and **R^{G}** are each independently:
(i) hydrogen, halogen, cyano (-C≡N), C₁-C₄ alkyl, C₁-C₄ alkoxy, C₁-C₄ haloalkyl, - C(=O)O**R**^{s}, -C(=O)N**R^{p}R^{q}**, -C**R**^{p}(=N)O**R**^{s}, or -O**R**^{s}; wherein:
the C₁-C₄ alkyl of any one of **R^{E}**, **R^{F},** and **R^{G}** is optionally substituted with 1 to 3 groups selected from cyano, -C(=O)R^{s}, -C(=O)O**R**^{s}, -C(=O)N**R**^{p}**R**^{q}, -O**R**^{s}, - OC(=O)**R**^{s}, -OC(=O)O**R**^{s}, -OC(=O)N**R**^{p}**R**^{q}, and -S(=O)₂**R**^{s}; wherein:
**R^{p}** and **R^{q},** for each occurrence, are each independently hydrogen, C₁-C₂ alkyl, C₃-C₅ cycloalkyl, or 5 or 6-membered heterocyclyl; wherein:
the C₁-C₂ alkyl of any one of **R^{p}** and **R^{q}** is optionally substituted with 1 to 3 groups selected from halogen, cyano, and -OH;
the C₃-C₅ cycloalkyl or the 5 or 6-membered heteroaryl of **R^{p}** and **R^{q} is** optionally substituted with 1 to 3 groups selected from halogen, cyano, and -OH;
**R^{s},** for each occurrence, is independently hydrogen, C₁-C₂ alkyl, or 5 or 6-membered heteroaryl; wherein the C₁-C₂ alkyl of **R^{s} is** optionally substituted with 1 to 3 groups selected from halogen, cyano, -OH, and -NH₂; wherein:
the 5 or 6-membered heteroaryl of **R^{s}** is optionally substituted with 1 to 3 groups selected from halogen, cyano, -OH, and -NH₂;
(ii) hydrogen, F, Cl, C₁-C₂ alkyl, C₁-C₂ haloalkyl, -C(=O)N**R^{p}R^{q}**, -C**R^{p}**(=N)O**R^{s}**, or -O**R^{s}**; wherein:
the C₁-C₂ alkyl of any one of **R^{E}, R^{F},** and **R^{G} is** optionally substituted with 1 to 3 groups selected from cyano, -C(=O)N**R^{p}R^{q}**, -O**R^{s}**, -OC(=O)N**R^{p}R^{q}**, and -S(=O)₂**R^{s}**; wherein:
**R^{p}** and **R^{q},** for each occurrence, are each independently hydrogen, C₁-C₂ alkyl, cyclopentyl, or tetrahydrofuranyl; wherein:
the C₁-C₂ alkyl of any one of **R^{p}** and **R^{q}** is optionally substituted with 1 to 3 halogen groups selected from F and Cl;
**R^{s},** for each occurrence, is independently hydrogen, C₁-C₂ alkyl, pyridinyl, or pyrimidinyl; wherein:
the C₁-C₂ alkyl of **R^{s}** is optionally substituted with 1 to 3 halogen groups selected from F and Cl;
(iii) hydrogen, F, -OH, -CH(OH)CH₃, -C(=O)NHCH₃, -C(=N)OCH₃, -CH₃, -CF₃, -CH₂F, -CH₂CN, -(CH₂)₂CN, -CH₂OH, -C₂H₅, -(CH₂)₂OH, - CH₂OCH₃, -CH₂OC₂H₅, -(CH₂)₂OCH₃, -CH₂OCHF₂, -(CH₂)₂OCHF₂, - CH₂C(=O)NH₂, -CH₂C(=O)N(CH₃)₂, -CH₂S(=O)₂CH₃, -(CH₂)₂S(=O)₂CH₃, - CH₂(O)C(=O)NHCH₃, -CH₂(0)C(=O)N(CH₃)C₂H₅,-CH₂(0)C(=O)N(CH₃)₂, - CH₂(0)C(=O)N(C₂H₅)₂, -CH₂(O)C(=O)NH(cyclopentyl), - CH₂(0)C(=O)NH(tetrahydrofuranyl), -CH₂(O)(pyridin-2-yl), or - CH₂(O)(pyrimidin-2-yl); and/or
(iv) hydrogen, F, -CH(OH)CH₃, -CH₃, -CH₂CN, -CH₂OH, or - CH₂OCH₃;
and wherein all other variables not specifically defined herein are as defined in any one of the preceding claims.

10. The compound, tautomer, deuterated derivative, or pharmaceutically acceptable salt according to any one of claims 1 to 9:
(i) represented by one of the following structural formulae: 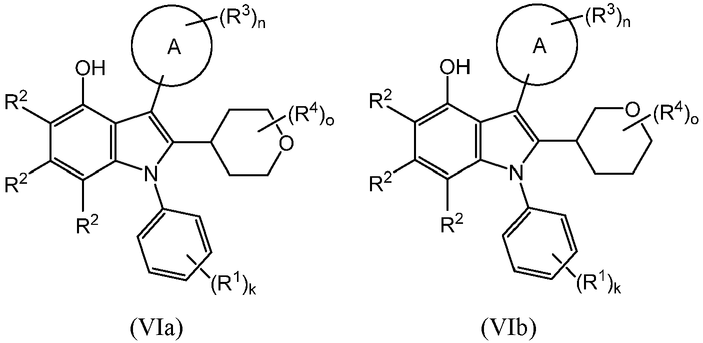 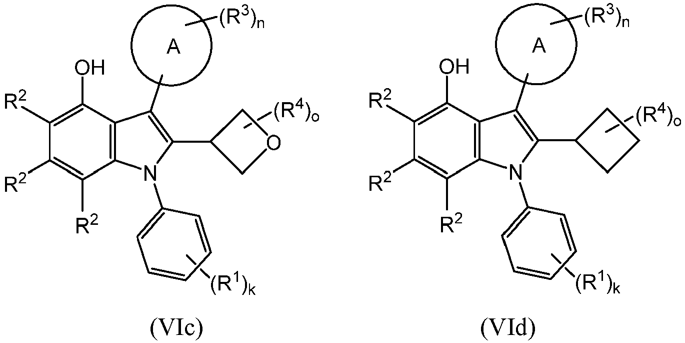 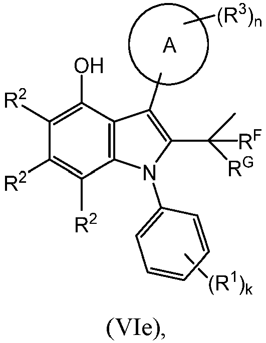 wherein **o** is an integer selected from 0, 1, and 2;
(ii) represented by one of the following structural formulae: 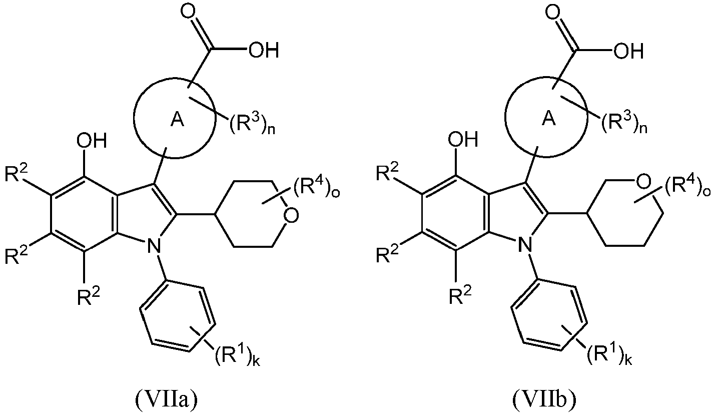 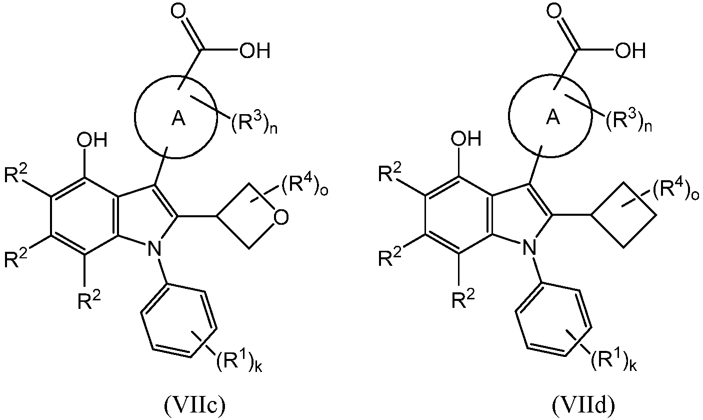 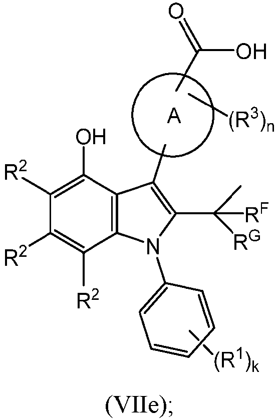 wherein **n** is an integer selected from 0, 1, and 2, and wherein **o** is an integer selected from 0, 1, and 2;
(iii) represented by one of the following structural formulae: 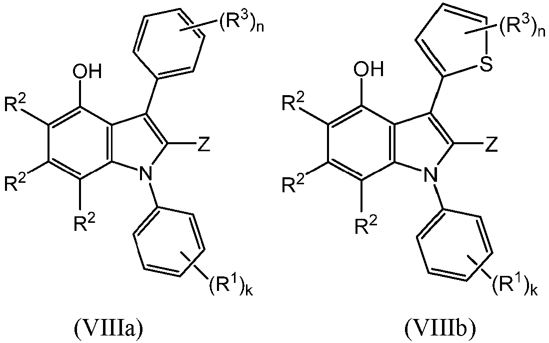 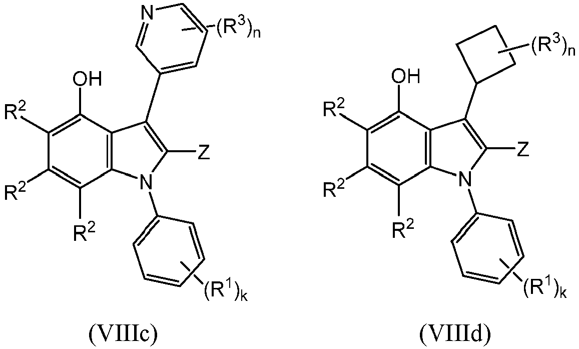 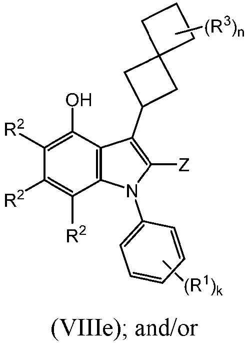
(iv) represented by one of the following structural formulae: 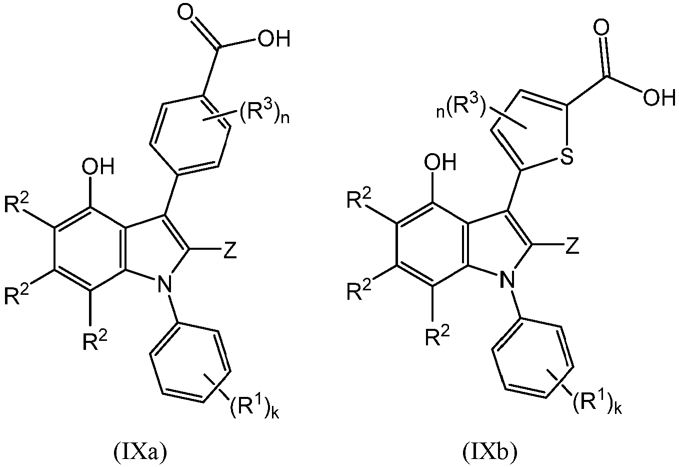 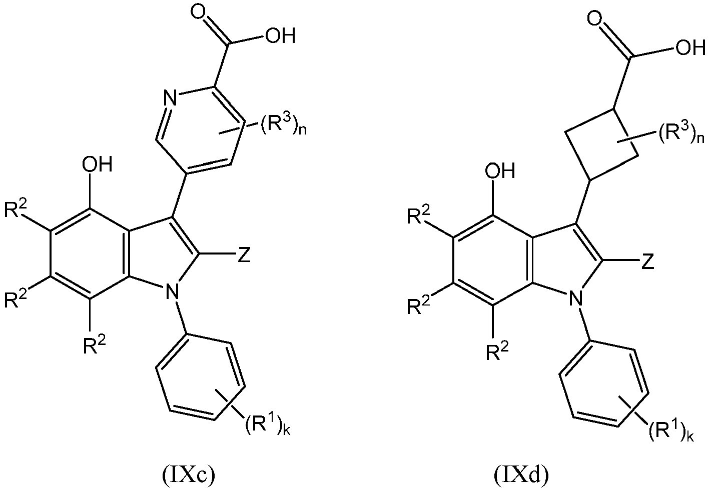 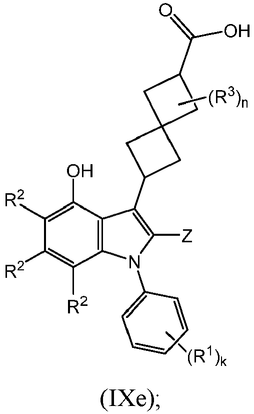
wherein **n** is an integer selected from 0, 1, and 2;
and wherein all other variables not specifically defined herein are as defined in any one of the preceding claims.

11. The compound, tautomer, deuterated derivative, or pharmaceutically acceptable salt according to any one of claims 1 to 10, wherein:
(i) **R²**, for each occurrence, is independently hydrogen, halogen, cyano, C₁-C₆ alkyl (optionally substituted with 1 to 3 groups selected from cyano, -OH, -OCH₃, and -NH₂), C₁-C₆ alkoxy, C₁-C₆ haloalkyl, -N**R^{h}Rⁱ**, or C₃-C₆ cycloalkyl; wherein **R^{h}** and **Rⁱ**, for each occurrence, are each independently hydrogen or C₁-C₄ alkyl;
(ii) **R²**, for each occurrence, is independently hydrogen, halogen, cyano, C₁-C₄ alkyl (optionally substituted with 1 to 3 groups selected from cyano, -OH, -OCH₃, and -NH₂), C₁-C₄ alkoxy, C₁-C₄ haloalkyl, -N**R^{h}Rⁱ**, or C₃-C₅ cycloalkyl; wherein **R^{h}** and **Rⁱ**, for each occurrence, are each independently hydrogen or C₁-C₄ alkyl;
(iii) **R²**, for each occurrence, is independently hydrogen, halogen, cyano, C₁-C₂ alkyl (optionally substituted with 1 to 3 groups selected from cyano, -OH, -OCH₃, and -NH₂), C₁-C₂ haloalkyl, -N**R^{h}Rⁱ**, or C₃-C₄ cycloalkyl; wherein **R^{h}** and **Rⁱ**, for each occurrence, are each independently hydrogen or -CH₃;
(iv) **R²**, for each occurrence, is independently hydrogen, F, Cl, cyano, -CH₃, -CHF₂, -CF₃, -NH₂, or cyclopropyl;
(v) **R³,** for each occurrence, is independently halogen, cyano, =O, C₁-C₆ alkyl, C₁-C₆ alkoxy, C₁-C₆ haloalkyl, -C(=O)**R^{y}**, -C(=O)O**R^{y}**, -C(=O)N**R^{v}R^{w}**, -C(=O)N**R^{v}**O**R^{y}**,-C(=O)NR^{v}S(=O)₂**R^{y}**, -N**R^{v}R^{w}**, -O**R^{y}**, -S(=O)₂**R^{y}**, -S(=O)₂N**R^{v}R^{w}**, -S(=O)₂N**R^{v}**C(=O)**R^{y}**, or - P(=O)**R^{z}R^{z}**; wherein:
the C₁-C₄ alkyl of **R³** is optionally substituted with 1 to 3 groups selected from cyano, -O**R^{y}**, -C(=O)O**R^{y}**, and -N**R^{v}R^{w}**; wherein:
**R^{v}, R^{w},** and **R^{x},** for each occurrence, are each independently hydrogen or C₁-C₂ alkyl; wherein the C₁-C₂ alkyl of any one of **R^{v}, R^{w},** and **R^{x}** is optionally substituted with 1 to 3 groups selected from halogen, cyano, -OH, and -NH₂; and
**R^{y}**, for each occurrence, is independently hydrogen, C₁-C₄ alkyl, or 5 or 6-membered heterocyclyl; wherein:
the C₁-C₄ alkyl of **R^{y}** is optionally substituted with 1 to 3 groups selected from halogen, cyano, -OH, -NH₂, and -C(=O)OH; and
the 5 or 6-membered heterocyclyl of **R^{y}** is optionally substituted with 1 to 3 groups selected from halogen, cyano, -OH, -NH₂, and -C(=O)OH;
(vi) **R³,** for each occurrence, is independently halogen, cyano, =O, C₁-C₄ alkyl, C₁-C₄ alkoxy, C₁-C₄ haloalkyl, -C(=O)**R^{y}**, -C(=O)O**R^{y}**, -C(=O)N**R^{v}R^{w}**, - C(=O)N**R^{v}**O**R^{y}**, -C(=O)N**R^{v}**S(=O)₂**R^{y}**, -N**R^{v}R^{w}**, -O**R^{y}**, -S(=O)₂**R^{y}**, -S(=O)₂N**R^{v}R^{w}**, - S(=O)₂N**R^{v}**C(=O)**R^{y}**, or -P(=O)**R^{z}R^{z}**; wherein:
the C₁-C₄ alkyl of **R³** is optionally substituted with 1 to 3 groups selected from cyano, -O**R^{y}** and -C(=O)O**R^{y}**; wherein:
**R^{v}**, **R^{w}**, and **R^{x}**, for each occurrence, are each independently hydrogen or C₁-C₄ alkyl; wherein the C₁-C₄ alkyl of any one of **R^{v}**, **R^{w}**, and **R^{x}** is optionally substituted with -OH; and
**R^{y}**, for each occurrence, is independently hydrogen, C₁-C₂ alkyl, or 6-membered heterocyclyl; wherein:
the C₁-C₂ alkyl of **R^{y}** is optionally substituted with 1 to 3 groups selected from -OH and -C(=O)OH; and
the 6-membered heterocyclyl of **R^{y}** is optionally substituted with 1 to 3 groups selected from -OH and -C(=O)OH;
**R^{z}**, for each occurrence, is independently -CH₃, -OH, or -OCH₃;
(vii) **R³**, for each occurrence, is independently halogen, cyano, =O, C₁-C₂ alkyl, C₁-C₂ alkoxy, C₁-C₄ haloalkyl, -C(=O)**R^{y}**, -C(=O)O**R^{y}**, -C(=O)N**R^{v}R^{w}**,-C(=O)N**R^{v}**O**R^{y}**, - C(=O)N**R^{v}**S(=O)₂**R^{y}**, -N**R^{v}R^{w}**, -O**R^{y}**, -S(=O)₂**R^{y}**, -S(=O)₂N**R^{v}R^{w}**, -S(=O)₂N**R^{v}**C(=O)**R^{y}**, or - P(=O)**R^{z}R^{z}**; wherein:
the C₁-C₂ alkyl of **R³** is optionally substituted with 1 to 3 groups selected from -OH and -C(=O)O**R^{y}**; wherein:
**R^{v}, R^{w},** and **R^{x},** for each occurrence, are each independently hydrogen or C₁-C₂ alkyl; wherein the C₁-C₂ alkyl of any one of **R^{v}, R^{w},** and **R^{x}** is optionally substituted with -OH; and
**R^{y}**, for each occurrence, is independently hydrogen, C₁-C₂ alkyl, or tetrahydro-2H-pyranyl; wherein:
the C₁-C₂ alkyl of **R^{y}** is optionally substituted with -C(=O)OH; and
the tetrahydro-2H-pyranyl of **R^{y}** is optionally substituted with 1 to 3 groups selected from -OH and -C(=O)OH;
**R^{z},** for each occurrence, is independently -CH₃ or -OH;
(viii) **R³,** for each occurrence, is independently F, Cl, cyano, -OH, =O, -CH₃, -OCH₃, -CF₃, -CH₃CN, -C(CH₃)₂CH₂OH, -CH₂COOH, -CH₂OCH₃, -C(=O)CHCH₃OH, -COOH, - C(=O)O(2-tetrahydro-2H-pyranyl), -C(=O)NH₂, -C(=O)NH(CH₂)₂OH, -C(=O)NHOH, - C(=O)NHS(=O)₂CH₃, -NH₂, -NHCH₃, -OCH₂COOH, NHS(=O)₂CH₃,-S(=O)₂CH₃, - S(=O)₂NH₂, -S(=O)₂NHC(=O)CH₃, or -P(=O)(CH₃)₂; wherein the 2-tetrahydro-2H-pyranyl in - C(=O)O(2-tetrahydro-2H-pyranyl) is substituted with 1 to 3 groups selected from -OH and - C(=O)OH;
(ix) **R⁴,** for each occurrence, is independently halogen, cyano, C₁-C₆ alkyl, C₁-C₆ alkoxy, C₁-C₆ haloalkyl, -C(=O)**R^{y}**, -C(=O)O**R^{y}**, -O**R^{y}**, or -S(=O)₂**R^{y}**; wherein:
the C₁-C₆ alkyl of **R⁴** is optionally substituted with 1 to 3 groups selected from cyano, -O**R^{y}**, -C(=O)O**R^{y}**, and -N**R^{v}R^{w}**; wherein:
**R^{v}** and **R^{w}**, for each occurrence, are each independently hydrogen or C₁-C₄ alkyl; and
**R^{y}**, for each occurrence, is independently hydrogen or C₁-C₄ alkyl;
wherein:
the C₁-C₄ alkyl of **R^{y}** is optionally substituted with 1 to 3 groups selected from halogen, cyano, -OH, -OCH₃, and -NH₂;
(x) **R⁴**, for each occurrence, is independently halogen, cyano, C₁-C₄ alkyl, C₁-C₄ alkoxy, C₁-C₄ haloalkyl, -C(=O)**R^{y}**, -C(=O)O**R^{y}**, -O**R^{y}**, or -S(=O)₂**R^{y}**; wherein:
the C₁-C₄ alkyl of **R⁴** is optionally substituted with 1 to 3 groups selected from cyano, -O**R^{y}**, -C(=O)O**R^{y}**, and -N**R^{v}R^{w}**; wherein:
**R^{v}** and **R^{w}**, for each occurrence, are each independently hydrogen or C₁-C₄ alkyl;
**R^{y}**, for each occurrence, is independently hydrogen or C₁-C₂ alkyl;
wherein:
the C₁-C₂ alkyl of any one of **R^{y}** is optionally substituted with 1 to 3 groups selected from halogen, cyano, -OH, -OCH₃, and -NH₂; and
wherein **o** or **m** is an integer selected from 0, 1, and 2;
(xi) **R⁴**, for each occurrence, is independently cyano, C₁-C₂ alkyl, C₁-C₂ alkoxy, C₁-C₂ haloalkyl, -C(=O)**R^{y}**, -C(=O)O**R^{y}**, -O**R^{y}**, or -S(=O)₂**R^{y}**; wherein:
the C₁-C₂ alkyl of **R⁴** is optionally substituted with cyano, -OH, or -OCH₃;
**R^{y}**, for each occurrence, is independently hydrogen or C₁-C₂ alkyl;
wherein:
the C₁-C₂ alkyl of **R^{y}** is optionally substituted with -OCH₃;
wherein **o** or **m** is an integer selected from 0 and 1; and/or
(xii) **R⁴**, for each occurrence, is independently cyano, -OH, -OCH₃, - CH₃, -C₂H₅, -CF₃, -CH₂CN, -CH₂OH, -CH₂OCH₃, -COOH, -C(=O)CH₃, - C(=O)OCH₃, -C(=O)CH₂OCH₃, -S(=O)₂CH₃, -S(=O)₂C₂H₅, or S(=O)₂CF₃; and wherein all other variables not specifically defined herein are as defined in any one of the preceding claims.

12. The compound according to claim 1, wherein the compound is selected from: 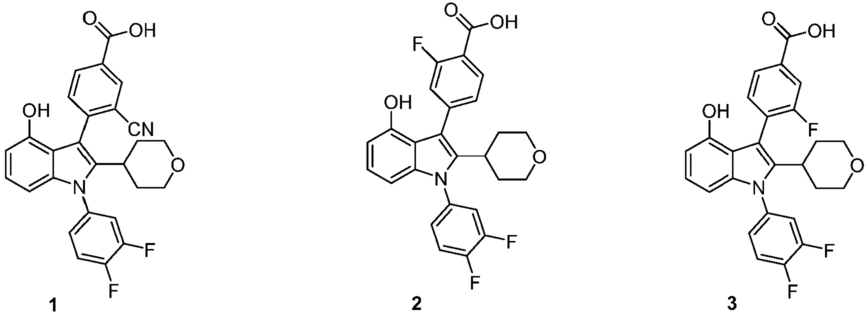 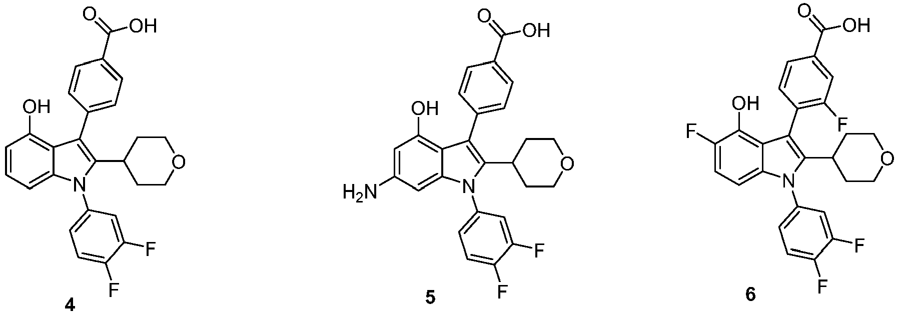 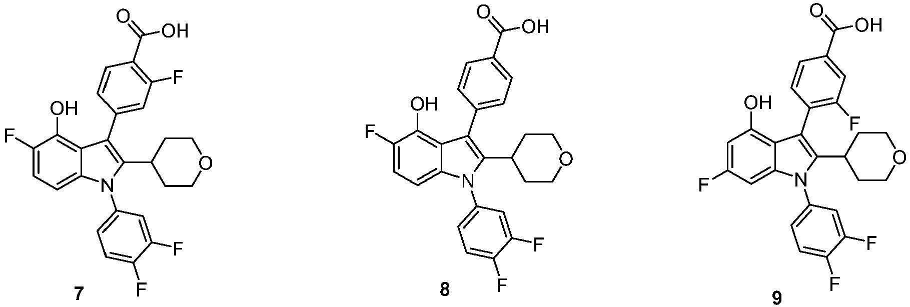 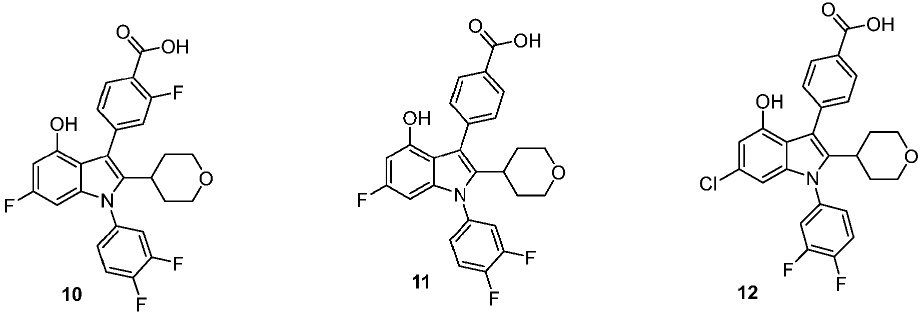 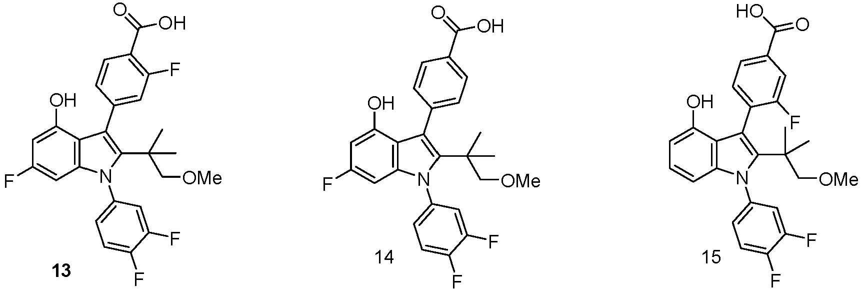 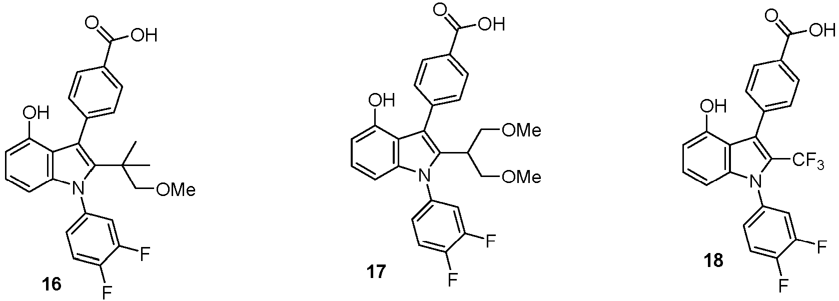 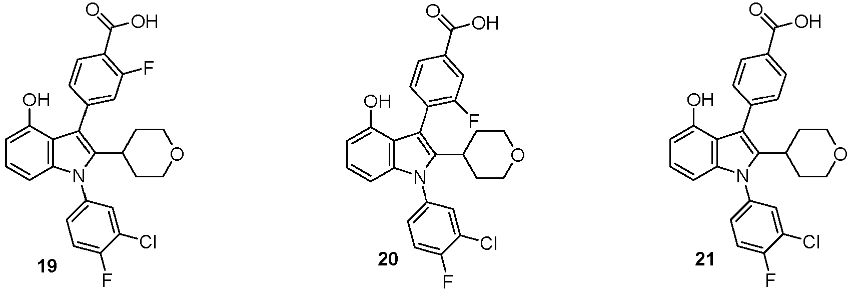 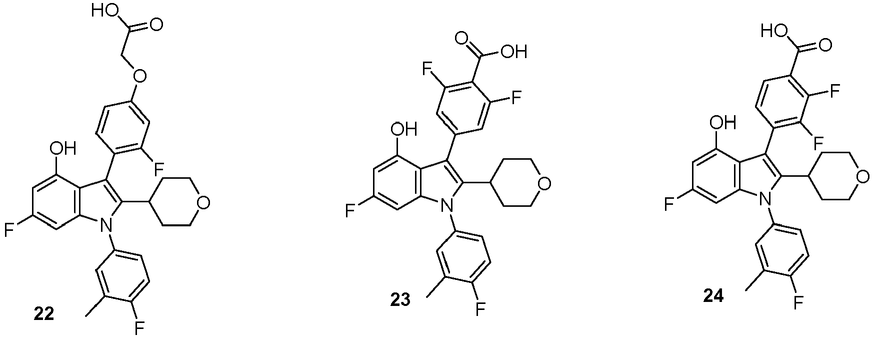 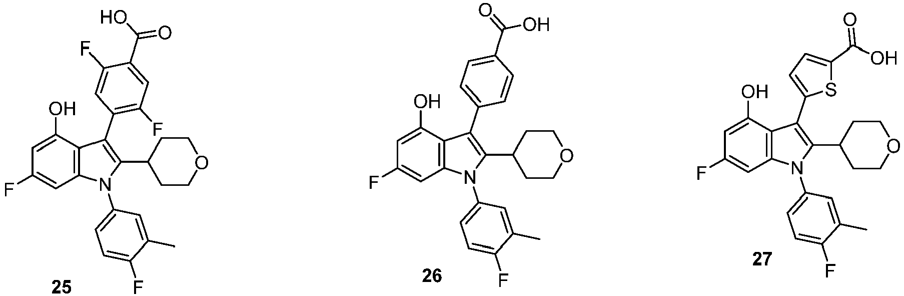 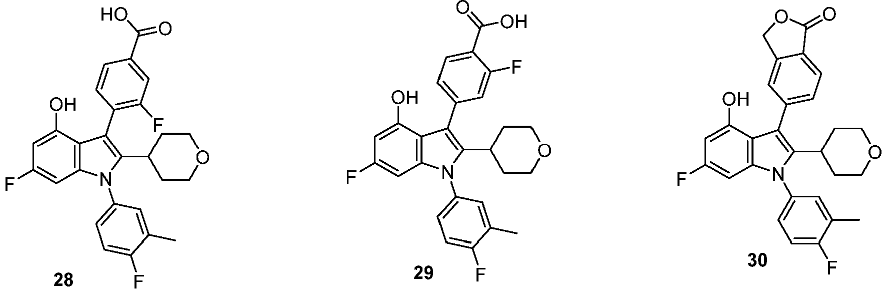 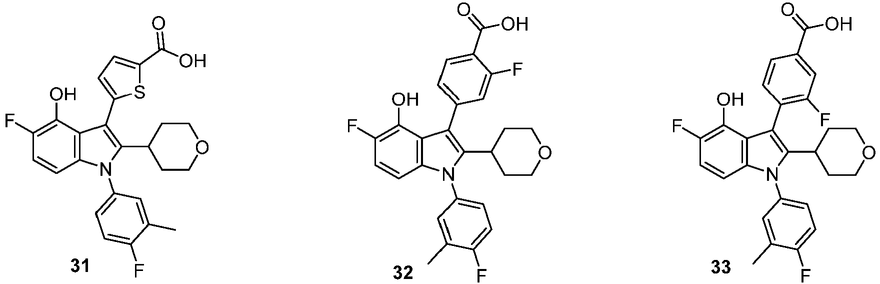 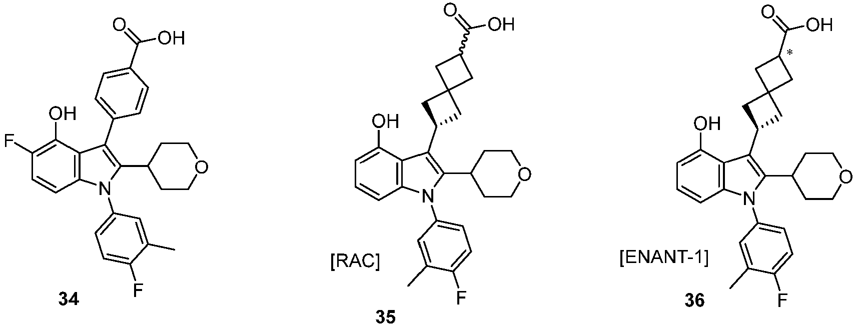 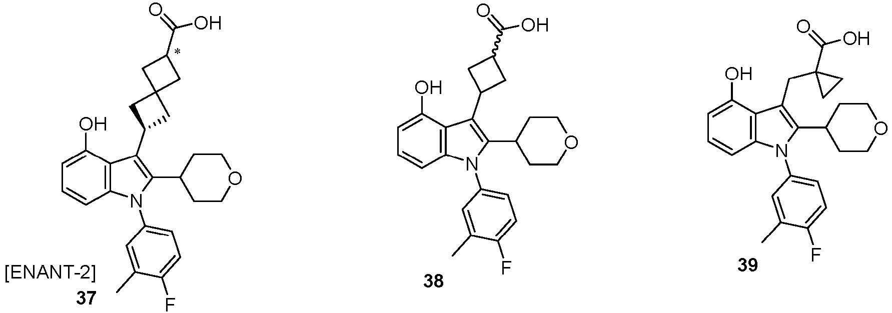 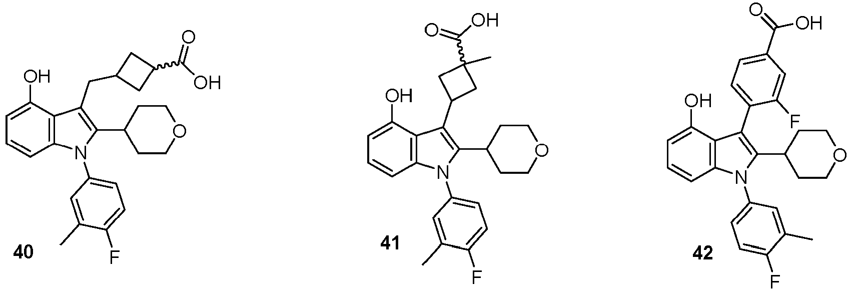 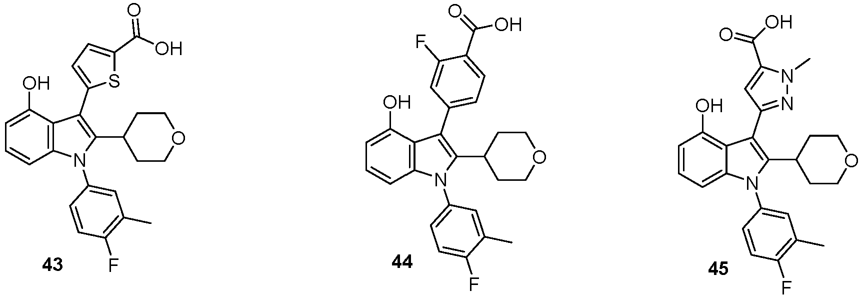 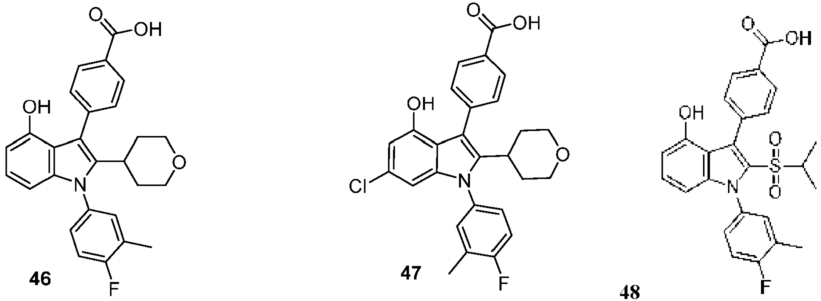 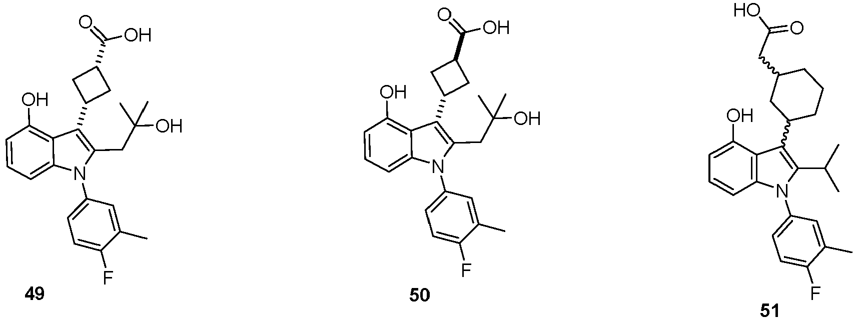 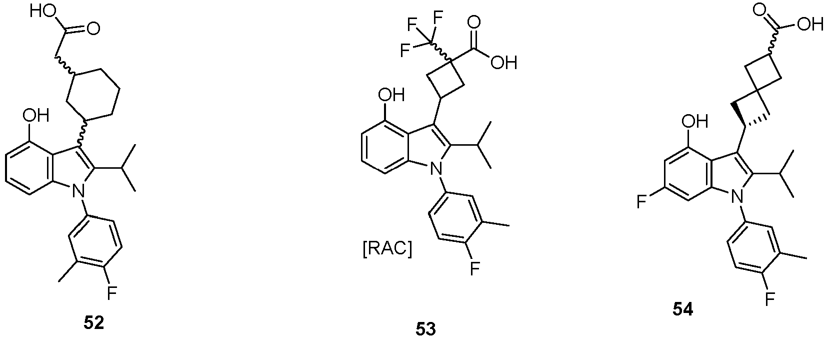 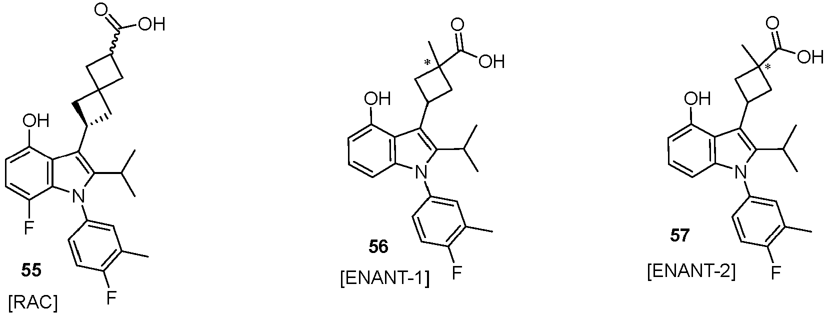 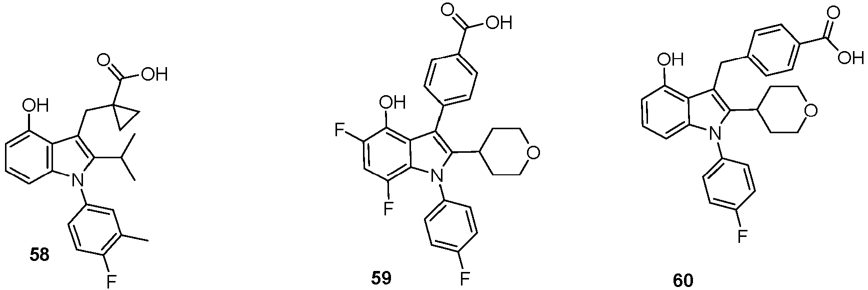 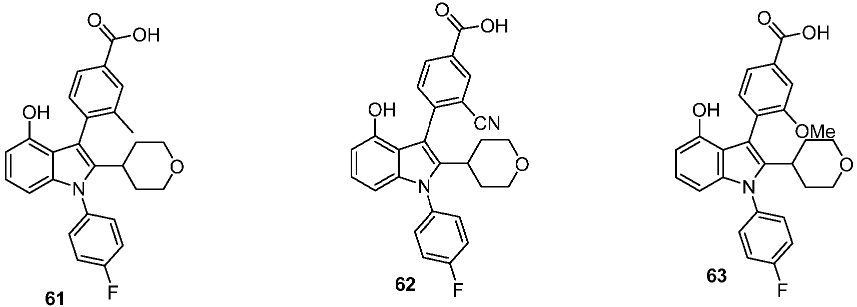 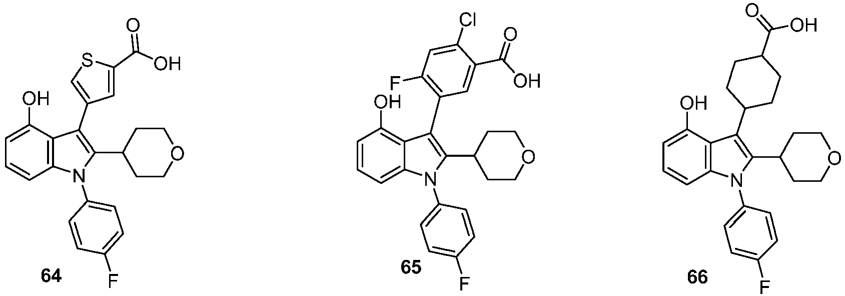 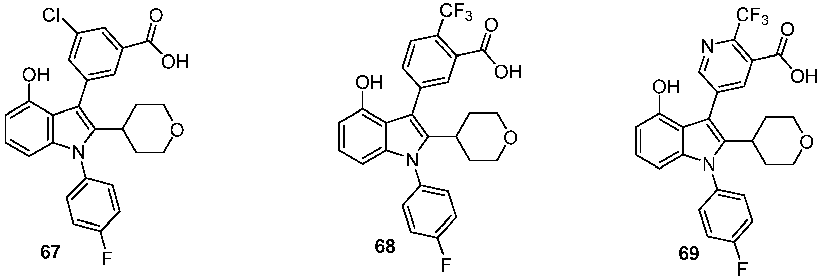 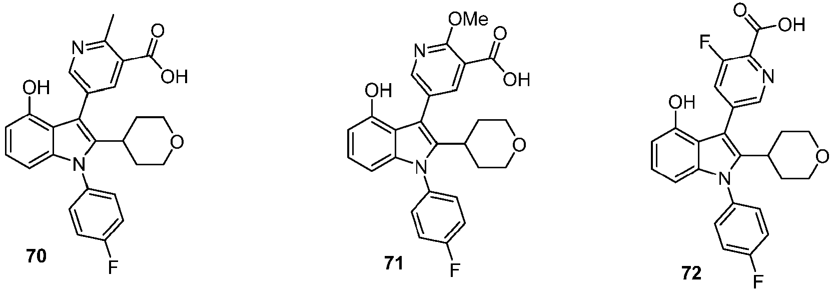 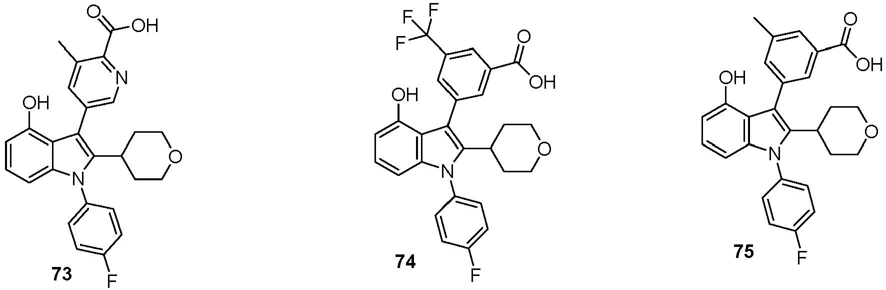 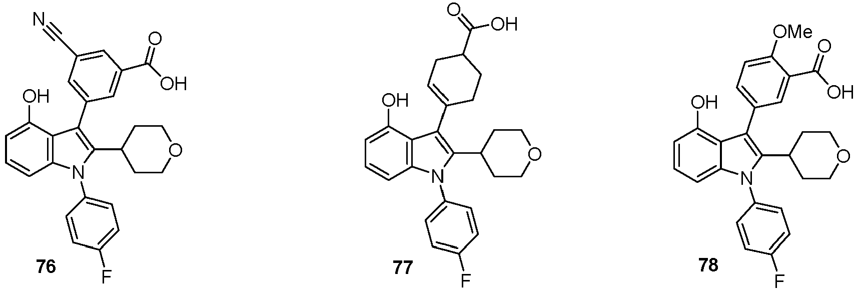 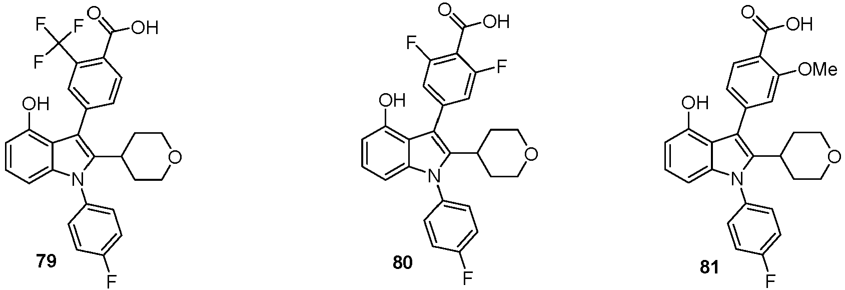 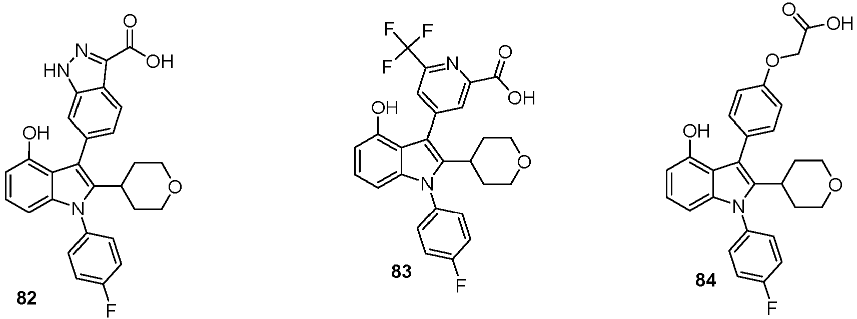 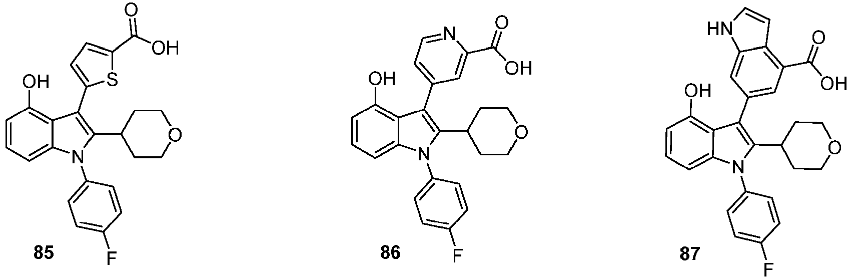 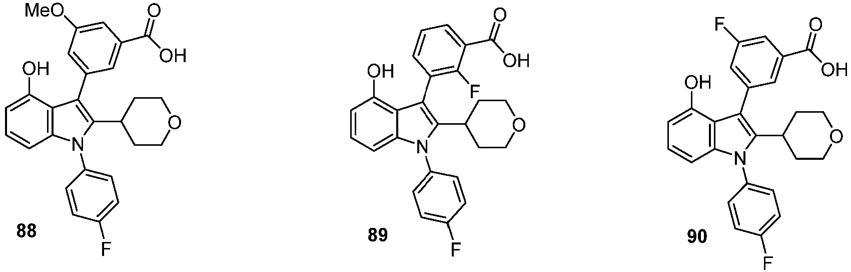 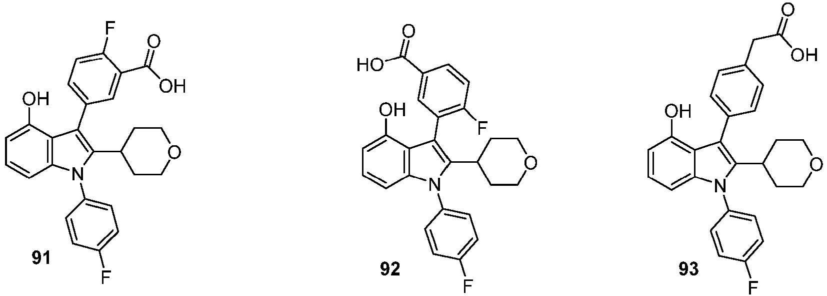 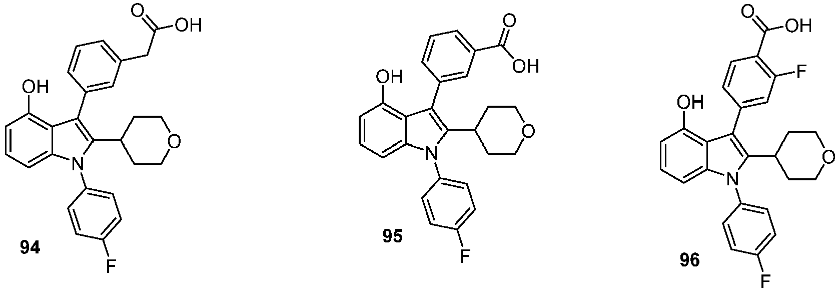 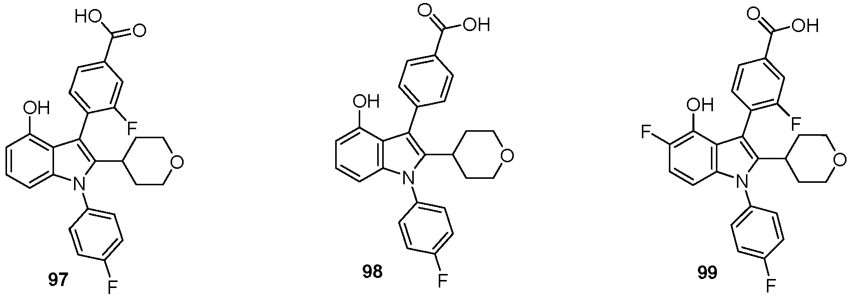 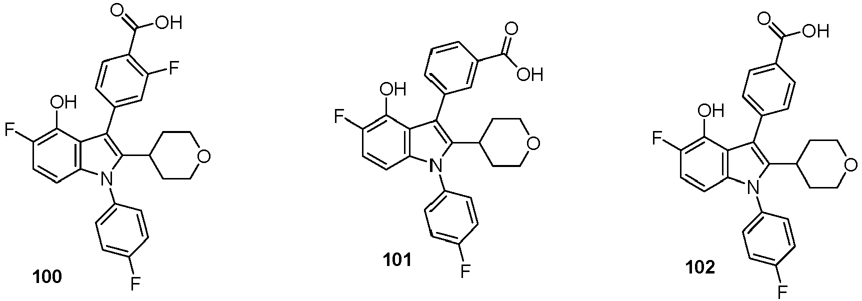 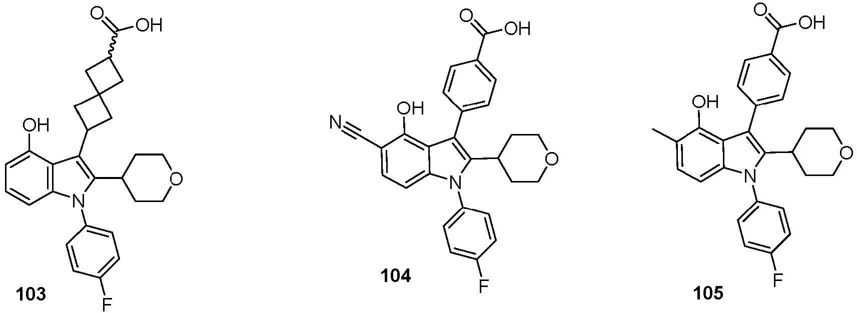 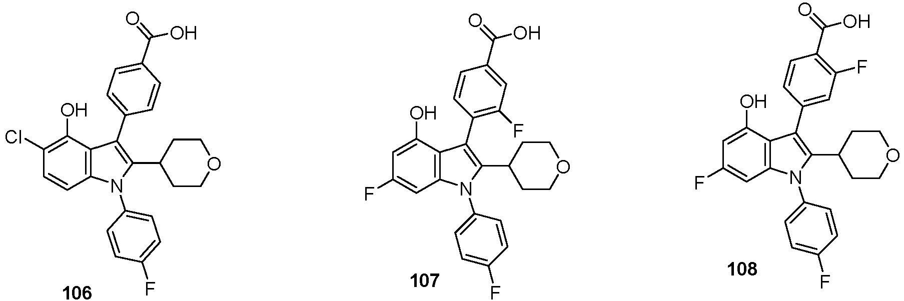 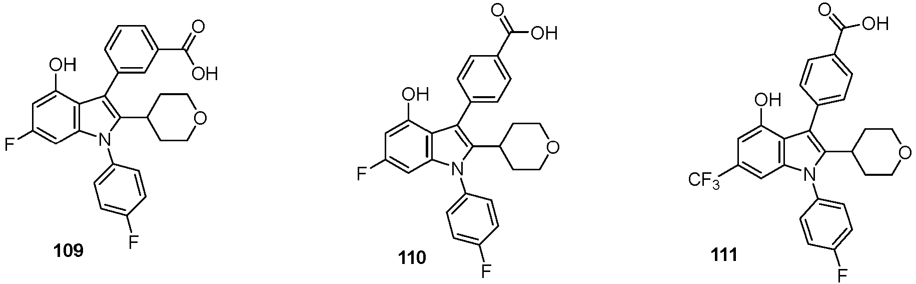 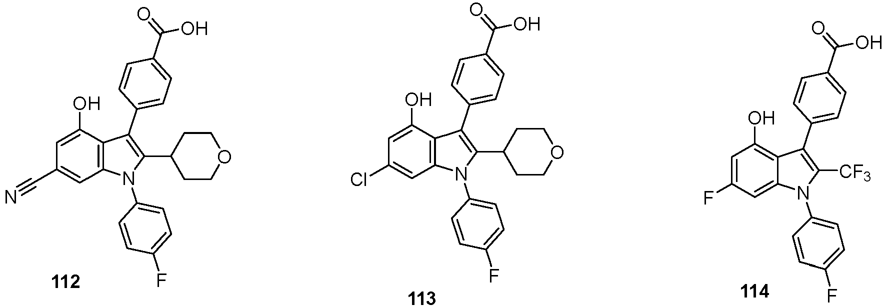 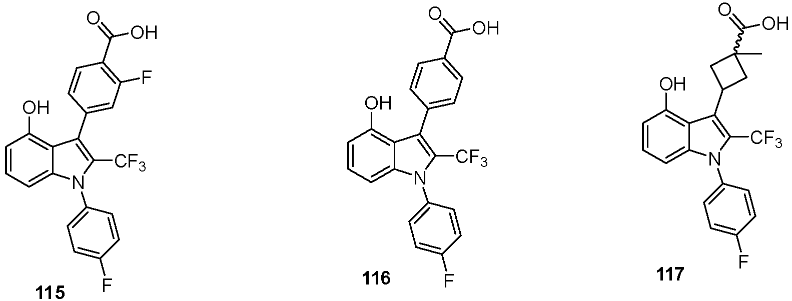 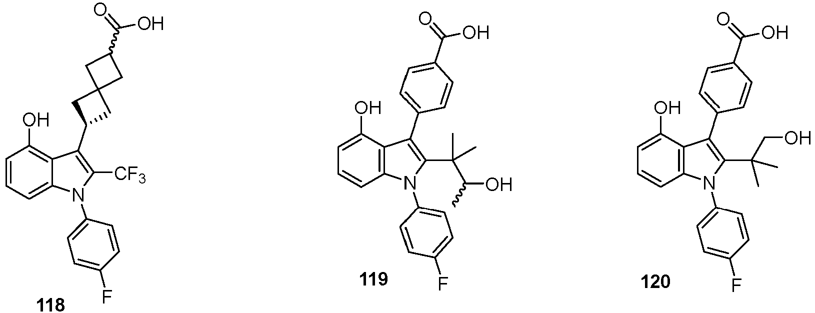 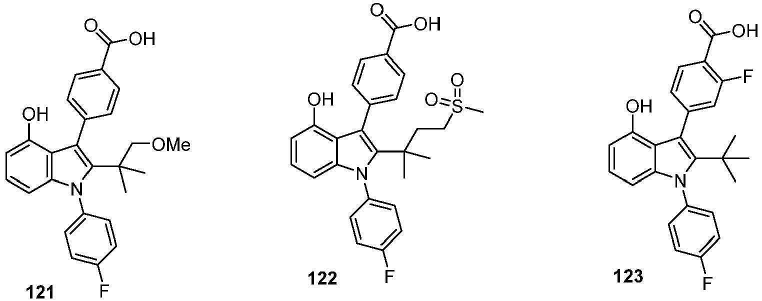 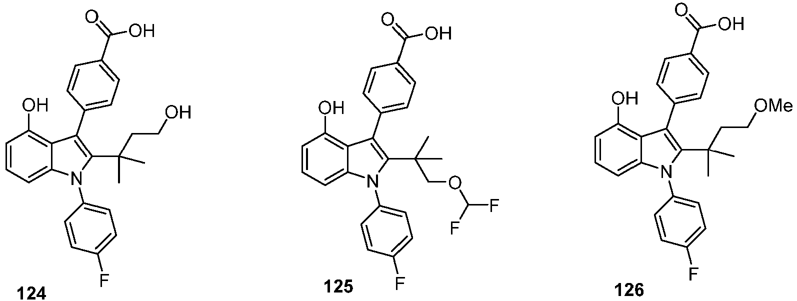 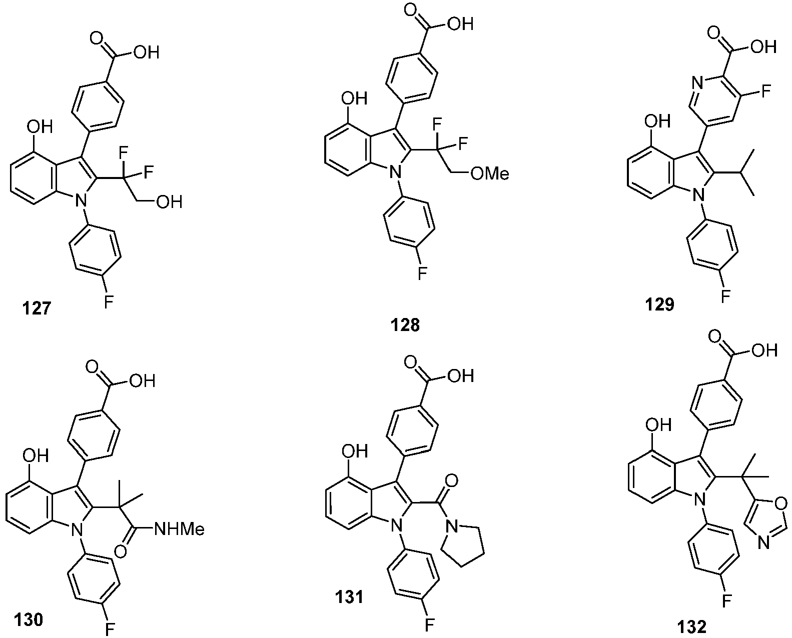 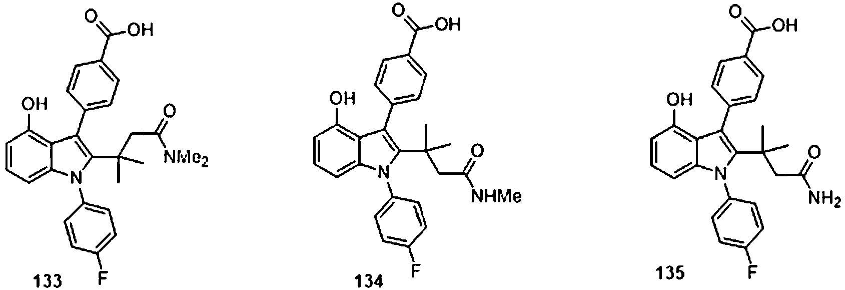 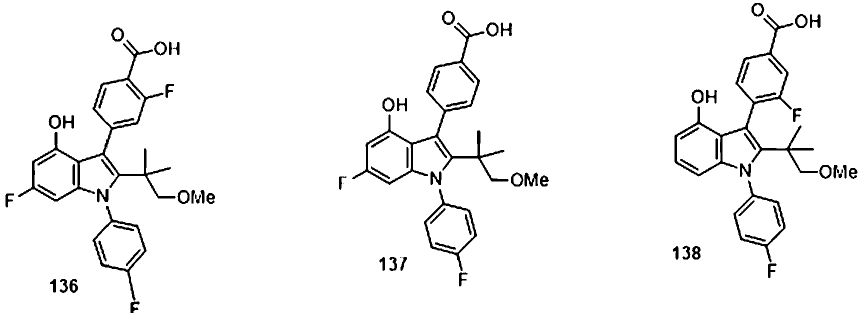 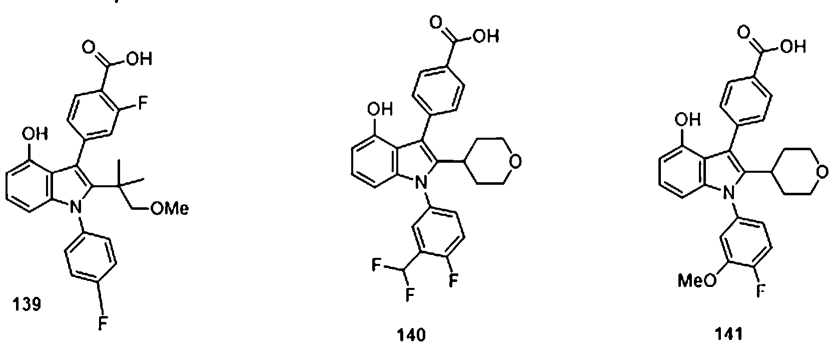 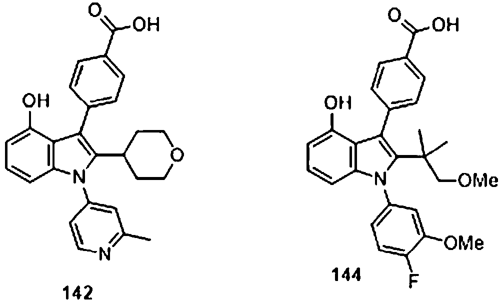 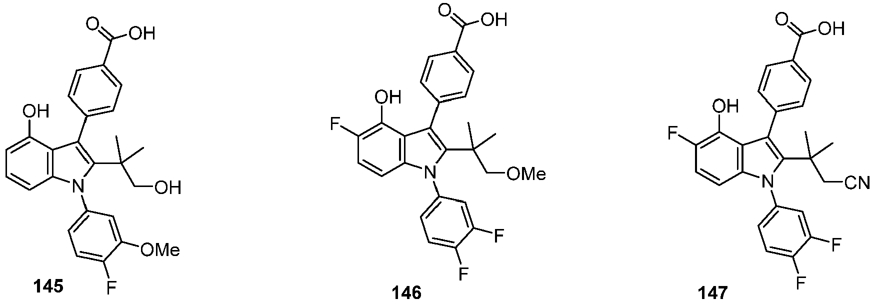 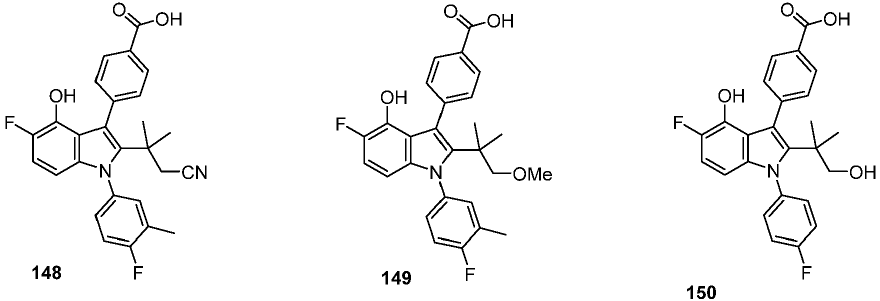 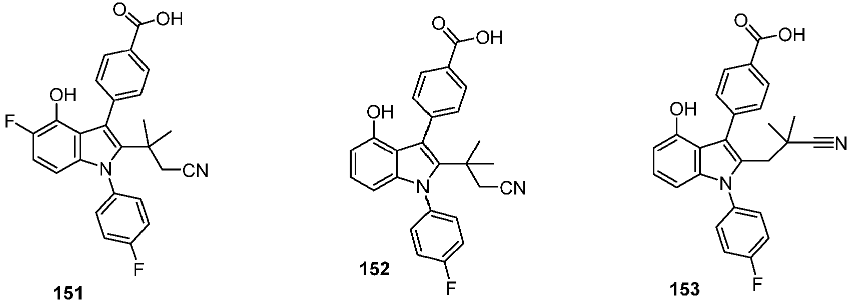 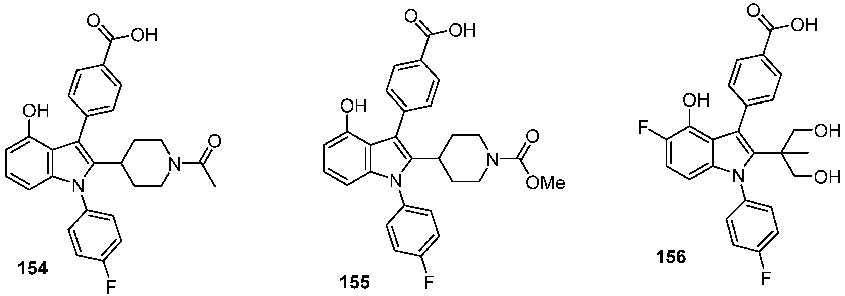 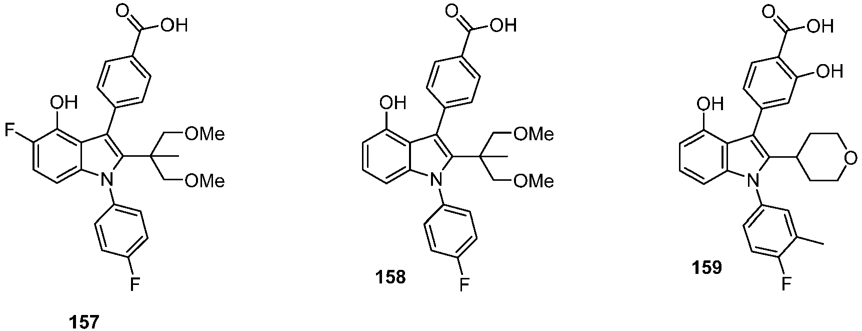 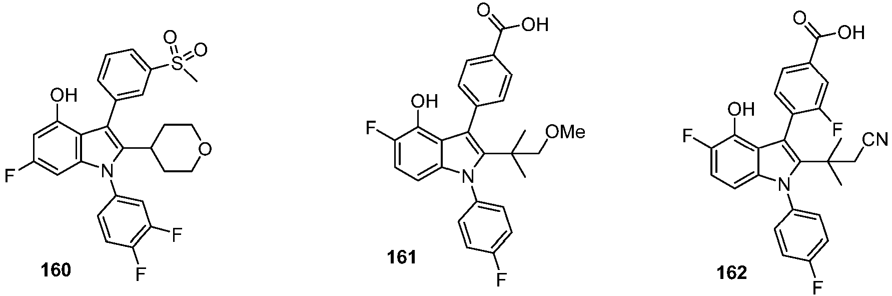 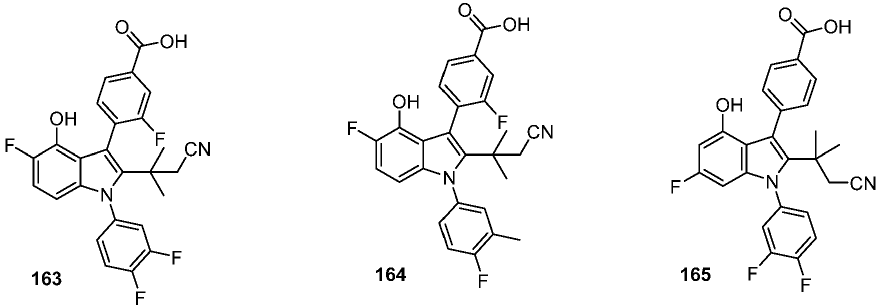 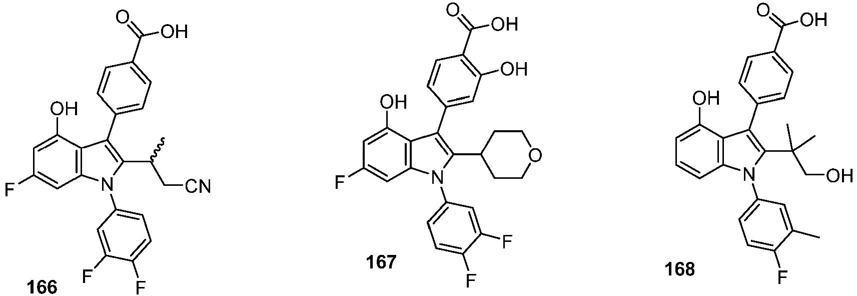 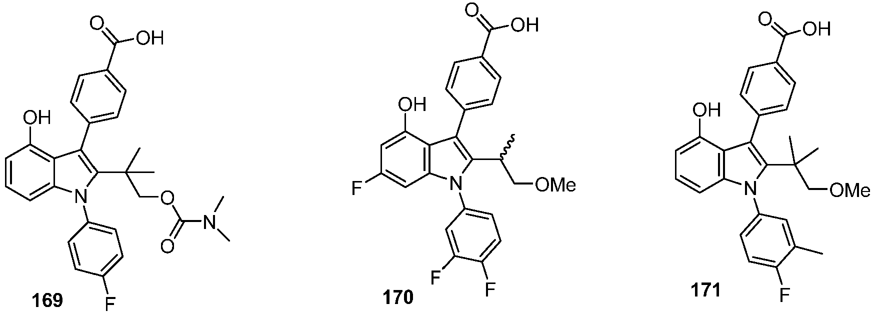 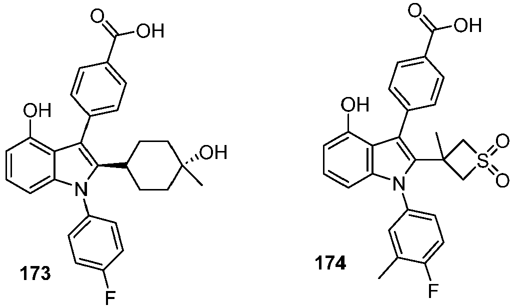 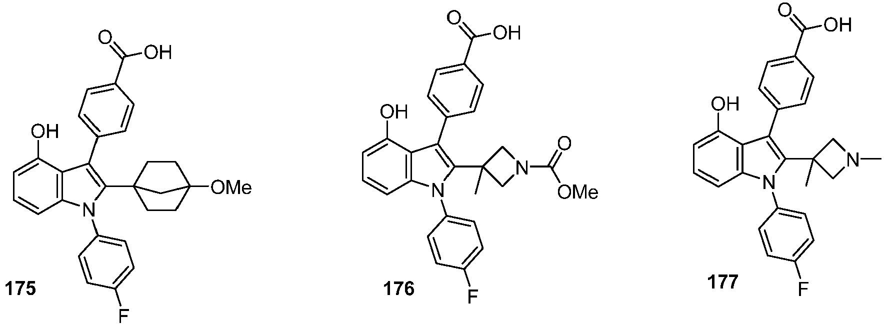 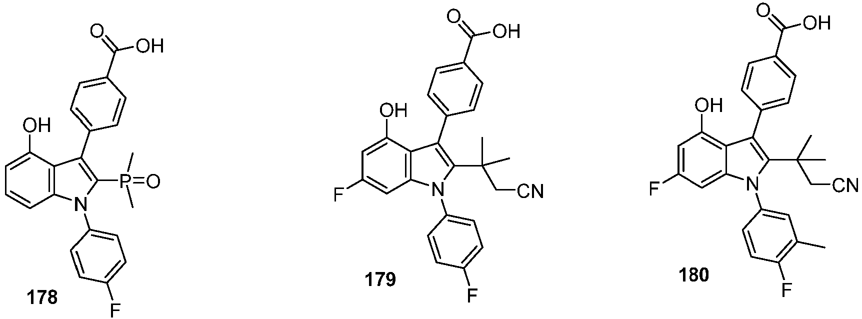 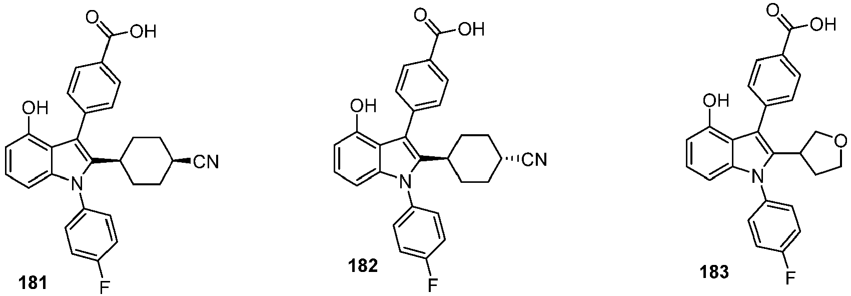 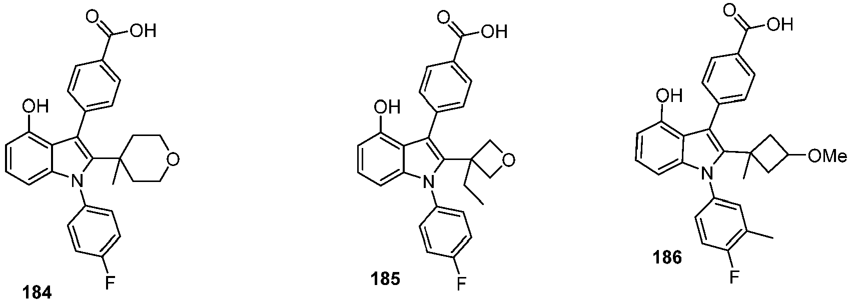 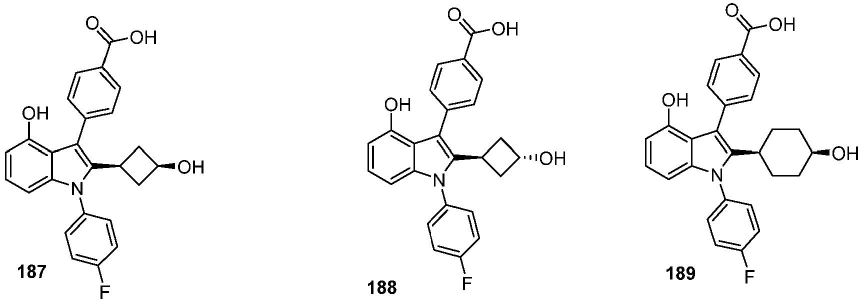 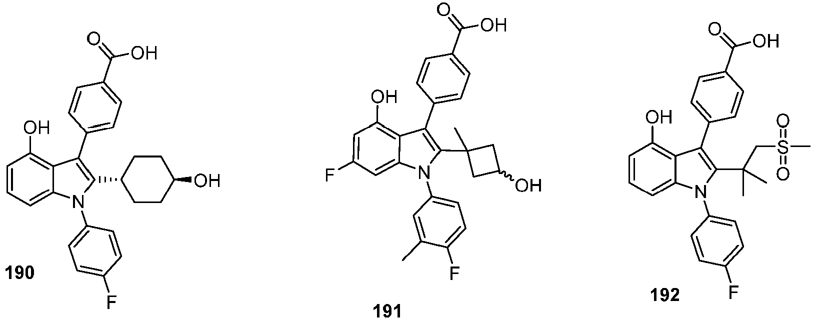 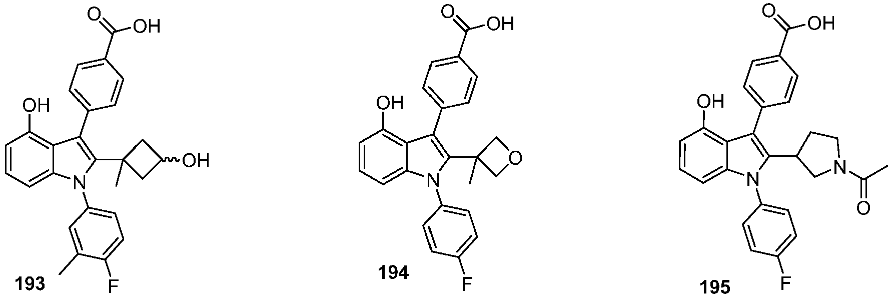 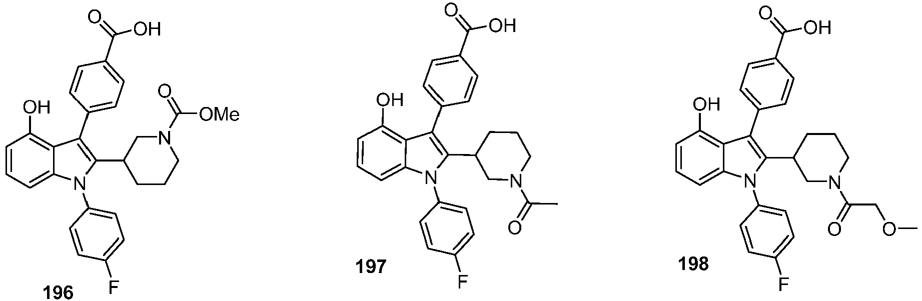 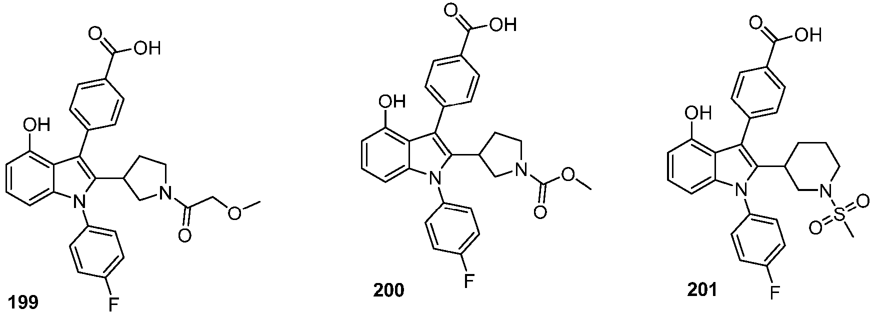 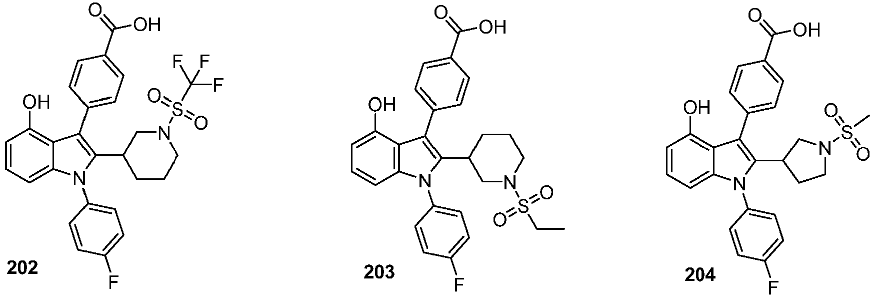 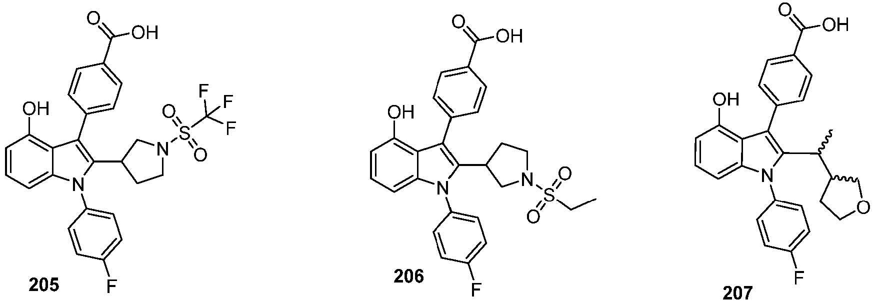 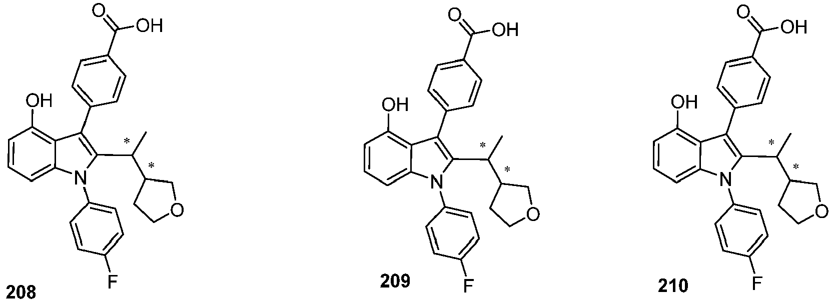 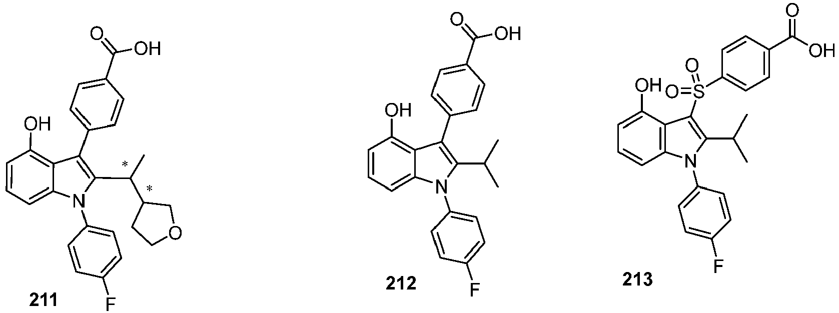 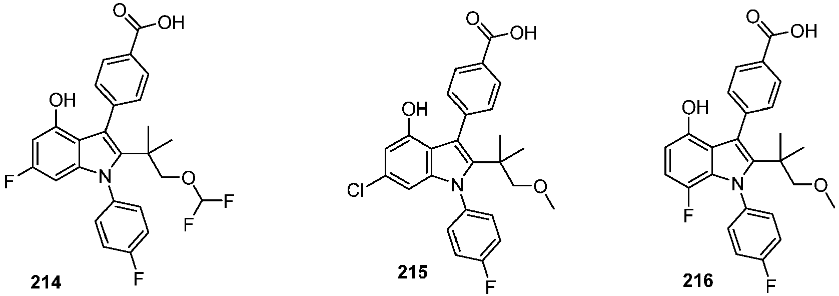 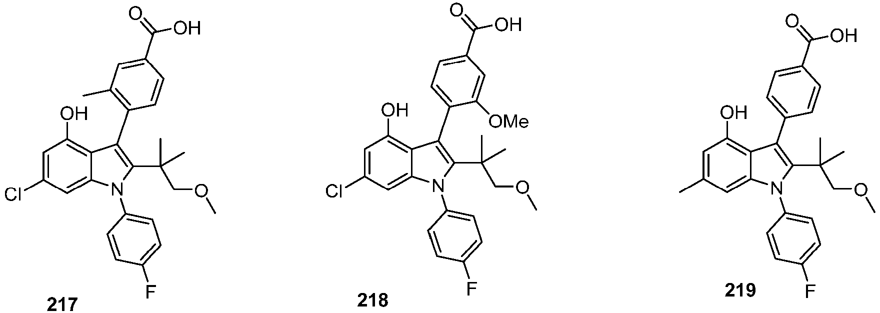 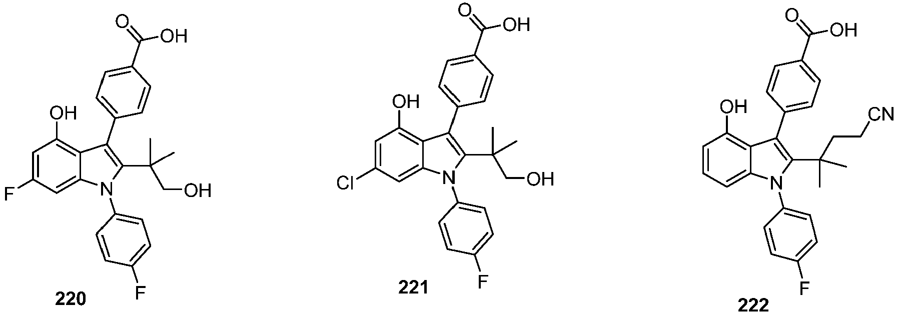 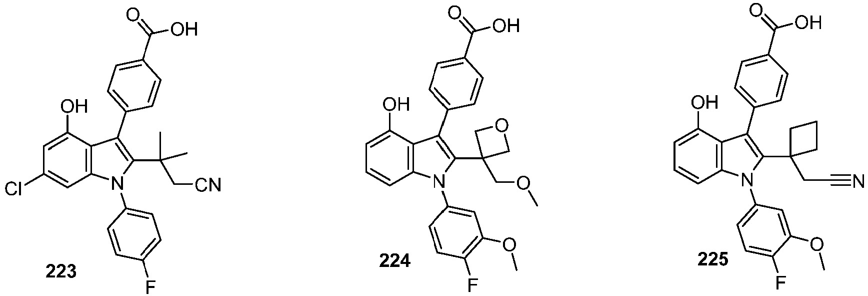 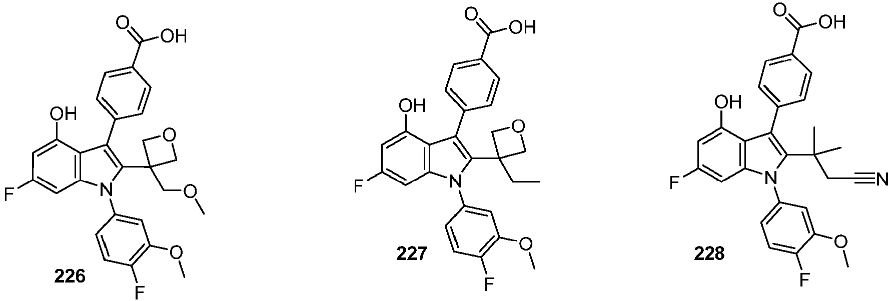 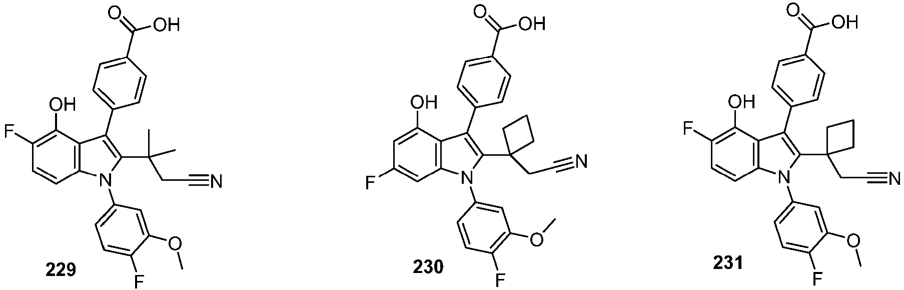 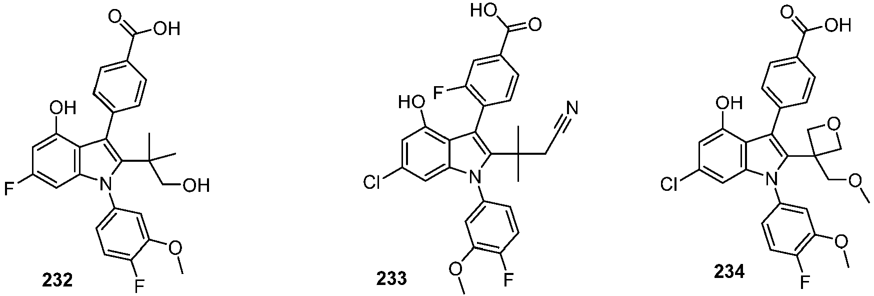 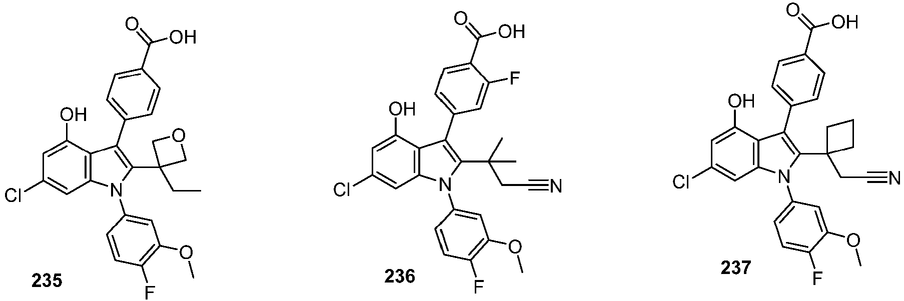 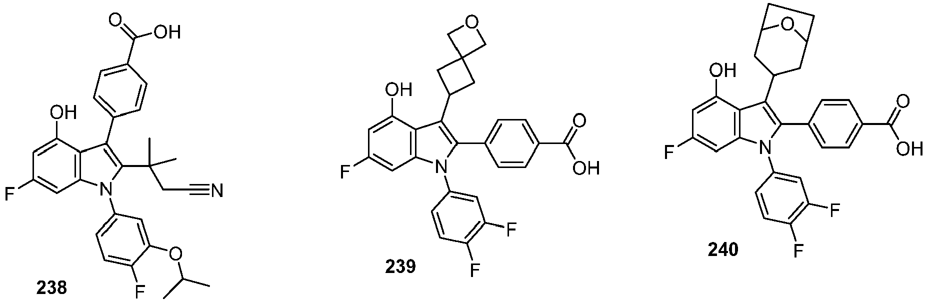 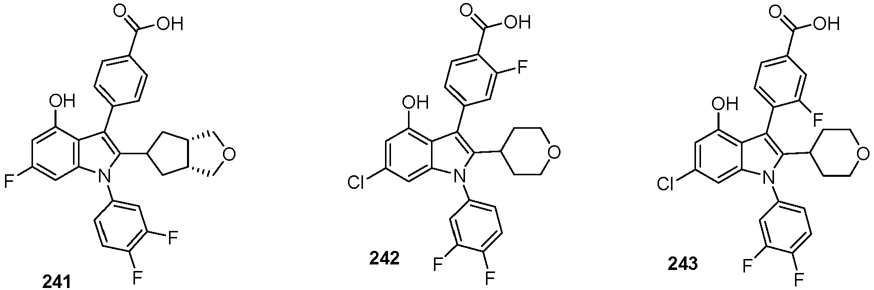 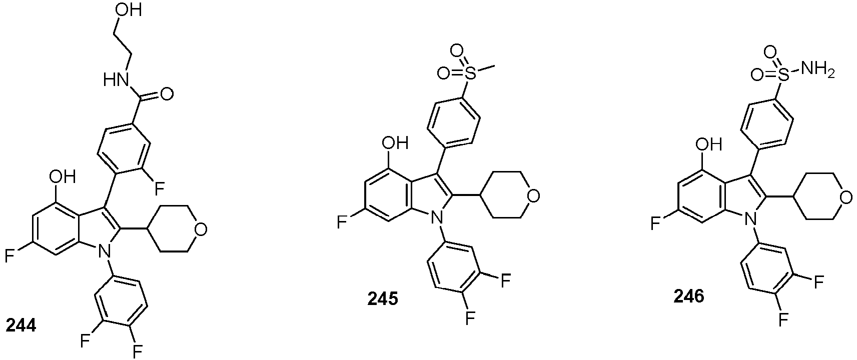 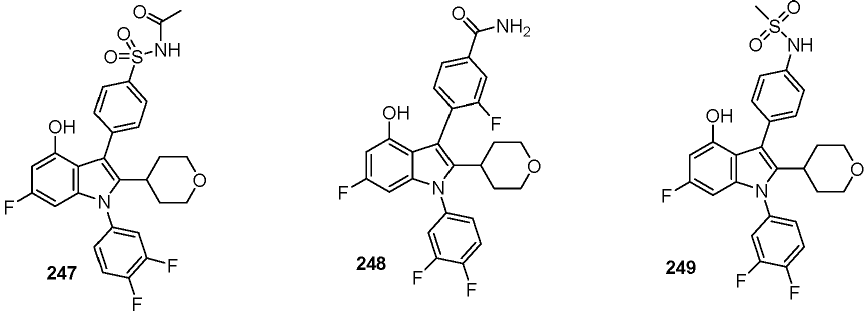 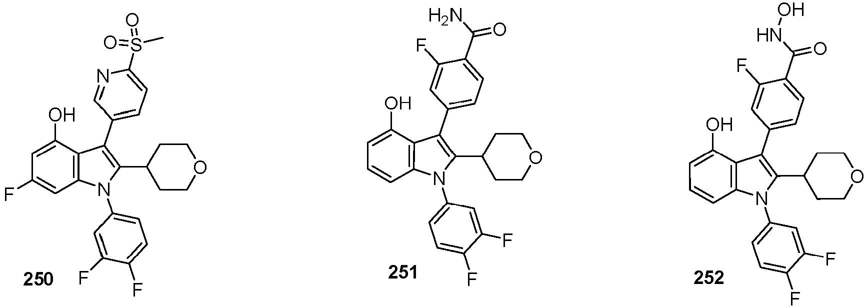 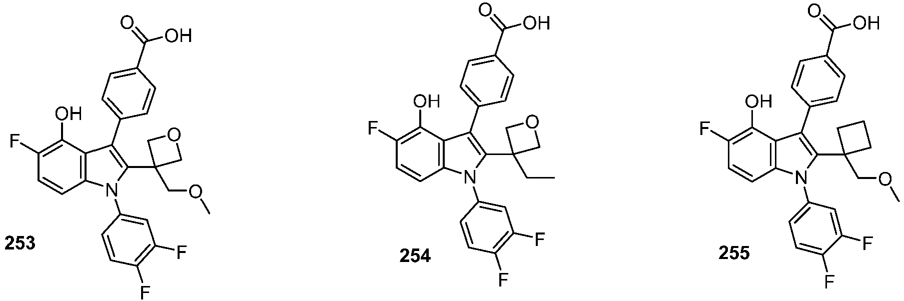 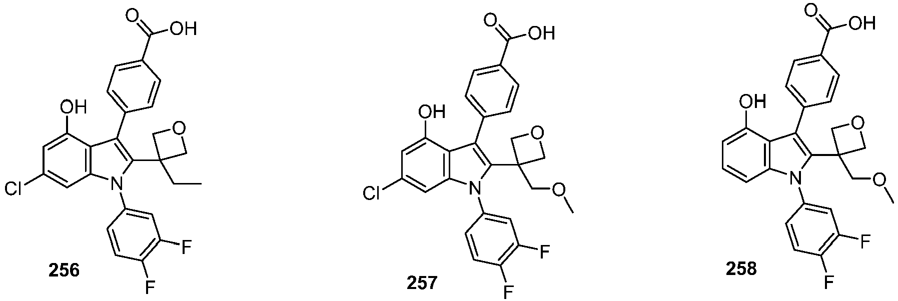 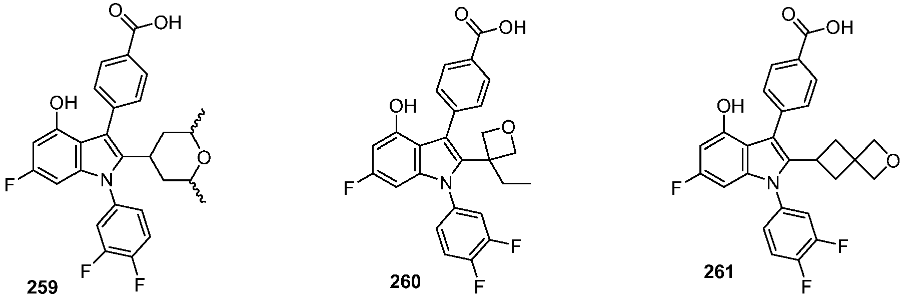 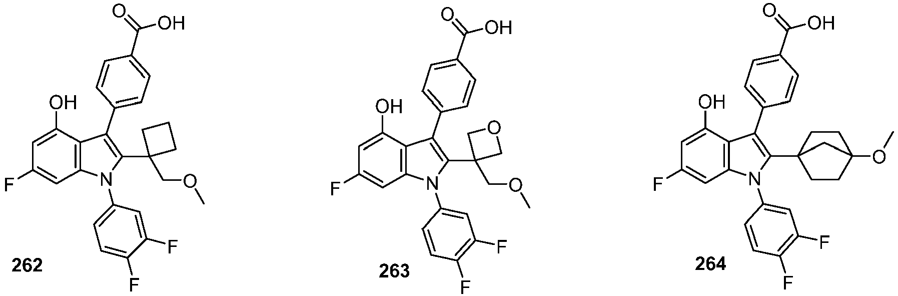 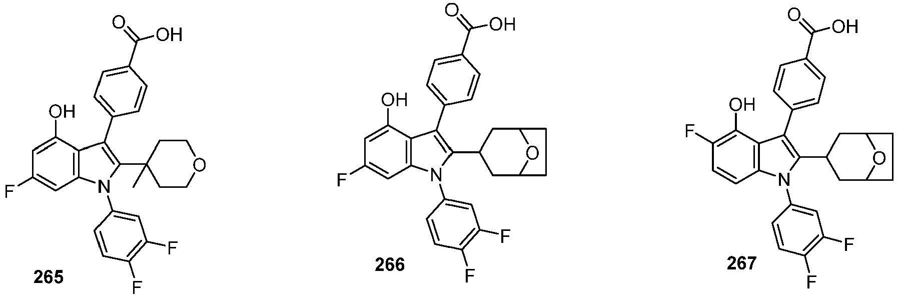 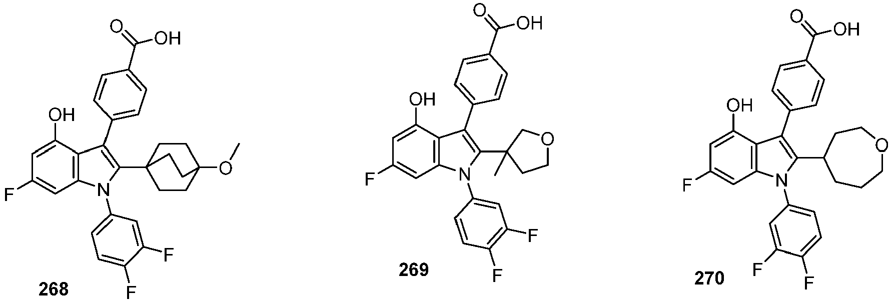 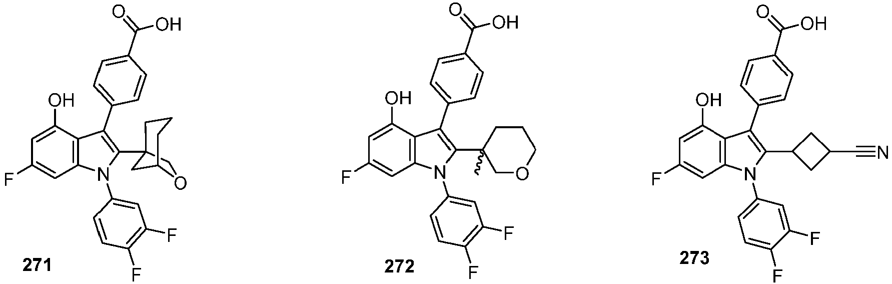 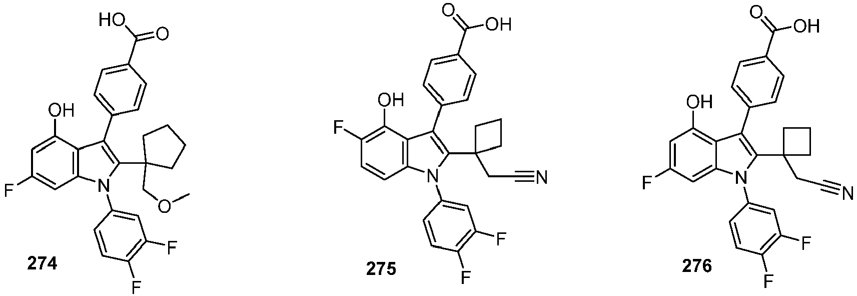 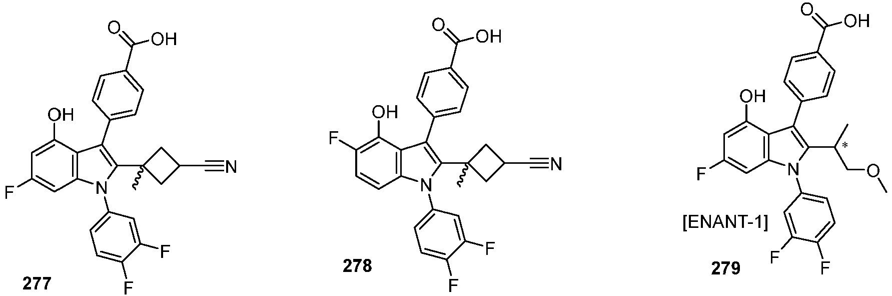 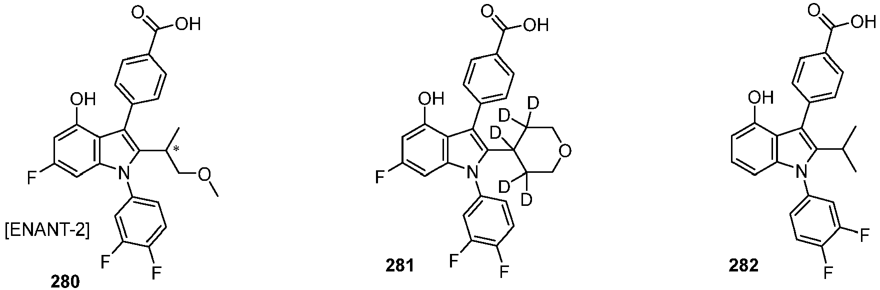 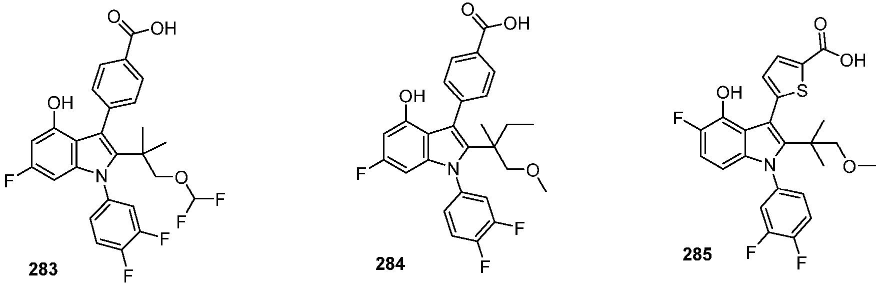 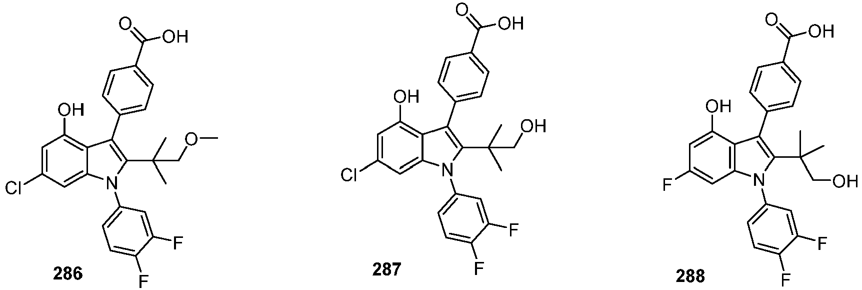 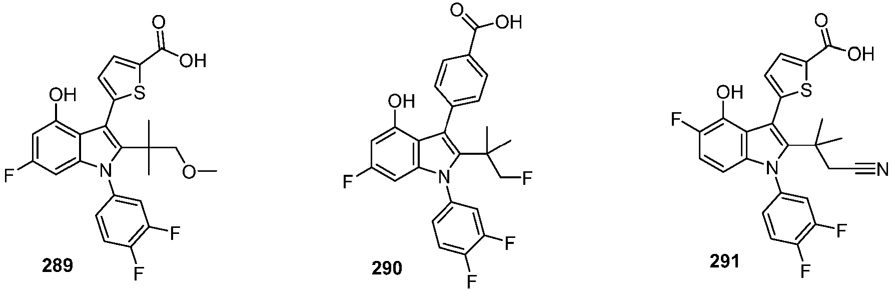 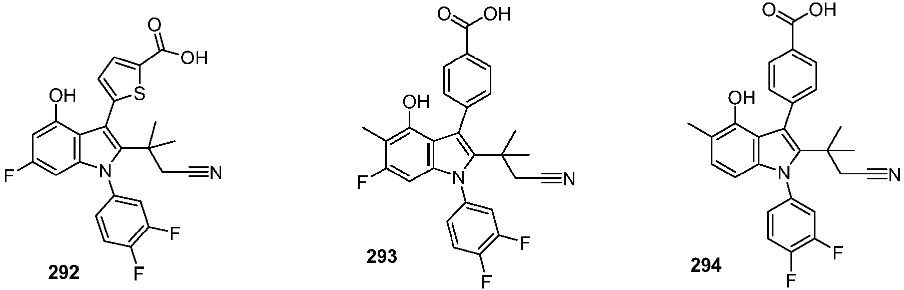 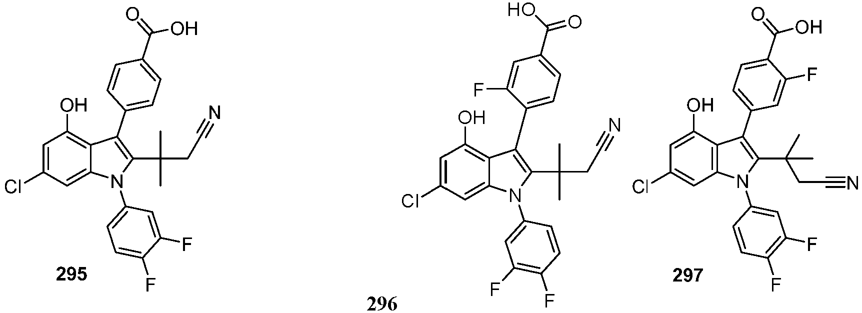 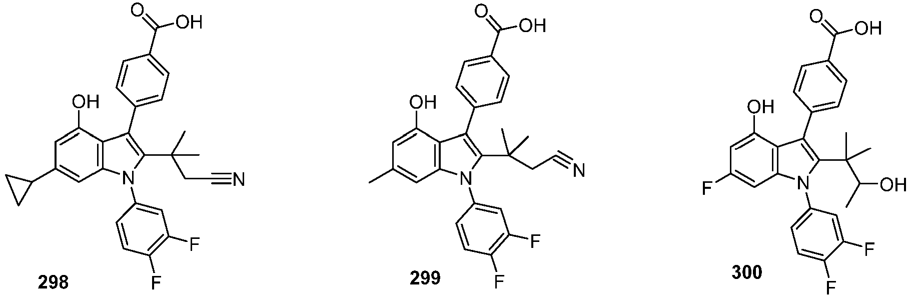 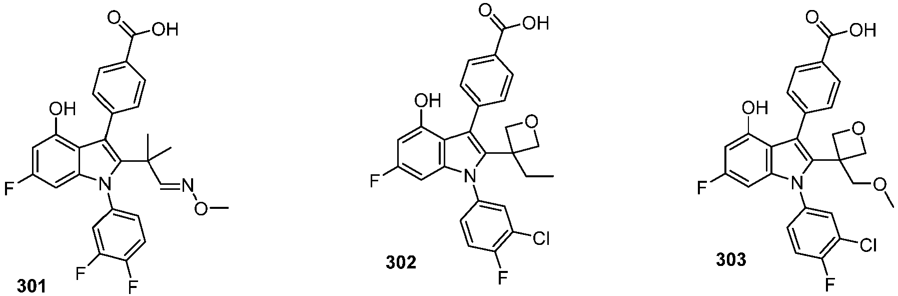 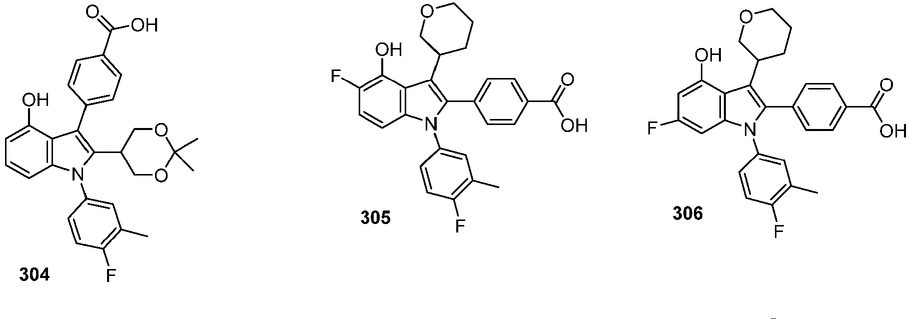 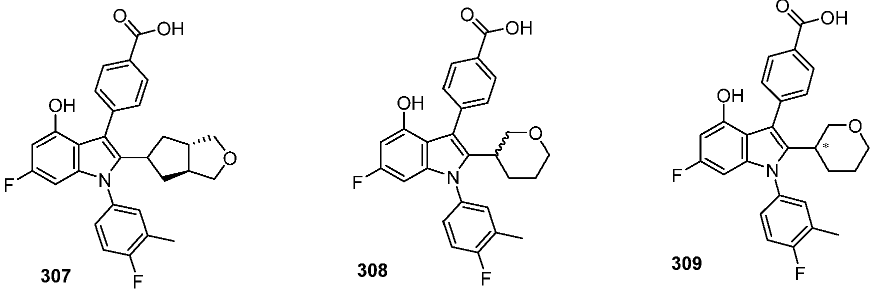 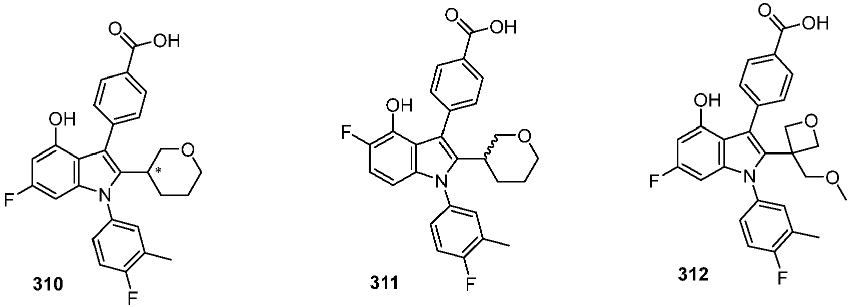 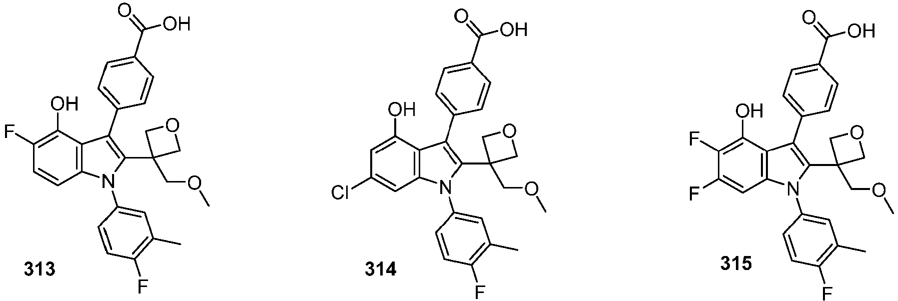 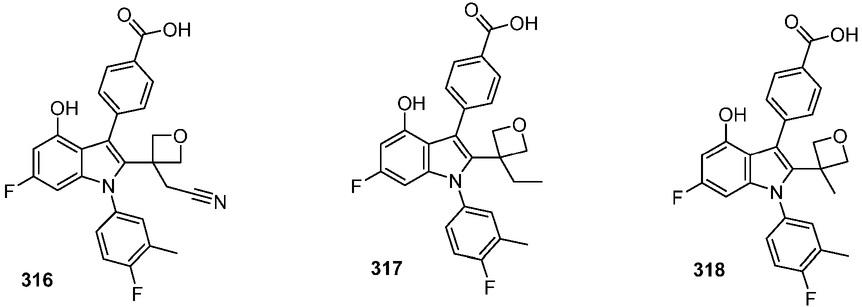 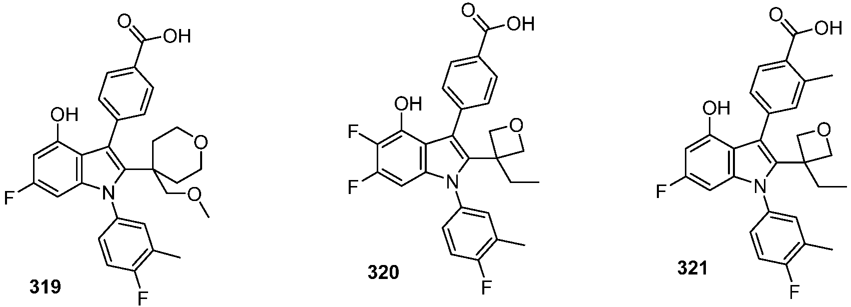 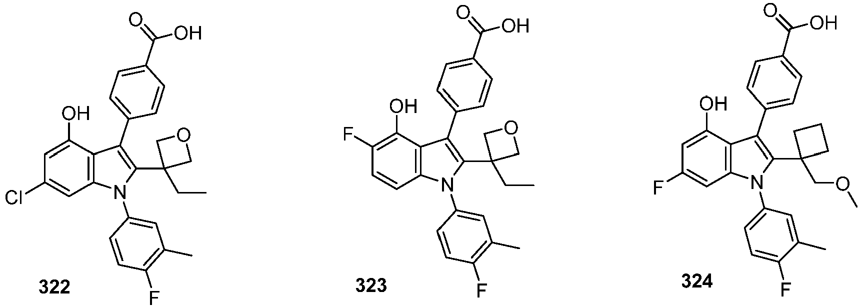 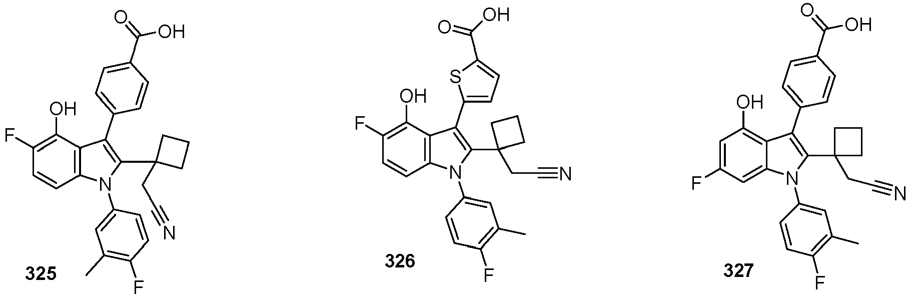 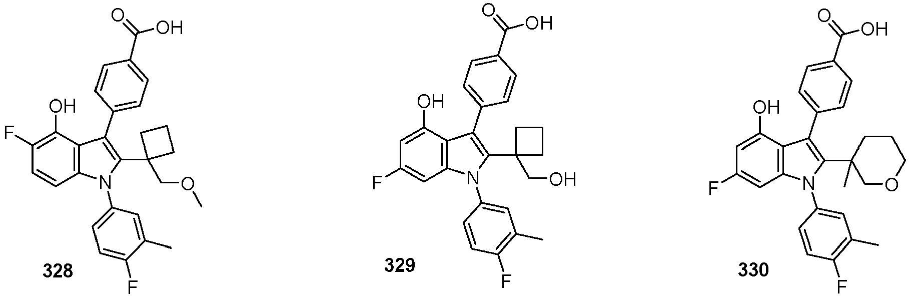 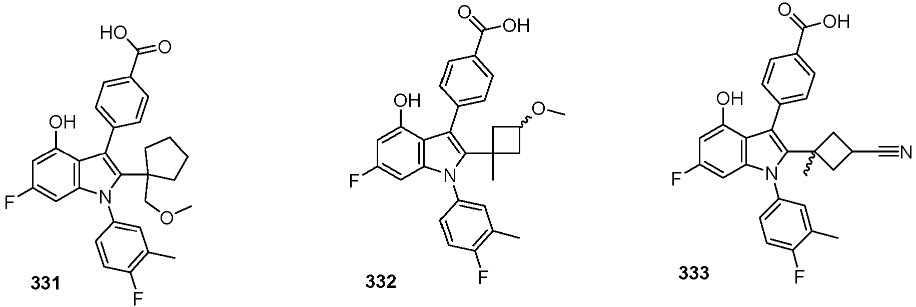 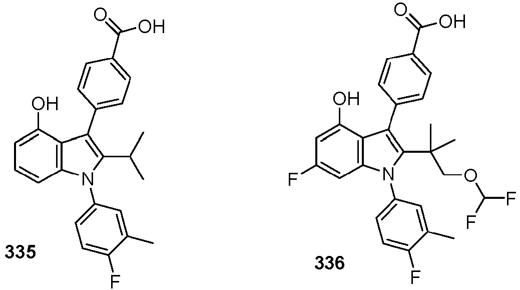 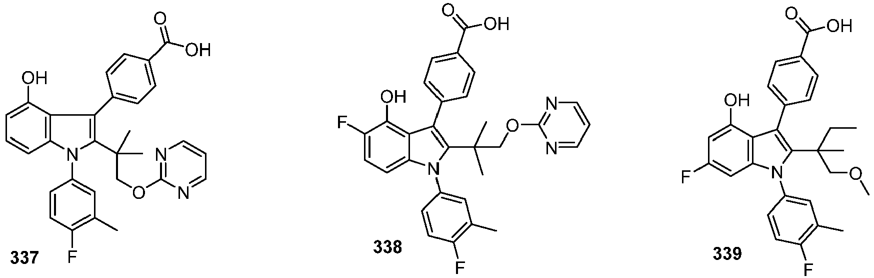 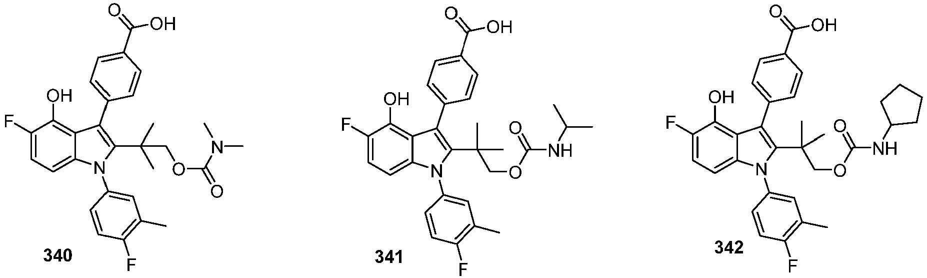 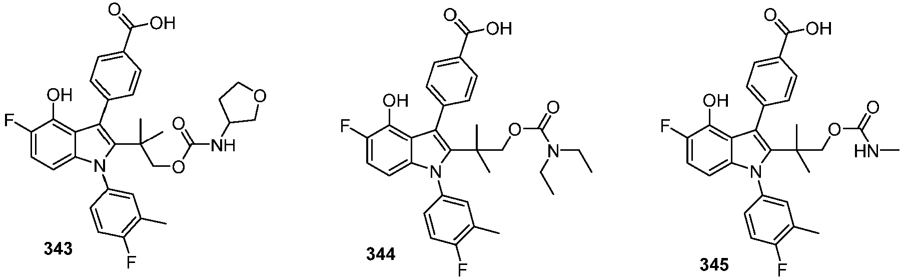 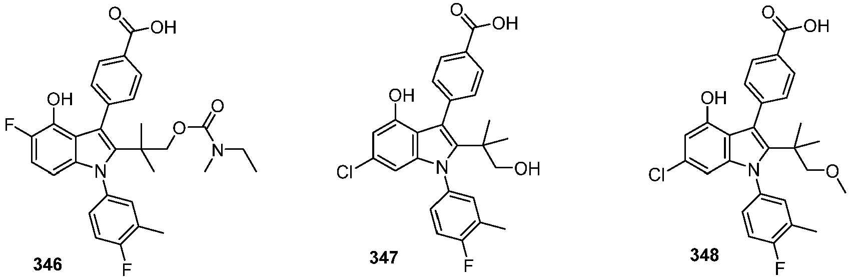 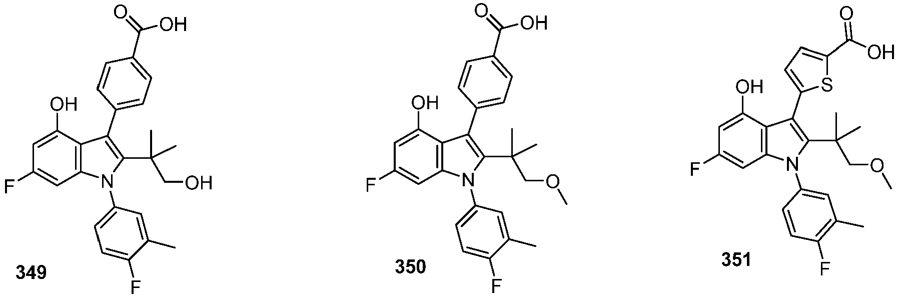 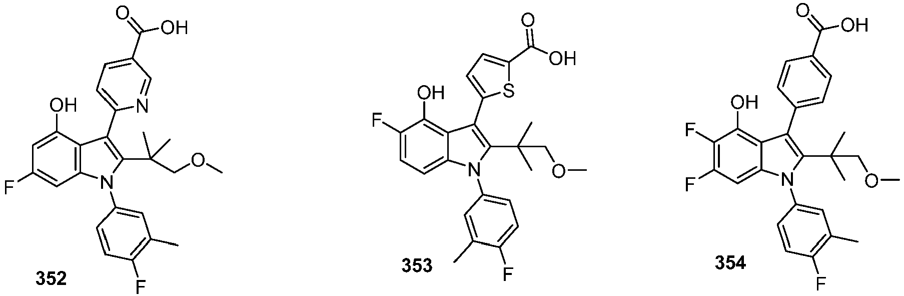 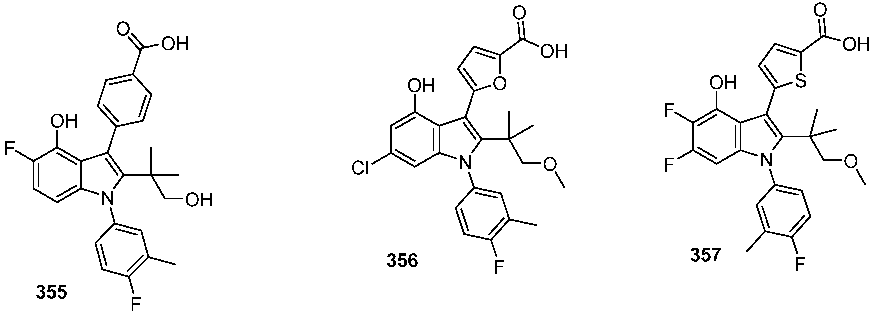 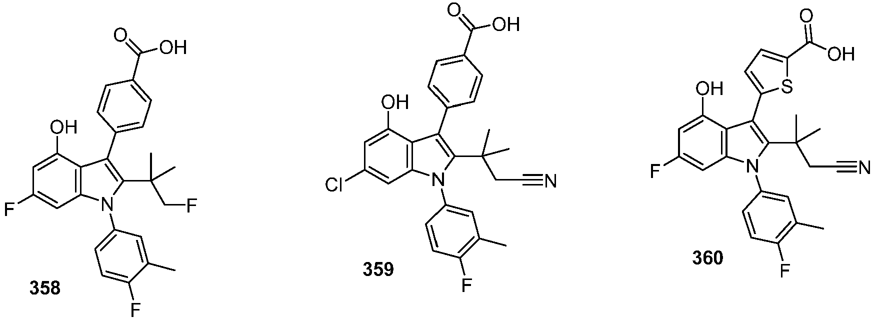 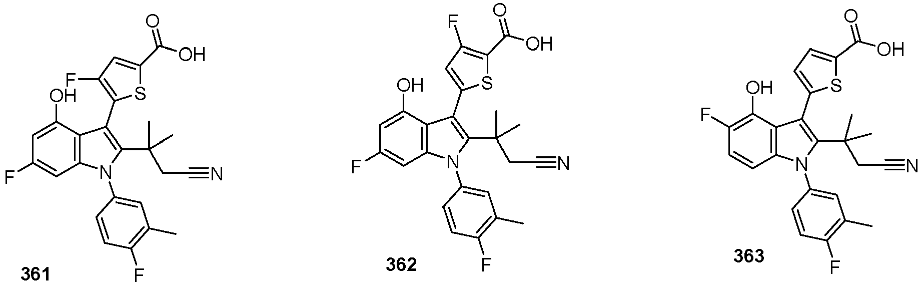 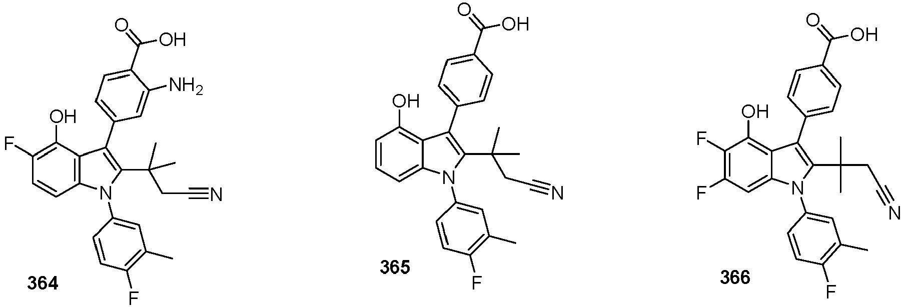 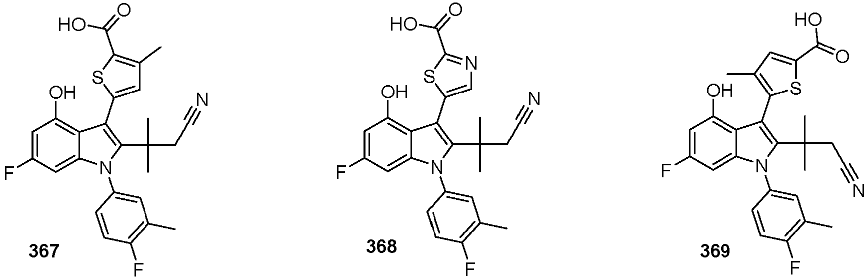 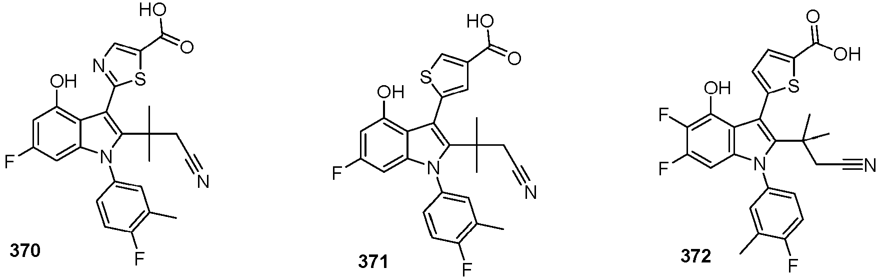 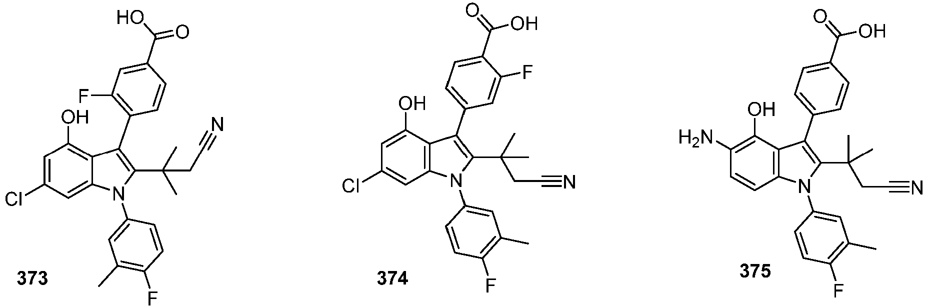 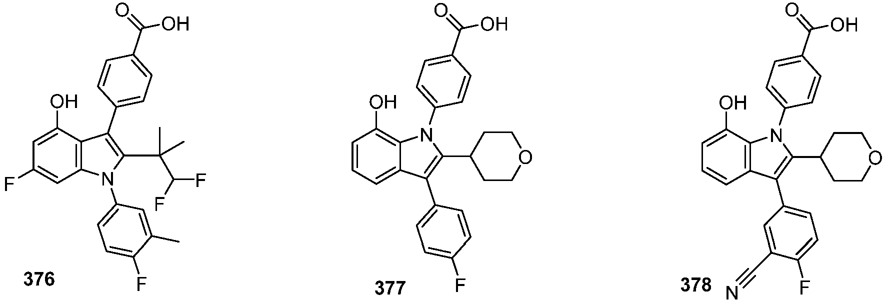 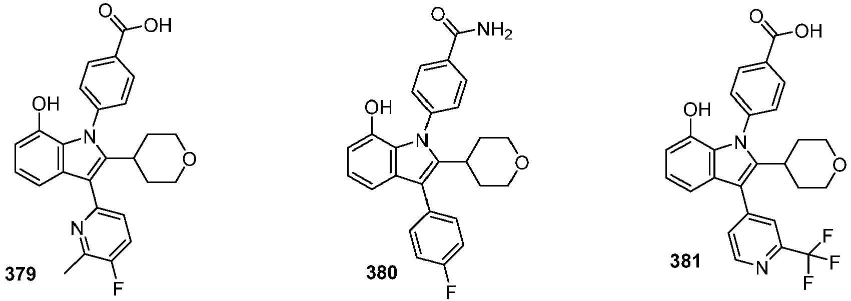 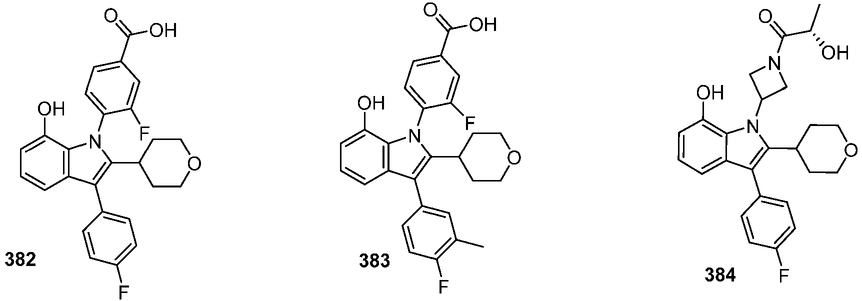 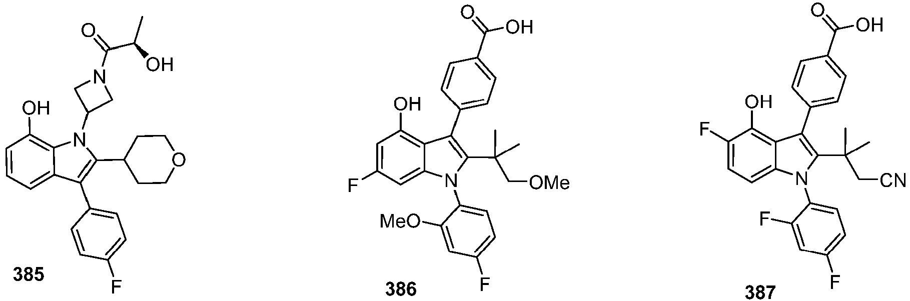 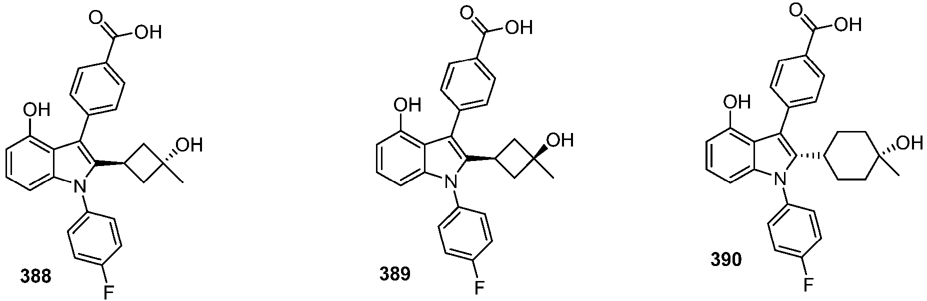 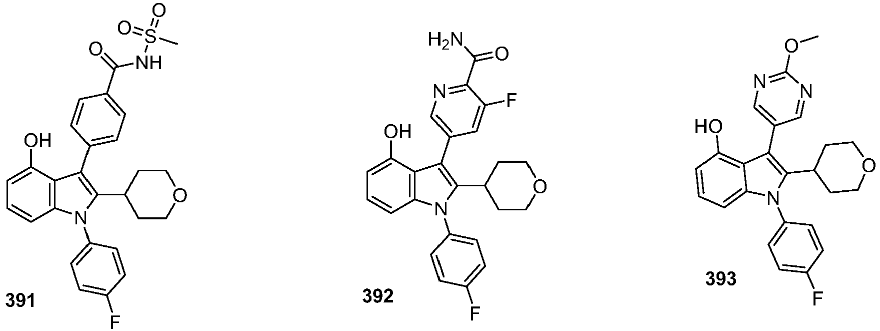 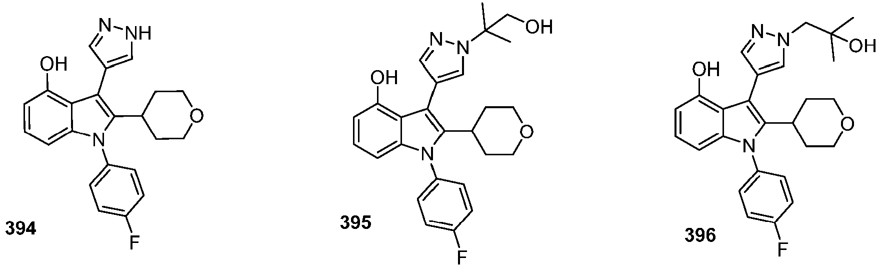 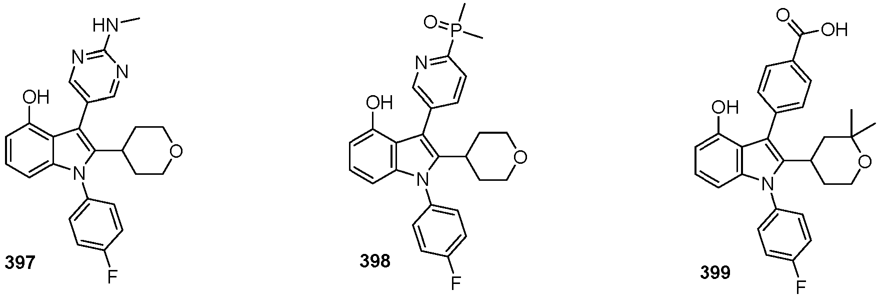 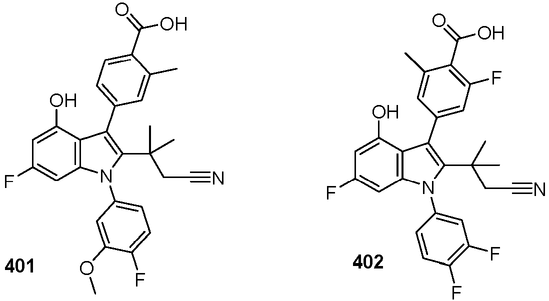 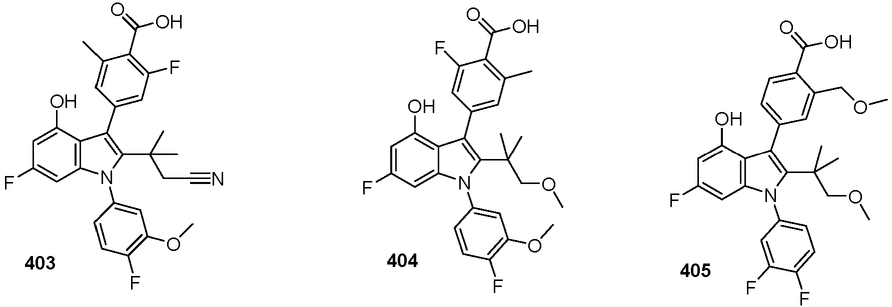 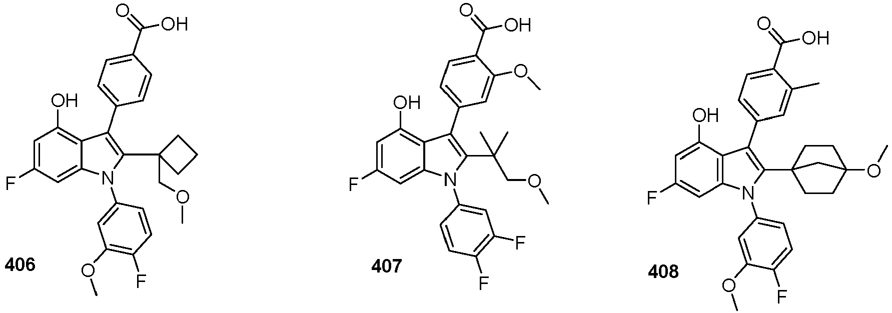 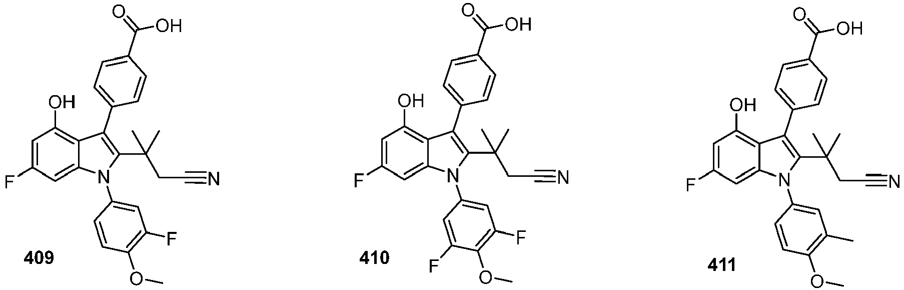 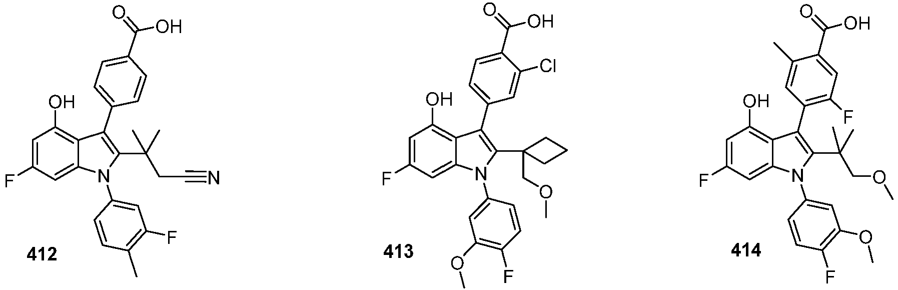 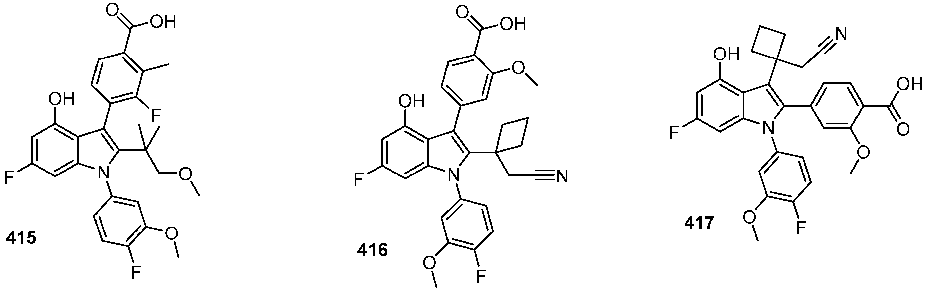 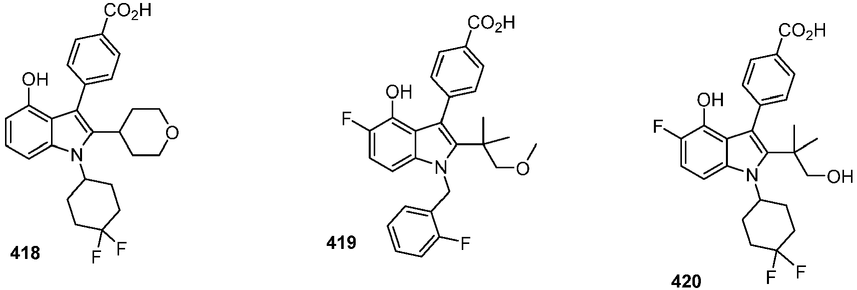 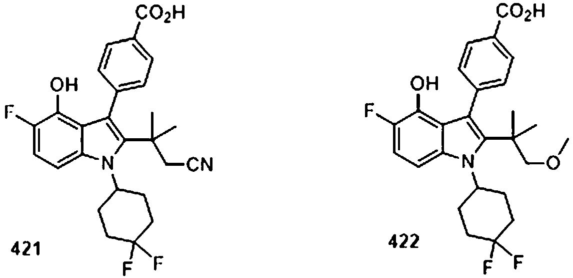 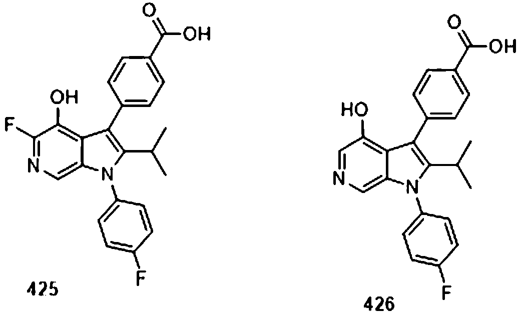 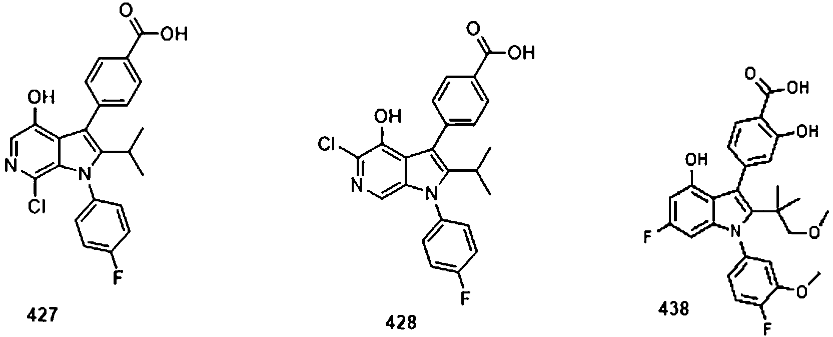 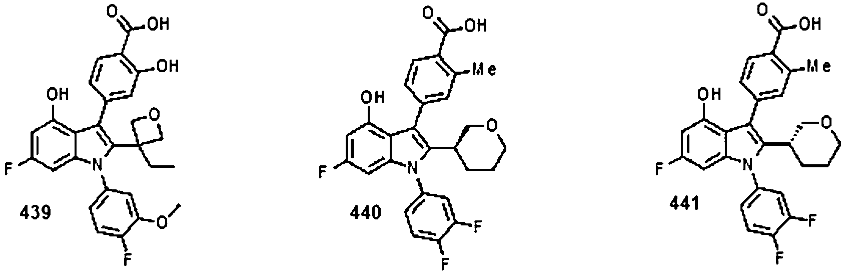 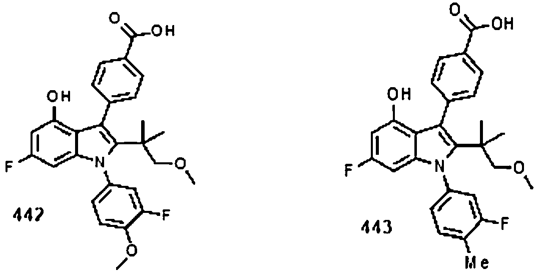 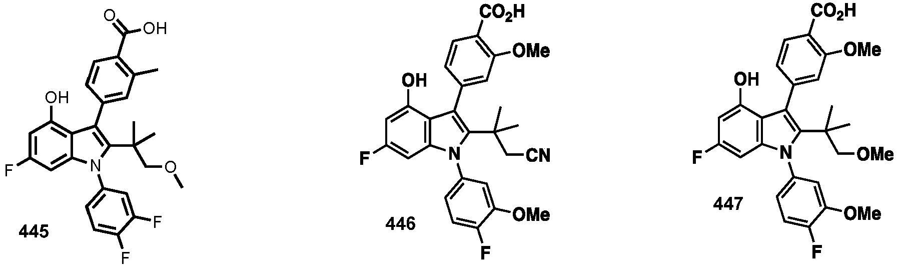 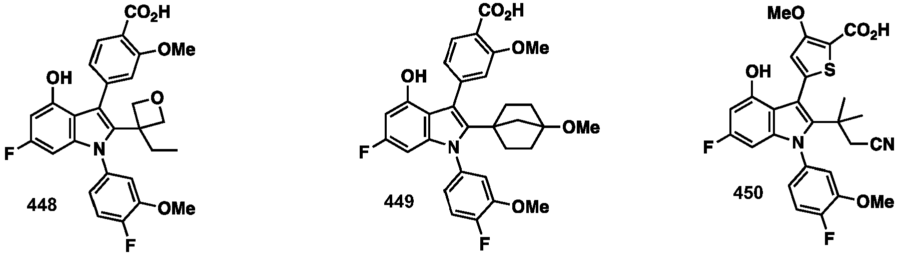 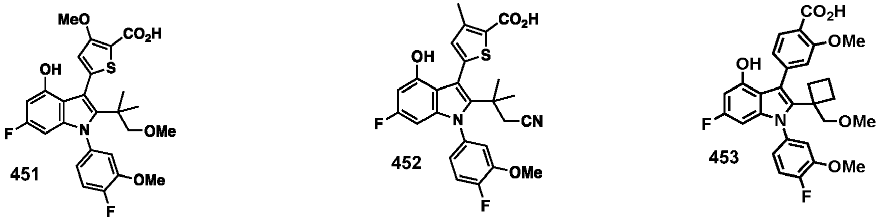 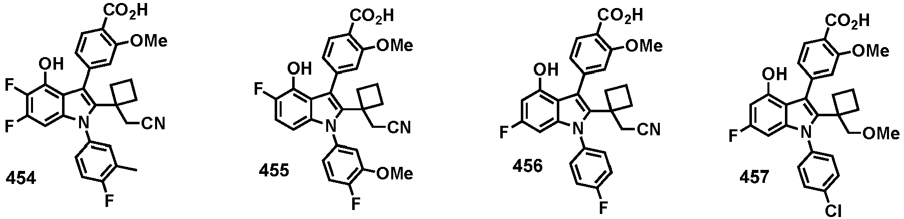 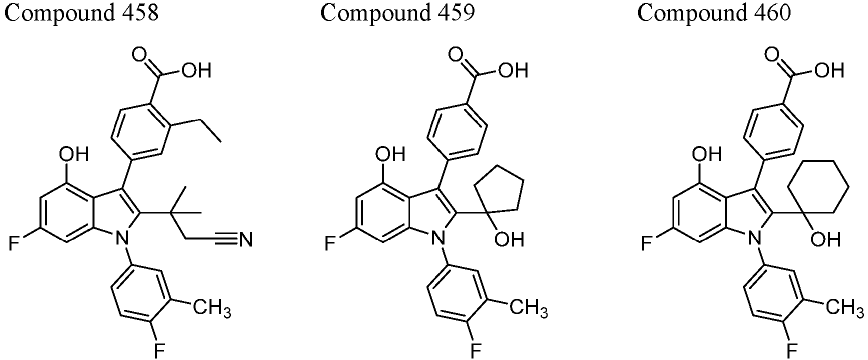 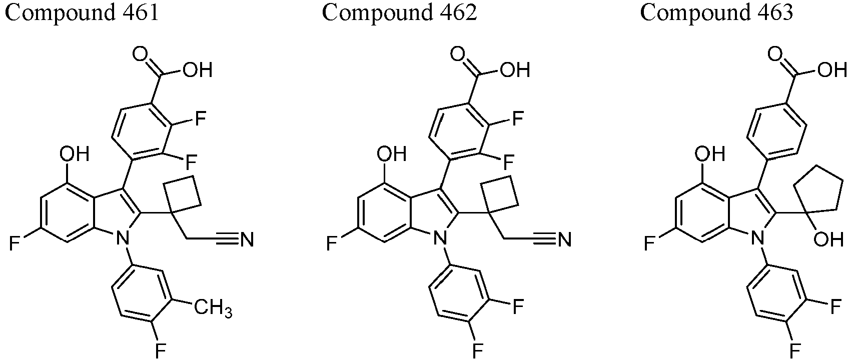 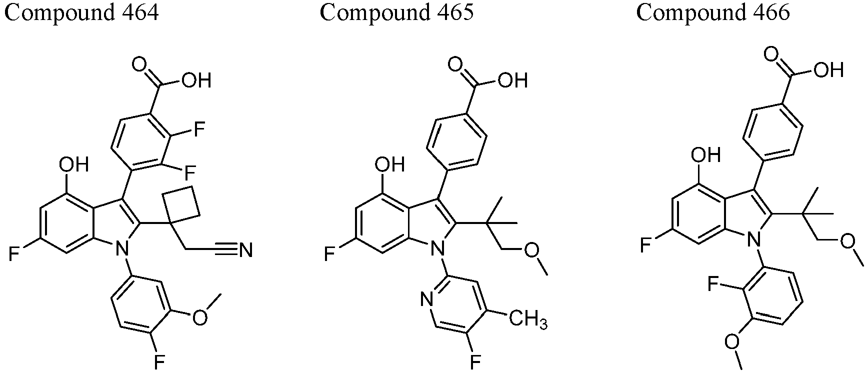 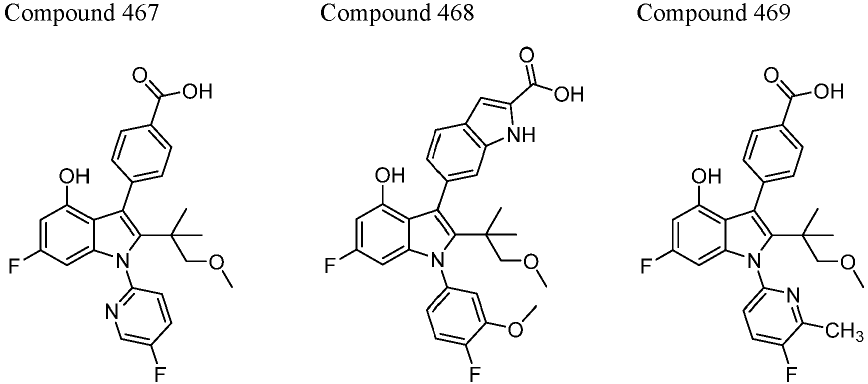 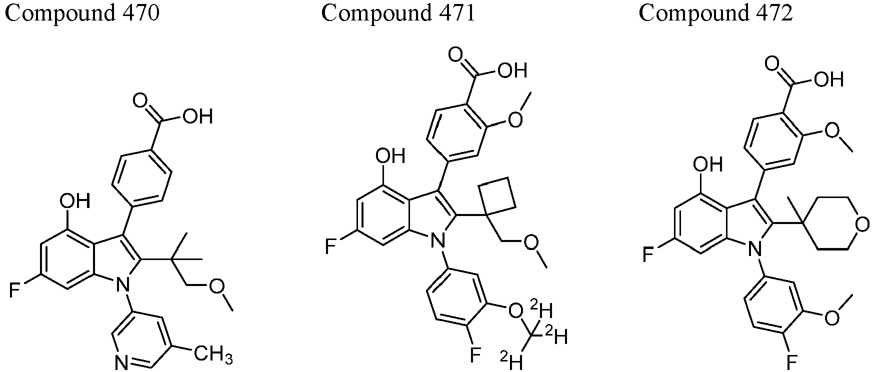 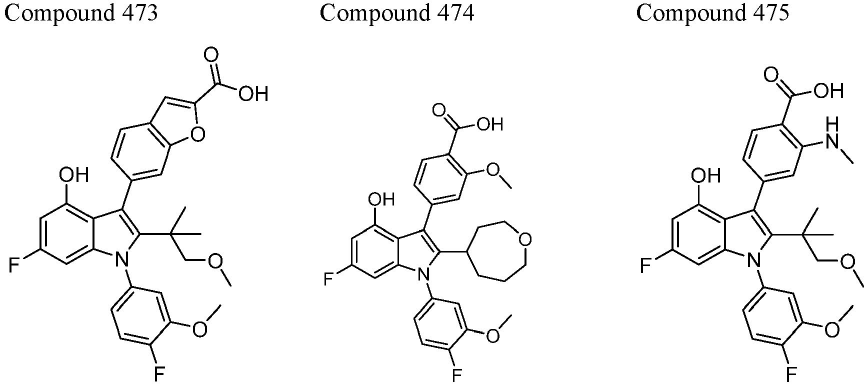 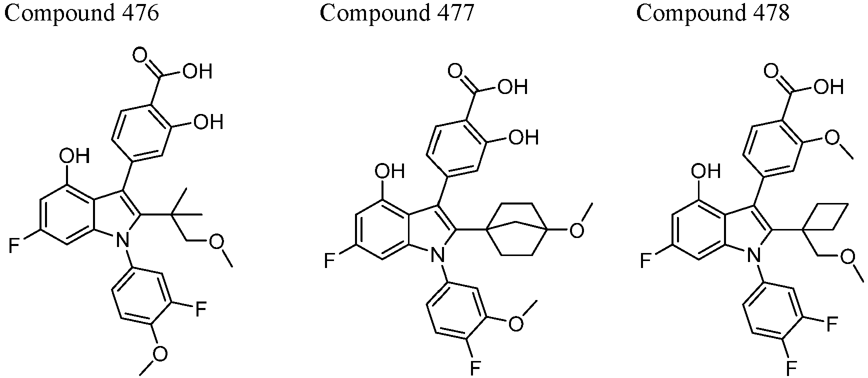 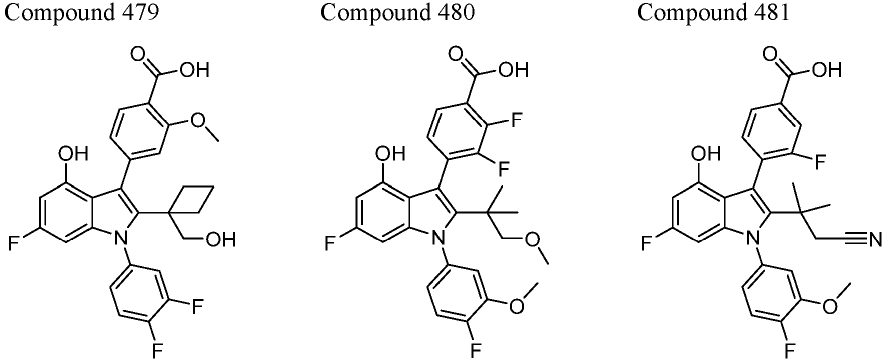 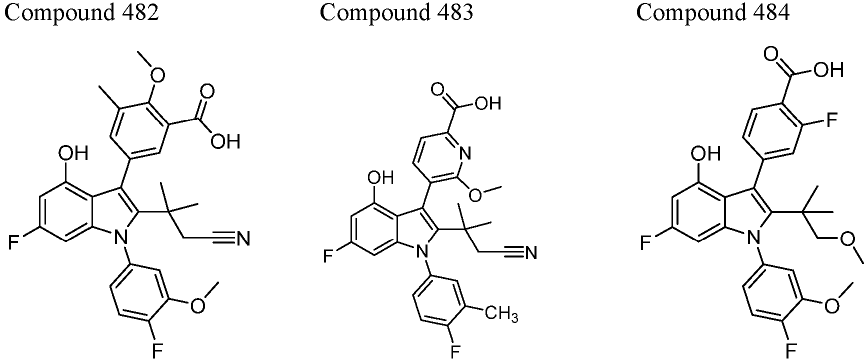 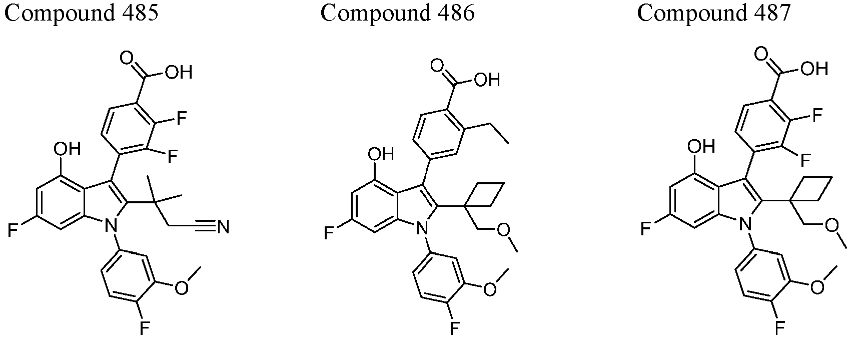 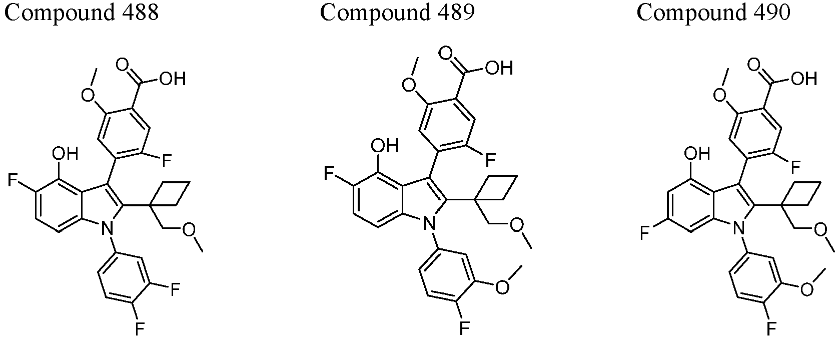 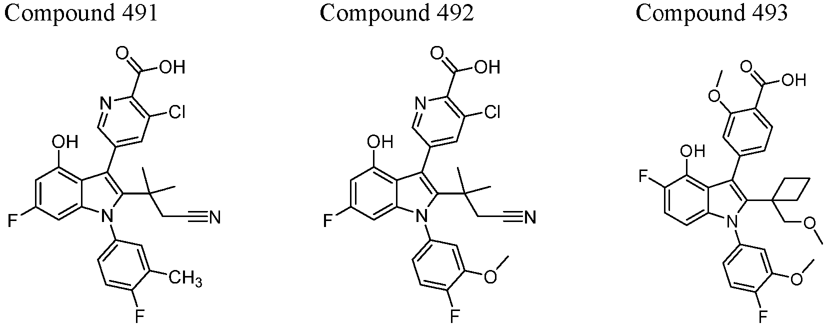 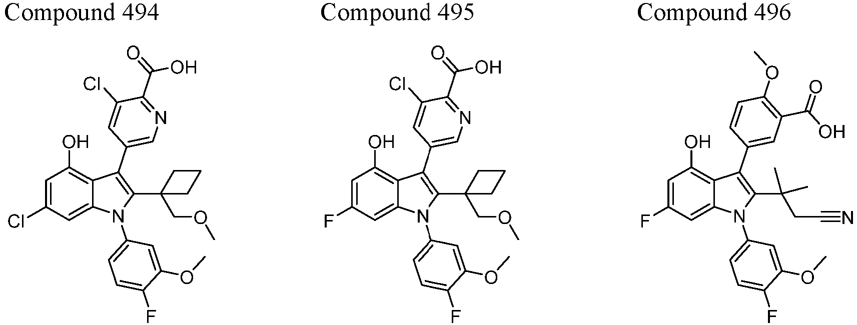 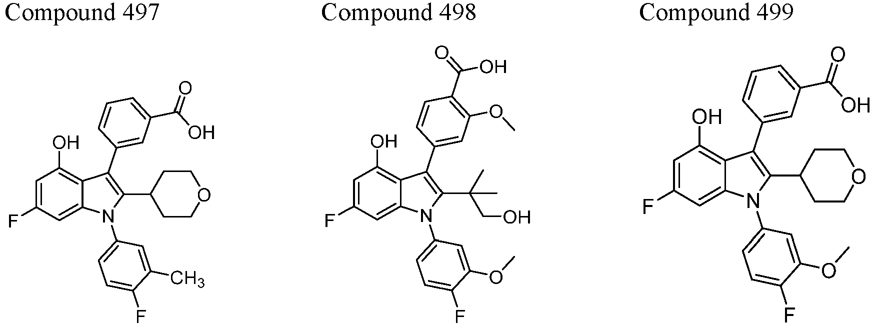 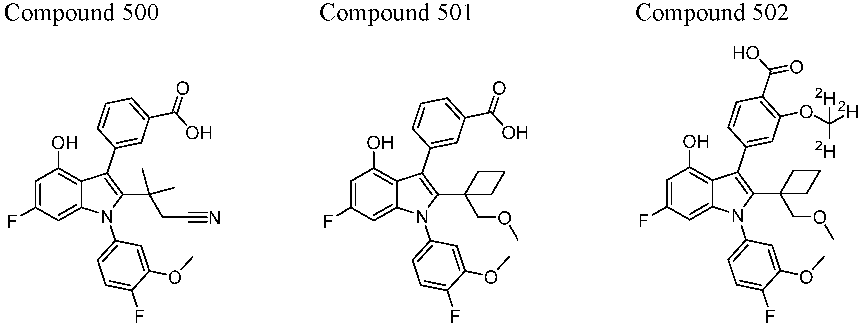 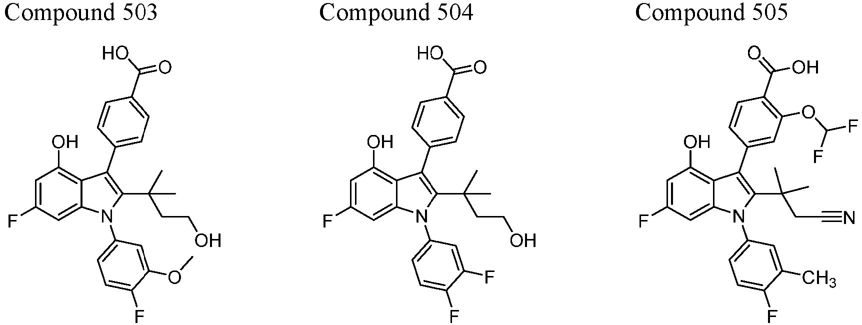 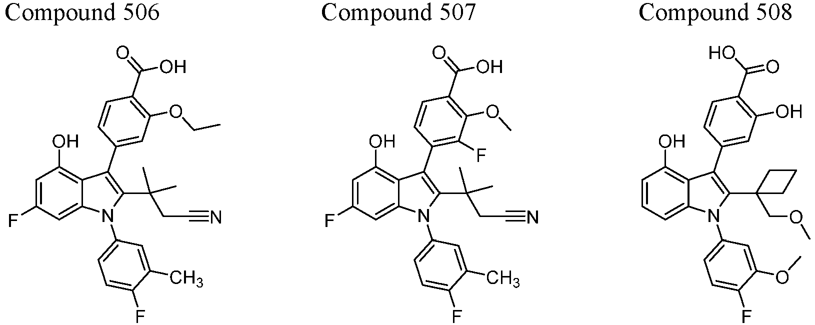 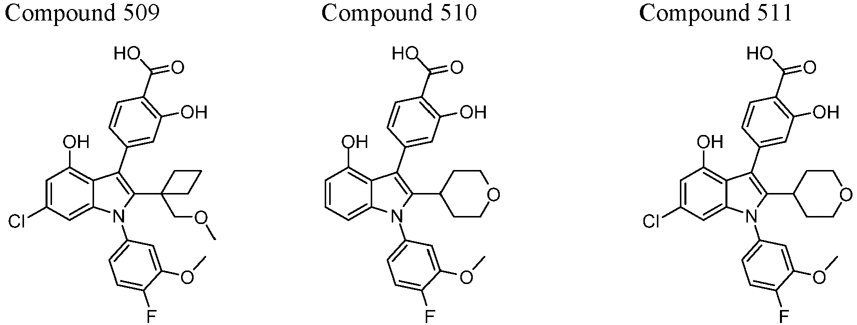 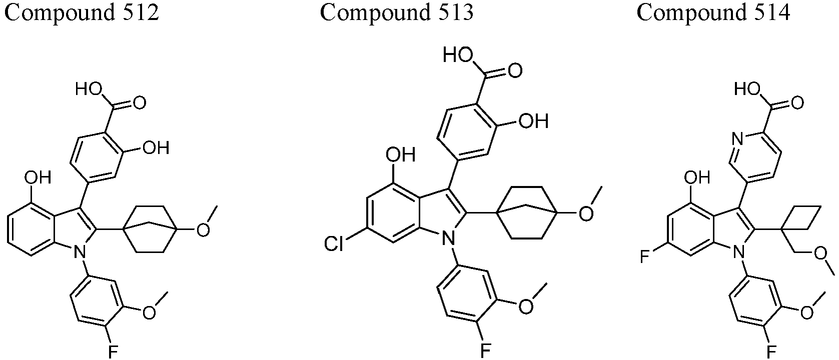 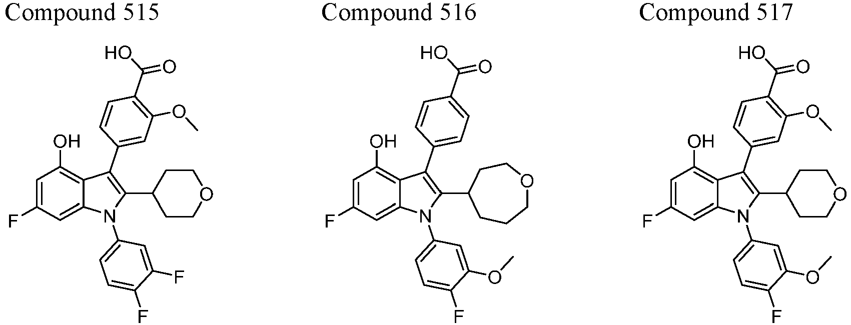 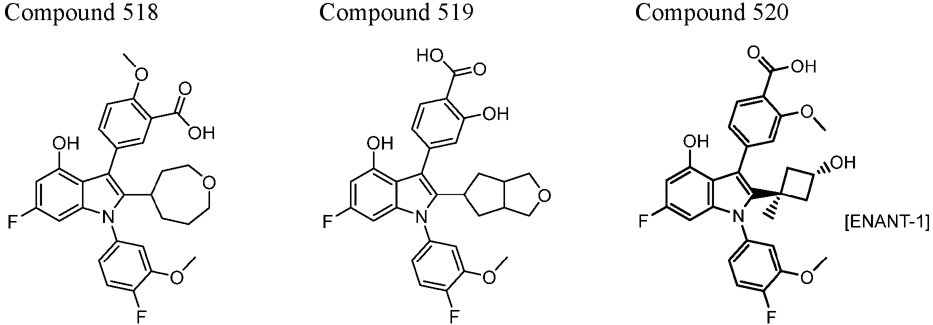 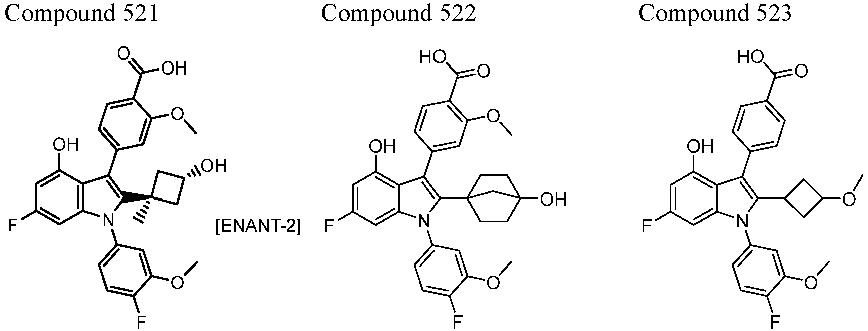 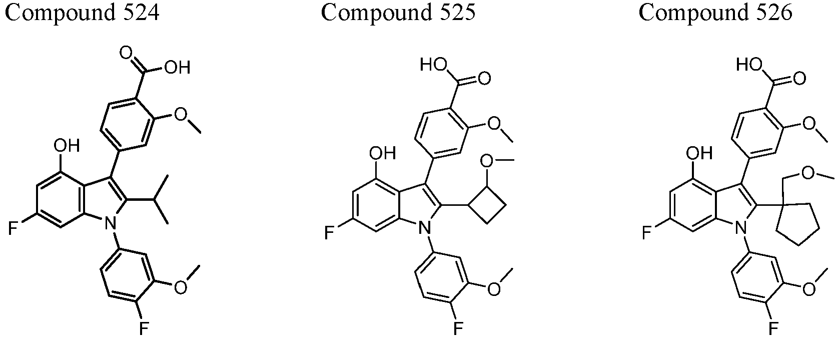 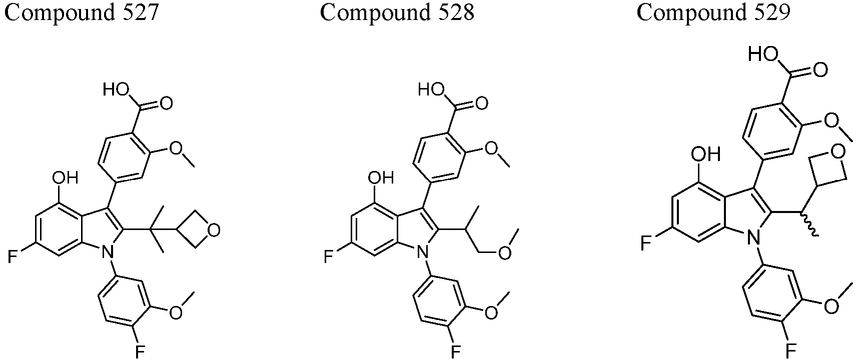 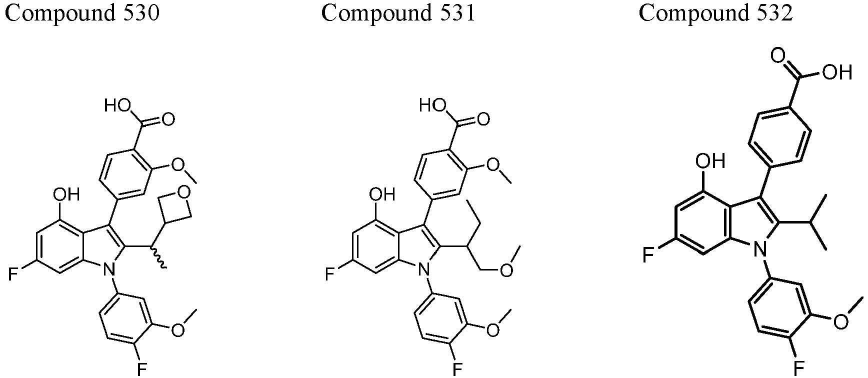 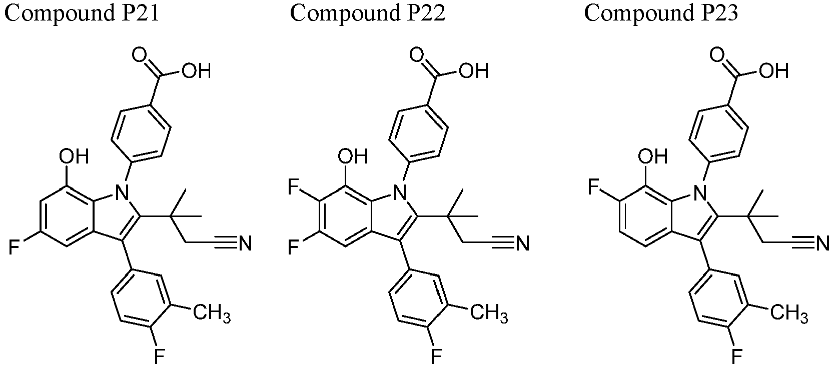 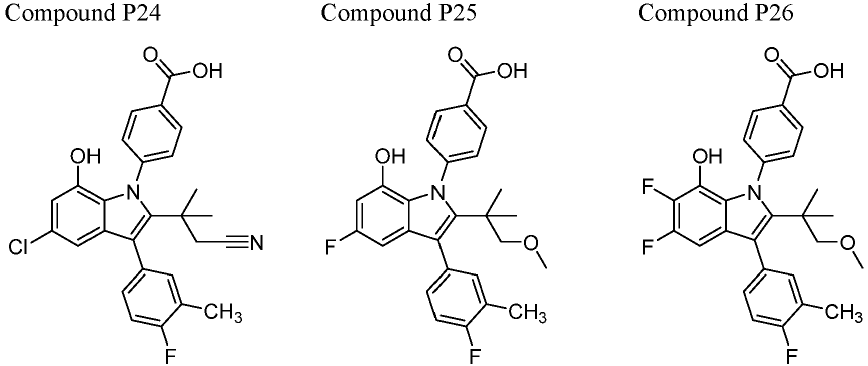 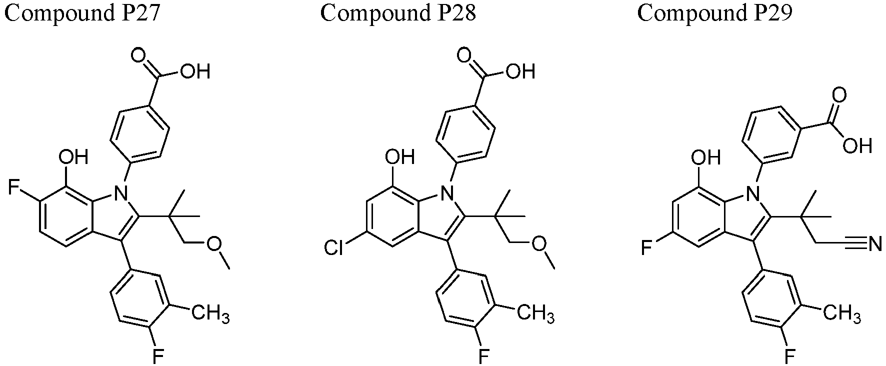 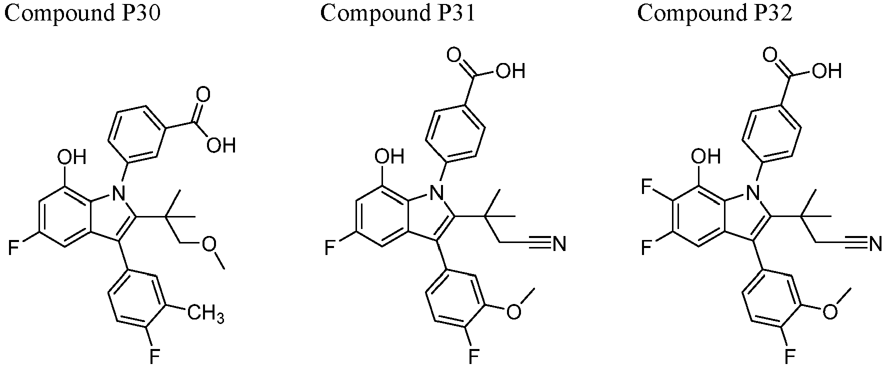 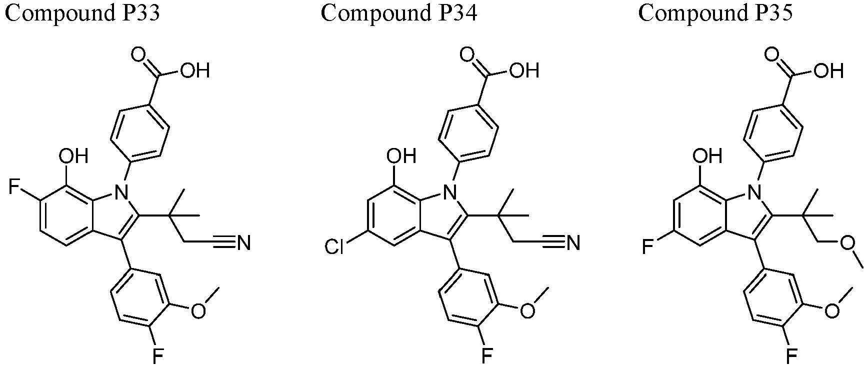 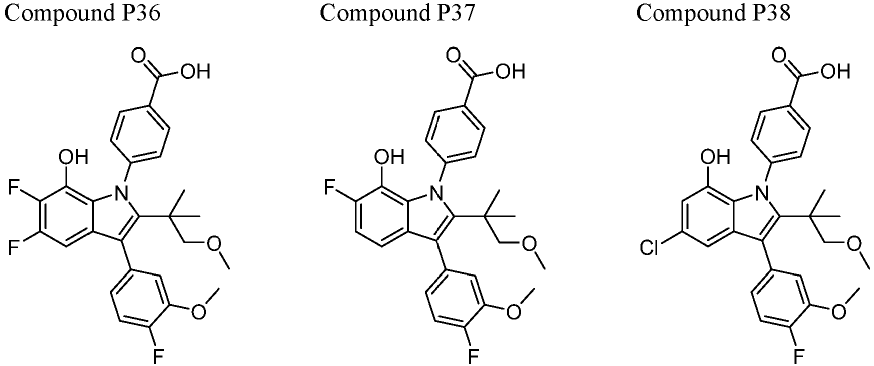 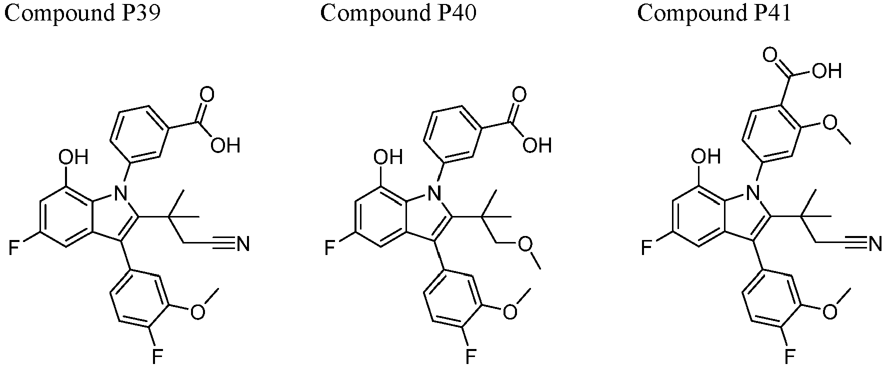 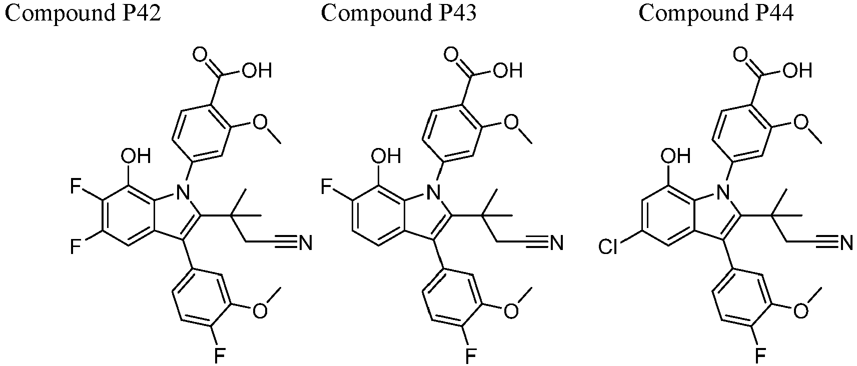 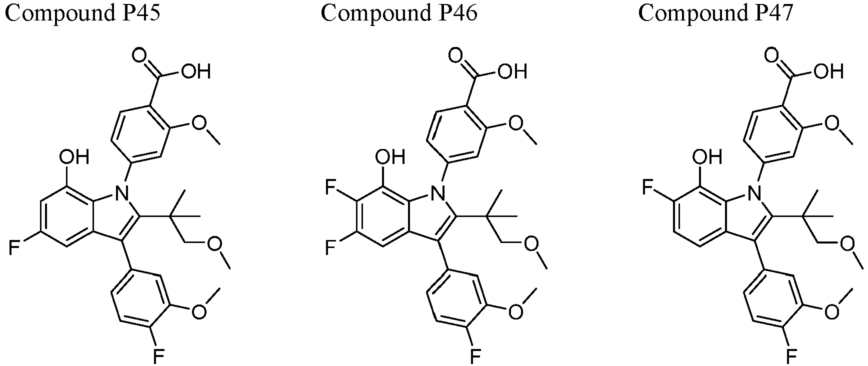 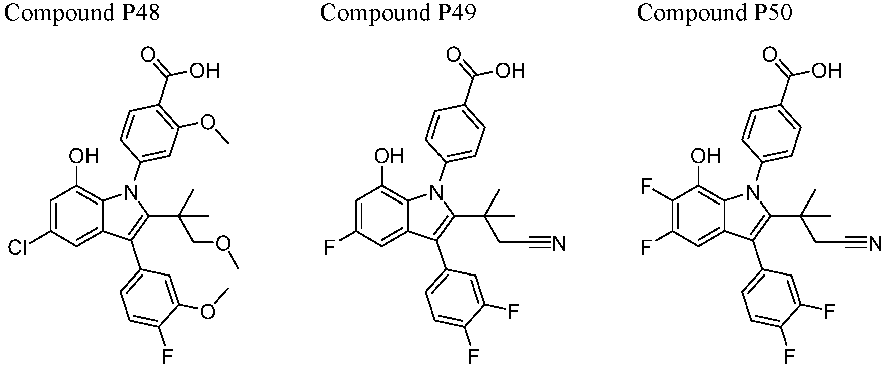 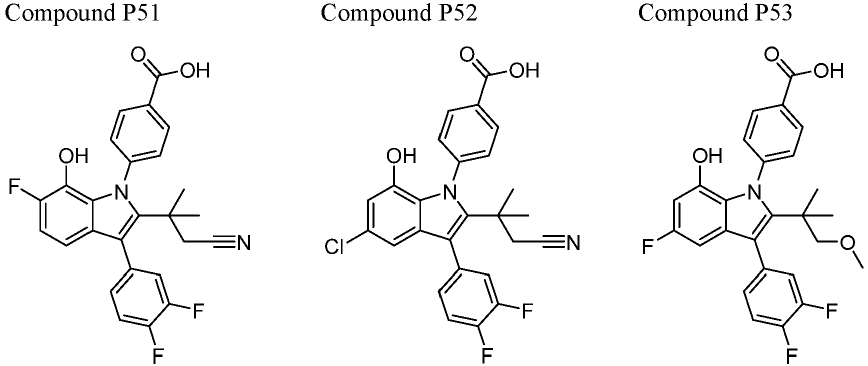 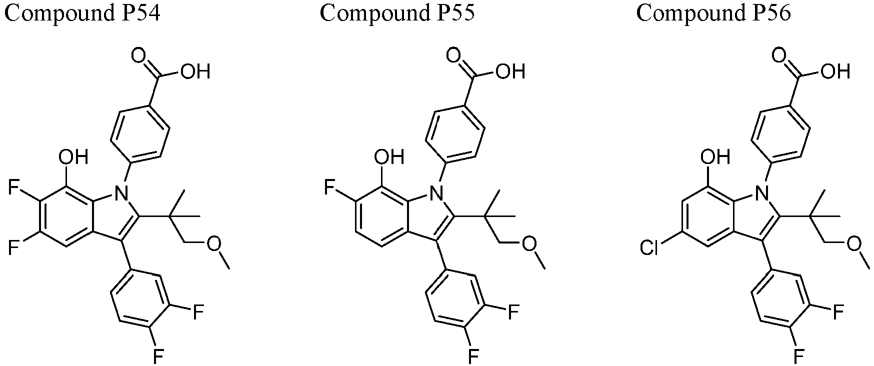 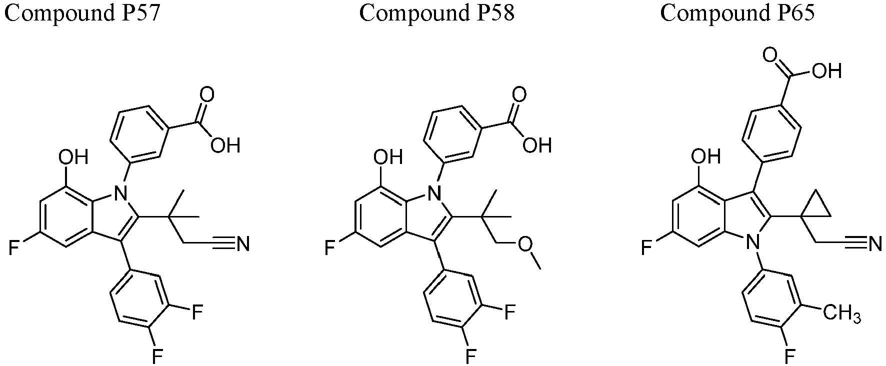 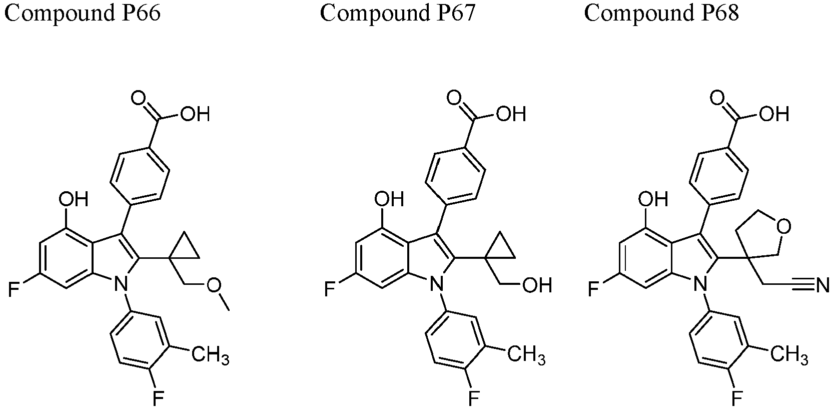 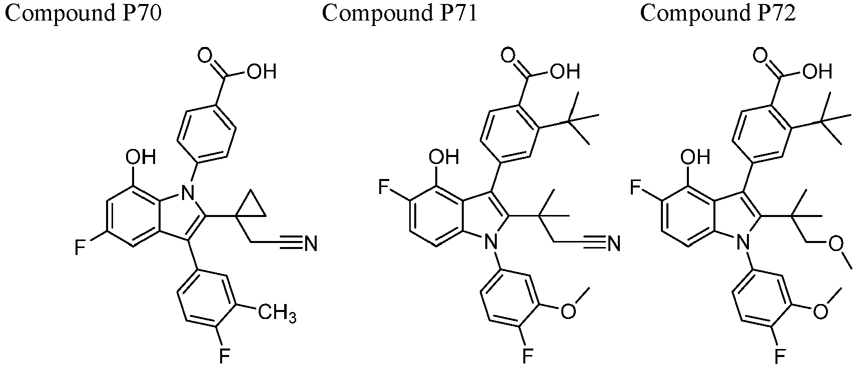 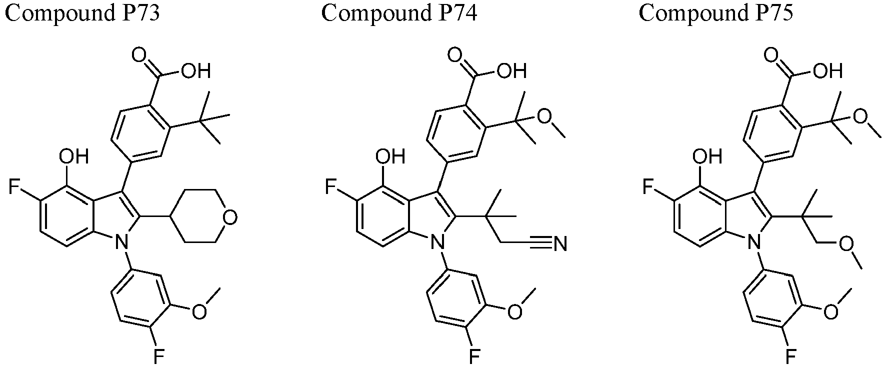 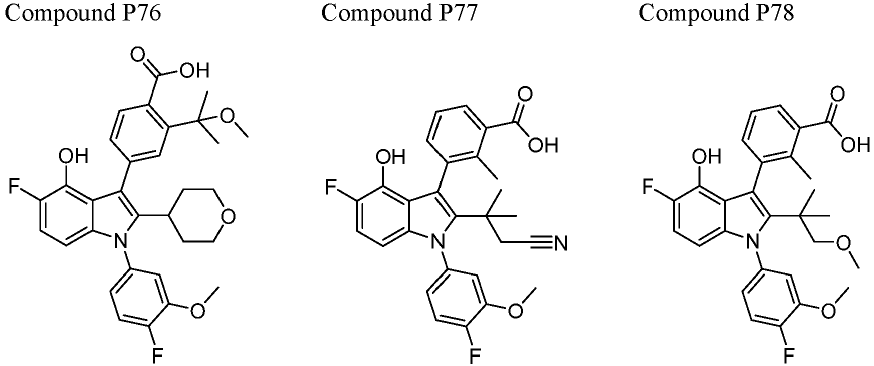 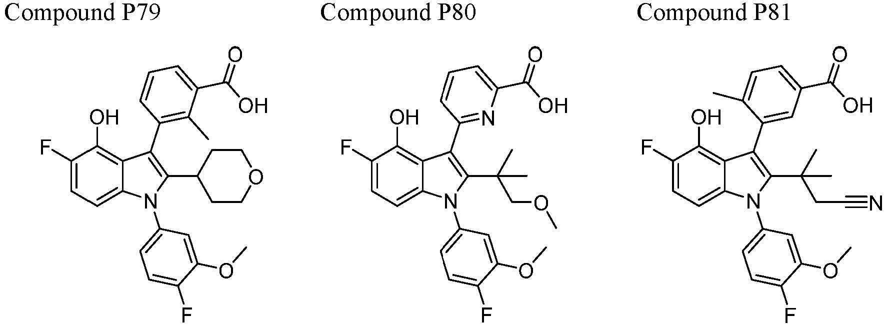 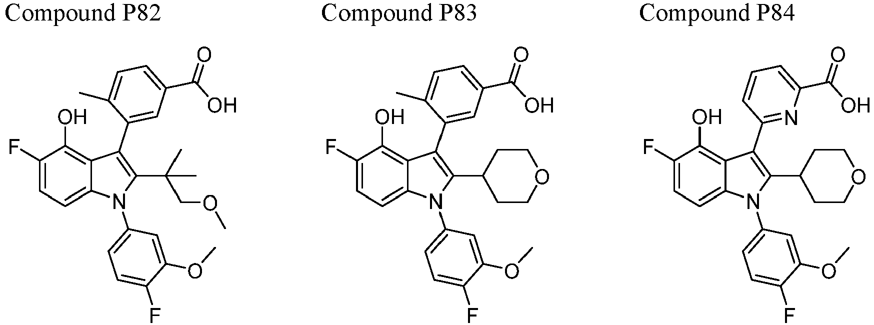 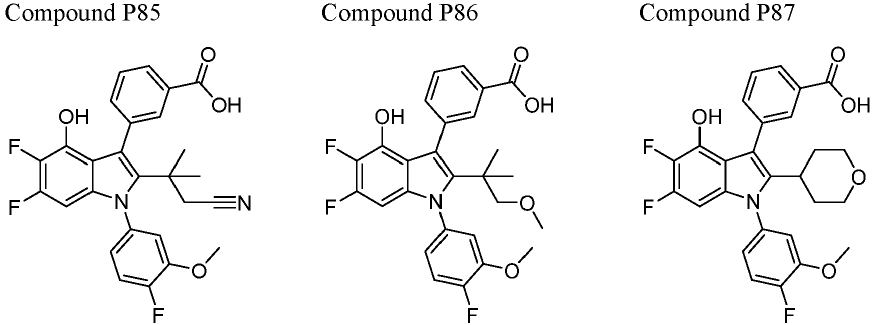 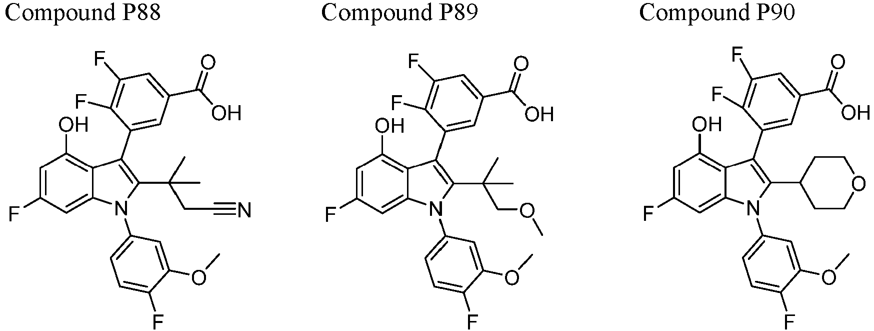 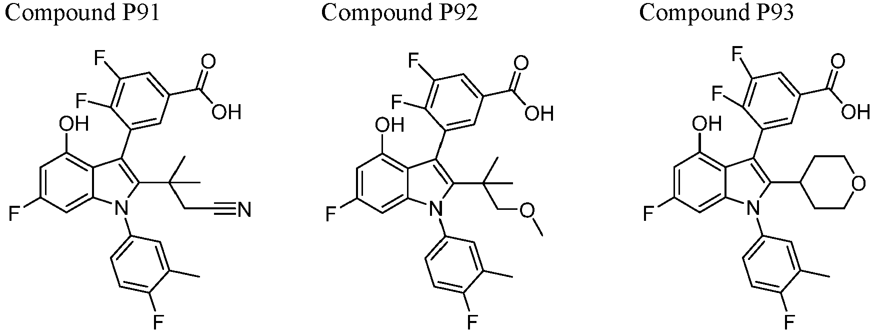 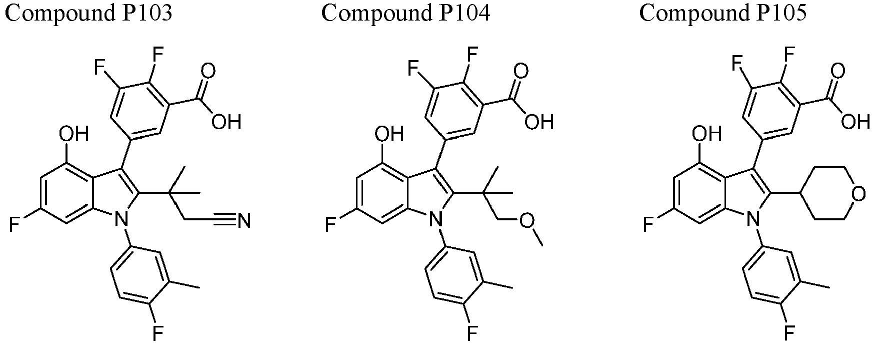 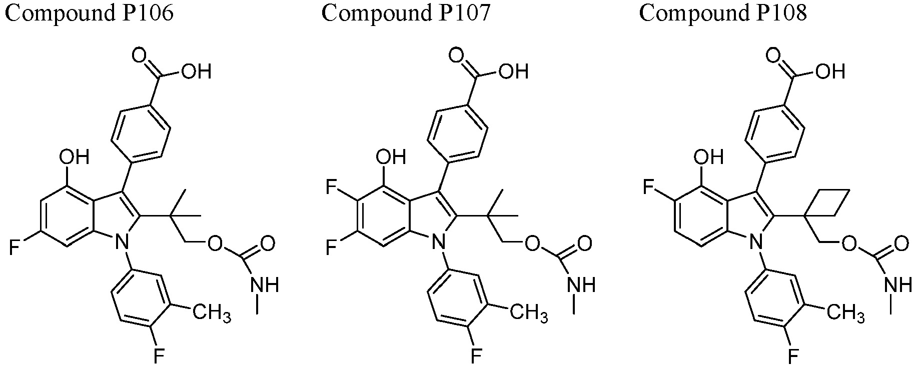 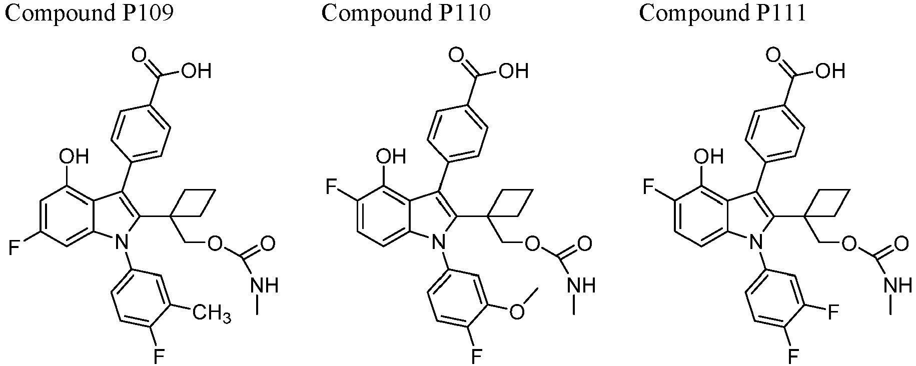 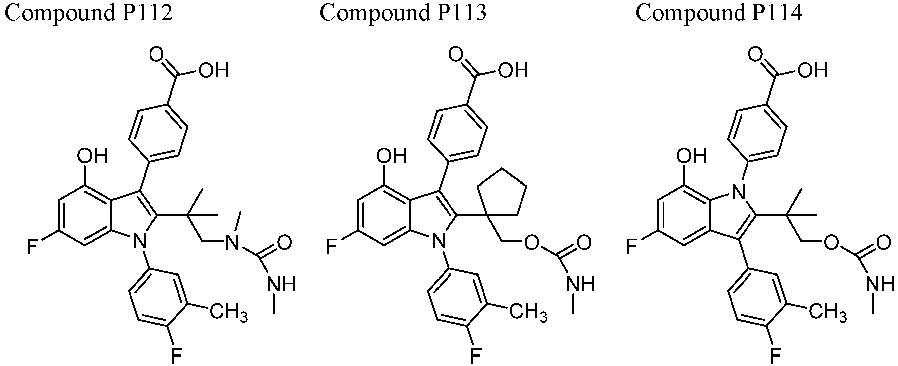 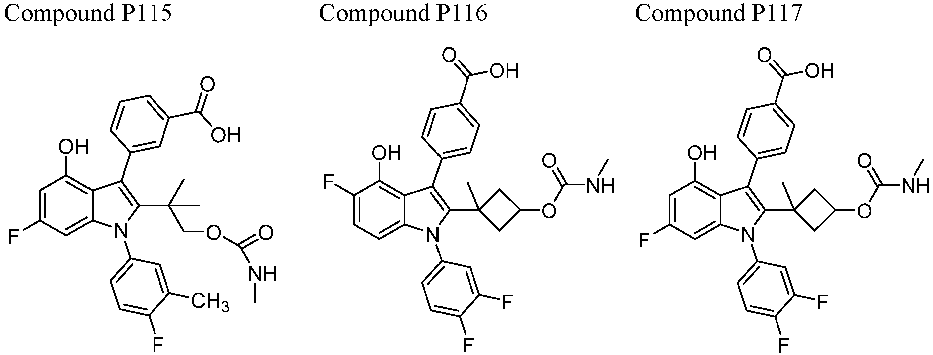 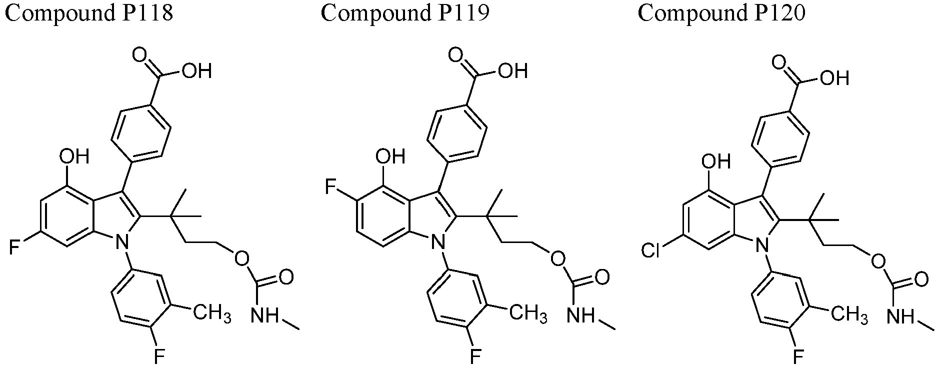 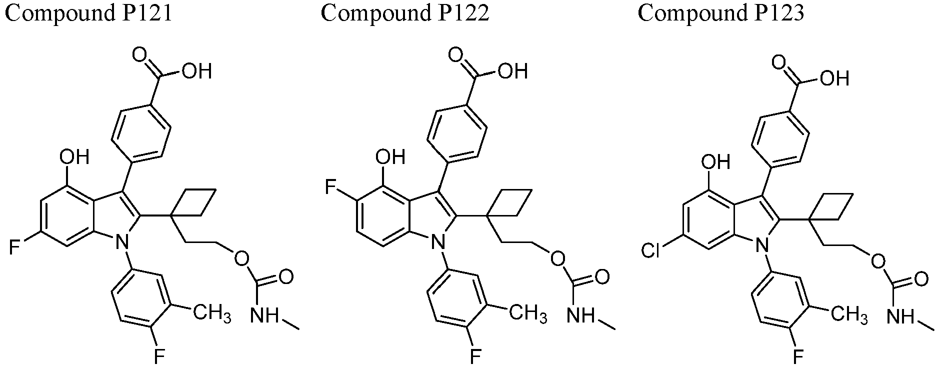 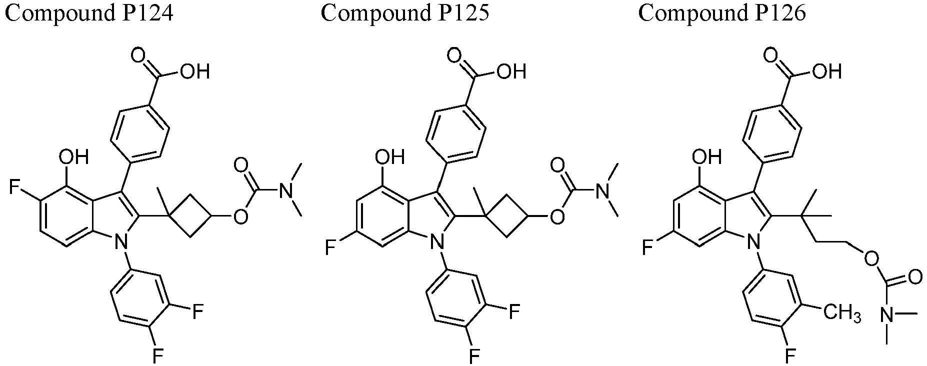 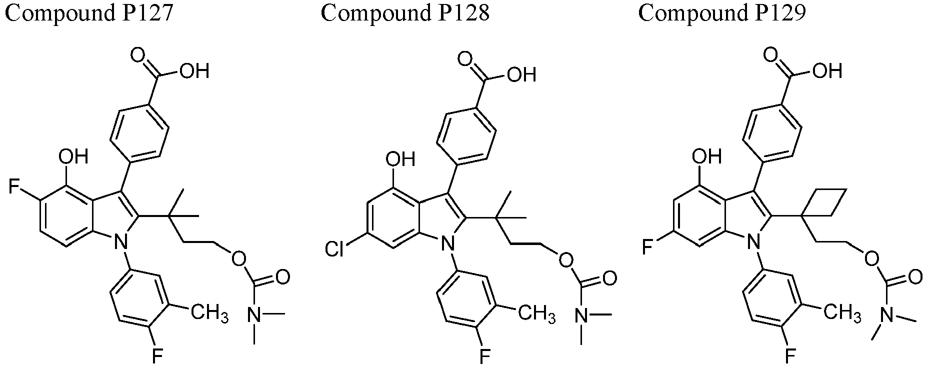 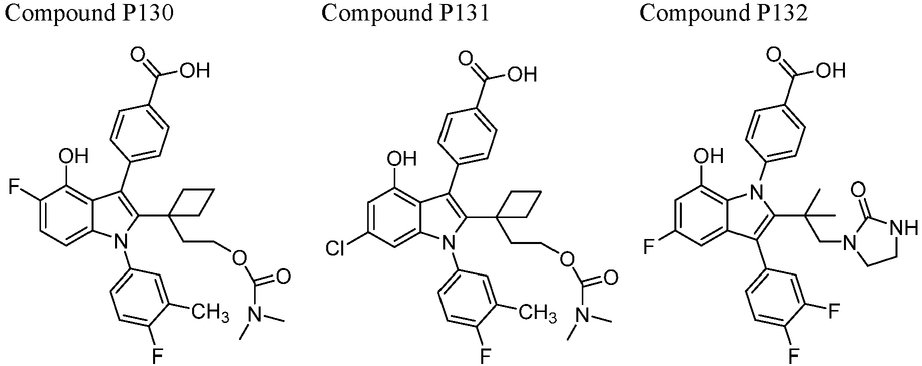 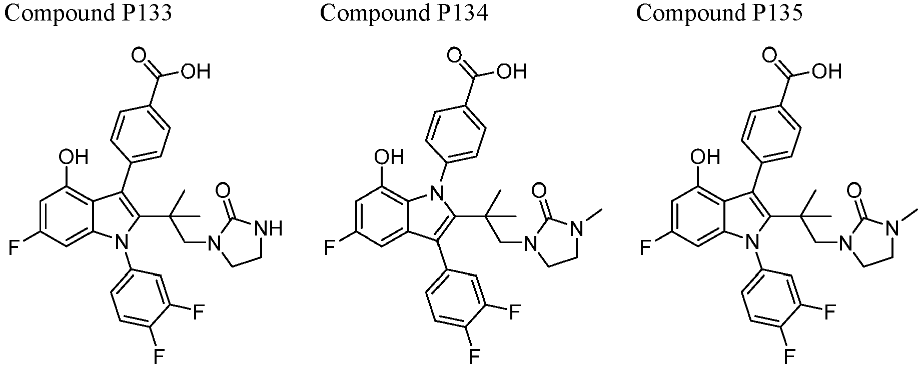 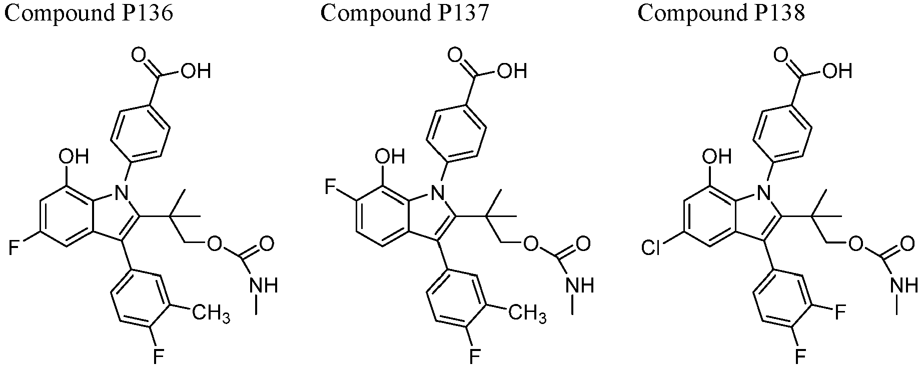 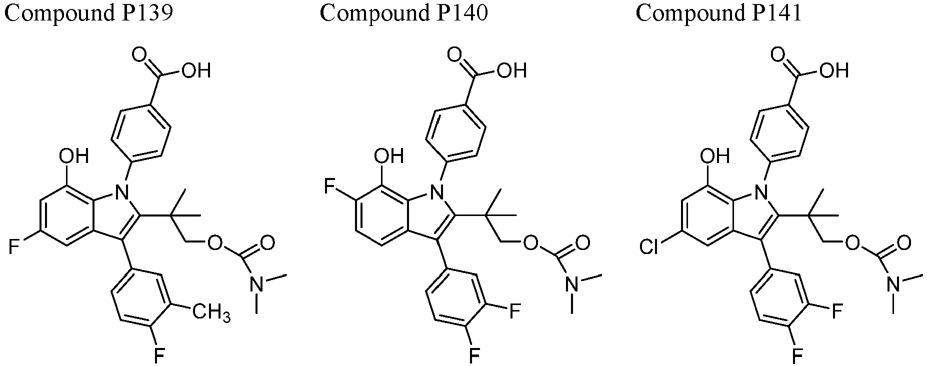 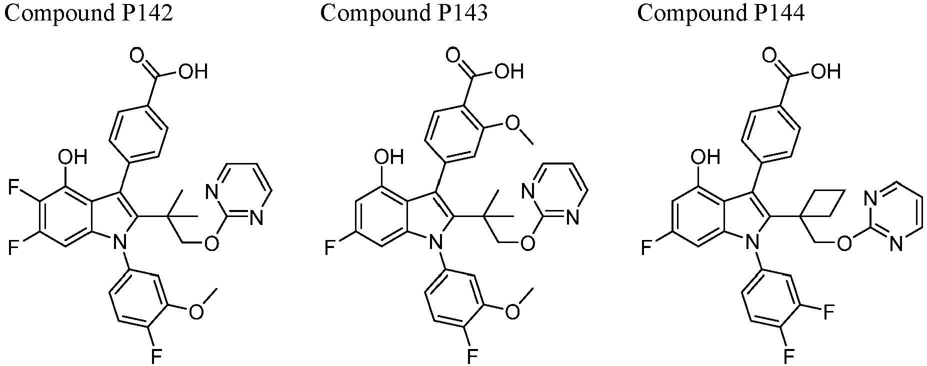 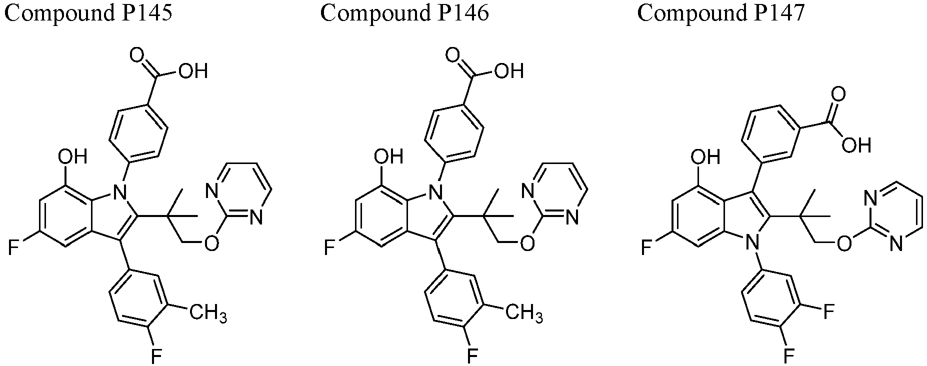 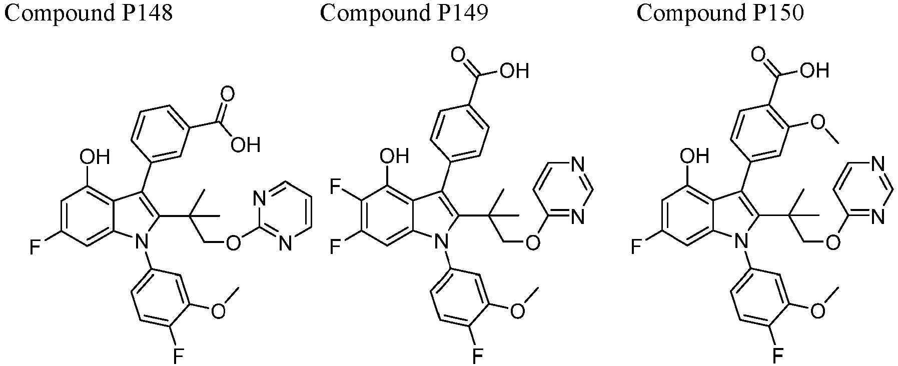 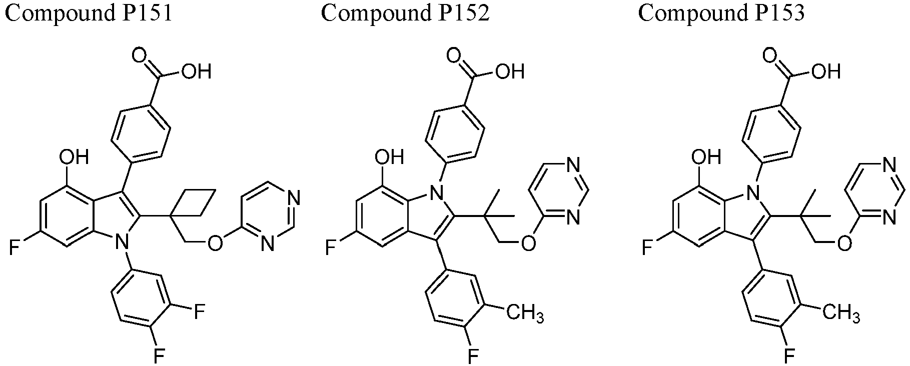 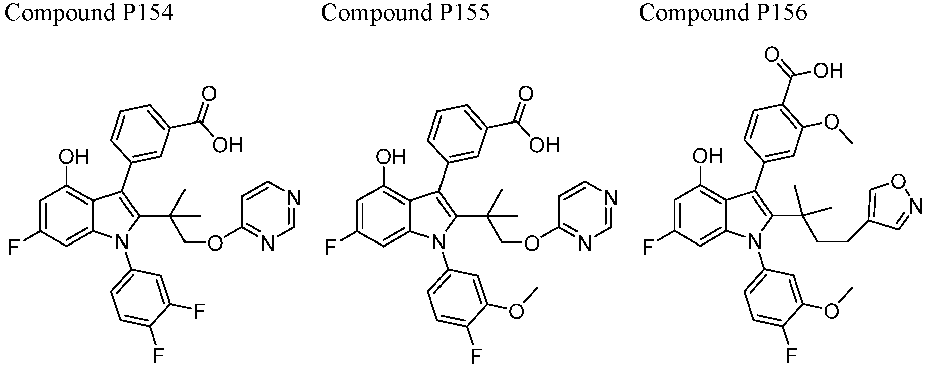 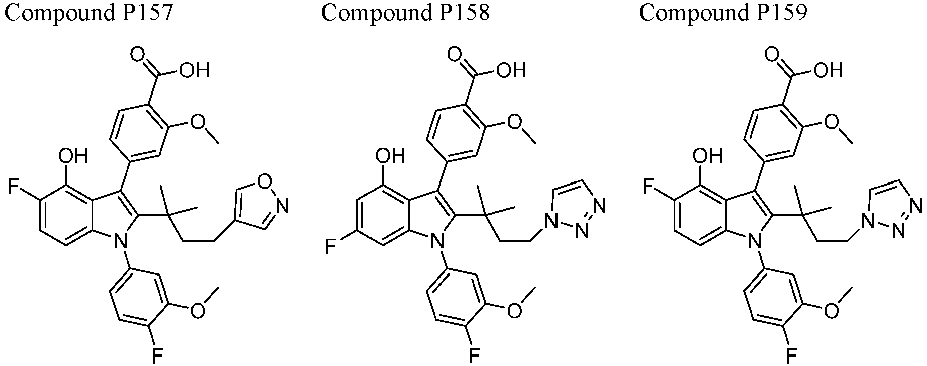 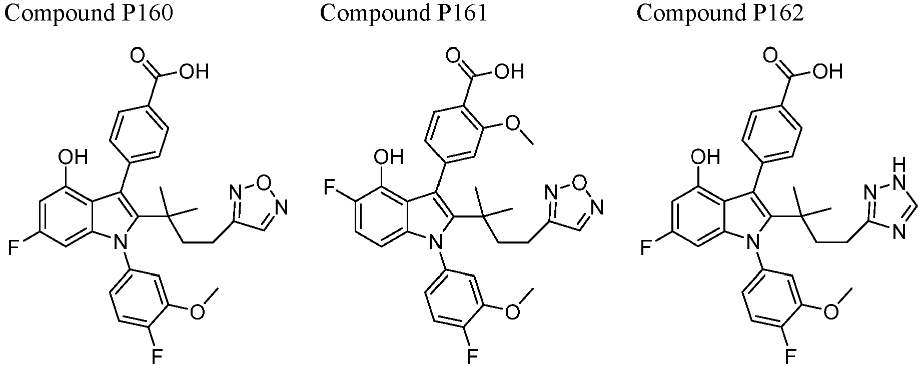 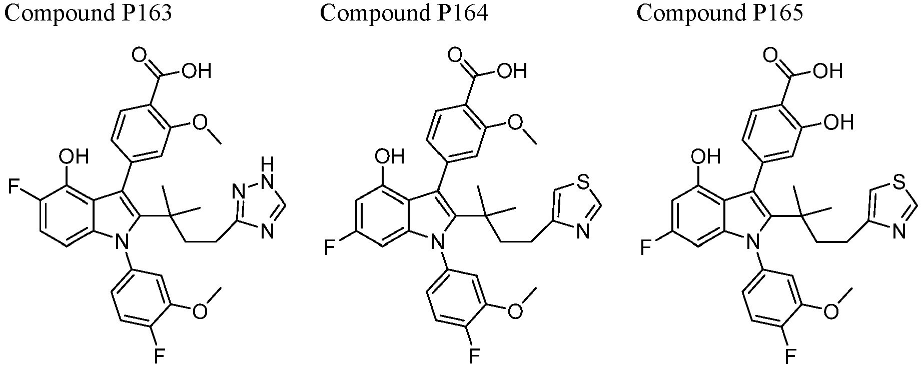 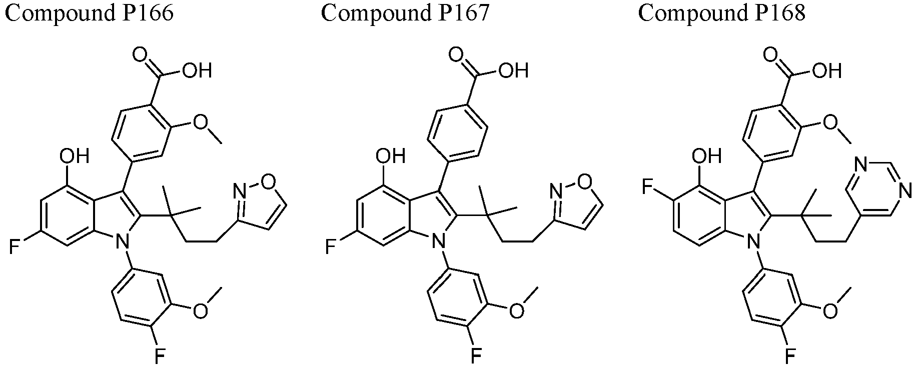 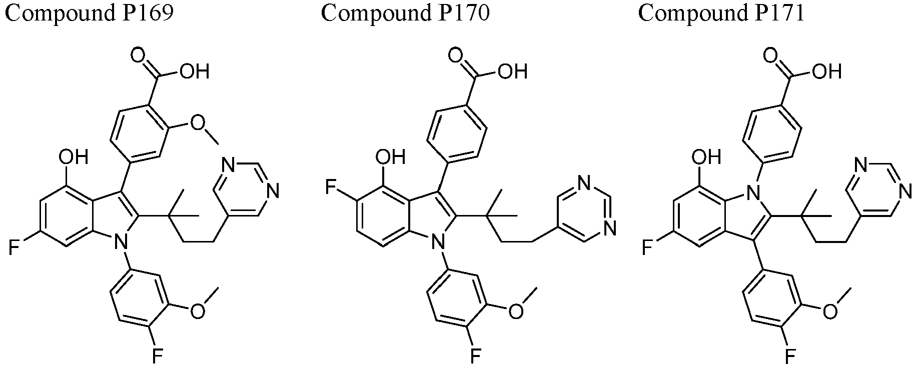 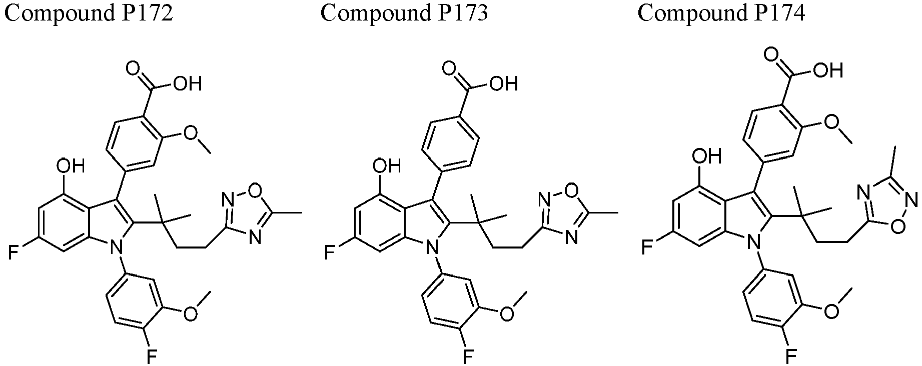 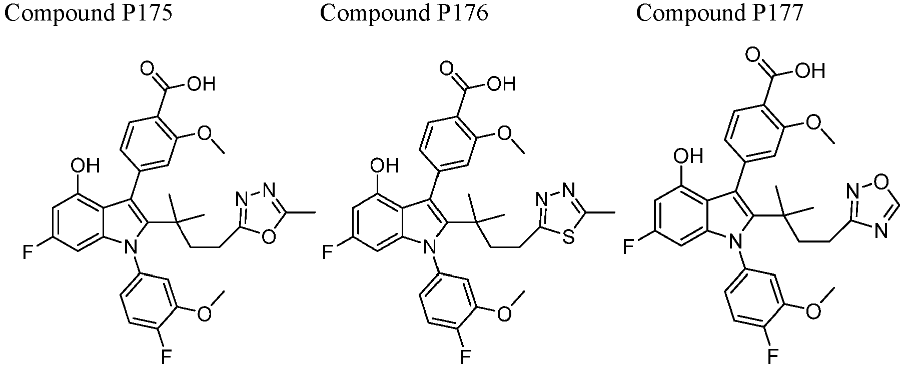 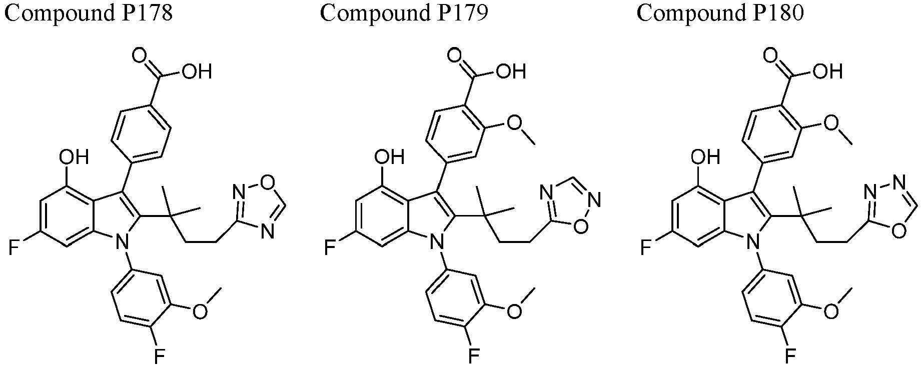 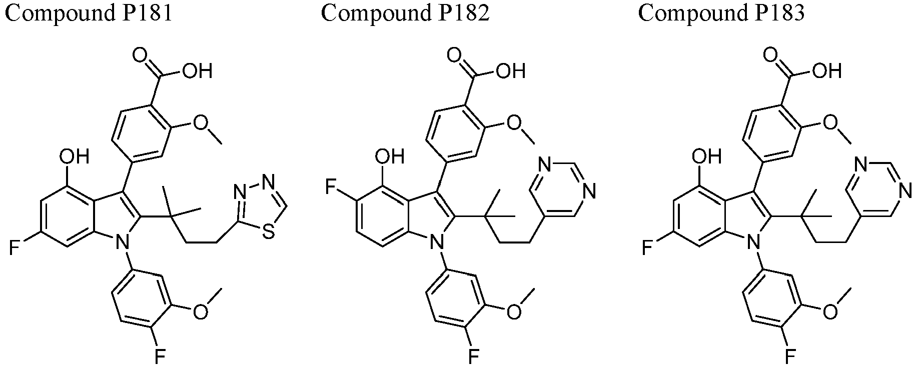 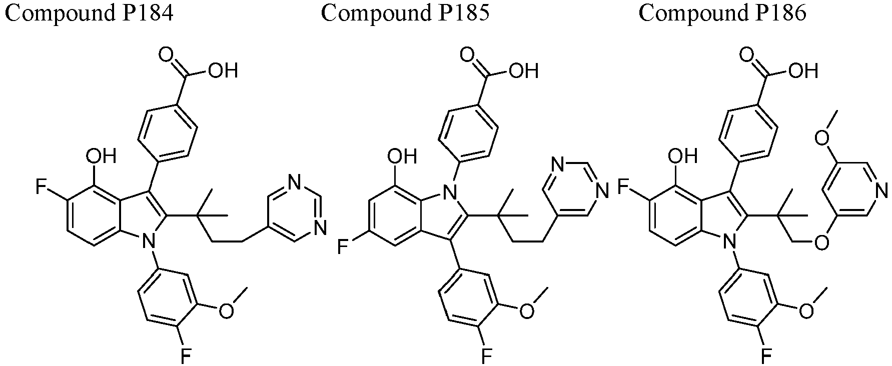 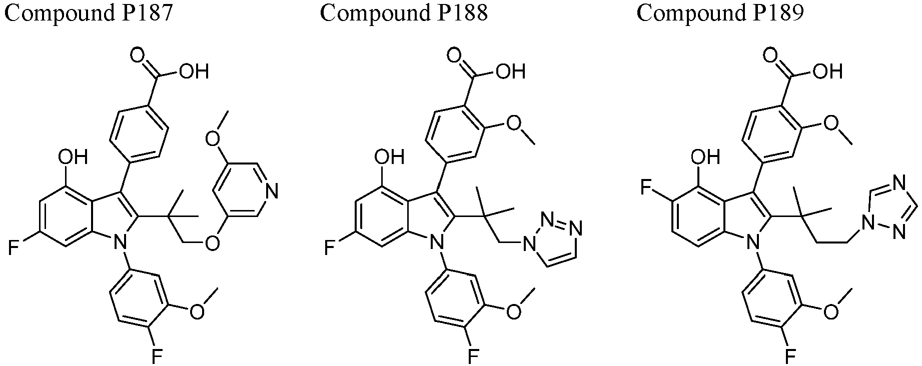 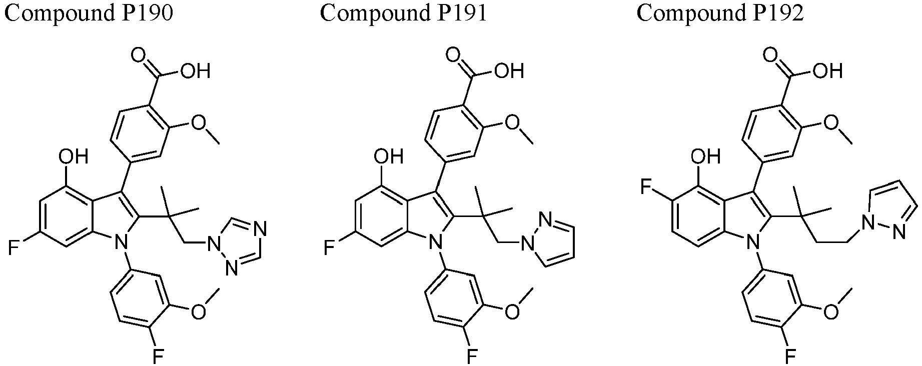 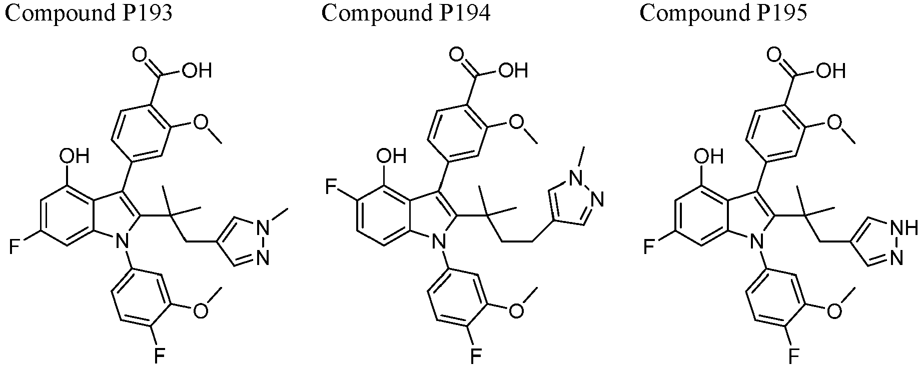 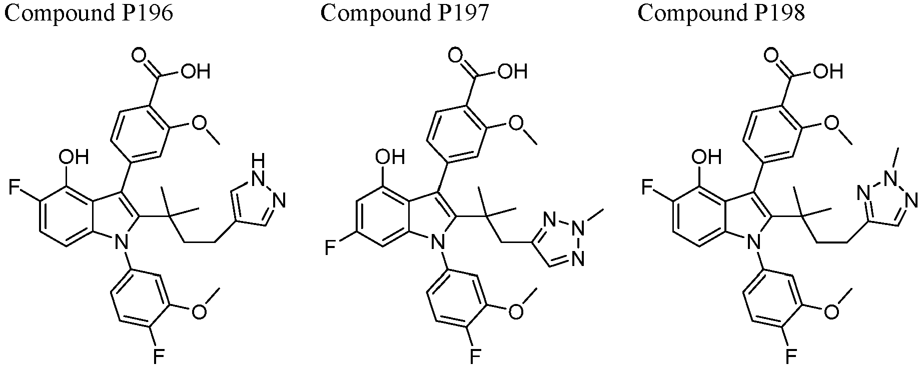 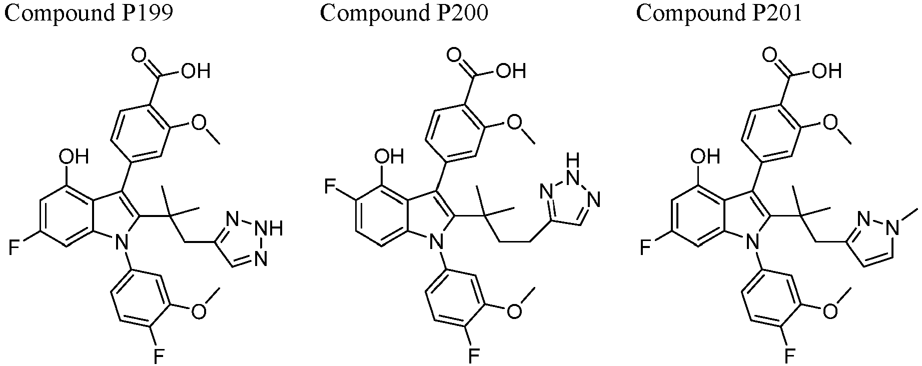 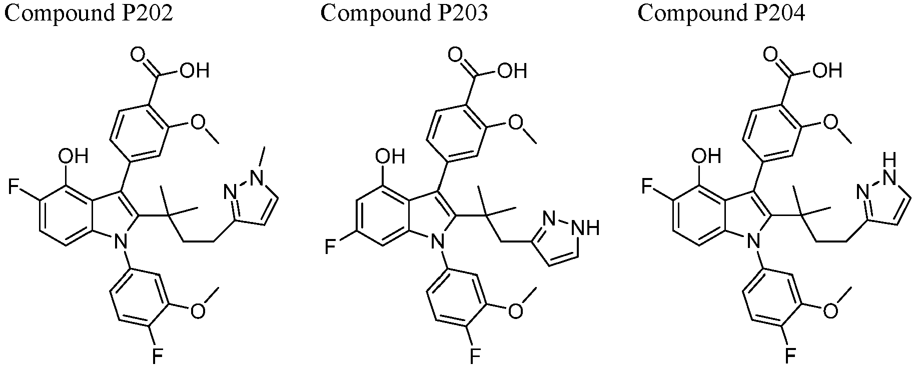 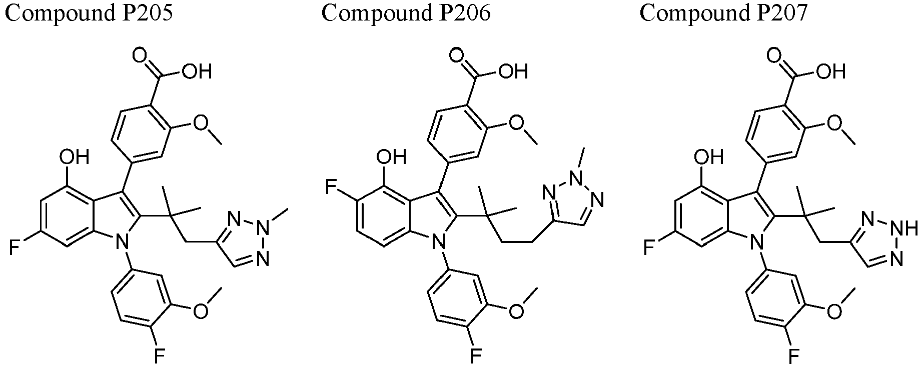 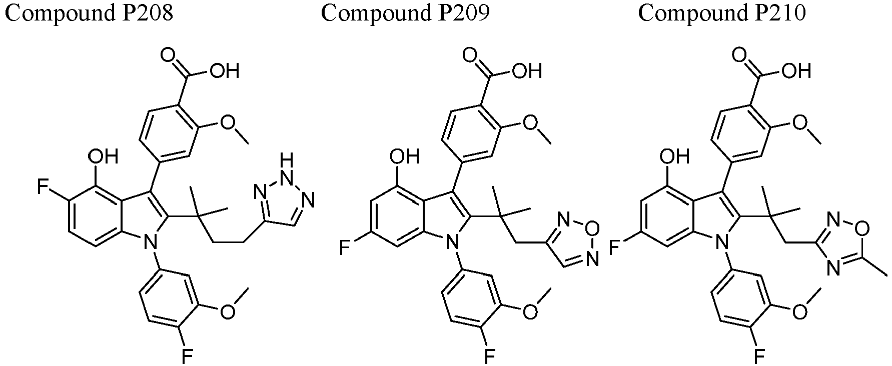 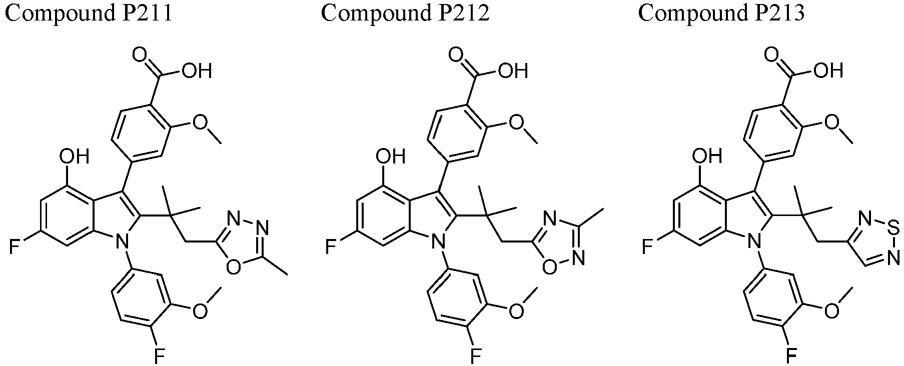 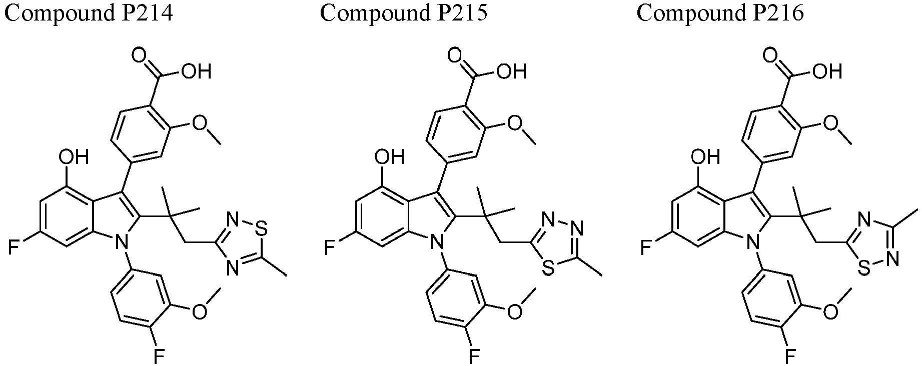 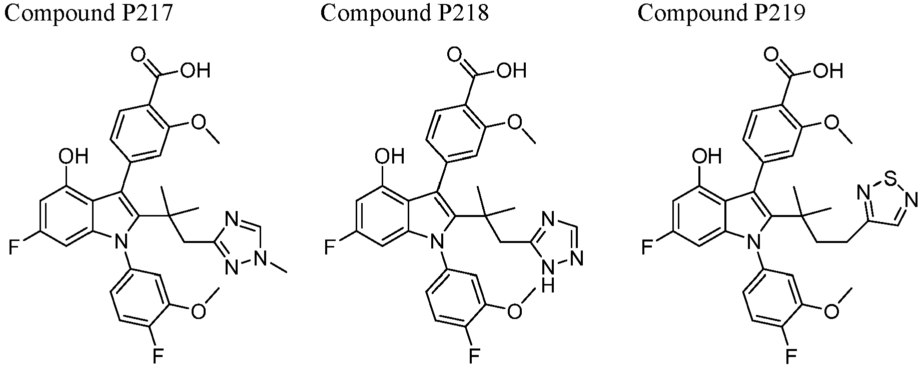 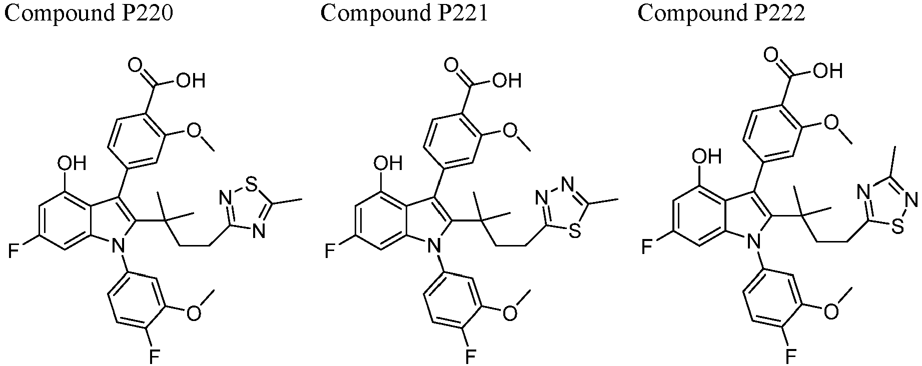 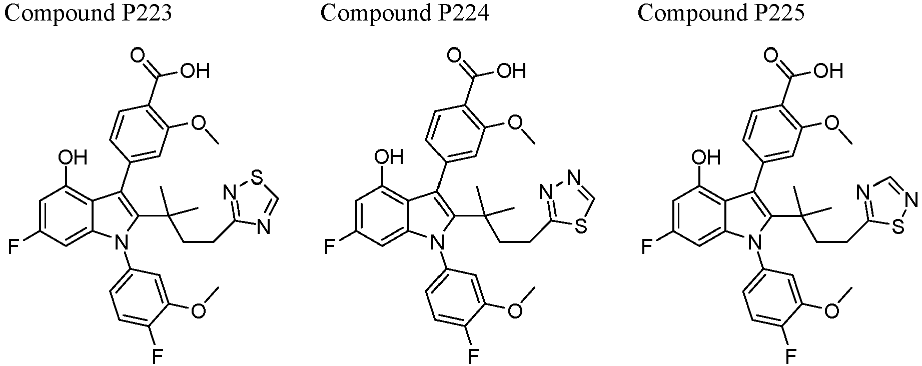 tautomers thereof, deuterated derivatives of those compounds and tautomers and pharmaceutically acceptable salts of the compounds, tautomers, and deuterated derivatives.

13. A compound selected from: 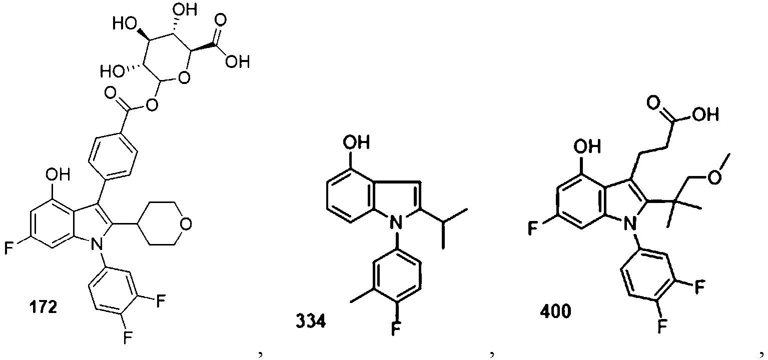 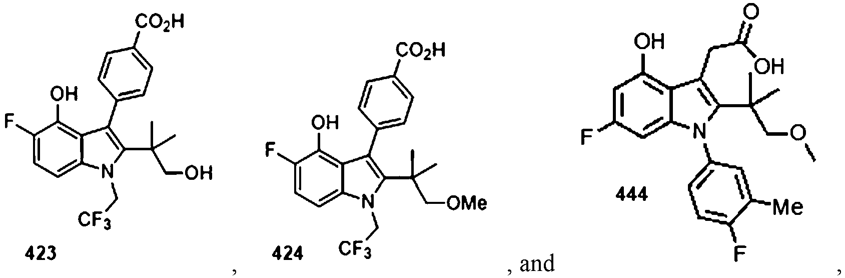 a tautomer thereof, a deuterated derivative of the compound or tautomer, or a pharmaceutically acceptable salt of the compound, tautomer, or deuterated derivative.

14. A pharmaceutical composition comprising at least one compound according to any one of claims 1 to 13, a tautomer thereof, a deuterated derivative of that compound or tautomer, or a pharmaceutically acceptable salt of the foregoing.

15. A compound, tautomer, deuterated derivative, or pharmaceutically acceptable salt according to any one of claims 1 to 13, or pharmaceutical composition according to claim 14, for use in a method of treating alpha-1 antitrypsin (AAT) deficiency comprising administering to a patient in need thereof a therapeutically effective amount of at least one compound, tautomer, deuterated derivative, or pharmaceutically acceptable salt according to any one of claims 1 to 13, or a therapeutically effective amount of a pharmaceutical composition according to claim 14, optionally wherein said therapeutically effective amount of the at least one compound, tautomer, deuterated derivative, or pharmaceutically acceptable salt is administered in combination with AAT augmentation therapy and/or AAT replacement therapy.

## Patentansprüche

1. Verbindung, dargestellt durch die folgende Strukturformel: 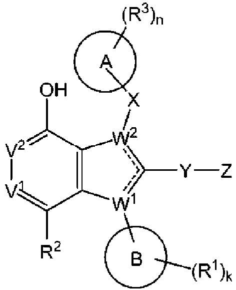 ein Tautomer davon, ein deuteriertes Derivat dieser Verbindung oder dieses Tautomers, oder ein pharmazeutisch verträgliches Salz des Vorstehenden, wobei:
-̅ -̅ -̅ -̅ -̅ -̅, für jedes der zwei Auftreten, eine Einfachbindung oder eine Doppelbindung ist, mit der Maßgabe, dass eine eine Einfachbindung und die andere eine Doppelbindung ist;
**V¹** und **V²** jeweils unabhängig N oder -C**R²** sind;
**W¹** und **W²** jeweils unabhängig N oder C sind, mit der Maßgabe, dass eines von **W¹** und **W²** N ist und das andere C ist;
**X** abwesend oder eine Bindung, **-(**C**R^{a}R^{b})ₚ-**, oder -SO₂- ist;
**Y** abwesend oder eine Bindung, **-(**C**R^{c}R^{d})_{q}-**, -C(=O)-, oder -SO₂- ist; **R^{a}** und **R^{b},** für jedes Auftreten, jeweils unabhängig Wasserstoff, Halogen, -OH, C₁-C₆ Alkyl, C₂-C₆ Alkenyl, C₁-C₆ Halogenalkyl, C₁-C₆ Alkoxy oder C₁-C₆ Halogenalkoxy sind;
**R^{c}** und **R^{d},** für jedes Auftreten, jeweils unabhängig Wasserstoff, Halogen, -OH, C₁-C₆ Alkyl, C₂-C₆ Alkenyl, C₁-C₆ Halogenalkyl, C₁-C₆ Alkoxy oder C₁-C₆ Halogenalkoxy sind;
**Ring A** C₃-C₁₂ Carbocyclyl, 3- bis 12-gliedriges Heterocyclyl, C₆ oder C₁₀ Aryl, oder 5- bis 10-gliedriges Heteroaryl ist; mit der Maßgabe, dass, wenn **W¹** N ist und **W²** C ist, **Ring A** nicht 1,5,6,7-Tetrahydro-4*H*-indol-4-onyl oder ein Tautomer davon ist;
**Ring B** C₄-C₁₂ Cycloalkyl, C₆ oder C₁₀ Aryl, 5- bis 10-gliedriges Heteroaryl oder Benzyl ist;
**Z** 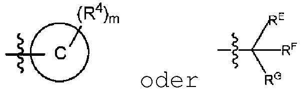 ist; wobei:
**Ring C** C₃-C₁₂ Cycloalkyl, 3- bis 12-gliedriges Heterocyclyl, C₆ oder C₁₀ Aryl, oder 5- bis 10-gliedriges Heteroaryl ist;
mit der Maßgabe, dass, wenn **Ring C** Phenyl ist, das Phenyl mit **R⁴** substituiert ist; mit der Maßgabe, dass, wenn **Ring C** Phenyl ist, **Y** nicht -SO₂- sein kann; und
mit der Maßgabe, dass, wenn **Ring B** Benzyl ist, **Ring C** nicht Pyridinyl oder Indolyl sein kann;
**R^{E}, R^{F}** und **R^{G}** jeweils unabhängig Wasserstoff, Halogen, Cyano, C₁-C₆ Alkyl, C₂-C₆ Alkenyl, C₁-C₆ Alkoxy, C₁-C₆ Halogenalkyl, C₁-C₆ Halogenalkoxy, -C(=O)**R^{s}**, -C(=O)O**R^{s}**, -C(=O)N**R^{p}R^{q}**, -**CR^{p}**(=N)O**R^{s}**, - N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}** oder - OC(=O)N**R^{p}R^{q}** sind; wobei:
das C₁-C₆ Alkyl oder das C₂-C₆ Alkenyl eines von **R^{E}**, R**^{F}** und **R^{G}** gegebenenfalls mit 1 bis 3 Gruppen substituiert ist, ausgewählt aus Cyano, -C(=O)**R^{s}**, -C(=O)O**R^{s}**, -C(=O)N**R^{p}R^{q}**, -N**R^{p}**C(=O)**R^{s}**, - N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, - OC(=O)O**R^{s}**, -OC(=O)N**R^{p}R^{q}**, -S(=O)**ᵣR^{s}** und -S(=O)**ᵣ**N**R^{p}R^{q}**; wobei:
**R^{p}**, **R^{q}** und **R^{r}**, für jedes Auftreten, jeweils unabhängig Wasserstoff, C₁-C₄ Alkyl, C₃-C₆ Cycloalkyl oder 3- bis 6-gliedriges Heterocyclyl sind; wobei:
das C₁-C₄ Alkyl eines von **R^{p}**, **R^{q}** und **R^{r}** gegebenenfalls mit 1 bis 3 Gruppen substituiert ist, ausgewählt aus Halogen, Cyano, -OH, C₁-C₃ Alkoxy, -C(=O)NH(C₁-C₂ Alkyl) und -C(=O)N(C₁-C₂ Alkyl)₂; und das C₃-C₆ Cycloalkyl oder das 3- bis 6-gliedrige Heterocyclyl eines von **R^{p}**, **R^{q}** und **R^{r}** gegebenenfalls mit 1 bis 3 Gruppen substituiert ist, ausgewählt aus Halogen, Cyano, -OH, C₁-C₃ Alkyl, C₁-C₃ Alkoxy, C₁-C₃ Halogenalkyl, C₁-C₃ Halogenalkoxy, -C(=O)O(C₁-C₂ Alkyl), -C(=O)NH(C₁-C₂ Alkyl) und -C(=O)N(C₁-C₂ Alkyl)₂;
**R^{s}**, für jedes Auftreten, unabhängig Wasserstoff, C₁-C₄ Alkyl, C₃-C₆ Cycloalkyl, Phenyl oder 5- oder 6-gliedriges Heteroaryl ist; wobei:
das C₁-C₄ Alkyl von **R^{s}** gegebenenfalls mit 1 bis 3 Gruppen substituiert ist, ausgewählt aus Halogen, Cyano, -OH, -NH₂, - NH(C₁-C₂ Alkyl), -N(C₁-C₂ Alkyl)₂, C₁-C₃ Alkoxy, -C(=O)OH, - C(=O)O(C₁-C₂ Alkyl), -C(=O)NH₂, -C(=O)NH(C₁-C₂ Alkyl) und - C(=O)N(C₁-C₂ Alkyl)₂; und
das C₃-C₆ Cycloalkyl, das Phenyl oder das 5- oder 6-gliedrige Heteroaryl von **R^{s}** gegebenenfalls mit 1 bis 3 Gruppen substituiert ist, ausgewählt aus Halogen, Cyano, -OH, -OCH₃, -NH₂, -NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁-C₂ Alkyl)₂;
**R¹** Halogen, Cyano, C₁-C₃ Alkyl, C₁-C₃ Halogenalkyl, C₁-C₃ Alkoxy, C₁-C₃ Halogenalkoxy oder -O-(C₃-C₆ Cycloalkyl) ist;
**R²**, für jedes Auftreten, unabhängig Wasserstoff, Halogen, Cyano, C₁-C₆ Alkyl, C₂-C₆ Alkenyl, C₁-C₆ Alkoxy, C₁-C₆ Halogenalkyl, C₁-C₆ Halogenalkoxy, C₃-C₆ Cycloalkyl, -N**R^{h}Rⁱ**, Phenyl oder 5- oder 6-gliedriges Heteroaryl ist; wobei:
das C₁-C₆ Alkyl, das C₂-C₆ Alkenyl oder das C₃-C₆ Cycloalkyl von **R²** gegebenenfalls mit 1 bis 3 Gruppen substituiert ist, ausgewählt aus Cyano, -C(=O)**R^{k}**, -C(=O)O**R^{k}**, -C(=O)N**R^{h}Rⁱ**, -N**R^{h}Rⁱ**, - N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k}**, - OC(=O)**R^{k}**, -OC(=O)O**R^{k}**, -OC(=O)N**R^{h}Rⁱ**, -S(=O)**ₛR^{k}** und S(=O)ₛN**R^{h}Rⁱ**; wobei:
**R^{h}**, **Rⁱ** und **R^{j}**, für jedes Auftreten, jeweils unabhängig Wasserstoff, C₁-C₄ Alkyl oder C₃-C₆ Cycloalkyl sind; wobei:
das C₁-C₄ Alkyl eines von **R^{h}, Rⁱ** und **R^{j}** gegebenenfalls mit 1 bis 3 Gruppen substituiert ist, ausgewählt aus Halogen, Cyano, -OH, -NH₂, -NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁-C₂ Alkyl)₂; und
das C₃-C₆ Cycloalkyl eines von **R^{h}, Rⁱ** und **R^{j}** gegebenenfalls mit 1 bis 3 Gruppen substituiert ist, ausgewählt aus Halogen, Cyano, -OH, -NH₂, -NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, - C(=O)O(C₁-C₂ Alkyl), -C(=O)NH₂, -C(=O)NH(C₁-C₂ Alkyl) und - C(=O)N(C₁-C₂ Alkyl)₂;
**R^{k}**, für jedes Auftreten, unabhängig Wasserstoff, C₁-C₄ Alkyl, C₃-C₆ Cycloalkyl, Phenyl oder 5- oder 6-gliedriges Heteroaryl ist;
wobei:
-O**R^{k}** nicht -OH sein kann;
das C₁-C₄ Alkyl von **R^{k}** gegebenenfalls mit 1 bis 3 Gruppen substituiert ist, ausgewählt aus Halogen, Cyano, -OH, -NH₂, - NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁-C₂ Alkyl)₂; und
das C₃-C₆ Cycloalkyl von **R^{k}** gegebenenfalls mit 1 bis 3 Gruppen substituiert ist, ausgewählt aus Halogen, Cyano, -OH, -NH₂, - NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁- C₂ Alkyl)₂;
**R³** und **R⁴**, für jedes Auftreten, jeweils unabhängig Halogen, Cyano, =O, C₁-C₆ Alkyl, C₂-C₆ Alkenyl, C₁-C₆ Alkoxy, C₁-C₆ Halogenalkyl, C₁-C₆ Halogenalkoxy, C₃-C₆ Cycloalkyl, -C(=O)**R^{y}**, -C(=O)O**R^{y}**, - C(=O)N**R^{v}R^{w}**, -C(=O)N**R^{v}**O**R^{y}**, -C(=O)N**R^{v}**S(=O)**ₜR^{y}**, -N**R^{v}R^{w}**, -N**R^{v}**C(=O)**R^{y}**, - N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, - OC(=O)O**R^{y}**, -OC(=O)N**R^{v}R^{w}**, -S(=O)ₜ**R^{y}**, -S(=O)ₜN**R^{v}R^{w}**, -S(=O)ₜN**R^{v}**C(=O)**R^{y}**, -P(=O)**R^{z}R^{z}**, Phenyl oder ein 5- oder 6-gliedriges Heteroaryl sind;
wobei:
das C₁-C₆ Alkyl, das C₂-C₆ Alkenyl oder das C₃-C₆ Cycloalkyl eines von **R³** und **R⁴** gegebenenfalls mit 1 bis 3 Gruppen substituiert ist, ausgewählt aus Cyano, -C(=O)**R^{y}**, -C(=O)O**R^{y}**, -C(=O)N**R**^{v}**R**^{w}, - N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R**^{v}S(=O)ᵣ**R^{y}**, - O**R^{y}**, -OC(=O)**R^{y}**, -OC(=O)O**R^{y}**, -OC(=O)N**R^{v}R^{w}**, -S(=O)**ₜR^{y}** und - S(=O)**ₜ**N**R^{v}R^{w}**; wobei:
**R^{v}**, **R^{w}** und **R^{x}**, für jedes Auftreten, jeweils unabhängig Wasserstoff, C₁-C₄ Alkyl, C₃-C₆ Cycloalkyl, 5- oder 6-gliedriges Heterocyclyl oder 5- oder 6-gliedriges Heteroaryl sind; wobei:
das C₁-C₄ Alkyl eines von **R^{v}**, **R^{w}** und **R^{x}** gegebenenfalls mit 1 bis 3 Gruppen substituiert ist, ausgewählt aus Halogen, Cyano -OH, -NH₂, -NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁-C₂ Alkyl)₂; und
das C₃-C₆ Cycloalkyl, das 5- oder 6-gliedrige Heterocyclyl oder das 5- oder 6-gliedrige Heteroaryl eines von **R^{v}**, **R^{w}** und **R^{x}** gegebenenfalls mit 1 bis 3 Gruppen substituiert ist, ausgewählt aus Halogen, Cyano, -OH, -NH₂, -NH(C₁-C₂ Alkyl), -N(C₁-C₂ Alkyl)₂, C₁-C₃ Alkyl, C₁-C₃ Alkoxy, C₁-C₃ Halogenalkyl, C₁-C₃ Halogenalkoxy, -C(=O)OH, -C(=O)O(C₁-C₂ Alkyl), -C(=O)NH₂, -C(=O)NH(C₁-C₂ Alkyl) und -C(=O)N(C₁-C₂ Alkyl)₂;
**R^{y}** bei jedem Auftreten unabhängig Wasserstoff, C₁-C₄-Alkyl, C₃-C₆-Cycloalkyl, Phenyl, ein 5- oder 6-gliedriges Heterocyclyl oder ein 5- oder 6-gliedriges Heteroaryl ist; wobei
das C₁-C₄-Alkyl von **R^{y}** gegebenenfalls mit 1 bis 3 Gruppen substituiert ist, ausgewählt aus Halogen, Cyano, -OH, -NH₂, - NH(C₁-C₂-Alkyl), -N(C₁-C₂-Alkyl)₂, C₁-C₃-Alkyl, C₁-C₃-Alkoxy, C₁-C₃-Halogenalkyl, C₁-C₃-Halogenalkoxy, -C(=O)OH, -C(=O)O(C₁-C₂-Alkyl), -C(=O)NH₂, -C(=O)NH(C₁-C₂-Alkyl) und -C(=O)N(C₁-C₂-Alkyl)₂; und
das C₃-C₆-Cycloalkyl, das Phenyl, das 5- oder 6-gliedrige Heterocyclyl oder das 5- oder 6-gliedrige Heteroaryl von **R^{y}** gegebenenfalls mit 1 bis 3 Gruppen substituiert ist, ausgewählt aus Halogen, Cyano, -OH, -NH₂, NH(C₁-C₂-Alkyl), -N(C₁-C₂-Alkyl)₂, C₁-C₃-Alkyl, C₁-C₃-Alkoxy, C₁-C₃-Halogenalkyl, C₁-C₃-Halogenalkoxy, -C(=O)OH, -C(=O)O(C₁-C₂-Alkyl), -C(=O)NH₂, -C(=O)NH(C₁-C₂-Alkyl) und -C(=O)N(C₁-C₂-Alkyl)₂;
**R^{z}** bei jedem Auftreten unabhängig C₁-C₂-Alkyl, -OH oder -O(C₁-C₂-Alkyl) ist;
**k** eine ganze Zahl ist, ausgewählt aus 1, 2 und 3;
**m** und **n** jeweils unabhängig eine ganze Zahl sind, ausgewählt aus 0, 1, 2 und 3;
**p, r, s,** und **t** jeweils unabhängig eine ganze Zahl sind, ausgewählt aus 1 und 2; und
**q** eine ganze Zahl ist, ausgewählt aus 1, 2 und 3.

2. Verbindung, Tautomer, deuteriertes Derivat oder pharmazeutisch verträgliches Salz nach Anspruch 1, dargestellt durch eine der folgenden Strukturformeln: 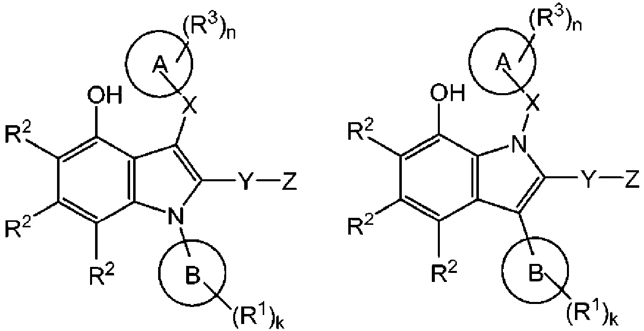 wobei alle anderen hierin nicht spezifisch definierten Variablen wie in Anspruch 1 definiert sind.

3. Verbindung, Tautomer, deuteriertes Derivat oder pharmazeutisch verträgliches Salz nach Anspruch 1, dargestellt durch eine der folgenden Strukturformeln: 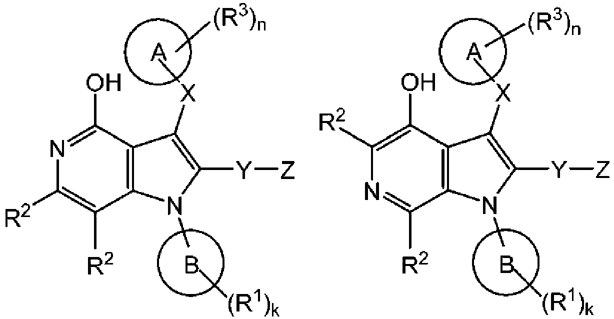 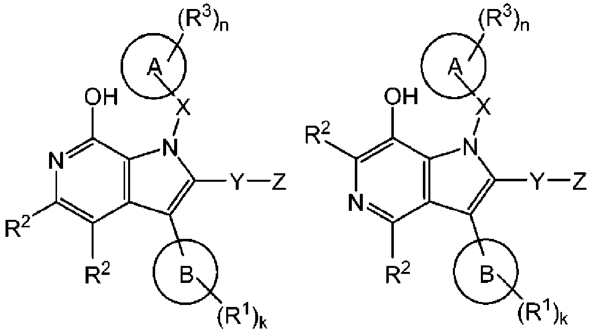 wobei alle anderen hierin nicht spezifisch definierten Variablen wie in Anspruch 1 definiert sind.

4. Verbindung, Tautomer, deuteriertes Derivat oder pharmazeutisch verträgliches Salz nach Anspruch 1 oder Anspruch 2, dargestellt durch eine der folgenden Strukturformeln: 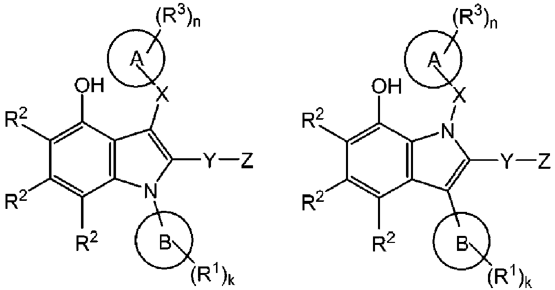 wobei:
**Ring A** gegebenenfalls mit **R³** substituiert ist und **Ring A** C₃-C₇-Carbocyclyl, 6- bis 9-gliedriges Heterocyclyl, Phenyl oder 5-bis 9-gliedriges Heteroaryl ist;
**Ring B** mit **R¹** substituiert ist und **Ring B** C₄-C₆-Cycloalkyl, Phenyl, 5- bis 6-gliedriges Heteroaryl oder Benzyl ist; und
wenn **Z** ein gegebenenfalls mit **R⁴** substituierter **Ring C** ist, **Ring C** C₄-C₈-Cycloalkyl, 4- bis 8-gliedriges Heterocyclyl, Phenyl oder 5- oder 6-gliedriges Heteroaryl ist;
und wobei alle anderen hierin nicht spezifisch definierten Variablen wie in Anspruch 1 oder Anspruch 2 definiert sind.

5. Verbindung, Tautomer, deuteriertes Derivat oder pharmazeutisch verträgliches Salz nach einem der Ansprüche 1, 2 und 4, dargestellt durch eine der folgenden Strukturformeln: 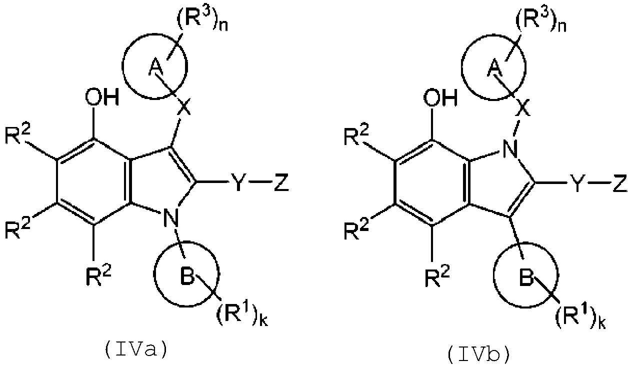 wobei:
**Ring B** mit **R¹** substituiert ist und **Ring B** Cyclohexyl, Phenyl, Pyridinyl oder Benzyl ist; und wobei alle anderen hierin nicht spezifisch definierten Variablen wie in einem der Ansprüche 1, 2 und 4 definiert sind,
gegebenenfalls wobei die Verbindung, das Tautomer, das deuterierte Derivat oder das pharmazeutisch verträgliche Salz durch eine der folgenden Strukturformeln dargestellt ist: 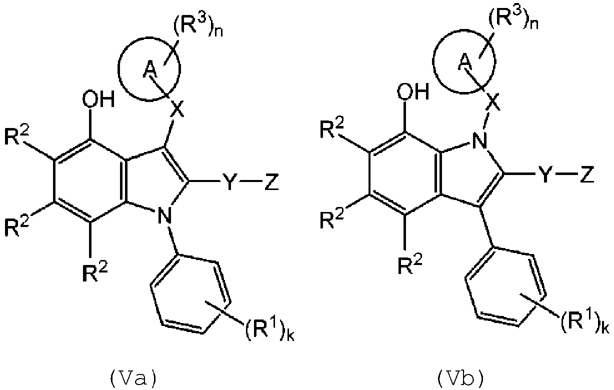
wobei:
**R¹** Halogen, Cyano, C₁-C₂-Alkyl, C₁-C₂-Halogenalkyl oder C₁-C₂-Alkoxy ist; und
**k** eine ganze Zahl ist, ausgewählt aus 1 und 2.

6. Verbindung, Tautomer, deuteriertes Derivat oder pharmazeutisch verträgliches Salz nach einem der Ansprüche 1 bis 5, wobei:
(i) **R¹** Cyano, F, Cl, -CH₃, -CHF₂, -CF₃, -OCH₃ oder -OCH(CH₃)₂ ist;
(ii) mindestens ein **R¹** F ist;
(iii) **X** abwesend oder eine Bindung, -(C**R^{a}R^{b}**)- oder -SO₂- ist; **R^{a}** und **R^{b}** bei jedem Auftreten jeweils unabhängig Wasserstoff, C₁-C₃-Alkyl, C₁-C₃-Halogenalkyl oder C₁-C₃-Alkoxy sind;
(iv) **X** abwesend oder eine Bindung, -CH₂- oder -SO₂- ist;
(v) **Y** abwesend oder eine Bindung, -(C**R^{c}R^{d}**)_{q}-, -C(=O)- oder -SO₂- ist;
**R^{c}** und **R^{d}** sind bei jedem Auftreten jeweils unabhängig Wasserstoff, C₁-C₃-Alkyl, C₁-C₃-Halogenalkyl oder C₁-C₃-Alkoxy;
(vi) **q** eine ganze Zahl ist, ausgewählt aus 1 und 2; und/oder
(vii) **Y** abwesend ist oder eine Bindung, -CH₂-, -CHCH₃-, -C(CH₃)₂-, -C(=O)- oder -SO₂- ist;
und wobei alle anderen hierin nicht spezifisch definierten Variablen wie in einem der vorangehenden Ansprüche definiert sind.

7. Verbindung, Tautomer, deuteriertes Derivat oder pharmazeutisch verträgliches Salz nach einem der Ansprüche 1 bis 6, wobei **Ring A** gegebenenfalls mit **R³** substituiert ist und **Ring A** ist:
(i) C₃-C₇-Carbocyclyl, 6- bis 9-gliedriges Heterocyclyl, das 1 bis 3 Sauerstoffatome enthält, Phenyl oder 5- bis 9-gliedriges Heteroaryl, das 1 bis 3 Heteroatome enthält, ausgewählt aus O und N;
(ii) C₃-C₇-Carbocyclyl, 6- bis 9-gliedriges Heterocyclyl, das ein oder zwei Sauerstoffatome enthält, Phenyl oder 5- bis 9-gliedriges Heteroaryl, das ein oder zwei Stickstoffatome oder ein oder zwei Sauerstoffatome enthält;
(iii) ausgewählt aus 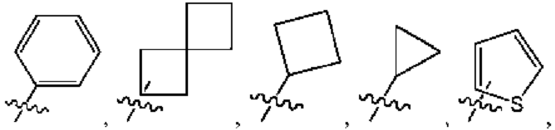 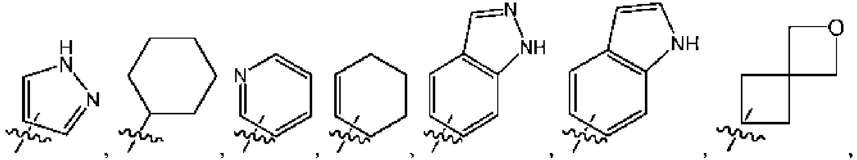 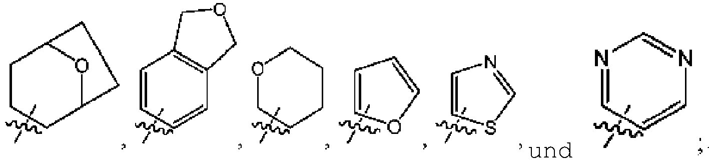 und/oder
(iv) ausgewählt aus 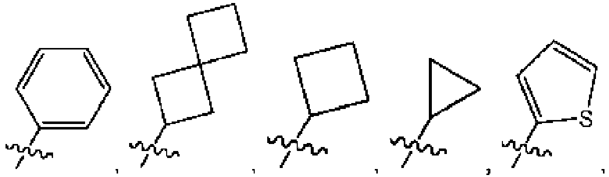 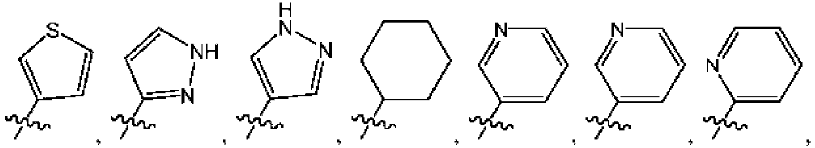 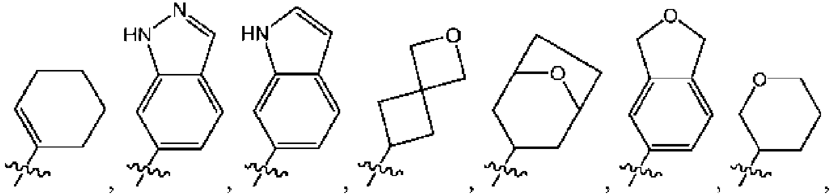 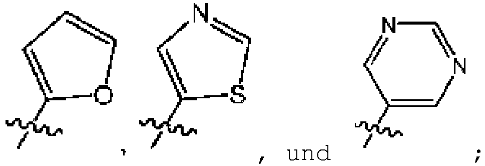
und wobei alle anderen hierin nicht spezifisch definierten Variablen wie in einem der vorangehenden Ansprüche definiert sind.

8. Verbindung, Tautomer, deuteriertes Derivat oder pharmazeutisch verträgliches Salz nach einem der Ansprüche 1 bis 7, wobei **Z** ein gegebenenfalls mit **R⁴** substituierter **Ring C** ist und **Ring C** ist:
(i) C₄-C₈-Cycloalkyl, 4- bis 8-gliedriges Heterocyclyl, das ein oder zwei Heteroatome enthält, ausgewählt aus O, N und S, Phenyl oder 5-gliedriges Heteroaryl, das ein oder zwei Heteroatome enthält, ausgewählt aus O und N;
(ii) ausgewählt aus 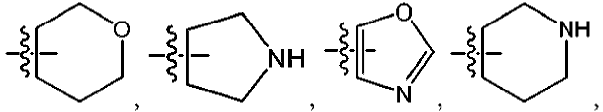 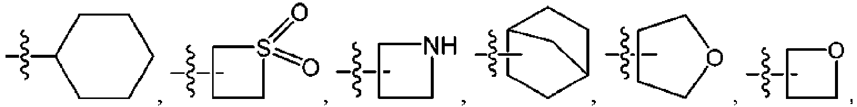 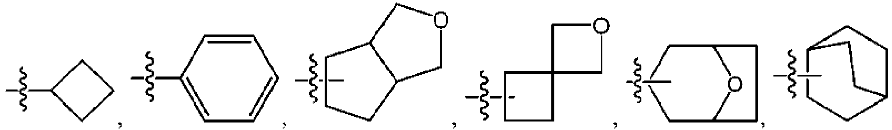 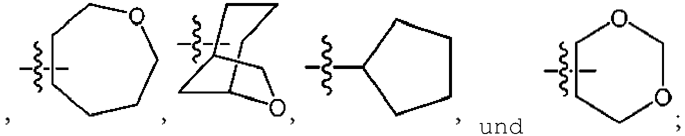 und/oder
(iii) ausgewählt aus 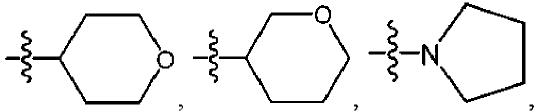 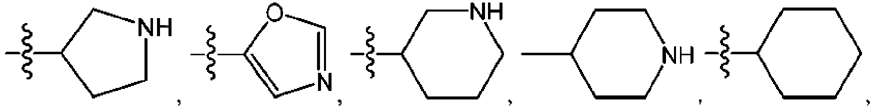 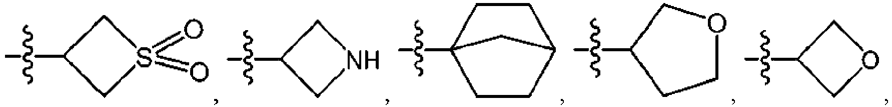 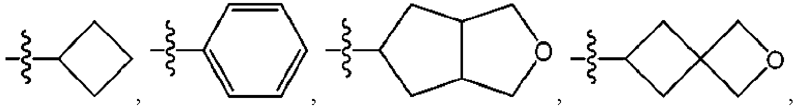 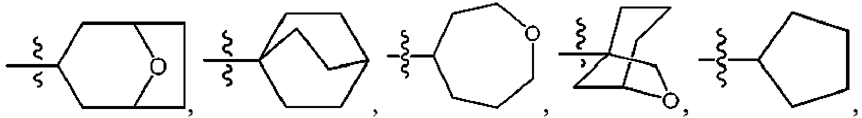 und 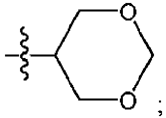
und wobei alle anderen hierin nicht spezifisch definierten Variablen wie in einem der vorangehenden Ansprüche definiert sind.

9. Verbindung, Tautomer, deuteriertes Derivat oder pharmazeutisch verträgliches Salz nach einem der Ansprüche 1 bis 8, wobei **R^{E}**, **R^{F}** und **R^{G}** jeweils unabhängig sind:
(i) Wasserstoff, Halogen, Cyano (-C≡N), C₁-C₄-Alkyl, C₁-C₄-Alkoxy, C₁-C₄-Halogenalkyl, -C(=O)O**R^{s}**, -C(=O)N**R^{p}R^{q}**, -C**R^{p}**(=N)O**R^{s}** oder -O**R^{s}**; wobei:
das C₁-C₄-Alkyl eines von **R^{E}**, **R^{F}** und **R^{G}** gegebenenfalls mit 1 bis 3 Gruppen substituiert ist, ausgewählt aus Cyano, -C(=O)**R^{s}**, - C(=O)O**R^{s}**, -C(=O)N**R^{p}R^{q}**, -O**R^{s}**, -OC(=O)**R^{s}**, -OC(=O)O**R^{s}**, -OC(=O)N**R^{p}R^{q}** und -S(=O)₂**R^{s}**; wobei:
**R^{p}** und **R^{q}** bei jedem Auftreten jeweils unabhängig Wasserstoff, C₁-C₂-Alkyl, C₃-C₅-Cycloalkyl oder 5- oder 6-gliedriges Heterocyclyl sind; wobei:
das C₁-C₂-Alkyl eines von **R^{p}** und **R^{q}** gegebenenfalls mit 1 bis 3 Gruppen substituiert ist, ausgewählt aus Halogen, Cyano und - OH;
das C₃-C₅-Cycloalkyl oder das 5- oder 6-gliedrige Heteroaryl von **R^{p}** und **R^{q}** gegebenenfalls mit 1 bis 3 Gruppen substituiert ist, ausgewählt aus Halogen, Cyano und -OH;
**R^{s}** bei jedem Auftreten unabhängig Wasserstoff, C₁-C₂-Alkyl oder 5- oder 6-gliedriges Heteroaryl ist; wobei das C₁-C₂-Alkyl von **R^{s}** gegebenenfalls mit 1 bis 3 Gruppen substituiert ist, ausgewählt aus Halogen, Cyano, -OH und -NH₂; wobei:
das 5- oder 6-gliedrige Heteroaryl von **R^{s}** gegebenenfalls mit 1 bis 3 Gruppen substituiert ist, ausgewählt aus Halogen, Cyano, -OH und -NH₂;
(ii) Wasserstoff, F, Cl, C₁-C₂-Alkyl, C₁-C₂-Halogenalkyl, - C(=O)N**R^{p}R^{q}**, -C**R^{p}**(=N)O**R^{s}** oder -O**R^{s}**; wobei:
das C₁-C₂-Alkyl eines von **R^{E}**, **R^{F}** und **R^{G}** gegebenenfalls mit 1 bis 3 Gruppen substituiert ist, ausgewählt aus Cyano, -C(=O)N**R^{p}R^{q}**, - O**R^{s}**, -OC(=O)N**R^{p}R^{q}** und -S(=O)₂**R^{s}**; wobei:
**R^{p}** und **R^{q}** bei jedem Auftreten jeweils unabhängig Wasserstoff, C₁-C₂-Alkyl, Cyclopentyl oder Tetrahydrofuranyl sind; wobei:
das C₁-C₂-Alkyl eines von **R^{p}** und **R^{q}** gegebenenfalls mit 1 bis 3 Halogengruppen substituiert ist, ausgewählt aus F und Cl;
**R^{s}** bei jedem Auftreten unabhängig Wasserstoff, C₁-C₂-Alkyl, Pyridinyl oder Pyrimidinyl ist; wobei:
das C₁-C₂-Alkyl von **R^{s}** gegebenenfalls mit 1 bis 3 Halogengruppen substituiert ist, ausgewählt aus F und Cl;
(iii) Wasserstoff, F, -OH, -CH(OH)CH₃, -C(=O)NHCH₃, -C(=N)OCH₃, -CH₃, -CF₃, -CH₂F, -CH₂CN, -(CH₂)₂CN, -CH₂OH, -C₂H₅, -(CH₂)₂OH, - CH₂OCH₃, -CH₂OC₂H₅, -(CH₂)₂OCH₃, -CH₂OCHF₂, -(CH₂)₂OCHF₂, - CH₂C(=O)NH₂, -CH₂C(=O)N(CH₃)₂, -CH₂S(=O)₂CH₃, -(CH₂)₂S(=O)₂CH₃, - CH₂(O)C(=O)NHCH₃, -CH₂(O)C(=O)N(CH₃)C₂H₅,-CH₂(O)C(=O)N(CH₃)₂, - CH₂(O)C(=O)N(C₂H₅)₂, -CH₂(O)C(=O)NH(Cyclopentyl), - CH₂(O)C(=O)NH (Tetrahydrofuranyl), -CH₂(O)(Pyridin-2-yl) oder - CH₂(O) (Pyrimidin-2-yl); und/oder
(iv) Wasserstoff, F, -CH(OH)CH₃, -CH₃, -CH₂CN, -CH₂OH oder - CH₂OCH₃; und wobei alle anderen hierin nicht spezifisch definierten Variablen wie in einem der vorangehenden Ansprüche definiert sind.

10. Verbindung, Tautomer, deuteriertes Derivat oder pharmazeutisch verträgliches Salz nach einem der Ansprüche 1 bis 9:
(i) dargestellt durch eine der folgenden Strukturformeln: 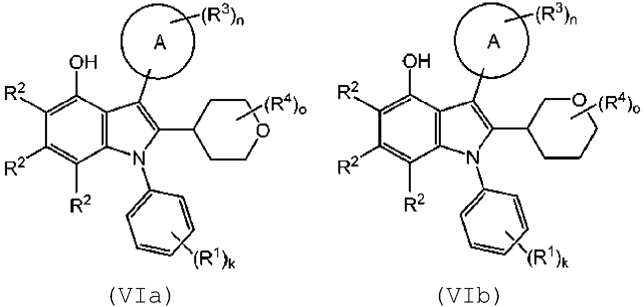 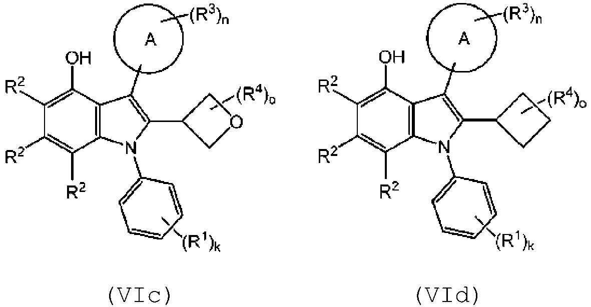 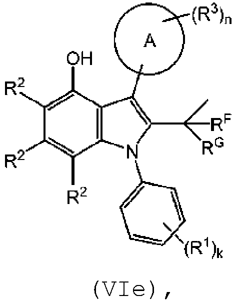 wobei **o** eine ganze Zahl ist, ausgewählt aus 0, 1 und 2;
(ii) dargestellt durch eine der folgenden Strukturformeln: 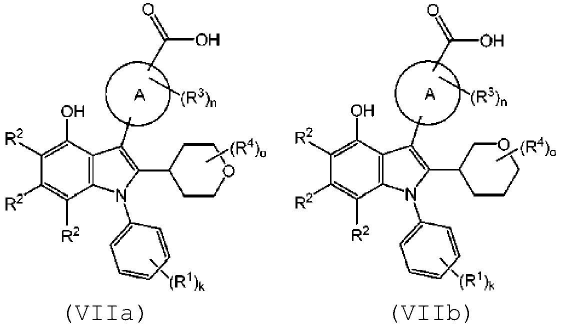 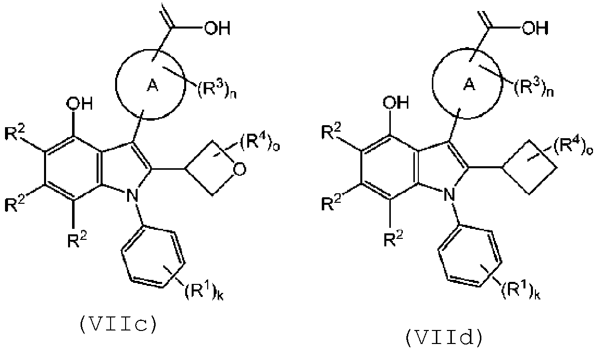 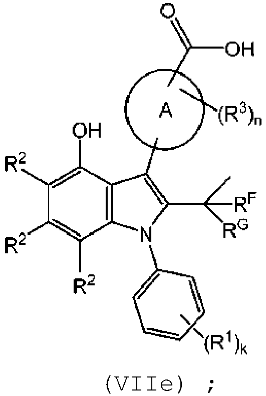 wobei **n** eine ganze Zahl ist, ausgewählt aus 0, 1 und 2, und wobei **o** eine ganze Zahl ist, ausgewählt aus 0, 1 und 2;
(iii) dargestellt durch eine der folgenden Strukturformeln: 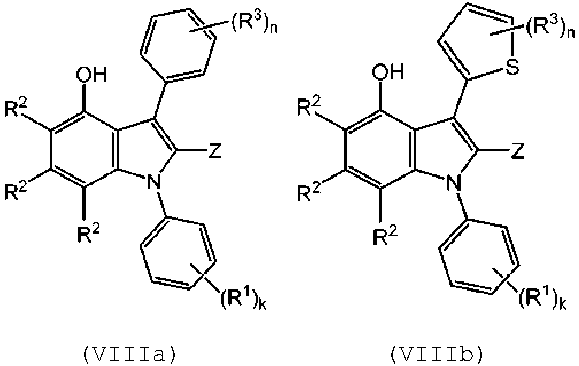 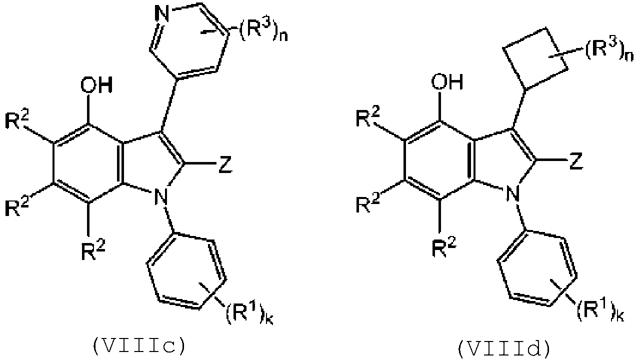 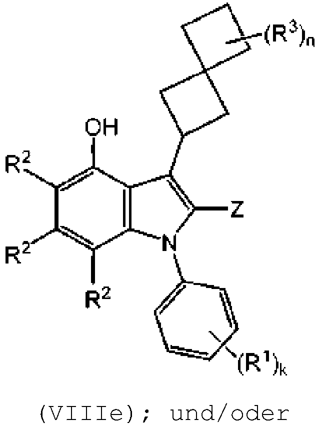
(iv) dargestellt durch eine der folgenden Strukturformeln: 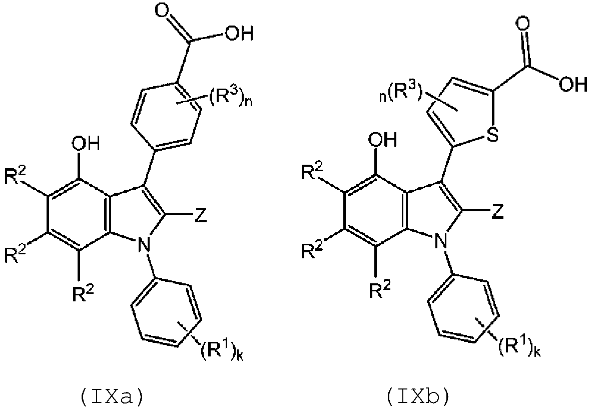 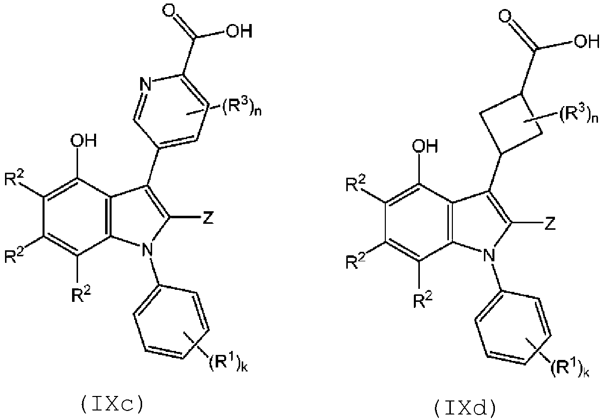 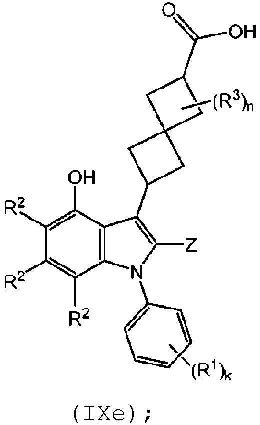
wobei **n** eine ganze Zahl ist, ausgewählt aus 0, 1 und 2;
und wobei alle anderen hierin nicht spezifisch definierten Variablen wie in einem der vorstehenden Ansprüche definiert sind.

11. Verbindung, Tautomer, deuteriertes Derivat oder pharmazeutisch verträgliches Salz nach einem der Ansprüche 1 bis 10, wobei:
(i) **R²** bei jedem Auftreten unabhängig Wasserstoff, Halogen, Cyano, C₁-C₆-Alkyl (gegebenenfalls substituiert mit 1 bis 3 Gruppen, ausgewählt aus Cyano, -OH, -OCH₃ und -NH₂), C₁-C₆-Alkoxy, C₁-C₆-Halogenalkyl, -N**R^{h}Rⁱ** oder C₃-C₆-Cycloalkyl ist; wobei **R^{h}** und **Rⁱ** bei jedem Auftreten jeweils unabhängig Wasserstoff oder C₁-C₄-Alkyl sind;
(ii) **R²** bei jedem Auftreten unabhängig Wasserstoff, Halogen, Cyano, C₁-C₄-Alkyl (gegebenenfalls substituiert mit 1 bis 3 Gruppen, ausgewählt aus Cyano, -OH, -OCH₃ und -NH₂), C₁-C₄-Alkoxy, C₁-C₄-Halogenalkyl, -N**R^{h}Rⁱ** oder C₃-C₅-Cycloalkyl ist; wobei **R^{h}** und **Rⁱ** bei jedem Auftreten jeweils unabhängig Wasserstoff oder C₁-C₄-Alkyl sind;
(iii) **R²** bei jedem Auftreten unabhängig Wasserstoff, Halogen, Cyano, C₁-C₂-Alkyl (gegebenenfalls substituiert mit 1 bis 3 Gruppen, ausgewählt aus Cyano, -OH, -OCH₃ und -NH₂), C₁-C₂-Halogenalkyl, -N**R^{h}Rⁱ** oder C₃-C₄-Cycloalkyl ist; wobei **R^{h}** und **Rⁱ** bei jedem Auftreten jeweils unabhängig Wasserstoff oder -CH₃ sind;
(iv) **R²** bei jedem Auftreten unabhängig Wasserstoff, F, Cl, Cyano, -CH₃, -CHF₂, -CF₃, -NH₂ oder Cyclopropyl ist;
(v) **R³** bei jedem Auftreten unabhängig Halogen, Cyano, =O, C₁-C₆-Alkyl, C₁-C₆-Alkoxy, C₁-C₆-Halogenalkyl, -C(=O)**R^{y}**, -C(=O)O**R^{y}**, - C(=O)N**R^{v}R^{w}**, -C(=O)N**R^{v}**O**R^{y}**, -C(=O)NR^{v}S(=O)₂**R^{y}**, -N**R^{v}R^{w}**, -O**R^{y}**, - S(=O)₂**R^{y}**, -S(=O)₂N**R^{v}R^{w}**, -S(=O)₂N**R^{v}**C(=O)**R^{y}** oder -P(=O)**R^{z}R^{z}** ist; wobei:
das C₁-C₄-Alkyl von **R³** gegebenenfalls mit 1 bis 3 Gruppen substituiert ist, ausgewählt aus Cyano, -O**R^{y}**, -C(=O)O**R^{y}** und - N**R^{v}R^{w}**; wobei:
**R^{v}**, **R^{w}** und **R^{x}** bei jedem Auftreten jeweils unabhängig Wasserstoff oder C₁-C₂-Alkyl sind; wobei das C₁-C₂-Alkyl eines von **R^{v}**, **R^{w}** und
**R^{x}** gegebenenfalls mit 1 bis 3 Gruppen substituiert ist, ausgewählt aus Halogen, Cyano, -OH und -NH₂; und
**R^{y}** bei jedem Auftreten unabhängig Wasserstoff, C₁-C₄-Alkyl oder 5- oder 6-gliedriges Heterocyclyl ist; wobei:
das C₁-C₄-Alkyl von **R^{y}** gegebenenfalls mit 1 bis 3 Gruppen substituiert ist, ausgewählt aus Halogen, Cyano, -OH, -NH₂ und -C(=O)OH; und
das 5- oder 6-gliedrige Heterocyclyl von **R^{y}** gegebenenfalls mit 1 bis 3 Gruppen substituiert ist, ausgewählt aus Halogen, Cyano, -OH, -NH₂ und -C(=O)OH;
(vi) **R³** bei jedem Auftreten unabhängig Halogen, Cyano, =O, C₁-C₄-Alkyl, C₁-C₄-Alkoxy, C₁-C₄-Halogenalkyl, -C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)₂**R^{y}**, -N**R^{v}R^{w}**, -O**R^{y}**, - S(=O)₂**R^{y}**, -S(=O)₂N**R^{v}R^{w}**, -S(=O)₂N**R^{v}**C(=O)**R^{y}** oder -P(=O)**R^{z}R^{z}** ist; wobei:
das C₁-C₄-Alkyl von **R³** gegebenenfalls mit 1 bis 3 Gruppen substituiert ist, ausgewählt aus Cyano, -O**R^{y}** und -C(=O)O**R^{y}**; wobei: **R^{v}**, **R^{w}** und **R^{x}** bei jedem Auftreten jeweils unabhängig Wasserstoff oder C₁-C₄-Alkyl sind; wobei das C₁-C₄-Alkyl eines von **R^{v}**, **R^{w}** und
**R^{x}** gegebenenfalls mit -OH substituiert ist; und
**R^{y}** bei jedem Auftreten unabhängig Wasserstoff, C₁-C₂-Alkyl oder 6-gliedriges Heterocyclyl ist; wobei:
das C₁-C₂-Alkyl von **R^{y}** gegebenenfalls mit 1 bis 3 Gruppen substituiert ist, ausgewählt aus -OH und -C(=O)OH; und das 6-gliedrige Heterocyclyl von **R^{y}** gegebenenfalls mit 1 bis 3 Gruppen substituiert ist, ausgewählt aus -OH und -C(=O)OH;
**R^{z}** bei jedem Auftreten unabhängig -CH₃, -OH oder -OCH₃ ist;
(vii) **R³** bei jedem Auftreten unabhängig Halogen, Cyano, =O, C₁-C₂-Alkyl, C₁-C₂-Alkoxy, C₁-C₄-Halogenalkyl, -C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)₂**R^{y}**, -N**R^{v}R^{w}**, -O**R^{y}**, - S(=O)₂**R^{y}**, -S(=O)₂N**R^{v}R^{w}**, -S(=O)₂N**R^{v}**C(=O)**R^{y}** oder -P(=O)**R^{z}R^{z}** ist; wobei:
das C₁-C₂-Alkyl von **R³** gegebenenfalls mit 1 bis 3 Gruppen substituiert ist, ausgewählt aus -OH und -C(=O)O**R^{y}**; wobei:
**R^{v}**, **R^{w}** und **R^{x}** bei jedem Auftreten jeweils unabhängig Wasserstoff oder C₁-C₂-Alkyl sind; wobei das C₁-C₂-Alkyl eines von **R^{v}**, **R^{w}** und
**R^{x}** gegebenenfalls mit -OH substituiert ist; und
**R^{y}** bei jedem Auftreten unabhängig Wasserstoff, C₁-C₂-Alkyl oder Tetrahydro-2H-pyranyl ist; wobei:
das C₁-C₂-Alkyl von **R^{y}** gegebenenfalls mit -C(=O)OH substituiert ist; und
das Tetrahydro-2H-pyranyl von **R^{y}** gegebenenfalls mit 1 bis 3 Gruppen substituiert ist, ausgewählt aus -OH und -C(=O)OH;
**R^{z}** bei jedem Auftreten unabhängig -CH₃ oder -OH ist;
(viii) **R³** bei jedem Auftreten unabhängig F, Cl, Cyano, -OH, =O, -CH₃, -OCH₃, -CF₃, -CH₃CN, -C(CH₃)₂CH₂OH, -CH₂COOH, -CH₂OCH₃, - C(=O)CHCH₃OH, -COOH, -C(=O)O(2-Tetrahydro-2H-pyranyl), -C(=O)NH₂, -C(=O)NH(CH₂)₂OH, -C(=O)NHOH, -C(=O)NHS(=O)₂CH₃, -NH₂, -NHCH₃, - OCH₂COOH, NHS(=O)₂CH₃, -S(=O)₂CH₃, -S(=O)₂NH₂, -S(=O)₂NHC(=O)CH₃ oder -P(=O)(CH₃)₂ ist; wobei das 2-Tetrahydro-2H-pyranyl in - C(=O)O(2-Tetrahydro-2H-pyranyl) mit 1 bis 3 Gruppen substituiert ist, ausgewählt aus -OH und -C(=O)OH;
(ix) **R⁴** bei jedem Auftreten unabhängig Halogen, Cyano, C₁-C₆-Alkyl, C₁-C₆-Alkoxy, C₁-C₆-Halogenalkyl, -C(=O)**R^{y}**, -C(=O)O**R^{y}**, - O**R^{y}** oder -S(=O)₂**R^{y}** ist; wobei:
das C₁-C₆-Alkyl von **R⁴** gegebenenfalls mit 1 bis 3 Gruppen substituiert ist, ausgewählt aus Cyano, -O**R^{y}**, -C(=O)O**R^{y}** und - N**R^{v}R^{w}**; wobei:
**R^{v}** und **R^{w}** bei jedem Auftreten jeweils unabhängig Wasserstoff oder C₁-C₄-Alkyl sind; und
**R^{y}** bei jedem Auftreten unabhängig Wasserstoff oder C₁-C₄-Alkyl ist; wobei:
das C₁-C₄-Alkyl von **R^{y}** gegebenenfalls mit 1 bis 3 Gruppen substituiert ist, ausgewählt aus Halogen, Cyano, -OH, -OCH₃ und -NH₂;
(x) **R⁴** bei jedem Auftreten unabhängig Halogen, Cyano, C₁-C₄-Alkyl, C₁-C₄-Alkoxy, C₁-C₄-Halogenalkyl, -C(=O)**R^{y}**, -C(=O)O**R^{y}**, -O**R^{y}** oder -S(=O)₂**R^{y}** ist; wobei:
das C₁-C₄-Alkyl von **R⁴** gegebenenfalls mit 1 bis 3 Gruppen substituiert ist, ausgewählt aus Cyano, -O**R^{y}**, -C(=O)O**R^{y}** und - N**R^{v}R^{w}**; wobei:
**R^{v}** und **R^{w}** bei jedem Auftreten jeweils unabhängig Wasserstoff oder C₁-C₄-Alkyl sind;
**R^{y}** bei jedem Auftreten unabhängig Wasserstoff oder C₁-C₂-Alkyl ist; wobei:
das C₁-C₂-Alkyl eines von **R^{y}** gegebenenfalls mit 1 bis 3 Gruppen substituiert ist, ausgewählt aus Halogen, Cyano, -OH, -OCH₃ und -NH₂; und
wobei **o** oder **m** eine ganze Zahl ist, ausgewählt aus 0, 1 und 2;
(xi) **R⁴** bei jedem Auftreten unabhängig Cyano, C₁-C₂-Alkyl, C₁-C₂-Alkoxy, C₁-C₂-Halogenalkyl, -C(=O)R^{y}, -C(=O)O**R^{y}**, -O**R^{y}** oder - S(=O)₂**R^{y}** ist; wobei:
das C₁-C₂-Alkyl von **R⁴** gegebenenfalls mit Cyano, -OH oder -OCH₃ substituiert ist;
**R^{y}** bei jedem Auftreten unabhängig Wasserstoff oder C₁-C₂-Alkyl ist; wobei:
das C₁-C₂-Alkyl von **R^{y}** gegebenenfalls mit -OCH₃ substituiert ist; wobei **o** oder **m** eine ganze Zahl, ausgewählt aus 0 und 1, ist; und/oder
(xii) **R⁴** bei jedem Auftreten unabhängig Cyano, -OH, -OCH₃, -CH₃, -C₂H₅, -CF₃, -CH₂CN, -CH₂OH, -CH₂OCH₃, -COOH, -C(=O)CH₃, -C(=O)OCH₃, -C(=O)CH₂OCH₃, -S(=O)₂CH₃, -S(=O)₂C₂H₅ oder S(=O)₂CF₃ ist; und wobei alle anderen, hierin nicht spezifisch definierten Variablen wie in einem der vorangehenden Ansprüche definiert sind.

12. Verbindung nach Anspruch 1, wobei die Verbindung ausgewählt ist aus: 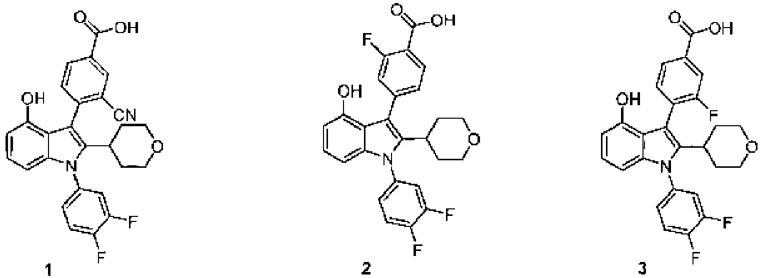 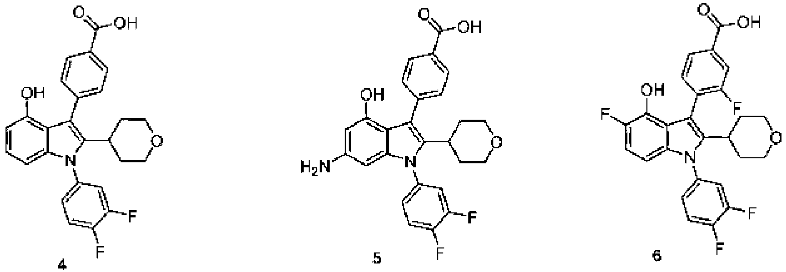 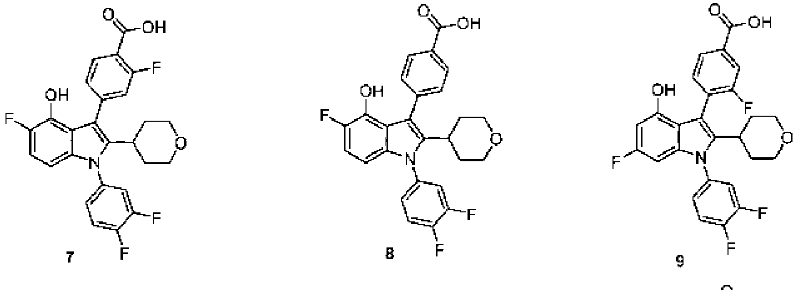 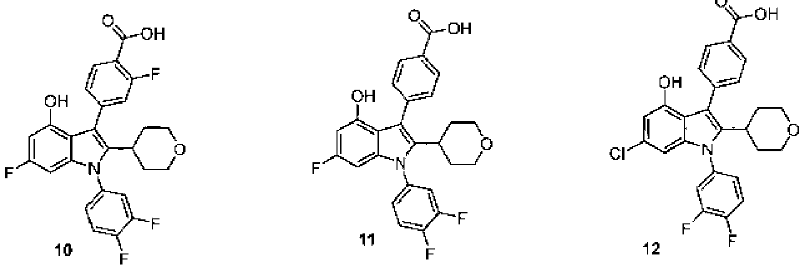 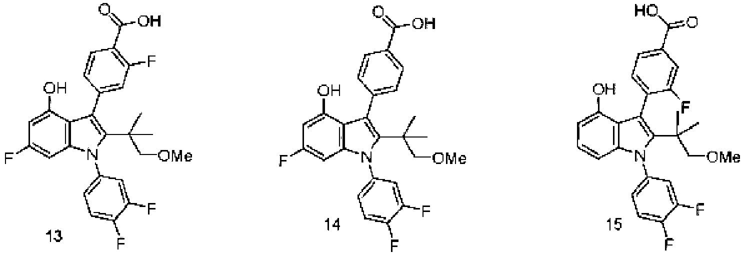 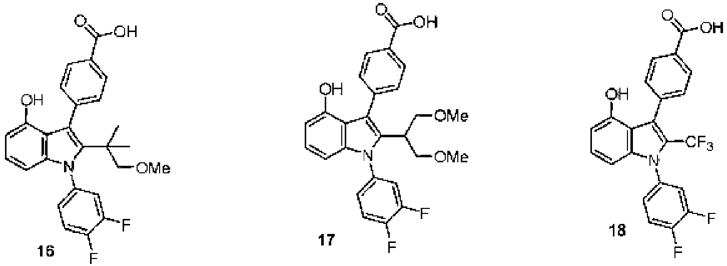 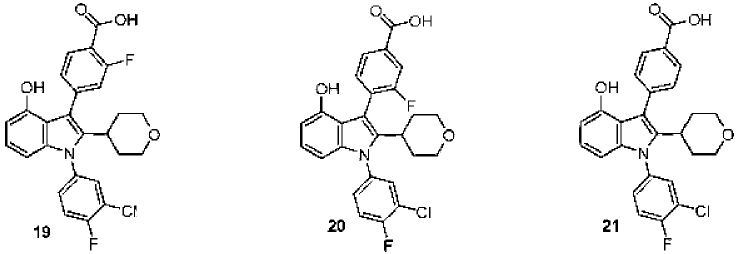 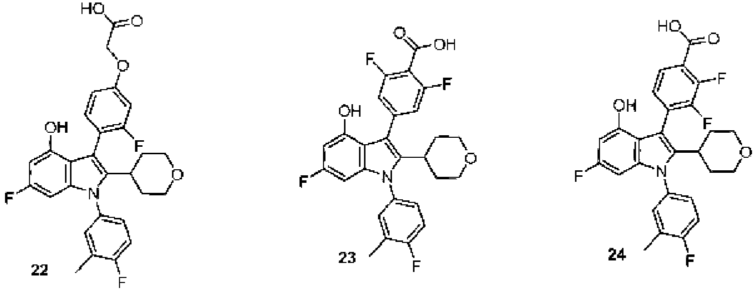 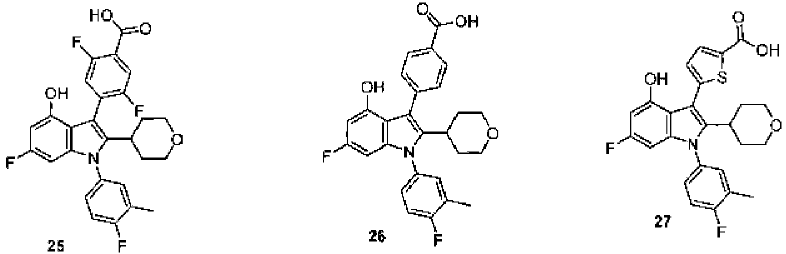 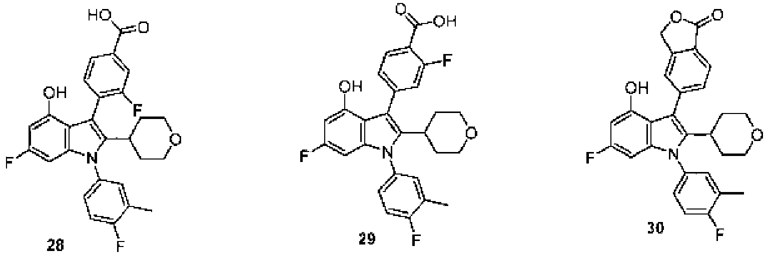 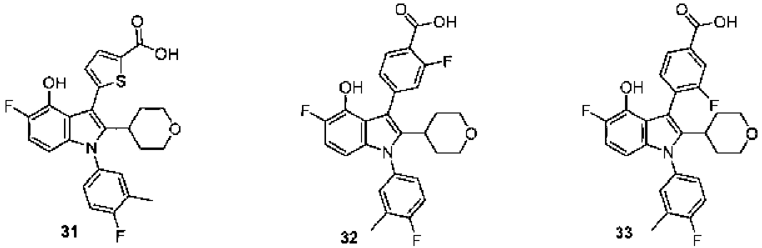 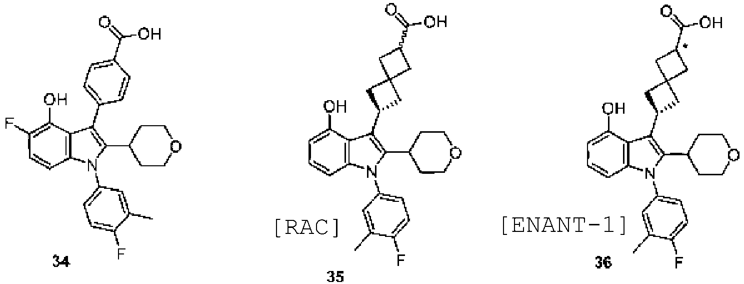 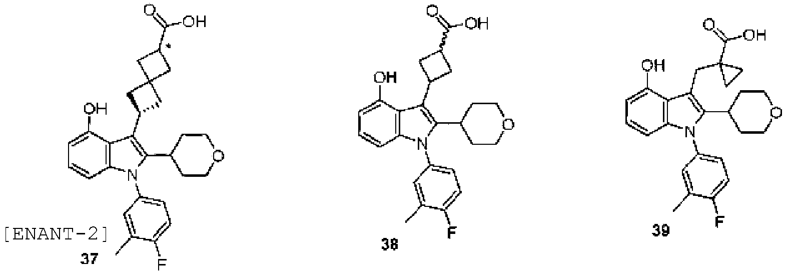 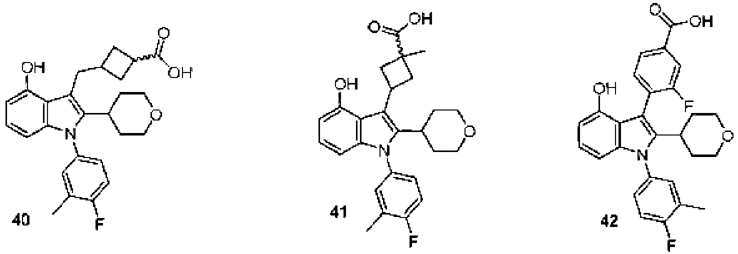 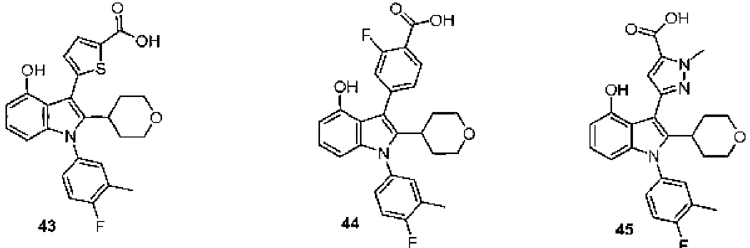 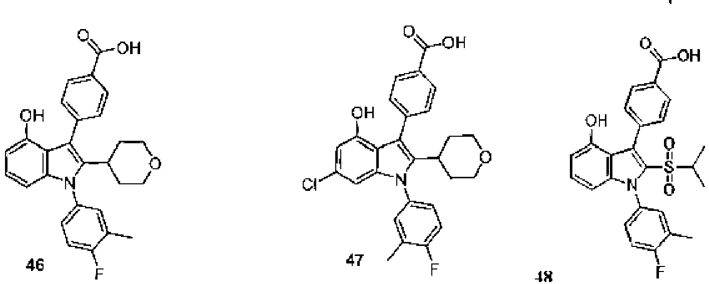 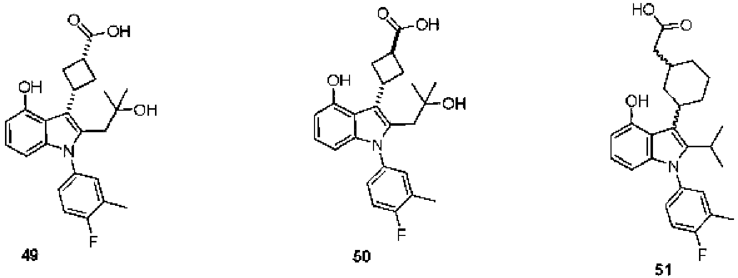 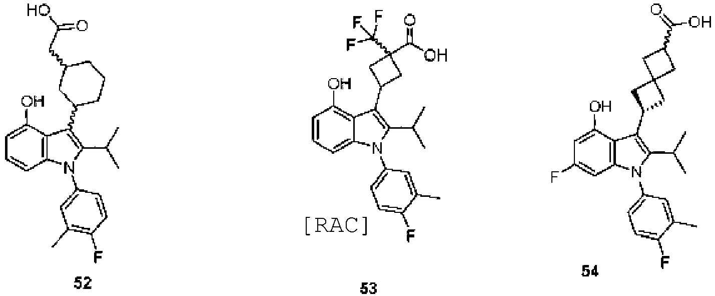 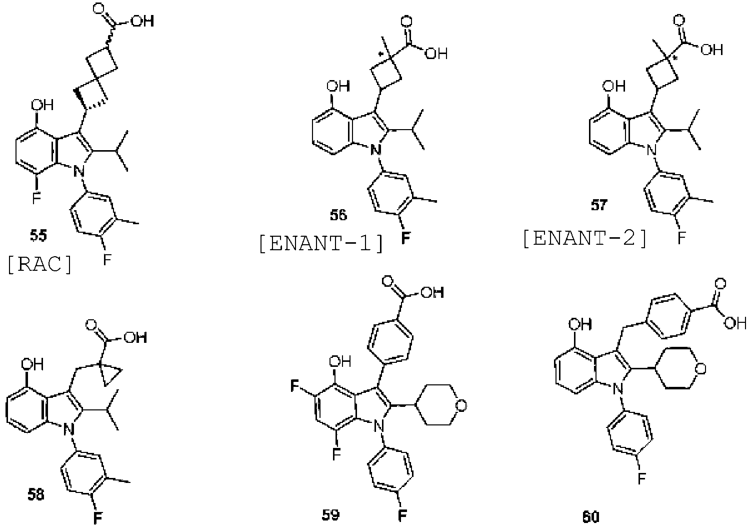 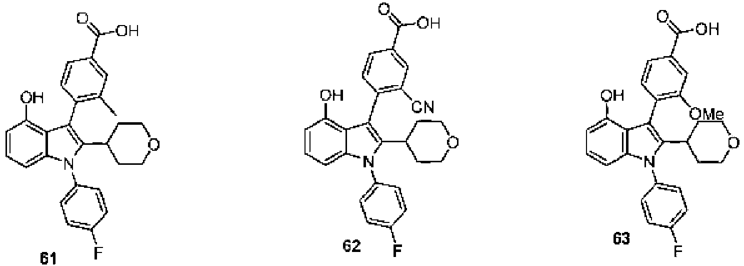 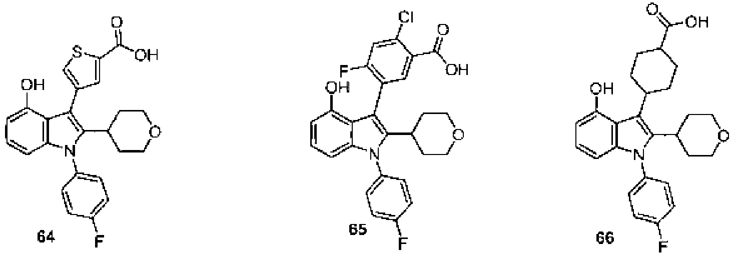 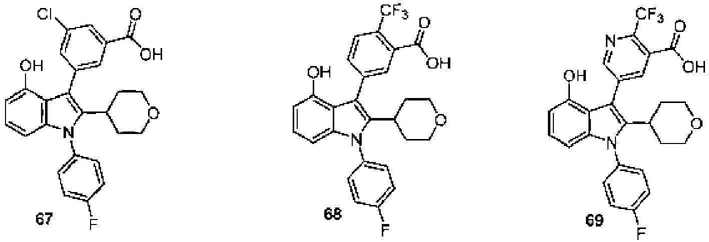 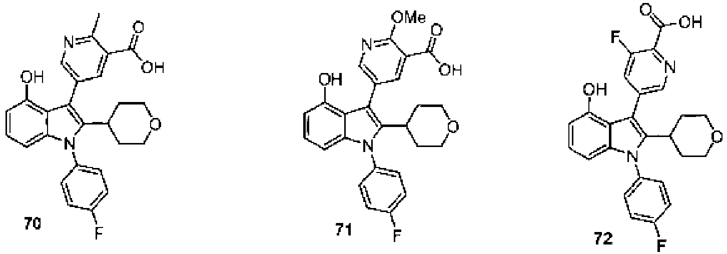 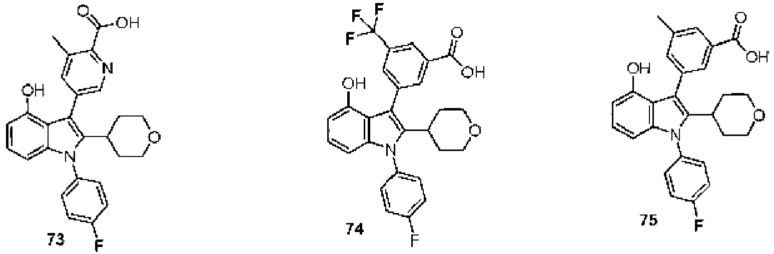 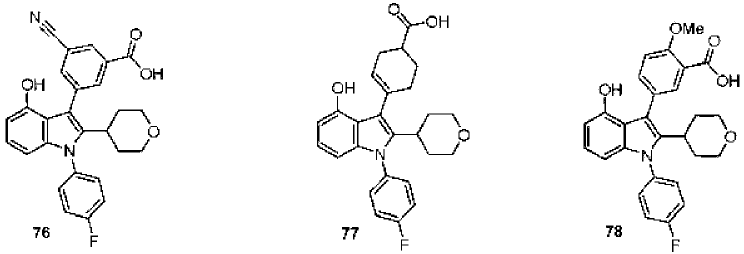 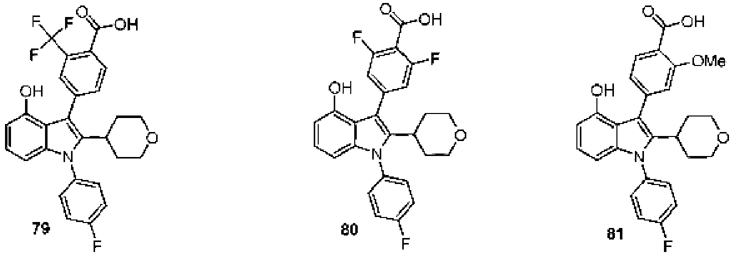 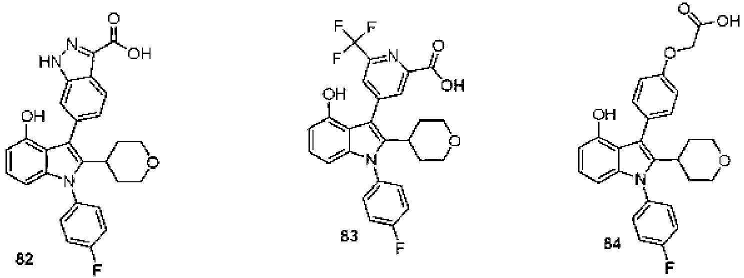 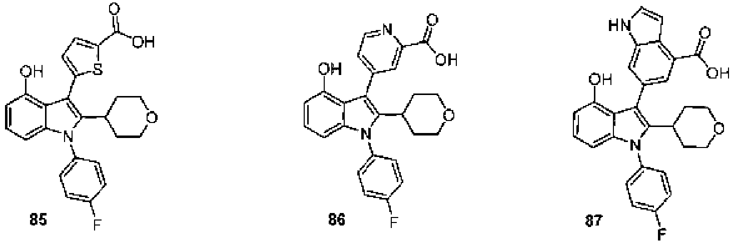 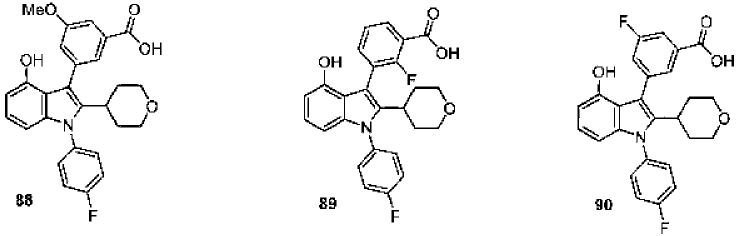 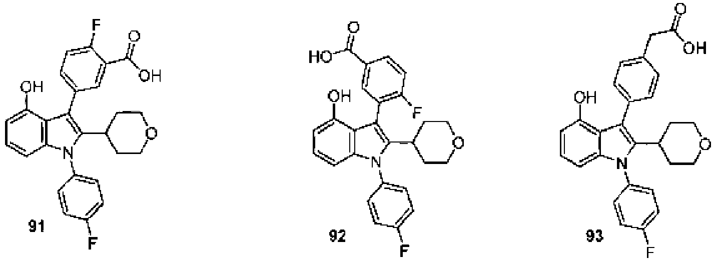 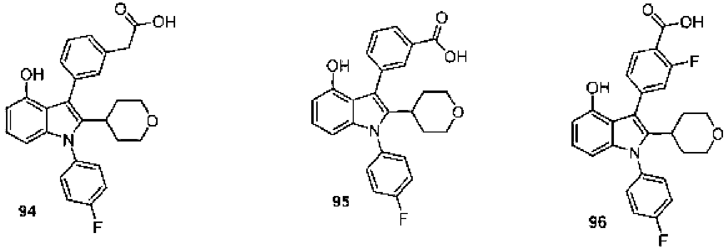 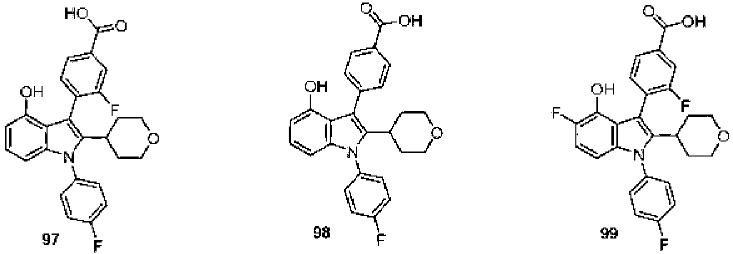 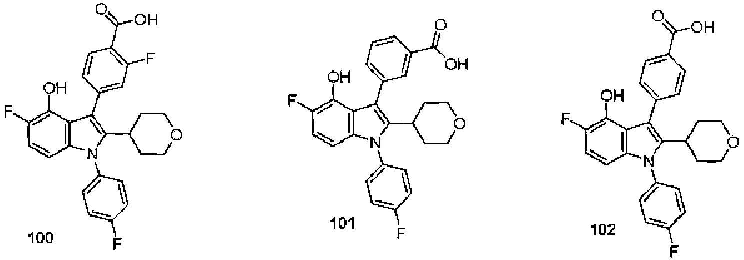 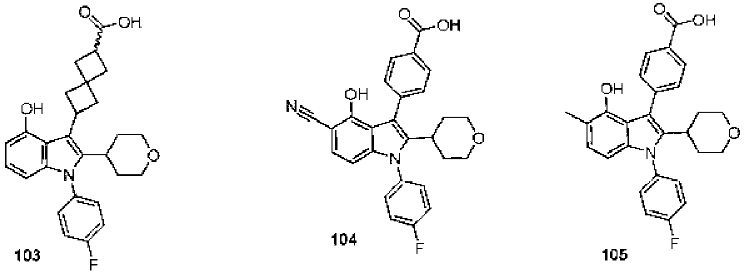 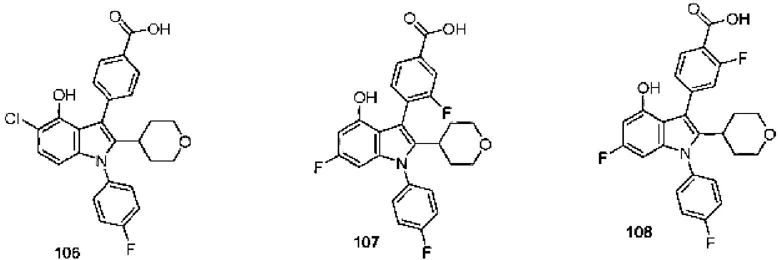 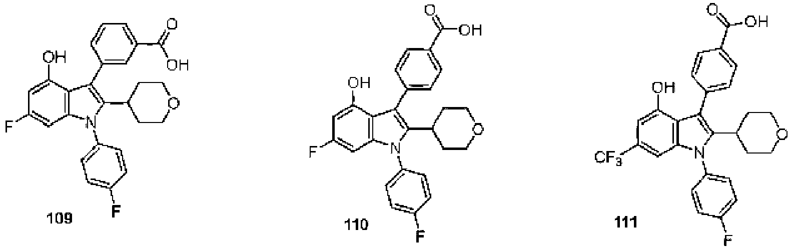 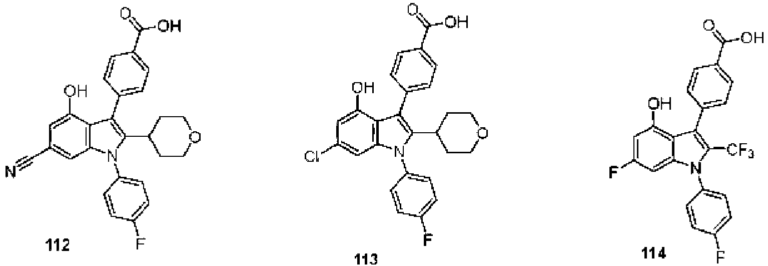 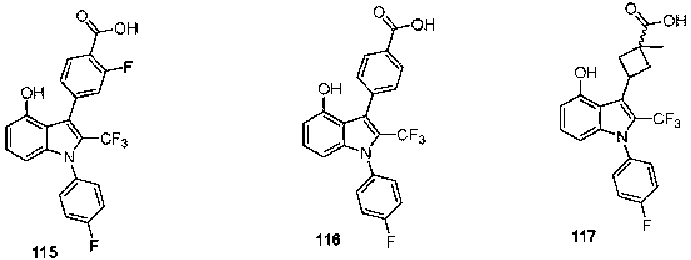 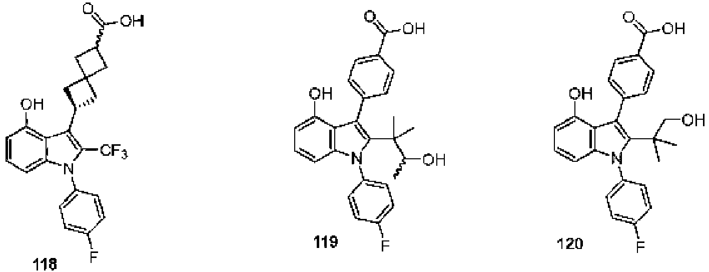 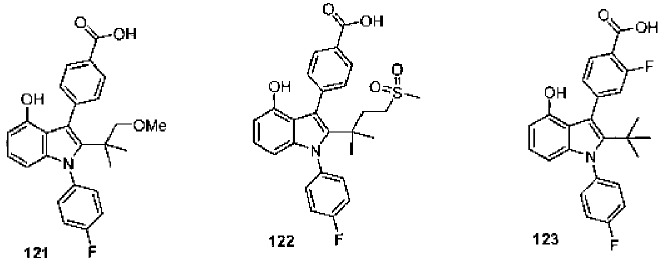 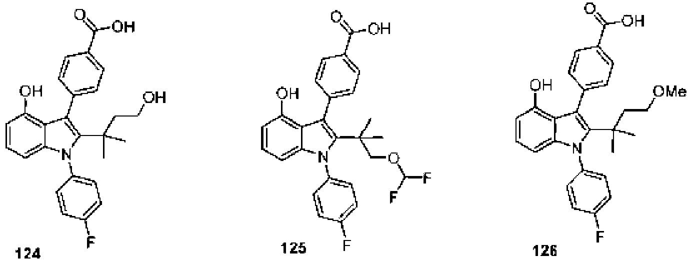 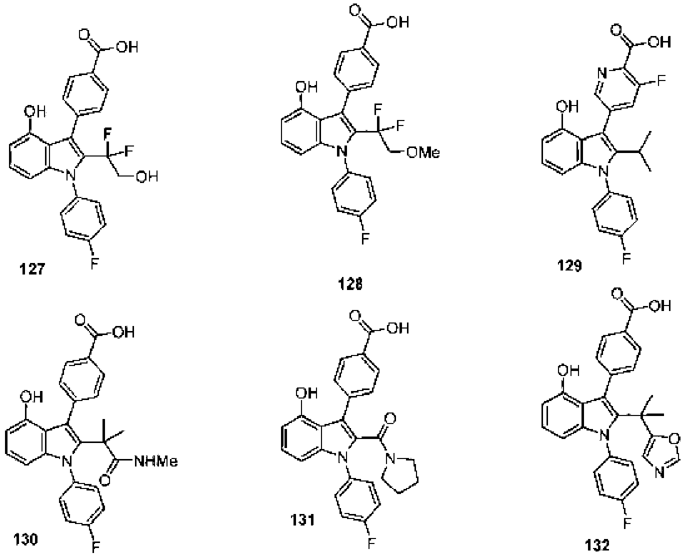 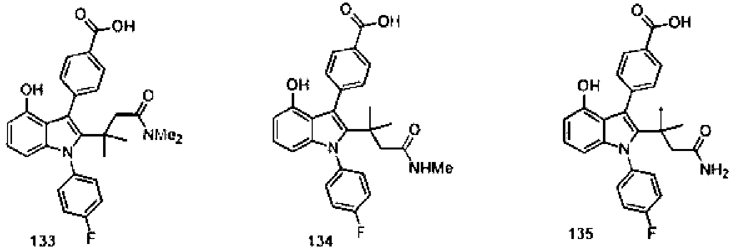 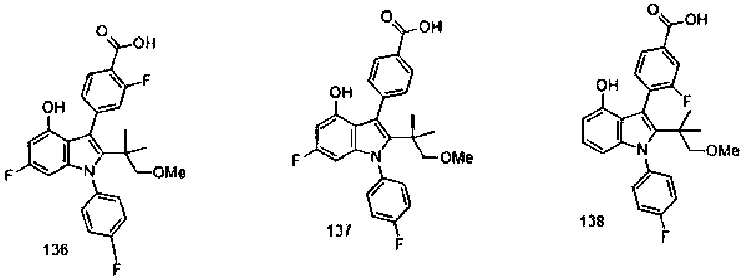 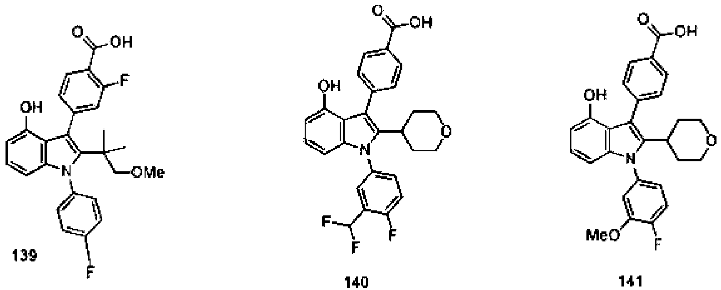 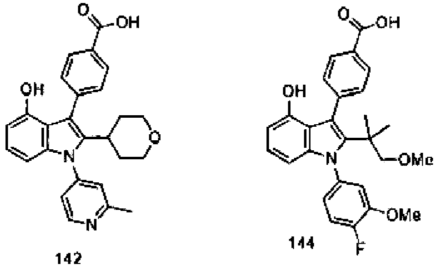 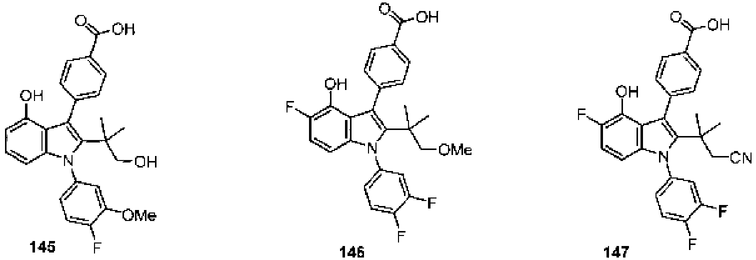 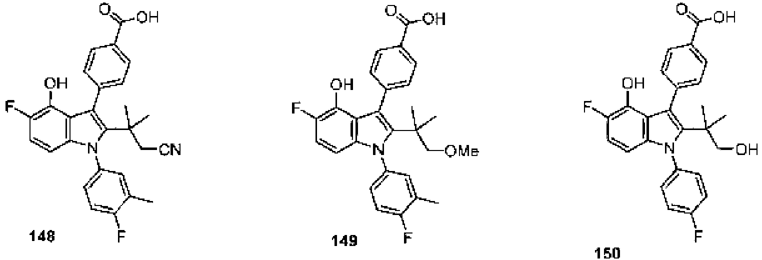 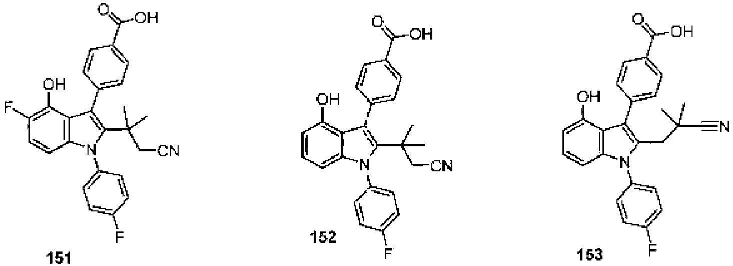 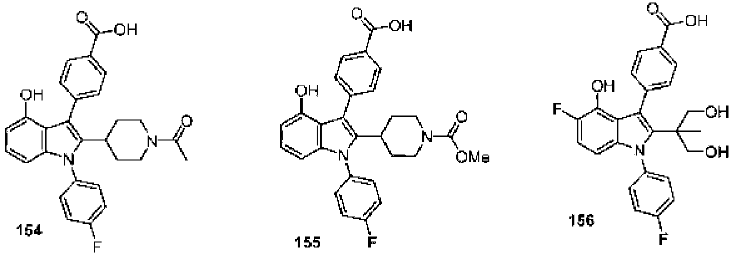 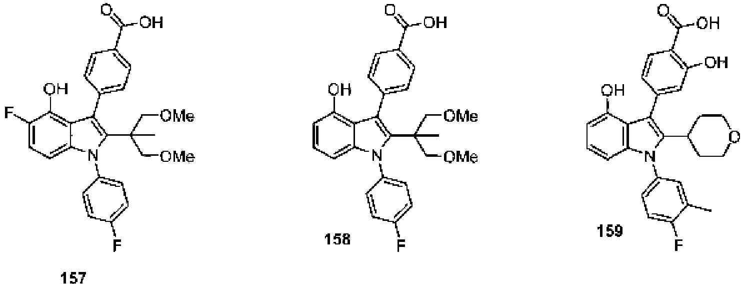 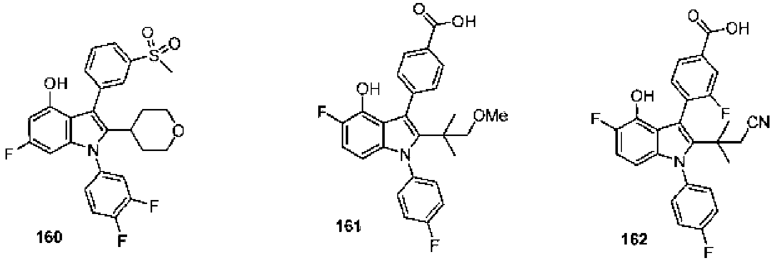 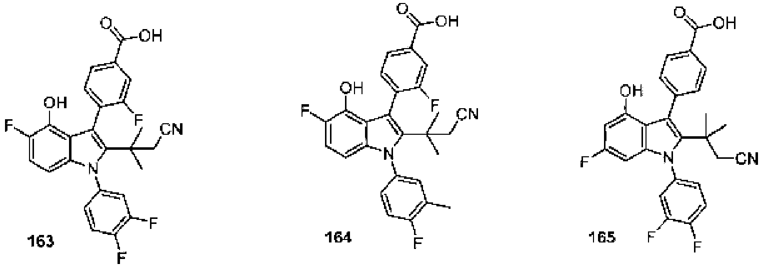 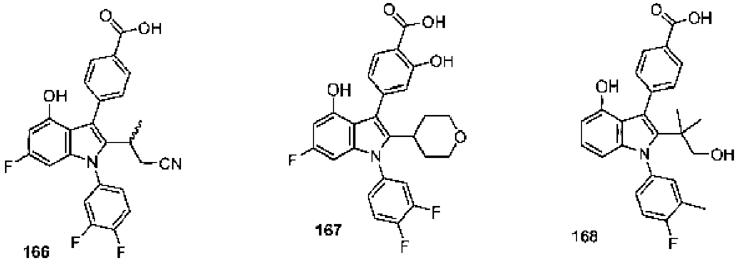 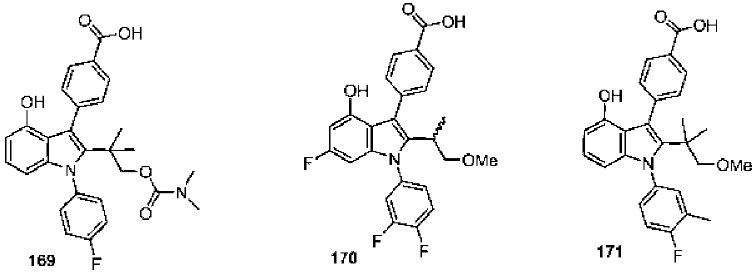 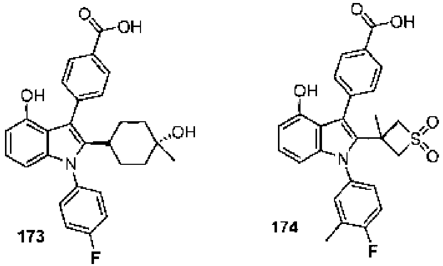 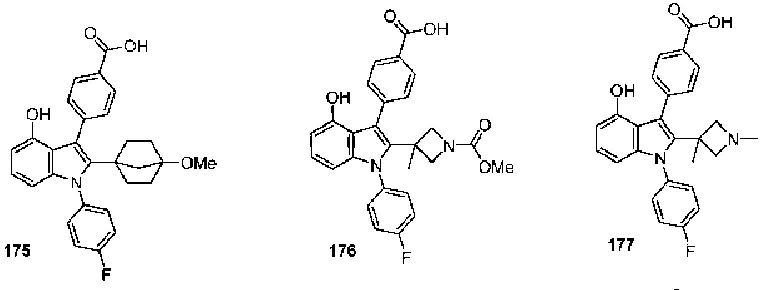 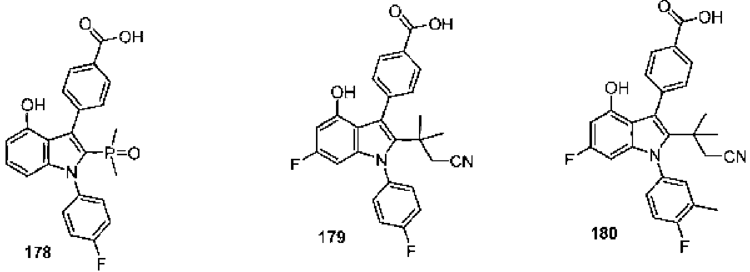 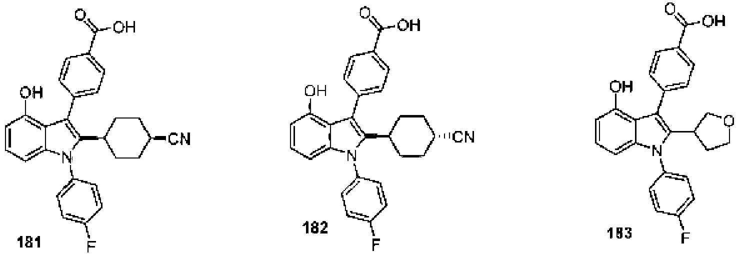 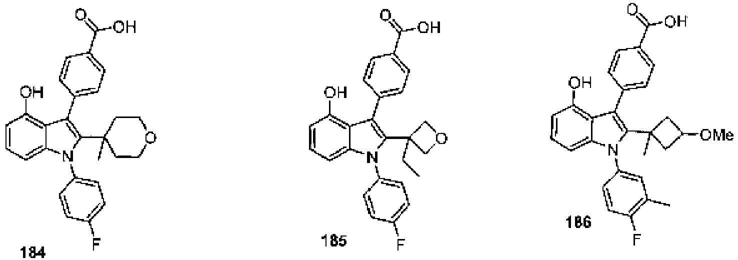 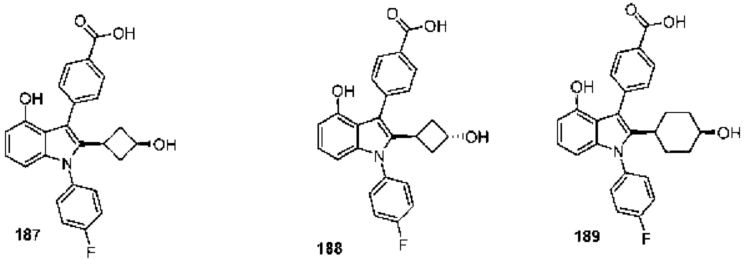 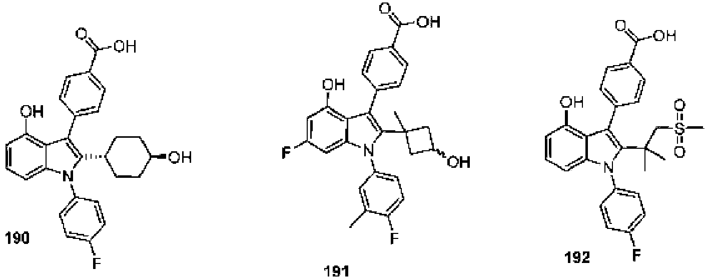 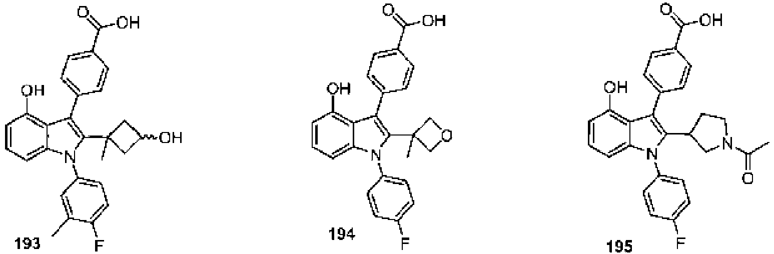 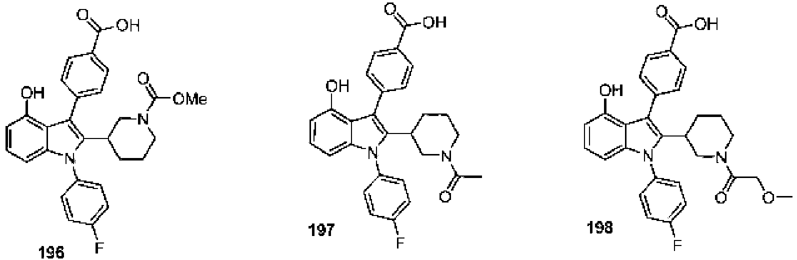 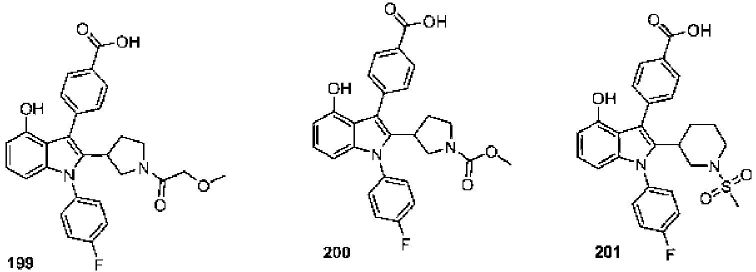 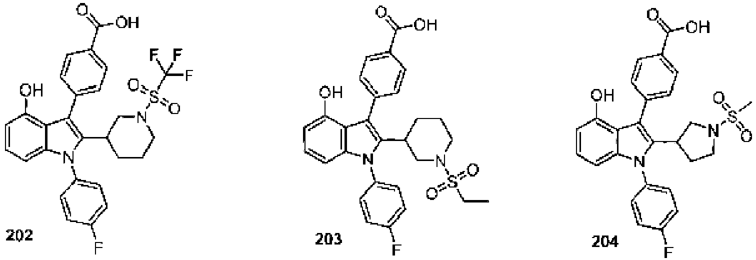 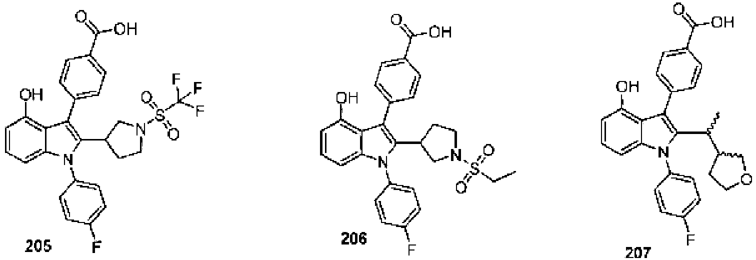 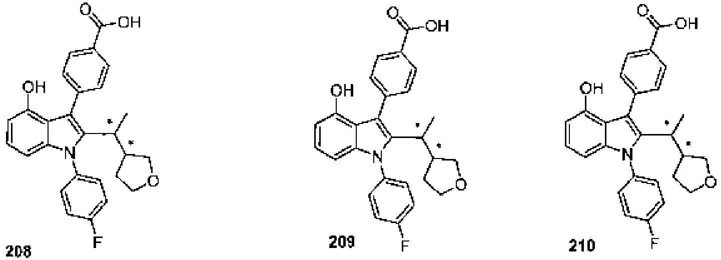 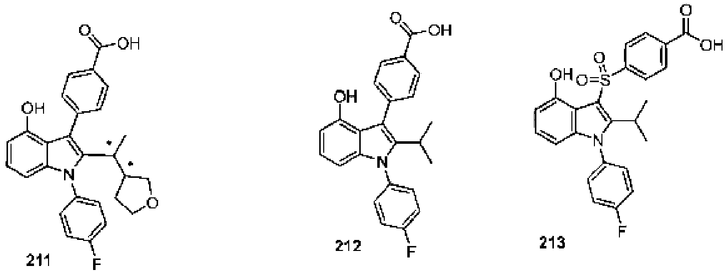 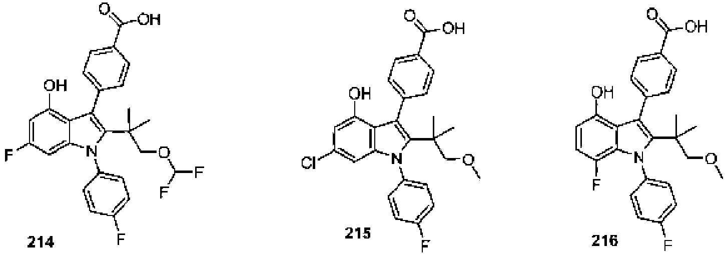 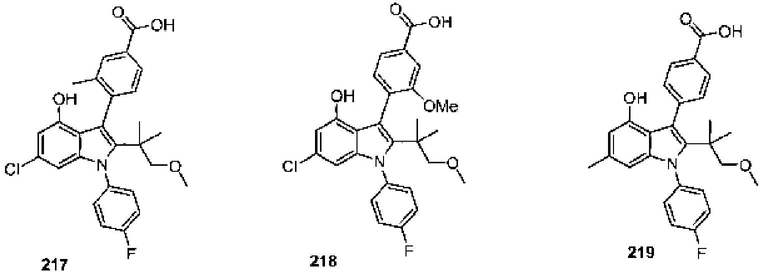 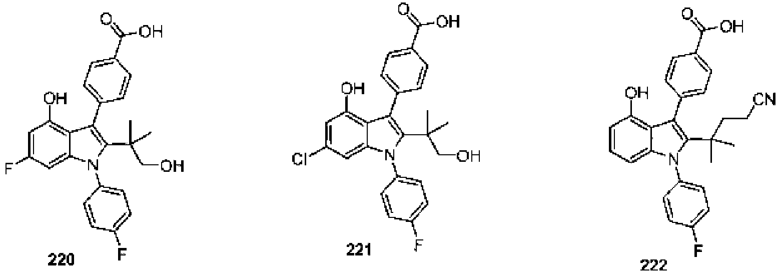 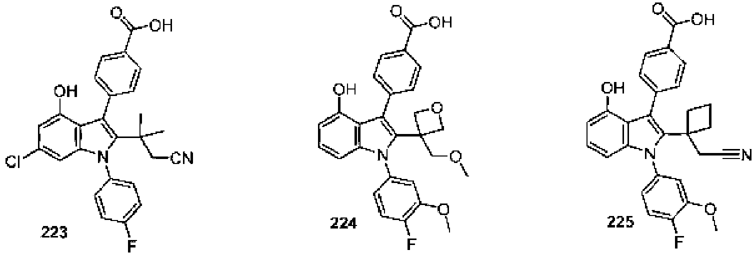 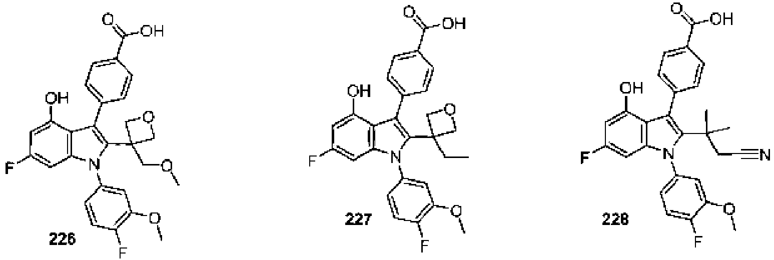 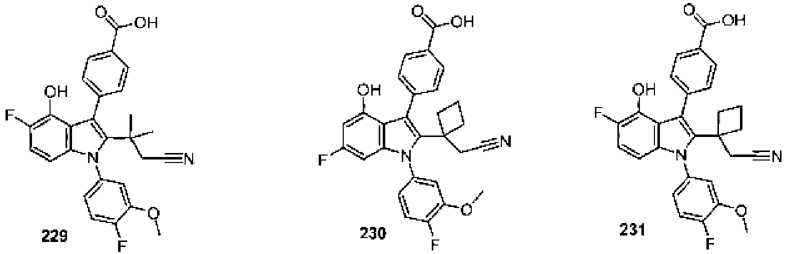 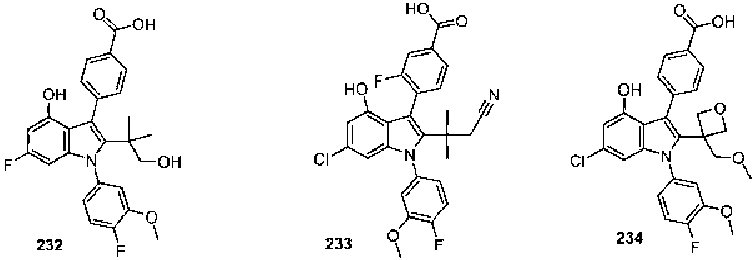 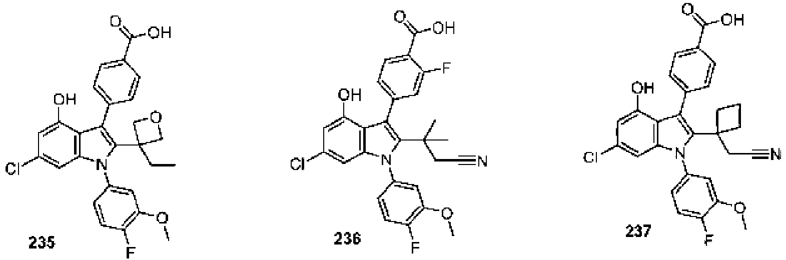 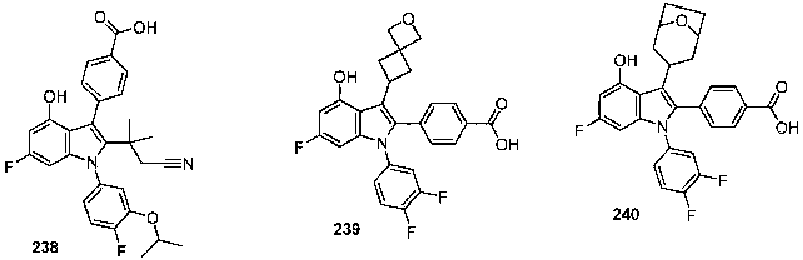 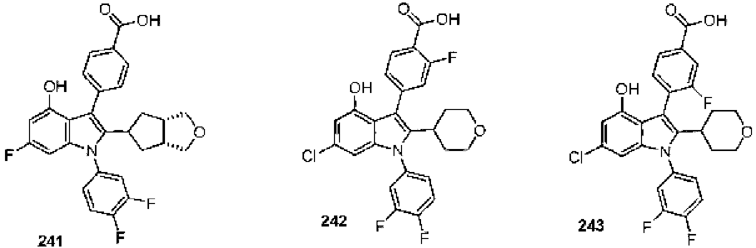 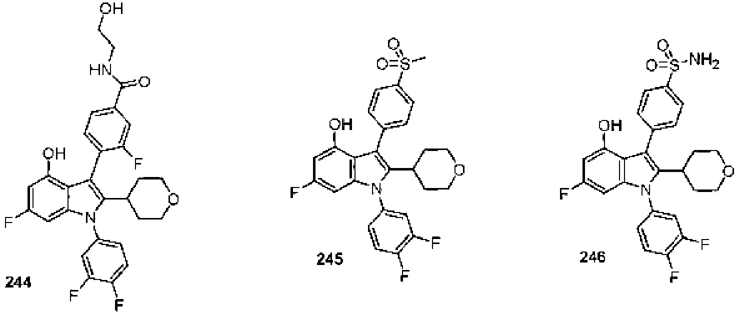 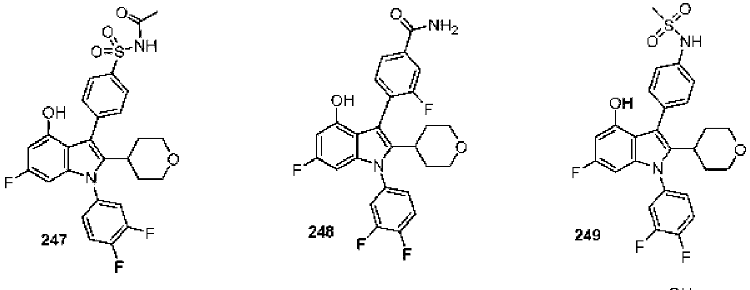 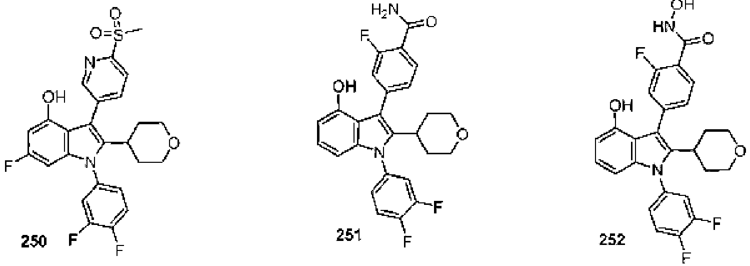 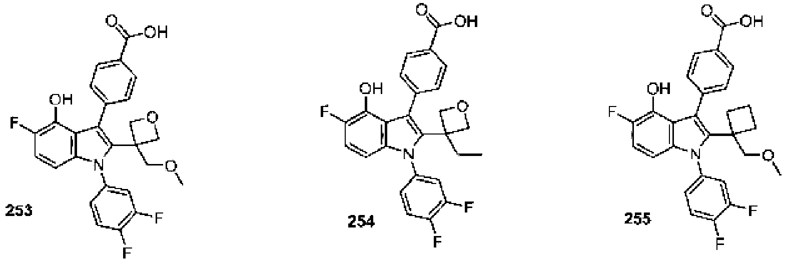 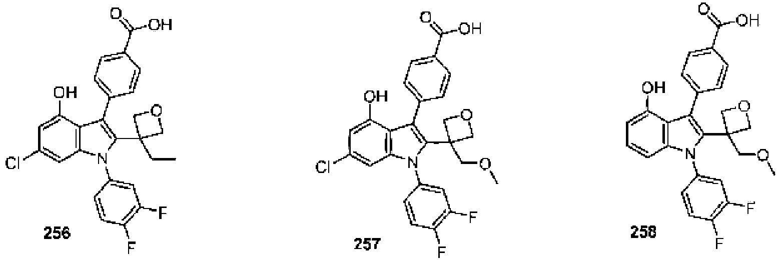 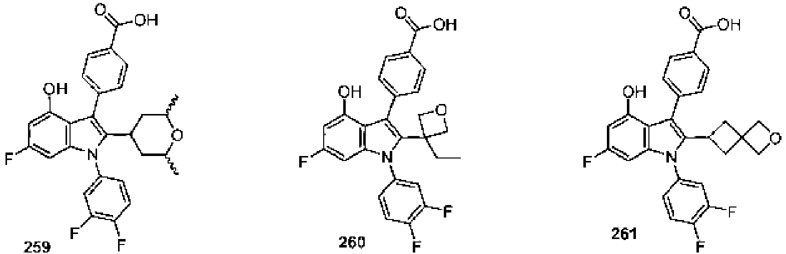 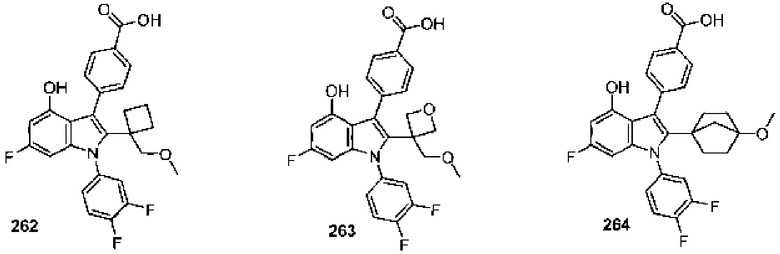 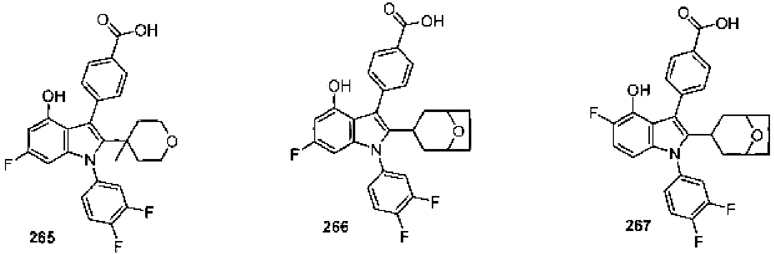 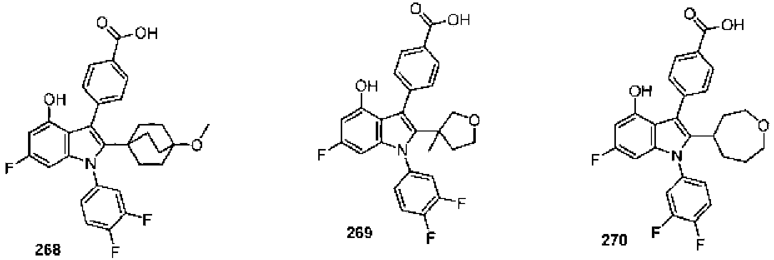 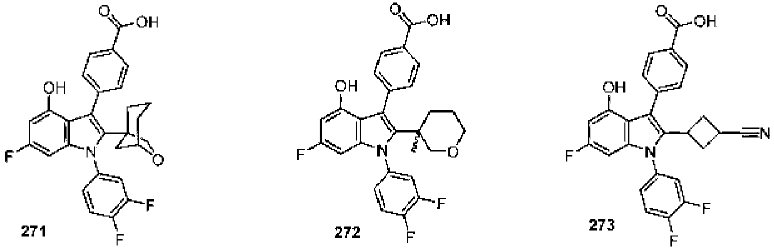 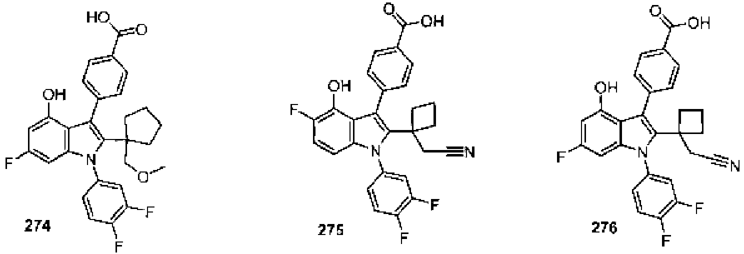 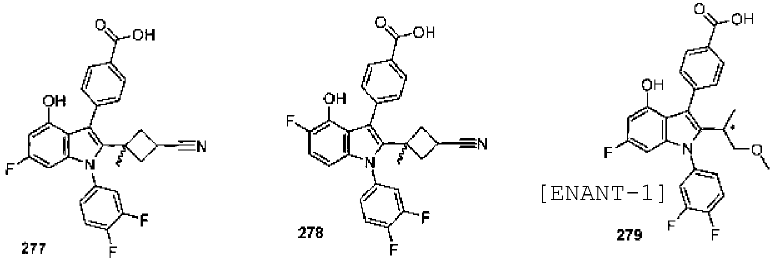 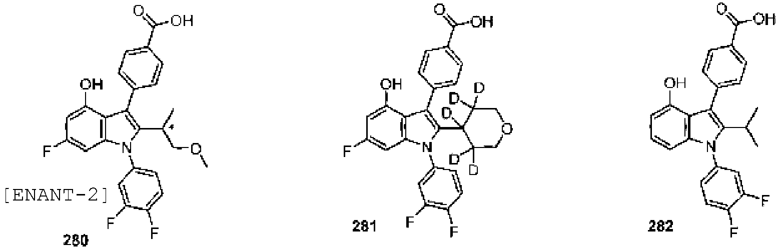 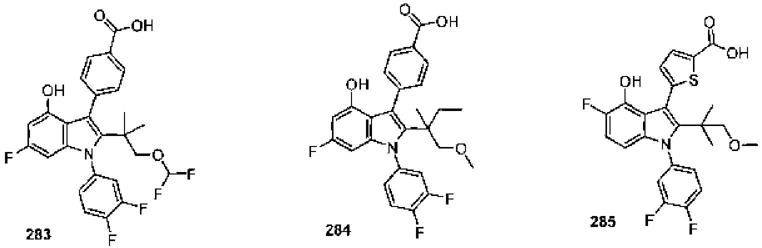 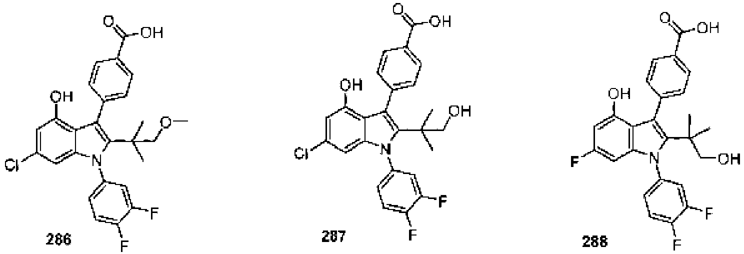 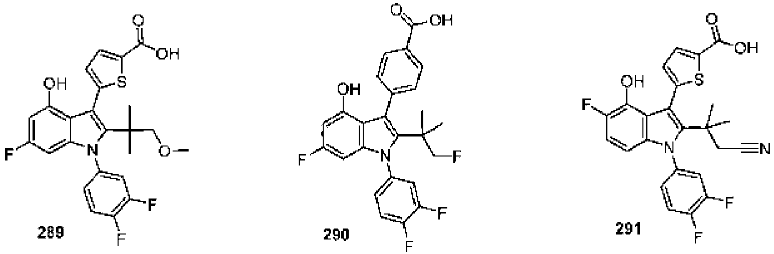 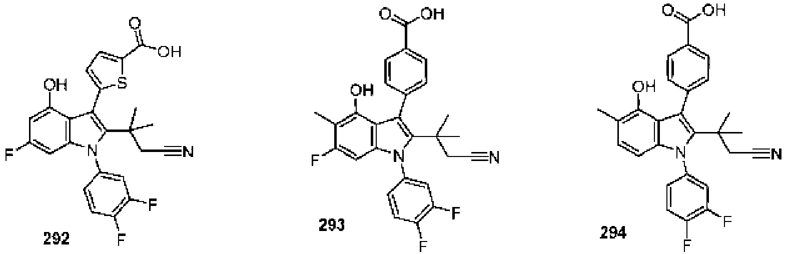 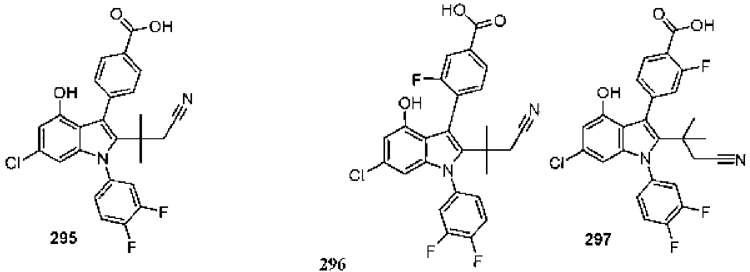 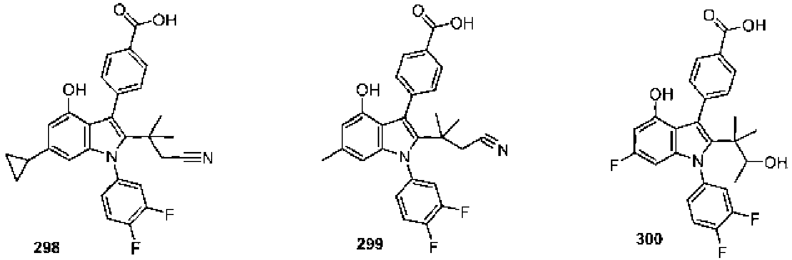 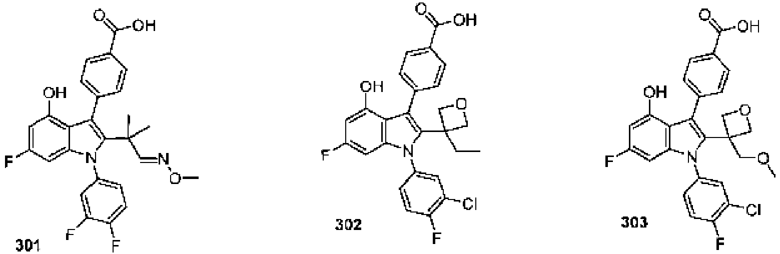 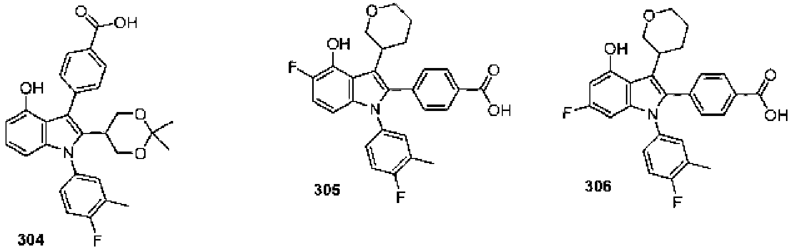 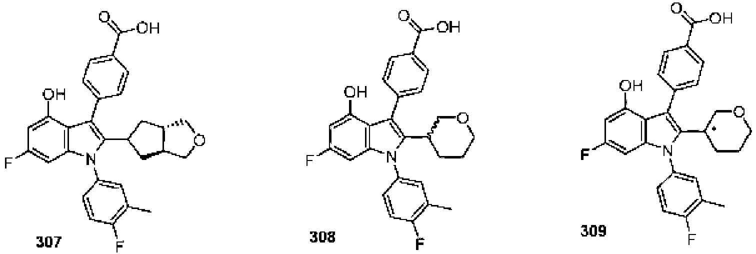 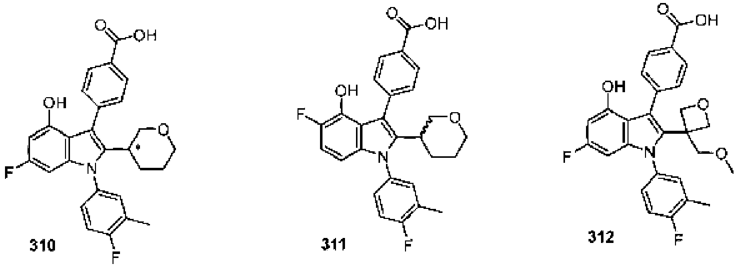 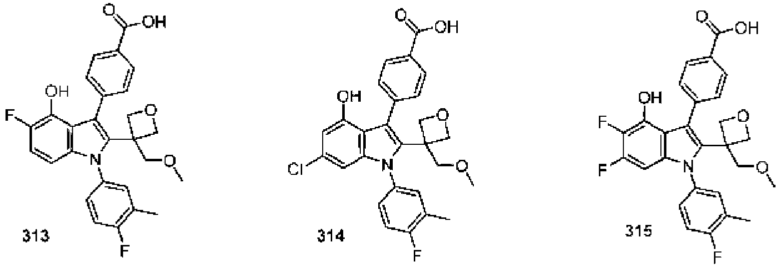 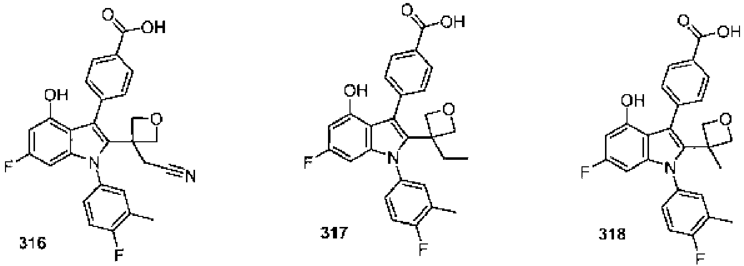 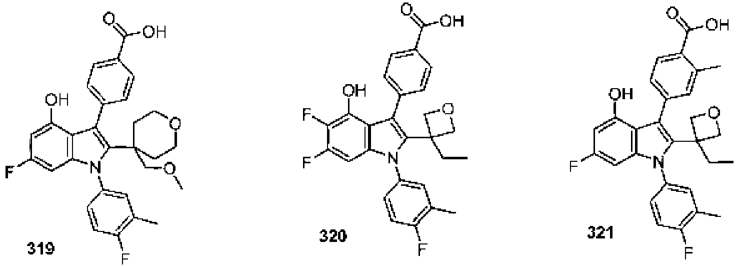 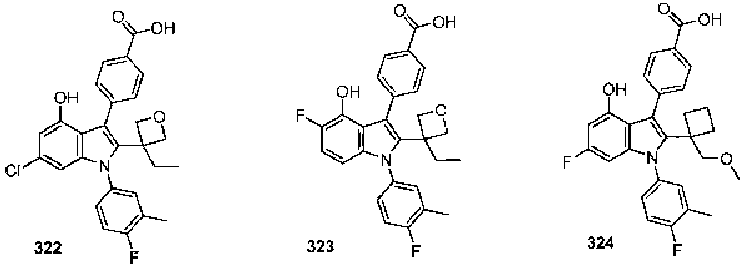 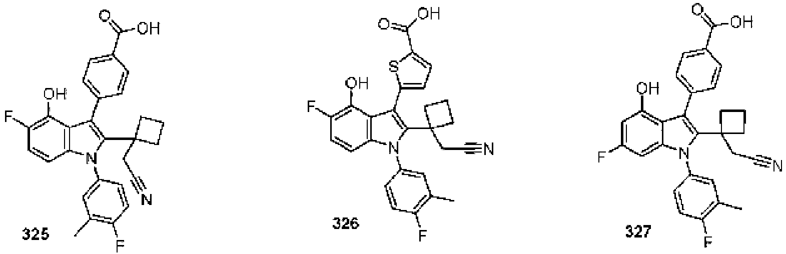 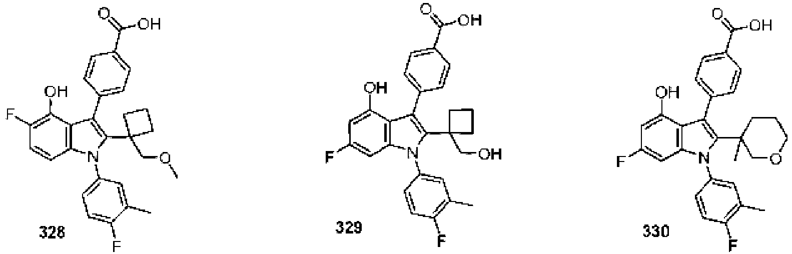 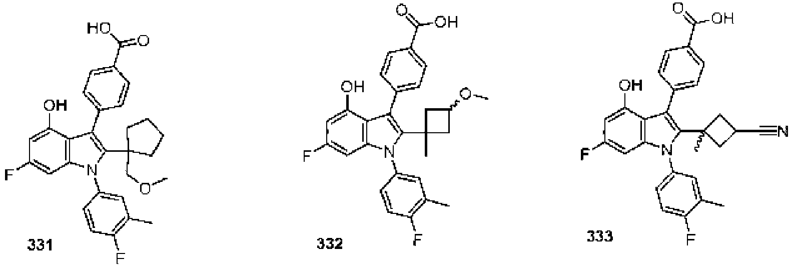 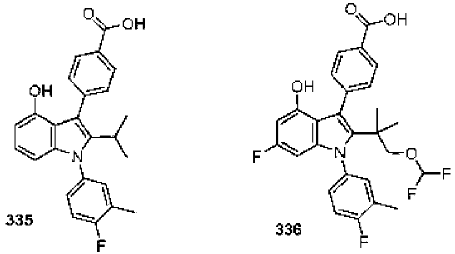 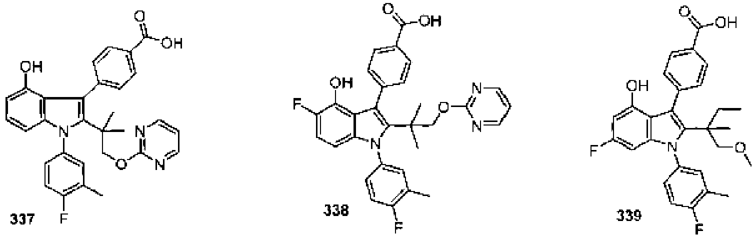 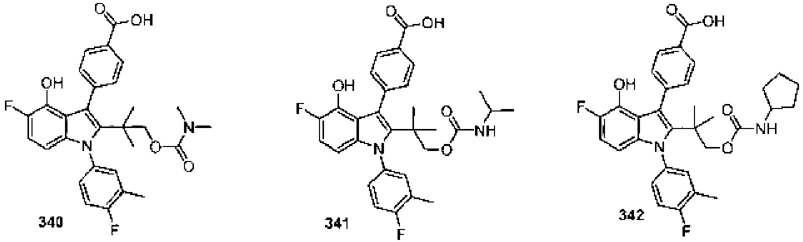 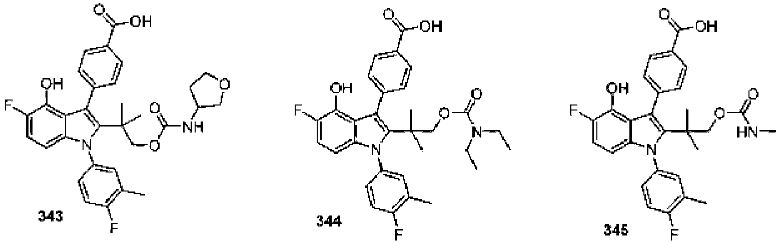 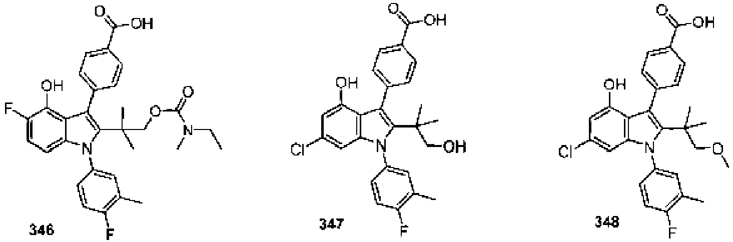 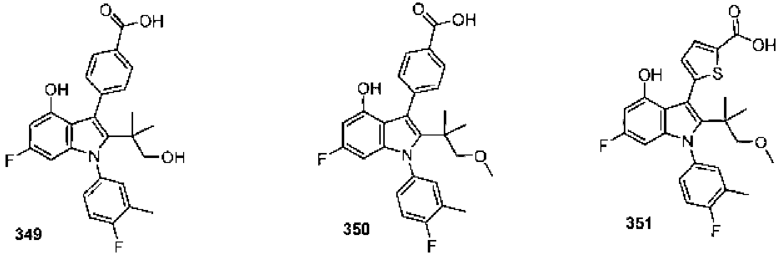 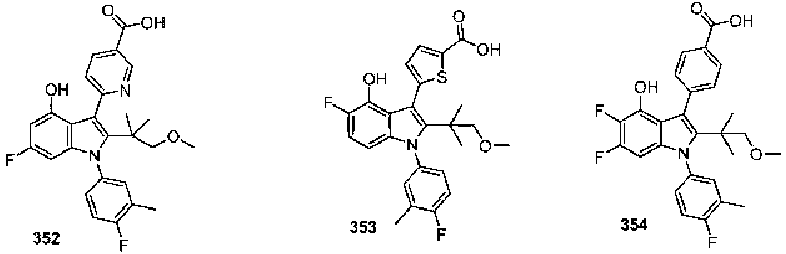 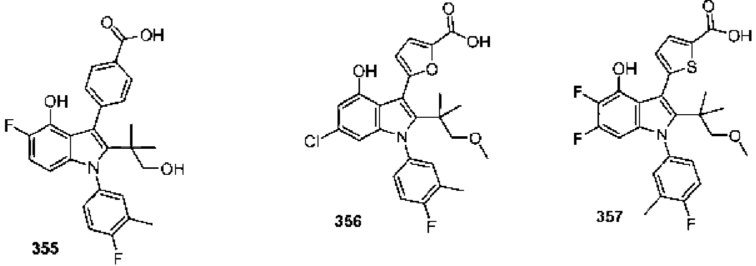 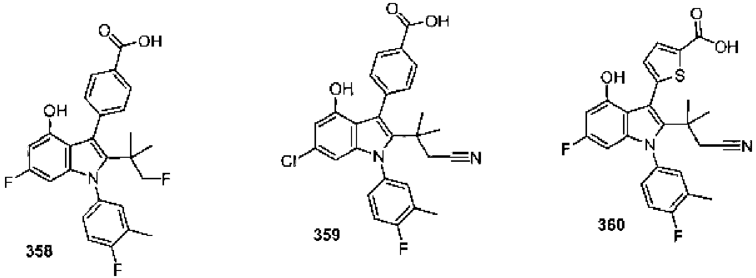 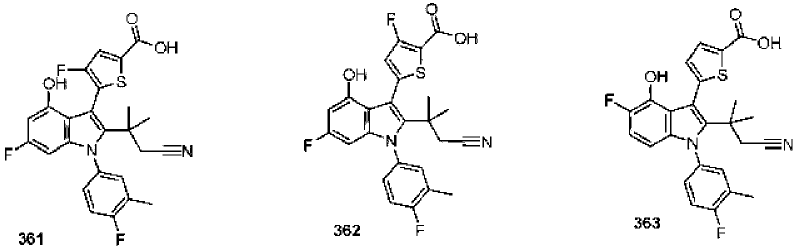 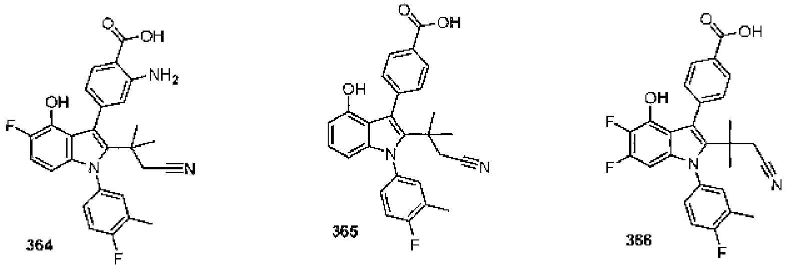 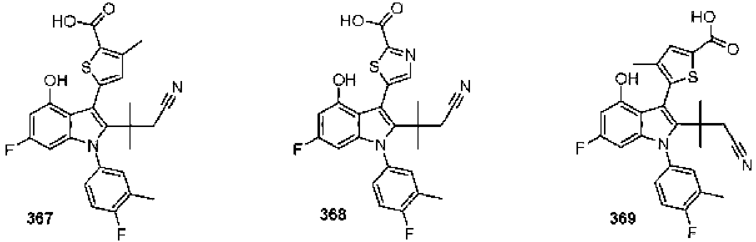 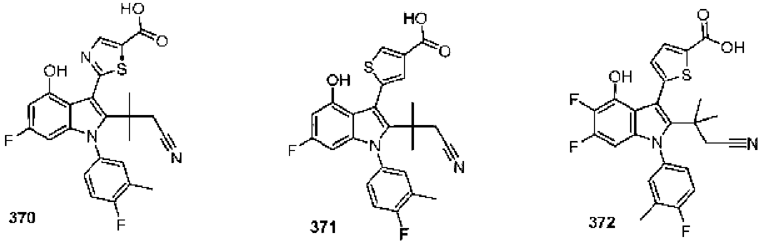 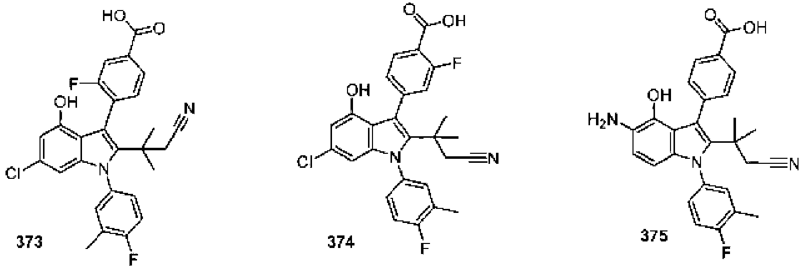 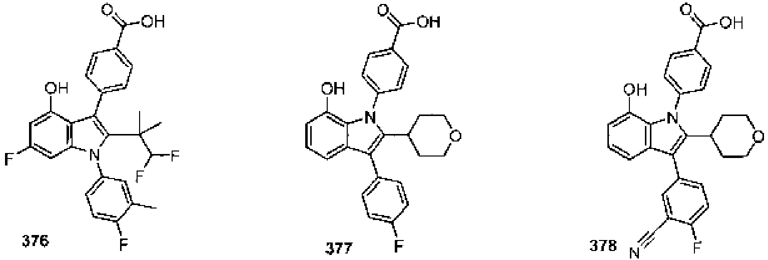 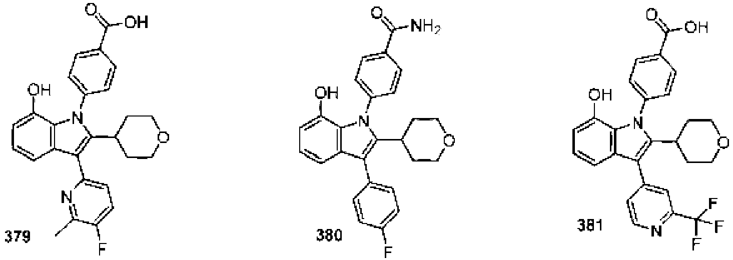 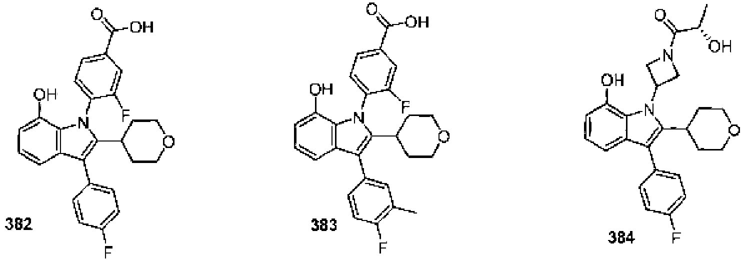 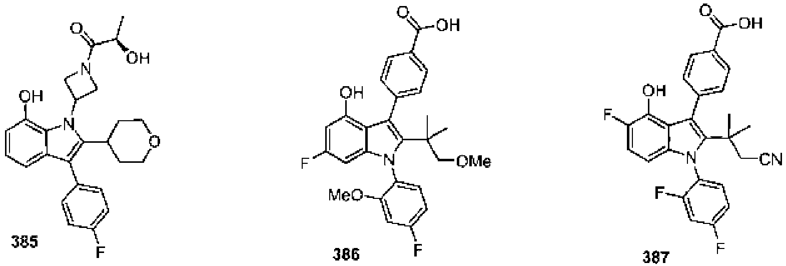 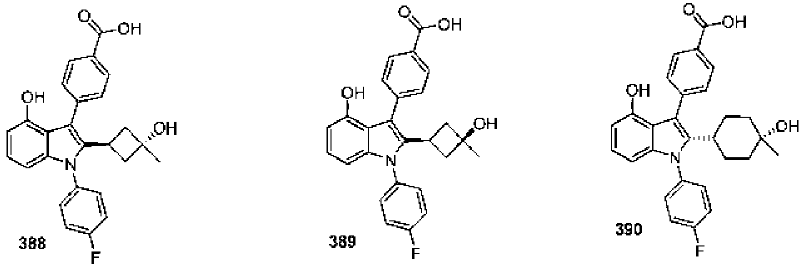 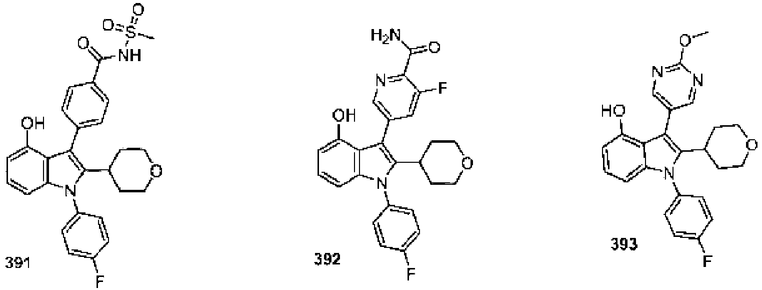 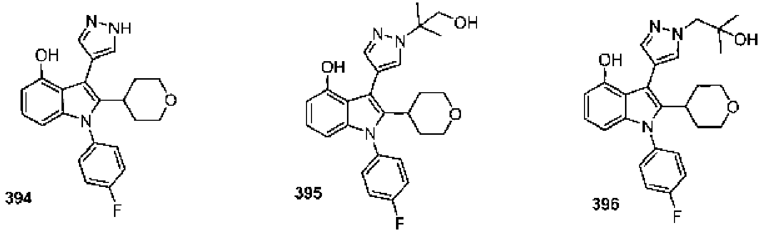 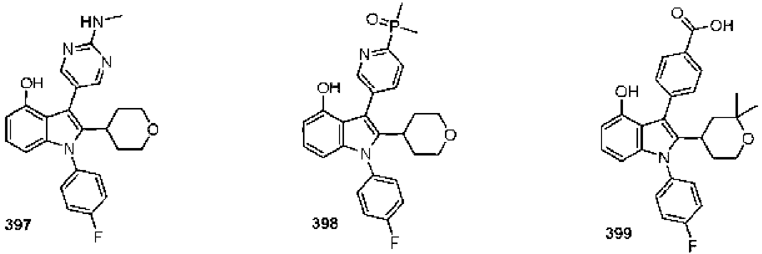 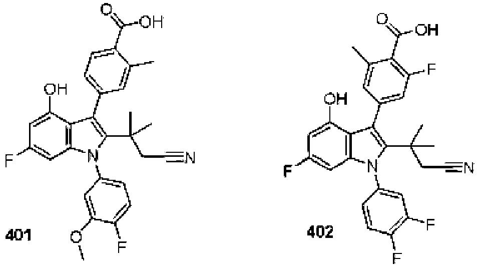 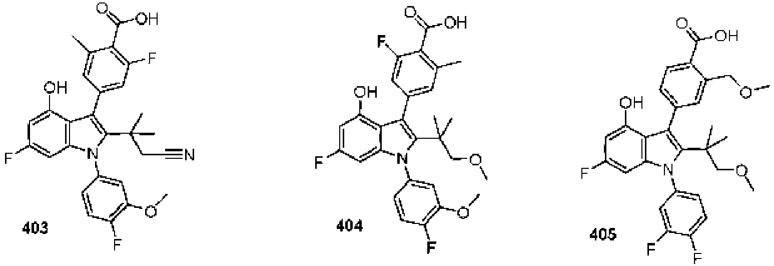 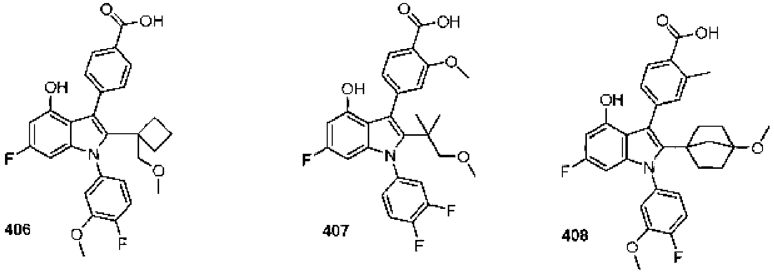 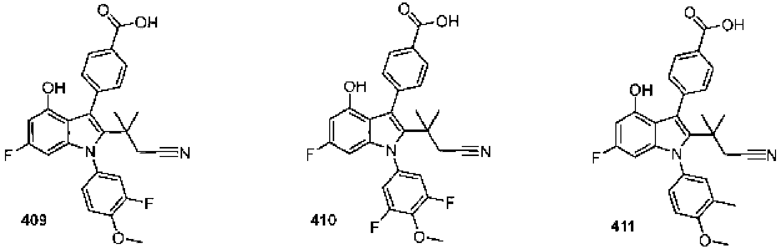 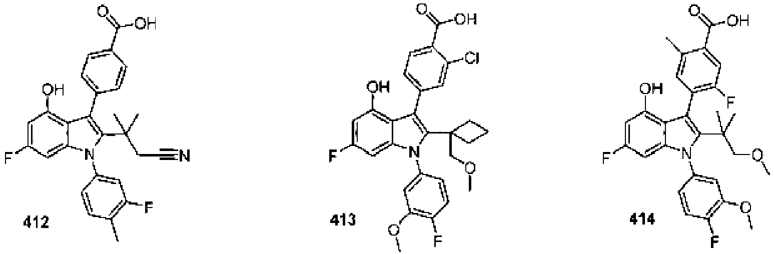 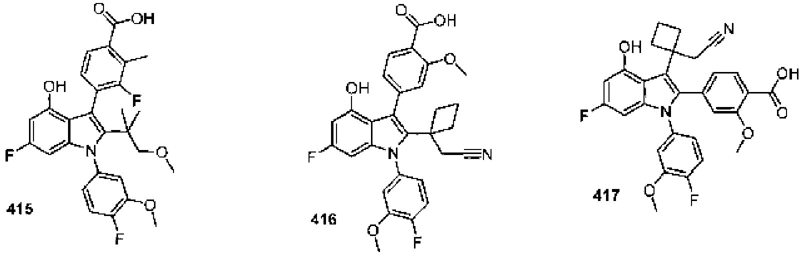 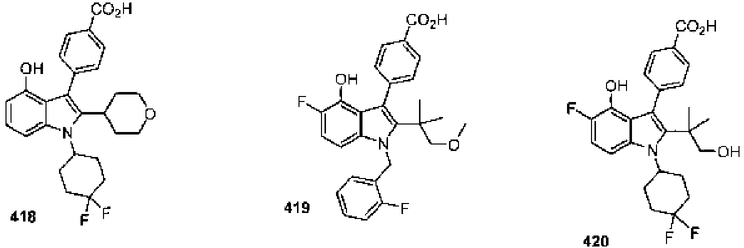 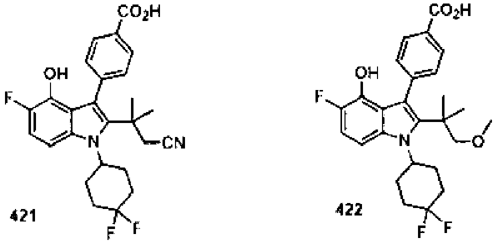 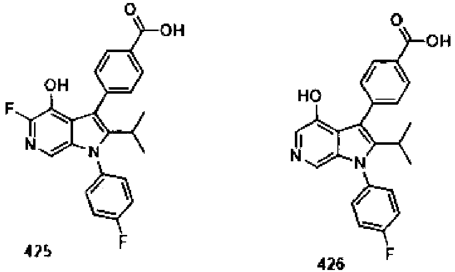 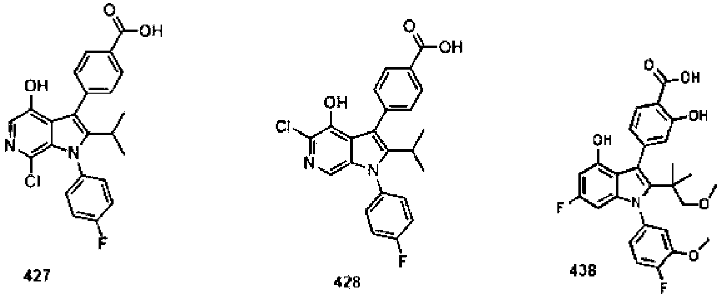 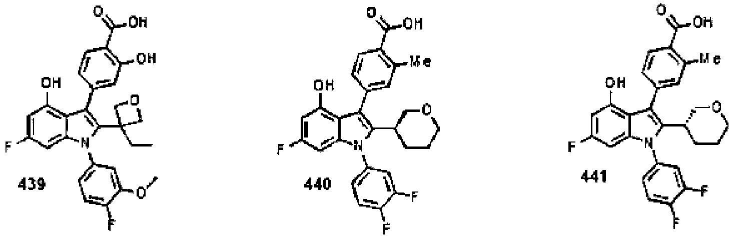 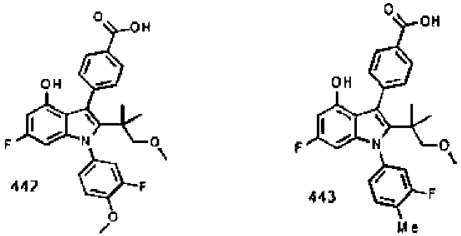 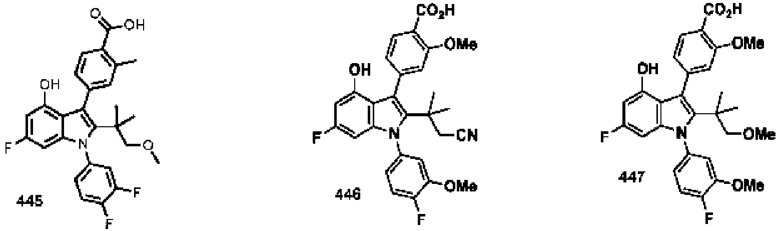 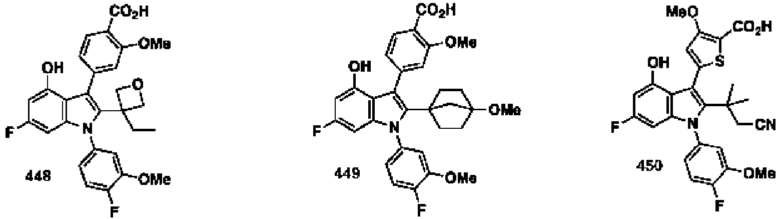 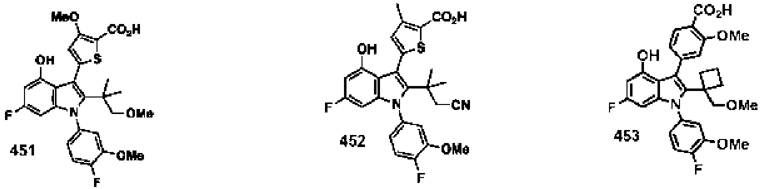 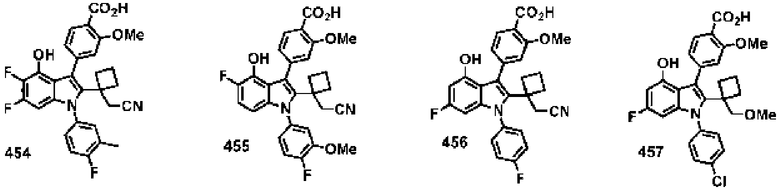 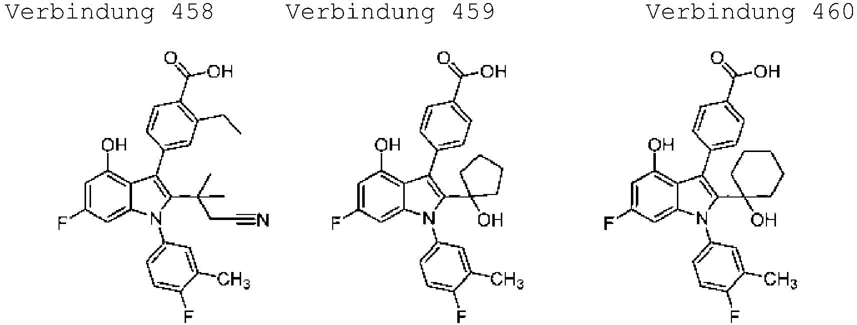 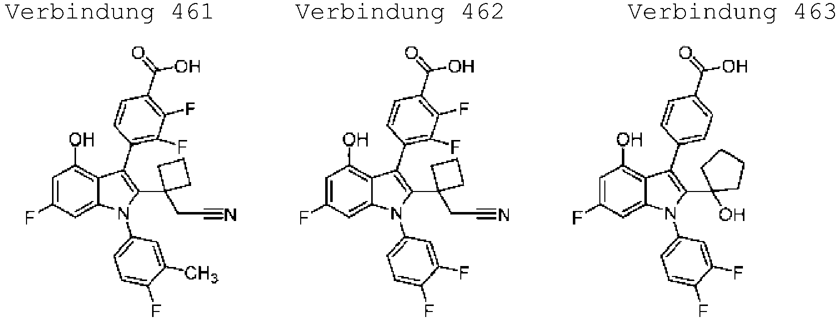 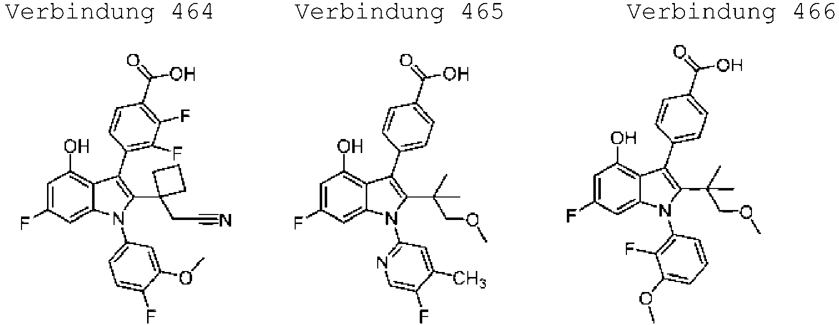 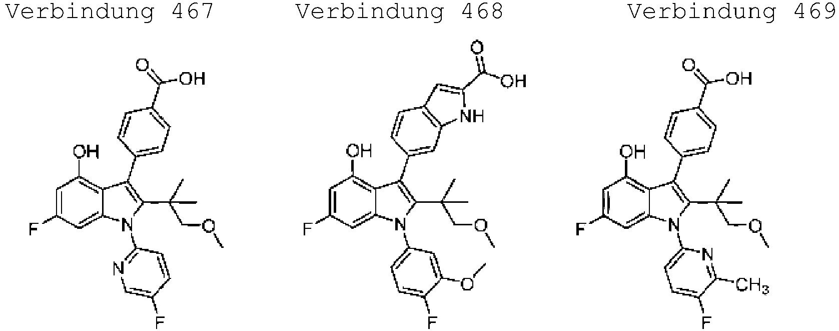 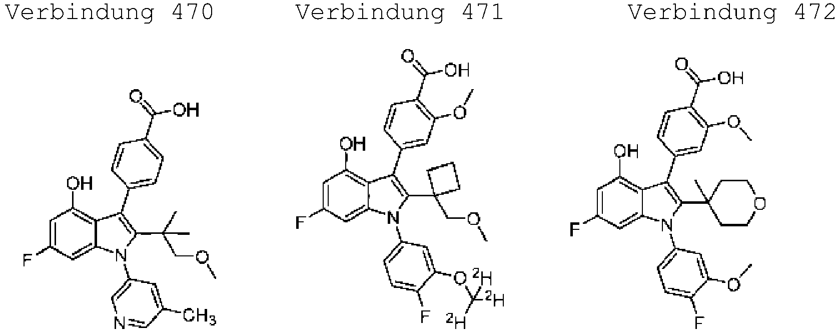 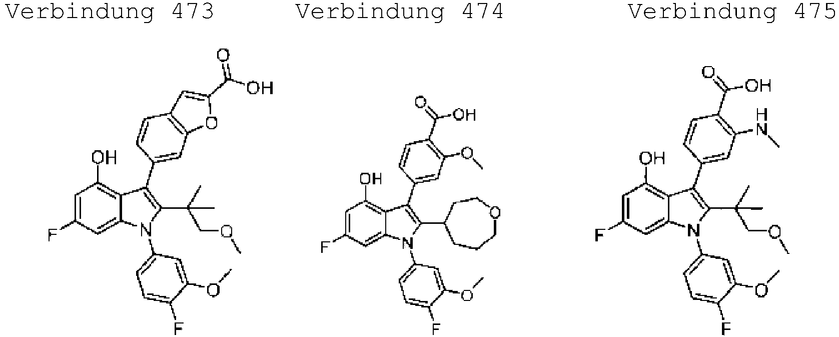 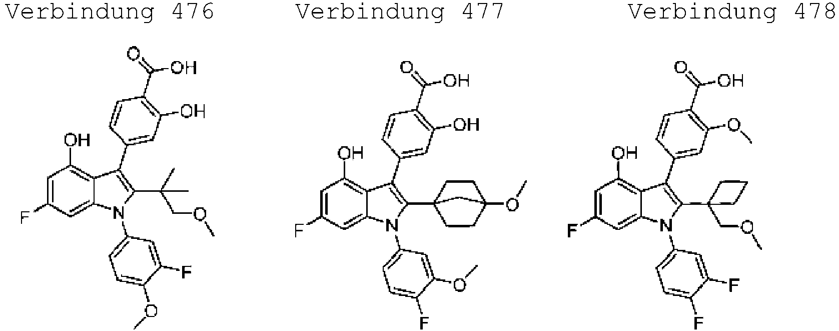 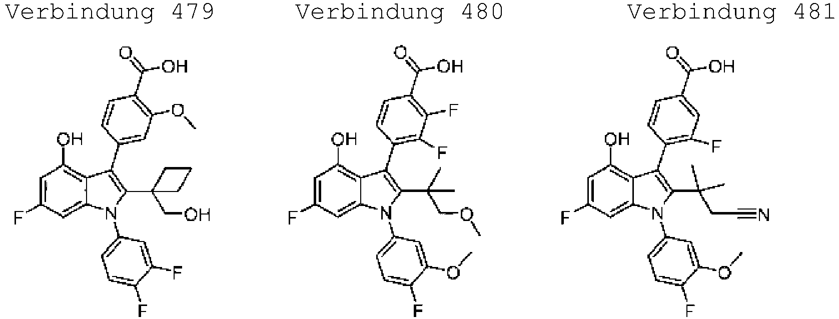 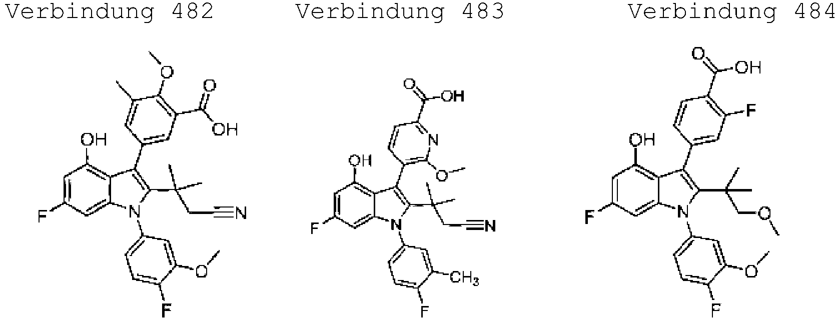 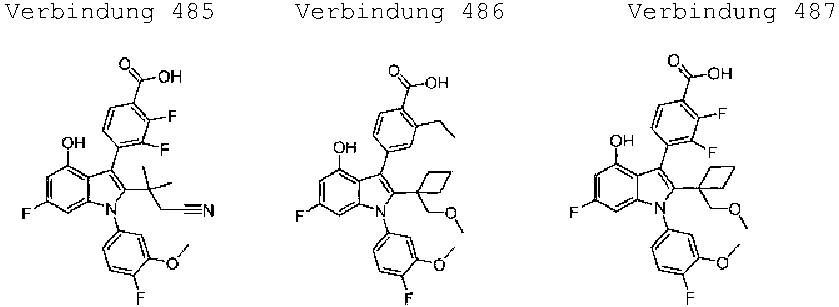 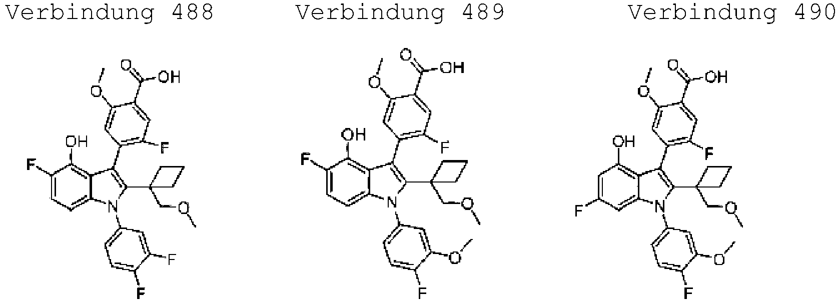 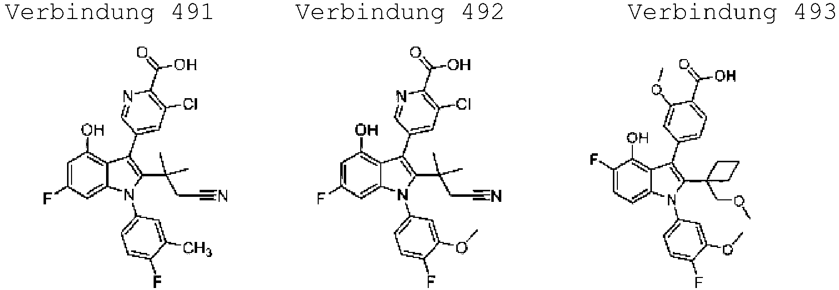 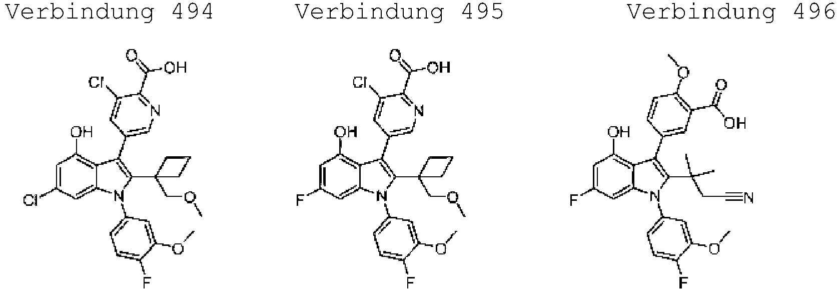 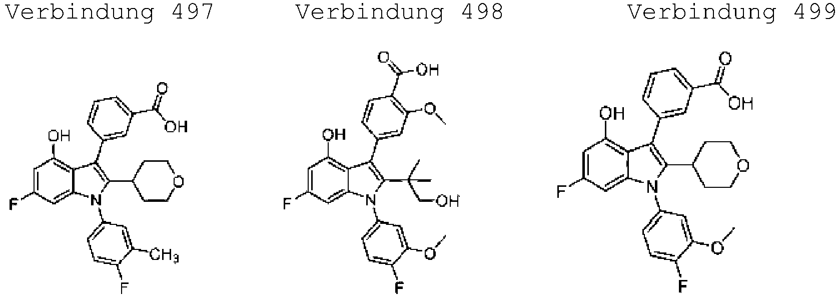 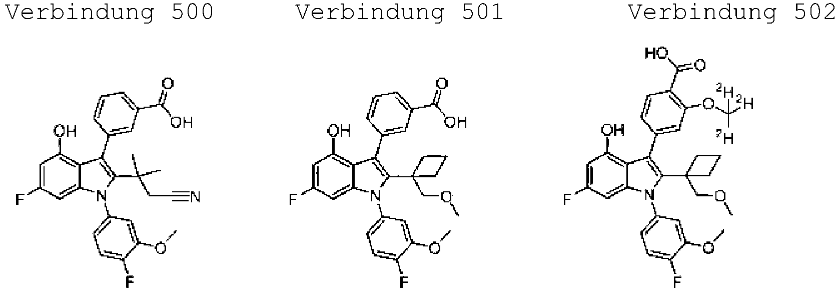 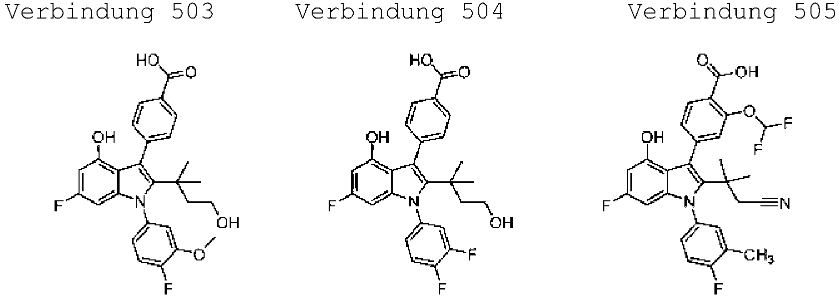 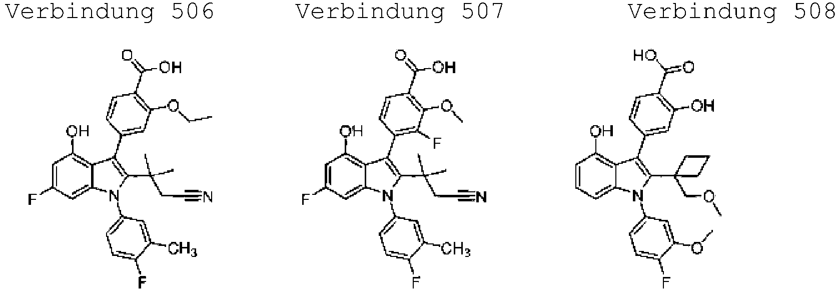 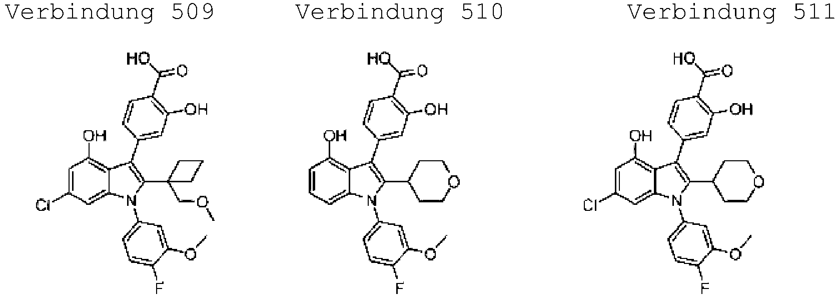 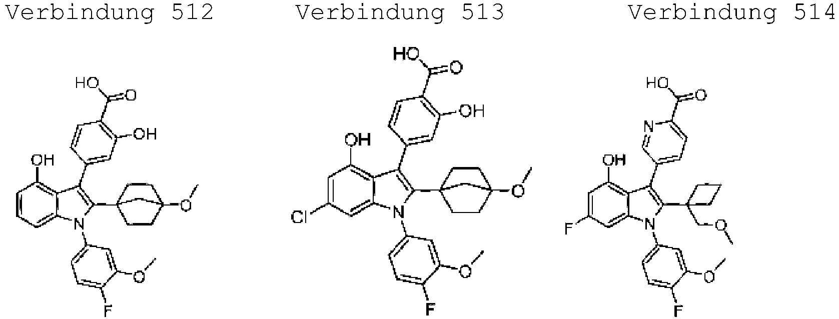 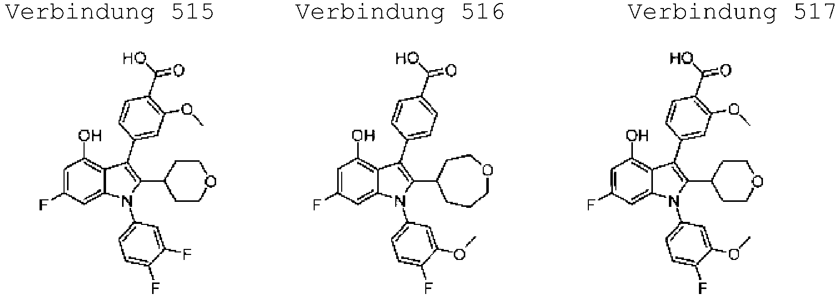 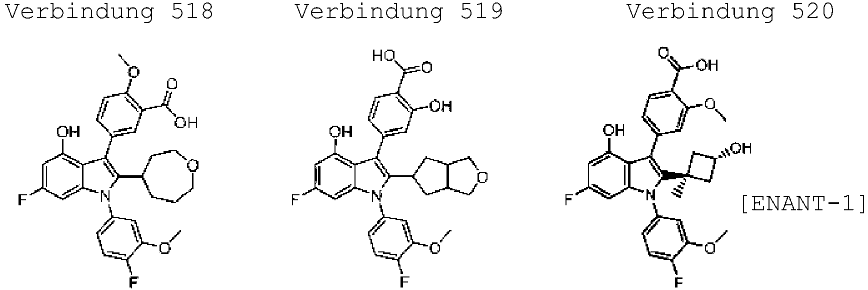 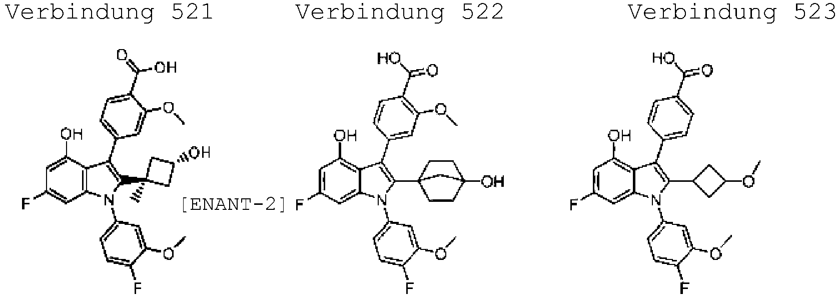 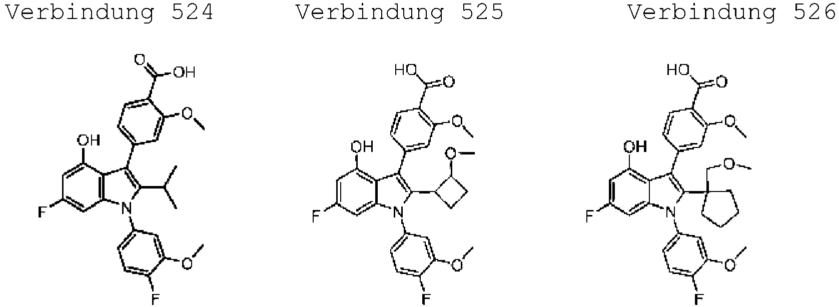 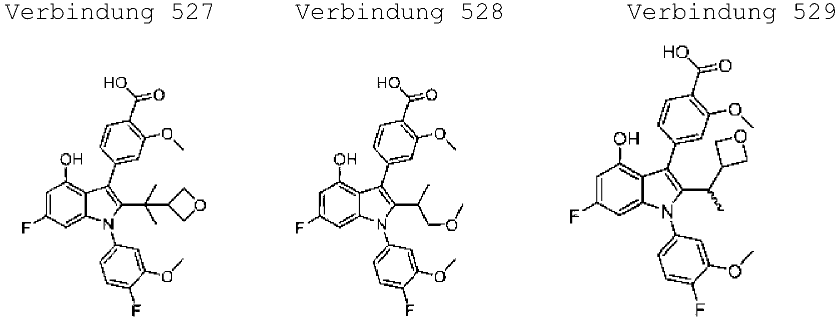 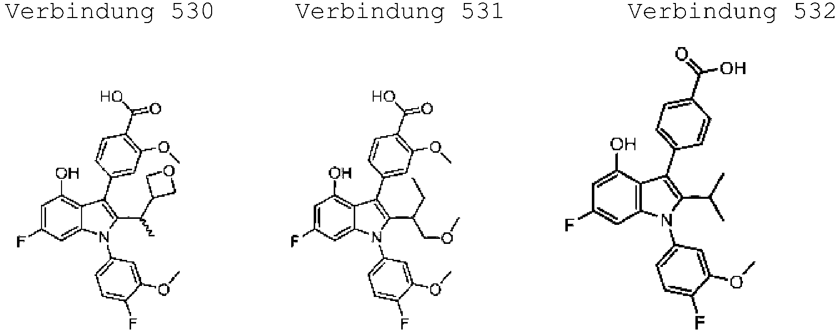 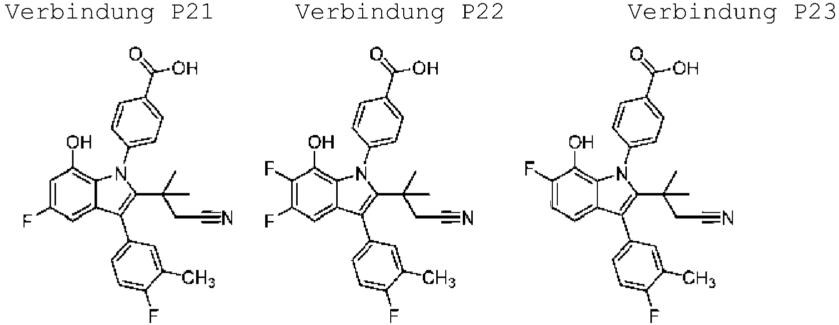 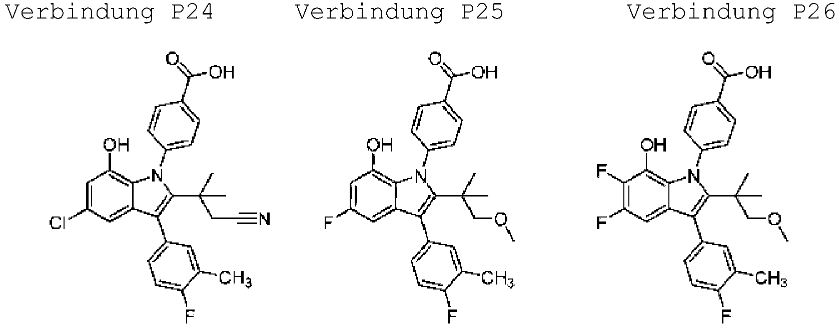 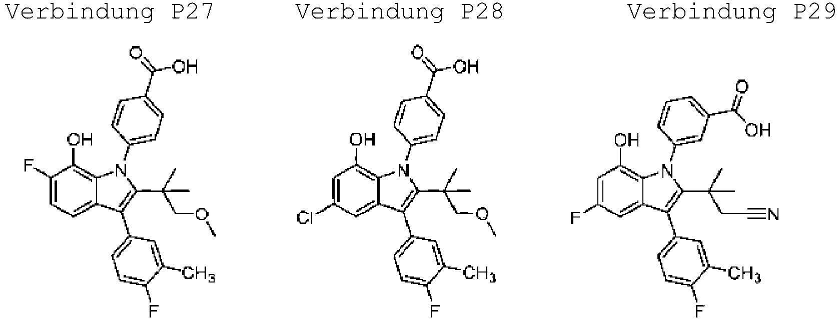 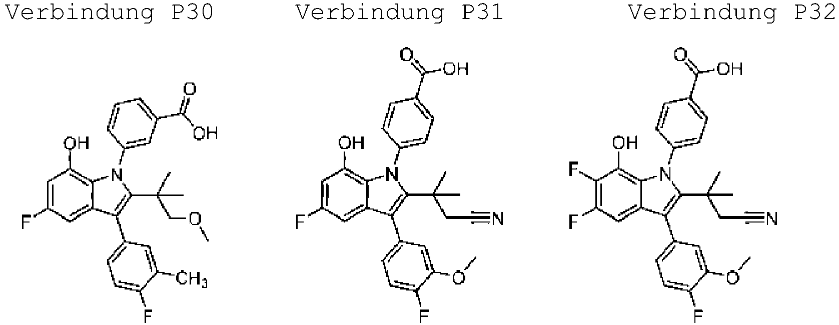 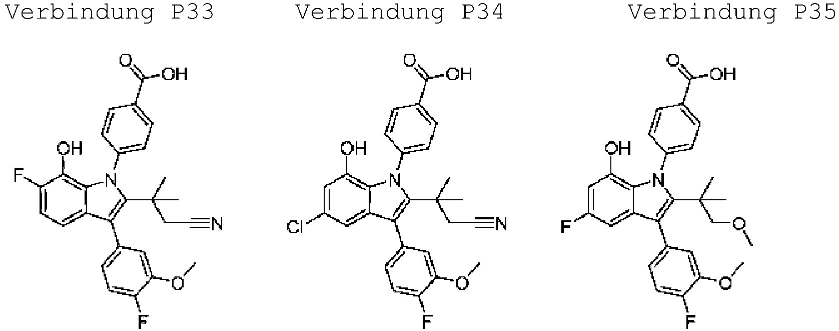 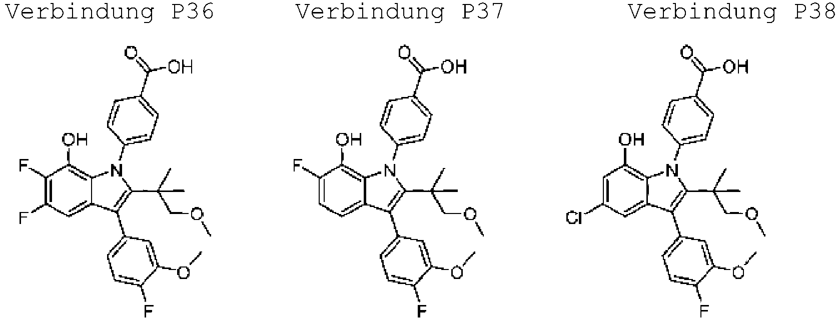 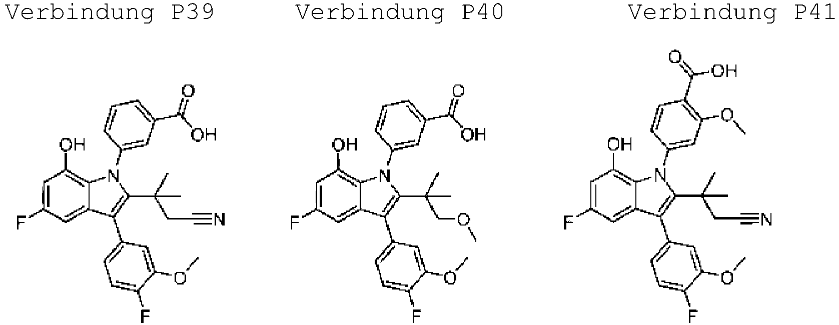 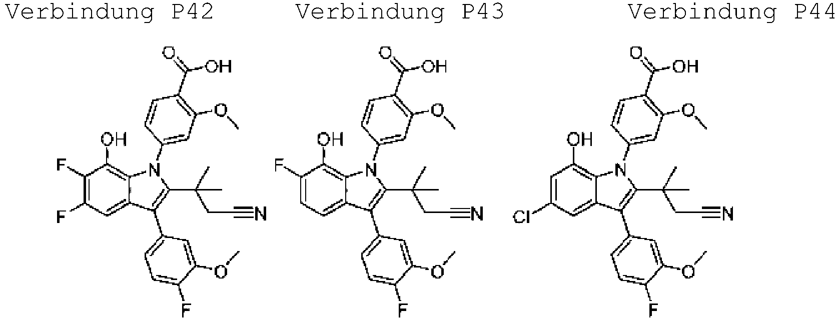 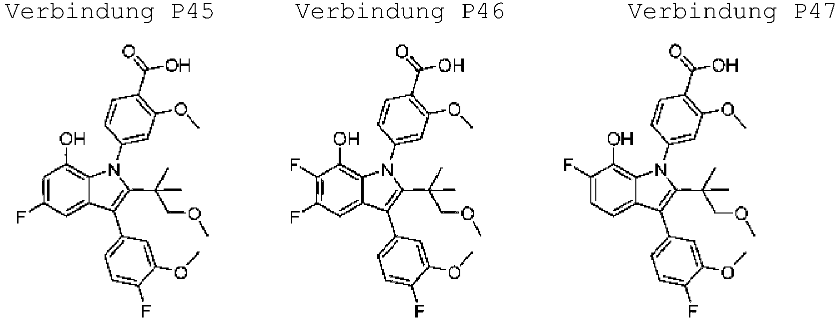 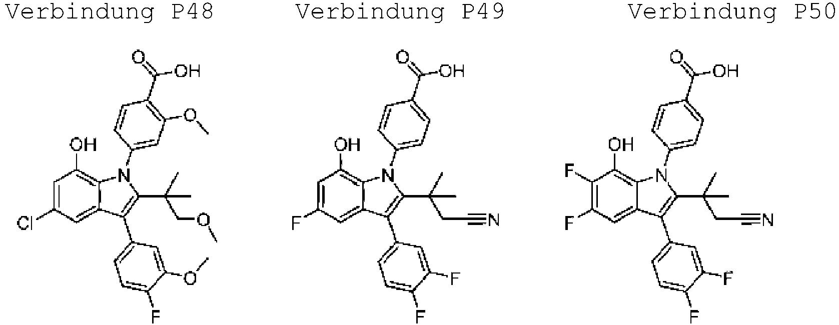 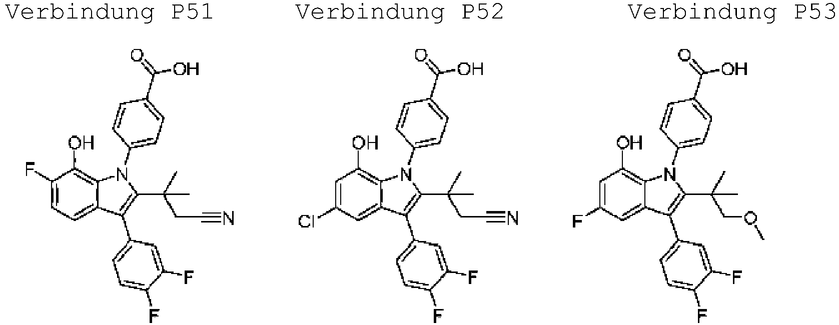 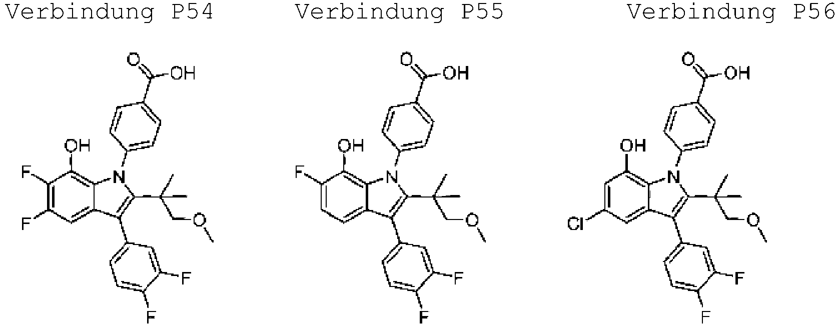 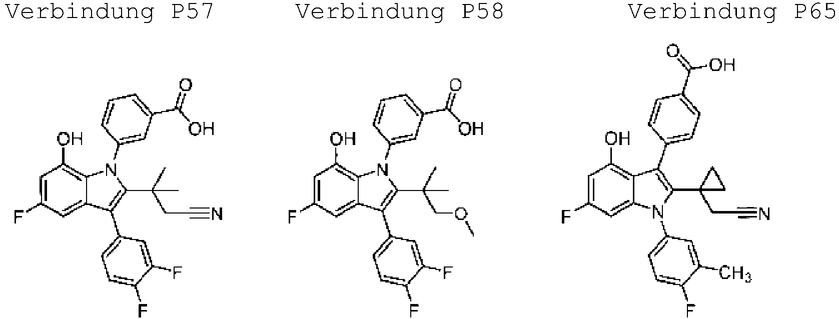 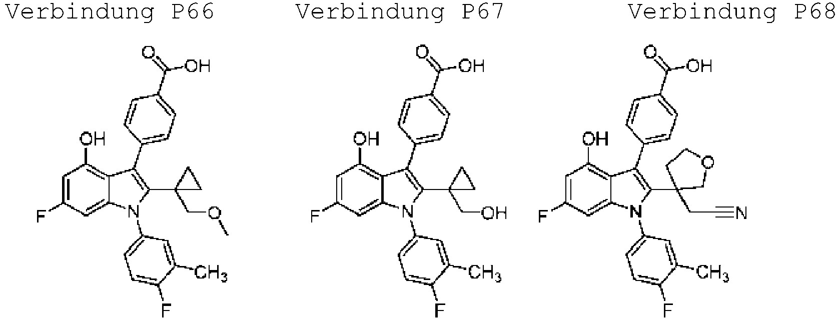 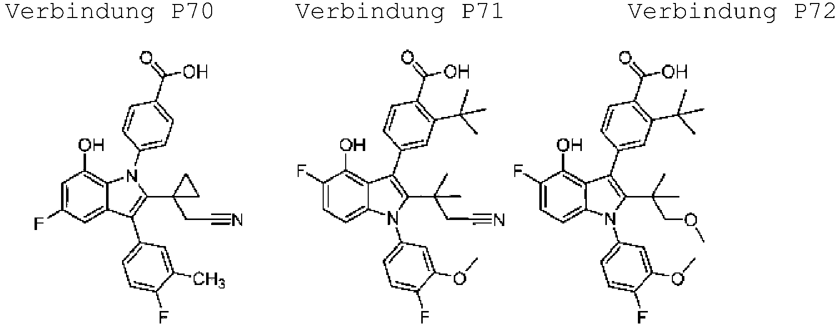 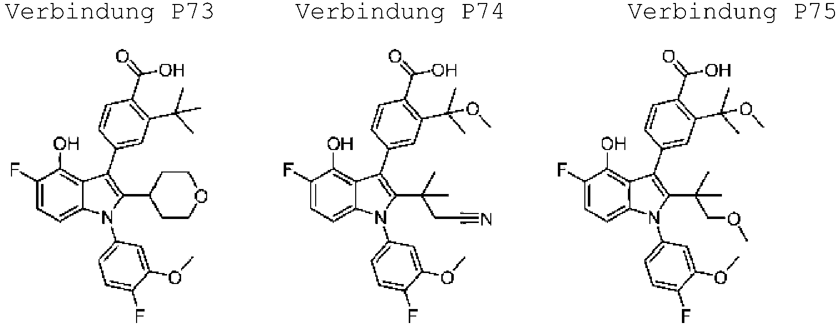 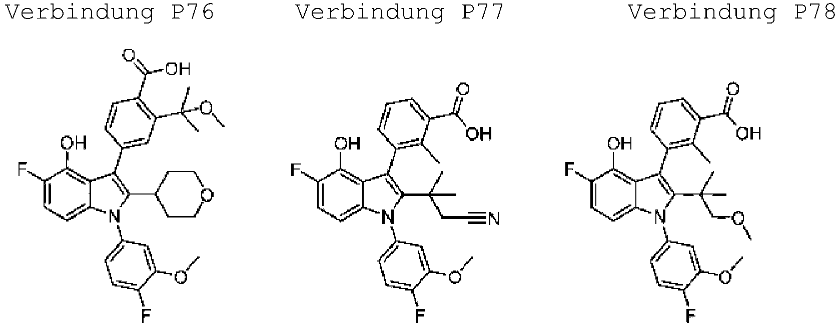 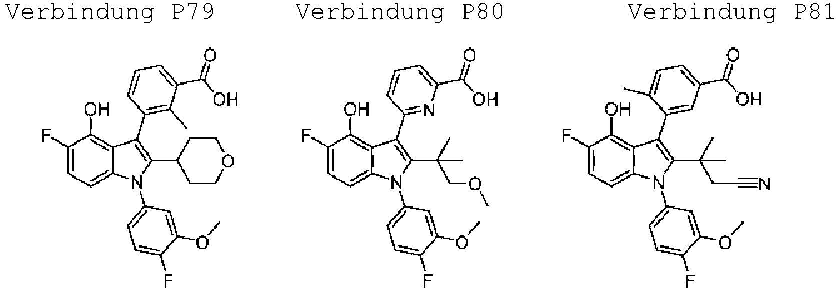 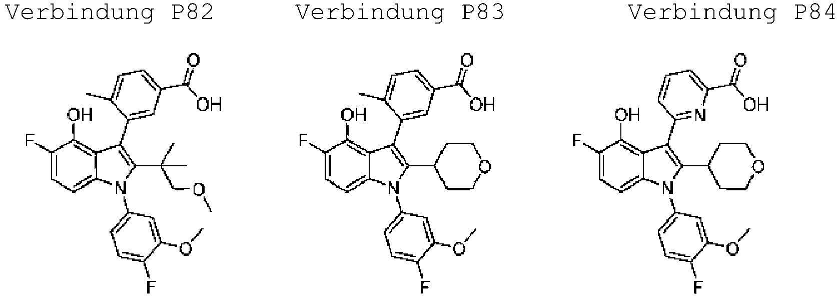 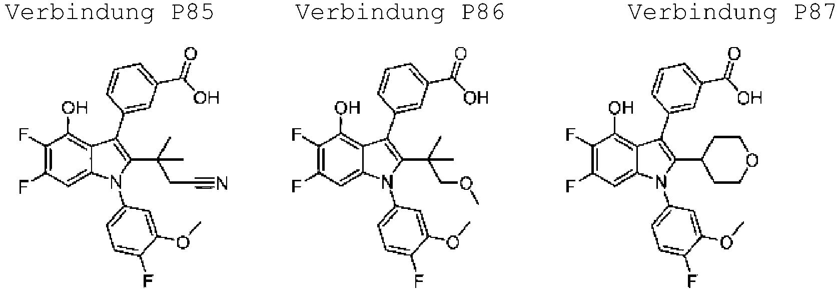 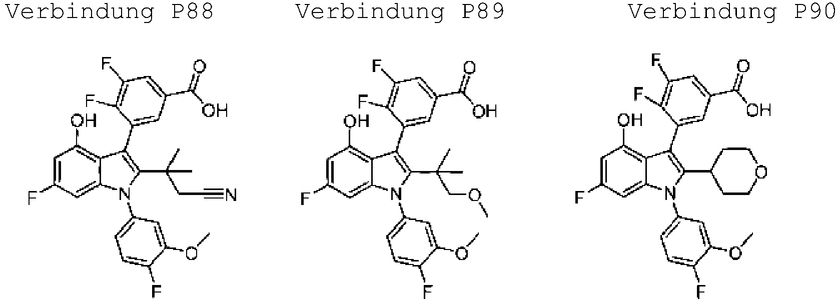 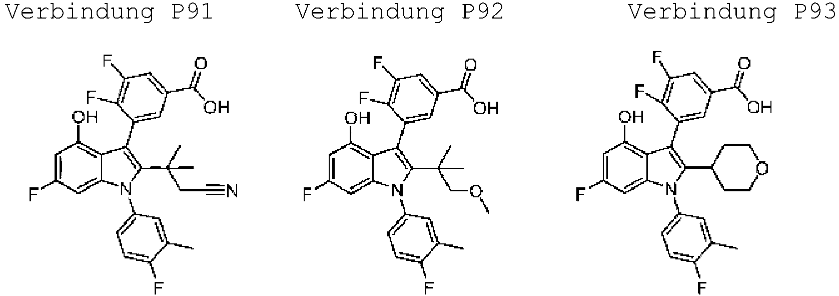 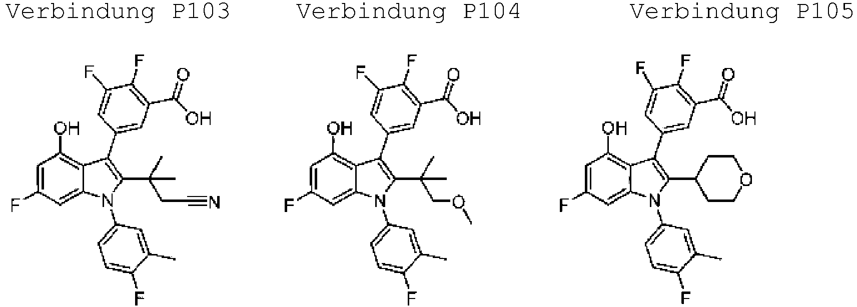 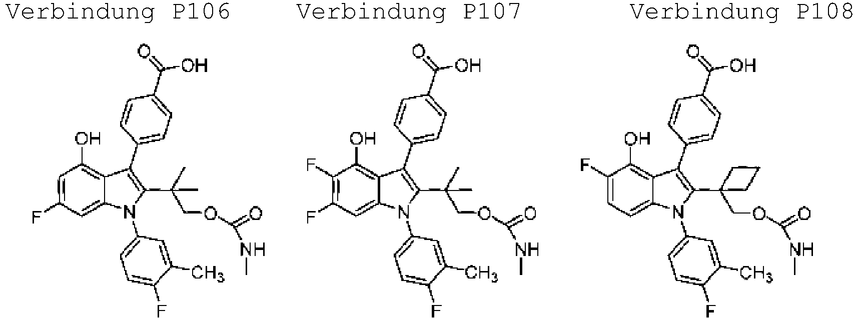 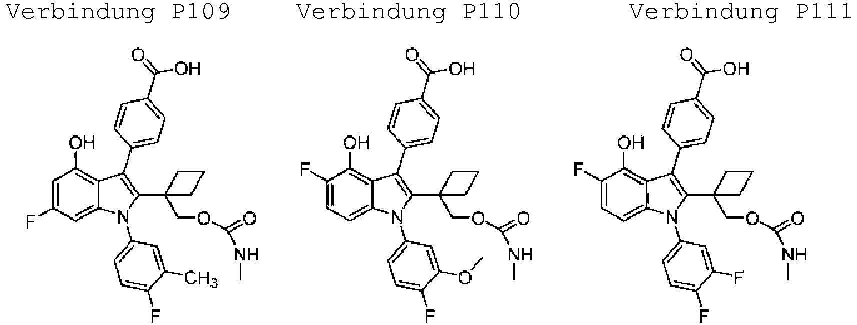 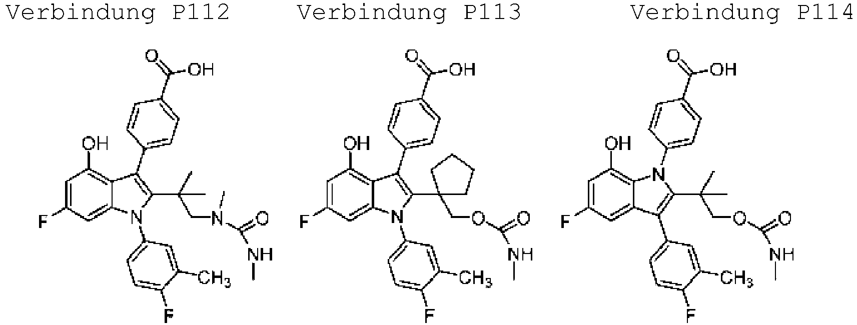 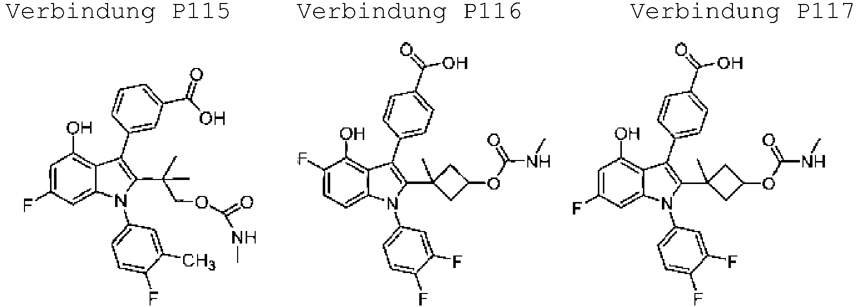 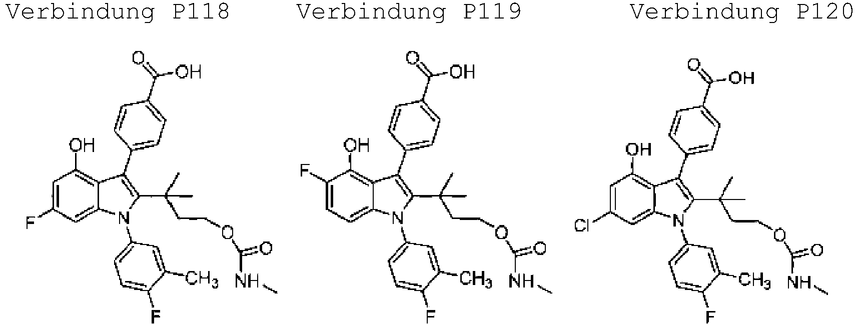 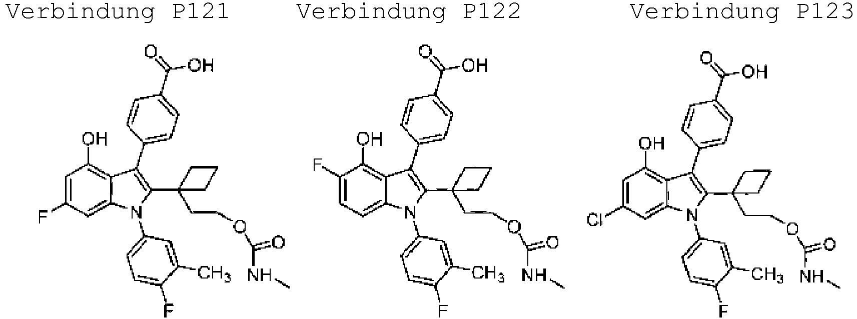 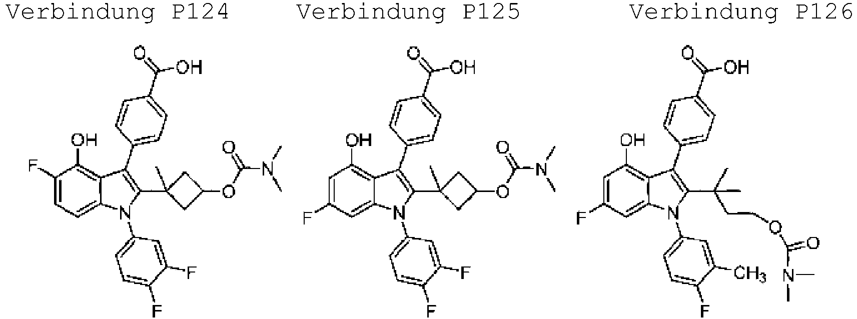 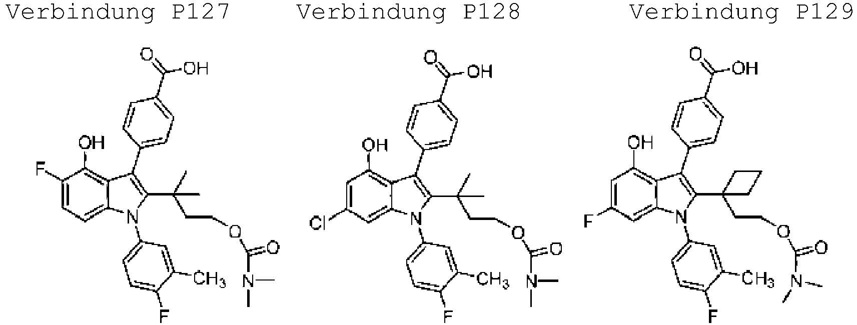 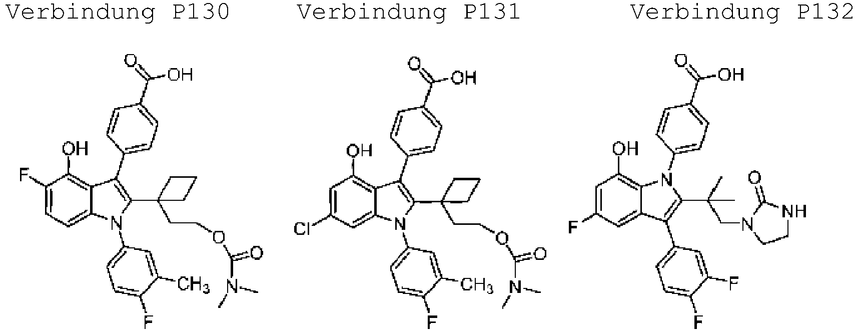 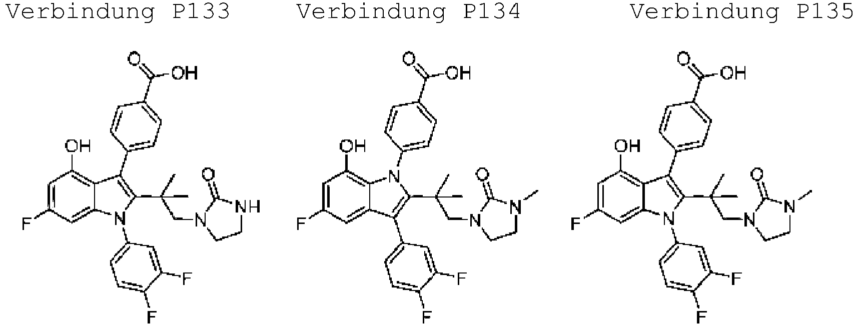 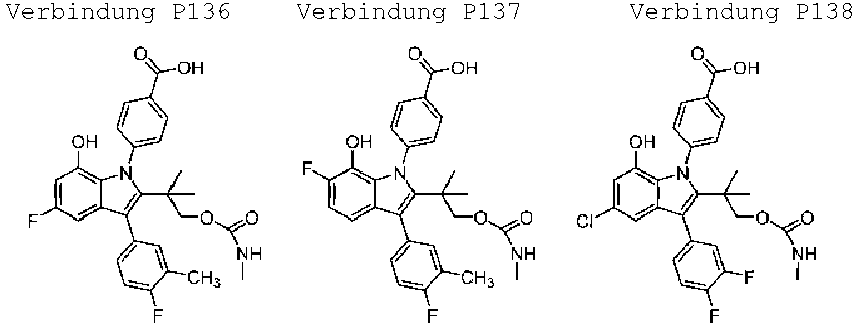 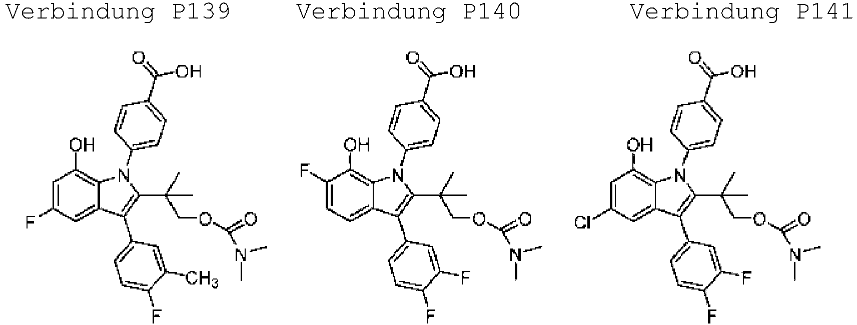 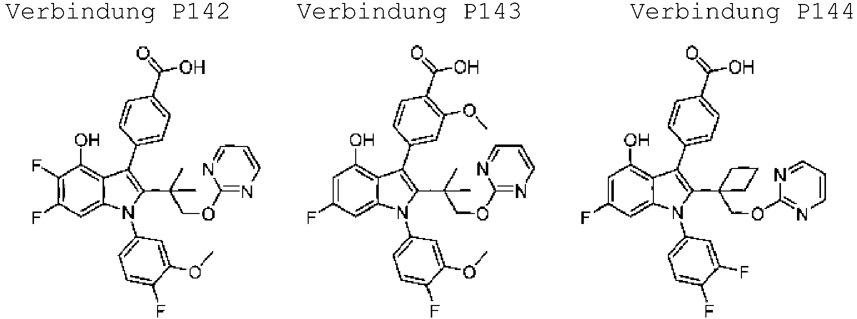 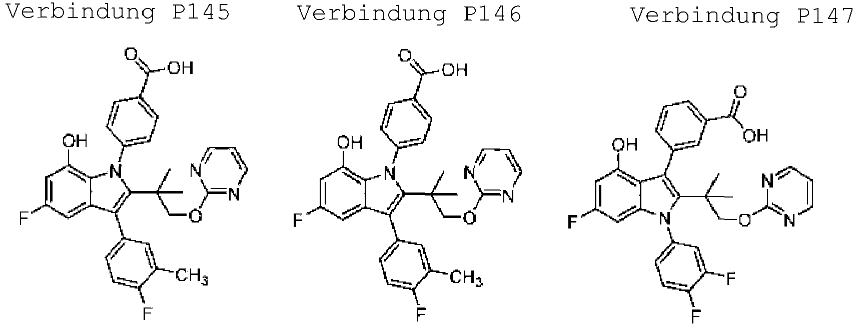 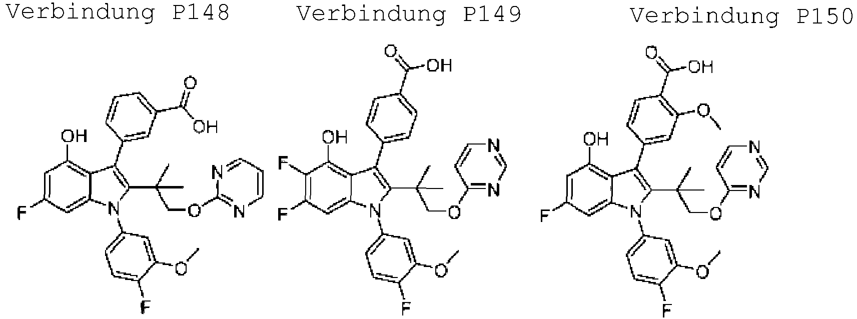 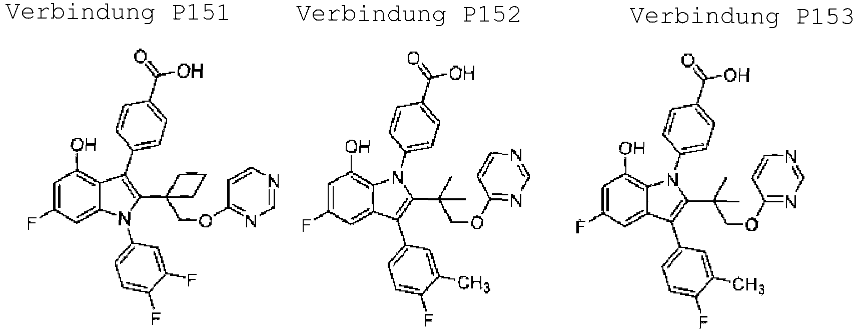 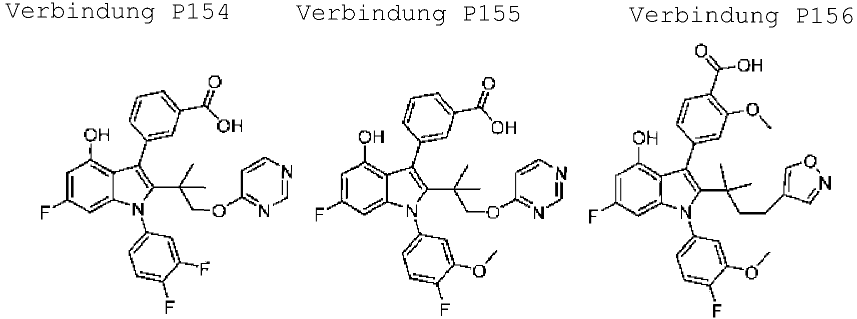 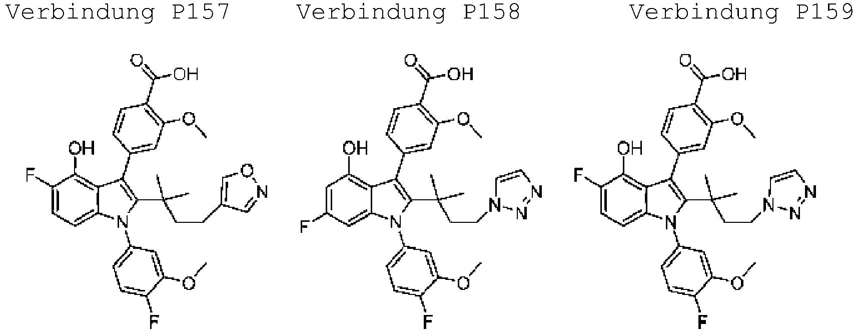 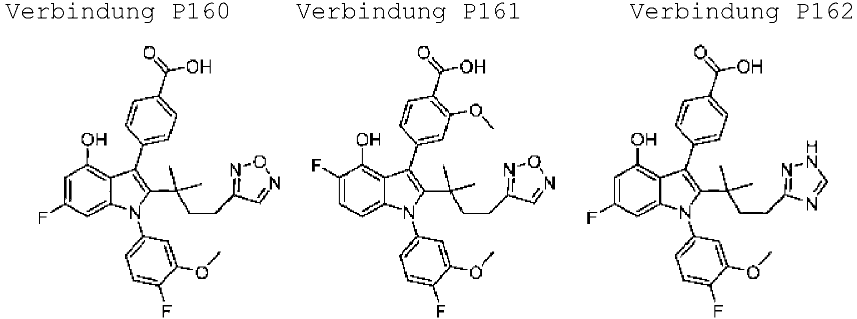 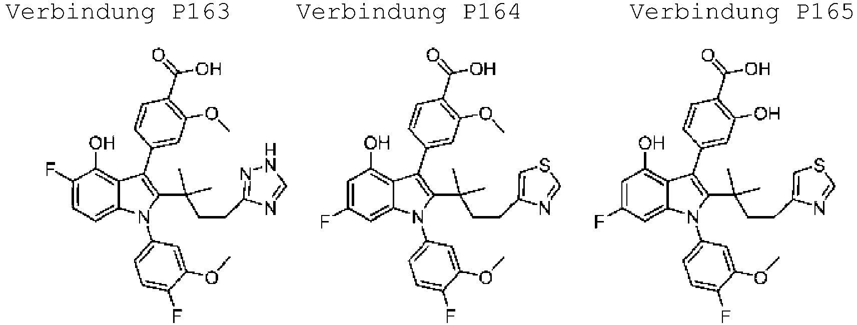 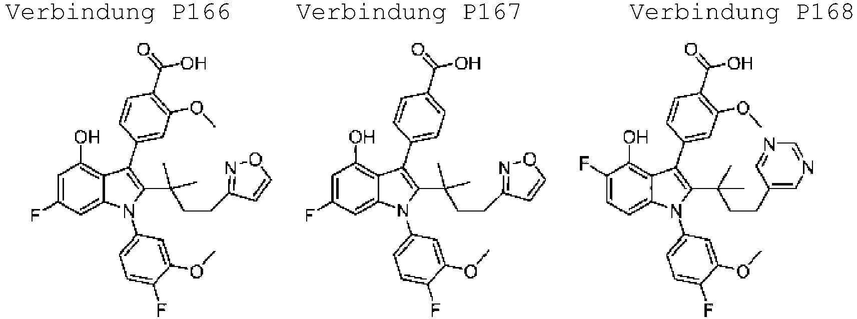 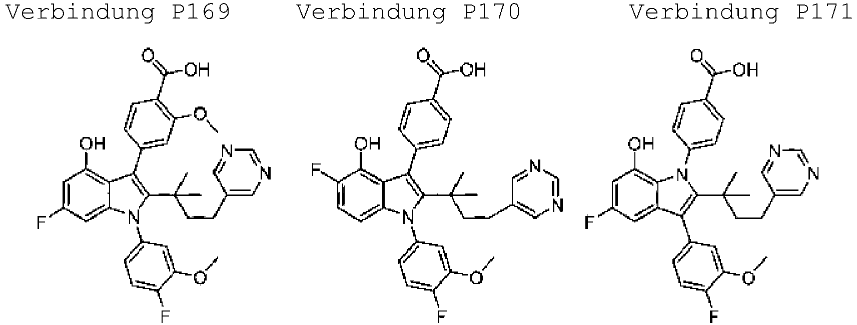 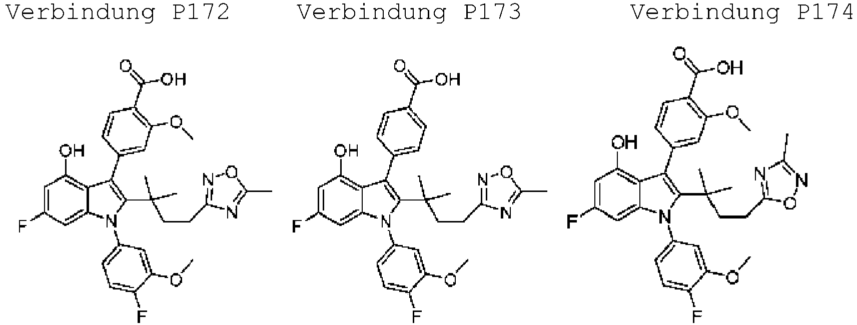 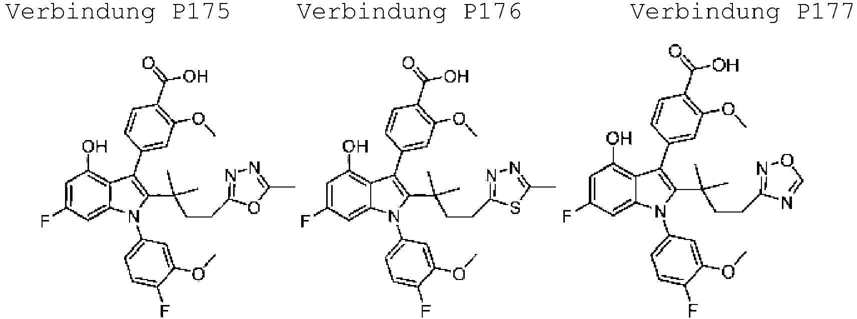 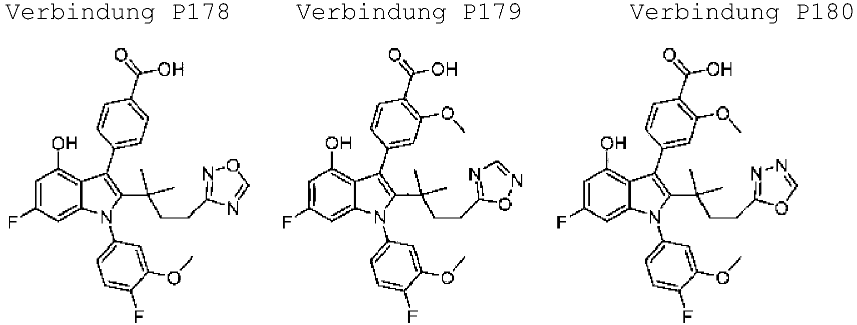 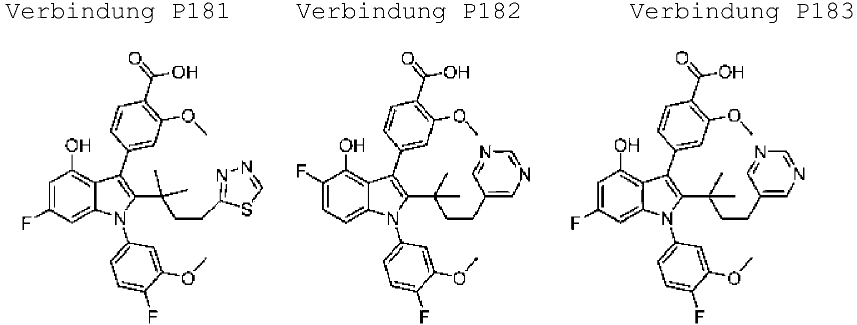 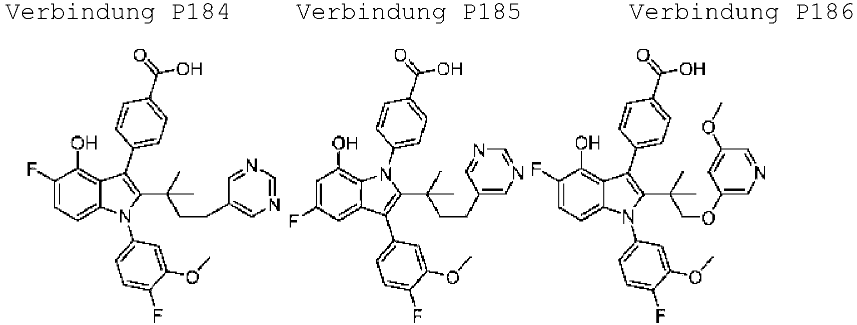 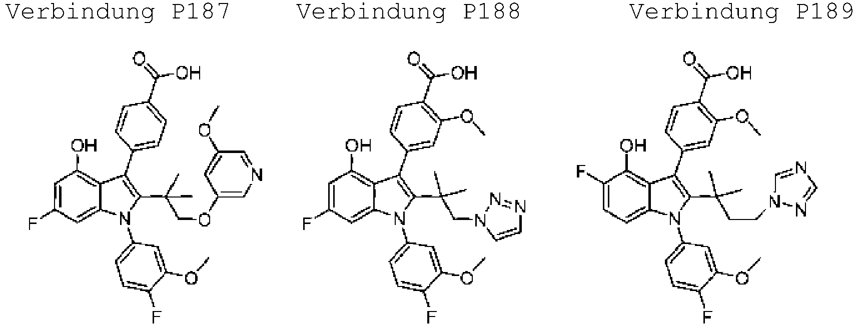 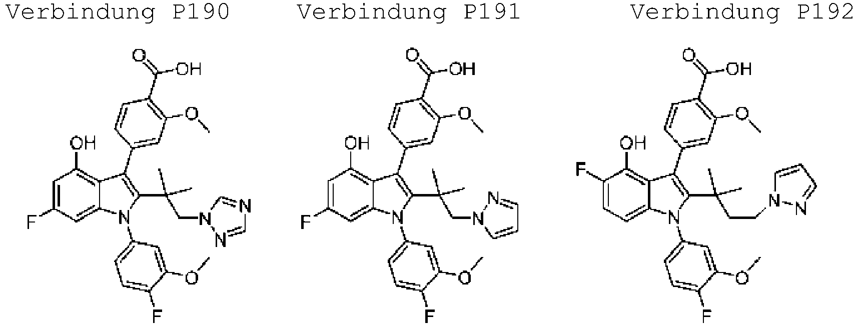 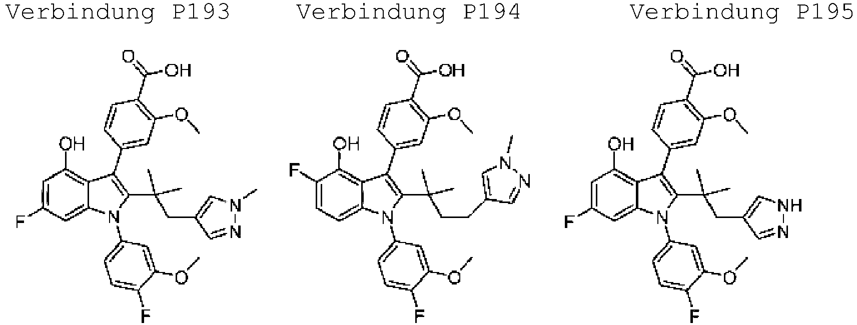 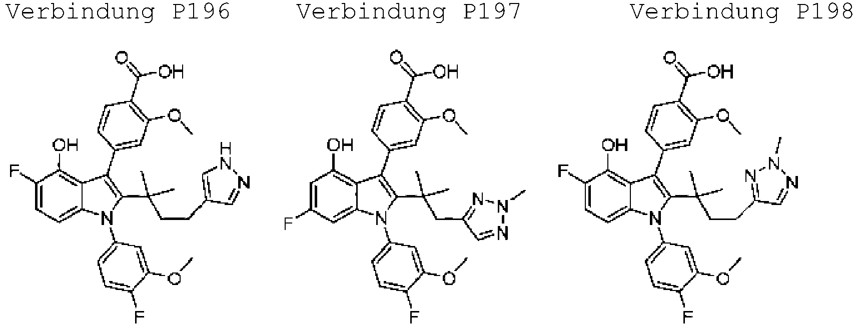 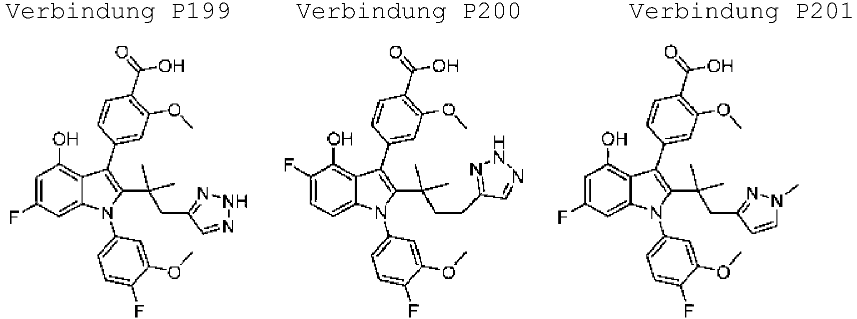 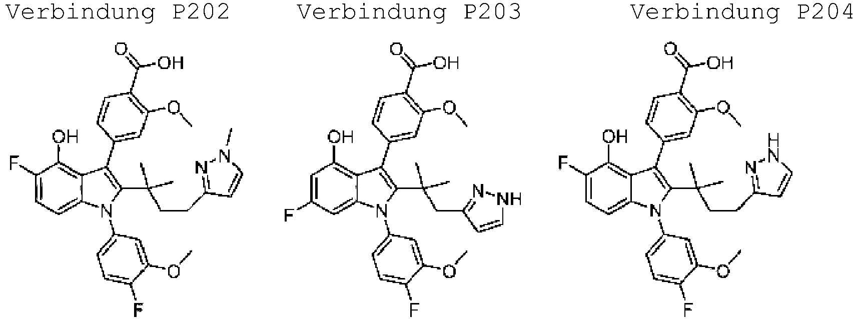 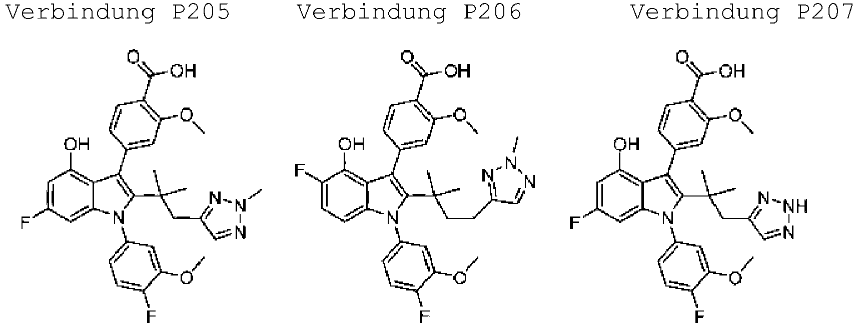 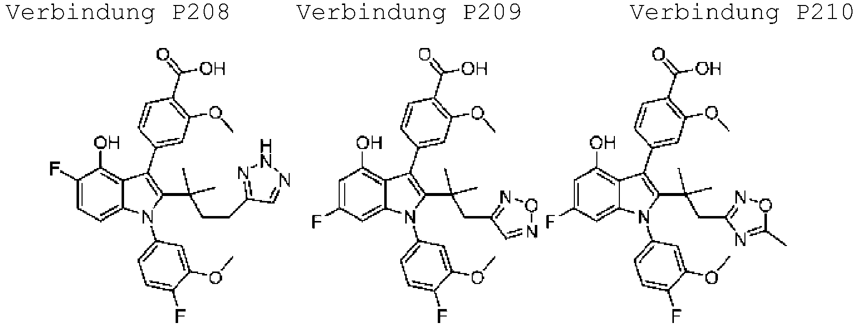 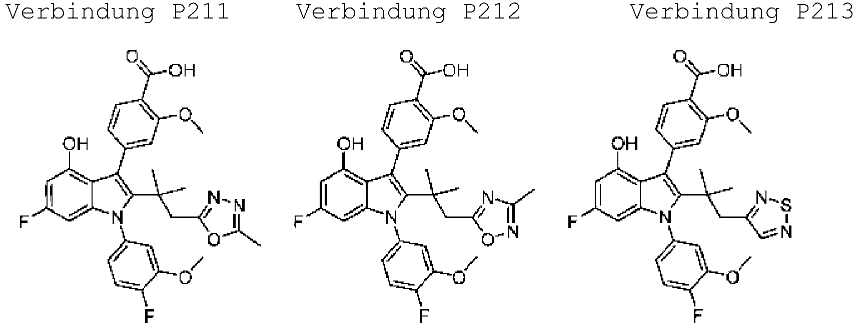 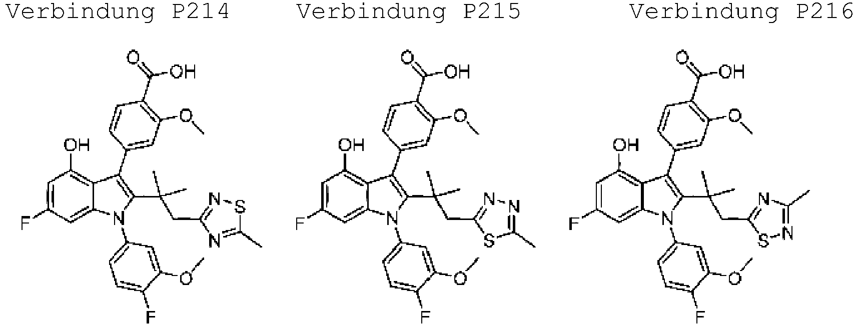 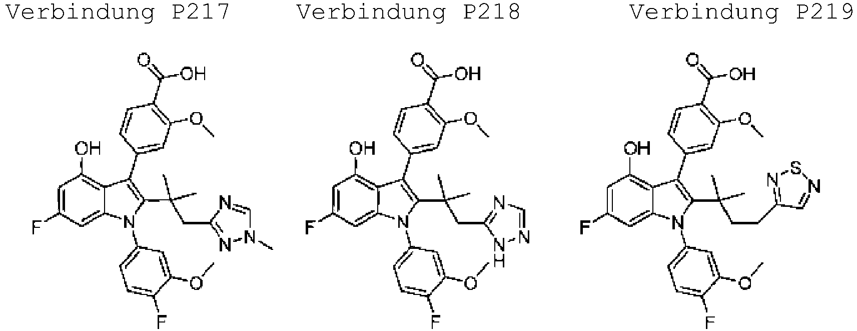 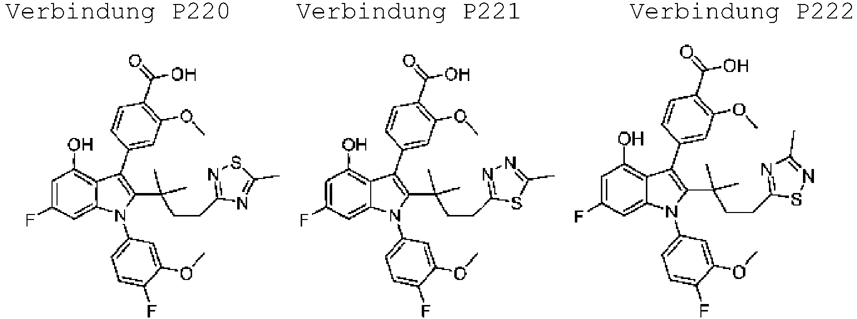 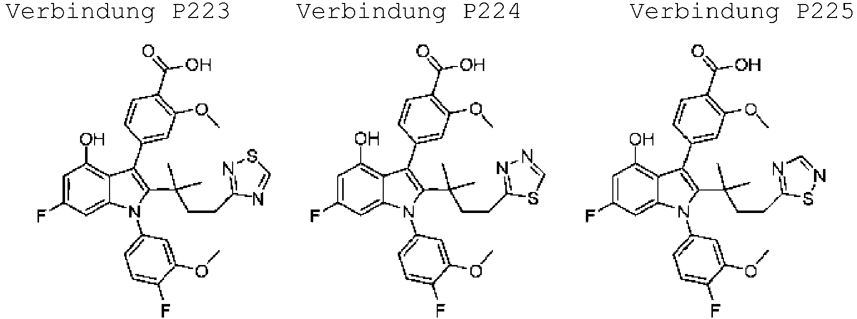 Tautomere davon, deuterierte Derivate dieser Verbindungen und Tautomere und pharmazeutisch verträgliche Salze der Verbindungen, Tautomere und deuterierten Derivate.

13. Verbindung, ausgewählt aus: 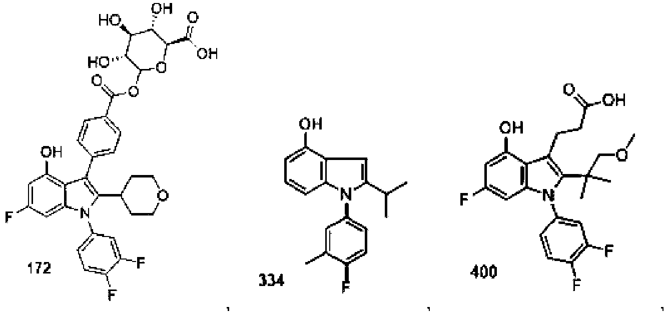 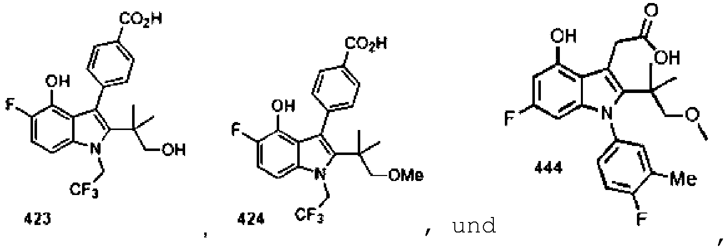 einem Tautomer davon, einem deuterierten Derivat der Verbindung oder des Tautomers, oder einem pharmazeutisch verträglichen Salz der Verbindung, des Tautomers oder des deuterierten Derivats.

14. Pharmazeutische Zusammensetzung, umfassend mindestens eine Verbindung nach einem der Ansprüche 1 bis 13, ein Tautomer davon, ein deuteriertes Derivat dieser Verbindung oder dieses Tautomers, oder ein pharmazeutisch verträgliches Salz des Vorstehenden.

15. Verbindung, Tautomer, deuteriertes Derivat oder pharmazeutisch verträgliches Salz nach einem der Ansprüche 1 bis 13 oder pharmazeutische Zusammensetzung nach Anspruch 14 zur Verwendung in einem Verfahren zum Behandeln von Alpha-1-Antitrypsin(AAT)-Mangel, umfassend Verabreichen einer therapeutisch wirksamen Menge mindestens einer Verbindung, eines Tautomers, eines deuterierten Derivats oder eines pharmazeutisch verträglichen Salzes nach einem der Ansprüche 1 bis 13 oder einer therapeutisch wirksamen Menge einer pharmazeutischen Zusammensetzung nach Anspruch 14 an einen behandlungsbedürftigen Patienten, wobei die therapeutisch wirksame Menge der mindestens einen Verbindung, des Tautomers, des deuterierten Derivats oder des pharmazeutisch verträglichen Salzes optional in Kombination mit AAT-Augmentationstherapie und/oder AAT-Ersatztherapie verabreicht wird.

## Revendications

1. Composé représenté par la formule structurale suivante : 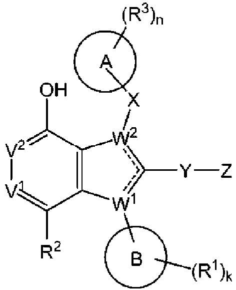 un tautomère de celui-ci, un dérivé deutéré de ce composé ou tautomère, ou un sel pharmaceutiquement acceptable des éléments précédents, dans lequel :
-̅ -̅ -̅ -̅ -̅ -̅, pour chacune des deux occurrences, est une liaison simple ou une liaison double, à condition que l'une soit une liaison simple et l'autre une liaison double ;
**V¹** et **V²** sont chacun indépendamment N ou -C**R²** ;
**W¹** et **W²** sont chacun indépendamment N ou C, à condition que l'un de W¹ et W² soit N et l'autre C ;
**X** est absent ou une liaison, -**(**C**R^{a}R^{b})ₚ-** ou -SO₂- ;
**Y** est absent ou une liaison, -**(**C**R^{c}R^{d})_{q}**-, -C(=O)- ou -SO₂- ;
**R^{a}** et **R^{b}**, pour chaque occurrence, sont chacun indépendamment hydrogène, halogène, -OH, alkyle en C₁-C₆, alcényle en C₂-C₆, haloalkyle en C₁-C₆, alcoxy en C₁-C₆ ou haloalcoxy en C₁-C₆ ;
**R^{c}** et **R^{d}**, pour chaque occurrence, sont chacun indépendamment hydrogène, halogène, -OH, alkyle en C₁-C₆, alcényle en C₂-C₆, haloalkyle en C₁-C₆, alcoxy en C₁-C₆ ou haloalcoxy en C₁-C₆ ;
le **cycle A** est carbocyclyle en C₃-C₁₂, hétérocyclyle à 3 à 12 chaînons, un aryle en C₆ ou C₁₀, ou hétéroaryle à 5 à 10 chaînons ; à condition que lorsque **W¹** est N et **W²** est C, le **cycle A** ne soit pas le 1,5,6,7-tétrahydro-4*H*-indol-4-onyle ou un tautomère de celui-ci ;
le **cycle B** est cycloalkyle en C₄-C₁₂, aryle en C₆ ou C₁₀, hétéroaryle à 5 à 10 chaînons, ou benzyle ;
**Z** est 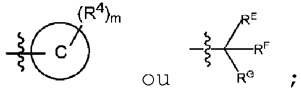 dans lequel :
le **cycle C** est cycloalkyle en C₃-C₁₂, hétérocyclyle à 3 à 12 chaînons, aryle en C₆ ou C₁₀ ou hétéroaryle à 5 à 10 chaînons ; à condition que lorsque le **cycle C** est phényle, phényle soit substitué par **R⁴** ; à condition que, lorsque le **cycle C** est phényle, **Y** ne puisse pas être -SO₂- ; et
à condition que lorsque le **cycle B** est benzyle, le **cycle C** ne puisse pas être pyridinyle ou indolyle ;
**R^{E}, R^{F}** et **R^{G}** sont chacun indépendamment hydrogène, halogène, cyano, alkyle en C₁-C₆, alcényle en C₂-C₆, alcoxy en C₁-C₆, haloalkyle en C₁-C₆, haloalcoxy en C₁-C₆, -C(=O)**R^{s}**, -C(=O)O**R^{s}**, - C(=O)N**R^{p}R^{q}**, -**CR^{p}**(=N)O**R^{s}**, -N**R^{p}**C(=O)**R^{s}**, -N**R^{p}**C(=O)O**R^{s}**, -N**R^{p}**C(=O)N**R^{q}R^{r}**, -O**R^{s}**, -OC(=O)**R^{s}** ou -OC(=O)N**R^{p}R^{q}** ; dans lequel :
alkyle en C₁-C₆ ou alcényle en C₂-C₆ de l'un quelconque de **R^{E}**, R**^{F}** et **R^{G}** est éventuellement substitué par 1 à 3 groupes sélectionnés parmi cyano, -C(=O)**R^{s}**, -C(=O)O**R^{s}**, -C(=O)N**R^{p}R^{q}**, -N**R^{p}**C(=O)**R^{s}**, - N**R^{p}**C(=O)O**R^{s}**, -N**R^{p}**C(=O)N**R^{q}R^{r}**, -N**R^{p}**S(=O)ᵣ**R^{s}**, -O**R^{s}**, -OC(=O)**R^{s}**, - OC(=O)O**R^{s}**, -OC(=O)N**R^{p}R^{q}**, -S(=O)**ᵣR^{s}** et -S(=O)**ᵣ**N**R^{p}R^{q}** ; dans lequel :
**R^{p}**, **R^{q}** et **R^{r}**, pour chaque occurrence, sont chacun indépendamment hydrogène, alkyle en C₁-C₄, cycloalkyle en C₃-C₆ ou hétérocyclyle à 3 à 6 chaînons ; dans lequel :
alkyle en C₁-C₄ de l'un quelconque de **R^{p}**, **R^{q}** et **R^{r}** est éventuellement substitué par 1 à 3 groupes sélectionnés parmi halogène, cyano, -OH, alcoxy en C₁-C₃, -C(=O)NH(alkyle en C₁-C₂) et -C(=O)N(alkyle en C₁-C₂)₂ ; et
cycloalkyle en C₃-C₆ ou hétérocyclyle à 3 à 6 chaînons de l'un quelconque de **R^{p}**, **R^{q}** et **R^{r}** est éventuellement substitué par 1 à 3 groupes sélectionnés parmi halogène, cyano, -OH, alkyle en C₁-C₃, alcoxy en C₁-C₃, haloalkyle en C₁-C₃, haloalcoxy en C₁-C₃, - C(=O)O(alkyle en C₁-C₂), -C(=O)NH(alkyle en C₁-C₂) et - C(=O)N(alkyle en C₁-C₂)₂ ;
**R^{s}**, pour chaque occurrence, est indépendamment hydrogène, alkyle en C₁-C₄, cycloalkyle en C₃-C₆, phényle ou hétéroaryle à 5 ou 6 chaînons ; dans lequel :
alkyle en C₁-C₄ de **R^{s}** est éventuellement substitué par 1 à 3 groupes sélectionnés parmi halogène, cyano, -OH, -NH₂, - NH(alkyle en C₁-C₂), -N(alkyle en C₁-C₂)₂, alcoxy en C₁-C₃, - C(=O)OH, -C(=O)O(alkyle en C₁-C₂), -C(=O)NH₂, -C(=O)NH(alkyle en C₁-C₂) et -C(=O)N(alkyle en C₁-C₂)₂ ; et
cycloalkyle en C₃-C₆, phényle ou heteroaryle à 5 ou 6 chaînons de **R^{s}** est éventuellement substitué par 1 à 3 groupes sélectionnés parmi halogène, cyano, -OH, -OCH₃, -NH₂, -NH(alkyle en C₁-C₂), - N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ;
**R¹** est halogène, cyano, alkyle en C₁-C₃, haloalkyle en C₁-C₃, alcoxy en C₁-C₃, haloalcoxy en C₁-C₃ ou -O-(cycloalkyle en C₃-C₆) ;
**R²**, pour chaque occurrence, est indépendamment hydrogène, halogène, cyano, alkyle en C₁-C₆, alcényle en C₂-C₆, alcoxy en C₁-C₆, haloalkyle en C₁-C₆, haloalcoxy en C₁-C₆, cycloalkyle en C₃-C₆, -N**R^{h}Rⁱ**, phényle ou hétéroaryle à 5 ou 6 chaînons ; dans lequel :
alkyle en C₁-C₆, alcényle en C₂-C₆ ou cycloalkyle en C₃-C₆ de **R²** est éventuellement substitué par 1 à 3 groupes sélectionnés parmi cyano, -C(=O)**R^{k}**, -C(=O)O**R^{k}**, -C(=O)N**R^{h}Rⁱ**, -N**R^{h}Rⁱ**, -N**R^{h}**C(=O)**R^{k}**, -N**R^{h}**C(=O)O**R^{k}**, -N**R^{h}**C(=O)N**RⁱR^{j}**, -N**R^{h}**S(=O)ₛ**R^{k}**, -O**R^{k}**, -OC(=O)**R^{k}**, - OC(=O)O**R^{k}**, -OC(=O)N**R^{h}Rⁱ**, -S(=O)**ₛR^{k}** et S(=O)ₛN**R^{h}Rⁱ** ; dans lequel :
**R^{h}**, **Rⁱ** et **R^{j}**, pour chaque occurrence, sont chacun indépendamment hydrogène, alkyle en C₁-C₄ ou cycloalkyle en C₃-C₆ ; dans lequel :
alkyle en C₁-C₄ de l'un quelconque de **R^{h}, Rⁱ** et **R^{j}** est éventuellement substitué par 1 à 3 groupes sélectionnés parmi halogène, cyano, -OH, -NH₂, -NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ; et
cycloalkyle en C₃-C₆ de l'un quelconque de **R^{h}, Rⁱ** et **R^{j}** est éventuellement substitué par 1 à 3 groupes sélectionnés parmi halogène, cyano, -OH, -NH₂, -NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ;
**R^{k}**, pour chaque occurrence, est indépendamment hydrogène, alkyle en C₁-C₄, cycloalkyle en C₃-C₆, phényle ou hétéroaryle à 5 ou 6 chaînons ; dans lequel :
-O**R^{k}** ne peut pas être -OH ;
alkyle en C₁-C₄ de **R^{k}** est éventuellement substitué par 1 à 3 groupes sélectionnés parmi halogène, cyano, -OH, -NH₂, - NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, - C(=O)O(alkyle en C₁-C₂), -C(=O)NH₂, -C(=O)NH(alkyle en C₁-C₂) et -C(=O)N(alkyle en C₁-C₂)₂ ; et
cycloalkyle en C₃-C₆ de **R^{k}** est éventuellement substitué par 1 à 3 groupes sélectionnés parmi halogène, cyano, -OH, -NH₂, - NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, - C(=O)O(alkyle en C₁-C₂), -C(=O)NH₂, -C(=O)NH(alkyle en C₁-C₂) et -C(=O)N(alkyle en C₁- C₂)₂ ;
**R³** et **R⁴**, pour chaque occurrence, sont chacun indépendamment halogène, cyano, =O, alkyle en C₁-C₆, alcényle en C₂-C₆, alcoxy en C₁-C₆, haloalkyle en C₁-C₆, haloalcoxy en C₁-C₆, cycloalkyle en C₃-C₆, -C(=O)**R^{y}**, -C(=O)O**R^{y}**, -C(=O)N**R^{v}R^{w}**, -C(=O)N**R^{v}**O**R^{y}**, - C(=O)N**R^{v}**S(=O)**ₜR^{y}**, -N**R^{v}R^{w}**, -N**R^{v}**C(=O)**R^{y}**, -N**R^{v}**C(=O)O**R^{y}**, -N**R^{v}**C(=O)N**R^{w}R^{x}**, -N**R^{v}**S(=O)ₜ**R^{y}**, -O**R^{y}**, -OC(=O)**R^{y}**, -OC(=O)O**R^{y}**, -OC(=O)N**R^{v}R^{w}**, -S(=O)ₜ**R^{y}**, -S(=O)ₜN**R^{v}R^{w}**, -S(=O)ₜN**R^{v}**C(=O)**R^{y}**, -P(=O)**R^{z}R^{z}**, phényle ou hétéroaryle à 5 ou 6 chaînons ; dans lequel :
alkyle en C₁-C₆, alcényle en C₂-C₆ ou cycloalkyle en C₃-C₆ de l'un quelconque de **R³** et **R⁴** est éventuellement substitué par 1 à 3 groupes sélectionnés parmi cyano, -C(=O)**R^{y}**, -C(=O)O**R^{y}**, - C(=O)N**R**^{v}**R**^{w}, -N**R^{v}R^{w}**, -N**R^{v}**C(=O)**R^{y}**, -N**R^{v}**C(=O)O**R^{y}**, -N**R^{v}**C(=O)N**R^{w}R^{x}**, - N**R**^{v}S(=O)ᵣ**R^{y}**, -O**R^{y}**, -OC(=O)**R^{y}**, -OC(=O)O**R^{y}**, -OC(=O)N**R^{v}R^{w}**, -S(=O)**ₜR^{y}** et -S(=O)**ₜ**N**R^{v}R^{w}** ; dans lequel :
**R^{v}**, **R^{w}** et **R^{x}**, pour chaque occurrence, sont chacun indépendamment hydrogène, alkyle en C₁-C₄, cycloalkyle en C₃-C₆, hétérocyclyle à 5 ou 6 chaînons ou hétéroaryle à 5 ou 6 chaînons ; dans lequel:
alkyle en C₁-C₄ de l'un quelconque de **R^{v}**, **R^{w}** et **R^{x}** est éventuellement substitué par 1 à 3 groupes sélectionnés parmi halogène, cyano -OH, -NH₂, -NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ; et
cycloalkyle en C₃-C₆, hétérocyclyle à 5 ou 6 chaînons ou hétéroaryle à 5 ou 6 chaînons de l'un quelconque de **R^{v}**, **R^{w}** et **R^{x}** est éventuellement substitué par 1 à 3 groupes sélectionnés parmi halogène, cyano, -OH, -NH₂, -NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ;
**R^{y}**, pour chaque occurrence, est indépendamment hydrogène, alkyle en C₁-C₄, cycloalkyle en C₃-C₆, phényle, hétérocyclyle à 5 ou 6 chaînons ou hétéroaryle à 5 ou 6 chaînons ; dans lequel
alkyle en C₁-C₄ de **R^{y}** est éventuellement substitué par 1 à 3 groupes sélectionnés parmi halogène, cyano, -OH, -NH₂, - NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, - C(=O)O(alkyle en C₁-C₂), -C(=O)NH₂, -C(=O)NH(alkyle en C₁-C₂) et -C(=O)N(alkyle en C₁-C₂)₂ ; et
cycloalkyle en C₃-C₆, phényle, hétérocyclyle à 5 ou 6 chaînons ou hétéroaryle à 5 ou 6 chaînons de **R^{y}** est éventuellement substitué par 1 à 3 groupes sélectionnés parmi halogène, cyano, -OH, -NH₂, NH(alkyle en C₁-C₂), -N(alkyle en C₁-C₂)₂, alkyle en C₁-C₃, alcoxy en C₁-C₃, haloalkyle en C₁-C₃, haloalcoxy en C₁-C₃, -C(=O)OH, -C(=O)O(alkyle en C₁-C₂), -C(=O)NH₂, -C(=O)NH(alkyle en C₁-C₂) et -C(=O)N(alkyle en C₁-C₂)₂ ;
**R^{z}**, pour chaque occurrence, est indépendamment alkyle en C₁-C₂, -OH ou -O(alkyle en C₁-C₂) ;
**k** est un entier sélectionné parmi 1, 2 et 3 ;
**m** et **n** a sont chacun indépendamment un entier sélectionné parmi 0, 1, 2 et 3 ;
**p, r, s,** et **t** sont chacun indépendamment un entier sélectionné parmi 1 et 2 ; et
**q** est un entier sélectionné parmi 1, 2 et 3.

2. Composé, tautomère, dérivé deutéré ou sel pharmaceutiquement acceptable selon la revendication 1, représenté par l'une des formules structurales suivantes : 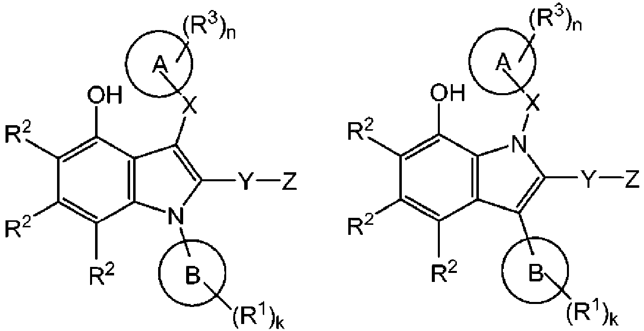 dans lequel toutes les autres variables non spécifiquement définies ici sont telles que définies dans la revendication 1.

3. Composé, tautomère, dérivé deutéré ou sel pharmaceutiquement acceptable selon la revendication 1, représenté par l'une des formules structurales suivantes : 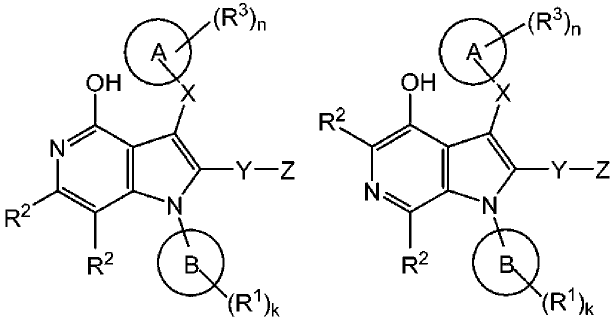 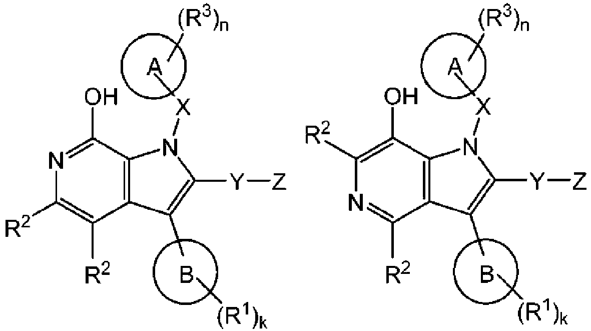 dans lequel toutes les autres variables non spécifiquement définies ici sont telles que définies dans la revendication 1.

4. Composé, tautomère, dérivé deutéré ou sel pharmaceutiquement acceptable selon la revendication 1 ou la revendication 2, représenté par l'une des formules structurales suivantes : 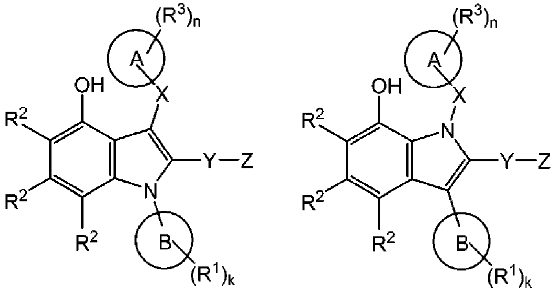 dans lequel :
le **cycle A** est éventuellement substitué par **R³** et le **cycle A** est carbocyclyle en C₃-C₇, hétérocyclyle à 6 à 9 chaînons, phényle ou hétéroaryle à 5 à 9 chaînons ;
le **cycle B** est substitué par **R¹** et le **cycle B** est cycloalkyle en C₄-C₆, phényle, hétéroaryle à 5 à 6 chaînons ou benzyle ; et
lorsque **Z** est le **cycle C** éventuellement substitué par **R⁴**, le **cycle C** est cycloalkyle en C₄-C₈, hétérocyclyle à 4 à 8 chaînons, phényle ou hétéroaryle à 5 ou 6 chaînons ;
et dans lequel toutes les autres variables non spécifiquement définies ici sont telles que définies dans la revendication 1 ou la revendication 2.

5. Composé, tautomère, dérivé deutéré ou sel pharmaceutiquement acceptable selon l'une quelconque des revendications 1, 2 et 4, représenté par l'une des formules structurales suivantes : 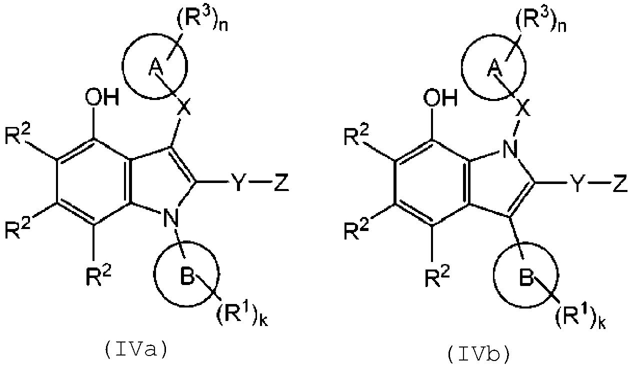 dans lequel :
**le cycle B** est substitué par **R¹** et le **cycle B** est cyclohexyle, phényle, pyridinyle ou benzyle ; et dans lequel toutes les autres variables non spécifiquement définies ici sont telles que définies dans l'une quelconque des revendications 1, 2 et 4,
éventuellement dans lequel le composé, le tautomère, le dérivé deutéré ou le sel pharmaceutiquement acceptable est représenté par l'une des formules structurales suivantes : 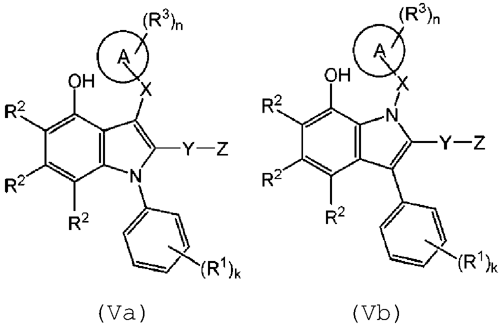
dans lequel :
**R¹** est halogène, cyano, alkyle en C₁-C₂, haloalkyle en C₁-C₂ ou alcoxy en C₁-C₂ ; et
**k** est un entier sélectionné parmi 1 et 2.

6. Composé, tautomère, dérivé deutéré ou sel pharmaceutiquement acceptable selon l'une quelconque des revendications 1 à 5, dans lequel :
(i) **R¹** est cyano, F, Cl, -CH₃, -CHF₂, -CF₃, -OCH₃ ou -OCH(CH₃)₂ ;
(ii) au moins un **R¹** est F ;
(iii) **X** est absent ou une liaison, -(C**R^{a}R^{b}**)- ou -SO₂- ;
**R^{a}** et **R^{b}**, pour chaque occurrence, sont chacun indépendamment hydrogène, alkyle en C₁-C₃, haloalkyle en C₁-C₃ ou alcoxy en C₁-C₃ ;
(iv) **X** est absent ou une liaison, -CH₂- ou -SO₂- ;
(v) **Y** est absent ou une liaison, -(C**R^{c}R^{d}**)_{q}-, -C(=O)- ou -SO₂- ; **R^{c}** et **R^{d}**, pour chaque occurrence, sont chacun indépendamment hydrogène, alkyle en C₁-C₃, haloalkyle en C₁-C₃ ou alcoxy en C₁-C₃ ;
(vi) **q** est un entier sélectionné parmi 1 et 2 ; et/ou
(vii) **Y** est absent ou une liaison, -CH₂-, -CHCH₃-, -C(CH₃)₂-, - C(=O)- ou -SO₂- ;
et dans lequel toutes les autres variables non spécifiquement définies ici sont telles que définies dans l'une quelconque des revendications précédentes.

7. Composé, tautomère, dérivé deutéré ou sel pharmaceutiquement acceptable selon l'une quelconque des revendications 1 à 6, dans lequel le **cycle A** est éventuellement substitué par **R³** et le **cycle A** est :
(i) carbocyclyle en C₃-C₇, hétérocyclyle à 6 à 9 chaînons contenant 1 à 3 atomes d'oxygène, phényle ou hétéroaryle à 5 à 9 chaînons contenant 1 à 3 hétéroatomes sélectionnés parmi O et N ;
(ii) carbocyclyle en C₃-C₇, hétérocyclyle à 6 à 9 chaînons contenant un ou deux atomes d'oxygène, phényle ou hétéroaryle à 5 à 9 chaînons contenant un ou deux atomes d'azote ou un ou deux atomes d'oxygène ;
(iii) sélectionné parmi 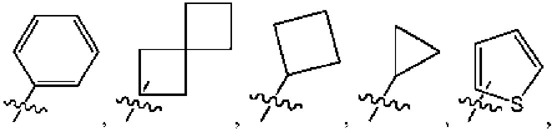 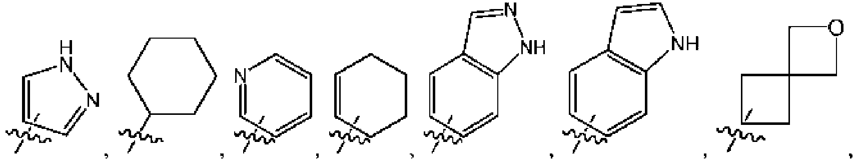 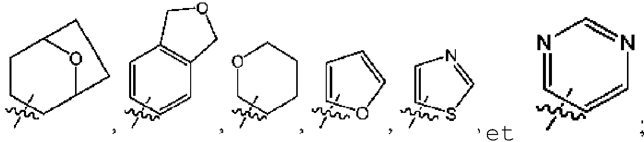 et/ou
(iv) sélectionné parmi 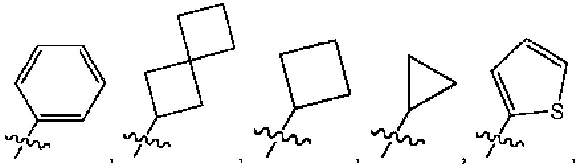 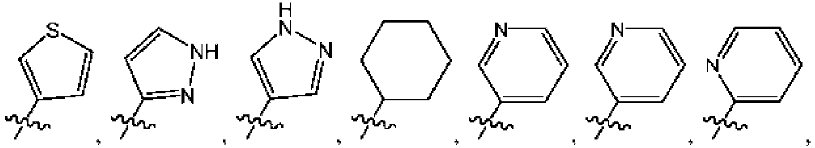 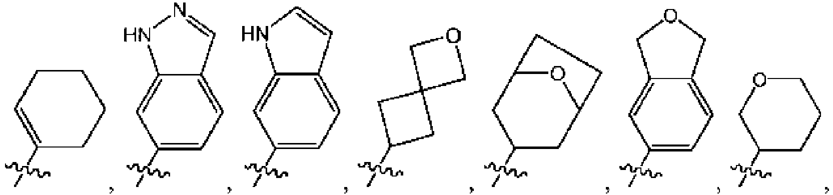 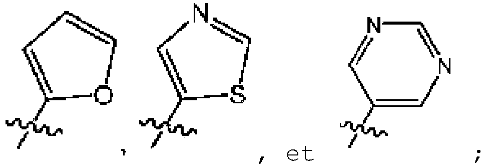
et dans lequel toutes les autres variables non spécifiquement définies ici sont telles que définies dans l'une quelconque des revendications précédentes.

8. Composé, tautomère, dérivé deutéré ou sel pharmaceutiquement acceptable selon l'une quelconque des revendications 1 à 7, dans lequel **Z** est le **cycle C** éventuellement substitué par **R⁴** et le **cycle C** est :
(i) cycloalkyle en C₄-C₈, hétérocyclyle à 4 à 8 chaînons contenant un ou deux hétéroatomes sélectionnés parmi O, N et S, phényle ou hétéroaryle à 5 chaînons contenant un ou deux hétéroatomes sélectionnés parmi O et N ;
(ii) sélectionné parmi 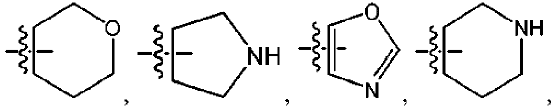 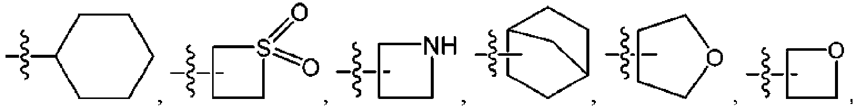 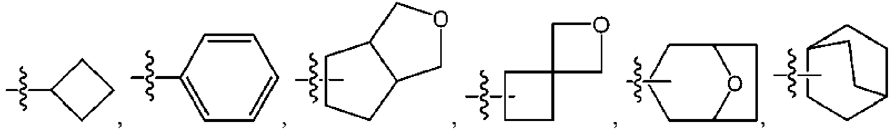 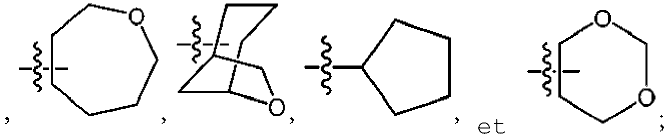 et/ou
(iii) sélectionné parmi 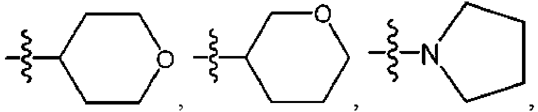 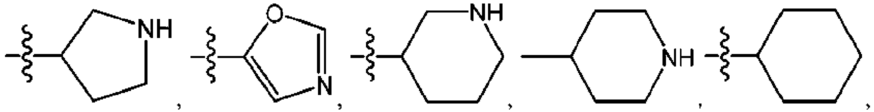 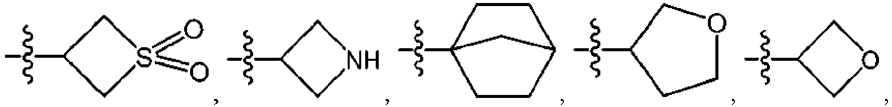 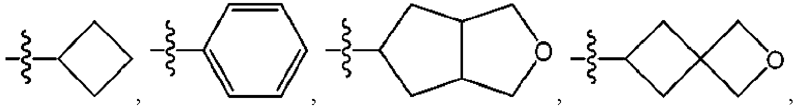 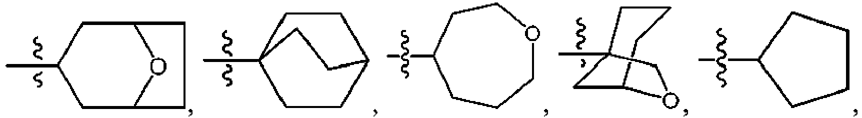 et 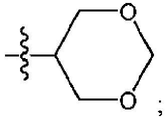
et dans lequel toutes les autres variables non spécifiquement définies ici sont telles que définies dans l'une quelconque des revendications précédentes.

9. Composé, tautomère, dérivé deutéré ou sel pharmaceutiquement acceptable selon l'une quelconque des revendications 1 à 8, dans lequel **R^{E}**, **R^{F}** et **R^{G}** sont chacun indépendamment :
(i) hydrogène, halogène, cyano (-C≡N), alkyle en C₁-C₄, alcoxy en C₁-C₄, haloalkyle en C₁-C₄, -C(=O)O**R^{s}**, -C(=O)N**R^{p}R^{q}**, -C**R^{p}**(=N)O**R^{s}** ou -O**R^{s}** ; dans lequel :
alkyle en C₁-C₄ de l'un quelconque de **R^{E}**, **R^{F}** et **R^{G}** est éventuellement substitué par 1 à 3 groupes sélectionnés parmi cyano, -C(=O)**R^{s}**, -C(=O)O**R^{s}**, -C(=O)N**R^{p}R^{q}**, -O**R^{s}**, -OC(=O)**R^{s}**, - OC(=O)O**R^{s}**, -OC(=O)N**R^{p}R^{q}** et -S(=O)₂**R^{s}** ; dans lequel :
**R^{p}** et **R^{q}**, pour chaque occurrence, sont chacun indépendamment hydrogène, alkyle en C₁-C₂, cycloalkyle en C₃-C₅ ou hétérocyclyle à 5 ou 6 chaînons ; dans lequel :
alkyle en C₁-C₂ de l'un quelconque de **R^{p}** et **R^{q}** est éventuellement substitué par 1 à 3 groupes sélectionnés parmi halogène, cyano et -OH ;
cycloalkyle en C₃-C₅ ou hétéroaryle à 5 ou 6 chaînons de **R^{p}** et **R^{q}** est éventuellement substitué par 1 à 3 groupes sélectionnés parmi halogène, cyano et -OH ;
**R^{s}**, pour chaque occurrence, est indépendamment hydrogène, alkyle en C₁-C₂ ou hétéroaryle à 5 ou 6 chaînons ; dans lequel alkyle en C₁-C₂ de **R^{s}** est éventuellement substitué par 1 à 3 groupes sélectionnés parmi halogène, cyano, -OH et -NH₂ ; dans lequel : hétéroaryle à 5 ou 6 chaînons de **R^{s}** est éventuellement substitué par 1 à 3 groupes sélectionnés parmi halogène, cyano, -OH et - NH₂ ;
(ii) hydrogène, F, Cl, alkyle en C₁-C₂, haloalkyle en C₁-C₂, - C(=O)N**R^{p}R^{q}**, -C**R^{p}**(=N)O**R^{s}** ou -O**R^{s}** ; dans lequel :
alkyle en C₁-C₂ de l'un quelconque de **R^{E}**, **R^{F}** et **R^{G}** est éventuellement substitué par 1 à 3 groupes sélectionnés parmi cyano, -C(=O)N**R^{p}R^{q}**, -O**R^{s}**, -OC(=O)N**R^{p}R^{q}** et -S(=O)₂**R^{s}** ; dans lequel :
**R^{p}** et **R^{q}**, pour chaque occurrence, sont chacun indépendamment hydrogène, alkyle en C₁-C₂, cyclopentyle ou tétrahydrofuranyle ;
dans lequel :
alkyle en C₁-C₂ de l'un quelconque de **R^{p}** et **R^{q}** est éventuellement substitué par 1 à 3 groupes halogène sélectionnés parmi F et Cl ;
**R^{s}**, pour chaque occurrence, est indépendamment hydrogène, alkyle en C₁-C₂, pyridinyle ou pyrimidinyle ; dans lequel :
alkyle en C₁-C₂ de **R^{s}** est éventuellement substitué par 1 à 3 groupes halogène sélectionnés parmi F et Cl ;
(iii) hydrogène, F, -OH, -CH(OH)CH₃, -C(=O)NHCH₃, -C(=N)OCH₃, - CH₃, -CF₃, -CH₂F, -CH₂CN, -(CH₂)₂CN, -CH₂OH, -C₂H₅, -(CH₂)₂OH, - CH₂OCH₃, -CH₂OC₂H₅, -(CH₂)₂OCH₃, -CH₂OCHF₂, -(CH₂)₂OCHF₂, - CH₂C(=O)NH₂, -CH₂C(=O)N(CH₃)₂, -CH₂S(=O)₂CH₃, -(CH₂)₂S(=O)₂CH₃, - CH₂(O)C(=O)NHCH₃, -CH₂(O)C(=O)N(CH₃)C₂H₅,-CH₂(O)C(=O)N(CH₃)₂, - CH₂(O)C(=O)N(C₂H₅)₂, -CH₂(O)C(=O)NH(cyclopentyle), - CH₂(O)C(=O)NH(tétrahydrofuranyle), -CH₂(O)(pyridin-2-yle), ou - CH₂(O)(pyrimidin-2-yle) ; et/ou
(iv) hydrogène, F, -CH(OH)CH₃, -CH₃, -CH₂CN, -CH₂OH ou -CH₂OCH₃ ; et dans lequel toutes les autres variables non spécifiquement définies ici sont telles que définies dans l'une quelconque des revendications précédentes.

10. Composé, tautomère, dérivé deutéré ou sel pharmaceutiquement acceptable selon l'une quelconque des revendications 1 à 9 :
(i) représenté par l'une des formules structurales suivantes : 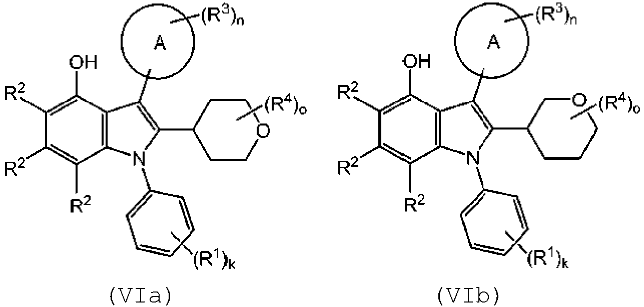 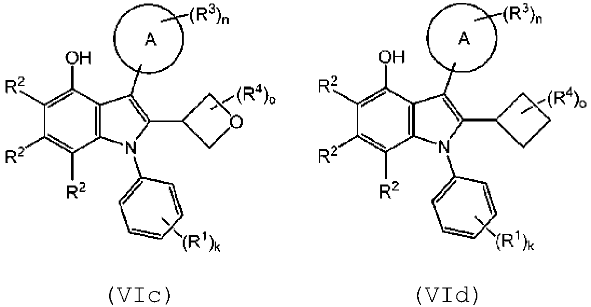 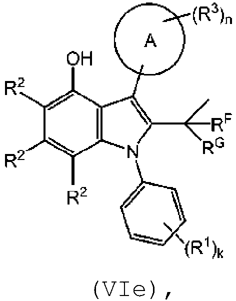 dans lequel **o** est un entier sélectionné parmi 0, 1 et 2 ;
(ii) représenté par l'une des formules structurales suivantes : 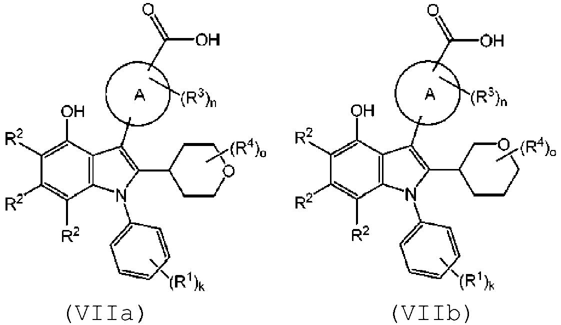 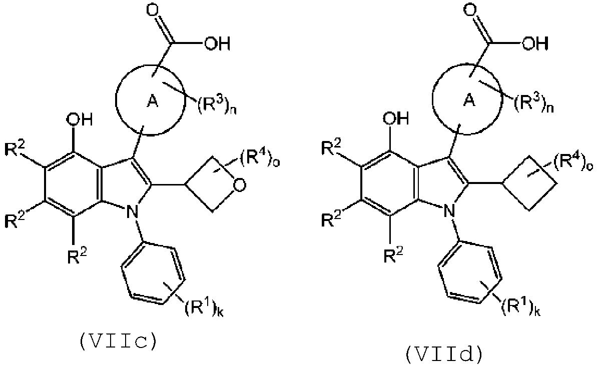 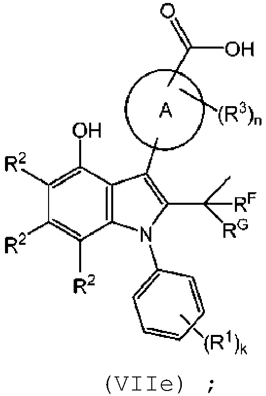 dans lequel **n** est un entier sélectionné parmi 0, 1 et 2, et dans lequel **o** est un entier sélectionné parmi 0, 1 et 2 ;
(iii) représenté par l'une des formules structurales suivantes : 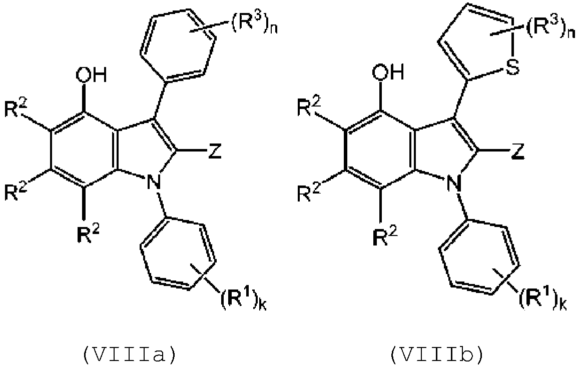 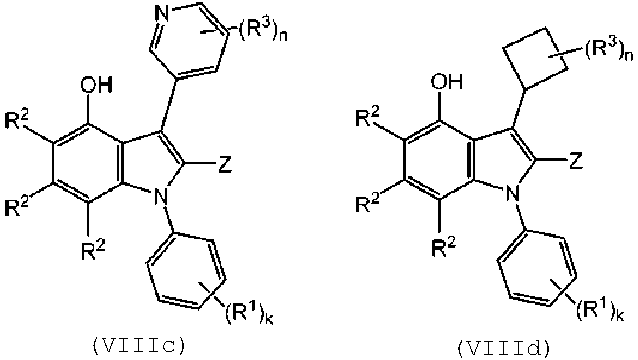 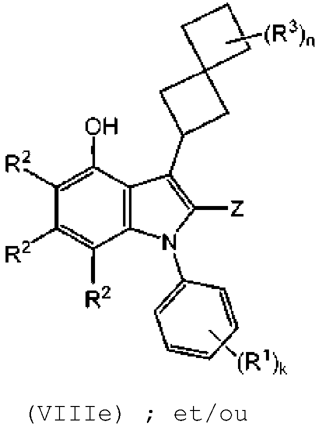
(iv) représenté par l'une des formules structurales suivantes : 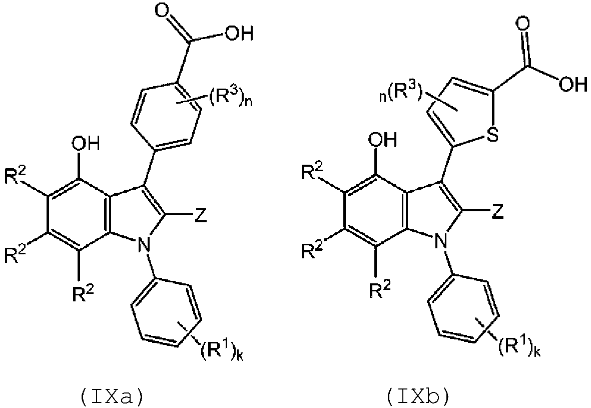 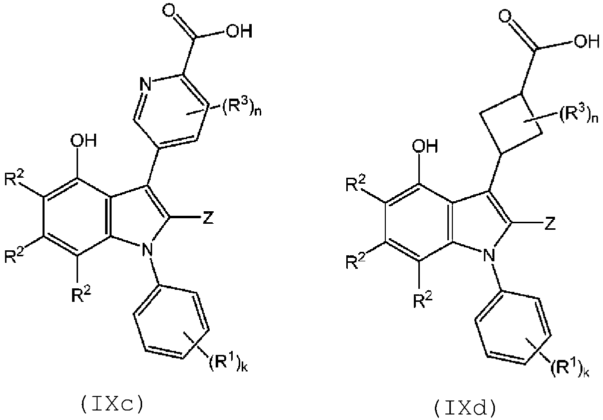 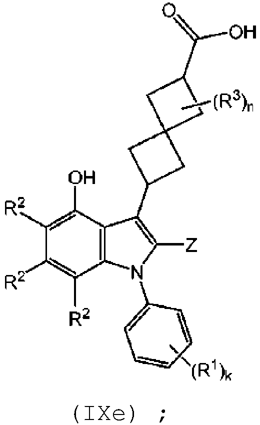
dans lequel **n** est un entier sélectionné parmi 0, 1 et 2 ; et dans lequel toutes les autres variables non spécifiquement définies ici sont telles que définies dans l'une quelconque des revendications précédentes.

11. Composé, tautomère, dérivé deutéré ou sel pharmaceutiquement acceptable selon l'une quelconque des revendications 1 à 10, dans lequel :
(i) **R²**, pour chaque occurrence, est indépendamment hydrogène, halogène, cyano, alkyle en C₁-C₆ (éventuellement substitué par 1 à 3 groupes sélectionnés parmi cyano, -OH, -OCH₃ et -NH₂), alcoxy en C₁-C₆, haloalkyle en C₁-C₆, -N**R^{h}Rⁱ** ou cycloalkyle en C₃-C₆ ;
dans lequel **R^{h}** et **Rⁱ**, pour chaque occurrence, sont indépendamment hydrogène ou alkyle en C₁-C₄ ;
(ii) **R²**, pour chaque occurrence, est indépendamment hydrogène, halogène, cyano, alkyle en C₁-C₄ (éventuellement substitué par 1 à 3 groupes sélectionnés parmi cyano, -OH, -OCH₃ et -NH₂), alcoxy en C₁-C₄, haloalkyle en C₁-C₄, -N**R^{h}Rⁱ** ou cycloalkyle en C₃-C₅ ; dans lequel **R^{h}** et **Rⁱ**, pour chaque occurrence, sont chacun indépendamment hydrogène ou alkyle en C₁-C₄ ;
(iii) **R²**, pour chaque occurrence, est indépendamment hydrogène, halogène, cyano, alkyle en C₁-C₂ (éventuellement substitué par 1 à 3 groupes sélectionnés parmi cyano, -OH, -OCH₃ et -NH₂), haloalkyle en C₁-C₂, -N**R^{h}Rⁱ**, ou cycloalkyle en C₃-C₄ ; dans lequel **R^{h}** et **Rⁱ**, pour chaque occurrence, sont chacun indépendamment hydrogène ou -CH₃ ;
(iv) **R²**, pour chaque occurrence, est indépendamment hydrogène, F, Cl, cyano, -CH₃, -CHF₂, -CF₃, -NH₂ ou cyclopropyle ;
(v) **R³**, pour chaque occurrence, est indépendamment halogène, cyano, =O, alkyle en C₁-C₆, alcoxy en C₁-C₆, haloalkyle en C₁-C₆, -C(=O)**R^{y}**, -C(=O)O**R^{y}**, -C(=O)N**R^{v}R^{w}**, -C(=O)N**R^{v}**O**R^{y}**, -C(=O)NR^{v}S(=O)₂**R^{y}**, -N**R^{v}R^{w}**, -O**R^{y}**, -S(=O)₂**R^{y}**, -S(=O)₂N**R^{v}R^{w}**, -S(=O)₂N**R^{v}**C(=O)**R^{y}** ou - P(=O)**R^{z}R^{z}** ; dans lequel :
alkyle en C₁-C₄ de **R³** est éventuellement substitué par 1 à 3 groupes sélectionnés parmi cyano, -O**R^{y}**, -C(=O)O**R^{y}** et -N**R^{v}R^{w}** ;
dans lequel :
**R^{v}**, **R^{w}** et **R^{x}**, pour chaque occurrence, sont chacun indépendamment hydrogène ou alkyle en C₁-C₂ ; dans lequel alkyle en C₁-C₂ de l'un quelconque de **R^{v}**, **R^{w}** et **R^{x}** est éventuellement substitué par 1 à 3 groupes sélectionnés parmi halogène, cyano, -OH et -NH₂ ; et
**R^{y}**, pour chaque occurrence, est éventuellement hydrogène, alkyle en C₁-C₄ ou hétérocyclyle à 5 ou 6 chaînons ; dans lequel :
alkyle en C₁-C₄ de **R^{y}** est éventuellement substitué par 1 à 3 groupes sélectionnés parmi halogène, cyano, -OH, -NH₂ et - C(=O)OH ; et
hétérocyclyle à 5 ou 6 chaînons de **R^{y}** est éventuellement substitué par 1 à 3 groupes sélectionnés parmi halogène, cyano, -OH, -NH₂ et-C(=O)OH ;
(vi) **R³**, pour chaque occurrence, est indépendamment halogène, cyano, =O, alkyle en C₁-C₄, alcoxy en C₁-C₄, haloalkyle en C₁-C₄, -C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)₂**R^{y}**, -N**R^{v}R^{w}**, -O**R^{y}**, -S(=O)₂**R^{y}**, -S(=O)₂N**R^{v}R^{w}**, -S(=O)₂N**R^{v}**C(=O)**R^{y}** ou - P(=O)**R^{z}R^{z}** ; dans lequel :
alkyle en C₁-C₄ de **R³** est éventuellement substitué par 1 à 3 groupes sélectionnés parmi cyano, -O**R^{y}** et -C(=O)O**R^{y}** ; dans lequel :
**R^{v}**, **R^{w}** et **R^{x}**, pour chaque occurrence, sont chacun indépendamment hydrogène ou alkyle en C₁-C₄ ; dans lequel alkyle en C₁-C₄ de l'un quelconque de **R^{v}**, **R^{w}** et **R^{x}** est éventuellement substitué par -OH ; et
**R^{y}**, pour chaque occurrence, est éventuellement hydrogène, alkyle en C₁-C₂ ou hétérocyclyle à 6 chaînons ; dans lequel :
alkyle en C₁-C₂ de **R^{y}** est éventuellement substitué par 1 à 3 groupes sélectionnés parmi -OH et -C(=O)OH ; et
hétérocyclyle à 6 chaînons de **R^{y}** est éventuellement substitué par 1 à 3 groupes sélectionnés parmi -OH et -C(=O)OH ;
**R^{z}**, pour chaque occurrence, est indépendamment CH₃, -OH ou -OCH₃ ;
(vii) **R³**, pour chaque occurrence, est indépendamment halogène, cyano, =O, alkyle en C₁-C₂, alcoxy en C₁-C₂, haloalkyle en C₁-C₄, -C(=O)**R^{y}**, -C(=O)O**R^{y}**, -C(=O)N**R^{v}R^{w}**, -C(=O)N**R^{v}**O**R^{y}**, -C(=O)N**R^{v}**S(=O)₂**R^{y}**, -N**R^{v}R^{w}**, -O**R^{y}**, -S(=O)₂**R^{y}**, -S(=O)₂N**R^{v}R^{w}**, -S(=O)₂N**R^{v}**C(=O)**R^{y}** ou - P(=O)**R^{z}R^{z}** ; dans lequel :
alkyle en C₁-C₂ de **R³** est éventuellement substitué par 1 à 3 groupes sélectionnés parmi OH et -C(=O)O**R^{y}** ; dans lequel :
**R^{v}**, **R^{w}** et **R^{x}**, pour chaque occurrence, sont chacun indépendamment hydrogène ou alkyle en C₁-C₂ ; dans lequel alkyle en C₁-C₂ de l'un quelconque de **R^{v}**, **R^{w}** et **R^{x}** est éventuellement substitué par -OH ; et
**R^{y}**, pour chaque occurrence, est indépendamment hydrogène, alkyle en C₁-C₂ ou tétrahydro-2H-pyranyle ; dans lequel :
alkyle en C₁-C₂ de **R^{y}** est éventuellement substitué par -C(=O)OH ; et
tétrahydro-2H-pyranyle de **R^{y}** est éventuellement substitué par 1 à 3 groupes sélectionnés parmi -OH et -C(=O)OH ;
**R^{z}**, pour chaque occurrence, est indépendamment -CH₃ ou -OH ;
(viii) **R³**, pour chaque occurrence, est indépendamment F, Cl, cyano, -OH, =O, -CH₃, -OCH₃, -CF₃, -CH₃CN, -C(CH₃)₂CH₂OH, -CH₂COOH, -CH₂OCH₃, -C(=O)CHCH₃OH, -COOH, -C(=O)O(2-tetrahydro-2H-pyranyl), -C(=O)NH₂, -C(=O)NH(CH₂)₂OH, -C(=O)NHOH, -C(=O)NHS(=O)₂CH₃, -NH₂, -NHCH₃, -OCH₂COOH, NHS(=O)₂CH₃, -S(=O)₂CH₃, -S(=O)₂NH₂, - S(=O)₂NHC(=O)CH₃ ou -P(=O)(CH₃)₂ ; dans lequel 2-tétrahydro-2H-pyranyle dans -C(=O)O(2-tétrahydro-2H-pyranyl) est substitué par 1 à 3 groupes sélectionnés parmi -OH et -C(=O)OH ;
(ix) **R⁴**, pour chaque occurrence, est indépendamment halogène, cyano, alkyle en C₁-C₆, alcoxy en C₁-C₆, haloalkyle en C₁-C₆, - C(=O)**R^{y}**, -C(=O)O**R^{y}**, -O**R^{y}** ou -S(=O)₂**R^{y}**; dans lequel :
alkyle en C₁-C₆ de **R⁴** est éventuellement substitué par 1 à 3 groupes sélectionnés parmi cyano, -O**R^{y}**, -C(=O)O**R^{y}** et -N**R^{v}R^{w}** ;
dans lequel :
**R^{v}** et **R^{w}**, pour chaque occurrence, sont chacun indépendamment hydrogène ou alkyle en C₁-C₄ ; et
**R^{y}**, pour chaque occurrence, est éventuellement hydrogène ou alkyle en C₁-C₄ ; dans lequel :
alkyle en C₁-C₄ de **R^{y}** est éventuellement substitué par 1 à 3 groupes sélectionnés parmi halogène, cyano, -OH, -OCH₃ et - NH₂ ;
(x) **R⁴**, pour chaque occurrence, est indépendamment halogène, cyano, alkyle en C₁-C₄, alcoxy en C₁-C₄, haloalkyle en C₁-C₄, - C(=O)**R^{y}**, -C(=O)O**R^{y}**, -O**R^{y}** ou -S(=O)₂**R^{y}** ; dans lequel :
alkyle en C₁-C₄ de **R⁴** est éventuellement substitué par 1 à 3 groupes sélectionnés parmi cyano, -O**R^{y}**, -C(=O)O**R^{y}** et -N**R^{v}R^{w}** ;
dans lequel :
**R^{v}** et **R^{w}**, pour chaque occurrence, sont chacun indépendamment hydrogène ou alkyle en C₁-C₄ ;
**R^{y}**, pour chaque occurrence, est indépendamment hydrogène ou alkyle en C₁-C₂ ; dans lequel :
alkyle en C₁-C₂ de l'un quelconque de **R^{y}** est éventuellement substitué par 1 à 3 groupes sélectionnés parmi halogène, cyano, -OH, -OCH₃ et -NH₂ ; et
dans lequel **o** ou **m** est un entier sélectionné parmi 0, 1 et 2 ;
(xi) **R⁴**, pour chaque occurrence, est indépendamment cyano, alkyle en C₁-C₂, alcoxy en C₁-C₂, haloalkyle en C₁-C₂, -C(=O)R^{y}, -C(=O)O**R^{y}**, -O**R^{y}** ou -S(=O)₂**R^{y}** ; dans lequel :
alkyle en C₁-C₂ de **R⁴** est éventuellement substitué par cyano, - OH ou -OCH₃ ;
**R^{y}**, pour chaque occurrence, est indépendamment hydrogène ou alkyle en C₁-C₂ ; dans lequel :
alkyle en C₁-C₂ de **R^{y}** est éventuellement substitué par -OCH₃ ; dans lequel **o** ou **m** est un entier sélectionné parmi 0 et 1 ; et/ou
(xii) **R⁴**, pour chaque occurrence, est indépendamment cyano, -OH, -OCH₃, -CH₃, -C₂H₅, -CF₃, -CH₂CN, -CH₂OH, -CH₂OCH₃, -COOH, -C(=O)CH₃, -C(=O)OCH₃, -C(=O)CH₂OCH₃, -S(=O)₂CH₃, -S(=O)₂C₂H₅ ou S(=O)₂CF₃ ; et dans lequel toutes les autres variables non spécifiquement définies ici sont telles que définies dans l'une des revendications précédentes.

12. Composé selon la revendication 1, dans lequel le composé est sélectionné parmi : 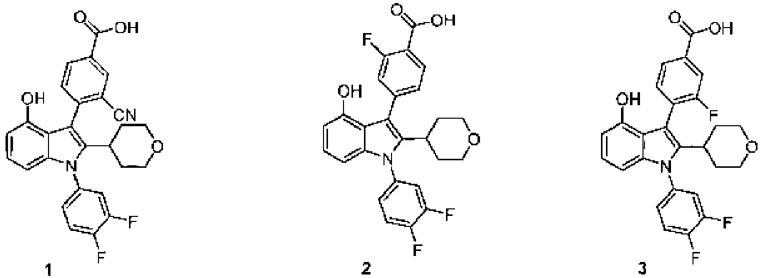 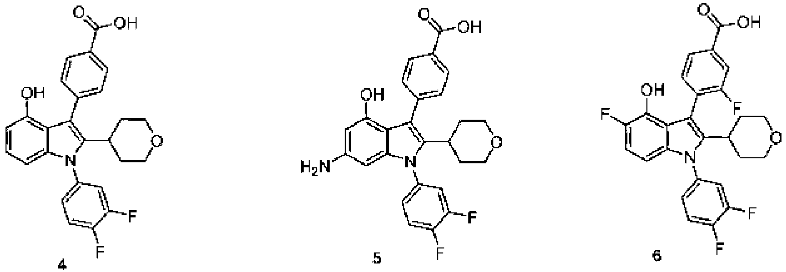 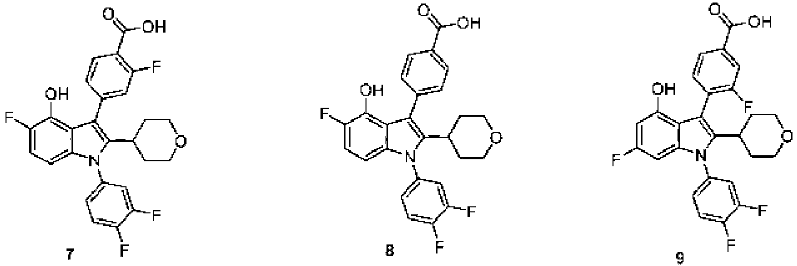 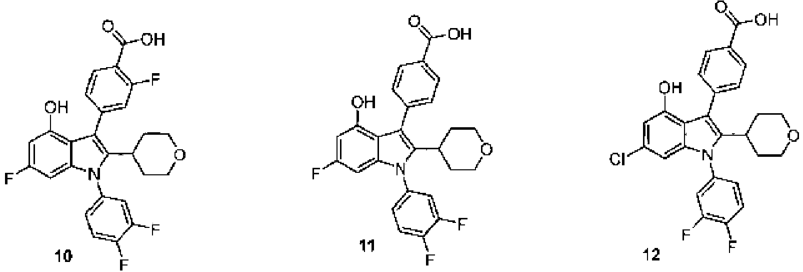 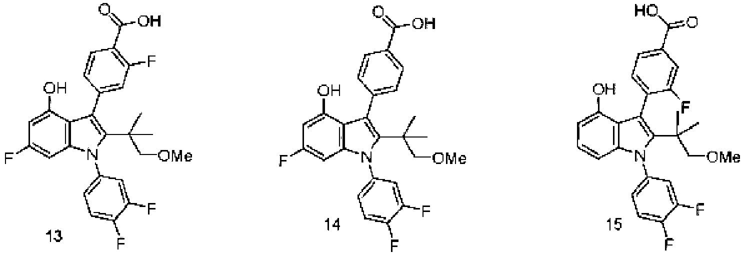 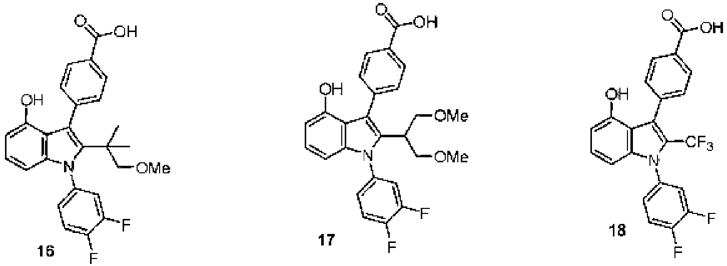 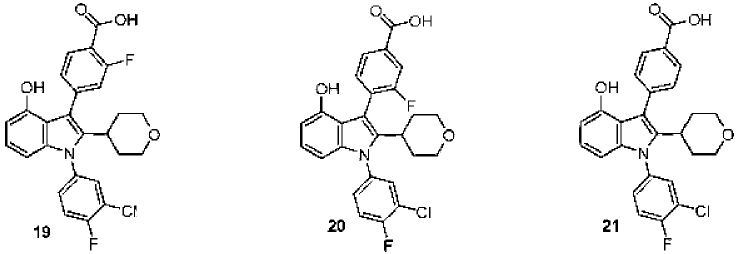 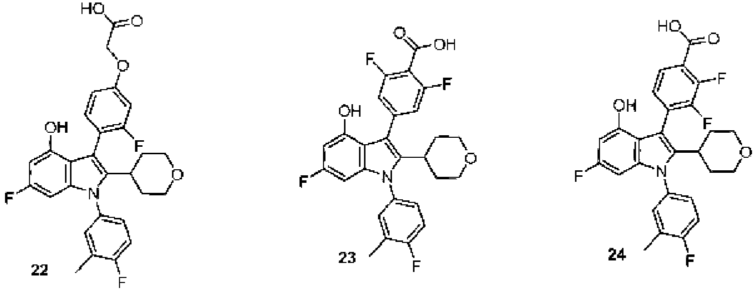 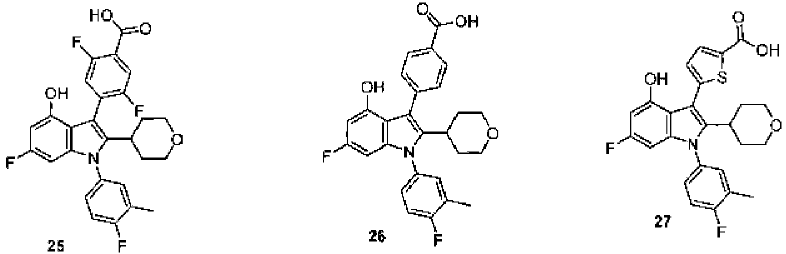 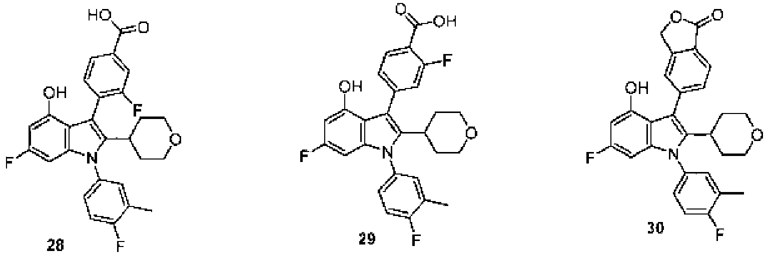 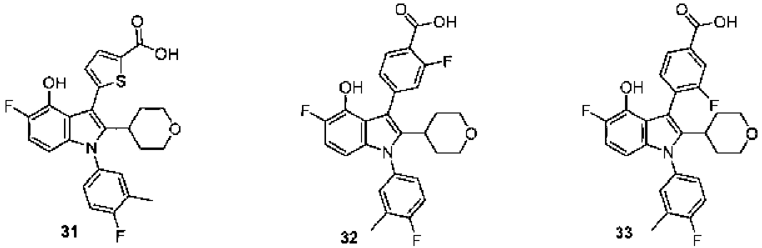 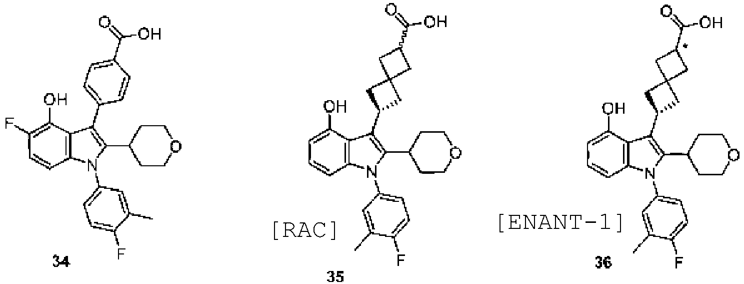 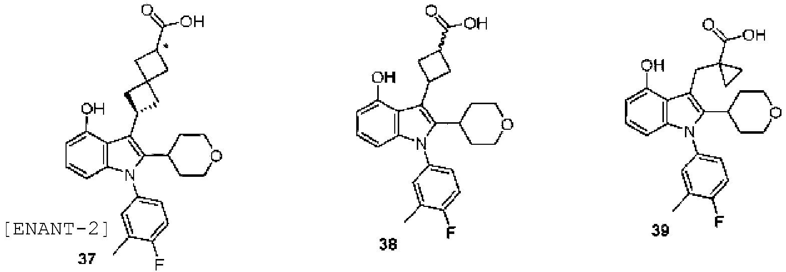 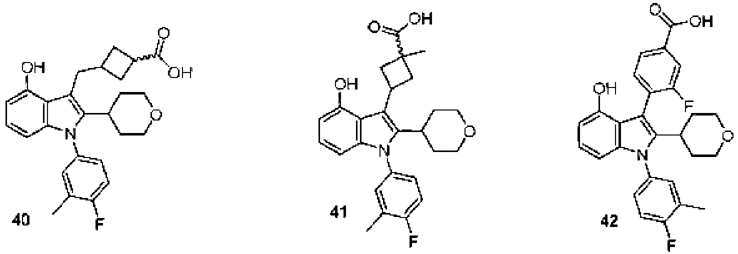 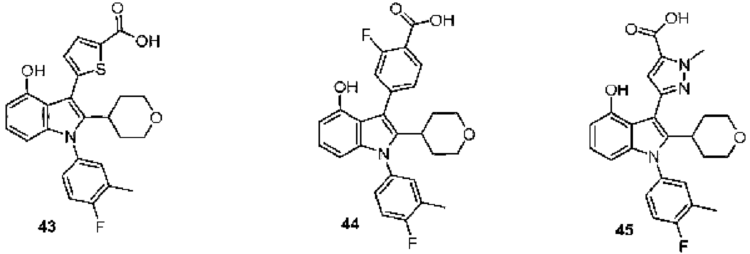 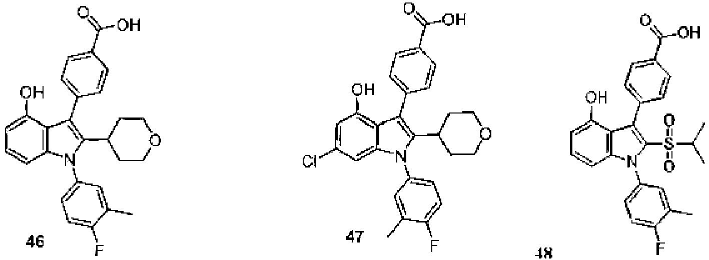 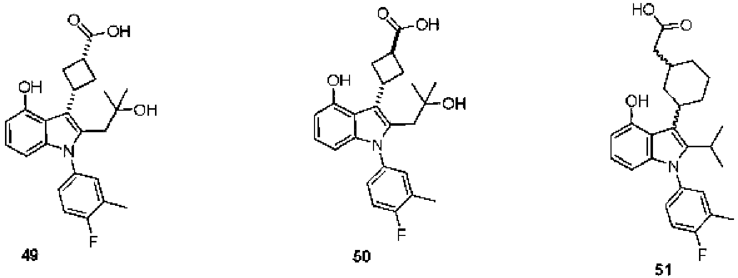 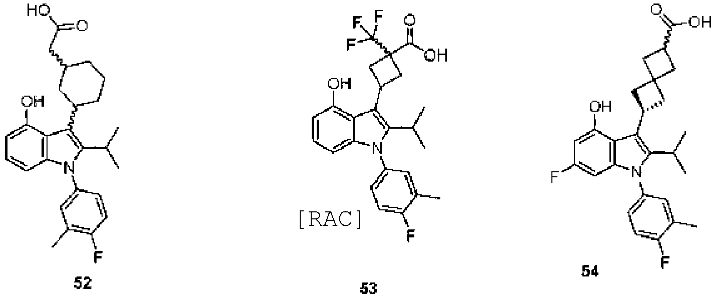 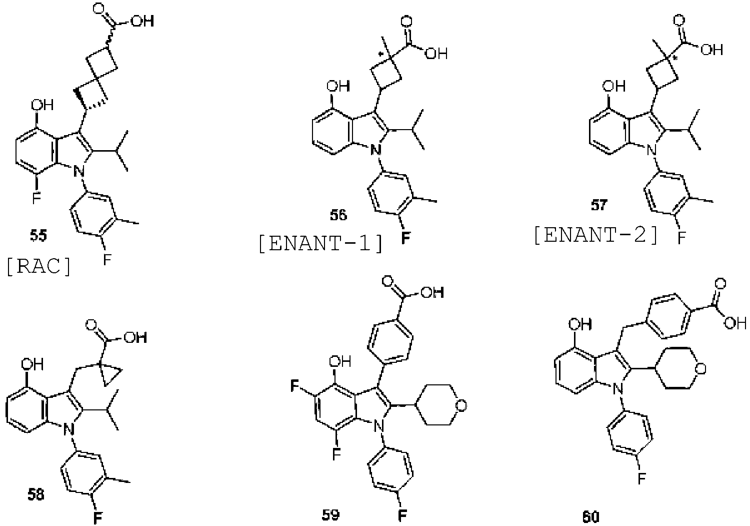 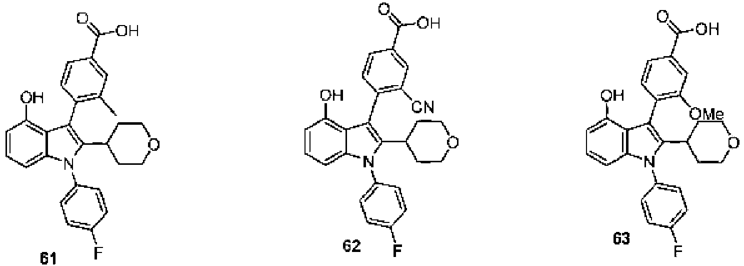 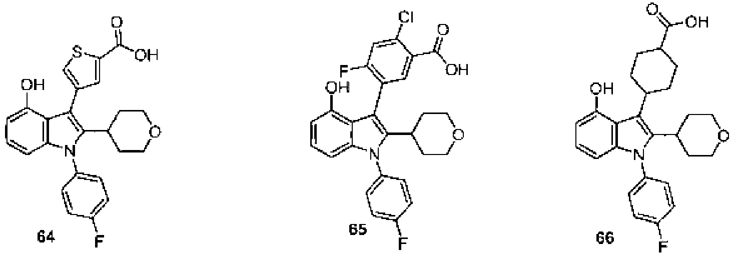 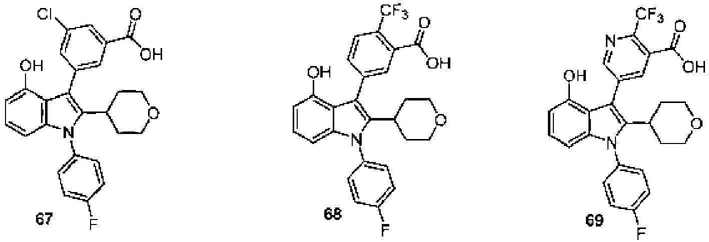 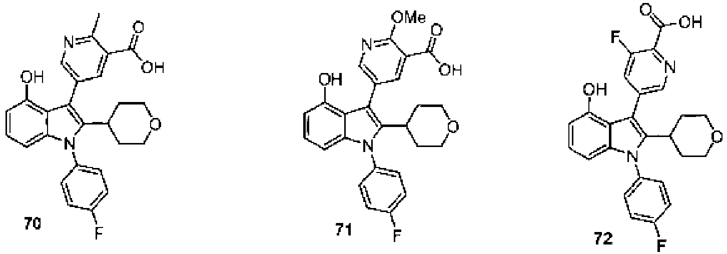 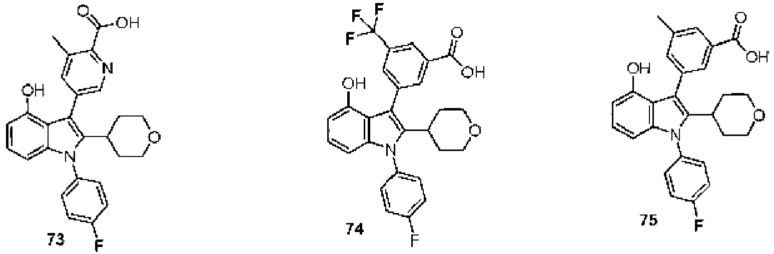 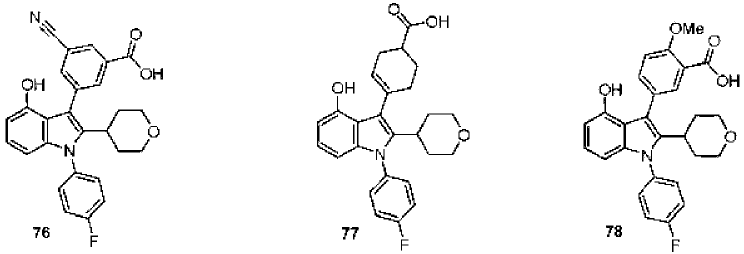 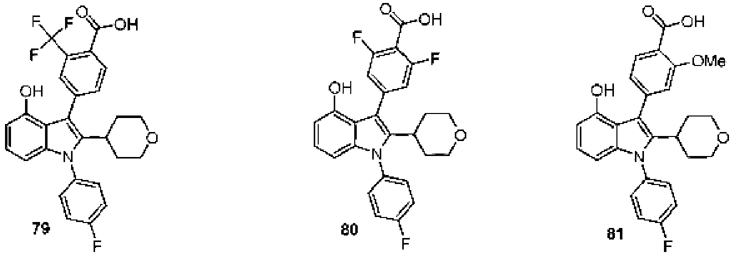 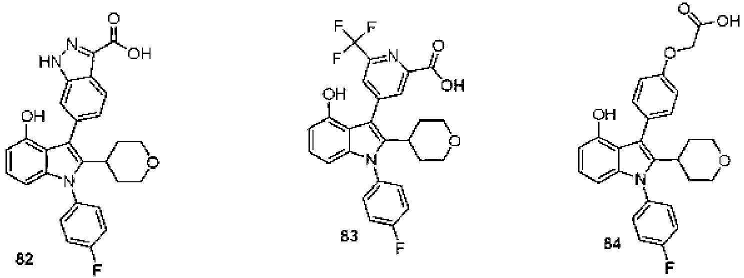 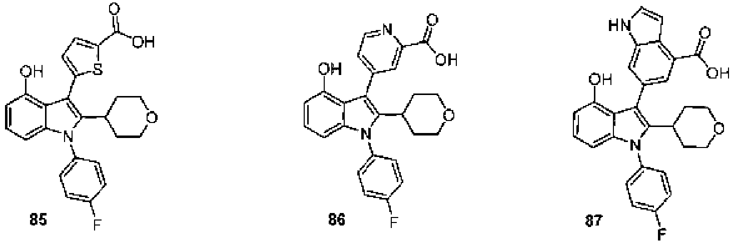 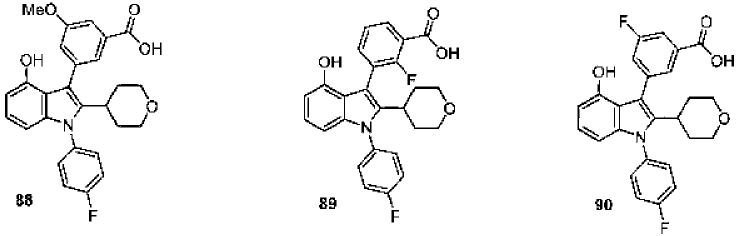 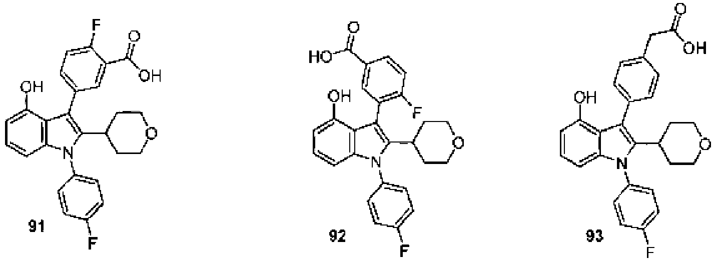 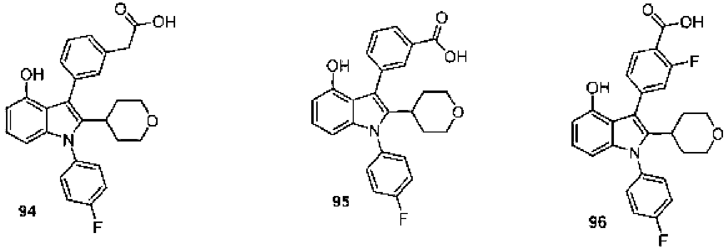 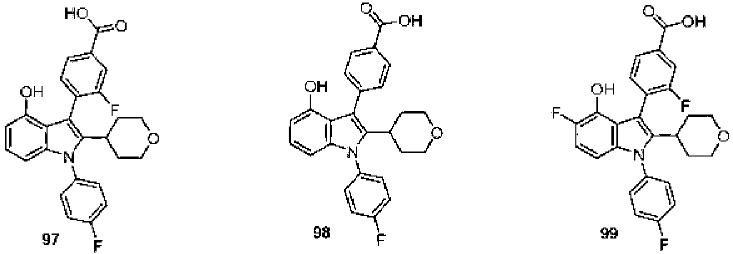 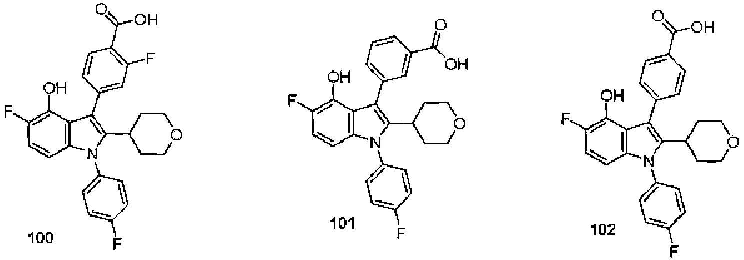 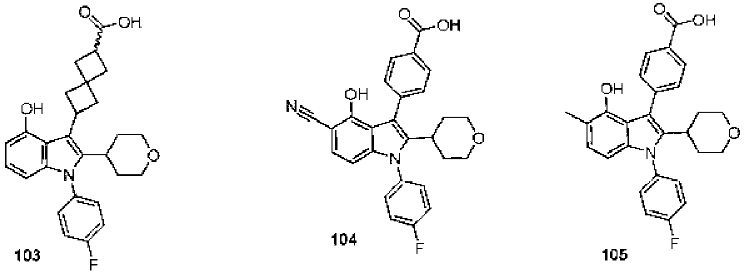 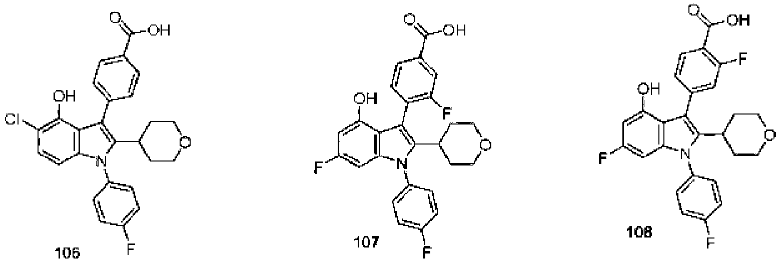 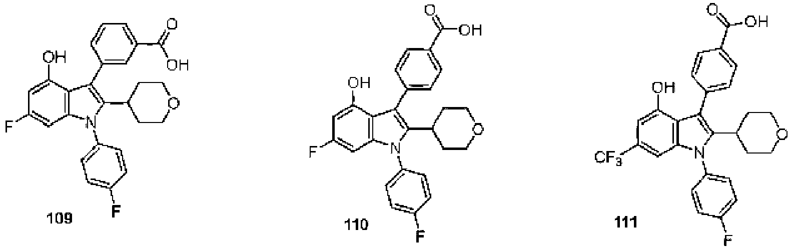 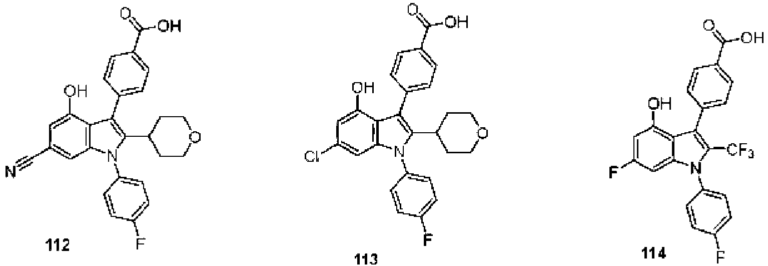 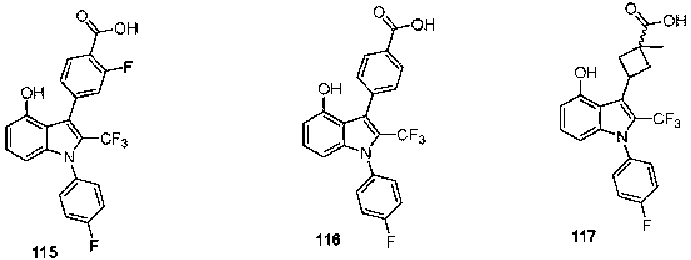 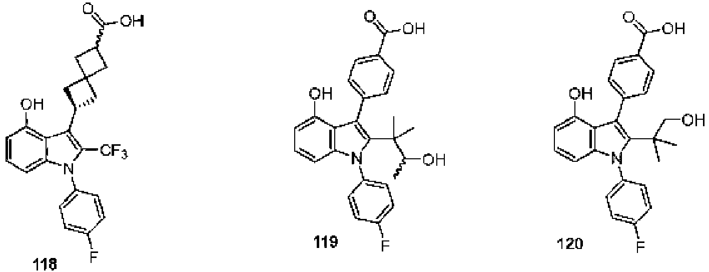 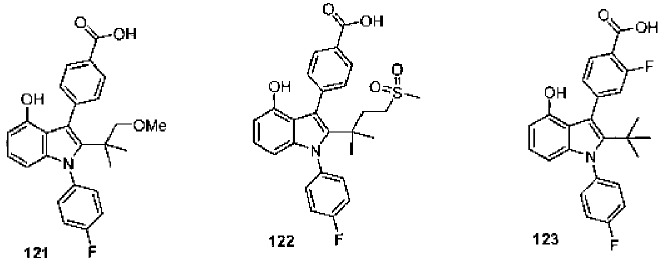 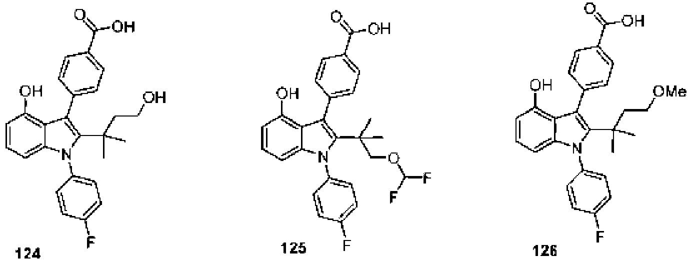 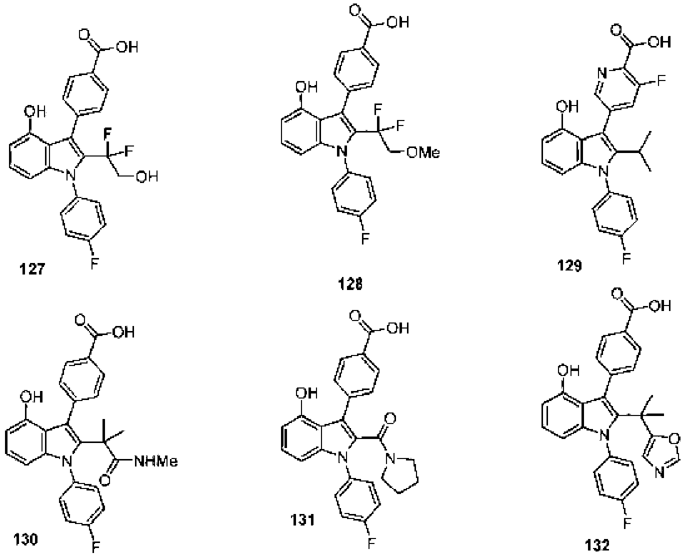 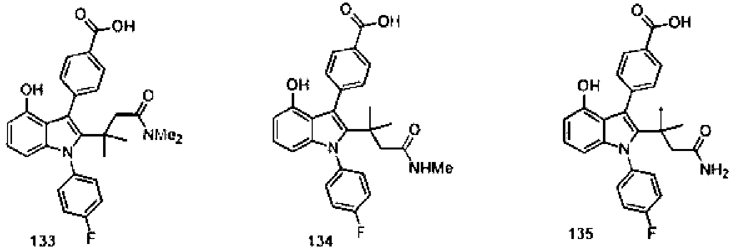 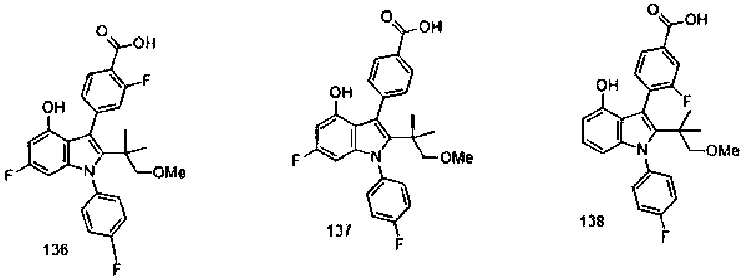 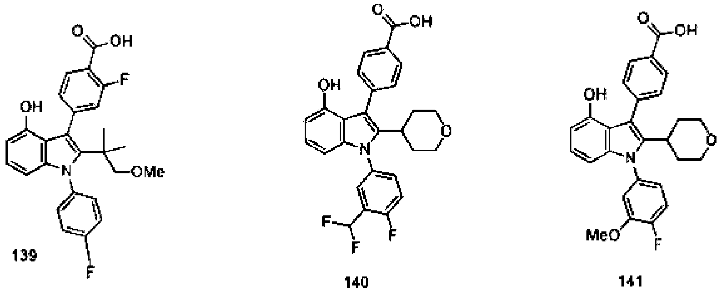 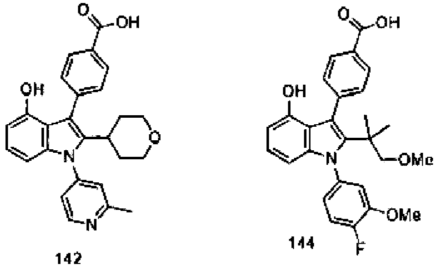 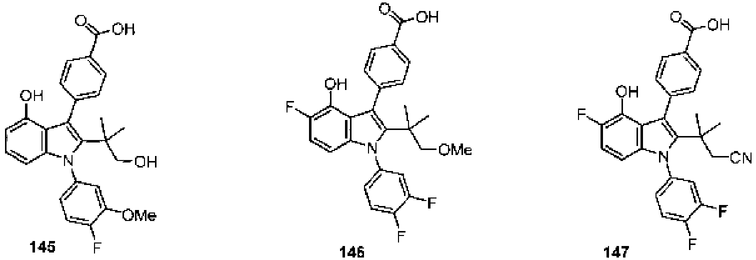 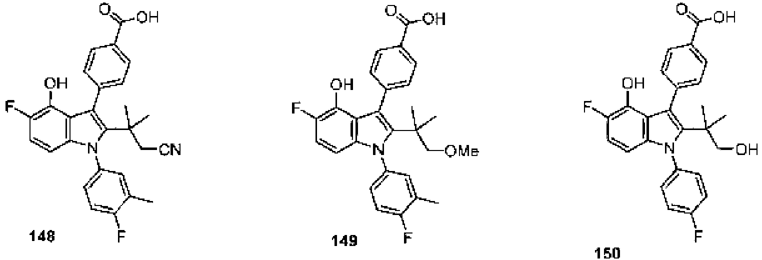 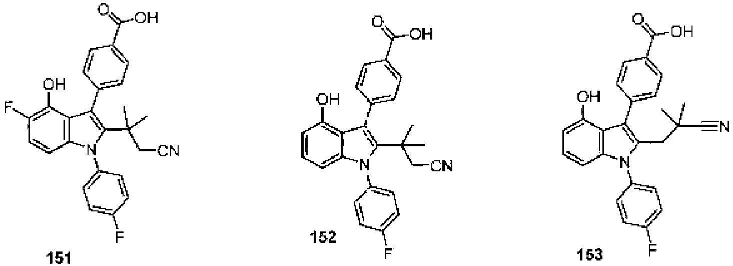 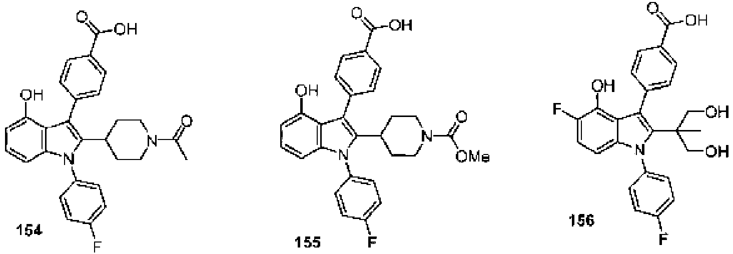 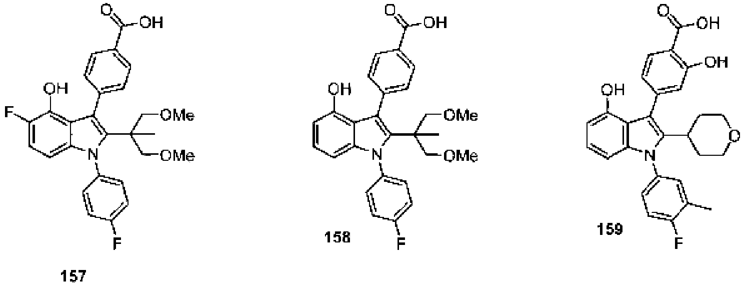 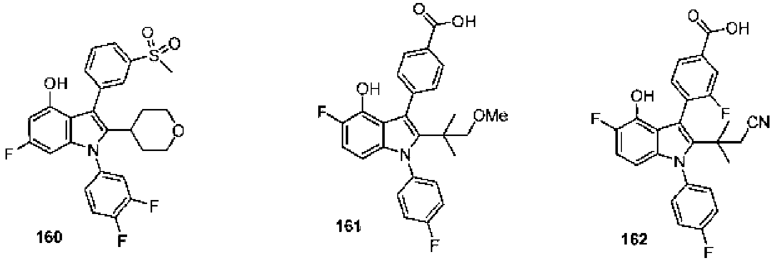 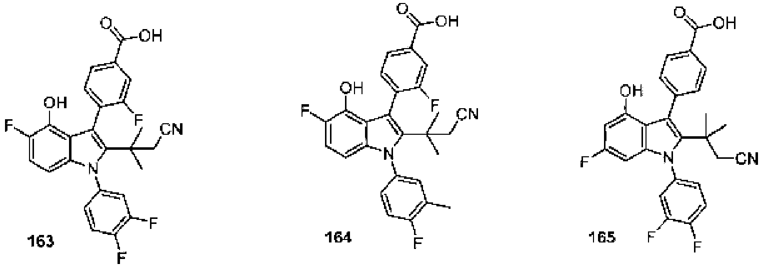 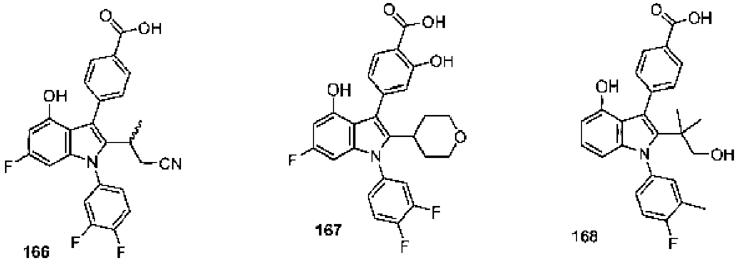 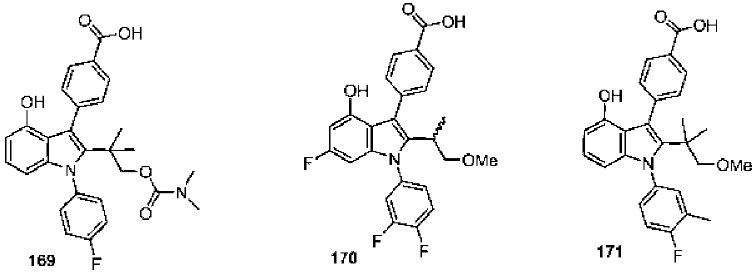 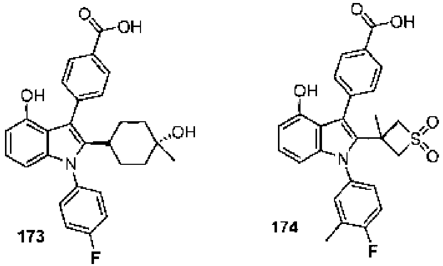 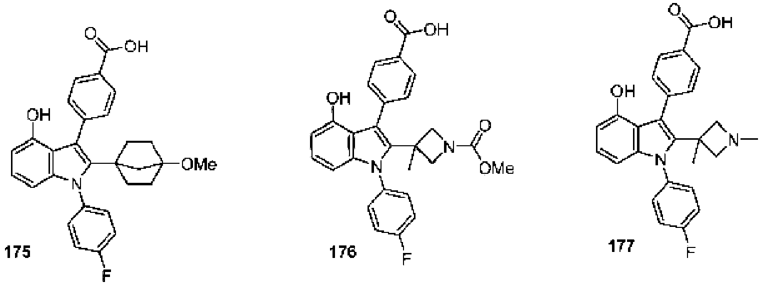 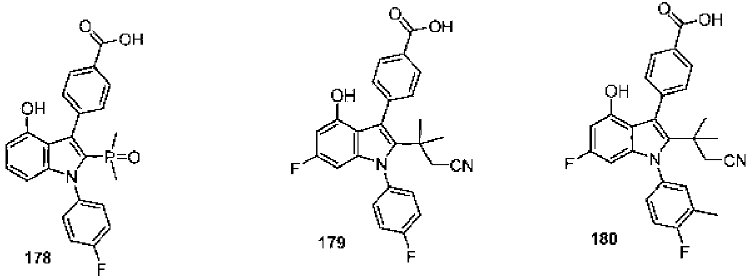 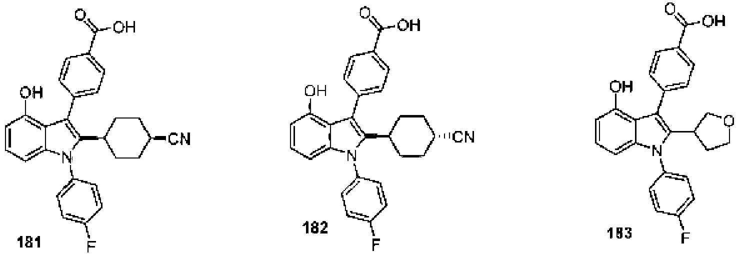 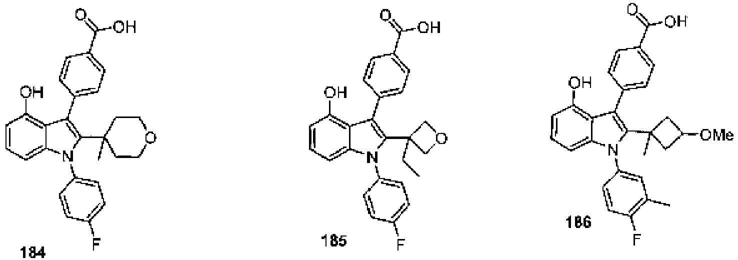 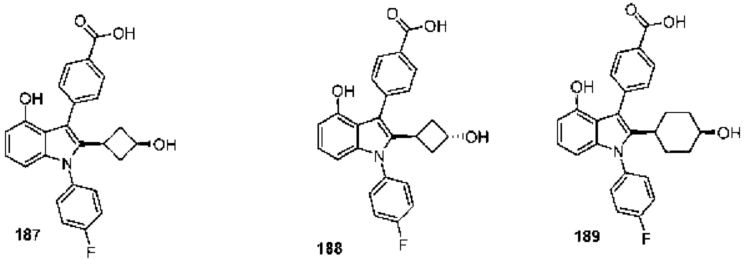 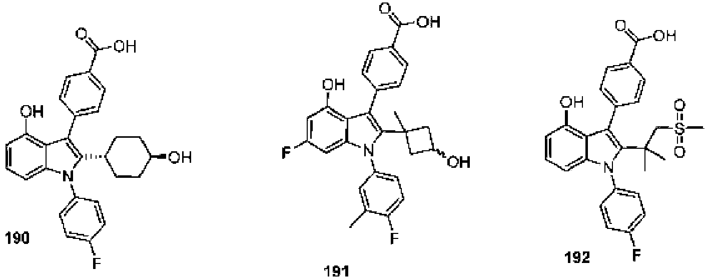 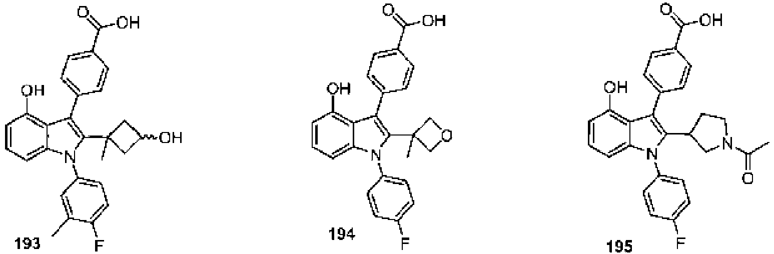 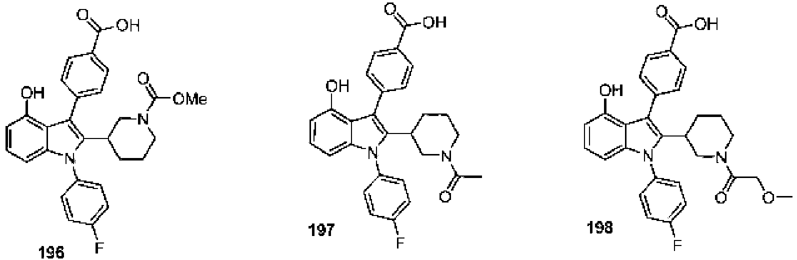 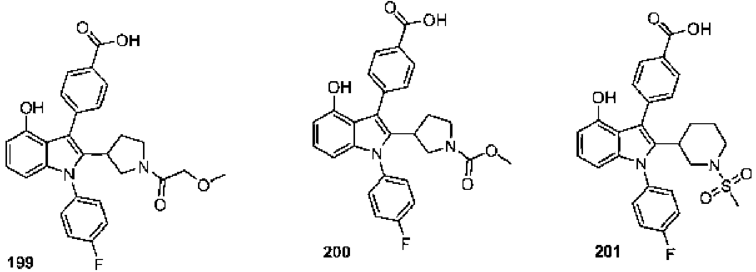 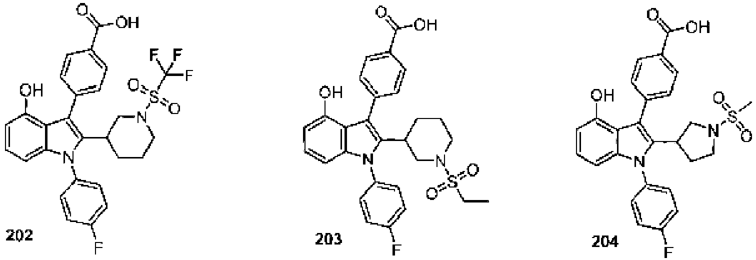 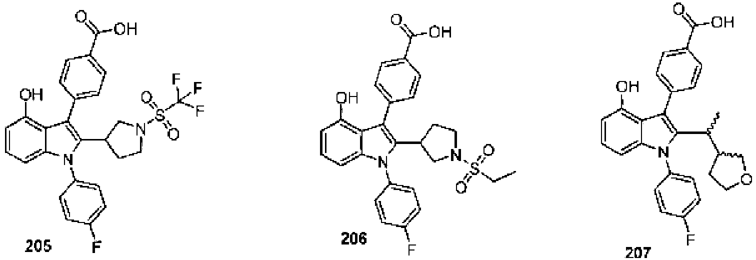 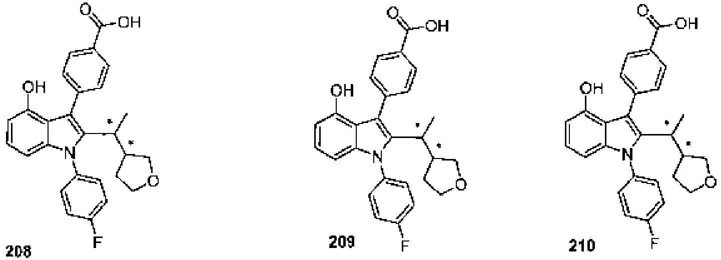 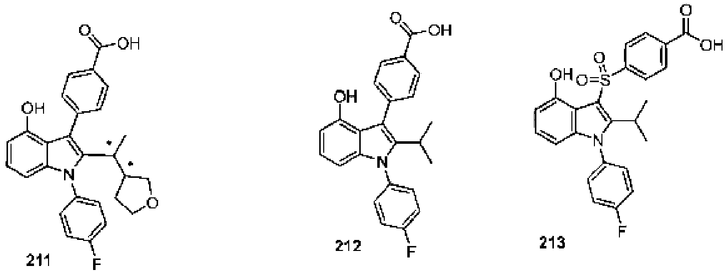 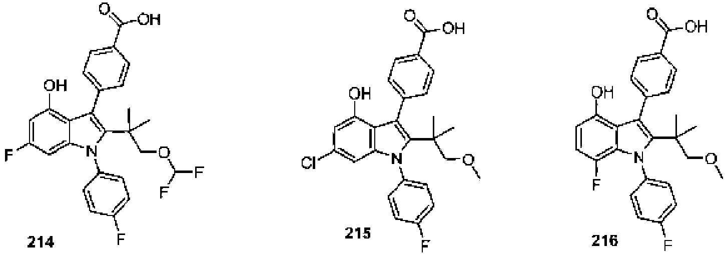 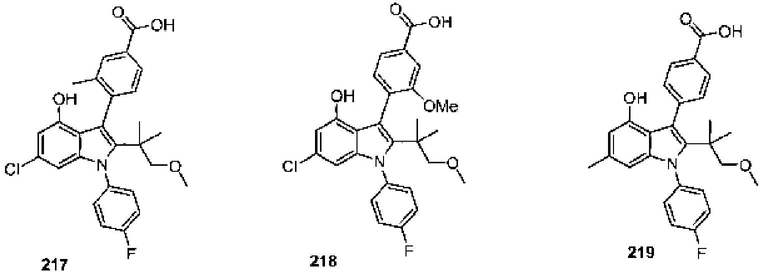 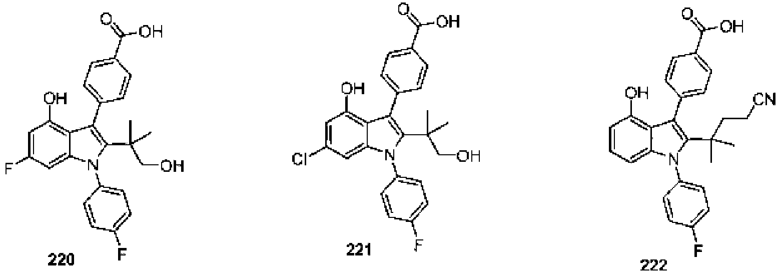 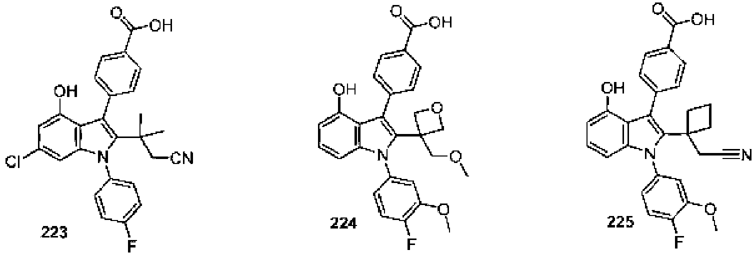 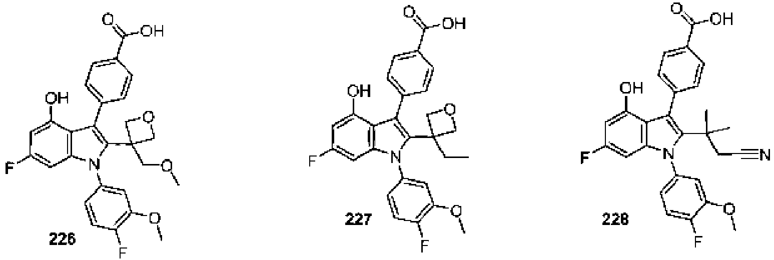 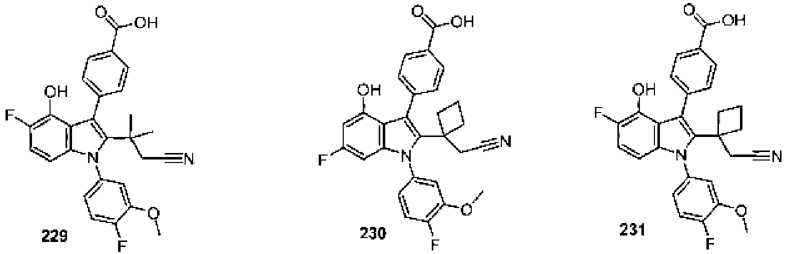 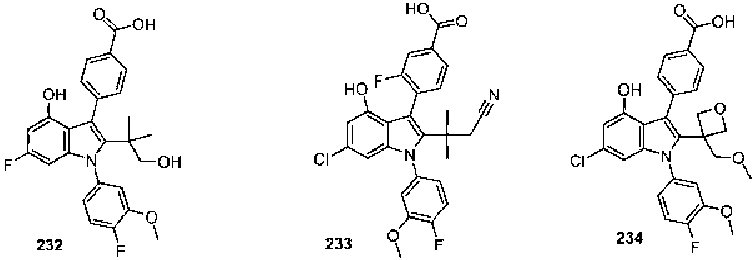 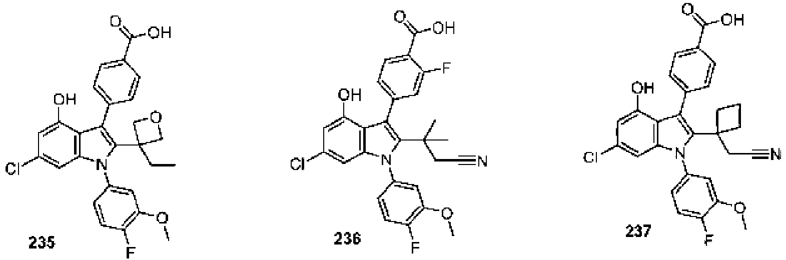 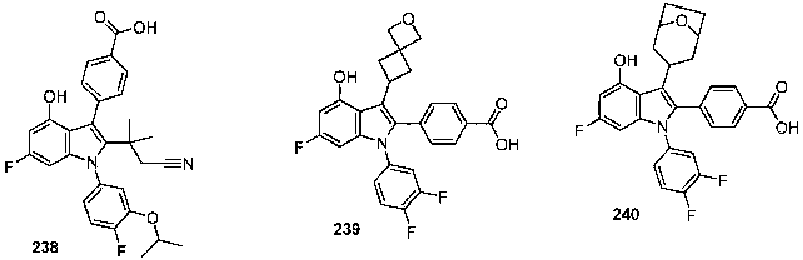 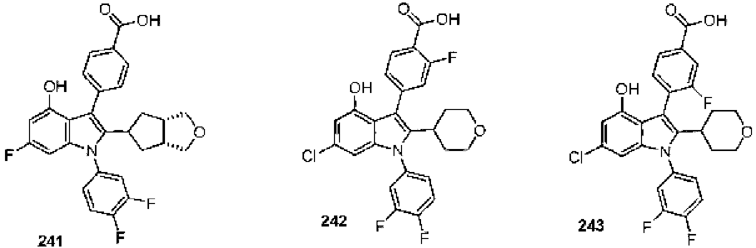 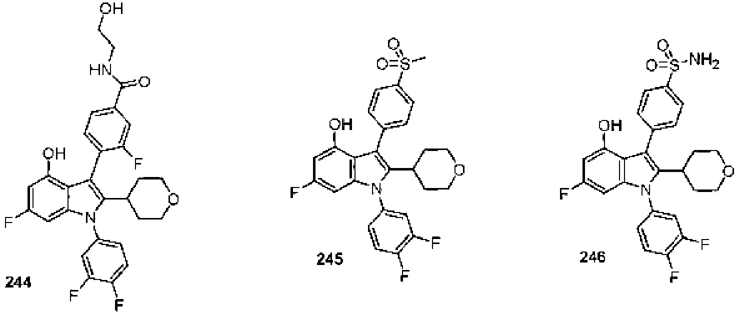 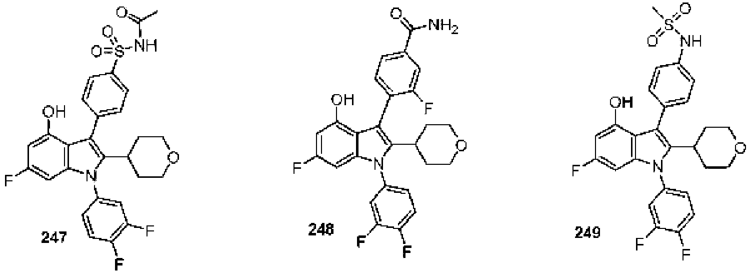 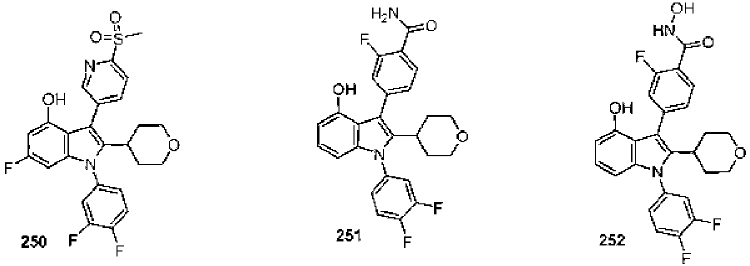 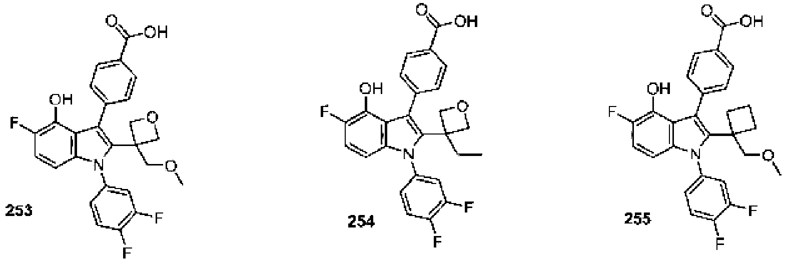 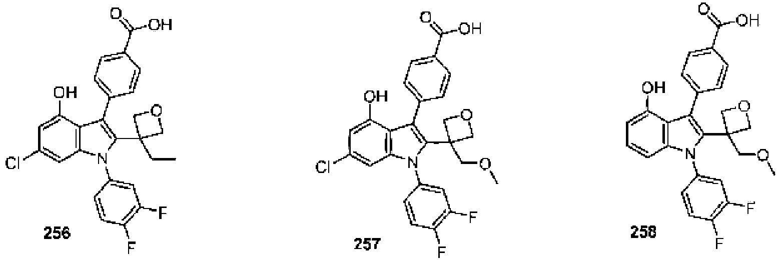 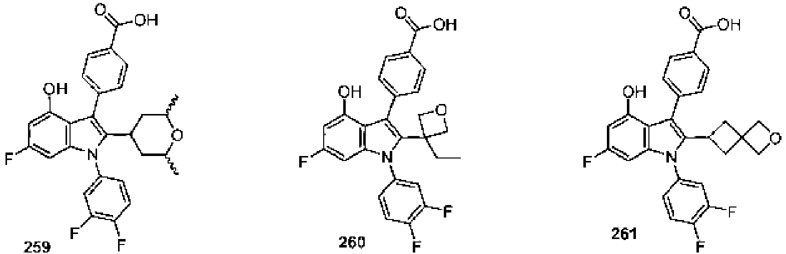 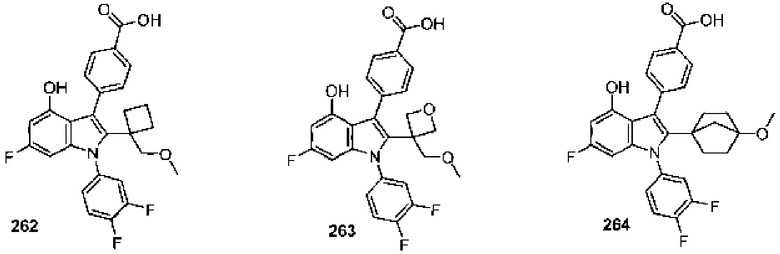 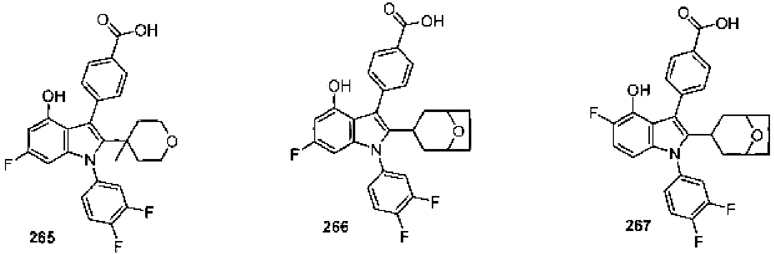 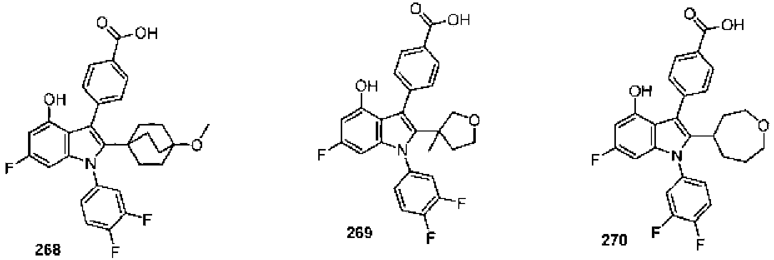 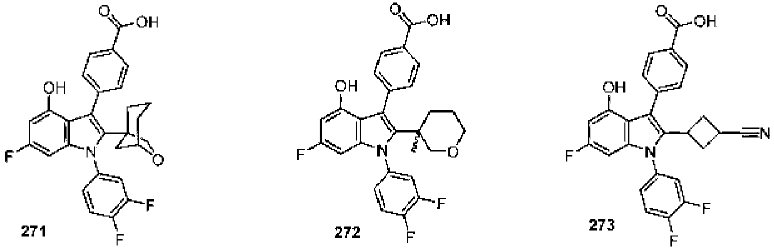 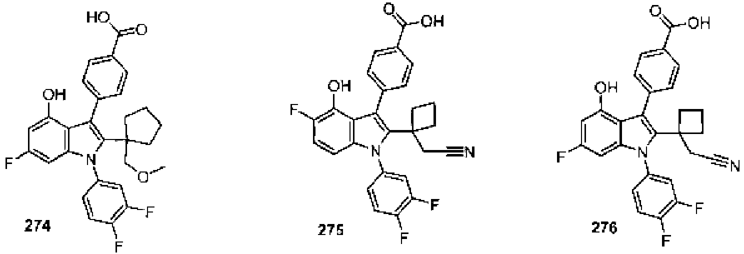 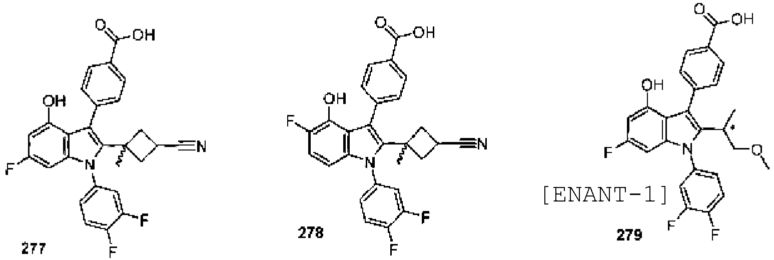 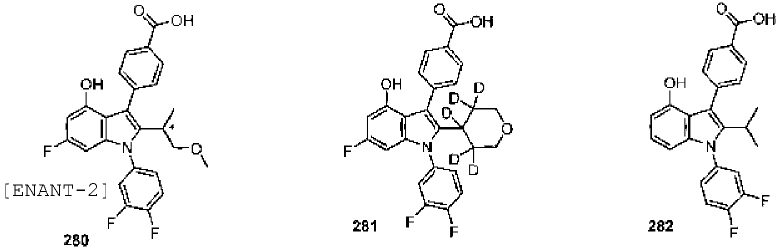 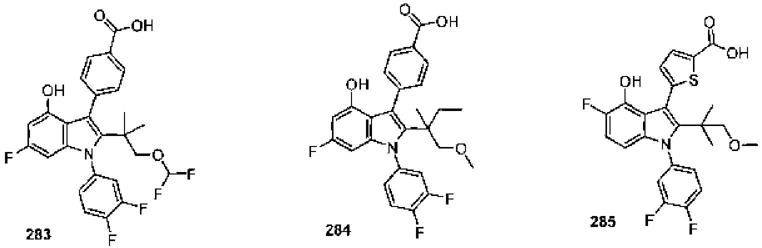 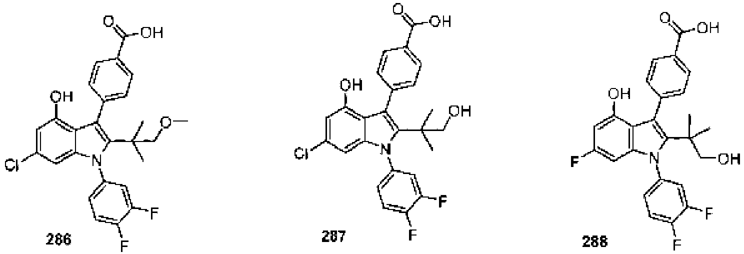 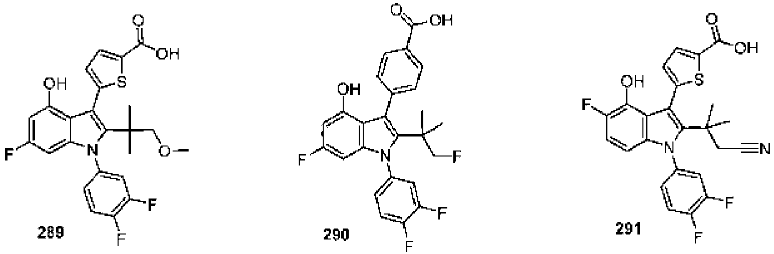 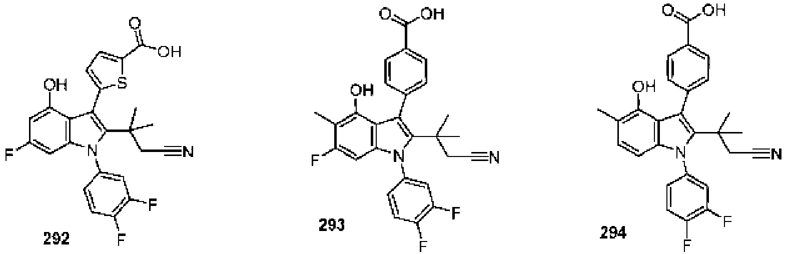 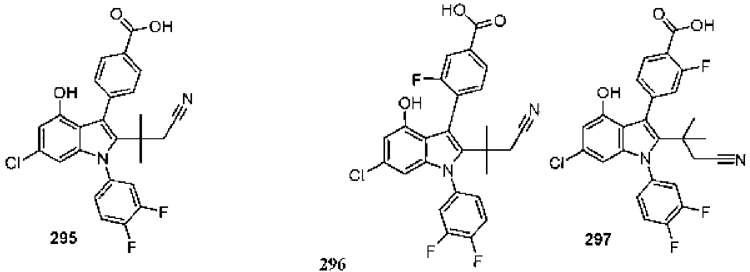 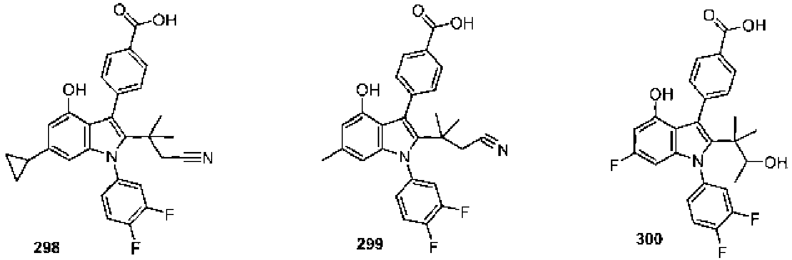 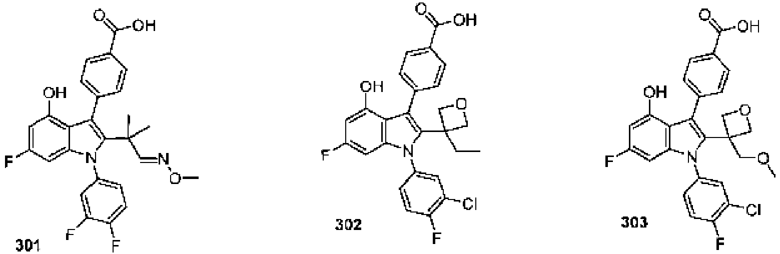 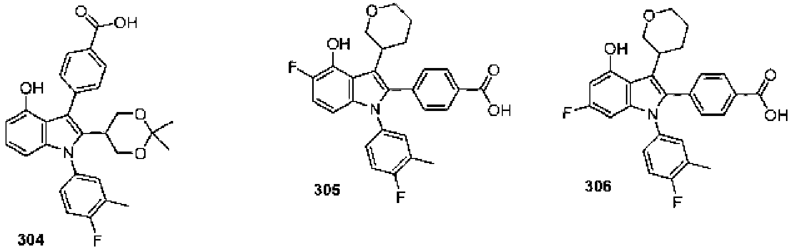 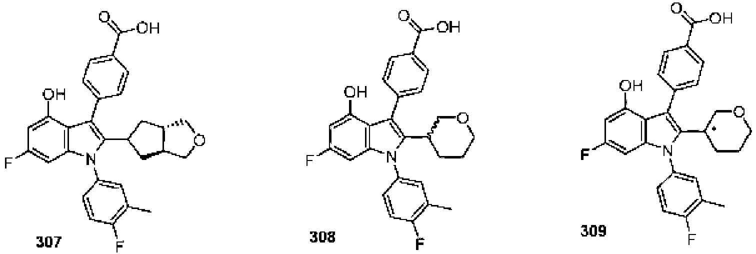 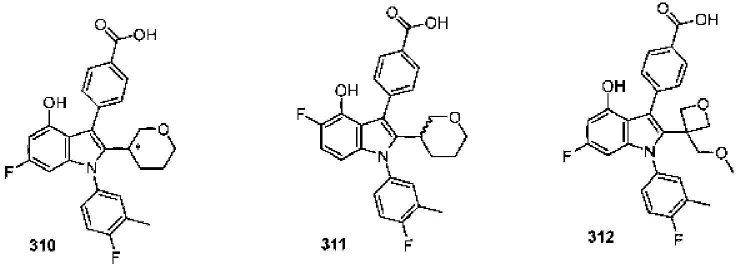 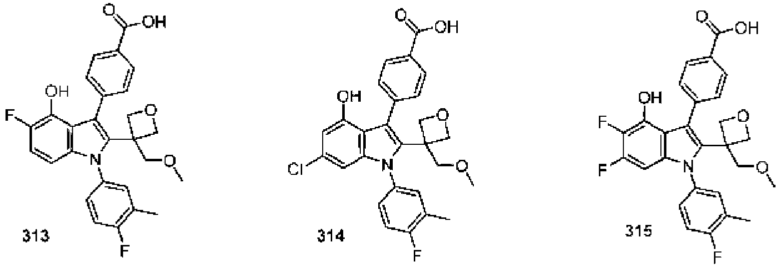 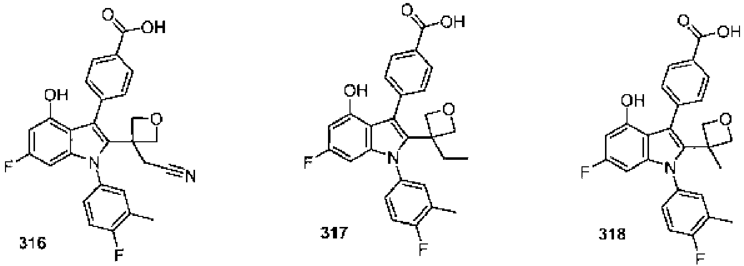 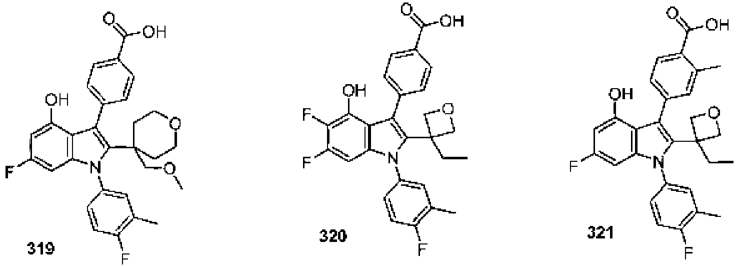 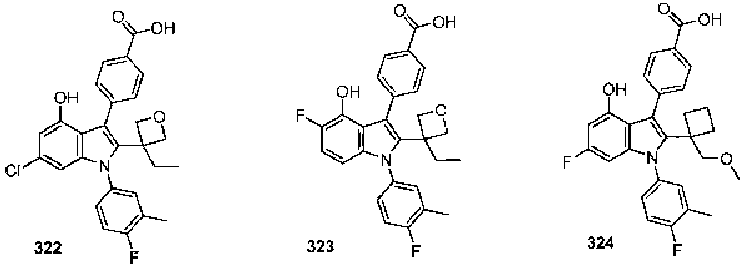 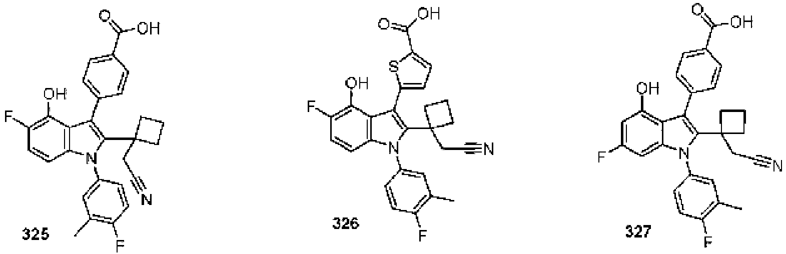 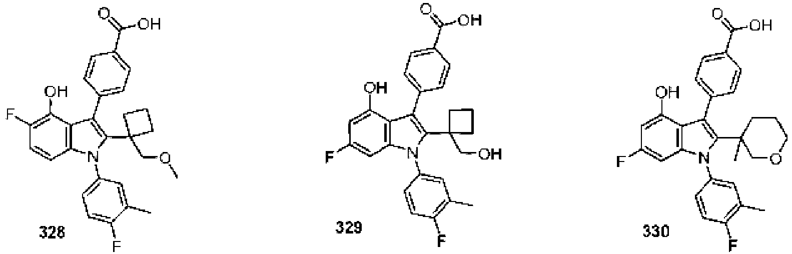 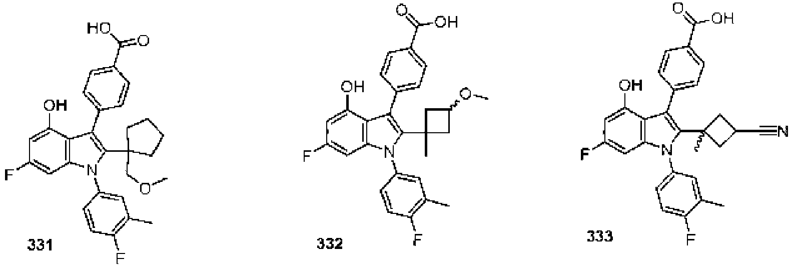 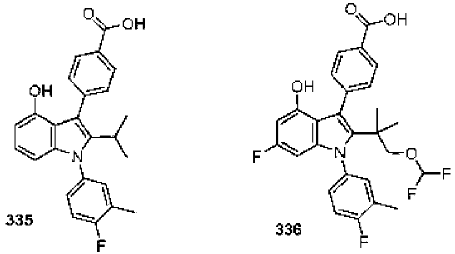 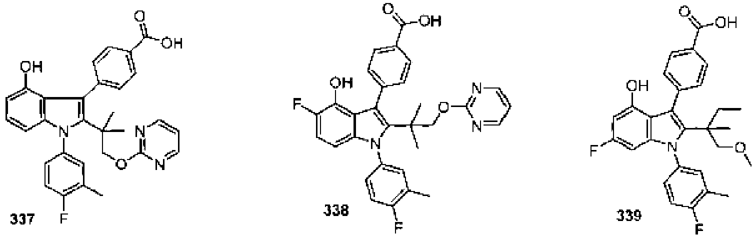 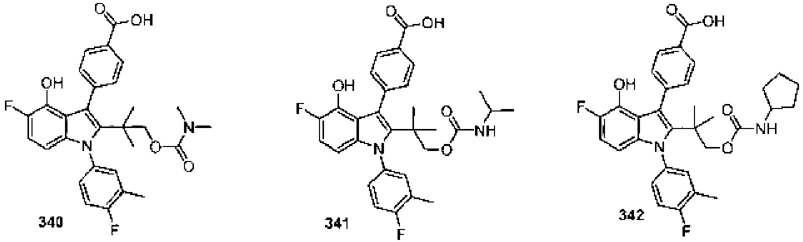 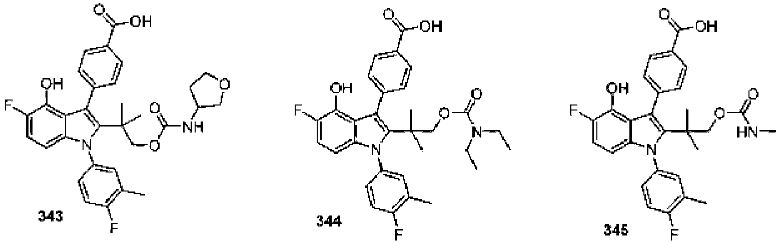 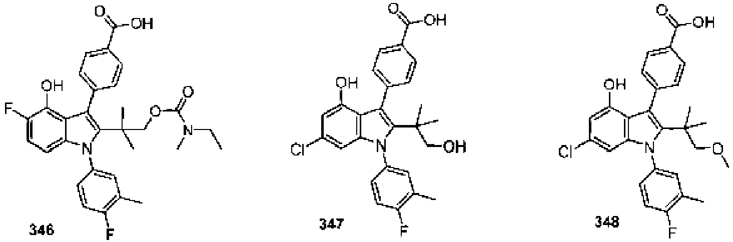 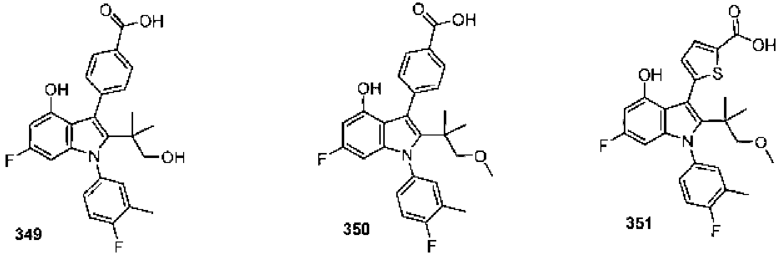 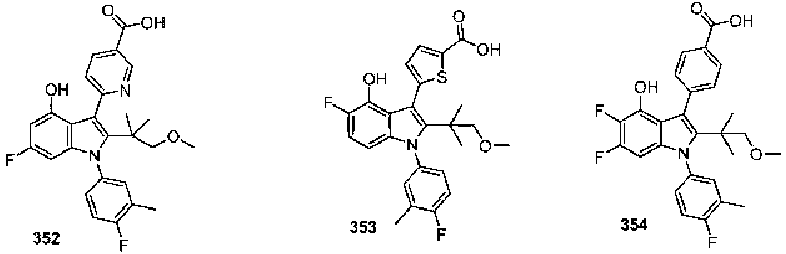 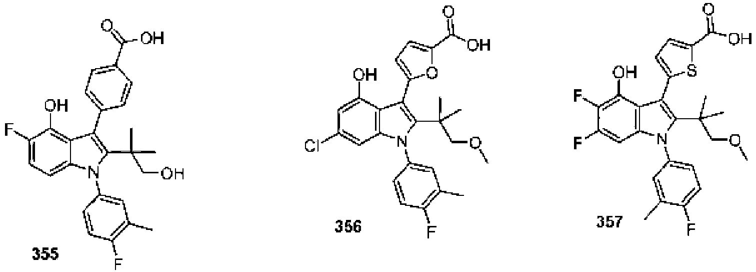 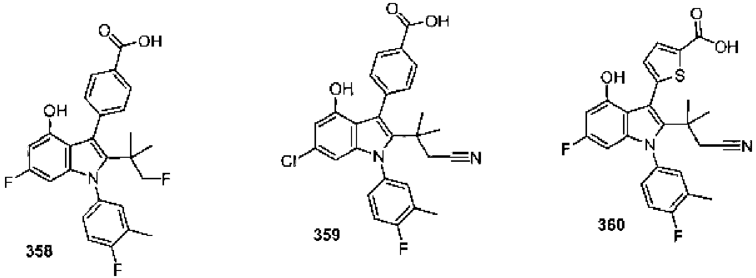 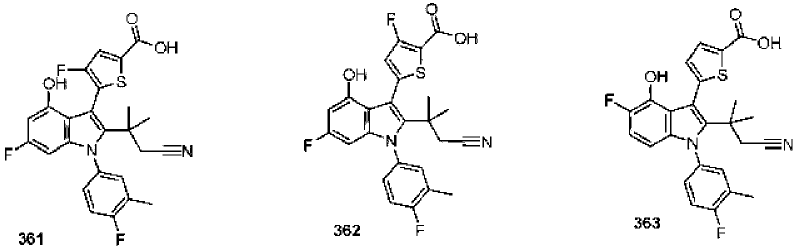 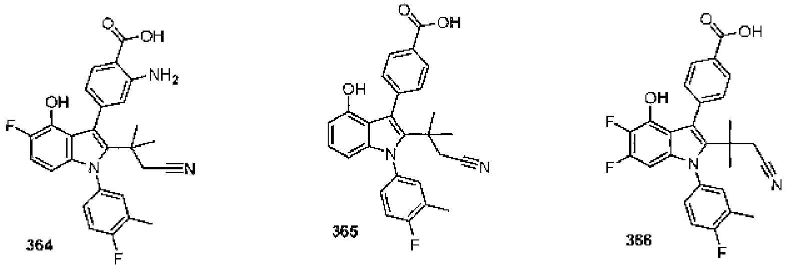 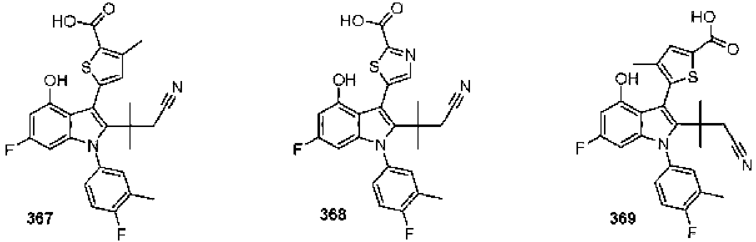 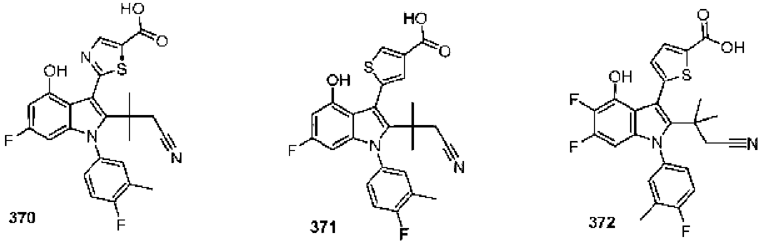 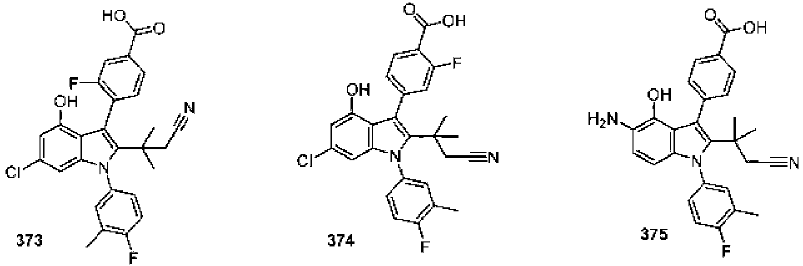 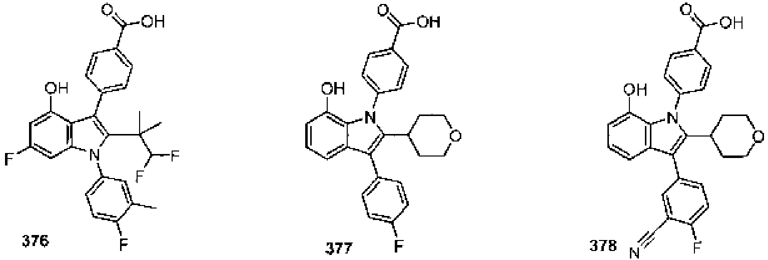 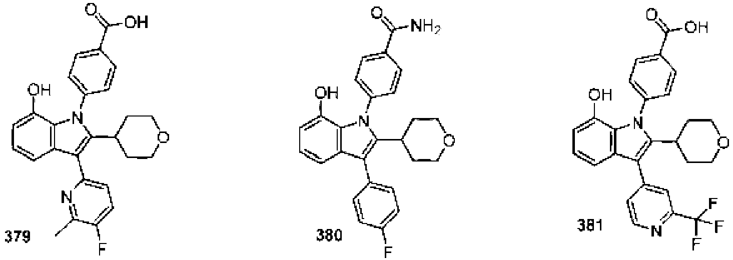 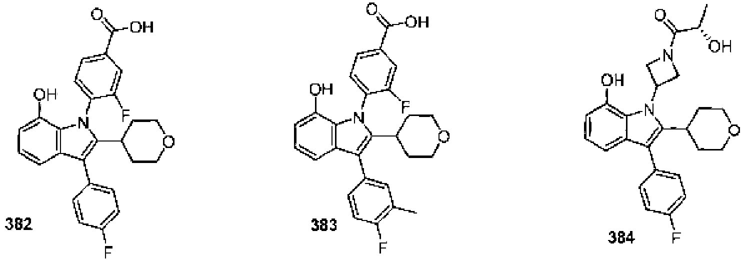 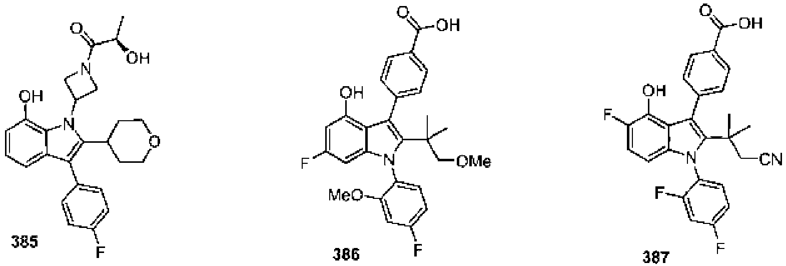 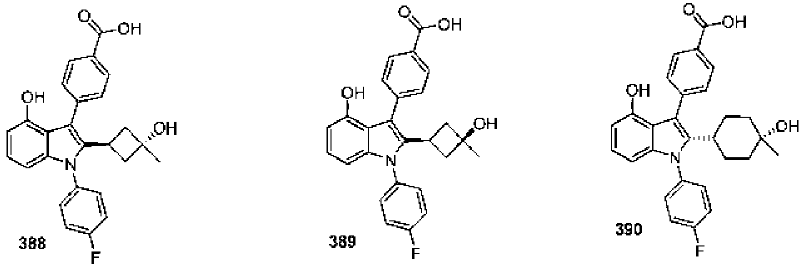 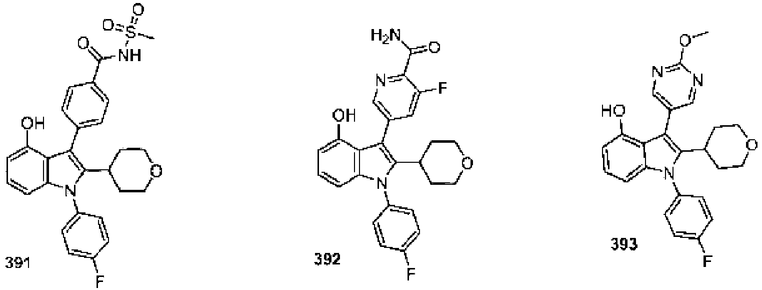 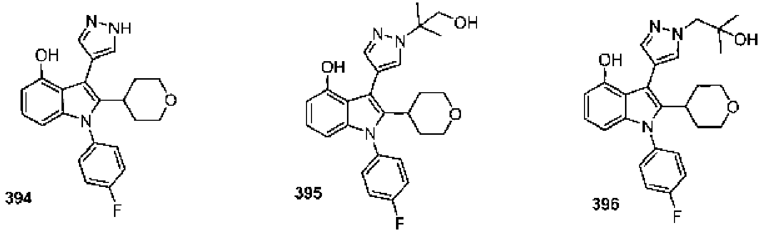 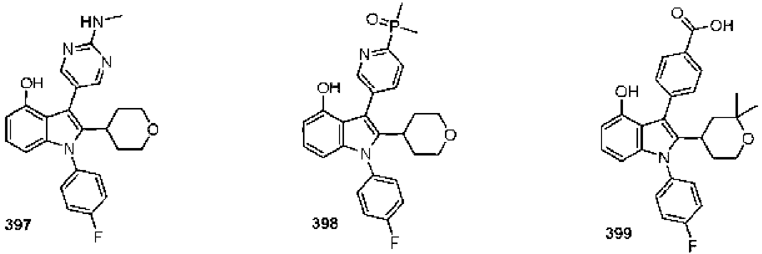 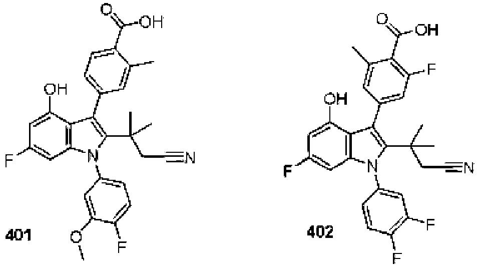 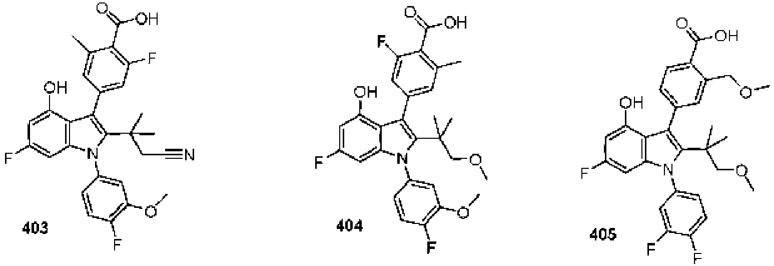 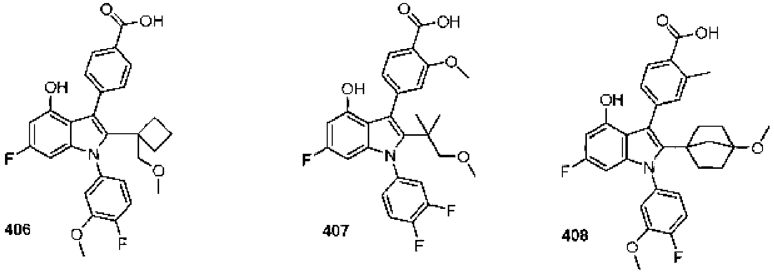 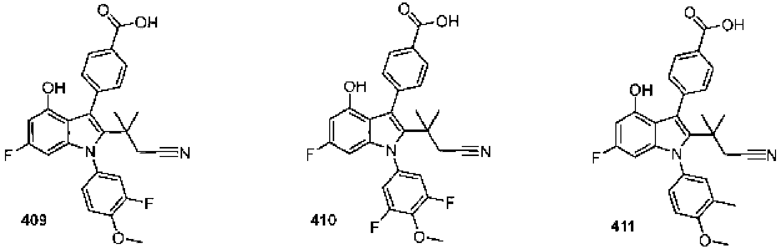 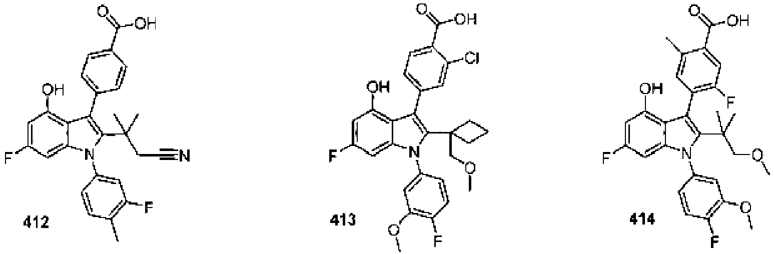 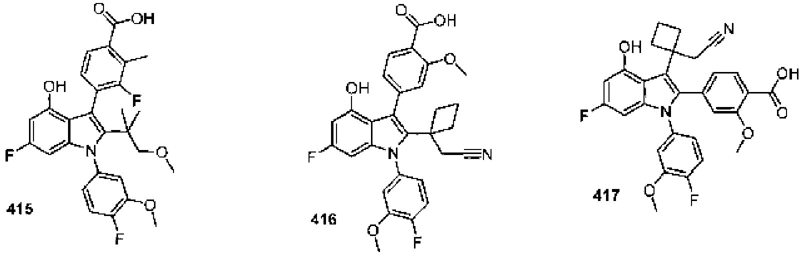 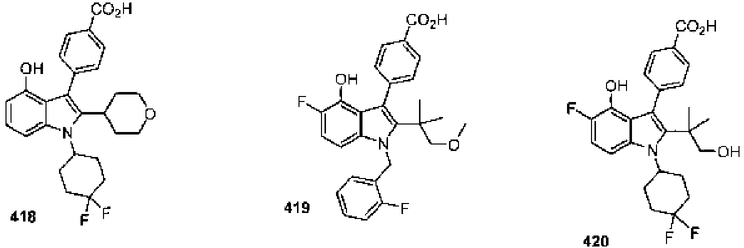 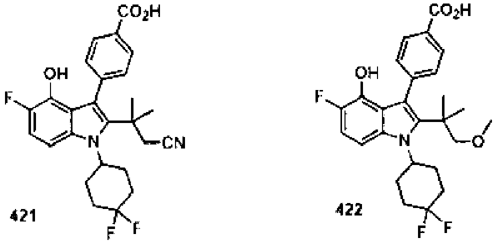 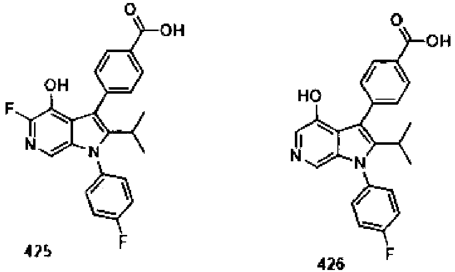 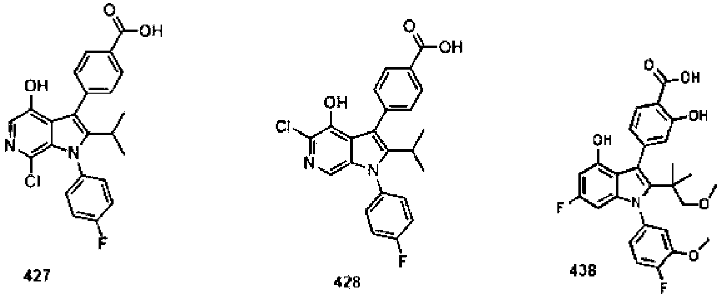 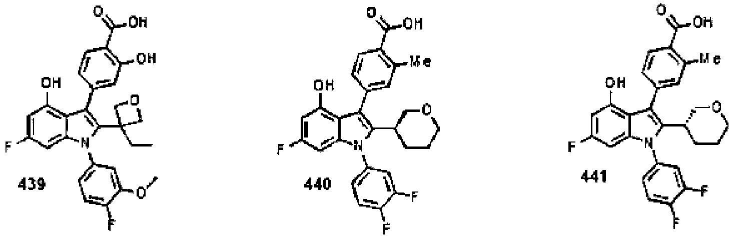 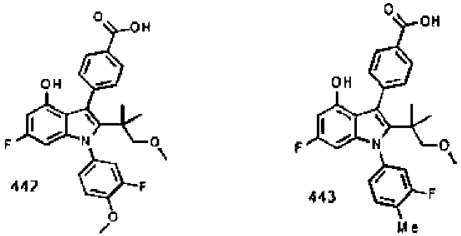 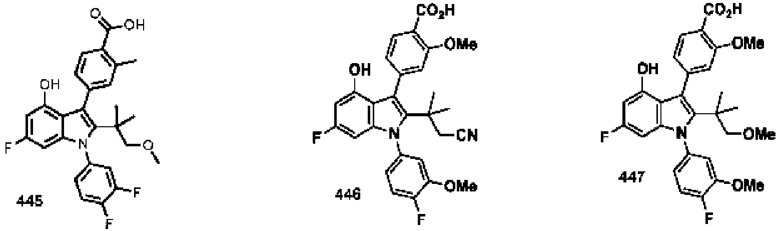 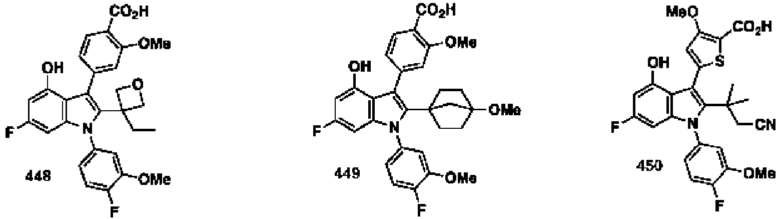 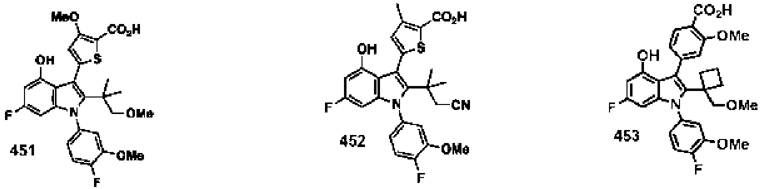 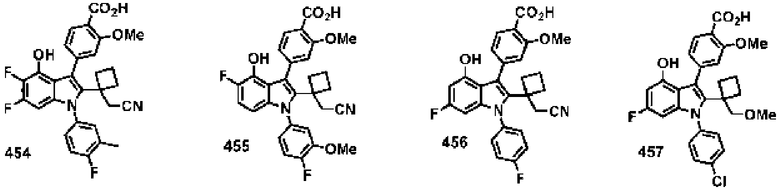 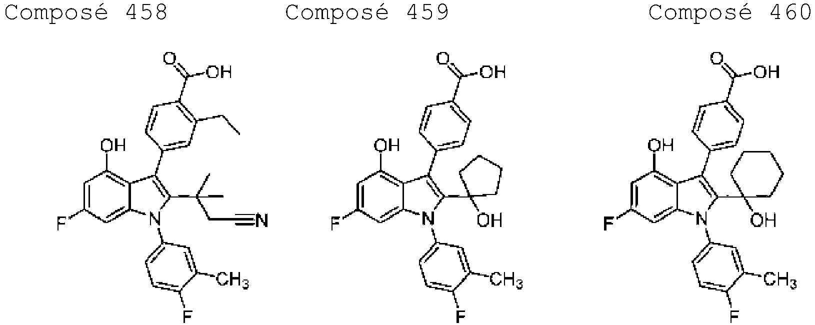 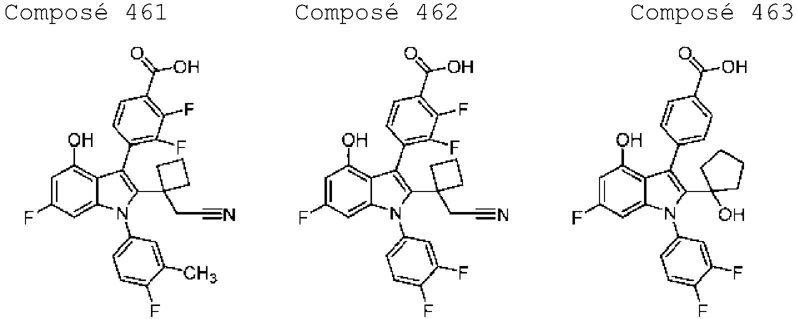 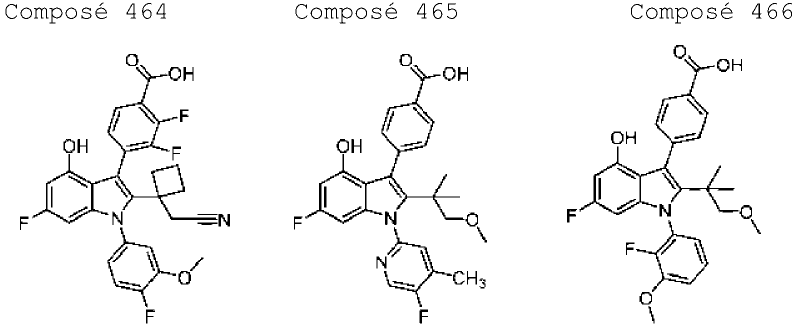 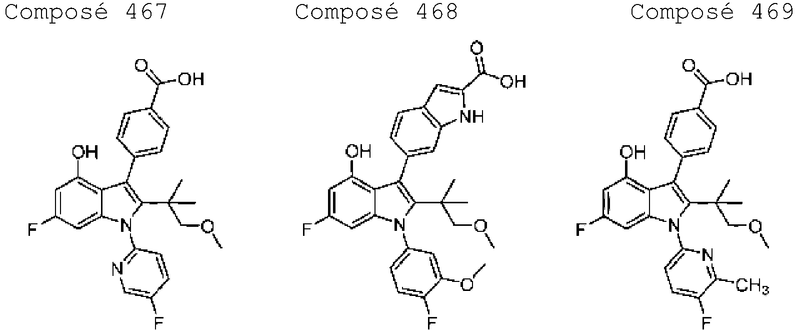 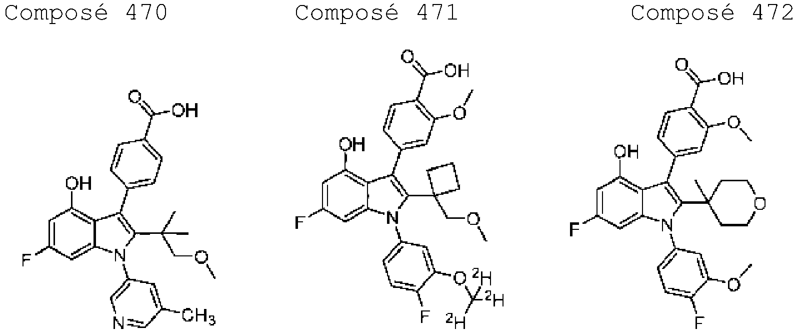 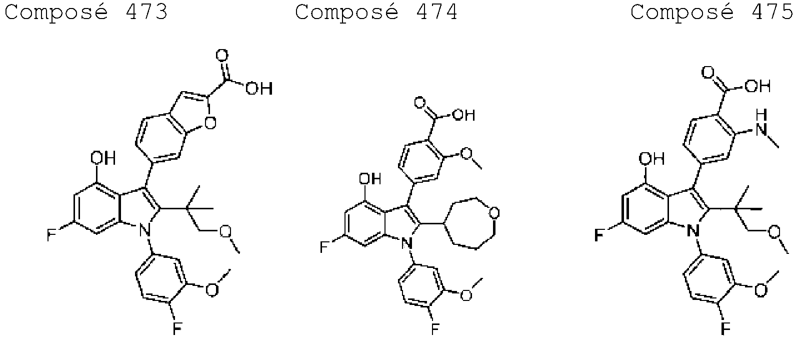 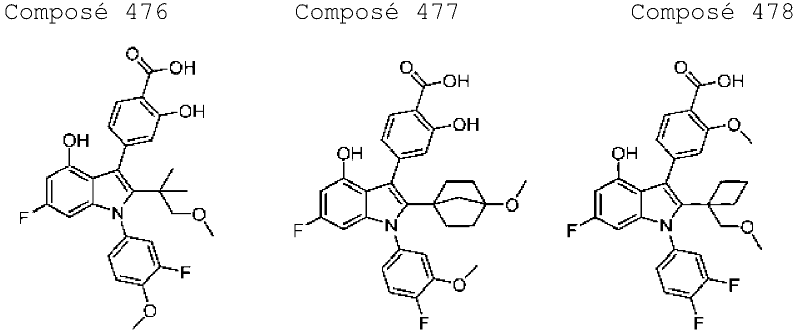 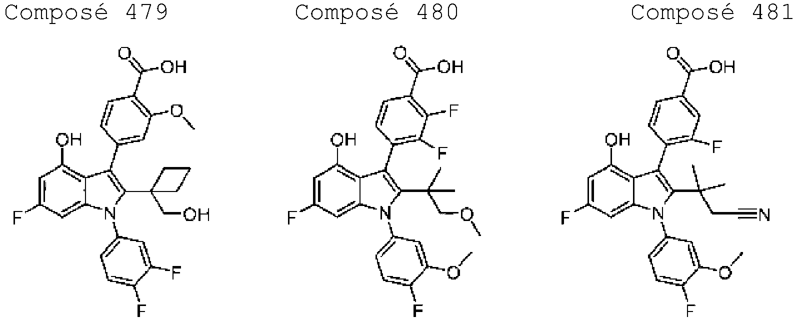 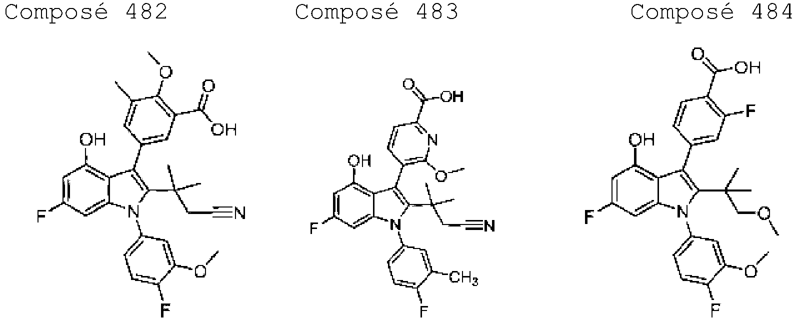 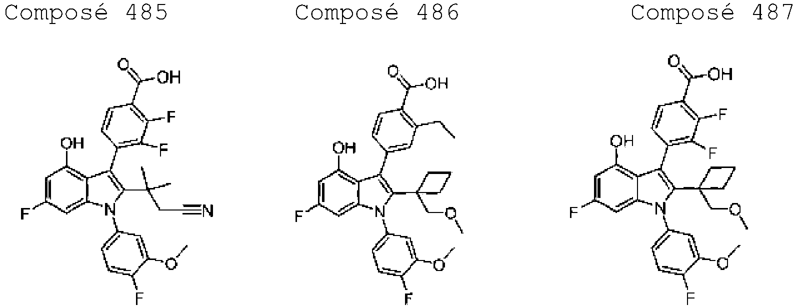 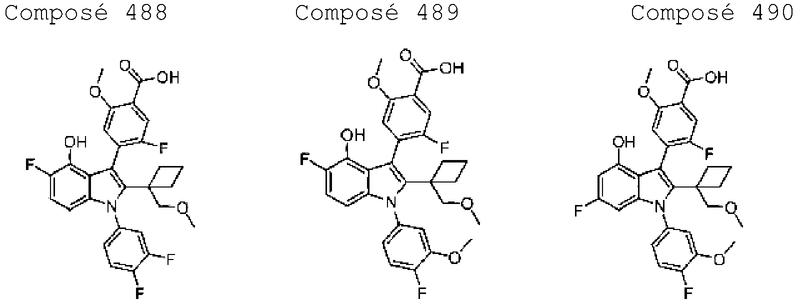 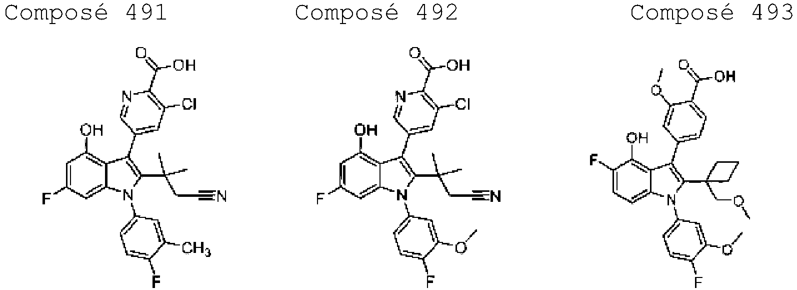 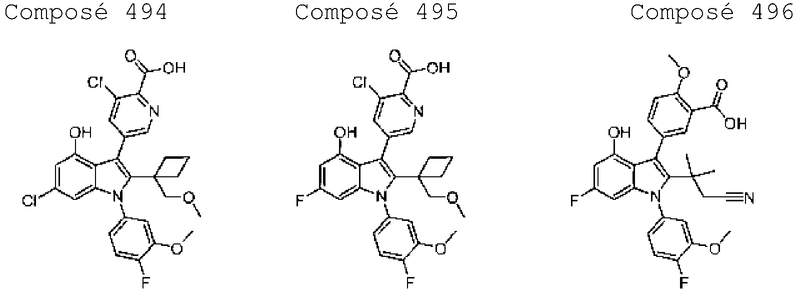 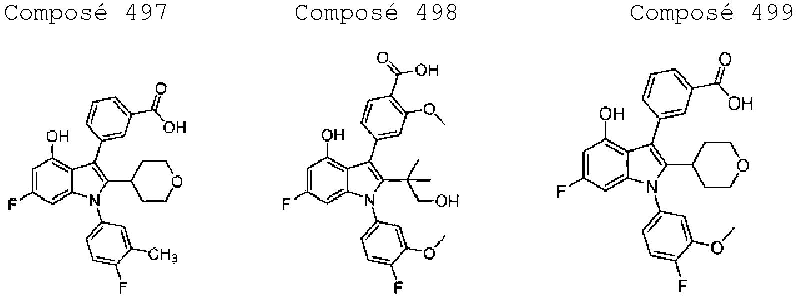 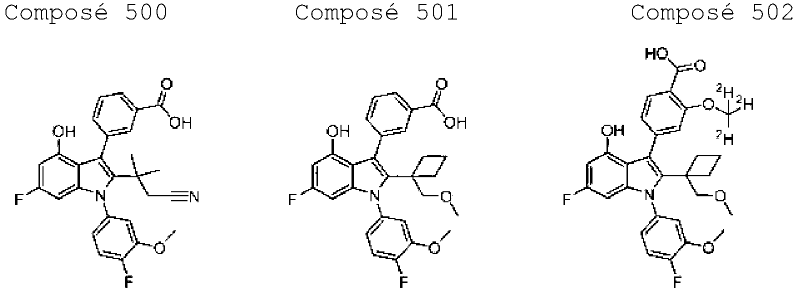 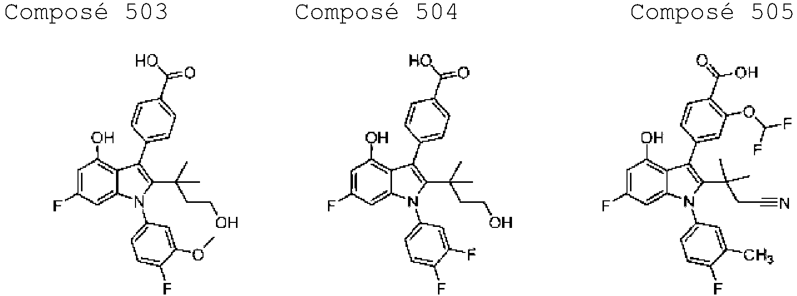 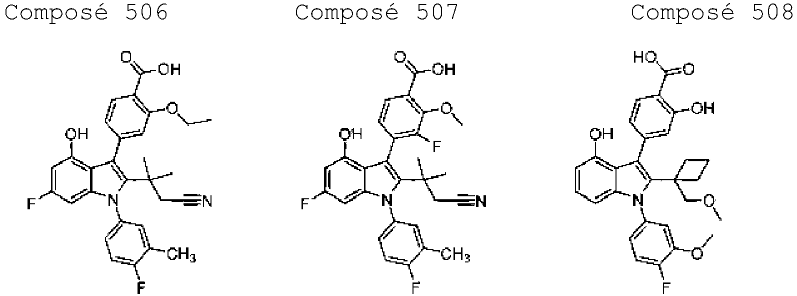 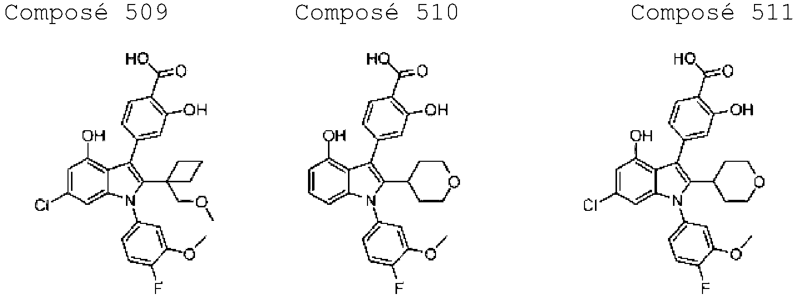 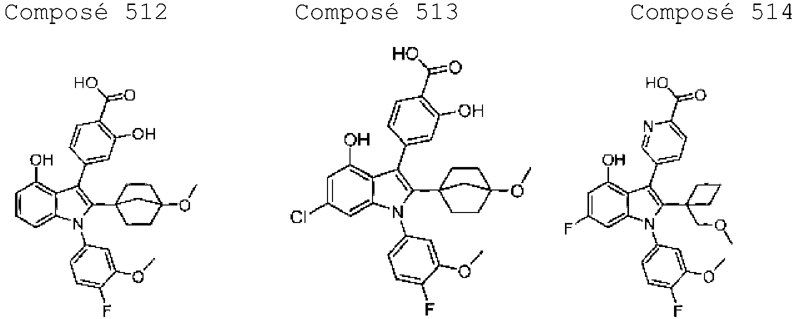 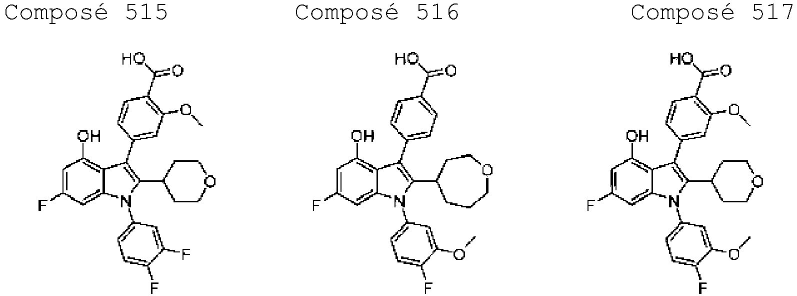 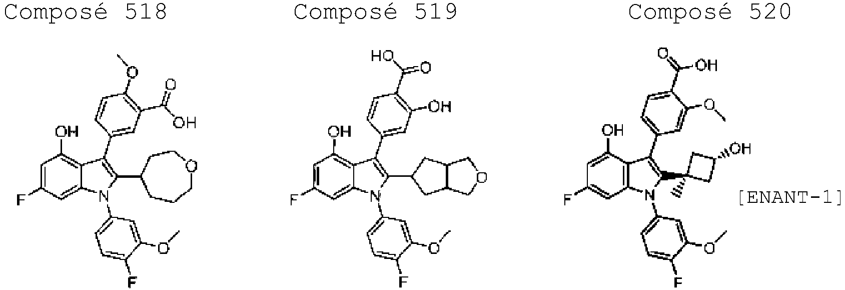 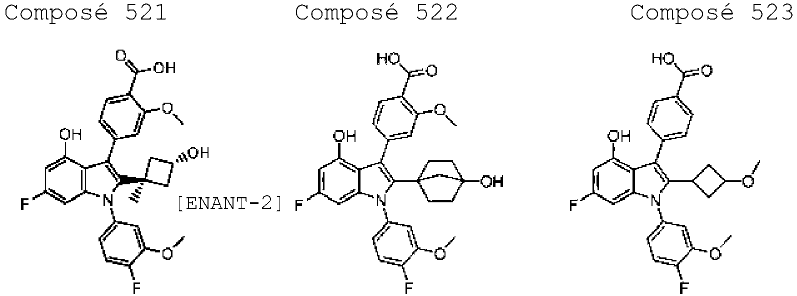 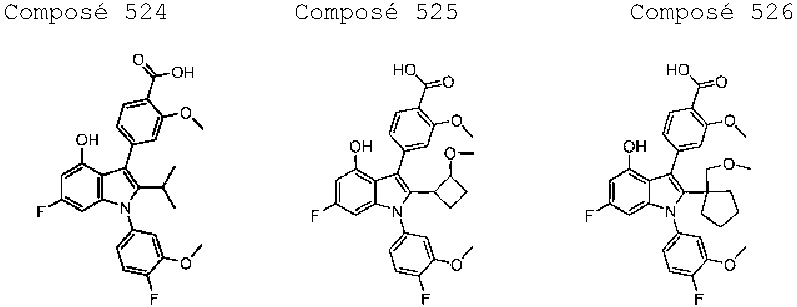 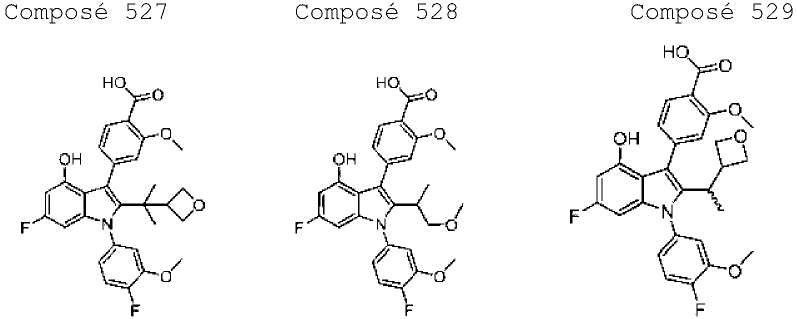 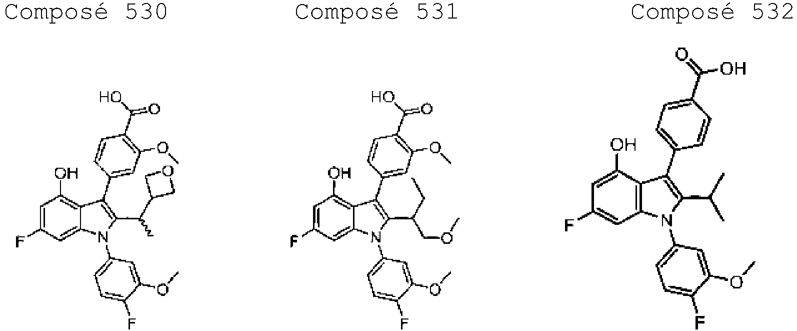 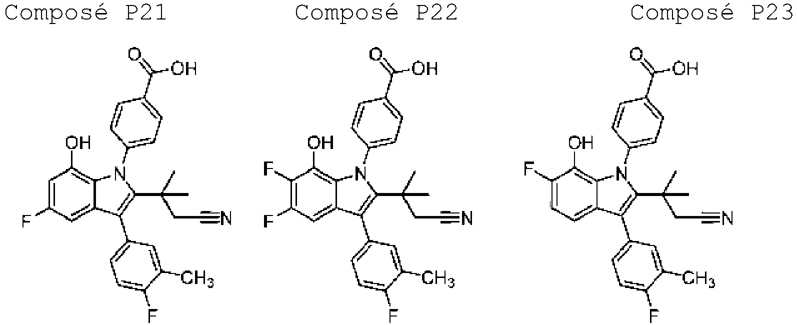 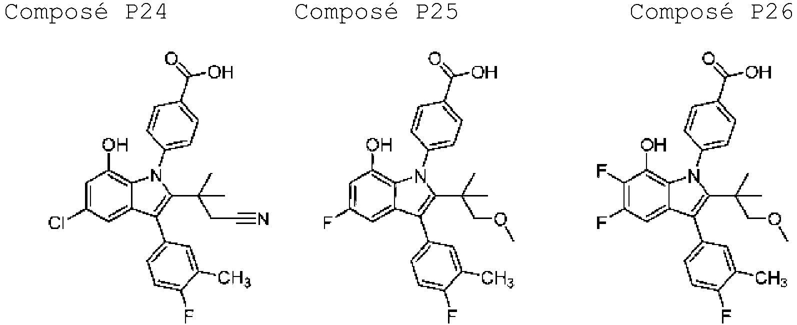 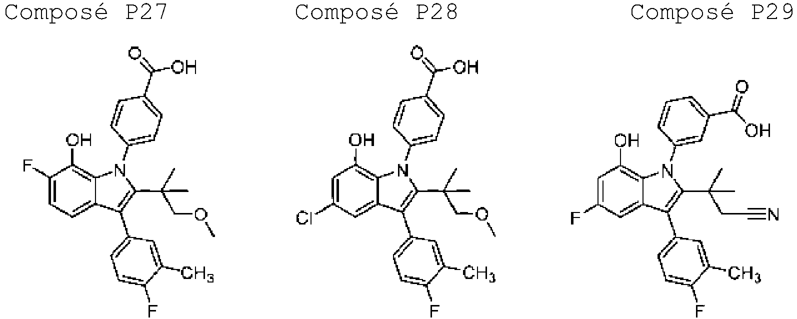 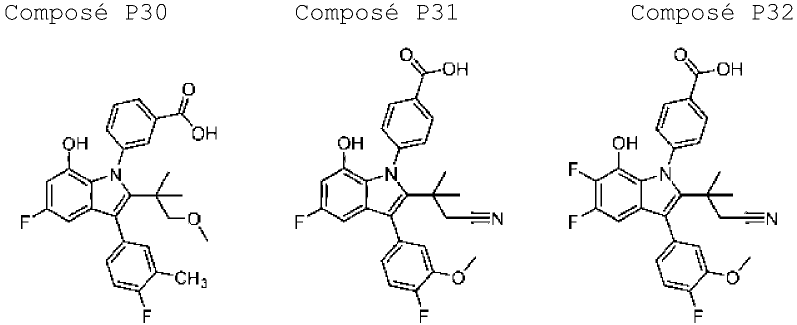 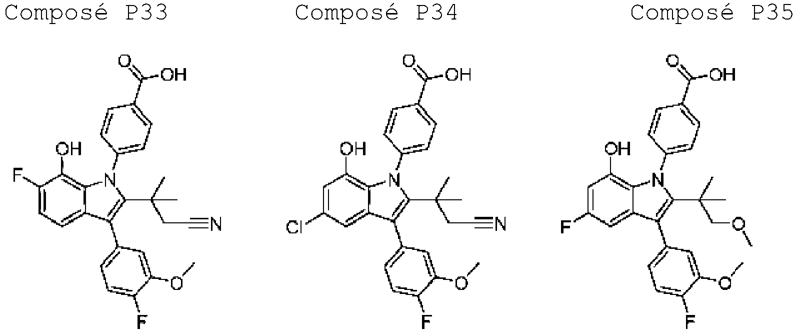 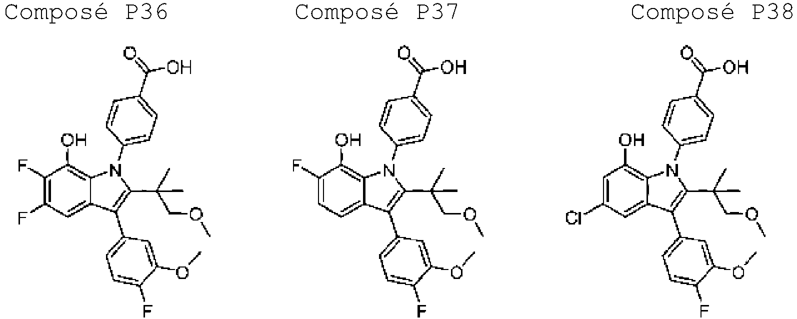 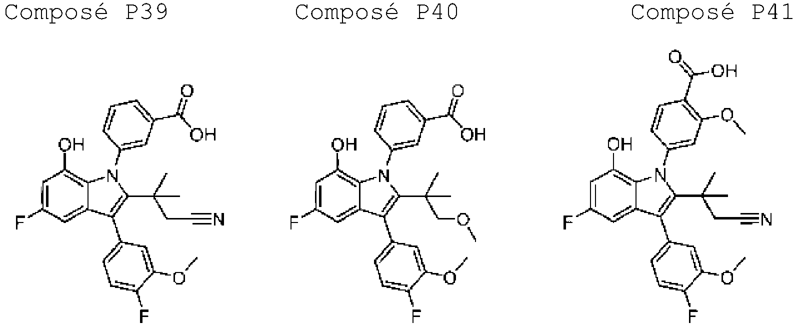 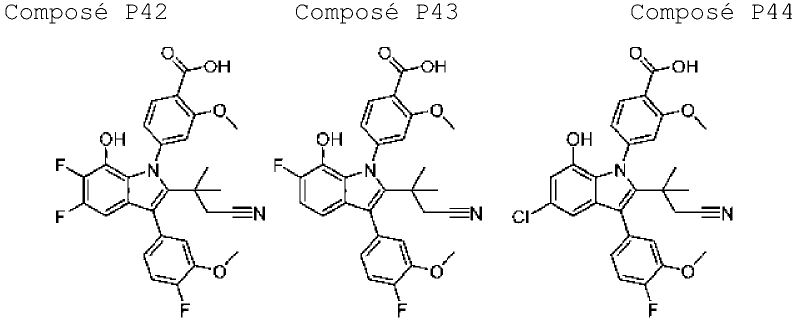 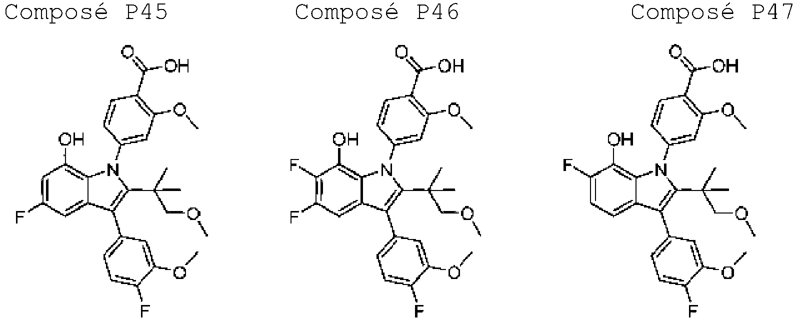 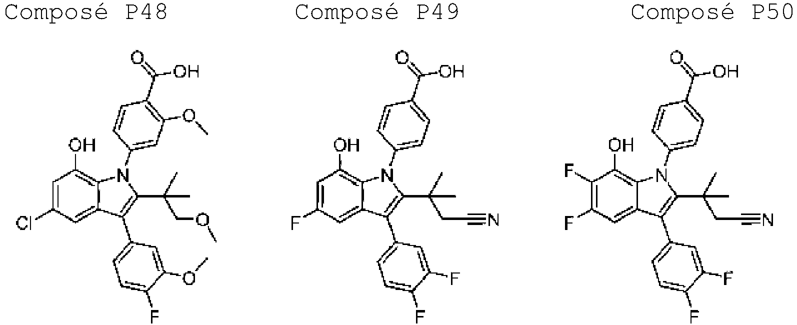 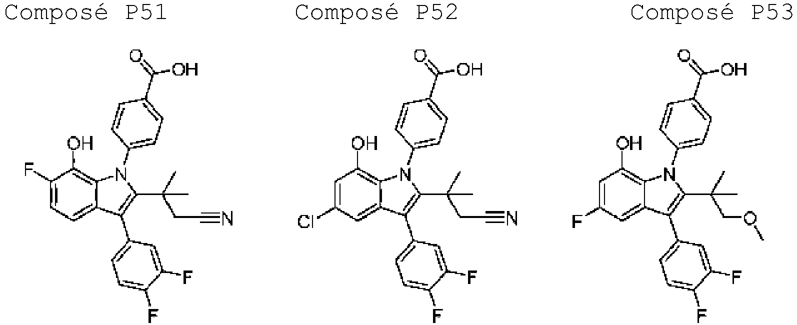 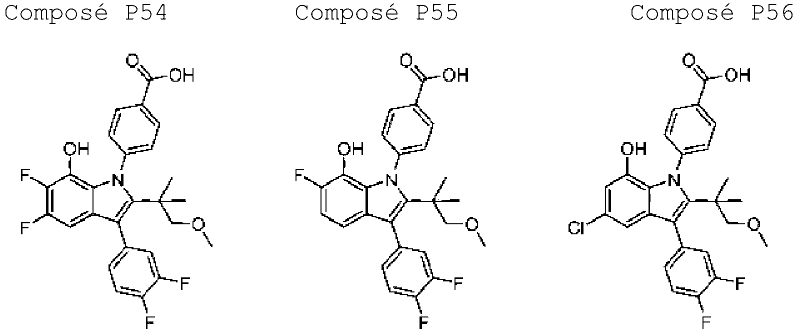 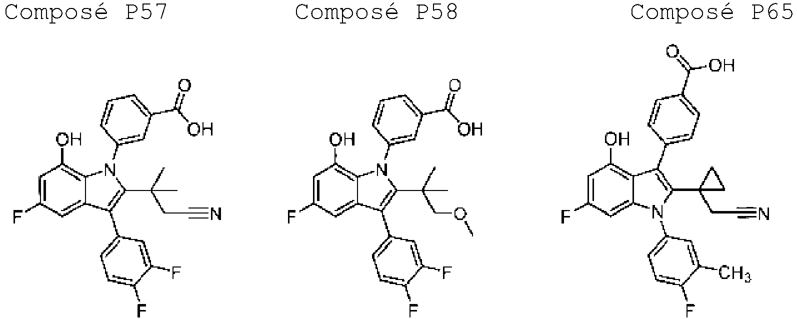 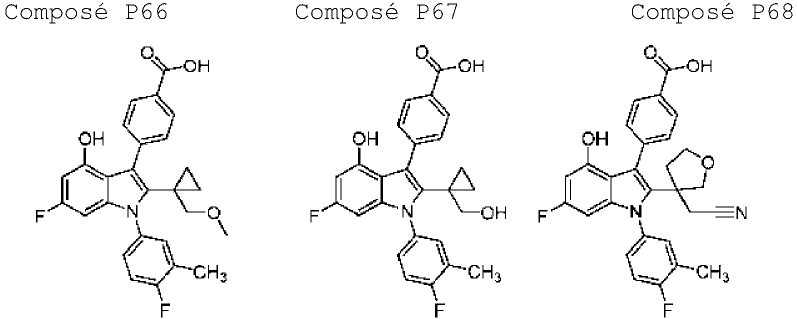 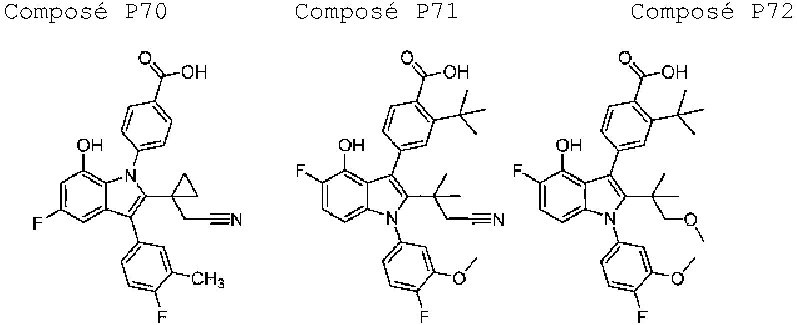 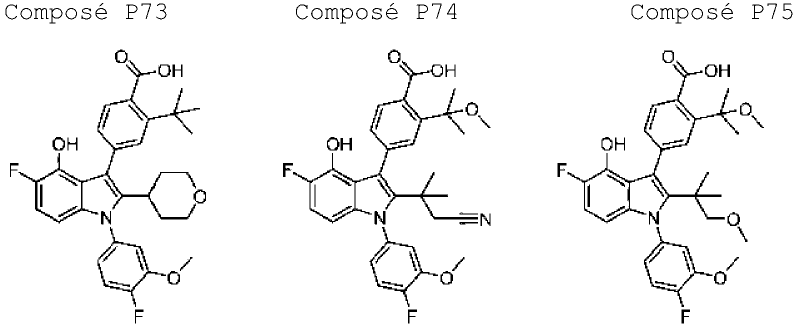 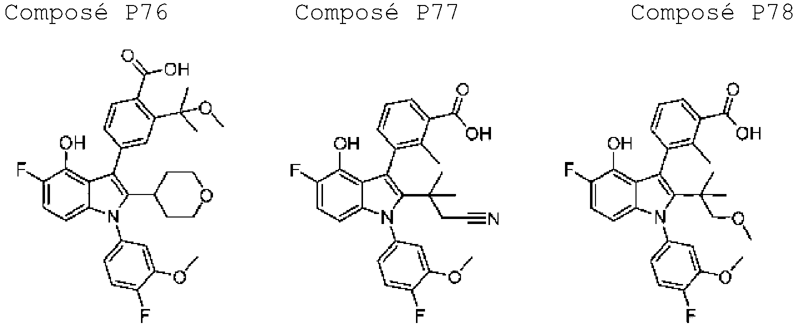 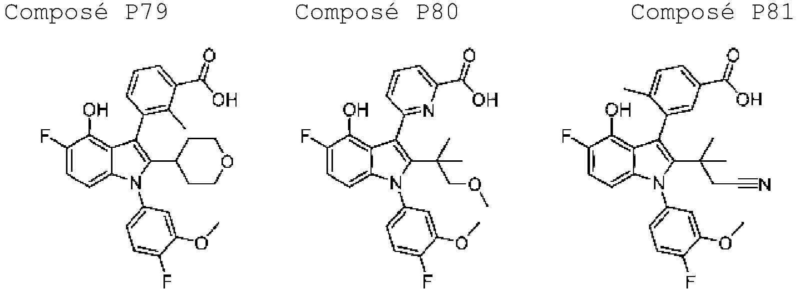 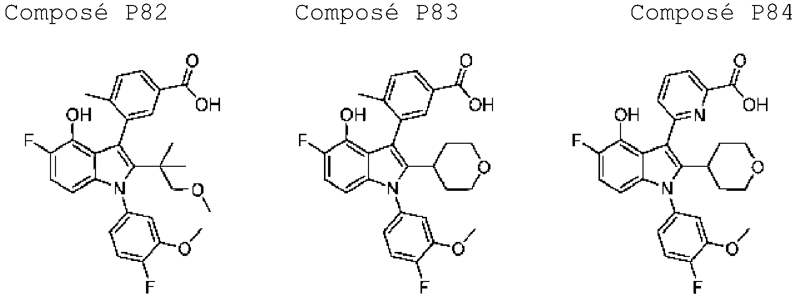 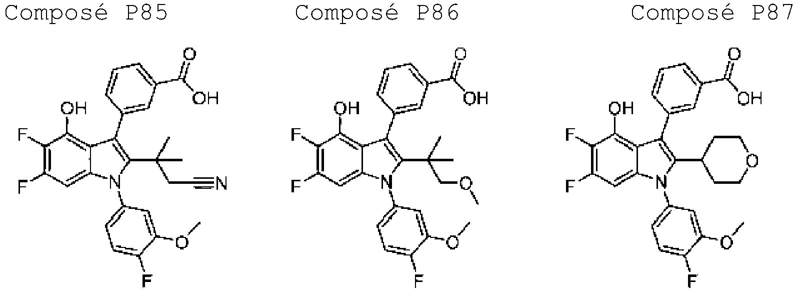 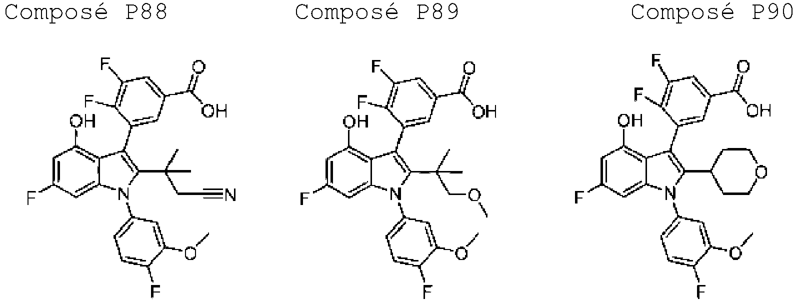 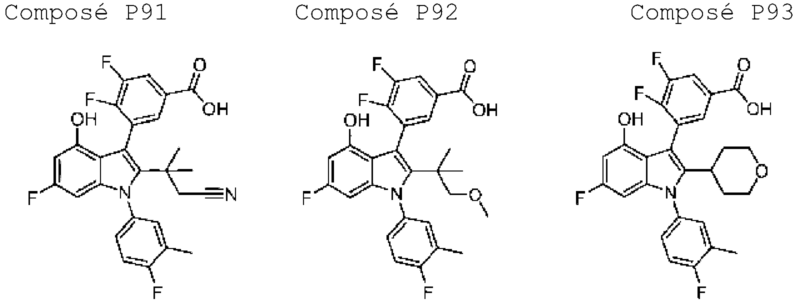 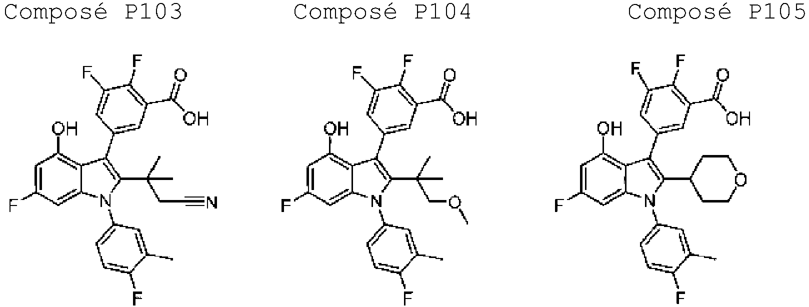 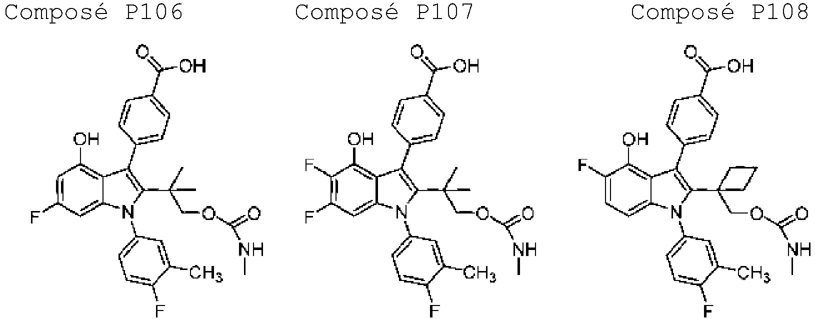 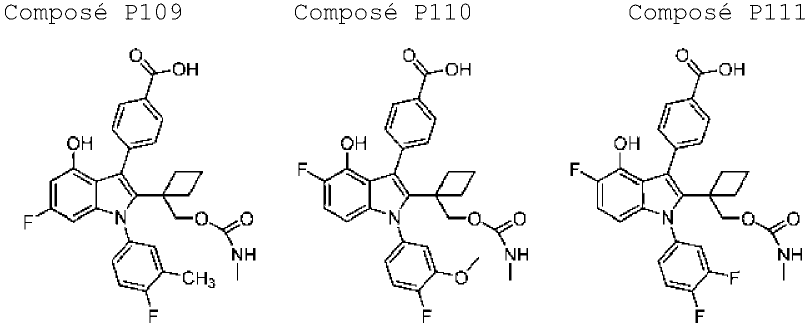 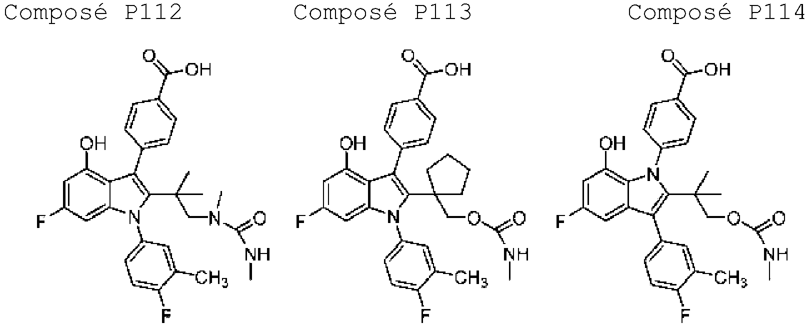 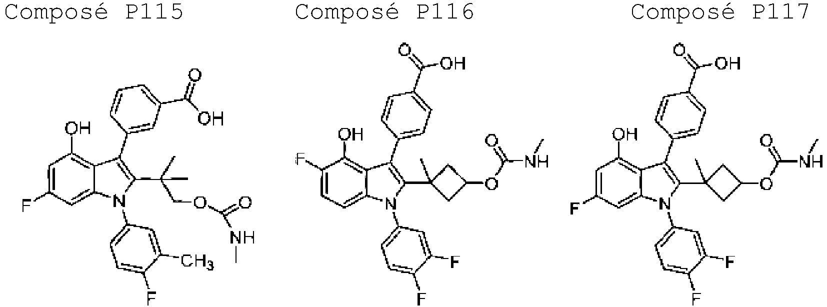 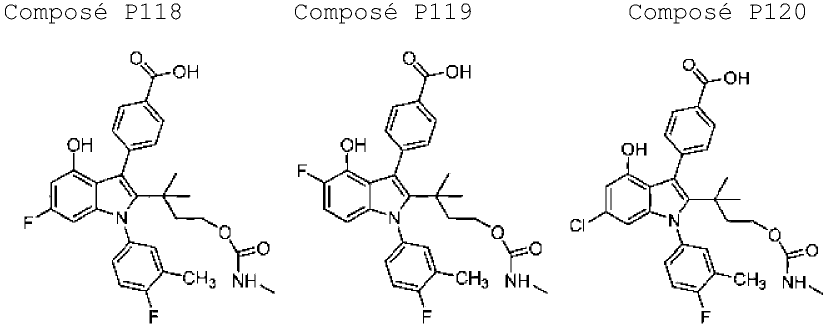 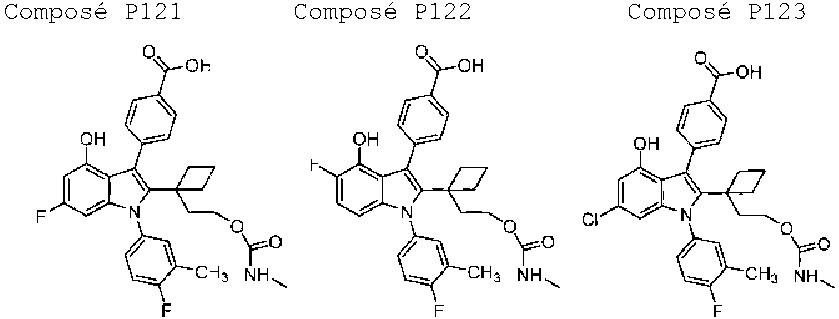 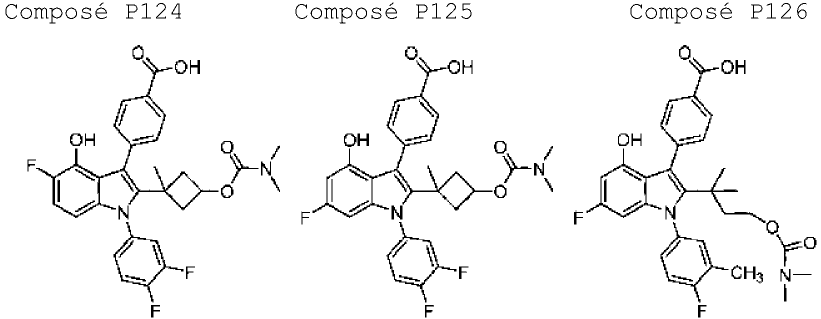 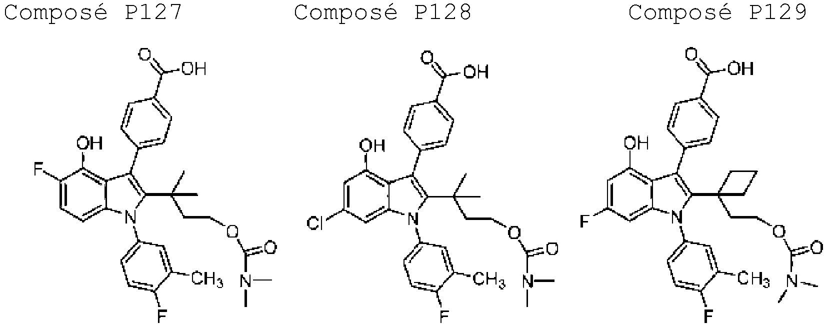 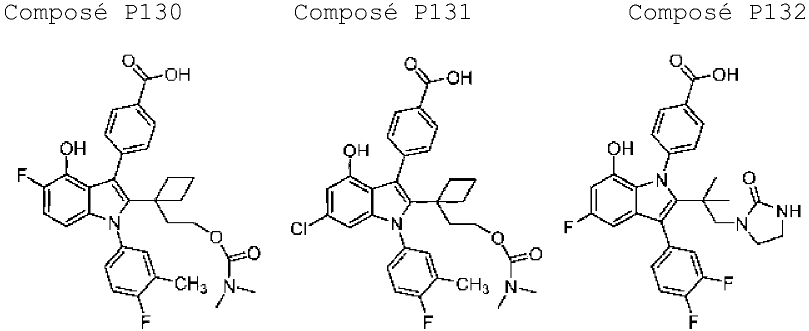 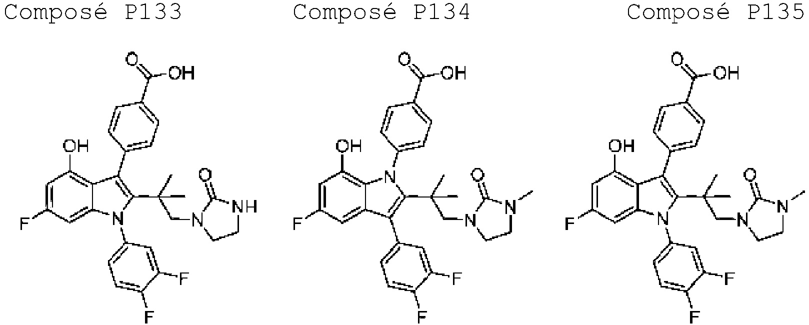 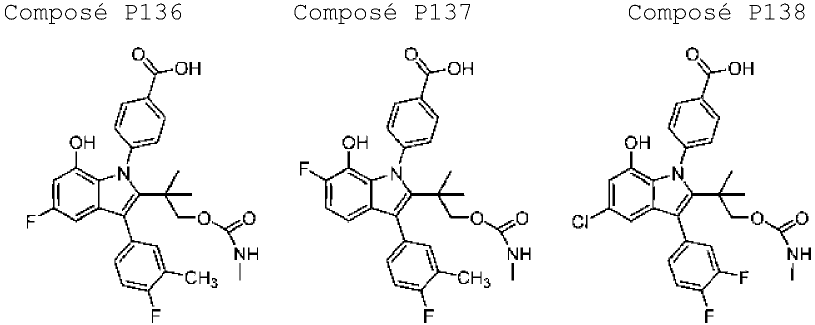 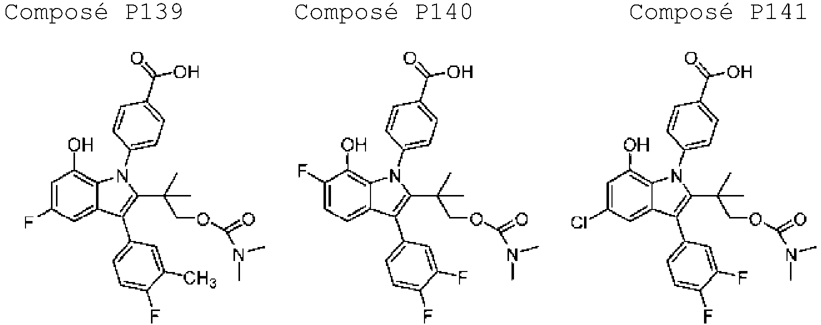 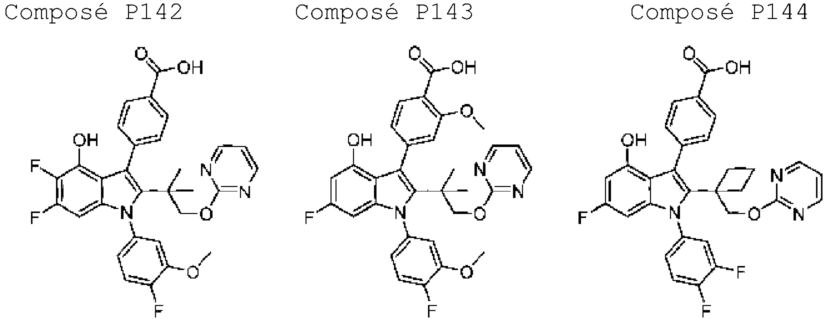 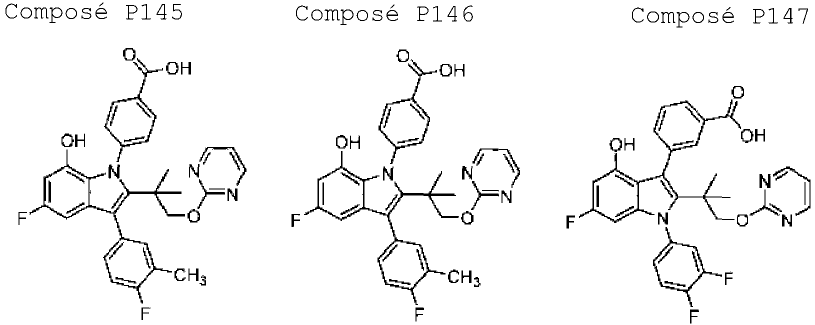 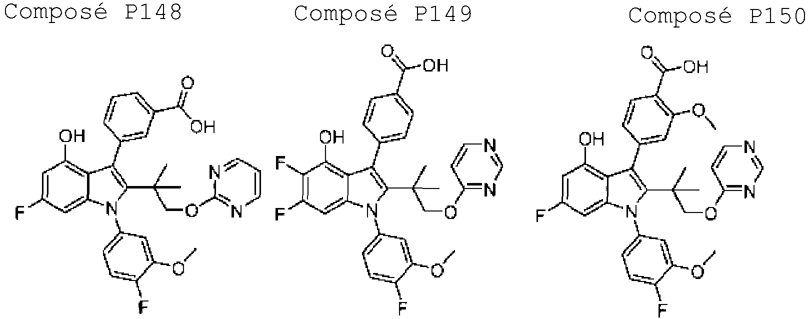 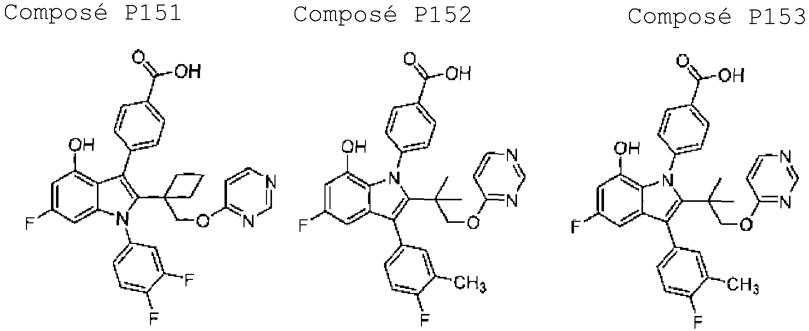 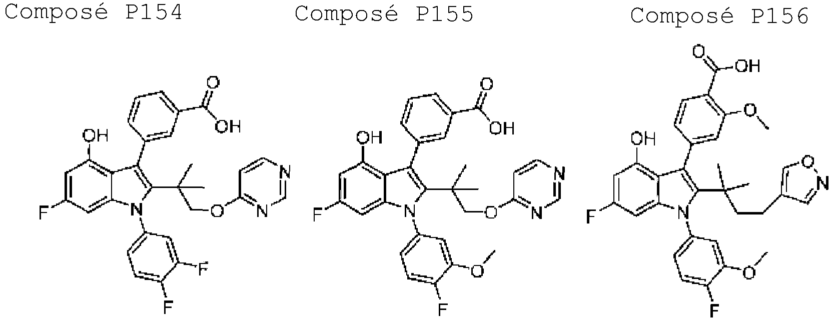 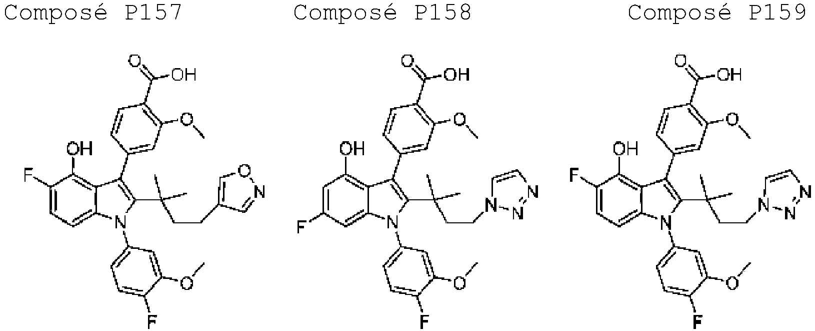 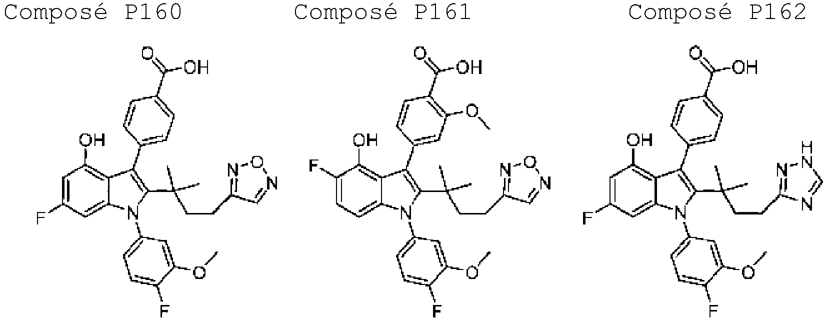 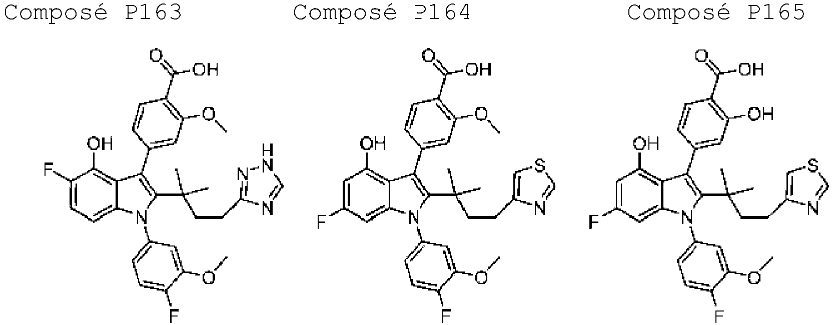 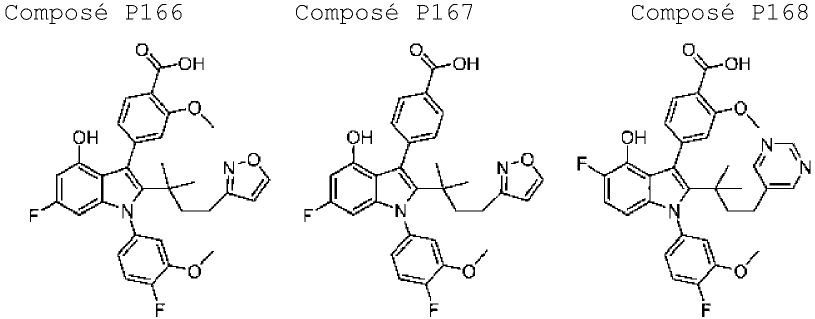 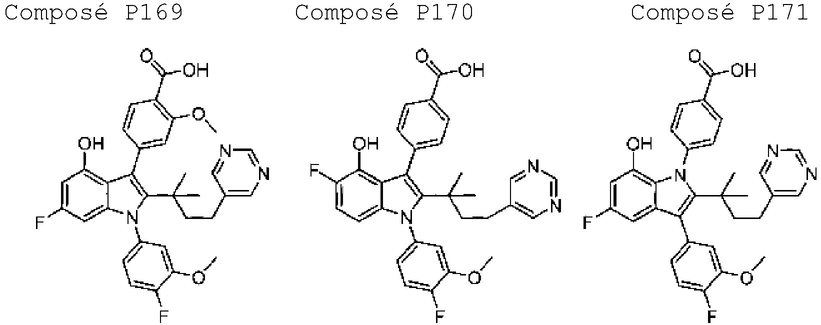 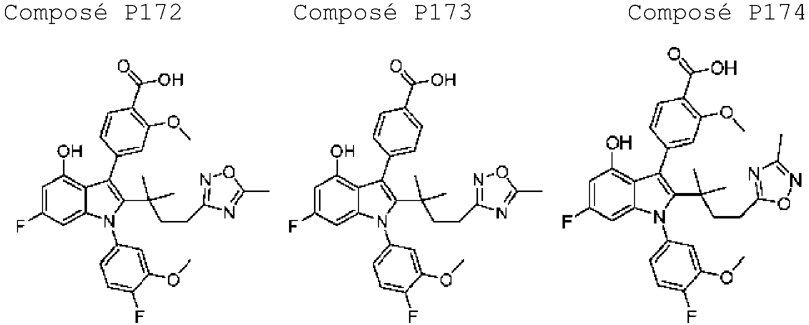 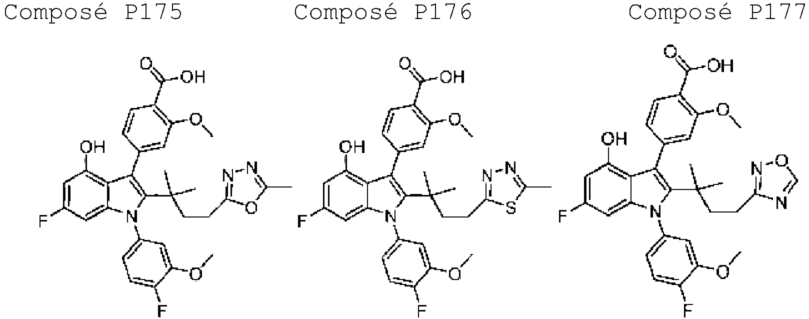 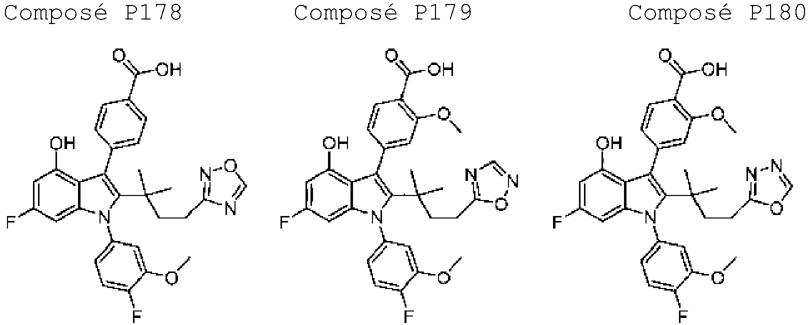 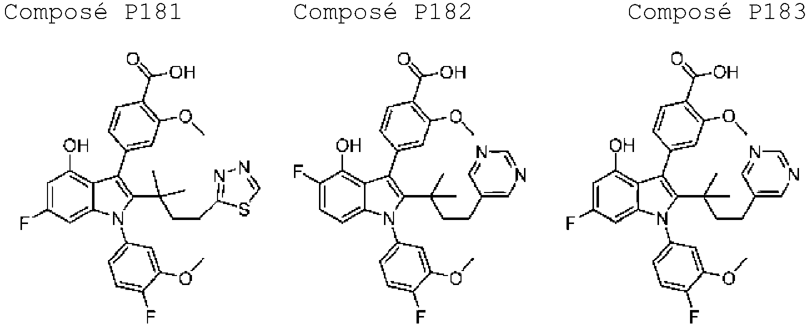 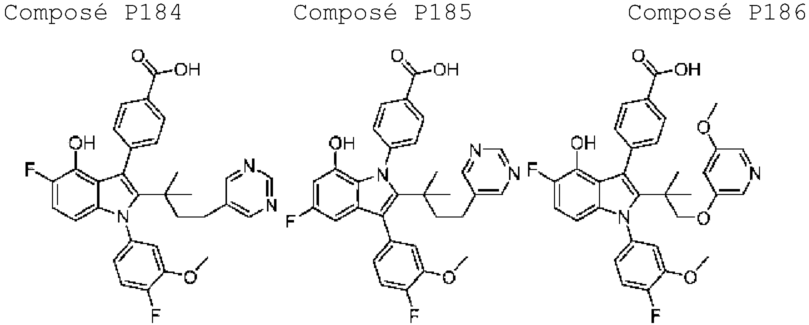 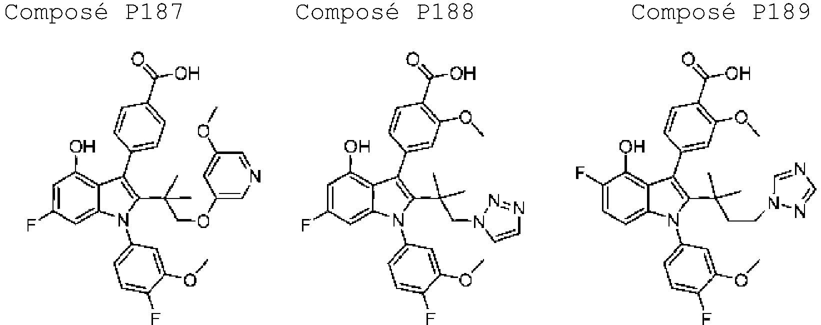 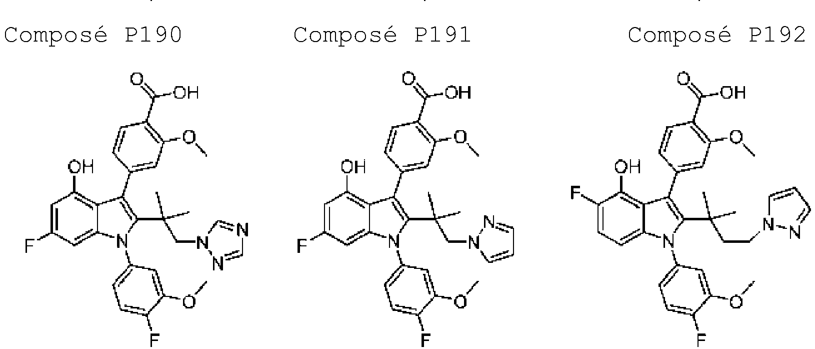 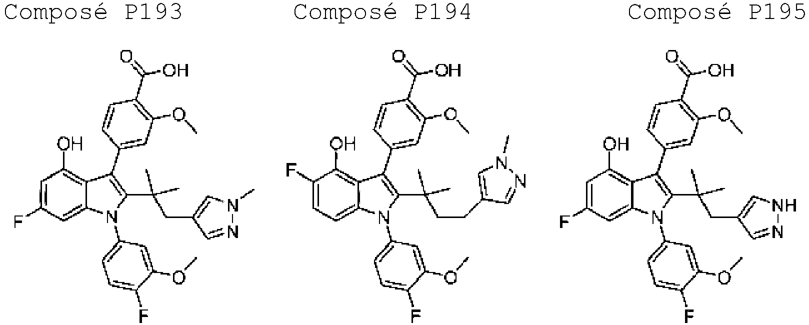 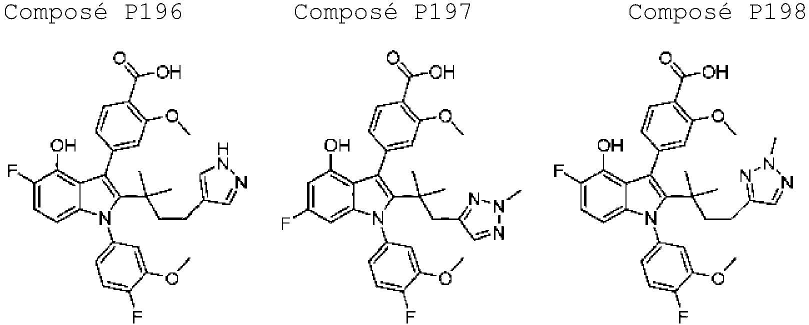 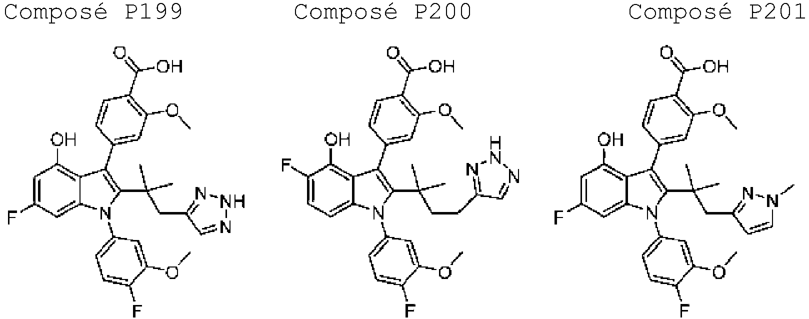 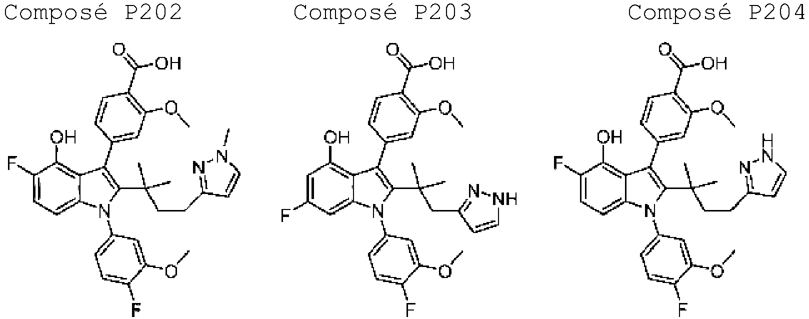 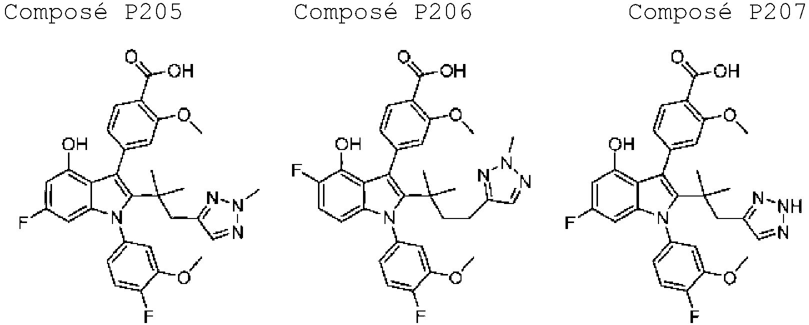 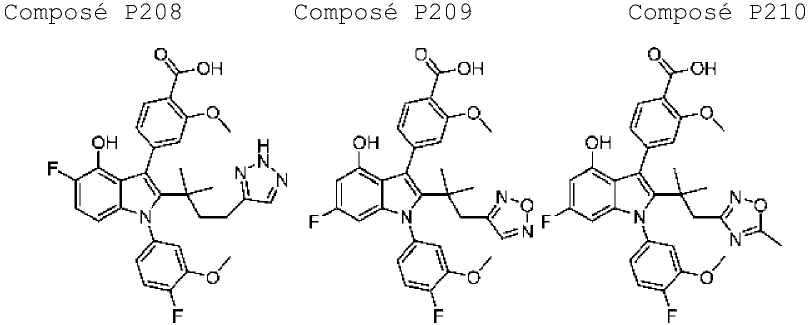 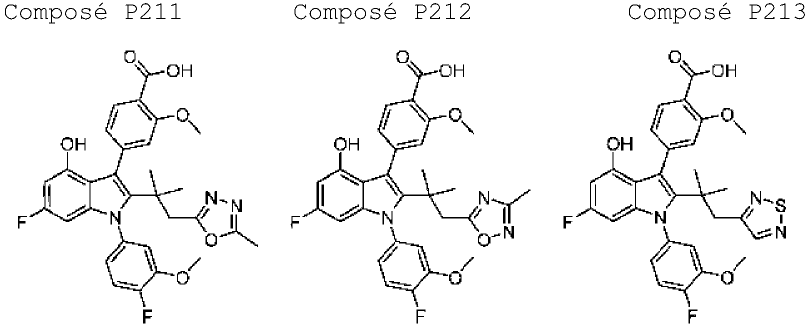 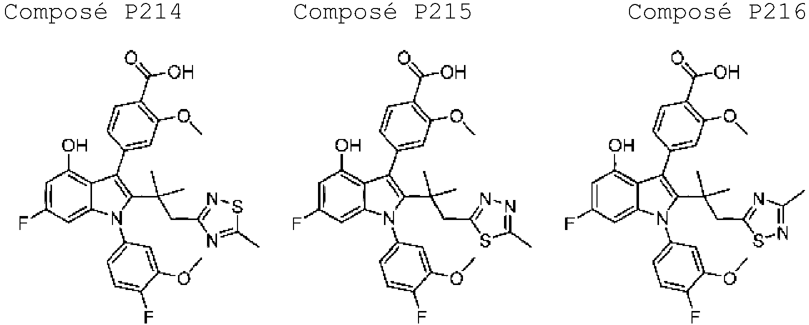 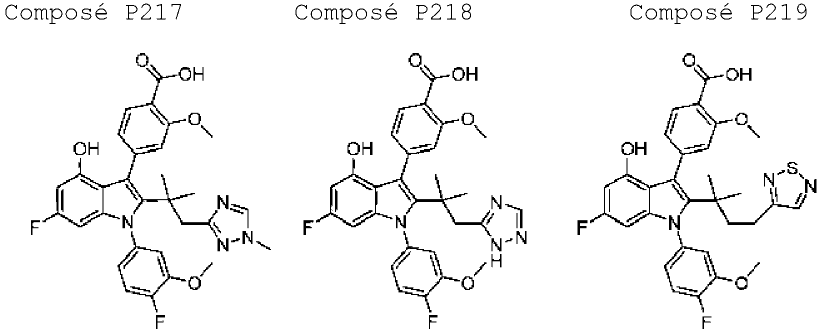 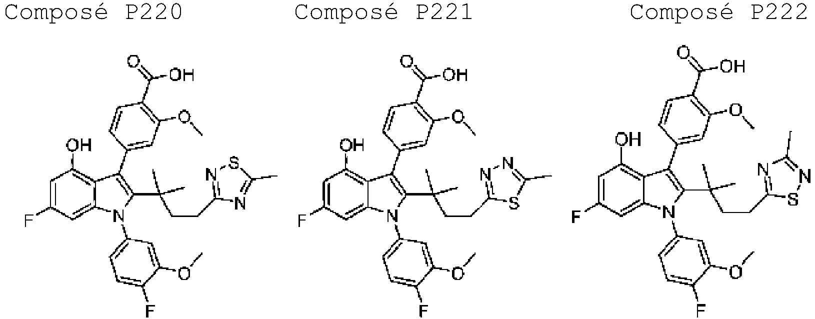 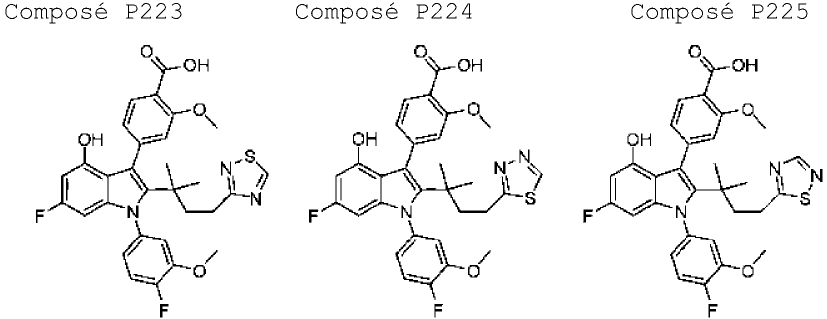 des tautomères de ceux-ci, des dérivés deutérés de ces composés et de ces tautomères, ainsi que des sels pharmaceutiquement acceptables des composés, des tautomères et des dérivés deutérés.

13. Composé sélectionné parmi : 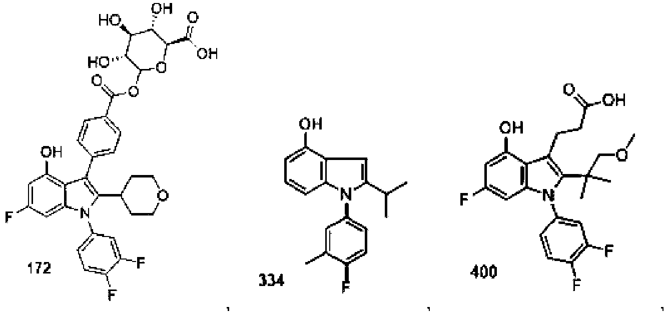 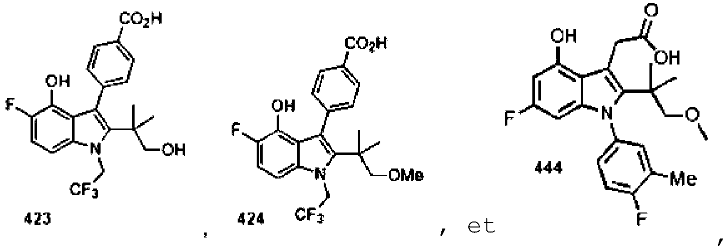 un tautomère de celui-ci, un dérivé deutéré du composé ou du tautomère ou un sel pharmaceutiquement acceptable du composé, du tautomère ou du dérivé deutéré.

14. Composition pharmaceutique comprenant au moins un composé selon l'une quelconque des revendications 1 à 13, un tautomère de celui-ci, un dérivé deutéré de ce composé ou de ce tautomère, ou un sel pharmaceutiquement acceptable des éléments précédents.

15. Composé, tautomère, dérivé deutéré ou sel pharmaceutiquement acceptable selon l'une quelconque des revendications 1 à 13, ou composition pharmaceutique selon la revendication 14, pour une utilisation dans une méthode de traitement d'une carence d'alpha-1-antitrypsin (AAT) comprenant l'administration à un patient qui en a besoin d'une quantité thérapeutiquement efficace d'au moins un composé, tautomère, dérivé deutéré ou sel pharmaceutiquement acceptable selon l'une quelconque des revendications 1 à 13, ou d'une quantité thérapeutiquement efficace d'une composition pharmaceutique selon la revendication 14, de préférence dans lequel ladite quantité thérapeutiquement efficace de l'au moins un composé, tautomère, dérivé deutéré ou sel pharmaceutiquement acceptable est administrée en combinaison avec une thérapie d'augmentation de l'AAT et/ou une thérapie de remplacement de l'AAT.
